(19) Europäisches Patentamt
European Patent Office
Office européen des brevets

(11) EP 3 274 352 B1

(12) # EUROPÄISCHE PATENTSCHRIFT

(45) Veröffentlichungstag und Bekanntmachung des Hinweises auf die Patenterteilung:
**02.01.2019 Patentblatt 2019/01**

(21) Anmeldenummer: **16710764.8**

(22) Anmeldetag: **21.03.2016**

(51) Int Cl.:
*C07D 495/04* (2006.01)
*A61K 31/519* (2006.01)
*A61P 37/00* (2006.01)
*A61P 35/00* (2006.01)
*A61P 11/00* (2006.01)

(86) Internationale Anmeldenummer:
**PCT/EP2016/056106**

(87) Internationale Veröffentlichungsnummer:
**WO 2016/150901 (29.09.2016 Gazette 2016/39)**

(54) **HETEROCYCLYLMETHYL-THIENOURACILE ALS ANTAGONISTEN DES ADENOSIN-A2B-REZEPTORS**

HETEROCYCLYLMETHYL-THIENOURACILE AS ANTAGONISTS OF THE ADENOSINE-A2B-RECEPTOR

HÉTÉROCYCLYLMÉTHYLE-THIÉNOURACILES UTILISÉS COMME ANTAGONISTES DU RÉCEPTEUR A2B DE L'ADÉNOSINE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(30) Priorität: **26.03.2015 EP 15161165**
**30.06.2015 EP 15174566**
**10.09.2015 EP 15184732**

(43) Veröffentlichungstag der Anmeldung:
**31.01.2018 Patentblatt 2018/05**

(73) Patentinhaber: **Bayer Pharma Aktiengesellschaft**
**13353 Berlin (DE)**

(72) Erfinder:
- **HÄRTER, Michael**
  **51375 Leverkusen (DE)**
- **KOSEMUND, Dirk**
  **10245 Berlin (DE)**
- **DELBECK, Martina**
  **42579 Heiligenhaus (DE)**
- **KALTHOF, Bernd**
  **42329 Wuppertal (DE)**
- **WASNAIRE, Pierre**
  **40225 Düsseldorf (DE)**
- **SÜSSMEIER, Frank**
  **80992 München (DE)**
- **LUSTIG, Klemens**
  **42113 Wuppertal (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**WO-A1-00/12514**
**WO-A1-2009/037468**
**WO-A1-2015/052065**
**WO-A2-2007/103776**

EP 3 274 352 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Anmeldung betrifft neue, in 6-Position einen bestimmten Typus von (Aza-Hetero-cyclyl)methyl-Substituenten tragende Thieno[2,3-d]pyrimidin-2,4-dion ("Thienouracil")-Derivate, Verfahren zu ihrer Herstellung, ihre Verwendung allein oder in Kombinationen zur Behandlung und/oder Prävention von Krankheiten sowie ihre Verwendung zur Herstellung von Arzneimitteln zur Behandlung und/oder Prävention von Krankheiten, insbesondere zur Behandlung und/oder Prävention von Erkrankungen der Lunge und des Herz-Kreislauf-Systems sowie von Krebserkrankungen.

Das endogene Purin-Nuldeosid Adenosin wird ubiquitär gebildet und moduliert als wichtiges Signalmolekül eine Vielzahl von physiologischen als auch pathophysiologischen Prozessen. Es entsteht zum größeren Teil beim intra- und extrazellulären Abbau von Adenin-Nukleotiden und zum geringeren Teil bei der intrazellulären Hydrolyse von *S*-Adenosyl-Homocystein. Unter physiologischen Bedingungen kann extrazelluläres Adenosin durch die Adenosinkinase wieder zu Adenosinmonophosphat (AMP) phosphoryliert oder durch die Adenosindeaminase zu Inosin umgebaut werden. Die extrazelluläre Konzentration liegt zwischen 30 und 300 nM. Bei Gewebeschäden, bedingt z.B. durch Hypoxie, bei Entzündungsreaktionen und bei oxidativem Stress kommt es zu einer vermehrten Bildung und Akkumulation von Adenosin, so dass die extrazelluläre Konzentration bis auf 15 μM ansteigen kann.

Die biologischen Wirkungen von Adenosin werden über G-Protein-gekoppelte Plasmamembranständige Rezeptoren vermittelt. Derzeit sind vier Adenosin-Rezeptor-Subtypen nachgewiesen: A1-Adenosin-Rezeptor (A1R), A2a-Adenosin-Rezeptor (A2aR), A2b-Adenosin-Rezeptor (A2bR) und A3-Adenosin-Rezeptor (A3R). Der A2b-Rezeptor hat von den vier oben genannten Adenosin-Rezeptoren die schwächste Affinität für Adenosin. Aus diesem Grunde wird er unter physiologischen Normalbedingungen im Gegensatz zu den anderen Adenosinrezeptoren nicht aktiviert. A1- und A3-Rezeptoren sind an Gi-Proteine gekoppelt, die die Adenylatzyklase hemmen, während A2a- und A2b-Rezeptoren über Gs-Proteine eine Stimulierung der Adenylatzyklase und damit einen intrazellulären Anstieg von cAMP bewirken. Über Gq-Proteine aktivieren sowohl der A1-, der A3- als auch der A2b-Rezeptor die Phospholipase C, welche membranständiges Phosphatidyl-inositol-4,5-bisphosphat in Inositol-1,4,5-triphosphat und Diacylglycerol spaltet. Dies führt wiederum zur Erhöhung der intrazellulären Calcium-Konzentration und Aktivierung weiterer Zielproteine, wie der Proteinkinase C und der MAP-Kinasen.

A2b-Rezeptoren sind auf pulmonalen Epithel- und Glattmuskelzellen, vaskulären Endothel- und Glattmuskelzellen, Fibroblasten sowie Entzündungszellen exprimiert. Die Expression des A2b-Rezeptors an der Zelloberfläche ist ein dynamischer Prozess und wird z.B. durch Hypoxie, inflammatorische Faktoren und freie Radikale stark gesteigert. Die durch Adenosin aktivierten A2b-Rezeptoren führen zur Bildung und Ausschüttung von pro-inflammatorischen und pro-fibrotischen Zytokinen wie z.B. IL-6, IL-4 und IL-8. Studien haben gezeigt, dass der A2b-Rezeptor im chronischen Stadium von Lungenerkrankungen beim Gewebe-Remodeling eine wichtige Rolle spielt und unter anderem die Differenzierung von Fibroblasten in Myofibroblasten fördert, was zu einer verstärkten Synthese und Deposition von Kollagen führt.

In Lungengewebeproben von Patienten mit idiopathischer pulmonaler Fibrose, COPD und pulmonaler Hypertonie assoziiert mit COPD [Zhou et al., PLoS One 5, e9224 (2010); Selmann et al., PLoS One 2, e482 (2007)] sowie in unterschiedlichen Tiermodellen fibro-proliferativer Lungenerkrankungen [Karmouty-Quintana et al., Am. J. Respir. Cell. Mol. Biol., publ. online, 15. Juli 2013; Karmouty-Quintana et al., Faseb J. 26, 2546-2557 (2012); Sun et al., J. Clin. Invest. 116. 2173-2182 (2006)] konnte eine erhöhte Expression des A2b-Rezeptors festgestellt werden. Im Tiermodell der Bleomycin-induzierten Lungenfibrose und pulmonalen Hypertonie an der Maus führte ein genetischer Knock-out des A2b-Rezeptors sowohl zu einer Hemmung der Progression der Lungenfibrose als auch des pulmonalen vaskulären Remodelings und der daraus resultierenden pulmonalen Hypertonie [Karmouty-Quintana et al., Faseb J. 26, 2546-2557 (2012)]. Man vermutet, dass bei der Entwicklung der pulmonalen Hypertonie assoziiert mit Lungenfibrose die durch den A2b-Rezeptor modulierte Freisetzung von unter anderem Endothelin-1 (ET-1) und Interleukin-6 (IL-6) aus den Gefäßzellen eine Rolle spielt. Die Stimulation von humanen pulmonalen arteriellen Endothel- und Glattmuskelzellen mit 5'-(*N*-Ethylcarboxamido)adenosin (NECA), einem Adenosin-Analogon, führt zur Freisetzung von ET-1 und IL-6, die durch A2b-Rezeptor-Hemmung verhindert werden kann [Karmouty-Quintana et al, Faseb J. 26, 2546-2557 (2012)]. Im Lungengewebe und Serum von Patienten mit einer pulmonalen Hypertonie konnten erhöhte Endothelin-1- und IL-6-Spiegel festgestellt werden [Giaid et al., N. Engl. J. Med. 329, 1967-1968 (1993); Steiner et al., Circ. Res. 104. 236-244 (2009)]. Des Weiteren wird vermutet, dass die vom A2b-Rezeptor vermittelte Freisetzung von unter anderem IL-6 und weiteren profibrotischen Mediatoren sowie die Stimulation der Differenzierung von Fibroblasten in Myofibroblasten in der Lunge zur Induktion von Fibrose führt. Die Stimulation von humanen Fibroblasten mit NECA führt zur Freisetzung von IL-6, die durch Hypoxie verstärkt wird und durch A2b-Rezeptor-Hemmung verhindert werden kann. In Patienten mit idiopathischer pulmonaler Fibrose und in Tiermodellen der Lungenfibrose konnte eine erhöhte IL-6-Expression gezeigt werden [Zhong et al., Am. J. Respir. Cell. Mol. Biol. 32,2-8 (2005); Cavarra et al., Am. J. Physiol. Lung Cell. Mol. Physiol. 287, L1186-L1192 (2004)].

Der A2b-Rezeptor spielt auch beim Gewebe-Remodeling nach Myokardinfarkt eine wichtige Rolle. Im Tiermodell der permanenten Ligatur der Koronararterie bei der Maus führte eine Hemmung des A2b-Rezeptors zu einer Reduktion

der Caspase-1-Aktivität und der eingewanderten Entzündungszellen im Herzgewebe sowie der Zytokine und Adhäsionsmoleküle im Plasma und zu einer Verbesserung der systolischen und diastolischen Herzfunktion [Toldo et al., J. Pharmacol. Exp. Ther. 343, 587-595 (2012)].

In Tumoren und tumorumgebenden Geweben ist die lokale Adenosin-Konzentration infolge von auftretender Hypoxie, infolge nekrotischer Prozesse oder auch aufgrund genetischer und epigenetischer Veränderungen von Tumorzellen, die zu einer vermehrten extrazellulären Produktion von Adenosin bei gleichzeitig vermindertem Abbau und verminderter Zellaufnahme von Adenosin führen, häufig stark erhöht [J. Blay et al., Cancer Res. 57 (13), 2602-2605 (1997); G. Schulte, B. B. Fredholm, Cell Signal. 15 (9), 813-827 (2003)]. Dies führt zu einer Aktivierung der zuvor beschriebenen Adenosin-Rezeptoren an Tumorzellen, tumorassoziierten Zellen und Zellen des tumorumgebenden Gewebes. Die dadurch initiierten Signalketten lösen verschiedenartige Prozesse aus, die in ihrer Mehrzahl das Tumorwachstum und seine Verbreitung an andere Orte im Organismus begünstigen. Von daher stellt die Inhibition der Adenosin-Signalwege eine wertvolle Strategie zur Behandlung von Krebserkrankungen dar. So führt beispielsweise die Inhibition des A2b-Rezeptor-vermittelten Adenosin-Signalwegs mit dem A2b-Rezeptor-Antagonisten MRS1754 zu einem verminderten Wachstum von Dickdarmkrebs-Zelllinien [D.-F. Ma et al., Hum. Palhol. 41 (11), 1550-1557 (2010)]. Der A2b-Rezeptor-Antagonist PSB603 vermindert das Wachstum von mehreren Prostatakrebs-Zelllinien [Q. Wei et al., Purinergic Signal. 9 (2), 271-280 (2013)].

Größer als der direkte Einfluss auf die Proliferation von Tumorzellen scheint der Einfluss von Adenosin auf die Tumormetastasierung zu sein. Insbesondere A2b-Rezeptor-vermittelte Adenosin-Signalketten sind daran beteiligt, und die Blockade des A2b-Rezeptors - sowohl genetisch als auch pharmakologisch mit A2b-Rezeptor-Antagonisten - führt zu einer verminderten Migration von Tumorzellen *in vitro* und einer verminderten Bildung von Metastasen in Tiermodellen [J. Stagg et al., Proc. Natl. Acad. Sci. USA 107 (4), 1547-1552 (2010); C. J. Desmet et al., Proc. Natl. Acad. Sci. USA 110 (13), 5139-5144 (2013); E. Ntantie et al., Sci. Signal. 6 (277), ra39 (2013)].

Adenosin hat auch Einfluss auf das tumorassoziierte Gefäßendothel: A2b-Rezeptor-vermittelte Adenosin-Signalketten fuhren zur Freisetzung von pro-angiogenen Faktoren aus verschiedenen humanen Tumorzelllinien, aber auch aus tumorassoziierten Immunzellen, und regen so die das Tumorwachstum fördernde Gefäßneubildung an [S. Ryzhov et al., Neoplasia 10 (9), 987-995 (2008); S. Merighi et al., Mol. Pharmacol. 72 (2), 395-406 (2007); S. Merighi et al., Neoplasia 11 (10), 1064-1073 (2009)].

Zunehmend besser verstanden wird die Bedeutung, die dem Immunsystem bei der Unterdrückung von Tumorentstehung, Tumorwachstum und Metastasierung zukommt. Dabei zeigt sich, dass Adenosin in der Lage ist, die Immunreaktion zu vermindern [S. Gessi et al., Biochim. Biophys. Acta Biomembranes 1808 (5), 1400-1412 (2011); J. Stagg et al., Proc. Natl. Acad. Sci. USA 107 (4), 1547-1552 (2010); D. Jin et al., Cancer Res. 70 (6), 2245-2255 (2010); S. F. M. Häusler et al., Cancer Immunol. Immunother. 60 (10), 1405-1418 (2011); J. Spychala, Pharmacol. Ther. 87 (2-3), 161-173 (2000)]. Die Inhibition des A2b-Rezeptor-vermittelten Adenosin-Signalwegs mit dem A2b-Rezeptor-Antagonisten PSB603 hingegen führt in Melanom-Tiermodellen zu einer Reduktion von Tumorwachstum und Metastasierung, was auf eine Inhibition der tumorinduzierten Suppression des Immunsystems zurückgeführt wird [W. Kaji et al., J. Toxicol. Sci. 39 (2), 191-198 (2014)]. Diese Verbesserung beruht darauf, dass der Anteil von regulatorischen T-Zellen, die die Immunantwort reduzieren, am gesamten Immunzellinfiltrat in Gegenwart des A2b-Rezeptor-Antagonisten verringert ist. Gleichzeitig sind die Populationen an cytotoxischen CD8+ T-Zellen und CD4+ T-Helferzellen erhöht. Darüber hinaus sind immunsupprimierende Effekte von Adenosin auf weitere Zellen des Immunsystems beschrieben (M1- und M2-Makrophagen, dendritische Zellen, myeloide Suppressorzellen), die zum Teil über den A2b-Rezeptor vermittelt werden [B. Csoka et al., FASEB J. 26 (1), 376-386 (2012); S. V. Novitskiy et al., Blood 112 (5), 1822-1831 (2008); M. Yang et al., Immunol. Cell Biol. 88 (2), 165-171 (2010); S. Ryzhov et al., J. Immunol. 187 (11), 6120-6129 (2011)]. In Tiermodellen von Blasen- und Brusttumoren bewirkt der A2b-Rezeptor-Antagonist ATL801 eine Verlangsamung des Tumorwachstums und eine deutliche Reduktion der Metastasierung [C. Cekic et al., J. Immunol. 188 (1), 198-205 (2012)]. Diese Effekte gehen einher mit einer ATL801-induzierten Zunahme der Zahl an Tumorantigen-präsentierenden dendritischen Zellen sowie einer starken Erhöhung des Interferon-γ-Spiegels und in der Folge erhöhten Konzentrationen des Chemokins CXCL10, was wiederum zur Aktivierung von CXCR3+ T-Zellen und letztlich zu einer verbesserten Immunabwehr von Tumorwachstum und Metastasierung führt.

Es wird daher angenommen, dass der A2b-Rezeptor bei vielen Erkrankungen, Verletzungen und pathologischen Veränderungen, deren Entstehung und/oder Progression mit einem entzündlichen Geschehen und/oder einem proliferativen und fibro-proliferativen Gewebe- und Gefäßumbau in Zusammenhang steht, eine wichtige Rolle spielt. Dies können insbesondere Erkrankungen und/ oder Schädigungen der Lunge, des Herz-Kreislauf-Systems oder der Niere sein, oder es kann sich hierbei um eine Erkrankung des Blutes, eine Krebs-Erkrankung oder um andere entzündliche Erkrankungen handeln.

In diesem Zusammenhang zu nennende Erkrankungen und Schädigungen der Lunge sind insbesondere die idiopathische Lungenfibrose, die pulmonale Hypertonie, das Bronchiolitis obliterans-Syndrom (BOS), die chronisch-obstruktive Lungenerkrankung (COPD), Asthma und zystische Fibrose. Erkrankungen und Schädigungen des Herz-Kreislauf-Systems, in denen der A2b-Rezeptor involviert ist, sind zum Beispiel Gewebeveränderungen nach einem

Myokardinfarkt und bei der Herzinsuffizienz. Erkrankungen der Niere sind zum Beispiel Niereninsuffizienz und Nierenversagen. Eine Erkrankung des Blutes ist zum Beispiel die Sichelzellanämie. Beispiele für einen Gewebeab- und -umbau bei Krebsprozessen sind das Einwandern von Krebszellen in das gesunde Gewebe (Metastasenbildung) und die Neuausbildung von versorgenden Blutgefäßen (Neo-Angiogenese). Eine andere entzündliche Krankheit, bei denen der A2b-Rezeptor eine Rolle spielt, ist zum Beispiel die Multiple Sklerose.

Die idiopathische Lungenfibrose oder idiopathische pulmonale Fibrose (IPF) ist eine progrediente Lungenerkrankung, die unbehandelt durchschnittlich innerhalb von 2.5 bis 3.5 Jahren nach Diagnosestellung zum Tode führt. Die Patienten sind zum Zeitpunkt der Diagnosestellung meist älter als 60 Jahre, und Männer sind etwas häufiger betroffen als Frauen. Der Krankheitsbeginn der IPF ist schleichend und durch eine zunehmende Atemnot und trockenen Reizhusten gekennzeichnet. IPF gehört zur Gruppe der idiopathischen interstitiellen Pneumonien (IIP), einer heterogenen Gruppe von Lungenerkrankungen, die durch Fibrose und Inflammation unterschiedlichen Grades charakterisiert sind und die mit Hilfe klinischer, bildgebender und feingeweblicher Kriterien unterschieden werden. Innerhalb dieser Gruppe hat die idiopathische pulmonale Fibrose aufgrund ihrer Häufigkeit und des aggressiven Verlaufs eine besondere Bedeutung [Ley et al., Am. J. Respir. Crit. Care Med. 183, 431-440 (2011)]. Die IPF kann entweder sporadisch oder familiär gehäuft auftreten. Die Ursachen sind derzeit nicht geklärt. In den letzten Jahren wurden jedoch zahlreiche Hinweise dafür gefunden, dass eine chronische Schädigung des Alveolarepithels zur Freisetzung von profibrotischen Zytokinen/Mediatoren führt, gefolgt von einer gesteigerten Fibroblastenproliferation und einer vermehrten Kollagenfaserbildung, wodurch es zu einer fleckenförmigen Fibrose und der typischen honigwabenartigen Struktur der Lunge kommt [Strieter et al.,Chest 136, 1364-1370 (2009)]. Die klinischen Folgen der Fibrosierung sind eine Abnahme der Elastizität des Lungengewebes, eine Verminderung der Diffusionskapazität sowie die Entwicklung einer schweren Hypoxie. Lungenfunktionell kann entsprechend eine Verschlechterung der forcierten Vitalkapazität (FVC) und der Diffusionskapazität (DLCO) festgestellt werden. Wesentliche und prognostisch bedeutende Komorbiditäten der IPF sind die akute Exazerbation und die pulmonale Hypertonie [Beck et al., Pneumologe 10, 105-111 (2013)]. Die Prävalenz der pulmonalen Hypertonie bei interstitiellen Lungenerkrankungen liegt bei 10-40% [Lettieri et al., Chest 129, 746-752 (2006); Behr et al., Eur. Respir. J. 31, 1357-1367 (2008)]. Es gibt gegenwärtig keine kurative Behandlung für die IPF - mit Ausnahme der Lungentransplantation.

Die Pulmonale Hypertonie (PH) ist eine progrediente Lungenerkrankung, die unbehandelt durchschnittlich innerhalb von 2.8 Jahren nach Diagnosestellung zum Tode führt. Definitionsgemäß liegt bei einer chronischen pulmonalen Hypertonie ein pulmonal-arterieller Mitteldruck (mPAP) von > 25 mmHg in Ruhe oder > 30 mmHg unter Belastung vor (Normalwert < 20 mmHg). Die Pathophysiologie der pulmonalen Hypertonie ist gekennzeichnet durch Vasokonstriktion und ein Remodeling der Pulmonalgefäße. Bei der chronischen PH kommt es zu einer Neomuskularisierung primär nicht nmskularisierter Lungengefäße, und die Gefäßmuskulatur der bereits muskularisierten Gefäße nimmt an Umfang zu. Durch diese zunehmende Obliteration der Lungenstrombahn kommt es zu einer progredienten Belastung des rechten Herzens, die zu einer verminderten Auswurfleistung des rechten Herzens führt und letztlich in einem Rechtsherzversagen endet [M. Humbert et al., J. Am. Coll. Cardiol. 2004, 43, 13S-24S]. Wenn auch die idiopathische (oder primäre) pulmonal-arterielle Hypertonie (IPAH) eine sehr seltene Erkrankung ist, so ist die sekundäre pulmonale Hypertonie (non-PAH PH, NPAHPH) weit verbreitet, und es wird zur Zeit angenommen, dass PH die dritthäufigste kardiovaskuläre Krankheitsgruppe nach koronarer Herzkrankheit und systemischem Bluthochdruck ist [Naeije, in: A. J. Peacock et al. (Eds.), Pulmonary Circulation. Diseases and their treatment, 3rd edition, Hodder Arnold Publ., 2011, S. 3]. Die Einteilung der pulmonalen Hypertonie in verschiedene Gruppen gemäß der jeweiligen Ätiologie erfolgt seit 2008 nach der Dana Point-Klassifikation [D. Montana und G. Simonneau, in: A. J. Peacock et al. (Eds.), Pulmonary Circulation. Diseases and their treatment, 3rd edition, Hodder Arnold Publ., 2011, S. 197-206].

Trotz aller Fortschritte in der Therapie der PH gibt es bisher keine Aussicht auf Heilung dieser schwerwiegenden Erkrankung. Auf dem Markt befindliche Therapien (z.B. Prostacyclin-Analoga, Endothelinrezeptor-Antagonisten, Phosphodiesterase-Inhibitoren) sind in der Lage, die Lebensqualität, die körperliche Belastbarkeit und die Prognose der Patienten zu verbessern. Hierbei handelt es sich um systemisch applizierte, primär hämodynamisch wirkende Therapieprinzipien, die den Gefäßtonus beeinflussen. Die Anwendbarkeit dieser Medikamente ist durch die z. T. gravierenden Nebenwirkungen und/oder aufwendigen Applikationsformen eingeschränkt. Der Zeitraum, über den unter einer spezifischen Monotherapie die klinische Situation der Patienten stabilisiert oder verbessert werden kann, ist begrenzt (z.B. aufgrund einer Toleranzentwicklung). Es erfolgt schließlich eine Therapieeskalation und somit eine Kombinationstherapie, bei der mehrere Medikamente gleichzeitig gegeben werden müssen. Zur Zeit sind diese Standardtherapeutika nur zur Behandlung der pulmonal-arteriellen Hypertonie (PAH) zugelassen. Bei sekundären Formen der PH, wie z.B. PH-COPD, scheiterten diese Therapieprinzipien (z.B. Sildenafil, Bosentan) in klinischen Studien, da sie infolge einer unselektiven Vasodilatation zu einer Absenkung (Entsättigung) des arteriellen Sauerstoffgehalts bei den Patienten führten. Ursache hierfür ist wahrscheinlich eine ungünstige Beeinflussung der Ventilations-Perfusions-Anpassung innerhalb der Lunge bei heterogenen Lungenerkrankungen aufgrund der systemischen Gabe unselektiver Vasodilatatoren [I. Blanco et al., Am. J. Respir. Crit. Care Med. 2010, 181, 270-278; D. Stolz et al., Eur. Respir. J. 2008, 32, 619-628].

Neue Kombinationstherapien sind eine der aussichtsreichsten zukünftigen Therapieoptionen zur Behandlung

der pulmonalen Hypertonie. In diesem Zusammenhang ist die Erkundung neuer pharmakologischer Mechanismen zur Behandlung der PH von besonderem Interesse [Ghofrani et al., Herz 2005, 30, 296-302; E. B. Rosenzweig, Expert Opin. Emerging Drugs 2006, 11, 609-619; T. Ito et al., Curr. Med. Chem. 2007, 14, 719-733]. Vor allem solche neuen Therapieansätze, die mit den bereits auf dem Markt befindlichen Therapiekonzepten kombinierbar sind, könnten Grundlage einer effizienteren Behandlung sein und somit einen großen Vorteil für die Patienten bringen.

Im Sinne der vorliegenden Erfindung schließt der Begriff pulmonale Hypertonie sowohl primäre als auch sekundäre Unterformen (NPAHPH) ein, wie sie nach der Dana Point-Klassifikation gemäß ihrer jeweiligen Ätiologie definiert worden sind [D. Montana und G. Simonneau, in: A. J. Peacock et al. (Eds.), Pulmonary Circulation. Diseases and their treatment, 3rd edition, Hodder Arnold Publ., 2011, S. 197-206; Hoeper et al., J. Am. Coll. Cardiol., 2009, 54 (1), Suppl. S, S85-S96]. Hierzu gehört insbesondere in Gruppe 1 die pulmonal-arterielle Hypertonie (PAH), zu der unter anderem die idiopathischen und die familiären Formen zählen (IPAH bzw. FPAH). Des weiteren umfasst PAH auch die persistierende pulmonale Hypertonie bei Neugeborenen sowie die assoziierte pulmonal-arterielle Hypertonie (APAH), welche assoziiert ist mit Kollagenosen, kongenitalen systemisch-pulmonalen Shuntvitien, portaler Hypertension, HIV-Infektionen, der Einnahme bestimmter Drogen und Medikamente (z.B. von Appetitzüglern), mit Erkrankungen mit einer signifikanten venösen/kapillären Beteiligung wie der pulmonal-venookklusiven Erkrankung und der pulmonal-kapillären Hämangiomatose, oder mit anderen Erkrankungen wie Schilddrüsenerkrankungen, Glykogenspeicherkrankheiten, Morbus Gaucher, hereditärer Teleangiektasie, Hämoglobinopathien, myeloproliferativen Erkrankungen und Splenektomie. In Gruppe 2 der Dana Point-Klassifikation werden PH-Patienten mit einer ursächlichen Linksherzerkrankung, wie ventrikulären, atrialen oder valvulären Erkrankungen, zusammengefasst. Gruppe 3 umfasst Formen der pulmonalen Hypertonie, die mit einer Lungenerkrankung, wie z.B. chronisch-obstruktiver Lungenerkrankung (COPD), interstitieller Lungenkrankheit (ILD), Lungenfibrose (IPF), und/oder einer Hypoxämie (z.B. Schlafapnoe-Syndrom, alveoläre Hypoventilation, chronische Höhenkrankheit, anlagebedingte Fehlbildungen) assoziiert sind. Zur Gruppe 4 zählen PH-Patienten mit chronischthrombotischen und/oder embolischen Erkrankungen, z.B. bei thromboembolischer Obstruktion von proximalen und distalen Lungenarterien (CTEPH) oder bei nicht-thrombotischen Embolisierungen (z.B. infolge von Tumorerkrankungen, Parasiten, Fremdkörpern). Seltenere Formen der pulmonalen Hypertonie, wie z.B. bei Patienten mit Sarkoidose, Histiozytose X oder Lymphangiomatose, sind in der Gruppe 5 zusammengefasst.

Beim Bronchiolitis obliterans-Syndrom (BOS) handelt es sich um eine chronische Abstoßungsreaktion nach erfolgter Lungentransplantation. Innerhalb der ersten fünf Jahre nach Lungentransplantation sind ca. 50-60% aller Patienten, innerhalb der ersten neun Jahre über 90% der Patienten betroffen [Estenne et al., Am. J. Respir. Crit. Care Med. 166, 440-444 (2003)]. Die Ursache der Erkrankung ist nicht geklärt. Trotz vieler Fortschritte bei der Behandlung von Transplantationspatienten haben sich die BOS-Fallzahlen in den vergangenen Jahren kaum verändert. Das BOS ist die wichtigste langfristige Komplikation bei Lungentransplantationen und gilt als Hauptgrund dafür, dass die Überlebensraten nach wie vor deutlich unter denen anderer Organtransplantationen liegen. Beim BOS handelt es sich um ein entzündliches Geschehen, das mit Veränderungen des Lungengewebes einhergeht, die vor allem die kleinen Atemwege betreffen. Die Schädigung und entzündlichen Veränderungen der Epithelzellen sowie der subepithelialen Strukturen der kleineren Atemwege führen aufgrund einer nicht effektiven Regeneration des Epithels und einer aberrierenden Gewebereparation zu einer exzessiven Fibroproliferation. Es kommt zur Vernarbung und schließlich Zerstörung der Bronchiolen sowie zu Pfropfen von Granulationsgewebe in den kleinen Atemwegen und Alveolen, gelegentlich auch mit vaskulärer Beteiligung. Die Diagnose wird aufgrund der Lungenfunktion gestellt. Beim BOS kommt es zu einer Verschlechterung des FEV1 im Vergleich zum Durchschnitt der zwei besten postoperativ gemessenen Werte. Gegenwärtig gibt es keine kurative Behandlung für BOS. Ein Teil der Patienten verbessert sich unter intensivierter Immunsuppression, bei den nicht darauf ansprechenden Patienten kommt es zu einer anhaltenden Verschlechterung, so dass eine erneute Transplantation (Retransplantation) indiziert ist.

Die chronisch-obstruktive Lungenerkrankung (COPD) ist eine langsam fortschreitende Lungenerkrankung, die durch eine Behinderung der Atemströmung charakterisiert ist, welche durch ein Lungenemphysem und/oder eine chronische Bronchitis hervorgerufen wird. Die ersten Symptome der Erkrankung zeigen sich in der Regel ab dem vierten bis fünften Lebensjahrzehnt. In den darauffolgenden Lebensjahren verschlimmert sich häufig die Kurzatmigkeit und es manifestiert sich Husten, verbunden mit einem ausgiebigen und stellenweise eitrigen Auswurf und einer Stenose-Atmung bis hin zu einer Atemnot (Dyspnoe). COPD ist in erster Linie eine Krankheit von Rauchern: Rauchen ist verantwortlich für 90% aller COPD-Fälle und 80-90% aller COPD-Todesfälle. COPD ist ein großes medizinisches Problem und stellt weltweit die sechsthäufigste Todesursache dar. Von den über 45-jährigen Menschen sind ca. 4-6% betroffen. Obwohl die Behinderung der Atemströmung nur partiell und zeitlich befristet sein kann, ist COPD nicht heilbar. Behandlungsziel ist folglich eine Verbesserung der Lebensqualität, die Linderung der Symptome, die Verhinderung akuter Verschlechterungen und die Verlangsamung der fortschreitenden Beeinträchtigung der Lungenfunktion. Bestehende Phannakotherapien, die sich seit den letzten zwei bis drei Jahrzehnten kaum geändert haben, sind das Verwenden von Bronchodilatoren, um blockierte Atemwege zu öffnen, und in bestimmten Situationen Kortikosteroide, um die Entzündung der Lunge einzudämmen [P. J. Barnes, N. Engl. J. Med. 343, 269-280 (2000)]. Die chronische Entzündung der Lunge, hervorgerufen durch Zigarettenrauch oder andere Reizstoffe, ist die treibende Kraft der Krankheitsentwicklung. Der

zugrunde liegende Mechanismus beinhaltet Immunzellen, die im Zuge der inflammatorischen Reaktion der Lunge Proteasen und verschiedene Zytokine ausschütten, die zu einem Lungenemphysem und Remodeling der Bronchien führen.

Die Aufgabe der vorliegenden Erfindung liegt in der Bereitstellung neuer Substanzen, die als potente und selektive Antagonisten des Adenosin-A2b-Rezeptors wirken und sich als solche zur Behandlung und/oder Prävention insbesondere von Erkrankungen der Lunge und des Herz-Kreislauf-Systems sowie von Krebserkrankungen eignen.

Aus WO 2009/037468-A1 sind 2-Aminothieno[3,2-d]pyrimidin-4-carboxamide als Adenosin-A2b-Antagonisten zur Behandlung von Asthma, COPD, Diabetes und Krebs bekannt. Als Antagonisten des Adenosin A2a-Rezeptors, welche insbesondere zur Behandlung von ZNS- und Sucht-Erkrankungen geeignet sind, werden in WO 2007/103776-A2 6-Heteroaryl-substituierte und in WO 2008/ 070529-A2 6-Styryl-substituierte Thieno[2,3-d]pyrimidin-2,4-dione beschrieben. In WO 98/54190-A1, WO 00/12514-A1, GB 2 363 377-A und US 2004/0122028-A1 werden verschiedene Thieno-[2,3-d]pyrimidin-2,4-dione offenbart, welche unter anderem zur Behandlung von inflammatorischen und proliferativen Erkrankungen eingesetzt werden können. Aus US 6 140 325 sind Carboxylat-substituierte Thieno[2,3-d]pyrimidin-2,4-dione als Endothelin-Rezeptor-Antagonisten bekannt. In WO 00/61583-A1 werden Xanthin-Analoga beansprucht, welche sich zur Behandlung von inflammatorischen, neurodegenerativen und Autoimmun-Erkrankungen eignen. In WO 02/ 064598-A1 und WO 2004/014916-A1 werden bicyclische Pyrimidindione als Inhibitoren von Matrix-Metalloproteinasen (MMPs), insbesondere von MMP-13, beschrieben. In WO 2013/ 071169-A1, WO 2014/182943-A1 und WO 2014/182950-A1 wurden vor kurzem Thieno[2,3-d]-pyrimidin-2,4-dione als ACC-Inhibitoren zur Behandlung von Infektionen und metabolischen Erkrankungen offenbart.

Gegenstand der vorliegenden Erfindung sind Verbindungen der allgemeinen Formel (I)

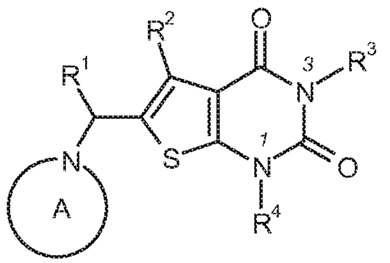

(I),

in welcher
der Ring A für einen Aza-Heterocyclus der Formel

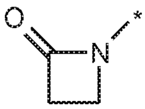 , 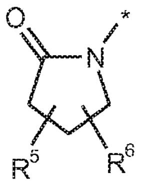 , 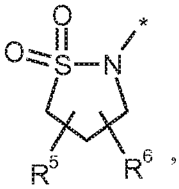 , 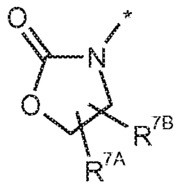 , 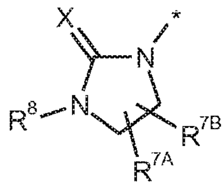 ,

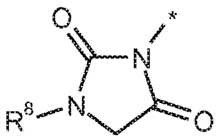 , 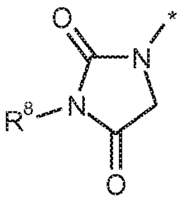 , 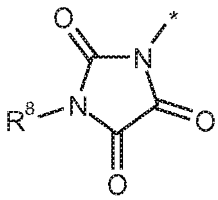 , 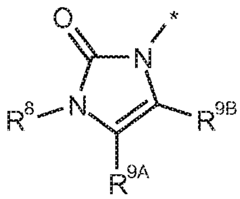 ,

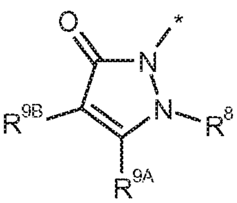 , 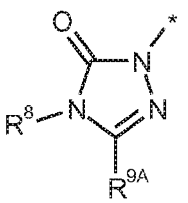 , 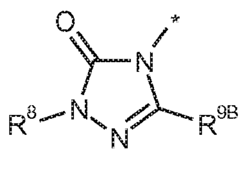 , 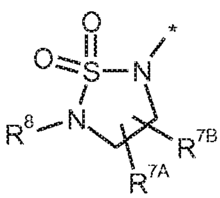 ,

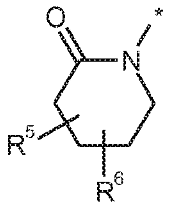 , 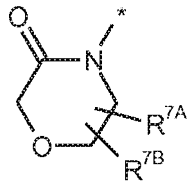 , 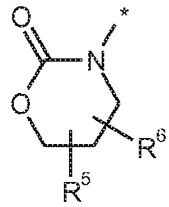 , 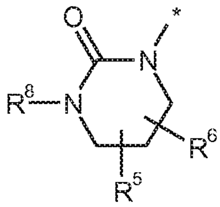 ,

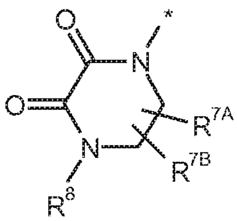 , 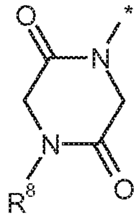 oder 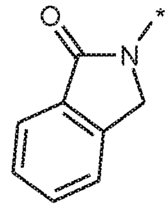

steht,
worin * die Verknüpfung zur angrenzenden $CH(R^1)$-Gruppe markiert,

$R^5$ Wasserstoff, $(C_1-C_4)$-Alkyl, Hydroxy, $(C_1-C_4)$-Alkoxy, Amino, $(C_1-C_5)$-Alkanoylamino oder $(C_1-C_4)$-Alkoxycarbonylamino bedeutet,

$R^6$ Wasserstoff, Methyl oder Ethyl bedeutet,

$R^{7A}$ und $R^{7B}$ gleich oder verschieden sind und unabhängig voneinander Wasserstoff oder $(C_1-C_4)$-Alkyl bedeuten,

$R^8$ Wasserstoff, $(C_1-C_4)$-Alkyl, $(C_2-C_4)$-Alkenyl, $(C_1-C_5)$-Alkanoyl oder $(C_1-C_4)$-Alkoxycarbonyl bedeutet, wobei $(C_1-C_4)$-Alkyl bis zu zweifach mit Hydroxy substituiert sein kann,

$R^{9A}$ und $R^{9B}$ gleich oder verschieden sind und unabhängig voneinander Wasserstoff oder $(C_1-C_4)$-Alkyl bedeutenund

X O, $N(R^{10})$ oder S bedeutet, worin

$R^{10}$ Wasserstoff, Cyano oder $(C_1-C_4)$-Alkoxycarbonyl darstellt,

$R^1$ für Wasserstoff oder Methyl steht,

$R^2$ für Wasserstoff, Methyl oder Ethyl steht, wobei Methyl und Ethyl bis zu dreifach mit Fluor substituiert sein können,

$R^3$ für $(C_2-C_6)$-Alkyl, $(C_2-C_6)$-Alkenyl oder $(C_2-C_6)$-Alkinyl steht,
wobei $(C_2-C_6)$-Alkyl mit einem Rest ausgewählt aus der Reihe Hydroxy, Methoxy, Ethoxy, Trifluormethoxy, Cyclopropyl, Cyclobutyl, Oxetanyl und Phenyl sowie bis zu dreifach mit Fluor substituiert sein kann und
$(C_2-C_6)$-Alkenyl bis zu dreifach mit Fluor substituiert sein kann,
wobei die genannten Cyclopropyl- und Cyclobutyl-Gruppen ihrerseits bis zu zweifach, gleich oder verschieden, mit einem Rest ausgewählt aus Fluor und Methyl substituiert sein können,

oder

$R^3$ für eine Gruppe der Formel $-CH_2-R^{14}$ steht, worin
$R^{14}$ Cyclopropyl, Cyclobutyl, Oxetanyl oder Tetrahydrofuranyl bedeutet,
wobei Cyclopropyl, Cyclobutyl und Oxetanyl bis zu zweifach, gleich oder verschieden, mit einem Rest ausgewählt aus Fluor und Methyl substituiert sein können,

und

$R^4$ für $(C_1-C_6)$-Alkyl oder $(C_2-C_6)$-Alkenyl steht,
wobei $(C_1-C_6)$-Alkyl bis zu fünffach und $(C_2-C_6)$-Alkenyl bis zu dreifach mit Fluor substituiert sein können und
wobei in $(C_1-C_6)$-Alkyl eine $CH_2$-Gruppe gegen -O-, -S- oder $-S(O)_2-$ ausgetauscht sein kann, mit der Maßgabe, dass sich zwischen einem solchen Heteroatom und dem Uracil-$N^1$-Atom mindestens zwei Kohlenstoffatome befinden,

oder

$R^4$ für eine Gruppe der Formel $-(CH_2)_m-CN$, $-(CH_2)_n-R^{11}$ oder $4CH_2)_p-R^{12}$ steht, worin
m die Zahl 1, 2, 3 oder 4 bedeutet,
n die Zahl 2 oder 3 bedeutet,
p die Zahl 1 oder 2 bedeutet,
$R^{11}$ Dimethylamino, Diethylamino oder Azetidino bedeutetund
$R^{12}$ $(C_3-C_6)$-Cycloalkyl, Oxetanyl, Tetrahydrofuranyl, Tetrahydropyranyl oder 5-gliedriges Aza-Heteroaryl bedeutet,
wobei $(C_3-C_6)$-Cycloalkyl bis zu zweifach, gleich oder verschieden, mit einem Rest ausgewählt aus Fluor und Methyl substituiert sein kann
und
Aza-Heteroaryl bis zu zweifach, gleich oder verschieden, mit einem Rest ausgewählt aus Methyl und Trifluormethyl substituiert sein kann,

oder

$R^4$ für eine Gruppe der Formel $-(CH_2)_2-O-R^{13}$ steht, worin
$R^{13}$ $(C_3-C_6)$-Cycloalkyl bedeutet,

sowie ihre Salze, Solvate und Solvate der Salze.

Erfindungsgemäße Verbindungen sind die Verbindungen der Formel (I) und deren Salze, Solvate und Solvate der Salze, die von Formel (I) umfassten Verbindungen der nachfolgend aufgeführten Formeln (I-1), (I-2), (I-3), (I-4), (I-4a), (I-5), (I-6), (I-7), (I-8), (I-9), (I-10), (I-11) und (I-12) und deren Salze, Solvate und Solvate der Salze, die Verbindungen der nachfolgend aufgeführten Formel (I-A) und deren Salze, Solvate und Solvate der Salze, sowie die von den Formeln (I) und (1-A) umfassten, nachfolgend als Ausführunmgsbeispiele beschriebenen Verbindungen und deren Salze, Solvate und Solvate der Salze, soweit es sich bei den nachfolgend aufgeführten Verbindungen nicht bereits um Salze, Solvate oder Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung, Reinigung oder Lagerung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Benzolsulfonsäure, Toluolsulfonsäure, Naphthalindisulfonsäure, Ameisensäure, Essigsäure, Trifluoressigsäure, Propionsäure, Bernsteinsäure, Fumarsäure, Maleinsäure, Milchsäure, Weinsäure, Äpfelsäure, Zitronensäure, Gluconsäure, Benzoesäure und Embonsäure.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Im Rahmen der vorliegenden Erfindung haben die Substituenten und Reste, soweit nicht anders spezifiziert, die folgende Bedeutung:

$(C_1-C_6)$-Alkyl und $(C_1-C_4)$-Alkyl stehen im Rahmen der Erfindung für einen geradkettigen oder verzweigten Alkylrest mit 1 bis 6 bzw. 1 bis 4 Kohlenstoffatomen. Beispielhaft und vorzugsweise seien genannt: Methyl, Ethyl, *n*-Propyl, Isopropyl, *n*-Butyl, Isobutyl, *sec.*-Butyl, *tert.*-Butyl, *n*-Pentyl, 2-Pentyl, 3-Pentyl, Neopentyl, *n*-Hexyl, 2-Hexyl und 3-Hexyl.

$(C_2-C_6)$-Alkyl, $(C_2-C_5)$-Alkyl und $(C_2-C_4)$-Alkyl stehen im Rahmen der Erfindung für einen geradkettigen oder verzweigten Alkylrest mit 2 bis 6, 2 bis 5 bzw. 2 bis 4 Kohlenstoffatomen. Beispielhaft und vorzugsweise seien genannt:

Ethyl, *n*-Propyl, Isopropyl, *n*-Butyl, Isobutyl, *sec*.-Butyl, *tert*.-Butyl, *n*-Pentyl, 2-Pentyl, 3-Pentyl, Neopentyl, *n*-Hexyl, 2-Hexyl und 3-Hexyl.

$(C_1-C_6)$-Alkenyl und $(C_2-C_4)$-Alkenyl stehen im Rahmen der Erfindung für einen geradkettigen oder verzweigten Alkenylrest mit einer Doppelbindung und 2 bis 6 bzw. 2 bis 4 Kohlenstoffatomen. Bevorzugt ist ein geradkettiger oder verzweigter Alkenylrest mit 2 bis 4 Kohlenstoffatomen. Beispielhaft und vorzugsweise seien genannt: Vinyl, Prop-1-en-1-yl, Prop-2-en-1-yl (Allyl), Prop-1-en-2-yl (Isopropenyl), 2-Methylprop-2-en-1-yl, But-1-en-1-yl, But-1-en-2-yl, But-2-en-1-yl, But-2-en-2-yl, But-3-en-1-yl, Pent-2-en-1-yl, Pent-3-en-1-yl, Pent-4-en-1-yl, 3-Methylbut-2-en-1-yl und 4-Methylpent-3-en-1-yl.

$(C_2-C_6)$-Alkinyl steht im Rahmen der Erfindung für einen geradkettigen oder verzweigten Alkinylrest mit einer Dreifachbindung und 2 bis 6 Kohlenstoffatomen. Bevorzugt ist ein geradkettiger oder verzweigter Alkinylrest mit 2 bis 4 Kohlenstoffatomen [$(C_2-C_4)$-Alkinyl]. Beispielhaft und vorzugsweise seien genannt: Ethinyl, Prop-1-in-1-yl, Prop-2-in-1-yl (Propargyl), But-1-in-1-yl, But-2-in-1-yl, But-3-in-1-yl und But-3-in-2-yl.

$(C_1-C_6)$-cycloalkyl steht im Rahmen der Erfindung für eine monocyclische, gesättigte Cycloalkylgruppe mit 3 bis 6 Ring-Kohlenstoffatomen. Beispielhaft und vorzugsweise seien genannt: Cyclopropyl, Cyclobutyl, Cyclopentyl und Cyclohexyl.

$(C_1-C_5)$-Alkanoyl steht im Rahmen der Erfindung für einen geradkettigen oder verzweigten Alkylrest mit 1 bis 5 Kohlenstoffatomen, der in 1-Position eine Oxo-Gruppe trägt und über die 1-Position verknüpft ist. Beispielhaft und vorzugsweise seien genannt: Formyl, Acetyl, Propionyl (Propanoyl), *n*-Butyryl (*n*-Butanoyl), Isobutyryl (*iso*-Butanoyl), Valeryl (*n*-Pentanoyl), Isovaleryl (*iso*-Pentanoyl) und Pivaloyl (*neo*-Pentanoyl).

$(C_1-C_5)$-Alkanoylamino steht im Rahmen der Erfindung für eine Amino-Gruppe mit einem geradkettigen oder verzweigten Alkanoyl-Substituenten, der 1 bis 5 Kohlenstoffatome aufweist und über die Carbonylgruppe mit dem N-Atom verknüpft ist. Beispielhaft und vorzugsweise seien genannt: Formylamino, Acetylamino, Propionylamino, n-Butyrylamino, Isobutyrylamino, Valerylamino, Isovalerylamino und Pivaloylamino.

$(C_1-C_4)$-Alkoxy steht im Rahmen der Erfindung für einen geradkettigen oder verzweigten Alkoxyrest mit 1 bis 4 Kohlenstoffatomen. Beispielhaft und vorzugsweise seien genannt: Methoxy, Ethoxy, n-Propoxy, Isopropoxy, *n*-Butoxy, Isobutoxy, *sec*.-Butoxy und *tert*.-Butoxy.

$(C_1-C_4)$-Aloxycarbonyl steht im Rahmen der Erfindung für einen geradkettigen oder verzweigten Alkoxyrest mit 1 bis 4 Kohlenstoffatomen, der über eine an das O-Atom gebundene CarbonylGruppe [-C(=O)-] verknüpft ist. Beispielhaft und vorzugsweise seien genannt: Methoxycarbonyl, Ethoxycarbonyl, *n*-Propoxycarbonyl, Isopropoxycaibonyl, n-Butoxycarbonyl und *tert*.-Butoxycarbonyl.

$(C_1-C_4)$-Alkoxycarbonylamino steht im Rahmen der Erfindung für eine Amino-Gruppe mit einem geradkettigen oder verzweigten Alkoxycarbonyl-Substituenten, der 1 bis 4 Kohlenstoffatome im Alkoxyrest aufweist und über die Carbonylgruppe mit dem N-Atom verknüpft ist. Beispielhaft und vorzugsweise seien genannt: Methoxycarbonylamino, Ethoxycarbonylamino, n-Propoxycarbonylamino, Isopropoxycarbonylamino, *n*-Butoxycarbonylamino und tert.-Butoxycarbonylamino.

5-gliedriges Aza-Heteroaryl in der Definition des Restes $R^{12}$ steht für einen aromatischen Heterocyclus (Heteroaromaten) mit insgesamt 5 Ringatomen, der ein Ring-Stickstoffatom enthält, über dieses hinaus ein oder zwei weitere Ring-Heteroatome aus der Reihe N, O und/oder S enthalten kann und der über ein Ring-Kohlenstoffatom oder alternativ, sofern es die Valenz erlaubt, über ein Ring-Stickstoffatom verknüpft ist. Beispielhaft seien genannt: Pyrrolyl, Pyrazolyl, Imidazolyl, 1,2-Oxazolyl, 1,3-Oxazolyl, 1,2-Thiazolyl, 1,3-Thiazolyl, 1,2,3-Triazolyl, 1,2,4-Triazolyl, 1,2,4-Oxadiazolyl, 1,2,5-Oxadiazolyl, 1,3,4-Oxadiazolyl und 1,3,4-Thiadiazolyl. Bevorzugt sind 1,2-Oxazolyl, 1,3-Oxazolyl, 1,2,4-Oxadiazolyl, 1,2,5-Oxadiazolyl und 1,3,4-Oxadiazolyl.

Eine Oxo-Gruppe steht im Rahmen der Erfindung für ein Sauerstoffatom, das über eine Doppelbindung an ein Kohlenstoffatom gebunden ist.

Im Rahmen der vorliegenden Erfindung gilt, dass für alle Reste, die mehrfach auftreten, deren Bedeutung unabhängig voneinander ist. Wenn Reste in den erfindungsgemäßen Verbindungen substituiert sind, können die Reste, soweit nicht anders spezifiziert, ein- oder mehrfach substituiert sein. Eine Substitution mit einem oder mit zwei gleichen oder verschiedenen Substituenten ist bevorzugt. Besonders bevorzugt ist die Substitution mit einem Substituenten.

Bevorzugt im Rahmen der vorliegenden Erfindung sind Verbindungen der Formel (I), in welcher der Ring A für einen Aza-Heterocyclus der Formel

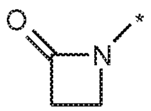, 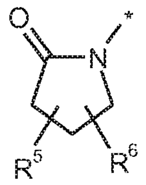, 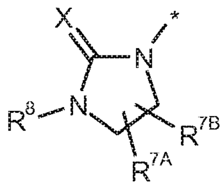, 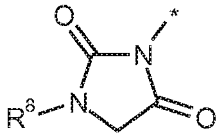,

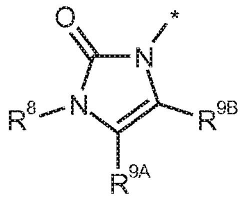, 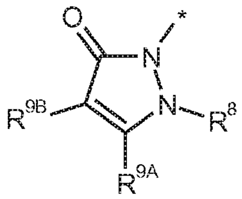, 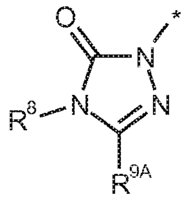, 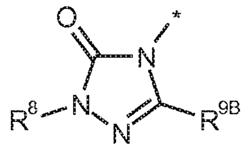,

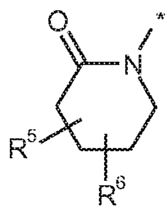, 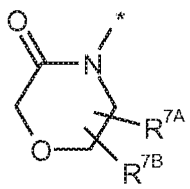, 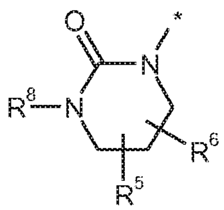 oder 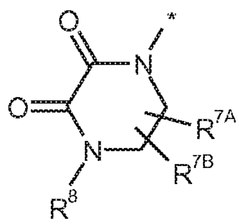

steht, worin * die Verknüpfung zur angrenzenden $CH(R^1)$-Gruppe markiert,

$R^5$ Wasserstoff, $(C_1-C_4)$-Alkyl, Hydroxy, $(C_1-C_4)$-Alkoxy, Amino, $(C_1-C_5)$-Alkanoylamino oder $(C_1-C_4)$-Alkoxycarbonylamino bedeutet,

$R^6$ Wasserstoff, Methyl oder Ethyl bedeutet,

$R^{7A}$ und $R^{7B}$ gleich oder verschieden sind und unabhängig voneinander Wasserstoff oder $(C_1-C_4)$-Alkyl bedeuten,

$R^8$ Wasserstoff, $(C_1-C_4)$-Alkyl, $(C_2-C_4)$-Alkenyl, $(C_1-C_5)$-Alkanoyl oder $(C_1-C_4)$-Alkoxycarbonyl bedeutet, wobei $(C_1-C_4)$-Alkyl bis zu zweifach mit Hydroxy substituiert sein kann,

$R^{9A}$ und $R^{9B}$ gleich oder verschieden sind und unabhängig voneinander Wasserstoff oder $(C_1-C_4)$-Alkyl bedeuten

und

X O, $N(R^{10})$ oder S bedeutet, worin
$R^{10}$ Wasserstoff, Cyano oder $(C_1-C_4)$-Alkoxycarbonyl darstellt,

$R^1$ für Wasserstoff steht,

$R^2$ für Methyl oder Ethyl, welche bis zu dreifach mit Fluor substituiert sein können, steht,

$R^3$ für $(C_2-C_6)$-Alkyl, $(C_2-C_6)$-Alkenyl oder $(C_2-C_6)$-Alkinyl steht,
wobei $(C_2-C_6)$-Alkyl mit einem Rest ausgewählt aus der Reihe Hydroxy, Methoxy, Ethoxy, Trifluormethoxy, Cyclopropyl, Cyclobutyl, Oxetanyl und Phenyl sowie bis zu dreifach mit Fluor substituiert sein kann
und
$(C_2-C_6)$-Alkenyl bis zu dreifach mit Fluor substituiert sein kann,
wobei die genannten Cyclopropyl- und Cyclobutyl-Gruppen ihrerseits bis zu zweifach, gleich oder verschieden, mit einem Rest ausgewählt aus Fluor und Methyl substituiert sein können,

oder

$R^3$ für eine Gruppe der Formel $-CH_2-R^{14}$ steht, worin
$R^{14}$ Cyclopropyl, Cyclobutyl, Oxetanyl oder Tetrahydrofuranyl bedeutet,
wobei Cyclopropyl und Cyclobutyl bis zu zweifach, gleich oder verschieden, mit einem Rest ausgewählt aus Fluor

und Methyl substituiert sein können,

und

$R^4$ für ($C_1$-$C_6$)-Alkyl oder ($C_2$-$C_6$)-Alkenyl steht, wobei ($C_1$-$C_6$)-Alkyl und ($C_2$-$C_6$)-Alkenyl bis zu dreifach mit Fluor substituiert sein können und wobei in ($C_1$-$C_6$)-Alkyl eine $CH_2$-Gruppe gegen -O-, -S- oder -$S(O)_2$- ausgetauscht sein kann, mit der Maßgabe, dass sich zwischen einem solchen Heteroatom und dem Uracil-$N^1$-Atom mindestens zwei Kohlenstoffatome befinden,

oder

$R^4$ für eine Gruppe der Formel -$(CH_2)_m$-CN, -$(CH_2)_n$-$R^{11}$ oder -$(CH_2)_p$-$R^{12}$ steht, worin m die Zahl 1, 2, 3 oder 4 bedeutet, n die Zahl 2 oder 3 bedeutet, p die Zahl 1 oder 2 bedeutet, $R^{11}$ Dimethylamino, Diethylamino oder Azetidino bedeutetund $R^{12}$ ($C_3$-$C_6$)-Cycloalkyl, Oxetanyl, Tetrahydrofuranyl, Tetrahydropyranyl oder 5-gliedriges Aza-Heteroaryl bedeutet, wobei ($C_3$-$C_6$)-Cycloalkyl bis zu zweifach, gleich oder verschieden, mit einem Rest ausgewählt aus Fluor und Methyl substituiert sein kann und Aza-Heteroaryl bis zu zweifach, gleich oder verschieden, mit einem Rest ausgewählt aus Methyl und Trifluormethyl substituiert sein kann,

oder

$R^4$ für eine Gruppe der Formel -$(CH_2)_2$-O-$R^{13}$ steht, worin $R^{13}$ Cyclopropyl oder Cyclobutyl bedeutet,

sowie ihre Salze, Solvate und Solvate der Salze.

Besonders bevorzugt im Rahmen der vorliegenden Erfindung sind Verbindungen der Formel (I), in welcher der Ring A für einen Aza-Heterocyclus der Formel

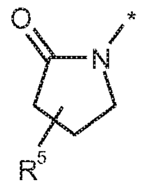 , 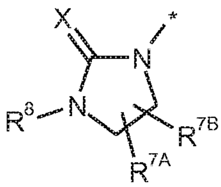 , 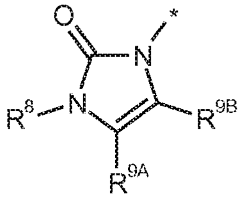 , 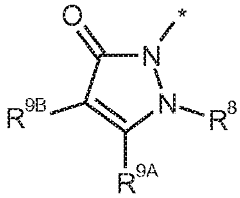 ,

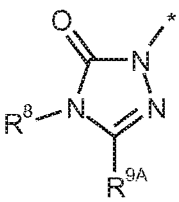 , 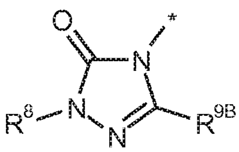 , 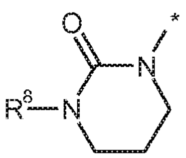 oder 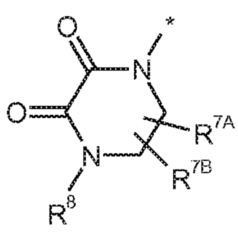

steht, worin * die Verknüpfung zur angrenzenden $CH(R^1)$-Gruppe markiert,

| | |
|---|---|
| $R^5$ | Hydroxy, Methoxy oder Ethoxy bedeutet, |
| $R^{7A}$ und $R^{7B}$ | unabhängig voneinander Wasserstoff oder Methyl bedeuten, |
| $R^8$ | Wasserstoff, Methyl, Ethyl, n-Propyl, 2-Hydroxyethyl, 3-Hydroxypropyl, 2,3-Dihydroxypropyl, Allyl, Formyl oder Acetyl bedeutet, |

$R^{9A}$ und $R^{9B}$ unabhängig voneinander Wasserstoff oder Methyl bedeuten

und

X O, $N(R^{10})$ oder S bedeutet, worin
$R^{10}$ Cyano oder $(C_1-C_4)$-Alkoxycarbonyl darstellt,

$R^1$ für Wasserstoff steht,
$R^2$ für Methyl oder Ethyl, welche bis zu dreifach mit Fluor substituiert sein können, steht,
$R^3$ für $(C_2-C_5)$-Alkyl oder $(C_2-C_4)$-Alkenyl steht,
wobei $(C_2-C_5)$-Alkyl mit einem Rest ausgewählt aus der Reihe Hydroxy, Methoxy, Cyclopropyl, Cyclobutyl, Oxetanyl und Phenyl sowie bis zu dreifach mit Fluor substituiert sein kann
und
$(C_2-C_4)$-Alkenyl bis zu dreifach mit Fluor substituiert sein kann,
wobei die genannten Cyclopropyl- und Cyclobutyl-Gruppen ihrerseits bis zu zweifach mit Fluor substituiert sein können,

oder

$R^3$ für eine Gruppe der Formel $-CH_2-R^{14}$ steht, worin
$R^{14}$ Cyclopropyl, Cyclobutyl oder Oxetanyl bedeutet,
wobei Cyclopropyl und Cyclobutyl bis zu zweifach, gleich oder verschieden, mit einem Rest ausgewählt aus Fluor und Methyl substituiert sein können,und
$R^4$ für $(C_1-C_6)$-Alkyl oder $(C_2-C_6)$-Alkenyl steht,
wobei $(C_1-C_6)$-Alkyl und $(C_2-C_6)$-Alkenyl bis zu dreifach mit Fluor substituiert sein können
und
wobei in $(C_1-C_6)$-Alkyl eine $CH_2$-Gruppe gegen -O- oder -S- ausgetauscht sein kann, mit der Maßgabe, dass sich zwischen einem solchen Heteroatom und dem Uracil-$N^1$-Atom mindestens zwei Kohlenstoffatome befinden,

oder

$R^4$ für die Gruppe $-CH_2-R^{12}$ steht, worin
$R^{12}$ $(C_3-C_6)$-Cycloalkyl, Oxetanyl, Tetrahydrofuranyl oder Tetrahydropyranyl bedeutet,
wobei $(C_3-C_6)$-Cycloalkyl bis zu zweifach, gleich oder verschieden, mit einem Rest ausgewählt aus Fluor und Methyl substituiert sein kann,

sowie ihre Salze, Solvate und Solvate der Salze.

Eine besondere Ausführungsform der vorliegenden Erfindung betrifft Verbindungen der Formel (I), in welcher der Ring A für einen Aza-Heterocyclus der Formel

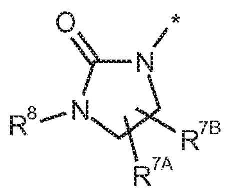

steht,

worin * die Verknüpfung zur angrenzenden $CH(R^1)$-Gruppe markiert
und

$R^{7A}$, $R^{7B}$ und $R^8$ unabhängig voneinander Wasserstoff oder Methyl bedeuten,

sowie ihre Salze, Solvate und Solvate der Salze.

Eine weitere besondere Ausführungsform der vorliegenden Erfindung betrifft Verbindungen der Formel (I), in welcher der Ring A für einen Aza-Heterocyclus der Formel

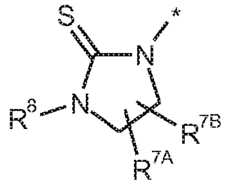

steht,

worin * die Verknüpfung zur angrenzenden $CH(R^1)$-Gruppe markiert und
$R^{7A}$, $R^{7B}$ und $R^8$ unabhängig voneinander Wasserstoff oder Methyl bedeuten,

sowie ihre Salze, Solvate und Solvate der Salze.

Eine weitere besondere Ausführungsform der vorliegenden Erfindung betrifft Verbindungen der Formel (I), in welcher
der Ring A für einen Aza-Heterocyclus der Formel

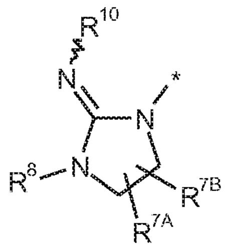

steht,

worin * die Verknüpfung zur angrenzenden $CH(R^1)$-Gruppe markiert,
$R^{7A}$, $R^{7B}$ und $R^8$ unabhängig voneinander Wasserstoff oder Methyl bedeuten und
$R^{10}$ Cyano, Methoxycarbonyl, Ethoxycarbonyl oder tert.-Butoxycarbonyl bedeutet,

sowie ihre Salze, Solvate und Solvate der Salze.

Eine weitere besondere Ausführungsform der vorliegenden Erfindung betrifft Verbindungen der Formel (I), in welcher
der Ring A für einen Aza-Heterocyclus der Formel

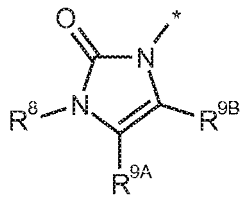

steht,

worin * die Verknüpfung zur angrenzenden $CH(R^1)$-Gruppe markiert und
$R^8$, $R^{9A}$ und $R^{9B}$ unabhängig voneinander Wasserstoff oder Methyl bedeuten,

sowie ihre Salze, Solvate und Solvate der Salze.

Eine weitere besondere Ausführungsform der vorliegenden Erfindung betrifft Verbindungen der Formel (I), in welcher
der Ring A für einen Aza-Heterocyclus der Formel

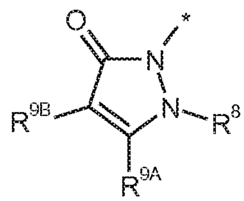

steht,

worin * die Verknüpfung zur angrenzenden $CH(R^1)$-Gruppe markiert
und
$R^8$, $R^{9A}$ und $R^{9B}$ unabhängig voneinander Wasserstoff oder Methyl bedeuten,

sowie ihre Salze, Solvate und Solvate der Salze.

Eine weitere besondere Ausführungsform der vorliegenden Erfindung betrifft Verbindungen der Formel (I), in welcher
der Ring A für einen Aza-Heterocyclus der Formel

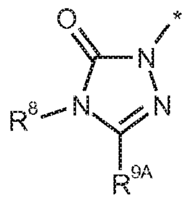

steht,

worin * die Verknüpfung zur angrenzenden $CH(R^1)$-Gruppe markiert
und
$R^8$ und $R^{9A}$ unabhängig voneinander Wasserstoff oder Methyl bedeuten,

sowie ihre Salze, Solvate und Solvate der Salze.

Eine weitere besondere Ausführungsform der vorliegenden Erfindung betrifft Verbindungen der Formel (I), in welcher
der Ring A für einen Aza-Heterocyclus der Formel

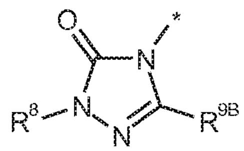

steht,

worin * die Verknüpfung zur angrenzenden $CH(R^1)$-Gruppe markiert
und
$R^8$ und $R^{9B}$ unabhängig voneinander Wasserstoff oder Methyl bedeuten,

sowie ihre Salze, Solvate und Solvate der Salze.

Eine weitere besondere Ausführungsform der vorliegenden Erfindung betrifft Verbindungen der Formel (I), in welcher
der Ring A für einen Aza-Heterocyclus der Formel

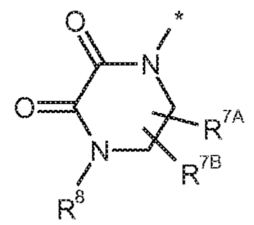

steht,

worin * die Verknüpfung zur angrenzenden $CH(R^1)$-Gruppe markiert
und
$R^{7A}$, $R^{7B}$ und $R^8$ unabhängig voneinander Wasserstoff oder Methyl bedeuten,

sowie ihre Salze, Solvate und Solvate der Salze.

Eine weitere besondere Ausführungsform der vorliegenden Erfindung betrifft Verbindungen der Formel (I), in welcher

$R^1$ für Wasserstoff steht,

sowie ihre Salze, Solvate und Solvate der Salze.

Eine weitere besondere Ausführungsform der vorliegenden Erfindung betrifft Verbindungen der Formel (I), in welcher

$R^2$ für Methyl, Difluormethyl oder Trifluormethyl steht,

sowie ihre Salze, Solvate und Solvate der Salze.

Eine weitere besondere Ausführungsform der vorliegenden Erfindung betrifft Verbindungen der Formel (I), in welcher

$R^3$ für $(C_2\text{-}C_4)$-Alkyl, das mit Hydroxy, Methoxy, Cyclopropyl oder Oxetanyl oder bis zu dreifach mit Fluor substituiert sein kann, steht,

sowie ihre Salze, Solvate und Solvate der Salze.

Eine weitere besondere Ausführungsform der vorliegenden Erfindung betrifft Verbindungen der Formel (I), in welcher

$R^3$ für $(C_2\text{-}C_5)$-Alkyl, das mit Hydroxy, Methoxy, Cyclopropyl oder Oxetanyl oder bis zu dreifach mit Fluor substituiert sein kann, steht,
wobei Cyclopropyl seinerseits bis zu zweifach, gleich oder verschieden, mit einem Rest ausgewählt aus Fluor und Methyl substituiert sein kann,

sowie ihre Salze, Solvate und Solvate der Salze.

Eine weitere besondere Ausführungsform der vorliegenden Erfindung betrifft Verbindungen der Formel (I), in welcher

$R^3$ für eine Gruppe der Formel $-CH_2-R^{14}$ steht, worin
$R^{14}$ Cyclopropyl, Cyclobutyl oder Oxetanyl bedeutet,
wobei Cyclopropyl und Cyclobutyl bis zu zweifach, gleich oder verschieden, mit einem Rest ausgewählt aus Fluor und Methyl substituiert sein können,

sowie ihre Salze, Solvate und Solvate der Salze.

Eine weitere besondere Ausführungsform der vorliegenden Erfindung betrifft Verbindungen der Formel (I), in welcher

$R^4$ für $(C_1\text{-}C_4)$-Alkyl, das bis zu dreifach mit Fluor substituiert sein kann, für 2-Methoxyethyl oder 2-Ethoxyethyl oder für die Gruppe $-CH_2-R^{12}$ steht, worin

$R^{12}$ Cyclopropyl, Cyclobutyl, Oxetanyl, Tetrahydrofuranyl oder Tetrahydropyranyl bedeutet, wobei Cyclopropyl und Cyclobutyl bis zu zweifach mit Fluor substituiert sein können,

sowie ihre Salze, Solvate und Solvate der Salze.

Insbesondere bevorzugt im Rahmen der vorliegenden Erfindung sind Verbindungen der Formel (I), in welcher der Ring A für einen Aza-Heterocyclus der Formel

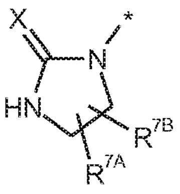 , 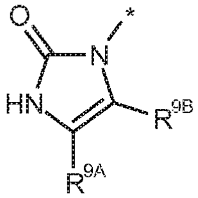 , 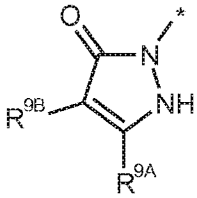 , 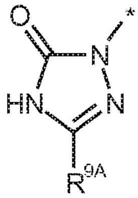 ,

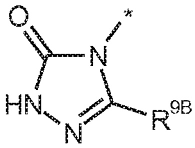 oder 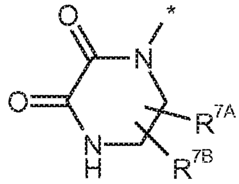

steht,
worin * die Verknüpfung zur angrenzenden $CH(R^1)$-Gruppe markiert,

$R^{7A}$ und $R^{7B}$ unabhängig voneinander Wasserstoff oder Methyl bedeuten,
$R^{9A}$ und $R^{9B}$ unabhängig voneinander Wasserstoff oder Methyl bedeuten

und

X O, $N(R^{10})$ oder S bedeutet, worin
$R^{10}$ Cyano, Methoxycarbonyl, Ethoxycarbonyl oder tert.-Butoxycarbonyl darstellt,

$R^1$ für Wasserstoff steht,

$R^2$ für Methyl, Difluormethyl oder Trifluormethyl steht,

$R^3$ für $(C_2-C_5)$-Alkyl, das mit Hydroxy, Methoxy oder Cyclopropyl oder bis zu dreifach mit Fluor substituiert sein kann, steht,
wobei Cyclopropyl seinerseits bis zu zweifach mit Fluor substituiert sein kann,

und

$R^4$ für $(C_1-C_4)$-Alkyl, das bis zu dreifach mit Fluor substituiert sein kann, für 2-Methoxyethyl oder 2-Ethoxyethyl oder für die Gruppe $-CH_2-R^{12}$ steht, worin
$R^{12}$ Cyclopropyl, Cyclobutyl, Oxetanyl, Tetrahydrofuranyl oder Tetrahydropyranyl bedeutet, wobei Cyclopropyl und Cyclobutyl bis zu zweifach mit Fluor substituiert sein können,

sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Ganz besonders bevorzugt sind Kombinationen von zwei oder mehreren der oben genannten Vorzugsbereiche.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert

werden können, sind solche von Wasserstoff, Kohlenstoff, Stickstoff, Sauerstoff, Phosphor, Schwefel, Fluor, Chlor, Brom und Iod, wie $^{2}H$ (Deuterium), $^{3}H$ (Tritium), $^{13}C$, $^{14}C$, $^{15}N$, $^{17}O$, $^{18}O$, $^{32}P$, $^{33}P$, $^{33}S$, $^{34}S$, $^{3S}S$, $^{36}S$, $^{18}F$, $^{36}Cl$, $^{82}Br$, $^{123}I$, $^{12A}I$, $^{129}I$ und $^{131}I$. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit $^{3}H$- oder $^{14}C$-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise zu einer Verlängerung der Halbwertszeit im Körper oder zu einer Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach allgemein gebräuchlichen, dem Fachmann bekannten Verfahren hergestellt werden, so beispielsweise nach den weiter unten beschriebenen Methoden und den bei den Ausführungsbeispielen wiedergegebenen Vorschriften, indem hierbei entsprechende isotopische Modifikationen der jeweiligen Reagentien und/oder Ausgangsverbindungen eingesetzt werden.

Eine besondere Ausführungsform solcher isotopischen Varianten der erfindungsgemäßen Verbindungen stellen Verbindungen der Formel (I-A)

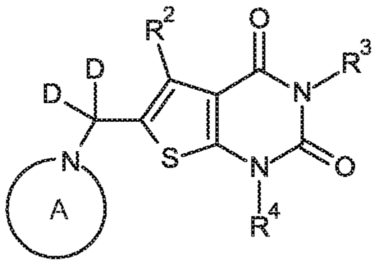

(I-A),

in welcher der Ring A sowie die Reste $R^2$, $R^3$ und $R^4$ die zuvor angegebenen Bedeutungen haben,

und deren Salze, Solvate und Solvate der Salze dar. Die Verbindungen der Formel (I-A) sowie die Verwendung dieser Verbindungen zu den in der weiteren Beschreibung genannten Zwecken sind daher gleichfalls Gegenstand der vorliegenden Erfindung.

Darüber hinaus umfasst die vorliegende Erfindung auch Prodrugs der erfindungsgemäßen Verbindungen. Der Begriff "Prodrugs" bezeichnet hierbei Verbindungen, welche selbst biologisch aktiv oder inaktiv sein können, jedoch während ihrer Verweilzeit im Körper auf beispielsweise metabolischem oder hydrolytischem Wege zu erfindungsgemäßen Verbindungen umgesetzt werden.

Die erfindungsgemäßen Verbindungen der Formel (I) können in Abhängigkeit von der jeweiligen Art des Aza-Heterocyclus A auf unterschiedlichen, zum Teil auch alternativen Wegen hergestellt werden.

So können erfindungsgemäße Verbindungen der Formel (I-1)

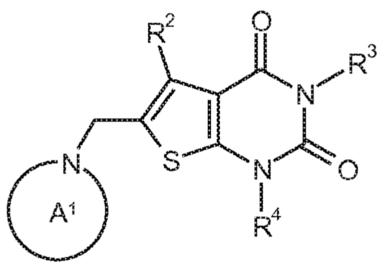

(I-1),

in welcher

der Ring $A^1$ für einen Aza-Heterocyclus der Formel

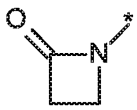 , 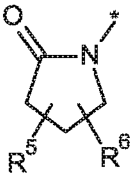 , 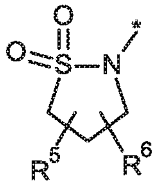 , 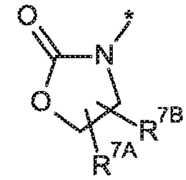 , 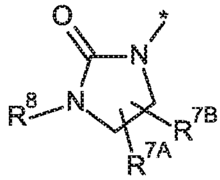 ,

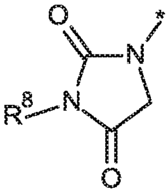 , 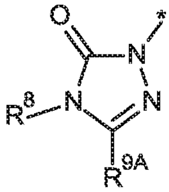 , 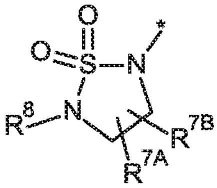 , 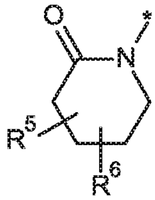 ,

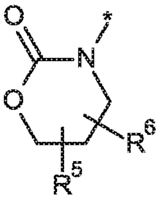 , 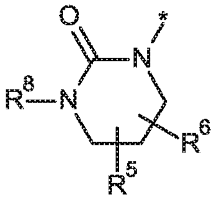 , 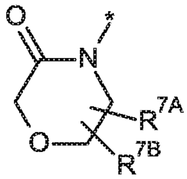 , 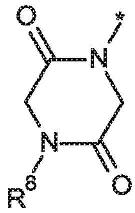 ,

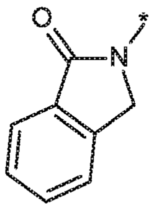 oder 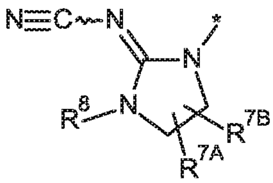

steht,

worin * die Verknüpfung zur angrenzenden $CH_2$-Gruppe markiert und $R^5$, $R^6$, $R^{7A}$, $R^{7B}$, $R^8$ und $R^{9A}$ die oben angegebenen Bedeutungen haben,

und

$R^2$, $R^3$ und $R^4$ die oben angegebenen Bedeutungen haben,

nach einem allgemeinen Verfahren gemäß dem nachfolgenden Reaktionsschema 1 hergestellt werden:

Schema 1

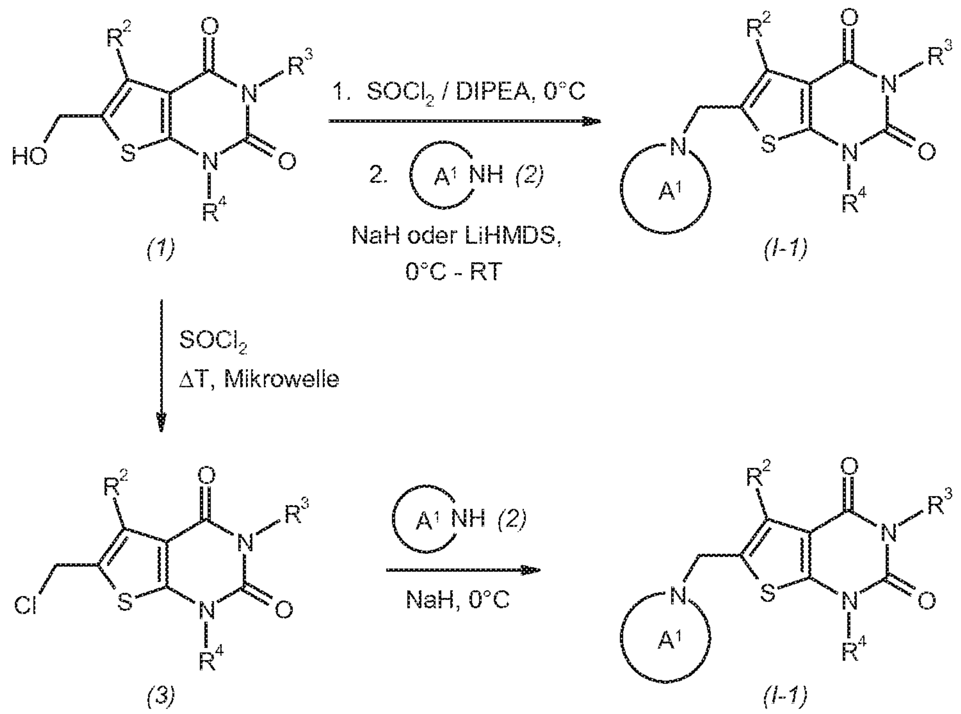


In der "Eintopf"-Variante dieses Verfahrens wird ein Alkohol der Formel (1) zunächst mit einem Chlorierungsmittel, wie vorzugsweise Thionylchlorid, in Gegenwart einer tertiären Aminbase, wie zum Beispiel *N,N*-Diisopropylethylamin oder Triethylamin, in die korrespondierende Chlorverbindung [entsprechend Formel (3)] überfuhrt. Diese Chlorverbindung wird nicht isoliert, sondern im selben Reaktionsgefäß mit einer Lösung des deprotonierten Aza-Heterocyclus der Formel (2) versetzt, um so in einem Schritt die Zielverbindung der Formel (I-1) zu erhalten. Für die Deprotonierung des Heterocyclus (2) eignen sich starke Basen, wie zum Beispiel Alkalimetallhydride oder Alkalimetallamide; bevorzugt wird Natriumhydrid oder Lithiumhexamethyldisilazid verwendet. Der Chlorierungsteilschritt erfolgt üblicherweise in einem halogenierten Kohlenwasserstoff als inertem Lösungsmittel - bevorzugt ist hier Dichlormethan - im Temperaturbereich um 0°C. Bei derselben Temperatur wird mit der Lösung des deprotonierten Heterocyclus (2) versetzt. Die Substitutionsreaktion zu (I-1) erfolgt dann bevorzugt bei RT. Als Lösungsmittel zur Herstellung des deprotonierten Heterocyclus (2) eignen sich insbesondere *N,N*-Dimethylformamid (DMF), Tetrahydrofuran (THF) oder Gemische hieraus. Die Deprotonierung selbst erfolgt bevorzugt in einem Temperaturbereich zwischen 0°C und +60°C.

Weniger Hydrolyse-empfindliche Chlorverbindungen der Formel (3) lassen sich herstellen und auch isolieren, indem - ähnlich wie zuvor - Alkohole der Formel (1) mit einem Chlorierungsmittel, wie bevorzugt Thionylchlorid, in einem inerten Lösungsmittel, wie zum Beispiel Chloroform oder Dichlormethan, umgesetzt werden. Die Reaktion erfolgt hier bevorzugt in einem Temperaturbereich zwischen RT und +80°C, wobei sich für das Erwärmen über den Siedepunkt des jeweiligen Lösungsmittel hinaus der Einsatz eines Mikrowellenofens, unter Verwendung verschlossener Reaktionsgefäße, als besonders vorteilhaft erwiesen hat. In einem nachfolgenden, separaten Reaktionsschritt werden dann die isolierten Chlorverbindungen der Formel (3) unter ähnlichen Bedingungen, wie oben erläutert, mit einer Lösung des deprotonierten Heterocyclus (2) umgesetzt.

Anstelle des Restes $R^8$ in den betreffenden Aza-Heterocyclen der Formel (2) kann bei dem zuvor beschriebenen Verfahren zunächst auch eine geeignete Amid-Schutzgruppe eingesetzt werden, wenn dies zur Vermeidung von Nebenreaktionen zweckmäßig oder erforderlich ist. Nach Abspaltung einer solchen Schutzgruppe am Ende der obigen Reaktionssequenz kann dann eine weitere Derivatisierung im Bedeutungsumfang von $R^8$ über entsprechende Alkylierungs- oder Acylierungsreaktionen erfolgen, wie sie dem Fachmann geläufig sind [zur Eignung, Einführung und Entfernung von Amid-Schutzgruppen siehe z.B. T.W. Greene und P.G.M. Wuts, Protective Groups in Organic Synthesis, Wiley, New York, 1999].

Erfindungsgemäße Verbindungen der Formel (I-2)

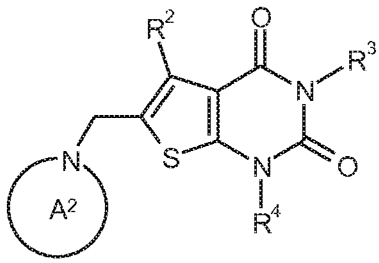

(I-2),

in welcher

der Ring $A^2$ für ein α-Hydroxylactam der Formel

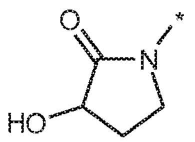

oder

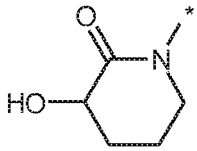

steht,
worin * die Verknüpfung zur angrenzenden $CH_2$-Gruppe markiert,
und

$R^2$, $R^3$ und $R^4$ die oben angegebenen Bedeutungen haben,

können auch alternativ nach einem speziellen Verfahren gemäß nachfolgendem Reaktionsschema 2 hergestellt werden:

Schema 2

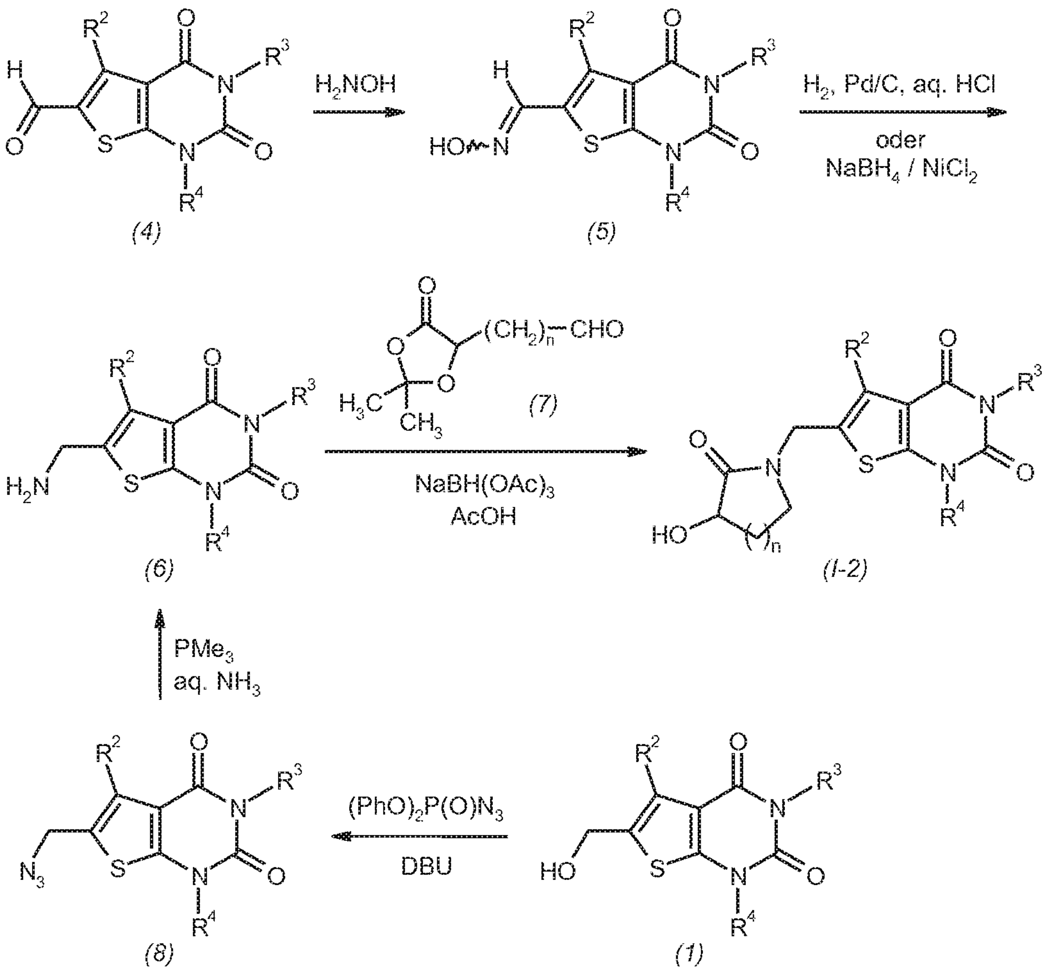


(4) (5) (6) (7) (I-2) (8) (1)

[n = 1 oder 2].

Hier werden Aldehyde der Formel (4) zunächst mit Hydroxylamin in die entsprechenden Oxime der Formel (5) überfuhrt. Die Reaktion erfolgt bevorzugt bei RT unter Verwendung einer wässrigen Hydroxylamin-Lösung in einem mit Wasser mischbaren Ether, wie Tetrahydrofuran (THF), als Lösungsmittel. Die nachfolgende Reduktion zu den Aminomethylverbindungen (6) kann durch Hydrierung in Gegenwart eines Edelmetall-Katalysators erfolgen. Bevorzugte Reaktionsbedingungen sind 1 bar Wasserstoffdruck bei RT in Gegenwart einer katalytischen Menge von Palladium (10% auf Kohle) in Methanol oder Ethanol als Lösungsmittel. Bevorzugt erfolgt die Hydrierung in Gegenwart von wässriger Mineralsäure, wie zum Beispiel konzentrierter Salzsäure. Alternativ kann die Reduktion zu den Aminomethylverbindungen (6) auch mit Natriumborhydrid in Gegenwart von geeigneten Metallsalzen, wie zum Beispiel Nickel- oder Kobaltchlorid, erfolgen. Bevorzugte Reaktionsbedingungen beinhalten hier die Verwendung von Natriumborhydrid in Kombination mit Nickel(II)chlorid-Hexahydrat in Methanol als Lösungsmittel bei RT. Ein anderer Weg zu den Aminomethylverbindungen der Formel (6) geht von den Alkoholen der Formel (1) aus. Diese werden zunächst in die korrespondierenden Azide der Formel (8) überführt, indem sie mit Phosphorsäurediphenylesterazid in Gegenwart einer Aminbase, wie zum Beispiel DBU, bei 0°C bis RT in Tetrahydrofuran (THF) zur Reaktion gebracht werden. Die Reduktion der Azide (8) zu den Aminomethylverbindungen (6) kann dann zum Beispiel durch Umsetzen mit Trimethylphosphin in Tetrahydrofuran (THF) und konzentriertem Ammoniakwasser bei RT erfolgen.

Die auf einem der genannten Wege erhaltenen Aminomethylverbindungen der Formel (6) werden dann im letzten Reaktionsschritt im Sinne einer reduktiven Aminierung mit Aldehyden der Formel (7) umgesetzt. Als Reduktionsmittel eignet sich hier insbesondere Natriumtriacetoxyborhydrid in Gegenwart von Essigsäure. Ein geeignetes Lösungsmittel ist 1,2-Dichlorethan, und die Reaktion erfolgt bevorzugt bei RT. Nach dem initialen Schritt der reduktiven Aminierung erfolgt spontan eine Cyclisierungsreaktion, die unter Aceton-Freisetzung die Hydroxylactame der Formel (I-2) ergibt.

Erfindungsgemäße Verbindungen der Formel (I-3)

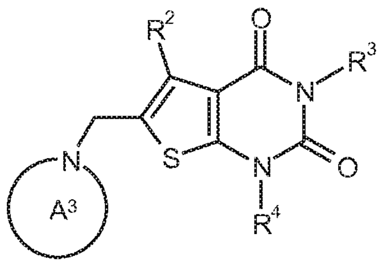

(I-3),

in welcher

der Ring $A^3$ für einen Aza-Heterocyclus der Formel

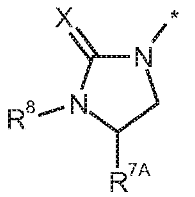

oder

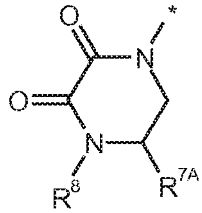

steht,

worin * die Verknüpfung zur angrenzenden $CH_2$-Gruppe markiert und $R^{7A}$, $R^8$ und X die oben angegebenen Bedeutungen haben,

und $R^2$, $R^3$ und $R^4$ die oben angegebenen Bedeutungen haben, lassen sich gemäß dem nachfolgenden Reaktionsschema 3 erhalten:

Schema 3

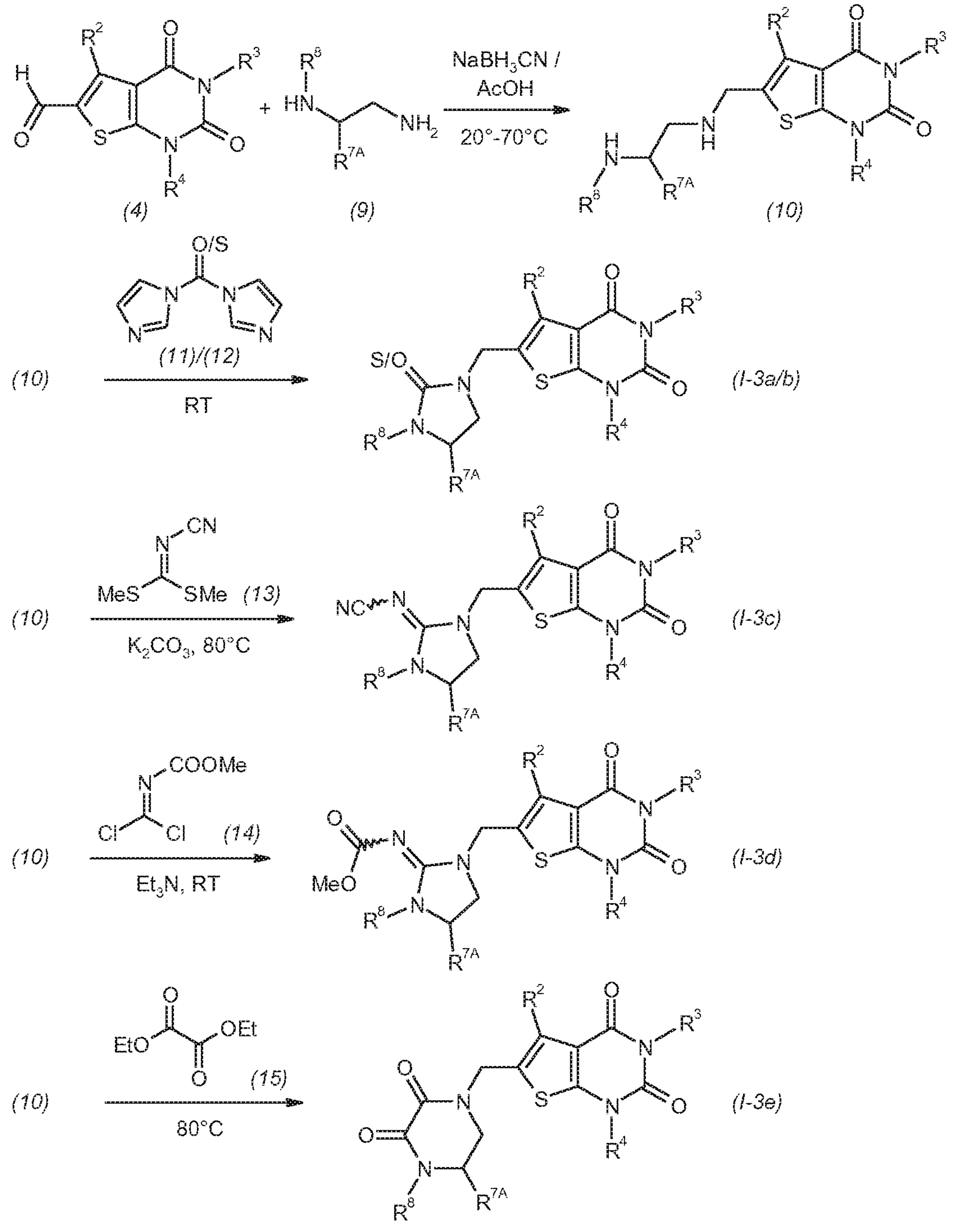


Aldehyde der Formel (4) werden hier zunächst mit 1,2-Diaminoethan-Derivaten der Formel (9) in einer reduktiven Aminierung zu den Diaminoverbindungen der Formel (10) umgesetzt. Als Reduktionsmittel eignet sich insbesondere Natriumcyanoborhydrid in Gegenwart von Essigsäure. Ein geeignetes Lösungsmittel ist Methanol, gegebenenfalls im Gemisch mit Dichlormethan, und die Reaktion erfolgt bevorzugt in einem Temperaturbereich zwischen RT und +70°C.

Die Zielverbindungen der Formeln (I-3a) und (I-3b) werden durch anschließende Reaktion der Diaminoverbindungen (10) mit *N,N'*-Carbonyldiimidazol (11) [für (I-3a)] bzw. *N,N'*-Thiocarbonyldiimidazol (12) [für (I-3b)] erhalten. Die Umsetzungen erfolgen bevorzugt bei RT und in Lösungsmitteln wie Tetrahydrofuran (THF), 1,4-Dioxan oder Dimethylsulfoxid (DMSO), gegebenenfalls in Anwesenheit einer tertiären Aminbase wie zum Beispiel Triethylamin. Die Produkte der Formel (I-3c) werden durch Reaktion der Diaminoverbindungen (10) mit Dimethyl-*N*-cyanodithioiminocarbonat (13) erhalten. Die Umsetzung erfolgt bevorzugt in *N,N*-Dimethylformamid (DMF) als Lösungsmittel in Gegenwart von Alkalimetallcarbonaten, wie zum Beispiel Kaliumcarbonat, als Base bei erhöhten Temperaturen um +80°C. Die Produkte der Formel (I-3d) werden durch Reaktion der Diaminoverbindungen (10) mit Methyl-(dichlormethylen)carbamat (14) erhalten. Die Umsetzung erfolgt bevorzugt in Dichlormethan als Lösungsmittel in Gegenwart einer tertiären Aminbase, wie zum Beispiel Triethylamin, bei RT. Die Produkte der Formel (I-3e) schließlich werden durch Reaktion der Diaminoverbindungen (10) mit Diethyloxalat (15) erhalten. Die Umsetzung erfolgt bevorzugt in Ethanol als Lösungsmittel bei erhöhten Temperaturen um +80°C.

Erfindungsgemäße Verbindungen der Formel (I-4)

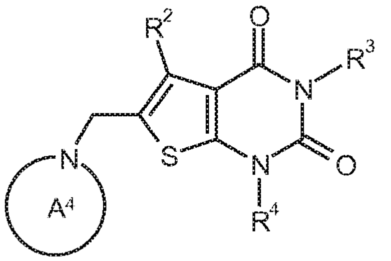

(I-4),

in welcher

der Ring $A^4$ für ein cyclisches Harnstoff-Derivat der Formel

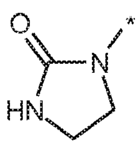

oder

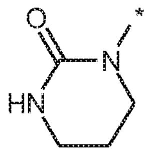

steht,
worin * die Verknüpfung zur angrenzenden $CH_2$-Gruppe markiert,
und

$R^2$, $R^3$ und $R^4$ die oben angegebenen Bedeutungen haben,

sind auch auf alternativem Wege gemäß nachfolgendem Reaktionsschema 4 zugänglich:

Schema 4

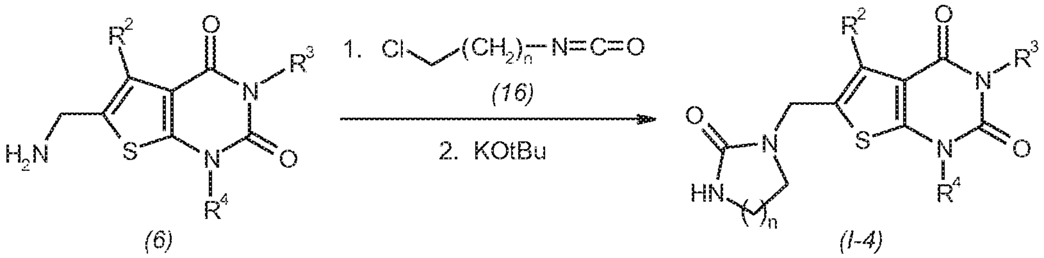


(6) (I-4)

[n = 1 oder 2].

Die zuvor in Schema 2 beschriebenen Aminomethylverbindungen der Formel (6) werden hier in einem Eintopfverfahren zunächst mit Chloralkylisocyanaten der Formel (16) umgesetzt, wobei sich zunächst ein offenkettiges Harnstoff-Derivat bildet. Die Umsetzung erfolgt bevorzugt bei RT in einem Lösungsmittelgemisch aus *N,N*-Dimethylformamid (DMF) und Tetrahydrofuran (THF). Durch anschließenden Zusatz einer starken Base, wie zum Beispiel Kalium-tert.-butanolat, zum Reaktionsgemisch erfolgt dann bei RT der Ringschluss zu den Zielverbindungen der Formel (I-4).

Erfmdungsgemäße Verbindungen der Formel (I-4a)

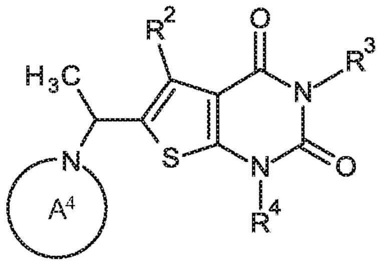

(I-4a),

in welcher

der Ring $A^4$ für ein cyclisches Harnstoff-Derivat der Formel

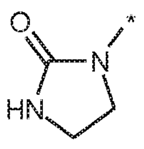

oder

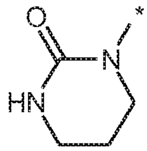

steht,
worin * die Verknüpfung zur angrenzenden $CH_2$-Gruppe markiert,
und
$R^2$, $R^3$ und $R^4$ die oben angegebenen Bedeutungen haben,
lassen sich in analoger Weise gemäß Reaktionsschema 4a erhalten:

Schema 4a

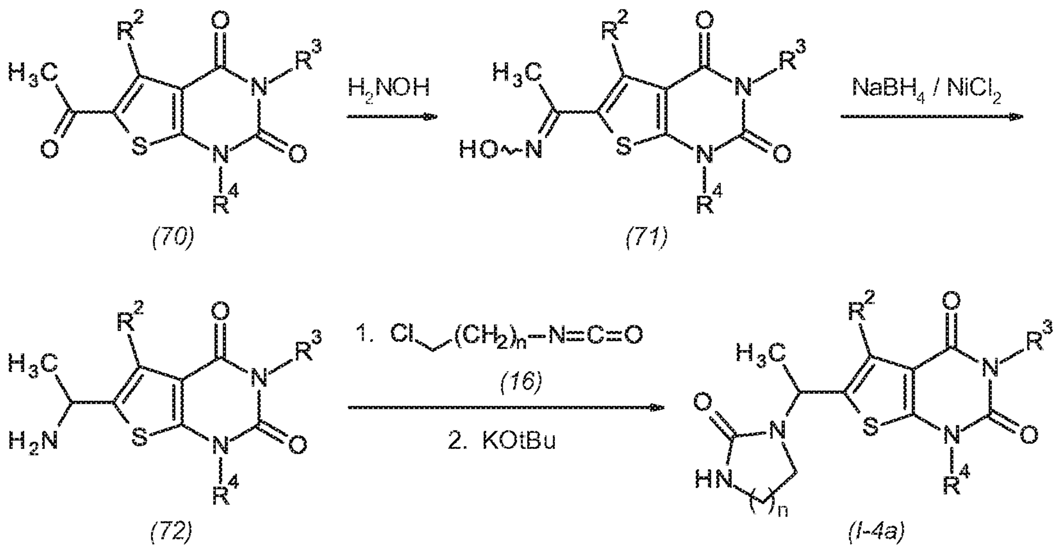


(70) (71) (72) (I-4a)

[n = 1 oder 2].

Hier werden Methylketone der Formel (70) zunächst mit Hydroxylamin in die entsprechenden Oxime der Formel (71) überfuhrt. Die Reaktion erfolgt unter Einsatz einer wässrigen Hydroxylamin-Lösung vorzugsweise bei erhöhter Temperatur in einem alkoholischen Lösungsmittel wie Ethanol. Die nachfolgende Reduktion zu den 1-Aminoethyl-Verbindungen der Formel (72) wird mit Hilfe von Natriumborhydrid in Gegenwart eines geeigneten Metallsalzes, wie zum Beispiel Nickel- oder Kobaltchlorid, durchgeführt. Bevorzugte Reaktionsbedingungen beinhalten die Verwendung von Natriumborhydrid in Kombination mit Nickel(II)chlorid-Hexahydrat in Methanol als Lösungsmittel bei RT [vgl. auch Synthesesequenz (4) → (5) → (6) in Schema 2]. Abschließend werden die 1-Aminoethyl-Verbindungen der Formel (72) in einem Eintopfverfahren zunächst mit Chloralkylisocyanaten der Formel (16) umgesetzt, wobei sich zunächst ein offenkettiges Harnstoff-Derivat bildet. Die Umsetzung erfolgt bevorzugt bei RT in einem Lösungsmittelgemisch aus *N,N*-

Dimethylformamid (DMF) und Tetrahydrofuran (THF). Durch anschließenden Zusatz einer starken Base, wie zum Beispiel Kalium-*tert*.-butanolat, zum Reaktionsgemisch erfolgt dann bei RT der Ringschluss zu den Zielverbindungen der Formel (I-4a) [vgl. Schema 4].

Erfindungsgemäße Verbindungen der Formel (I-5)

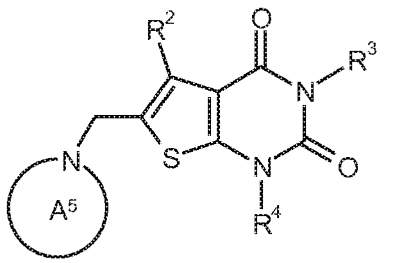

(I-5),

in welcher

der Ring $A^5$ für einen Aza-Heterocyclus der Formel

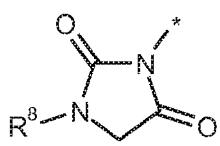

oder

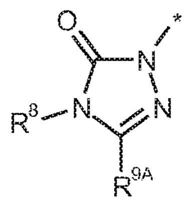

steht,

worin * die Verknüpfung zur angrenzenden $CH_2$-Gruppe markiert und

$R^8$ und $R^{9A}$ die oben angegebenen Bedeutungen haben,

und

$R^2$, $R^3$ und $R^4$ die oben angegebenen Bedeutungen haben,

können gemäß dem nachfolgenden Reaktionsschema 5 hergestellt werden:

Schema 5

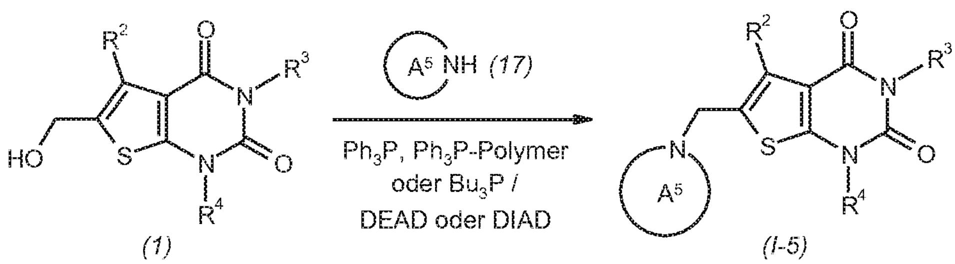


Alkohole der Formel (1) werden hier mit Aza-Heterocyclen der Formel (17) im Sinne einer Mitsunobu-Reaktion zu den Produkten der Formel (I-5) umgesetzt. Als Reagenzien für diese Transformation eignen sich zum Beispiel Triphenylphosphin, polymergebundenes Triphenylphosphin, Tributylphosphin oder Trimethylphosphin jeweils in Kombination

mit Diethylazodicarboxylat (DEAD), Diisopropyldiazodicarboxylat (DIAD) oder Azodicaibonsäuredipiperidid (ADDP) [vgl. z.B. D. L. Hughes, Org. Reactions 42, 335 (1992); D. L. Hughes, Org. Prep. Proced. Int. 28 (2), 127 (1996)]. Die Reaktion wird vorzugsweise in Tetrahydrofuran (THF) oder Dichlormethan als Lösungsmittel in einem Temperaturbereich zwischen 0°C und RT durchgeführt.

Anstelle des Restes $R^8$ in den Aza-Heterocyclen der Formel (17) kann bei dem zuvor beschriebenen Verfahren zunächst auch eine geeignete Amid-Schutzgruppe eingesetzt werden, wenn dies zur Vermeidung von Nebenreaktionen zweckmäßig oder erforderlich ist. Nach Abspaltung einer solchen Schutzgruppe am Ende der obigen Reaktionssequenz kann dann eine weitere Derivatisierung im Bedeutungsumfang von $R^8$ über entsprechende Alkylierungs- oder Acylierungsreaktionen erfolgen, wie sie dem Fachmann geläufig sind [zur Eignung, Einführung und Entfernung von Amid-Schutzgruppen siehe z.B. T.W. Greene und P.G.M. Wuts, Protective Groups in Organic Synthesis, Wiley, New York, 1999].

Erfindungsgemäße Verbindungen der Formel (I-6)

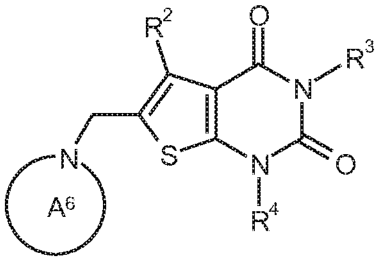

(I-6),

in welcher

der Ring $A^6$ für ein Imidazol-2-on-Derivat der Formel

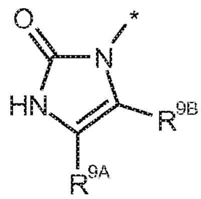

steht,

worin * die Verknüpfung zur angrenzenden $CH_2$-Gruppe markiert
und

$R^{9A}$ und $R^{9B}$ die oben angegebenen Bedeutungen haben,

und

$R^2$, $R^3$ und $R^4$ die oben angegebenen Bedeutungen haben,

lassen sich gemäß nachfolgendem Reaktionsschema 6 herstellen:

Schema 6

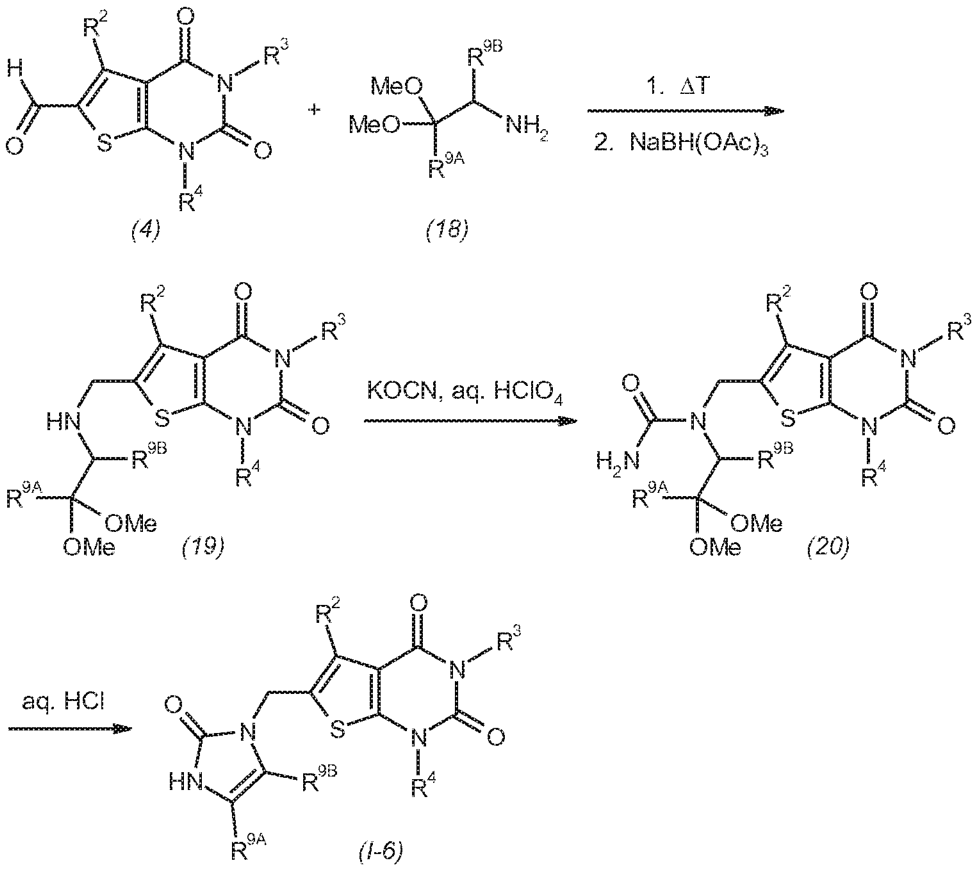


(4) (18) (19) (20) (I-6)

Aldehyde der Formel (4) werden hier mit Aminoacetalen oder Aminoketalen der Formel (18) im Sinne einer reduktiven Aminierung zunächst in einem geeigneten Lösungsmittel, wie Methanol oder Dichlormethan, zum Rückfluss erhitzt und dann bei RT mit Natriumtriacetoxyborhydrid zu den Verbindungen der Formel (19) reduziert. Diese werden anschließend mit Kaliumcyanat und wässriger Perchlorsäure in Methanol bei RT in die Harnstoff-Derivate der Formel (20) überfuhrt. Im letzten Reaktionsschritt erfolgt die simultane, säurekatalysierte Acetal- bzw. Ketalspaltung und der Ringschluss zu den Zielverbindungen der Formel (I-6). Die Reaktion erfolgt in Methanol bei RT mit Salzsäure unterschiedlicher Konzentration (von 0.5 mol/L bis zu konzentrierter Salzsäure).

In den Formeln (18), (19) und (20) sind jeweils die Dimethylacetale bzw. Dimethylketale dargestellt; es können bei diesem Verfahren jedoch auch andere übliche Acetale und Ketale zum Einsatz gebracht werden, insbesondere cyclische wie beispielsweise 1,3-Dioxolan- oder 1,3-Dioxan-Derivate.

Erfindungsgemäße Verbindungen der Formel (I-7)

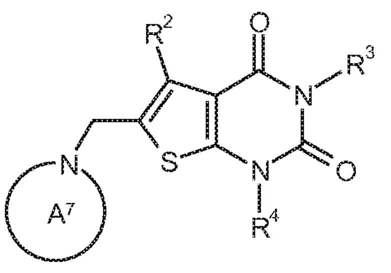

(I-7),

in welcher

der Ring $A^7$ für ein 1,2,4-Triazol-3-on-Derivat der Formel

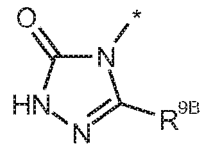 steht,

worin * die Verknüpfung zur angrenzenden $CH_2$-Gruppe markiert und $R^{9B}$ die oben angegebene Bedeutung hat,

und
$R^2$, $R^3$ und $R^4$ die oben angegebenen Bedeutungen haben,
sind gemäß dem nachfolgenden Reaktionsschema 7 zugänglich:

Schema 7

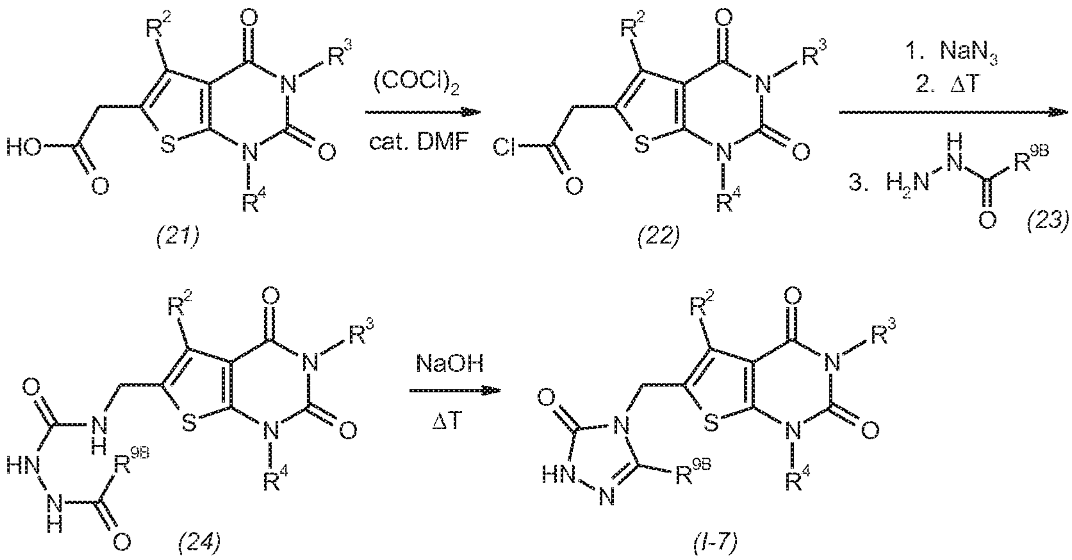

(21) (22) (23) (24) (I-7)

Carbonsäuren der Formel (21) werden hier zunächst auf übliche Weise, zum Beispiel durch Umsetzung mit Oxalylchlorid in Dichlormethan bei RT in Gegenwart einer katalytischen Menge an *N,N*-Dimethylformamid (DMF), in die korrespondierenden Säurechloride (22) überführt. Anschließend werden in einem mehrstufigen Eintopfverfahren die offenkettigen Intermediate der Formel (24) hergestellt, indem zunächst die Säurechloride der Formel (22) in Toluol gelöst und bei RT mit Natriumazid in die entsprechenden Carbonsäureazide überführt werden. Nach Filtration zur Abtrennung anorganischer Salze werden die so erhaltenen Lösungen in Toluol zum Rückfluss erhitzt, wodurch im Rahmen einer Curtius-Umlagerung die entsprechenden Isocyanate erhalten werden. Diese werden im letzten Teilschritt der Umsetzung bei RT mit der Lösung eines Acylhydrazins der Formel (23) in Tetrahydrofuran (THF) versetzt und liefern so die offenkettigen Intermediate der Formel (24). Erwärmung dieser Intermediate mit anorganischen Basen, zum Beispiel Natriumhydroxid, in einem alkoholischen Lösungsmittel wie Methanol führt schließlich durch Cyclisierung zu den Zielverbindungen der Formel (I-7).

Erfindungsgemäße Verbindungen der Formel (I-8)

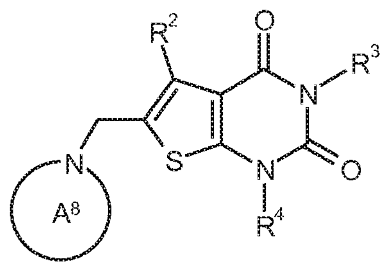
(I-8),

in welcher

der Ring $A^8$ für ein Pyrazol-3-on-Derivat der Formel

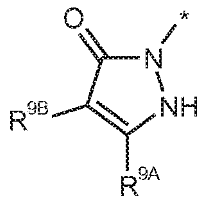

steht,

worin * die Verknüpfung zur angrenzenden $CH_2$-Gruppe markiert und

$R^{9A}$ und $R^{9B}$ die oben angegebenen Bedeutungen haben,

und

$R^2$, $R^3$ und $R^4$ die oben angegebenen Bedeutungen haben,

sind gemäß nachfolgendem Reaktionsschema 8 erhältlich:

Schema 8

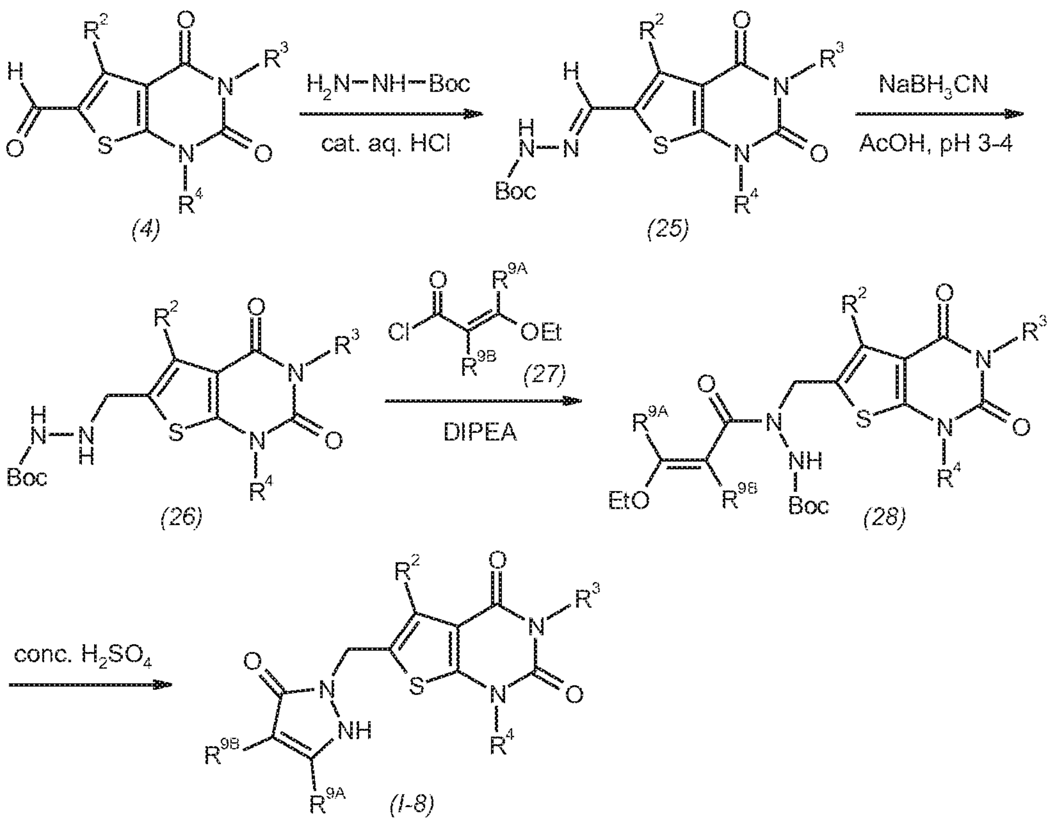


(4) (25) (26) (28) (I-8)

Aldehyde der Formel (4) werden hier durch Reaktion mit Boc-geschütztem Hydrazin in Ethanol und in Gegenwart einer katalytischen Menge konzentrierter Salzsäure bei RT in die Hydrazone der Formel (25) überfuhrt, die dann mit Natriumcyanoborhydrid in Methanol bei +65°C zu den Hydrazinderivaten der Formel (26) reduziert werden. Bei letzterer Reaktion spielt die genaue Kontrolle des pH-Wertes eine große Rolle: In Gegenwart von Bromkresolgrün als Indikator wird durch portionsweisen Zusatz von Essigsäure während der gesamten Reaktionsdauer ein pH-Wert von ca. 3-4

eingehalten. Die anschließende Umsetzung mit den Acrylsäurechloriden der Formel (27) wird unter üblichen Bedingungen durchgeführt, beispielsweise in Dichlormethan als Lösungsmittel in einem Temperaturbereich zwischen 0°C und RT und in Gegenwart einer tertiären Aminbase, wie beispielsweise *N,N*-Diisopropylethylamin. Die abschließende säurekatalysierte Entfernung der Boc-Schutzgruppe und der daraufhin erfolgende Ringschluss zu den Zielverbindungen der Formel (I-8) erfolgt bei RT entweder in reiner konzentrierter Schwefelsäure oder in Dichlormethan, dem eine katalytische Menge konzentrierter Schwefelsäure zugesetzt ist.

Erfindungsgemäße Verbindungen der Formel (I-9)

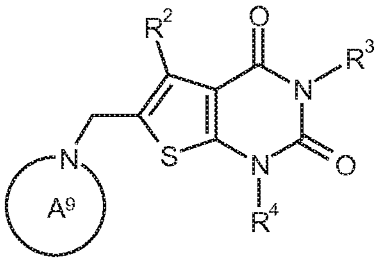

(I-9),

in welcher

der Ring $A^9$ für einen Aza-Heterocyclus der Formel

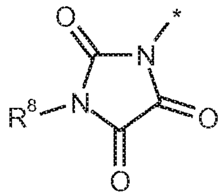

steht,

worin * die Verknüpfung zur angrenzenden $CH_2$-Gruppe markiert
und
$R^8$ die oben angegebene Bedeutung hat,

und
$R^2$, $R^3$ und $R^4$ die oben angegebenen Bedeutungen haben,
können gemäß dem nachfolgenden Reaktionsschema 9 erhalten werden:

Schema 9

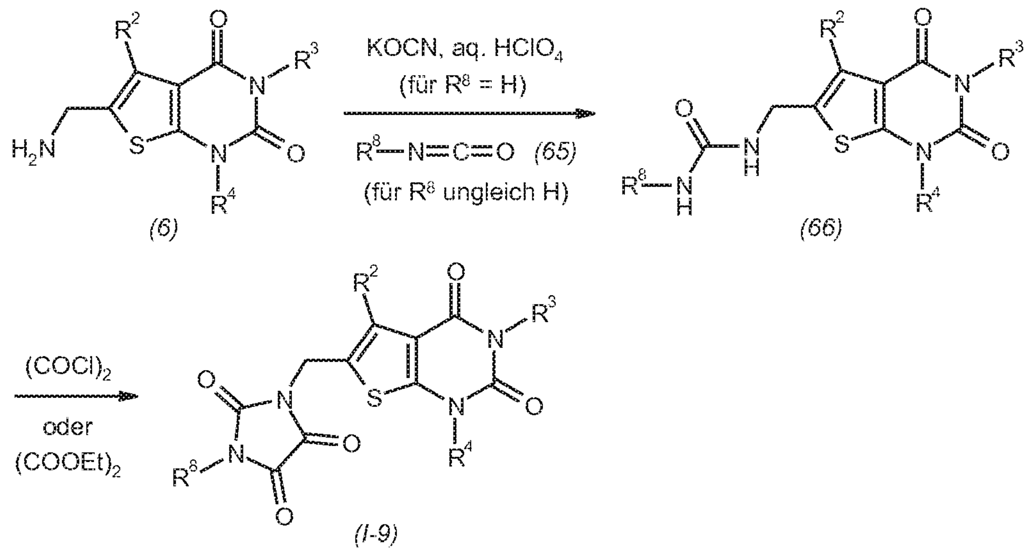


(6) (66) (I-9)

Die zuvor in Schema 2 beschriebene Aminomethylverbindung der Formel (6) wird hier im Fall, dass $R^8$ im Zielprodukt (I-9) für Wasserstoff steht, mit einem Gemisch aus Kaliumcyanat und wässriger Perchlorsäure in Methanol bei RT oder, wenn $R^8$ von Wasserstoff verschieden ist, mit einem Isocyanat der Formel (65) in das korrespondierende Harnstoff-Derivat der Formel (66) überführt. Die Isocyanat-Reaktion kann in einem etherischen Lösungsmittel, wie zum Beispiel Tetrahydrofuran (THF) oder 1,4-Dioxan, gegebenenfalls unter Zusatz einer tertiären Aminbase, wie zum Beispiel Triethylamin, erfolgen; alternativ läßt sich die Reaktion beispielsweise auch in Pyridin als Lösungsmittel und als Base durchführen. Die Umsetzung mit dem Isocyanat (65) erfolgt im Allgemeinen in einem Temperaturbereich zwischen 0°C und ca. +50°C, bevorzugt bei RT. Die nachfolgende Ringbildung zur Zielverbindung (I-9) wird durch Umsetzung von (66) mit einem Oxalsäure-Derivat, wie zum Beispiel Oxalylchlorid oder Oxalsäurediethylester, erreicht. Die Reaktion mit Oxalylchlorid wird bevorzugt in einem etherischen Lösungsmittel, wie zum Beispiel Tetrahydrofuran (THF) oder 1,4-Dioxan, oder in einem halogenierten Kohlenwasserstoff, wie zum Beispiel Dichlormethan oder 1,2-Dichlorethan, oder in Acetonitril durchgeführt. Die Umsetzung kann in Gegenwart einer üblichen Aminbase, wie zum Beispiel Triethylamin, *N,N*-Diisopropylethylamin oder Pyridin, erfolgen und wird in der Regel in einem Temperaturbereich zwischen 0°C und ca. +60°C durchgeführt, bevorzugt bei 0°C bis RT. Die Umsetzung mit Oxalsäurediethylester erfolgt bevorzugt in einem alkoholischen Lösungsmittel, wie Methanol oder Ethanol, in Gegenwart von Natriummethylat oder Natriumethylat als Base. Die Reaktionstemperatur liegt hierbei im Bereich zwischen RT und dem Siedepunkt des betreffenden Alkohols.

Anstelle des Restes $R^8$ im Isocyanat der Formel (65) kann bei dem zuvor beschriebenen Verfahren auch eine temporäre Amid-Schutzgruppe eingesetzt werden, wenn dies zur Vermeidung von Nebenreaktionen zweckmäßig oder erforderlich ist. Nach Abspaltung einer solchen Schutzgruppe am Ende der obigen Reaktionssequenz kann dann eine weitere Derivatisierung im Bedeutungsumfang von $R^8$ über entsprechende Alkylierungs- oder Acylierungsreaktionen erfolgen, wie sie dem Fachmann geläufig sind [zur Eignung, Einführung und Entfernung von Amid-Schutzgruppen siehe z.B. T.W. Greene und P.G.M. Wuts, Protective Groups in Organic Synthesis, Wiley, New York, 1999].

Erfindungsgemäße Verbindungen der Formel (I-10)

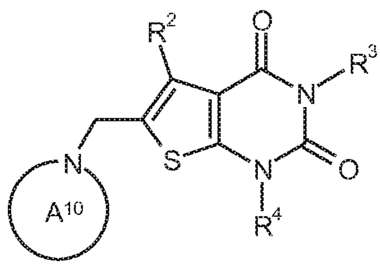

(I-10),

in welcher

der Ring $A^{10}$ für ein 1,2,4-Triazol-3-on-Derivat der Formel

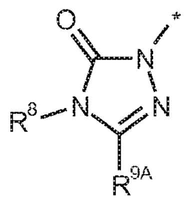

steht,

worin * die Verknüpfung zur angrenzenden $CH_2$-Gruppe markiert und

$R^8$ und $R^{9A}$ die oben angegebenen Bedeutungen haben,

und

$R^2$, $R^3$ und $R^4$ die oben angegebenen Bedeutungen haben,

können nach dem folgenden Reaktionsschema 10 hergestellt weiden:

Schema 10

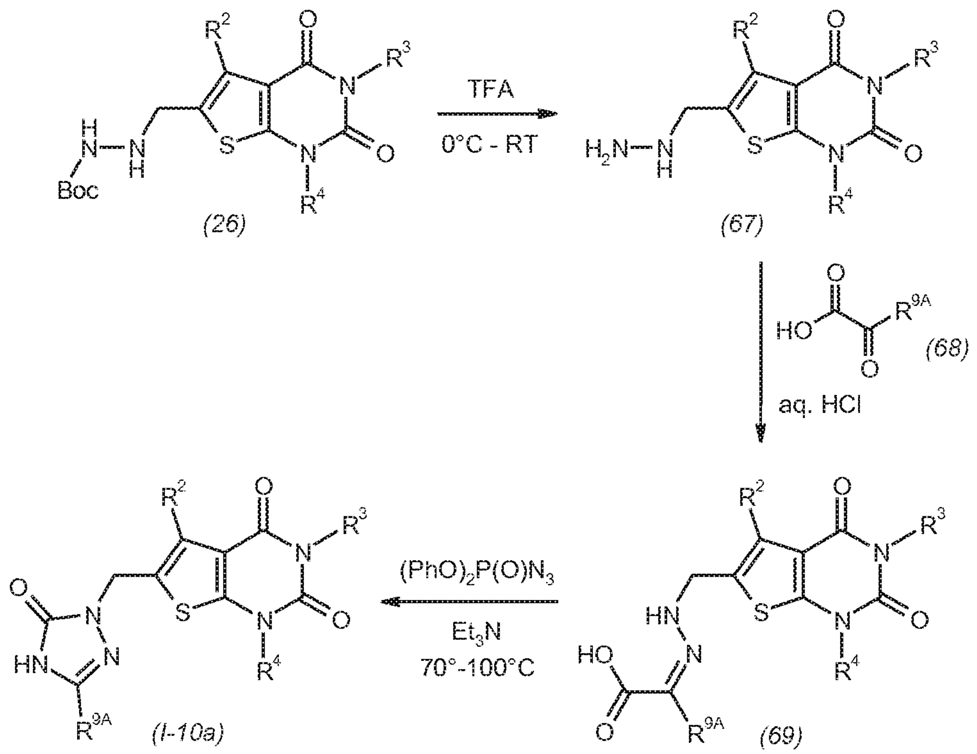


In diesem Verfahren wird das geschützte Hydrazin-Derivat der Formel (26) (siehe Schema 8) zunächst mit Trifluoressigsäure in Dichlormethan in das freie Hydrazin der Formel (67) überführt. Die Boc-Abspaltung erfolgt in einem Temperaturbereich zwischen 0°C und RT, bevorzugt bei 0°C. Um eine Zersetzung des Produkts zu vermeiden, sollte hier keine längere Reaktionszeit gewählt werden als erforderlich, außerdem sollten nachfolgende Aufarbeitungs- und Reinigungsoperationen bei RT durchgeführt werden. In Analogie zu einem vorbeschriebenen, zweistufigen Verfahren [siehe US-Patent US 6 077 814, Referential Production Examples 1-4] wird das Hydrazin der Formel (67) zunächst mit Glyoxalsäure (68) [$R^{9A}$ = H] unter Säure-Katalyse zum Hydrazon der Formel (69) kondensiert. Die Umsetzung erfolgt in Wasser in Gegenwart von Salzsäure in einem Temperaturbereich zwischen 0°C und RT, bevorzugt bei +10°C bis +20°C. Anschließend wird die Hydrazonocarbonsäure (69) mit Diphenylphosphorylazid (DPPA) in das entsprechende Carbonsäureazid überführt, das dann *in situ* im Sinne einer Curtius-Umlagerung das korrespondierende Isocyanat ergibt,

welches spontan zum Triazolon-Derivat der Formel (I-10a) cyclisiert. Die Reaktion erfolgt in einem inerten Lösungsmittel, wie beispielsweise Toluol, und in Gegenwart einer tertiären Aminbase, wie zum Beispiel Triethylamin. Die Umsetzung wird anfangs in einem Temperaturbereich zwischen ca. +40°C und +80°C durchgeführt; im weiteren Verlauf wird die Reaktionstemperatur dann auf +100°C bis +110°C erhöht.

Durch Einsatz entsprechender 2-Oxocarbonsäuren (68) sind nach diesem Verfahren prinzipiell auch diejenigen erfindungsgemäßen Verbindungen der Formel (I-10a) zugänglich, in denen $R^{9A}$ für ($C_1$-$C_4$)-Alkyl steht. Ferner können durch nachgelagerte Umsetzungen in Form von Alkylierungs- und Acylierungsreaktionen, wie sie dem Fachmann allgemein bekannt sind, solche erfindungsgemäßen Verbindungen der Formel (I-10) erhalten werden, in denen $R^8$ im oben angegebenen Bedeutungsumfang von Wasserstoff verschieden ist.

Erfindungsgemäße Verbindungen der Formel (I-11)

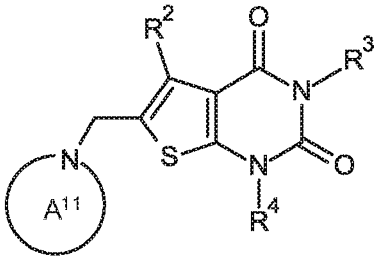

(I-11),

in welcher

der Ring $A^{11}$ für ein Dihydro-1,2,4-triazol-3-on der Formel

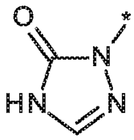

steht,

worin * die Verknüpfung zur angrenzenden $CH_2$-Gruppe markiert,
und

$R^2$, $R^3$ und $R^4$ die oben angegebenen Bedeutungen haben,

können auch auf alternativem Wege gemäß Reaktionsschema 11 hergestellt werden:

Schema 11

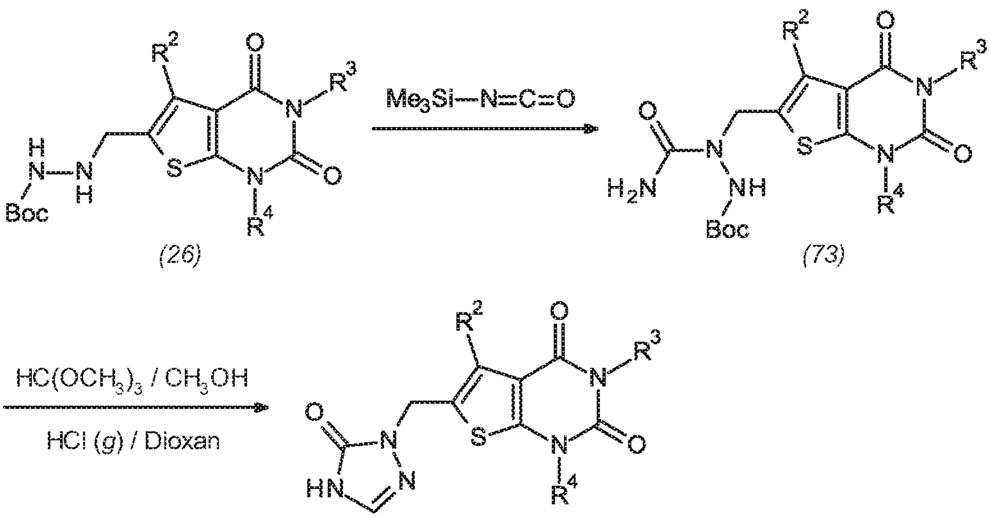


(26) (73)

(I-11)

Verbindungen der Formel (26) (siehe Schema 8) werden hier zunächst mit Trimethylsilylisocyanat zu Harnstoff-Derivaten der Formel (73) umgesetzt. Die Reaktion wird in einem Alkohol als Lösungsmittel, bevorzugt in Isopropanol, bei erhöhter Temperatur, bevorzugt bei ca. +50°C, durchgeführt. Unter diesen Bedingungen erfolgt simultan auch eine Abspaltung der Trimethylsilyl-Gruppe. Der Ringschluss zu den Zielverbindungen der Formel (I-11) wird durch Säure-vermittelte Umsetzung mit Trimethylorthoformiat erreicht. Dazu werden die Verbindungen der Formel (73) in Gegenwart von Chlorwasserstoff mit einem Überschuss an Trimethylorthoformiat in Methanol behandelt. Die Reaktion wird bevorzugt bei Raumtemperatur durchgeführt.

Erfindungsgemäße Verbindungen der Formel (I-12)

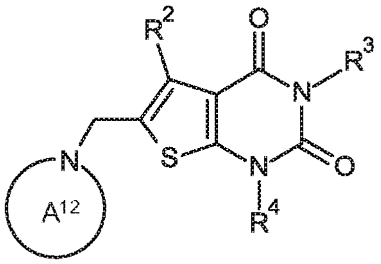

(I-12),

in welcher

der Ring $A^{12}$ für ein Piperazin-2,5-dion-Derivat der Formel

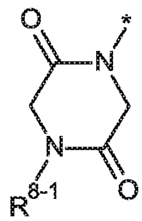

steht,

worin * die Verknüpfung zur angrenzenden $CH_2$-Gruppe markiert und $R^{8-1}$ für Wasserstoff, ($C_1$-$C_4$)-Alkyl oder ($C_2$-$C_4$)-Alkenyl steht,

und $R^2$, $R^3$ und $R^4$ die oben angegebenen Bedeutungen haben, können gemäß dem nachfolgenden Reaktionsschema 12 erhalten werden:

Schema 12

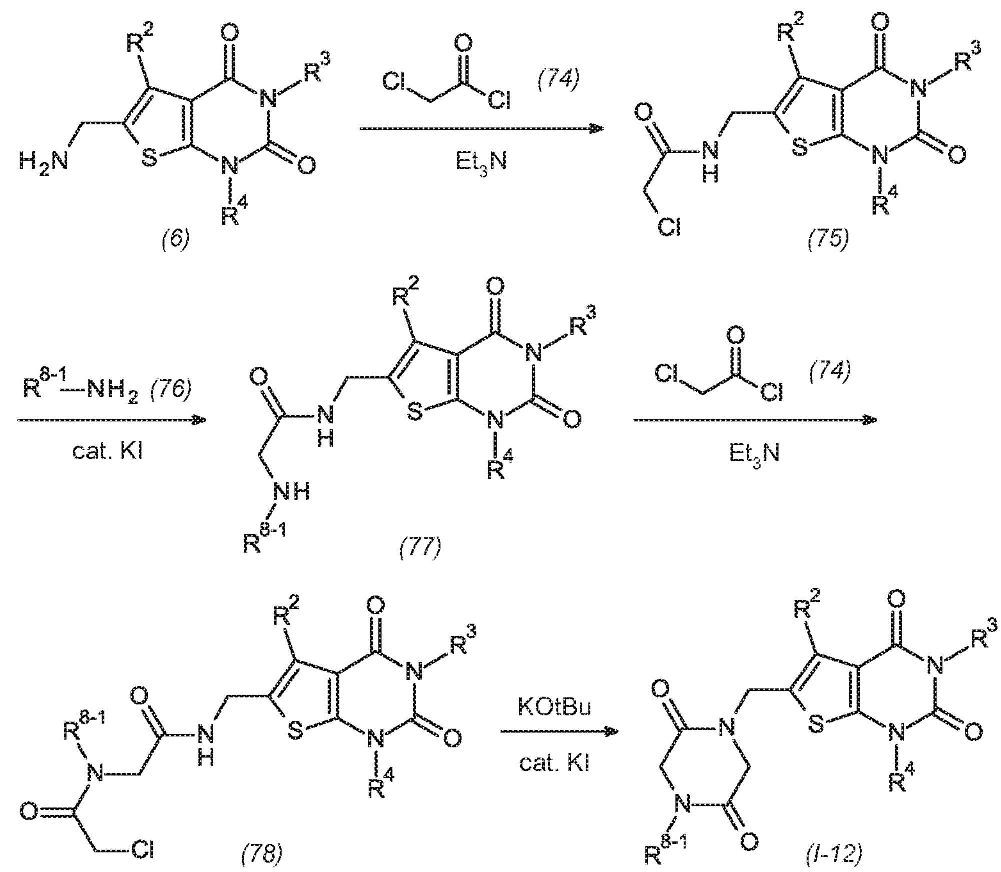


Die Aminomethyl-Verbindungen der Formel (6) (siehe Schema 2) werden hier zunächst mit Chloracetylchlorid (74) zu α-Chloramiden der Formel (75) umgesetzt. Die Reaktion erfolgt in einem inerten Lösungsmittel, wie beispielsweise und bevorzugt Dichlormethan, in Gegenwart einer üblichen tertiären Aminbase, wie beispielsweise Triethylamin oder *N,N*-Diisopropylethylamin, in einem Temperaturbereich zwischen 0°C und RT. Die Verbindungen der Formel (75) werden dann mit Ammoniak oder einem primären Amin der Formel (76) zu α-Aminoamiden der Formel (77) umgesetzt. Diese Reaktion erfolgt entweder mit konzentriertem Ammoniakwasser oder dem entsprechenden primären Amin (76) in *N,N*-Dimethylformamid (DMF) als Lösungsmittel in Gegenwart einer katalytischen Menge Kaliumiodids bei einer Temperatur von ca. +50°C. Die α-Aminoamide (77) werden anschließend erneut mit Chloracetylchlorid (74) unter gleichen Bedingungen, wie zuvor beschrieben, zu α-Chloramiden der Formel (78) umgesetzt. Der abschließende Ringschluss zu den Zielverbindungen der Formel (I-12) wird mit Hilfe einer starken Base, wie zum Beispiel Kalium-*tert*.-butylat, in Gegenwart einer katalytischen Menge Kaliumiodids erreicht. Die Reaktion wird in einem etherischen Lösungsmittel, wie beispielsweise und bevorzugt Tetrahydrofuran (THF), bei RT durchgeführt.

Weitere am Aza-Heterocyclus A substituierte Beispiele der erfindungsgemäßen Verbindungen gemäß Formel (I) können auf analoge Weise erhalten werden, indem entsprechend substituierte Ausgangsverbindungen in den zuvor beschriebenen Verfahren der Schemata 1-12 eingesetzt werden. In solchen Ausgangsverbindungen gegebenenfalls vorhandene Hydroxy-, Amino- und/oder Amido-Gruppen können dabei, falls zweckmäßig oder erforderlich, auch in temporär geschützter Form eingesetzt und dann am Ende der jeweiligen Reaktionssequenz wieder freigesetzt werden [zur Eignung, Einführung und Entfernung solcher Schutzgruppen siehe z.B. T.W. Greene und P.G.M. Wuts, Protective Groups in Organic Synthesis, Wiley, New York, 1999].

Die Synthese der für die Herstellung der erfindungsgemäßen Verbindungen verwendeten Thienouracil-Intermediate der Formeln (1), (4), (21) und (70) [siehe Schemata 1-12] ist in den nachfolgenden Reaktionsschemata 13-21 dargestellt:

Schema 13

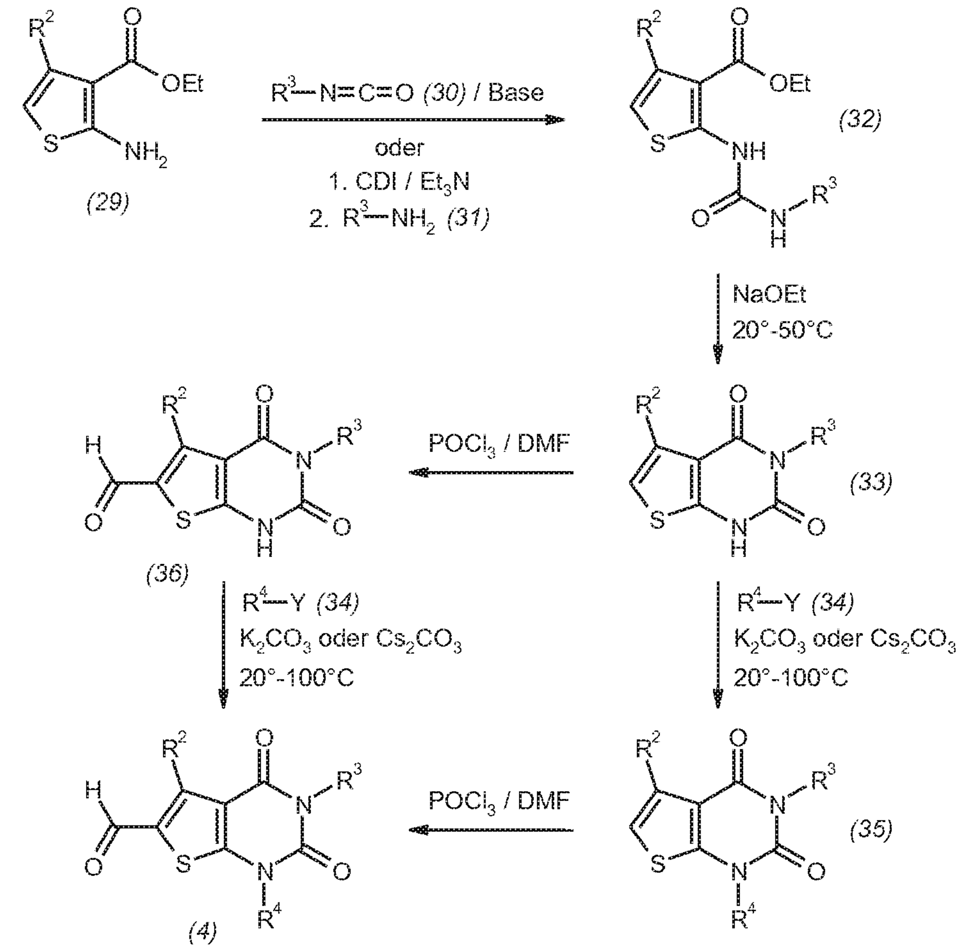


[Y = Cl, Br, I oder OTs].

2-Aminothiophen-3-carbonsäureester der Formel (29) werden hierbei entweder mit Isocyanaten der Formel (30) oder, nach Aktivierung mit *N,N'*-Carbonyldiimidazol (CDI), durch Reaktion mit Aminen der Formel (31) in die Harnstoffe der Formel (32) überführt. Die Reaktion mit den Isocyanaten (30) erfolgt bevorzugt in einem etherischen Lösungsmittel, beispielsweise in Tetrahydrofuran (THF), und in Gegenwart einer tertiären Aminbase, wie beispielsweise Triethylamin, unter Rückflussbedingungen, oder in reinem Pyridin als Lösungsmittel und Base bei einer Temperatur von ca. +50°C. Die Aktivierung der 2-Aminothiophen-3-carbonsäureester (29) mit CDI wird gleichfalls in Gegenwart einer tertiären Aminbase, wie beispielsweise Triethylamin, in einem inerten Lösungsmittel, vorzugsweise in Tetrahydrofuran (THF) oder Dichlormethan, bei RT durchgeführt und erfordert mitunter längere Reaktionszeiten von mehreren Tagen. Nach Zugabe der Amin-Komponente (31) zum CDI-aktivierten 2-Aminothiophen-3-carbonsäureester erfolgt bei RT in der Regel schnelle Weiterreaktion zu den Harnstoffen der Formel (32). Durch nachfolgende Behandlung mit Alkalimetall-Alkoholaten im entsprechenden Alkohol als Lösungsmittel (beispielsweise und bevorzugt Natriumethanolat in Ethanol) wird in sauberer Reaktion der Ringschluss zu den Thienouracilen der Formel (33) erreicht. In Abhängigkeit vom Substituenten $R^3$ erfolgt die Reaktion bereits bei RT, oder sie erfordert etwas erhöhte Temperatur um +50°C.

Die anschließende Alkylierung mit den Verbindungen der Formel (34) wird in Gegenwart einer anorganischen Base, wie zum Beispiel Kalium- oder Cäsiumcarbonat, in einem inerten Lösungsmittel, wie beispielsweise und bevorzugt *N,N*-Dimethylformamid (DMF), Tetrahydrofuran (THF), Acetonitril oder Gemischen hiervon, durchgeführt. Die Reaktionstemperatur liegt üblicherweise zwischen RT und ca. +100°C. Bei leicht flüchtigen Alkylierungsmitteln (34) erweist sich die Verwendung von verschlossenen Reaktionsgefäßen und die Erwärmung mittels eines Mikrowellenofens als hilfreich. In Abhängigkeit von der Natur der Abgangsgruppe Y kann es vorteilhaft sein, die Alkylierung in Gegenwart einer katalytischen Menge Kaliumiodids durchzuführen. Die so erhaltenen Verbindungen der Formel (35) werden dann in einer Vilsmeier-Haack-Reaktion mit einem Gemisch aus Phosphoroxychlorid und *N,N*-Dimethylformamid (DMF) in exothermer Reaktion in die Aldehyde der Formel (4) überführt. Üblicherweise reicht die während der Reaktion freigesetzte Wärme aus, um vollständigen Umsatz zu erreichen. Manchmal kann es jedoch auch erforderlich sein, das Gemisch

nach Abklingen der Reaktionswärme noch einige Zeit auf ca. +90°C zu erwärmen.

Die obige Reaktionssequenz aus Alkylierung und Formylierung läßt sich auch in vertauschter Reihenfolge durchführen, indem die Thienouracile der Formel (33) zunächst unter den bereits beschriebenen Bedingungen der Vilsmeier-Haack-Reaktion in die Formylderivate der Formel (36) überführt werden und dann unter den ebenfalls bereits beschriebenen Bedingungen mit den Verbindungen der Formel (34) zu den Ziel-Aldehyden der Formel (4) alkyliert werden.

Schema 14

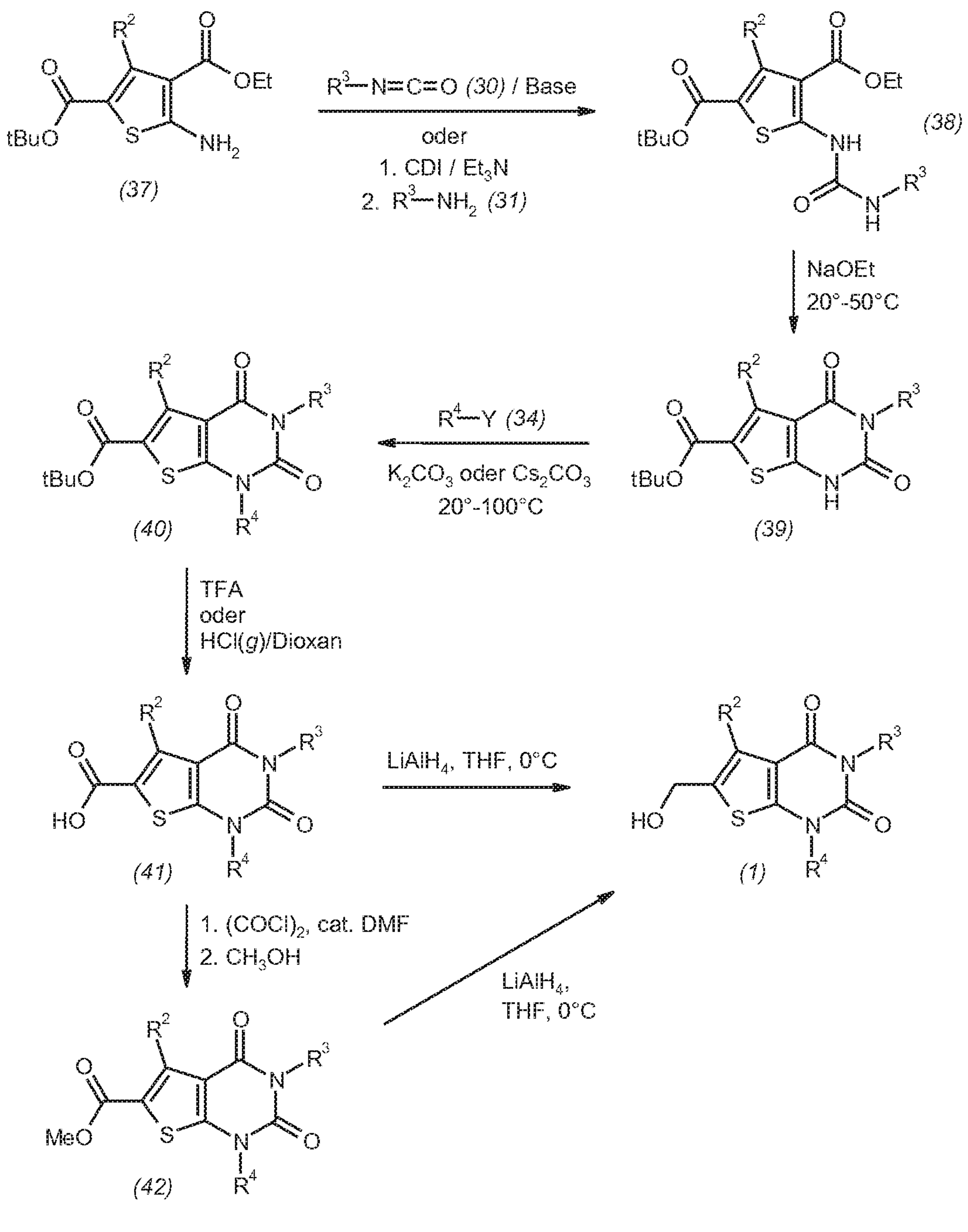


[Y = Cl, Br, I oder OTs].

Aus 5-Aminothiophen-2,4-dicarbonsäureestern der Formel (37) lassen sich die Thienouracil-*tert.*-butylester der Formel (40) in völlig analoger Weise zu den in Schema 13 für die Herstellung der Intermediate (32), (33) und (35) beschriebenen Reaktionen erhalten. Anschließende Behandlung der *tert.*-Butylester (40) bei RT mit entweder Trifluoressigsäure in Dichlormethan oder einer Lösung von Chlorwasserstoff in 1,4-Dioxan liefert die Carbonsäuren der Formel

(41). Diese können dann entweder direkt durch Reduktion mit Lithiumaluminiumhydrid bei ca. 0°C in einem inerten Lösungsmittel, wie beispielsweise und bevorzugt Tetrahydrofuran (THF), in die Alkohole der Formel (1) umgewandelt werden, oder nach vorheriger Überführung in die entsprechenden Methylester der Formel (42). Letztere können in einem Eintopfverfahren erhalten werden, indem die Carbonsäuren der Formel (41) zunächst bei RT mit Oxalylchlorid in Dichlormethan und in Gegenwart einer katalytischen Menge *N,N*-Dimethylformamid (DMF) in die korrespondierenden Säurechloride überführt werden, die dann durch Quenchen mit Methanol die Methylester der Formel (42) ergeben.

Schema 15

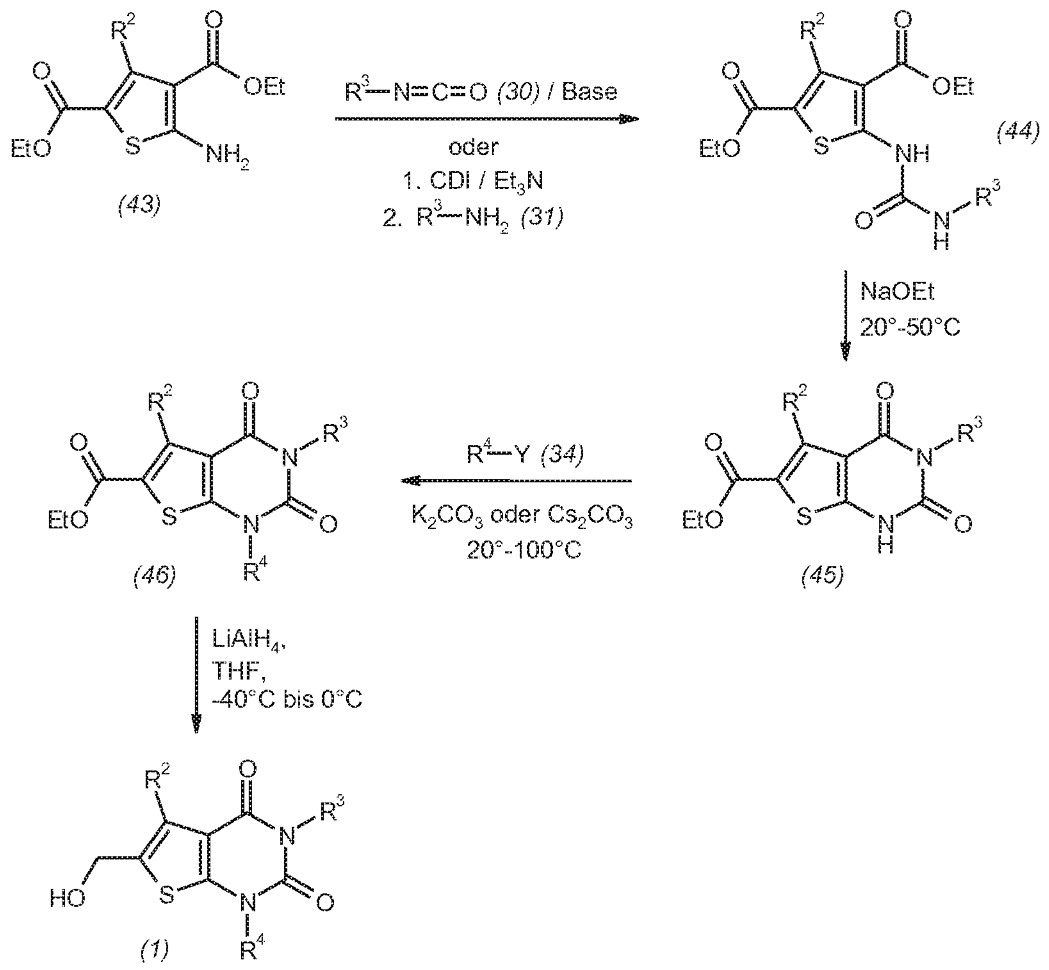


[Y = Cl, Br, I oder OTs].

Aus 5-Aminothiophen-2,4-dicarbonsäurediethylestern der Formel (43) lassen sich die Thienouracilethylester der Formel (46) ebenfalls in völlig analoger Weise zu den in Schema 13 für die Herstellung der Intermediate (32), (33) und (35) beschriebenen Reaktionen erhalten. Die anschließende Reduktion mit einem komplexen Metallhydrid, wie beispielsweise und bevorzugt Lithiumaluminiumhydrid, liefert dann in ähnlicher Weise, wie zuvor in Schema 14 beschrieben, die Alkohole der Formel (1). Die Reaktion erfolgt typischerweise in einem Temperaturbereich zwischen -40°C und 0°C in einem inerten Lösungsmittel, wie beispielhaft und vorzugsweise Tetrahydrofuran (THF).

Schema 16

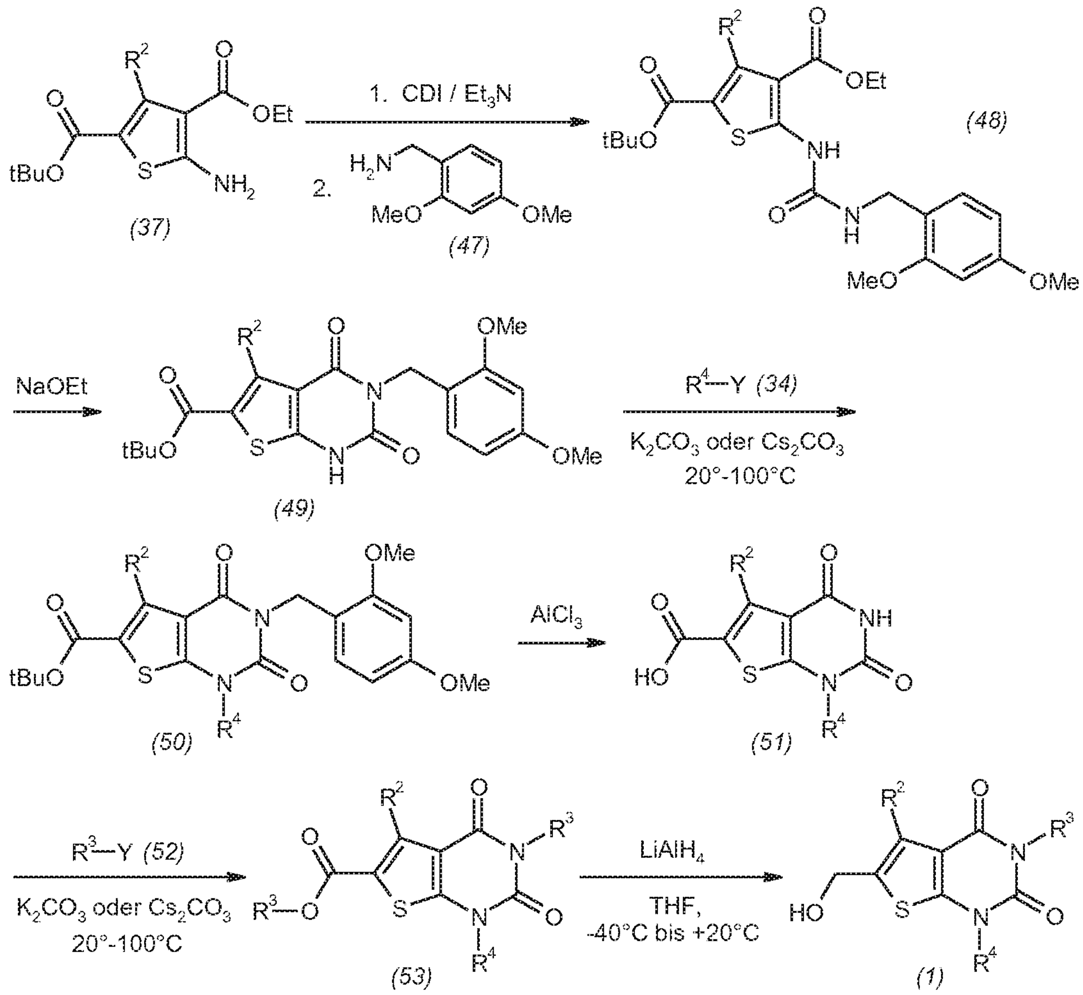


[Y = Cl, Br, I oder OTs].

Analog zu dem in Schema 14 [Verbindung (37) bis Verbindung (40)] beschriebenen Verfahren lassen sich aus 5-Aminothiophen-2,4-dicarbonsäureestern der Formel (37) und 2,4-Dimethoxybenzylamin (47) die $N^3$-geschützten Thienouracil-*tert.*-butylester der Formel (50) darstellen. Bei der nachfolgenden Behandlung mit Aluminiumtrichlorid, die in Toluol bei ca. +65°C erfolgt, werden durch simultane Abspaltung der 2,4-Dimethoxybenzyl-Schutzgruppe und des *tert.*-Butylesters die Carbonsäuren der Formel (51) erhalten. Diese werden anschließend in einer doppelten N,O-Alkylierung mit den Verbindungen der Formel (52) zu den Verbindungen der Formel (53) umgesetzt. Diese Alkylierung wird unter den gleichen Bedingungen durchgeführt, wie sie bereits für die Alkylierungsschritte in den vorangegangenen Syntheseschemata beschrieben wurden (siehe zum Beispiel Schema 13). Die abschließende Reduktion mit einem komplexen Metallhydrid, wie beispielsweise und bevorzugt Lithiumaluminiumhydrid, liefert dann die Ziel-Alkohole der Formel (1). Die Reaktion erfolgt typischerweise in einem Temperaturbereich zwischen -40°C und RT in einem inerten Lösungsmittel, wie beispielhaft und vorzugsweise Tetrahydrofuran (THF).

Schema 17

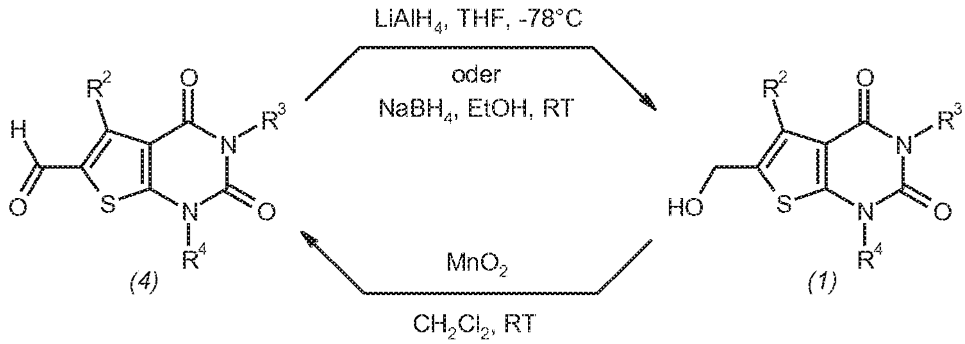


(4) (1)

Die nach einem der zuvor beschriebenen Verfahren erhaltenen Aldehyde der Formel (4) und Alkohole der Formel (1) lassen sich, falls es für Synthesezwecke wünschenswert erscheint, nach mehreren, dem Fachmann geläufigen Methoden ineinander umwandeln. So können beispielsweise die Alkohole der Formel (1) mit Mangandioxid in Dichlormethan bei RT zu den Aldehyden der Formel (4) oxidiert werden. Umgekehrt lassen sich die Aldehyde der Formel (4) mit komplexen Hydriden, wie zum Beispiel Lithiumaluminiumhydrid oder Natriumborhydrid, zu den Alkoholen der Formel (1) reduzieren. Die Reduktion mit Lithiumaluminiumhydrid erfolgt dabei vorzugsweise in Tetrahydrofuran (THF) bei -78°C, während die Reduktion mit Natriumborhydrid beispielsweise in Ethanol bei RT erfolgen kann.

Schema 18

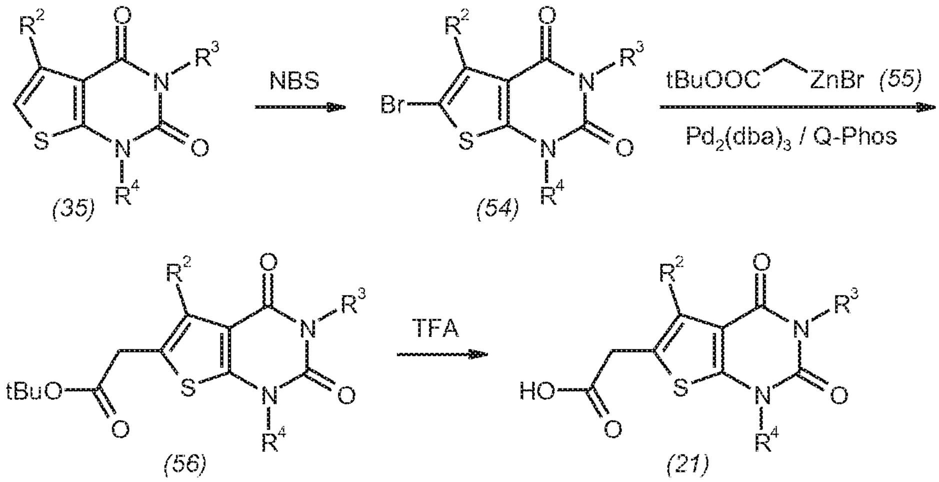


(35) (54) (56) (21)

Die Thienouracil-Intermediate der Formel (21) lassen sich aus den Thienouracilen der Formel (35), deren Herstellung in Schema 13 beschrieben ist, erhalten, indem zunächst die Verbindungen der Formel (35) mit *N*-Bromsuccinimid (NBS), *N*-Iodsuccinimid (NIS) oder *N*-Chlorsuccinimid (NCS) in die entsprechenden Halogenide der Formel (54) (hier: Halogen = Brom) überfuhrt werden. Die Reaktion erfolgt vorzugsweise in Chloroform in einem Temperaturbereich zwischen 0°C und RT. Durch anschließende Negishi-Kupplung mit dem Zinkorganyl (55) werden die *tert*.-Butylester-Derivate der Formel (56) erhalten. Die Kupplungsreaktion wird vorzugsweise in einem etherischen Lösungsmittel, wie zum Beispiel Tetrahydrofuran (THF), bei einer Temperatur von ca. +60°C durchgeführt. Für diese Umsetzung eignet sich eine Vielzahl von homogenen Palladium(0)-Katalysatoren in Kombination mit verschiedenen Phosphinliganden; bevorzugt wird Tris(diben-zylidenacton)dipalladium(0) in Verbindung mit 1,2,3,4,5-Pentaphenyl-1'-(di-*tert*.-butylphosphino)-ferrocen (Q-Phos) eingesetzt. Die abschließende Spaltung der *tert*.-Butylester-Gruppierung, beispielsweise mit Trifluoressigsäure in Dichlormethan bei RT, liefert in sauberer Reaktion die Ziel-Carbonsäuren der Formel (21).

6-Acetylthienouracile der Formel (70) (siehe Schema 4a) können auf einfache Weise nach dem in Schema 19 wiedergegebenen Verfahren erhalten werden:

Schema 19

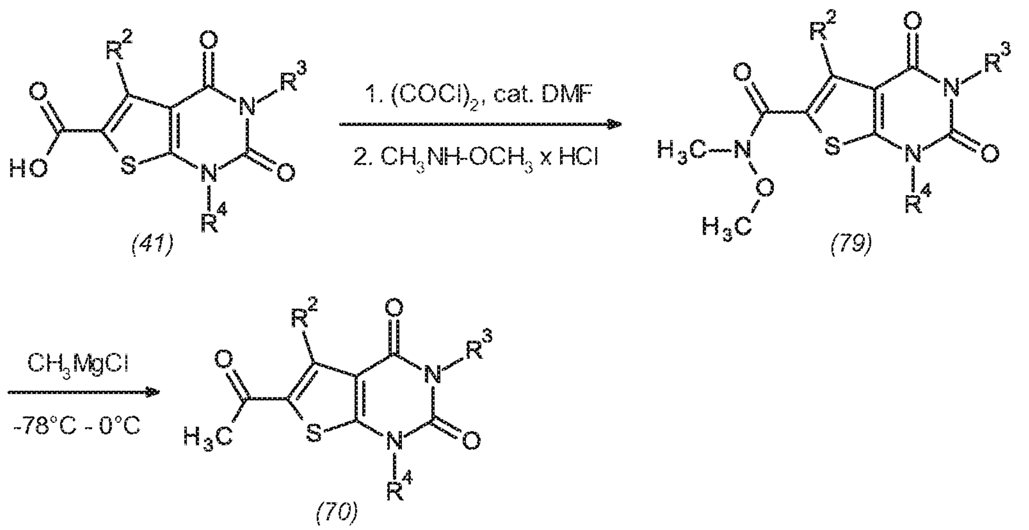


(41) (79) (70)

Thienouracilcarbonsäuren der Formel (41) (siehe Schema 14) werden hier zunächst in Weinreb-Amide der Formel (79) überführt. Dazu werden die Carbonsäuren (41) mit einem Chlorierungsmittel, wie zum Beispiel Oxalylchlorid in Gegenwart einer katalytischen Menge DMF, zu den korrespondierenden Säurechloriden umgesetzt, die anschließend mit *N*,*O*-Dimethylhydroxylamin-Hydrochlorid zu den Amiden der Formel (79) reagieren. Der erste Teilschritt der Reaktion erfolgt bevorzugt in Dichlormethan bei RT. Die Amidbildung des zweiten Teilschritts erfolgt bei RT in einem etherischen Lösungsmittel, wie bevorzugt Tetrahydrofuran (THF), in Gegenwart einer üblichen tertiären Aminbase, wie bevorzugt *N,N*-Diisopropylethylamin oder Triethylamin. Die Überführung in die Methylketone der Formel (70) wird dann durch Umsetzung mit einer Methyl-Metallverbindung erreicht. Bevorzugt wird hierfür Methylmagnesiumchlorid oder -bromid eingesetzt. Die Reaktion erfolgt in inerten Lösungsmitteln, wie bevorzugt Tetrahydrofuran (THF), bei tiefer Temperatur, bevorzugt im Temperaturbereich zwischen -78°C und 0°C.

Alkyl-substituierte 5-Aminothiophen-2,4-dicarbonsäureester wie die Verbindungen der Formeln (37) und (43) [Schemata 14-16, mit $R^2$ = Methyl oder Ethyl] lassen sich nach einem bekannten Verfahren über die 3-Komponenten-Reaktion eines Acetessigsäure- bzw. β-Ketovaleriansäureesters mit einem α-Cyanoessigsäureester und elementarem Schwefel erhalten ["Gewald-Reaktion"; siehe z.B. B. P. McKibben et al., Tetrahedron Lett. 40, 5471-5474 (1999) und dort zitierte weitere Literatur].

Thienouracil-Intermediate der Formel (1), in welcher $R^2$ für Difluormethyl oder Trifluormethyl steht, sind in vorteilhafter Weise auch nach dem in Schema 20 dargestellten Verfahren über die Betain-Verbindung (62) zugänglich:

Schema 20

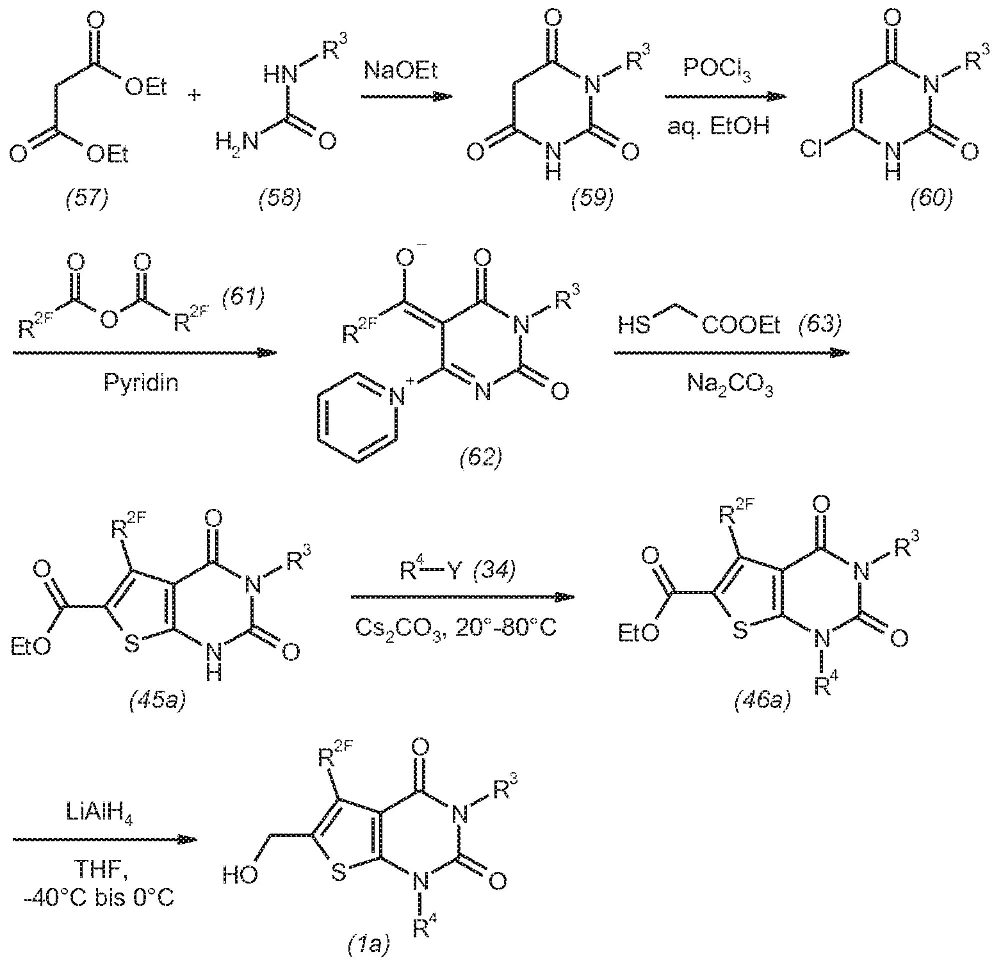


(57) (58) (59) (60) (61) (62) (63) (45a) (34) (46a) (1a)

[$R^{2F}$ = $CHF_2$ oder $CF_3$; Y = Cl, Br, I oder OTs].

Das Verfahren beginnt mit einer baseninduzierten Kondensation des Malonsäureesters (57) mit dem Harnstoff-Derivat (58) zum Barbitursäure-Derivat der Formel (59). Die Kondensation erfolgt üblicherweise mit Hilfe eines Alkalialkoholats, wie Natrium- oder Kaliummethanolat, Natrium- oder Kaliumethanolat oder Natrium- oder Kalium-*tert*.-butylat, in dem betreffenden Alkohol als Lösungsmittel oder mit Hilfe eines Alkalihydrids, wie Natrium- oder Kaliumhydrid, in Tetrahydrofuran oder *N,N*-Dimethylformamid als inertem Lösungsmittel; bevorzugt wird Natriumethanolat in Ethanol verwendet. Die Reaktion wird im Allgemeinen in einem Temperaturbereich von +20°C bis +100°C durchgeführt. Die Umwandlung der Verbindung (59) in das 6-Chlorpyrimidindion (60) erfolgt durch Behandlung mit überschüssigem Phosphoroxychlorid in einem wässrigen Alkohol, wie Methanol oder Ethanol, als Lösungsmittel in einem Temperaturbereich von 0°C bis +100°C. Die nachfolgende Überführung in das Pyridinium-Enolat-Betain (62) wird in Anlehnung an eine in der Literatur beschriebene Methode zur Synthese von 3-substituierten Chromon-Derivaten [I. Yokoe et al., Chem. Pharm. Bull. 42 (8), 1697-1699 (1994)] durchgeführt, indem das 6-Chlorpyrimidindion (60) in Gegenwart eines größeren Überschusses von Pyridin (ca. zehnfach) mit dem Anhydrid (61) umgesetzt wird. Die Reaktion erfolgt in der Regel in einem Temperaturbereich von 0°C bis +40°C, und als inertes Lösungsmittel wird vorzugsweise Acetonitril verwendet. Für die nachfolgende Kondensation des Betains (62) mit dem Mercaptoessigsäureester (63) zu den Thienouracilen der Formel (45a) eignen sich als Base insbesondere Alkalicarbonate wie Lithium-, Natrium-, Kalium- oder Cäsiumcarbonat, Alkalialkoholate wie Natrium- oder Kaliummethanolat, Natrium- oder Kaliumethanolat oder Natrium- oder Kalium-*tert*.-butylat, oder Alkalihydride wie Natrium- oder Kaliumhydrid; bevorzugt wird Natrium- oder Kaliumcarbonat verwendet [vgl. auch K. Hirota et al., J. Heterocycl. Chem. 27 (3), 717-721 (1990)]. Die Umsetzung wird vorzugsweise in einem alkoholischen Lösungsmittel, wie Methanol, Ethanol, Isopropanol oder *tert*.-Butanol, oder in einem inerten polar-aprotischen Lösungsmittel, wie *N,N*-Dimethylformamid (DMF), *N*-Methylpyrrolidinon (NMP) oder *N,N'*-Dimethylpropylenharnstoff (DMPU), in einem Temperaturbereich von +20°C bis +150°C durchgeführt, wobei sich eine Reaktionsführung unter Mikrowellenbestrahlung als vorteilhaft erwiesen hat. Bevorzugt wird Ethanol als Lösungsmittel verwendet.

Die Alkylierung der Verbindungen der Formel (45a) mit den Verbindungen der Formel (34) erfolgt analog zu den in Schema 13 bereits beschriebenen Alkylierungsreaktionen und liefert die Ethylester der Formel (46a), die in einer abschließenden Reduktion mit einem komplexen Metallhydrid, wie zum Beispiel Lithiumaluminiumhydrid, in die Alkohole der Formel (1a) überführt werden. Die Reduktion wird üblicherweise in einem etherischen Lösungsmittel, wie beispielsweise Tetrahydrofuran (THF), durchgeführt und erfolgt im Allgemeinen in einem Temperaturbereich von -40°C bis 0°C.

Thienouracil-Intermediate der Formel (1), in welcher $R^2$ für Wasserstoff steht, können auch nach dem in Schema 21 dargestellten Verfahren erhalten werden:

Schema 21

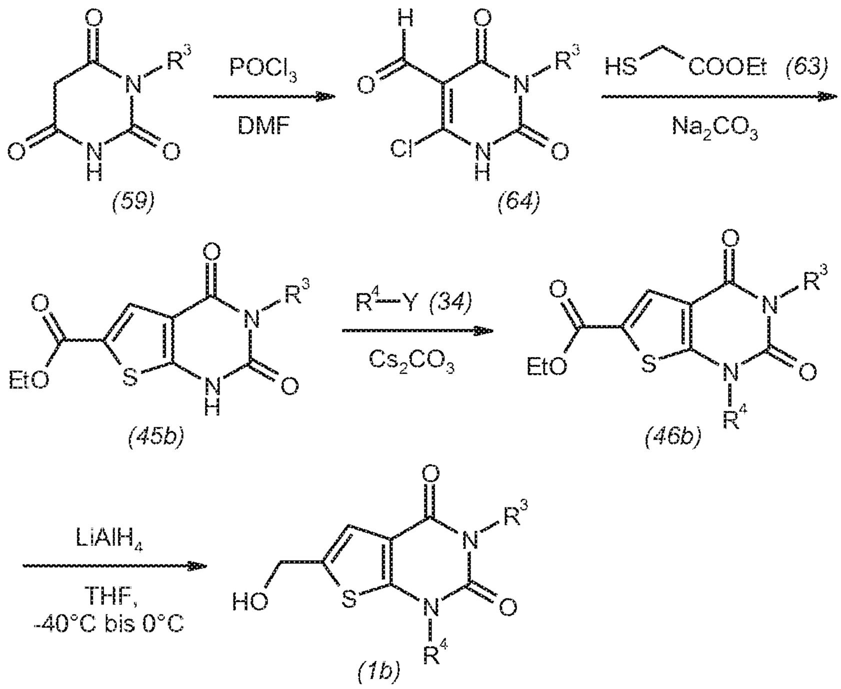


[Y = Cl, Br, I oder OTs].

In diesem Verfahren wird das Barbitursäure-Derivat der Formel (59) (siehe Schema 20) zunächst mit einer Mischung aus Phosphoroxychlorid und *N,N*-Dimethylformamid in eine Verbindung der Formel (64) überführt und letztere dann in Gegenwart einer Base mit dem Mercaptoessigsäureester (63) zum Thienouracil der Formel (45b) kondensiert. Die Umwandlung der Verbindung (59) in das 6-Chlor-5-formylpyrimidindion (64) erfolgt über eine regioselektive Vilsmeier-Haack-Reaktion durch Behandlung mit einer vorgebildeten Mischung aus Phosphoroxychlorid und *N,N*-Dimethylformamid, welche im großen Überschuss eingesetzt wird und gleichzeitig auch als Lösungsmittel dient [vgl. z.B. K. Tanaka et al., Chem. Pharm. Bull. 35 (4), 1397-1404 (1987)]. Die Umsetzung erfolgt im Allgemeinen in einem Temperaturbereich von +20°C bis +120°C. Die nachfolgenden Reaktionen - die Kondensation zu den Thienouracilen der Formel (45b), die Alkylierung zu den Verbindungen der Formel (46b) und die Reduktion zu den Alkoholen der Formel (1b) - werden analog zu den Bedingungen, wie sie in Schema 20 bereits beschrieben sind, durchgeführt.

Die Verbindungen der zuvor aufgeführten Formeln (2), (7), (9), (11), (12), (13), (14), (15), (16), (17), (18), (23), (27), (29), (30), (31), (34), (47), (52), (55), (57), (58), (61), (63), (65), (68), (74) und (76) sind entweder kommerziell erhältlich oder als solche in der Literatur beschrieben, oder sie können, ausgehend von anderen kommerziell erhältlichen Verbindungen, nach dem Fachmann geläufigen, literaturbekannten Methoden hergestellt werden. Zahlreiche detaillierte Vorschriften und weitere Literaturangaben befinden sich auch im Experimentellen Teil im Abschnitt zur Herstellung der Ausgangsverbindungen und Intermediate.

Die erfindungsgemäßen Verbindungen besitzen wertvolle pharmakologische Eigenschaften und können zur Vorbeugung und Behandlung von Erkrankungen bei Menschen und Tieren verwendet werden.

Die erfindungsgemäßen Verbindungen stellen potente und selektive Antagonisten des Adenosin-A2b-Rezeptors dar und eignen sich daher zur Behandlung und/oder Prävention von Erkrankungen und pathologischen Prozessen, insbesondere solcher, bei denen im Zuge eines Entzündungsgeschehens und/oder eines Gewebe- oder Gefäßumbaus der A2b-Rezeptor involviert ist.

Dazu zählen im Sinne der vorliegenden Erfindung insbesondere Erkrankungen wie die Gruppe der interstitiellen idiopathischen Pneumonien, zu denen die idiopathische pulmonale Fibrose (IPF), die akute interstitielle Pneumonie, nicht-spezifische interstitielle Pneumonien, lymphoide interstitielle Pneumonien, respiratorische Bronchiolitis mit interstitieller Lungenerkrankung, kryptogene organisierende Pneumonien, desquamative interstitielle Pneumonien und nicht-klassifizierbare idiopathische interstitielle Pneumonien gehören, ferner granulomatöse interstitielle Lungenerkrankungen, interstitielle Lungenerkrankungen bekannter Ursache und andere interstitielle Lungenerkrankungen unbekannter Ursache, die pulmonale arterielle Hypertonie (PAH) und andere Formen der pulmonalen Hypertonie (PH), das Bronchiolitis obliterans-Syndrom (BOS), die chronisch-obstruktive Lungenerkrankung (COPD), das akute Atemwegssyndrom (ARDS), akute Lungenschädigung (ALI), alpha-1-Antitrypsin-Defizienz (AATD), Lungenemphysem (z.B. durch Zigarettenrauch induziertes Lungenemphysem), zystische Fibrose (CF), entzündliche und fibrotische Erkrankungen der Niere, chronische Darmentzündungen (IBD, Morbus Crohn, Colitis ulcerosa), Peritonitis, Peritonealfibrose, rheumatoide Erkrankungen, multiple Sklerose, entzündliche und fibrotische Hauterkrankungen, Sichelzellanämie sowie entzündliche und fibrotische Augenerkrankungen.

Die erfindungsgemäßen Verbindungen können weiterhin verwendet werden zur Behandlung und/ oder Prävention von asthmatischen Erkrankungen unterschiedlicher Schweregrade mit intermittierendem oder persistierendem Verlauf (refraktives Asthma, bronchiales Asthma, allergisches Asthma, intrinsisches Asthma, extrinsisches Asthma, durch Medikamente oder durch Staub induziertes Asthma), von verschiedenen Formen der Bronchitis (chronische Bronchitis, infektiöse Bronchitis, eosinophile Bronchitis), von Bronchiektasien, Pneumonie, Farmerlunge und verwandten Krankheiten, Husten- und Erkältungskrankheiten (chronischer entzündlicher Husten, iatrogener Husten), Nasenschleimhautentzündungen (einschließlich medikamentöse Rhinitis, vasomotorische Rhinitis und jahreszeitabhängige, allergische Rhinitis, z.B. Heuschnupfen) und von Polypen.

Die erfindungsgemäßen Verbindungen können darüber hinaus zur Behandlung und/oder Prävention von kardiovaskulären Erkrankungen eingesetzt werden, wie beispielsweise Bluthochdruck (Hypertonie), Herzinsuffizienz, koronare Herzerkrankung, stabile und instabile Angina pectoris, renale Hypertonie, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden des Grades I-III, supraventrikuläre Tachyarrhythmie, Vorhofflimmern, Vorhofflattern, Kammerflimmern, Kammerflattern, ventrikuläre Tachyarrhythmie, Torsade de pointes-Tachykardie, Extrasystolen des Vorhofs und des Ventrikels, AV-junktionale Extrasystolen, Sick-Sinus-Syndrom, Synkopen, AV-Knoten-Reentry-Tachykardie, Wolff-Parkinson-White-Syndrom, akutes Koronarsyndrom (ACS), autoimmune Herzerkrankungen (Perikarditis, Endokarditis, Valvolitis, Aortitis, Kardiomyopathien), Boxerkardiomyopathie, Aneurysmen, Schock wie kardiogener Schock, septischer Schock und anaphylaktischer Schock, ferner zur Behandlung und/oder Prävention von thromboembolischen Erkrankungen und Ischämien, wie myokardiale Ischämie, Myokardinfarkt, Hirnschlag, Herzhypertrophie, transistorische und ischämische Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, periphere Durchblutungsstörungen, Reperfusionsschäden, arterielle und venöse Thrombosen, Mikroalbuminurie, Herzmuskelschwäche, endotheliale Dysfunktion, mikro- und makrovaskuläre Schädigungen (Vaskulitis), sowie zur Verhinderung von Restenosen beispielsweise nach Thrombolyse-Therapien, percutan-transluminalen Angioplastien (PTA), percutan-transluminalen Koronarangioplastien (PTCA), Herztransplantationen und Bypass-Operationen.

Im Sinne der vorliegenden Erfindung umfasst der Begriff Herzinsuffizienz sowohl akute als auch chronische Erscheinungsformen der Herzinsuffizienz wie auch spezifische oder verwandte Krankheitsformen hiervon, wie akute dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen sowie diastolische und systolische Herzinsuffizienz.

Die erfindungsgemäßen Verbindungen eignen sich außerdem zur Behandlung und/oder Prävention von Nierenerkrankungen, insbesondere von Niereninsuffizienz und Nierenversagen. Im Sinne der vorliegenden Erfindung umfassen die Begriffe Niereninsuffizienz und Nierenversagen sowohl akute als auch chronische Erscheinungsformen hiervon wie auch diesen zugrundeliegende oder verwandte Nierenerkrankungen, wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantat-Abstoßung und Immunkomplex-induzierte Nierenerkrankungen, durch toxische Substanzen induzierte Nephropathie, Kontrastmittel-induzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder

Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prävention von Folgeerscheinungen einer Niereninsuffizienz, wie beispielsweise Hypertonie, Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Darüber hinaus sind die erfindungsgemäßen Verbindungen zur Behandlung und/oder Prävention von Erkrankungen des Urogenitalsystems geeignet, wie beispielsweise benignes Prostata-Syndrom (BPS), benigne Prostatahyperplasie (BPH), benigne Prostatavergrößerung (BPE), Blasenentleerungsstörungen (BOO), untere Harnwegssyndrome (LUTS), neurogene überaktive Blase (OAB), Inkontinenz wie beispielsweise Misch-, Drang-, Stress- oder Überlauf-Inkontinenz (MUI, UUI, SUI, OUI), Beckenschmerzen sowie erektile Dysfunktion und weibliche sexuelle Dysfunktion.

Zudem besitzen die erfindungsgemäßen Verbindungen anti-inflammatorische Wirkung und können daher als entzündungshemmende Mittel zur Behandlung und/oder Prävention von Sepsis (SIRS), multiplem Organversagen (MODS, MOF), entzündlichen Erkrankungen der Niere, chronischen Darmentzündungen (IBD, Morbus Crohn, Colitis ulcerosa), Pankreatitis, Peritonitis, Cystitis, Urethritis, Prostatitis, Epidimytitis, Oophoritis, Salpingitis, Vulvovaginitis, rheumatoiden Erkrankungen, entzündlichen Erkrankungen des Zentralnervensystems, multipler Sklerose, entzündlichen Hauterkrankungen und entzündlichen Augenerkrankungen eingesetzt werden.

Die erfindungsgemäßen Verbindungen sind ferner zur Behandlung und/oder Prävention von fibrotischen Erkrankungen der inneren Organe, wie beispielsweise der Lunge, des Herzens, der Niere, des Knochenmarks und insbesondere der Leber, sowie von dermatologischen Fibrosen und fibrotischen Erkrankungen des Auges geeignet. Im Sinne der vorliegenden Erfindung umfasst der Begriff fibrotische Erkrankungen insbesondere solche Erkrankungen wie Leberfibrose, Leberzirrhose, Lungenfibrose, Endomyokardfibrose, Nephropathie, Glomerulonephritis, interstitielle Nierenfibrose, fibrotische Schäden in Folge von Diabetes, Knochenmarksfibrose, Peritonealfibrose und ähnliche fibrotische Erkrankungen, Sklerodermie, Morphaea, Keloide, hypertrophe Narbenbildung, Naevi, diabetische Retinopathie, proliferative Vitroretinopathie und Erkrankungen des Bindegewebes (z.B. Sarkoidose). Die erfindungsgemäßen Verbindungen können ebenso verwendet werden zur Förderung der Wundheilung, zur Bekämpfung postoperativer Narbenbildung, z.B. nach Glaukom-Operationen, und zu kosmetischen Zwecken bei alternder oder verhornender Haut.

Auch können die erfindungsgemäßen Verbindungen zur Behandlung und/oder Prävention von Anämien verwendet werden, wie hämolytischen Anämien, insbesondere Hämoglobinopathien wie Sichelzellanämie und Thalassämien, megaloblastären Anämien, Eisenmangel-Anämien, Anämien durch akuten Blutverlust, Verdrängungsanämien und aplastischen Anämien.

Die erfindungsgemäßen Verbindungen sind zudem zur Behandlung von Krebserkrankungen geeignet, wie beispielsweise von Hautkrebs, Hirntumoren, Kopf- und Halstumoren, Speiseröhrenkrebs, Brustkrebs, Knochenmarktumoren, Leukämien, Liposarcomen, Karzinomen des Magen-DarmTraktes, der Leber, Bauchspeicheldrüse, Lunge, Niere, Harnleiter, Prostata und des Genitaltraktes, Blasenkrebs sowie von bösartigen Tumoren des lymphoproliferativen Systems, wie z.B. Hodgkin's und Non-Hodgkin's Lymphom.

Darüber hinaus können die erfindungsgemäßen Verbindungen eingesetzt werden zur Behandlung und/oder Prävention von Arteriosklerose, Lipidstoffwechselstörungen und Dyslipidämien (Hypolipoproteinämie, Hypertriglyceridämie, Hyperlipidämie, kombinierte Hyperlipidämien, Hypercholesterolämie, Abetalipoproteinämie, Sitosterolämie), Xanthomatose, Tangier-Krankheit, Fettsucht (Adipositas), Fettleibigkeit (Obesitas), metabolischen Erkrankungen (Metabolisches Syndrom, Hyperglykämie, Insulin-abhängiger Diabetes, nicht-Insulin-abhängiger Diabetes, Gestationsdiabetes, Hyperinsulinämie, Insulinresistenz, Glukose-Intoleranz und diabetische Spätfolgen wie Retinopathie, Nephropathie und Neuropathie), von Erkrankungen des Gastrointestinaltrakts und des Abdomen (Glossitis, Gingivitis, Periodontitis, Oesophagitis, eosinophile Gastroenteritis, Mastocytose, Morbus Crohn, Colitis, Proctitis, Pruritis ani, Diarrhöe, Zöliakie, Hepatitis, Leberfibrose, Leberzirrhose, Pankreatitis und Cholecystitis), von Erkrankungen des Zentralen Nervensystems und von neurodegenerativen Störungen (Schlaganfall, Alzheimer'sche Krankheit, Parkinson'sche Krankheit, Demenz, Epilepsie, Depressionen, Multiple Sklerose), Immunerkrankungen, Schilddrüsenerkrankungen (Hyperthyreose), Hauterkrankungen (Psoriasis, Akne, Ekzeme, Neurodermitis, vielfältige Formen der Dermatitis wie z.B. Dermatitis abacribus, Dermatitis actinica, Dermatitis allergica, Dermatitis ammoniacalis, Dermatitis artefacta, Dermatitis autogenica, Dermatitis atrophicans, Dermatitis calorica, Dermatitis combustionis, Dermatitis congelationis, Dermatitis cosmetica, Dermatitis escharotica, Dermatitis exfoliativa, Dermatitis gangraenose, Dermatitis haemostatica, Dermatitis herpetiformis, Dermatitis lichenoides, Dermatitis linearis, Dermatitis maligna, Dermatitis medimencatosa, Dermatitis palmaris et plantaris, Dermatitis parasitaria, Dermatitis photoallergica, Dermatitis phototoxica, Dermatitis pustularis, Dermatitis seborrhoica, Dermatitis solaris, Dermatitis toxica, Dermatitis ulcerosa, Dermatitis veneata, infektiöse Dermatitis, pyogene Dermatitis und Rosazea-artige Dermatitis, sowie Keratitis, Bullosis, Vasculitis, Cellulitis, Panniculitis, Lupus erythematodes, Erythema, Lymphome, Hautkrebs, Sweet-Syndrom, Weber-Christian-Syndrom, Narbenbildung, Warzenbildung, Frostbeulen), von entzündlichen Augenerkrankungen (Saccoidosis, Blepharitis, Conjunctivitis, Iritis, Uveitis, Chorioiditis, Ophthalmitis), viralen Erkrankungen (durch Influenza-, Adeno- und Coronaviren, wie z.B. HPV, HCMV, HIV, SARS), von Erkrankungen des Skelettknochens und der Gelenke sowie der Skelettmuskel (vielfältige Formen der Arthritis wie z.B. Arthritis alcaptonurica, Arthritis ankylosans, Arthritis dysenterica, Arthritis exsudativa, Arthritis fungosa, Arthritis gonorrhoica, Arthritis mutilans, Arthritis psoriatica, Arthritis purulenta, Arthritis rheumatica, Arthritis serosa, Arthritis syphilitica, Arthritis tuberculosa, Arthritis urica, Arthritis villonodularis pigmentosa, atypische Arthritis, hämophile Arthritis, juvenile chronische Arthritis, rheumatoide Arthritis und metastatische Arthritis, des weiteren das Still-Syndrom, Felty-Syndrom, Sjörgen-Syndrom, Clutton-Syndrom, Poncet-Syndrom, Pott-Syndrom und Reiter-Syndrom, vielfältige Formen der Arthropathien wie z.B. Arthropathie deformans, Arthropathie neuropathica, Arthropathie ovaripriva, Arthropathie psoriatica und Arthropathie tabica, systemische Sklerosen, vielfältige Formen der entzündlichen Myopathien wie z.B. Myopathie epidemica, Myopathie fibrosa, Myopathie myoglobinurica, Myopathie ossificans, Myopathie ossificans neurotica, Myopathie ossificans progressiva multiplex, Myopathie purulenta, Myopathie rheumatica, Myopathie trichinosa, Myopathie tropica und Myopathie typhosa, sowie das Günther-Syndrom und das Münchmeyer-Syndrom), von entzündlichen Arterienveränderungen (vielfältige Formen der Arteritis wie z.B. Endarteritis, Mesarteritis, Periarteritis, Panarteritis, Arteritis rheumatica, Arteritis deformans, Arteritis temporalis, Arteritis cranialis, Arteritis gigantocellularis und Arteritis granulomatosa, sowie das Horton-Syndrom, Churg-Strauss-Syndrom und die Takayasu-Arteritis), des Muckle-Well-Syndroms, der Kikuchi-Krankheit, von Polychondritis, Sklerodermia sowie von weiteren Erkrankungen mit einer entzündlichen oder immunologischen Komponente, wie beispielsweise Katarakt, Kachexie, Osteoporose, Gicht, Inkontinenz, Lepra, Sezary-Syndrom und paraneoplastisches Syndrom, bei Abstossungsreaktionen nach Organtransplantationen und zur Wundheilung und Angiogenese insbesondere bei chronischen Wunden.

Aufgrund ihres Eigenschaftsprofils eignen sich die erfindungsgemäßen Verbindungen insbesondere zur Behandlung und/oder Prävention von interstitiellen Lungenerkrankungen, vor allem der idiopathischen Lungenfibrose (IPF), sowie von pulmonaler Hypertonie (PH), Bronchiolitis obliterans-Syndrom (BOS), chronisch-obstruktiven Lungenerkrankungen (COPD), Asthma, zystischer Fibrose (CF), Myokardinfarkt, Herzinsuffizienz und Hämoglobinopathien, insbesondere Sichelzellanämie, und von Krebserkrankungen.

Die zuvor genannten, gut charakterisierten Krankheiten des Menschen können mit vergleichbarer Ätiologie auch in anderen Säugetieren vorkommen und dort ebenfalls mit den Verbindungen der vorliegenden Erfindung behandelt werden.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Weiterer Gegenstand der vorliegenden Erfindung sind Verbindungen für die die Verwendung der erfindungsgemäßen Verbindungen zur Behandlung und/oder Prävention von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prävention von Erkrankungen, insbesondere der zuvor genannten Erkrankungen.

Weiterer Gegenstand der vorliegenden Erfindung ist ein Arzneimittel, enthaltend mindestens eine der erfindungsgemäßen Verbindungen, zur Behandlung und/oder Prävention von Erkrankungen, insbesondere der zuvor genannten Erkrankungen.

Weiterer Gegenstand der vorliegenden Erfindung sind Verbindungen für die Verwendung der erfindungsgemäßen Verbindungen in einem Verfahren zur Behandlung und/oder Prävention von Erkrankungen, insbesondere der zuvor genannten Erkrankungen.

Weiterer Gegenstand der vorliegenden Erfindung sind Verbindungen für ein Verfahren zur Behandlung und/oder Prävention von Erkrankungen, insbesondere der zuvor genannten Erkrankungen, unter Verwendung einer wirksamen Menge von mindestens einer der erfindungsgemäßen Verbindungen.

Die erfindungsgemäßen Verbindungen können allein oder bei Bedarf in Kombination mit einer oder mehreren anderen pharmakologisch wirksamen Substanzen eingesetzt werden, solange diese Kombination nicht zu unerwünschten und inakzeptablen Nebenwirkungen führt. Weiterer Gegenstand der vorliegenden Erfindung sind daher Arzneimittel, enthaltend mindestens eine der erfindungsgemäßen Verbindungen und einen oder mehrere weitere Wirkstoffe, insbesondere zur Behandlung und/oder Prävention der zuvor genannten Erkrankungen. Als hierfür geeignete Kombinationswirkstoffe seien beispielhaft und vorzugsweise genannt:

- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) und/oder cyclischem Adenosinmonophosphat (cAMP) inhibieren, wie beispielsweise Inhibitoren der Phosphodiesterasen (PDE) 1, 2, 3, 4 und/oder 5, insbesondere PDE 5-Inhibitoren wie Sildenafil, Vardenafil, Tadalafil, Udenafil, Dasantafil, Avanafil, Mirodenafil oder Lodenafil;
- NO- und Häm-unabhängige Aktivatoren der löslichen Guanylatcyclase (sGC), wie insbesondere die in WO 01/19355, WO 01/19776, WO 01/19778, WO 01/19780, WO 02/070462 und WO 02/070510 beschriebenen Verbindungen;
- NO-unabhängige, jedoch Häm-abhängige Stimulatoren der löslichen Guanylatcyclase (sGC), wie insbesondere Riociguat, Nelociguat und Vericiguat sowie die in WO 00/06568, WO 00/ 06569, WO 02/42301, WO 03/095451, WO 2011/147809, WO 2012/004258, WO 2012/028647 und WO 2012/059549 beschriebenen Verbindungen;
- Prostacyclin-Analoga und IP-Rezeptor-Agonisten, wie beispielhaft und vorzugsweise Iloprost, Beraprost, Treprostinil, Epoprostenol oder Selexipag;
- Endothelin-Rezeptor-Antagonisten, wie beispielhaft und vorzugsweise Bosentan, Darusentan, Ambrisentan oder Sitaxsentan;
- Verbindungen, die die humane neutrophile Elastase (HNE) inhibieren, wie beispielhaft und vorzugsweise Sivelestat oder DX-890 (Reltran);
- die Signaltransduktionskaskade inhibierende Verbindungen, beispielhaft und vorzugsweise aus der Gruppe der Kinase-Inhibitoren, insbesondere aus der Gruppe der Tyrosinkinase- und/oder Serin/Threoninkinase-Inhibitoren, wie beispielhaft und vorzugsweise Nintedanib, Dasatinib, Nilotinib, Bosutinib, Regorafenib, Sorafenib, Sunitinib, Cediranib, Axitinib, Telatinib, Imatinib, Brivanib, Pazopanib, Vatalanib, Gefitinib, Erlotinib, Lapatinib, Canertinib, Lestaurtinib, Pelitinib, Semaxanib oder Tandutinib;
- Verbindungen, die den Ab- und Umbau der Extrazellulärmatrix inhibieren, beispielhaft und vorzugsweise Inhibitoren der Matrix-Metalloproteasen (MMPs), insbesondere Inhibitoren von Stromelysin, Kollagenasen, Gelatinasen und Aggrecanasen (hierbei vor allem von MMP-1, MMP-3, MMP-8, MMP-9, MMP-10, MMP-11 und MMP-13) sowie der Metallo-Elastase (MMP-12);
- Verbindungen, die die Bindung von Serotonin an dessen Rezeptor blockieren, beispielhaft und vorzugsweise Antagonisten des 5-$HT_{2B}$-Rezeptors wie PRX-08066;
- Antagonisten von Wachstumsfaktoren, Zytokinen und Chemokinen, beispielhaft und vorzugsweise Antagonisten von TGF-β, CTGF, IL-1, IL-4, IL-5, IL-6, IL-8, IL-13 und Integrinen;
- die Rho-Kinase inhibierende Verbindungen, wie beispielhaft und vorzugsweise Fasudil, Y-27632, SLx-2119, BF-66851, BF-66852, BF-66853, KI-23095 oder BA-1049;
- Verbindungen, die die lösliche Epoxidhydrolase (sEH) inhibieren, wie beispielsweise *N,N'*-Dicyclohexylharnstoff, 12-(3-Adamantan-1-yl-ureido)-dodecansäure oder 1-Adamantan-1-yl-3-{5-[2-(2-ethoxyethoxy)ethoxy]pentyl}-harnstoff;
- den Energiestoffwechsel des Herzens beeinflussende Verbindungen, wie beispielhaft und vorzugsweise Etomoxir, Dichloracetat, Ranolazin oder Trimetazidin;
- anti-obstruktiv wirkende Mittel, wie sie z.B. zur Therapie der chronisch-obstruktiven Lungenerkrankung (COPD) oder eines Asthma bronchiale eingesetzt werden, beispielhaft und vorzugsweise aus der Gruppe der inhalativ oder systemisch angewendeten beta-adrenergen Rezeptor-Agonisten (beta-Mimetika) und der inhalativ angewendeten anti-muscarinergen Substanzen;

- entzündungshemmende, immunmodulierende, immunsuppressive und/oder zytotoxische Mittel, beispielhaft und vorzugsweise aus der Gruppe der systemisch oder inhalativ angewendeten Corticosteroide sowie Acetylcystein, Montelukast, Tipelukast, Azathioprin, Cyclophosphamid, Hydroxycarbamid, Azithromycin, IFN-$\gamma$, Pirfenidon oder Etanercept;

- antifibrotisch wirkende Mittel, wie beispielhaft und vorzugsweise Pirfenidon, Lysophosphatidsäure-Rezeptor 1 (LPA-1)-Antagonisten, Sphingosin-1-phosphat-Rezeptor 3 (S1P3)-Antagonisten, Autotaxin-Inhibitoren, FP-Rezeptor-Antagonisten, Lysyloxidase (LOX)-Inhibitoren, Lysyloxidase-like-2-Inhibitoren, Vasoaktives intestinales Peptid (VIP), VIP-Analoga, $\alpha_v\beta_6$-Integrin-Antagonisten, Interferone, KCa3.1-Blocker, CTGF-Inhibitoren, IL-4-Antagonisten, IL-13-Antagonisten, TGF-$\beta$-Antagonisten, Inhibitoren des WNT-Signalwegs oder CCR2-Antagonisten;

- therapeutische Antikörper sowie Antikörper-Wirkstoff-Konjugate, wie beispielhaft und vorzugsweise Bevacizumab, Cetuximab, Trastuzumab, Trastuzumab Emtansin, Brentuximab Vedotin oder Anetumab Ravtansin;

- immuntherapeutische Antikörper, wie beispielhaft und vorzugsweise Ipilimumab, Nivolumab, Pembrolizumab, Pidilizumab, BMS-935559, MPDL3280A, MEDI4736, MSB0010718C oder AMP-224;

- antithrombotisch wirkende Mittel, beispielhaft und vorzugsweise aus der Gruppe der Thrombozytenaggregationshemmer, der Antikoagulantien und der profibrinolytischen Substanzen;

- den Blutdruck senkende Wirkstoffe, beispielhaft und vorzugsweise aus der Gruppe der Calcium-Antagonisten, Angiotensin AII-Antagonisten, ACE-Hemmer, Vasopeptidase-Inhibitoren, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika;

- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten; und/oder

- Chemotherapeutika, wie sie z.B. zur Therapie von Neubildungen (Neoplasien) der Lunge oder anderer Organe eingesetzt werden.

Bei einer bevorzugten Ausführungsform der Erfindung werden die erfindungsgemäßen Verbindungen in Kombination mit einem beta-adrenergen Rezeptor-Agonisten, wie beispielhaft und vorzugsweise Albuterol, Isoproterenol, Metaproterenol, Terbutalin, Fenoterol, Formoterol, Reproterol, Salbutamol oder Salmeterol, verabreicht.

Bei einer bevorzugten Ausführungsform der Erfindung werden die erfindungsgemäßen Verbindungen in Kombination mit einer anti-muscarinergen Substanz, wie beispielhaft und vorzugsweise Ipratropiumbromid, Tiotropiumbromid oder Oxitropiumbromid, verabreicht.

Bei einer bevorzugten Ausführungsform der Erfindung werden die erfindungsgemäßen Verbindungen in Kombination mit einem Corticosteroid, wie beispielhaft und vorzugsweise Prednison, Prednisolon, Methylprednisolon, Triamcinolon, Dexamethason, Beclomethason, Betamethason, Flunisolid, Budesonid oder Fluticason, verabreicht.

Unter antithrombotisch wirkenden Mittel werden vorzugsweise Verbindungen aus der Gruppe der Thrombozytenaggregationshemmer, der Antikoagulantien und der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Melagatran, Dabigatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban, Apixaban, Fidexaban, Razaxaban, Fondaparinux, Idraparinux, DU-176b, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan, Irbesartan, Olmesartan, Eprosartan oder Azilsartan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton, Eplerenon oder Finerenon, verabreicht.

Bei einer bevorzugten Ausführungsform der Erfindung werden die erfindungsgemäßen Verbindungen in Kombination mit einem Diuretikum, wie beispielhaft und vorzugsweise Furosemid, Bumetanid, Torsemid, Bendroflumethiazid, Chlorthiazid, Hydrochlorthiazid, Hydroflumethiazid, Methyclothiazid, Polythiazid, Trichlormethiazid, Chlorthalidon, Indapamid, Metolazon, Quinethazon, Acetazolamid, Dichlorphenamid, Methazolamid, Glycerin, Isosorbid, Mannitol, Amilorid oder Triamteren, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Torcetrapib (CP-529 414), JJT-705 oder CETP-vaccine (Avant), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Besonders bevorzugt sind Kombinationen der erfindungsgemäßen Verbindungen mit einem oder mehreren weiteren Wirkstoffen ausgewählt aus der Gruppe bestehend aus PDE 5-Inhibitoren, sGC-Aktivatoren, sGC-Stimulatoren, Prostacyclin-Analoga, IP-Rezeptor-Agonisten, Endothelin-Antagonisten, antifibrotisch wirkenden Mitteln, entzündungshemmend, immunmodulierend, immunsuppressiv und/oder zytotoxisch wirkenden Mitteln und die Signaltransduktionskaskade inhibierenden Verbindungen.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. inhalativ, intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer, Dosieraerosole), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale und die parenterale Applikation, insbesondere die orale, die intravenöse und die intrapulmonale (inhalative) Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B.

anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.01 bis 100 mg/kg, vorzugsweise etwa 0.01 bis 20 mg/kg und ganz besonders bevorzugt 0.1 bis 10 mg/kg Körpergewicht. Bei intrapulmonaler Applikation beträgt die Menge im Allgemeinen etwa 0.1 bis 50 mg je Inhalation.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

## A. Beispiele

### Abkürzungen und Akronyme:

| | |
|---|---|
| abs. | absolut |
| Ac | Acetyl |
| aq. | wässrig, wässrige Lösung |
| Boc | *tert.*-Butoxycarbonyl |
| br. | breit (bei NMR-Signal) |
| Bsp. | Beispiel |
| Bu | Butyl |
| c | Konzentration |
| ca. | *circa,* ungefähr |
| cat. | katalytisch |
| CDI | *N,N*-Carbonyldiimidazol |
| CI | chemische Ionisation (bei MS) |
| conc. | konzentriert, konzentrierte Lösung |
| d | Dublett (bei NMR) |
| d | Tag(e) |
| DAD | Diodenarraydetektor (bei HPLC) |
| dba | Dibenzylidenaceton |
| DBU | Diazabicyclo[5.4.0]undec-7-en |
| DC | Dünnschichtchromatographie |
| DCI | direkte chemische Ionisation (bei MS) |
| dd | Dublett von Dublett (bei NMR) |
| DEAD | Diethylazodicarboxylat |
| DIAD | Diisopropylazodicarboxylat |
| DIPEA | *N,N*-Diisopropylethylamin |
| DME | 1,2-Dimethoxyethan |
| DMF | *N,N*-Dimethylformamid |
| DMSO | Dimethylsulfoxid |
| dq | Dublett von Quartett (bei NMR) |
| dt | Dublett von Triplett (bei NMR) |
| $\Delta T$ | Temperaturerhöhung, Erwärmung (eines Reaktionsgemisches) |
| d. Th. | der Theorie (bei chemischer Ausbeute) |
| ee | Enantiomerenüberschuss |
| EI | Elektronenstoß-Ionisation (bei MS) |
| eq. | Äquivalent(e) |
| ESI | Elektrospray-Ionisation (bei MS) |
| Et | Ethyl |
| GC | Gaschromatographie |

| | |
|---|---|
| GC/MS | Gaschromatographie-gekoppelte Massenspektrometrie |
| h | Stunde(n) |
| HPLC | Hochdruck-, Hochleistungsflüssigchromatographie |
| iPr | Isopropyl |
| konz. | konzentriert (bei Lösung) |
| LC | Flüssigchromatographie |
| LC/MS | Flüssigchromatographie-gekoppelte Massenspektrometrie |
| LiHMDS | Lithiumhexamethyldisilazid |
| Lit. | Literatur(stelle) |
| m | Multiplett (bei NMR) |
| Me | Methyl |
| min | Minute(n) |
| MPLC | Mitteldruckflüssigchromatographie (über Kieselgel; auch "flash-Chromatographie" genannt) |
| MS | Massenspektrometrie |
| NBS | *N*-Bromsuccinimid |
| NMM | *N*-Methylmorpholin |
| NMO | *N*-Methylmorpholin-*N*-oxid |
| NMP | *N*-Methyl-2-pyrrolidinon |
| NMR | Kernresonanzspektrometrie |
| Pd/C | Palladium auf Aktivkohle |
| PEG | Polyethylenglykol |
| Ph | Phenyl |
| Pr | Propyl |
| q | Quartett (bei NMR) |
| Q-Phos | 1,2,3,4,5-Pentaphenyl-1'-(di-*tert.*-butylphosphino)ferrocen |
| quant. | quantitativ (bei chemischer Ausbeute) |
| quin | Quintett (bei NMR) |
| $R_f$ | Retentionsindex (bei DC) |
| RP | reverse phase (Umkehrphase, bei HPLC) |
| RT | Raumtemperatur |
| $R_t$ | Retentionszeit (bei HPLC, LC/MS) |
| s | Singulett (bei NMR) |
| sept | Septett (bei NMR) |
| sext | Sextett (bei NMR) |
| SFC | superkritische Flüssigchromatographie |
| t | Triplett (bei NMR) |
| TBME | *tert.*-Butyl-methylether |
| tBu | *tert.*-Butyl |
| TFA | Trifluoressigsäure |
| THF | Tetrabydrofuran |
| TMS | Tetramethylsilan |
| Ts | *para*-Toluolsulfonyl |
| UV | Ultraviolett-Spektrometrie |
| v/v | Volumen zu Volumen-Verhältnis (einer Lösung) |
| zus. | zusammen |

**HPLC-, LC/MS- und GC/MS-Methoden:**

Methode 1 (LC/MS):

Instrument: Waters Acquity SQD UPLC System; Säule: Waters Acquity UPLC HSS T3 1.8 μm, 50 mm x 1 mm; Eluent A: 1l Wasser + 0.25 ml 99%-ige Ameisensäure, Eluent B: 1l Acetonitril + 0.25 ml 99%-ige Ameisensäure; Gradient: 0.0 min 90% A → 1.2 min 5% A → 2.0 min 5% A; Temperatur: 50°C; Fluss: 0.40 ml/min; UV-Detektion: 210-400 nm.

Methode 2 (LC/MS):

Instrument: Waters Acquity SQD UPLC System; Säule: Waters Acquity UPLC HSS T3 1.8 μm, 50 mm x 1 mm;

Eluent A: 1l Wasser + 0.25 ml 99%-ige Ameisensäure, Eluent B: 1l Acetonitril + 0.25 ml 99%-ige Ameisensäure; Gradient: 0.0 min 95% A → 6.0 min 5% A → 7.5 min 5% A; Temperatur: 50°C; Fluss: 0.35 ml/min; UV-Detektion: 210-400 nm.

Methode 3 (LC/MS):

Instrument: Waters Acquity UPLC-MS SingleQuad; Säule: Waters Acquity UPLC BEH C18 1.7 μm, 50 mm x 2.1 mm; Eluent A: Wasser + 0.1 Vol.-% Ameisensäure (99%), Eluent B: Acetonitril; Gradient: 0.0-1.6 min 1-99% B, 1.6-2.0 min 99% B; Fluss: 0.8 ml/min; Temperatur: 60°C; DAD-Scan: 210-400 nm.

Methode 4 (LC/MS):

Instrument: Micromass Quattro Premier mit Waters UPLC Acquity; Säule: Thermo Hypersil GOLD 1.9 μm, 50 mm x 1 mm; Eluent A: 1l Wasser + 0.5 ml 50%-ige Ameisensäure, Eluent B: 1l Acetonitril + 0.5 ml 50%-ige Ameisensäure; Gradient: 0.0 min 97% A → 0.5 min 97% A → 3.2 min 5% A → 4.0 min 5% A; Temperatur: 50°C; Fluss: 0.30 ml/min; UV-Detektion: 210 nm.

Methode 5 (LC/MS):

Instrument: Agilent MS Quad 6150 mit HPLC Agilent 1290; Säule: Waters Acquity UPLC HSS T3 1.8 μm, 50 mm x 2.1 mm; Eluent A: 1l Wasser + 0.25 ml 99%-ige Ameisensäure, Eluent B: 1l Acetonitril + 0.25 ml 99%-ige Ameisensäure; Gradient: 0.0 min 90% A → 0.3 min 90% A → 1.7 min 5% A → 3.0 min 5% A; Fluss: 1.20 ml/min; Temperatur: 50°C; UV-Detektion: 205-305 nm.

Methode 6 (LC/MS):

Instrument: Waters Micromass Quattro Micro mit HPLC Waters UPLC Acquity; Säule: Waters BEH C18 1.7 μm, 50 mm x 2.1 mm; Eluent A: 1l Wasser + 0.01 mol Ammoniumformiat, Eluent B: 1l Acetonitril; Gradient: 0.0 min 95% A → 0.1 min 95% A → 2.0 min 15% A → 2.5 min 15% A → 2.51 min 10% A → 3.0 min 10% A; Fluss: 0.5 ml/min; Temperatur: 40°C; UV-Detektion: 210 nm.

Methode 7 (GC/MS):

Instrument: Thermo DFS, Trace GC Ultra; Säule: Restek RTX-35, 15 m x 200 μm x 0.33 μm; konstanter Fluss mit Helium: 1.20 ml/min; Ofen: 60°C; Einlass: 220°C; Gradient: 60°C, 30°C/min → 300°C (3.33 min halten).

Methode 8 (präparative HPLC):

Säule: Chromatorex C18, 10 μm, 125 mm x 30 mm; Eluent: Acetonitril / Wasser mit 0.1% Ameisensäure; Gradient: 20:80 → 95:5 innerhalb von 20 min.

Methode 9 (präparative HPLC):

Säule: Chromatorex C18, 10 μm, 125 mm x 30 mm; Eluent: Acetonitril / Wasser mit 0.1% Ameisensäure; Gradient: 30:70 → 95:5 innerhalb von 20 min.

Methode 10 (präparative HPLC):

Säule: Chromatorex C18, 10 μm, 125 mm x 30 mm; Eluent A: Wasser + 0.05% Trifluoressigsäure, Eluent B: Acetonitril + 0.05% Trifluoressigsäure; Gradient: 0.0 min 75% A → 5.0 min 75% A → 7.0 min 60% A → 16 min 45% A → 18 min 45% A.

Methode 11 (präparative HPLC):

Säule: Chromatorex C18, 10 μm, 125 mm x 30 mm; Eluent: Acetonitril / Wasser mit 0.1% Ameisensäure; Gradient: 15:85 → 95:5 innerhalb von 20 min.

Methode 12 (präparative HPLC):

Säule: Reprosil-Pur C18, 10 µm, 125 mm x 30 mm; Eluent: Acetonitril / Wasser mit 0.05% Trifluoressigsäure; Gradient: 10:90 → 100:0 innerhalb von 12 min.

Methode 13 (präparative HPLC):

Säule: Reprosil-Pur C18, 10 µm, 125 mm x 30 mm; Eluent: Acetonitril / Wasser mit 0.05% Trifluoressigsäure; Gradient: 30:70 → 100:0 innerhalb von 18 min.

Methode 14 (präparative HPLC):

Säule: Chromatorex C18, 10 µm, 125 mm x 30 mm; Eluent: Acetonitril / Wasser mit 0.1% Ameisensäure; Gradient: 10:90 → 100:0 innerhalb von 10 min.

Methode 15 (präparative HPLC):

Säule: Chromatorex C18, 10 µm, 290 mm x 100 mm; Eluent A: Acetonitril, Eluent B: Wasser; Gradient: 0-5 min 10% A, 5-30 min 10% → 55% A, 30-32 min 55% A, 32-35.4 min 90% A.

Methode 16 (präparative HPLC):

Instrument: Agilent 1260; Säule: Phenomenex Luna 5 µm C18, 100 mm x 21.2 mm; Eluent A: Wasser + 0.1% Ameisensäure, Eluent B: Acetonitril; Gradient: 0.0 min 20% B → 1 min 20% B → 10 min 80% B → 12 min 95% B → 18 min 95% B → 18.1 min 20% B → 20 min 20% B; Fluss: 25 ml/min; UV-Detektion: 210 nm.

Methode 17 (LC/MS):

Instrument MS: Thermo Scientific FT-MS; Gerät UHPLC: Thermo Scientific UltiMate 3000; Säule: Waters HSST3 C18 1.8 µm, 75 mm x 2.1 mm; Eluent A: 1l Wasser + 0.01% Ameisensäure, Eluent B: 1l Acetonitril + 0.01% Ameisensäure; Gradient: 0.0 min 10% B → 2.5 min 95% B → 3.5 min 95% B; Temperatur: 50°C; Fluss: 0.90 ml/min; UV-Detektion: 210-300 nm.

Methode 18 (präparative HPLC):

Säule: Chromatorex C18, 10 µm, 250 mm x 30 mm; Eluent: Acetonitril / Wasser mit 0.1% Trifluoressigsäure; Gradient: 10:90 → 95:5 innerhalb von 30 min.

Weitere Angaben:

Die nachfolgenden Beschreibungen der Kopplungsmuster von $^1$H-NMR-Signalen orientieren sich an dem optischen Erscheinungsbild der betreffenden Signale und entsprechen nicht notwendigerweise einer strengen, physikalisch korrekten Interpretation. In der Regel bezieht sich die Angabe zur chemischen Verschiebung auf das Zentrum des betreffenden Signals; bei breiten Multipletts erfolgt in der Regel die Angabe eines Intervalls.

Schmelzpunkte und Schmelzbereiche, soweit angegeben, sind nicht korrigiert.

In den Fällen, in denen Reaktionsprodukte durch Ausrühren, Verrühren oder Umkristallisieren gewonnen wurden, war es oft möglich, weitere Produktmengen aus der jeweiligen Mutterlauge durch Chromatographie zu isolieren. Auf die Beschreibung dieser Chromatographie wird im Folgenden jedoch verzichtet, es denn, ein großer Teil der Gesamtausbeute konnte erst in diesem Schritt isoliert werden.

Für alle Reaktanden oder Reagenzien, deren Herstellung im Folgenden nicht explizit beschrieben ist, gilt, dass sie von allgemein zugänglichen Quellen kommerziell bezogen wurden. Für alle übrigen Reaktanden oder Reagenzien, deren Herstellung im Folgenden ebenfalls nicht beschrieben ist, und die nicht kommerziell erhältlich waren, oder von Quellen bezogen wurden, die nicht allgemein zugänglich sind, ist ein Verweis auf die veröffentlichte Literatur angegeben, in der ihre Herstellung beschrieben ist.

Wenn bei den im Folgenden beschriebenen Synthese-Intermediaten und Ausführungsbeispielen der Erfindung eine Verbindung in der Form eines Salzes der korrespondierenden Base bzw. Säure aufgeführt ist, so ist die exakte stöchiometrische Zusammensetzung eines solchen Salzes, wie es nach dem jeweiligen Herstell- und/oder Reinigungsverfahren erhalten wurde, in der Regel nicht bekannt. Sofern nicht genauer spezifiziert, sind daher Namens- und Strukturformel-Zusätze wie beispielsweise "Hydrochlorid", "Formiat", "Acetat", "Trifluoracetat", "Natrium-Salz" bzw. "x HCl", "x HCOOH", "x $CH_3COOH$", "x $CF_3COOH$", "x $Na^+$" bei solchen Salzen nicht stöchiometrisch zu verstehen, sondern haben allein deskriptiven Charakter bezüglich der enthaltenen salzbildenden Komponenten.

**Ausgangsverbindungen und Intermediate:**

**Beispiel 1A**

2-*tert.*-Butyl-4-ethyl-5-amino-3-methylthiophen-2,4-dicarboxylat

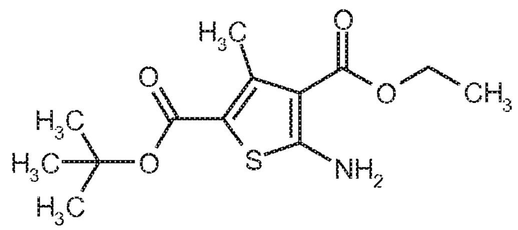

10.0 g (63.2 mmol) Acetessigsäure-*tert.*-butylester, 7.15 g (63.2 mmol) Cyanessigsäureethylester und 2.23 g (69.5 mmol) Schwefel wurden in 15 ml Ethanol vorgelegt und auf 45°C erwärmt. Zu diesem Gemisch wurden 7.5 ml (72.7 mmol) Diethylamin hinzugetropft. Anschließend wurde das Reaktionsgemisch 8 h bei 65°C gerührt. Danach wurden alle flüchtigen Bestandteile am Rotationsverdampfer entfernt. Der verbliebene Rückstand wurde mit ca. 500 ml Wasser versetzt und dreimal mit je ca. 200 ml Ethylacetat extrahiert. Die vereinigten organischen Extrakte wurden mit ca. 200 ml gesättigter Kochsalz-Lösung gewaschen und über wasserfreiem Magnesiumsulfat getrocknet. Nach Filtration wurde zur Trockene eingedampft. Das erhaltene Rohprodukt wurde mittels MPLC gereinigt (ca. 300 g Kieselgel, Cyclohexan/Ethylacetat 10:1). Nach Vereinigen der Produktfraktionen, Eindampfen und Trocknen des Rückstands im Hochvakuum wurden 9.72 g (52% d. Th.) der Titelverbindung erhalten.
$^1$H-NMR (400 MHz, $CDCl_3$, $\delta$/ppm): 6.44 (br. s, 2H), 4.31 (q, 2H), 2.66 (s, 3H), 1.54 (s, 9H), 1.37 (t, 3H).
LC/MS (Methode 1, ESIpos): $R_t$ = 1.20 min, m/z = 286 $[M+H]^+$.

**Beispiel 2A**

2-*tert.*-Butyl-4-ethyl-3-methyl-5-{[(2-phenylethyl)carbamoyl]amino}thiophen-2,4-dicarboxylat

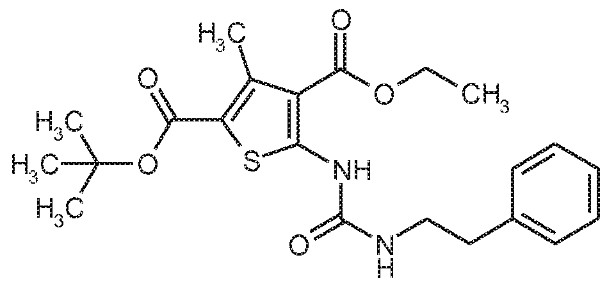

10.0 g (35.0 mmol) 2-*tert.*-Butyl-4-ethyl-5-amino-3-methylthiophen-2,4-dicarboxylat (Beispiel 1A) wurden in 500 ml Dichlormethan gelöst und mit 11.4 g (70.1 mmol) *N,N'*-Carbonyldiimidazol (CDI) und 19.6 ml (140 mmol) Triethylamin versetzt. Das Reaktionsgemisch wurde 2 Tage bei RT gerührt, bevor 8.8 ml (70.1 mmol) 2-Phenethylamin hinzugefügt wurden. Nach weiteren 2 h Rühren bei RT wurde das Gemisch am Rotationsverdampfer zur Trockene eingedampft. Der verbliebene Rückstand wurde mittels MPLC gereinigt (Kieselgel, Cyclohexan/Ethylacetat 20:1 → 10:1). Nach Eindampfen der Produktfraktionen und Trocknen des Rückstands im Hochvakuum wurden 14.4 g (95% d. Th.) der Titelverbindung erhalten.
$^1$H-NMR (400 MHz, DMSO-$d_6$, $\delta$/ppm): 10.54 (s, 1H), 8.17 (t, 1H), 7.33-7.29 (m, 2H), 7.26-7.19 (m, 3H), 4.30 (q, 2H), 3.36 (q, 2H), 2.77 (t, 2H), 2.62 (s, 3H), 1.50 (s, 9H), 1.32 (t, 3H).

LC/MS (Methode 1, ESIpos): $R_t$ = 1.43 min, m/z = 433 $[M+H]^+$.

**Beispiel 3A**

2-*tert.*-Butyl-4-ethyl-5-[(ethylcarbamoyl)amino]-3-methylthiophen-2,4-dicarboxylat

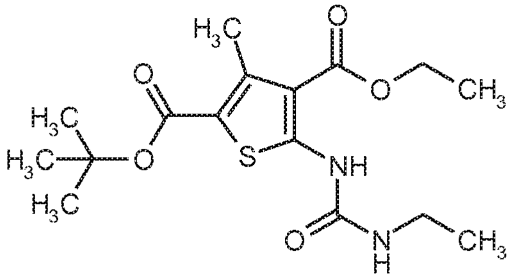

6.0 g (21.0 mmol) 2-*tert.*-Butyl-4-ethyl-5-amino-3-methylthiophen-2,4-dicarboxylat (Beispiel 1A) wurden in 300 ml Dichlormethan gelöst und mit 6.82 g (42.1 mmol) CDI und 11.7 ml (84.1 mmol) Triethylamin versetzt. Das Reaktionsgemisch wurde 2 Tage bei RT gerührt, bevor 42 ml (84.1 mmol) einer 2 M Lösung von Ethylamin in THF hinzugefügt wurden. Nach weiteren 2 h Rühren bei RT wurde das Gemisch am Rotationsverdampfer zur Trockene eingedampft. Der verbliebene Rückstand wurde mittels MPLC gereinigt (Biotage-Kartusche mit 340 g Kieselgel, Cyclohexan/ Ethylacetat 10:1 → 5:1). Nach Eindampfen der Produktfraktionen und Trocknen des Rückstands im Hochvakuum wurden 6.98 g (93% d. Th.) der Titelverbindung erhalten.
$^1$H-NMR (400 MHz, DMSO-$d_6$, δ/ppm): 10.52 (br. s, 1H), 8.06 (br. t, 1H), 4.31 (q, 2H), 3.13 (dq, 2H), 2.77 (t, 2H), 2.62 (s, 3H), 1.50 (s, 9H), 1.32 (t, 3H), 1.07 (t, 3H).
LC/MS (Methode 1, ESIpos): $R_t$ = 1.26 min, m/z = 357 $[M+H]^+$.

**Beispiel 4A**

2-*tert.*-Butyl-4-ethyl-5-{[(2,4-dimethoxybenzyl)carbamoyl]amino}-3-methylthiophen-2,4-di-carboxylat

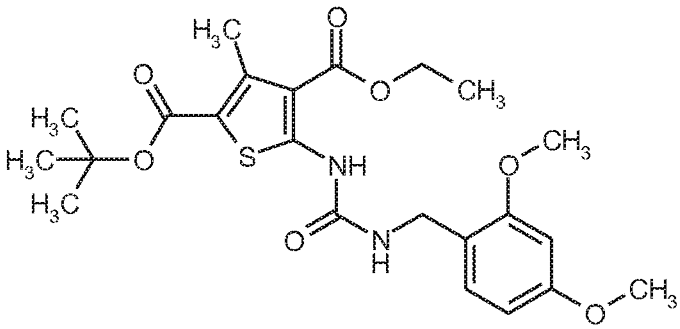

8.78 g (30.8 mmol) 2-*tert.*-Butyl-4-ethyl-5-amino-3-methylthiophen-2,4-dicarboxylat (Beispiel 1A) wurden in 300 ml Dichlormethan gelöst und mit 9.98 g (61.5 mmol) CDI und 17.2 ml (123 mmol) Triethylamin versetzt. Das Reaktionsgemisch wurde 2 Tage bei RT gerührt, bevor 9.3 ml (61.5 mmol) 2,4-Dimethoxybenzylamin hinzugefügt wurden. Nach weiteren 2 h Rühren bei RT wurde das Gemisch mit 200 ml Dichlormethan verdünnt und nacheinander mit je ca. 200 ml Wasser und gesättigter Natriumchlorid-Lösung gewaschen. Nach Trocknen über wasserfreiem Magnesiumsulfat wurde filtriert und eingedampft. Der verbliebene Rückstand wurde in Dichlormethan aufgenommen, wobei ein Teil ungelöst blieb, der durch Filtration abgetrennt wurde. Das Filtrat wurde auf Kieselgel aufgezogen und an Kieselgel mit Cyclohexan/Ethylacetat 2:1 → 1:1 als Laufmittel chromatographiert. Nach Vereinigen der Produktfraktionen, Eindampfen und Trocknen des Rückstands im Hochvakuum wurden 12.8 g (87% d. Th.) der Titelverbindung erhalten.
$^1$H-NMR (400 MHz, DMSO-$d_6$, δ/ppm): 10.56 (s, 1H), 8.32 (t, 1H), 7.13 (d, 1H), 6.57 (d, 1H), 6.49 (dd, 1H), 4.30 (q, 2H), 4.19 (d, 2H), 3.80 (s, 3H), 3.75 (s, 3H), 2.62 (s, 3H), 1.50 (s, 9H), 1.32 (t, 3H).

LC/MS (Methode 1, ESIpos): $R_t$ = 1.40 min, m/z = 479 $[M+H]^+$.

**Beispiel 5A**

Diethyl-5-[(ethylcarbamoyl)amino]-3-methylthiophen-2,4-dicarboxylat

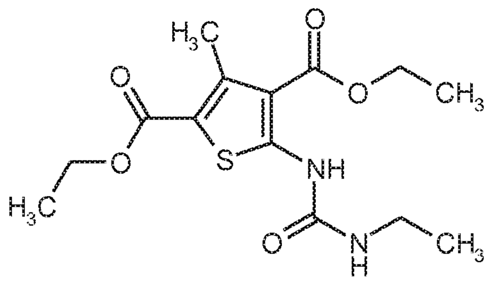

100 mg (0.377 mmol) Diethyl-2-amino-4-methylthiophen-2,5-dicarboxylat (kommerziell erhältlich) wurden in 0.4 ml Pyridin gelöst. Die Lösung wurde mit 0.122 ml (1.51 mmol) Ethylisocyanat versetzt und bei 80°C gerührt. Nach 28 h wurde auf RT abgekühlt und das Pyridin abdestilliert. Der Rückstand wurde in Dichlormethan aufgenommen und erneut eingeengt. Die Titelverbindung (135 mg, 98% d. Th.) wurde als braunes Kristallisat erhalten.
$^1$H-NMR (400 MHz, DMSO-$d_6$, δ/ppm): 10.53 (s, 1H), 8.06 (br. s, 1H), 4.32 (q, 2H), 4.22 (q, 2H), 3.14 (qd, 2H), 2.65 (s, 3H), 1.33 (t, 3H), 1.27 (t, 3H), 1.07 (t, 3H).
LC/MS (Methode 3): $R_t$ = 1.29 min, m/z = 329 $[M+H]^+$.

**Beispiel 6A**

*tert.*-Butyl-5-methyl-2,4-dioxo-3-(2-phenylethyl)-1,2,3,4-tetrahydrothieno[2,3-d]pyrimidin-6-carboxylat

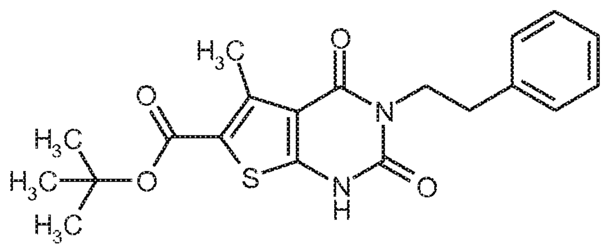

7.34 g (17.0 mmol) der Verbindung aus Bsp. 2A wurden in 145 ml Ethanol gelöst und mit 9.5 ml (25.4 mmol) einer 20%-igen Lösung von Natriumethanolat in Ethanol versetzt. Das Reaktionsgemisch wurde 2 h bei RT gerührt. Danach wurde es in ca. 400 ml Wasser gegossen und mit 5 M Essigsäure auf einen pH-Wert von ca. 5 gebracht. Dabei fiel das Produkt aus. Es wurde abgesaugt, mit Wasser neutral gewaschen und im Hochvakuum getrocknet. Es wurden 5.89 g (91% d. Th.) der Titelverbindung erhalten.
$^1$H-NMR (400 MHz, DMSO-$d_6$, δ/ppm): 12.44 (s, 1H), 7.33-7.29 (m, 2H), 7.25-7.20 (m, 3H), 4.01 (m, 2H), 2.83 (m, 2H), 2.72 (s, 3H), 1.52 (s, 9H).
LC/MS (Methode 1, ESIpos): $R_t$ = 1.28 min, m/z = 387 $[M+H]^+$.

**Beispiel 7A**

*tert.*-Butyl-3-ethyl-5-methyl-2,4-dioxo-1,2,3,4-tetrahydrothieno[2,3-d]pyrimidin-6-carboxylat

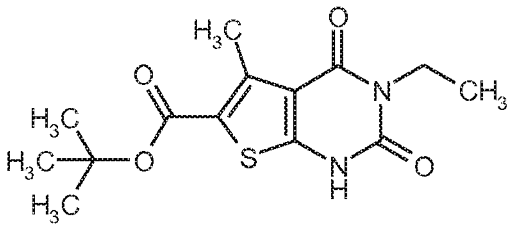

6.98 g (19.6 mmol) der Verbindung aus Bsp. 3A wurden in 130 ml Ethanol gelöst und mit 11 ml (29.4 mmol) einer 20%-igen Lösung von Natriumethanolat in Ethanol versetzt. Das Reaktionsgemisch wurde 2 h bei RT gerührt. Danach wurde es in ca. 400 ml Wasser gegossen und mit 5 M Essigsäure auf einen pH-Wert von ca. 5 gebracht. Dabei fiel das Produkt aus. Es wurde abgesaugt, mit Wasser neutral gewaschen und im Hochvakuum getrocknet. Es wurden 5.89 g (97% d. Th.) der Titelverbindung erhalten.
$^1$H-NMR (400 MHz, DMSO-$d_6$, δ/ppm): 12.39 (s, 1H), 3.85 (q, 2H), 2.71 (s, 3H), 1.51 (s, 9H), 1.11 (t, 3H).
LC/MS (Methode 1, ESIpos): $R_t$ = 1.06 min, m/z = 311 $[M+H]^+$.

**Beispiel 8A**

*tert.*-Butyl-3-(2,4-dimethoxybenzy1)-5-methyl-2,4-dioxo-1,2,3,4-tetrahydrothieno[2,3-d]pyrimidin-6-carboxylat

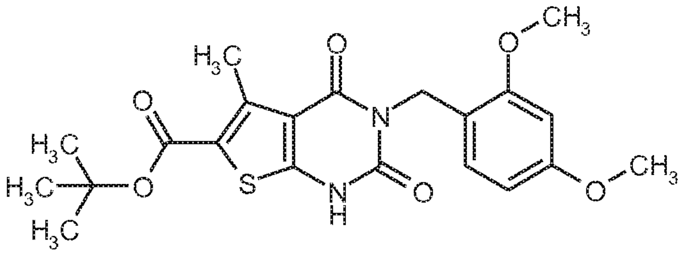

12.8 g (26.8 mmol) der Verbindung aus Bsp. 4A wurden in 250 ml Ethanol gelöst und mit 15 ml (40.2 mmol) einer 20%-igen Lösung von Natriumethanolat in Ethanol versetzt. Das Reaktionsgemisch wurde ca. 16 h bei RT gerührt. Danach wurde es in ca. 1.5 Liter Wasser gegossen und mit Essigsäure auf einen pH-Wert von ca. 5 gebracht. Dabei fiel das Produkt aus. Es wurde abgesaugt, mit Wasser neutral gewaschen und im Hochvakuum getrocknet. Es wurden 11.3 g (97% d. Th.) der Titelverbindung erhalten.
$^1$H-NMR (400 MHz, DMSO-$d_6$, δ/ppm): 12.50 (s, 1H), 6.72 (d, 1H), 6.56 (d, 1H), 6.39 (dd, 1H), 4.89 (s, 2H), 3.82 (s, 3H), 3.72 (s, 3H), 2.69 (s, 3H), 1.52 (s, 9H).
LC/MS (Methode 1, ESIpos): $R_t$ = 1.20 min, m/z = 433 $[M+H]^+$.

**Beispiel 9A**

Ethyl-3-ethyl-5-methyl-2,4-dioxo-1,2,3,4-tetrahydrothieno[2,3-d]pyrimidin-6-carboxylat

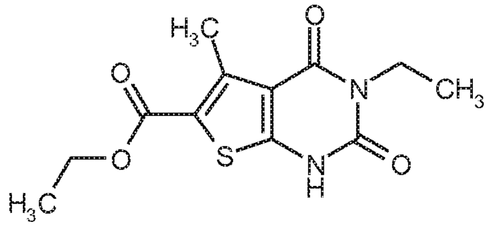

470 mg (1.26 mmol) der Verbindung aus Bsp. 5A wurden in 7.5 ml Ethanol gelöst und mit 0.94 ml Natriumethoxid-Lösung (21 Gew.-% in Ethanol) versetzt. Es wurde 1 h bei RT gerührt und dann 2.89 ml 1 M Salzsäure zugesetzt. Das Ethanol wurde am Rotationsverdampfer weitestgehend entfernt. Der verbliebene Rückstand wurde mit Wasser versetzt und der Feststoff abfiltriert, mit Wasser neutral gewaschen und trocken gesaugt. Es wurden 386 mg (99% d. Th.) der Titelverbindung erhalten.
$^1$H-NMR (400 MHz, DMSO-$d_6$. δ/ppm): 12.40 (br. s, 1H), 4.26 (q, 2H), 3.85 (q, 2H), 2.74 (s, 3H), 1.28 (t, 3H), 1.11 (t, 3H).
LC/MS (Methode 3): $R_t$ = 1.08 min, m/z = 283 $[M+H]^+$.

**Beispiel 10A**

*tert.*-Butyl-1-(2-methoxyethyl)-5-methyl-2,4-dioxo-3-(2-phenylethyl)-1,2,3,4-tetrahydrothieno-[2,3-d]pyrimidin-6-carboxylat

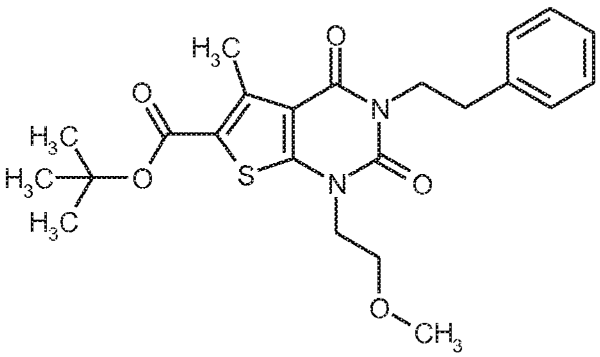

Eine Lösung von 2.0 g (5.18 mmol) der Verbindung aus Bsp. 6A in 60 ml DMF wurde mit 1.85 g (5.69 mmol) Cäsiumcarbonat versetzt und 10 min bei RT gerührt. Dann wurden 863 mg (6.21 mmol) 2-Bromethyl-methylether hinzugefügt, und das Gemisch wurde 30 min auf 100°C erhitzt. Danach wurde am Rotationsverdampfer zur Trockene eingedampft. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingedampft. Das Rohprodukt wurde mittels MPLC gereinigt (Puriflash-Säule mit 80 g Kieselgel, Cyclohexan/Ethylacetat 10:1). Nach Filtration, Eindampfen und Trocknen des Rückstands im Hochvakuum wurden 2.22 g (96% d. Th.) der Titelverbindung erhalten.
$^1$H-NMR (400 MHz, DMSO-$d_6$, δ/ppm): 7.32-7.28 (m, 2H), 7.24-7.20 (m, 3H), 4.09-4.04 (m, 4H), 3.61 (t, 2H), 3.24 (s, 3H), 2.84 (dd, 2H), 2.74 (s, 3H), 1.53 (s, 9H).
LC/MS (Methode 1, ESIpos): $R_t$ = 1.40 min, m/z = 445 $[M+H]^+$.

**Beispiel 11A**

*tert.*-Butyl-3-ethyl-5-methyl-2,4-dioxo-1-(3,3,3-trifluorpropyl)-1,2,3,4-tetrahydrothieno[2,3-d]-pyrimidin-6-carboxylat

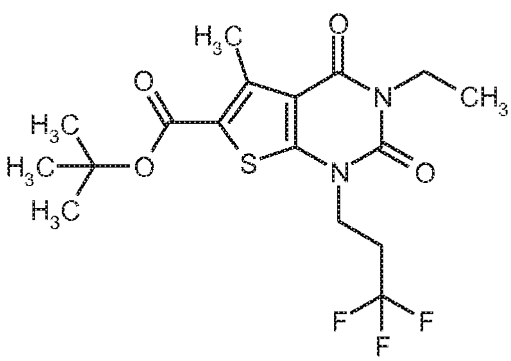

Eine Lösung von 3.0 g (9.67 mmol) der Verbindung aus Bsp. 7A in 100 ml DMF wurde mit 4.72 g (14.5 mmol) Cäsiumcarbonat versetzt und 20 min bei RT gerührt. Dann wurden 2.57 g (14.5 mmol) 3,3,3-Trifluor-1-iodpropan hinzugefügt, und das Gemisch wurde 5 h auf 100°C erhitzt. Danach wurde am Rotationsverdampfer zur Trockene eingedampft. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingedampft. Das Rohprodukt wurde mittels MPLC gereinigt (Puriflash-Säule mit 120 g Kieselgel, Cyclohexan/ Ethylacetat 10:1). Die Produktfraktionen wurden vereinigt, eingedampft und der Rückstand im Hochvakuum getrocknet. Es wurden 3.24 g (76% d. Th., 93% Reinheit) der Titelverbindung erhalten.
$^1$H-NMR (400 MHz, DMSO-$d_6$, $\delta$/ppm): 4.15 (t, 2H), 3.90 (q, 2H), 2.84-2.74 (m, 2H), 2.76 (s, 3H), 1.53 (s, 9H), 1.13 (t, 3H).
LC/MS (Methode 1, ESIpos): $R_t$ = 1.36 min, m/z = 407 [M+H]$^+$.

**Beispiel 12A**

*tert.*-Butyl-3-ethyl-1-(2-methoxyethyl)-5-methyl-2,4-dioxo-1,2,3,4-tetrahydrothieno[2,3-d]pyrimidin-6-carboxylat

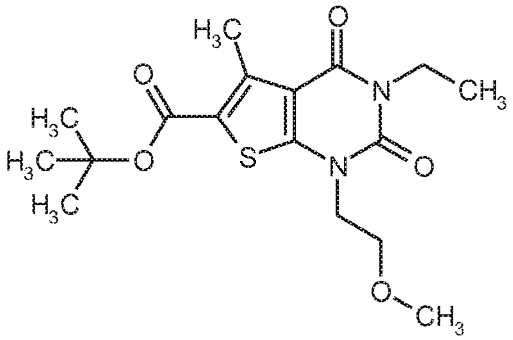

Analog zu dem unter Bsp. 11A beschriebenen Verfahren wurden aus 2.50 g (8.05 mmol) der Verbindung aus Bsp. 7A, 3.94 g (12.1 mmol) Cäsiumcarbonat und 1.68 g (12.1 mmol) 2-Bromethyl-methylether 2.13 g (71% d. Th.) der Titelverbindung erhalten. Abweichend von dem zuvor beschriebenen Verfahren wurde hier für die MPLC-Reinigung eine 50 g Kieselgel enthaltende Biotage-Kartusche verwendet, und das Produkt wurde abschließend durch Verrühren in einem Gemisch aus 60 ml Pentan und 0.5 ml Dichlormethan gereinigt.
$^1$H-NMR (400 MHz, DMSO-$d_6$, $\delta$/ppm): 4.07 (t, 2H), 3.90 (q, 2H), 3.65 (t, 2H), 3.25 (s, 3H), 2.74 (s, 3H), 1.53 (s, 9H), 1.13 (t, 3H).
LC/MS (Methode 1, ESIpos): $R_t$ = 1.21 min, m/z = 369 [M+H]$^+$.

**Beispiel 13A**

*tert.*-Butyl-3-(2,4-dimethoxybenzyl)-1-(2-methoxyethyl)-5-methyl-2,4-dioxo-1,2,3,4-tetrahydrothieno[2,3-d]pyrimidin-6-carboxylat

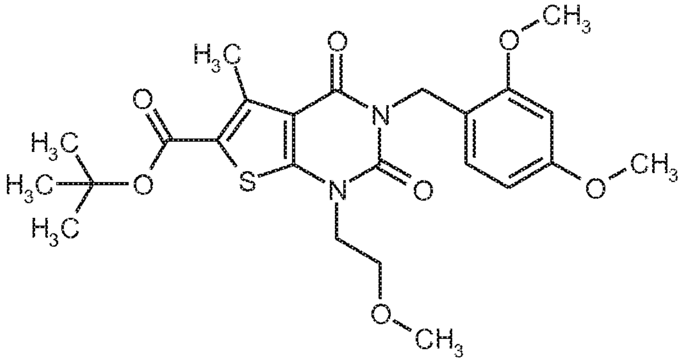

Eine Lösung von 7.50 g (15.6 mmol) der Verbindung aus Bsp. 8A in 200 ml Acetonitril wurde mit 3.24 g (23.4 mmol) Kaliumcarbonat versetzt und 10 min bei RT gerührt. Dann wurden 6.63 g (23.4 mmol) 2-Bromethyl-methylether hinzugefügt, und das Gemisch wurde ca. 18 h unter Rückfluss erhitzt. Nach Abkühlen auf RT wurden 300 ml Wasser zugesetzt, worauf das Produkt ausfiel. Es wurde abgesaugt, mit Wasser gewaschen und im Hochvakuum getrocknet. Es wurden 6.21 g (73% d. Th., 90% Reinheit) der Titelverbindung erhalten.
$^1$H-NMR (300 MHz, DMSO-$d_6$, δ/ppm): 6.74 (d, 1H), 6.57 (d, 1H), 6.40 (dd, 1H), 4.95 (s, 2H), 4.09 (t, 2H), 3.81 (s, 3H), 3.72 (s, 3H), 3.66 (t, 2H), 3.25 (s, 3H), 2.72 (s, 3H), 1.54 (s, 9H).

**Beispiel 14A**

Ethyl-3-ethyl-1-(3-fluoropropyl)-5-methyl-2,4-dioxo-1,2,3,4-tetrahydrothieno[2,3-d]pyrimidin-6-carboxylat

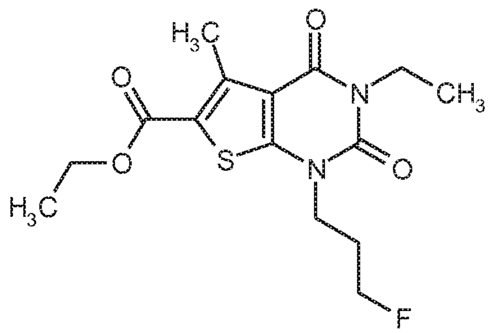

Eine Lösung von 385 mg (1.25 mmol) der Verbindung aus Bsp. 9A in 11 ml DMF wurde mit 433 mg (3.14 mmol) Kaliumcarbonat versetzt und 15 min bei RT gerührt. Dann wurden 1.06 g (5.64 mmol) 1-Fluor-3-iodpropan zugesetzt und das Gemisch 20 h bei 50°C gerührt. Das DMF wurde weitestgehend entfernt und der verbliebene Rückstand zwischen halbgesättigter Natriumchlorid-Lösung (100 ml) und Ethylacetat (100 ml) verteilt. Die wässrige Phase wurde mit Ethylacetat extrahiert. Die vereinigten organischen Phasen wurden über Natriumsulfat getrocknet, filtriert und eingeengt. Es wurden 469 mg der Titelverbindung erhalten.
$^1$H-NMR (400 MHz, DMSO-$d_6$, δ/ppm): 4.61 (t, 1H), 4.49 (t, 1H), 4.29 (q, 2H), 4.03 (t, 2H), 3.90 (q, 2H), 2.78 (s, 3H), 2.17-2.01 (m, 2H), 1.30 (t, 3H), 1.13 (t, 3H).
LC/MS (Methode 3): $R_t$ = 1.29 min, m/z = 343 $[M+H]^+$.

**Beispiel 15A**

1-(2-Methoxyethyl)-5-methyl-2,4-dioxo-3-(2-phenylethyl)-1,2,3,4-tetrahydrothieno[2,3-d]pyrimidin-6-carbonsäure

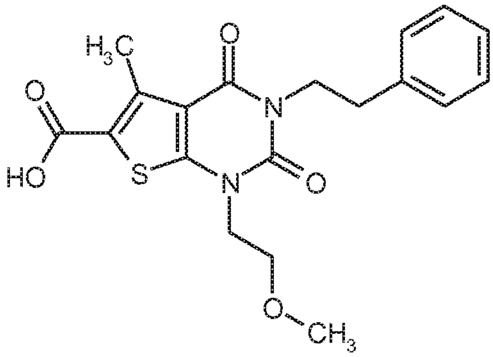

Eine Lösung von 5.0 g (11.2 mmol) der Verbindung aus Bsp. 10A in 225 ml Dichlormethan wurde mit 75 ml Trifluoressigsäure versetzt und 2 h bei RT gerührt. Danach wurde das Reaktionsgemisch am Rotationsverdampfer zur Trockene eingedampft. Der verbliebene Rückstand wurde in Diethylether verrührt, abgesaugt und der Feststoff im

Hochvakuum getrocknet. Es wurden 4.1 g (92% d. Th.) der Titelverbindung erhalten.
$^1$H-NMR (400 MHz, DMSO-$d_6$, δ/ppm): 13.37 (br. s, 1H), 7.33-7.29 (m, 2H), 7.25-7.20 (m, 3H), 4.09-4.04 (m, 4H), 3.62 (t, 2H), 3.25 (s, 3H), 2.84 (dd, 2H), 2.75 (s, 3H).
LC/MS (Methode 1, ESIpos): $R_t$ = 1.03 min, m/z = 389 $[M+H]^+$.

**Beispiel 16A**

3-Ethyl-5-methyl-2,4-dioxo-1-(3,3,3-trifluorpropyl)-1,2,3,4-tetrahydrothieno[2,3-d]pyrimidin-6-carbonsäure

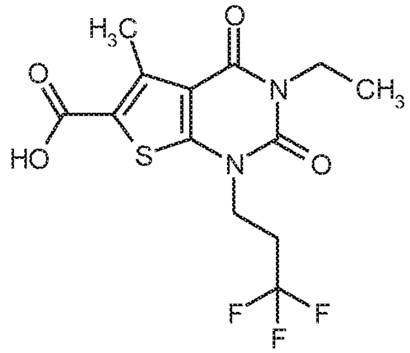

Eine Lösung von 6.89 g (16.9 mmol) der Verbindung aus Bsp. 11A in 240 ml Dichlormethan wurde mit 80 ml Trifluoressigsäure versetzt und 2 h bei RT gerührt. Danach wurde das Reaktionsgemisch am Rotationsverdampfer zur Trockene eingedampft. Der verbliebene Rückstand wurde in Diethylether verrührt, abgesaugt und der Feststoff im Hochvakuum getrocknet. Es wurden 5.13 g (86% d. Th.) der Titelverbindung erhalten.
$^1$H-NMR (400 MHz, DMSO-$d_6$, δ/ppm): 13.46 (br. s, 1H), 4.15 (t, 2H), 3.91 (q, 2H), 2.85-2.73 (m, 2H), 2.76 (s, 3H), 1.13 (t, 3H).
LC/MS (Methode 1, ESIpos): $R_t$ = 0.94 min, m/z = 351 $[M+H]^+$.

**Beispiel 17A**

3-Ethyl-1-(2-methoxyethyl)-5-methyl-2,4-dioxo-1,2,3,4-tetrahydrothieno[2,3-d]pyiimidin-6-carbonsäure

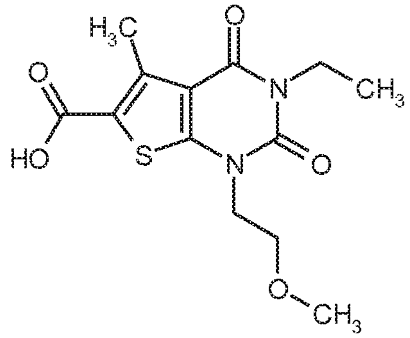

Analog zu dem unter Bsp. 16A beschriebenen Verfahren wurden aus 2.50 g (6.78 mmol) der Verbindung aus Bsp. 12A 1.82 g (85% d. Th.) der Titelverbindung erhalten. Die Reaktionszeit betrug in diesem Fall 1 h.
$^1$H-NMR (400 MHz, DMSO-$d_6$, δ/ppm): 13.32 (br. s, 1H), 4.07 (t, 2H), 3.90 (q, 2H), 3.65 (t, 2H), 3.25 (s, 3H), 2.75 (s, 3H), 1.13 (t, 3H).
LC/MS (Methode 1, ESIpos): $R_t$ = 0.99 min, m/z = 313 $[M+H]^+$.

**Beispiel 18A**

1-(2-Methoxyethyl)-5-methyl-2,4-dioxo-1,2,3,4-tetrahydiothieno[2,3-d]pyrimidin-6-caibonsäure

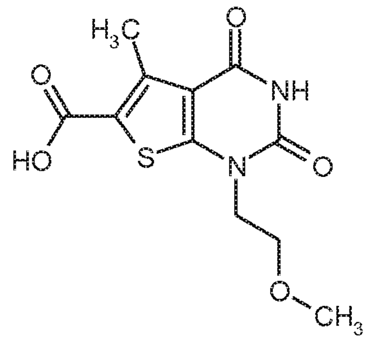

13.5 g (27.5 mmol) der Verbindung aus Bsp. 13A wurden in 350 ml Toluol gelöst und bei RT mit 22.0 g (165 mmol) festem Aluminiumtrichlorid versetzt. Anschließend wurde das Reaktionsgemisch 90 min bei 65°C gerührt. Nach dem Abkühlen auf RT wurde das Gemisch mit einem Eis/Wasserbad gekühlt und mit ca. 200 g Eis versetzt. Nachdem das Eis geschmolzen war, wurde das Gemisch durch Zusatz von konzentrierter Salzsäure auf einen pH-Wert von ca. 1 eingestellt. Dabei fiel das Produkt aus. Das Gemisch wurde 3 h bei RT gerührt, dann wurde das Produkt abgesaugt, mit wenig Acetonitril gewaschen und im Hochvakuum getrocknet. Es wurden 7.82 g (76% d. Th., 86% Reinheit) der Titelverbindung erhalten.
$^1$H-NMR (400 MHz, DMSO-$d_6$, $\delta$/ppm): 13.29 (breit, 1H), 11.52 (s, 1H), 4.02 (t, 2H), 3.63 (t, 2H), 3.24 (s, 3H), 2.71 (s, 3H).
LC/MS (Methode 1, ESIpos): $R_t$ = 0.52 min, m/z = 285 [M+H]$^+$.

**Beispiel 19A**

Methyl-1-(2-methoxyethyl)-5-methyl-2,4-dioxo-3-(2-phenylethyl)-1,2,3,4-tetrahydrothieno[2,3-d]-pyrimidin-6-carboxylat

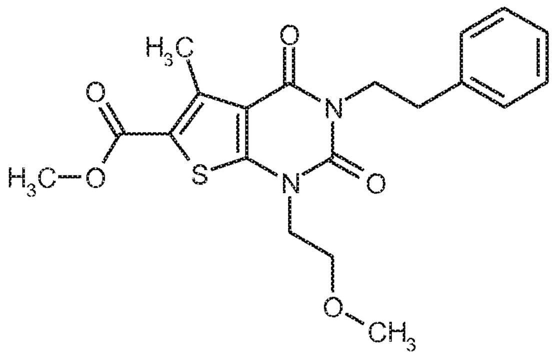

3.30 g (8.50 mmol) der Verbindung aus Bsp. 15A wurden in 125 ml Dichlormethan suspendiert und tropfenweise mit 7.4 ml (85.0 mmol) Oxalylchlorid sowie einem Tropfen DMF versetzt. Nach 2 h wurde das Reaktionsgemisch zur Trockene eingeengt. Der erhaltene Rückstand wurde wieder in 75 ml Dichlormethan aufgelöst und mit 8.6 ml (212 mmol) Methanol versetzt. Nachdem das Reaktionsgemisch ca. 18 h bei RT gerührt worden war, wurde es wiederum zur Trockene eingeengt und der Rückstand über eine Kieselgel-Kartusche chromatographiert (Biotage, 100 g Kieselgel, Cyclohexan/Ethylacetat 5:1 → 1:1). Nach Vereinigen der Produktfraktionen, Eindampfen und Trocknen im Hochvakuum wurden 3.35 g (97% d. Th.) der Titelverbindung erhalten.
$^1$H-NMR (400 MHz, $CDCl_3$, $\delta$/ppm): 7.33-7.28 (m, 4H), 7.26-7.20 (m, 1H), 4.21 (m, 2H), 4.13 (t, 2H), 3.88 (s, 3H), 3.71 (t, 2H), 3.35 (s, 3H), 2.95 (m, 2H), 2.89 (s, 3H).
LC/MS (Methode 1, ESIpos): $R_t$ = 1.23 min, m/z = 403 [M+H]$^+$.

**Beispiel 20A**

Methyl-1-(2-methoxyethyl)-3,5-dimethyl-2,4-dioxo-1,2,3,4-tetmhydrothieno[2,3-d]pyrimidin-6-carboxylat

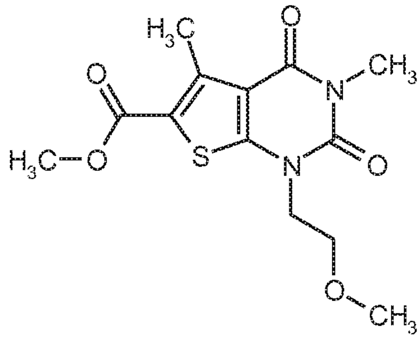

Eine Lösung von 402 mg (1.25 mmol) der Verbindung aus Bsp. 18A in 12.3 ml DMF wurde mit 1.246 g (3.825 mmol) Cäsiumcarbonat versetzt und 10 min bei RT gerührt. Dann wurden 452 mg (3.18 mmol) Iodmethan zugesetzt und das Gemisch 22 h bei RT gerührt. Das Reaktionsgemisch wurde danach zwischen Wasser (75 ml) und Dichlormethan (75 ml) verteilt. Die wässrige Phase wurde mit Dichlormethan extrahiert. Die vereinigten organischen Phasen wurden über Natriumsulfat getrocknet, filtriert und eingeengt. Der erhaltene Rückstand wurde an Kieselgel chromatographiert (Laufmittel Hexan/Ethylacetat). Es wurden 248 mg (59% d. Th.) der Titelverbindung erhalten.
$^1$H-NMR (400 MHz, DMSO-$d_6$, δ/ppm): 4.08 (t, 2H), 3.81 (s, 3H), 3.65 (t, 2H), 3.24 (s, 3H), 3.23 (s, 3H), 2.77 (s, 3H).
LC/MS (Methode 3): $R_t$ = 1.06 min, m/z = 313 $[M+H]^+$.

**Beispiel 21A**

Methyl-3-ethyl-5-methyl-2,4-dioxo-1-(3,3,3-trifluorpropyl)-1,2,3,4-tetrahydrothieno[2,3-d]-pyrimidin-6-carboxylat

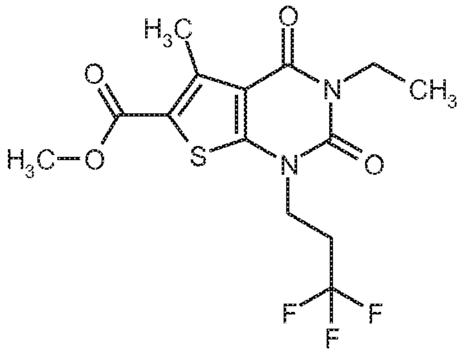

Analog zu dem unter Bsp. 19A beschriebenen Verfahren wurden aus 10.0 g (28.5 mmol) der Verbindung aus Bsp. 16A 10.8 g (99% d. Th., 96% Reinheit) der Titelverbindung erhalten. Auf eine chromatographische Reinigung des Produktes konnte hier verzichtet werden.
$^1$H-NMR (400 MHz, DMSO-$d_6$, δ/ppm): 4.16 (t, 2H), 3.91 (q, 2H), 3.83 (s, 3H), 2.90-2.70 (m, 2H), 2.79 (s, 3H), 1.13 (t, 3H).
LC/MS (Methode 1, ESIpos): $R_t$ = 1.12 min, m/z = 365 $[M+H]^+$.

**Beispiel 22A**

Methyl-3-ethyl-1-(2-methoxyethyl)-5-methyl-2,4dioxo-1,2,3,4-tetrahydrothieno[2,3-d]pyrimidin-6-carboxylat

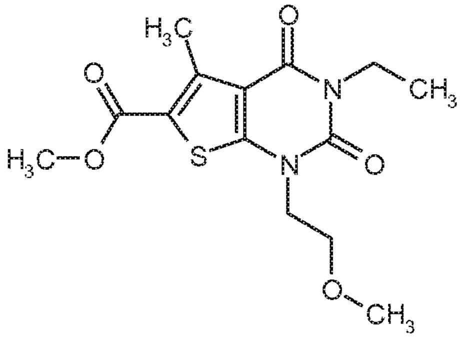

Analog zu dem unter Bsp. 19A beschriebenen Verfahren wurden aus 2.05 g (6.56 mmol) der Verbindung aus Bsp. 17A 2.11 g (98% d. Th.) der Titelverbindung erhalten. Auf eine chromatographische Reinigung des Produktes konnte hier verzichtet werden.
$^1$H-NMR (400 MHz, DMSO-$d_6$, $\delta$/ppm): 4.08 (t, 2H), 3.90 (q, 2H), 3.81 (s, 3H), 3.65 (t, 2H), 3.24 (s, 3H), 2.77 (s, 3H), 1.13 (t, 3H).
LC/MS (Methode 1, ESIpos): $R_t$ = 0.99 min, m/z = 327 $[M+H]^+$.

**Beispiel 23A**

2-Phenylethyl-1-(2-methoxyethyl)-5-methyl-2,4-dioxo-3-(2-phenylethyl)-1,2,3,4-tetrahydrothieno-[2,3-d]pyrimidin-6-carboxylat

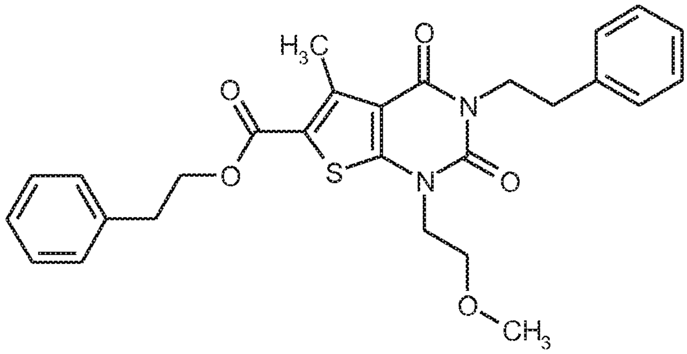

Analog zu dem unter Bsp. 20A beschriebenen Verfahren wurden aus 500 mg (1.583 mmol) der Verbindung aus Bsp. 18A und 879 mg (4.749 mmol) Phenethylbromid 432 mg (54% d. Th.) der Titelverbindung erhalten.
$^1$H-NMR (400 MHz, DMSO-$d_6$, $\delta$/ppm): 7.34-7.27 (m, 6H), 7.27-7.19 (m, 4H), 4.45 (t, 2H), 4.09-4.02 (m, 4H), 3.62 (t, 2H), 3.25 (s, 3H), 3.00 (t, 2H), 2.88-2.79 (m, 2H), 2.70 (s, 3H).
LC/MS (Methode 3): $R_t$ = 1.60 min, m/z = 493 $[M+H]^+$.

**Beispiel 24A**

Propyl-1-(2-methoxyethyl)-5-methyl-2,4-dioxo-3-propyl-1,2,3,4-tetrahydrothieno[2,3-d]pyrimidin-6-carboxylat

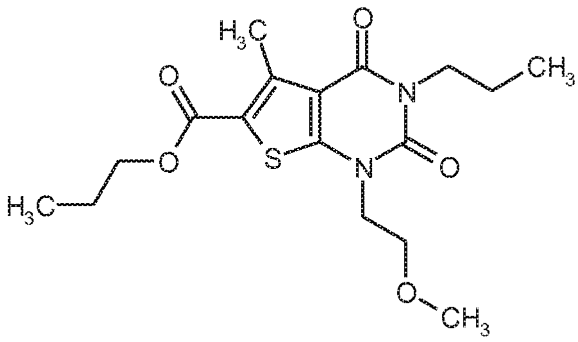

Analog zu dem unter Bsp. 20A beschriebenen Verfahren wurden aus 400 mg (1.266 mmol) der Verbindung aus Bsp. 18A und 538 mg (3.166 mmol) 1-Iodpropan 273 mg (57% d. Th.) der Titelverbindung erhalten.
$^1$H-NMR (400 MHz, DMSO-$d_6$, $\delta$/ppm): 4.20 (t, 2H), 4.08 (t, 2H), 3.85-3.79 (m, 2H), 3.65 (t, 2H), 3.24 (s, 3H), 2.77 (s, 3H), 1.69 (sext, 2H), 1.57 (sext, 2H), 0.95 (t, 3H), 0.87 (t, 3H).
LC/MS (Methode 3): $R_t$ = 1.42 min, m/z = 369 $[M+H]^+$.

**Beispiel 25A**

Allyl-3-allyl-1-(2-methoxyethyl)-5-methyl-2,4-dioxo-1,2,3,4-tetrahydrothieno[2,3-d]pyrimidin-6-carboxylat

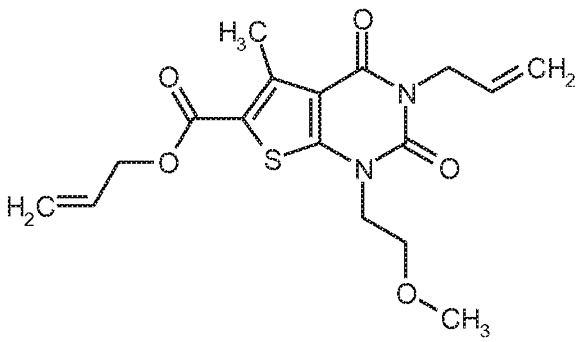

Analog zu dem unter Bsp. 20A beschriebenen Verfahren wurden aus 503 mg (1.592 mmol) der Verbindung aus Bsp. 18A und 481 mg (3.981 mmol) Allylbromid 352 mg (59% d. Th.) der Titelverbindung erhalten.
$^1$H-NMR (400 MHz, DMSO-$d_6$, $\delta$/ppm): 6.02 (ddt, 1H), 5.85 (ddt, 1H), 5.38 (dq, 1H), 5.29 (dd, 1H), 5.14 (dd, 1H), 5.12-5.08 (m, 1H), 4.78 (dt, 2H), 4.47 (d, 2H), 4.10 (t, 2H), 3.65 (t, 2H), 3.24 (s, 3H), 2.77 (s, 3H).
LC/MS (Methode 3): $R_t$ = 1.30 min, m/z = 365 $[M+H]^+$.

**Beispiel 26A**

Isopropyl-3-isopropyl-1-(2-methoxyethyl)-5-methyl-2,4-dioxo-1,2,3,4-tetrahydrothieno[2,3-d]-pyrimidin-6-carboxylat

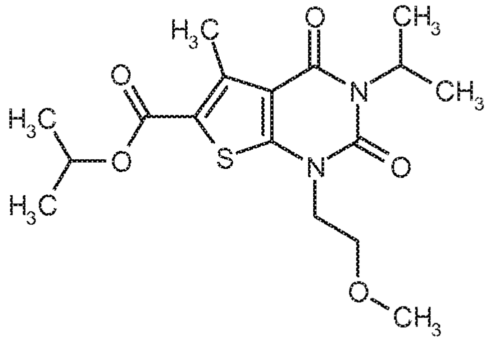

Analog zu dem unter Bsp. 20A beschriebenen Verfahren wurden aus 500 mg (1.583 mmol) der Verbindung aus Bsp. 18A und 949 mg (5.539 mmol) 2-Iodpropan 293 mg (49% d. Th.) der Titelverbindung erhalten. Die Reaktionszeit betrug hier 28 h bei einer Temperatur von 50°C.
$^1$H-NMR (400 MHz, DMSO-$d_6$, $\delta$/ppm): 5.18-5.03 (m, 2H), 4.05 (t, 2H), 3.64 (t, 2H), 3.25 (s, 3H), 2.75 (s, 3H), 1.40 (d, 6H), 1.30 (d, 6H).
LC/MS (Methode 3): $R_t$ = 1.43 min, m/z = 369 $[M+H]^+$.

**Beispiel 27A**

*sec.*-Butyl-3-*sec.*-butyl-1-(2-methoxyethyl)-5-methyl-2,4-dioxo-1,2,3,4-tetrahydrothieno[2,3-d]-pyrimidin-6-carboxylat (*Diastereomerengemisch*)

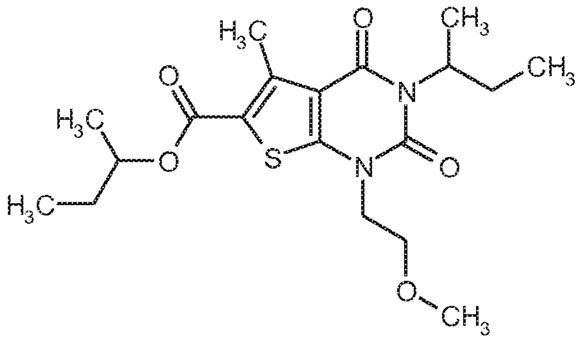

Analog zu dem unter Bsp. 20A beschriebenen Verfahren wurden aus 500 mg (1.583 mmol) der Verbindung aus Bsp. 18A und 685 mg (4.749 mmol) 2-Brombutan 325 mg (49% d. Th.) der Titelverbindung erhalten. Die Reaktionszeit betrug hier 19 h bei einer Temperatur von 70°C.
$^1$H-NMR (400 MHz, DMSO-$d_6$, $\delta$/ppm): 5.00-4.84 (m, 1H), 4.13-3.99 (m, 1H), 3.63 (t, 1H), 3.23 (s, 1H), 2.76 (s, 1H), 2.07-1.94 (m, 1H), 1.80-1.69 (m, 1H), 1.68-1.59 (m, 2H), 1.41-1.35 (m, 3H), 1.27 (d, 3H), 0.90 (t, 3H), 0.76 (t, 3H).
LC/MS (Methode 3): $R_t$ = 1.58 min, m/z = 397 $[M+H]^+$.

**Beispiel 28A**

3-Methylbut-2-en-1-yl-1-(2-methoxyethyl)-5-methyl-3-(3-methylbut-2-en-1-yl)-2,4-dioxo-1,2,3,4-tetrahydrothieno[2,3-d]pyrimidin-6-carboxylat

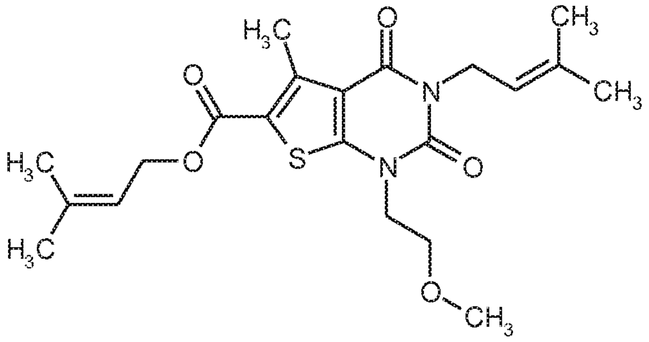

Analog zu dem unter Bsp. 20A beschriebenen Verfahren wurden aus 500 mg (1.583 mmol) der Verbindung aus Bsp. 18A und 786 mg (4.749 mmol) 1-Brom-3-methylbut-2-en 408 mg (58% d. Th.) der Titelverbindung erhalten.
$^1$H-NMR (400 MHz, DMSO-$d_6$, δ/ppm): 5.44-5.36 (m, 1H), 5.20-5.12 (m, 1H), 4.74 (d, 2H), 4.44 (d, 2H), 4.07 (t, 2H), 3.64 (t, 2H), 3.24 (s, 3H), 2.75 (s, 3H), 1.75 (s, 6H), 1.72 (s, 3H), 1.66 (s, 3H).
LC/MS (Methode 3): $R_t$ = 1.60 min, m/z = 421 $[M+H]^+$.

## Beispiel 29A

Cyclopropylmethyl-3-(cyclopropylmethyl)-1-(2-methoxyethyl)-5-methy1-2,4-dioxo-1,2,3,4-tetrahydrothieno[2,3-d]pyrimidin-6-carboxylat

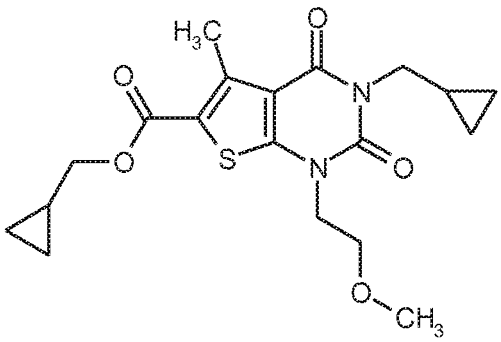

Analog zu dem unter Bsp. 20A beschriebenen Verfahren wurden aus 508 mg (1.608 mmol) der Verbindung aus Bsp. 18A und 542 mg (4.020 mmol) Cyclopropylmethylbromid 357 mg (55% d. Th.) der Titelverbindung erhalten.
$^1$H-NMR (400 MHz, DMSO-$d_6$, δ/ppm): 4.13-4.07 (m, 4H), 3.77 (d, 2H), 3.66 (t, 2H), 3.25 (s, 3H), 2.78 (s, 3H), 1.27-1.10 (m, 2H), 0.61-0.54 (m, 2H), 0.48-0.40 (m, 2H), 0.38-0.31 (m, 4H).
LC/MS (Methode 3): $R_t$ = 1.45 min, m/z = 393 $[M+H]^+$.

## Beispiel 30A

2-Fluorethyl-3-(2-fluorethyl)-1-(2-methoxyethyl)-5-methyl-2,4-dioxo-1,2,3,4-tetrahydrothieno-[2,3-d]pyrimidin-6-carboxylat

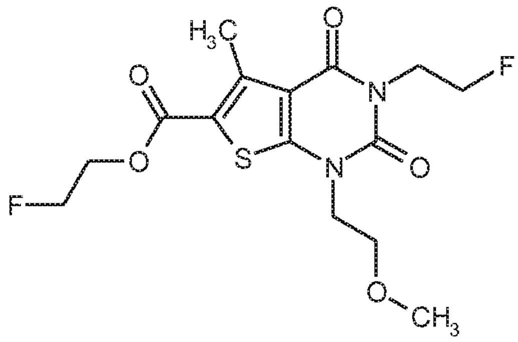

Analog zu dem unter Bsp. 20A beschriebenen Verfahren wurden aus 500 mg (1.583 mmol) der Verbindung aus Bsp. 18A und 507 mg (3.957 mmol) 1-Brom-2-fluorethan 317 mg (51% d. Th.) der Titelverbindung erhalten. Die Reaktionszeit betrug hier 18 h bei einer Temperatur von 50°C.
$^1$H-NMR (400 MHz, DMSO-$d_6$, δ/ppm): 4.78 (dd, 1H), 4.70-4.63 (m, 2H), 4.58-4.51 (m, 2H), 4.47 (dd, 1H), 4.24 (t, 1H), 4.21-4.16 (m, 1H), 4.10 (t, 2H), 3.66 (t, 2H), 3.25 (s, 3H), 2.78 (s, 3H).
LC/MS (Methode 3): $R_t$ = 1.09 min, m/z = 377 $[M+H]^+$.

**Beispiel 31A**

3,3,3-Trifluorpropyl-1-(2-methoxyethyl)-5-methyl-2,4-dioxo-3-(3,3,3-trifluorpropyl)-1,2,3,4-tetrahydrothieno[2,3-d]pyrimidin-6-carboxylat

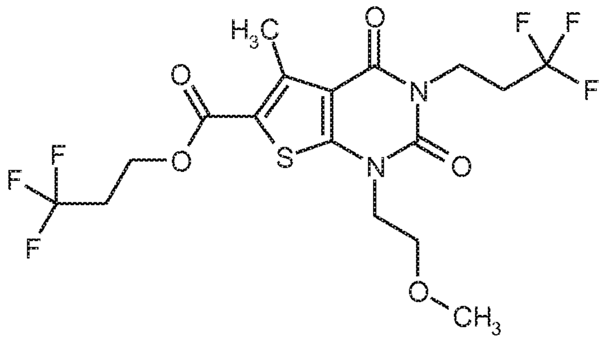

Analog zu dem unter Bsp. 20A beschriebenen Verfahren wurden aus 501 mg (1.586 mmol) der Verbindung aus Bsp. 18A und 888 mg (3.966 mmol) 1-Iod-3,3,3-trifluorpropan 455 mg (57% d. Th.) der Titelverbindung erhalten. Die Reaktionszeit betrug hier 44 h bei einer Temperatur von 50°C.
$^1$H-NMR (400 MHz, DMSO-$d_6$, δ/ppm): 4.47 (t, 2H), 4.14-4.06 (m, 4H), 3.65 (t, 2H), 3.24 (s, 3H), 2.86-2.73 (m, 5H), 2.65-2.54 (m, 2H).
LC/MS (Methode 3): $R_t$ = 1.39 min, m/z = 477 $[M+H]^+$.

**Beispiel 32A**

3-Fluorpropyl-3-(3-fluorpropyl)-1-(2-methoxyethyl)-5-methyl-2,4-dioxo-1,2,3,4-tetrahydrothieno-[2,3-d]pyrimidin-6-carboxylat

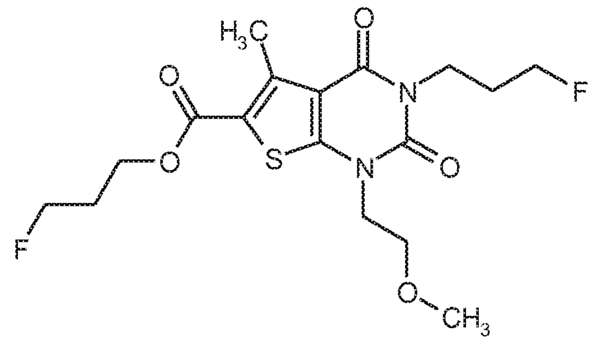

Analog zu dem unter Bsp. 20A beschriebenen Verfahren wurden aus 503 mg (1.594 mmol) der Verbindung aus Bsp. 18A und 898 mg (4.782 mmol) 3-Fluor-1-iodpropan 310 mg (45% d. Th.) der Titelverbindung erhalten. Die Reaktionszeit betrug hier 42 h.
$^1$H-NMR (400 MHz, DMSO-$d_6$, δ/ppm): 4.63 (t, 1H), 4.55 (t, 1H), 4.52 (t, 1H), 4.43 (t, 1H), 4.35 (t, 2H), 4.08 (t, 2H), 3.99 (t, 2H), 3.65 (t, 2H), 3.24 (s, 3H), 2.77 (s, 3H), 2.12 (quin, 1H), 2.05 (quin, 1H), 2.02-1.95 (m, 1H), 1.95-1.88 (m, 1H).
LC/MS (Methode 3): $R_t$ = 1.22 min, m/z = 405 $[M+H]^+$.

**Beispiel 33A**

2-Methoxyethyl-1,3-bis(2-methoxyethyl)-5-methyl-2,4-dioxo-1,2,3,4-tetrahydrothieno[2,3-d]-pyrimidin-6-carboxylat

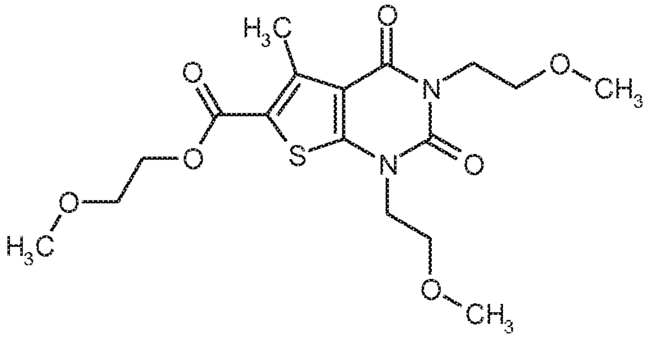

Analog zu dem unter Bsp. 20A beschriebenen Verfahren wurden aus 500 mg (1.583 mmol) der Verbindung aus Bsp. 18A und 550 mg (3.957 mmol) 1-Brom-2-methoxyethan 297 mg (47% d. Th.) der Titelverbindung erhalten.
$^1$H-NMR (400 MHz, DMSO-$d_6$, δ/ppm): 4.39-4.34 (m, 2H), 4.12-4.02 (m, 4H), 3.69-3.60 (m, 4H), 3.54-3.49 (m, 2H), 3.30 (s, 3H), 3.24 (d, 6H), 2.77 (s, 3H).
LC/MS (Methode 3): $R_t$ = 1.08 min, m/z = 401 $[M+H]^+$.

**Beispiel 34A**

1-Methoxypropan-2-yl-1-(2-methoxyethyl)-3-(1-methoxypropan-2-yl)-5-methyl-2,4-dioxo-1,2,3,4-tetrahydrothieno[2,3-d]pyrimidin-6-carboxylat (*Diastereomerengemisch*)

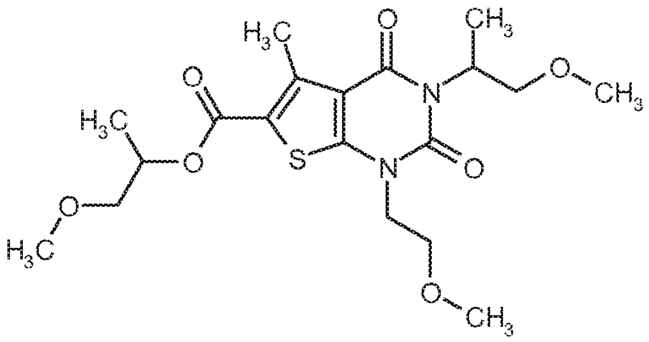

Analog zu dem unter Bsp. 20A beschriebenen Verfahren wurden aus 644 mg (2.038 mmol) der Verbindung aus Bsp. 18A und 985 mg (6.115 mmol) 2-Brom-1-methoxypropan 391 mg (44% d. Th.) der Titelverbindung erhalten. Die Reaktionszeit betrug hier 97 h bei einer Temperatur von 70°C.
$^1$H-NMR (400 MHz, DMSO-$d_6$, δ/ppm): 5.22-5.12 (m, 2H), 4.08-4.00 (m, 2H), 3.90 (dd, 1H), 3.63 (t, 2H), 3.57-3.52 (m, 1H), 3.51-3.43 (m, 2H), 3.29 (s, 3H), 3.24 (s, 3H), 3.21 (s, 3H), 2.75 (s, 3H), 1.34 (d, 3H), 1.25 (d, 3H).
LC/MS (Methode 3): $R_t$ = 1.28 min, m/z = 429 $[M+H]^+$.

**Beispiel 35A**

2-Methoxypropyl-1-(2-methoxyethyl)-3-(2-methoxypropyl)-5-methyl-2,4-dioxo-1,2,3,4-tetrahydrothieno[2,3-d]pyrimidin-6-carboxylat (*Diastereomerengemisch*)

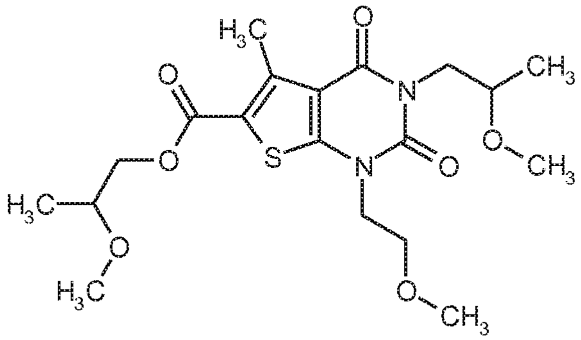

Analog zu dem unter Bsp. 20A beschriebenen Verfahren wurden aus 505 mg (1.599 mmol) der Verbindung aus Bsp. 18A und 773 mg (4.797 mmol) 1-Brom-2-methoxypropan 381 mg (53% d. Th.) der Titelverbindung erhalten. Die Reaktionszeit betrug hier 19 h bei einer Temperatur von 80°C.
$^1$H-NMR (400 MHz, DMSO-$d_6$, δ/ppm): 4.32-4.27 (m, 1H), 4.17 (dd, 1H), 4.12-4.01 (m, 3H), 3.76 (dd, 1H), 3.68-3.58 (m, 4H), 3.29 (s, 3H), 3.23 (s, 3H), 3.21 (s, 3H), 2.77 (s, 3H), 1.14 (d, 3H), 1.06 (d, 3H).
LC/MS (Methode 3): $R_t$ = 1.22 min, m/z = 429 $[M+H]^+$.

**Beispiel 36A**

Ethyl-2-[(ethylcarbamoyl)amino]-4-methylthiophen-3-carboxylat

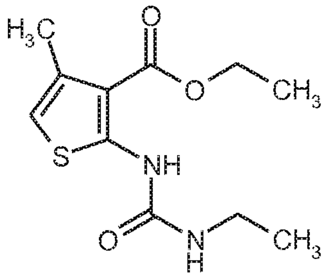

*Methode A:*

Eine Lösung von 150 g (0.810 mol) Ethyl-2-amino-4-methylthiophen-3-carboxylat und 113 ml (0.810 mol) Triethylamin in 1.5 Liter THF wurde mit 96 ml (1.21 mol) Ethylisocyanat versetzt. Anschließend wurde das Reaktionsgemisch 2 Tage unter Rückfluss erhitzt. Nach dem Abkühlen auf RT wurde das Gemisch in ca. 2 Liter Wasser gegossen und viermal mit insgesamt 1.1 Liter Dichlormethan extrahiert. Der organische Extrakt wurde über wasserfreiem Natriumsulfat getrocknet, filtriert und zur Trockene eingeengt. Nach Trocknen des Rückstands im Hochvakuum wurden 200 g (89% d. Th, ca. 93% Reinheit) der Titelverbindung erhalten, die ohne weitere Reinigung im nächsten Schritt verwendet wurde.

*Methode B:*

Eine Lösung von 440 g (2.37 mol) Ethyl-2-amino-4-methylthiophen-3-carboxylat in 4.4 Liter THF wurde bei RT mit 1.32 Liter (9.50 mol) Triethylamin und 770 g (4.75 mol) *N,N'*-Carbonyldiimidazol (CDI) versetzt. Nach 4 Tagen wurde das Reaktionsgemisch mit 4.75 Liter (9.50 mol) einer 2 M Lösung von Ethylamin in THF versetzt. Nach einer Reaktionszeit von 2 h wurde das Gemisch in ca. 27 Liter Wasser eingerührt. Das dabei ausgefallene Produkt wurde abgesaugt, mit wenig Wasser gewaschen und im Hochvakuum getrocknet. Es wurden 578 g (95% d. Th) der Titelverbindung erhalten.
$^1$H-NMR (300 MHz, DMSO-$d_6$, δ/ppm): 10.28 (s, 1H), 7.83 (breit, 1H), 6.39 (s, 1H), 4.27 (q, 2H), 3.13 (m, 2H), 2.26 (s, 3H), 1.31 (t, 3H), 1.06 (t, 3H).
LC/MS (Methode 1, ESIpos): $R_t$ = 0.92 min, m/z = 257 $[M+H]^+$.

**Beispiel 37A**

Ethyl-2-[(isopropylcarbamoyl)amino]-4-methylthiophen-3-carboxylat

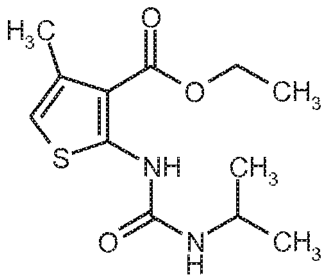

Eine Lösung von 10.0 g (54.0 mmol) Ethyl-2-amino-4-methylthiophen-3-carboxylat in 55 ml Pyridin wurde mit 10.6 ml (108 mmol) Isopropylisocyanat versetzt. Anschließend wurde das Reaktionsgemisch 95 h bei 55°C gerührt. Danach wurde am Rotationsverdampfer zur Trockene eingeengt, und der verbliebene Rückstand wurde mittels MPLC gereinigt (Biotage-Kartusche, 340 g Kieselgel, Cyclohexan/Ethylacetat 3:1). Nach Einengen der Produktfraktionen und Trocknen im Hochvakuum wurden 14.3 g (97% d. Th.) der Titelverbindung erhalten.
$^1$H-NMR (400 MHz, $CDCl_3$, δ/ppm): 10.61 (br. s, 1H), 6.23 (s, 1H), 4.61 (d, 1H), 4.32 (q, 2H), 4.08-3.87 (m, 1H), 2.33 (s, 3H), 1.38 (t, 3H), 1.22 (d, 6H).
LC/MS (Methode 5, ESIpos): $R_t$ = 1.34 min, m/z = 271 $[M+H]^+$.

**Beispiel 38A**

Ethyl-2-[(isobutylcarbamoyl)amino]-4-methylthiophen-3-carboxylat

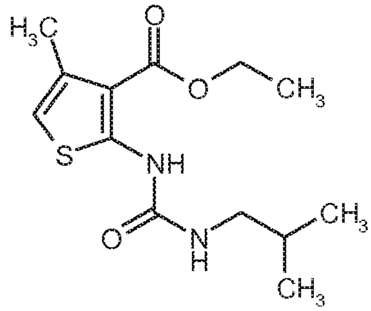

Eine Lösung von 10.0 g (54.0 mmol) Ethyl-2-amino-4-methylthiophen-3-carboxylat in 55 ml Pyridin wurde mit 12.3 ml (108 mmol) Isobutylisocyanat versetzt. Anschließend wurde das Reaktionsgemisch 43 h bei 50°C gerührt. Danach wurde am Rotationsverdampfer zur Trockene eingeengt, und der verbliebene Rückstand wurde mittels MPLC gereinigt (Biotage-Kartusche, 340 g Kieselgel, Cyclohexan/Ethylacetat 10:1). Nach Einengen der Produktfraktionen und Trocknen im Hochvakuum wurden 12.35 g (80% d. Th.) der Titelverbindung erhalten.
$^1$H-NMR (300 MHz, DMSO-$d_6$, δ/ppm): 10.32 (s, 1H), 7.87 (br. t, 1H), 6.41 (s, 1H), 4.28 (q, 2H), 2.93 (t, 2H), 2.26 (s, 3H), 1.70 (sept, 1H), 1.31 (t, 3H), 0.87 (d, 6H).
LC/MS (Methode 1, ESIpos): $R_t$ = 1.14 min, m/z = 285 $[M+H]^+$.

**Beispiel 39A**

Ethyl-4-methyl-2-{[(2,2,2-trifluorethyl)carbamoyl]amino}thiophen-3-carboxylat

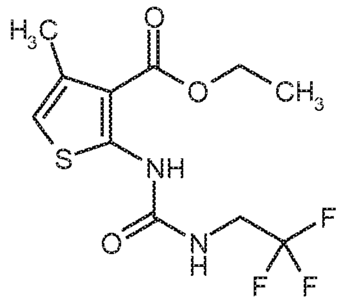

Eine Lösung von 1.5 g (7.854 mmol) Ethyl-2-amino-4-methylthiophen-3-carboxylat in 7.5 ml Pyridin wurde mit 1.473 g (11.782 mmol) 2,2,2-Trifluorethylisocyanat versetzt. Das Reaktionsgemisch wurde danach 30 min bei 70°C gerührt. Anschließend wurde am Rotationsverdampfer zur Trockene eingeengt. Der verbliebene Rückstand wurde in Dichlormethan gelöst und erneut zur Trockene eingeengt. Es wurden 2.84 g (89% d. Th. bei 77% Reinheit) der Titelverbindung erhalten.
$^1$H-NMR (400 MHz, DMSO-$d_6$, δ/ppm): 10.48 (s, 1H), 8.58 (t, 1H), 6.50 (s, 1H), 4.29 (q, 2H), 3.96 (qd, 2H), 2.28 (d, 3H), 1.32 (t, 3H).
LC/MS (Methode 3): $R_t$ = 1.26 min, m/z = 311 $[M+H]^+$.

**Beispiel 40A**

Ethyl-2-{[(2,2-difluorethyl)carbamoyl]amino}-4-methylthiophen-3-carboxylat

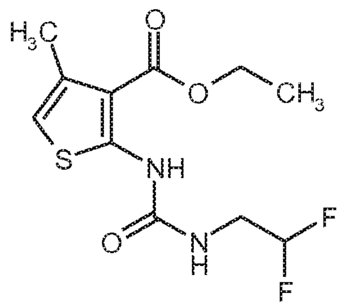

Eine Lösung von 1.19 g (6.277 mmol) Ethyl-2-amino-4-methylthiophen-3-carboxylat in 5.8 ml Pyridin wurde mit 1.4 g (9.415 mmol) 1,1-Difluor-2-isocyanatoethan versetzt. Das Reaktionsgemisch wurde danach 1 h bei 50°C gerührt. Anschließend wurde am Rotationsverdampfer zur Trockene eingeengt. Der verbliebene Rückstand wurde in Dichlormethan gelöst und erneut zur Trockene eingeengt. Es wurden 2.03 g der Titelverbindung erhalten.
$^1$H-NMR (400 MHz, DMSO-$d_6$, δ/ppm): 10.41 (s, 1H), 8.30 (t, 1H), 6.47 (s, 1H), 6.23-5.92 (m, 1H), 4.29 (q, 2H), 3.54 (tdd, 2H), 2.27 (d, 3H), 1.31 (t, 3H).
LC/MS (Methode 3): $R_t$ = 1.17 min, m/z = 293 $[M+H]^+$.

**Beispiel 41A**

Ethyl-2-{[(2-methoxyethyl)carbamoyl]amino}-4-methylthiophen-3-carboxylat

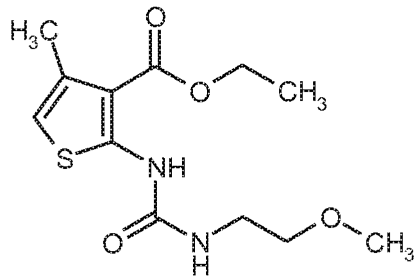

Eine Lösung von 2 g (10.473 mmol) Ethyl-2-amino-4-methylthiophen-3-carboxylat in 10 ml Pyridin wurde mit 2.12 g (20.945 mmol) 1-Isocyanato-2-methoxyethan versetzt. Das Reaktionsgemisch wurde danach 24 h bei 50°C gerührt. Anschließend wurde am Rotationsverdampfer zur Trockene eingeengt. Der verbliebene Rückstand wurde in Dichlormethan gelöst und erneut zur Trockene eingeengt. Das so erhaltene Material wurde in Hexan suspendiert und der Feststoff abgesaugt und getrocknet. Es wurden 3.32 g der Titelverbindung erhalten.
$^1$H-NMR (400 MHz, DMSO-$d_6$, δ/ppm): 10.30 (s, 1H), 8.02 (br. s, 1H), 6.42 (s, 1H), 4.27 (q, 2H), 3.39-3.36 (m, 2H), 3.28-3.23 (m, 5H), 2.26 (d, 3H), 1.31 (t, 3H).
LC/MS (Methode 3): $R_t$ = 1.12 min, m/z = 287 $[M+H]^+$.

**Beispiel 42A**

3-Ethyl-5-methylthieno[2,3-d]pyrimidin-2,4(1*H*,3*H*)-dion

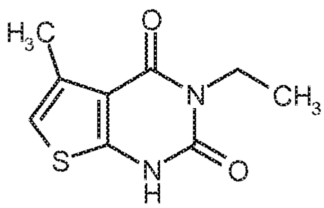

67 g (261 mmol) der Verbindung aus Bsp. 36A wurden in 1.6 Liter Ethanol gelöst und mit 141 ml (392 mmol) einer 21%-igen Lösung von Natriumethylat in Ethanol versetzt. Nachdem das Gemisch ca. 16 h bei RT gerührt worden war, wurde es in ca. 500 ml kaltes Wasser gegossen und durch Zusatz von Eisessig auf einen pH-Wert von ca. 5 gebracht. Der dabei ausgefallene Niederschlag wurde abgesaugt, mit Wasser neutral gewaschen und getrocknet. Es wurden 50 g (91% d. Th.) der Titelverbindung erhalten.
$^1$H-NMR (300 MHz, DMSO-$d_6$, δ/ppm): 12.10 (br. s, 1H), 6.66 (s, 1H), 3.86 (q, 2H), 2.35 (s, 3H), 1.11 (t, 3H).
LC/MS (Methode 1, ESIpos): $R_t$ = 0.67 min, m/z = 211 $[M+H]^+$.

**Beispiel 43A**

3-Isopropyl-5-methylthieno[2,3-d]pyrimidin-2,4(1*H*,3*H*)-dion

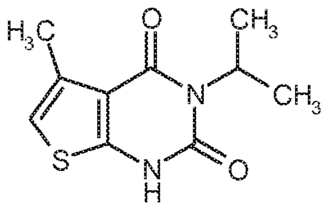

7.88 g (29.1 mmol) der Verbindung aus Bsp. 37A wurden in 80 ml Ethanol gelöst und mit 22 ml (58.3 mmol) einer 21%-igen Lösung von Natriumethylat in Ethanol versetzt. Nachdem das Gemisch 3 h bei 50°C gerührt worden war, wurde es bei RT mit 67 ml (67 mmol) 1 M Salzsäure versetzt, wobei das Produkt ausfiel. Das heterogene Gemisch wurde zunächst am Rotationsverdampfer auf ca. die Hälfte des ursprünglichen Volumens eingeengt. Nach dem Erkalten auf RT wurde das Produkt dann abgesaugt, mit Wasser neutral gewaschen und im Hochvakuum getrocknet. Es wurden 5.87 g (89% d. Th.) der Titelverbindung erhalten.
$^1$H-NMR (300 MHz, DMSO-$d_6$, δ/ppm): 11.95 (br. s, 1H), 6.65 (d, 1H), 5.10 (sept, 1H), 2.34 (d, 3H), 1.40 (d, 6H).
LC/MS (Methode 6, ESIpos): $R_t$ = 1.44 min, m/z = 225 $[M+H]^+$.

**Beispiel 44A**

3-Isobutyl-5-methylthieno[2,3-d]pyrimidin-2,4(1*H*,3*H*)-dion

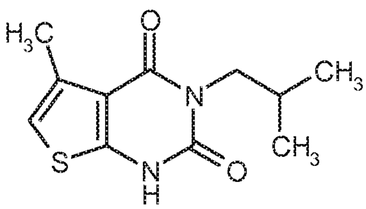

12.3 g (43.4 mmol) der Verbindung aus Bsp. 38A wurden in 120 ml Ethanol gelöst und mit 32.4 ml (86.8 mmol) einer 21 %-igen Lösung von Natriumethylat in Ethanol versetzt. Nachdem das Gemisch ca. 16 h bei RT gerührt worden war, wurde es mit 99.8 ml (99.8 mmol) 1 M Salzsäure versetzt. Der dabei ausgefallene Niederschlag wurde abgesaugt, mit Wasser neutral gewaschen und getrocknet. Es wurden 10.1 g (97% d. Th.) der Titelverbindung erhalten.
$^1$H-NMR (300 MHz, DMSO-$d_6$, δ/ppm): 12.09 (s, 1H), 6.68 (s, 1H), 3.67 (d, 2H), 2.35 (s, 3H), 2.04 (sept, 1H), 0.85 (d, 6H).
LC/MS (Methode 1, ESIpos): $R_t$ = 0.84 min, m/z = 239 $[M+H]^+$.

**Beispiel 45A**

5-Methyl-3-(2,2,2-trifluorethyl)thieno[2,3-d]pyrimidin-2,4(1*H*,3*H*)-dion

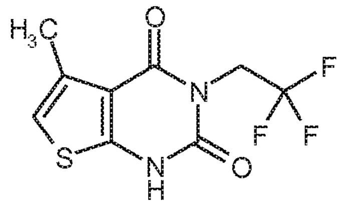

2.78 g (8.33 mmol) der Verbindung aus Bsp. 39A wurden in 30 ml Ethanol gelöst und mit 6.22 ml (16.66 mmol) einer 21%-igen Lösung von Natriumethylat in Ethanol versetzt. Nachdem das Gemisch ca. 14 h bei RT gerührt worden war, wurde es mit 19 ml 1 M Salzsäure versetzt. Der dabei ausgefallene Niederschlag wurde abgesaugt, mit Wasser neutral gewaschen und getrocknet. Es wurden 2.15 g (89% d. Th.) der Titelverbindung erhalten.
$^1$H-NMR (400 MHz, DMSO-$d_6$, $\delta$/ppm): 12.42 (br. s, 1H), 6.76 (d, 1H), 4.64 (q, 2H), 2.35 (d, 3H).
LC/MS (Methode 3): $R_t$ = 0.97 min, m/z = 265 $[M+H]^+$.

**Beispiel 46A**

3-(2,2-Difluorethyl)-5-methylthieno[2,3-d]pyrimidin-2,4(1*H*,3*H*)-dion

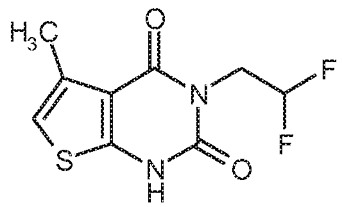

2.38 g (8 mmol) der Verbindung aus Bsp. 40A wurden in 23 ml Ethanol gelöst und mit 5.97 ml (16.01 mmol) einer 21%-igen Lösung von Natriumethylat in Ethanol versetzt. Nachdem das Gemisch ca. 1 h bei RT gerührt worden war, wurde es mit 18.4 ml 1 M Salzsäure versetzt. Der dabei ausgefallene Niederschlag wurde abgesaugt, mit Wasser neutral gewaschen und getrocknet. Es wurden 1.8 g (91% d. Th.) der Titelverbindung erhalten.
$^1$H-NMR (400 MHz, DMSO-$d_6$, $\delta$/ppm): 12.32 (br. s, 1H), 6.72 (d, 1H), 6.36-6.03 (m, 1H), 4.24 (td, 2H), 2.34 (d, 3H).
LC/MS (Methode 3): $R_t$ = 0.89 min, m/z = 211 $[M+H]^+$.

**Beispiel 47A**

3-(2-Methoxyethyl)-5-methylthieno[2,3-d]pyrimidin-2,4(1*H*,3*H*)-dion

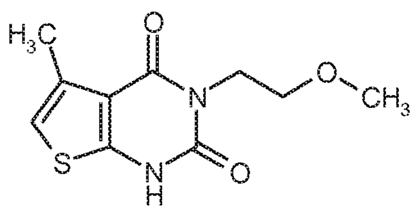

3.26 g (11.38 mmol) der Verbindung aus Bsp. 41A wurden in 30 ml Ethanol gelöst und mit 8.5 ml (22.76 mmol) einer 21%-igen Lösung von Natriumethylat in Ethanol versetzt. Nachdem das Gemisch ca. 2 h bei RT gerührt worden war, wurde es mit 26.1 ml 1 M Salzsäure versetzt. Der dabei ausgefallene Niederschlag wurde abgesaugt, mit Wasser neutral gewaschen und getrocknet. Es wurden 2.44 g (89% d. Th.) der Titelverbindung erhalten.
$^1$H-NMR (400 MHz, DMSO-$d_6$, $\delta$/ppm): 12.14 (br. s, 1H), 6.69 (d, 1H), 4.01 (t, 2H), 3.48 (t, 2H), 3.24 (s, 3H), 2.34 (d, 3H).
LC/MS (Methode 3): $R_t$ = 0.79 min, m/z = 241 $[M+H]^+$.

**Beispiel 48A**

3-Ethyl-5-methyl-2,4-dioxo-1,2,3,4-tetrahydrothieno[2,3-d]pyrimidin-6-carbaldehyd

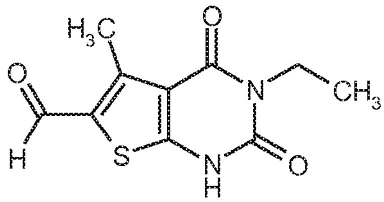

Eine Lösung von 442 g (2.10 mol) der Verbindung aus Bsp. 42A in 1.6 Liter (21.0 mol) DMF wurde vorsichtig mit 588 ml (6.31 mol) Phosphoroxychlorid versetzt (stark exotherme Reaktion). Nach Zugabe der halben Menge an Phosphoroxychlorid fiel ein Niederschlag aus, der durch Zusatz von einem weiteren Liter DMF in Lösung gebracht wurde. Dann wurde die Phosphoroxychlorid-Zugabe fortgesetzt. Nachdem die stark exotherme Reaktion (Temperaturanstieg bis 110°C) abgeklungen war, wurde das Gemisch noch 15 min nachgerührt. Danach wurde das Reaktionsgemisch vorsichtig in 10 Liter Eiswasser eingerührt. Nach 1 h Rühren wurde das ausgefallene Produkt abgesaugt, mit Wasser neutral gewaschen und getrocknet. Es wurden 437 g (87% d. Th.) der Titelverbindung erhalten.
$^1$H-NMR (400 MHz, DMSO-$d_6$, δ/ppm): 12.58 (breit, 1H), 10.06 (s, 1H), 3.86 (q, 2H), 2.76 (s, 3H), 1.12 (t, 3H).
LC/MS (Methode 1, ESIpos): $R_t$ = 0.68 min, m/z = 239 $[M+H]^+$.

**Beispiel 49A**

3-Isopropyl-5-methyl-2,4-dioxo-1,2,3,4-tetrahydrothieno[2,3-d]pyrimidin-6-carbaldehyd

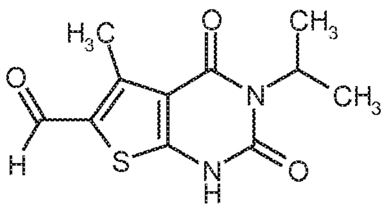

Eine Lösung von 6.90 g (30.8 mmol) der Verbindung aus Bsp. 43A in 28.4 ml (369 mol) DMF wurde vorsichtig mit 11.4 ml (123 mmol) Phosphoroxychlorid versetzt. Nachdem die stark exotherme Reaktion abgeklungen war, wurde das Gemisch noch 60 min bei RT nachgerührt. Da noch Edukt HPLC-analytisch detektierbar war, wurde das Reaktionsgemisch weitere 30 min auf 90°C erhitzt. Danach wurde das Reaktionsgemisch bei RT vorsichtig in 300 ml Wasser eingerührt, wobei das Produkt ausfiel. Das heterogene Gemisch wurde ca. 18 h bei RT gerührt, dann wurde das Produkt abgesaugt, mit Wasser neutral gewaschen und getrocknet. Das so erhaltene Rohprodukt wurde mit 30 ml Acetonitril bei RT verrührt. Der Feststoff wurde abgesaugt und ergab eine erste Fraktion der Titelverbindung. Die Mutterlauge des Verrührens wurde auf ca. 10 ml eingeengt und dann mit 30 ml Pentan versetzt. Dabei fiel eine zweite Fraktion des Produkts aus, die abgesaugt und mit der ersten vereinigt wurde. Insgesamt wurden so 7.25 g (93% d. Th.) der Titelverbindung erhalten.
$^1$H-NMR (400 MHz, DMSO-$d_6$, δ/ppm): 12.44 (br. s, 1H), 10.06 (s, 1H), 5.07 (sept, 1H), 2.75 (s, 3H), 1.40 (d, 6H).
LC/MS (Methode 1, ESIpos): $R_t$ = 0.76 min, m/z = 253 $[M+H]^+$.

**Beispiel 50A**

3-Isobutyl-5-methyl-2,4-dioxo-1,2,3,4-tetrahydrothieno[2,3-d]pyrimidin-6-carbaldehyd

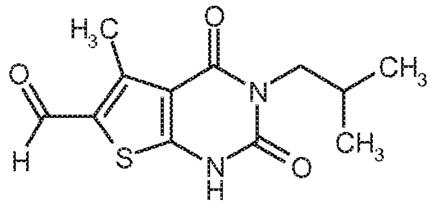

Eine Lösung von 300 mg (1.26 mmol) der Verbindung aus Bsp. 44A in 1 ml (12.6 mol) DMF wurde vorsichtig mit 1.4 ml (15.1 mmol) Phosphoroxychlorid versetzt. Nachdem die stark exotherme Reaktion abgeklungen war, wurde das Gemisch noch 15 min nachgerührt. Danach wurde das Reaktionsgemisch vorsichtig in 100 ml Eiswasser eingerührt. Nach 1 h Rühren wurde das ausgefallene Produkt abgesaugt, mit Wasser neutral gewaschen und getrocknet. Es wurden 323 mg (96% d. Th.) der Titelverbindung erhalten.
$^1$H-NMR (400 MHz, DMSO-$d_6$, $\delta$/ppm): 12.58 (breit, 1H), 10.06 (s, 1H), 3.66 (d, 2H), 2.76 (s, 3H), 2.03 (sept, 1H), 0.86 (d, 6H).
LC/MS (Methode 2, ESIpos): $R_t$ = 2.18 min, m/z = 267 $[M+H]^+$.

**Beispiel 51A**

5-Methyl-2,4-dioxo-3-(2,2,2-trifluorethyl)-1,2,3,4-tetrahydrothieno[2,3-d]pyrimidin-6-carbaldehyd

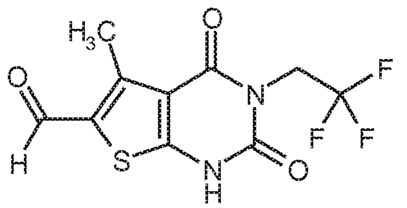

Eine Lösung von 4.67 g (15.37 mmol) der Verbindung aus Bsp. 45A in 143 ml DMF wurde unter Eisbadkühlung vorsichtig mit 14.33 ml (153.76 mmol) Phosphoroxychlorid versetzt. Das Gemisch wurde 5 h bei 50°C gerührt. Dann wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 4.52 g (99% d. Th.) der Titelverbindung erhalten.
$^1$H-NMR (400 MHz, DMSO-$d_6$, $\delta$/ppm): 12.93 (br. s, 1H), 10.08 (s, 1H), 4.64 (q, 2H), 2.76 (s, 3H).
LC/MS (Methode 3): $R_t$ = 0.93 min, m/z = 293 $[M+H]^+$.

**Beispiel 52A**

3-(2,2-Difluorethyl)-5-methyl-2,4-dioxo-1,2,3,4-tetrahydrothieno[2,3-d]pyrimidin-6-carbaldehyd

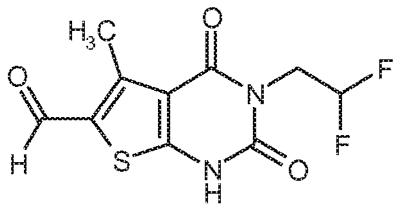

Eine Lösung von 995 mg (4.04 mmol) der Verbindung aus Bsp. 46A in 37.7 ml DMF wurde unter Eisbadkühlung vorsichtig mit 3.76 ml (40.4 mmol) Phosphoroxychlorid versetzt. Das Gemisch wurde 3 h bei 50°C gerührt. Dann wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 1.06 g (95% d. Th.) der Titelverbindung erhalten.

$^{1}H$-NMR (400 MHz, DMSO-$d_6$, δ/ppm): 12.84 (br. s, 1H), 10.07 (s, 1H), 6.37-6.04 (m, 1H), 4.24 (td, 2H), 2.76 (s, 3H). LC/MS (Methode 3): $R_t$ = 0.83 min, m/z = 275 $[M+H]^+$.

**Beispiel 53A**

3-(2-Methoxyethyl)-5-methyl-2,4-dioxo-1,2,3,4-tetrahydrothieno[2,3-d]pyrimidin-6-carbaldehyd

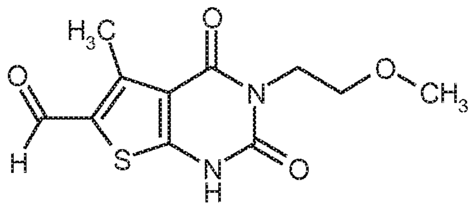

Eine Lösung von 500 mg (2.08 mmol) der Verbindung aus Bsp. 47A in 19.5 ml DMF wurde unter Eisbadkühlung vorsichtig mit 1.94 ml (20.8 mmol) Phosphoroxychlorid versetzt. Das Gemisch wurde 3 h bei 50°C gerührt. Dann wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 530 mg (93% d. Th.) der Titelverbindung erhalten.
$^{1}H$-NMR (400 MHz, DMSO-$d_6$, δ/ppm): 12.64 (br. s, 1H), 10.06 (s, 1H), 4.00 (t, 2H), 3.49 (t, 2H), 3.24 (s, 3H), 2.75 (s, 3H). LC/MS (Methode 3): $R_t$ = 0.76 min, m/z = 269 $[M+H]^+$.

**Beispiel 54A**

3-Ethyl-5-methyl-1-(2,2,2-trifluorethyl)thieno[2,3-d]pyrimidin-2,4(1*H*,3*H*)-dion

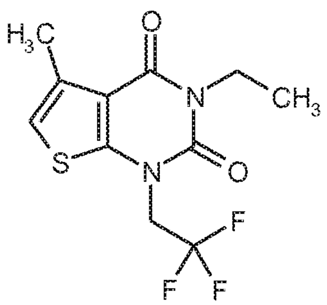

1.0 g (4.76 mmol) der Verbindung aus Bsp. 42A und 2.32 g (7.13 mmol) Cäsiumcarbonat wurden 10 min bei RT in 12 ml wasserfreiem DMF gerührt, bevor 700 µl (7.13 mmol) 1,1,1-Trifluor-2-iodethan hinzugefügt wurden. Dann wurde das Reaktionsgemisch in einem Mikrowellenofen (Biotage Initiator mit dynamischer Steuerung der Einstrahlleistung) 4 h lang auf 120°C erhitzt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und zur Trockene eingeengt. Der erhaltene Rückstand wurde mittels MPLC gereinigt (Biotage-Kartusche, 100 g Kieselgel, Cyclohexan/Ethylacetat 3:1). Nach Vereinigen der Produktfraktionen, Einengen und Trocknen im Hochvakuum wurden 592 mg (42% d. Th.) der Titelverbindung erhalten.
$^{1}H$-NMR (400 MHz, DMSO-$d_6$, δ/ppm): 6.91 (s, 1H), 4.84 (q, 2H), 3.92 (q, 2H), 2.39 (s, 3H), 1.13 (t, 3H).
LC/MS (Methode 1, ESIpos/neg): $R_t$ = 1.01 min, keine Ionisierung.

**Beispiel 55A**

3-Ethyl-5-methyl-1-(3,3,3-trifluorpropyl)thieno[2,3-d]pyrimidin-2,4(1*H*,3*H*)-dion

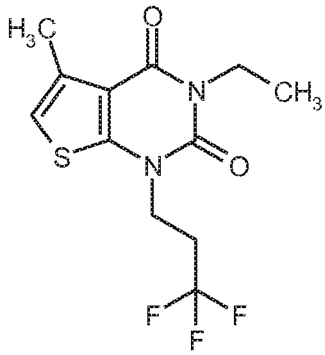

2.0 g (9.51 mmol) der Verbindung aus Bsp. 42A und 3.29 g (23.8 mmol) Kaliumcarbonat wurden 15 min bei RT in 50 ml wasserfreiem DMF gerührt, bevor 3.3 ml (28.5 mmol) 1,1,1-Trifluor-3-iodpropan hinzugefügt wurden. Da der Umsatz nach Rühren über Nacht bei RT noch nicht vollständig war, wurde mit weiteren 1.31 g (9.51 mmol) Kaliumcarbonat und 1.1 ml (9.51 mmol) 1,1,1-Trifluor-3-iodpropan versetzt und das Gemisch 2 h bei 60°C gerührt. Nach dem Abkühlen auf RT wurde mit Ethylacetat verdünnt und nacheinander mit Wasser (zweimal) und gesättigter Natriumchlorid-Lösung gewaschen. Nach Trocknen über wasserfreiem Magnesiumsulfat wurde filtriert und das Filtrat zur Trockene eingeengt. Das Rohprodukt wurde durch Chromatographie über eine Biotage-Kartusche gereinigt (340 g Kieselgel, Laufmittel: Cyclohexan/Ethylacetat 24:1 → 10:1). Nach Einengen und Trocknen der Produktfraktionen wurden 2.06 g (70% d. Th.) der Titelverbindung erhalten.
$^1$H-NMR (400 MHz, DMSO-$d_6$, δ/ppm): 6.88 (s, 1H), 4.12 (t, 2H), 3.91 (q, 2H), 2.84-2.71 (m, 2H), 2.39 (s, 3H), 1.12 (t, 3H).
LC/MS (Methode 1, ESIpos): $R_t$ = 1.02 min, m/z = 307 $[M+H]^+$.

**Beispiel 56A**

3-Ethyl-5-methyl-1-(4,4,4-trifluorbutyl)thieno[2,3-d]pyrimidin-2,4(1*H*,3*H*)-dion

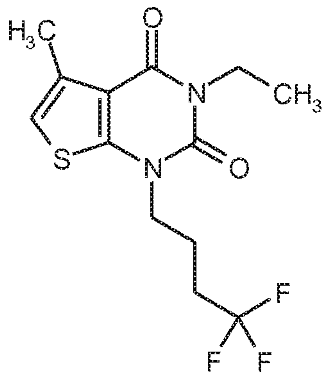

Analog zu dem unter Bsp. 54A beschriebenen Verfahren wurden aus 1.0 g (4.76 mmol) der Verbindung aus Bsp. 42A und 1.70 g (7.13 mmol) 1,1,1-Trifluor-4-iodbutan 1.35 g (90% d. Th.) der Titelverbindung hergestellt. Die Reaktion in der Mikrowelle erfolgte hier bei 100°C und die Reaktionszeit betrug 1 h. Als Laufmittelgradient bei der MPLC-Reinigung wurde Cyclohexan/Ethylacetat 20:1 → 10:1 verwendet.
$^1$H-NMR (400 MHz, DMSO-$d_6$, δ/ppm): 6.85 (s, 1H), 3.98 (t, 2H), 3.90 (q, 2H), 2.46-2.36 (m, 2H), 2.39 (s, 3H), 1.91 (quin, 2H), 1.12 (t, 3H).
LC/MS (Methode 1, ESIpos): $R_t$ = 1.07 min, m/z = 321 $[M+H]^+$.

**Beispiel 57A**

3-Ethyl-5-methyl-1-[2-(trifluormethoxy)ethyl]thieno[2,3-d]pyrimidin-2,4(1*H*,3*H*)-dion

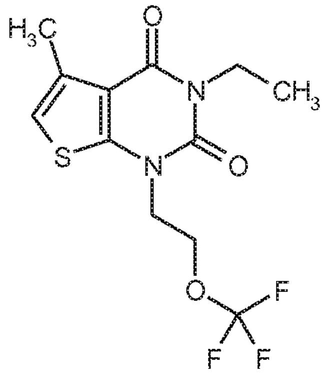

1.0 g (4.76 mmol) der Verbindung aus Bsp. 42A und 2.32 g (7.13 mmol) Cäsiumcarbonat wurden 10 min bei RT in 12 ml wasserfreiem DMF gerührt, bevor 1.38 g (7.13 mmol) 1-Brom-2-(trifluormethoxy)ethan [kommerziell erhältlich; Lit.: P.E. Aldrich, W.A. Sheppard, J. Org. Chem. 1964, 29 (1), 11-15] hinzugefügt wurden. Danach wurde das Reaktionsgemisch in einem Mikrowellenofen (Biotage Initiator mit dynamischer Steuerung der Einstrahlleistung) 1 h lang auf 100°C erhitzt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und zur Trockene eingeengt. Der erhaltene Rückstand wurde mittels MPLC gereinigt (Biotage-Kartusche, 100 g Kieselgel, Cyclohexan/Ethylacetat 10:1). Nach Vereinigen der Produktfraktionen, Einengen und Trocknen im Hochvakuum wurden 1.21 g (78% d. Th.) der Titelverbindung erhalten.
$^1$H-NMR (400 MHz, DMSO-$d_6$, $\delta$/ppm): 6.85 (s, 1H), 4.42 (t, 2H), 4.21 (t, 2H), 3.91 (q, 2H), 2.39 (s, 3H), 1.12 (t, 3H).
LC/MS (Methode 1, ESIpos): $R_t$ = 1.04 min, m/z = 323 $[M+H]^+$.

**Beispiel 58A**

3-Ethyl-5-methyl-1-(2-[(trifluormethyl)sulfanyl]ethyl)thieno[2,3-d]pyrimidin-2,4(1*H*,3*H*)-dion

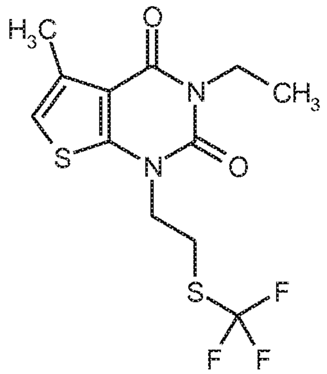

Analog zu dem unter Bsp. 57A beschriebenen Verfahren wurden aus 1.0 g (4.76 mmol) der Verbindung aus Bsp. 42A und 1.49 g (7.13 mmol) 1-Brom-2-[(trifluormethyl)sulfanyl]ethan 1.36 g (84% d. Th.) der Titelverbindung erhalten. Die MPLC-Reinigung erfolgte hier mit einem Laufmittelgradienten Cyclohexan/Ethylacetat 20:1 $\rightarrow$ 10:1. Dabei wurde eine erste, reine Fraktion der Titelverbindung erhalten (932 mg) sowie eine Mischfraktion, aus der nachfolgend eine zweite Fraktion reiner Titelverbindung (427 mg) mittels präparativer HPLC (Methode 9) isoliert wurde.
$^1$H-NMR (400 MHz, DMSO-$d_6$, $\delta$/ppm): 6.86 (s, 1H), 4.16 (t, 2H), 3.91 (q, 2H), 3.37 (t, 2H), 2.39 (s, 3H), 1.12 (t, 3H).
LC/MS (Methode 1, ESIpos): $R_t$ = 1.11 min, m/z = 339 $[M+H]^+$.

**Beispiel 59A**

3-Ethyl-1-(2-methoxyethyl)-5-methylthieno[2,3-d]pyrimidin-2,4(1*H*,3*H*)-dion

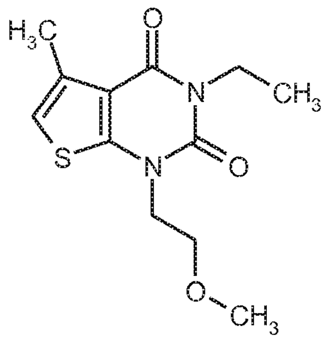

7.5 g (35.7 mmol) der Verbindung aus Bsp. 42A und 12.3 g (89.2 mmol) Kaliumcarbonat wurden 15 min bei RT in 200 ml wasserfreiem DMF gerührt, bevor 10 ml (107 mmol) 2-Bromethyl-methylether hinzugefügt wurden. Anschließend wurde das Reaktionsgemisch 2 h bei 50°C gerührt. Danach wurde der Großteil des DMFs am Rotationsverdampfer entfernt. Der Rückstand wurde mit ca. 800 ml Ethylacetat verdünnt und nacheinander mit Wasser (zweimal) und gesättigter Natriumchlorid-Lösung gewaschen. Nach Trocknen über wasserfreiem Magnesiumsulfat wurde filtriert und das Filtrat zur Trockene eingeengt. Das Rohprodukt wurde durch Chromatographie über eine Biotage-Kartusche gereinigt (340 g Kieselgel, Laufmittel: Cyclohexan/Ethylacetat 10:1 → 4:1). Nach Eindampfen und Trocknen der Produktfraktionen wurde eine erste Teilmenge von 6.51 g der Titelverbindung erhalten. Eine zweite Teilmenge von 0.91 g wurde durch Eindampfen der produkthaltigen Mischfraktionen (1.4 g) und anschließendes Verrühren mit 200 ml Pentan/Dichlormethan (25:1) gewonnen. Insgesamt wurden so 7.42 g (77% d. Th.) der Titelverbindung erhalten.
$^1$H-NMR (400 MHz, $CDCl_3$, δ/ppm): 6.42 (s, 1H), 4.12 (t, 2H), 4.08 (q, 2H), 3.74 (t, 2H), 3.35 (s, 3H), 2.49 (s, 3H), 1.26 (t, 3H).
LC/MS (Methode 1, ESIpos): $R_t$ = 0.87 min, m/z = 269 $[M+H]^+$.

**Beispiel 60A**

3-Ethyl-5-methyl-1-(tetrahydrofuran-2-ylmethyl)thieno[2,3-d]pyrimidin-2,4(1*H*,3*H*)-dion (*Racemat*)

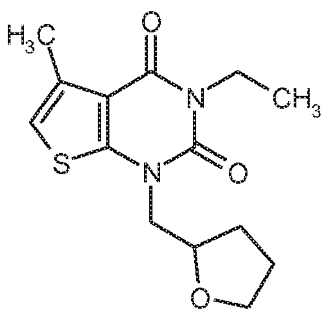

1.0 g (4.76 mmol) der Verbindung aus Bsp. 42A und 2.32 g (7.13 mmol) Cäsiumcarbonat wurden 10 min bei RT in 12 ml wasserfreiem DMF gerührt, bevor 1.18 g (7.13 mmol) racemisches 2-(Brommethyl)tetrahydrofuran hinzugefügt wurden. Anschließend wurde das Reaktionsgemisch in einem Mikrowellenofen (Biotage Initiator mit dynamischer Steuerung der Einstrahlleistung) 90 min lang auf 100°C erhitzt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und zur Trockene eingeengt. Der erhaltene Rückstand wurde zunächst mittels MPLC gereinigt (Biotage-Kartusche, 100 g Kieselgel, Cyclohexan/ Ethylacetat 10:1 → 8:1). Dabei wurde ein Gemisch aus *O*- und *N*-alkyliertem Produkt erhalten (1.27 g), das mittels präparativer HPLC (Methode 8) in seine Komponenten aufgetrennt wurde. Nach Vereinigen der Produktfraktionen, Einengen und Trocknen im Hochvakuum wurden 1.09 g (78% d. Th.) der Titelverbindung erhalten.
$^1$H-NMR (400 MHz, DMSO-$d_6$, δ/ppm): 6.80 (s, 1H), 4.27-4.21 (m, 1H), 4.05 (dd, 1H), 3.91 (q, 2H), 3.78-3.70 (m, 2H), 3.62 (dd, 1H), 2.38 (s, 3H), 2.03-1.95 (m, 1H), 1.93-1.76 (m, 2H), 1.71-1.62 (m, 1H), 1.12 (t, 3H).
LC/MS (Methode 1, ESIpos): $R_t$ = 0.93 min, m/z = 295 $[M+H]^+$.

**Beispiel 61A**

3-Ethyl-5-methyl-1-propylthieno[2,3-d]pyrimidin-2,4(1*H*,3*H*)-dion

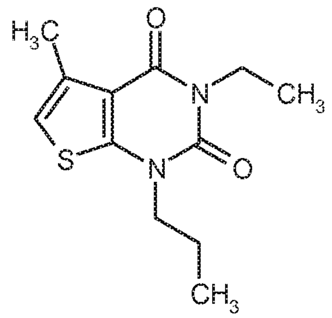

Analog zu dem unter Bsp. 54A beschriebenen Verfahren wurden aus 1.0 g (4.76 mmol) der Verbindung aus Bsp. 42A und 696 μl (7.13 mmol) 1-Iodpropan 1.08 g (95% d. Th.) der Titelverbindung hergestellt. Die Reaktionszeit betrug hier 60 min bei 100°C, und die MPLC-Reinigung wurde mit dem Laufmittelgradienten Cyclohexan/Ethylacetat 20:1 → 10:1 durchgeführt.
$^1$H-NMR (400 MHz, DMSO-$d_6$, δ/ppm): 6.83 (s, 1H), 3.91 (q, 2H), 3.85 (t, 2H), 2.38 (s, 3H), 1.71 (sext, 2H), 1.12 (t, 3H), 0.91 (t, 3H).
LC/MS (Methode 1, ESIpos): $R_t$ = 1.02 min, m/z = 253 $[M+H]^+$.

**Beispiel 62A**

3-Isopropyl-5-methyl-1-(3,3,3-trifluorpropyl)thieno[2,3-d]pyrimidin-2,4(1*H*,3*H*)-dion

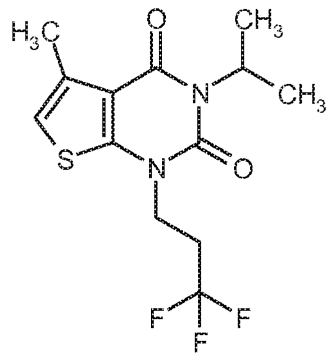

500 mg (2.23 mmol) der Verbindung aus Bsp. 43A und 1.90 g (3.34 mmol) Cäsiumcarbonat wurden 10 min bei RT in 10 ml wasserfreiem DMF gerührt, bevor 392 μl (3.34 mmol) 1,1,1-Trifluor-3-iodpropan hinzugefügt wurden. Anschließend wurde das Reaktionsgemisch in einem Mikrowellenofen (Biotage Initiator mit dynamischer Steuerung der Einstrahlleistung) zunächst 1 h auf 60°C und dann 1 h auf 100°C erhitzt. Danach wurden noch einmal die gleichen Mengen an Cäsiumcarbonat und 1,1,1-Trifluor-3-iodpropan zugesetzt und das Erhitzen auf 100°C 6 h lang fortgesetzt. Es wurde ein weiteres Mal die gleichen Mengen an Cäsiumcarbonat und 1,1,1-Trifluor-3-iod-propan zugegeben, und nach weiteren 30 min bei 100°C war der Umsatz vollständig. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und zur Trockene eingeengt. Der erhaltene Rückstand wurde mittels präparativer HPLC gereinigt (Methode 8). Nach Vereinigen der Produktfraktionen, Einengen und Trocknen im Hochvakuum wurden 553 mg (77% d. Th.) der Titelverbindung erhalten.
$^1$H-NMR (400 MHz, DMSO-$d_6$, δ/ppm): 6.85 (d, 1H), 5.14 (sept, 1H), 4.09 (t, 2H), 2.76 (qt, 2H), 2.38 (d, 3H), 1.40 (d, 6H).
LC/MS (Methode 1, ESIpos): $R_t$ = 1.14 min, m/z = 321 $[M+H]^+$.

**Beispiel 63A**

1-(2-Fluorethyl)-3-isopropyl-5-methylthieno[2,3-d]pyrimidin-2,4(1*H*,3*H*)-dion

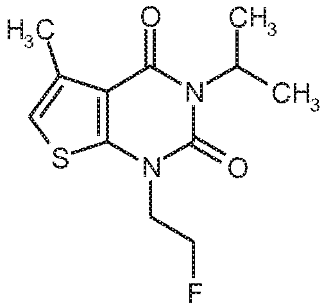

Analog zu dem unter Bsp. 54A beschriebenen Verfahren wurden aus 500 mg (2.23 mmol) der Verbindung aus Bsp. 43A und 277 µl (3.34 mmol) 1-Fluor-2-iodethan 462 mg (76% d. Th.) der Titelverbindung hergestellt. Die Reaktionszeit betrug hier 60 min bei 60°C, und die Aufreinigung des Produktes erfolgte mittels präparativer HPLC (Methode 8).
$^1$H-NMR (400 MHz, DMSO-$d_6$, δ/ppm): 6.80 (s, 1H), 5.14 (sept, 1H), 4.85-4.61 (dt, 2H), 4.28-4.07 (dt, 2H), 2.37 (s, 3H), 1.41 (d, 6H).
LC/MS (Methode 1, ESIpos): $R_t$ = 0.94 min, m/z = 271 $[M+H]^+$.

**Beispiel 64A**

3-Isopropyl-5-methyl-1-(tetrahydrofuran-2-ylmethyl)thieno[2,3-d]pyrimidin-2,4(1*H*,3*H*)-dion (*Racemat*)

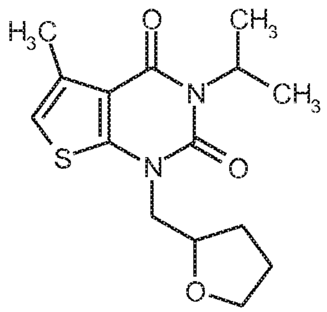

500 mg (2.23 mmol) der Verbindung aus Bsp. 43A und 1.09 g (3.34 mmol) Cäsiumcarbonat wurden 10 min bei RT in 10 ml wasserfreiem DMF gerührt, bevor 380 µl (3.34 mmol) 2-(Brommethyl)tetrahydrofuran hinzugefügt wurden. Anschließend wurde das Reaktionsgemisch in einem Mikrowellenofen (Biotage Initiator mit dynamischer Steuerung der Einstrahlleistung) 2 h lang auf 100°C erhitzt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und zur Trockene eingeengt. Der erhaltene Rückstand wurde in einem Acetonitril/Wasser-Gemisch verrührt. Das Ungelöste wurde abgesaugt und im Hochvakuum getrocknet, was eine erste Fraktion der Titelverbindung ergab (220 mg). Das Filtrat wurde mittels präparativer HPLC (Methode 8) gereinigt. Nach Eindampfen der Produktfraktionen und Trocknen im Hochvakuum wurde eine weitere Teilmenge der Titelverbindung erhalten (213 mg). Insgesamt wurden so 433 mg (57% d. Th., 90% Reinheit) der Titelverbindung gewonnen.
$^1$H-NMR (400 MHz, DMSO-$d_6$, δ/ppm): 6.77 (d, 1H), 5.15 (sept, 1H), 4.28-4.17 (m, 1H), 4.04 (dd, 1H), 3.80-3.56 (m, 3H), 2.37 (d, 3H), 2.06-1.73 (m, 3H), 1.71-1.60 (m, 1H), 1.40 (dd, 6H).
LC/MS (Methode 5, ESIpos): $R_t$ = 1.34 min, m/z = 309 $[M+H]^+$.

**Beispiel 65A**

3-Isopropyl-1,5-dimethylthieno[2,3-d]pyrimidin-2,4(1*H*,3*H*)-dion

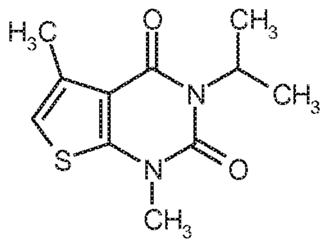

Analog zu dem unter Bsp. 54A beschriebenen Verfahren wurden aus 500 mg (2.23 mmol) der Verbindung aus Bsp. 43A und 316 µl (3.34 mmol) Dimethylsulfat 439 mg (82% d. Th.) der Titelverbindung hergestellt. Die Reaktionszeit betrug hier 60 min bei 100°C, und als Laufmittelgradient bei der MPLC-Reinigung wurde Cyclohexan/Ethylacetat 13:1 → 2:1 verwendet.
$^1$H-NMR (400 MHz, DMSO-$d_6$, δ/ppm): 6.80 (d, 1H), 5.15 (sept, 1H), 3.40 (s, 3H), 2.38 (d, 3H), 1.40 (d, 6H).
LC/MS (Methode 1, ESIpos): $R_t$ = 0.95 min, m/z = 239 $[M+H]^+$.

**Beispiel 66A**

3-Isobutyl-5-methyl-1-(3,3,3-trifluorpropyl)thieno[2,3-d]pyrimidin-2,4(1*H*,3*H*)-dion

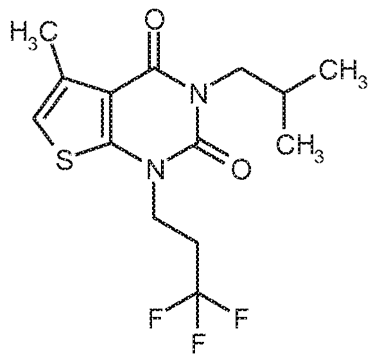

Eine Lösung von 2.23 g (9.35 mmol) der Verbindung aus Bsp. 44A in 82.6 ml DMF wurde mit 3.23 g (23.39 mmol) Kaliumcarbonat versetzt und 15 min bei RT gerührt. Dann wurden 6.48 g (28.07 mmol) 1,1,1-Trifluor-3-iodpropan zugesetzt und das Gemisch 73 h bei RT gerührt. Das DMF wurde danach weitestgehend abdestilliert und der verbliebene Rückstand zwischen halbgesättigter Natriumchlorid-Lösung (200 ml) und Ethylacetat (100 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 340 g Kieselgel, Laufmittel Hexan/Ethylacetat). Es wurden so 2.67 g (84% d. Th.) der Titelverbindung erhalten.
$^1$H-NMR (400 MHz, DMSO-$d_6$, δ/ppm): 6.88 (d, 1H), 4.13 (t, 2H), 3.71 (d, 2H), 2.84-2.70 (m, 2H), 2.38 (d, 3H), 2.11-1.96 (m, 1H), 0.85 (d, 6H).
LC/MS (Methode 3): $R_t$ = 1.4 min, m/z = 335 $[M+H]^+$.

**Beispiel 67A**

1-(2-Fluorethyl)-3-isobutyl-5-methylthieno[2,3-d]pyrimidin-2,4(1*H*,3*H*)-dion

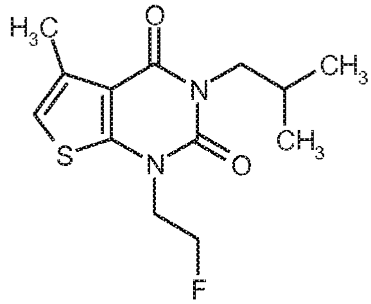

Analog zu dem unter Bsp. 54A beschriebenen Verfahren wurden aus 500 mg (2.10 mmol) der Verbindung aus Bsp. 44A und 261 µl (3.15 mmol) 1-Fluor-2-iodethan 458 mg (76% d. Th.) der Titelverbindung hergestellt. Die Reaktionszeit betrug hier 60 min bei 60°C, und die Aufreinigung des Produktes erfolgte mittels präparativer HPLC (Methode 8).
$^1$H-NMR (400 MHz, DMSO-$d_6$, δ/ppm): 6.83 (d, 1H), 4.82-4.64 (dt, 2H), 4.27-4.14 (dt, 2H), 3.72 (d, 2H), 2.38 (d, 3H), 2.05 (m, 1H), 0.86 (d, 6H).
LC/MS (Methode 1, ESIpos): $R_t$ = 1.10 min, m/z = 285 $[M+H]^+$.

**Beispiel 68A**

3-Isobutyl-1-(2-methoxyethyl)-5methylthieno[2,3-d]pyrimidin-2,4(1*H*,3*H*)-dion

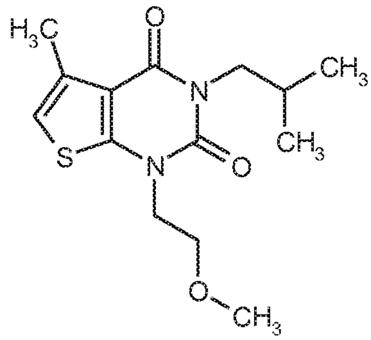

Eine Lösung von 2.23 g (9.35 mmol) der Verbindung aus Bsp. 44A in 80 ml DMF wurde mit 3.23 g (23.39 mmol) Kaliumcarbonat versetzt und 15 min bei RT gerührt. Dann wurden 3.9 g (28.07 mmol) 2-Bromethyl-methylether zugesetzt und das Gemisch 3 h bei 50°C gerührt. Das DMF wurde danach weitestgehend abdestilliert und der verbliebene Rückstand zwischen halbgesättigter Natriumchlorid-Lösung (300 ml) und Ethylacetat (150 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 340 g Kieselgel, Laufmittel Hexan/Ethylacetat). Es wurden so 2.37 g (85% d. Th.) der Titelverbindung erhalten.
$^1$H-NMR (400 MHz, DMSO-$d_6$, δ/ppm): 6.81 (d, 1H), 4.04 (t, 2H), 3.71 (d, 2H), 3.64 (t, 2H), 3.23 (s, 3H), 2.37 (d, 3H), 2.03 (dquin, 1H), 0.85 (d, 6H).
LC/MS (Methode 3): $R_t$ = 1.24 min, m/z = 297 $[M+H]^+$.

**Beispiel 69A**

3-Isobutyl-5-methyl-1-(tetrahydrofuran-2-ylmethyl)thieno[2,3-d]pyrimidin-2,4(1*H*,3*H*)-dion (*Racemat*)

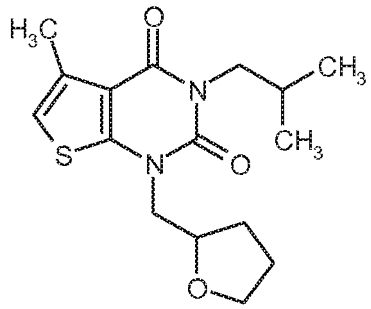

Analog zu dem unter Bsp. 54A beschriebenen Verfahren wurden aus 500 mg (2.10 mmol) der Verbindung aus Bsp. 44A und 358 µl (3.15 mmol) racemischem 2-(Brommethyl)tetrahydrofuran 516 mg (76% d. Th.) der Titelverbindung hergestellt. Die Reaktionszeit betrug hier 90 min bei 100°C, und die Aufreinigung des Produktes erfolgte mittels präparativer HPLC (Methode 8).
$^1$H-NMR (400 MHz, DMSO-$d_6$, δ/ppm): 6.81 (d, 1H), 4.28-4.19 (m, 1H), 4.04 (dd, 1H), 3.79-3.69 (m, 4H), 3.65-3.58 (m, 1H), 2.37 (d, 3H), 2.09-1.76 (m, 4H), 1.67 (m, 1H), 0.86 (d, 3H), 0.85 (d, 3H).
LC/MS (Methode 1, ESIpos): $R_t$ = 1.17 min, m/z = 323 $[M+H]^+$.

**Beispiel 70A**

3-Isobutyl-1,5-dimethylthieno[2,3-d]pyrimidin-2,4(1*H*,3*H*)-dion

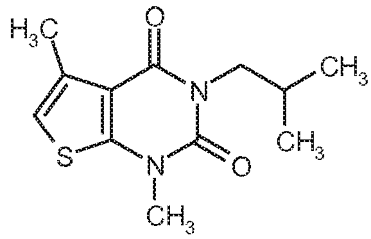

Analog zu dem unter Bsp. 54A beschriebenen Verfahren wurden aus 500 mg (2.10 mmol) der Verbindung aus Bsp. 44A und 596 µl (3.30 mmol) Dimethylsulfat 172 mg (32% d. Th.) der Titelverbindung hergestellt. Die Reaktionszeit betrug hier 8 h bei 60°C, und die Aufreinigung des Produktes erfolgte mittels präparativer HPLC (Methode 8).
$^1$H-NMR (400 MHz, DMSO-$d_6$, δ/ppm): 6.83 (d, 1H), 3.71 (d, 2H), 3.43 (s, 3H), 2.38 (d, 3H), 2.13-1.96 (m, 1H), 0.85 (d, 6H).
LC/MS (Methode 1, ESIpos): $R_t$ = 0.99 min, m/z = 253 $[M+H]^+$.

**Beispiel 71A**

1-(3-Fluorpropyl)-5-methyl-3-(2,2,2-trifluorethyl)thieno[2,3-d]pyrimidin-2,4(1*H*,3*H*)-dion

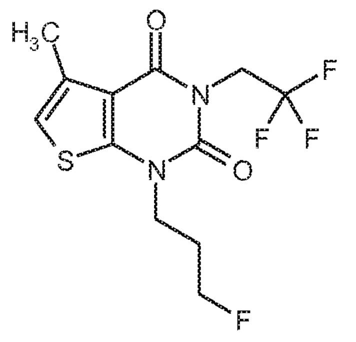

Eine Lösung von 1 g (3.45 mmol) der Verbindung aus Bsp. 45A in 30 ml DMF wurde mit 1.19 g (8.63 mmol) Kaliumcarbonat versetzt und 15 min bei RT gerührt. Dann wurden 1.94 g (10.35 mmol) 1-Fluor-3-iodpropan zugesetzt und das Gemisch 96 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die Wasserphase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 1.05 g (94% d. Th.) der Titelverbindung erhalten.
$^1$H-NMR (400 MHz, DMSO-$d_6$, δ/ppm): 6.92 (d, 1H), 4.69 (q, 2H), 4.60 (t, 1H), 4.48 (t, 1H), 4.03 (t, 2H), 2.39 (d, 3H), 2.17-2.01 (m, 2H).
LC/MS (Methode 3): $R_t$ = 1.2 min, m/z = 325 [M+H]$^+$.

**Beispiel 72A**

1-(2-Methoxyethyl)-5-methyl-3-(2,2,2-trifluorethyl)thieno[2,3-d]pyrinüdin-2,4(1*H*,3*H*)-dion

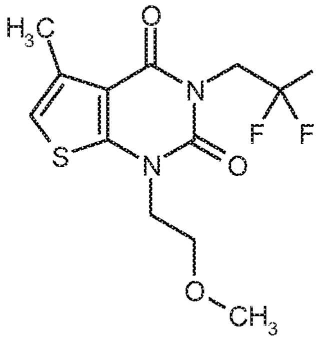

Eine Lösung von 1 g (3.45 mmol) der Verbindung aus Bsp. 45A in 30 ml DMF wurde mit 1.19 g (8.63 mmol) Kaliumcarbonat versetzt und 15 min bei RT gerührt. Dann wurden 1.43 g (8.61 mmol) 2-Bromethyl-methylether zugesetzt und das Gemisch 17 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 946 mg (85% d. Th.) der Titelverbindung erhalten.
$^1$H-NMR (400 MHz, DMSO-$d_6$, δ/ppm): 6.89 (d, 1H), 4.70 (q, 2H), 4.08 (t, 2H), 3.65 (t, 2H), 3.24 (s, 3H), 2.38 (d, 3H).
LC/MS (Methode 3): $R_t$ = 1.16 min, m/z = 323 [M+H]$^+$.

**Beispiel 73A**

3-Ethyl-5-methyl-2,4-dioxo-1-(2,2,2-trifluorethyl)-1,2,3,4-tetrahydrothieno[2,3-d]pyrimidin-6-carbaldehyd

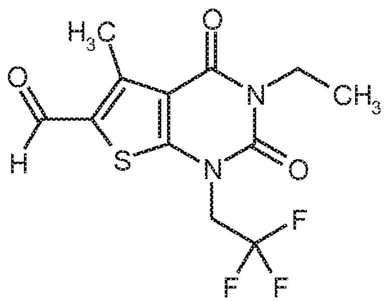

Eine Lösung von 581 mg (1.99 mmol) der Verbindung aus Bsp. 54A in 1.5 ml (19.9 mmol) DMF wurde mit 2.2 ml (23.8 mmol) Phosphoroxychlorid versetzt. Nachdem die stark exotherme Reaktion abgeklungen war, wurde das

Gemisch noch 30 min bei RT nachgerührt. Anschließend wurde das Reaktionsgemisch vorsichtig in 100 ml Wasser eingerührt. Nach 1 h Rühren wurde das ausgefallene Produkt abgesaugt, mit Wasser neutral gewaschen und getrocknet. Es wurden 600 mg (94% d. Th.) der Titelverbindung erhalten.
$^1$H-NMR (400 MHz, DMSO-$d_6$, $\delta$/ppm): 10.12 (s, 1H), 4.94 (q, 2H), 3.92 (q, 2H), 2.80 (s, 3H), 1.15 (t, 3H).
LC/MS (Methode 1, ESIpos): $R_t$ = 0.95 min, m/z = 321 $[M+H]^+$.

**Beispiel 74A**

3-Ethyl-5-methyl-2,4-dioxo-1-(3,3,3-trifluorpropyl)-1,2,3,4-tetrahydrothieno[2,3-d]pyrimidin-6-carbaldehyd

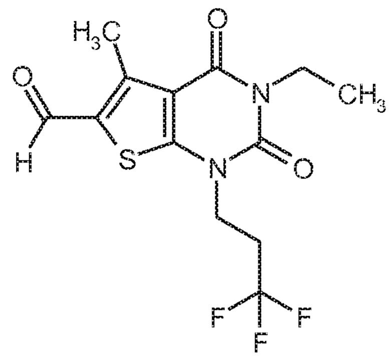

*Methode A:*

5.0 g (21.0 mmol) der Verbindung aus Bsp. 48A und 10.3 g (31.5 mmol) Cäsiumcarbonat wurden 15 min bei RT in 50 ml DMF gerührt, bevor 3.7 ml (31.5 mmol) 1,1,1-Trifluor-3-iodpropan hinzugefügt wurden. Anschließend wurde das Reaktionsgemisch ca. 18 h bei einer Temperatur von 60°C gerührt. Nach dem Abkühlen auf RT wurde mit ca. 200 ml Ethylacetat verdünnt und nacheinander mit Wasser und gesättigter Natriumchlorid-Lösung gewaschen. Nach Trocknen über wasserfreiem Magnesiumsulfat wurde filtriert und das Filtrat zur Trockene eingeengt. Das Rohprodukt wurde durch MPLC gereinigt (Biotage-Kartusche, 80 g Kieselgel, Laufmittel: Cyclohexan/Ethylacetat 3:1). Nach Einengen und Trocknen der Produktfraktionen wurden 4.8 g (62% d. Th.) der Titelverbindung erhalten.

*Methode B:*

Eine Lösung von 13.8 g (45.0 mmol) der Verbindung aus Bsp. 55A in 52 ml (676 mmol) DMF wurde zügig mit 12.6 ml (135 mmol) Phosphoroxychlorid versetzt. Nachdem die stark exotherme Reaktion fast abgeklungen war, wurde das Gemisch noch 30 min bei 90°C nachgerührt. Nach dem Erkalten auf RT wurde das Reaktionsgemisch vorsichtig in 300 ml lauwarmes Wasser eingerührt. Nach ca. 18 h Rühren wurde das ausgefallene Produkt abgesaugt, mit Wasser neutral gewaschen und getrocknet. Es wurden 13.9 g (92% d. Th.) der Titelverbindung erhalten.
$^1$H-NMR (400 MHz, DMSO-$d_6$, $\delta$/ppm): 10.11 (s, 1H), 4.18 (t, 2H), 3.91 (q, 2H), 2.86-2.74 (m, 2H), 2.80 (s, 3H), 1.14 (t, 3H).
LC/MS (Methode 1, ESIpos): $R_t$ = 0.98 min, m/z = 335 $[M+H]^+$.

**Beispiel 75A**

3-Ethyl-5-methyl-2,4-dioxo-1-(4,4,4-trifluorbutyl)-1,2,3,4-tetrahydrothieno[2,3-d]pyrimidin-6-carbaldehyd

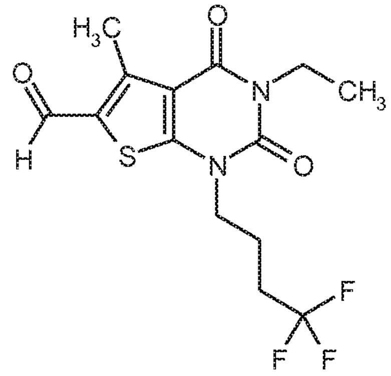

Analog zu dem unter Bsp. 73A beschriebenen Verfahren wurden aus 1.32 g (4.14 mmol) der Verbindung aus Bsp. 56A, 3.2 ml (41.3 mmol) wasserfreiem DMF und 4.6 ml (49.6 mmol) Phosphoroxychlorid 1.38 g (95% d. Th.) der Titelverbindung hergestellt.
$^1$H-NMR (400 MHz, DMSO-$d_6$, δ/ppm): 10.10 (s, 1H), 4.02 (t, 2H), 3.90 (q, 2H), 2.80 (s, 3H), 2.49-2.38 (m, 2H), 1.91 (quin, 2H), 1.14 (t, 3H).
LC/MS (Methode 1, ESIpos): $R_t$ = 1.03 min, m/z = 349 $[M+H]^+$.

## Beispiel 76A

1-(2,2-Difluorethyl)-3-ethyl-5-methyl-2,4-dioxo-1,2,3,4-tetrahydrothieno[2,3-d]pyrimidin-6-carbaldehyd

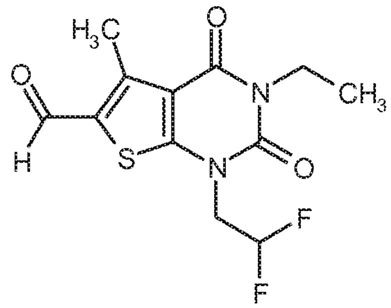

Eine Lösung von 349 mg (1.45 mmol) der Verbindung aus Bsp. 48A in 13 ml DMF wurde mit 601 mg (4.35 mmol) Kaliumcarbonat versetzt und 15 min bei RT gerührt. Dann wurden 1.11 g (5.8 mmol) 1,1-Difluor-2-iodethan zugesetzt und das Gemisch 90 h bei 80°C gerührt. Das DMF wurde danach weitestgehend abdestilliert und der verbliebene Rückstand zwischen halbgesättigter Natriumchlorid-Lösung (40 ml) und Ethylacetat (25 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Hexan/Ethylacetat). Es wurden 235 mg (54% d. Th.) der Titelverbindung erhalten.
$^1$H-NMR (400 MHz, DMSO-$d_6$, δ/ppm): 10.10 (s, 1H), 6.52-6.22 (m, 1H), 4.43 (td, 2H), 3.91 (q, 2H), 2.79 (s, 3H), 1.14 (t, 3H).
LC/MS (Methode 3): $R_t$ = 1.05 min, m/z = 303 $[M+H]^+$.

## Beispiel 77A

3-Ethyl-1-(2-fluorethyl)-5-methyl-2,4-dioxo-1,2,3,4-tetrahydrothieno[2,3-d]pyrinüdin-6-carbaldehyd

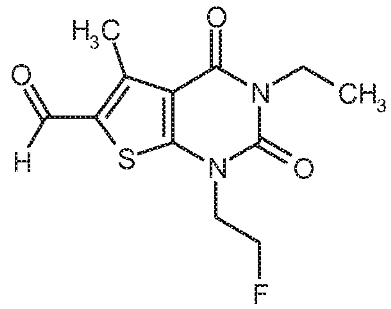

Analog zu dem unter Bsp. 54A beschriebenen Verfahren wurden aus 500 mg (2.10 mmol) der Verbindung aus Bsp. 48A und 547 mg (3.15 mmol) 1-Fluor-2-iodethan 258 mg (43% d. Th.) der Titelverbindung hergestellt. Die Reaktion in der Mikrowelle erfolgte hier bei 60°C mit einer Reaktionszeit von 3 h. Als Laufmittel bei der MPLC-Reinigung wurde Cyclohexan/Ethylacetat 2:1 verwendet.
$^1$H-NMR (400 MHz, DMSO-$d_6$, $\delta$/ppm): 10.10 (s, 1H), 4.75 (dt, 2H), 4.28 (dt, 2H), 3.91 (q, 2H), 2.79 (s, 3H), 1.15 (t, 3H).
LC/MS (Methode 1, ESIpos): $R_t$ = 0.80 min, m/z = 285 [M+H]$^+$.

**Beispiel 78A**

3-Ethyl-1-(3-fluorpropyl)-5-methyl-2,4-dioxo-1,2,3,4-tetrahydrothieno[2,3-d]pyrimidin-6-carbaldehyd

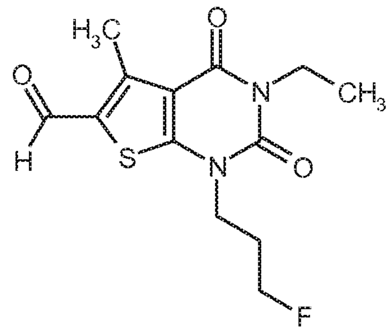

Eine Lösung von 471 mg (1.97 mmol) der Verbindung aus Bsp. 48A in 18 ml DMF wurde mit 683 mg (4.92 mmol) Kaliumcarbonat versetzt und 15 min bei RT gerührt. Dann wurden 1.11 g (5.93 mmol) 1-Fluor-3-iodpropan zugesetzt und das Gemisch 2.5 h bei 50°C gerührt. Das DMF wurde danach weitestgehend abdestilliert und der verbliebene Rückstand zwischen halbgesättigter Natriumchlorid-Lösung (300 ml) und Ethylacetat (150 ml) verteilt. Die wässrige Phase wurde mit Ethylacetat extrahiert. Die vereinigten organischen Phasen wurden über Natriumsulfat getrocknet, filtriert und eingeengt. Es wurden 545 mg (90% d. Th.) der Titelverbindung erhalten.
$^1$H-NMR (400 MHz, DMSO-$d_6$, $\delta$/ppm): 10.10 (s, 1H), 4.61 (t, 1H), 4.49 (t, 1H), 4.05 (t, 2H), 3.90 (q, 2H), 2.79 (s, 3H), 2.17-2.09 (m, 1H), 2.05 (quin, 1H), 1.13 (t, 3H).
LC/MS (Methode 3): $R_t$ = 1.03 min, m/z = 299 [M+H]$^+$.

**Beispiel 79A**

3-Ethyl-1-(4-fluorbutyl)-5-methyl-2,4-dioxo-1,2,3,4-tetrahydrothieno[2,3-d]pyrimidin-6-carbaldehyd

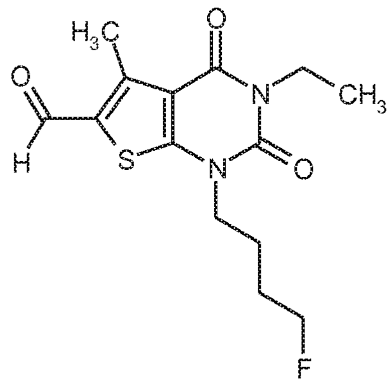

Eine Lösung von 446 mg (1.57 mmol) der Verbindung aus Bsp. 48A in 15 ml DMF wurde mit 543 mg (3.93 mmol) Kaliumcarbonat versetzt und 15 min bei RT gerührt. Dann wurden 497 mg (3.14 mmol) 1-Brom-4-fluoroutan zugesetzt und das Gemisch 21 h bei 50°C gerührt. Das DMF wurde danach weitestgehend abdestilliert und der verbliebene Rückstand zwischen halbgesättigter Natriumchlorid-Lösung (100 ml) und Ethylacetat (150 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 219 mg (44% d. Th.) der Titelverbindung erhalten.
$^1$H-NMR (400 MHz, DMSO-$d_6$, δ/ppm): 10.10 (s, 1H), 4.53 (t, 1H), 4.42 (t, 1H), 3.97 (t, 2H), 3.90 (q, 2H), 2.79 (s, 3H), 1.84-1.74 (m, 3H), 1.74-1.65 (m, 1H), 1.13 (t, 3H).
LC/MS (Methode 3): $R_t$ = 1.11 min, m/z = 313 [M+H]$^+$.

**Beispiel 80A**

3-Ethyl-5-methyl-2,4-dioxo-1-[2-(trifluormethyl)prop-2-en-1-yl]-1,2,3,4-tetrahydrothieno[2,3-d]-pyrimidin-6-carbaldehyd

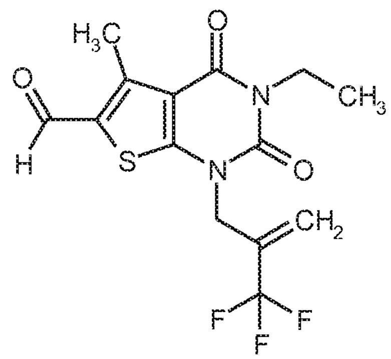

Analog zu dem unter Bsp. 54A beschriebenen Verfahren wurden aus 500 mg (2.10 mmol) der Verbindung aus Bsp. 48A und 595 mg (3.15 mmol) 2-Brommethyl-3,3,3-trifluorpropen 220 mg (30% d. Th.) der Titelverbindung hergestellt. Die Reaktion in der Mikrowelle erfolgte hier bei 80°C mit einer Reaktionszeit von 2 h. Als Laufmittelgradient bei der MPLC-Reinigung wurde Cyclohexan/ Ethylacetat 10:1 → 2:1 verwendet.
$^1$H-NMR (400 MHz, DMSO-$d_6$, δ/ppm): 10.09 (s, 1H), 6.02 (s, 1H), 5.87 (s, 1H), 4.84 (s, 2H), 3.93 (q, 2H), 2.81 (s, 3H), 1.14 (t, 3H).
LC/MS (Methode 1, ESIpos): $R_t$ = 0.96 min, m/z = 347 [M+H]$^+$.

**Beispiel 81A**

1-[(2,2-Difluorcyclopropyl)methyl]-3-ethyl-5-methyl-2,4-dioxo-1-,2,3,4-tetrahydrothieno[2,3-d]-pyrimidin-6-carbaldehyd (*Racemat*)

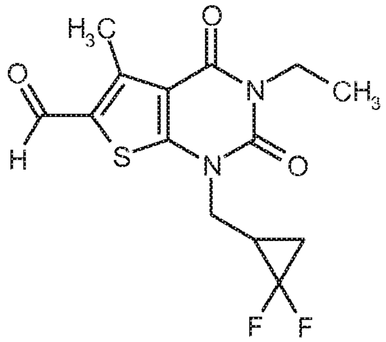

Analog zu dem unter Bsp. 54A beschriebenen Verfahren wurden aus 500 mg (2.10 mmol) der Verbindung aus Bsp. 48A und 538 mg (3.15 mmol) 2-Brommethyl-1,1-difluorcyclopropan 415 mg (60% d. Th.) der Titelverbindung hergestellt. Die Reaktion in der Mikrowelle erfolgte hier bei 100°C mit einer Reaktionszeit von 2 h. Das Produkt wurde mittels MPLC (Biotage-Kartusche, 50 g Kieselgel, Cyclohexan/Ethylacetat 2:1) und anschließendes Verrühren mit Pentan gereinigt.
$^1$H-NMR (400 MHz, DMSO-$d_6$, δ/ppm): 10.10 (s, 1H), 4.20 (ddd, 1H), 4.00 (dd, 1H), 3.91 (q, 2H), 2.80 (s, 3H), 2.30-2.18 (m, 1H), 1.76-1.67 (m, 1H), 1.54-1.46 (m, 1H), 1.14 (t, 3H).
LC/MS (Methode 1, ESIpos): $R_t$ = 0.91 min, m/z = 329 $[M+H]^+$.

## Beispiel 82A

3-Ethyl-5-methyl-2,4-dioxo-1-[2-(trifluormethoxy)ethyl]-1,2,3,4-tetrahydrothieno[2,3-d]pyrimidin-6-carbaldehyd

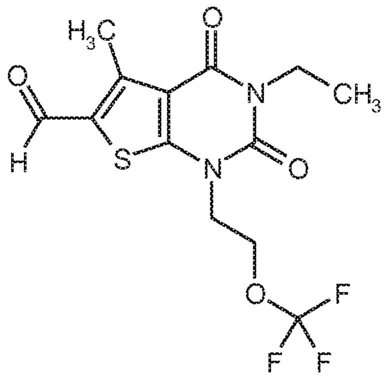

Eine Lösung von 1.16 g (3.61 mmol) der Verbindung aus Bsp. 57A in 2.8 ml (36.1 mmol) DMF wurde mit 4 ml (43.4 mmol) Phosphoroxychlorid versetzt. Nachdem die stark exotherme Reaktion abgeklungen war, wurde das Gemisch noch 30 min ohne weitere Wärmezufuhr nachgerührt. Dann wurde das Reaktionsgemisch vorsichtig in 100 ml eiskaltes Wasser eingerührt. Nach 1 h Rühren wurde das ausgefallene Produkt abgesaugt, mit Wasser neutral gewaschen und getrocknet. Es wurden 1.23 g (94% d. Th., 97% Reinheit) der Titelverbindung erhalten.
$^1$H-NMR (400 MHz, DMSO-$d_6$, δ/ppm): 10.10 (s, 1H), 4.42 (t, 2H), 4.28 (t, 2H), 3.91 (q, 2H), 2.80 (s, 3H), 1.14 (t, 3H).
LC/MS (Methode 1, ESIpos): $R_t$ = 0.99 min, m/z = 351 $[M+H]^+$.

## Beispiel 83A

3-Ethyl-5-methyl-2,4-dioxo-1-(2-[(trifluormethyl)sulfanyl]ethyl)-1,2,3,4-tetrahydrothieno[2,3-d]-pyrimidin-6-carbaldehyd

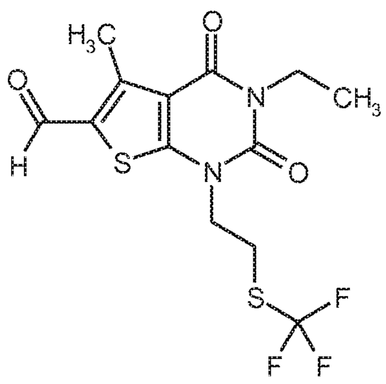

Analog zu dem unter Bsp. 82A beschriebenen Verfahren wurden aus 1.32 g (3.61 mmol) der Verbindung aus Bsp. 58A 1.39 g (97% d. Th.) der Titelverbindung erhalten.
$^1$H-NMR (400 MHz, DMSO-$d_6$, $\delta$/ppm): 10.10 (s, 1H), 4.21 (t, 2H), 3.91 (q, 2H), 3.37 (t, 2H), 2.80 (s, 3H), 1.13 (t, 3H).
LC/MS (Methode 1, ESIpos): $R_t$ = 1.06 min, m/z = 367 $[M+H]^+$.

**Beispiel 84A**

3-Ethyl-1-(2-methoxyethyl)-5-methyl-2,4-dioxo-1,2,3,4-tetrahydrothieno[2,3-d]pyrimidin-6-carbaldehyd

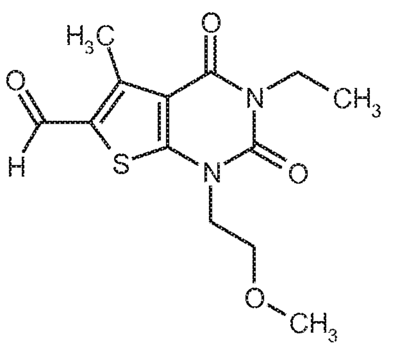

*Methode A:*

200 g (839 mmol) der Verbindung aus Bsp. 48A und 290 g (2.10 mol) Kaliumcarbonat wurden 15 min bei RT in einem Gemisch aus 670 ml DMF und 4 Liter Acetonitril gerührt, bevor 251 ml (2.52 mol) 2-Bromethyl-methylether hinzugefügt wurden. Anschließend wurde das Reaktionsgemisch ca. 18 h bei einer Temperatur von 100°C gerührt. Nach dem Abkühlen auf RT wurde mit Wasser verdünnt und mit Dichlormethan extrahiert. Nach Trocknen des Extrakts über wasserfreiem Magnesiumsulfat wurde filtriert und das Filtrat zur Trockene eingeengt. Das Rohprodukt wurde in 1 Liter Diethylether verrührt. Nach Zusatz von 400 ml Petrolether wurde noch eine Weile weiter gerührt. Dann wurde das Produkt abgesaugt und getrocknet. Es wurden 203 g (81% d. Th.) der Titelverbindung erhalten.

*Methode B:*

Eine Lösung von 7.22 g (26.9 mmol) der Verbindung aus Bsp. 59A in 20.7 ml (269 mmol) DMF wurde mit 30.1 ml (323 mmol) Phosphoroxychlorid versetzt. Nachdem die stark exotherme Reaktion abgeklungen war, wurde das Gemisch noch 60 min ohne weitere Wärmezufuhr nachgerührt. Danach wurde das Reaktionsgemisch vorsichtig in 300 ml eiskaltes Wasser eingerührt. Nach 1 h Rühren wurde das ausgefallene Produkt abgesaugt, mit Wasser neutral gewaschen und getrocknet. Es wurden 7.62 g (88% d. Th., 93% Reinheit) der Titelverbindung erhalten.
$^1$H-NMR (400 MHz, $CDCl_3$, $\delta$/ppm): 10.08 (s, 1H), 4.16 (t, 2H), 4.07 (q, 2H), 3.74 (t, 2H), 3.34 (s, 3H), 2.86 (s, 3H), 1.26 (t, 3H).
LC/MS (Methode 1, ESIpos): $R_t$ = 0.85 min, m/z = 297 $[M+H]^+$.

**Beispiel 85A**

1-(2-Ethoxyethyl)-3-ethyl-5-methyl-2,4-dioxo-1,2,3,4-tetrahydrothieno[2,3-d]pyrimidin-6-carbaldehyd

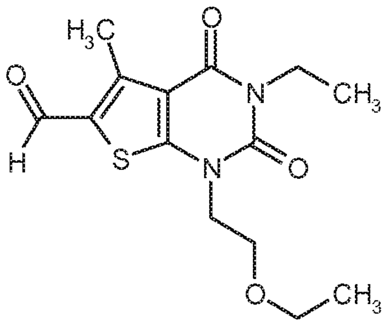

Eine Lösung von 301 mg (1.26 mmol) der Verbindung aus Bsp. 48A in 11 ml DMF wurde mit 437 mg (3.16 mmol) Kaliumcarbonat versetzt und 15 min bei RT gerührt. Dann wurden 653 mg (3.83 mmol) 2-Bromethyl-ethylether zugesetzt und das Gemisch 68 h bei 50°C gerührt. Das DMF wurde danach weitestgehend abdestilliert und der verbliebene Rückstand zwischen halbgesättigter Natriumchlorid-Lösung (70 ml) und Ethylacetat (70 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 224 mg (56% d. Th.) der Titelverbindung erhalten.
$^1$H-NMR (400 MHz, DMSO-$d_6$, δ/ppm): 10.09 (s, 1H), 4.09 (t, 2H), 3.91 (q, 2H), 3.69 (t, 2H), 3.44 (q, 2H), 2.79 (s, 3H), 1.14 (t, 3H), 1.03 (t, 3H).
LC/MS (Methode 3): $R_t$ = 1.1 min, m/z = 311 $[M+H]^+$.

**Beispiel 86A**

3-Ethyl-1-(2-isopropoxyethyl)-5-methyl-2,4-dioxo-1,2,3,4-tetrahydrothieno[2,3-d]pyrimidin-6-carbaldehyd

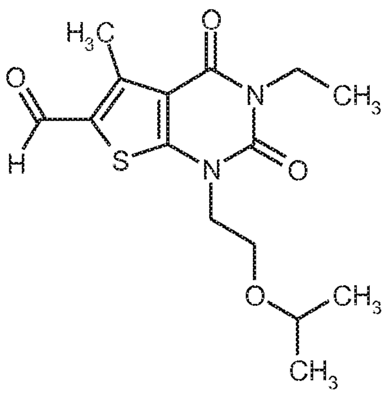

Eine Lösung von 284 mg (1.19 mmol) der Verbindung aus Bsp. 48A in 10.6 ml DMF wurde mit 412 mg (2.98 mmol) Kaliumcarbonat versetzt und 15 min bei RT gerührt. Dann wurden 705 mg (4.22 mmol) 2-(2-Bromethoxy)propan zugesetzt und das Gemisch 38 h bei 50°C gerührt. Das DMF wurde danach weitestgehend abdestilliert und der verbliebene Rückstand zwischen halbgesättigter Natriumchlorid-Lösung (70 ml) und Ethylacetat (70 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Hexan/Ethylacetat). Es wurden 165 mg (41% d. Th.) der Titelverbindung erhalten.
$^1$H-NMR (400 MHz, DMSO-$d_6$, δ/ppm): 10.09 (s, 1H), 4.06 (t, 2H), 3.91 (q, 2H), 3.68 (t, 2H), 3.55 (sept, 1H), 2.79 (s, 3H), 1.13 (t, 3H), 1.00 (d, 6H).

LC/MS (Methode 3): $R_t$ = 1.16 min, m/z = 325 $[M+H]^+$.

**Beispiel 87A**

3-Ethyl-1-(2-methoxypropyl)-5-methyl-2,4-dioxo-1,2,3,4-tetrahydrothieno[2,3-d]pyrimidin-6-carbaldehyd (*Racemat*)

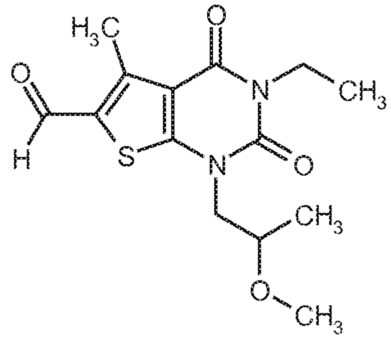

Analog zu dem unter Bsp. 54A beschriebenen Verfahren wurden aus 1.0 g (4.20 mmol) der Verbindung aus Bsp. 48A und 1.54 g (6.30 mmol) racemischem 2-Methoxypropyl-4-methylbenzolsulfonat 327 mg (25% d. Th.) der Titelverbindung hergestellt. Die Reaktion in der Mikrowelle erfolgte hier bei 110°C mit einer Reaktionszeit von 12 h. Als Laufinittel bei der MPLC-Reinigung wurde Cyclohexan/Ethylacetat 4:1 verwendet.
$^1$H-NMR (400 MHz, DMSO-$d_6$, δ/ppm): 10.09 (s, 1H), 4.03 (dd, 1H), 3.91 (q, 2H), 3.81 (dd, 1H), 3.78-3.71 (m, 1H), 3.18 (s, 3H), 2.79 (s, 3H), 1.16 (d, 3H), 1.14 (t, 3H).
LC/MS (Methode 1, ESIpos): $R_t$ = 0.86 min, m/z = 311 $[M+H]^+$.

**Beispiel 88A**

3-Ethyl-5-methyl-1-(oxetan-2-ylmethyl)-2,4-dioxo-1,2,3,4-tetrahydrothieno[2,3-d]pyrimidin-6-carbaldehyd *(Racemat)*

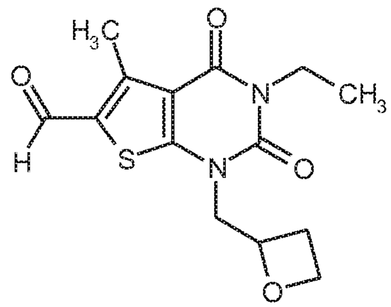

Analog zu dem unter Bsp. 86A beschriebenen Verfahren wurden aus 645 mg (2.46 mmol) der Verbindung aus Bsp. 48A und 1.48 g (9.48 mmol) racemischem 2-(Brommethyl)oxetan 759 mg (92% d. Th.) der Titelverbindung hergestellt. Die Umsetzung wurde hier bei 80°C durchgeführt.
$^1$H-NMR (400 MHz, DMSO-$d_6$, δ/ppm): 10.09 (s, 1H), 5.06-4.98 (m, 1H), 4.52-4.42 (m, 2H), 4.28-4.16 (m, 2H), 3.91 (q, 2H), 2.78 (s, 3H), 2.74-2.66 (m, 1H), 1.14 (t, 3H).
LC/MS (Methode 3): $R_t$ = 0.96 min, m/z = 309 $[M+H]^+$.

**Beispiel 89A**

3-Ethyl-5-methyl-2,4-dioxo-1-(tetrahydrofuran-2-ylmethyl)-1,2,3,4-tetrahydrothieno[2,3-d]-pyrimidin-6-carbaldehyd (*Racemat*)

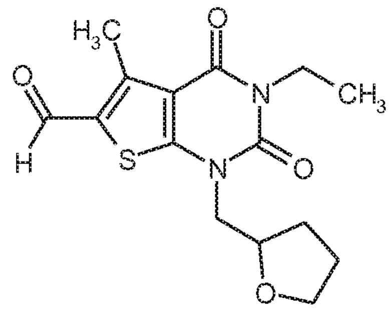

*Methode A:*

Analog zu dem unter Bsp. 86A beschriebenen Verfahren wurden aus 400 mg (1.66 mmol) der Verbindung aus Bsp. 48A und 1.44 g (8.31 mmol) racemischem 2-(Brommethyl)tetrahydrofuran 350 mg (53% d. Th.) der Titelverbindung hergestellt. Die Umsetzung wurde hier bei 80°C durchgeführt.

*Methode B:*

Eine Lösung von 1.08 g (3.68 mmol) der Verbindung aus Bsp. 60A in 2.8 ml (36.8 mmol) DMF wurde mit 4.1 ml (44.2 mmol) Phosphoroxychlorid versetzt. Nachdem die stark exotherme Reaktion abgeklungen war, wurde das Gemisch noch 15 min ohne weitere Wärmezufuhr nachgerührt. Danach wurde das Reaktionsgemisch vorsichtig in 100 ml eiskaltes Wasser eingerührt. Nach 1 h Rühren wurde das ausgefallene Produkt abgesaugt, mit Wasser neutral gewaschen und getrocknet. Es wurden 1.08 g (83% d. Th., 92% Reinheit) der Titelverbindung erhalten.
$^1$H-NMR (400 MHz, DMSO-$d_6$, δ/ppm): 10.09 (s, 1H), 4.27-4.20 (m, 1H), 4.13 (dd, 1H), 3.91 (q, 2H), 3.80-3.72 (m, 2H), 3.62 (dd, 1H), 2.78 (s, 3H), 2.05-1.96 (m, 1H), 1.95-1.77 (m, 2H), 1.72-1.64 (m, 1H), 1.14 (t, 3H).
LC/MS (Methode 1, ESIpos): $R_t$ = 0.89 min, m/z = 323 $[M+H]^+$.

**Beispiel 90A**

3-Ethyl-5-methyl-2,4-dioxo-1-(tetrahydro-2H-pyran-2-ylmethyl)-1,2,3,4-tetrahydrothieno[2,3-d]-pyrimidin-6-carbaldehyd (*Racemat*)

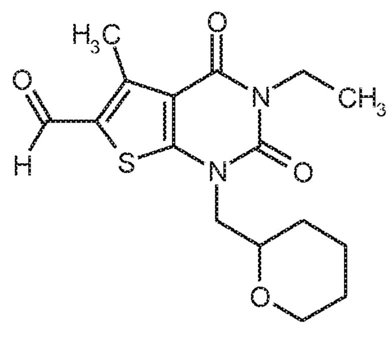

Analog zu dem unter Bsp. 86A beschriebenen Verfahren wurden aus 740 mg (2.86 mmol) der Verbindung aus Bsp. 48A und 2.08 g (11.43 mmol) racemischem 2-(Brommethyl)tetrahydro-2*H*-pyran 590 mg (61% d. Th.) der Titelverbindung hergestellt. Die Umsetzung wurde hier bei 70°C durchgeführt und die Reaktionsdauer betrug 43 h.
$^1$H-NMR (400 MHz, DMSO-$d_6$, δ/ppm): 10.08 (s, 1H), 4.05 (dd, 1H), 3.90 (q, 2H), 3.84-3.74 (m, 2H), 3.72-3.64 (m, 1H), 3.26 (dt, 1H), 2.77 (s, 3H), 1.78 (d, 1H), 1.65 (d, 1H), 1.50-1.37 (m, 3H), 1.36-1.21 (m, 1H), 1.13 (t, 3H).
LC/MS (Methode 3): $R_t$ = 1.21 min, m/z = 337 $[M+H]^+$.

**Beispiel 91A**

3-Ethyl-5-methyl-1-(oxetan-3-ybnethyl)-2,4-dioxo-1,2,3,4-tetrahydrothieno[2,3-d]pyrimidin-6-carbaldehyd

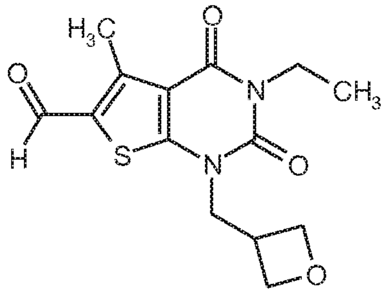

Analog zu dem unter Bsp. 54A beschriebenen Verfahren wurden aus 500 mg (2.10 mmol) der Verbindung aus Bsp. 48A und 335 mg (3.15 mmol) 3-(Chlormethyl)oxetan 85 mg (13% d. Th.) der Titelverbindung hergestellt. Die Reaktion in der Mikrowelle erfolgte hier bei 60°C mit einer Reaktionszeit von 2 h. Die MPLC-Reinigung wurde mit einer 50 g Kieselgel-Kartusche und Cyclohexan/Ethylacetat 2:1 als Laufmittel durchgeführt.
$^1$H-NMR (400 MHz, DMSO-$d_6$, δ/ppm): 10.09 (s, 1H), 4.61 (dd, 2H), 4.45 (t, 2H), 4.27 (d, 2H), 3.89 (q, 2H), 3.45 (sept, 1H), 2.79 (s, 3H), 1.12 (t, 3H).
LC/MS (Methode 1, ESIpos): $R_t$ = 0.70 min, m/z = 309 $[M+H]^+$.

**Beispiel 92A**

3-Ethyl-5-methyl-2,4-dioxo-1-(tetrahydrofuran-3-ylmethyl)-1,2,3,4-tetrahydrothieno[2,3-d]-pyrimidin-6-carbaldehyd (*Racemat*)

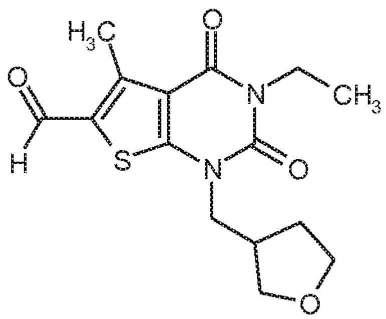

Analog zu dem unter Bsp. 54A beschriebenen Verfahren wurden aus 500 mg (2.10 mmol) der Verbindung aus Bsp. 48A und 519 mg (3.15 mmol) racemischem 3-(Brommethyl)tetrahydrofuran 188 mg (27% d. Th.) der Titelverbindung hergestellt. Die Reaktion in der Mikrowelle erfolgte hier zunächst bei 60°C (1 h), dann bei 80°C (5 h) und schließlich bei 100°C (3 h). Die Reinigung des Produktes wurde mittels präparativer HPLC (Methode 8) durchgeführt.
$^1$H-NMR (400 MHz, DMSO-$d_6$, δ/ppm): 10.10 (s, 1H), 3.95 (dd, 2H), 3.91 (q, 2H), 3.84-3.78 (m, 1H), 3.69-3.60 (m, 2H), 3.51 (dd, 1H), 2.80 (s, 3H), 2.79-2.70 (m, 1H), 2.02-1.93 (m, 1H), 1.71-1.63 (m, 1H), 1.14 (t, 3H).
LC/MS (Methode 1, ESIpos): $R_t$ = 0.78 min, m/z = 323 $[M+H]^+$.

**Beispiel 93A**

3-Ethyl-1,5-dimethyl-2,4-dioxo-1,2,3,4-tetrahydrothieno[2,3-d]pyrimidin-6-carbaldehyd

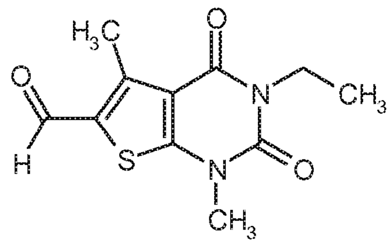

500 mg (2.10 mmol) der Verbindung aus Bsp. 48A und 1.03 g (3.15 mmol) Cäsiumcarbonat wurden 10 min bei RT in 8 ml wasserfreiem DMF gerührt, bevor 300 µl (3.15 mmol) Dimethylsulfat hinzugefügt wurden. Anschließend wurde das Reaktionsgemisch in einem Mikrowellenofen (Biotage Initiator mit dynamischer Steuerung der Einstrahlleistung) 1 h lang auf 60°C erwärmt. Nach dem Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und zur Trockene eingeengt. Der erhaltene Rückstand wurde mit Pentan verrührt. Nach Absaugen und Trocknen im Hochvakuum wurden 467 mg (88% d. Th.) der Titelverbindung erhalten.
$^1$H-NMR (400 MHz, DMSO-$d_6$, δ/ppm): 10.09 (s, 1H), 3.90 (q, 2H), 3.47 (s, 3H), 2.79 (s, 3H), 1.13 (t, 3H).
LC/MS (Methode 1, ESIpos): $R_t$ = 0.76 min, m/z = 253 $[M+H]^+$.

**Beispiel 94A**

1,3-Diethyl-5-methyl-2,4-dioxo-1,2,3,4-tetrahydrothieno[2,3-d]pyrimidin-6-carbaldehyd

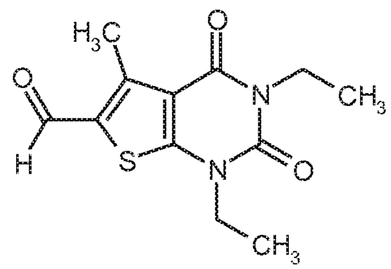

Analog zu dem unter Bsp. 54A beschriebenen Verfahren wurden aus 500 mg (2.10 mmol) der Verbindung aus Bsp. 48A und 252 µl (3.15 mmol) Iodethan 476 mg (85% d. Th.) der Titelverbindung erhalten. Die Reaktion in der Mikrowelle erfolgte hier für 60 min bei 60°C, und die MPLC-Reinigung wurde mit einer 50 g Kieselgel enthaltenden Biotage-Kartusche und Cyclohexan/Ethylacetat 5:1 als Laufmittel durchgeführt.
$^1$H-NMR (400 MHz, DMSO-$d_6$, δ/ppm): 10.09 (s, 1H), 3.97 (q, 2H), 3.90 (q, 2H), 2.79 (s, 3H), 1.26 (t, 3H), 1.14 (t, 3H).
LC/MS (Methode 1, ESIpos): $R_t$ = 0.87 min, m/z = 267 $[M+H]^+$.

**Beispiel 95A**

3-Ethyl-5-methyl-2,4-dioxo-1-propyl-1,2,3,4-tetrahydrothieno[2,3-d]pyrimidin-6-carbaldehyd

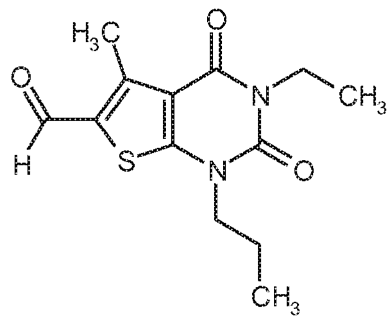

Analog zu dem unter Bsp. 89A (Methode B) beschriebenen Verfahren wurden aus 1.04 g (4.12 mmol) der Verbindung aus Bsp. 61A 1.08 g (93% d. Th.) der Titelverbindung erhalten.
$^1$H-NMR (400 MHz, DMSO-$d_6$, $\delta$/ppm): 10.09 (s, 1H), 3.97-3.84 (m, 4H), 2.79 (s, 3H), 1.72 (sext, 2H), 1.13 (t, 3H), 0.93 (t, 3H).
LC/MS (Methode 1, ESIpos): $R_t$ = 0.96 min, m/z = 281 $[M+H]^+$.

**Beispiel 96A**

1-Butyl-3-ethyl-5-methyl-2,4-dioxo-1,2,3,4-tetrahydrothieno[2,3-d]pyrimidin-6-carbaldehyd

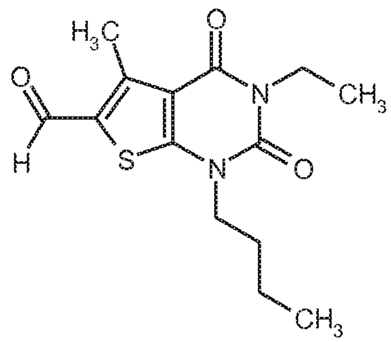

Analog zu dem unter Bsp. 54A beschriebenen Verfahren wurden aus 500 mg (2.10 mmol) der Verbindung aus Bsp. 48A und 362 µl (3.15 mmol) 1-Iodbutan 327 mg (52% d. Th.) der Titelverbindung erhalten. Die Reaktion in der Mikrowelle erfolgte hier für 60 min bei 60°C, und die MPLC-Reinigung wurde über eine Biotage-Kartusche mit 50 g Kieselgel und Cyclohexan/Ethylacetat 6:1 als Laufmittel durchgeführt.
$^1$H-NMR (400 MHz, DMSO-$d_6$, $\delta$/ppm): 10.09 (s, 1H), 3.93 (t, 2H), 3.90 (q, 2H), 2.79 (s, 3H), 1.67 (quin, 2H), 1.36 (sext, 2H), 1.13 (t, 3H), 0.92 (t, 3H).
LC/MS (Methode 1, ESIpos): $R_t$ = 1.03 min, m/z = 295 $[M+H]^+$.

**Beispiel 97A**

3-Ethyl-5-methyl-1-(3-methylbutyl)-2,4-dioxo-1,2,3,4-tetrahydiothieno[2,3-d]pyrimidin-6-carbaldehyd

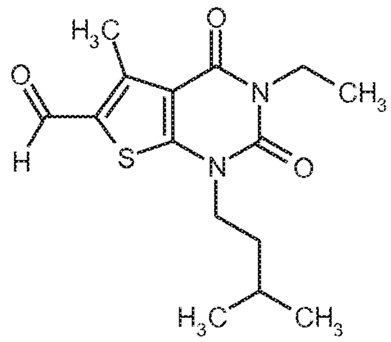

Analog zu dem unter Bsp. 54A beschriebenen Verfahren wurden aus 500 mg (2.10 mmol) der Verbindung aus Bsp. 48A und 414 µl (3.15 mmol) 1-Iod-3-methylbutan 398 mg (59% d. Th., 97% Reinheit) der Titelverbindung erhalten. Die Reaktion in der Mikrowelle erfolgte hier für 60 min bei 60°C, und die MPLC-Reinigung wurde über eine Biotage-Kartusche mit 50 g Kieselgel und Cyclohexan/Ethylacetat 10:1 → 5:1 als Laufmittel durchgeführt.
$^1$H-NMR (400 MHz, DMSO-$d_6$, δ/ppm): 10.09 (s, 1H), 3.93 (t, 2H), 3.90 (q, 2H), 2.79 (s, 3H), 1.72-1.61 (m, 1H), 1.61-1.52 (m, 2H), 1.13 (t, 3H), 0.95 (d, 6H).
LC/MS (Methode 1, ESIpos): $R_t$ = 1.11 min, m/z = 309 $[M+H]^+$.

**Beispiel 98A**

1-(3,3-Dimethylbutyl)-3-ethyl-5-methyl-2,4-dioxo-1,2,3,4-tetrahydrothieno[2,3-d]pyrimidin-6-carbaldehyd

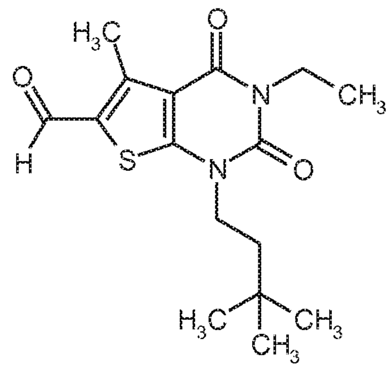

Analog zu dem unter Bsp. 86A beschriebenen Verfahren wurden aus 300 mg (1.12 mmol) der Verbindung aus Bsp. 48A und 636 mg (3.77 mmol) 1-Brom-3,3-dimethylbutan 273 mg (67% d. Th.) der Titelverbindung hergestellt. Die Umsetzung wurde hier bei 80°C durchgeführt.
$^1$H-NMR (400 MHz, DMSO-$d_6$, δ/ppm): 10.09 (s, 1H), 3.96-3.87 (m, 4H), 2.79 (s, 3H), 1.60-1.53 (m, 2H), 1.13 (t, 3H), 0.98 (s, 9H).
LC/MS (Methode 3): $R_t$ = 1.4 min, m/z = 323 $[M+H]^+$.

**Beispiel 99A**

1-(Cyclobutylmethyl)-3-ethyl-5-methyl-2,4-dioxo-1,2,3,4-tetrahydrothieno[2,3-d]pyrimidin-6-carbaldehyd

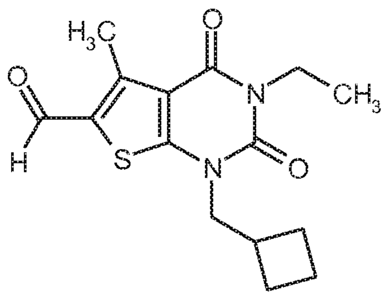

Analog zu dem unter Bsp. 54A beschriebenen Verfahren wurden aus 500 mg (2.10 mmol) der Verbindung aus Bsp. 48A und 469 mg (3.15 mmol) (Brommethyl)cyclobutan 308 mg (47% d. Th.) der Titelverbindung erhalten. Die Reaktion in der Mikrowelle erfolgte hier für 2 h bei 100°C, und die MPLC-Reinigung wurde über eine Biotage-Kartusche mit 50 g Kieselgel und Cyclohexan/Ethylacetat 2:1 als Laufmittel durchgeführt.
$^1$H-NMR (400 MHz, DMSO-$d_6$, $\delta$/ppm): 10.09 (s, 1H), 4.00 (d, 2H), 3.90 (q, 2H), 2.84-2.73 (m, 1H), 2.79 (s, 3H), 2.04-1.92 (m, 2H), 1.90-1.75 (m, 4H), 1.13 (t, 3H).
LC/MS (Methode 1, ESIpos): $R_t$ = 1.03 min, m/z = 307 $[M+H]^+$.

**Beispiel 100A**

(3-Ethyl-6-formyl-5-methyl-2,4-dioxo-3,4-dihydrothieno[2,3-d]pyrimidin-1(2*H*)-yl)acetonitril

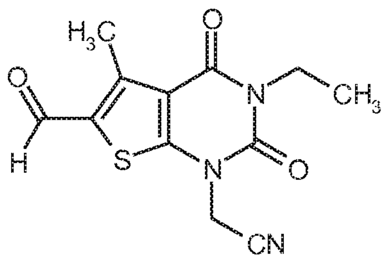

Analog zu dem unter Bsp. 54A beschriebenen Verfahren wurden aus 920 mg (3.86 mmol) der Verbindung aus Bsp. 48A und 1.39 g (11.6 mmol) Bromacetonitril 550 mg (51% d. Th.) der Titelverbindung erhalten. Die Reaktion in der Mikrowelle erfolgte hier für 60 min bei 50°C, und die MPLC-Reinigung wurde über eine Biotage-Kartusche mit 50 g Kieselgel und Cyclohexan/Ethylacetat 2:1 als Laufmittel durchgeführt.
$^1$H-NMR (400 MHz, DMSO-$d_6$, $\delta$/ppm): 10.12 (s, 1H), 5.22 (s, 2H), 3.91 (q, 2H), 2.80 (s, 3H), 1.15 (t, 3H).
LC/MS (Methode 1, ESIpos): $R_t$ = 0.76 min, m/z = 278 $[M+H]^+$.

**Beispiel 101A**

3-Ethyl-5-methyl-1-[(4-methyl-1,2,5-oxadiazol-3-yl)methyl]-2,4-dioxo-1,2,3,4-tetrahydrothieno-[2,3-d]pyrimidin-6-carbaldehyd

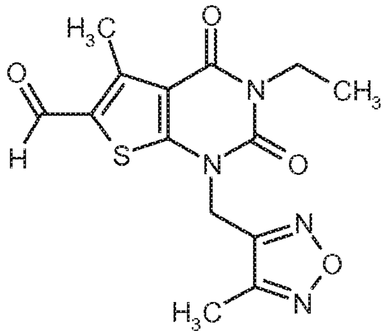

Analog zu dem unter Bsp. 54A beschriebenen Verfahren wurden aus 500 mg (2.10 mmol) der Verbindung aus Bsp. 48A und 417 mg (3.15 mmol) 3-(Chlormethyl)-4-methyl-1,2,5-oxadiazol 389 mg (55% d. Th.) der Titelverbindung erhalten. Die Reaktion in der Mikrowelle erfolgte hier für 2 h bei 100°C, und die MPLC-Reinigung wurde über eine Biotage-Kartusche mit 50 g Kieselgel und Cyclohexan/Ethylacetat 3:1 als Laufmittel durchgeführt.
$^1$H-NMR (400 MHz, DMSO-$d_6$, $\delta$/ppm): 10.09 (s, 1H), 5.42 (s, 2H), 3.92 (q, 2H), 2.80 (s, 3H), 2.44 (s, 3H), 1.14 (t, 3H).
LC/MS (Methode 1, ESIpos): $R_t$ = 0.91 min, m/z = 335 $[M+H]^+$.

## Beispiel 102A

1-[2-(Dimethylamino)ethyl]-3-ethyl-5-methyl-2,4-dioxo-1,2,3,4-tetrahydrothieno[2,3-d]pyrimidin-6-carbaldehyd

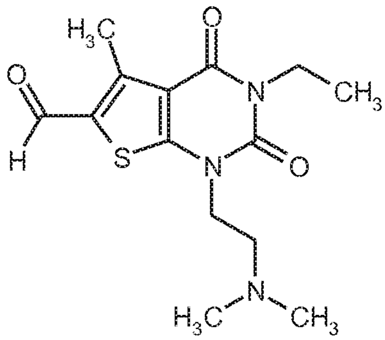

Analog zu dem unter Bsp. 54A beschriebenen Verfahren wurden aus 500 mg (2.10 mmol) der Verbindung aus Bsp. 48A und 733 mg (3.15 mmol) 2-Brom-*N,N*-dimethylethanamin-Hydrobromid 453 mg (69% d. Th.) der Titelverbindung erhalten. Die Reaktion in der Mikrowelle erfolgte hier für 2 h bei 100°C, und die MPLC-Reinigung wurde über eine Biotage-Kartusche mit 50 g Kieselgel und Cyclohexan/Ethylacetat 1:1 als Laufmittel durchgeführt.
$^1$H-NMR (400 MHz, DMSO-$d_6$, $\delta$/ppm): 10.09 (s, 1H), 4.00 (t, 2H), 3.91 (q, 2H), 2.79 (s, 3H), 2.58 (t, 2H), 2.20 (s, 6H), 1.13 (t, 3H).
LC/MS (Methode 1, ESIpos): $R_t$ = 0.39 min, m/z = 310 $[M+H]^+$.

## Beispiel 103A

3-Isopropyl-5-methyl-2,4-dioxo-1-(3,3,3-trifluorpropyl)-1,2,3,4-tetrahydrothieno[2,3-d]pyrimidin-6-carbaldehyd

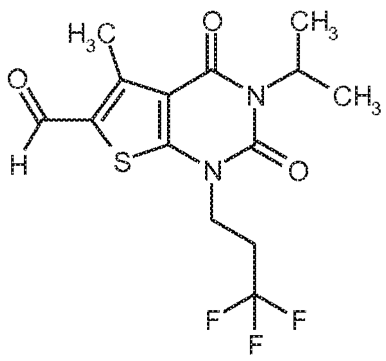

*Methode A:*

1.8 g (7.13 mmol) der Verbindung aus Bsp. 49A und 3.49 g (10.7 mmol) Cäsiumcarbonat wurden 10 min bei RT in 15 ml DMF gerührt, bevor 1.3 ml (10.7 mmol) 1,1,1-Trifluor-3-iodpropan hinzugefügt wurden. Anschließend wurde das Reaktionsgemisch in einem Mikrowellenofen (Biotage-Initiator mit dynamischer Steuerung der Einstrahlleistung) bei einer Temperatur von 100°C gerührt. Nach 2 h wurden weitere 167 µl (1.43 mmol) 1,1,1-Trifluor-3-iodpropan hinzugefügt und das Erhitzen 30 min fortgesetzt. Nach dem Abkühlen auf RT wurde mit ca. 75 ml Ethylacetat verdünnt und nacheinander mit Wasser und gesättigter Natriumchlorid-Lösung gewaschen. Nach Trocknen über wasserfreiem Magnesiumsulfat wurde filtriert und das Filtrat zur Trockene eingeengt. Das Rohprodukt wurde durch MPLC gereinigt (Biotage-Kartusche, 100 g Kieselgel SNAP KP-Sil, Laufmittel: Cyclohexan/Ethylacetat 1:2). Nach Eindampfen und Trocknen wurde ein Gemisch von *N*- und *O*-alkyliertem Produkt erhalten, aus dem das *N*-alkylierte Hauptprodukt durch Verrühren mit einem Gemisch aus 30 ml Pentan und 2 ml Dichlormethan als Feststoff gewonnen werden konnte. Weiteres *N*-alkyliertes Produkt wurde aus der aufkonzentrierten Mutterlauge des Verrührens mittels präparativer HPLC (Methode 8) erhalten. Nach Eindampfen der Produktfraktionen und Trocknen im Hochvakuum wurden zusammen mit dem Feststoff aus dem Verrühren 1.86 g (74% d. Th.) der Titelverbindung erhalten.

*Methode B:*

Eine Lösung von 508 mg (1.59 mmol) der Verbindung aus Bsp. 62A in 1.2 ml (15.9 mmol) DMF wurde zügig mit 1.8 ml (19.0 mmol) Phosphoroxychlorid versetzt. Nachdem die stark exotherme Reaktion abgeklungen war, wurde das Gemisch noch 15 min bei RT nachgerührt. Dann wurde das Reaktionsgemisch vorsichtig in 100 ml Wasser eingerührt. Nach 1 h Rühren wurde das ausgefallene Produkt abgesaugt, mit Wasser neutral gewaschen und getrocknet. Es wurden 490 mg (88% d. Th.) der Titelverbindung erhalten.

$^1$H-NMR (400 MHz, DMSO-$d_6$, δ/ppm): 10.10 (s, 1H), 5.11 (sept, 1H), 4.15 (t, 2H), 2.87-2.69 (m, 2H), 2.79 (s, 3H), 1.41 (d, 6H).

LC/MS (Methode 1, ESIpos): $R_t$ = 1.04 min, m/z = 349 $[M+H]^+$.

**Beispiel 104A**

1-(2-Fluorethyl)-3-isopropyl-5-methyl-2,4-dioxo-1,2,3,4-tetrahydrothieno[2,3-d]pyrimidin-6-carbaldehyd

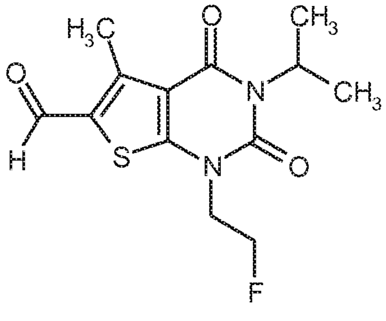

Analog zu dem unter Bsp. 89A (Methode B) beschriebenen Verfahren wurden aus 450 mg (1.66 mmol) der

Verbindung aus Bsp. 63A 422 mg (85% d. Th.) der Titelverbindung erhalten.
$^1$H-NMR (400 MHz, DMSO-$d_6$, $\delta$/ppm): 10.09 (s, 1H), 5.12 (sept, 1H), 4.86-4.63 (dt, 2H), 4.37-4.15 (dt, 2H), 2.78 (s, 3H), 1.42 (d, 6H).
LC/MS (Methode 1, ESIpos): $R_t$ = 0.93 min, m/z = 299 $[M+H]^+$.

**Beispiel 105A**

3-Isopropyl-1-(2-methoxyethyl)-5-methyl-2,4-dioxo-1,2,3,4-tetrahydrothieno[2,3-d]pyrimidin-6-carbaldehyd

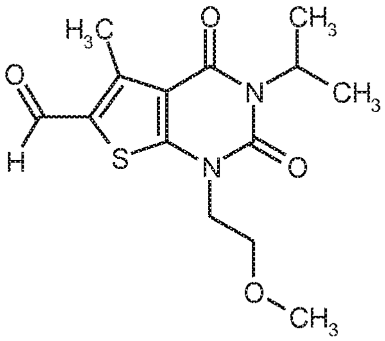

Analog zu dem unter Bsp. 54A beschriebenen Verfahren wurden aus 1.80 g (7.13 mmol) der Verbindung aus Bsp. 49A und 1.49 g (10.7 mmol) 2-Bromethyl-methylether 0.86 g (37% d. Th., 97% Reinheit) der Titelverbindung hergestellt. Die Reaktion in der Mikrowelle erfolgte hier für 2 h bei 100°C, und bei der MPLC-Reinigung wurde Cyclohexan/Ethylacetat 1:2 als Laufmittel verwendet.
$^1$H-NMR (400 MHz, DMSO-$d_6$, $\delta$/ppm): 10.08 (s, 1H), 5.11 (sept, 1H), 4.07 (t, 2H), 3.64 (t, 2H), 3.31 (s, 3H), 2.77 (s, 3H), 1.41 (d, 6H).
LC/MS (Methode 1, ESIpos): $R_t$ = 0.94 min, m/z = 311 $[M+H]^+$.

**Beispiel 106A**

3-Isopropyl-5-methyl-1-(oxetan-2-ylmethyl)-2,4-dioxo-1,2,3,4-tetrahydrothieno[2,3-d]pyrimidin-6-carbaldehyd (*Racemat*)

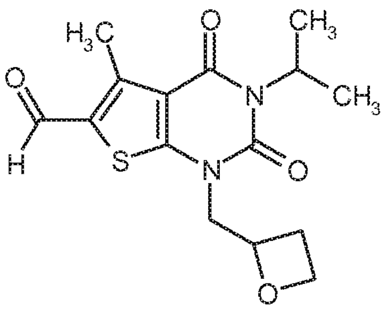

Analog zu dem unter Bsp. 54A beschriebenen Verfahren wurden aus 3.60 g (14.3 mmol) der Verbindung aus Bsp. 49A und 3.02 g (20.0 mmol) racemischem 2-(Chlormethyl)oxetan 2.40 g (50% d. Th., 97% Reinheit) der Titelverbindung hergestellt. Die Reaktion in der Mikrowelle erfolgte hier bei 100°C, und bei der MPLC-Reinigung wurde Cyclohexan/Ethylacelat 1:2 als Laufmittel verwendet.
$^1$H-NMR (400 MHz, DMSO-$d_6$, $\delta$/ppm): 10.08 (s, 1H), 5.12 (sept, 1H), 5.05-4.97 (m, 1H), 4.53-4.39 (m, 2H), 4.22-4.16 (m, 2H), 2.77 (s, 3H), 2.75-2.65 (m, 1H), 2.50-2.43 (m, 1H, teilweise überdeckt vom DMSO-Signal), 1.41 (d, 6H).
LC/MS (Methode 1, ESIpos): $R_t$ = 0.87 min, m/z = 323 $[M+H]^+$.

**Beispiel 107A**

3-Isopropyl-5-methyl-2,4-dioxo-1-(tetrahydrofuran-2-ylmethyl)-1,2,3,4-tetrahydrothieno[2,3-d]-pyrimidin-6-carbaldehyd (*Racemat*)

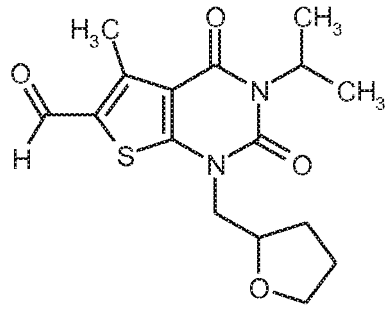

Analog zu dem unter Bsp. 89A (Methode B) beschriebenen Verfahren wurden aus 431 mg (1.40 mmol) der Verbindung aus Bsp. 64A 358 mg (70% d. Th., 93% Reinheit) der Titelverbindung erhalten.
$^1$H-NMR (400 MHz, DMSO-$d_6$, δ/ppm): 10.08 (s, 1H), 5.12 (sept, 1H), 4.28-4.18 (m, 1H), 4.12 (dd, 1H), 3.80-3.67 (m, 2H), 3.66-3.57 (m, 1H), 2.77 (s, 3H), 2.09-1.75 (m, 3H), 1.73-1.61 (m, 1H), 1.41 (dd, 6H).
LC/MS (Methode 1, ESIpos): $R_t$ = 1.01 min, m/z = 337 [M+H]$^+$.

**Beispiel 108A**

3-Isopropyl-5-methyl-1-(oxetan-3-ylmethyl)-2,4-dioxo-1,2,3,4-tetrahydrothieno[2,3-d]pyrimidin-6-carbaldehyd

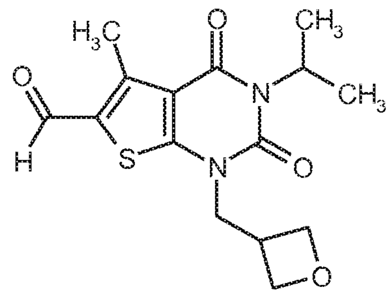

119 mg (0.473 mmol) der Verbindung aus Bsp. 49A, 231 mg (0.709 mmol) Cäsiumcarbonat und 39 mg (0.236 mmol) Kaliumiodid wurden 10 min bei RT in 2.1 ml wasserfreiem DMF gerührt, bevor 65 μl (0.709 mmol) 3-(Chlormethyl)oxetan hinzugefügt wurden. Anschließend wurde das Reaktionsgemisch in einem Mikrowellenofen (Biotage Initiator mit dynamischer Steuerung der Einstrahlleistung) 1.5 h lang auf 80°C erwärmt. Nach Abkühlen auf RT wurde mit Ethylacetat verdünnt und nacheinander mit Wasser und gesättigter Natriumchlorid-Lösung gewaschen. Nach Trocknen über wasserfreiem Magnesiumsulfat wurde filtriert und zur Trockene eingeengt. Es wurden 149 mg (96% d. Th., 98% Reinheit) der Titelverbindung erhalten.
$^1$H-NMR (400 MHz, DMSO-$d_6$, δ/ppm): 10.09 (s, 1H), 5.10 (sept, 1H), 4.61 (dd, 2H), 4.44 (t, 2H), 4.24 (d, 2H), 3.48-3.38 (m, 1H), 2.77 (s, 3H), 1.40 (d, 6H).
LC/MS (Methode 1, ESIpos): $R_t$ = 0.87 min, m/z = 323 [M+H]$^+$.

**Beispiel 109A**

3-Isopropyl-1,5-dimethyl-2,4-dioxo-1,2,3,4-tetrahydrothieno[2,3-d]pyrimidin-6-carbaldehyd

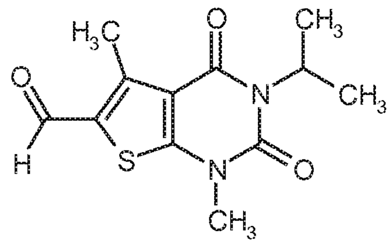

Analog zu dem unter Bsp. 89A (Methode B) beschriebenen Verfahren wurden aus 450 mg (1.89 mmol) der Verbindung aus Bsp. 65A 408 mg (81% d. Th.) der Titelverbindung erhalten.
$^1$H-NMR (400 MHz, DMSO-$d_6$, $\delta$/ppm): 10.08 (s, 1H), 5.13 (sept, 1H), 3.44 (s, 3H), 2.78 (s, 3H), 1.41 (d,6H).
LC/MS (Methode 1, ESIpos): $R_t$ = 0.86 min, m/z = 267 $[M+H]^+$.

**Beispiel 110A**

3-Isobutyl-5-methyl-2,4-dioxo-1-(3,3,3-trifluorpropyl)-1,2,3,4-tetrahydrothieno[2,3-d]pyrimidin-6-carbaldehyd

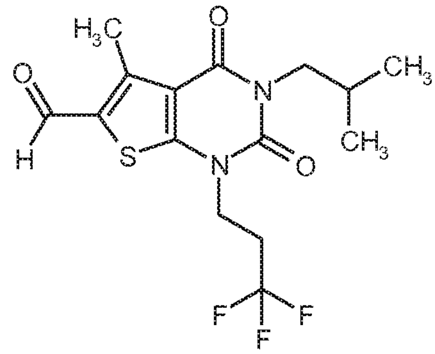

Eine Lösung von 2.39 g (7.07 mmol) der Verbindung aus Bsp. 66A in 7.68 ml DMF wurde unter Eisbadkühlung vorsichtig mit 15.37 ml (164.92 mmol) Phosphoroxychlorid versetzt. Nach Abklingen der exothermen Reaktion wurde das Gemisch auf RT gekühlt. Danach wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 2.56 g (99% d. Th.) der Titelverbindung erhalten.
$^1$H-NMR (400 MHz, DMSO-$d_6$, $\delta$/ppm): 10.11 (s, 1H), 4.18 (t, 2H), 3.72 (d, 2H), 2.87-2.72 (m, 5H), 2.03 (dquin, 1H), 0.86 (d, 6H).
LC/MS (Methode 3): $R_t$ = 1.33 min, m/z = 363 $[M+H]^+$.

**Beispiel 111A**

1-(2,2-Difluorethyl)-3-isobutyl-5-methyl-2,4-dioxo-1,2,3,4-tetrahydrothieno[2,3-d]pyrimidin-6-carbaldehyd

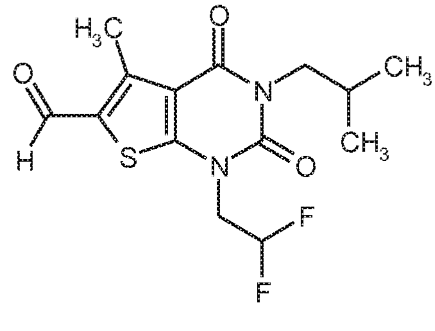

Analog zu dem unter Bsp. 86A beschriebenen Verfahren wurden aus 450 mg (1.69 mmol) der Verbindung aus Bsp. 50A und 1.29 g (6.76 mmol) 1,1-Difluor-2-iodethan 190 mg (34% d. Th.) der Titelverbindung hergestellt. Die Umsetzung wurde hier bei 80°C durchgeführt und die Reaktionsdauer betrug 40 h.
$^1$H-NMR (400 MHz, DMSO-$d_6$, δ/ppm): 10.11 (s, 1H), 6.52-6.21 (m, 1H), 4.43 (td, 2H), 3.71 (d, 2H), 2.78 (s, 3H), 2.10-1.96 (m, 1H), 0.87 (d, 6H).
LC/MS (Methode 3): $R_t$ = 1.23 min, m/z = 331 $[M+H]^+$.

**Beispiel 112A**

1-(2-Fluorethyl)-3-isobutyl-5-methyl-2,4-dioxo-1,2,3,4-tetrahydrothieno[2,3-d]pyrimidin-6-carbaldehyd

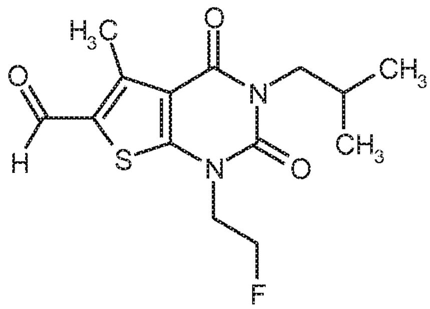

Analog zu dem unter Bsp. 89A (Methode B) beschriebenen Verfahren wurden aus 443 mg (1.56 mmol) der Verbindung aus Bsp. 67A 462 mg (94% d. Th.) der Titelverbindung erhalten.
$^1$H-NMR (400 MHz, DMSO-$d_6$, δ/ppm): 10.10 (s, 1H), 4.86-4.63 (dt, 2H), 4.36-4.18 (dt, 2H), 3.72 (d, 2H), 2.79 (s, 3H), 2.04 (m, 1H), 0.87 (d, 6H).
LC/MS (Methode 1, ESIpos): $R_t$ = 0.98 min, m/z = 313 $[M+H]^+$.

**Beispiel 113A**

1-(3-Fluorpropyl)-3-isobutyl-5-methyl-2,4-dioxo-1,2,3,4-teh-ahydrothieno[2,3-d]pyrimidin-6-carbaldehyd

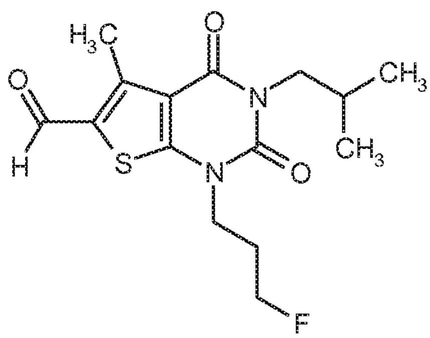

Analog zu dem unter Bsp. 86A beschriebenen Verfahren wurden aus 490 mg (1.84 mmol) der Verbindung aus Bsp. 50A und 1.03 g (5.52 mmol) 1-Fluor-3-iodpropan 571 mg (91% d. Th.) der Titelverbindung hergestellt. Die Reaktionsdauer betrug hier 17 h.
$^1$H-NMR (400 MHz, DMSO-$d_6$, δ/ppm): 10.10 (s, 1H), 4.60 (t, 1H), 4.48 (t, 1H), 4.05 (t, 2H), 3.71 (d, 2H), 2.78 (s, 3H), 2.16-2.09 (m, 1H), 2.09-1.97 (m, 2H), 0.86 (d, 6H).
LC/MS (Methode 3): $R_t$ = 1.23 min, m/z = 327 $[M+H]^+$.

**Beispiel 114A**

3-Isobutyl-1-(2-methoxyethyl)-5-methyl-2,4-dioxo-1,2,3,4-tetrahydrothieno[2,3-d]pyrimidin-6-carbaldehyd

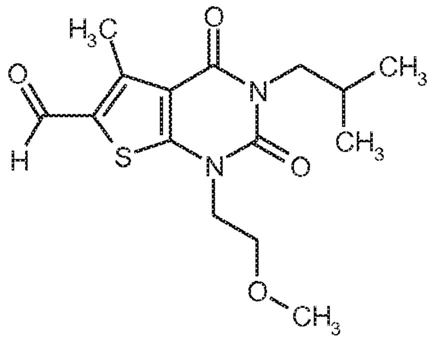

Eine Lösung von 2.33 g (7.86 mmol) der Verbindung aus Bsp. 68A in 8.53 ml DMF wurde unter Eisbadkühlung vorsichtig mit 17.07 ml (183.21 mmol) Phosphoroxychlorid versetzt. Nach Abklingen der exothermen Reaktion wurde das Gemisch auf RT gekühlt. Danach wurde das Reaktions-gemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 2.51 g (95% d. Th.) der Titelverbindung erhalten.
$^1$H-NMR (400 MHz, DMSO-$d_6$, $\delta$/ppm): 10.09 (s, 1H), 4.10 (t, 2H), 3.71 (d, 2H), 3.65 (t, 2H), 3.23 (s, 3H), 2.78 (s, 3H), 2.03 (dquin, 1H), 0.86 (d, 6H).
LC/MS (Methode 3): $R_t$ = 1.20 min, m/z = 324 $[M+H]^+$.

**Beispiel 115A**

1-(2-Ethoxyethyl)-3-isobutyl-5-methyl-2,4-dioxo-1,2,3,4-tetrahydrothieno[2,3-d]pyrimidin-6-carbaldehyd

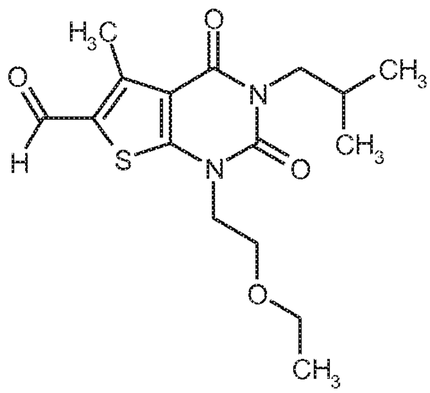

Analog zu dem unter Bsp. 86A beschriebenen Verfahren wurden aus 450 mg (1.69 mmol) der Verbindung aus Bsp. 50A und 862 mg (5.07 mmol) 2-Bromethyl-ethylether 362 mg (63% d. Th.) der Titelverbindung hergestellt. Die Reaktionsdauer betrug hier 40 h.
$^1$H-NMR (400 MHz, DMSO-$d_6$, $\delta$/ppm): 10.09 (s, 1H), 4.09 (t, 2H), 3.75-3.65 (m, 4H), 3.43 (q, 2H), 2.78 (s, 3H), 2.09-1.97 (m, 1H), 1.01 (t, 3H), 0.86 (d, 6H).
LC/MS (Methode 3): $R_t$ = 1.28 min, m/z = 339 $[M+H]^+$.

**Beispiel 116A**

3-Isobutyl-5-methyl-1-(oxetan-2-ylmethyl)-2,4-dioxo-1,2,3,4-tetrahydrothieno[2,3-d]pyrimidin-6-carbaldehyd *(Racemat)*

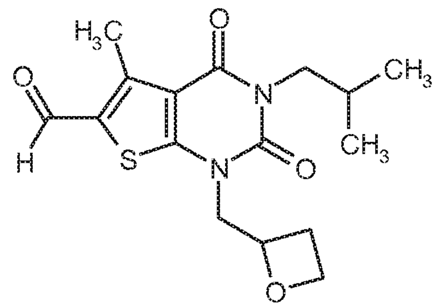

Analog zu dem unter Bsp. 86A beschriebenen Verfahren wurden aus 800 mg (3 mmol) der Verbindung aus Bsp. 50A und 1.84 g (12.01 mmol) racemischem 2-(Brommethyl)oxetan 849 mg (84% d. Th.) der Titelverbindung hergestellt. Die Umsetzung wurde hier bei 80°C durchgeführt und die Reaktionsdauer betrug 21 h.
$^1$H-NMR (400 MHz, DMSO-$d_6$, δ/ppm): 10.09 (s, 1H), 5.06-4.97 (m, 1H), 4.51-4.40 (m, 2H), 4.28-4.15 (m, 2H), 3.71 (d, 2H), 2.77 (s, 3H), 2.75-2.65 (m, 1H), 2.09-1.97 (m, 1H), 0.86 (dd, 6H).
LC/MS (Methode 3): $R_t$ = 1.15 min, m/z = 337 $[M+H]^+$.

**Beispiel 117A**

3-Isobutyl-5-methyl-2,4-dioxo-1-(tetrahydrofuran-2-ylmethyl)-1,2,3,4-tetrahydrothieno[2,3-d]-pyrimidin-6-carbaldehyd *(Racemat)*

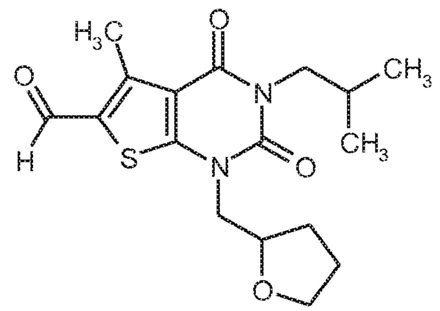

Analog zu dem unter Bsp. 89A (Methode B) beschriebenen Verfahren wurden aus 503 mg (1.56 mmol) der Verbindung aus Bsp. 69A 506 mg (92% d. Th.) der Titelverbindung erhalten.
$^1$H-NMR (400 MHz, DMSO-$d_6$, δ/ppm): 10.09 (s, 1H), 4.28-4.19 (m, 1H), 4.12 (dd, 1H), 3.83-3.70 (m, 4H), 3.65-3.58 (m, 1H), 2.78 (s, 3H), 2.09-1.76 (m, 4H), 1.68 (m, 1H), 0.87 (d, 3H), 0.86 (d, 3H).
LC/MS (Methode 1, ESIpos): $R_t$ = 1.14 min, m/z = 351 $[M+H]^+$.

**Beispiel 118A**

3-Isobutyl-5-methyl-1-(oxetan-3-ylmethyl)-2,4-dioxo-1,2,3,4-tetrahydrothieno[2,3-d]pyrimidin-6-carbaldehyd

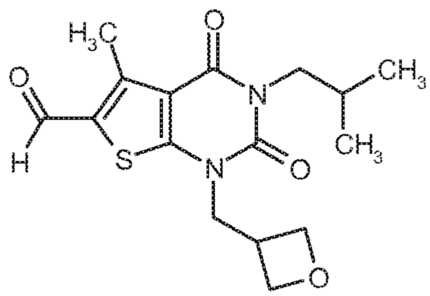

315 mg (1.18 mmol) der Verbindung aus Bsp. 50A und 578 mg (1.77 mmol) Cäsiumcarbonat wurden 10 min bei RT in 7 ml wasserfreiem DMF gerührt, bevor 189 mg (1.77 mmol) 3-(Chlormethyl)oxetan hinzugefügt wurden. Anschließend wurde das Reaktionsgemisch in einem Mikrowellenofen (Biotage Initiator mit dynamischer Steuerung der Einstrahlleistung) zunächst 2 h lang auf 100°C erhitzt. Nach dieser Zeit wurden noch einmal die gleichen Mengen an Cäsiumcarbonat und 3-(Chlormethyl)oxetan hinzugefügt. Nach weiteren 8 h bei 100°C wurde auf RT abgekühlt, mit Ethylacetat verdünnt und nacheinander mit Wasser und gesättigter Natriumchlorid-Lösung gewaschen. Nach Trocknen über wasserfreiem Magnesiumsulfat wurde filtriert und zur Trockene eingeengt. Der erhaltene Rückstand wurde mittels präparativer HPLC gereinigt (Methode 8). Nach Vereinigen der Produktfraktionen, Einengen und Trocknen im Hochvakuum wurden 75 mg (18% d. Th.) der Titelverbindung erhalten.
LC/MS (Methode 1, ESIpos): $R_t$ = 0.93 min, m/z = 337 $[M+H]^+$.

**Beispiel 119A**

3-Isobutyl-1,5-dimethyl-2,4-dioxo-1,2,3,4-tetrahydrothieno[2,3-d]pyrimidin-6-carbaldehyd

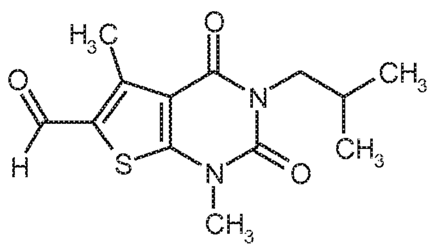

Analog zu dem unter Bsp. 89A (Methode B) beschriebenen Verfahren wurden aus 168 mg (0.67 mmol) der Verbindung aus Bsp. 70A 173 mg (92% d. Th.) der Titelverbindung erhalten.
$^1$H-NMR (400 MHz, DMSO-$d_6$, δ/ppm): 10.09 (s, 1H), 3.71 (d, 2H), 3.48 (s, 3H), 2.79 (s, 3H), 2.04 (m, 1H), 0.87 (d, 6H).
LC/MS (Methode 1, ESIpos): $R_t$ = 0.94 min, m/z = 281 $[M+H]^+$.

**Beispiel 120A**

5-Methyl-2,4-dioxo-3-(2,2,2-trifluorethyl)-1-(3,3,3-trifluorpropyl)-1,2,3,4-tetrahydrothieno[2,3-d]-pyrimidin-6-carbaldehyd

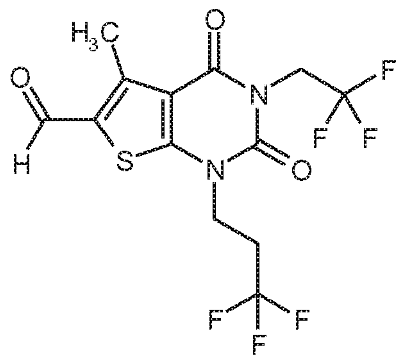

Analog zu dem unter Bsp. 86A beschriebenen Verfahren wurden aus 450 mg (1.5 mmol) der Verbindung aus Bsp. 51A und 1.01 g (4.52 mmol) 1,1,1-Trifluor-3-iodpropan 382 mg (65% d. Th.) der Titelverbindung hergestellt. Die Reaktionsdauer betrug hier 21 h.
$^1$H-NMR (400 MHz, DMSO-$d_6$, $\delta$/ppm): 10.12 (s, 1H), 4.72 (q, 2H), 4.21 (t, 2H), 2.88-2.73 (m, 5H).
LC/MS (Methode 3): $R_t$ = 1.22 min, m/z = 389 $[M+H]^+$.

**Beispiel 121A**

1-(3-Fluorpropyl)-5-methyl-2,4-dioxo-3-(2,2,2-trifluorethyl)-1,2,3,4-tetrahydrothieno[2,3-d]-pyrimidin-6-carbaldehyd

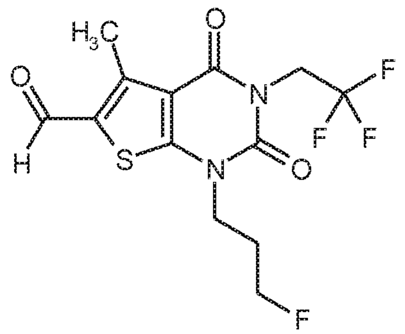

Eine Lösung von 1 g (3.08 mmol) der Verbindung aus Bsp. 71A in 28.7 ml DMF wurde unter Eisbadkühlung vorsichtig mit 2.87 ml (30.83 mmol) Phosphoroxychlorid versetzt. Das Gemisch wurde anschließend 3 h bei 50°C in einer Mikrowellenapparatur gerührt. Danach wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 1.02 g (94% d. Th.) der Titelverbindung erhalten.
$^1$H-NMR (400 MHz, DMSO-$d_6$, $\delta$/ppm): 10.11 (s, 1H), 4.70 (q, 2H), 4.61 (t, 1H), 4.49 (t, 1H), 4.08 (t, 2H), 2.79 (s, 3H), 2.17-2.02 (m, 2H).
LC/MS (Methode 3): $R_t$ = 1.11 min, m/z = 353 $[M+H]^+$.

**Beispiel 122A**

1-(2-Methoxyelhyl)-5-methyl-2,4-dioxo-3-(2,2,2-trifluorethyl)-1,2,3,4-tetrahydrothieno[2,3-d]-pyrimidin-6-carbaldehyd

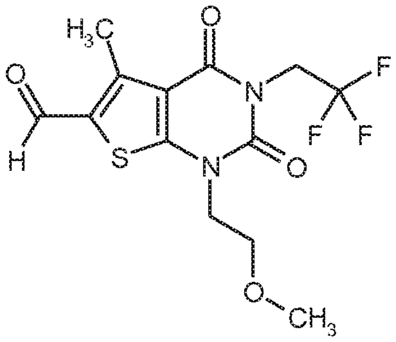

Eine Lösung von 525 mg (1.63 mmol) der Verbindung aus Bsp. 72A in 15.2 ml DMF wurde unter Eisbadkühlung vorsichtig mit 1.52 ml (16.28 mmol) Phosphoroxychlorid versetzt. Das Gemisch wurde anschließend 2 h bei 50°C in einer Mikrowellenapparatur gerührt. Danach wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 540 mg (94% d. Th.) der Titelverbindung erhalten.
$^{1}$H-NMR (400 MHz, DMSO-$d_6$, δ/ppm): 10.10 (s, 1H), 4.70 (q, 2H), 4.14 (t, 2H), 3.66 (t, 2H), 3.24 (s, 3H), 2.78 (s, 3H).
LC/MS (Methode 3): $R_t$ = 1.09 min, m/z = 351 $[M+H]^+$.

**Beispiel 123A**

1-(2-Ethoxyethyl)-5-methyl-2,4-dioxo-3-(2,2,2-trifluorethyl)-1,2,3,4-tetrahydrothieno[2,3-d]-pyrimidin-6-carbaldehyd

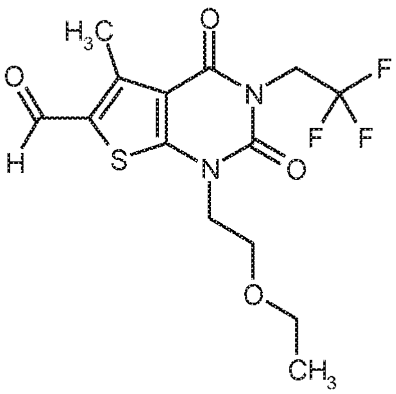

Analog zu dem unter Bsp. 86A beschriebenen Verfahren wurden aus 400 mg (1.37 mmol) der Verbindung aus Bsp. 51A und 628 mg (4.11 mmol) 1-Brom-2-ethoxyethan 201 mg (40% d. Th.) der Titelverbindung hergestellt. Die Reaktionsdauer betrug hier 70 h.
$^{1}$H-NMR (400 MHz, DMSO-$d_6$, δ/ppm): 10.11 (s, 1H), 4.71 (q, 2H), 4.13 (t, 2H), 3.69 (t, 2H), 3.44 (q, 2H), 2.78 (s, 3H), 1.02 (t, 3H).
LC/MS (Methode 3): $R_t$ = 1.18 min, m/z = 365 $[M+H]^+$.

**Beispiel 124A**

5-Methyl-1-(oxetan-2-ylmethyl)-2,4-dioxo-3-(2,2,2-trifluorethyl)-1,2,3,4-tetrahydrothieno[2,3-d]-pyrimidin-6-carbaldehyd *(Racemat)*

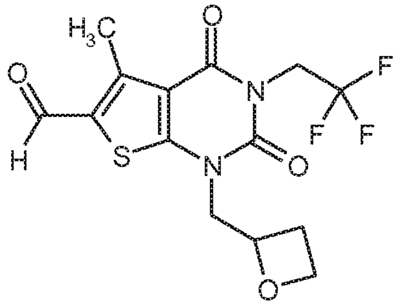

Analog zu dem unter Bsp. 86A beschriebenen Verfahren wurden aus 1.25 g (4.19 mmol) der Verbindung aus Bsp. 51A und 3.22 g (20.95 mmol) racemischem 2-(Brommethyl)oxetan 613 mg (39% d. Th.) der Titelverbindung hergestellt. Die Umsetzung wurde hier bei 70°C durchgeführt und die Reaktionsdauer betrug 114 h.
$^1$H-NMR (400 MHz, DMSO-$d_6$, $\delta$/ppm): 10.11 (s, 1H), 5.03 (ddt, 1H), 4.71 (q, 2H), 4.52-4.41 (m, 2H), 4.33-4.18 (m, 2H), 2.78 (s, 3H), 2.74-2.65 (m, 1H).
LC/MS (Methode 3): $R_t$ = 1.06 min, m/z = 363 $[M+H]^+$.

**Beispiel 125A**

5-Methyl-2,4-dioxo-1-(tetrahydrofuran-2-ylmethyl)-3-(2,2,2-trifluorethyl)-1,2,3,4-tetrahydro-thieno[2,3-d]pyrimidin-6-carbaldehyd *(Racemat)*

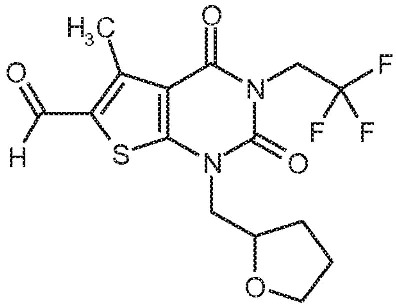

Analog zu dem unter Bsp. 86A beschriebenen Verfahren wurden aus 1.25 g (4.19 mmol) der Verbindung aus Bsp. 51A und 3.84 g (20.96 mmol) racemischem 2-(Brommethyl)tetrahydrofuran 689 mg (43% d. Th.) der Titelverbindung hergestellt. Die Umsetzung wurde hier bei 80°C durchgeführt.
$^1$H-NMR (400 MHz, DMSO-$d_6$, $\delta$/ppm): 10.10 (s, 1H), 4.71 (q, 2H), 4.27-4.19 (m, 1H), 4.15 (dd, 1H), 3.82 (dd, 1H), 3.78-3.71 (m, 1H), 3.62 (td, 1H), 2.78 (s, 3H), 2.06-1.97 (m, 1H), 1.97-1.76 (m, 2H), 1.73-1.63 (m, 1H).
LC/MS (Methode 3): $R_t$ = 1.16 min, m/z = 377 $[M+H]^+$.

**Beispiel 126A**

5-Methyl-1-(oxetan-3-ylmethyl)-2,4-dioxo-3-(2,2,2-trifluorethyl)-1,2,3,4-tetrahydrothieno[2,3-d]-pyrimidin-6-carbaldehyd

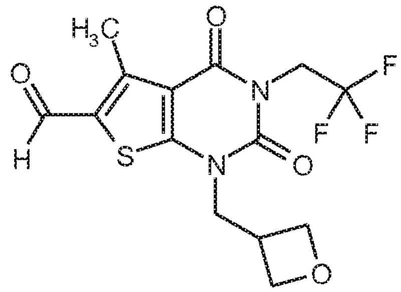

Analog zu dem unter Bsp. 86A beschriebenen Verfahren wurden aus 400 mg (1.34 mmol) der Verbindung aus Bsp. 51A und 810 mg (5.36 mmol) 3-(Brommethyl)oxetan 156 mg (32% d. Th.) der Titelverbindung hergestellt. Die Reaktionsdauer betrug hier 70 h.

$^1$H-NMR (400 MHz, DMSO-$d_6$, δ/ppm): 10.11 (s, 1H), 4.69 (q, 2H), 4.61 (dd, 2H), 4.44 (t, 2H), 4.30 (d, 2H), 3.51-3.41 (m, 1H), 2.78 (s, 3H).

LC/MS (Methode 3): Rt = 1.01 min, m/z = 363 $[M+H]^+$.

**Beispiel 127A**

1,5-Dimethyl-2,4-dioxo-3-(2,2,2-trifluorethyl)-1,2,3,4-tetrahydrothieno[2,3-d]pyrimidin-6-carbaldehyd

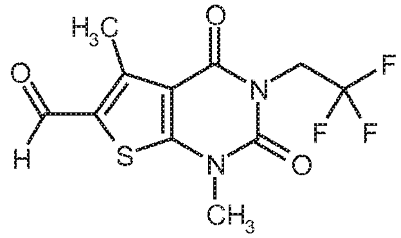

Analog zu dem unter Bsp. 86A beschriebenen Verfahren wurden aus 350 mg (1.17 mmol) der Verbindung aus Bsp. 51A und 505 mg (3.52 mmol) Iodmethan 401 mg der Titelverbindung hergestellt. Die Reaktionsdauer betrug hier 24 h.

$^1$H-NMR (400 MHz, DMSO-$d_6$, δ/ppm): 10.10 (s, 1H), 4.70 (q, 2H), 3.51 (s, 3H), 2.79 (s, 3H).

LC/MS (Methode 3): $R_t$ = 1.04 min, m/z = 307 $[M+H]^+$.

**Beispiel 128A**

3-(2,2-Difluorethyl)-5-methyl-2,4-dioxo-1-(3,3,3-trifluorpropyl)-1,2,3,4-tetrahydrothieno[2,3-d]-pyrimidin-6-carbaldehyd

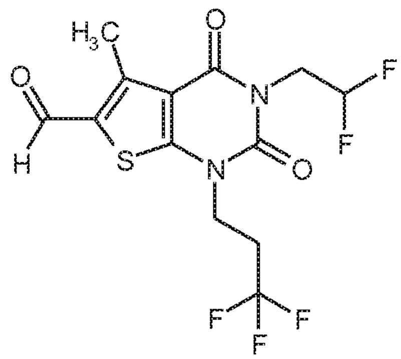

Analog zu dem unter Bsp. 86A beschriebenen Verfahren wurden aus 350 mg (1.26 mmol) der Verbindung aus

Bsp. 52A und 857 mg (3.83 mmol) 1,1,1-Trifluor-3-iodpropan 318 mg (67% d. Th.) der Titelverbindung hergestellt. Die Reaktionsdauer betrug hier 45 h.
$^1$H-NMR (400 MHz, DMSO-$d_6$, $\delta$/ppm): 10.12 (s, 1H), 6.38-6.05 (m, 1H), 4.31 (td, 2H), 4.20 (t, 2H), 2.88-2.73 (m, 5H).
LC/MS (Methode 3): $R_t$ = 1.16 min, m/z = 371 $[M+H]^+$.

**Beispiel 129A**

3-(2,2-Difluorethyl)-1-(3-fluorpropyl)-5-methyl-2,4-dioxo-1,2,3,4-tetrahydrothieno[2,3-d]pyrimidin-6-carbaldehyd

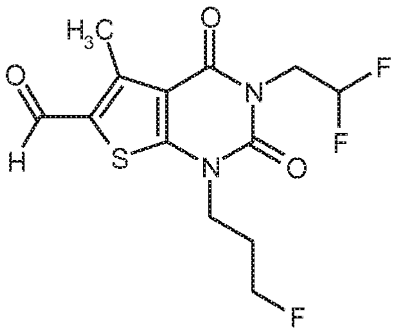

Analog zu dem unter Bsp. 86A beschriebenen Verfahren wurden aus 501 mg (1.79 mmol) der Verbindung aus Bsp. 52A und 1.01 g (5.37 mmol) 1-Fluor-3-iodpropan 526 mg (86% d. Th.) der Titelverbindung hergestellt. Die Reaktionsdauer betrug hier 16 h.
$^1$H-NMR (400 MHz, DMSO-$d_6$, $\delta$/ppm): 10.11 (s, 1H), 6.37-6.05 (m, 1H), 4.61 (t, 1H), 4.49 (t, 1H), 4.30 (td, 2H), 4.07 (t, 2H), 2.79 (s, 3H), 2.17-2.01 (m, 2H).
LC/MS (Methode 3): $R_t$ = 1.04 min, m/z = 335 $[M+H]^+$.

**Beispiel 130A**

3-(2,2-Difluorethyl)-1-(2-methoxyethyl)-5-methyl-2,4-dioxo-1,2,3,4-tetrahydrothieno[2,3-d]-pyrimidin-6-carbaldehyd

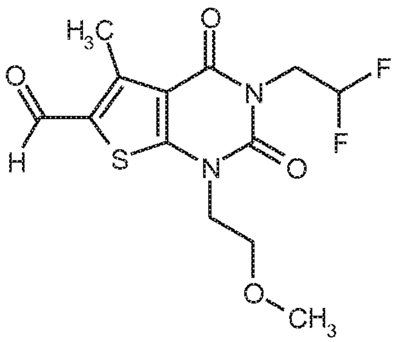

Analog zu dem unter Bsp. 86A beschriebenen Verfahren wurden aus 527 mg (1.92 mmol) der Verbindung aus Bsp. 52A und 827 mg (5.77 mmol) 2-Bromethyl-methylether 468 mg (72% d. Th.) der Titelverbindung hergestellt. Die Reaktionsdauer betrug hier 40 h.
$^1$H-NMR (400 MHz, DMSO-$d_6$, $\delta$/ppm): 10.10 (s, 1H), 6.38-6.06 (m, 1H), 4.30 (td, 2H), 4.12 (t, 2H), 3.66 (t, 2H), 3.24 (s, 3H), 2.78 (s, 3H).
LC/MS (Methode 3): $R_t$ = 1.0 min, m/z = 333 $[M+H]^+$.

**Beispiel 131A**

3-(2,2-Difluorethyl)-1-(2-ethoxyethyl)-5-methyl-2,4-dioxo-1,2,3,4-tetrahydrothieno[2,3-d]pyrimidin-6-carbaldehyd

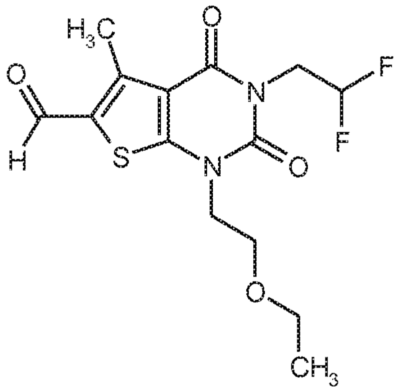

Analog zu dem unter Bsp. 86A beschriebenen Verfahren wurden aus 350 mg (1.27 mmol) der Verbindung aus Bsp. 52A und 651 mg (3.83 mmol) 2-Bromethyl-ethylether 189 mg (41% d. Th.) der Titelverbindung hergestellt. Die Reaktionsdauer betrug hier 116 h.
$^1$H-NMR (400 MHz, DMSO-$d_6$, δ/ppm): 10.10 (s, 1H), 6.38-6.06 (m, 1H), 4.30 (td, 2H), 4.11 (t, 2H), 3.69 (t, 2H), 3.44 (q, 2H), 2.78 (s, 3H), 1.03 (t, 3H).
LC/MS (Methode 3): $R_t$ = 1.11 min, m/z = 347 $[M+H]^+$.

**Beispiel 132A**

3-(2,2-Difluorethyl)-5-methyl-1-{oxetan-2-ylmethyl)-2,4-dioxo-1,2,3,4-tetrahydrothieno[2,3-d]-pyrimidin-6-carbaldehyd *(Racemat)*

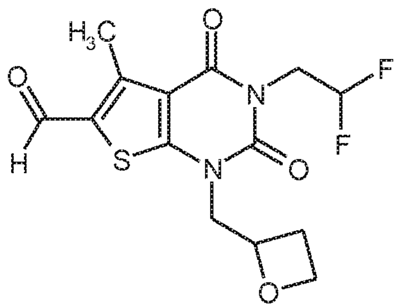

Analog zu dem unter Bsp. 86A beschriebenen Verfahren wurden aus 543 mg (1.98 mmol) der Verbindung aus Bsp. 52A und 1.19 g (7.92 mmol) racemischem 2-(Brommethyl)oxetan 333 mg (47% d. Th.) der Titelverbindung hergestellt. Die Umsetzung wurde hier bei 80°C durchgeführt und die Reaktionsdauer betrug 62 h.
$^1$H-NMR (400 MHz, DMSO-$d_6$, δ/ppm): 10.10 (s, 1H), 6.38-6.06 (m, 1H), 5.03 (ddt, 1H), 4.52-4.41 (m, 2H), 4.36-4.17 (m, 4H), 2.78 (s, 3H), 2.74-2.64 (m, 1H).
LC/MS (Methode 3): $R_t$ = 0.98 min, m/z = 345 $[M+H]^+$.

**Beispiel 133A**

3-(2,2-Difluorethyl)-5-methyl-2,4-dioxo-1-(tetrahydrofuran-2-ylmethyl)-1,2,3,4-tetrahydrothieno-[2,3-d]pyrimidin-6-carbaldehyd (*Racemat*)

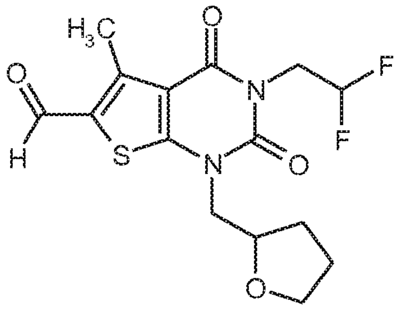

Analog zu dem unter Bsp. 86A beschriebenen Verfahren wurden aus 800 mg (2.92 mmol) der Verbindung aus Bsp. 52A und 2.53 g (14.58 mmol) racemischem 2-(Brommethyl)tetrahydrofuran 520 mg (49% d. Th.) der Titelverbindung hergestellt. Die Umsetzung wurde hier bei 70°C durchgeführt.
$^1$H-NMR (400 MHz, DMSO-$d_6$, δ/ppm): 10.10 (s, 1H), 6.39-6.07 (m, 1H), 4.36-4.19 (m, 3H), 4.15 (dd, 1H), 3.84-3.70 (m, 2H), 3.62 (td, 1H), 2.78 (s, 3H), 2.07-1.96 (m, 1H), 1.96-1.86 (m, 1H), 1.86-1.76 (m, 1H), 1.73-1.63 (m, 1H).
LC/MS (Methode 3): $R_t$ = 1.08 min, m/z = 359 $[M+H]^+$.

**Beispiel 134A**

3-(2,2-Difluorethyl)-5-methyl-1-(oxetan-3-ylmethyl)-2,4-dioxo-1,2,3,4-tetrahydrothieno[2,3-d]-pyrimidin-6-carbaldehyd

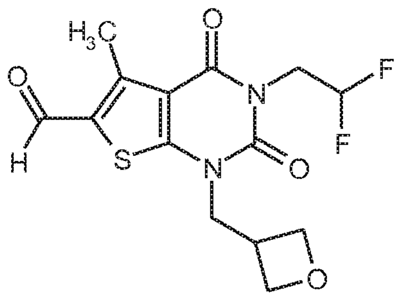

Analog zu dem unter Bsp. 86A beschriebenen Verfahren wurden aus 350 mg (1.27 mmol) der Verbindung aus Bsp. 52A und 771 mg (5.1 mmol) 3-(Brommethyl)oxetan 133 mg (29% d. Th.) der Titelverbindung hergestellt. Die Reaktionsdauer betrug hier 22 h.
$^1$H-NMR (400 MHz, DMSO-$d_6$, δ/ppm): 10.11 (s, 1H), 6.35-6.03 (m, 1H), 4.61 (dd, 2H), 4.45 (t, 2H), 4.34-4.23 (m, 4H), 3.51-3.39 (m, 1H), 2.78 (s, 3H).
LC/MS (Methode 3): $R_t$ = 0.93 min, m/z = 345 $[M+H]^+$.

**Beispiel 135A**

3-(2,2-Difluorethyl)-1,5-dimethyl-2,4-dioxo-1,2,3,4-tetrahydrothieno[2,3-d]pyrimidin-6-carbaldehyd

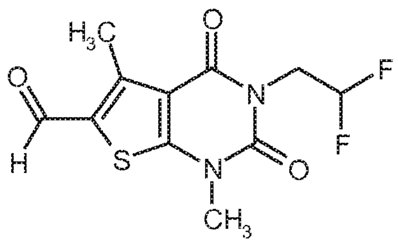

Analog zu dem unter Bsp. 86A beschriebenen Verfahren wurden aus 350 mg (1.27 mmol) der Verbindung aus

Bsp. 52A und 549 mg (3.83 mmol) Iodmethan 358 mg (88% d. Th.) der Titelverbindung hergestellt. Die Reaktionsdauer betrug hier 18 h.
$^1$H-NMR (400 MHz, DMSO-$d_6$, δ/ppm): 10.10 (s, 1H), 6.37-6.05 (m, 1H), 4.30 (td, 2H), 3.50 (s, 3H), 2.79 (s, 3H).
LC/MS (Methode 3): $R_t$ = 0.95 min, m/z = 289 $[M+H]^+$.

**Beispiel 136A**

1,3-Bis(2-methoxyethyl)-5-methyl-2,4-dioxo-1,2,3,4-tetrahydrothieno[2,3-d]pyrimidin-6-carbaldehyd

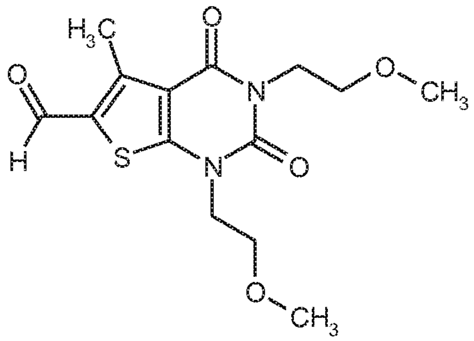

Analog zu dem unter Bsp. 86A beschriebenen Verfahren wurden aus 450 mg (1.66 mmol) der Verbindung aus Bsp. 53A und 692 mg (4.98 mmol) 2-Bromethyl-methylether 391 mg (71% d. Th.) der Titelverbindung hergestellt. Die Reaktionsdauer betrug hier 18 h.
$^1$H-NMR (400 MHz, DMSO-$d_6$, δ/ppm): 10.09 (s, 1H), 4.10 (t, 2H), 4.06 (t, 2H), 3.65 (t, 2H), 3.51 (t, 2H), 3.24 (2s, 6H), 2.78 (s, 3H).
LC/MS (Methode 3): $R_t$ = 0.93 min, m/z = 327 $[M+H]^+$.

**Beispiel 137A**

1-(2-Ethoxyethyl)-3-(2-methoxyethyl)-5-methyl-2,4-dioxo-1,2,3,4-tetrahydrothieno[2,3-d]pyrimidin-6-carbaldehyd

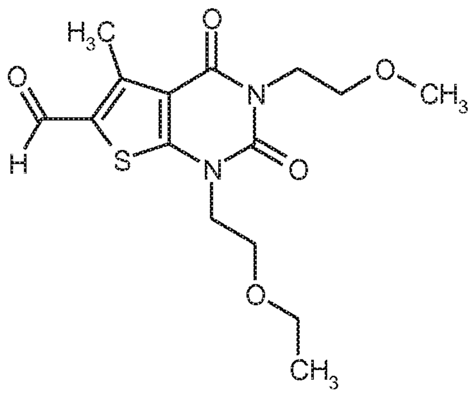

Analog zu dem unter Bsp. 86A beschriebenen Verfahren wurden aus 475 mg (1.77 mmol) der Verbindung aus Bsp. 53A und 903 mg (5.31 mmol) 2-Bromethyl-ethylether 381 mg (61% d. Th.) der Titelverbindung hergestellt. Die Reaktionsdauer betrug hier 24 h.
$^1$H-NMR (400 MHz, DMSO-$d_6$, δ/ppm): 10.09 (s, 1H), 4.12-4.02 (m, 4H), 3.68 (t, 2H), 3.51 (t, 2H), 3.44 (q, 2H), 3.24 (s, 3H), 2.78 (s, 3H), 1.02 (t, 3H).
LC/MS (Methode 3): $R_t$ = 1.03 min, m/z = 341 $[M+H]^+$.

**Beispiel 138A**

6-(Hydroxymethyl)-1-(2-methoxyethyl)-5-methyl-3-(2-phenylethyl)thieno[2,3-d]pyrimidin-2,4(1*H*,3*H*)-dion

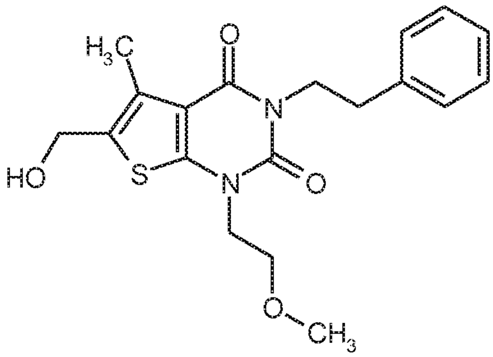

*Methode A:*

Eine Lösung von 100 mg (0.257 mmol) der Verbindung aus Bsp. 15A in 2 ml trockenem THF wurde bei 0°C tropfenweise mit 130 µl (0.129 mmol) einer 1 M Lösung von Lithiumaluminiumhydrid in THF versetzt. Nach 1 h bei 0°C war die Reaktion vollständig. Überschüssiges Lithiumaluminiumhydrid wurde durch Zugabe von wenig Methanol vernichtet. Anschließend wurde so viel DMF zugesetzt, dass eine klare Lösung entstand, die dann mittels präparativer HPLC (Methode 8) in ihre Komponenten aufgetrennt wurde. Nach Eindampfen der Produktfraktionen und Trocknen im Hochvakuum wurden 12 mg (13% d. Th.) der Titelverbindung erhalten.

*Methode B:*

Eine Lösung von 1.0 g (2.49 mmol) der Verbindung aus Bsp. 19A in 30 ml trockenem THF wurde bei 0°C tropfenweise mit 2.5 ml (2.49 mmol) einer 1 M Lösung von Lithiumaluminiumhydrid in THF versetzt. Nach 10 min bei 0°C wurde überschüssiges Lithiumaluminiumhydrid durch Zugabe von 1 ml Wasser vernichtet. Dann wurde mit 10 ml 1 M Natronlauge versetzt. Vom Ungelösten wurde abgesaugt und der Rückstand gründlich mit THF gewaschen. Das Filtrat wurde zur Trockene eingeengt. Der erhaltene Rückstand wurde mittels MPLC gereinigt (Biotage-Kartusche, 100 g Kieselgel, Cyclohexan/Ethylacetat 2:1 → 1:1). Nach Einengen der Produktfraktionen und Trocknen im Hochvakuum wurden 572 mg (61% d. Th.) der Titelverbindung erhalten.

*Methode C:*

Eine Lösung von 430 mg (0.847 mmol) der Verbindung aus Bsp. 23A in 23 ml trockenem THF wurde bei -40°C tropfenweise mit 0.847 ml (0.847 mmol) einer 1 M Lösung von Lithiumaluminiumhydrid in THF versetzt. Nach 30 min Rühren bei -40°C wurde vorsichtig 1 ml 10%-ige Salzsäure zugesetzt. Das Gemisch wurde auf RT gebracht und mit 100 ml gesättigter Kochsalz-Lösung versetzt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Hexan/Ethylacetat). Es wurden 235 mg (74% d. Th.) der Titelverbindung erhalten.
$^1$H-NMR (400 MHz, DMSO-$d_6$, δ/ppm): 7.32-7.27 (m, 2H), 7.26-7.20 (m, 3H), 5.58 (t, 1H), 4.57 (d, 2H), 4.06 (dd, 2H), 4.03 (t, 2H), 3.61 (t, 2H), 3.24 (s, 3H), 2.84 (dd, 2H), 2.33 (s, 3H).
LC/MS (Methode 1, ESIpos): $R_t$ = 0.97 min, m/z = 375 $[M+H]^+$.

**Beispiel 139A**

6-(Hydroxymethyl)-1-(2-methoxyethyl)-3,5-dimethylthieno[2,3-d]pyrimidin-2,4(1*H*,3*H*)-dion

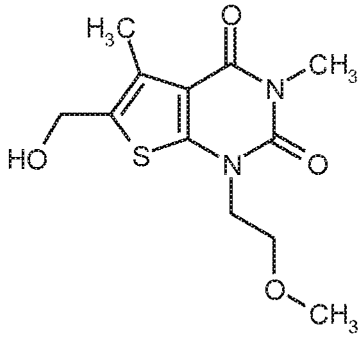

Eine Lösung von 240 mg (0.730 mmol) der Verbindung aus Bsp. 20A in 8.7 ml trockenem THF wurde bei -40°C tropfenweise mit 0.73 ml (0.73 mmol) einer 1 M Lösung von Lithiumaluminiumhydrid in THF versetzt. Nach 30 min Rühren bei -40°C wurde vorsichtig 10%-ige Salzsäure zugesetzt, bis eine klare Lösung vorlag. Das Gemisch wurde auf RT gebracht und mit 30 ml gesättigter Kochsalz-Lösung versetzt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 10 g Kieselgel, Laufmittel Hexan/Ethylacetat). Es wurden 129 mg (61% d. Th.) der Titelverbindung erhalten.
$^1$H-NMR (400 MHz, DMSO-$d_6$, δ/ppm): 5.56 (t, 1H), 4.57 (d, 2H), 4.04 (t, 2H), 3.64 (t, 2H), 3.24 (s, 3H), 3.22 (s, 3H), 2.32 (s, 3H).
LC/MS (Methode 3): $R_t$ = 0.74 min, m/z = 285 $[M+H]^+$.

**Beispiel 140A**

3-Ethyl-6-(hydroxymethyl)-5-methyl-1-(3,3,3-trifluorpropyl)thieno[2,3-d]pyrimidin-2,4(1*H*,3*H*)-dion

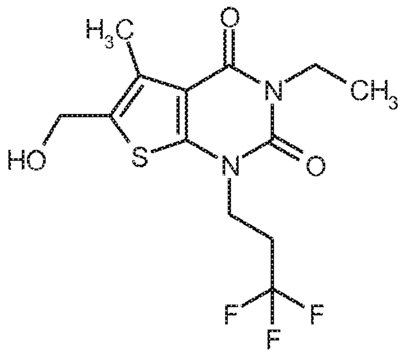

*Methode A:*

Eine Lösung von 1.0 g (2.75 mmol) der Verbindung aus Bsp. 21A in 30 ml trockenem THF wurde bei 0°C tropfenweise mit 1.4 ml (2.75 mmol) einer 1 M Lösung von Lithiumaluminiumhydrid in THF versetzt. Nach 15 min bei 0°C wurde überschüssiges Lithiumaluminiumhydrid durch Zugabe von 1 ml Wasser vernichtet. Dann wurde mit 10 ml 1 M Natronlauge versetzt. Es wurde Kieselgur hinzugefügt, vom Ungelösten abgesaugt und der Rückstand gründlich mit THF gewaschen. Das Filtrat wurde zur Trockene eingeengt. Der erhaltene Rückstand wurde mittels MPLC gereinigt (Interchim-Kartusche, 120 g Kieselgel, Cyclohexan/Ethylacetat 2:1 → 1:1). Da das so erhaltene Produkt noch nicht ganz rein war, wurde eine erneute Reinigung mittels präparativer HPLC (Methode 10) durchgeführt. Nach Eindampfen der Produktfraktionen und Trocknen im Hochvakuum wurden 385 mg (41% d. Th.) der Titelverbindung erhalten.

*Methode B:*

Eine Lösung von 3.83 g (11.4 mmol) der Verbindung aus Bsp. 74A in 100 ml trockenem THF wurde bei -78°C tropfenweise mit 3.4 ml (3.43 mmol) einer 1 M Lösung von Lithiumaluminiumhydrid in THF versetzt. Nach 15 min bei -78°C wurde überschüssiges Lithiumaluminiumhydrid durch Zugabe von 2.5 ml Wasser vernichtet. Dann wurde mit 10

ml 1 M Natronlauge versetzt. Es wurde Kieselgur hinzugefügt, das Gemisch auf RT kommen gelassen, vom Ungelösten abgesaugt und der Rückstand gründlich mit THF gewaschen. Das Filtrat wurde zunächst zur Trockene eingeengt, dann in 100 ml Ethylacetat aufgenommen und nacheinander mit Wasser und gesättigter Natriumchlorid-Lösung gewaschen. Die organische Phase wurde über wasserfreiem Magnesiumsulfat getrocknet und anschließend eingeengt. Der erhaltene Rückstand wurde mit einem Gemisch aus jeweils 10 ml Cyclohexan und Ethylacetat bei RT verrührt. Nach Absaugen und Trocknen des Feststoffs im Hochvakuum wurden 3.12 g (78% d. Th., 97% Reinheit) der Titelverbindung erhalten.
$^1$H-NMR (400 MHz, DMSO-$d_6$, $\delta$/ppm): 5.61 (t, 1H), 4.59 (d, 2H), 4.12 (t, 2H), 3.91 (q, 2H), 2.83-2.71 (m, 2H), 2.34 (s, 3H), 1.12 (t, 3H).
LC/MS (Methode 1, ESIpos): $R_t$ = 0.80 min, m/z = 337 [M+H]$^+$.

**Beispiel 141A**

3-Ethyl-1-(3-fluorpropyl)-6-(hydroxymethyl)-5-methylthieno[2,3-d]pyrimidin-2,4(1*H*,3*H*)-dion

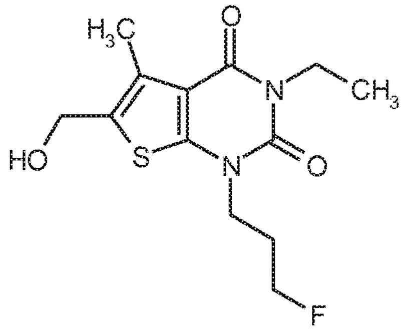

*Methode A:*

Analog zu dem unter Bsp. 138A (Methode C) beschriebenen Verfahren wurden aus 465 mg (1.23 mmol) der Verbindung aus Bsp. 14A 210 mg (56% d. Th.) der Titelverbindung hergestellt.
$^1$H-NMR (400 MHz, DMSO-$d_6$, $\delta$/ppm): 5.64-5.55 (m, 1H), 4.62-4.55 (m, 3H), 4.48 (t, 1H), 4.00 (t, 2H), 3.90 (q, 2H), 2.33 (s, 3H), 2.15-2.07 (m, 1H), 2.07-1.99 (m, 1H), 1.11 (t, 3H).
LC/MS (Methode 3): $R_t$ = 0.90 min, m/z = 301 [M+H]$^+$.

*Methode B:*

Eine Lösung von 580 mg (1.86 mmol) der Verbindung aus Bsp. 78A in 20 ml trockenem THF wurde bei -78°C tropfenweise mit 0.56 ml (0.56 mmol) einer 1 M Lösung von Lithiumaluminiumhydrid in THF versetzt. Nach 120 min Rühren bei -78°C wurde überschüssiges Lithiumaluminiumhydrid durch Zugabe von 1 ml Wasser vernichtet. Dann wurde mit 1.66 ml 1 M Natronlauge versetzt. Es wurde Kieselgur hinzugefügt, vom Ungelösten abgesaugt und der Rückstand gründlich mit THF gewaschen. Das Filtrat wurde zur Trockene eingeengt. Der erhaltene Rückstand wurde in 100 ml Ethylacetat aufgenommen. Diese Lösung wurde mit Wasser (100 ml) und gesättigter Kochsalz-Lösung gewaschen. Die organische Phase wurde über Natriumsulfat getrocknet, filtriert und eingeengt. Es wurden 569 mg (91 % d. Th.) der Titelverbindung erhalten.
LC/MS (Methode 3): $R_t$ = 0.88 min, m/z = 301 [M+H]$^+$.

**Beispiel 142A**

3-Ethyl-6-(hydroxymethyl)-5-methyl-1-[2-(trifluormethoxy)ethyl]thieno[2,3-d]pyrimidin-2,4(1*H*,3*H*)-dion

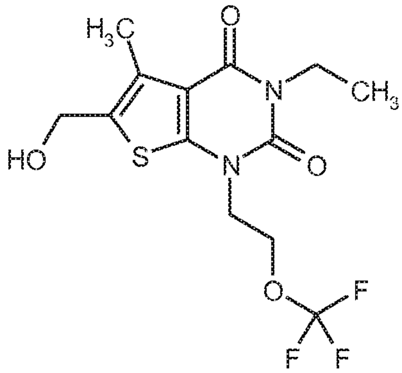

Eine Lösung von 100 mg (0.285 mmol) der Verbindung aus Bsp. 82A in 2.8 ml Ethanol wurde bei RT mit 16 mg (0.428 mmol) Natriumborhydrid versetzt. Nach 1 h wurde vorsichtig ca. 1 ml 1 M Salzsäure hinzugefügt. Anschließend wurde vom Ungelösten abgesaugt und das Filtrat direkt über präparative HPLC (Methode 12) in seine Komponenten aufgetrennt. Nach Einengen der Produktfraktionen und Trocknen im Hochvakuum wurden 82 mg (81% d. Th.) der Titelverbindung erhalten.

$^1$H-NMR (400 MHz, DMSO-$d_6$, $\delta$/ppm): 5.60 (t, 1H), 4.58 (d, 2H), 4.41 (t, 2H), 4.21 (t, 2H), 3.91 (q, 2H), 2.33 (s, 3H), 1.12 (t, 3H).

LC/MS (Methode 1, ESIpos): $R_t$ = 0.88 min, m/z = 353 $[M+H]^+$.

**Beispiel 143A**

3-Ethyl-6-(hydroxymethyl)-1-(2-methoxyethyl)-5-methylthieno[2,3-d]pyrimidin-2,4(1*H*,3*H*)-dion

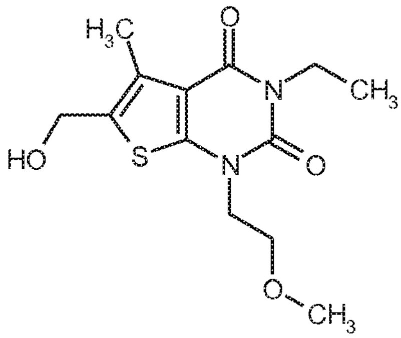

*Methode A:*

Analog zu dem unter Bsp. 138A (Methode A) beschriebenen Verfahren wurden aus 100 mg (0.320 mmol) der Verbindung aus Bsp. 17A 22 mg (23% d. Th.) der Titelverbindung erhalten. Die präparative HPLC erfolgte hier nach Methode 11.

*Methode B:*

Analog zu dem unter Bsp. 138A (Methode B) beschriebenen Verfahren wurden aus 2.08 g (6.37 mmol) der Verbindung aus Bsp. 18A 1.04 g (52% d. Th., 95% Reinheit) der Titelverbindung erhalten.

*Methode C:*

Eine Lösung von 10.0 g (33.7 mmol) der Verbindung aus Bsp. 84A in 300 ml trockenem THF wurde bei -78°C tropfenweise mit 10.1 ml (10.1 mmol) einer 1 M Lösung von Lithiumaluminiumhydrid in THF versetzt. Nach 2 h bei -78°C wurde das Reaktionsgemisch kurz (< 5 min) auf ca. -30°C erwärmt, bevor nach erneuter Abkühlung auf -78°C das überschüssige Lithiumaluminiumhydrid durch Zugabe von 5 ml Wasser vernichtet wurde. Dann wurde mit 30 ml 1 M Natronlauge versetzt. Es wurde Kieselgur hinzugefügt, das Gemisch auf RT kommen gelassen, vom Ungelösten abgesaugt und der Rückstand gründlich mit THF gewaschen. Das Filtrat wurde zunächst zur Trockene eingeengt, dann in 400 ml Ethylacetat aufgenommen und nacheinander mit Wasser und gesättigter Natriumchlorid-Lösung gewaschen. Die organische Phase wurde über wasserfreiem Natriumsulfat getrocknet und anschließend eingeengt. Nach Trocknen des erhaltenen Rückstands im Hochvakuum wurden 9.84 g (94% d. Th., 97% Reinheit) der Titelverbindung erhalten.
$^1$H-NMR (400 MHz, DMSO-$d_6$, δ/ppm): 5.55 (t, 1H), 4.57 (d, 2H), 4.04 (t, 2H), 3.90 (q, 2H), 3.64 (t, 2H), 3.24 (s, 3H), 2.33 (s, 3H), 1.12 (t, 3H).
LC/MS (Methode 1, ESIpos): $R_t$ = 0.63 min, m/z = 299 [M+H]$^+$.

**Beispiel 144A**

6-(Hydroxymethyl)-1-(2-methoxyethyl)-5-methyl-3-propylthieno[2,3-d]pyrimidin-2,4(1*H*,3*H*)-dion

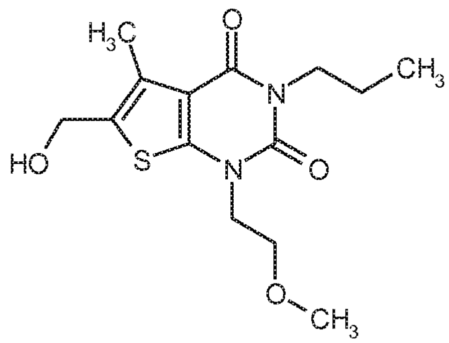

Analog zu dem unter Bsp. 138A (Methode C) beschriebenen Verfahren wurden aus 260 mg (0.69 mmol) der Verbindung aus Bsp. 24A 97 mg (44% d. Th.) der Titelverbindung hergestellt. Die Umsetzung erfolgte hier bei -40°C.
$^1$H-NMR (400 MHz, DMSO-$d_6$, δ/ppm): 5.55 (br. s, 1H), 4.57 (s, 2H), 4.04 (t, 2H), 3.86-3.78 (m, 2H), 3.64 (t, 2H), 3.24 (s, 3H), 1.56 (sext, 2H), 0.86 (t, 3H).
LC/MS (Methode 3): $R_t$ = 0.94 min, m/z = 313 [M+H]$^+$.

**Beispiel 145A**

3-Allyl-6-(hydroxymethyl)-1-(2-methoxyethyl)-5-methylthieno[2,3-d]pyrimidin-2,4(1*H*,3*H*)-dion

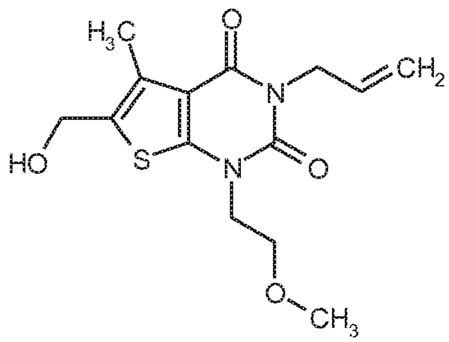

Analog zu dem unter Bsp. 138A (Methode C) beschriebenen Verfahren wurden aus 345 mg (0.92 mmol) der Verbindung aus Bsp. 25A 169 mg (59% d. Th.) der Titelverbindung hergestellt. Die Umsetzung erfolgte hier bei -40°C.
$^1$H-NMR (400 MHz, DMSO-$d_6$, δ/ppm): 5.84 (ddt, 1H), 5.59 (br. s, 1H), 5.13-5.02 (m, 2H), 4.57 (d, 2H), 4.46 (d, 2H), 4.04 (t, 2H), 3.63 (t, 2H), 3.23 (s, 3H), 2.32 (s, 3H).
LC/MS (Methode 3): $R_t$ = 0.88 min, m/z = 311 [M+H]$^+$.

**Beispiel 146A**

6-(Hydroxymethyl)-3-isopropyl-1-(2-methoxyethyl)-5-methylthieno[2,3-d]pyrimidin-2,4(1*H*,3*H*)-dion

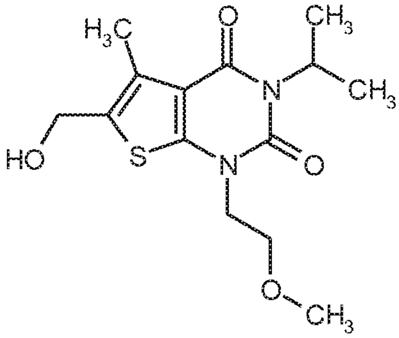

Analog zu dem unter Bsp. 138A (Methode C) beschriebenen Verfahren wurden aus 280 mg (0.74 mmol) der Verbindung aus Bsp. 26A 113 mg (45% d. Th.) der Titelverbindung hergestellt. Die Umsetzung erfolgte hier bei -40°C.
$^1$H-NMR (400 MHz, DMSO-$d_6$, δ/ppm): 5.55 (t, 1H), 5.13 (sept, 1H), 4.56 (d, 2H), 4.00 (t, 2H), 3.62 (t, 2H), 3.26-3.23 (m, 3H), 2.31 (s, 3H), 1.40 (d, 6H).
LC/MS (Methode 3): $R_t$ = 0.97 min, m/z = 313 $[M+H]^+$.

**Beispiel 147A**

3-*sec*.-Butyl-6-(hydroxymethyl)-1-(2-methoxyethyl)-5-methylthieno[2,3-d]pyrimidin-2,4(1*H*,3*H*)-dion *(Racemat)*

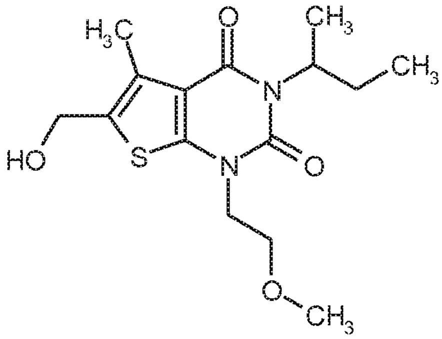

Analog zu dem unter Bsp. 138A (Methode C) beschriebenen Verfahren wurden aus 320 mg (0.77 mmol) der Verbindung aus Bsp. 27A 172 mg (69% d. Th.) der Titelverbindung hergestellt. Die Umsetzung erfolgte hier bei -40°C.
$^1$H-NMR (400 MHz, DMSO-$d_6$, δ/ppm): 5.56 (t, 1H), 4.91 (d, 1H), 4.56 (d, 2H), 4.08-3.95 (m, 2H), 3.62 (t, 2H), 3.23 (s, 3H), 2.31 (s, 3H), 2.09-1.97 (m, 1H), 1.73 (dquin, 1H), 1.37 (d, 3H), 0.75 (t, 3H).
LC/MS (Methode 3): $R_t$ = 1.06 min, m/z = 327 $[M+H]^+$.

**Beispiel 148A**

6-(Hydroxymethyl)-3-isobutyl-1-(2-methoxyethyl)-5-methylthieno[2,3-d]pyrimidin-2,4(1*H*,3*H*)-dion

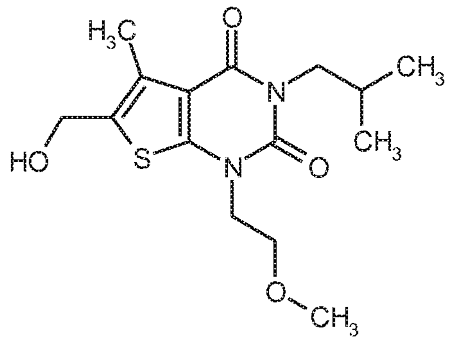

Eine Lösung von 395 mg (1.2 mmol) der Verbindung aus Bsp. 114A in 12 ml THF und 38 ml Ethanol wurde mit 68.4 mg (1.81 mmol) Natriumborhydrid (gelöst in 2 ml Ethanol) versetzt und bei RT gerührt. Nach 22 h wurde überschüssiges Natriumborhydrid durch Zugabe von Essigsäure zerstört. Das Reaktionsgemisch wurde am Rotationsverdampfer weitestgehend eingeengt. Der verbliebene Rückstand wurde in Ethylacetat aufgenommen. Es wurde mit Wasser gewaschen, und die organische Phase wurde über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 285 mg (72% d. Th.) der Titelverbindung erhalten.
$^1$H-NMR (400 MHz, DMSO-$d_6$, δ/ppm): 5.55 (br. s, 1H), 4.57 (br. s, 2H), 4.04 (t, 2H), 3.71 (d, 2H), 3.63 (t, 2H), 3.23 (s, 3H), 2.32 (s, 3H), 2.09-1.98 (m, 1H), 0.85 (d, 6H).
LC/MS (Methode 3): $R_t$ = 1.04 min, m/z = 327 $[M+H]^+$.

**Beispiel 149A**

6-(Hydroxymethyl)-1-(2-methoxyethyl)-5-methyl-3-(3-methylbut-2-en-1-yl)thieno[2,3-d]pyrimidin-2,4(1*H*,3*H*)-dion

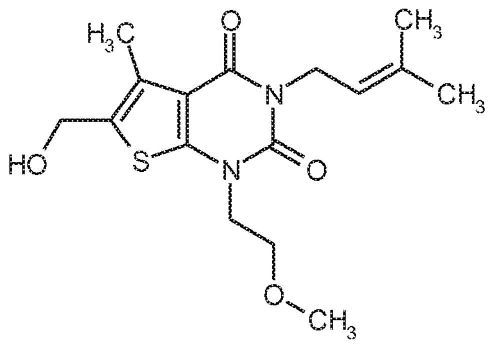

Analog zu dem unter Bsp. 138A (Methode C) beschriebenen Verfahren wurden aus 405 mg (0.92 mmol) der Verbindung aus Bsp. 28A 197 mg (59% d. Th.) der Titelverbindung hergestellt. Die Umsetzung erfolgte hier bei -40°C.
$^1$H-NMR (400 MHz, DMSO-$d_6$, δ/ppm): 5.58 (t, 1H), 5.15 (ddd, 1H), 4.57 (d, 2H), 4.44 (d, 2H), 4.03 (t, 2H), 3.63 (t, 2H), 3.24 (s, 3H), 2.32 (s, 3H), 1.75 (d, 3H), 1.66 (d, 3H).
LC/MS (Methode 3): $R_t$ = 1.09 min, m/z = 339 $[M+H]^+$.

**Beispiel 150A**

3-(Cyclopropylmethyl)-6-(hydroxymethyl)-1-(2-methoxyethyl)-5-methylthieno[2,3-d]pyrimidin-2,4(1*H*,3*H*)-dion

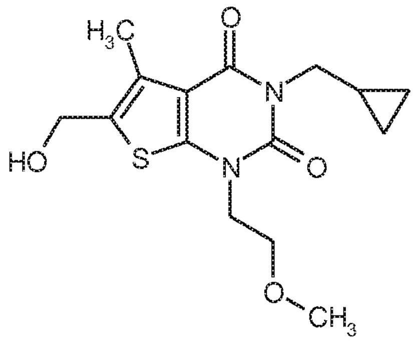

Analog zu dem unter Bsp. 138A (Methode C) beschriebenen Verfahren wurden aus 350 mg (0.86 mmol) der Verbindung aus Bsp. 29A 74 mg (26% d. Th.) der Titelverbindung hergestellt. Die Umsetzung erfolgte hier bei -40°C.
$^1$H-NMR (400 MHz, DMSO-$d_6$, $\delta$/ppm): 5.56 (t, 1H), 4.57 (d, 2H), 4.05 (t, 2H), 3.76 (d, 2H), 3.65 (t, 2H), 3.24 (s, 3H), 2.33 (s, 3H), 1.23-1.11 (m, 1H), 0.45-0.39 (m, 2H), 0.36-0.30 (m, 2H).
LC/MS (Methode 3): $R_t$ = 0.99 min, m/z = 325 $[M+H]^+$.

**Beispiel 151A**

3-(2-Fluorethyl)-6-(hydroxymethyl)-1-(2-methoxyethyl)-5-methylthieno[2,3-d]pyrimidin-2,4(1*H*,3*H*)-dion

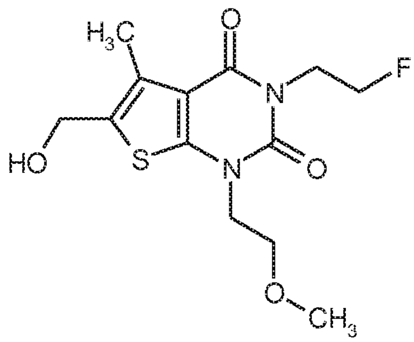

Analog zu dem unter Bsp. 138A (Methode C) beschriebenen Verfahren wurden aus 305 mg (0.78 mmol) der Verbindung aus Bsp. 30A 169 mg (66% d. Th.) der Titelverbindung hergestellt. Die Umsetzung erfolgte hier bei -40°C.
$^1$H-NMR (400 MHz, DMSO-$d_6$, $\delta$/ppm): 5.57 (t, 1H), 4.66 (t, 1H), 4.58 (d, 2H), 4.54 (t, 1H), 4.24 (t, 1H), 4.20-4.16 (m, 1H), 4.05 (t, 2H), 3.64 (t, 2H), 3.24 (s, 3H), 2.32 (s, 3H).
LC/MS (Methode 3): $R_t$ = 0.81 min, m/z = 317 $[M+H]^+$.

**Beispiel 152A**

6-(Hydroxymethyl)-1-(2-methoxyethyl)-5-methyl-3-(3,3,3-trifluorpropyl)thieno[2,3-d]pyrimidin-2,4(1*H*,3*H*)-dion

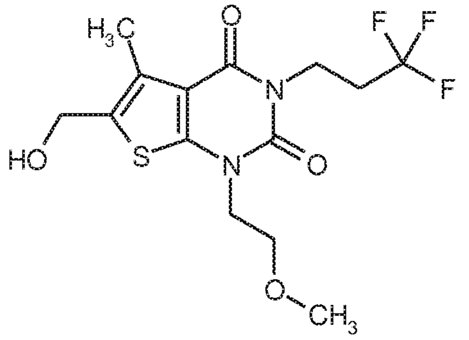

Analog zu dem unter Bsp. 138A (Methode C) beschriebenen Verfahren wurden aus 435 mg (0.86 mmol) der Verbindung aus Bsp. 31A 219 mg (68% d. Th.) der Titelverbindung hergestellt. Die Umsetzung erfolgte hier bei -40°C.
$^1$H-NMR (400 MHz, DMSO-$d_6$, δ/ppm): 5.60 (s, 1H), 4.57 (d, 2H), 4.11 (t, 2H), 4.04 (t, 2H), 3.64 (t, 2H), 3.24 (s, 3H), 2.58 (d, 2H), 2.32 (s, 3H).
LC/MS (Methode 3): $R_t$ = 1.02 min, m/z = 367 [M+H]$^+$.

**Beispiel 153A**

6-(Hydroxymethyl)-1,3-bis(2-methoxyethyl)-5-methylthieno[2,3-d]pyrimidin-2,4(1*H*,3*H*)-dion

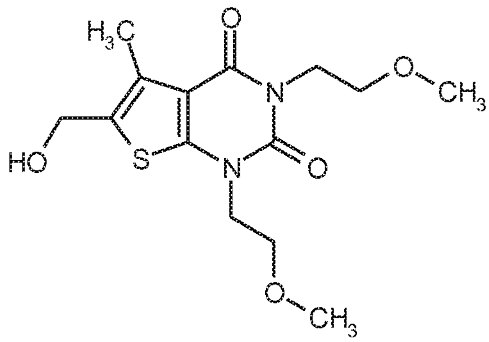

Eine Lösung von 100 mg (0.25 mmol) der Verbindung aus Bsp. 33A in 10 ml trockenem THF wurde bei 0°C mit ingesamt 0.936 ml (1.12 mmol) einer 1.2 M Lösung von Diisobutylaluminiumhydrid in Toluol versetzt. Das Reaktionsgemisch wurde bei RT gerührt. Nach vollständiger Reaktion wurden vorsichtig 30 ml 10%-ige Salzsäure und 50 ml gesättigte Kochsalz-Lösung zugegeben. Dieses Gemisch wurde mit Ethylacetat extrahiert. Die vereinigten organischen Phasen wurden über Natriumsulfat getrocknet, filtriert und eingeengt. Der erhaltene Rückstand wurde chromatographisch gereinigt. Es wurden 48 mg (55% d. Th.) der Titelverbindung erhalten.
LC/MS (Methode 3): $R_t$ = 0.8 min, m/z = 329 [M+H]$^+$.

**Beispiel 154A**

6-(Hydroxymethyl)-1-(2-methoxyethyl)-3-(1-methoxypropan-2-yl)-5-methylthieno[2,3-d]pyrimidin-2,4(1*H*,3*H*)-dion *(Racemat)*

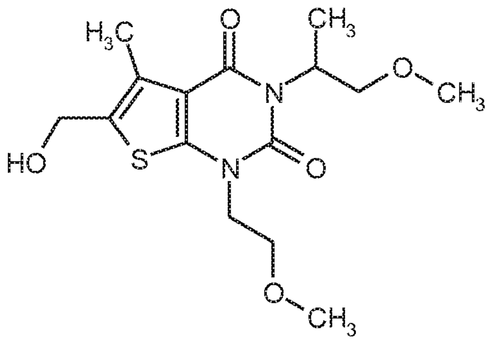

Analog zu dem unter Bsp. 138A (Methode C) beschriebenen Verfahren wurden aus 385 mg (0.89 mmol) der Verbindung aus Bsp. 34A 218 mg (68% d. Th.) der Titelverbindung hergestellt. Die Umsetzung erfolgte hier zunächst für 45 min bei -40°C, dann wurde bis zur vollständigen Reaktion bei RT weiter gerührt.
$^1$H-NMR (400 MHz, DMSO-$d_6$, $\delta$/ppm): 5.57 (t, 1H), 5.17 (d, 1H), 4.56 (d, 2H), 4.05-3.97 (m, 2H), 3.91 (dd, 1H), 3.62 (t, 2H), 3.54 (dd, 1H), 3.24 (s, 3H), 3.20 (s, 3H), 2.30 (s, 3H), 1.33 (d, 3H).
LC/MS (Methode 3): $R_t$ = 0.91 min, m/z = 343 $[M+H]^+$.

## Beispiel 155A

6-(Hydroxymethyl)-1-(2-methoxyethyl)-3-(2-methoxypropyl)-5-methylthieno[2,3-d]pyrimidin-2,4(1*H*,3*H*)-dion *(Racemat)*

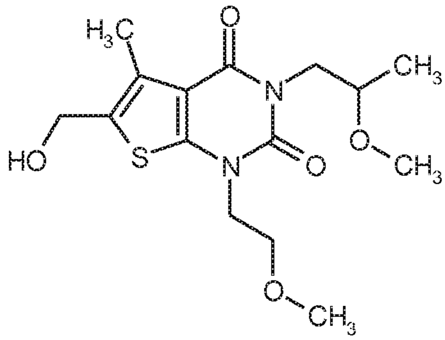

Analog zu dem unter Bsp. 138A (Methode C) beschriebenen Verfahren wurden aus 370 mg (0.83 mmol) der Verbindung aus Bsp. 35A 205 mg (67% d. Th.) der Titelverbindung hergestellt. Die Umsetzung erfolgte hier zunächst für 30 min bei -40°C, dann wurde bis zur vollständigen Reaktion bei RT weiter gerührt.
$^1$H-NMR (400 MHz, DMSO-$d_6$, $\delta$/ppm): 5.58 (t, 1H), 4.57 (d, 2H), 4.11-3.98 (m, 3H), 3.75 (dd, 1H), 3.68-3.60 (m, 3H), 3.23 (s, 3H), 3.21 (s, 3H), 2.32 (s, 3H), 1.05 (d, 3H).
LC/MS (Methode 3): $R_t$ = 0.86 min, m/z = 343 $[M+H]^+$.

## Beispiel 156A

3-(3-Fluorpropyl)-6-(hydroxymethyl)-1-(2-methoxyethyl)-5-methylthieno[2,3-d]pyrimidin-2,4(1*H*,3*H*)-dion

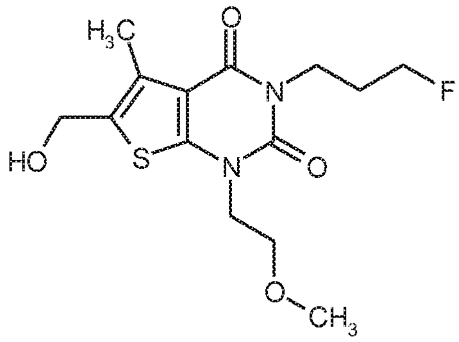

Analog zu dem unter Bsp. 138A (Methode C) beschriebenen Verfahren wurden aus 305 mg (0.7 mmol) der Verbindung aus Bsp. 32A 167 mg (71% d. Th.) der Titelverbindung hergestellt. Die Umsetzung erfolgte hier zunächst für 30 min bei -40°C, dann wurde bis zur vollständigen Reaktion bei RT weiter gerührt.
$^1$H-NMR (400 MHz, DMSO-$d_6$, δ/ppm): 5.58 (t, 1H), 4.60-4.51 (m, 3H), 4.42 (t, 1H), 4.04 (t, 2H), 3.98 (t, 2H), 3.63 (t, 2H), 3.24 (s, 3H), 2.32 (s, 3H), 2.01-1.85 (m, 2H).
LC/MS (Methode 3): $R_t$ = 0.87 min, m/z = 331 $[M+H]^+$.

**Beispiel 157A**

1-(2-Phenylethyl)pyrimidin-2,4,6(1*H*,3*H*,5*H*)-trion

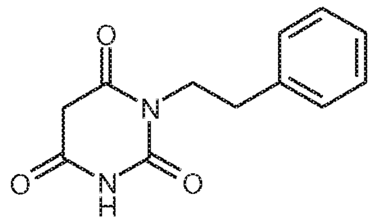

20.0 g (122 mmol) 2-Phenethylharnstoff [kommerziell erhältlich; Lit. z.B.: L. De Luca, A. Porcheddu, G. Giacomelli, I. Murgia, Synlett 2010 (16), 2439-2442] und 18.5 ml (122 mmol) Malonsäurediethylester wurden in 70 ml Ethanol gelöst und mit 45.5 ml (122 mmol) einer 20%-igen Lösung von Natriumethanolat in Ethanol versetzt. Das Gemisch wurde 16 h unter Rückfluss erhitzt. Danach wurde der Großteil des Lösungsmittels am Rotationsverdampfer entfernt und der verbliebene Rückstand mit ca. 100 ml Wasser versetzt. Vom Ungelösten wurde abfiltriert, und das Filtrat wurde mit konzentrierter Salzsäure auf pH 3-4 angesäuert. Dabei fiel das Produkt aus, das abgesaugt und zunächst mit Wasser und dann mit Hexan/Diethylether 1:1 gewaschen wurde. Nach Trocknen im Hochvakuum wurden 20.9 g (72% d. Th.) der Titelverbindung erhalten.
$^1$H-NMR (400 MHz, DMSO-$d_6$, δ/ppm): 11.36 (s, 1H), 7.33-7.29 (m, 2H), 7.25-7.20 (m, 3H), 3.86 (dd, 2H), 3.62 (s, 2H), 2.77 (dd, 2H).
LC/MS (Methode 1, ESIpos): $R_t$ = 0.76 min, m/z = 233 $[M+H]^+$.

**Beispiel 158A**

1-Ethylpyrinidin-2,4,6(1*H*,3*H*,5*H*)-trion

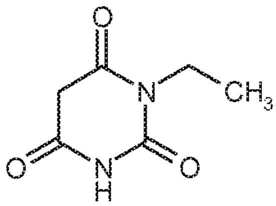

25.0 g (284 mmol) Ethylharnstoff und 43 ml (284 mmol) Malonsäurediethylester wurden in 150 ml Ethanol gelöst und mit 106 ml (284 mmol) einer 20%-igen Lösung von Natriumethanolat in Ethanol versetzt. Das Gemisch wurde 1 h unter Rückfluss erhitzt, wobei ein Niederschlag ausfiel. Nach dem Abkühlen auf RT wurde der Niederschlag abgetrennt und das Filtrat am Rotationsverdampfer vom Großteil des Lösungsmittels befreit. Der verbliebene Rückstand wurde mit ca. 500 ml Wasser versetzt und mit 5 M Salzsäure auf pH 3-4 angesäuert. Dann wurde die wässrige Lösung dreimal mit je ca. 100 ml Ethylacetat extrahiert. Nach Trocknen über wasserfreiem Magnesiumsulfat, Filtration und Eindampfen der vereinigten organischen Extrakte wurde eine erste Fraktion der Titelverbindung erhalten (14.1 g, 31% d. Th.). Die zuvor verbliebene wässrige Phase wurde bis auf ein Volumen von ca. 250 ml aufkonzentriert, mit 5 M Salzsäure auf pH 1 eingestellt und bis zur Sättigung mit festem Natriumchlorid versetzt. Es wurde erneut mit Ethylacetat extrahiert und die organische Phase über Magnesiumsulfat getrocknet, filtriert und eingedampft. Das so gewonnene Produkt wurde bei RT mit 200 ml Diethylether verrührt. Anschließend wurde filtriert und der Rückstand im Hochvakuum getrocknet. Auf diese Weise wurde eine zweite Fraktion der Titelverbindung gewonnen (6.0 g, 13% d. Th.). Insgesamt wurden so 20.1 g (45% d. Th.) der Titelverbindung erhalten.
$^1$H-NMR (400 MHz, DMSO-$d_6$, δ/ppm): 11.30 (s, 1H), 3.70 (q, 2H), 3.59 (s, 2H), 1.06 (t, 3H).
GC/MS (Methode 7, EIpos): $R_t$ = 4.28 min, m/z = 156 $[M]^+$.

**Beispiel 159A**

6-Chlor-3-(2-phenylethyl)pyrimidin-2,4(1*H*,3*H*)-dion

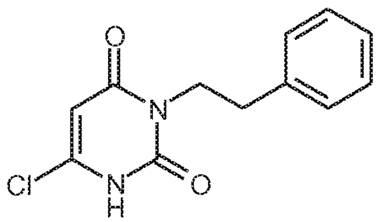

31.0 g (133 mmol) der Verbindung aus Bsp. 157A wurden in 7 ml Wasser suspendiert und anschließend über einen Zeitraum von ca. 45 min tropfenweise mit 111 ml (1.19 mol) Phosphoroxychlorid versetzt. Nach beendeter Zugabe wurde das Reaktionsgemisch zunächst 1 h auf 90°C erhitzt. Dabei entstand eine klare Lösung. Danach wurde das Reaktionsgemisch noch weitere 30 min auf 150°C erhitzt. Nach dem Abkühlen wurde der Großteil des nicht verbrauchten Phosphoroxychlorids am Rotationsverdampfer entfernt. Das verbliebene braune Öl wurde vorsichtig auf Eis gegossen. Nachdem das Eis geschmolzen war, wurde das ausgefallene Rohprodukt abgesaugt, mit Wasser neutral gewaschen, im Hochvakuum getrocknet und dann durch Verrühren in Dichlormethan gereinigt. Nach neuerlichem Absaugen und Trocknen wurden so 20.4 g (61% d. Th.) der Titelverbindung erhalten.
$^1$H-NMR (400 MHz, DMSO-$d_6$, δ/ppm): 12.37 (s, 1H), 7.32-7.28 (m, 2H), 7.23-7.19 (m, 3H), 5.90 (s, ca. 1H), 3.93 (dd, 2H), 2.79 (dd, 2H).
LC/MS (Methode 1, ESIpos): $R_t$ = 0.77 min, m/z = 251/253 $[M+H]^+$.

**Beispiel 160A**

6-Chlor-3-ethylpyrimidin-2,4(1*H*,3*H*)-dion

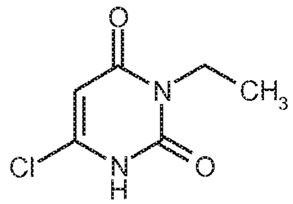

Bei einer Temperatur von 0°C wurden 28.8 ml (309 mmol) Phosphoroxychlorid vorsichtig zu 6.6 ml 50%-igem wässrigen Ethanol gegeben. Dann wurde, ebenfalls bei 0°C, portionsweise mit 5.4 g (34.6 mmol) der Verbindung aus Bsp. 158A versetzt. Nach beendeter Zugabe wurde das Reaktionsgemisch zunächst 30 min auf 50°C und dann 2 h auf 100°C erwärmt. Nachdem das Gemisch auf RT abgekühlt war, wurde es in ca. 100 ml Eiswasser gegossen. Der dabei ausgefallene Feststoff wurde abgesaugt und mit Wasser gewaschen. Nach Trocknen im Hochvakuum wurden 2.78 g (46% d. Th.) der Titelverbindung erhalten.
$^1$H-NMR (400 MHz, DMSO-$d_6$, δ/ppm): 12.34 (s, 1H), 5.89 (s, ca. 1H), 3.76 (q, 2H), 1.07 (t, 3H).
LC/MS (Methode 1, ESIpos): $R_t$ = 0.42 min, m/z = 175/177 $[M+H]^+$.

**Beispiel 161A**

1-[2,6-Dioxo-1-(2-phenylethyl)-4-(pyridinium-1-yl)-1,6-dihydropyrimidin-5(2*H*)-yliden]-2,2,2-trifluorethanolat

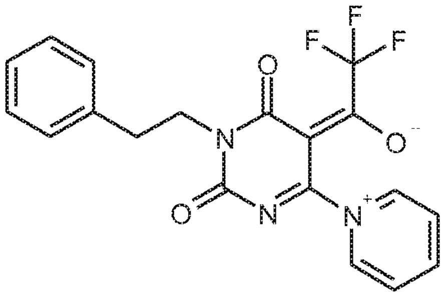

Eine Suspension von 5.0 g (19.9 mmol) der Verbindung aus Bsp. 159A in 50 ml Acetonitril wurde bei RT mit 16.1 ml (199 mmol) Pyridin versetzt. Dann wurden langsam 11.3 ml (79.8 mmol) Trifluoressigsäureanhydrid hinzugetropft. Nach beendeter Zugabe wurde das Gemisch noch ca. 45 min bei RT gerührt. Anschließend wurde mit ca. 500 ml Wasser versetzt und dreimal mit ca. 500 ml Ethylacetat extrahiert. Der organische Extrakt wurde mit gesättigter wässriger Kochsalz-Lösung gewaschen. Dann wurde der im Extrakt fein suspendierte Feststoff abgesaugt und mit wenig Ethylacetat nachgewaschen. Der Feststoff wurde im Hochvakuum getrocknet und ergab eine erste Fraktion der Titelverbindung (4.22 g, 54% d. Th.). Das erhaltene Filtrat wurde über wasserfreiem Magnesiumsulfat getrocknet, filtriert und bis auf ein Restvolumen von ca. 30 ml eingeengt. Der dabei ausgefallene Feststoff wurde erneut abgesaugt und im Hochvakuum getrocknet und ergab eine zweite Fraktion der Titelverbindung (2.63 g, 33% d. Th.). Insgesamt wurden so 6.85 g (88% d. Th.) der Titelverbindung erhalten.
$^1$H-NMR (400 MHz, DMSO-$d_6$, δ/ppm): 9.29 (d, 2H), 8.81 (t, 1H), 8.27 (t, 2H), 7.36-7.28 (m, 4H), 7.26-7.22 (m, 1H), 4.02 (dd, 2H), 2.84 (dd, 2H).
LC/MS (Methode 1, ESIpos): $R_t$ = 0.85 min, m/z = 390 $[M+H]^+$.

**Beispiel 162A**

1-[1-Ethyl-2,6-dioxo-4-(pyridinium-1-yl)-1,6-dihydropyrimidin-5(2*H*)-yliden]-2,2,2-trifluorethanolat

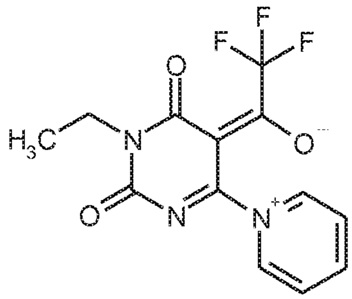

Eine Suspension von 1.0 g (5.73 mmol) der Verbindung aus Bsp. 160A in 15 ml Acetonitril wurde bei RT mit 4.6 ml (57.3 mmol) Pyridin versetzt. Dann wurden langsam 3.2 ml (22.9 mmol) Trifluoressigsäureanhydrid hinzugetropft. Nach beendeter Zugabe wurde das Gemisch noch 1 h bei RT gerührt. Anschließend wurde mit ca. 100 ml Wasser versetzt und zweimal mit je ca. 100 ml Ethylacetat extrahiert. Der organische Extrakt wurde mit gesättigter wässriger Kochsalz-Lösung gewaschen und anschließend zur Trockene eingedampft. Der verbliebene Feststoff wurde in einem Gemisch aus 25 ml Diisopropylether und 5 ml Ethylacetat 30 min lang bei 40°C verrührt. Nach dem Abkühlen auf RT wurde der Feststoff abgesaugt und mit wenig Pentan nachgewaschen. Nach Trocknen im Hochvakuum wurden 1.54 g (86% d. Th.) der Titelverbindung erhalten.
$^1$H-NMR (400 MHz, DMSO-$d_6$, δ/ppm): 9.28 (d, 2H), 8.80 (t, 1H), 8.26 (t, 2H), 3.87 (q, 2H), 1.13 (t, 3H).
LC/MS (Methode 1, ESIpos): $R_t$ = 0.56 min, m/z = 314 $[M+H]^+$.

**Beispiel 163A**

1-[1-Ethyl-2,6-dioxo-4-(pyridinium-1-yl)-1,6-dihydropyrimidin-5(2*H*)-yliden]-2,2-difluorethanolat

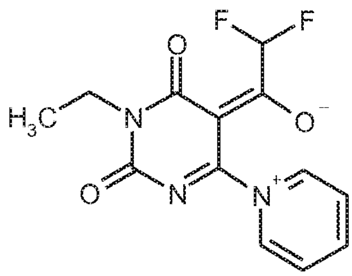

Eine Suspension von 7.5 g (43.0 mmol) der Verbindung aus Bsp. 160A in 110 ml Acetonitril wurde bei RT mit 35 ml (430 mmol) Pyridin versetzt. Dann wurden langsam 21.4 ml (172 mmol) Difluoressigsäureanhydrid hinzugetropft. Nach beendeter Zugabe wurde noch 1 h bei RT gerührt. Anschließend wurde mit ca. 300 ml Wasser versetzt und viermal mit je ca. 100 ml Ethylacetat extrahiert. Der organische Extrakt wurde mit gesättigter wässriger Kochsalz-Lösung gewaschen, über wasserfreiem Magnesiumsulfat getrocknet und anschließend zur Trockene eingeengt. Der verbliebene Feststoff wurde bei RT in einem Gemisch aus 50 ml Diisopropylether und 50 ml Diethylether verrührt. Nach Absaugen und Trocknen im Hochvakuum wurden 6.38 g (50% d. Th.) der Titelverbindung erhalten.
$^1$H-NMR (400 MHz, DMSO-$d_6$, δ/ppm): 9.20 (d, 2H), 8.80 (t, 1H), 8.25 (t, 2H), 6.97 (t, 1H), 3.89 (q, 2H), 1.14 (t, 3H).
LC/MS (Methode 1, ESIpos): $R_t$ = 0.46 min, m/z = 296 $[M+H]^+$.

**Beispiel 164A**

Ethyl-2,4-dioxo-3-(2-phenylethyl)-5-(trifluormethyl)-1,2,3,4-tetrahydrothieno[2,3-d]pyrimidin-6-carboxylat

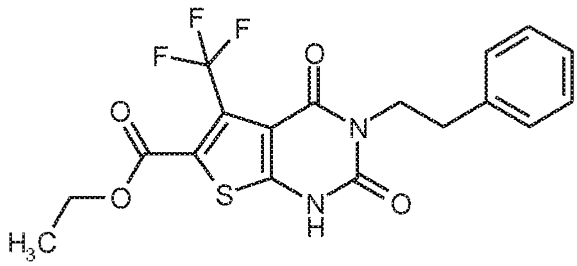

In einem Mikrowellenofen (Biotage Initiator mit dynamischer Steuerung der Einstrahlleistung) wurde, über zwei Reaktionsgefäße verteilt, ein Gemisch aus 4.21 g (10.8 mmol) der Verbindung aus Bsp. 161A, 2.52 g (23.8 mmol) Natriumcarbonat und 2.4 ml (21.7 mmol) Mercaptoessigsäureethylester in 26 ml Ethanol 1 h lang auf 120°C erhitzt. Anschließend wurden die beiden Ansätze vereinigt und am Rotationsverdampfer zur Trockene eingedampft. Der verbliebene Rückstand wurde in ca. 700 ml Ethylacetat aufgenommen und nacheinander mit je ca. 300 ml halbgesättigter wässriger Ammoniumchlorid-Lösung, Wasser und gesättigter Kochsalz-Lösung gewaschen. Nach Trocknen über wasserfreiem Magnesiumsulfat wurde filtriert und eingedampft. Das Rohprodukt wurde mittels MPLC gereinigt (Puriflash-Kartusche, 100 g Kieselgel, Cyclohexan/Ethylacetat 5:1 → 1:1). Nach Vereinigen und Eindampfen der Produktfraktionen und Trocknen des Rückstands im Hochvakuum wurden 3.2 g (71% d. Th.) der Titelverbindung erhalten.
$^1$H-NMR (400 MHz, DMSO-$d_6$, δ/ppm): 12.61 (s, 1H), 7.33-7.29 (m, 2H), 7.26-7.20 (m, 3H), 4.33 (q, 2H), 4.02 (m, 2H), 2.83 (m, 2H), 1.29 (t, 3H).
LC/MS (Methode 1, ESIpos): $R_t$ = 1.12 min, m/z = 413 $[M+H]^+$.

**Beispiel 165A**

Ethyl-3-ethyl-2,4-dioxo-5-(trifluormethyl)-1,2,3,4-tetrahydrothieno[2,3-d]pyrimidin-6-carboxylat

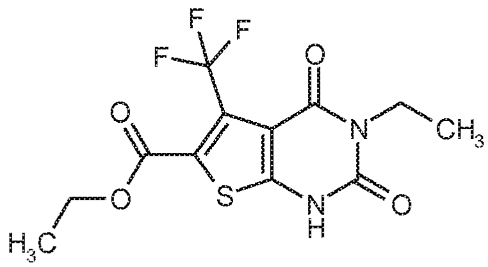

In einem Mikrowellenofen (Biotage Initiator mit dynamischer Steuerung der Einstrahlleistung) wurde ein Gemisch aus 4.75 g (15.2 mmol) der Verbindung aus Bsp. 162A, 3.54 g (33.4 mmol) Natriumcarbonat und 3.3 ml (30.3 mmol) Mercaptoessigsäureethylester in 39 ml Ethanol 1 h lang auf 120°C erhitzt. Anschließend wurde am Rotationsverdampfer zur Trockene eingedampft. Der verbliebene Rückstand wurde mit ca. 200 ml Wasser versetzt und mit Essigsäure schwach sauer gestellt (ca. pH 4). Es wurde dreimal mit je ca. 100 ml Dichlormethan extrahiert. Nach Waschen der vereinigten organischen Phasen mit gesättigter Natriumchlorid-Lösung und Trocknen über wasserfreiem Magnesiumsulfat wurde filtriert und eingedampft. Das Rohprodukt wurde mittels MPLC gereinigt (Puriflash-Kartusche, 25 g Kieselgel, Cyclohexan/Ethylacetat 3:1 → 1:1). Nach Vereinigen und Eindampfen der Produktfraktionen und Trocknen des Rückstands im Hochvakuum wurden 2.78 g (54% d. Th.) der Titelverbindung erhalten.
$^1$H-NMR (400 MHz, $CDCl_3$, δ/ppm): 11.38 (s, 1H), 4.40 (q, 2H), 4.10 (q, 2H), 1.39 (t, 3H), 1.29 (t, 3H).
LC/MS (Methode 4, ESIpos): $R_t$ = 2.06 min, m/z = 337 $[M+H]^+$.

**Beispiel 166A**

Ethyl-5-(difluormethyl)-3-ethyl-2,4-dioxo-1,2,3,4-tetrahydrothieno[2,3-d]pyrimidin-6-carboxylat

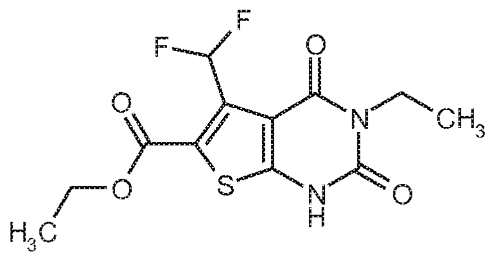

Eine Suspension von 2.04 g (6.92 mmol) der Verbindung aus Bsp. 163A in 15 ml Ethanol wurde mit 1.5 ml (13.8 mmol) Mercaptoessigsäureethylester versetzt und 5 min bei RT gerührt. Dann wurden 1.61 g (15.2 mmol) Natriumcarbonat hinzugefügt, und das Gemisch wurde in einem Mikrowellenofen (Biotage Initiator mit dynamischer Steuerung der Einstrahlleistung) 1 h lang auf 120°C erhitzt. Drei solcher Ansätze wurden vereinigt und am Rotationsverdampfer zur Trockene eingeengt. Der verbliebene Rückstand wurde in ca. 300 ml Wasser aufgenommen, durch Zugabe von Essigsäure schwach sauer gestellt und dreimal mit je ca. 100 ml Dichlormethan extrahiert. Der organische Extrakt wurde mit gesättigter wässriger Natriumchlorid-Lösung gewaschen, über wasserfreiem Magnesiumsulfat getrocknet, filtriert und eingeengt. Der hierbei verbliebene Feststoff wurde über eine Kieselgel-Kartusche chromatographiert (Puriflash, Cyclohexan/Ethylacetat 3:1 → 1:1). Nach Eindampfen der Produktfraktionen und Trocknen des Rückstands im Hochvakuum wurden 890 mg (12% d. Th.) der Titelverbindung erhalten.
$^1$H-NMR (400 MHz, DMSO-$d_6$, δ/ppm): 12.59 (s, 1H), 7.71 (t, 1H), 4.32 (q, 2H), 3.87 (q, 2H), 1.30 (t, 3H), 1.13 (t, 3H).
LC/MS (Methode 1, ESIpos): $R_t$ = 0.87 min, m/z = 319 $[M+H]^+$.

__Beispiel 167A__

Ethyl-1-(2-methoxyethyl)-2,4-dioxo-3-(2-phenylethyl)-5-(trifluonnethyl)-1,2,3,4-tetrahydrothieno-[2,3-d]pyrimidin-6-carboxylat

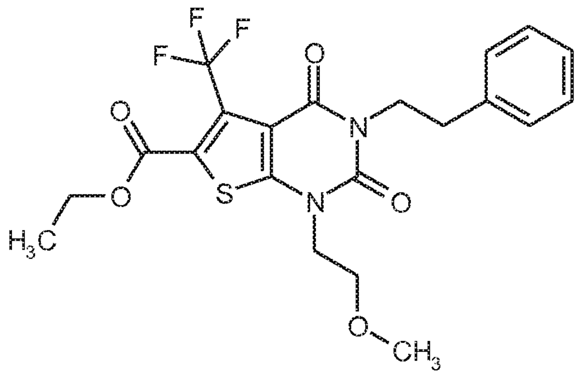

Eine Lösung von 3.15 g (7.64 mmol) der Verbindung aus Bsp. 164A in 60 ml DMF wurde mit 1.78 g (16.8 mmol) Cäsiumcarbonat versetzt und 15 min bei RT gerührt. Dann wurden 2.12 g (15.3 mmol) 2-Bromethyl-methylether hinzugefügt, und das Gemisch wurde 5.5 h auf 80°C erwärmt. Nach dem Abkühlen wurde es am Rotationsverdampfer zur Trockene eingeengt. Der erhaltene Rückstand wurde in 400 ml Ethylacetat aufgeschlämmt und nacheinander mit Wasser und gesättigter wässriger Natriumchlorid-Lösung gewaschen. Nach Trocknen über wasserfreiem Magnesiumsulfat wurde filtriert und eingeengt. Das Rohprodukt wurde mittels MPLC über eine Biotage-Kartusche gereinigt (100 g Kieselgel, Cyclohexan/Ethylacetat 5:1 → 1:1). Die Produktfraktionen wurden vereinigt, eingeengt und im Hochvakuum getrocknet. Das ergab eine erste Teilmenge von 2.28 g (63% d. Th.) der Titelverbindung. Daneben wurde noch eine Mischfraktion aus *N*- und *O*-alkyliertem Produkt erhalten, das mittels präparativer HPLC (Methode 8) aufgetrennt wurde und eine zweite Teilmenge von 0.55 g (14% d. Th.) der Titelverbindung ergab. Insgesamt wurden so 2.83 g (78% d. Th.) der Titelverbindung erhalten.
$^1$H-NMR (400 MHz, DMSO-$d_6$, δ/ppm): 7.32-7.28 (m, 2H), 7.25-7.20 (m, 3H), 4.35 (q, 2H), 4.12-4.06 (m, 4H), 3.63 (t, 2H), 3.25 (s, 3H), 2.85 (m, 2H), 1.30 (t, 3H).
LC/MS (Methode 1, ESIpos): $R_t$ = 1.24 min, m/z = 471 $[M+H]^+$.

**Beispiel 168A**

Ethyl-3-ethyl-2,4-dioxo-5-(trifluormethyl)-1-(3,3,3-trifluorpropyl)-1,2,3,4-tetrahydrothieno[2,3-d]-pyrimidin-6-carboxylat

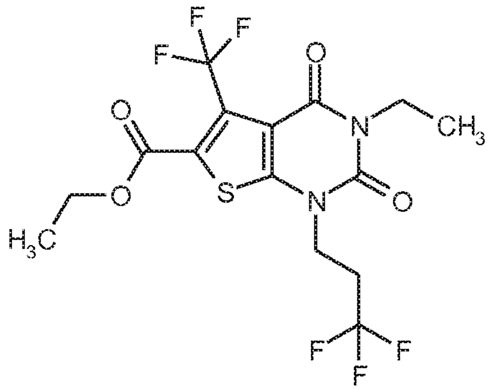

Eine Lösung von 2.0 g (5.95 mmol) der Verbindung aus Bsp. 165A in 40 ml DMF wurde mit 1.39 g (13.1 mmol) Cäsiumcarbonat versetzt und 15 min bei RT gerührt. Dann wurden 2.66 g (11.9 mmol) 3,3,3-Trifluor-1-iodpropan hinzugefügt, und das Gemisch wurde auf 60°C erwärmt. Nach 1 h wurden weitere 2.66 g (11.9 mmol) 3,3,3-Trifluor-1-iodpropan hinzugefügt. Das Rühren bei 60°C wurde 16 h lang fortgesetzt. Nach dem Abkühlen auf RT wurde mit ca. 160 ml Wasser versetzt und dreimal mit je ca. 80 ml Diethylether extrahiert. Die vereinigten organischen Extrakte wurden mit gesättigter Natriumchlorid-Lösung gewaschen. Nach Trocknen über wasserfreiem Magnesiumsulfat wurde filtriert und eingedampft. Das Rohprodukt wurde mittels MPLC über eine Puriflash-Kartusche gereinigt (100 g Kieselgel, Cyclohexan/Ethylacetat 7:1 → 1:1). Die Produktfraktionen wurden vereinigt, eingedampft und der Rückstand im Hochvakuum getrocknet. Es wurden 1.86 g (72% d. Th.) der Titelverbindung erhalten.

$^1$H-NMR (400 MHz, $CDCl_3$, δ/ppm): 4.41 (q, 2H), 4.20 (t, 2H), 4.08 (q, 2H), 2.73-2.61 (m, 2H), 1.40 (t, 3H), 1.26 (t, 3H).

LC/MS (Methode 1, ESIpos): $R_t$ = 1.15 min, m/z = 433 $[M+H]^+$.

**Beispiel 169A**

Ethyl-3-ethyl-1-(3-fluorpropyl)-2,4-dioxo-5-(trifluormethyl)-1,2,3,4-tetrahydrothieno[2,3-d]-pyrimidin-6-carboxylat

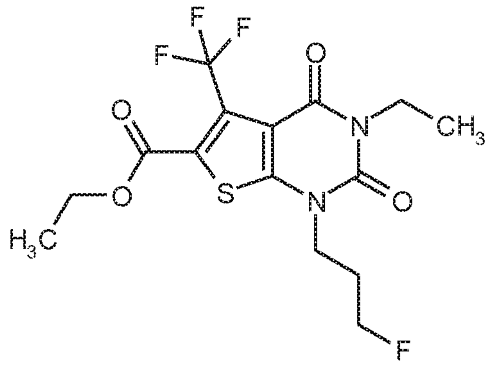

Analog zu dem unter Bsp. 86A beschriebenen Verfahren wurden aus 500 mg (1.48 mmol) der Verbindung aus Bsp. 165A und 838 mg (4.46 mmol) 1-Fluor-3-iodpropan 519 mg (84% d. Th.) der Titelverbindung hergestellt. Die Umsetzung wurde hier bei RT durchgeführt und die Reaktionsdauer betrug 24 h.

$^1$H-NMR (400 MHz, DMSO-$d_6$, δ/ppm): 4.62 (t, 1H), 4.50 (t, 1H), 4.35 (q, 2H), 4.06 (t, 2H), 3.91 (q, 2H), 2.17-2.02 (m, 2H), 1.30 (t, 3H), 1.13 (t, 3H).

LC/MS (Methode 3): $R_t$ = 1.28 min, m/z = 397 $[M+H]^+$.

**Beispiel 170A**

Ethyl-5-(difluormethyl)-3-ethyl-2,4-dioxo-1-(3,3,4,4,4-pentafluorbutyl)-1,2,3,4-tetrahydrothieno-[2,3-d]pyrimidin-6-carboxylat

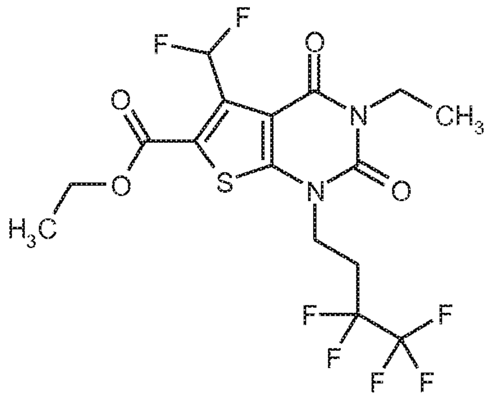

Analog zu dem unter Bsp. 86A beschriebenen Verfahren wurden aus 294 mg (0.92 mmol) der Verbindung aus Bsp. 166A und 783 mg (2.77 mmol) 1,1,1,2,2-Pentafluor-4-iodbutan 223 mg (48% d. Th.) der Titelverbindung hergestellt. Die Reaktionsdauer betrug hier 17 h.
$^1$H-NMR (400 MHz, DMSO-$d_6$, $\delta$/ppm): 7.91-7.58 (m, 1H), 4.39-4.30 (m, 2H), 4.28-4.20 (m, 2H), 3.92 (q, 2H), 2.83-2.65 (m, 2H), 1.32 (t, 3H), 1.14 (t, 3H).
LC/MS (Methode 3): $R_t$ = 1.44 min, m/z = 465 $[M+H]^+$.

**Beispiel 171A**

Ethyl-3-ethyl-1-(2-methoxyethyl)-2,4-dioxo-5-(trifluormethyl)-1,2,3,4-tetrahydrothieno[2,3-d]-pyrimidin-6-carboxylat

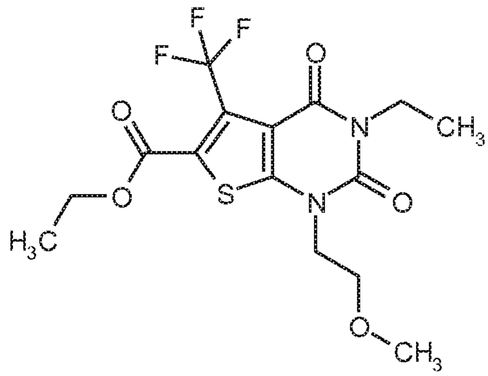

Analog zu dem unter Bsp. 168A beschriebenen Verfahren wurden aus 1.77 g (5.26 mmol) der Verbindung aus Bsp. 165A und 1 ml (10.5 mmol) 2-Bromethyl-methylether 1.34 g (64% d. Th.) der Titelverbindung erhalten. Die Umsetzung wurde 2 h bei 80°C durchgeführt, und als Laufmittel bei der MPLC-Reinigung wurde Cyclohexan/Ethylacetat 5:1 verwendet. Zur finalen Aufreinigung wurde das Produkt nochmals in Pentan/Dichlormethan 20:1 verrührt.
$^1$H-NMR (400 MHz, DMSO-$d_6$, $\delta$/ppm): 4.34 (q, 2H), 4.11 (t, 2H), 3.91 (q, 2H), 3.66 (t, 2H), 3.25 (s, 3H), 1.30 (t, 3H), 1.14 (t, 3H).
LC/MS (Methode 1, ESIpos): $R_t$ = 1.01 min, m/z = 395 $[M+H]^+$.

**Beispiel 172A**

Ethyl-5-(difluormethyl)-3-ethyl-2,4-dioxo-1-(3,3,3-trifluorpropyl)-1,2,3,4-tetrahydrothieno[2,3-d]-pyrimidin-6-carboxylat

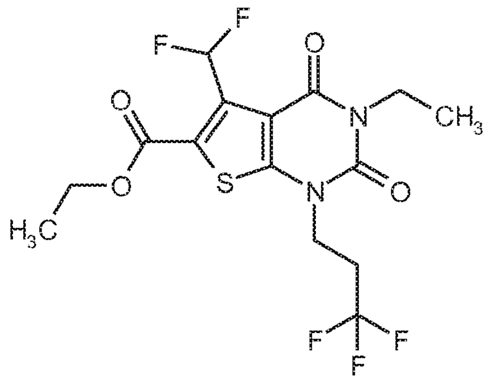

Eine Lösung von 880 mg (2.76 mmol) der Verbindung aus Bsp. 166A in 12 ml DMF wurde mit 1.35 g (4.15 mmol) Cäsiumcarbonat versetzt und 20 min bei RT gerührt. Dann wurden 929 mg (4.15 mmol) 3,3,3-Trifluor-1-iodpropan hinzugefügt, und das Gemisch wurde 2 h auf 80°C erwärmt. Nach dem Abkühlen auf RT wurde mit ca. 100 ml Ethylacetat verdünnt und nacheinander zweimal mit je ca. 100 ml Wasser und einmal mit ca. 100 ml gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 9). Die Produktfraktionen wurden vereinigt, eingeengt und im Hochvakuum getrocknet. Es wurden 750 mg (63% d. Th.) der Titelverbindung erhalten.

$^1$H-NMR (400 MHz, DMSO-$d_6$, $\delta$/ppm): 7.74 (t, 1H), 4.36 (q, 2H), 4.20 (t, 2H), 3.92 (q, 2H), 2.88-2.76 (m, 2H), 1.32 (t, 3H), 1.14 (t, 3H).

LC/MS (Methode 1, ESIpos): $R_t$ = 1.13 min, m/z = 415 [M+H]$^+$.

**Beispiel 173A**

Ethyl-5-(difluormethyl)-3-ethyl-1-(2-methoxyethyl)-2,4-dioxo-1,2,3,4-tetrahydrothieno[2,3-d]-pyrimidin-6-carboxylat

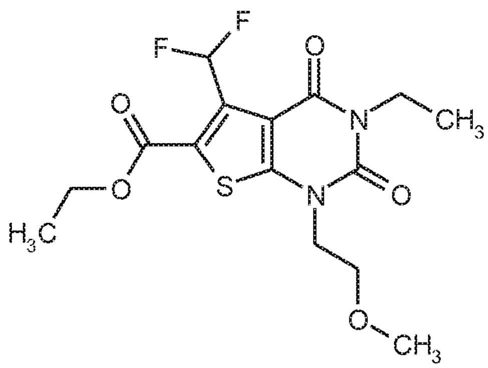

Analog zu dem unter Bsp. 86A beschriebenen Verfahren wurden aus 2 g (6.31 mmol) der Verbindung aus Bsp. 166A und 2.71 g (18.9 mmol) 2-Bromethyl-methylether 1.72 g (58% d. Th.) der Titelverbindung hergestellt. Die Reaktionsdauer betrug hier 12 h.

$^1$H-NMR (400 MHz, DMSO-$d_6$, $\delta$/ppm): 7.88-7.58 (m, 1H), 4.34 (q, 2H), 4.12 (t, 2H), 3.92 (q, 2H), 3.66 (t, 2H), 3.25 (s, 3H), 1.31 (t, 3H), 1.14 (t, 3H).

LC/MS (Methode 3): $R_t$ = 1.22 min, m/z = 377 [M+H]$^+$.

**Beispiel 174A**

6-(Hydroxymethyl)-1-(2-methoxyethyl)-3-(2-phenylethyl)-5-(trifluormethyl)thieno[2,3-d]pyrimidin-2,4(1*H*,3*H*)-dion

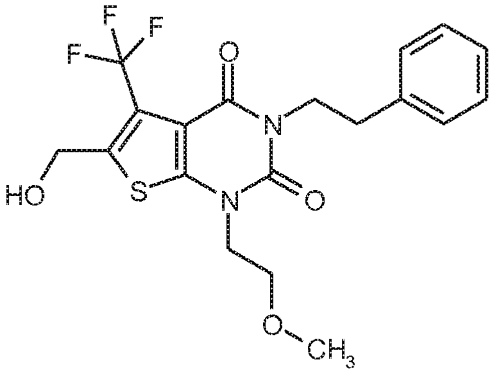

Eine Lösung von 2.65 g (5.63 mmol) der Verbindung aus Bsp. 167A in 55 ml trockenem THF wurde bei 0°C tropfenweise mit 5.6 ml (5.63 mmol) einer 1 M Lösung von Lithiumaluminiumhydrid in THF versetzt. Nach 10 min bei 0°C wurde überschüssiges Lithiumaluminiumhydrid durch Zugabe von 100 μl Wasser vernichtet. Dann wurde mit 300 μl 1 M Natronlauge versetzt. Es wurde etwas Kieselgur hinzugefügt und nach 2 min wasserfreies Magnesiumsulfat. Anschließend wurde vom Ungelösten abgesaugt und der Rückstand gründlich mit THF gewaschen. Das Filtrat wurde zur Trockene eingeengt. Der erhaltene Rückstand wurde mit Pentan/Ethylacetat verrührt. Durch Absaugen des Feststoffs und Trocknen im Hochvakuum wurde eine erste Portion der Titelverbindung gewonnen (1.35 g, 55% d. Th.). Das Filtrat wurde eingedampft und der Rückstand mittels MPLC gereinigt (Biotage-Kartusche, 50 g Kieselgel, Cyclohexan/Ethylacetat 2:1). Nach Einengen der Produktfraktionen, Verrühren des Rückstands mit Pentan, Absaugen des Feststoffs und Trocknen im Hochvakuum wurde eine zweite Portion der Titelverbindung erhalten (0.56 g, 23% d. Th.). Insgesamt betrug so die Ausbeute der Titelverbindung 1.91 g (78% d. Th.).
$^1$H-NMR (400 MHz, DMSO-$d_6$, δ/ppm): 7.32-7.28 (m, 2H), 7.24-7.20 (m, 3H), 6.46 (s, 1H), 4.82 (s, 2H), 4.10-4.05 (m, 4H), 3.62 (t, 2H), 3.25 (s, 3H), 2.84 (m, 2H).
LC/MS (Methode 1, ESIpos): $R_t$ = 1.06 min, m/z = 429 $[M+H]^+$.

**Beispiel 175A**

3-Ethyl-6-(hydroxymethyl)-5-(trifluormethyl)-1-(3,3,3-trifluorpropyl)thieno[2,3-d]pyrimidin-2,4(1*H*,3*H*)-dion

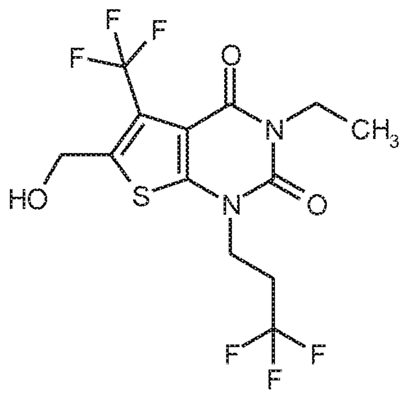

Analog zu dem unter Bsp. 138A (Methode B) beschriebenen Verfahren wurden aus 607 mg (1.40 mmol) der Verbindung aus Bsp. 168A 456 mg (82% d. Th.) der Titelverbindung erhalten. Die MPLC-Reinigung erfolgte hier über eine Biotage-Kartusche mit 50 g Kieselgel und Cyclohexan/ Ethylacetat 3:1 → 1:1 als Laufmittel.
$^1$H-NMR (400 MHz, $CDCl_3$, δ/ppm): 5.06 (m, 2H), 4.20 (dd, 2H), 4.08 (q, 2H), 2.72-2.60 (m, 2H), 2.53 (t, 1H), 1.26 (t, 3H).

LC/MS (Methode 1, ESIpos): $R_t$ = 0.96 min, m/z = 391 $[M+H]^+$.

**Beispiel 176A**

3-Ethyl-1-(3-fluorpropyl)-6-(hydroxymethyl)-5-(trifluormethyl)thieno[2,3-d]pyrimidin-2,4(1*H*,3*H*)-dion

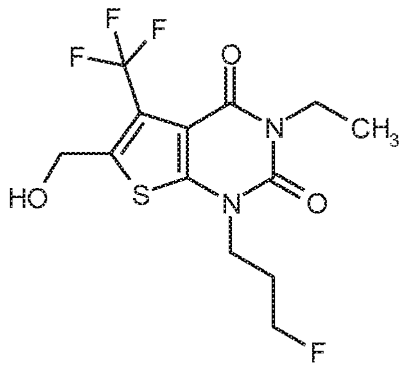

Analog zu dem unter Bsp. 138A (Methode B) beschriebenen Verfahren wurden aus 540 mg (1.31 mmol) der Verbindung aus Bsp. 169A 360 mg (77% d. Th.) der Titelverbindung hergestellt.
$^1$H-NMR (400 MHz, DMSO-$d_6$, δ/ppm): 6.48 (s, 1H), 4.82 (br. s, 2H), 4.61 (t, 1H), 4.50 (t, 1H), 4.05 (t, 2H), 3.90 (q, 2H), 2.18-2.00 (m, 2H), 1.12 (t, 3H).
LC/MS (Methode 3): $R_t$ = 1.01 min, m/z = 355 $[M+H]^+$.

**Beispiel 177A**

5-(Difluormethyl)-3-ethyl-6-(hydroxymethyl)-1-(3,3,4,4,4-pentafluorbutyl)thieno[2,3-d]pyrimidin-2,4(1*H*,3*H*)-dion

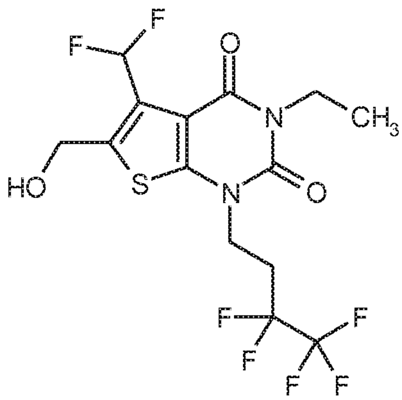

Analog zu dem unter Bsp. 138A (Methode C) beschriebenen Verfahren wurden aus 337 mg (0.73 mmol) der Verbindung aus Bsp. 170A 218 mg (66% d. Th.) der Titelverbindung hergestellt.
$^1$H-NMR (400 MHz, DMSO-$d_6$, δ/ppm): 7.59-7.27 (m, 1H), 6.28 (s, 1H), 4.83 (br. s, 2H), 4.25-4.17 (m, 2H), 3.92 (q, 2H), 2.81-2.64 (m, 2H), 1.13 (t, 3H).
LC/MS (Methode 3): $R_t$ = 1.23 min, m/z = 423 $[M+H]^+$.

**Beispiel 178A**

3-Ethyl-6-(hydroxymethyl)-1-(2-methoxyethyl)-5-(trifluormethyl)thieno[2,3-d]pyrimidin-2,4(1*H*,3*H*)-dion

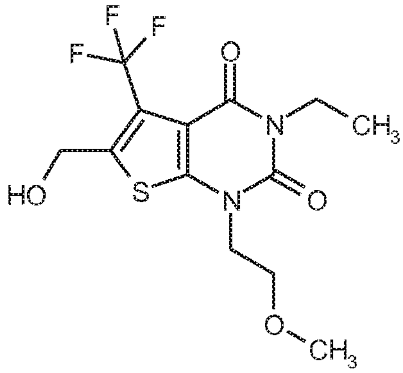

Analog zu dem unter Bsp. 138A (Methode B) beschriebenen Verfahren wurden aus 600 mg (1.52 mmol) der Verbindung aus Bsp. 171A 420 mg (78% d. Th.) der Titelverbindung erhalten. Die MPLC-Reinigung erfolgte hier mit dem Laufmittelgradienten Cyclohexan/Ethylacetat 2:1 → 1:1.
$^1$H-NMR (400 MHz, DMSO-$d_6$, δ/ppm): 5.04 (dd, 2H), 4.14 (t, 2H), 4.08 (q, 2H), 3.74 (t, 2H), 3.35 (s, 3H), 2.53 (t, 1H), 1.26 (t, 3H).
LC/MS (Methode 1, ESIpos): $R_t$ = 0.83 min, m/z = 353 [M+H]$^+$.

**Beispiel 179A**

5-(Difluormethyl)-3-ethyl-6-(hydroxymethyl)-1-(3,3,3-trifluorpropyl)thieno[2,3-d]pyrimidin-2,4(1*H*,3*H*)-dion

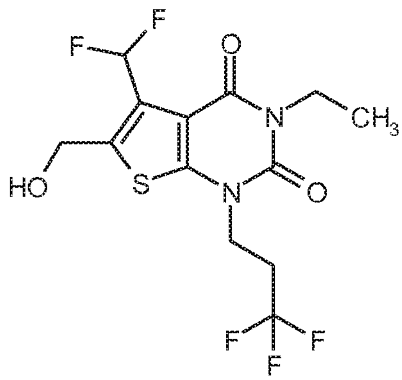

Analog zu dem unter Bsp. 138A (Methode C) beschriebenen Verfahren wurden aus 400 mg (0.96 mmol) der Verbindung aus Bsp. 172A 295 mg (82% d. Th.) der Titelverbindung hergestellt. Die Umsetzung erfolgte bei -40°C.
$^1$H-NMR (400 MHz, DMSO-$d_6$, δ/ppm): 7.61-7.26 (m, 1H), 6.30 (t, 1H), 4.82 (d, 2H), 4.16 (t, 2H), 3.91 (q, 2H), 2.86-2.72 (m, 2H), 1.13 (t, 3H).
LC/MS (Methode 3): $R_t$ = 1.09 min, m/z = 373 [M+H]$^+$.

**Beispiel 180A**

5-(Difluormethyl)-3-ethyl-6-(hydroxymethyl)-1-(2-methoxyethyl)thieno[2,3-d]pyrimidin-2,4(1*H*,3*H*)-dion

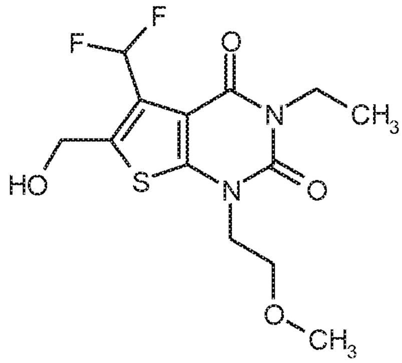

Analog zu dem unter Bsp. 138A (Methode C) beschriebenen Verfahren wurden aus 1.32 g (3.51 mmol) der Verbindung aus Bsp. 173A 1.03 g (86% d. Th.) der Titelverbindung hergestellt. Die Umsetzung erfolgte bei -40°C.
$^1$H-NMR (400 MHz, DMSO-$d_6$, δ/ppm): 7.58-7.27 (m, 1H), 6.23 (t, 1H), 4.80 (br. s, 2H), 4.08 (t, 2H), 3.91 (q, 2H), 3.65 (t, 2H), 3.25 (s, 3H), 1.13 (t, 3H).
LC/MS (Methode 3): $R_t$ = 0.95 min, m/z = 335 $[M+H]^+$.

**Beispiel 181A**

6-(Chlormethyl)-1-(2-methoxyethyl)-3-(2-phenylethyl)-5-(trifluormethyl)thieno[2,3-d]pyrimidin-2,4(1*H*,3*H*)-dion

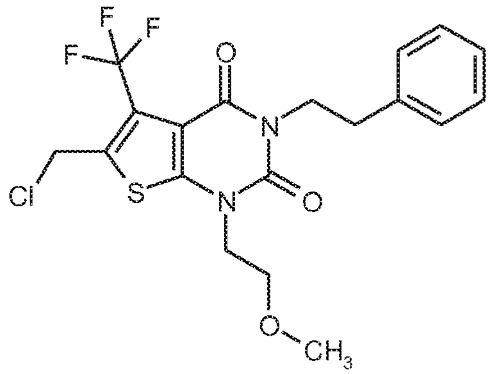

1.30 g (3.03 mmol) der Verbindung aus Bsp. 174A wurden in 13 ml Chloroform gelöst und mit 443 µl (6.07 mmol) Thionylchlorid versetzt. Das Reaktionsgemisch wurde in einem Mikrowellenofen (Biotage-Initiator mit dynamischer Steuerung der Einstrahlleistung) 30 min lang auf 80°C erhitzt. Anschließend wurden alle flüchtigen Bestandteile am Rotationsverdampfer entfernt. Der erhaltene Rückstand wurde zweimal mit Toluol aufgenommen und jeweils wieder eingeengt. Nach Trocknen im Hochvakuum wurden 1.34 g (99% d. Th.) der Titelverbindung erhalten.
$^1$H-NMR (400 MHz, DMSO-$d_6$, δ/ppm): 7.33-7.28 (m, 2H), 7.25-7.20 (m, 3H), 5.15 (s, 2H), 4.09-4.04 (m, 4H), 3.63 (t, 2H), 3.25 (s, 3H), 2.84 (m, 2H).
LC/MS (Methode 1, ESIpos): $R_t$ = 1.22 min, m/z = 447/449 $[M+H]^+$.

**Beispiel 182A**

6-(Chlormethyl)-3-ethyl-5-(trifluormethyl)-1-(3,3,3-trifluorpropyl)thieno[2,3-d]pyrimidin-2,4(1*H*,3*H*)-dion

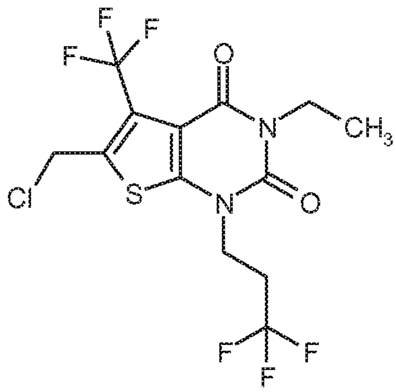

Analog zu dem unter Bsp. 181A beschriebenen Verfahren wurden aus 580 mg (1.48 mmol) der Verbindung aus Bsp. 175A und 217 μl (2.97 mmol) Thionylchlorid 505 mg (83% d. Th.) der Titelverbindung erhalten. Zusätzlich erfolgte hier noch eine Reinigung des Produkts mittels MPLC (Biotage-Kartusche, 50 g Kieselgel, Cyclohexan/Ethylacetat 10:1 → 5:1).
$^1$H-NMR (400 MHz, $CDCl_3$, δ/ppm): 4.90 (s, 2H), 4.19 (dd, 2H), 4.08 (q, 2H), 2.73-2.60 (m, 2H), 1.26 (t, 3H).
LC/MS (Methode 1, ESIpos): $R_t$ = 1.13 min, m/z = 409/411 $[M+H]^+$.

**Beispiel 183A**

6-(Chlormethyl)-3-ethyl-1-(3-fluorpropyl)-5-(trifluormethyl)thieno[2,3-d]pyrimidin-2,4(1*H*,3*H*)-dion

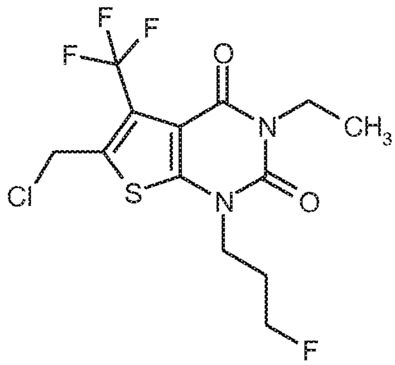

355 mg (1 mmol) der Verbindung aus Bsp. 176A wurden in 3.55 ml Chloroform gelöst und mit 241 mg (2 mmol) Thionylchlorid versetzt. Das Gemisch wurde 30 min in einer Mikrowellenapparatur bei einer Temperatur von 80°C gerührt. Die auf RT abgekühlte Lösung wurde dann am Rotationsverdampfer eingeengt. Der verbliebene Rückstand wurde über eine Kieselgel-Kartusche chromatographiert (Biotage, 50 g Kieselgel, Laufmittel Cyclohexan/Ethylacetat). Es wurden 343 mg (89% d. Th.) der Titelverbindung erhalten.
$^1$H-NMR (400 MHz, DMSO-$d_6$, δ/ppm): 5.16 (d, 1H), 4.62 (t, 1H), 4.50 (t, 1H), 4.03 (t, 2H), 3.90 (q, 2H), 2.17-2.02 (m, 2H), 1.12 (t, 3H).
LC/MS (Methode 3): $R_t$ = 1.24 min, m/z = 373 $[M+H]^+$.

**Beispiel 184A**

6-(Chlormethyl)-3-ethyl-1-(2-methoxyethyl)-5-(trifluormethyl)thieno[2,3-d]pyrimidin-2,4(1*H*,3*H*)-dion

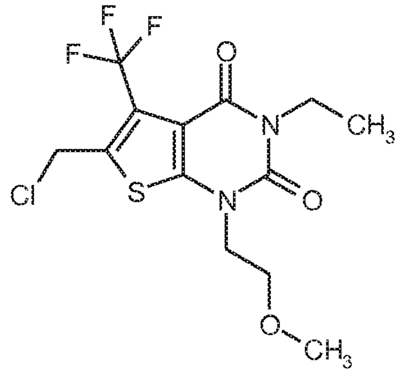

Analog zu dem unter Bsp. 181A beschriebenen Verfahren wurden aus 376 mg (10.7 mmol) der Verbindung aus Bsp. 178A und 234 μl (3.20 mmol) Thionylchlorid 395 mg (99% d. Th.) der Titelverbindung erhalten.
$^1$H-NMR (400 MHz, DMSO-$d_6$, δ/ppm): 5.14 (s, 2H), 4.08 (t, 2H), 3.91 (q, 2H), 3.66 (t, 2H), 3.25 (s, 3H), 1.13 (t, 3H).
LC/MS (Methode 1, ESIpos): $R_t$ = 0.97 min, m/z = 371/373 $[M+H]^+$.

**Beispiel 185A**

5-(Difluormethyl)-3-ethyl-2,4-dioxo-1-(3,3,3-trifluorpropyl)-1,2,3,4-tetrahydrothieno[2,3-d]-pyrimidin-6-carbaldehyd

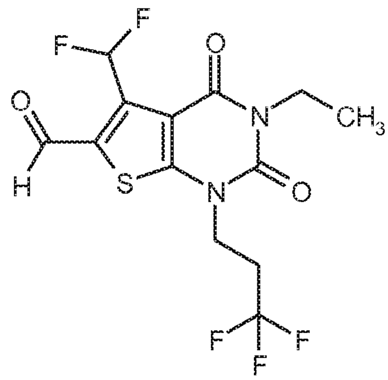

1.48 g (3.65 mmol) der Verbindung aus Bsp. 179A wurden in 120 ml Dichlormethan gelöst und mit 3.53 g (3.65 mmol) Mangan(IV)oxid versetzt. Das Gemisch wurde 16 h bei RT gerührt. Dann wurde über Celite abgesaugt und das Filtrat zur Trockene eingeengt. Der erhaltene Rückstand wurde über eine Kieselgel-Kartusche chromatographiert (Biotage, 100 g Kieselgel, Laufmittel Hexan/ Ethylacetat). Es wurden 1.31 g (85% d. Th.) der Titelverbindung erhalten.
$^1$H-NMR (400 MHz, DMSO-$d_6$, δ/ppm): 10.19 (s, 1H), 7.85-7.57 (m, 1H), 4.22 (t, 2H), 3.93 (q, 2H), 2.88-2.75 (m, 2H), 1.15 (t, 3H).
LC/MS (Methode 3): $R_t$ = 1.25 min, m/z = 371 $[M+H]^+$.

**Beispiel 186A**

5-(Difluormethyl)-3-ethyl-1-(2-methoxyethyl)-2,4-dioxo-1,2,3,4-tetrahydrothieno[2,3-d]pyrimidin-6-carbaldehyd

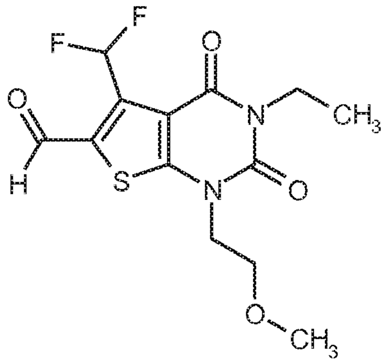

1.02 g (3.03 mmol) der Verbindung aus Bsp. 180A wurden in 50 ml Dichlormethan gelöst und mit 2.93 g (30.35 mmol) Mangan(IV)oxid versetzt. Das Gemisch wurde 22 h bei RT gerührt. Dann wurde über Celite abgesaugt und das Filtrat zur Trockene eingeengt. Es wurden 993 mg (97% d. Th.) der Titelverbindung erhalten.
$^1$H-NMR (400 MHz, DMSO-$d_6$, $\delta$/ppm): 10.18 (s, 1H), 7.86-7.54 (m, 1H), 4.15 (t, 2H), 3.92 (q, 2H), 3.67 (t, 2H), 3.25 (s, 3H), 1.15 (t, 3H).
LC/MS (Methode 3): $R_t$ = 1.13 min, m/z = 333 $[M+H]^+$.

**Beispiel 187A**

6-Brom-3-ethyl-5-methyl-1-(3,3,3-trifluorpropyl)thieno[2,3-d]pyrimidin-2,4(1*H*,3*H*)-dion

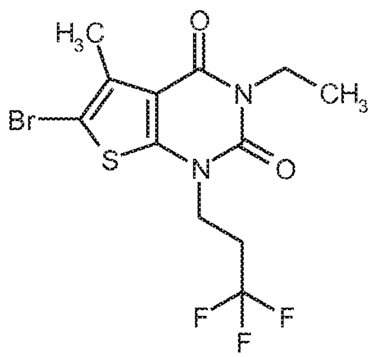

Eine Lösung von 2.50 g (8.16 mmol) der Verbindung aus Bsp. 55A in 40 ml Chloroform wurde bei 0°C und über einen Zeitraum von ca. 15 min portionsweise mit insgesamt 1.48 g (8.16 mmol) *N*-Bromsuccinimid (NBS) versetzt. Anschließend wurde das Kältebad entfernt und das Rühren bei RT fortgesetzt. Nach ca. 16 h wurde mit Dichlormethan verdünnt und nacheinander mit Wasser und gesättigter Natriumchlorid-Lösung gewaschen. Nach Trocknen über wasserfreiem Magnesiumsulfat wurde filtriert und eingeengt. Der erhaltene Rückstand wurde mittels Saugfiltration über Kieselgel mit Cyclohexan/Ethylacetat 5:1 als Laufmittel gereinigt. Einengen der Produktfraktionen und Trocknen des Rückstands im Hochvakuum ergab 2.92 g (92% d. Th.) der Titelverbindung.
$^1$H-NMR (400 MHz, DMSO-$d_6$, $\delta$/ppm): 4.09 (t, 2H), 3.91 (q, 2H), 2.88-2.69 (m, 2H), 2.37 (s, 3H), 1.12 (t, 3H).
LC/MS (Methode 5, ESIpos): $R_t$ = 1.48 min, m/z = 385/387 $[M+H]^+$.

**Beispiel 188A**

*tert.*-Butyl-[3-ethyl-5-methyl-2,4-dioxo-1-(3,3,3-trifluorpropyl)-1,2,3,4-tetrahydrothieno[2,3-d]-pyrimidin-6-yl]acetat

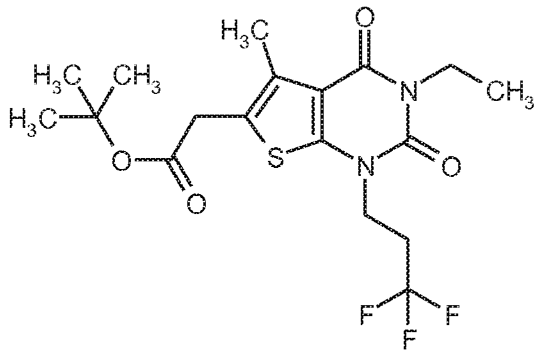

*Darstellung von 2-tert.-Butoxy-2-oxoethylzinkbromid:* 6.36 g (97.4 mmol) Zinkstaub wurden in 97.5 ml wasserfreiem Diethylether vorgelegt und bei RT tropfenweise mit 235 µl (1.85 mmol) Chlortrimethylsilan versetzt. Nach 15 min wurde das Gemisch zum Rückfluss erhitzt, und anschließend wurden 14.4 ml (97.4 mmol) Bromessigsäure-*tert*.-butylester so zugetropft, dass das Gemisch ohne äußere Wärmezufuhr am Sieden blieb. Nach beendetem Zutropfen wurde noch weitere 90 min unter Rückfluss erhitzt. Nach dem Erkalten ergab sich eine Lösung, die im nächsten Teilschritt eingesetzt wurde.

*Kupplungsreaktion zur Darstellung der Titelverbindung:* Eine Lösung von 2.50 g (6.49 mmol) der Verbindung aus Bsp. 187A in 25 ml wasserfreiem THF wurde bei RT mit 28 ml (ca. 26.0 mmol) der zuvor hergestellten Lösung von 2-*tert*.-Butoxy-2-oxoethylzinkbromid versetzt. Dann wurden 346 mg (0.487 mmol) Q-Phos und 297 mg (0.324 mmol) Tris(dibenzylidenaceton)dipalladium hinzugefügt und das Gemisch ca. 16 h bei 60°C gerührt. Nach dem Abkühlen auf RT wurde mit Wasser versetzt und mit Ethylacetat extrahiert. Der organische Extrakt wurde mit gesättigter Natriumchlorid-Lösung gewaschen, über wasserfreiem Magnesiumsulfat getrocknet, filtriert und eingeengt. Der verbliebene Rückstand wurde mittels MPLC gereinigt (Puriflash-Kartusche, 340 g Kieselgel, Cyclohexan/Ethylacetat 20:1 → 7:1). Nach Einengen der Produktfraktionen und Trocknen im Hochvakuum wurden 1.95 g (71 % d. Th.) der Titelverbindung erhalten.

$^1$H-NMR (400 MHz, DMSO-$d_6$, δ/ppm): 4.11 (t, 2H), 3.91 (q, 2H), 3.77 (s, 2H), 2.85-2.70 (m, 2H), 2.33 (s, 3H), 1.42 (s, 9H), 1.12 (t, 3H).

LC/MS (Methode 1, ESIpos): $R_t$ = 1.26 min, m/z = 421 [M+H]$^+$.

**Beispiel 189A**

[3-Ethyl-5-methyl-2,4-dioxo-1-(3,3,3-trifluorpropyl)-1,2,3,4-tetrahydrothieno[2,3-d]pyrimidin-6-yl]essigsäure

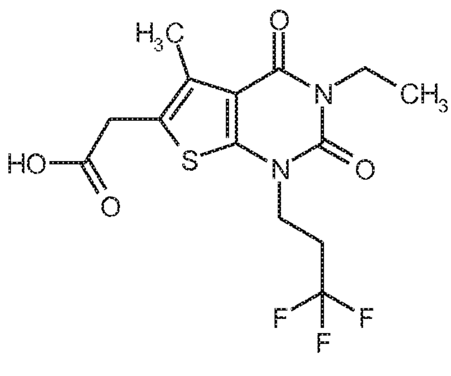

1.74 g (4.14 mmol) der Verbindung aus Bsp. 188A wurden in 100 ml Dichlormethan gelöst und mit 50 ml Trifluoressigsäure versetzt. Nachdem das Reaktionsgemisch 2 h bei RT gerührt worden war, wurden alle flüchtigen Komponenten am Rotationsverdampfer entfernt. Der verbliebene Rückstand wurde 90 min bei RT in einem Gemisch aus 20 ml Pentan und 5 ml Dichlormethan verrührt. Nach Absaugen und Trocknen des Feststoffs im Hochvakuum wurden 1.46 g (96% d. Th.) der Titelverbindung erhalten.

$^1$H-NMR (400 MHz, DMSO-$d_6$, δ/ppm): 12.68 (br. s, 1H), 4.11 (t, 2H), 3.91 (q, 2H), 3.79 (s, 2H), 2.85-2.68 (m, 2H), 2.33 (s, 3H), 1.12 (t, 3H).

LC/MS (Methode 1, ESIpos): $R_t$ = 0.86 min, m/z = 365 [M+H]$^+$.

**Beispiel 190A**

3-Ethyl-6-[(hydroxyimino)methyl]-5-methyl-1-(3,3,3-trifluorpropyl)thieno[2,3-d]pyrimidin-2,4(1*H*,3*H*)-dion

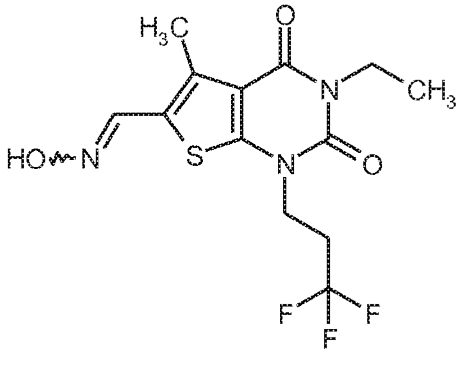

Eine Lösung von 500 mg (1.42 mmol) der Verbindung aus Bsp. 74A in 4.2 ml THF wurde mit 261 μl (4.26 mmol) Hydroxylamin-Lösung (50% in Wasser) versetzt. Nachdem das Reaktionsgemisch ca. 16 h bei RT gerührt worden war, wurden 25 ml Wasser hinzugefügt, worauf das Produkt ausfiel. Es wurde abgesaugt, mit wenig Wasser gewaschen und im Hochvakuum getrocknet. Es wurden 495 mg (99% d. Th.) der Titelverbindung als *E*/*Z*-Isomerengemisch (ca. 9:1) erhalten.
$^1$H-NMR (Hauptisomer; 400 MHz, DMSO-$d_6$, δ/ppm): 12.17 (s, 1H), 7.97 (s, 1H), 4.16 (t, 2H), 3.92 (q, 2H), 2.85-2.73 (m, 2H), 2.62 (s, 3H), 1.13 (t, 3H).
LC/MS (Hauptisomer; Methode 6, ESIpos): $R_t$ = 1.62 min, m/z = 350 $[M+H]^+$.

**Beispiel 191A**

3-Ethyl-6-[(hydroxyimino)methyl]-1-(2-methoxyethyl)-5-methylthieno[2,3-d]pyrimidin-2,4(1*H*,3*H*)-dion

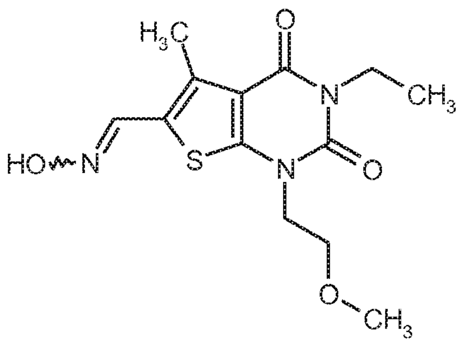

Eine Lösung von 2.0 g (6.75 mmol) der Verbindung aus Bsp. 84A in 20 ml THF wurde mit 1.2 ml (20.2 mmol) Hydroxylamin-Lösung (50% in Wasser) versetzt. Nachdem das Reaktionsgemisch ca. 16 h bei RT gerührt worden war, wurde der ausgefallene Feststoff abgesaugt, mit wenig Wasser gewaschen und im Hochvakuum getrocknet. Dies ergab eine erste Fraktion von 1.46 g der Titelverbindung. Das Filtrat wurde mit ca. 20 ml Dichlormethan verdünnt und nacheinander mit jeweils 10 ml gesättigter Natriumhydrogencarbonat-Lösung und gesättigter Natriumchlorid-Lösung gewaschen. Nach Trocknen über wasserfreiem Magnesiumsulfat wurde filtriert, eingeengt und im Hochvakuum getrocknet. Dies ergab eine zweite Fraktion von 0.50 g der Titelverbindung. Insgesamt wurden so 1.96 g (90% d. Th., 96% Reinheit) der Titelverbindung als *E*/*Z*-Isomerengemisch (ca. 8:1) erhalten.
$^1$H-NMR (Hauptisomer; 400 MHz, DMSO-$d_6$, δ/ppm): 12.10 (s, 1H), 7.94 (s, 1H), 4.08 (t, 2H), 3.91 (q, 2H), 3.65 (t, 2H), 3.24 (s, 3H), 2.60 (s, 3H), 1.13 (t, 3H).
LC/MS (Hauptisomer; Methode 5, ESIpos): $R_t$ = 0.99 min, m/z = 312 $[M+H]^+$.

**Beispiel 192A**

1-(2-Fluorethyl)-6-[(hydroxyimino)methyl]-3-isopropyl-5-methylthieno[2,3-d]pyrimidin-2,4(1*H*,3*H*)-dion

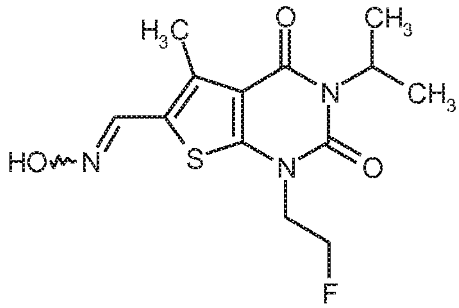

Analog zu dem unter Bsp. 191A beschriebenen Verfahren wurden aus 420 mg (1.41 mmol) der Verbindung aus Bsp. 104A und 259 µl (4.22 mmol) Hydroxylamin-Lösung (50% in Wasser) 404 mg (92% d. Th.) der Titelverbindung als *E*/*Z*-Isomerengemisch (ca. 9:1) erhalten.
$^1$H-NMR (Hauptisomer; 400 MHz, DMSO-$d_6$, δ/ppm): 12.11 (s, 1H), 7.94 (s, 1H), 5.14 (sept, 1H), 4.84-4.64 (dt, 2H), 4.30-4.14 (dt, 2H), 2.60 (s, 3H), 1.42 (d, 6H).
LC/MS (Hauptisomer; Methode 1, ESIpos): $R_t$ = 0.93 min, m/z = 314 $[M+H]^+$.

**Beispiel 193A**

6-[(Hydroxyimino)methyl]-3-isopropyl-5-methyl-1-(3,3,3-trifluorpropyl)thieno[2,3-d]pyrimidin-2,4(1*H*,3*H*)-dion

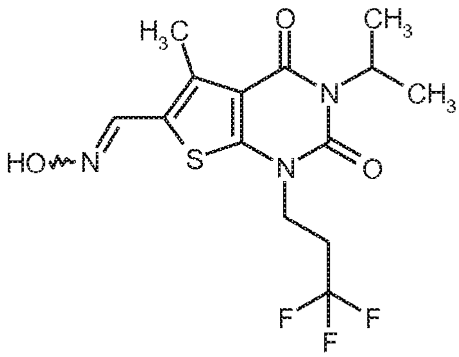

Eine Lösung von 488 mg (1.40 mmol) der Verbindung aus Bsp. 103A in 4.2 ml THF wurde mit 257 µl (4.20 mmol) Hydroxylamin-Lösung (50% in Wasser) versetzt. Nach 1 h Rühren bei RT wurde mit ca. 10 ml Dichlormethan verdünnt und nacheinander mit jeweils ca. 10 ml gesättigter Natriumhydrogencarbonat-Lösung und gesättigter Natriumchlorid-Lösung gewaschen. Nach dem Trocknen über wasserfreiem Magnesiumsulfat wurde filtriert, eingeengt und im Hochvakuum getrocknet. Es wurden 486 mg (95% d. Th.) der Titelverbindung als *E*/*Z*-Isomerengemisch (ca. 6:1) erhalten.
$^1$H-NMR (Hauptisomer; 400 MHz, DMSO-$d_6$, δ/ppm): 12.15 (s, 1H), 7.96 (s, 1H), 5.14 (sept, 1H), 4.13 (t, 2H), 2.86-2.69 (m, 2H), 2.60 (s, 3H), 1.41 (d, 6H).
LC/MS (Hauptisomer; Methode 1, ESIpos): $R_t$ = 1.01 min, m/z = 364 $[M+H]^+$.

**Beispiel 194A**

6-[(Hydroxyimino)methyl]-3-isopropyl-5-methyl-1-(oxetan-2-ylmethyl)thieno[2,3-d]pyrimidin-2,4(1*H*,3*H*)-dion (*Racemat*)

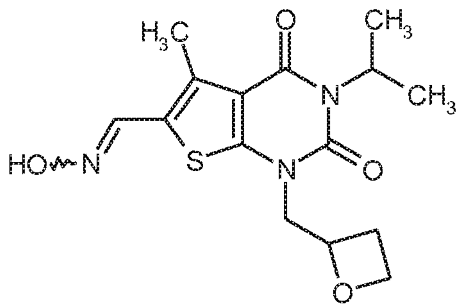

Analog zu dem unter Bsp. 191A beschriebenen Verfahren wurden aus 400 mg (1.24 mmol) der Verbindung aus Bsp. 106A und 228 µl (3.72 mmol) Hydroxylamin-Lösung (50% in Wasser) 395 mg (95% d. Th.) der Titelverbindung als *E*/*Z*-Isomerengemisch (ca. 5:1) erhalten.
$^1$H-NMR (Hauptisomer; 400 MHz, DMSO-$d_6$, δ/ppm): 12.06 (s, 1H), 7.93 (s, 1H), 5.15 (sept, 1H), 5.07-4.96 (m, 1H), 4.54-4.34 (m, 2H), 4.25-4.07 (m, 2H), 2.75-2.63 (m, 1H), 2.59 (s, 3H), 2.50-2.43 (m, 1H, teilweise überdeckt vom DMSO-Signal), 1.41 (d, 6H).
LC/MS (Hauptisomer; Methode 1, ESIpos): $R_t$ = 0.80 min, m/z = 338 $[M+H]^+$.

**Beispiel 195A**

6-[(Hydroxyimino)methyl]-3-isopropyl-5-methyl-1-(tetrahydrofuran-2-ylmethyl)thieno[2,3-d]-pyrimidin-2,4(1*H*,3*H*)-dion (*Racemat*)

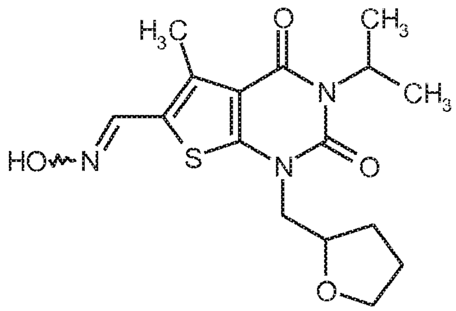

Analog zu dem unter Bsp. 193A beschriebenen Verfahren wurden aus 334 mg (0.925 mmol) der Verbindung aus Bsp. 107A und 170 µl (2.77 mmol) Hydroxylamin-Lösung (50% in Wasser) 328 mg (98% d. Th., 97% Reinheit) der Titelverbindung als *E*/*Z*-Isomerengemisch (ca. 6:1) erhalten. Die Reaktionszeit betrug hier 3.5 h.
$^1$H-NMR (Hauptisomer; 400 MHz, DMSO-$d_6$, δ/ppm): 12.05 (s, 1H), 7.93 (s, 1H), 5.15 (sept, 1H), 4.31-4.15 (m, 1H), 4.06 (dd, 1H), 3.83-3.70 (m, 2H), 3.67-3.55 (m, 1H), 2.59 (s, 3H), 2.08-1.73 (m, 3H), 1.72-1.59 (m, 1H), 1.41 (dd, 6H).
LC/MS (Hauptisomer; Methode 1, ESIpos): $R_t$ = 0.92 min, m/z = 352 $[M+H]^+$.

**Beispiel 196A**

6-[(Hydroxyimino)methyl]-3-isopropyl-5-methyl-1-(oxetan-3-ylmethyl)thieno[2,3-d]pyrimidin-2,4(1*H*,3*H*)-dion

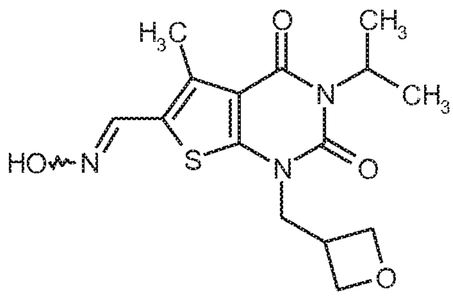

Analog zu dem unter Bsp. 193A beschriebenen Verfahren wurden aus 299 mg (0.863 mmol) der Verbindung aus Bsp. 108A und 159 µl (2.59 mmol) Hydroxylamin-Lösung (50% in Wasser) 290 mg (79% d. Th., 80% Reinheit) der Titelverbindung als *E*/*Z*-Isomerengemisch (ca. 6:1) erhalten. Die Reaktionszeit betrug hier ca. 16 h.
$^1$H-NMR (Hauptisomer; 400 MHz, DMSO-$d_6$, δ/ppm): 12.12 (s, 1H), 7.94 (s, 1H), 5.13 (sept, 1H), 4.63 (dd, 2H), 4.45 (t, 2H), 4.23 (d, 2H), 3.48-3.41 (m, 1H), 2.59 (s, 3H), 1.40 (d, 6H).
LC/MS (Hauptisomer; Methode 1, ESIpos): $R_t$ = 0.81 min, m/z = 338 $[M+H]^+$.

**Beispiel 197A**

6-[(Hydroxyimino)methyl]-3-isopropyl-1,5-dimethylthieno[2,3-d]pyrimidin-2,4(1*H*,3*H*-dion

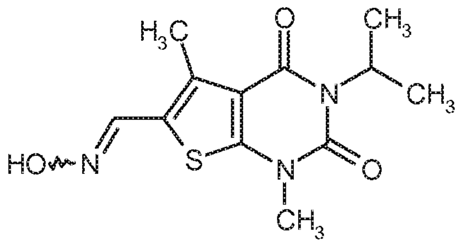

Analog zu dem unter Bsp. 191A beschriebenen Verfahren wurden aus 407 mg (1.53 mmol) der Verbindung aus Bsp. 109A und 281 µl (4.59 mmol) Hydroxylamin-Lösung (50% in Wasser) 402 mg (86% d. Th., 91% Reinheit) der Titelverbindung als *E*/*Z*-Isomerengemisch (ca. 13:1) erhalten. Die Reaktionszeit betrug hier 1 h.
$^1$H-NMR (Hauptisomer; 400 MHz, DMSO-$d_6$, δ/ppm): 12.09 (s, 1H), 7.93 (s, 1H), 5.15 (sept, 1H), 3.43 (s, 3H), 2.60 (s, 3H), 1.41 (d, 6H).
LC/MS (Hauptisomer; Methode 1, ESIpos): $R_t$ = 0.84 min, m/z = 282 $[M+H]^+$.

**Beispiel 198A**

6-(Azidomethyl)-3-ethyl-1-(2-methoxyethyl)-5-methylthieno[2,3-d]pyrimidin-2,4(1*H*,3*H*)-dion

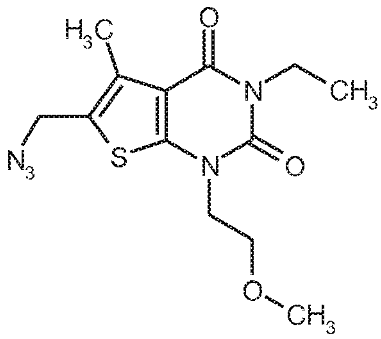

600 mg (1.95 mmol) der Verbindung aus Bsp. 143A wurden in 9 ml THF gelöst und auf 0°C gekühlt. Es wurden 767 mg (2.73 mmol) Phosphorsäurediphenylesterazid zugegeben und dann 5 min bei 0°C gerührt. Anschließend wurden 356 mg (2.34 mmol) 1,8-Diazabicyclo[5.4.0]undec-7-en (DBU) zugegeben, und das Reaktionsgemisch wurde 67 h bei RT weiter gerührt. Das Gemisch wurde dann mit Wasser (75 ml) versetzt und mit Ethylacetat extrahiert. Die wässrige Phase wurde nochmals mit Ethylacetat extrahiert. Die vereinigten organischen Phasen wurden über Natriumsulfat getrocknet, filtriert und eingeengt. Der Rückstand wurde über eine Kieselgel-Kartusche chromatographiert (Biotage, 25 g Kieselgel, Laufmittel Hexan/Ethylacetat). Es wurden 541 mg der Titelverbindung erhalten.
$^1$H-NMR (400 MHz, DMSO-$d_6$, δ/ppm): 4.67 (s, 2H), 4.04 (t, 2H), 3.90 (q, 2H), 3.64 (t, 2H), 3.24 (s, 3H), 2.41 (s, 3H), 1.12 (t, 3H).
LC/MS (Methode 3): $R_t$ = 1.15 min, m/z = 324 $[M+H]^+$.

**Beispiel 199A**

6-(Aminomethyl)-3-ethyl-5-methyl-1-(3,3,3-trifluorpropyl)thieno[2,3-d]pyrimidin-2,4(1*H*,3*H*)-dion-Hydrochlorid

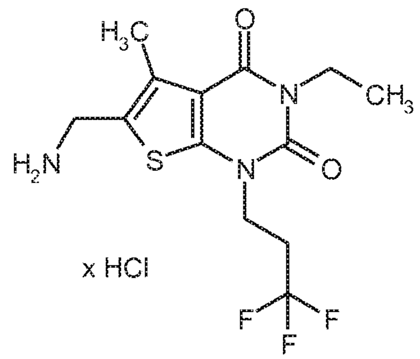

Eine Lösung von 495 mg (1.42 mmol) der Verbindung aus Bsp. 190A in 35 ml Methanol wurde mit 295 µl (3.54 mmol) konzentrierter Salzsäure und 45 mg Palladium auf Kohle (10%) versetzt. Anschließend wurde bei einem Wasserstoffdruck von 1 bar 2.5 h lang bei RT hydriert. Danach wurde vom Katalysator über wenig Kieselgur abfiltriert und das Filtrat am Rotationsverdampfer eingeengt. Nach Trocknen des Produkts im Hochvakuum wurden 526 mg (89% d. Th., 90% Reinheit) der Titelverbindung erhalten.
$^1$H-NMR (400 MHz, DMSO-$d_6$, δ/ppm): 8.29 (br. s, 3H), 4.21 (m, 2H), 4.13 (t, 2H), 3.92 (q, 2H), 2.88-2.71 (m, 2H), 2.46 (s, 3H), 1.13 (t, 3H).
LC/MS (Methode 1, ESIpos): $R_t$ = 0.54 min, m/z = 319 $[M+H-NH_3]^+$.

**Beispiel 200A**

6-(Aminomethyl)-3-ethyl-1-(2-methoxyethyl)-5-methylthieno[2,3-d]pyrimidin-2,4(1*H*,3*H*)-dion

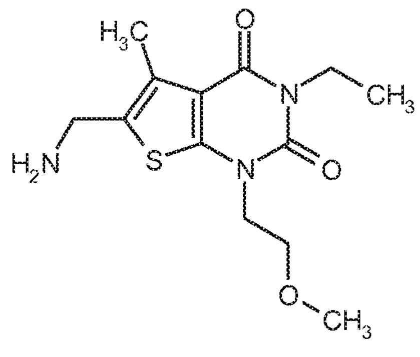

260 mg (0.710 mmol, 85% Reinheit) der Verbindung aus Bsp. 191A, gelöst in 15 ml Methanol, wurden zu einer

Lösung von 169 mg (0.710 mmol) Nickel(II)chlorid-Hexahydrat und 27 mg (0.710 mmol) Natriumborhydrid in 10 ml Methanol gegeben. Anschließend wurden weitere 146 mg (3.91 mmol) festes Natriumborhydrid hinzugefügt. Nach 10 min Rühren bei RT wurden die festen Bestandteile des Reaktionsgemisches durch Absaugen über wenig Kieselgur abgetrennt. Das Filtrat wurde am Rotationsverdampfer eingeengt. Der verbliebene Rückstand wurde mit Ammoniakwasser versetzt und mit Ethylacetat extrahiert. Der organische Extrakt wurde mit gesättigter Natriumchlorid-Lösung gewaschen, über wasserfreiem Magnesiumsulfat getrocknet, filtriert und eingeengt. Nach Trocknen im Hochvakuum wurden 210 mg (79% d. Th., 80% Reinheit) der Titelverbindung erhalten.
$^1$H-NMR (400 MHz, DMSO-$d_6$, $\delta$/ppm): 4.04 (t, 2H), 3.90 (q, 2H), 3.82 (s, 2H), 3.64 (t, 2H), 3.24 (s, 3H), 2.31 (s, 3H), 1.11 (t, 3H).
LC/MS (Methode 1, ESIpos): $R_t$ = 0.45 min, m/z = 298 $[M+H]^+$.

**Beispiel 201A**

6-(Aminomethyl)-3-ethyl-1-(2-methoxyethyl)-5-methylthieno[2,3-d]pyrimidin-2,4(1*H*,3*H*)-dion-Hydrochlorid

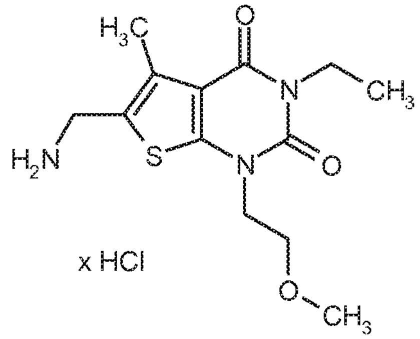

Eine Lösung von 1.45 g (4.65 mmol) der Verbindung aus Bsp. 191A in 145 ml Methanol wurde mit 970 µl (11.6 mmol) konzentrierter Salzsäure und 145 mg Palladium auf Kohle (10%) versetzt. Anschließend wurde bei einem Wasserstoffdruck von ca. 1 bar 4.5 h lang bei RT hydriert. Danach wurde vom Katalysator über wenig Kieselgur abfiltriert und das Filtrat am Rotationsverdampfer eingeengt. Nach Trocknen des Produkts im Hochvakuum wurden 1.52 g (97% d. Th.) der Titelverbindung erhalten.
$^1$H-NMR (400 MHz, DMSO-$d_6$, $\delta$/ppm): 8.25 (br. s, 3H), 4.18 (br. s, 2H), 4.04 (t, 2H), 3.91 (q, 2H), 3.66 (t, 2H), 3.25 (s, 3H), 2.45 (s, 3H), 1.13 (t, 3H).
LC/MS (Methode 5, ESIpos): $R_t$ = 0.58 min, m/z = 298 $[M+H]^+$.

**Beispiel 202A**

6-(Aminomethyl)-3-ethyl-1-(2-methoxyethyl)-5-methylthieno[2,3-d]pyrimidin-2,4(1*H*,3*H*)-dion-Formiat

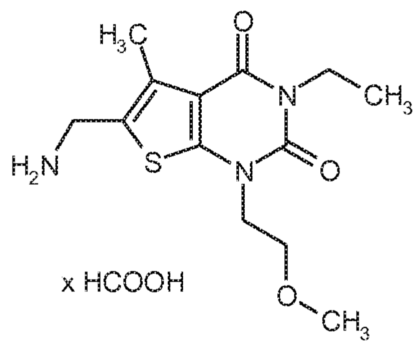

Eine Lösung von 540 mg (1.6 mmol) der Verbindung aus Bsp. 198A in 28.3 ml THF wurde mit 272 mg (3.57 mmol) Trimethylphosphin versetzt und 2 h bei RT gerührt. Daraufhin wurden 3.56 ml 25%-ige wässrige Ammoniak-Lösung zugesetzt und 3 h bei RT weiter gerührt. Das Reaktionsgemisch wurde dann am Rotationsverdampfer eingeengt. Der Rückstand wurde mit Toluol versetzt und erneut eingeengt. Das erhaltene Material wurde mittels präparativer HPLC gereinigt (Methode 14). Nach Vereinigen der Produktfraktionen und Gefriertrocknung wurden 359 mg (65% d. Th.) der Titelverbindung erhalten.
$^1$H-NMR (400 MHz, DMSO-$d_6$, δ/ppm): 8.26 (s, 1H), 4.04 (t, 2H), 3.94-3.86 (m, 4H), 3.64 (t, 2H), 2.34 (s, 3H), 1.11 (t, 3H).
LC/MS (Methode 3): $R_t$ = 0.59 min.

**Beispiel 203A**

6-(Aminomethyl)-1-(2-fluorethyl)-3-isopropyl-5-methylthieno[2,3-d]pyrimidin-2,4(1*H*,3*H*)-dion-Hydrochlorid

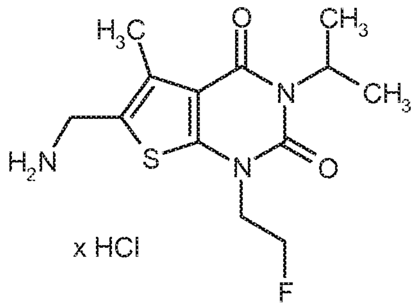

Analog zu dem unter Bsp. 201A beschriebenen Verfahren wurden aus 401 mg (1.28 mmol) der Verbindung aus Bsp. 192A 379 mg (83% d. Th., 94% Reinheit) der Titelverbindung erhalten. Die Reaktionszeit betrug hier 4 h; das Produkt wurde nach dem Eindampfen noch durch Verrühren mit Cyclohexan/Ethylacetat (10:1) gereinigt.
$^1$H-NMR (400 MHz, DMSO-$d_6$, δ/ppm): 8.16 (br. s, 3H), 5.14 (sept, 1H), 4.88-4.62 (dt, 2H), 4.29-4.10 (dt, 2H), 4.19 (s, 2H), 2.43 (s, 3H), 1.41 (d, 6H).
LC/MS (Methode 1, ESIpos): $R_t$ = 0.53 min, m/z = 283 $[M+H-NH_3]^+$.

**Beispiel 204A**

6-(Aminomethyl)-3-isopropyl-5-methyl-1-(3,3,3-trifluorpropyl)thieno[2,3-d]pyrimidin-2,4(1*H*,3*H*)-dion-Hydrochlorid

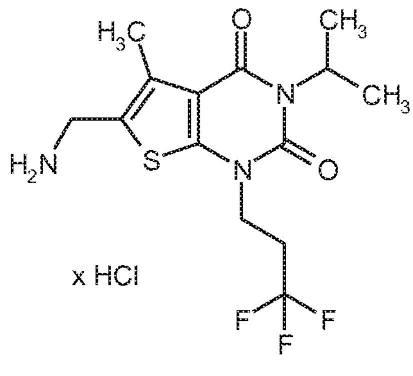

Analog zu dem unter Bsp. 201A beschriebenen Verfahren wurden aus 473 mg (1.30 mmol) der Verbindung aus Bsp. 193A 469 mg (98% d. Th.) der Titelverbindung erhalten. Das Produkt wurde nach dem Eindampfen noch durch Verrühren mit Cyclohexan/Ethylacetat (10:1) gereinigt.
$^1$H-NMR (400 MHz, DMSO-$d_6$, δ/ppm): 8.14 (br. s, 3H), 5.13 (sept, 1H), 4.21 (br. s, 2H), 4.10 (t, 2H), 2.86-2.69 (m, 2H), 2.44 (s, 3H), 1.41 (d, 6H).
LC/MS (Methode 1, ESIpos): $R_t$ = 0.57 min, m/z = 333 $[M+H-NH_3]^+$.

**Beispiel 205A**

6-(Aminomethyl)-3-isopropyl-5-methyl-1-(oxetan-2-ylmethyl)thieno[2,3-d]pyrimidin-2,4(1*H*,3*H*)-dion (*Racemat*)

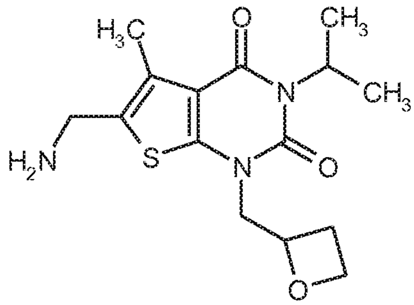

Analog zu dem unter Bsp. 200A beschriebenen Verfahren wurden aus 372 mg (1.10 mmol) der Verbindung aus Bsp. 194A 290 mg (69% d. Th., 85% Reinheit) der Titelverbindung erhalten.
$^1$H-NMR (400 MHz, DMSO-$d_6$, δ/ppm): 5.14 (sept, 1H), 5.05-4.93 (m, 1H), 4.53-4.36 (m, 2H), 4.12 (d, 2H), 2.75-2.63 (m, 1H), 2.52-2.43 (m, 1H, teilweise überdeckt vom DMSO-Signal), 2.29 (s, 3H), 1.39 (d, 6H).
LC/MS (Methode 1, ESIpos): $R_t$ = 0.49 min, m/z = 307 [M+H-$NH_3$]$^+$.

**Beispiel 206A**

6-(Aminomethyl)-3-isopropyl-5-methyl-1-(tetrahydrofuran-2-ylmethyl)thieno[2,3-d]pyrimidin-2,4(1*H*,3*H*)-dion-Hydrochlorid (*Racemat*)

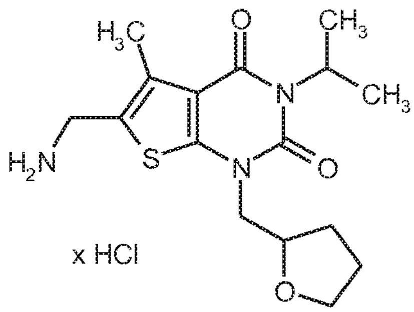

Analog zu dem unter Bsp. 201A beschriebenen Verfahren wurden aus 320 mg (0.883 mmol) der Verbindung aus Bsp. 195A 267 mg (71% d. Th., 88% Reinheit) der Titelverbindung erhalten. Die Reaktionszeit betrug hier 5 h; das Produkt wurde nach dem Eindampfen noch durch Verrühren mit Cyclohexan/Ethylacetat (10:1) gereinigt.
$^1$H-NMR (400 MHz, DMSO-$d_6$, δ/ppm): 8.13 (br. s, 3H), 5.14 (sept, 1H), 4.28-4.15 (m, 3H), 4.09 (dd, 1H), 3.83-3.72 (m, 1H), 3.69-3.56 (m, 2H), 2.43 (s, 3H), 2.06-1.95 (m, 1H), 1.94-1.76 (m, 2H), 1.74-1.59 (m, 1H), 1.41 (d, 6H).
LC/MS (Methode 1, ESIpos): $R_t$ = 0.59 min, m/z = 321 [M+H-$NH_3$]$^+$.

**Beispiel 207A**

6-(Aminomethyl)-3-isopropyl-5-methyl-1-(oxetan-3-ylmethyl)thieno[2,3-d]pyrimidin-2,4(1*H*,3*H*)-dion

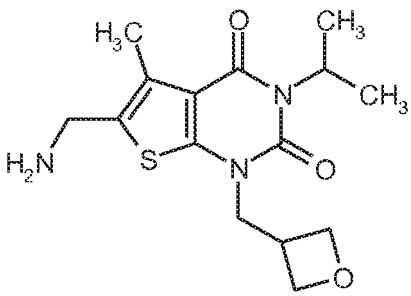

Analog zu dem unter Bsp. 200A beschriebenen Verfahren wurden aus 279 mg (0.663 mmol) der Verbindung aus Bsp. 196A 216 mg (70% d. Th., 70% Reinheit) der Titelverbindung erhalten.
$^1$H-NMR (400 MHz, DMSO-$d_6$, δ/ppm): 5.12 (sept, 1H), 4.68-4.57 (m, 2H), 4.49-4.39 (m, 2H), 4.18 (d, 2H), 3.81 (s, 1H), 3.43 (dt, 1H), 2.29 (s, 3H), 1.39 (d, 6H).
LC/MS (Methode 1, ESIpos): $R_t$ = 0.50 min, m/z = 307 $[M+H-NH_3]^+$.

**Beispiel 208A**

6-(Aminomethyl)-3-isopropyl-1,5-dimethylthieno[2,3-d]pyrimidin-2,4(1*H*,3*H*)-dion

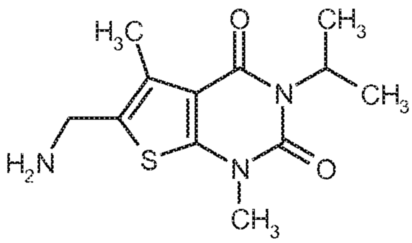

Analog zu dem unter Bsp. 200A beschriebenen Verfahren wurden aus 20 mg (0.066 mmol) der Verbindung aus Bsp. 197A 16 mg (91% d. Th.) der Titelverbindung erhalten.
LC/MS (Methode 1, ESIpos): $R_t$ = 0.49 min, m/z = 251 $[M+H-NH_3]^+$,

**Beispiel 209A**

6-{[(2-Aminoethyl)amino]methyl}-3-ethyl-5-methyl-1-(2,2,2-trifluorethyl)thieno[2,3-d]pyrimidin-2,4(1*H*,3*H*)-dion

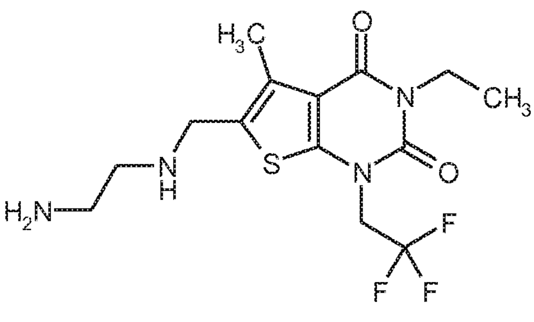

570 mg (1.71 mmol) der Verbindung aus Bsp. 73A wurden in einem Gemisch aus 10 ml Methanol und 4 ml Dichlormethan gelöst. Anschließend wurden bei RT 457 µl (6.83 mmol) 1,2-Diaminoethan, 391 µl (6.83 mmol) Essigsäure und, portionsweise über ca. 2 h verteilt, 429 mg (6.83 mmol) Natriumcyanoborhydrid hinzugefügt. Nachdem das Reaktionsgemisch 3 Tage bei RT gerührt worden war, wurde mit 2 M Natronlauge versetzt und mit Ethylacetat extrahiert. Der organische Extrakt wurde mit gesättigter Natriumchlorid-Lösung gewaschen, über wasserfreiem Magnesiumsulfat

getrocknet, filtriert und am Rotationsverdampfer eingeengt. Das so erhaltene Rohprodukt ergab nach dem Trocknen im Hochvakuum 730 mg (83% d. Th., 71 % Reinheit) der Titelverbindung, welche ohne weitere Reinigung für Folgereaktionen verwendet wurden.
LC/MS (Methode 1, ESIpos): $R_t$ = 0.41 min, m/z = 365 $[M+H]^+$.

**Beispiel 210A**

6-{[(2-Aminoethyl)amino]methyl}-3-ethyl-5-methyl-1-(3,3,3-trifluorpropyl)thieno[2,3-d]pyrimidin-2,4(1*H*,3*H*)-dion

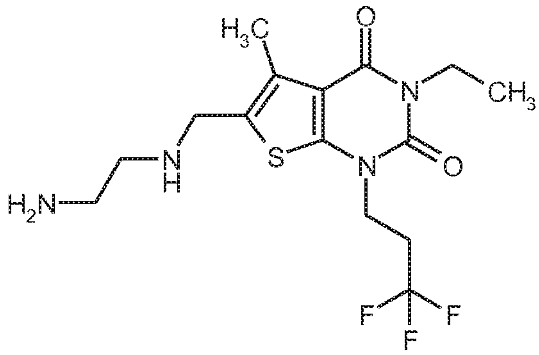

330 mg (0.89 mmol) der Verbindung aus Bsp. 74A wurden in einem Gemisch aus 7.36 ml Methanol und 3.16 ml Dichlormethan gelöst. Dann wurden bei RT 240 µl (3.59 mmol) 1,2-Diaminoethan und 205 µl (3.59 mmol) Essigsäure zugesetzt. Es wurde 30 min bei RT gerührt, und danach wurden 237 mg (3.59 mmol) Natriumcyanoborhydrid hinzugefügt. Nachdem das Reaktionsgemisch 20 h bei 60°C gerührt worden war, wurde mit 30 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 369 mg (83% d. Th., 77% Reinheit) der Titelverbindung und wurde ohne weitere Reinigung für Folgereaktionen verwendet.
LC/MS (Methode 3): $R_t$ = 0.57 min, m/z = 319 $[M+H-C_2H_8N_2]^+$.

**Beispiel 211A**

6-{[(2-Aminoethyl)amino]methyl}-3-ethyl-5-methyl-1-(4,4,4-trifluorbutyl)thieno[2,3-d]pyrimidin-2,4(1*H*,3*H*)-dion

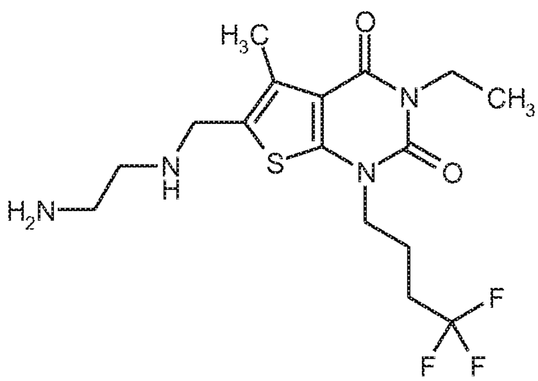

Analog zu dem unter Bsp. 209A beschriebenen Verfahren wurden aus 200 mg (2.30 mmol) der Verbindung aus Bsp. 75A und 1,2-Diaminoethan 310 mg (96% d. Th., 70% Reinheit) der Titelverbindung hergestellt. Die Reaktionszeit betrug hier ca. 18 h.
LC/MS (Methode 1, ESIpos): $R_t$ = 0.50 min, m/z = 393 $[M+H]^+$.

**Beispiel 212A**

6-{[(2-Aminoethyl)amino]methyl}-1-(2,2-difluorethyl)-3-ethyl-5-methylthieno[2,3-d]pyrimidin-2,4(1*H*,3*H*)-dion

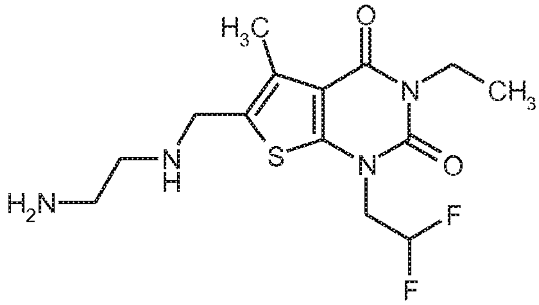

235 mg (0.77 mmol) der Verbindung aus Bsp. 76A wurden in einem Gemisch aus 15.74 ml Methanol und 7.5 ml Dichlormethan gelöst. Dann wurden bei RT 520 µl (7.74 mmol) 1,2-Diaminoethan und 178 µl (3.11 mmol) Essigsäure zugesetzt. Es wurde 30 min bei RT gerührt, und danach wurden 206 mg (3.11 mmol) Natriumcyanoborhydrid hinzugefügt. Nachdem das Reaktionsgemisch 116 h bei 60°C gerührt worden war, wurde mit 50 ml Wasser versetzt (pH-Wert 9-10) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 353 mg (85% Reinheit) der Titelverbindung und wurde ohne weitere Reinigung für Folgereaktionen verwendet.
LC/MS (Methode 3): $R_t$ = 0.55 min, m/z = 347 $[M+H]^+$.

**Beispiel 213A**

6-{[(2-Aminoethyl)amino]methyl}-3-ethyl-1-(2-fluorethyl)-5-methylthieno[2,3-d]pyrimidin-2,4(1*H*,3*H*)-dion

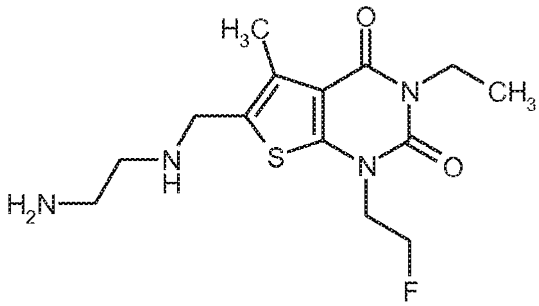

Analog zu dem unter Bsp. 209A beschriebenen Verfahren wurden aus 252 mg (0.886 mmol) der Verbindung aus Bsp. 77A und 1,2-Diaminoethan 292 mg (86% d. Th., 86% Reinheit) der Titelverbindung hergestellt. Die Reaktionszeit betrug hier 2 Tage.
LC/MS (Methode 1, ESIpos): $R_t$ = 0.26 min, m/z = 329 $[M+H]^+$.

**Beispiel 214A**

6-{[(2-Aminoethyl)amino]methyl}-3-ethyl-1-(3-fluorpropyl)-5-methylthieno[2,3-d]pyrimidin-2,4(1*H*,3*H*)-dion

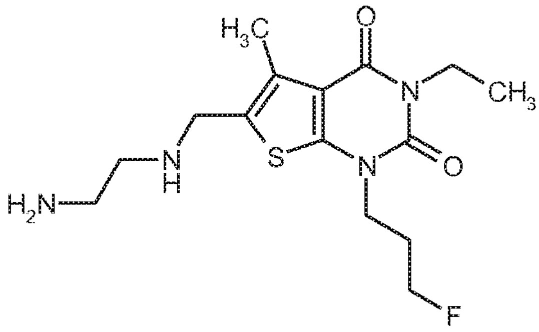

150 mg (0.49 mmol) der Verbindung aus Bsp. 78A wurden in einem Gemisch aus 4.04 ml Methanol und 1.73 ml Dichlormethan gelöst. Dann wurden bei RT 132 µl (1.97 mmol) 1,2-Diaminoethan und 113 µl (1.97 mmol) Essigsäure zugesetzt. Es wurde 30 min bei RT gerührt, und danach wurden 130 mg (1.97 mmol) Natriumcyanoborhydrid hinzugefügt. Nachdem das Reaktionsgemisch 2 h bei 60°C gerührt worden war, wurde mit 5 ml 2 M Natronlauge versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt wurde mittels präparativer HPLC gereinigt (Methode 14). Nach Vereinigen der Produktfraktionen und Gefriertrocknung wurden 116 mg (62% d. Th.) der Titelverbindung erhalten.
$^1$H-NMR (400 MHz, DMSO-$d_6$ + $D_2O$, δ/ppm): 4.59 (t, 1H), 4.47 (t, 1H), 3.99 (t, 2H), 3.89 (q, 2H), 3.80 (s, 2H), 2.85-2.79 (m, 2H), 2.73-2.67 (m, 2H), 2.35 (s, 2H), 2.16-1.97 (m, 2H), 1.10 (t, 3H).
LC/MS (Methode 3): $R_t$ = 0.52 min; m/z = 343 $[M+H]^+$, 283 $[M+H-C_2H_8N_2]^+$.

## Beispiel 215A

6-{[(2-Aminoethyl)amino]methyl}-3-ethyl-1-(4-fluorbutyl)-5-methylthieno[2,3-d]pyrimidin-2,4(1*H*,3*H*)-dion

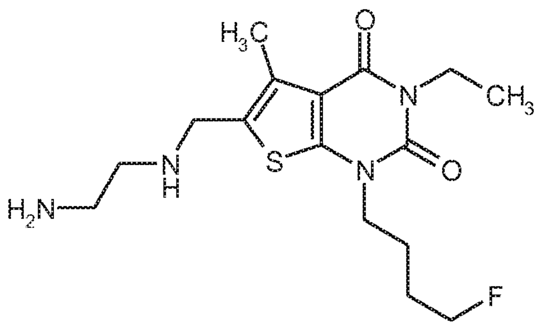

216 mg (0.68 mmol) der Verbindung aus Bsp. 79A wurden in einem Gemisch aus 13.86 ml Methanol und 6.6 ml Dichlormethan gelöst. Dann wurden bei RT 458 µl (6.85 mmol) 1,2-Diaminoethan und 157 µl (2.74 mmol) Essigsäure zugesetzt. Es wurde 30 min bei RT gerührt. Anschließend wurden 181 mg (2.74 mmol) Natriumcyanoborhydrid hinzugefügt. Nachdem das Reaktionsgemisch 92 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 250 mg (47% d. Th., 46% Reinheit) der Titelverbindung und wurde ohne weitere Reinigung für Folgereaktionen verwendet.
LC/MS (Methode 3): $R_t$ = 0.59 min, m/z = 297 $[M+H-C_2H_8N_2]^+$.

## Beispiel 216A

6-{[(2-Aminoethyl)amino]methyl}-3-ethyl-5-methyl-1-[2-(trifluormethyl)prop-2-en-1-yl]thieno-[2,3-d]pyrimidin-2,4(1*H*,3*H*)-dion

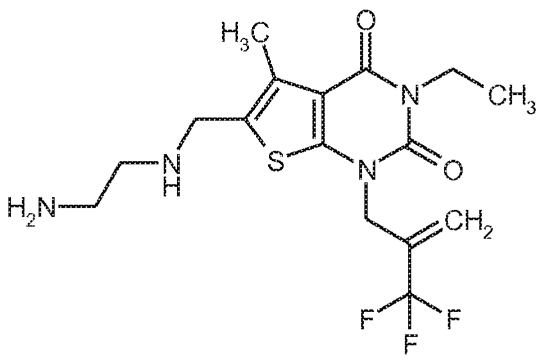

Analog zu dem unter Bsp. 209A beschriebenen Verfahren wurden aus 215 mg (0.621 mmol) der Verbindung aus Bsp. 80A und 1,2-Diaminoethan 246 mg (86% d. Th., 84% Reinheit) der Titelverbindung hergestellt.
LC/MS (Methode 1, ESIpos): $R_t$ = 0.43 min, m/z = 391 $[M+H]^+$.

**Beispiel 217A**

6-{[(2-Aminoethyl)amino]methyl}-1-[(2,2-difluorcyclopropyl)methyl]-3-ethyl-5-methylthieno-[2,3-d]pyrimidin-2,4(1*H*,3*H*)-dion

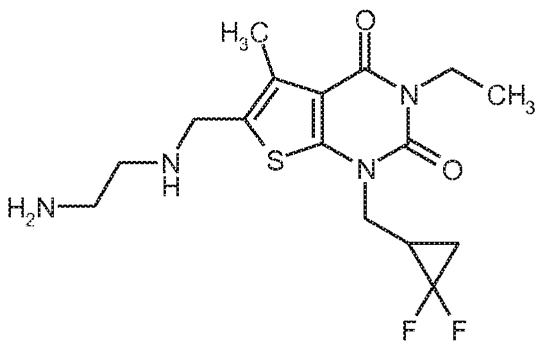

Analog zu dem unter Bsp. 209A beschriebenen Verfahren wurden aus 408 mg (1.24 mmol) der Verbindung aus Bsp. 81A und 1,2-Diaminoethan 548 mg (97% d. Th., 82% Reinheit) der Titelverbindung hergestellt.
LC/MS (Methode 1, ESIpos): $R_t$ = 0.42 min, m/z = 373 $[M+H]^+$.

**Beispiel 218A**

6-{[(2-Aminoethyl)amino]methyl}-3-ethyl-5-methyl-1-[2-(trifluormethoxy)ethyl]thieno[2,3-d]-pyrimidin-2,4(1*H*,3*H*)-dion

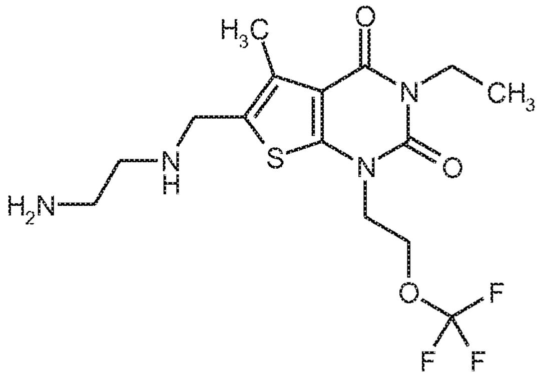

Analog zu dem unter Bsp. 209A beschriebenen Verfahren wurden aus 200 mg (0.571 mmol) der Verbindung aus Bsp. 82A und 1,2-Diaminoethan 288 mg (99% d. Th., 78% Reinheit) der Titelverbindung hergestellt. Die Reaktionszeit betrug hier ca. 18 h.
LC/MS (Methode 2, ESIpos): $R_t$ = 0.97 min, m/z = 395 $[M+H]^+$.

## Beispiel 219A

6-{[(2-Aminoethyl)amino]methyl}-3-ethyl-5-methyl-1-{2-[(trifluormethyl)sulfanyl]ethyl}thieno-[2,3-d]pyrimidin-2,4(1*H*,3*H*)-dion

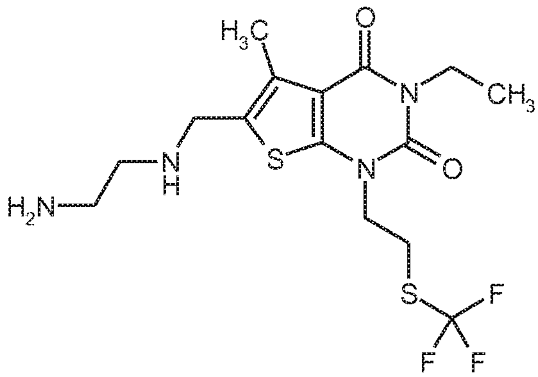

Analog zu dem unter Bsp. 209A beschriebenen Verfahren wurden aus 200 mg (0.546 mmol) der Verbindung aus Bsp. 83A und 1,2-Diaminoethan 271 mg (96% d. Th., 80% Reinheit) der Titelverbindung hergestellt. Die Reaktionszeit betrug hier ca. 18 h.
LC/MS (Methode 2, ESIpos): $R_t$ = 1.09 min, m/z = 411 $[M+H]^+$.

## Beispiel 220A

6-{[(2-Aminoethyl)amino]methyl}-3-ethyl-1-(2-methoxyethyl)-5-methylthieno[2,3-d]pyrimidin-2,4(1*H*,3*H*)-dion

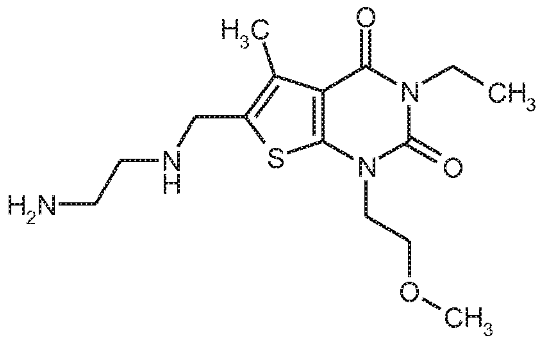

40.0 g (135 mmol) der Verbindung aus Bsp. 84A wurden in einem Gemisch aus 1120 ml Methanol und 440 ml Dichlormethan gelöst. Anschließend wurden bei RT 54.1 ml (810 mmol) 1,2-Diaminoethan, 31 ml (540 mmol) Essigsäure und, portionsweise über ca. 6 h verteilt, 33.9 g (540 mmol) Natriumcyanoborhydrid hinzugefügt. Nachdem das Reaktionsgemisch 6 Tage bei RT gerührt worden war, wurde mit 1000 ml 2 M Natronlauge versetzt und viermal mit insgesamt 2800 ml Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach dem Trocknen im Hochvakuum 45.9 g (69% d. Th., 69% Reinheit) der Titelverbindung, welche ohne weitere Reinigung für Folgereaktionen verwendet wurden.
$^1$H-NMR (400 MHz, DMSO-$d_6$, δ/ppm): 4.04 (t, 2H), 3.90 (q, 2H), 3.79 (s, 2H), 3.64 (t, 2H), 3.24 (s, 3H), 2.69-2.62 (m, 2H), 2.59-2.56 (m, 2H), 2.34 (s, 3H), 1.11 (t, 3H).
LC/MS (Methode 6, ESIpos): $R_t$ = 1.02 min, m/z = 281 $[M+H-C_2H_8N_2]^+$.

**Beispiel 221A**

6-{[(2-Aminoethyl)amino]methyl}-1-(2-ethoxyethyl)-3-ethyl-5-methylthieno[2,3-d]pyrimidin-2,4(1*H*,3*H*)-dion

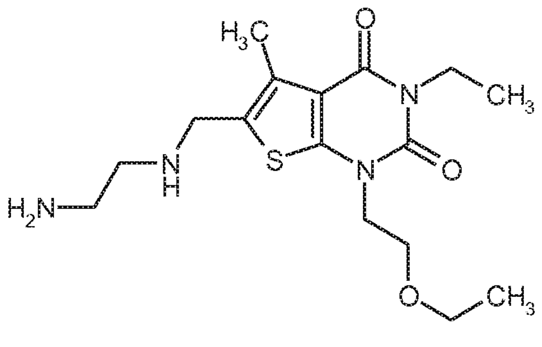

Analog zu dem unter Bsp. 210A beschriebenen Verfahren wurden aus 215 mg (0.686 mmol) der Verbindung aus Bsp. 85A und 1,2-Diaminoethan 255 mg (82% d. Th., 79% Reinheit) der Titelverbindung hergestellt. Die Reaktionszeit betrug hier ca. 92 h.
LC/MS (Methode 3): $R_t$ = 0.54 min, m/z = 295 $[M+H-C_2H_8N_2]^+$.

**Beispiel 222A**

6-{[(2-Aminoethyl)amino]methyl}-3-ethyl-1-(2-isopropoxyethyl)-5-methylthieno[2,3-d]pyrimidin-2,4(1*H*,3*H*)-dion

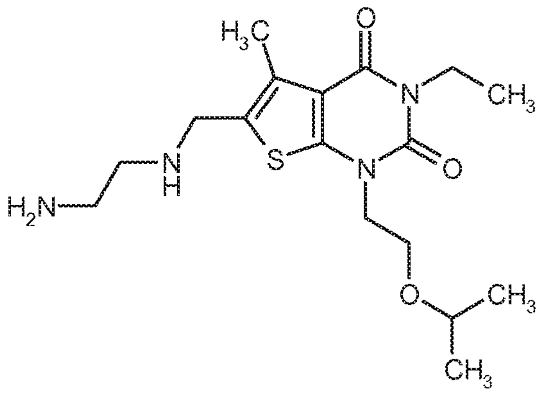

Analog zu dem unter Bsp. 210A beschriebenen Verfahren wurden aus 169 mg (0.495 mmol) der Verbindung aus Bsp. 86A und 1,2-Diaminoethan 206 mg (75% d. Th., 67% Reinheit) der Titelverbindung hergestellt. Die Reaktionszeit betrug hier ca. 64 h.
LC/MS (Methode 3): $R_t$ = 0.59 min, m/z = 369 $[M+H]^+$.

**Beispiel 223A**

6-{[(2-Aminoethyl)amino]methyl}-3-ethyl-1-(2-methoxypropyl)-5-methylthieno[2,3-d]pyrimidin-2,4(1*H*,3*H*)-dion (*Racemat*)

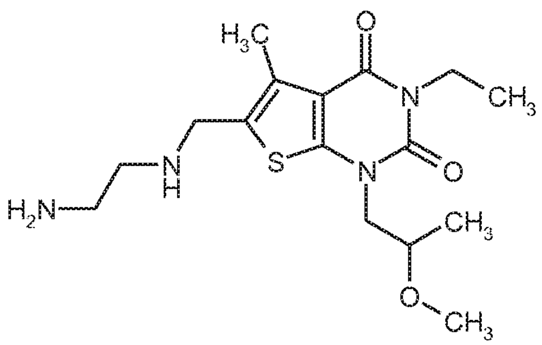

Analog zu dem unter Bsp. 209A beschriebenen Verfahren wurden aus 325 mg (1.05 mmol) der Verbindung aus Bsp. 87A und 1,2-Diaminoethan 425 mg (98% d. Th., 86% Reinheit) der Titelverbindung hergestellt.
LC/MS (Methode 1, ESIpos): $R_t$ = 0.37 min, m/z = 295 $[M+H-C_2H_8N_2]^+$.

**Beispiel 224A**

6-([(2-Aminoethyl)amino]methyl)-3-ethyl-5-methyl-1-(oxetan-2-ylmethyl)thieno[2,3-d]pyrünidin-2,4(1*H*,3*H*)-dion (*Racemat*)

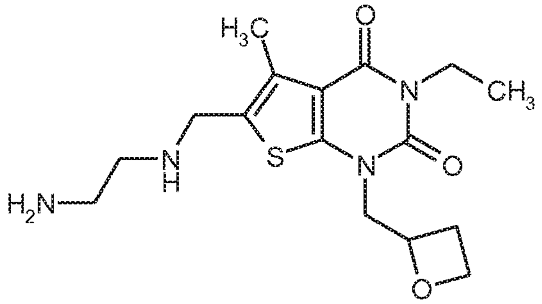

375 mg (1.1 mmol) der Verbindung aus Bsp. 88A wurden in einem Gemisch aus 18.15 ml Methanol und 7.83 ml Dichlormethan gelöst. Dann wurden bei RT 740 µl (11.06 mmol) 1,2-Diaminoethan und 253 µl (4.42 mmol) Essigsäure zugesetzt. Es wurde 30 min bei RT gerührt. Anschließend wurden 293 mg (4.42 mmol) Natriumcyanoborhydrid hinzugefügt. Nachdem das Reaktionsgemisch 65 h bei 60°C gerührt worden war, wurde mit 50 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt wurde mittels präparativer HPLC aufgereinigt (Methode 14). Nach Vereinigen der Produktfraktionen und Gefriertrocknung wurden 339 mg (64% d. Th., 63% Reinheit) der Titelverbindung erhalten.
LC/MS (Methode 3): $R_t$ = 0.48 min, m/z = 293 $[M+H-C_2H_8N_2]^+$.

**Beispiel 225A**

6-{[(2-Aminoethyl)amino]methyl}-3-ethyl-5-methyl-1-(tetrahydrofuran-2-ylmethyl)thieno[2,3-d]-pyrimidin-2,4(1*H*,3*H*)-dion (*Racemat*)

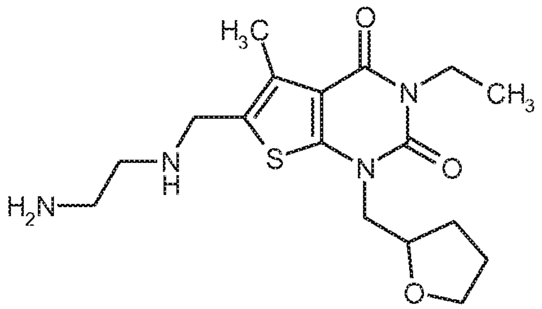

1.36 g (4.20 mmol) der Verbindung aus Bsp. 89A wurden in einem Gemisch aus 40 ml Methanol und 15 ml Dichlormethan gelöst. Anschließend wurden bei RT 1.01 g (16.8 mmol) 1,2-Diaminoethan, 962 µl (16.8 mmol) Essigsäure und, portionsweise über ca. 4 h verteilt, 1.06 g (16.8 mmol) Natriumcyanoborhydrid hinzugefügt. Nachdem das Reaktionsgemisch 2 Tage bei RT gerührt worden war, wurde mit 2 M Natronlauge versetzt und mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach dem Trocknen im Hochvakuum 2.12 g (76% d. Th., 75% Reinheit) der Titelverbindung, welche ohne weitere Reinigung für Folgereaktionen verwendet wurden.
LC/MS (Methode 1, ESIpos): $R_t$ = 0.39 min, m/z = 367 $[M+H]^+$.

**Beispiel 226A**

6-([(2-Aminoethyl)amino]methyl)-3-ethyl-5-methyl-1-(tetrahydro-2*H*-pyran-2-ylmethyl)thieno-[2,3-d]pyrimidin-2,4(1*H*,3*H*)-dion (*Racemat*)

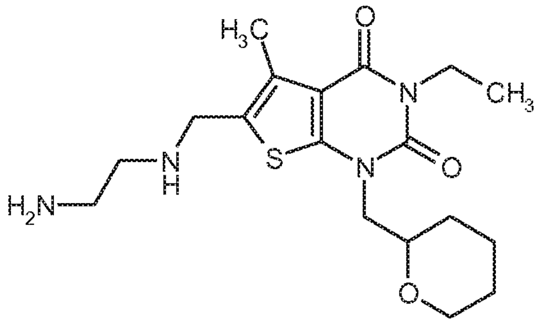

Analog zu dem unter Bsp. 210A beschriebenen Verfahren wurden aus 585 mg (1.739 mmol) der Verbindung aus Bsp. 90A und 1,2-Diaminoethan 642 mg (84% d. Th., 87% Reinheit) der Titelverbindung hergestellt.
LC/MS (Methode 3): $R_t$ = 0.59 min, m/z = 381 $[M+H]^+$.

**Beispiel 227A**

6-{[(2-Aminoethyl)amino]methyl}-3-ethyl-5-methyl-1-(oxetan-3-ylmethyl)thieno[2,3-d]pyrimidin-2,4(1*H*,3*H*)-dion

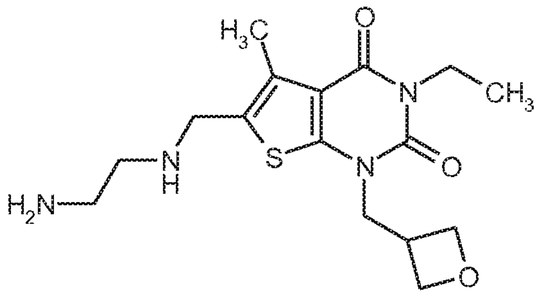

82 mg (0.266 mmol) der Verbindung aus Bsp. 91A wurden in einem Gemisch aus 2.3 ml Methanol und 0.9 ml Dichlormethan gelöst. Anschließend wurden bei RT 71 µl (1.06 mmol) 1,2-Diaminoethan, 61 µl (1.06 mmol) Essigsäure und, portionsweise über ca. 2 h verteilt, 67 mg (1.06 mmol) Natriumcyanoborhydrid hinzugefügt. Nachdem das Reaktionsgemisch ca. 18 h bei RT gerührt worden war, wurde mit 2 M Natronlauge versetzt und mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach dem Trocknen im Hochvakuum 110 mg (93% d. Th., 80% Reinheit) der Titelverbindung, welche ohne weitere Reinigung für Folgereaktionen verwendet wurden.
LC/MS (Methode 1, ESIpos): $R_t$ = 0.26 min, m/z = 353 $[M+H]^+$.

**Beispiel 228A**

6-{[(2-Aminoethyl)amino]methyl}-3-ethyl-5-methyl-1-(tetrahydrofuran-3-ylmethyl)thieno[2,3-d]-pyrimidin-2,4(1*H*,3*H*)-dion (*Racemat*)

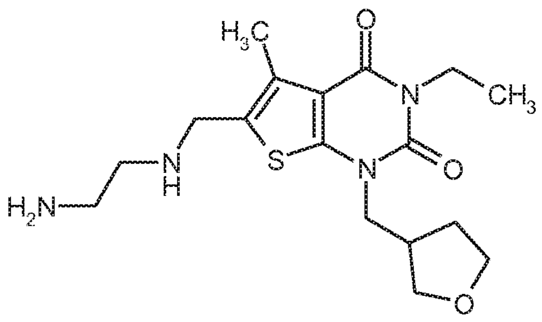

Analog zu dem unter Bsp. 209A beschriebenen Verfahren wurden aus 182 mg (0.565 mmol) der Verbindung aus Bsp. 92A und 1,2-Diaminoethan 215 mg (91% d. Th., 87% Reinheit) der Titelverbindung hergestellt.
LC/MS (Methode 1, ESIpos): $R_t$ = 0.33 min, m/z = 367 $[M+H]^+$.

**Beispiel 229A**

6-{[(2-Aminoethyl)amino]methyl}-3-ethyl-1,5-dimethylthieno[2,3-d]pyrimidin-2,4(1*H*,3*H*)-dion

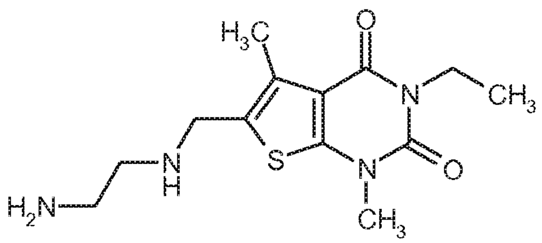

Analog zu dem unter Bsp. 209A beschriebenen Verfahren wurden aus 300 mg (1.19 mmol) der Verbindung aus Bsp. 93A und 1,2-Diaminoethan 380 mg (82% d. Th., 76% Reinheit) der Titelverbindung hergestellt. Die Reaktionszeit betrug hier ca. 18 h.
LC/MS (Methode 1, ESIpos): $R_t$ = 0.21 min, m/z = 237 $[M+H-C_2H_8N_2]^+$.

**Beispiel 230A**

6-{[(2-Aminoethyl)amino]methyl}-1,3-diethyl-5-methylthieno[2,3-d]pyrimidin-2,4(1*H*,3*H*)-dion

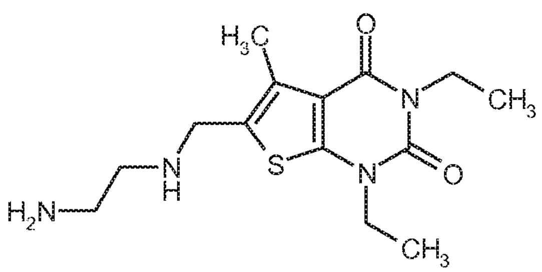

Analog zu dem unter Bsp. 209A beschriebenen Verfahren wurden aus 300 mg (1.13 mmol) der Verbindung aus Bsp. 94A und 1,2-Diaminoethan 380 mg (88% d. Th., 81% Reinheit) der Titelverbindung hergestellt. Die Reaktionszeit betrug hier ca. 18 h.
$^1$H-NMR (400 MHz, DMSO-$d_6$, $\delta$/ppm): 3.92 (q, 2H), 3.90 (q, 2H), 3.80 (s, 2H), 2.72-2.63 (m, 2H), 2.62-2.56 (m, 2H), 2.35 (s, 3H), 1.25 (t, 3H), 1.12 (t, 3H).
LC/MS (Methode 1, ESIpos): $R_t$ = 0.26 min, m/z = 251 $[M+H-C_2H_8N_2]^+$.

**Beispiel 231A**

6-{[(2-Aminoethyl)amino]methyl)-3-ethyl-5-methyl-1-propylthieno[2,3-d]pyrimidin-2,4(1*H*,3*H*)-dion

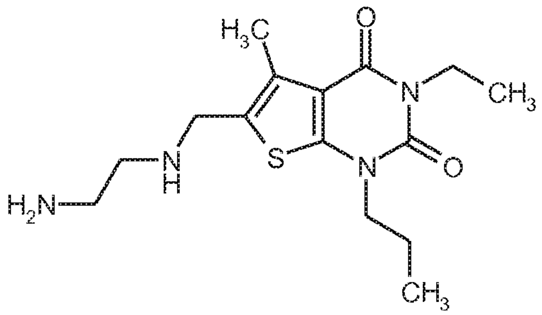

Analog zu dem unter Bsp. 209A beschriebenen Verfahren wurden aus 198 mg (0.706 mmol) der Verbindung aus Bsp. 95A und 1,2-Diaminoethan 287 mg (88% d. Th., 70% Reinheit) der Titelverbindung hergestellt.
$^1$H-NMR (400 MHz, DMSO-$d_6$, δ/ppm): 3.96-3.74 (m, 6H), 2.78-2.72 (m, 2H), 2.67-2.61 (m, 2H), 2.35 (s, 3H), 1.77-1.64 (m, 2H), 1.11 (t, 3H), 0.91 (t, 3H).
LC/MS (Methode 1, ESIpos): $R_t$ = 0.41 min, m/z = 325 $[M+H]^+$.

**Beispiel 232A**

6-{[(2-Aminoethyl)amino]methyl)-1-butyl-3-ethyl-5-methylthieno[2,3-d]pyrimidin-2,4(1*H*,3*H*)-dion

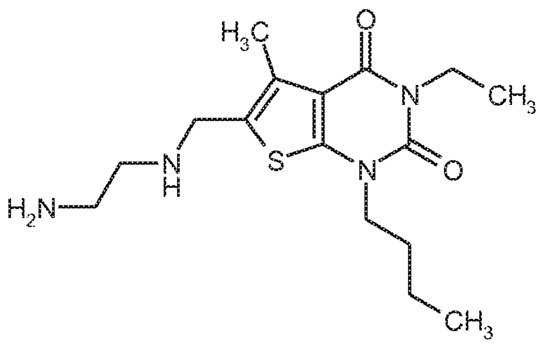

Analog zu dem unter Bsp. 209A beschriebenen Verfahren wurden aus 300 mg (1.02 mmol) der Verbindung aus Bsp. 96A und 1,2-Diaminoethan 388 mg (73% d. Th., 65% Reinheit) der Titelverbindung hergestellt.
$^1$H-NMR (400 MHz, DMSO-$d_6$, δ/ppm): 3.89 (m, 4H), 3.80 (s, 2H), 2.73-2.65 (m, 2H), 2.61-2.58 (m, 2H), 2.35 (s, 3H), 1.72-1.59 (m, 2H), 1.39-1.30 (m, 2H), 1.11 (t, 3H), 0.92 (t, 3H).
LC/MS (Methode 1, ESIpos): $R_t$ = 0.50 min, m/z = 339 $[M+H]^+$.

**Beispiel 233A**

6-{[(2-Aminoethyl)amino]methyl}-3-ethyl-5-methyl-1-(3-methylbutyl)thieno[2,3-d]pyrimidin-2,4(1*H*,3*H*)-dion

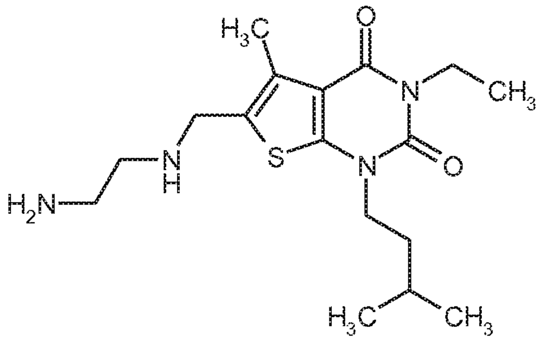

Analog zu dem unter Bsp. 209A beschriebenen Verfahren wurden aus 300 mg (0.973 mmol) der Verbindung aus Bsp. 97A und 1,2-Diaminoethan 368 mg (96% d. Th., 90% Reinheit) der Titelverbindung hergestellt. Die Reaktionszeit betrug hier ca. 18 h.
LC/MS (Methode 1, ESIpos): $R_t$ = 0.56 min, m/z = 353 $[M+H]^+$.

**Beispiel 234A**

6-{[(2-Aminoethyl)amino]methyl}-1-(3,3-dimethylbutyl)-3-ethyl-5-methylthieno[2,3-d]pyrimidin-2,4(1*H*,3*H*)-dion

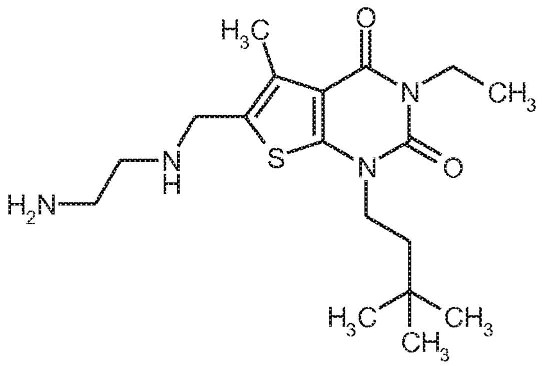

Analog zu dem unter Bsp. 210A beschriebenen Verfahren wurden aus 272 mg (0.844 mmol) der Verbindung aus Bsp. 98A und 1,2-Diaminoethan 370 mg (89% d. Th., 75% Reinheit) der Titelverbindung hergestellt.
LC/MS (Methode 3): $R_t$ = 0.77 min, m/z = 367 $[M+H]^+$.

**Beispiel 235A**

6-{[(2-Aminoethyl)amino]methyl}-1-(cyclobutylmethyl)-3-ethyl-5-methylthieno[2,3-d]pyrimidin-2,4(1*H*,3*H*)-dion

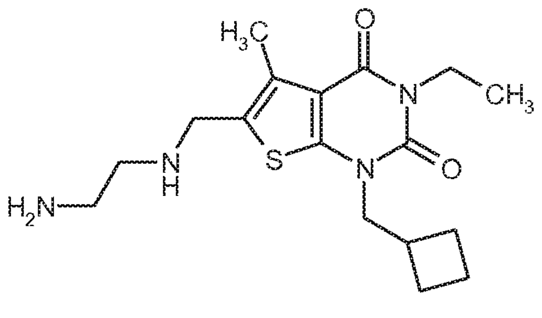

Analog zu dem unter Bsp. 209A beschriebenen Verfahren wurden aus 300 mg (0.979 mmol) der Verbindung

aus Bsp. 99A und 1,2-Diaminoethan 350 mg (92% d. Th., 90% Reinheit) der Titelverbindung hergestellt. Die Reaktionszeit betrug hier 4 Tage.
LC/MS (Methode 1, ESIpos): $R_t$ = 0.44 min, m/z = 291 $[M+H-C_2H_8N_2]^+$.

**Beispiel 236A**

[6-{[(2-Aminoethyl)amino]methyl}-3-ethyl-5-methyl-2,4-dioxo-3,4-dihydrothieno[2,3-d]pyrimidin-1(2*H*)-yl]acetonitril

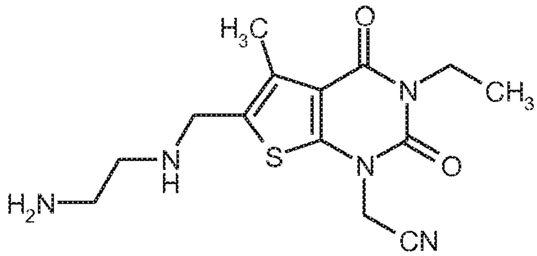

Analog zu dem unter Bsp. 209A beschriebenen Verfahren wurden aus 300 mg (1.08 mmol) der Verbindung aus Bsp. 100A und 1,2-Diaminoethan 289 mg (80% d. Th., 80% Reinheit) der Titelverbindung hergestellt. Die Reaktionszeit betrug hier 5 Tage.
LC/MS (Methode 1, ESIpos): $R_t$ = 0.17 min, keine Ionisierung.

**Beispiel 237A**

6-{[(2-Aminoethyl)amino]methyl}-3-ethyl-5-melhyl-1-[(4-methyl-1,2,5-oxadiazol-3-yl)methyl]-thieno[2,3-d]pyrimidin-2,4(1*H*,3*H*)-dion

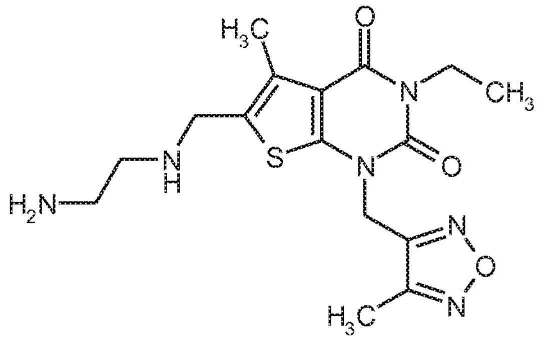

Analog zu dem unter Bsp. 209A beschriebenen Verfahren wurden aus 380 mg (1.34 mmol) der Verbindung aus Bsp. 101A und 1,2-Diaminoethan 492 mg (94% d. Th., 82% Reinheit) der Titelverbindung hergestellt.
LC/MS (Methode 1, ESIpos): $R_t$ = 0.42 min, m/z = 379 $[M+H]^+$.

**Beispiel 238A**

6-{[(2-Aminoethyl)amino]memyl}-1-[2-(dimethylamino)emyl]-3-ethyl-5-methylthieno[2,3-d]-pyrimidin-2,4(1*H*,3*H*)-dion

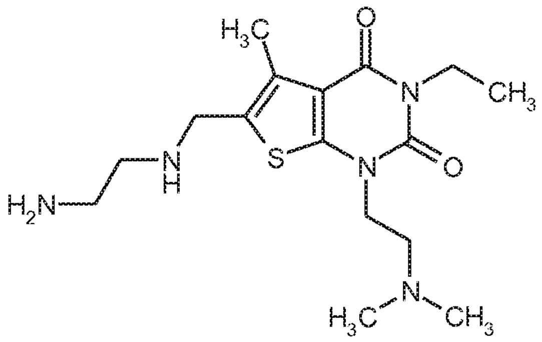

Analog zu dem unter Bsp. 209A beschriebenen Verfahren wurden aus 434 mg (1.40 mmol) der Verbindung aus Bsp. 102A und 1,2-Diaminoethan 665 mg (99% d. Th., 73% Reinheit) der Titelverbindung hergestellt. Die Reaktionszeit betrug hier 5 Tage.
LC/MS (Methode 1, ESIpos): $R_t$ = 0.15 min, keine Ionisierung.

**Beispiel 239A**

6-{[(2-Aminoethyl)amino]methyl}-3-isobutyl-5-methyl-1-(3,3,3-trifluorpropyl)thieno[2,3-d]-pyrimidin-2,4(1*H*,3*H*)-dion

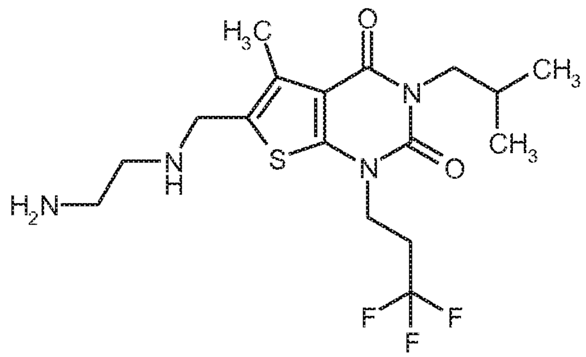

600 mg (1.65 mmol) der Verbindung aus Bsp. 110A wurden in einem Gemisch aus 13.59 ml Methanol und 5.84 ml Dichlormethan gelöst. Dann wurden bei RT 1.77 ml (26.49 mmol) 1,2-Diaminoethan und 569 µl (9.93 mmol) Essigsäure zugesetzt. Es wurde 30 min bei RT gerührt. Anschließend wurden 657 mg (9.93 mmol) Natriumcyanoborhydrid hinzugefügt. Nachdem das Reaktionsgemisch 24 h bei 70°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt wurde mittels präparativer HPLC gereinigt (Methode 14). Nach Vereinigen der Produktfraktionen und Gefriertrocknung wurden 228 mg (32% d. Th., 95% Reinheit) der Titelverbindung erhalten.
$^1$H-NMR (400 MHz, DMSO-$d_6$, δ/ppm): 8.56-8.24 (m, 1H), 4.12 (t, 2H), 3.82 (br. s, 3H), 3.71 (d, 3H), 2.84-2.64 (m, 6H), 2.35 (s, 3H), 2.03 (dquin, 1H), 0.84 (d, 6H).
LC/MS (Methode 3): $R_t$ = 0.74 min, m/z = 407 [M+H]$^+$.

**Beispiel 240A**

6-([(2-Aminoethyl)amino]methyl}-1-(2-fluorethyl)-3-isobutyl-5-methylthieno[2,3-d]pyrimidin-2,4(1*H*,3*H*)-dion

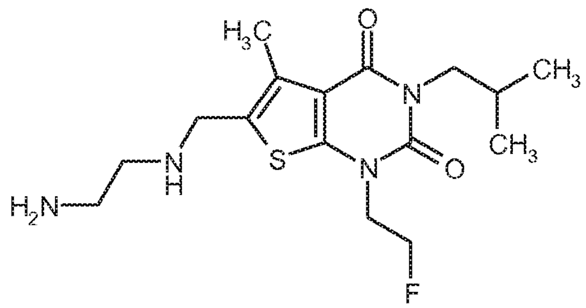

Analog zu dem unter Bsp. 209A beschriebenen Verfahren wurden aus 465 mg (1.49 mmol) der Verbindung aus Bsp. 112A und 1,2-Diaminoethan 528 mg (75% d. Th., 75% Reinheit) der Titelverbindung hergestellt. Die Reaktionszeit betrug hier 5 Tage.
LC/MS (Methode 5, ESIpos): $R_t$ = 0.56 min, m/z = 297 $[M+H-C_2H_3N_2]^+$.

**Beispiel 241A**

6-{[(2-Aminoethyl)amino]methyl}-1-(3-fluorpropyl)-3-isobutyl-5-methylthieno[2,3-d]pyrimidin-2,4(1*H*,3*H*)-dion

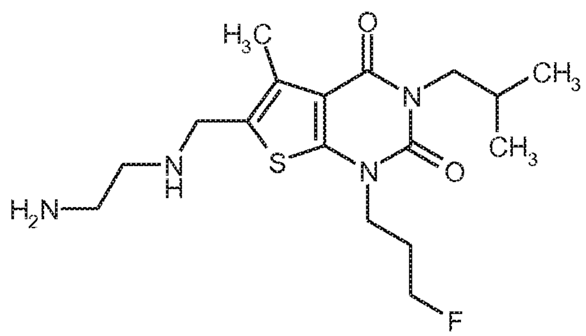

Analog zu dem unter Bsp. 210A beschriebenen Verfahren wurden aus 320 mg (0.941 mmol) der Verbindung aus Bsp. 113A und 1,2-Diaminoethan 446 mg (74% d. Th., 58% Reinheit) der Titelverbindung hergestellt.
LC/MS (Methode 3): $R_t$ = 0.64 min, m/z = 371 $[M+H]^+$.

**Beispiel 242A**

6-([(2-Aminoethyl)amino]methyl)-3-isobutyl-1-(2-methoxyethyl)-5-methylthieno[2,3-d]pyrimidin-2,4(1*H*,3*H*)-dion

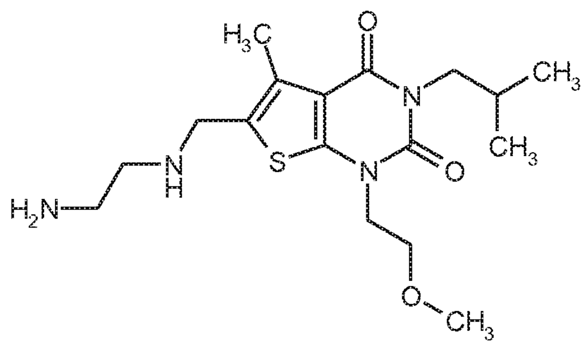

500 mg (1.49 mmol) der Verbindung aus Bsp. 114A wurden in einem Gemisch aus 30.28 ml Methanol und 14.43 ml Dichlormethan gelöst. Dann wurden bei RT 999 µl (14.95 mmol) 1,2-Diaminoethan und 342 µl (5.98 mmol)

Essigsäure zugesetzt. Es wurde 30 min bei RT gerührt. Anschließend wurden 395 mg (5.98 mmol) Natriumcyanoborhydrid hinzugefügt. Nachdem das Reaktionsgemisch 89 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 580 mg (73% d. Th., 70% Reinheit) der Titelverbindung und wurde ohne weitere Reinigung für Folgereaktionen verwendet.
LC/MS (Methode 3): $R_t$ = 0.63 min, m/z = 369 $[M+H]^+$.

**Beispiel 243A**

6-([(2-Aminoethyl)amino]methyl)-3-isobutyl-5-methyl-1-(oxetan-2-ylmethyl)thieno[2,3-d]-pyrimidin-2,4(1*H*,3*H*)-dion *(Racemat)*

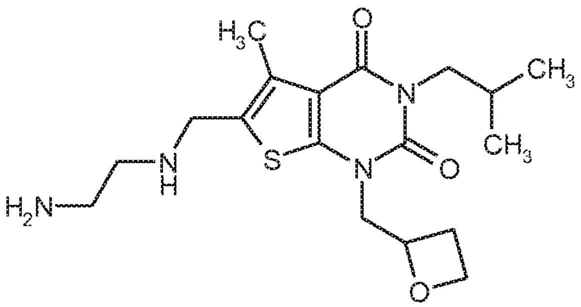

845 mg (2.51 mmol) der Verbindung aus Bsp. 116A wurden in einem Gemisch aus 50.87 ml Methanol und 24.24 ml Dichlormethan gelöst. Dann wurden bei RT 1.68 ml (25.12 mmol) 1,2-Diaminoethan und 575 µl (10.04 mmol) Essigsäure zugesetzt. Es wurde 30 min bei RT gerührt. Anschließend wurden 664 mg (10.04 mmol) Natriumcyanoborhydrid hinzugefügt. Nachdem das Reaktionsgemisch 65 h bei 60°C gerührt worden war, wurde mit 250 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 1.22 g (86% Reinheit) der Titelverbindung und wurde ohne weitere Reinigung für Folgereaktionen verwendet.
LC/MS (Methode 3): $R_t$ = 0.62 min, m/z = 381 $[M+H]^+$.

**Beispiel 244A**

6-{[(2-Aminoethyl)amino]methyl)-3-isobutyl-5-methyl-1-(tetrahydrofuran-2-ylmethyl)thieno-[2,3-d]pyrimidin-2,4(1*H*,3*H*)-dion (*Racemat*)

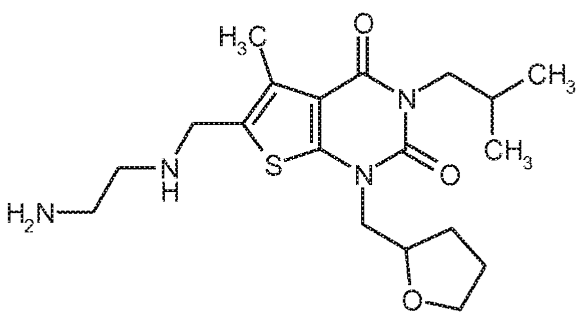

498 mg (1.42 mmol) der Verbindung aus Bsp. 117A wurden in einem Gemisch aus 13 ml Methanol und 5 ml Dichlormethan gelöst. Anschließend wurden bei RT 380 µl (5.68 mmol) 1,2-Diaminoethan, 325 µl (5.68 mmol) Essigsäure und, portionsweise über ca. 4 h verteilt, 357 mg (5.68 mmol) Natriumcyanoborhydrid hinzugefügt. Nachdem das Reaktionsgemisch 2 Tage bei RT gerührt worden war, wurde mit 2 M Natronlauge versetzt und mit Ethylacetat extrahiert.

Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach dem Trocknen im Hochvakuum 710 mg (98% d. Th., 79% Reinheit) der Titelverbindung, welche ohne weitere Reinigung für Folgereaktionen verwendet wurden.
LC/MS (Methode 1, ESIpos): $R_t$ = 0.50 min, m/z = 395 $[M+H]^+$.

**Beispiel 245A**

6-{[(2-Aminoethyl)amino]methyl}-3-isobutyl-5-methyl-1-(oxetan-3-ylmethyl)thieno[2,3-d]-pyrimidin-2,4(1*H*,3*H*)-dion

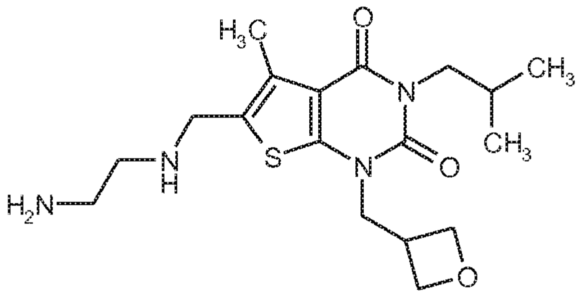

75 mg (0.223 mmol) der Verbindung aus Bsp. 118A wurden in einem Gemisch aus 2 ml Methanol und 0.8 ml Dichlormethan gelöst. Anschließend wurden bei RT 60 µl (0.892 mmol) 1,2-Diaminoethan, 51 µl (0.892 mmol) Essigsäure und, portionsweise über ca. 2 h verteilt, 56 mg (0.892 mmol) Natriumcyanoborhydrid hinzugefügt. Nachdem das Reaktionsgemisch 3 Tage bei RT gerührt worden war, wurde mit 2 M Natronlauge versetzt und mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach dem Trocknen im Hochvakuum 84 mg (70% d. Th., 70% Reinheit) der Titelverbindung, welche ohne weitere Reinigung für Folgereaktionen verwendet wurden.
LC/MS (Methode 1, ESIpos): $R_t$ = 0.47 min, m/z = 381 $[M+H]^+$.

**Beispiel 246A**

6-{[(2-Aminoethyl)amino]methyl}-3-isobutyl-1,5-dimethylthieno[2,3-d]pyrimidin-2,4(1*H*,3*H*)-dion

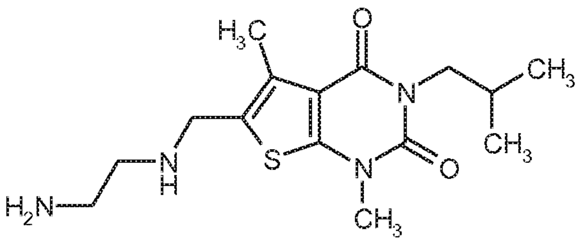

Analog zu dem unter Bsp. 209A beschriebenen Verfahren wurden aus 170 mg (0.606 mmol) der Verbindung aus Bsp. 119A und 1,2-Diaminoethan 195 mg (80% d. Th., 80% Reinheit) der Titelverbindung hergestellt. Die Reaktionszeit betrug hier 5 Tage.
LC/MS (Methode 1, ESIpos): $R_t$ = 0.48 min, m/z = 265 $[M+H-C_2H_8N_2]^+$.

**Beispiel 247A**

6-{[(2-Aminoethyl)amino]methyl)-5-methyl-3-(2,2,2-trifluorethyl)-1-(3,3,3-trifluorpropyl)thieno-[2,3-d]pyrimidin-2,4(1*H*,3*H*)-dion

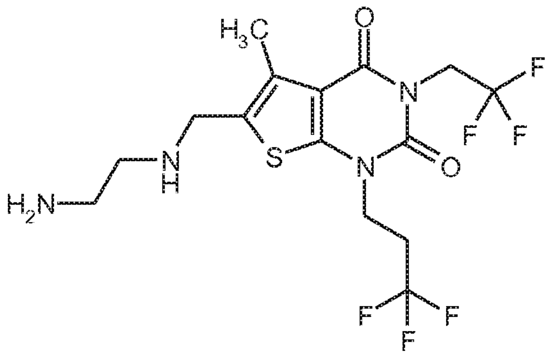

380 mg (0.979 mmol) der Verbindung aus Bsp. 120A wurden in einem Gemisch aus 19.82 ml Methanol und 9.44 ml Dichlormethan gelöst. Dann wurden bei RT 654 μl (9.78 mmol) 1,2-Diaminoethan und 224 μl (3.91 mmol) Essigsäure zugesetzt. Es wurde 30 min bei RT gerührt. Anschließend wurden 258 mg (3.91 mmol) Natriumcyanoborhydrid hinzugefügt. Nachdem das Reaktionsgemisch 89 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 526 mg (ca. 80% Reinheit) der Titelverbindung und wurde ohne weitere Reinigung für Folgereaktionen verwendet.
LC/MS (Methode 3): $R_t$ = 0.68 min, m/z = 433 $[M+H]^+$.

**Beispiel 248A**

6-{[(2-Aminoethyl)amino]methyl}-1-(3-fluorpropyl)-5-methyl-3-(2,2,2-trifluorethyl)thieno[2,3-d]-pyrimidin-2,4(1*H*,3*H*)-dion

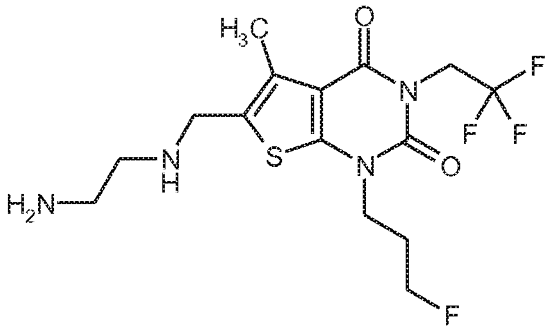

Analog zu dem unter Bsp. 210A beschriebenen Verfahren wurden aus 450 mg (1.27 mmol) der Verbindung aus Bsp. 121A und 1,2-Diaminoethan 521 mg (74% d. Th., 72% Reinheit) der Titelverbindung hergestellt.
LC/MS (Methode 3): $R_t$ = 0.64 min, m/z = 397 $[M+H]^+$.

**Beispiel 249A**

6-{[(2-Aminoethyl)amino]methyl}-1-(2-methoxyethyl)-5-methyl-3-(2,2,2-trifluorethyl)thieno-[2,3-d]pyrimidin-2,4(1*H*,3*H*)-dion

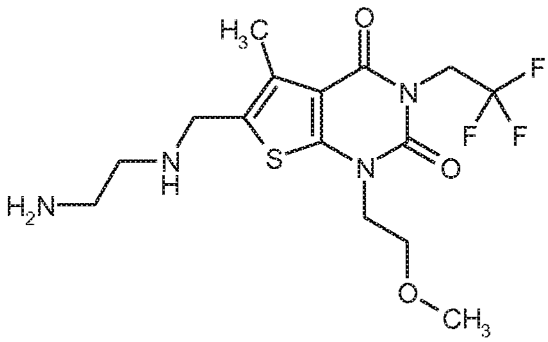

382 mg (1.09 mmol) der Verbindung aus Bsp. 122A wurden in einem Gemisch aus 18.11 ml Methanol und 7.71 ml Dichlormethan gelöst. Dann wurden bei RT 729 µl (10.9 mmol) 1,2-Diaminoethan und 250 µl (1.05 mmol) Essigsäure zugesetzt. Es wurde 30 min bei RT gerührt. Anschließend wurden 288 mg (4.36 mmol) Natriumcyanoborhydrid hinzugefügt. Nachdem das Reaktionsgemisch 112 h bei 60°C gerührt worden war, wurde mit 50 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 408 mg (62% d. Th., 66% Reinheit) der Titelverbindung und wurde ohne weitere Reinigung für Folgereaktionen verwendet.
LC/MS (Methode 3): $R_t$ = 0.60 min, m/z = 395 $[M+H]^+$.

**Beispiel 250A**

6-{[(2-Aminoethyl)amino]methyl}-1-(2-ethoxyethyl)-5-methyl-3-(2,2,2-trifluorethyl)thieno[2,3-d]-pyrimidin-2,4(1*H*,3*H*)-dion

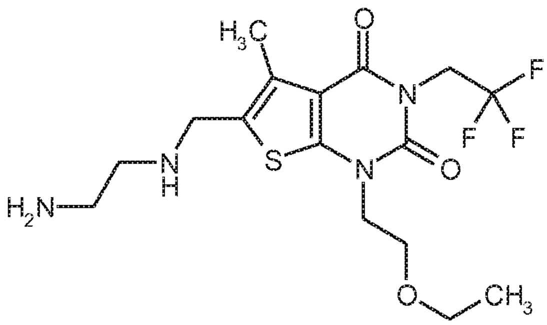

Analog zu dem unter Bsp. 210A beschriebenen Verfahren wurden aus 198 mg (0.538 mmol) der Verbindung aus Bsp. 123A und 1,2-Diaminoethan 227 mg (69% d. Th., 64% Reinheit) der Titelverbindung hergestellt.
LC/MS (Methode 3): $R_t$ = 0.64 min, m/z = 409 $[M+H]^+$.

**Beispiel 251A**

6-{[(2-Aminoethyl)amino]methyl}-5-methyl-1-(oxetan-2-ylmethyl)-3-(2,2,2-trifluorethyl)thieno-[2,3-d]pyrimidin-2,4(1*H*,3*H*)-dion (*Racemat*)

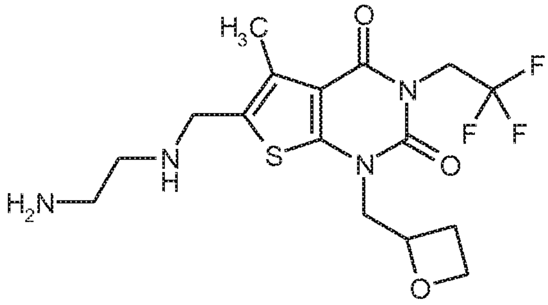

605 mg (1.67 mmol) der Verbindung aus Bsp. 124A wurden in einem Gemisch aus 33.82 ml Methanol und 16.11 ml Dichlormethan gelöst. Dann wurden bei RT 1.11 ml (16.69 mmol) 1,2-Diaminoethan und 382 µl (6.68 mmol) Essigsäure zugesetzt. Es wurde 30 min bei RT gerührt. Anschließend wurden 442 mg (6.68 mmol) Natriumcyanoborhydrid hinzugefügt. Nachdem das Reaktionsgemisch 89 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 855 mg (ca. 75% Reinheit) der Titelverbindung und wurde ohne weitere Reinigung für Folgereaktionen verwendet.
LC/MS (Methode 3): $R_t$ = 0.55 min, m/z = 407 $[M+H]^+$.

**Beispiel 252A**

6-{[(2-Aminoethyl)amino]methyl}-5-methyl-1-(tetrahydrofuran-2-ylmethyl)-3-(2,2,2-trifluor-ethyl)thieno[2,3-d]pyrimidin-2,4(1*H*,3*H*)-dion (*Racemat*)

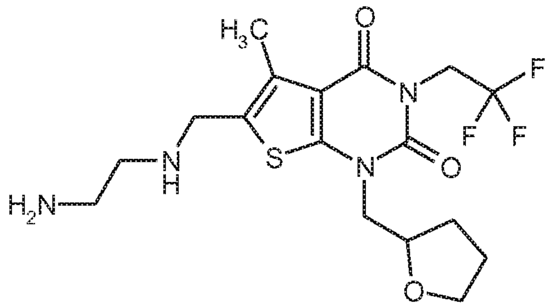

685 mg (1.82 mmol) der Verbindung aus Bsp. 125A wurden in einem Gemisch aus 36.86 ml Methanol und 17.56 ml Dichlormethan gelöst. Dann wurden bei RT 1.217 ml (18.2 mmol) 1,2-Diaminoethan und 417 µl (7.28 mmol) Essigsäure zugesetzt. Es wurde 30 min bei RT gerührt. Anschließend wurden 481 mg (7.28 mmol) Natriumcyanoborhydrid hinzugefügt. Nachdem das Reaktionsgemisch 65 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 855 mg (90% d. Th., 81% Reinheit) der Titelverbindung und wurde ohne weitere Reinigung für Folgereaktionen verwendet.
LC/MS (Methode 3): $R_t$ = 0.63 min, m/z = 421 $[M+H]^+$.

**Beispiel 253A**

6-{[(2-Aminoethyl)amino]methyl)-5-methyl-1-(oxetan-3-ylmethyl)-3-(2,2,2-trifluorethyl)thieno-[2,3-d]pyrimidin-2,4(1*H*,3*H*)-dion

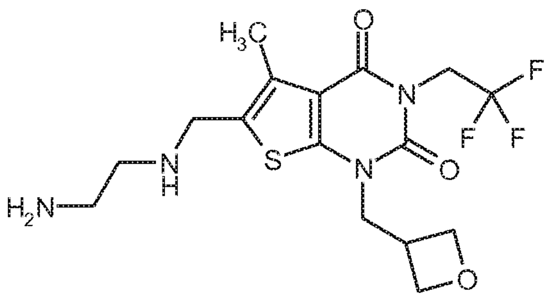

209 mg (0.577 mmol) der Verbindung aus Bsp. 126A wurden in einem Gemisch aus 11.68 ml Methanol und 5.56 ml Dichlormethan gelöst. Dann wurden bei RT 386 µl (5.76 mmol) 1,2-Diaminoethan und 132 µl (2.31 mmol) Essigsäure zugesetzt. Es wurde 30 min bei RT gerührt. Anschließend wurden 152 mg (2.31 mmol) Natriumcyanoborhydrid hinzugefügt. Nachdem das Reaktionsgemisch 89 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 218 mg (31% d. Th., 34% Reinheit) der Titelverbindung und wurde ohne weitere Reinigung für Folgereaktionen verwendet.
LC/MS (Methode 3): $R_t$ = 0.58 min, m/z = 407 $[M+H]^+$.

## Beispiel 254A

6-{[(2-Aminoethyl)amino]methyl}-1,5-dimethyl-3-(2,2,2-trifluorethyl)thieno[2,3-d]pyrimidin-2,4(1*H*,3*H*)-dion

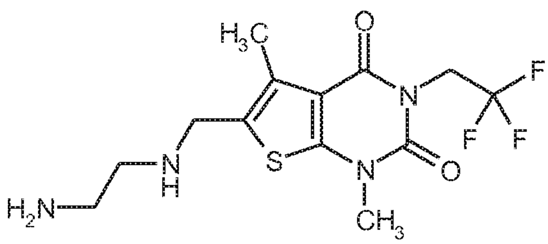

Analog zu dem unter Bsp. 210A beschriebenen Verfahren wurden aus 400 mg (1.28 mmol) der Verbindung aus Bsp. 127A und 1,2-Diaminoethan 508 mg (49% d. Th., 44% Reinheit) der Titelverbindung hergestellt.
LC/MS (Methode 3): $R_t$ = 0.53 min, m/z = 351 $[M+H]^+$.

## Beispiel 255A

6-{[(2-Aminoethyl)amino]methyl}-3-(2,2-difluorethyl)-5-methyl-1-(3,3,3-trifluorpropyl)thieno-[2,3-d]pyrimidin-2,4(1*H*,3*H*)-dion

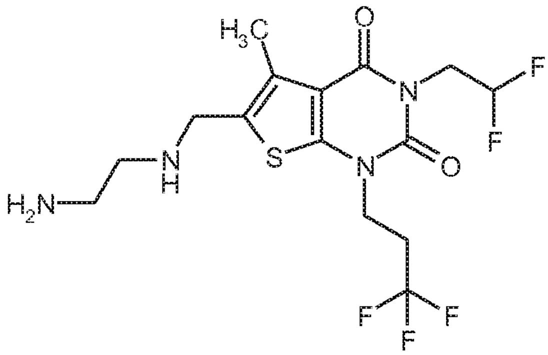

315 mg (0.815 mmol) der Verbindung aus Bsp. 128A wurden in einem Gemisch aus 17.23 ml Methanol und 8.21 ml Dichlormethan gelöst. Dann wurden bei RT 569 µl (8.5 mmol) 1,2-Diaminoethan und 195 µl (3.4 mmol) Essigsäure zugesetzt. Es wurde 30 min bei RT gerührt. Anschließend wurden 225 mg (3.4 mmol) Natriumcyanoborhydrid hinzugefügt. Nachdem das Reaktionsgemisch 90 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 402 mg (81% d. Th., 71% Reinheit) der Titelverbindung und wurde ohne weitere Reinigung für Folgereaktionen verwendet.
LC/MS (Methode 3): $R_t$ = 0.61 min, m/z = 415 $[M+H]^+$.

**Beispiel 256A**

6-{[(2-Aminoethyl)amino]methyl}-3-(2,2-difluorethyl)-1-(3-fluorpropyl)-5-methylthieno[2,3-d]-pyrimidin-2,4(1*H*,3*H*)-dion

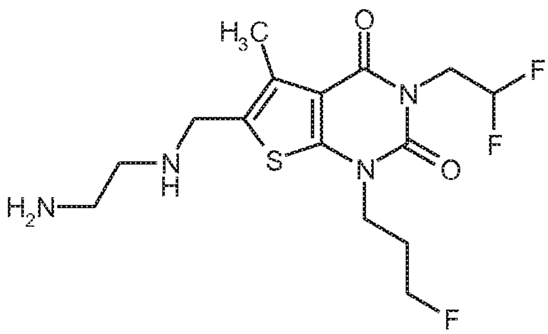

Analog zu dem unter Bsp. 210A beschriebenen Verfahren wurden aus 300 mg (0.879 mmol) der Verbindung aus Bsp. 129A und 1,2-Diaminoethan 395 mg (99% d. Th., 84% Reinheit) der Titelverbindung hergestellt.
LC/MS (Methode 3): $R_t$ = 0.54 min, m/z = 379 $[M+H]^+$.

**Beispiel 257A**

6-{[(2-Aminoethyl)amino]methyl}-3-(2,2-difluorethyl)-1-(2-methoxyethyl)-5-methylthieno[2,3-d]-pyrimidin-2,4(1*H*,3*H*)-dion

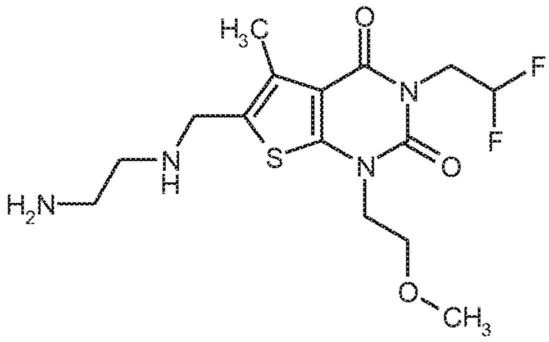

300 mg (0.894 mmol) der Verbindung aus Bsp. 130A wurden in einem Gemisch aus 18.1 ml Methanol und 8.62 ml Dichlormethan gelöst. Dann wurden bei RT 597 µl (8.93 mmol) 1,2-Diaminoethan und 205 µl (3.57 mmol) Essigsäure zugesetzt. Es wurde 30 min bei RT gerührt. Anschließend wurden 236 mg (3.57 mmol) Natriumcyanoborhydrid hinzugefügt. Nachdem das Reaktionsgemisch 64 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 356 mg (76% d. Th., 72% Reinheit) der Titelverbindung und wurde ohne weitere Reinigung für Folgereaktionen verwendet.
LC/MS (Methode 3): $R_t$ = 0.48 min, m/z = 377 $[M+H]^+$.

**Beispiel 258A**

6-{[(2-Aminoethyl)amino]methyl}-3-(2,2-difluorethyl)-1-(2-ethoxyethyl)-5-methylthieno[2,3-d]-pyrimidin-2,4(1*H*,3*H*)-dion

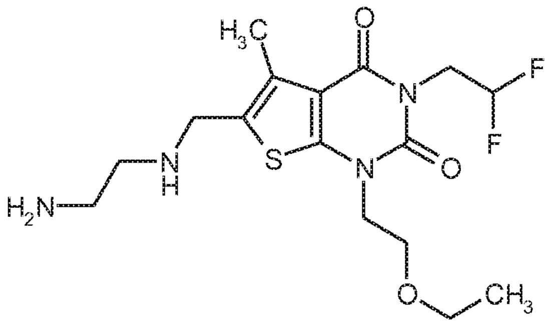

Analog zu dem unter Bsp. 210A beschriebenen Verfahren wurden aus 185 mg (0.518 mmol) der Verbindung aus Bsp. 131A und 1,2-Diaminoethan 240 mg (89% d. Th., 75% Reinheit) der Titelverbindung hergestellt.
LC/MS (Methode 3): $R_t$ = 0.58 min, m/z = 391 $[M+H]^+$.

**Beispiel 259A**

6-{[(2-Aminoethyl)amino]methyl}-3-(2,2-difluorethyl)-5-methyl-1-(oxetan-2-ylmethyl)thieno-[2,3-d]pyrimidin-2,4(1*H*,3*H*)-dion (*Racemat*)

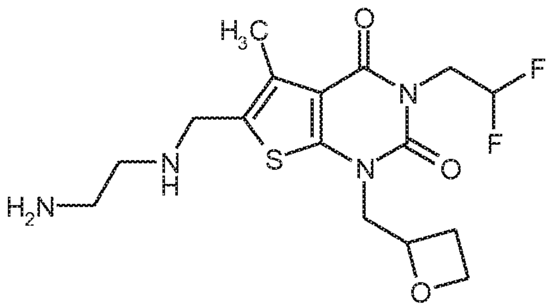

330 mg (0.939 mmol) der Verbindung aus Bsp. 132A wurden in einem Gemisch aus 19 ml Methanol und 9 ml Dichlormethan gelöst. Dann wurden bei RT 628 µl (9.39 mmol) 1,2-Diaminoethan und 215 µl (3.75 mmol) Essigsäure zugesetzt. Es wurde 30 min bei RT gerührt. Anschließend wurden 248 mg (3.75 mmol) Natriumcyanoborhydrid hinzugefügt. Nachdem das Reaktionsgemisch 60 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 446 mg (96% d. Th., 79% Reinheit) der Titelverbindung und wurde ohne weitere Reinigung für Folgereaktionen verwendet.
LC/MS (Methode 3): $R_t$ = 0.51 min, m/z = 389 $[M+H]^+$.

**Beispiel 260A**

6-{[(2-Aminoethyl)amino]methyl}-3-(2,2-difluorethyl)-5-methyl-1-(tetrahydrofuran-2-ylmethyl)-thieno[2,3-d]pyrimidin-2,4(1*H*,3*H*)-dion (*Racemat*)

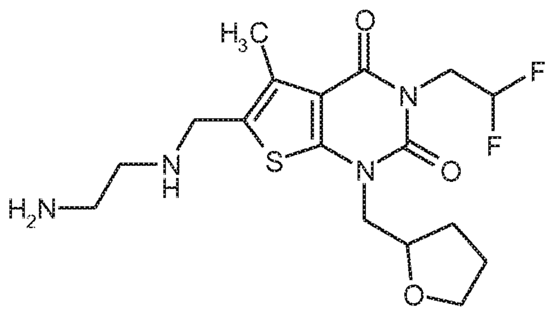

515 mg (1.437 mmol) der Verbindung aus Bsp. 133A wurden in einem Gemisch aus 29.1 ml Methanol und 13.87 ml Dichlormethan gelöst. Dann wurden bei RT 961 µl (14.37 mmol) 1,2-Diaminoethan und 329 µl (5.74 mmol) Essigsäure zugesetzt. Es wurde 30 min bei RT gerührt. Anschließend wurden 380 mg (5.74 mmol) Natriumcyanoborhydrid hinzugefügt. Nachdem das Reaktionsgemisch 67 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 667 mg (72% d. Th., 63% Reinheit) der Titelverbindung und wurde ohne weitere Reinigung für Folgereaktionen verwendet.
LC/MS (Methode 3): $R_t$ = 0.55 min, m/z = 403 $[M+H]^+$.

**Beispiel 261A**

6-{[(2-Aminoethyl)amino]methyl}-3-(2,2-difluorethyl)-5-methyl-1-(oxetan-3-ylmethyl)thieno-[2,3-d]pyrimidin-2,4(1*H*,3*H*)-dion

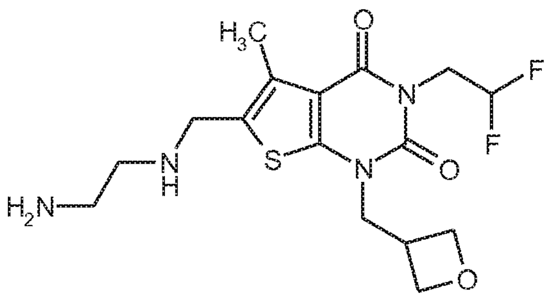

197 mg (0.571 mmol) der Verbindung aus Bsp. 134A wurden in einem Gemisch aus 11.56 ml Methanol und 5.51 ml Dichlormethan gelöst. Dann wurden bei RT 382 µl (5.71 mmol) 1,2-Diaminoethan und 131 µl (2.28 mmol) Essigsäure zugesetzt. Es wurde 30 min bei RT gerührt. Anschließend wurden 151 mg (2.28 mmol) Natriumcyanoborhydrid hinzugefügt. Nachdem das Reaktionsgemisch 63 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 171 mg (51% d. Th., 67% Reinheit) der Titelverbindung und wurde ohne weitere Reinigung für Folgereaktionen verwendet.
LC/MS (Methode 3): $R_t$ = 0.51 min, m/z = 389 $[M+H]^+$.

**Beispiel 262A**

6-{[(2-Aminoethyl)amino]methyl}-1,3-bis(2-methoxyethyl)-5-methylthieno[2,3-d]pyrimidin-2,4(1*H*,3*H*)-dion

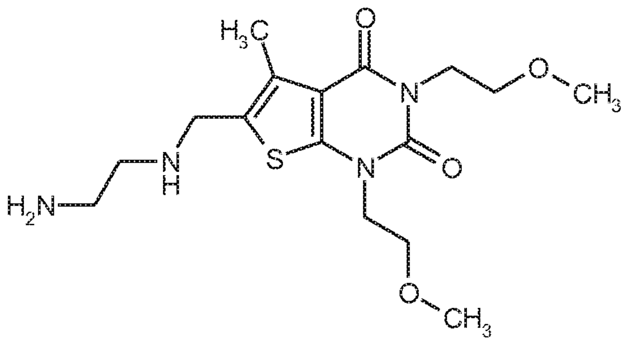

357 mg (1.08 mmol) der Verbindung aus Bsp. 136A wurden in einem Gemisch aus 21.93 ml Methanol und 10.45 ml Dichlormethan gelöst. Dann wurden bei RT 724 µl (10.83 mmol) 1,2-Diaminoethan und 248 µl (4.33 mmol) Essigsäure zugesetzt. Es wurde 30 min bei RT gerührt. Anschließend wurden 286 mg (4.33 mmol) Natriumcyanoborhydrid hinzugefügt. Nachdem das Reaktionsgemisch 112 h bei 60°C gerührt worden war, wurde mit 100 ml Wasser versetzt und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 597 mg (73% d. Th. 49% Reinheit) der Titelverbindung und wurde ohne weitere Reinigung für Folgereaktionen verwendet.
LC/MS (Methode 3): $R_t$ = 0.49 min, m/z = 371 $[M+H]^+$.

**Beispiel 263A**

6-{[(2-Aminoethyl)amino]methyl}-1-(2-ethoxyethyl)-3-(2-methoxyethyl)-5-methylthieno[2,3-d]-pyrimidin-2,4(1*H*,3*H*)-dion

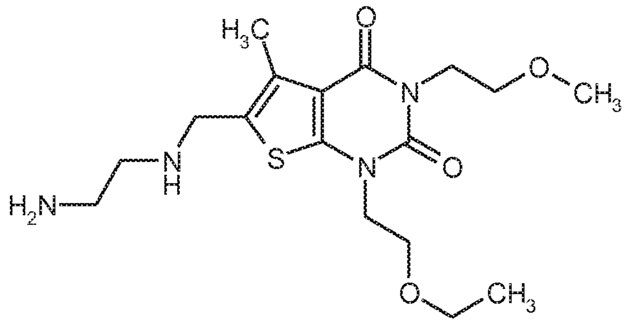

Analog zu dem unter Bsp. 210A beschriebenen Verfahren wurden aus 355 mg (1.01 mmol) der Verbindung aus Bsp. 136A und 1,2-Diaminoethan 287 mg (42% d. Th., 58% Reinheit) der Titelverbindung hergestellt. LC/MS (Methode 3): $R_t$ = 0.54 min, m/z = 385 $[M+H]^+$.

**Beispiel 264A**

6-{[(2-Aminoethyl)amino]methyl}-5-(difluormethyl)-3-ethyl-1-(3,3,3-trifluorpropyl)thieno[2,3-d]-pyrimidin-2,4(1*H*,3*H*)-dion

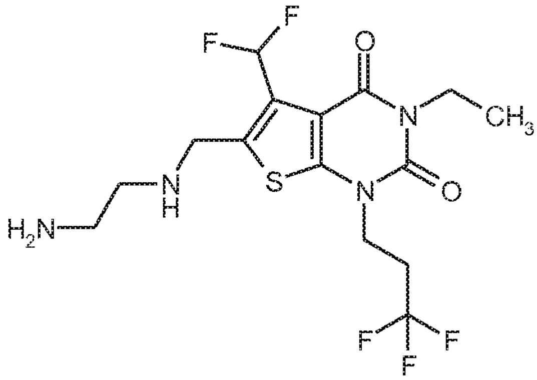

Analog zu dem unter Bsp. 210A beschriebenen Verfahren wurden aus 300 mg (0.81 mmol) der Verbindung aus Bsp. 185A und 1,2-Diaminoethan 370 mg (24% d. Th., 22% Reinheit) der Titelverbindung hergestellt. LC/MS (Methode 3): $R_t$ = 0.74 min, m/z = 415 $[M+H]^+$.

**Beispiel 265A**

6-{[(2-Aminoethyl)amino]methyl}-5-(difluormethyl)-3-ethyl-1-(2-methoxyethyl)thieno[2,3-d]-pyrimidin-2,4(1*H*,3*H*)-dion

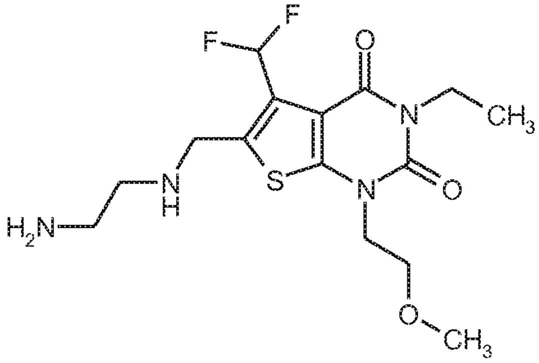

Analog zu dem unter Bsp. 210A beschriebenen Verfahren wurden aus 300 mg (0.894 mmol) der Verbindung aus Bsp. 186A und 1,2-Diaminoethan 345 mg (15% d. Th., 15% Reinheit) der Titelverbindung hergestellt.
LC/MS (Methode 3): $R_t$ = 0.62 min, m/z = 377 $[M+H]^+$.

**Beispiel 266A**

6-{[(2-Aminopropyl)amino]methyl}-3-ethyl-1-(2-methoxyethyl)-5-methylthieno[2,3-d]pyrimidin-2,4(1*H*,3*H*)-dion *(Racemat)*

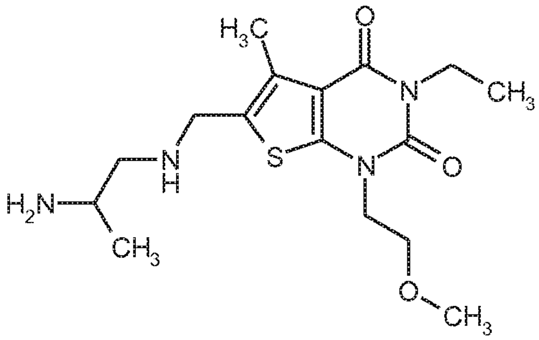

Analog zu dem unter Bsp. 210A beschriebenen Verfahren wurden aus 300 mg (0.894 mmol) der Verbindung aus Bsp. 84A und racemischem 1,2-Diaminopropan 411 mg (79% d. Th., 69% Reinheit) der Titelverbindung hergestellt.
LC/MS (Methode 3): $R_t$ = 0.53 min, m/z = 355 $[M+H]^+$.

**Beispiel 267A**

6-{[(2,2-Dimethoxyethyl)amino]methyl}-3-ethyl-5-methyl-1-(3,3,3-trifluorpropyl)thieno[2,3-d]-pyrimidin-2,4(1*H*,3*H*)-dion

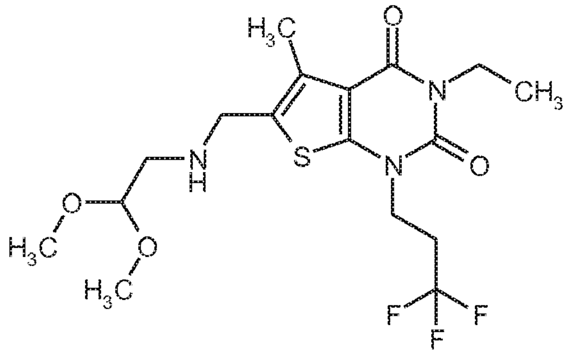

150 mg (0.413 mmol, 92% Reinheit) der Verbindung aus Bsp. 74A wurden in 10 ml Methanol gelöst und mit 65 mg (0.619 mmol) 2,2-Dimethoxyethanamin versetzt. Dann wurde das Gemisch 4 h auf 70°C erwärmt. Nach dem Abkühlen auf RT wurden 276 mg (1.24 mmol) Natriumtriacetoxyborhydrid hinzugefügt. Es wurde bei RT weiter gerührt. Nach 18 h wurden erneut 65 mg (0.619 mmol) 2,2-Dimethoxyethanamin und 30 min später weitere 276 mg (1.24 mmol) Natriumtriacetoxyborhydrid hinzugefügt. Nach weiteren 18 h und 36 h Rühren bei RT wurden jeweils nochmals die gleichen Mengen an 2,2-Dimethoxyethanamin und Natriumtriacetoxyborhydrid hinzugegeben. Zuletzt wurde das Reaktionsgemisch 3 h unter Rückfluss erhitzt. Nach dem Abkühlen auf RT wurde mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und das Filtrat zur Trockene eingeengt. Der erhaltene Rückstand wurde mittels präparativer HPLC (Methode 8) in seine Komponenten aufgetrennt. Nach Einengen der Produktfraktionen und Trocknen im Hochvakuum wurden 115 mg (62% d. Th., 95% Reinheit) der Titelverbindung erhalten.
LC/MS (Methode 1, ESIpos): $R_t$ = 0.63 min, m/z = 424 $[M+H]^+$.

**Beispiel 268A**

6-{[(2,2-Dimethoxyethyl)amino]methyl}-3-ethyl-1-(2-methoxyethyl)-5-methylthieno[2,3-d]pyrimidin-2,4(1*H*,3*H*)-dion

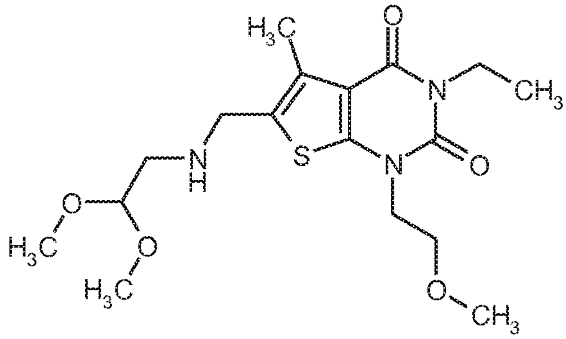

300 mg (0.922 mmol, 91% Reinheit) der Verbindung aus Bsp. 84A wurden in 21 ml Dichlormethan gelöst und mit 145 mg (1.38 mmol) 2,2-Dimethoxyethanamin versetzt. Das Gemisch wurde 1 h zum Rückfluss erwärmt. Nach dem Abkühlen auf RT wurden 586 mg (2.76 mmol) Natriumtriacetoxyborhydrid hinzugefügt. Es wurde bei RT weiter gerührt. Nach 18 h wurde mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und das Filtrat zur Trockene eingeengt. Der erhaltene Rückstand wurde mittels Chromatographie an Kieselgel mit Cyclohexan/Ethylacetat 2:1 und dann Dichlormethan/Methanol 20:1 als Laufmittel gereinigt. Nach Einengen der Produktfraktionen und Trocknen im Hochvakuum wurden 300 mg (78% d. Th., 93% Reinheit) der Titelverbindung erhalten.
$^1$H-NMR (400 MHz, DMSO-$d_6$, δ/ppm): 4.41 (t, 1H), 4.03 (t, 2H), 3.90 (q, 2H), 3.82 (s, 2H), 3.63 (t, 2H), 3.26 (s, 6H), 3.24 (s, 3H), 2.62 (d, 2H), 2.33 (s, 3H), 1.11 (t, 3H).
LC/MS (Methode 1, ESIpos): $R_t$ = 0.53 min, m/z = 281 $[M+H-C_4H_{11}NO_2]^+$.

**Beispiel 269A**

6-{[(1,1-Dimethoxypropan-2-yl)amino]methyl}-3-ethyl-5-methyl-1-(3,3,3-trifluorpropyl)thieno-[2,3-d]pyrimidin-2,4(1*H*,3*H*)-dion *(Racemat)*

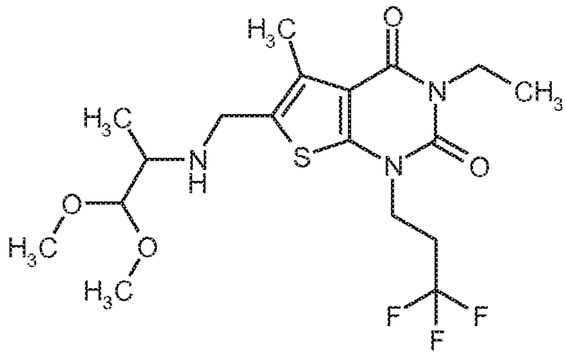

Analog zu dem unter Bsp. 268A beschriebenen Verfahren wurden aus 250 mg (0.680 mmol, 91% Reinheit) der Verbindung aus Bsp. 74A und 162 mg (1.36 mmol) racemischem 2-Aminopropanal-dimethylacetal 214 mg (71% d. Th.) der Titelverbindung hergestellt. Die Chromatographie erfolgte hier an einer Biotage Isolera One-Anlage mit einer Biotage-Kartusche (SNAP KP-Sil, 50 g Kieselgel) und Cyclohexan/Ethylacetat 1:2 als Laufmittel.
$^1$H-NMR (400 MHz, DMSO-$d_6$, δ/ppm): 4.17-4.07 (m, 3H), 3.90 (q, 2H), 3.87 (s, 2H), 3.31 (s, 3H), 3.30 (s, 3H), 2.84-2.67 (m, 3H), 2.34 (s, 3H), 1.11 (t, 3H), 0.97 (d, 3H).
LC/MS (Methode 6, ESIpos): $R_t$ = 1.88 min, m/z = 438 $[M+H]^+$.

**Beispiel 270A**

6-{[(1,1-Dimethoxypropan-2-yl)amino]methyl}-3-ethyl-1-(2-methoxyethyl)-5-methylthieno[2,3-d]-pyrimidin-2,4(1*H*,3*H*)-dion (*Racemat*)

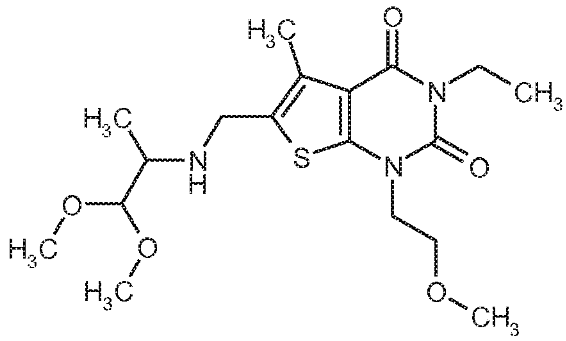

Analog zu dem unter Bsp. 268A beschriebenen Verfahren wurden aus 250 mg (0.844 mmol) der Verbindung aus Bsp. 84A und 201 mg (1.69 mmol) racemischem 2-Aminopropanal-dimethylacetal 154 mg (45% d. Th.) der Titelverbindung hergestellt. Die Chromatographie erfolgte hier an einer Biotage Isolera One-Anlage mit einer Biotage-Kartusche (SNAP KP-Sil, 25 g Kieselgel) und Cyclohexan/Ethylacetat 1:2 als Laufmittel.
$^1$H-NMR (400 MHz, DMSO-$d_6$, δ/ppm): 4.13 (d, 1H), 4.03 (t, 2H), 3.90 (q, 2H), 3.85 (s, 2H), 3.63 (t, 2H), 3.33 (s, 3H), 3.31 (s, 3H), 3.30 (s, 3H), 2.76-2.70 (m, 1H), 2.33 (s, 3H), 1.11 (t, 3H), 0.97 (d, 3H).
LC/MS (Methode 6, ESIpos): $R_t$ = 1.58 min, m/z = 400 $[M+H]^+$.

**Beispiel 271A**

3-Ethyl-5-methyl-6-({[(2-methyl-1,3-dioxolan-2-yl)methyl]amino)methyl)-1-(3,3,3-trifluorpropyl)-thieno[2,3-d]pyrimidin-2,4(1*H*,3*H*)-dion

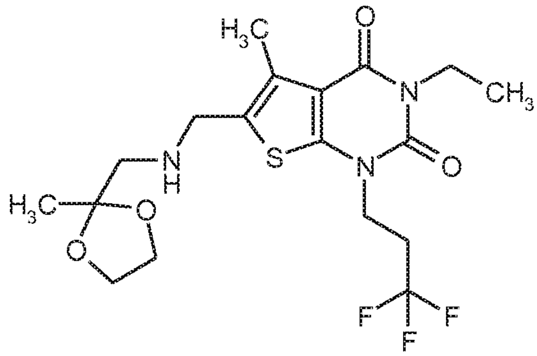

Analog zu dem unter Bsp. 268A beschriebenen Verfahren wurden aus 250 mg (0.748 mmol) der Verbindung aus Bsp. 74A und 175 mg (1.50 mmol) 2-(Aminomethyl)-2-methyl-1,3-dioxolan 344 mg (85% d. Th., 81% Reinheit) der Titelverbindung hergestellt. Auf eine chromatographische Reinigung wurde hier verzichtet.
LC/MS (Methode 1, ESIpos): $R_t$ = 0.61 min, m/z = 436 $[M+H]^+$.

**Beispiel 272A**

3-Ethyl-1-(2-methoxyethyl)-5-methyl-6-({[(2-methyl-1,3-dioxolan-2-yl)methyl]amino}methyl)-thieno[2,3-d]pyrimidin-2,4(1*H*,3*H*)-dion

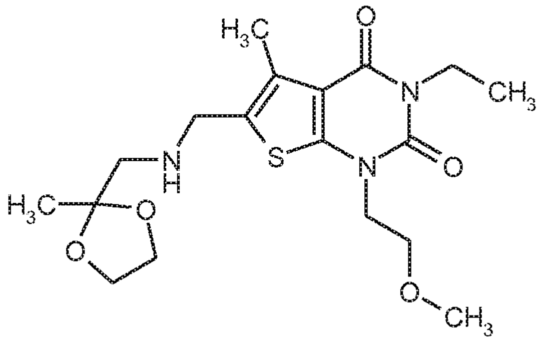

Analog zu dem unter Bsp. 268A beschriebenen Verfahren wurden aus 250 mg (0.844 mmol) der Verbindung aus Bsp. 84A und 197 mg (1.69 mmol) 2-(Aminomethyl)-2-methyl-1,3-dioxolan 340 mg (84% d. Th., 83% Reinheit) der Titelverbindung hergestellt. Auf eine chromatographische Reinigung wurde hier verzichtet.
LC/MS (Methode 1, ESIpos): $R_t$ = 0.51 min, m/z = 398 $[M+H]^+$.

**Beispiel 273A**

1-(2,2-Dimethoxyethyl)-1-{[3-ethyl-5-methyl-2,4-dioxo-1-(3,3,3-trifluorpropyl)-1,2,3,4-tetrahydro-thieno[2,3-d]pyrimidin-6-yl]methyl}harnstoff

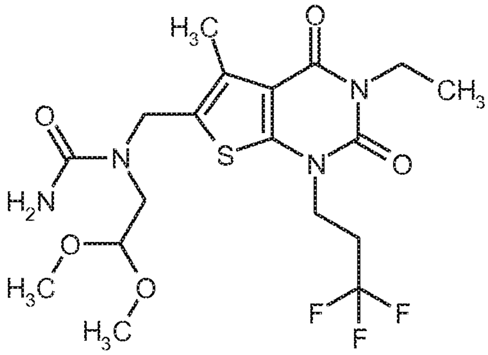

Eine Lösung von 154 mg (0.364 mmol) der Verbindung aus Bsp. 267A in 6 ml Methanol wurde bei RT zunächst mit 68 mg (0.836 mmol) Kaliumcyanat und dann mit 53 µl (0.618 mmol) Perchlorsäure (70% in Wasser) versetzt. Nach 1 h wurde das Reaktionsgemisch mit gesättigter wässriger Natriumhydrogencarbonat-Lösung versetzt und anschließend mit Ethylacetat extrahiert. Der organische Extrakt wurde nacheinander mit Wasser und gesättigter Natriumchlorid-Lösung gewaschen, über wasserfreiem Magnesiumsulfat getrocknet, filtriert und eingeengt. Nach Trocknen des erhaltenen Rückstands im Hochvakuum wurden 103 mg (47% d. Th., 78% Reinheit) der Titelverbindung erhalten, die ohne weitere Aufreinigung für Folgereaktionen verwendet wurden.
LC/MS (Methode 2, ESIpos): $R_t$ = 2.45 min, m/z = 467 $[M+H]^+$.

## Beispiel 274A

1-(2,2-Dimethoxyethyl)-1-{[3-ethyl-1-(2-methoxyethyl)-5-methyl-2,4-dioxo-1,2,3,4-tetrahydro-thieno[2,3-d]pyrimidin-6-yl]methyl}harnstoff

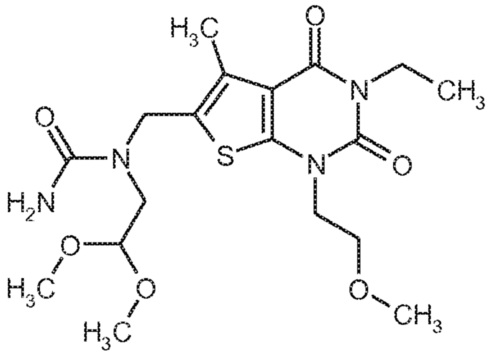

Analog zu dem unter Bsp. 273A beschriebenen Verfahren wurden aus 300 mg (0.724 mmol, 93% Reinheit) der Verbindung aus Bsp. 268A, 135 mg (1.67 mmol) Kaliumcyanat und 106 µl (1.23 mmol) Perchlorsäure (70% in Wasser) 200 mg (54% d. Th., 84% Reinheit) der Titelverbindung hergestellt. Die Reaktionszeit betrug hier 2 h.
$^1$H-NMR (400 MHz, DMSO-$d_6$, δ/ppm): 6.03 (s, 2H), 4.54 (s, 2H), 4.44 (t, 1H), 4.01 (t, 2H), 3.90 (q, 2H), 3.62 (t, 2H), 3.31 (s, 6H, teilweise vom Wassersignal überdeckt), 3.23 (s, 3H), 3.16 (d, 2H), 2.39 (s, 3H), 1.11 (t, 3H).
LC/MS (Methode 1, ESIpos): $R_t$ = 0.76 min, m/z = 429 $[M+H]^+$.

## Beispiel 275A

1-(1,1-Dimethoxypropan-2-yl)-1-{[3-ethyl-5-methyl-2,4-dioxo-1-(3,3,3-trifluorpropyl)-1,2,3,4-tetrahydrothieno[2,3-d]pyrimidin-6-yl]methyllhamstoff *(Racemat)*

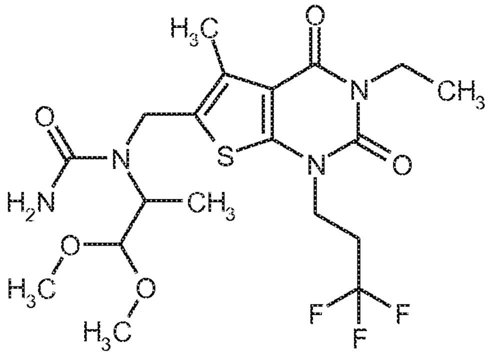

Analog zu dem unter Bsp. 273A beschriebenen Verfahren wurden aus 202 mg (0.462 mmol) der Verbindung aus Bsp. 269A, 86 mg (1.06 mmol) Kaliumcyanat und 68 µl (0.785 mmol) Perchlorsäure (70% in Wasser) 221 mg (64% d. Th., 65% Reinheit) der Titelverbindung hergestellt. Die Reaktionszeit betrug hier 3 h.
LC/MS (Methode 1, ESIpos): $R_t$ = 0.91 min, m/z = 481 $[M+H]^+$.

**Beispiel 276A**

1-(1,1-Dimethoxypropan-2-yl)-1-{[3-ethyl-1-(2-methoxyethyl)-5-methyl-2,4-dioxo-1,2,3,4-tetra-hydrothieno[2,3-d]pyrimidin-6-yl]methyl}harnstoff (*Racemat*)

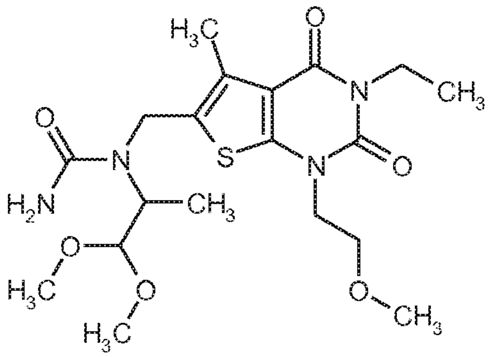

Analog zu dem unter Bsp. 273A beschriebenen Verfahren wurden aus 145 mg (0.363 mmol) der Verbindung aus Bsp. 270A, 68 mg (0.835 mmol) Kaliumcyanat und 53 µl (0.617 mmol) Perchlorsäure (70% in Wasser) 160 mg (59% d. Th., 60% Reinheit) der Titelverbindung hergestellt. Die Reaktionszeit betrug hier 3 h.
LC/MS (Methode 1, ESIpos): $R_t$ = 0.77 min, m/z = 443 $[M+H]^+$.

**Beispiel 277A**

1-{[3-Ethyl-5-methyl-2,4-dioxo-1-(3,3,3-trifluorpropyl)-1,2,3,4-tetrahydrothieno[2,3-d]pyrimidin-6-yl]methyl}-1-[(2-methyl-1,3-dioxolan-2-yl)methyl]harnstoff

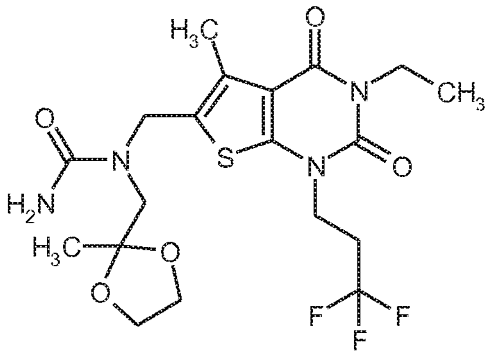

Analog zu dem unter Bsp. 273A beschriebenen Verfahren wurden aus 344 mg (0.640 mmol, 81% Reinheit) der Verbindung aus Bsp. 271A, 119 mg (1.42 mmol) Kaliumcyanat und 94 µl (1.09 mmol) Perchlorsäure (70% in Wasser) 124 mg (34% d. Th., 85% Reinheit) der Titelverbindung hergestellt. Die Reaktionszeit betrug hier 2 h, und das Produkt wurde mittels präparativer HPLC (Methode 8) aus dem Reaktionsgemisch isoliert.
$^1$H-NMR (400 MHz, DMSO-$d_6$, δ/ppm): 6.01 (s, 2H), 4.60 (s, 2H), 4.08 (t, 2H), 3.96-3.81 (m, 6H), 3.21 (s, 2H), 2.83-2.66 (m, 2H), 2.41 (s, 3H), 1.24 (s, 3H), 1.11 (t, 3H).
LC/MS (Methode 1, ESIpos): $R_t$ = 0.90 min, m/z = 479 [M+H]$^+$.

**Beispiel 278A**

1-{[3-Ethyl-1-(2-methoxyethyl)-5-methyl-2,4-dioxo-1,2,3,4-tetrahydrothieno[2,3-d]pyrimidin-6-yl]methyl}-1-[(2-methyl-1,3-dioxolan-2-yl)methyl]hamstoff

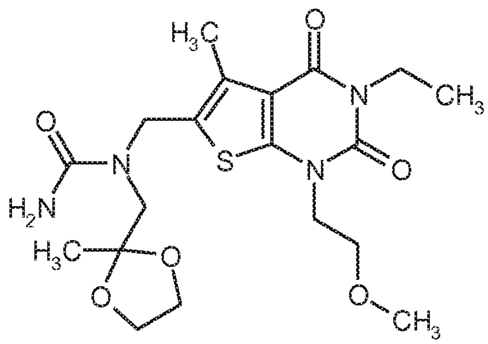

Analog zu dem unter Bsp. 273A beschriebenen Verfahren wurden aus 339 mg (0.708 mmol, 83% Reinheit) der Verbindung aus Bsp. 272A, 132 mg (1.63 mmol) Kaliumcyanat und 104 µl (1.09 mmol) Perchlorsäure (70% in Wasser) 182 mg (58% d. Th.) der Titelverbindung hergestellt. Die Reaktionszeit betrug hier 2 h, und das Produkt wurde mittels präparativer HPLC (Methode 8) aus dem Reaktionsgemisch isoliert.
$^1$H-NMR (400 MHz, DMSO-$d_6$, δ/ppm): 5.99 (s, 2H), 4.58 (s, 2H), 4.00 (t, 2H), 3.95-3.82 (m, 6H), 3.62 (t, 2H), 3.23 (s, 3H), 3.20 (s, 2H), 2.39 (s, 3H), 1.24 (s, 3H), 1.11 (t, 3H).
LC/MS (Methode 1, ESIpos): $R_t$ = 0.80 min, m/z = 441 [M+H]$^+$.

**Beispiel 279A**

*N*-([3-Ethyl-5-methyl-2,4-dioxo-1-(3,3,3-trifluorpropyl)-1,2,3,4-tetrahydrothieno[2,3-d]pyrimidin-6-yl]methyl}-2-formylhydrazincarboxamid

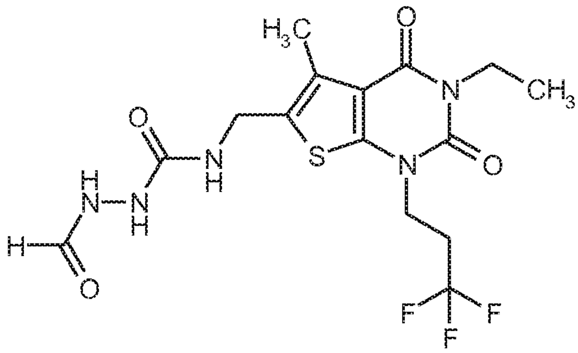

*Darstellung des Säurechlorids:* Eine Lösung von 250 mg (0.686 mmol) der Verbindung aus Bsp. 189A in 12 ml Dichlormethan wurde bei RT zunächst mit 78 µl (0.892 mmol) Oxalylchlorid und dann mit einem kleinen Tropfen DMF versetzt. Nachdem das Reaktionsgemisch 2 h bei RT gerührt worden war, wurde es am Rotationsverdampfer zur Trockene eingeengt. Der verbliebene Rückstand wurde im Hochvakuum getrocknet und anschließend im nächsten Teilschritt weiter umgesetzt.

*Darstellung des Säureazids:* Das zuvor erhaltene Säurechlorid wurde in 12 ml Toluol gelöst und mit 67 mg (1.03 mmol) Natriumazid versetzt. Das Reaktionsgemisch wurde ca. 18 h bei RT gerührt. Dabei fiel ein feiner Niederschlag aus, von dem abfiltriert wurde. Das Filtrat wurde als solches im nächsten Teilschritt weiter umgesetzt.

*Darstellung des Isocyanats:* Das zuvor erhaltene Filtrat (Lösung des Säureazids) wurde 1.5 h unter Rückfluss erhitzt. Nach dem Abkühlen auf RT wurde die Lösung des so gewonnenen Isocyanats als solche im nächsten Teilschritt weiter umgesetzt.

*Darstellung des Harnstoffs:* Die zuvor erhaltene Lösung des Isocyanats wurde mit einer Lösung von 41 mg (0.686 mmol) Formylhydrazin in 3 ml THF und 105 µl (0.755 mmol) Triethylamin versetzt. Nachdem das Reaktionsgemisch ca. 18 h bei RT gerührt worden war, wurde es mit 20 ml gesättigter wässriger Ammoniumchlorid-Lösung versetzt. Es wurde viermal mit je ca. 20 ml Ethylacetat extrahiert. Der vereinigte organische Extrakt wurde mit gesättigter Natriumchlorid-Lösung gewaschen, über wasserfreiem Magnesiumsulfat getrocknet, filtriert und eingeengt. Der verbliebene Rückstand wurde mittels präparativer HPLC (Methode 8) in seine Komponenten aufgetrennt. Die Produktfraktionen wurden vereinigt, eingeengt und im Hochvakuum getrocknet. Es wurden so 83 mg (28% d. Th., 98% Reinheit) der Titelverbindung erhalten.

$^1$H-NMR (Hauptrotamer; 500 MHz, DMSO-$d_6$, δ/ppm): 9.61 (s, 1H), 8.05 (s, 1H), 7.97 (s, 1H), 7.10 (br. s, 1H), 4.35-4.25 (m, 2H), 4.09 (t, 2H), 3.90 (q, 2H), 2.84-2.68 (m, 2H), 2.39 (s, 3H), 1.11 (t, 3H).

LC/MS (Methode 1, ESIpos): $R_t$ = 0.72 min, m/z = 422 $[M+H]^+$.

## Beispiel 280A

Ethyl-2-{[(2,2-dimethylpropyl)carbamoyl]amino}-4-methylthiophen-3-carboxylat

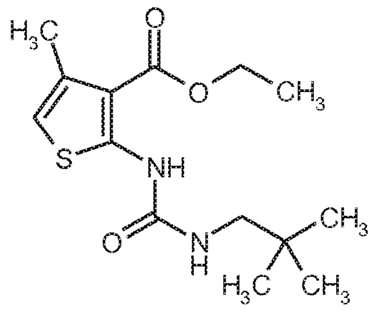

Eine Lösung von 2 g (10.5 mmol) Ethyl-2-amino-4-methylthiophen-3-carboxylat in 10 ml Pyridin wurde mit 2.42 g (20.9 mmol) 2,2-Dimethylpropylisocyanat versetzt. Das Reaktionsgemisch wurde 21 h bei 50°C gerührt. Danach wurden nochmals 1.18 g (10.5 mmol) 2,2-Dimethylpropylisocyanat zugesetzt und weitere 23 h bei 50°C gerührt. Anschließend wurde das Reaktionsgemisch am Rotationsverdampfer zur Trockene eingeengt. Der verbliebene Rückstand wurde in Dichlormethan gelöst und erneut zur Trockene eingeengt. Das so gewonnene Material wurde über eine Kieselgel-Kartusche chromatographiert (Biotage, 340 g Kieselgel, Laufmittel Hexan/Ethylacetat). Es wurden 3.07 g (98% d. Th.) der Titelverbindung erhalten.
$^{1}$H-NMR (400 MHz, DMSO-$d_6$, δ/ppm): 10.37 (s, 1H), 7.84 (t, 1H), 6.41 (s, 1H), 4.28 (q, 2H), 2.92 (d, 2H), 2.26 (d, 3H), 1.31 (t, 3H), 0.87 (s, 9H).
LC/MS (Methode 3, ESIpos): $R_t$ = 1.39 min, m/z = 299 $[M+H]^+$.

**Beispiel 281A**

Ethyl-2- {[(2-fluor-2-methylpropyl)carbamoyl]amino}-4-methylthiophen-3-carboxylat

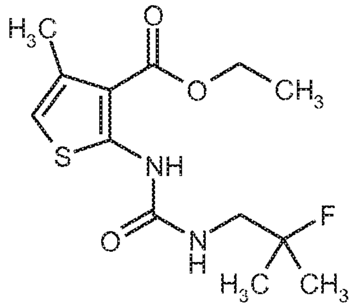

Eine Lösung von 5.49 g (28.7 mmol) Ethyl-2-amino-4-methylthiophen-3-carboxylat in 80 ml THF wurde mit 9.32 g (57.5 mmol) 1,1'-Carbonyldiimidazol (CDI) und 16 ml (115 mmol) Triethylamin versetzt und 4 Tage bei RT gerührt. Dann wurde zu dem Gemisch eine Lösung von 5.50 g (43.1 mmol) 2-Fluor-2-methylpropanamin-Hydrochlorid [WO 2006/029115-A2, Example 43, Part A/B] und 8 ml (57 mmol) Triethylamin in 70 ml THF gegeben. Da nach 3 h bei RT der Umsatz nicht vollständig war, wurde 1 h bei 50°C nachgerührt. Dann wurde das Reaktionsgemisch 2 Tage bei RT und anschließend weitere 4 h bei 50°C gerührt. Da der Umsatz noch nicht vollständig war, wurden 50 ml Pyridin zugesetzt und das Rühren bei 50°C 15 h lang fortgesetzt. Nach dem Abkühlen auf RT wurde der dabei ausgefallene Feststoff abgesaugt und verworfen. Das Filtrat wurde in Wasser eingerührt. Der dabei ausgefallene Niederschlag wurde erneut abfiltriert und ebenfalls verworfen. Aus dem Filtrat wurde der Großteil des THFs am Rotationsverdampfer entfernt. Die verbliebene wässrige Phase wurde mit Ethylacetat extrahiert. Der organische Extrakt wurde nacheinander mit Wasser und gesättigter Natriumchlorid-Lösung gewaschen. Nach dem Eindampfen wurde der verbliebene Rückstand mittels Flash-Chromatographie gereinigt (Combiflash, 100 g Kieselgel, Laufmittel Cyclohexan/Ethylacetat). Nach Vereinigen und Einengen der Produktfraktionen wurden so 3.80 g (43% d. Th.) der Titelverbindung erhalten.
$^{1}$H-NMR (400 MHz, DMSO-$d_6$, δ/ppm): 10.38 (s, 1H), 8.13 (t, 1H), 6.43 (s, 1H), 4.38-4.20 (m, 2H), 3.39-3.21 (m, 2H), 2.27 (s, 3H), 1.37-1.23 (m, 9H).
LC/MS (Methode 17, ESIpos): $R_t$ = 1.97min, m/z = 303.12 $[M+H]^+$.

**Beispiel 282A**

Ethyl-2-{[(2-methoxy-2-methylpropyl)carbamoyl]amino}-4-methylthiophen-3-carboxylat

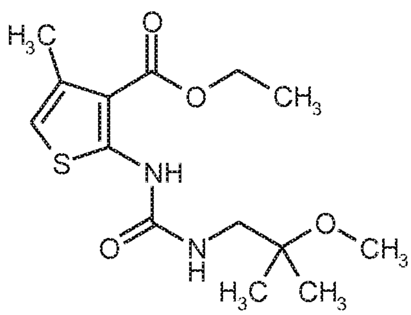

Eine Lösung von 1.11 g (5.8 mmol) Ethyl-2-amino-4-methylthiophen-3-carboxylat in 6 ml Pyridin wurde mit 1.5

g (11.6 mmol) 1-Isocyanato-2-methoxy-2-methylpropan versetzt. Das Reaktionsgemisch wurde 72 h bei 50°C gerührt. Anschließend wurde das Gemisch am Rotationsverdampfer zur Trockene eingeengt. Der verbliebene Rückstand wurde in Dichlormethan gelöst und erneut zur Trockene eingeengt. Das so gewonnene Material wurde über eine Kieselgel-Kartusche chromatographiert (Biotage, 340 g Kieselgel, Laufmittel Hexan/Ethylacetat). Es wurden 2.03 g der Titelverbindung erhalten.
$^1$H-NMR (400 MHz, DMSO-$d_6$, δ/ppm): 10.37 (s, 1H), 7.90 (br. s, 1H), 6.41 (s, 1H), 4.28 (q, 2H), 3.15 (d, 2H), 3.11 (s, 3H), 2.26 (d, 3H), 1.31 (t, 3H), 1.08 (s, 6H).
LC/MS (Methode 3, ESIpos): $R_t$ = 1.23 min, m/z = 314 $[M+H]^+$.

## Beispiel 283A

3-(2,2-Dimethylpropyl)-5-methylthieno[2,3-d]pyrimidin-2,4(1*H*,3*H*)-dion

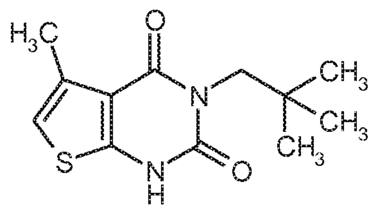

1.51 g (5.07 mmol) der Verbindung aus Bsp. 280A wurden in 14 ml Ethanol gelöst und mit 3.8 ml Natriumethoxid-Lösung (21 Gew.-% in Ethanol) versetzt. Es wurde 27 h bei 40°C gerührt und dann 11.7 ml 1 M Salzsäure zugesetzt. Das Ethanol wurde aus der entstandenen Suspension am Rotationsverdampfer weitestgehend entfernt. Der verbliebene Rückstand wurde mit Wasser versetzt, und der Feststoff wurde abfiltriert, mit Wasser neutral gewaschen und trocken gesaugt. Es wurden 1.28 g (98% d. Th.) der Titelverbindung erhalten.
$^1$H-NMR (400 MHz, DMSO-$d_6$, δ/ppm): 12.05 (br. s, 1H), 6.68 (d, 1H), 3.76 (s, 2H), 2.34 (d, 3H), 0.89 (s, 9H).
LC/MS (Methode 3): $R_t$ = 1.13 min, m/z = 253 $[M+H]^+$.

## Beispiel 284A

3-(2-Fluor-2-methylpropyl)-5-methylthieno[2,3-d]pyrimidin-2,4(1*H*,3*H*)-dion

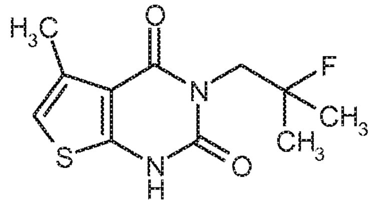

3.70 g (12.2 mmol) der Verbindung aus Bsp. 281A wurden in 40 ml Ethanol gelöst und mit 9.1 ml (24.5 mmol) einer 21%-igen Lösung von Natriummethylat in Ethanol versetzt. Nachdem das Gemisch 15 h bei RT gerührt worden war, wurde es mit 28.1 ml (28.1 mmol) 1 M Salzsäure versetzt, wobei das Produkt ausfiel. Der Feststoff wurde abgesaugt, mit Wasser neutral gewaschen und im Hochvakuum getrocknet. Es wurden 2.60 g (82% d. Th.) der Titelverbindung erhalten.
$^1$H-NMR (400 MHz, DMSO-$d_6$, δ/ppm): 12.13 (s, 1H), 6.70 (d, 1H), 4.10 (d, 2H), 2.35 (d, 3H), 1.41-1.23 (m, 6H).
LC/MS (Methode 6, ESIneg): $R_t$ = 1.38 min, m/z = 255 $[M-H]^-$.

## Beispiel 285A

3-(2-Methoxy-2-methylpropyl)-5-methylthieno[2,3-d]pyrimidin-2,4(1*H*,3*H*)-dion

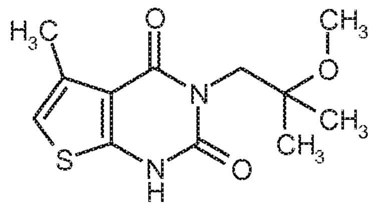

2.03 g (6.45 mmol) der Verbindung aus Bsp. 282A wurden in 18 ml Ethanol gelöst und mit 4.8 ml Natriumethoxid-Lösung (21 Gew.-% in Ethanol) versetzt. Es wurde 88 h bei RT gerührt und dann 15 ml 1 M Salzsäure zugesetzt. Das Ethanol wurde aus der entstandenen Suspension am Rotationsverdampfer weitestgehend entfernt. Der verbliebene Rückstand wurde mit Wasser versetzt, und der Feststoff wurde abfiltriert, mit Wasser neutral gewaschen und trocken gesaugt. Es wurden 1.7 g (98% d. Th.) der Titelverbindung erhalten.
$^1$H-NMR (400 MHz, DMSO-$d_6$, $\delta$/ppm): 12.07 (br. s, 1H), 6.68 (d, 1H), 3.95 (s, 2H), 3.15 (s, 3H), 2.34 (d, 3H), 1.08 (s, 6H).
LC/MS (Methode 3, ESIpos): $R_t$ = 0.92 min, m/z = 267 [M+H]$^+$.

**Beispiel 286A**

3-(2,2-Dimethylpropyl)-5-methyl-2,4-dioxo-1,2,3,4-tetrahydrothieno[2,3-d]pyrimidin-6-carbaldehyd

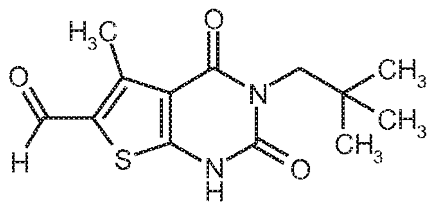

Eine Lösung von 1.26 g (5.02 mmol) der Verbindung aus Bsp. 283A in 47 ml DMF wurde unter Eisbadkühlung vorsichtig mit 4.7 ml (50.2 mmol) Phosphoroxychlorid versetzt. Das Gemisch wurde danach 4 h bei 70°C gerührt. Dann wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 1.2 g (85% d. Th.) der Titelverbindung erhalten.
$^1$H-NMR (400 MHz, DMSO-$d_6$, $\delta$/ppm): 12.55 (br. s, 1H), 10.06 (s, 1H), 3.75 (s, 2H), 2.75 (s, 3H), 0.90 (s, 9H).
LC/MS (Methode 3, ESIpos): $R_t$ = 1.10 min, m/z = 281 [M+H]$^+$.

**Beispiel 287A**

3-(2-Fluor-2-methylpropyl)-5-methyl-2,4-dioxo-1,2,3,4-tetrahydrothieno[2,3-d]pyrimidin-6-carbaldehyd

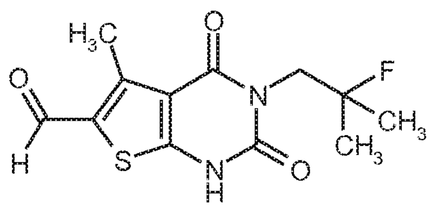

Eine Lösung von 2.55 g (9.95 mmol) der Verbindung aus Bsp. 284A in 7.7 ml (99.5 mmol) DMF wurde vorsichtig mit 11.1 ml (119 mmol) Phosphoroxychlorid versetzt. Nachdem die stark exotherme Reaktion abgeklungen war, wurde das Gemisch noch 15 min nachgerührt. Danach wurde das Reaktionsgemisch vorsichtig in 350 ml Wasser eingerührt. Nach 1 h Rühren wurde das ausgefallene Produkt abgesaugt, mit Wasser neutral gewaschen und getrocknet. Es wurden 2.63 g (90% d. Th., 97% Reinheit) der Titelverbindung erhalten.
$^1$H-NMR (400 MHz, DMSO-$d_6$, $\delta$/ppm): 12.62 (s, 1H), 10.07 (s, 1H), 4.09 (d, 2H), 2.75 (s, 3H), 1.40-1.23 (m, 6H).

LC/MS (Methode 17, ESIpos): $R_t$ = 1.84 min, m/z = 285.07 $[M+H]^+$, 265.06 $[M-F]^+$.

**Beispiel 288A**

3-(2-Methoxy-2-methylpropyl)-5-methyl-2,4-dioxo-1,2,3,4-tetrahydrothieno[2,3-d]pyrimidin-6-carbaldehyd

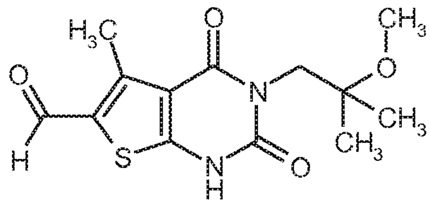

Eine Lösung von 1.18 g (4.39 mmol) der Verbindung aus Bsp. 285A in 41 ml DMF wurde unter Eisbadkühlung vorsichtig mit 4.1 ml (44.0 mmol) Phosphoroxychlorid versetzt. Das Gemisch wurde danach 2 h bei 70°C gerührt. Dann wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 1.06 g (77% d. Th.) der Titelverbindung erhalten.
$^1$H-NMR (400 MHz, DMSO-$d_6$, $\delta$/ppm): 12.56 (br. s, 1H), 10.06 (s, 1H), 3.94 (s, 2H), 3.15 (s, 3H), 2.75 (s, 3H), 1.09 (s, 6H).
LC/MS (Methode 3, ESIpos): $R_t$ = 0.89 min, m/z = 295 $[M+H]^+$.

**Beispiel 289A**

Ethyl-(6-formyl-5-methyl-2,4-dioxo-1,4-dihydrothieno[2,3-d]pyrimidin-3(2*H*)-yl)acetat

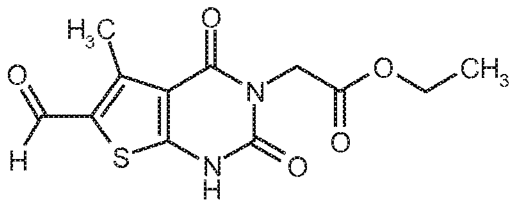

Eine Lösung von 4.50 g (16.8 mmol) Ethyl-(5-methyl-2,4-dioxo-1,4-dihydrothieno[2,3-d]pyrimidin-3(2*H*)-yl)acetat [US Pat. 6 140 325, Reference Example 2 (2)] in 12.9 ml (168 mmol) DMF wurde vorsichtig mit 18.8 ml (201 mmol) Phosphoroxychlorid versetzt. Nachdem die stark exotherme Reaktion abgeklungen war, wurde das Gemisch noch 15 min nachgerührt. Danach wurde das Reaktionsgemisch vorsichtig in 100 ml Wasser eingerührt. Nach 1 h Rühren wurde das ausgefallene Produkt abgesaugt, mit Wasser neutral gewaschen und im Hochvakuum getrocknet. Es wurden 4.88 g (98% d. Th.) der Titelverbindung erhalten.
$^1$H-NMR (400 MHz, DMSO-$d_6$, $\delta$/ppm): 12.86 (br. s, 1H), 10.08 (s, 1H), 4.58 (s, 2H), 4.15 (q, 2H), 2.75 (s, 3H), 1.21 (t, 3H).
LC/MS (Methode 1, ESIpos): $R_t$ = 0.68 min, m/z = 297 $[M+H]^+$.

**Beispiel 290A**

1-[2-(Cyclopropyloxy)ethyl]-3-ethyl-5-methyl-2,4-dioxo-1,2,3,4-tetrahydrothieno[2,3-d]pyrimidin-6-carbaldehyd

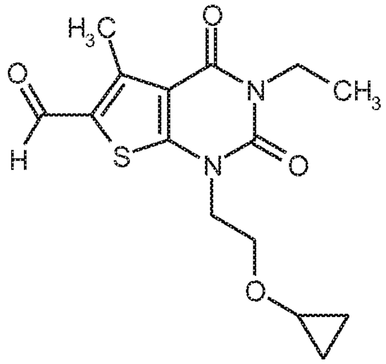

Eine Lösung von 560 mg (2.28 mmol) der Verbindung aus Bsp. 48A in 22 ml DMF wurde mit 948 mg (6.85 mmol) Kaliumcarbonat versetzt und 15 min bei RT gerührt. Dann wurden 827 mg (6.86 mmol) (2-Chlorethoxy)cyclopropan sowie 85 mg (0.57 mmol) Natriumiodid zugesetzt und das Gemisch 115 h bei 90°C gerührt. Das DMF wurde danach weitestgehend abdestilliert und der verbliebene Rückstand zwischen halbgesättigter Natriumchlorid-Lösung (40 ml) und Ethylacetat (25 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 390 mg (53% d. Th.) der Titelverbindung erhalten.

$^1$H-NMR (400 MHz, DMSO-$d_6$, $\delta$/ppm): 10.09 (s, 1H), 4.08 (t, 2H), 3.90 (q, 2H), 3.75 (t, 2H), 2.78 (s, 3H), 1.13 (t, 3H), 0.42-0.36 (m, 4H).

LC/MS (Methode 3, ESIpos): $R_t$ = 1.14 min, m/z = 323 $[M+H]^+$.

**Beispiel 291A**

3-(2,2-Dimethylpropyl)-5-methyl-2,4-dioxo-1-(3,3,3-trifluorpropyl)-1,2,3,4-tetrahydrothieno-[2,3-d]pyrimidin-6-carbaldehyd

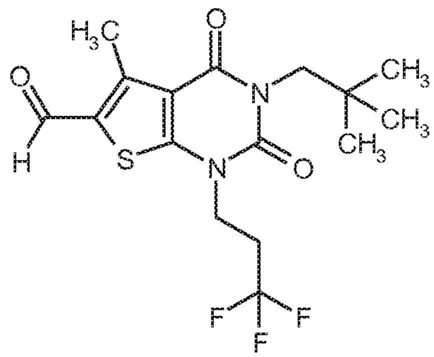

Eine Lösung von 450 mg (1.6 mmol) der Verbindung aus Bsp. 286A in 17 ml DMF wurde mit 554 mg (4.01 mmol) Kaliumcarbonat versetzt und 15 min bei RT gerührt. Dann wurden 1.11 g (4.81 mmol) 1,1,1-Trifluor-3-iodpropan zugesetzt und das Gemisch 21 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 491 mg (81% d. Th.) der Titelverbindung erhalten.

$^1$H-NMR (400 MHz, DMSO-$d_6$, $\delta$/ppm): 10.11 (s, 1H), 4.17 (t, 2H), 3.81 (br. s, 2H), 2.86-2.71 (m, 5H), 0.90 (s, 9H).

LC/MS (Methode 3, ESIpos): $R_t$ = 1.40 min, m/z = 377 $[M+H]^+$.

**Beispiel 292A**

3-(2,2-Dimethylpropyl)-1-(3-fluorpropyl)-5-methyl-2,4-dioxo-1,2,3,4-tetrahydrothieno[2,3-d]-pyrimidin-6-carbaldehyd

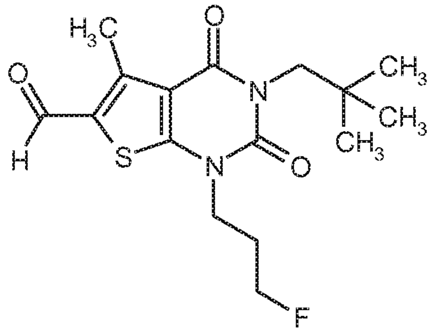

Eine Lösung von 450 mg (1.6 mmol) der Verbindung aus Bsp. 286A in 16 ml DMF wurde mit 554 mg (4.01 mmol) Kaliumcarbonat versetzt und 15 min bei RT gerührt. Dann wurden 905 mg (4.81 mmol) 1-Fluor-3-iodpropan zugesetzt und das Gemisch 21 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 423 mg (77% d. Th.) der Titelverbindung erhalten.
$^1$H-NMR (400 MHz, DMSO-$d_6$, $\delta$/ppm): 10.10 (s, 1H), 4.60 (t, 1H), 4.48 (t, 1H), 4.05 (t, 2H), 3.81 (br. s, 2H), 2.78 (s, 3H), 2.11 (quin, 1H), 2.04 (t, 1H), 0.90 (s, 9H).
LC/MS (Methode 3, ESIpos): $R_t$ = 1.32 min, m/z = 341 $[M+H]^+$.

**Beispiel 293A**

3-(2,2-Dimethylpropyl)-1-(2-methoxyethyl)-5-methyl-2,4-dioxo-1,2,3,4-tetrahydrothieno[2,3-d]-pyrimidin-6-carbaldehyd

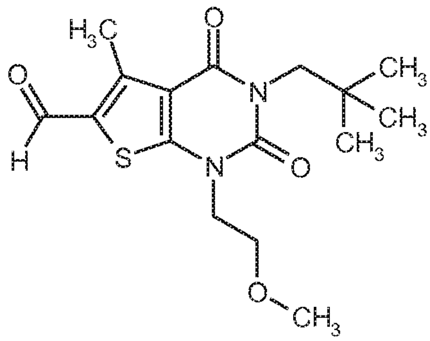

Eine Lösung von 450 mg (1.6 mmol) der Verbindung aus Bsp. 286A in 15 ml DMF wurde mit 555 mg (4.01 mmol) Kaliumcarbonat versetzt und 15 min bei RT gerührt. Dann wurden 669 mg (4.81 mmol) 2-Bromethyl-methylether zugesetzt und das Gemisch 16 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 417 mg (76% d. Th.) der Titelverbindung erhalten.
$^1$H-NMR (400 MHz, DMSO-$d_6$, $\delta$/ppm): 10.09 (s, 1H), 4.10 (t, 2H), 3.81 (br. s, 2H), 3.64 (t, 2H), 3.33 (s, 3H), 2.77 (s, 3H), 0.89 (s, 9H).
LC/MS (Methode 3, ESIpos): $R_t$ = 1.29 min, m/z = 339 $[M+H]^+$.

**Beispiel 294A**

3-(2-Fluor-2-methylpropyl)-5-methyl-2,4-dioxo-1-(3,3,3-trifluorpropyl)-1,2,3,4-tetrahydrothieno-[2,3-d]pyrimidin-6-carbaldehyd

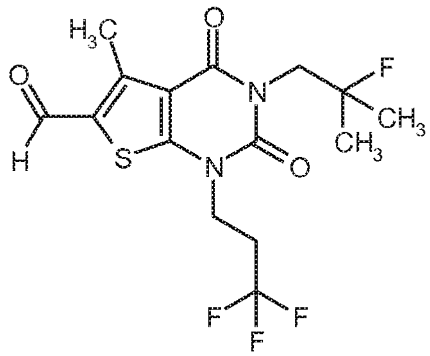

1.30 g (4.57 mmol) der Verbindung aus Bsp. 287A wurden in 22 ml DMF gelöst und mit 1.26 g (9.15 mmol) Kaliumcarbonat versetzt. Das Gemisch wurde auf 60°C erwärmt und bei dieser Temperatur mit 1.02 g (4.57 mmol) 1,1,1-Trifluor-3-iodpropan versetzt. Nach 2 h bei 60°C wurde noch einmal die gleiche Menge an 1,1,1-Trifluor-3-iodpropan hinzugefügt. Nach weiteren 3.5 h bei 60°C wurde das Reaktionsgemisch auf RT abgekühlt und anschließend auf ca. 100 ml Eiswasser gegossen. Dabei fiel das Produkt aus. Es wurde abgesaugt, mit Wasser gewaschen und im Hochvakuum getrocknet. Es wurden 1.63 g (88% d. Th., 94% Reinheit) der Titelverbindung erhalten.
$^1$H-NMR (400 MHz, DMSO-$d_6$, $\delta$/ppm): 10.11 (s, 1H), 4.24-4.08 (m, 4H), 2.90-2.71 (m, 2H), 2.79 (s, 3H), 1.42-1.23 (m, 6H).
LC/MS (Methode 17, ESIpos): $R_t$ = 1.88 min, m/z = 381.09 [M+H]$^+$.

**Beispiel 295A**

3-(2-Fluor-2-methylpropyl)-1-(2-methoxyethyl)-5-methyl-2,4-dioxo-1,2,3,4-tetrahydrothieno-[2,3-d]pyrimidin-6-carbaldehyd

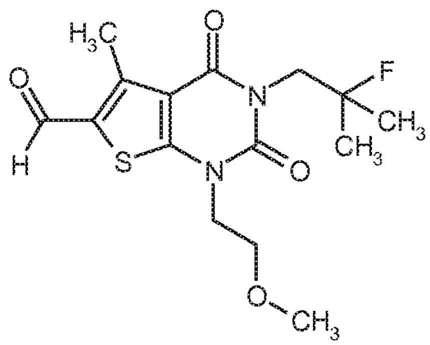

Analog zu dem unter Bsp. 294A beschriebenen Verfahren wurden aus 1.30 g (4.57 mmol) der Verbindung aus Bsp. 287A und insgesamt 1.27 g (9.14 mmol) 2-Bromethyl-methylether 1.36 g (86% d. Th.) der Titelverbindung erhalten.
$^1$H-NMR (400 MHz, DMSO-$d_6$, $\delta$/ppm): 10.09 (s, 1H), 4.21-4.06 (m, 4H), 3.65 (t, 2H), 3.24 (s, 3H), 2.78 (s, 3H), 1.40-1.26 (m, 6H).
LC/MS (Methode 17, ESIpos): $R_t$ = 1.63 min, m/z = 343.11 [M+H]$^+$.

**Beispiel 296A**

3-(2-Methoxyethyl)-5-methyl-2,4-dioxo-1-(3,3,3-trifluorpropyl)-1,2,3,4-tetrahydrothieno[2,3-d]-pyrimidin-6-carbaldehyd

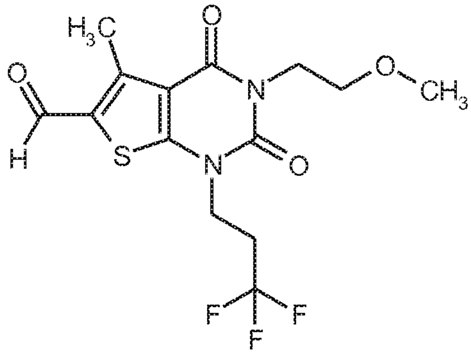

Eine Lösung von 285 mg (0.98 mmol) der Verbindung aus Bsp. 53A in 9 ml DMF wurde mit 341 mg (2.47 mmol) Kaliumcarbonat versetzt und 15 min bei RT gerührt. Dann wurden 664 mg (2.96 mmol) 1,1,1-Trifluor-3-iodpropan zugesetzt und das Gemisch 19 h bei 50°C gerührt. Das DMF wurde danach weitestgehend abdestilliert und der verbliebene Rückstand zwischen halbgesättigter Natriumchlorid-Lösung (200 ml) und Ethylacetat (100 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 254 mg (69% d. Th.) der Titelverbindung erhalten.
$^1$H-NMR (400 MHz, DMSO-$d_6$, δ/ppm): 10.11 (s, 1H), 4.18 (t, 2H), 4.06 (t, 2H), 3.53-3.48 (m, 2H), 3.24 (s, 3H), 2.84-2.75 (m, 5H).
LC/MS (Methode 3, ESIpos): $R_t$ = 1.10 min, m/z = 365 [M+H]$^+$.

**Beispiel 297A**

1-(3-Fluorpropyl)-3-(2-methoxyethyl)-5-methyl-2,4-dioxo-1,2,3,4-tetrahydrothieno[2,3-d]pyrimidin-6-carbaldehyd

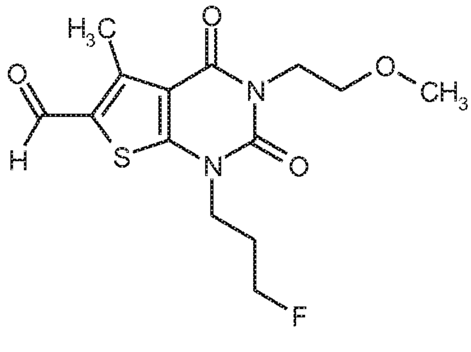

Eine Lösung von 285 mg (0.98 mmol) der Verbindung aus Bsp. 53A in 9 ml DMF wurde mit 341 mg (2.47 mmol) Kaliumcarbonat versetzt und 15 min bei RT gerührt. Dann wurden 557 mg (2.96 mmol) 1-Fluor-3-iodpropan zugesetzt und das Gemisch 19 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 175 mg (52% d. Th.) der Titelverbindung erhalten.
$^1$H-NMR (400 MHz, DMSO-$d_6$, δ/ppm): 10.10 (s, 1H), 4.60 (t, 1H), 4.48 (t, 1H), 4.09-4.01 (m, 4H), 3.53-3.47 (m, 2H), 3.24 (s, 3H), 2.79 (s, 3H), 2.15-2.08 (m, 1H), 2.08-2.00 (m, 1H).
LC/MS (Methode 3, ESIpos): $R_t$ = 0.98 min, m/z = 329 [M+H]$^+$.

**Beispiel 298A**

3-(2-Methoxyethyl)-5-methyl-2,4-dioxo-1-(tetrahydrofuran-2-ylmethy1)-1,2,3,4-tetrahydrothieno-[2,3-d]pyrimidin-6-carbaldehyd (*Racemat*)

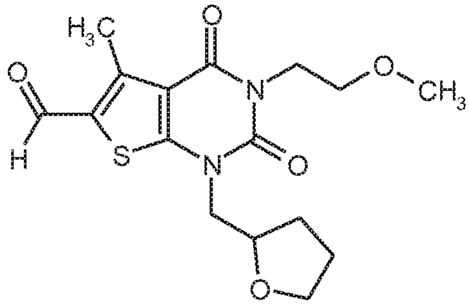

Eine Lösung von 900 mg (3.12 mmol) der Verbindung aus Bsp. 53A in 30 ml DMF wurde mit 1.29 g (9.35 mmol) Kaliumcarbonat versetzt und 15 min bei RT gerührt. Dann wurden 2.86 g (15.6 mmol) racemisches 2-(Brommethyl)tetrahydrofuran zugesetzt und das Gemisch 94 h bei 70°C gerührt. Das DMF wurde danach weitestgehend abdestilliert und der verbliebene Rückstand zwischen halbgesättigter Natriumchlorid-Lösung (200 ml) und Ethylacetat (100 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 340 g Kieselgel, Laufmittel Hexan/Ethylacetat). Es wurden 440 mg (39% d. Th.) der Titelverbindung erhalten.
$^1$H-NMR (400 MHz, DMSO-$d_6$, $\delta$/ppm): 10.09 (s, 1H), 4.23 (dtd, 1H), 4.13 (dd, 1H), 4.06 (t, 2H), 3.81-3.70 (m, 2H), 3.62 (td, 1H), 3.54-3.48 (m, 2H), 3.24 (s, 3H), 2.78 (s, 3H), 2.06-1.95 (m, 1H), 1.95-1.75 (m, 2H), 1.73-1.62 (m, 1H).
LC/MS (Methode 3, ESIpos): $R_t$ = 1.0 min, m/z = 353 $[M+H]^+$.

**Beispiel 299A**

3-(2-Methoxy-2-methylpropyl)-5-methyl-2,4-dioxo-1-(3,3,3-trifluorpropyl)-1,2,3,4-tetrahydrothieno[2,3-d]pyrimidin-6-carbaldehyd

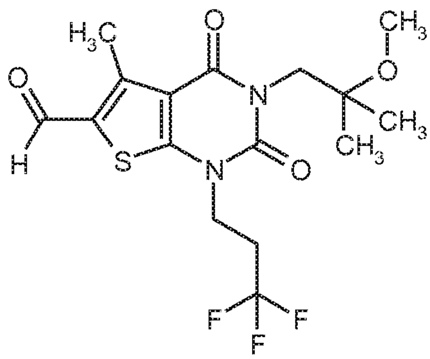

Eine Lösung von 450 mg (1.44 mmol) der Verbindung aus Bsp. 288A in 15 ml DMF wurde mit 498 mg (3.6 mmol) Kaliumcarbonat versetzt und 15 min bei RT gerührt. Dann wurden 969 mg (4.32 mmol) 1,1,1-Trifluor-3-iodpropan zugesetzt und das Gemisch 18 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 463 mg (79% d. Th.) der Titelverbindung erhalten.
$^1$H-NMR (400 MHz, DMSO-$d_6$, $\delta$/ppm): 10.11 (s, 1H), 4.17 (t, 2H), 4.00 (br. s, 2H), 3.15 (s, 3H), 2.86-2.71 (m, 5H), 1.09

(s, 6H).
LC/MS (Methode 3, ESIpos): $R_t$ = 1.22 min, m/z = 361 $[M+H-CH_3OH]^+$.

**Beispiel 300A**

1-(3-Fluorpropyl)-3-(2-methoxy-2-methylpropyl)-5-methyl-2,4-dioxo-1,2,3,4-tetrahydrothieno-[2,3-d]pyrimidin-6-carbaldehyd

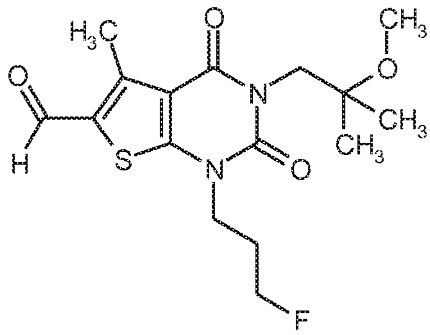

Eine Lösung von 450 mg (1.44 mmol) der Verbindung aus Bsp. 288A in 15 ml DMF wurde mit 498 mg (3.6 mmol) Kaliumcarbonat versetzt und 15 min bei RT gerührt. Dann wurden 814 mg (4.32 mmol) 1-Fluor-3-iodpropan zugesetzt und das Gemisch 18 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 456 mg (88% d. Th.) der Titelverbindung erhalten.
$^1$H-NMR (400 MHz, DMSO-$d_6$, $\delta$/ppm): 10.10 (s, 1H), 4.60 (t, 1H), 4.48 (t, 1H), 4.05 (t, 2H), 3.99 (br. s, 2H), 3.15 (s, 3H), 2.78 (s, 3H), 2.11 (quin, 1H), 2.04 (quin, 1H), 1.09 (s, 6H).
LC/MS (Methode 3, ESIpos): $R_t$ = 1.11 min, m/z = 325 $[M+H-CH_3OH]^+$.

**Beispiel 301A**

1-(2-Methoxyethyl)-3-(2-methoxy-2-methylpropyl)-5-methyl-2,4-dioxo-1,2,3,4-tetrahydrothieno-[2,3-d]pyrimidin-6-carbaldehyd

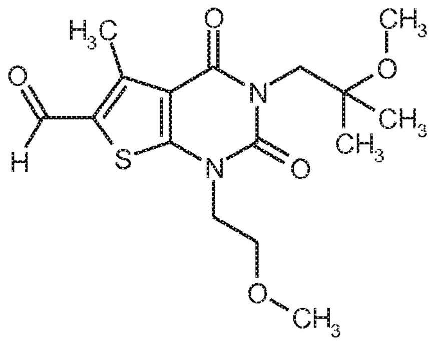

Eine Lösung von 446 mg (1.5 mmol) der Verbindung aus Bsp. 288A in 14 ml DMF wurde mit 520 mg (3.76 mmol) Kaliumcarbonat versetzt und 15 min bei RT gerührt. Dann wurden 628 mg (4.51 mmol) 2-Bromethyl-methylether zugesetzt und das Gemisch 14 h bei 50°C gerührt. Das DMF wurde danach weitestgehend abdestilliert und der verbliebene Rückstand zwischen halbgesättigter Natriumchlorid-Lösung (70 ml) und Ethylacetat (70 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 50

g Kieselgel, Laufmittel Hexan/Ethylacetat). Es wurden 395 mg (71% d. Th.) der Titelverbindung erhalten.
$^1$H-NMR (400 MHz, DMSO-$d_6$, δ/ppm): 10.09 (s, 1H), 4.10 (t, 2H), 4.00 (br. s, 2H), 3.64 (t, 2H), 3.23 (s, 3H), 3.15 (s, 3H), 2.77 (s, 3H), 1.09 (s, 6H).
LC/MS (Methode 3, ESIpos): $R_t$ = 1.07 min, m/z = 355 $[M+H]^+$.

**Beispiel 302A**

Ethyl-[6-formyl-5-methyl-2,4-dioxo-1-(3,3,3-trifluorpropyl)-1,4-dihydrothieno[2,3-d]pyrimidin-3(2*H*)-yl]acetat

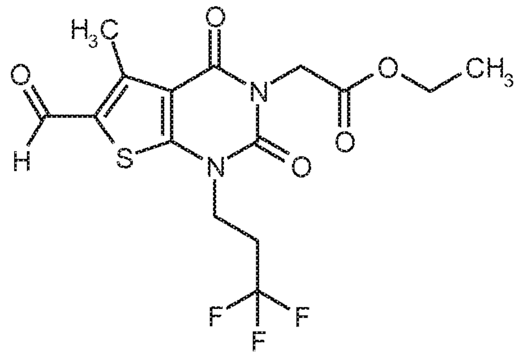

1.0 g (3.37 mmol) der Verbindung aus Bsp. 289A und 1.65 g (5.06 mmol) Cäsiumcarbonat wurden 10 min bei RT in 17 ml DMF gerührt, bevor 1.13 g (5.06 mmol) 1,1,1-Trifluor-3-iodpropan hinzugefügt wurden. Anschließend wurde das Reaktionsgemisch in einem Mikrowellenofen (Biotage-Initiator mit dynamischer Steuerung der Einstrahlleistung) bei einer Temperatur von 100°C gerührt. Nach 1.5 h wurde auf RT abgekühlt, mit ca. 75 ml Ethylacetat verdünnt und nacheinander mit Wasser und gesättigter Natriumchlorid-Lösung gewaschen. Nach Trocknen über wasserfreiem Magnesiumsulfat wurde filtriert und das Filtrat zur Trockene eingeengt. Das Rohprodukt wurde mittels präparativer HPLC (Methode 8) gereinigt. Nach Eindampfen der Produktfraktionen und Trocknen im Hochvakuum wurden 938 mg (70% d. Th.) der Titelverbindung erhalten.
$^1$H-NMR (500 MHz, DMSO-$d_6$, δ/ppm): 10.12 (s, 1H), 4.64 (s, 2H), 4.22 (t, 2H), 4.15 (q, 2H), 2.89-2.74 (m, 2H), 2.79 (s, 3H), 1.21 (t, 3H).
LC/MS (Methode 1, ESIpos): $R_t$ = 0.98 min, m/z = 393 $[M+H]^+$.

**Beispiel 303A**

Ethyl-[6-formyl-1-(2-methoxyethyl)-5-methyl-2,4-dioxo-1,4-dihydrothieno[2,3-d]pyrimidin-3(2*H*)-yl]acetat

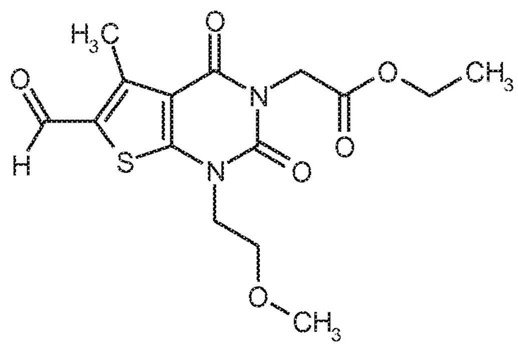

Analog zu dem unter Bsp. 302A beschriebenen Verfahren wurden aus 1.0 g (3.37 mmol) der Verbindung aus Bsp. 289A und 704 mg (5.06 mmol) 2-Bromethyl-methylether 530 mg (43% d. Th.) der Titelverbindung erhalten. Die Reaktion wurde in diesem Fall bei einer Temperatur von 80°C durchgeführt, und die präparative HPLC-Reinigung erfolgte nach Methode 9.

$^1$H-NMR (500 MHz, DMSO-$d_6$, δ/ppm): 10.10 (s, 1H), 4.63 (s, 2H), 4.22-4.08 (m, 4H), 3.66 (t, 2H), 3.31 (s, 3H), 2.77 (s, 3H), 1.21 (t, 3H).
LC/MS (Methode 1, ESIpos): $R_t$ = 0.87 min, m/z = 355 $[M+H]^+$.

**Beispiel 304A**

3-(2-Fluor-2-methylpropyl)-6-(hydroxymethyl)-5-methyl-1-(3,3,3-trifluorpropyl)thieno[2,3-d]-pyrimidin-2,4(1*H*,3*H*)-dion

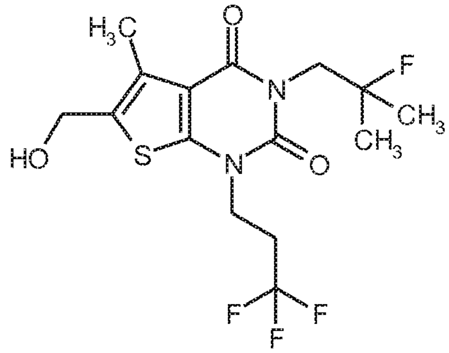

400 mg (1.05 mmol) der Verbindung aus Bsp. 294A wurden in 8.3 ml wasserfreiem THF gelöst und bei -78°C mit 315 µl (0.315 mmol) einer 1 M Lösung von Lithiumaluminiumhydrid in THF versetzt. Nachdem das Reaktionsgemisch bei derselben Temperatur 20 min gerührt worden war, wurden 211 µl Wasser, 914 µl 1 M Natronlauge und etwas Kieselgur hinzugefügt. Anschließend wurde das Gemisch auf RT erwärmt und dann filtriert. Das Filtrat wurde zur Trockene eingeengt und dann wieder in Ethylacetat gelöst. Es wurde nacheinander mit Wasser und gesättigter Natriumchlorid-Lösung gewaschen. Nach Trocknen über wasserfreiem Magnesiumsulfat und Filtration wurde erneut zur Trockene eingeengt. Es wurden 400 mg (93% d. Th., 94% Reinheit) der Titelverbindung erhalten, die ohne weitere Aufreinigung für Folgereaktionen verwendet wurden.
$^1$H-NMR (400 MHz, DMSO-$d_6$, δ/ppm): 5.62 (t, 1H), 4.60 (d, 2H), 4.21-4.07 (m, 4H), 2.87-2.68 (m, 2H), 2.33 (s, 3H), 1.39-1.23 (m, 6H).
LC/MS (Methode 17, ESIpos): $R_t$ = 1.64 min, m/z = 383.10 $[M+H]^+$, 365.09 $[M-OH]^+$, 363.10 $[M-F]^+$.

**Beispiel 305A**

3-(2-Fluor-2-methylpropyl)-6-(hydroxymethyl)-1-(2-methoxyethyl)-5-methylthieno[2,3-d]pyrimidin-2,4(1*H*,3*H*)-dion

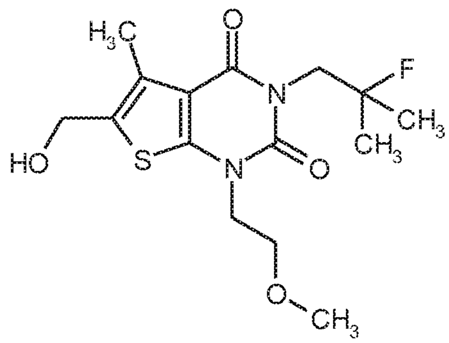

Analog zu dem unter Bsp. 304A beschriebenen Verfahren wurden aus 360 mg (1.01 mmol) der Verbindung aus Bsp. 295A und 303 µl (0.303 mmol) einer 1 M Lösung von Lithiumaluminiumhydrid in THF 142 mg (40% d. Th.) der Titelverbindung erhalten. Das Produkt wurde in diesem Fall mittels präparativer HPLC (Methode 8) gereinigt.
$^1$H-NMR (400 MHz, DMSO-$d_6$, δ/ppm): 5.56 (t, 1H), 4.57 (d, 2H), 4.15 (d, 2H), 4.05 (t, 2H), 3.64 (t, 2H), 3.24 (s, 3H), 2.32 (s, 3H), 1.40-1.21 (m, 6H).

LC/MS (Methode 17, ESIpos): $R_t$ = 1.33 min, m/z = 345.13 $[M+H]^+$, 327.12 $[M-OH]^+$, 325.12 $[M-F]^+$.

**Beispiel 306A**

Ethyl-[6-(hydroxymethyl)-5-methyl-2,4-dioxo-1-(3,3,3-trifluorpropyl)-1,4-dihydrothieno[2,3-d]-pyrimidin-3(2*H*)-yl]acetat

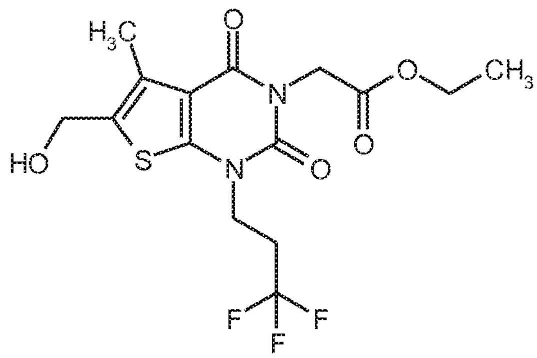

300 mg (0.765 mmol) der Verbindung aus Bsp. 302A wurden in 7.6 ml Ethanol gelöst und bei RT mit 43 mg (1.15 mmol) Natriumborhydrid versetzt. Nach 1 h wurde das Reaktionsgemisch mit je ca. 1 ml Wasser und 1 M Salzsäure versetzt. Nach dem Einengen am Rotationsverdampfer wurde der verbliebene Rückstand in Ethylacetat aufgenommen und nacheinander mit Wasser und gesättigter Natriumchlorid-Lösung gewaschen. Nach Trocknen über wasserfreiem Magnesiumsulfat wurde filtriert und erneut eingeengt. Trocknen des Rückstands im Hochvakuum ergab 279 mg (92% d. Th.) der Titelverbindung.
$^1$H-NMR (500 MHz, DMSO-$d_6$, $\delta$/ppm): 5.66 (t, 1H), 4.62 (s, 2H), 4.61 (d, 2H), 4.16 (t, 2H), 4.13 (q, 2H), 2.85-2.70 (m, 2H), 2.32 (s, 3H), 1.20 (t, 3H).
LC/MS (Methode 1, ESIpos): $R_t$ = 0.85 min, m/z = 395 $[M+H]^+$, 377 $[M-OH]^+$.

**Beispiel 307A**

Ethyl-[6-(hydroxymethyl)-1-(2-methoxyethyl)-5-methyl-2,4-dioxo-1,4-dihydrothieno[2,3-d]pyrimidin-3(2*H*)-yl]acetat

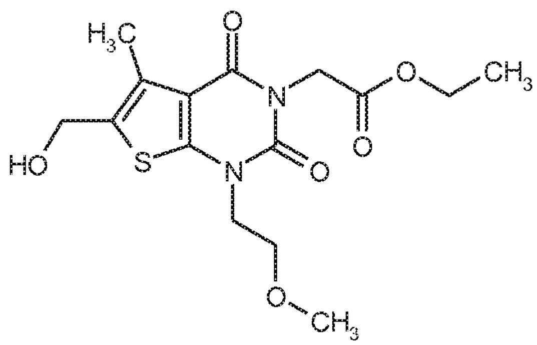

Analog zu dem unter Bsp. 306A beschriebenen Verfahren wurden aus 300 mg (0.847 mmol) der Verbindung aus Bsp. 303A und 48 mg (1.27 mmol) Natriumborhydrid 268 mg (88% d. Th.) der Titelverbindung erhalten.
$^1$H-NMR (400 MHz, DMSO-$d_6$, $\delta$/ppm): 5.60 (t, 1H), 4.62 (s, 2H), 4.59 (d, 2H), 4.14 (q, 2H), 4.07 (t, 2H), 3.64 (t, 2H), 3.24 (s, 3H), 2.31 (s, 3H), 1.20 (t, 3H).
LC/MS (Methode 1, ESIpos): $R_t$ = 0.72 min, m/z = 357 $[M+H]^+$, 339 $[M-OH]^+$.

**Beispiel 308A**

6-{[(2-Aminoethyl)amino]methyl}-1-[2-(cyclopropyloxy)ethyl]-3-ethyl-5-methylthieno[2,3-d]-pyrimidin-2,4(1*H*,3*H*)-dion

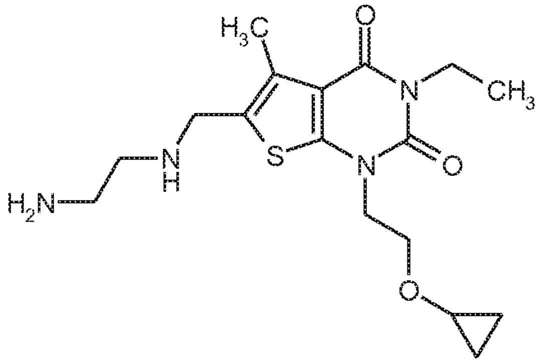

388 mg (1.17 mmol) der Verbindung aus Bsp. 290A wurden in einem Gemisch aus 20 ml Methanol und 8 ml Dichlormethan gelöst. Dann wurden bei RT 788 µl (11.8 mmol) 1,2-Diaminoethan und 270 µl (4.72 mmol) Essigsäure zugesetzt. Es wurde 30 min bei RT gerührt, und danach wurden 312 mg (4.72 mmol) Natriumcyanoborhydrid hinzugefügt. Nachdem das Reaktionsgemisch 111 h bei 60°C gerührt worden war, wurde mit 50 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 385 mg (63% d. Th., 71% Reinheit) der Titelverbindung, welche ohne weitere Reinigung für Folgereaktionen verwendet wurden.
LC/MS (Methode 3, ESIpos): $R_t$ = 0.59 min, m/z = 307 $[M+H-C_2H_8N_2]^+$.

**Beispiel 309A**

6-{[(2-Aminoethyl)amino]methyl}-3-isopropyl-5-methyl-1-(3,3,3-trifluorpropyl)thieno[2,3-d]-pyrimidin-2,4(1*H*,3*H*)-dion

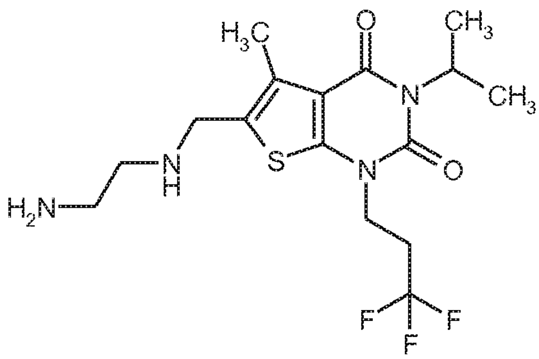

300 mg (0.861 mmol) der Verbindung aus Bsp. 103A wurden in einem Gemisch aus 7.5 ml Methanol und 3 ml Dichlormethan gelöst und bei RT mit 345 µl (5.17 mmol) 1,2-Diaminoethan und 197 µl (3.45 mmol) Essigsäure versetzt. Nach 30 min wurden 216 mg (3.45 mmol) Natriumcyanoborhydrid hinzugefügt, und das Reaktionsgemisch wurde auf 50°C erwärmt. Nach 5 h ließ man das Reaktionsgemisch auf RT abkühlen, und das Rühren wurde 2 Tage bei RT fortgesetzt. Da der Umsatz nach dieser Zeit noch nicht vollständig war, wurden weitere 100 mg (1.59 mmol) Natriumcyanoborhydrid hinzugefügt und erneut auf 50°C erwärmt. Nach 2 h ließ man wieder auf RT abkühlen. Das Gemisch wurde mit 50 ml 2 M Natronlauge versetzt und gründlich mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach Trocknen im Hochvakuum 360 mg (76% d. Th., 72% Reinheit) der Titelverbindung, welche ohne weitere Reinigung für Folgereaktionen verwendet wurden.

LC/MS (Methode 1, ESIpos): $R_t$ = 0.49 min, m/z = 393 $[M+H]^+$, 333 $[M+H-C_2H_8N_2]^+$.

**Beispiel 310A**

6-{[(2-Aminoethyl)amino]methyl}-3-isopropyl-1-(2-methoxyethyl)-5-methylthieno[2,3-d]pyrimidin-2,4(1*H*,3*H*)-dion

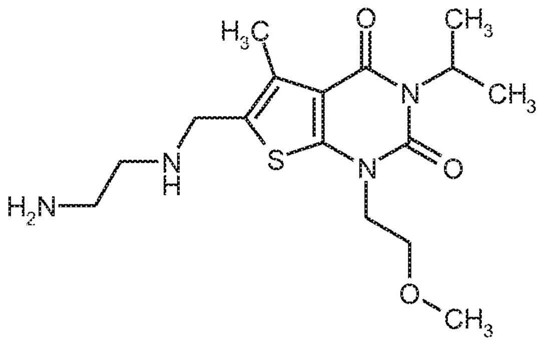

270 mg (0.870 mmol) der Verbindung aus Bsp. 105A wurden in einem Gemisch aus 7.5 ml Methanol und 3 ml Dichlormethan gelöst und bei RT mit 349 µl (5.22 mmol) 1,2-Diaminoethan und 199 µl (3.48 mmol) Essigsäure versetzt. Nach 30 min wurden 218 mg (3.48 mmol) Natriumcyanoborhydrid hinzugefügt, und das Reaktionsgemisch wurde auf 50°C erwärmt. Nach 5 h ließ man das Reaktionsgemisch auf RT abkühlen, und das Rühren wurde 2 Tage bei RT fortgesetzt. Da der Umsatz nach dieser Zeit noch nicht vollständig war, wurden weitere 100 mg (1.59 mmol) Natriumcyanoborhydrid hinzugefügt und erneut auf 50°C erwärmt. Nach 2 h ließ man wieder auf RT abkühlen. Das Gemisch wurde mit 50 ml 2 M Natronlauge versetzt und gründlich mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach Trocknen im Hochvakuum 466 mg (90% d. Th., 60% Reinheit) der Titelverbindung, welche ohne weitere Reinigung für Folgereaktionen verwendet wurden.
LC/MS (Methode 1, ESIpos): $R_t$ = 0.39 min, m/z = 295 $[M+H-C_2H_8N_2]^+$.

**Beispiel 311A**

6-{[(2-Aminoethyl)amino]methyl}-3-isopropyl-5-methyl-1-(oxetan-2-ylmethyl)thieno[2,3-d]-pyrimidin-2,4(1*H*,3*H*)-dion (*Racemat*)

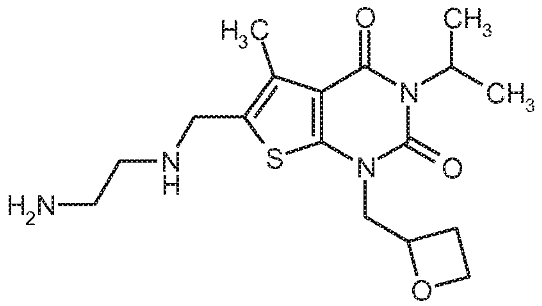

Analog zu dem unter Bsp. 310A beschriebenen Verfahren wurden aus 830 mg (2.57 mmol) der Verbindung aus Bsp. 106A, 1 ml (15.4 mmol) 1,2-Diaminoethan und insgesamt 747 mg (11.9 mmol) Natriumcyanoborhydrid 880 mg (65% d. Th., 70% Reinheit) der Titelverbindung erhalten.
LC/MS (Methode 1, ESIpos): $R_t$ = 0.49 min, m/z = 367 $[M+H]^+$, 307 $[M+H-C_2H_8N_2]^+$.

**Beispiel 312A**

6-{[(2-Aminoethyl)amino]methyl}-3-isopropyl-5-methyl-1-(tetrahydrofuran-2-ylmethyl)thieno-[2,3-d]pyrmidin-2,4(1*H*,3*H*)-dion (*Racemat*)

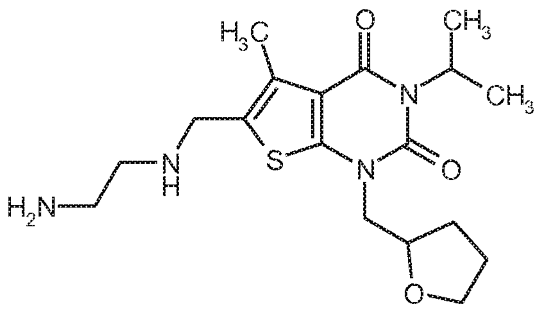

900 mg (2.57 mmol) der Verbindung aus Bsp. 107A wurden in einem Gemisch aus 22 ml Methanol und 9 ml Dichlormethan gelöst und bei RT mit 1 ml (15.4 mmol) 1,2-Diaminoethan und 588 µl (10.3 mmol) Essigsäure versetzt. Nach 30 min wurden 645 mg (10.3 mmol) Natriumcyanoborhydrid hinzugefügt, und das Reaktionsgemisch wurde auf 60°C erwärmt. Nach ca. 15 h ließ man das Reaktionsgemisch auf RT abkühlen. Der Großteil des Lösungsmittels wurde am Rotationsverdampfer entfernt. Der verbliebene Rückstand wurde mit 50 ml 2 M Natronlauge versetzt und gründlich mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach Trocknen im Hochvakuum 937 mg (52% d. Th., 55% Reinheit) der Titelverbindung, welche ohne weitere Reinigung für Folgereaktionen verwendet wurden.
LC/MS (Methode 1, ESIpos): $R_t$ = 0.49 min, m/z = 381 $[M+H]^+$, 321 $[M+H-C_2H_8N_2]^+$.

**Beispiel 313A**

6-{[(2-Aminoethyl)amino]methyl}-1-(2,2-difluorethyl)-3-isobutyl-5-methylthieno[2,3-d]pyrimidin-2,4(1*H*,3*H*)-dion

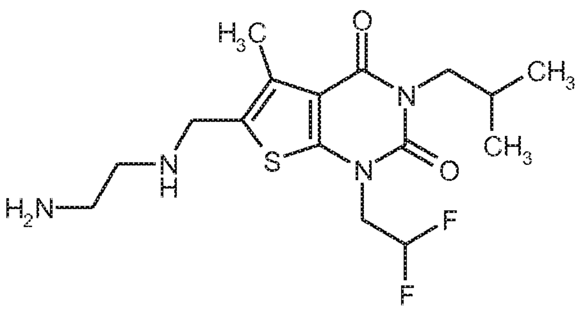

184 mg (0.55 mmol) der Verbindung aus Bsp. 111A wurden in einem Gemisch aus 11 ml Methanol und 5 ml Dichlormethan gelöst. Dann wurden bei RT 372 µl (5.57 mmol) 1,2-Diaminoethan und 127 µl (2.22 mmol) Essigsäure zugesetzt. Es wurde 30 min bei RT gerührt, und danach wurden 147 mg (2.23 mmol) Natriumcyanoborhydrid hinzugefügt. Nachdem das Reaktionsgemisch 93 h bei 60°C gerührt worden war, wurde mit 100 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 275 mg (100% d. Th., 76% Reinheit) der Titelverbindung, welche ohne weitere Reinigung für Folgereaktionen verwendet wurden.
LC/MS (Methode 3, ESIpos): $R_t$ = 0.72 min, m/z = 315

**Beispiel 314A**

6-{[(2-Aminoethyl)amino]methyl}-1-(2-ethoxyethyl)-3-isobutyl-5-methylthieno[2,3-d]pyrimidin-2,4(1*H*,3*H*)-dion

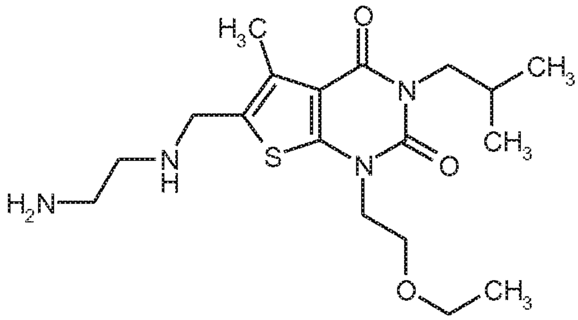

357 mg (1.05 mmol) der Verbindung aus Bsp. 115A wurden in einem Gemisch aus 21 ml Methanol und 10 ml Dichlormethan gelöst. Dann wurden bei RT 705 µl (10.6 mmol) 1,2-Diaminoethan und 241 µl (4.22 mmol) Essigsäure zugesetzt. Es wurde 30 min bei RT gerührt, und danach wurden 279 mg (4.22 mmol) Natriumcyanoborhydrid hinzugefügt. Nachdem das Reaktionsgemisch 140 h bei 60°C gerührt worden war, wurde mit 100 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 585 mg (83% Reinheit) der Titelverbindung, welche ohne weitere Reinigung für Folgereaktionen verwendet wurden.
LC/MS (Methode 3, ESIpos): $R_t$ = 0.72 min, m/z = 323

**Beispiel 315A**

6-{[(2-Aminoethyl)amino]methyl}-3-(2,2-dimethylpropyl)-5-methyl-1-(3,3,3-trifluorpropyl)thieno-[2,3-d]pyrimidin-2,4(1*H*,3*H*)-dion

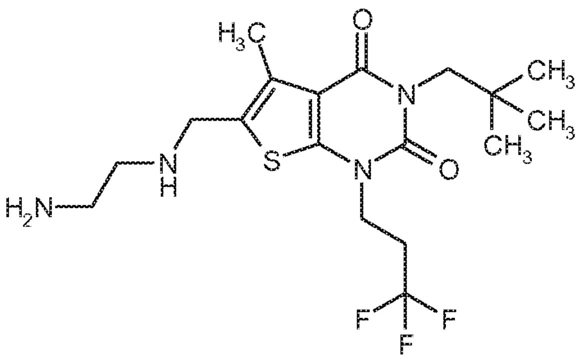

386 mg (1.02 mmol) der Verbindung aus Bsp. 291A wurden in einem Gemisch aus 21 ml Methanol und 10 ml Dichlormethan gelöst. Dann wurden bei RT 686 µl (10.2 mmol) 1,2-Diaminoethan und 235 µl (4.1 mmol) Essigsäure zugesetzt. Es wurde 30 min bei RT gerührt, und danach wurden 271 mg (4.1 mmol) Natriumcyanoborhydrid hinzugefügt. Nachdem das Reaktionsgemisch 93 h bei 60°C gerührt worden war, wurde mit 100 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 610 mg (83% Reinheit) der Titelverbindung, welche ohne weitere Reinigung für Folgereaktionen verwendet wurden.
LC/MS (Methode 3, ESIpos): $R_t$ = 0.75 min, m/z = 421 $[M+H]^+$, 361 $[M+H-C_2H_8N_2]^+$.

**Beispiel 316A**

6-{[(2-Aminoethyl)amino]methyl)-3-(2,2-dimethylpropyl)-1-(3-fluorpropyl)-5-methylthieno-[2,3-d]pyrimidin-2,4(1*H*,3*H*)-dion

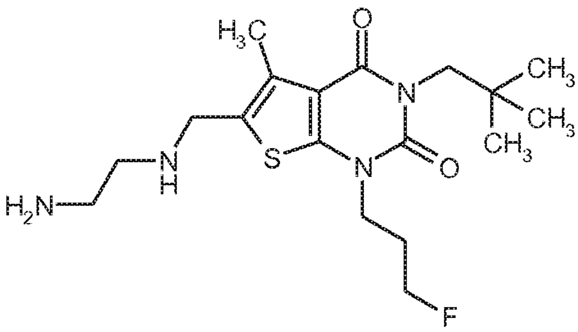

321 mg (0.94 mmol) der Verbindung aus Bsp. 292A wurden in einem Gemisch aus 16 ml Methanol und 7 ml Dichlormethan gelöst. Dann wurden bei RT 630 µl (9.43 mmol) 1,2-Diaminoethan und 216 µl (3.77 mmol) Essigsäure zugesetzt. Es wurde 30 min bei RT gerührt, und danach wurden 249 mg (3.77 mmol) Natriumcyanoborhydrid hinzugefügt. Nachdem das Reaktionsgemisch 42 h bei 60°C gerührt worden war, wurde mit 100 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 396 mg (89% d. Th., 82% Reinheit) der Titelverbindung, welche ohne weitere Reinigung für Folgereaktionen verwendet wurden.
LC/MS (Methode 3, ESIpos): $R_t$ = 0.69 min, m/z = 385 $[M+H]^+$, 325 $[M+H-C_2H_8N_2]^+$.

**Beispiel 317A**

6-{[(2-Aminoethyl)amino]methyl}-3-(2,2-dimethylpropyl)-1-(2-methoxyethyl)-5-methylthieno-[2,3-d]pyrimidin-2,4(1*H*,3*H*)-dion

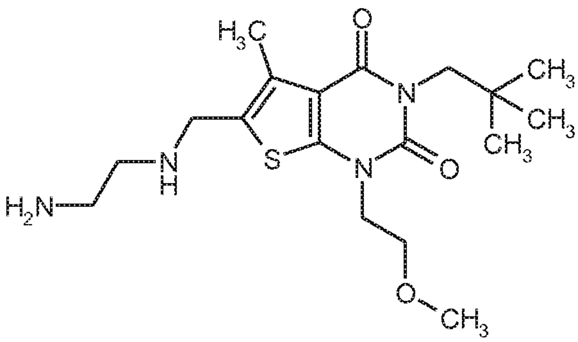

417 mg (1.23 mmol) der Verbindung aus Bsp. 293A wurden in einem Gemisch aus 25 ml Methanol und 12 ml Dichlormethan gelöst. Dann wurden bei RT 824 µl (12.3 mmol) 1,2-Diaminoethan und 282 µl (4.93 mmol) Essigsäure zugesetzt. Es wurde 30 min bei RT gerührt, und danach wurden 326 mg (4.93 mmol) Natriumcyanoborhydrid hinzugefügt. Nachdem das Reaktionsgemisch 66 h bei 60°C gerührt worden war, wurde mit 100 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 645 mg (81% Reinheit) der Titelverbindung, welche ohne weitere Reinigung für Folgereaktionen verwendet wurden.
LC/MS (Methode 3, ESIpos): $R_t$ = 0.65 min, m/z = 383 $[M+H]^+$, 323 $[M+H-C_2H_8N_2]^+$.

**Beispiel 318A**

6-{[(2-Aminoethyl)amino]methyl)}3-(2,2-difluorethyl)-1,5-dimethylthieno[2,3-d]pyrimidin-2,4(1*H*,3*H*)-dion

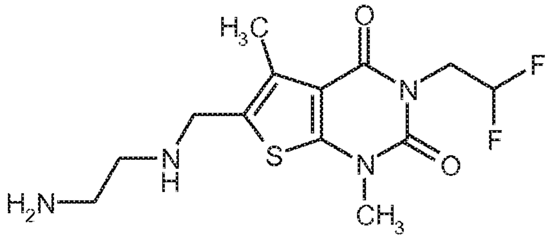

355 mg (1.12 mmol) der Verbindung aus Bsp. 135A wurden in einem Gemisch aus 19 ml Methanol und 8 ml Dichlormethan gelöst. Dann wurden bei RT 749 µl (11.2 mmol) 1,2-Diaminoethan und 257 µl (4.48 mmol) Essigsäure zugesetzt. Es wurde 30 min bei RT gerührt, und danach wurden 296 mg (4.48 mmol) Natriumcyanoborhydrid hinzugefügt. Nachdem das Reaktionsgemisch 132 h bei 60°C gerührt worden war, wurde mit 50 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 350 mg (65% d. Th., 70% Reinheit) der Titelverbindung, welche ohne weitere Reinigung für Folgereaktionen verwendet wurden.
LC/MS (Methode 3, ESIpos): $R_t$ = 0.46 min, m/z = 333 $[M+H]^+$, 273 $[M+H-C_2H_8N_2]^+$.

**Beispiel 319A**

6-{[(2-Aminoethyl)amino]methyl)-3-(2-methoxyethyl)-5-methyl-1-(3,3,3-trifluorpropyl)thieno-[2,3-d]pyrimidin-2,4(1*H*,3*H*)-dion

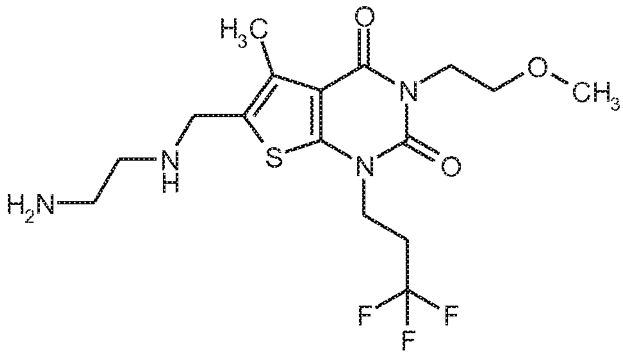

250 mg (0.67 mmol) der Verbindung aus Bsp. 296A wurden in einem Gemisch aus 11 ml Methanol und 5 ml Dichlormethan gelöst. Dann wurden bei RT 450 µl (6.72 mmol) 1,2-Diaminoethan und 154 µl (2.69 mmol) Essigsäure zugesetzt. Es wurde 30 min bei RT gerührt, und danach wurden 178 mg (2.69 mmol) Natriumcyanoborhydrid hinzugefügt. Nachdem das Reaktionsgemisch 118 h bei 60°C gerührt worden war, wurde mit 50 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 277 mg (81% d. Th., 81% Reinheit) der Titelverbindung, welche ohne weitere Reinigung für Folgereaktionen verwendet wurden.
LC/MS (Methode 3, ESIpos): $R_t$ = 0.57 min, m/z = 409 $[M+H]^+$, 349

**Beispiel 320A**

6-{[(2-Aminoethyl)amino]methyl}-1-(3-fluorpropyl)-3-(2-methoxyethyl)-5-methylthieno[2,3-d]-pyrimidin-2,4(1*H*,3*H*)-dion

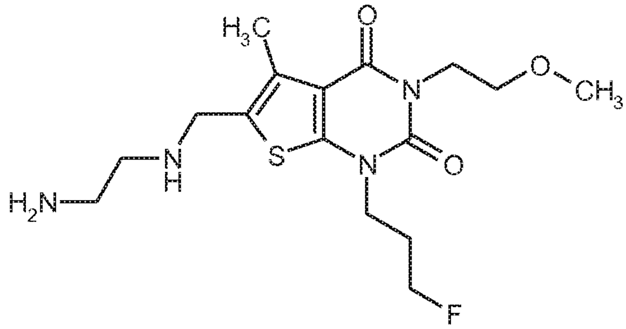

175 mg (0.52 mmol) der Verbindung aus Bsp. 297A wurden in einem Gemisch aus 9 ml Methanol und 4 ml Dichlormethan gelöst. Dann wurden bei RT 349 µl (5.22 mmol) 1,2-Diaminoethan und 120 µl (2.09 mmol) Essigsäure zugesetzt. Es wurde 30 min bei RT gerührt, und danach wurden 138 mg (2.09 mmol) Natriumcyanoborhydrid hinzugefügt. Nachdem das Reaktionsgemisch 92 h bei 60°C gerührt worden war, wurde mit 50 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 150 mg (49% d. Th., 64% Reinheit) der Titelverbindung, welche ohne weitere Reinigung für Folgereaktionen verwendet wurden.
LC/MS (Methode 3, ESIpos): $R_t$ = 0.49 min, m/z = 373 $[M+H]^+$, 313 $[M+H-C_2H_8N_2]^+$.

**Beispiel 321A**

6-{[(2-Aminoethyl)amino]methyl}-3-(2-methoxyethyl)-5-methyl-1-(tetrahydrofuran-2-ylmethyl)-thieno[2,3-d]pyrimidin-2,4(1*H*,3*H*)-dion (*Racemat*)

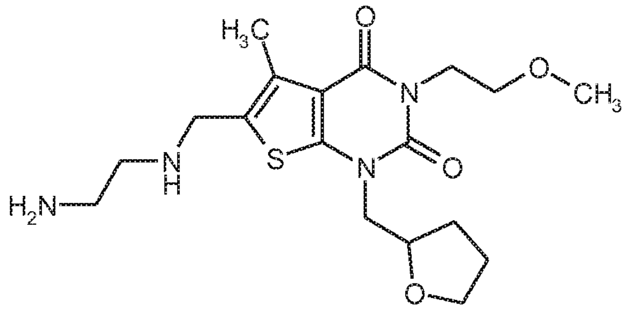

440 mg (1.22 mmol) der Verbindung aus Bsp. 298A wurden in einem Gemisch aus 20 ml Methanol und 9 ml Dichlormethan gelöst. Dann wurden bei RT 818 µl (12.2 mmol) 1,2-Diaminoethan und 280 µl (4.89 mmol) Essigsäure zugesetzt. Es wurde 30 min bei RT gerührt, und danach wurden 324 mg (4.89 mmol) Natriumcyanoborhydrid hinzugefügt. Nachdem das Reaktionsgemisch 117 h bei 60°C gerührt worden war, wurde mit 50 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 305 mg (41% d. Th., 66% Reinheit) der Titelverbindung, welche ohne weitere Reinigung für Folgereaktionen verwendet wurden.
LC/MS (Methode 3, ESIpos): $R_t$ = 0.53 min, m/z = 397 $[M+H]^+$, 337 $[M+H-C_2H_8N_2]^+$.

**Beispiel 322A**

6-{[(2-Aminoethyl)amino]methyl}-3-(2-methoxy-2-methylpropyl)-5-methyl-1-(3,3,3-trifluor-propyl)thieno[2,3-d]pyrimidin-2,4(1*H*,3*H*)-dion

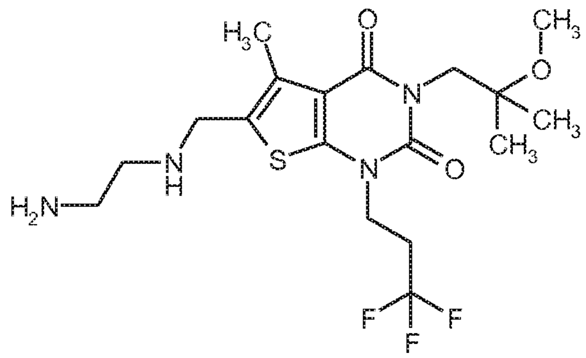

463 mg (1.18 mmol) der Verbindung aus Bsp. 299A wurden in einem Gemisch aus 24 ml Methanol und 11 ml Dichlormethan gelöst. Dann wurden bei RT 789 μl (11.8 mmol) 1,2-Diaminoethan und 270 μl (4.72 mmol) Essigsäure zugesetzt. Es wurde 30 min bei RT gerührt, und danach wurden 312 mg (4.72 mmol) Natriumcyanoborhydrid hinzugefügt. Nachdem das Reaktionsgemisch 69 h bei 60°C gerührt worden war, wurde mit 100 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 630 mg (99% d. Th., 81% Reinheit) der Titelverbindung, welche ohne weitere Reinigung für Folgereaktionen verwendet wurden.
LC/MS (Methode 3, ESIpos): $R_t$ = 0.64 min, m/z = 437 $[M+H]^+$, 377

**Beispiel 323A**

6-{[(2-Aminoethyl)amino]methyl}-1-(3-fluorpropyl)-3-(2-methoxy-2-methylpropyl)-5-methyl-thieno[2,3-d]pyrimidin-2,4(1*H*,3*H*)-dion

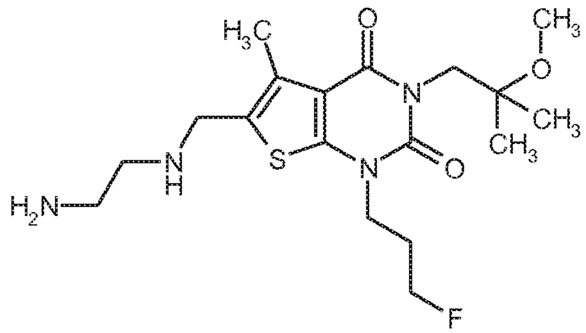

456 mg (1.28 mmol) der Verbindung aus Bsp. 300A wurden in einem Gemisch aus 26 ml Methanol und 12 ml Dichlormethan gelöst. Dann wurden bei RT 855 μl (12.8 mmol) 1,2-Diaminoethan und 293 μl (5.12 mmol) Essigsäure zugesetzt. Es wurde 30 min bei RT gerührt, und danach wurden 338 mg (5.12 mmol) Natriumcyanoborhydrid hinzugefügt. Nachdem das Reaktionsgemisch 66 h bei 60°C gerührt worden war, wurde mit 100 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 730 mg (85% Reinheit) der Titelverbindung, welche ohne weitere Reinigung für Folgereaktionen verwendet wurden.
LC/MS (Methode 3, ESIpos): $R_t$ = 0.56 min, m/z = 401 $[M+H]^+$, 341 $[M+H-C_2H_8N_2]^+$.

**Beispiel 324A**

6-{[(2-Aminoethyl)amino]methyl}-1-(2-methoxyethyl)-3-{2-methoxy-2-methylpropyl)-5-methyl-thieno[2,3-d]pyrimidin-2,4(1*H*,3*H*)-dion

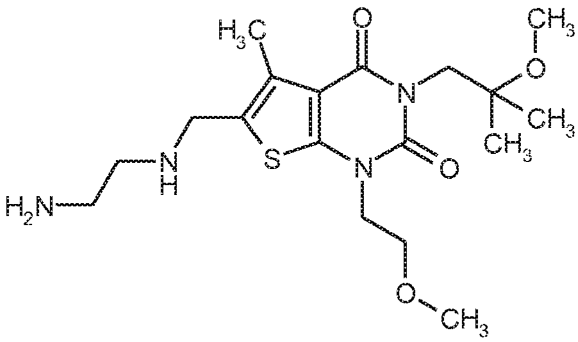

390 mg (1.05 mmol) der Verbindung aus Bsp. 301A wurden in einem Gemisch aus 21 ml Methanol und 10 ml Dichlormethan gelöst. Dann wurden bei RT 706 µl (10.56 mmol) 1,2-Diaminoethan und 242 µl (4.22 mmol) Essigsäure zugesetzt. Es wurde 30 min bei RT gerührt, und danach wurden 279 mg (4.22 mmol) Natriumcyanoborhydrid hinzugefügt. Nachdem das Reaktionsgemisch 87 h bei 60°C gerührt worden war, wurde mit 100 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 355 mg (65% d. Th., 78% Reinheit) der Titelverbindung, welche ohne weitere Reinigung für Folgereaktionen verwendet wurden.
LC/MS (Methode 3, ESIpos): $R_t$ = 0.56 min, m/z = 399 $[M+H]^+$, 339 $[M+H-C_2H_8N_2]^+$.

**Beispiel 325A**

6-{[(2,2-Dimethoxyethyl)amino]methyl}-3-ethyl-5-methyl-1-(tetrahydrofuran-2-ylmethyl)thieno-[2,3-d]pyrimidin-2,4(1*H*,3*H*)-dion (*Racemat*)

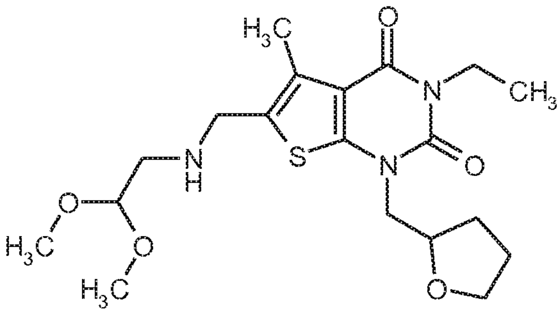

1.0 g (3.10 mmol) der Verbindung aus Bsp. 89A wurden in 70 ml Dichlormethan gelöst und mit 326 mg (3.10 mmol) 2,2-Dimethoxyethanamin versetzt. Das Gemisch wurde 1 h zum Rückfluss erwärmt. Nach dem Abkühlen auf RT wurden 1.31 g (6.20 mmol) Natriumtriacetoxyborhydrid hinzugefügt, und es wurde bei RT weiter gerührt. Da nach ca. 18 h der Umsatz noch nicht vollständig war, wurden weitere 163 mg (1.55 mmol) 2,2-Dimethoxyethanamin und 657 mg (3.10 mmol) Natriumtriacetoxyborhydrid hinzugefügt. Nach weiteren 20 h Rühren bei RT wurde mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und das Filtrat zur Trockene eingeengt. Der erhaltene Rückstand wurde mittels Chromatographie gereinigt (Biotage Isolera One, Kartusche SNAP KP-Sil, 50 g Kieselgel, Laufmittel Cyclohexan/Ethylacetat 50:50 → 0:100). Nach Einengen der Produktfraktionen und Trocknen im Hochvakuum wurden 1.22 g (94% d. Th.) der Titelverbindung erhalten.
LC/MS (Methode 17, ESIpos): $R_t$ = 0.82 min, m/z = 307 $[M+H-C_4H_{11}NO_2]^+$.

**Beispiel 326A**

6-{[(2,2-Dimethoxyethyl)amino]methyl}-3-isopropyl-5-methyl-1-(3,3,3-trifluorpropyl)thieno-[2,3-d]pyrimidin-2,4(1*H*,3*H*)-dion

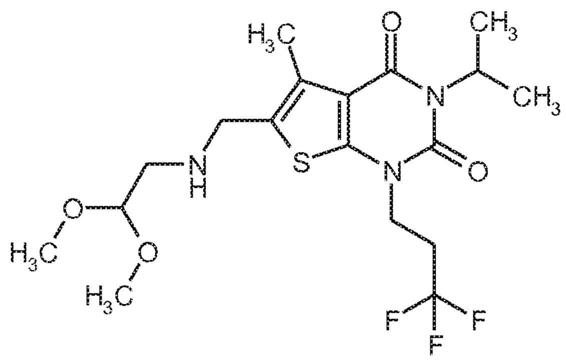

500 mg (1.43 mmol) der Verbindung aus Bsp. 103A wurden in 30 ml Dichlormethan gelöst und mit 223 µl (2.15 mmol) 2,2-Dimethoxyethanamin versetzt. Das Gemisch wurde 1 h zum Rückfluss erwärmt. Nach dem Abkühlen auf RT wurden 960 mg (4.31 mmol) Natriumtriacetoxyborhydrid hinzugefügt, und es wurde bei RT weiter gerührt. Nach ca. 18 h wurde mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und das Filtrat zur Trockene eingeengt. Der erhaltene Rückstand wurde mittels Chromatographie gereinigt (Biotage Isolera One, Kartusche SNAP KP-Sil, 50 g Kieselgel, Laufmittel Cyclohexan/ Ethylacetat 2:1). Nach Einengen der Produktfraktionen und Trocknen im Hochvakuum wurden 438 mg (68% d. Th.) der Titelverbindung erhalten.
$^1$H-NMR (400 MHz, DMSO-$d_6$, δ/ppm): 5.13 (sept, 1H), 4.41 (t, 1H), 4.08 (t, 2H), 3.83 (s, 2H), 3.26 (s, 6H), 2.83-2.67 (m, 2H), 2.62 (d, 2H), 2.33 (s, 3H), 2.30 (breit, 1H), 1.40 (d, 6H).
LC/MS (Methode 17, ESIpos): $R_t$ = 1.10 min, m/z = 333.09 $[M+H-C_4H_{11}NO_2]^+$.

**Beispiel 327A**

6-{[(2,2-Dimethoxyethyl)amino]methyl}-3-isopropyl-1-(2-methoxyethyl)-5-methylthieno[2,3-d]-pyrimidin-2,4(1*H*,3*H*)-dion

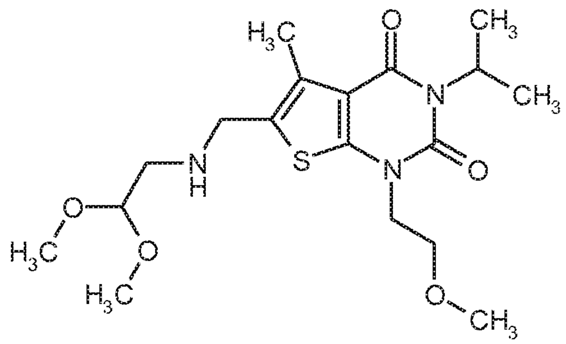

500 mg (1.61 mmol) der Verbindung aus Bsp. 105A wurden in 30 ml Dichlormethan gelöst und mit 261 µl (2.42 mmol) 2,2-Dimethoxyethanamin versetzt. Das Gemisch wurde 1 h zum Rückfluss erwärmt. Nach dem Abkühlen auf RT wurden 1.02 g (4.83 mmol) Natriumtriacetoxyborhydrid hinzugefügt, und es wurde bei RT weiter gerührt. Nach ca. 18 h wurde mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und das Filtrat zur Trockene eingeengt. Der erhaltene Rückstand wurde mittels Chromatographie gereinigt (Biotage Isolera One, Kartusche SNAP KP-Sil, 50 g Kieselgel, Laufmittel Cyclohexan/Ethylacetat 2:1). Nach Einengen der Produktfraktionen und

Trocknen im Hochvakuum wurden 461 mg (70% d. Th.) der Titelverbindung erhalten.
$^1$H-NMR (400 MHz, DMSO-$d_6$, δ/ppm): 5.13 (sept, 1H), 4.40 (t, 1H), 4.00 (t, 2H), 3.81 (br. s, 2H), 3.62 (t, 2H), 3.26 (s, 6H), 3.24 (s, 3H), 2.62 (br. s, 2H), 2.31 (s, 3H), 2.24 (breit, 1H), 1.40 (d, 6H).
LC/MS (Methode 1, ESIpos): $R_t$ = 0.60 min, m/z = 400 $[M+H]^+$, 295 $[M+H-C_4H_{11}NO_2]^+$.

**Beispiel 328A**

6-{[(2,2-Dimethoxyethyl)amino]methyl}-3-isopropyl-5-methyl-1-(tetrahydrofuran-2-ylmethyl)-thieno[2,3-d]pyrimidin-2,4(1*H*,3*H*)-dion (*Racemat*)

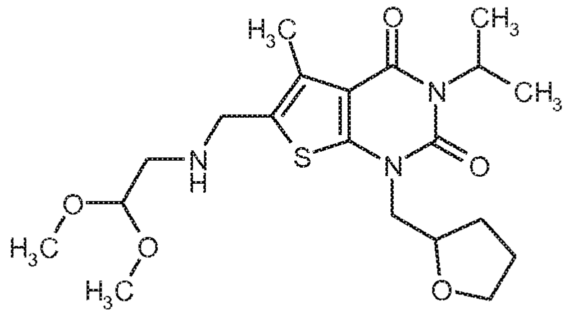

Analog zu dem unter Bsp. 327A beschriebenen Verfahren wurden aus 900 mg (2.67 mmol) der Verbindung aus Bsp. 107A, 434 µl (4.01 mmol) 2,2-Dimethoxyethanamin und 1.70 g (8.03 mmol) Natriumtriacetoxyborhydrid 1.01 g (88% d. Th.) der Titelverbindung erhalten. Die Chromatographie des Rohprodukts erfolgte hier unter folgenden Bedingungen: Biotage Isolera One, Kartusche SNAP KP-Sil, 100 g Kieselgel, Laufmittel Cyclohexan/Ethylacetat 50:50 → 0:100.
$^1$H-NMR (400 MHz, DMSO-$d_6$, δ/ppm): 5.14 (sept, 1H), 4.40 (t, 1H), 4.27-4.17 (m, 1H), 4.07-3.97 (m, 1H), 3.81 (s, 2H), 3.78-3.57 (m, 3H), 3.26 (s, 6H), 2.61 (d, 2H), 2.32 (s, 3H), 2.04-1.74 (m, 3H), 1.72-1.59 (m, 1H), 1.40 (d, 6H).
LC/MS (Methode 17, ESIpos): $R_t$ = 0.98 min, m/z = 321.13 $[M+H-C_4H_{11}NO_2]^+$.

Beispiel 329A

1-(2,2-Dimethoxyethyl)-1-{[3-ethyl-5-methyl-2,4-dioxo-1-(tetrahydrofuran-2-ylmethyl)-1,2,3,4-tetrahydrothieno[2,3-d]pyrimidin-6-yl]methyl}harnstoff (*Racemat*)

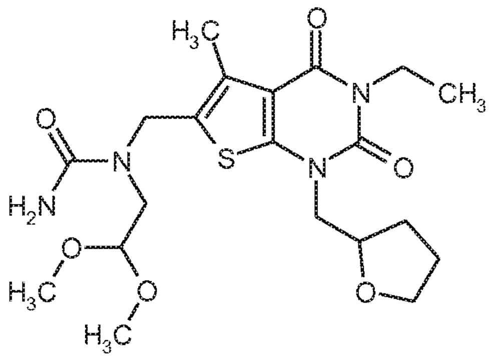

Analog zu dem unter Bsp. 273A beschriebenen Verfahren wurden aus 1.20 g (2.92 mmol) der Verbindung aus Bsp. 325A, 544 mg (6.71 mmol) Kaliumcyanat und 428 µl (4.96 mmol) Perchlorsäure (70% in Wasser) 912 mg (61% d. Th., 90% Reinheit) der Titelverbindung hergestellt. Die Reaktionszeit betrug hier 3 h, und das isolierte Rohprodukt wurde zusätzlich noch mit Ethylacetat bei RT verrührt.
$^1$H-NMR (400 MHz, DMSO-$d_6$, δ/ppm): 6.03 (s, 2H), 4.54 (s, 2H), 4.44 (t, 1H), 4.27-4.15 (m, 1H), 4.01 (dd, 1H), 3.90 (q, 2H), 3.78-3.66 (m, 2H), 3.64-3.56 (m, 1H), 3.31 (m, 7H), 3.16 (t, 1H), 2.39 (s, 3H), 2.04-1.73 (m, 3H), 1.71-1.59 (m, 1H),

1.11 (t, 3H).
LC/MS (Methode 17, ESIpos): $R_t$ = 1.35 min, m/z = 423.17 $[M+H-CH_3OH]^+$, 307.11 $[M+H-C_5H_{12}N_2O_3]^+$.

**Beispiel 330A**

1-(2,2-Dimethoxyethyl)-1-{[3-isopropyl-5-methyl-2,4-dioxo-1-(3,3,3-trifluorpropyl)-1,2,3,4-tetra-hydrothieno[2,3-d]pyrimidin-6-yl]methyl}harnstoff

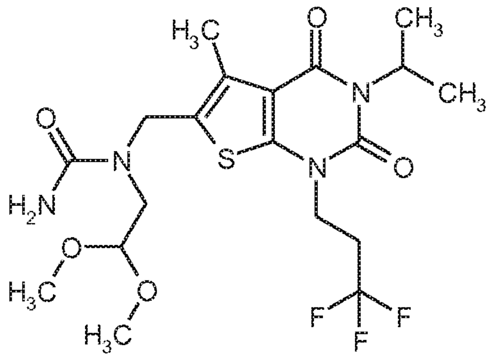

Eine Lösung von 425 mg (0.971 mmol) der Verbindung aus Bsp. 326A in 9 ml Methanol wurde bei RT zunächst mit 181 mg (2.23 mmol) Kaliumcyanat und dann mit 142 μl (1.65 mmol) Perchlorsäure (70% in Wasser) versetzt. Nach 6 Tagen wurde das Reaktionsgemisch mit gesättigter wässriger Natriumhydrogencarbonat-Lösung versetzt und anschließend mit Ethylacetat extrahiert. Der organische Extrakt wurde nacheinander mit Wasser und gesättigter Natriumchlorid-Lösung gewaschen, über wasserfreiem Magnesiumsulfat getrocknet, filtriert und eingeengt. Nach Trocknen des erhaltenen Rückstands im Hochvakuum wurden 294 mg (62% d. Th.) der Titelverbindung erhalten.
$^1$H-NMR (400 MHz, DMSO-$d_6$, δ/ppm): 6.04 (s, 2H), 5.13 (sept, 1H), 4.55 (s, 2H), 4.45 (t, 1H), 4.06 (t, 2H), 3.31 (s, 6H, teilweise überlagert vom Wassersignal), 3.16 (d, 2H), 2.84-2.65 (m, 2H), 2.39 (s, 3H), 1.39 (d, 6H).
LC/MS (Methode 17, ESIpos): $R_t$ = 1.77 min, m/z = 449.15 $[M+H-CH_3OH]^+$, 333.09 $[M+H-C_5H_{12}N_2O_3]^+$.

**Beispiel 331A**

1-(2,2-Dimethoxyethyl)-1-{[3-isopropyl-1-(2-methoxyethyl)-5-methy1-2,4-dioxo-1,2,3,4-tetra-hydrothieno[2,3-d]pyrimidin-6-yl]methyl}harnstoff

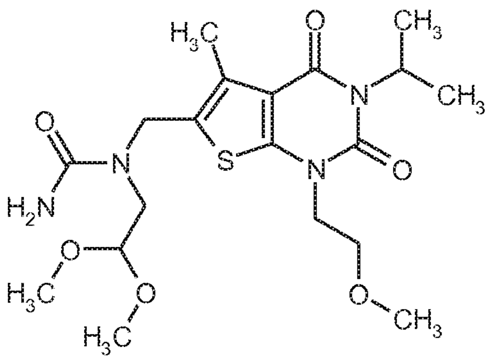

Eine Lösung von 450 mg (1.13 mmol) der Verbindung aus Bsp. 327A in 12 ml Methanol wurde bei RT zunächst mit 210 mg (2.59 mmol) Kaliumcyanat und dann mit 165 μl (1.91 mmol) Perchlorsäure (70% in Wasser) versetzt. Nach 6 Tagen wurde das Reaktionsgemisch mit gesättigter wässriger Natriumhydrogencarbonat-Lösung versetzt und anschließend mit Ethylacetat extrahiert. Der organische Extrakt wurde nacheinander mit Wasser und gesättigter Natriumchlorid-Lösung gewaschen, über wasserfreiem Magnesiumsulfat getrocknet, filtriert und eingeengt. Nach Trocknen des

erhaltenen Rückstands im Hochvakuum wurden 580 mg (76% d. Th., 66% Reinheit) der Titelverbindung erhalten, welche ohne weitere Aufreinigung für Folgereaktionen verwendet wurden.
LC/MS (Methode 1, ESIpos): $R_t$ = 0.86 min, m/z = 443 $[M+H]^+$, 411 $[M+H-CH_3OH]^+$, 295 $[M+H-C_5H_{12}N_2O_3]^+$.

**Beispiel 332A**

1-(2,2-Dimethoxyethyl)-1-{[3-isopropyl-5-methyl-2,4-dioxo-1-(tetrahydrofuran-2-ylmethyl)-1,2,3,4-tetrahydrothieno[2,3-d]pyrimidin-6-yl]methyl}harntoff (*Racemat*)

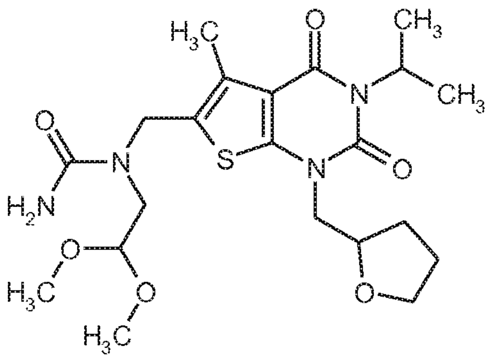

Analog zu dem unter Bsp. 273A beschriebenen Verfahren wurden aus 985 mg (2.31 mmol) der Verbindung aus Bsp. 328A, 432 mg (5.32 mmol) Kaliumcyanat und 339 μl (3.93 mmol) Perchlorsäure (70% in Wasser) 1.15 g (81% d. Th., 77% Reinheit) der Titelverbindung hergestellt, die ohne weitere Aufreinigung für Folgereaktionen verwendet wurden. Die Reaktionszeit betrug hier ca. 18 h.
LC/MS (Methode 1, ESIpos): $R_t$ = 0.85 min, m/z = 469 $[M+H]^+$, 437 $[M+H-CH_3OH]^+$, 321 $[M+H-C_5H_{12}N_2O_3]^+$.

**Beispiel 333A**

*tert.*-Butyl-2-{[3-ethyl-5-methyl-2,4-dioxo-1-(3,3,3-trifluorpropyl)-1,2,3,4-tetrahydrothieno[2,3-d]-pyrimidin-6-yl]methylen}hydrazincarboxylat

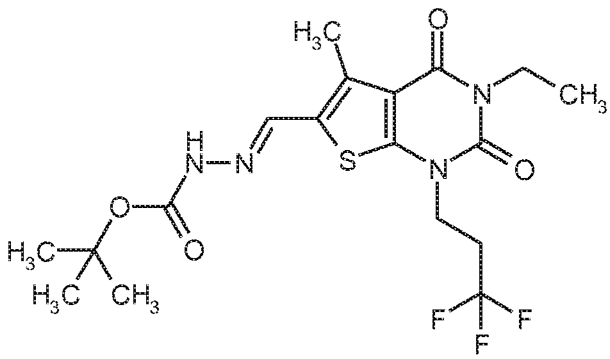

Eine Lösung von 1.0 g (2.99 mmol) der Verbindung aus Bsp. 74A in 30 ml Ethanol wurde zunächst mit 395 mg (2.99 mmol) tert.-Butyl-hydrazincarboxylat und dann mit 5 Tropfen konzentrierter Salzsäure versetzt. Nachdem das Reaktionsgemisch ca. 18 h bei RT gerührt worden war, wurde der Großteil des Ethanols am Rotationsverdampfer entfernt. Der verbliebene Rückstand wurde mit 150 ml Wasser verdünnt und durch Zusatz von gesättigter wässriger Natriumhydrogencarbonat-Lösung neutralisiert. Der dabei ausgefallene Feststoff wurde abgesaugt, mit wenig Wasser gewaschen und im Hochvakuum getrocknet. Es wurden 1.25 g (93% d. Th.) der Titelverbindung erhalten.
$^1$H-NMR (400 MHz, DMSO-$d_6$, δ/ppm): 10.88 (br. s, 1H), 8.30 (s, 1H), 4.15 (t, 2H), 3.90 (q, 2H), 2.88-2.71 (m, 2H), 2.46 (s, 3H), 1.46 (s, 9H), 1.12 (t, 3H).

LC/MS (Methode 1, ESIpos): $R_t$ = 1.13 min, m/z = 449 $[M+H]^+$.

**Beispiel 334A**

*tert.*-Butyl-2-{[3-ethyl-1-(2-methoxyethyl)-5-methyl-2,4-dioxo-1,2,3,4-tetrahydrothieno[2,3-d]-pyrimidin-6-yl]methylen}hydrazincarboxylat

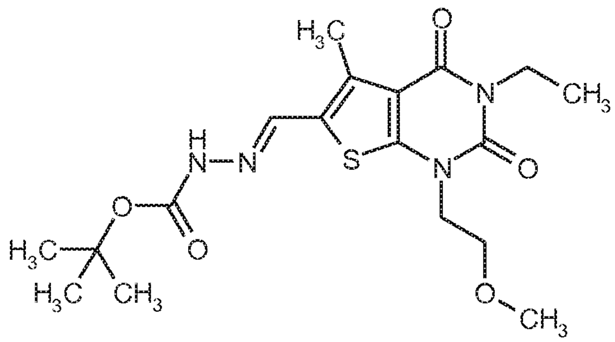

Eine Lösung von 1.0 g (3.37 mmol) der Verbindung aus Bsp. 84A in 30 ml Ethanol wurde zunächst mit 669 mg (5.06 mmol) tert.-Butyl-hydrazincarboxylat und dann mit 5 Tropfen konzentrierter Salzsäure versetzt. Nachdem das Reaktionsgemisch ca. 18 h bei RT gerührt worden war, wurde der Großteil des Ethanols am Rotationsverdampfer entfernt. Der verbliebene Rückstand wurde mit 150 ml Wasser verdünnt und durch Zusatz von gesättigter wässriger Natriumhydrogencarbonat-Lösung neutralisiert. Der dabei ausgefallene Feststoff wurde abgesaugt, mit wenig Wasser gewaschen und im Hochvakuum getrocknet. Es wurden 1.34 g (96% d. Th.) der Titelverbindung erhalten.
$^1$H-NMR (400 MHz, DMSO-$d_6$, δ/ppm): 10.85 (breit, 1H), 8.29 (s, 1H), 4.07 (t, 2H), 3.90 (q, 2H), 3.65 (t, 2H), 3.25 (s, 3H), 2.45 (s, 3H), 1.45 (s, 9H), 1.12 (t, 3H).
LC/MS (Methode 1, ESIpos): $R_t$ = 1.00 min, m/z = 411 $[M+H]^+$.

**Beispiel 335A**

*tert.*-Butyl-2-{[3-ethyl-5-methyl-2,4-dioxo-1-(tetrahydrofuran-2-ylmethyl)-1,2,3,4-tetrahydro-thieno[2,3-d]pyrimidin-6-yl]methylen}hydrazincarboxylat (*Racemat*)

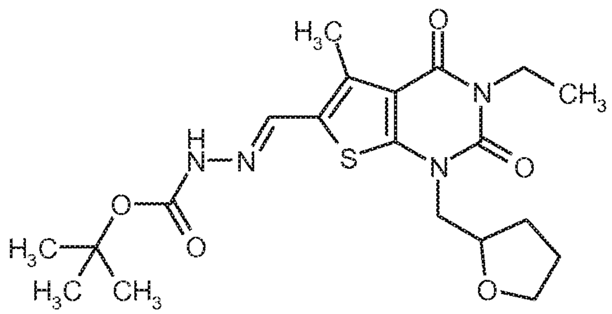

Eine Lösung von 1.10 g (3.41 mmol) der Verbindung aus Bsp. 89A in 25 ml Ethanol wurde zunächst mit 676 mg (5.12 mmol) *tert.*-Butyl-hydrazincarboxylat und dann mit 5 Tropfen konzentrierter Salzsäure versetzt. Nachdem das Reaktionsgemisch ca. 18 h bei RT gerührt worden war, wurde der Großteil des Ethanols am Rotationsverdampfer entfernt. Der verbliebene Rückstand wurde mit 150 ml Wasser verdünnt und durch Zusatz von gesättigter wässriger Natriumhydrogencarbonat-Lösung neutralisiert. Der dabei ausgefallene Feststoff wurde abgesaugt, mit wenig Wasser gewaschen und im Hochvakuum getrocknet. Es wurden 1.49 g (99% d. Th.) der Titelverbindung erhalten.
$^1$H-NMR (400 MHz, DMSO-$d_6$, δ/ppm): 10.85 (breit, 1H), 8.29 (s, 1H), 4.29-4.16 (m, 1H), 4.09 (dd, 1H), 3.90 (q, 2H), 3.81-3.71 (m, 2H), 3.67-3.57 (m, 1H), 2.45 (s, 3H), 2.08-1.77 (m, 3H), 1.72-1.59 (m, 1H), 1.45 (s, 9H), 1.12 (t, 3H).
LC/MS (Methode 17, ESIpos): $R_t$ = 1.94 min, m/z = 437.18 $[M+H]^+$.

**Beispiel 336A**

*tert.*-Butyl-2-{[3-isopropyl-5-methyl-2,4-dioxo-1-(3,3,3-trifluorpropyl)-1,2,3,4-tetrahydrothieno-[2,3-d]pyrimidin-6-yl]methylen}hydrazincarboxylat

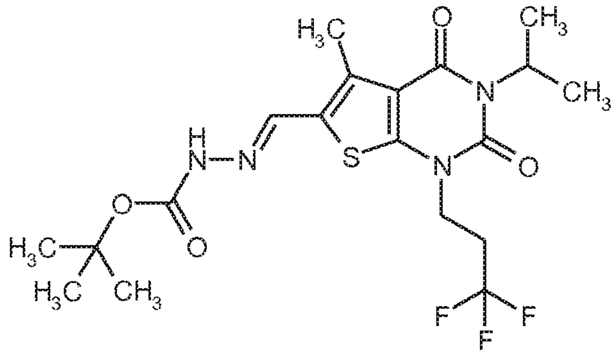

Eine Lösung von 1.02 g (2.93 mmol) der Verbindung aus Bsp. 103A in 25 ml Ethanol wurde zunächst mit 580 mg (4.39 mmol) *tert.*-Butyl-hydrazincarboxylat und dann mit 5 Tropfen konzentrierter Salzsäure versetzt. Nachdem das Reaktionsgemisch ca. 18 h bei RT gerührt worden war, wurde der Großteil des Ethanols am Rotationsverdampfer entfernt. Der verbliebene Rückstand wurde mit 150 ml Wasser verdünnt und durch Zusatz von gesättigter wässriger Natriumhydrogencarbonat-Lösung neutralisiert. Der dabei ausgefallene Feststoff wurde abgesaugt, mit wenig Wasser gewaschen und im Hochvakuum getrocknet. Es wurden 1.27 g (93% d. Th.) der Titelverbindung erhalten.
$^1$H-NMR (400 MHz, DMSO-$d_6$, δ/ppm): 10.87 (breit, 1H), 8.30 (s, 1H), 5.12 (sept, 1H), 4.12 (t, 2H), 2.89-2.69 (m, 2H), 2.45 (s, 3H), 1.45 (s, 9H), 1.40 (d, 6H).
LC/MS (Methode 17, ESIpos): $R_t$ = 2.23 min, m/z = 463.16 [M+H]$^+$.

**Beispiel 337A**

*tert.*-Butyl-2-{[3-isopropyl-1-(2-methoxyethyl)-5-methyl-2,4-dioxo-1,2,3,4-tetrahydrothieno-[2,3-d]pyrimidin-6-yl]methylen}hydrazincarboxylat

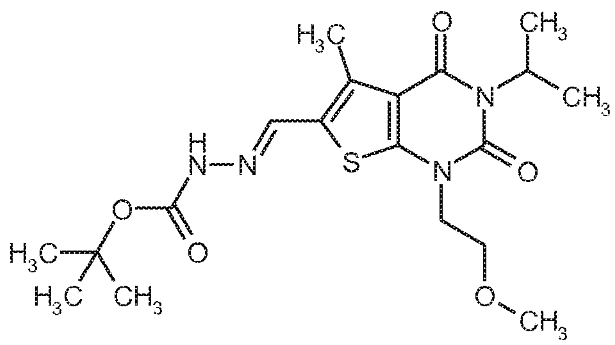

Analog zu dem unter Bsp. 333A beschriebenen Verfahren wurden aus 650 mg (2.09 mmol) der Verbindung aus Bsp. 105A und 415 mg (3.14 mmol) tert.-Butyl-hydrazincarboxylat 824 mg (92% d. Th.) der Titelverbindung erhalten.
$^1$H-NMR (400 MHz, DMSO-$d_6$, δ/ppm): 10.84 (breit, 1H), 8.28 (s, 1H), 5.12 (sept, 1H), 4.04 (t, 2H), 3.64 (t, 2H), 3.26 (s, 3H), 2.44 (s, 3H), 1.45 (s, 9H), 1.40 (d, 6H).
LC/MS (Methode 1, ESIpos): $R_t$ = 1.06 min, m/z = 425 [M+H]$^+$.

**Beispiel 338A**

*tert.*-Butyl-2-{[3-isopropyl-5-methyl-2,4-dioxo-1-(tetrahydrofuran-2-ylmethyl)-1,2,3,4-tetrahydro-thieno[2,3-d]pyrimidin-6-yl]methylen}hydrazincarboxylat (*Racemat*)

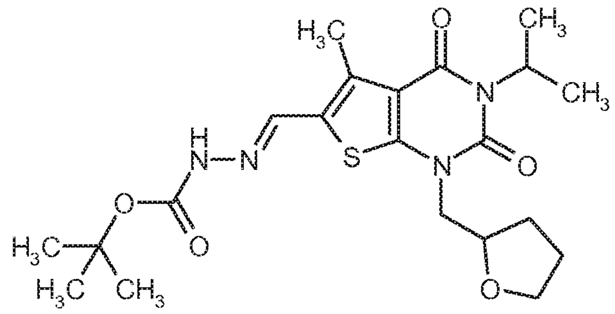

Analog zu dem unter Bsp. 333A beschriebenen Verfahren wurden aus 900 mg (2.67 mmol) der Verbindung aus Bsp. 107A und 530 mg (4.01 mmol) *tert.*-Butyl-hydrazincarboxylat 1.17 g (87% d. Th., 90% Reinheit) der Titelverbindung erhalten, welche ohne weitere Aufreinigung für Folgereaktionen verwendet wurden.
$^1$H-NMR (400 MHz, DMSO-$d_6$, δ/ppm): 10.84 (breit, 1H), 8.29 (s, 1H), 5.13 (sept, 1H), 4.27-4.17 (m, 1H), 4.08 (dd, 1H), 3.82-3.67 (m, 2H), 3.66-3.58 (m, 1H), 2.44 (s, 3H), 2.08-1.73 (m, 3H), 1.71-1.59 (m, 1H), 1.45 (s, 9H), 1.40 (d, 6H).
LC/MS (Methode 1, ESIpos): $R_t$ = 1.11 min, m/z = 451 $[M+H]^+$.

**Beispiel 339A**

*tert.*-Butyl-2-{[3-ethyl-5-methyl-2,4-dioxo-1-(3,3,3-trifluorpropyl)-1,2,3,4-tetrahydrothieno[2,3-d]-pyrimidin-6-yl]methyl}hydrazincarboxylat

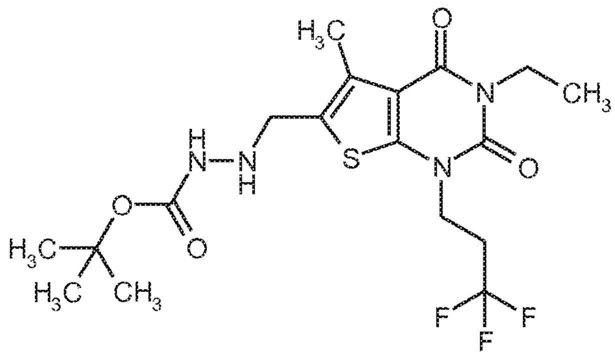

Eine Lösung von 6.50 g (14.5 mmol) der Verbindung aus Bsp. 333A in 150 ml Methanol wurde mit 4.55 g (72.5 mmol) Natriumcyanoborhydrid und etwas Bromkresolgrün versetzt. Anschließend wurde soviel Essigsäure hinzutitriert, dass die Indikatorfarbe gerade von blau nach gelb umschlug. Dann wurde das Reaktionsgemisch auf 65°C erwärmt. Nach 1 h, nach 3 h und nach 4 h wurden jeweils weitere 2.28 g (36.2 mmol) Natriumcyanoborhydrid hinzugefügt. Während der gesamten Reaktionszeit wurde durch Zusatz von weiterer Essigsäure der pH-Wert immer wieder so reguliert, dass die Indikatorfarbe gerade gelb blieb. Nach insgesamt 8 h wurden die flüchtigen Bestandteile des Reaktionsgemisches weitgehend am Rotationsverdampfer entfernt. Der verbliebene Rückstand wurde in Ethylacetat aufgenommen und nacheinander mit gesättigter wässriger Natriumhydrogencarbonat-Lösung, Wasser und gesättigter wässriger Natriumchlorid-Lösung gewaschen. Nach Trocknen über wasserfreiem Magnesiumsulfat wurde filtriert und zur Trockene eingeengt. Das Produkt wurde mittels Chromatographie isoliert (Biotage Isolera One, Kartusche SNAP KP-Sil, 340 g Kieselgel, Laufmittel Cyclohexan/Ethylacetat 2:1). Die Produktfraktionen wurden vereinigt, gegen 1 M Natronlauge ausgeschüttelt und nach Phasentrennung wieder eingeengt. Nach Trocknen im Hochvakuum wurden 5.23 g (80% d. Th.) der Titelverbindung erhalten.

$^1H$-NMR (400 MHz, DMSO-$d_6$, δ/ppm): 8.25 (br. s, 1H), 5.07 (br. d, 1H), 4.13 (t, 2H), 3.99 (d, 2H), 3.91 (q, 2H), 2.86-2.68 (m, 2H), 2.33 (s, 3H), 1.39 (s, 9H), 1.11 (t, 3H).
LC/MS (Methode 1, ESIpos): $R_t$ = 1.07 min, m/z = 451 $[M+H]^+$.

**Beispiel 340A**

*tert*-Butyl-2-{[3-ethyl-1-(2-methoxyethyl)-5-methyl-2,4-dioxo-1,2,3,4-tetrahydrothieno[2,3-d]-pyrimidin-6-yl]methyl}hydrazincarboxylat

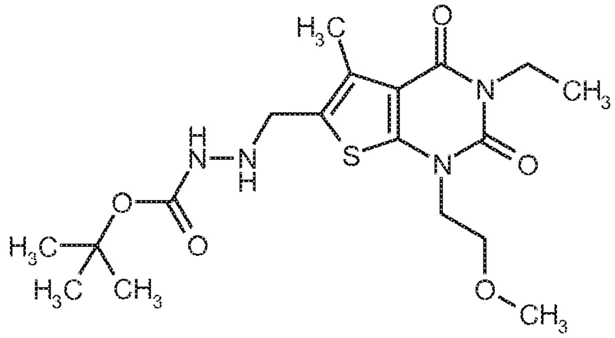

Eine Lösung von 1.34 g (3.26 mmol) der Verbindung aus Bsp. 334A in 33 ml Methanol wurde mit 1.03 g (16.3 mmol) Natriumcyanoborhydrid und etwas Bromkresolgrün versetzt. Anschließend wurde soviel Essigsäure hinzutitriert, dass die Indikatorfarbe gerade von blau nach gelb umschlug. Dann wurde das Reaktionsgemisch auf 65°C erwärmt. Nach 1 h, nach 3 h und nach 4 h wurden jeweils weitere 0.52 g (8.2 mmol) Natriumcyanoborhydrid hinzugefügt. Während der gesamten Reaktionszeit wurde durch Zusatz von weiterer Essigsäure der pH-Wert immer wieder so reguliert, dass die Indikatorfarbe gerade gelb blieb. Nach insgesamt 5 h wurden die flüchtigen Bestandteile des Reaktionsgemisches weitgehend am Rotationsverdampfer entfernt. Der verbliebene Rückstand wurde in Ethylacetat aufgenommen und nacheinander mit gesättigter wässriger Natriumhydrogencarbonat-Lösung, Wasser und gesättigter wässriger Natriumchlorid-Lösung gewaschen. Nach Trocknen über wasserfreiem Magnesiumsulfat wurde filtriert und zur Trockene eingeengt. Das Rohprodukt wurde mit Acetonitril verrührt. Der Feststoff wurde abgesaugt und im Hochvakuum getrocknet. Dies ergab eine erste Fraktion von 810 mg der Titelverbindung. Aus der Mutterlauge des Verrührens wurden mittels präparativer HPLC (Methode 8) weitere 166 mg des Produkts gewonnen. Insgesamt wurden so 976 mg (72% d. Th.) der Titelverbindung erhalten.
$^1H$-NMR (400 MHz, DMSO-$d_6$, δ/ppm): 8.25 (br. s, 1H), 4.99 (br. s, 1H), 4.04 (t, 2H), 3.97 (d, 2H), 3.90 (q, 2H), 3.65 (t, 2H), 3.25 (s, 3H), 2.33 (s, 3H), 1.39 (s, 9H), 1.11 (t, 3H).
LC/MS (Methode 1, ESIpos): $R_t$ = 0.92 min, m/z = 413 $[M+H]^+$, 281 $[M+H-C_5H_{12}N_2O_2]^+$.

**Beispiel 341A**

*tert.*-Butyl-2-{[3-ethyl-5-methyl-2,4-dioxo-1-(tetrahydrofuran-2-ylmethyl)-1,2,3,4-tetrahydro-thieno[2,3-d]pyrimidin-6-yl]methyl}hydrazincarboxylat *(Racemat)*

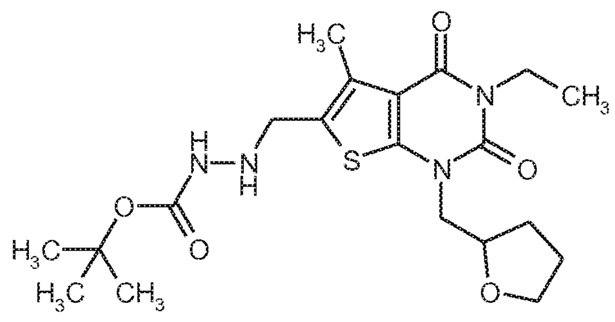

Eine Lösung von 1.40 g (3.21 mmol) der Verbindung aus Bsp. 335A in 32 ml Methanol wurde mit 1.01 g (16.0 mmol) Natriumcyanoborhydrid und etwas Bromkresolgrün versetzt. Anschließend wurde soviel Essigsäure hinzutitriert, dass die Indikatorfarbe gerade von blau nach gelb umschlug. Dann wurde das Reaktionsgemisch auf 65°C erwärmt. Nach 1 h, nach 3 h und nach 4 h wurden jeweils weitere 0.50 g (8.0 mmol) Natriumcyanoborhydrid hinzugefügt. Während der gesamten Reaktionszeit wurde durch Zusatz von weiterer Essigsäure der pH-Wert immer wieder so reguliert, dass die Indikatorfarbe gerade gelb blieb. Nach insgesamt 5 h wurden die flüchtigen Bestandteile des Reaktionsgemisches weitgehend am Rotationsverdampfer entfernt. Der verbliebene Rückstand wurde in Ethylacetat aufgenommen und nacheinander mit gesättigter wässriger Natriumhydrogencarbonat-Lösung, Wasser und gesättigter wässriger Natriumchlorid-Lösung gewaschen. Nach Trocknen über wasserfreiem Magnesiumsulfat wurde filtriert und zur Trockene eingeengt. Das Rohprodukt wurde mit Acetonitril verrührt. Der Feststoff wurde abgesaugt und im Hochvakuum getrocknet. Es wurden 900 mg (64% d. Th.) der Titelverbindung erhalten.
$^1$H-NMR (400 MHz, DMSO-$d_6$, δ/ppm): 8.26 (br. s, 1H), 4.98 (br. s, 1H), 4.31-4.21 (m, 1H), 4.06-3.85 (m, 5H), 3.83-3.68 (m, 2H), 3.62 (q, 1H), 2.33 (s, 3H), 2.05-1.75 (m, 3H), 1.73-1.61 (m, 1H), 1.39 (s, 9H), 1.11 (t, 3H).
LC/MS (Methode 1, ESIpos): $R_t$ = 0.95 min, m/z = 307 $[M+H-C_5H_{12}N_2O_2]^+$.

**Beispiel 342A**

*tert.*-Butyl-2-{[3-isopropyl-5-methyl-2,4-dioxo-1-(3,3,3-trifluorpropyl)-1,2,3,4-tetrahydrothieno-[2,3-d]pyrimidin-6-yl]methyl}hydrazincarboxylat

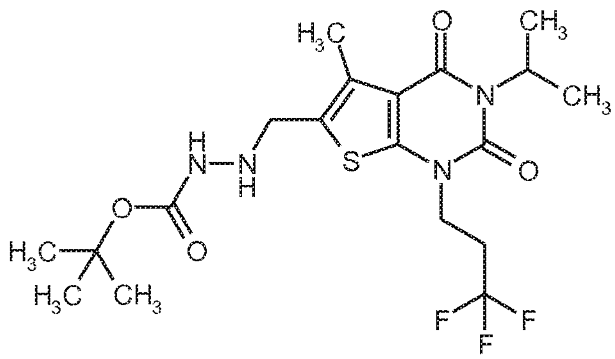

Eine Lösung von 1.24 g (2.68 mmol) der Verbindung aus Bsp. 336A in 25 ml Methanol wurde mit 842 mg (13.4 mmol) Natriumcyanoborhydrid und etwas Bromkresolgrün versetzt. Anschließend wurde soviel Essigsäure hinzutitriert, dass die Indikatorfarbe gerade von blau nach gelb umschlug. Dann wurde das Reaktionsgemisch auf 65°C erwärmt. Nach 1 h, nach 3 h und nach 4 h wurden jeweils weitere 421 mg (6.7 mmol) Natriumcyanoborhydrid hinzugefügt. Während der gesamten Reaktionszeit wurde durch Zusatz von weiterer Essigsäure der pH-Wert immer wieder so reguliert, dass die Indikatorfarbe gerade gelb blieb. Nach insgesamt 5 h wurden die flüchtigen Bestandteile des Reaktionsgemisches weitgehend am Rotationsverdampfer entfernt. Der verbliebene Rückstand wurde in Ethylacetat aufgenommen und nacheinander mit gesättigter wässriger Natriumhydrogencarbonat-Lösung, Wasser und gesättigter wässriger Natriumchlorid-Lösung gewaschen. Nach Trocknen über wasserfreiem Magnesiumsulfat wurde filtriert und zur Trockene eingeengt. Das Produkt wurde mittels Chromatographie isoliert (Biotage Isolera One, Kartusche SNAP KP-Sil, 100 g Kieselgel, Laufmittel Cyclohexan/Ethylacetat 2:1). Nach Vereinigen der Produktfraktionen, Einengen und Trocknen im Hochvakuum wurden 1.07 g (85% d. Th.) der Titelverbindung erhalten.
$^1$H-NMR (400 MHz, DMSO-$d_6$, δ/ppm): 8.24 (br. s, 1H), 5.13 (sept, 1H), 5.05 (br. s, 1H), 4.09 (t, 2H), 3.98 (d, 2H), 2.85-2.66 (m, 2H), 2.32 (s, 3H), 1.44-1.34 (m, 15H).
LC/MS (Methode 1, ESIpos): $R_t$ = 1.12 min, m/z = 333 $[M+H-C_5H_{12}N_2O_2]^+$.

**Beispiel 343A**

*tert.*-Butyl-2-{[3-isopropyl-1-(2-methoxyethyl)-5-methyl-2,4-dioxo-1,2,3,4-tetrahydrothieno-[2,3-d]pyrimidin-6-yl]methyl}hydrazincarboxylat

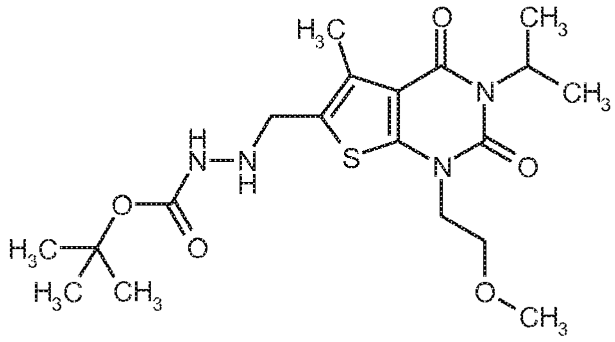

Eine Lösung von 1.08 g (2.19 mmol) der Verbindung aus Bsp. 337A in 25 ml Methanol wurde mit 687 mg (10.9 mmol) Natriumcyanoborhydrid und etwas Bromkresolgrün versetzt. Anschließend wurde soviel Essigsäure hinzutitriert, dass die Indikatorfarbe gerade von blau nach gelb umschlug. Dann wurde das Reaktionsgemisch auf 65°C erwärmt. Nach 1 h wurden weitere 343 mg (5.45 mmol) Natriumcyanoborhydrid hinzugefügt. Während der gesamten Reaktionszeit wurde durch Zusatz von weiterer Essigsäure der pH-Wert immer wieder so reguliert, dass die Indikatorfarbe gerade gelb blieb. Nach insgesamt 4 h wurden die flüchtigen Bestandteile des Reaktionsgemisches weitgehend am Rotationsverdampfer entfernt. Der verbliebene Rückstand wurde in Ethylacetat aufgenommen und nacheinander mit gesättigter wässriger Natriumhydrogencarbonat-Lösung, Wasser und gesättigter wässriger Natriumchlorid-Lösung gewaschen. Nach Trocknen über wasserfreiem Magnesiumsulfat wurde filtriert und zur Trockene eingeengt. Das Produkt wurde mittels Chromatographie isoliert (Biotage Isolera One, Kartusche SNAP KP-Sil, 100 g Kieselgel, Laufmittel Cyclohexan/Ethylacetat 1:1). Nach Vereinigen der Produktfraktionen, Einengen und Trocknen im Hochvakuum wurden 800 mg (85% d. Th.) der Titelverbindung erhalten.

$^1$H-NMR (400 MHz, DMSO-$d_6$, δ/ppm): 8.24 (br. s, 1H), 5.14 (sept, 1H), 4.97 (br. d, 1H), 4.00 (t, 2H), 3.96 (d, 2H), 3.63 (t, 2H), 3.26 (s, 3H, weitgehend überdeckt vom Wassersignal), 2.31 (s, 3H), 1.46-1.34 (m, 15H).

LC/MS (Methode 1, ESIpos): $R_t$ = 1.04 min, m/z = 427 $[M+H]^+$, 295 $[M+H-C_5H_{12}N_2O_2]^+$.

**Beispiel 344A**

*tert.*-Butyl-2-{[3-isopropyl-5-methyl-2,4-dioxo-1-{tetrahydrofuran-2-ylmethyl)-1,2,3,4-tetrahydro-thieno[2,3-d]pyrimidin-6-yl]methyl}hydrazincarboxylat (*Racemat*)

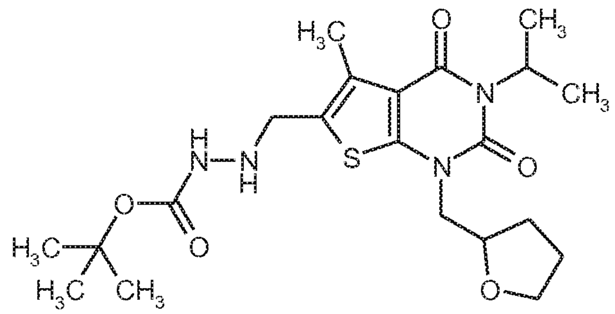

Analog zu dem unter Bsp. 343A beschriebenen Verfahren wurden aus 1.15 g (2.55 mmol) der Verbindung aus Bsp. 338A und insgesamt 1.20 g (19.1 mmol) Natriumcyanoborhydrid 876 mg (73% d. Th., 97% Reinheit) der Titelverbindung erhalten. Die Reaktionszeit betrug hier 3 h.

$^1$H-NMR (400 MHz, DMSO-$d_6$, δ/ppm): 8.24 (br. s, 1H), 5.23-5.06 (sept, 1H), 4.95 (br. s, 1H), 4.31-4.18 (m, 1H), 4.04-3.92 (m, 3H), 3.82-3.74 (m, 1H), 3.72-3.55 (m, 2H), 2.32 (s, 3H), 2.06-1.76 (m, 3H), 1.72-1.60 (m, 1H), 1.43-1.34 (m, 15H).

LC/MS (Methode 17, ESIpos): $R_t$ = 2.03 min, m/z = 453.22 $[M+H]^+$, 321.13 $[M+H-C_5H_{12}N_2O_2]^+$.

**Beispiel 345A**

*tert.*-Butyl-2-{3-ethoxyprop-2-enoyl)-2-{[3-ethyl-5-methyl-2,4-dioxo-1-(3,3,3-trifluorpropyl)-1,2,3,4-tetrahydrothieno[2,3-d]pyrimidin-6-yl]methyl}hydrazincarboxylat

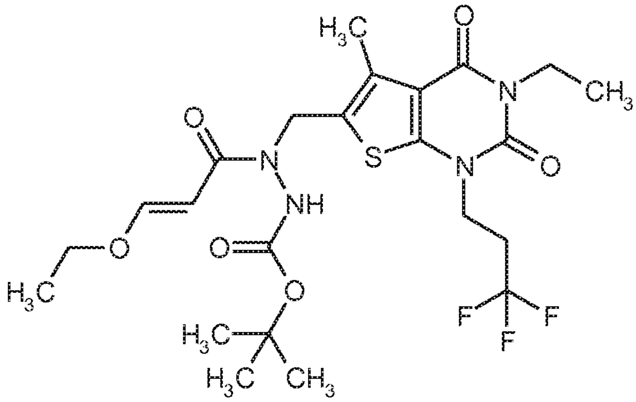

Eine Lösung von 100 mg (0.222 mmol) der Verbindung aus Bsp. 339A in 3 ml Dichlormethan wurde bei 0°C mit 50 µl (0.289 mmol) *N,N*-Diisopropylethylamin und 35 mg (0.222 mmol, Gehalt 85%) 3-Ethoxyacryloylchlorid versetzt. Dann wurde das Kältebad entfernt und das Reaktionsgemisch ca. 18 h bei RT gerührt. Nach dieser Zeit wurden weitere 116 µl (0.667 mmol) *N,N*-Diisopropylethylamin und 105 mg (0.666 mmol, Gehalt 85%) 3-Ethoxyacryloylchlorid hinzugefügt. Nach weiteren 3 h bei RT wurde das Reaktionsgemisch mit Dichlormethan verdünnt und mit Wasser gewaschen. Nach Trocknen der organischen Phase über wasserfreiem Magnesiumsulfat wurde filtriert und eingeengt. Das Produkt wurde mittels präparativer HPLC isoliert (Methode 8). Nach Einengen der Produktfraktionen und Trocknen im Hochvakuum wurden 108 mg (88% d. Th.) der Titelverbindung erhalten.
LC/MS (Methode 1, ESIneg): $R_t$ = 1.13 min, m/z = 547 $[M-H]^-$.

**Beispiel 346A**

*tert.*-Butyl-2-(3-ethoxyprop-2-enoyl)-2-{[3-ethyl-1-(2-methoxyethyl)-5-methyl-2,4-dioxo-1,2,3,4-tetrahydrothieno[2,3-d]pyrimidin-6-yl]methyl}hydrazincarboxylat

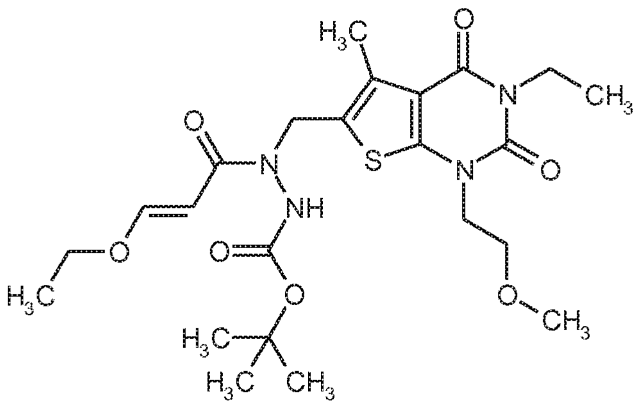

Eine Lösung von 300 mg (0.727 mmol) der Verbindung aus Bsp. 340A in 8 ml Dichlormethan wurde bei 0°C mit 165 µl (0.945 mmol) *N,N*-Diisopropylethylamin und 138 mg (0.873 mmol, Gehalt 85%) 3-Ethoxyacryloylchlorid versetzt. Dann wurde das Kältebad entfernt und das Reaktionsgemisch ca. 18 h bei RT gerührt. Anschließend wurde das Reaktionsgemisch mit Dichlormethan verdünnt und mit Wasser gewaschen. Nach Trocknen der organischen Phase über wasserfreiem Magnesiumsulfat wurde filtriert und eingeengt. Das Produkt wurde mittels präparativer HPLC isoliert (Methode 8). Nach Einengen der Produktfraktionen und Trocknen im Hochvakuum wurden 278 mg (73% d. Th., 97%

Reinheit) der Titelverbindung erhalten.
$^1$H-NMR (400 MHz, DMSO-$d_6$, δ/ppm): 9.48 (br. s, 1H), 7.50 (d, 1H), 5.57 (d, 1H), 5.04-4.34 (m, 2H), 4.03 (t, 2H), 3.97-3.84 (m, 4H), 3.63 (t, 2H), 3.24 (s, 3H), 2.36 (s, 3H), 1.39 (s, 9H), 1.23 (t, 3H), 1.11 (t, 3H).
LC/MS (Methode 1, ESIneg): $R_t$ = 1.13 min, m/z = 509 [M-H]$^-$.

## Beispiel 347A

*tert.*-Butyl-2-(-3-ethoxyprop-2-enoyl)-2-{[3-ethyl-5-methyl-2,4-dioxo-1-(tetrahydrofuran-2-yl-methyl)-1,2,3,4-tetrahydrothieno[2,3-d]pyrimidin-6-yl]methyl}hydrazincarboxylat (*Racemat*)

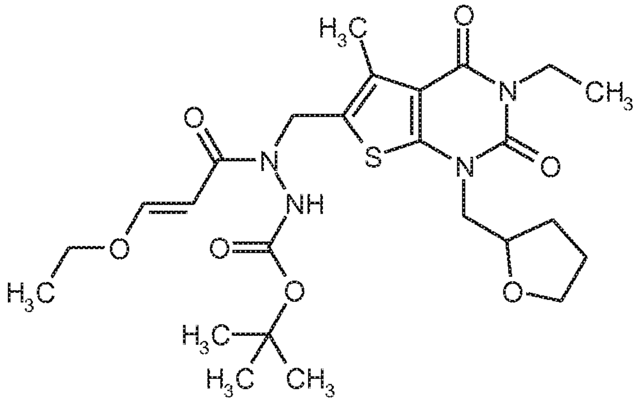

Eine Lösung von 500 mg (1.14 mmol) der Verbindung aus Bsp. 341A in 12 ml Dichlormethan wurde bei 0°C mit 258 µl (1.48 mmol) *N,N*-Diisopropylethylamin und 217 mg (1.37 mmol, Gehalt 85%) 3-Ethoxyacryloylchlorid versetzt. Dann wurde das Kältebad entfernt und das Reaktionsgemisch ca. 18 h bei RT gerührt. Anschließend wurde das Reaktionsgemisch mit Dichlormethan verdünnt und mit Wasser gewaschen. Nach Trocknen der organischen Phase über wasserfreiem Magnesiumsulfat wurde filtriert und eingeengt. Das Produkt wurde mittels präparativer HPLC isoliert (Methode 8). Nach Einengen der Produktfraktionen und Trocknen im Hochvakuum wurden 378 mg (61% d. Th.) der Titelverbindung erhalten.
$^1$H-NMR (400 MHz, DMSO-$d_6$, δ/ppm): 9.48 (br. s, 1H), 7.49 (d, 1H), 5.57 (d, 1H), 5.07-4.32 (m, 2H), 4.28-4.19 (m, 1H), 4.06-3.83 (m, 5H), 3.81-3.67 (m, 2H), 3.66-3.53 (m, 1H), 2.36 (s, 3H), 2.02-1.75 (m, 3H), 1.73-1.61 (m, 1H), 1.39 (s, 9H), 1.23 (t, 3H), 1.11 (t, 3H).
LC/MS (Methode 1, ESIneg): Rt = 1.08 min, m/z = 535 [M-H]$^-$.

## Beispiel 348A

*tert.*-Butyl-2-(3-ethoxyprop-2-enoyl)-2-{[3-isopropyl-5-methyl-2,4-dioxo-1-(3,3,3-trifluorpropyl)-1,2,3,4-tetrahydrothieno[2,3-d]pyrimidin-6-yl]methyl}hydrazincarboxylat

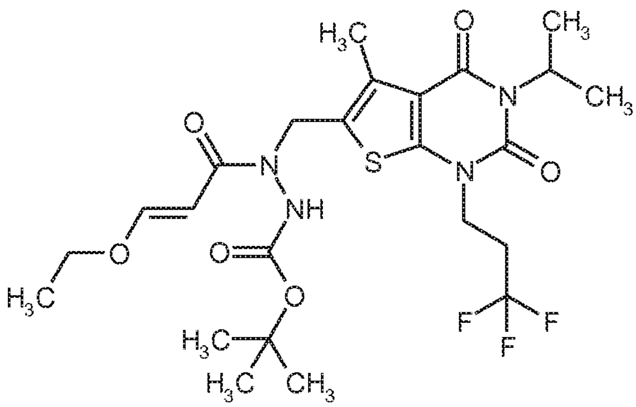

Eine Lösung von 300 mg (0.646 mmol) der Verbindung aus Bsp. 342A in 7 ml Dichlormethan wurde bei 0°C mit 146 µl (0.840 mmol) *N,N*-Diisopropylethylamin und 123 mg (0.775 mmol, Gehalt 85%) 3-Ethoxyacryloylchlorid versetzt. Dann wurde das Kältebad entfernt und das Reaktionsgemisch ca. 18 h bei RT gerührt. Anschließend wurde das Reaktionsgemisch mit Dichlormethan verdünnt und mit Wasser gewaschen. Nach Trocknen der organischen Phase über wasserfreiem Magnesiumsulfat wurde filtriert und eingeengt. Das Produkt wurde mittels präparativer HPLC isoliert (Methode 8). Nach Einengen der Produktfraktionen und Trocknen im Hochvakuum wurden 278 mg (76% d. Th.) der Titelverbindung erhalten.
$^1$H-NMR (400 MHz, DMSO-$d_6$, δ/ppm): 9.46 (br. s, 1H), 7.50 (d, 1H), 5.57 (d, 1H), 5.13 (sept, 1H), 5.00-4.34 (m, 2H), 4.09 (t, 2H), 3.93 (m, 2H), 2.83-2.62 (m, 2H), 2.36 (s, 3H), 1.43-1.35 (m, 15H), 1.23 (t, 3H).
LC/MS (Methode 17, ESIneg): $R_t$ = 2.23 min, m/z = 561.20 $[M-H]^-$.

## Beispiel 349A

*tert.*-Butyl-2-(3-ethoxyprop-2-enoyl)-2-([3-isopropyl-1-(2-methoxyethyl)-5-methy1-2,4-dioxo-1,2,3,4-tetrahydro-thieno[2,3-d]pyrimidin-6-yl]methyl} hydrazincarboxylat

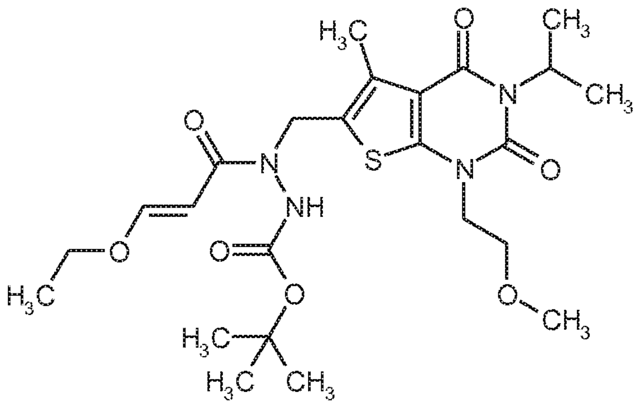

Analog zu dem unter Bsp. 348A beschriebenen Verfahren wurden aus 300 mg (0.703 mmol) der Verbindung aus Bsp. 343A und 134 mg (0.844 mmol, 85% Gehalt) 3-Ethoxyacryloylchlorid 293 mg (79% d. Th.) der Titelverbindung erhalten.
$^1$H-NMR (400 MHz, DMSO-$d_6$, δ/ppm): 9.46 (br. s, 1H), 7.50 (d, 1H), 5.57 (d, 1H), 5.13 (sept, 1H), 5.02-4.33 (m, 2H), 3.99 (t, 2H), 3.93 (m, 2H), 3.62 (t, 2H), 3.24 (s, 3H), 2.34 (s, 3H), 1.39 (m, 15H), 1.23 (t, 3H).
LC/MS (Methode 1, ESIneg): $R_t$ = 1.08 min, m/z = 523 $[M-H]^-$.

## Beispiel 350A

*tert.*-Butyl-2-(3-etboxyprop-2-enoyl)-2-{[3-isopropyl-5-methyl-2,4-dioxo-1-{tetrahydrofuran-2-yl-methyl)-1,2,3,4-tetrahydrothieno[2,3-d]pyrimidin-6-yl]methyllhydrazincarboxylat (*Racemat*)

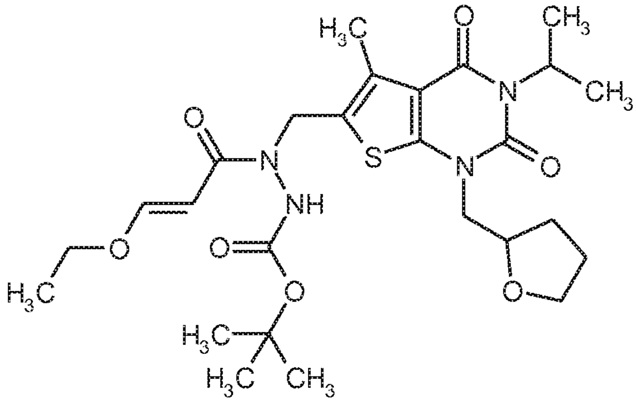

Analog zu dem unter Bsp. 348A beschriebenen Verfahren wurden aus 500 mg (1.11 mmol) der Verbindung aus Bsp. 344A und 210 mg (1.33 mmol, 85% Gehalt) 3-Ethoxyacryloylchlorid 514 mg (84% d. Th.) der Titelverbindung erhalten.
$^1$H-NMR (400 MHz, DMSO-$d_6$, δ/ppm): 9.46 (br. s, 1H), 7.49 (d, 1H), 5.57 (d, 1H), 5.14 (sept, 1H), 5.07-4.32 (m, 2H), 4.28-4.15 (m, 1H), 4.06-3.86 (m, 3H), 3.82-3.53 (m, 3H), 2.34 (s, 3H), 2.04-1.75 (m, 3H), 1.73-1.58 (m, 1H), 1.39 (m, 15H), 1.23 (t, 3H).
LC/MS (Methode 1, ESIneg): $R_t$ = 1.13 min, m/z = 549 $[M-H]^-$.

**Beispiel 351A**

6-(Hydrazinomethyl)-3-isopropyl-5-methyl-1-(3,3,3-trifluorpropyl)thieno[2,3-d]pyrimidin-2,4(1*H*,3*H*)-dion

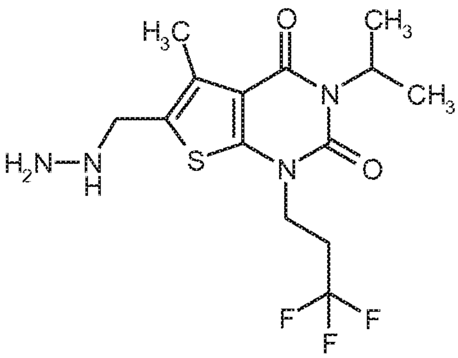

1.55 g (3.34 mmol) der Verbindung aus Bsp. 342A wurden in 30 ml Dichlormethan gelöst und bei 0°C mit 15 ml Trifluoressigsäure versetzt. Das Reaktionsgemisch wurde zunächst 90 min bei 0°C und dann 60 min bei RT gerührt. Anschließend wurden alle flüchtigen Bestandteile bei RT am Rotationsverdampfer entfernt. Der verbliebene Rückstand wurde mittels präparativer HPLC gereinigt (Methode 8). Nach Einengen der Produktfraktionen und Trocknen im Hochvakuum wurden 647 mg (51% d. Th., 96% Reinheit) der Titelverbindung erhalten.
$^1$H-NMR (400 MHz, DMSO-$d_6$, δ/ppm): 8.46-7.32 (m, 3H), 5.14 (sept, 1H), 4.11 (t, 2H), 4.10 (s, 2H), 2.86-2.67 (m, 2H), 2.39 (s, 3H), 1.40 (d, 6H).
LC/MS (Methode 1, ESIpos): $R_t$ = 0.61 min, m/z = 333 $[M+H-N_2H_4]^+$.

**Beispiel 352A**

6-(Hydrazinomethyl)-3-isopropyl-1-(2-methoxyethyl)-5-methylthieno[2,3-d]pyrimidin-2,4(1*H*,3*H*)-dion

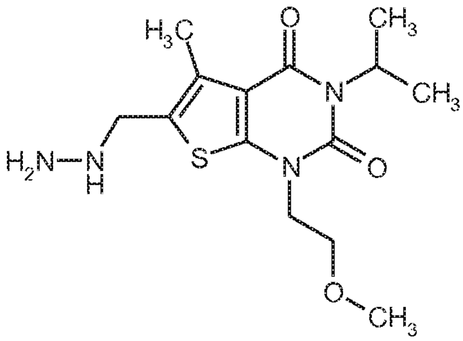

800 mg (1.88 mmol) der Verbindung aus Bsp. 343A wurden in 16 ml Dichlormethan gelöst und bei 0°C mit 8 ml Trifluoressigsäure versetzt. Das Reaktionsgemisch wurde 10 min bei 0°C gerührt. Dann wurden alle flüchtigen Bestandteile bei RT am Rotationsverdampfer entfernt. Der verbliebene Rückstand wurde mittels präparativer HPLC gereinigt (Methode 8). Nach Einengen der Produktfraktionen und Trocknen im Hochvakuum wurden 235 mg (38% d. Th.) der Titelverbindung erhalten.
$^1$H-NMR (400 MHz, DMSO-$d_6$, $\delta$/ppm): 9.09 (breit, 2H), 5.83 (breit, 1H), 5.14 (sept, 1H), 4.10 (breit, 2H), 4.02 (t, 2H), 3.64 (t, 2H), 3.25 (s, 3H), 2.38 (s, 3H), 1.40 (d, 6H).
LC/MS (Methode 1, ESIpos): $R_t$ = 0.50 min, m/z = 295 $[M+H-N_2H_4]^+$.

## Beispiel 353A

({[3-Isopropyl-5-methyl-2,4-dioxo-1-(3,3,3-trifluorpropyl)-1,2,3,4-tetrahydrothieno[2,3-d]pyrimidin-6-yl]methyl}hydrazono)essigsäure

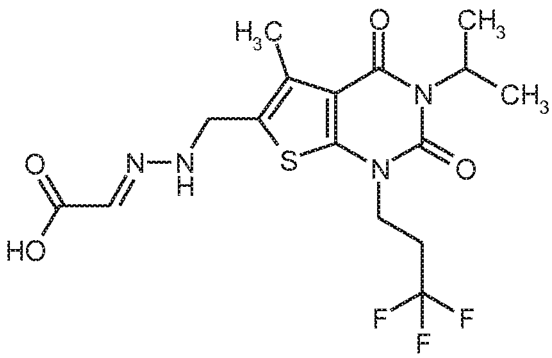

Bei 0°C wurden 190 µl (1.72 mmol) Glyoxalsäure zu einer Lösung von 647 mg (1.72 mmol, 97% Reinheit) der Verbindung aus Bsp. 351A in 13 ml Wasser und 2.6 ml (2.58 mmol) 1 M Salzsäure hinzugetropft. Nachdem das Reaktionsgemisch 1 h bei 10-20°C gerührt worden war, wurde der ausgefallene Niederschlag abgesaugt, mit wenig Wasser gewaschen und im Hochvakuum getrocknet. Dies ergab 495 mg (68% d. Th.) der Titelverbindung.
$^1$H-NMR (400 MHz, DMSO-$d_6$, $\delta$/ppm): 11.91 (breit, 1H), 8.97 (t, 1H), 6.73 (s, 1H), 5.13 (sept, 1H), 4.50 (d, 2H), 4.07 (t, 2H), 2.86-2.62 (m, 2H), 2.39 (s, 3H), 1.40 (d, 6H).
LC/MS (Methode 1, ESIneg): $R_t$ = 0.91 min, m/z = 419 $[M-H]^-$.

## Beispiel 354A

({[3-Isopropyl-1-(2-methoxyethyl)-5-methyl-2,4-dioxo-1,2,3,4-tetrahydrothieno[2,3-d]pyrimidin-6-yl]methyl}hydrazono)essigsäure

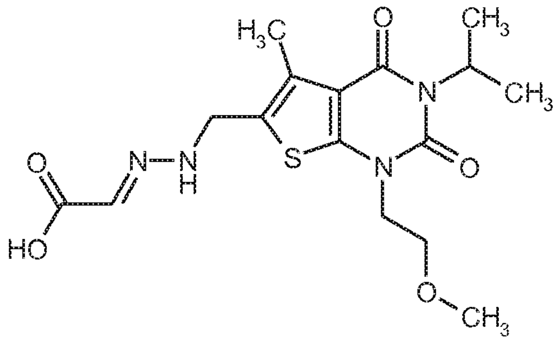

Analog zu dem unter Bsp. 353A beschriebenen Verfahren wurden aus 220 mg (0.674 mmol) der Verbindung aus Bsp. 352A und 74 µl (0.674 mmol) Glyoxalsäure 89 mg (34% d. Th.) der Titelverbindung hergestellt. In diesem Fall erfolgte noch eine zusätzliche Aufreinigung des Produkts mittels präparativer HPLC (Methode 8).
$^1$H-NMR (400 MHz, DMSO-$d_6$, δ/ppm): 11.82 (breit, 1H), 8.95 (t, 1H), 6.72 (s, 1H), 5.13 (sept, 1H), 4.47 (d, 2H), 3.99 (t, 2H), 3.62 (t, 2H), 3.23 (s, 3H), 2.37 (s, 3H), 1.40 (d, 6H).
LC/MS (Methode 1, ESIneg): $R_t$ = 0.75 min, m/z = 381 $[M-H]^-$.

**Beispiel 355A**

Ethyl-{5-methyl-2,4-dioxo-6-[(2-oxoimidazolidin-1-yl)methyl]-1-(3,3,3-trifluorpropyl)-1,4-dihydrothieno[2,3-d]pyrimidin-3(2*H*)-yl}acetat

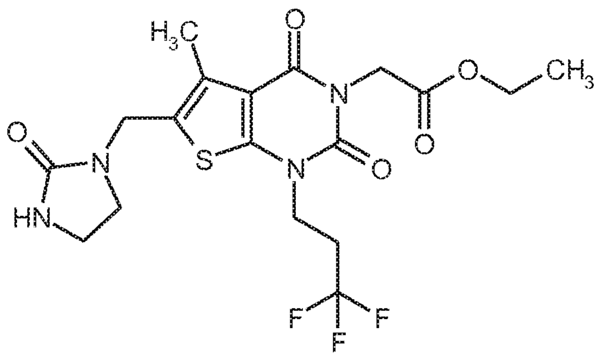

Eine Lösung von 218 mg (2.54 mmol) Imidazolidin-2-on in 6 ml DMF wurde mit 101 mg (2.54 mmol) Natriumhydrid (60% Suspension in Mineralöl) versetzt, dann 5 min auf 60°C erwärmt und anschließend wieder auf RT abgekühlt ("Lösung 1"). In einem anderen Reaktionsgefäß wurde eine Lösung von 250 mg (0.634 mmol) der Verbindung aus Bsp. 306A in 5 ml Dichlormethan bei 0°C mit 221 µl (1.27 mmol) *N,N*-Diisopropylethylamin und 49 µl (0.666 mmol) Thionylchlorid versetzt. Nach 20 min Rühren bei 0°C wurde Lösung 1 portionsweise hinzugefügt und anschließend das Kältebad entfernt. Das Reaktionsgemisch wurde ca. 18 h bei RT gerührt. Danach wurden alle flüchtigen Bestandteile am Rotationsverdampfer entfernt. Der verbliebene Rückstand wurde mittels präparativer HPLC (Methode 8) in seine Komponenten aufgetrennt. Nach Einengen der Produktfraktionen und Trocknen im Hochvakuum wurden 87 mg (28% d. Th., 97% Reinheit) der Titelverbindung erhalten.
$^1$H-NMR (400 MHz, DMSO-$d_6$, δ/ppm): 6.55 (s, 1H), 4.62 (s, 2H), 4.38 (s, 2H), 4.14 (q, 2H), 4.13 (t, 2H), 3.30-3.16 (m, 4H), 2.87-2.66 (m, 2H), 2.39 (s, 3H), 1.20 (t, 3H).
LC/MS (Methode 1, ESIpos): $R_t$ = 0.85 min, m/z = 463 $[M+H]^+$.

**Beispiel 356A**

Ethyl-[1-(2-metboxyethyl)-5-methyl-2,4-dioxo-6-[(2-oxoimidazolidin-1-yl)methyl]-1,4-dihydro-thieno[2,3-d]pyrimidin-3(2*H*)-yl]acetat

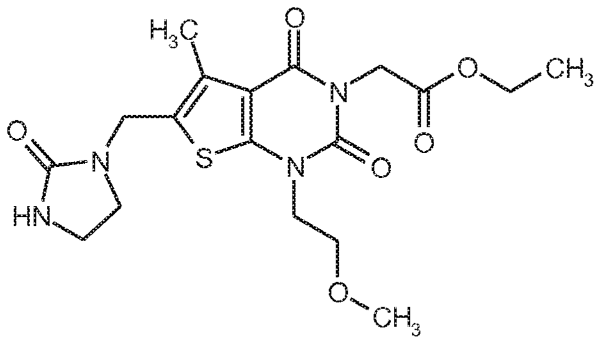

Analog zu dem unter Bsp. 355A beschriebenen Verfahren wurden aus 250 mg (0.701 mmol) der Verbindung aus Bsp. 307A und 242 mg (2.81 mmol) Imidazolidin-2-on 158 mg (50% d. Th., 95% Reinheit) der Titelverbindung erhalten.
$^1$H-NMR (400 MHz, DMSO-$d_6$, $\delta$/ppm): 6.54 (s, 1H), 4.61 (s, 2H), 4.36 (s, 2H), 4.14 (q, 2H), 4.05 (t, 2H), 3.63 (t, 2H), 3.29-3.16 (m, 4H), 3.23 (s, 3H), 2.38 (s, 3H), 1.20 (t, 3H).
LC/MS (Methode 1, ESIpos): $R_t$ = 0.72 min, m/z = 425 $[M+H]^+$.

**Beispiel 357A**

Prop-2-in-1-yl-1-(2-methoxyethyl)-5-methyl-2,4-dioxo-3-(prop-2-in-1-yl)-1,2,3,4-tetrahydrothieno-[2,3-d]pyrimidin-6-carboxylat

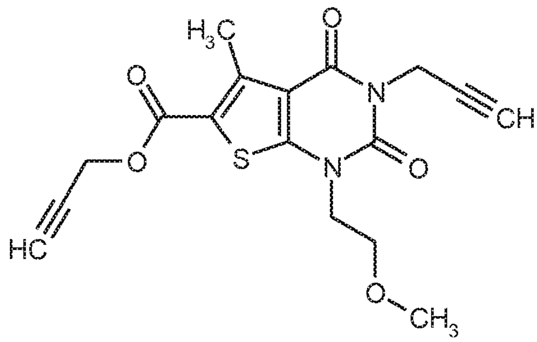

Analog zu dem unter Bsp. 20A beschriebenen Verfahren wurden aus 450 mg (1.43 mmol) der Verbindung aus Bsp. 18A und 635 mg (4.27 mmol) Propargylbromid 321 mg (59% d. Th.) der Titelverbindung erhalten. Die Reaktionszeit betrug hier 74 h.
$^1$H-NMR (400 MHz, DMSO-$d_6$, $\delta$/ppm): 4.93 (d, 2H), 4.60 (d, 2H), 4.12 (t, 2H), 3.69-3.63 (m, 3H), 3.24 (s, 3H), 3.18-3.15 (m, 1H), 2.78 (s, 3H).
LC/MS (Methode 3): $R_t$ = 1.13 min, m/z = 361 $[M+H]^+$.

**Beispiel 358A**

But-3-en-1-yl-3-(but-3-en-1-yl)-1-(2-methoxyethyl)-5-methyl-2,4-dioxo-1,2,3,4-tetrahydrothieno-[2,3-d]pyrimidin-6-carboxylat

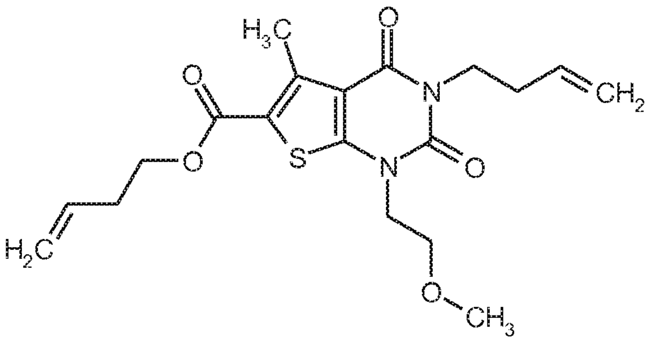

Analog zu dem unter Bsp. 20A beschriebenen Verfahren wurden aus 450 mg (1.43 mmol) der Verbindung aus Bsp. 18A und 594 mg (4.27 mmol) 1-Brom-3-buten 335 mg (60% d. Th.) der Titelverbindung erhalten. Die Reaktionszeit betrug hier 16 h.
$^1$H-NMR (400 MHz, DMSO-$d_6$, $\delta$/ppm): 5.90-5.73 (m, 1H), 5.15 (dq, 1H), 5.11-5.07 (m, 1H), 5.06-4.97 (m, 2H), 4.30 (t, 2H), 4.07 (t, 2H), 3.93 (t, 2H), 3.64 (t, 2H), 3.23 (s, 3H), 2.76 (s, 3H), 2.44 (q, 2H), 2.36-2.27 (m, 2H).
LC/MS (Methode 3): $R_t$ = 1.45 min, m/z = 393 $[M+H]^+$.

**Beispiel 359A**

But-2-in-1-yl-3-(but-2-in-1-yl)-1-(2-methoxyethyl)-5-methyl-2,4-dioxo-1,2,3,4-tetrahydrothieno-[2,3-d]pyrimidin-6-carboxylat

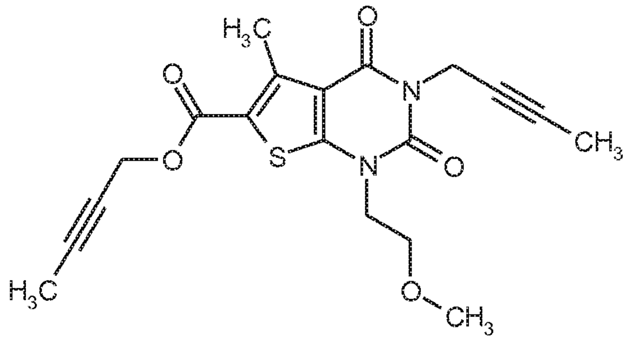

Analog zu dem unter Bsp. 20A beschriebenen Verfahren wurden aus 450 mg (1.43 mmol) der Verbindung aus Bsp. 18A und 574 mg (4.27 mmol) 1-Brom-2-butin 310 mg (57% d. Th.) der Titelverbindung erhalten. Die Reaktionszeit betrug hier 74 h.
$^1$H-NMR (400 MHz, DMSO-$d_6$, $\delta$/ppm): 4.89 (q, 2H), 4.56 (d, 2H), 4.11 (t, 2H), 3.66 (t, 2H), 3.24 (s, 3H), 2.77 (s, 3H), 1.86 (t, 3H), 1.75 (t, 3H).
LC/MS (Methode 3): $R_t$ = 1.30 min, m/z = 389 $[M+H]^+$.

**Beispiel 360A**

But-3-in-1-yl-3-(but-3-in-1-yl)-1-(2-methoxyethyl)-5-methyl-2,4-dioxo-1,2,3,4-tetrahydrothieno-[2,3-d]pyrimidin-6-carboxylat

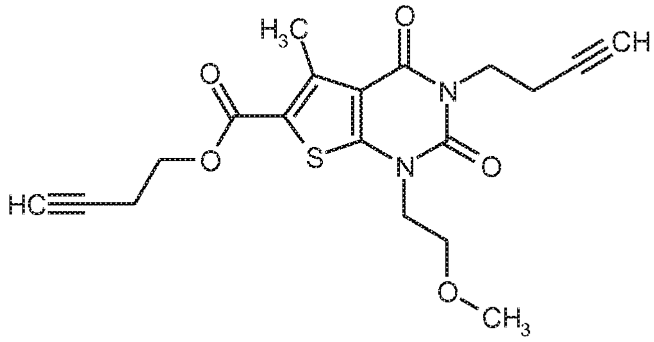

Analog zu dem unter Bsp. 20A beschriebenen Verfahren wurden aus 450 mg (1.43 mmol) der Verbindung aus Bsp. 18A und 586 mg (4.27 mmol) 1-Brom-3-butin 305 mg (50% d. Th.) der Titelverbindung erhalten. Die Reaktionszeit betrug hier 113 h.
$^1$H-NMR (400 MHz, DMSO-$d_6$, δ/ppm): 4.31 (t, 2H), 4.08 (t, 2H), 4.00 (t, 2H), 3.65 (t, 2H), 3.24 (s, 3H), 2.92 (t, 1H), 2.88 (t, 1H), 2.78 (s, 3H), 2.63 (td, 2H), 2.48-2.43 (m, 2H).
LC/MS (Methode 3): $R_t$ = 1.24 min, m/z = 389 $[M+H]^+$.

**Beispiel 361A**

3-Methylbut-3-en-1-yl-1-(2-methoxyethyl)-5-methyl-3-(3-methylbut-3-en-1-yl)-2,4-dioxo-1,2,3,4-tetrahydrothieno[2,3-d]pyrimidin-6-carboxylat

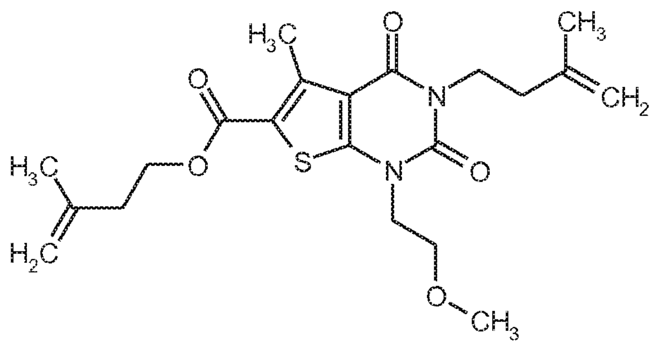

Eine Lösung von 450 mg (1.43 mmol) der Verbindung aus Bsp. 18A in 15 ml DMF wurde mit 1.39 g (4.27 mmol) Cäsiumcarbonat versetzt und 15 min bei RT gerührt. Dann wurden 670 mg (4.27 mmol) 4-Brom-2-methylbut-1-en zugesetzt und das Gemisch 113 h bei RT gerührt. Das Reaktionsgemisch wurde danach zwischen halbgesättigter wässriger Natriumchlorid-Lösung (70 ml) und Ethylacetat (70 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde an Kieselgel chromatographiert (Laufmittel Hexan/Ethylacetat). Es wurden 300 mg (47% d. Th.) der Titelverbindung erhalten.
$^1$H-NMR (400 MHz, DMSO-$d_6$, δ/ppm): 4.79 (d, 2H), 4.75-4.62 (m, 2H), 4.36 (t, 2H), 4.07 (t, 2H), 4.01-3.94 (m, 2H), 3.64 (t, 2H), 3.23 (s, 3H), 2.75 (s, 3H), 2.40 (t, 2H), 2.25 (t, 2H), 1.75 (d, 6H).
LC/MS (Methode 3): $R_t$ = 1.57 min, m/z = 421 $[M+H]^+$.

**Beispiel 362A**

4-Methylpent-3-en-1-yl-1-(2-methoxyethyl)-5-methyl-3-(4-methylpent-3-en-1-yl)-2,4-dioxo-1,2,3,4-tetrahydrothieno[2,3-d]pyrimidin-6-carboxylat

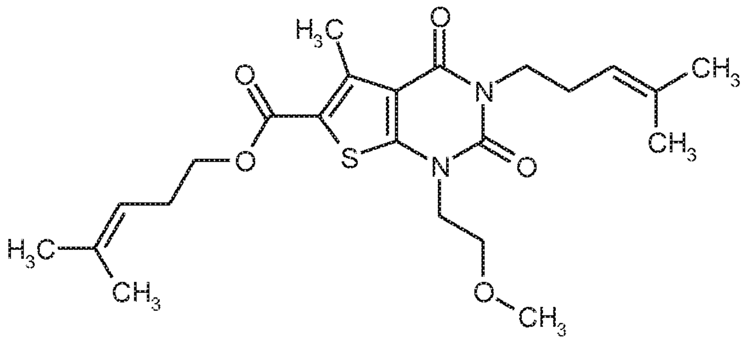

Analog zu dem unter Bsp. 361A beschriebenen Verfahren wurden aus 450 mg (1.43 mmol) der Verbindung aus Bsp. 18A und 718 mg (4.27 mmol) 5-Brom-2-methylpent-2-en 313 mg (49% d. Th.) der Titelverbindung erhalten. Die Reaktionszeit betrug hier 16 h.

$^1$H-NMR (400 MHz, DMSO-$d_6$, δ/ppm): 5.18-5.09 (m, 2H), 4.20 (t, 2H), 4.07 (t, 2H), 3.86-3.78 (m, 2H), 3.64 (t, 2H), 3.24 (s, 3H), 2.75 (s, 3H), 2.37 (q, 2H), 2.23 (q, 2H), 1.68 (s, 3H), 1.64 (s, 3H), 1.61 (s, 3H), 1.54 (s, 3H).

LC/MS (Methode 3): $R_t$ = 1.69 min, m/z = 449 $[M+H]^+$.

## Beispiel 363A

3,4,4-Trifluorbut-3-en-1-yl-1-(2-methoxyethyl)-5-methyl-2,4-dioxo-3-(3,4,4-trifluorbut-3-en-1-yl)-1,2,3,4-tetrahydrothieno[2,3-d]pyrimidin-6-carboxylat

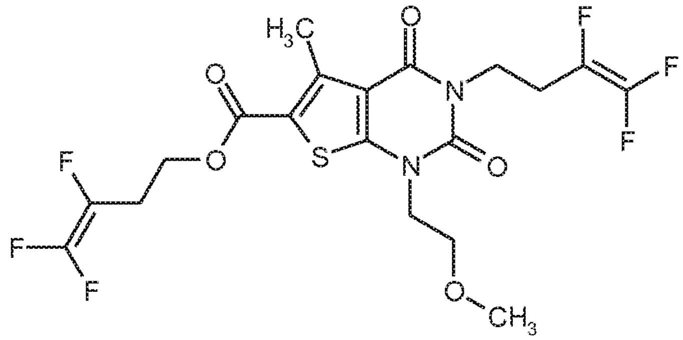

Analog zu dem unter Bsp. 361A beschriebenen Verfahren wurden aus 450 mg (1.43 mmol) der Verbindung aus Bsp. 18A und 824 mg (4.27 mmol) 4-Brom-1,1,2-trifluorbut-1-en 427 mg (55% d. Th.) der Titelverbindung erhalten. Die Reaktionszeit betrug hier 74 h.

$^1$H-NMR (400 MHz, DMSO-$d_6$, δ/ppm): 4.42 (t, 1H), 4.12-4.04 (m, 4H), 3.64 (t, 1H), 3.22 (s, 3H), 2.87-2.80 (m, 1H), 2.80-2.73 (m, 4H), 2.72-2.66 (m, 1H), 2.66-2.59 (m, 1H).

LC/MS (Methode 3): $R_t$ = 1.45 min, m/z = 501 $[M+H]^+$.

## Beispiel 364A

4,4-Difluorbut-3-en-1-yl-3-(4,4-difluorbut-3-en-1-yl)-1-(2-methoxyethyl)-5-methyl-2,4-dioxo-1,2,3,4-tetrahydrothieno[2,3-d]pyrimidin-6-carboxylat

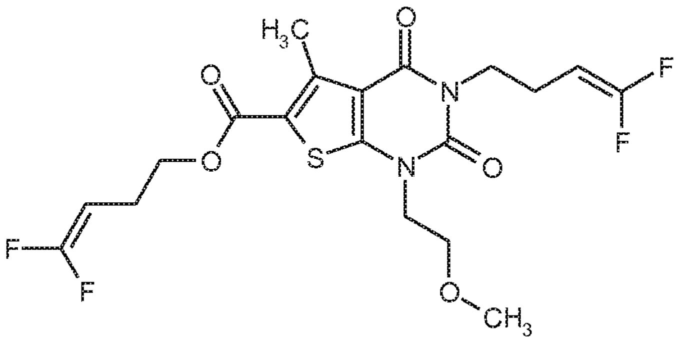

Eine Lösung von 450 mg (1.43 mmol) der Verbindung aus Bsp. 18A in 13.5 ml DMF wurde mit 1.39 g (4.27 mmol) Cäsiumcarbonat versetzt und 15 min bei RT gerührt. Dann wurden 769 mg (4.27 mmol) 4-Brom-1,1-difluorbut-1-en zugesetzt und das Gemisch 16 h bei RT gerührt. Das Reaktionsgemisch wurde danach zwischen Wasser (75 ml) und Ethylacetat (75 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde an Kieselgel chromatographiert (Laufmittel Hexan/Ethylacetat). Es wurden 350 mg (52% d. Th.) der Titelverbindung erhalten.
$^1$H-NMR (400 MHz, DMSO-$d_6$, δ/ppm): 4.65-4.48 (m, 2H), 4.27 (t, 2H), 4.08 (t, 2H), 3.91 (t, 2H), 3.64 (t, 2H), 3.23 (s, 3H), 2.78-2.75 (m, 3H), 2.41-2.34 (m, 2H), 2.26 (q, 2H).
LC/MS (Methode 3): $R_t$ = 1.47 min, m/z = 465 [M+H]$^+$.

## Beispiel 365A

(2,2-Difluorcyclopropyl)methyl-3-[(2,2-difluorcyclopropyl)methyl]-1-(2-methoxyethyl)-5-methyl-2,4-dioxo-1,2,3,4-tetrahydrothieno[2,3-d]pyrimidin-6-carboxylat (*Stereoisomerengemisch*)

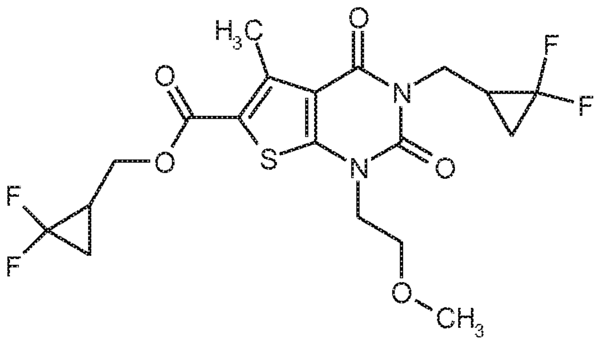

Analog zu dem unter Bsp. 361A beschriebenen Verfahren wurden aus 500 mg (1.58 mmol) der Verbindung aus Bsp. 18A und 812 mg (4.75 mmol) 2-Brommethyl-1,1-difluorcyclopropan 437 mg (55% d. Th.) der Titelverbindung erhalten. Die Reaktionszeit betrug hier 20 h bei einer Temperatur von 80°C.
$^1$H-NMR (400 MHz, DMSO-$d_6$, δ/ppm): 4.50-4.41 (m, 1H), 4.26-4.17 (m, 1H), 4.15-4.02 (m, 3H), 4.01-3.91 (m, 1H), 3.66 (t, 2H), 3.24 (s, 3H), 2.82-2.75 (m, 3H), 2.29-2.16 (m, 1H), 2.15-2.01 (m, 1H), 1.80-1.68 (m, 1H), 1.67-1.48 (m, 2H), 1.41-1.30 (m, 1H).
LC/MS (Methode 3): $R_t$ = 1.38 min, m/z = 465 [M+H]$^+$.

## Beispiel 366A

3-Methoxypropyl-1-(2-methoxyethyl)-3-{3-methoxypropyl)-5-methyl-2,4-dioxo-1,2,3,4-tetrahydro-thieno[2,3-d]pyrimidin-6-carboxylat

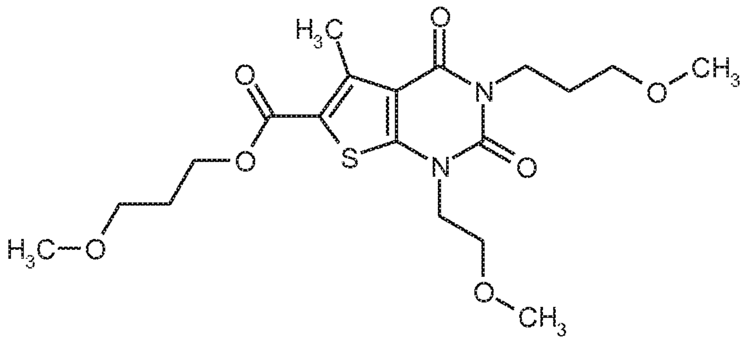

Analog zu dem unter Bsp. 361A beschriebenen Verfahren wurden aus 450 mg (1.43 mmol) der Verbindung aus Bsp. 18A und 654 mg (4.27 mmol) 1-Brom-3-methoxypropan 316 mg (48% d. Th.) der Titelverbindung erhalten. Die Reaktionszeit betrug hier 113 h.
$^1$H-NMR (400 MHz, DMSO-$d_6$, δ/ppm): 4.28 (t, 2H), 4.08 (t, 2H), 3.92 (t, 2H), 3.65 (t, 2H), 3.43 (t, 2H), 3.39-3.34 (m, 2H), 3.24 (d, 6H), 3.20 (s, 3H), 2.76 (s, 3H), 1.91 (quin, 2H), 1.78 (quin, 2H).
LC/MS (Methode 3): $R_t$ = 1.20 min, m/z = 429 $[M+H]^+$.

## Beispiel 367A

Tetrahydrofuran-2-ylmethyl-1-(2-methoxyethyl)-5-methyl-2,4-dioxo-3-(tetrahydrofuran-2-yl-methyl)-1,2,3,4-tetrahydrothieno[2,3-d]pyrimidin-6-carboxylat (*Stereoisomerengemisch*)

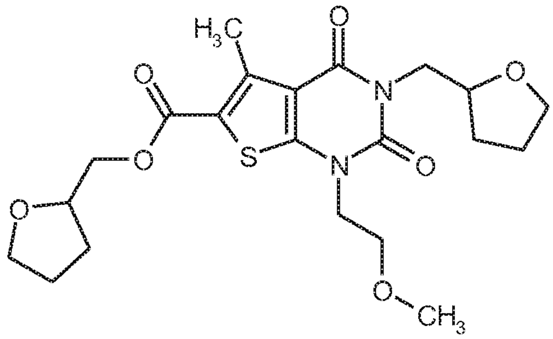

Analog zu dem unter Bsp. 361A beschriebenen Verfahren wurden aus 550 mg (1.74 mmol) der Verbindung aus Bsp. 18A und 907 mg (5.22 mmol) racemischem 2-(Brommethyl)tetrahydrofuran 433 mg (52% d. Th.) der Titelverbindung erhalten. Die Reaktionszeit betrug hier 40 h bei einer Temperatur von 80°C.
$^1$H-NMR (400 MHz, DMSO-$d_6$, δ/ppm): 4.29-4.24 (m, 1H), 4.21-3.99 (m, 6H), 3.80-3.71 (m, 3H), 3.71-3.56 (m, 4H), 3.24 (s, 3H), 2.77 (s, 3H), 2.03-1.75 (m, 6H), 1.68-1.57 (m, 2H).
LC/MS (Methode 3): $R_t$ = 1.21 min, m/z = 453 $[M+H]^+$.

## Beispiel 368A

Oxetan-3-ylmethyl-1-(2-methoxyethyl)-5-methyl-3-(oxetan-3-ylmethyl)-2,4-dioxo-1,2,3,4-tetra-hydrothieno[2,3-d]pyrimidin-6-carboxylat

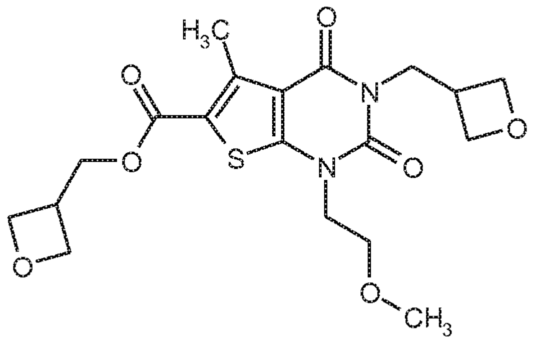

Analog zu dem unter Bsp. 361A beschriebenen Verfahren wurden aus 450 mg (1.43 mmol) der Verbindung aus Bsp. 18A und 645 mg (4.27 mmol) 3-(Brommethyl)oxetan 349 mg (55% d. Th.) der Titelverbindung erhalten. Die Reaktionszeit betrug hier 21 h.
$^1$H-NMR (400 MHz, DMSO-$d_6$, $\delta$/ppm): 4.68 (dd, 1H), 4.58 (dd, 1H), 4.45 (d, 1H), 4.41 (td, 2H), 4.17 (d, 1H), 4.07 (t, 1H), 3.64 (t, 1H), 3.41-3.35 (m, 1H), 3.32-3.25 (m, 1H), 3.23 (s, 3H), 2.76 (s, 3H).
LC/MS (Methode 3): $R_t$ = 0.97 min, m/z = 425 $[M+H]^+$.

**Beispiel 369A**

Ethyl-2-{[(2-ethoxyethyl)carbamoyl]amino}-4-methylthiophen-3-carboxylat

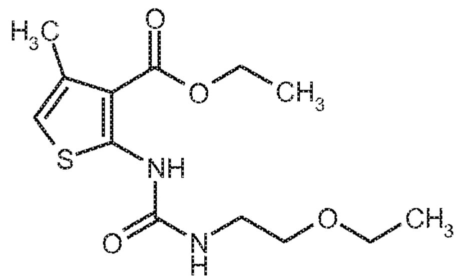

Eine Lösung von 2.79 g (15.1 mmol) Ethyl-2-amino-4-methylthiophen-3-carboxylat in 15 ml Pyridin wurde mit 2.6 g (22.6 mmol) 1-Ethoxy-2-isocyanatoethan versetzt. Das Reaktionsgemisch wurde danach 21 h bei 70°C gerührt. Anschließend wurde am Rotationsverdampfer zur Trockene eingeengt. Der verbliebene Rückstand wurde in Dichlormethan gelöst und erneut zur Trockene eingeengt. Es wurden 4.68 g (quant.) der Titelverbindung erhalten.
$^1$H-NMR (400 MHz, DMSO-$d_6$, $\delta$/ppm): 10.30 (s, 1H), 8.00 (br. s, 1H), 6.42 (s, 1H), 4.28 (q, 2H), 3.48-3.38 (m, 4H), 3.24 (q, 2H), 2.26 (d, 3H), 1.31 (t, 3H), 1.12 (t, 3H).
LC/MS (Methode 3): $R_t$ = 1.19 min, m/z = 301 $[M+H]^+$.

**Beispiel 370A**

3-(2-Ethoxyethyl)-5-methylthieno[2,3-d]pyrimidin-2,4(1*H*,3*H*)-dion

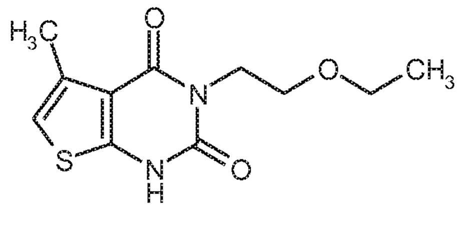

4.68 g (15.6 mmol) der Verbindung aus Bsp. 369A wurden in 42 ml Ethanol gelöst und mit 11.6 ml (31.2 mmol)

einer 21%-igen Lösung von Natriumethylat in Ethanol versetzt. Nachdem das Gemisch ca. 1 h bei RT gerührt worden war, wurde es mit 35.8 ml 1 M Salzsäure versetzt. Der dabei ausgefallene Niederschlag wurde abgesaugt, mit Wasser neutral gewaschen und getrocknet. Es wurden 3.86 g (97% d. Th.) der Titelverbindung erhalten.
$^1$H-NMR (400 MHz, DMSO-$d_6$, δ/ppm): 12.15 (br. s, 1H), 6.69 (d, 1H), 3.99 (t, 2H), 3.50 (t, 2H), 3.44 (q, 2H), 2.34 (d, 3H), 1.07 (t, 3H).
LC/MS (Methode 3): $R_t$ = 0.88 min, m/z = 255 $[M+H]^+$.

**Beispiel 371A**

3-(2-Ethoxyethyl)-5-methyl-2,4-dioxo-1,2,3,4-tetrahydrothieno[2,3-d]pyrimidin-6-carbaldehyd

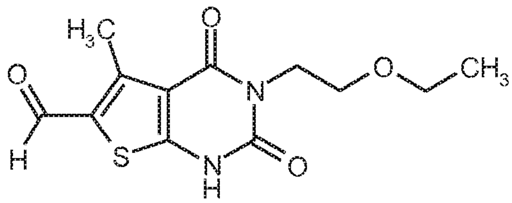

Eine Lösung von 2.87 g (11.3 mmol) der Verbindung aus Bsp. 370A in 105 ml DMF wurde unter Eisbadkühlung vorsichtig mit 10.5 ml (113 mmol) Phosphoroxychlorid versetzt. Das Gemisch wurde 90 min bei 70°C gerührt. Dann wurde das Reaktionsgemisch am Rotationsverdampfer weitestgehend eingeengt. Der verbliebene Rückstand wurde auf Eiswasser gegeben und gerührt. Das ausgefallene Produkt wurde abgesaugt, mit Wasser neutral gewaschen und getrocknet. Es wurden 3.08 g (96% d. Th.) der Titelverbindung erhalten.
$^1$H-NMR (400 MHz, DMSO-$d_6$, δ/ppm): 12.64 (br. s, 1H), 10.06 (s, 1H), 3.99 (t, 2H), 3.51 (t, 2H), 3.44 (q, 2H), 2.75 (s, 3H), 1.07 (t, 3H).
LC/MS (Methode 3): $R_t$ = 0.84 min, m/z = 283 $[M+H]^+$.

**Beispiel 372A**

3-Ethyl-1-(fluormethyl)-5-methyl-2,4-dioxo-1,2,3,4-tetrahydrothieno[2,3-d]pyrimidin-6-carbaldehyd

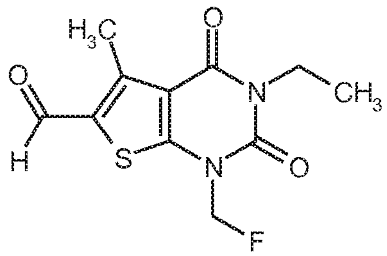

Eine Lösung von 690 mg (2.89 mmol) der Verbindung aus Bsp. 48A in 30 ml DMF wurde mit 1.2 g (8.68 mmol) Kaliumcarbonat versetzt und 15 min bei RT gerührt. Dann wurden 4.3 ml (8.68 mmol) einer 2 M Lösung von Bromfluormethan in DMF sowie 108 mg (0.724 mmol) Natriumiodid zugesetzt und das Gemisch 21 h bei 50°C gerührt. Das DMF wurde danach weitestgehend abdestilliert und der verbliebene Rückstand zwischen halbgesättigter wässriger Natriumchlorid-Lösung (100 ml) und Ethylacetat (150 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 332 mg (39% d. Th.) der Titelverbindung erhalten.
$^1$H-NMR (400 MHz, DMSO-$d_6$, δ/ppm): 10.12 (s, 1H), 6.10 (d, 2H), 3.91 (q, 2H), 2.80 (s, 3H), 1.15 (t, 3H).
LC/MS (Methode 3): $R_t$ = 1.0 min, m/z = 271 $[M+H]^+$.

**Beispiel 373A**

1-[2-(Cyclopentyloxy)ethyl]-3-ethyl-5-methyl-2,4-dioxo-1,2,3,4-tetrahydrothieno[2,3-d]pyrimidin-6-carbaldehyd

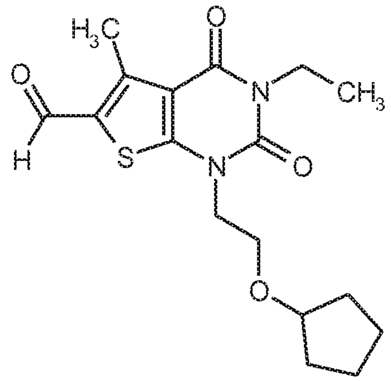

Eine Lösung von 650 mg (2.7 mmol) der Verbindung aus Bsp. 48A in 24 ml DMF wurde mit 933 mg (6.75 mmol) Kaliumcarbonat versetzt und 15 min bei RT gerührt. Dann wurden 1.66 g (8.2 mmol) (2-Bromethoxy)cyclopentan zugesetzt und das Gemisch 20 h bei 50°C gerührt. Das DMF wurde danach weitestgehend abdestilliert und der verbliebene Rückstand zwischen halbgesättigter wässriger Natriumchlorid-Lösung (70 ml) und Ethylacetat (70 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 646 mg (68% d. Th.) der Titelverbindung erhalten.
$^1$H-NMR (400 MHz, DMSO-$d_6$, δ/ppm): 10.09 (s, 1H), 4.06 (t, 2H), 3.94-3.85 (m, 3H), 3.65 (t, 2H), 2.78 (s, 3H), 1.61-1.49 (m, 2H), 1.49-1.37 (m, 7H), 1.13 (t, 3H).
LC/MS (Methode 3): $R_t$ = 1.31 min, m/z = 351 [M+H]$^+$.

**Beispiel 374A**

3-Ethyl-5-methyl-1-[2-(methylsulfanyl)ethyl]-2,4-dioxo-1,2,3,4-tetrahydrothieno[2,3-d]pyrimidin-6-carbaldehyd

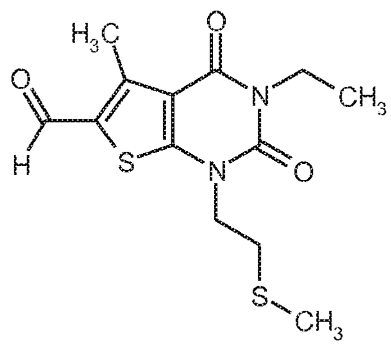

Eine Lösung von 500 mg (2.08 mmol) der Verbindung aus Bsp. 48A in 19 ml DMF wurde mit 718 mg (5.19 mmol) Kaliumcarbonat versetzt und 15 min bei RT gerührt. Dann wurden 644 mg (4.16 mmol) 1-Brom-2-(methylsulfanyl)ethan zugesetzt und das Gemisch 16 h bei 50°C gerührt. Das DMF wurde danach weitestgehend abdestilliert und der verbliebene Rückstand zwischen halbgesättigter wässriger Natriumchlorid-Lösung (300 ml) und Ethylacetat (150 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 340 g Kieselgel, Laufmittel Hexan/Ethylacetat). Es wurden 360 mg (51% d. Th.) der Titelverbindung erhalten.
$^1$H-NMR (400 MHz, DMSO-$d_6$, δ/ppm): 10.10 (s, 1H), 4.12 (t, 2H), 3.91 (q, 2H), 2.87-2.81 (m, 2H), 2.79 (s, 3H), 2.14 (s,

3H), 1.13 (t, 3H).
LC/MS (Methode 3): $R_t$ = 1.13 min, m/z = 313 $[M+H]^+$.

**Beispiel 375A**

3-Ethyl-1-[2-(ethylsulfanyl)ethyl]-5-methyl-2,4-dioxo-1,2,3,4-tetrahydrothieno[2,3-d]pyrimidin-6-carbaldehyd

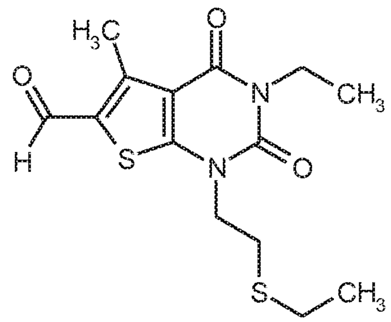

Eine Lösung von 750 mg (3.12 mmol) der Verbindung aus Bsp. 48A in 28 ml DMF wurde mit 1.08 g (7.79 mmol) Kaliumcarbonat versetzt und 15 min bei RT gerührt. Dann wurden 1.05 g (6.23 mmol) 1-Brom-2-(ethylsulfanyl)ethan zugesetzt und das Gemisch 16 h bei 50°C gerührt. Das DMF wurde danach weitestgehend abdestilliert und der verbliebene Rückstand zwischen halbgesättigter wässriger Natriumchlorid-Lösung (300 ml) und Ethylacetat (150 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 340 g Kieselgel, Laufmittel Hexan/Ethylacetat). Es wurden 666 mg (63% d. Th.) der Titelverbindung erhalten.
$^1H$-NMR (400 MHz, DMSO-$d_6$, $\delta$/ppm): 10.10 (s, 1H), 4.12-4.06 (m, 2H), 3.90 (q, 2H), 2.89-2.83 (m, 2H), 2.79 (s, 3H), 2.60 (q, 2H), 1.19 (t, 3H), 1.13 (t, 3H).
LC/MS (Methode 3): $R_t$ = 1.22 min, m/z = 327 $[M+H]^+$.

**Beispiel 376A**

3-Ethyl-5methylsulfonyl)ethyl]-2,4-dioxo-1,2,3,4-tetrahydrothieno[2,3-d]pyrimidin-6-carbaldehyd

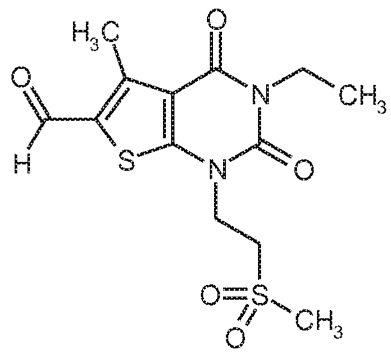

Eine Lösung von 375 mg (1.09 mmol) der Verbindung aus Bsp. 374A in 37 ml Dichlormethan wurde bei 0°C mit 592 mg (2.40 mmol) 3-Chlorperoxybenzoesäure (Gehalt 70%) versetzt. Das Gemisch wurde 30 min bei 0°C gerührt. Dann wurde das Kältebad entfernt und für weitere 2 h bei RT gerührt. Anschließend wurde das Reaktionsgemisch mit 50 ml Wasser und 220 mg (2.62 mmol) Natriumhydrogencarbonat versetzt. Die organische Phase wurde abgetrennt und die wässrige Phase mit Dichlormethan extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 353 mg (92% d. Th.) der Titelverbindung erhalten.

$^1$H-NMR (400 MHz, DMSO-$d_6$, δ/ppm): 10.11 (s, 1H), 4.37-4.31 (m, 2H), 3.91 (q, 2H), 3.60 (t, 2H), 3.12 (s, 3H), 2.80 (s, 3H), 1.14 (t, 3H).
LC/MS (Methode 3): $R_t$ = 0.84 min, m/z = 345 $[M+H]^+$.

**Beispiel 377A**

3-Ethyl-1-(3-methoxypropyl)-5-methyl-2,4-dioxo-1,2,3,4-tetrahydrothieno[2,3-d]pyrimidin-6-carbaldehyd

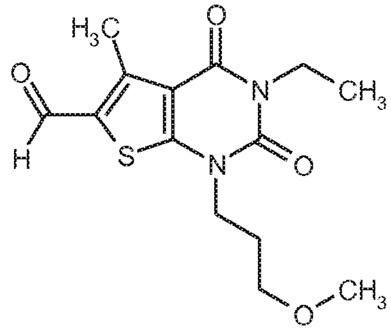

Eine Lösung von 639 mg (2.68 mmol) der Verbindung aus Bsp. 48A in 24 ml DMF wurde mit 927 mg (6.71 mmol) Kaliumcarbonat versetzt und 15 min bei RT gerührt. Dann wurden 1.25 g (8.15 mmol) 1-Brom-3-methoxypropan zugesetzt und das Gemisch 15 h bei 50°C gerührt. Das DMF wurde danach weitestgehend abdestilliert und der verbliebene Rückstand zwischen halbgesättigter wässriger Natriumchlorid-Lösung (70 ml) und Ethylacetat (70 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 521 mg (60% d. Th.) der Titelverbindung erhalten.
$^1$H-NMR (400 MHz, DMSO-$d_6$, δ/ppm): 10.09 (s, 1H), 3.99 (t, 2H), 3.90 (q, 2H), 3.40 (t, 2H), 3.20 (s, 3H), 2.79 (s, 3H), 1.97-1.88 (m, 2H), 1.13 (t, 3H).
LC/MS (Methode 3): $R_t$ = 1.05 min, m/z = 311 $[M+H]^+$.

**Beispiel 378A**

1-(Fluormethyl)-3-isopropyl-5-methyl-2,4-dioxo-1,2,3,4-tetrahydrothieno[2,3-d]pyrimidin-6-carbaldehyd

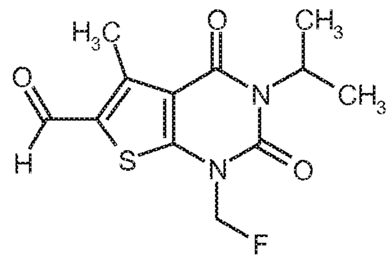

Eine Lösung von 800 mg (3.17 mmol) der Verbindung aus Bsp. 49A in 39 ml DMF wurde mit 1.31 g (9.51 mmol) Kaliumcarbonat versetzt und 15 min bei RT gerührt. Dann wurden 4.8 ml (9.51 mmol) einer 2 M Lösung von Bromfluormethan in DMF zugesetzt und das Gemisch 44 h bei 50°C gerührt. Das DMF wurde danach weitestgehend abdestilliert und der verbliebene Rückstand zwischen halbgesättigter wässriger Natriumchlorid-Lösung (100 ml) und Ethylacetat (150 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 488 mg (51% d. Th.) der Titelverbindung erhalten.
$^1$H-NMR (400 MHz, DMSO-$d_6$, δ/ppm): 10.12 (s, 1H), 6.16-5.98 (m, 2H), 5.11 (quin, 1H), 2.79 (s, 3H), 1.42 (d, 6H).

LC/MS (Methode 3): $R_t$ = 1.12 min, m/z = 285 $[M+H]^+$.

**Beispiel 379A**

1-(3-Fluorpropyl)-3-isopropyl-5-methyl-2,4-dioxo-1,2,3,4-tetrahydrothieno[2,3-d]pyrimidin-6-carbaldehyd

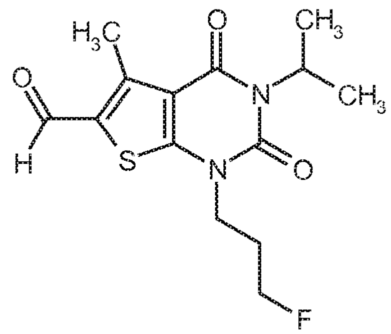

Eine Lösung von 800 mg (3.17 mmol) der Verbindung aus Bsp. 49A in 33 ml DMF wurde mit 1.09 g (7.93 mmol) Kaliumcarbonat versetzt und 15 min bei RT gerührt. Dann wurden 1.79 g (9.51 mmol) 1-Fluor-3-iodpropan zugesetzt und das Gemisch 20 h bei 50°C gerührt. Das DMF wurde danach weitestgehend abdestilliert und der verbliebene Rückstand zwischen halbgesättigter wässriger Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 796 mg (79% d. Th.) der Titelverbindung erhalten.
$^1$H-NMR (400 MHz, DMSO-$d_6$, δ/ppm): 10.09 (s, 1H), 5.11 (sept, 1H), 4.61 (t, 1H), 4.49 (t, 1H), 4.02 (t, 2H), 2.78 (s, 3H), 2.15-2.07 (m, 1H), 2.07-1.99 (m, 1H), 1.40 (d, 6H).
LC/MS (Methode 3): $R_t$ = 1.16 min, m/z = 313 $[M+H]^+$.

**Beispiel 380A**

3-(2-Methoxyethyl)-5-methyl-1-(oxetan-2-ylmelhyl)-2,4-dioxo-1,2,3,4-tetrahydrothieno[2,3-d]-pyrimidin-6-carbaldehyd (*Racemat*)

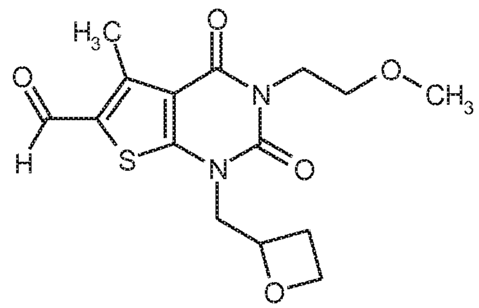

In einem verschlossenen Reaktionsgefäß wurde eine Mischung aus 3.0 g (11.2 mmol) der Verbindung aus Bsp. 53A, 3.86 g (27.9 mmol) Kaliumcarbonat, 186 mg (1.12 mmol) Kaliumiodid und 2.36 g (15.7 mmol) racemischem 2-(Brommethyl)oxetan in 15 ml DMF 36 h auf 80°C erwärmt.

Nach dem Abkühlen auf RT wurde das Reaktionsgemisch mit ca. 75 ml Wasser verdünnt und mit Ethylacetat extrahiert. Der organische Extrakt wurde nacheinander mit Wasser und gesättigter Natriumchlorid-Lösung gewaschen. Nach Trocknen über wasserfreiem Magnesiumsulfat wurde filtriert und eingeengt. Der erhaltene Rückstand wurde mit Diisopropylether, dem etwas Ethylacetat beigemischt war, verrührt. Der Feststoff wurde abgesaugt und im Hochvakuum getrocknet. Dies ergab eine erste Fraktion der Titelverbindung. Die Mutterlauge des Verrührens wurde wieder eingeengt und der Rückstand mittels MPLC gereinigt (340 g Kieselgel, Laufmittel Cyclohexan/ Ethylacetat 88:12 → 0: 100). Das

Einengen der Produktfraktionen ergab eine zweite Teilmenge der Titelverbindung, die mit der ersten vereinigt wurde. Insgesamt wurden so 2.33 g (60% d. Th.) der Titelverbindung erhalten.
$^1$H-NMR (400 MHz, DMSO-$d_6$, δ/ppm): 10.09 (s, 1H), 5.09-4.96 (m, 1H), 4.54-4.38 (m, 2H), 4.31-4.14 (m, 2H), 4.06 (t, 2H), 3.52 (t, 2H), 3.31 (s, 3H), 2.78 (s, 3H), 2.76-2.64 (m, 1H), 2.55-2.46 (m, 1H).
LC/MS (Methode 17, ESIpos): $R_t$ = 1.31 min, m/z = 339.10 [M+H]$^+$.

**Beispiel 381A**

3-(2-Ethoxyethyl)-5-methyl-2,4-dioxo-1-(3,3,3-trifluorpropyl)-1,2,3,4-tetrahydrothieno[2,3-d]-pyrimidin-6-carbaldehyd

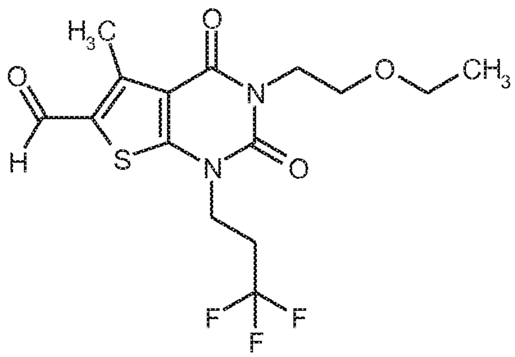

Eine Lösung von 520 mg (1.84 mmol) der Verbindung aus Bsp. 371A in 18 ml DMF wurde mit 636 mg (4.6 mmol) Kaliumcarbonat versetzt und 15 min bei RT gerührt. Dann wurden 1.24 g (5.52 mmol) 1-Iod-3,3,3-trifluorpropan zugesetzt und das Gemisch 19 h bei 50°C gerührt. Das DMF wurde danach weitestgehend abdestilliert und der verbliebene Rückstand zwischen halbgesättigter wässriger Natriumchlorid-Lösung (200 ml) und Ethylacetat (100 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 560 mg (80% d. Th.) der Titelverbindung erhalten.
$^1$H-NMR (400 MHz, DMSO-$d_6$, δ/ppm): 10.11 (s, 1H), 4.18 (t, 2H), 4.05 (t, 2H), 3.53 (t, 2H), 3.44 (q, 2H), 2.85-2.74 (m, 5H), 1.06 (t, 3H).
LC/MS (Methode 3): $R_t$ = 1.18 min, m/z = 379 [M+H]$^+$.

**Beispiel 382A**

3-(2-Ethoxyethyl)-1-(2-methoxyethyl)-5-methyl-2,4-dioxo-1,2,3,4-tetrahydrothieno[2,3-d]pyrimidin-6-carbaldehyd

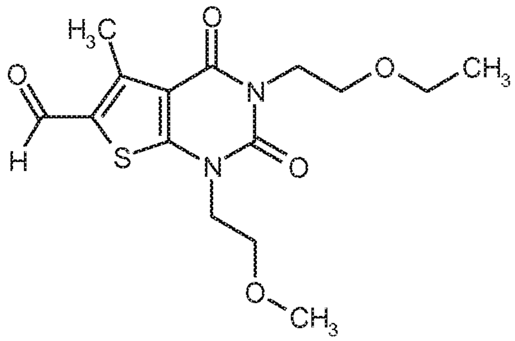

Eine Lösung von 520 mg (1.84 mmol) der Verbindung aus Bsp. 371A in 18 ml DMF wurde mit 636 mg (4.6 mmol) Kaliumcarbonat versetzt und 15 min bei RT gerührt. Dann wurden 768 mg (5.52 mmol) 1-Brom-2-methoxyethan zugesetzt und das Gemisch 25 h bei 50°C gerührt. Das DMF wurde danach weitestgehend abdestilliert und der verbliebene Rückstand zwischen halbgesättigter wässriger Natriumchlorid-Lösung (70 ml) und Ethylacetat (70 ml) verteilt. Die

wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 100 g Kieselgel, Laufmittel Hexan/Ethylacetat). Es wurden 586 mg (71% d. Th.) der Titelverbindung erhalten.
$^1$H-NMR (600 MHz, DMSO-$d_6$, $\delta$/ppm): 10.09 (s, 1H), 4.10 (t, 2H), 4.05 (t, 2H), 3.65 (t, 2H), 3.53 (t, 2H), 3.45 (q, 2H), 3.24 (s, 3H), 2.78 (s, 3H), 1.06 (t, 3H).
LC/MS (Methode 3): $R_t$ = 1.02 min, m/z = 342 $[M+H]^+$.

**Beispiel 383A**

3-Ethyl-1-(fluormethyl)-6-(hydroxymethyl)-5-methylthieno[2,3-d]pyrimidin-2,4(1*H*,3*H*)-dion

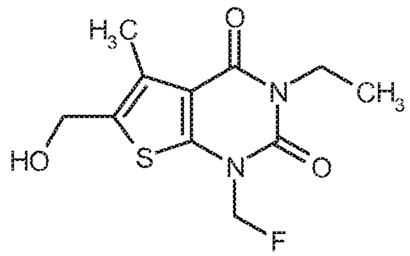

Analog zu dem unter Bsp. 143A (Methode C) beschriebenen Verfahren wurden aus 444 mg (1.65 mmol) der Verbindung aus Bsp. 372A 432 mg (93% d. Th.) der Titelverbindung hergestellt. Die Umsetzung erfolgte hier für 2 h bei -78°C.
$^1$H-NMR (400 MHz, DMSO-$d_6$, $\delta$/ppm): 6.03 (d, 2H), 5.67 (t, 1H), 4.60 (d, 2H), 3.91 (q, 2H), 2.33 (s, 3H), 1.13 (t, 3H).
LC/MS (Methode 3): $R_t$ = 0.86 min, m/z = 273 $[M+H]^+$.

**Beispiel 384A**

3-Ethyl-6-(hydroxymethyl)-1-(3-methoxypropyl)-5-methylthieno[2,3-d]pyrimidin-2,4(1*H*,3*H*)-dion

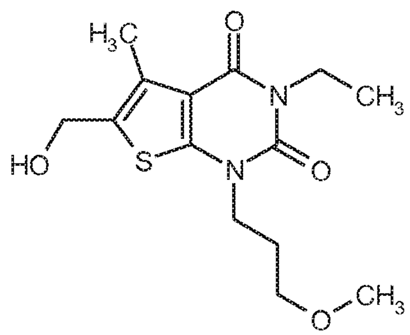

Analog zu dem unter Bsp. 143A (Methode C) beschriebenen Verfahren wurden aus 121 mg (0.378 mmol) der Verbindung aus Bsp. 377A 106 mg (87% d. Th.) der Titelverbindung hergestellt. Die Umsetzung erfolgte hier für 2 h bei -78°C.
$^1$H-NMR (400 MHz, DMSO-$d_6$, $\delta$/ppm): 5.58 (t, 1H), 4.57 (d, 2H), 3.96-3.86 (m, 4H), 3.39 (t, 2H), 3.21 (s, 3H), 2.33 (s, 3H), 1.94-1.87 (m, 2H), 1.11 (t, 3H).
LC/MS (Methode 3): $R_t$ = 0.89 min, m/z = 313 $[M+H]^+$.

**Beispiel 385A**

6-(Hydroxymethyl)-1-(2-methoxyethyl)-5-methyl-3-(prop-2-in-1-yl)thieno[2,3-d]pyrimidin-2,4(1*H*,3*H*)-dion

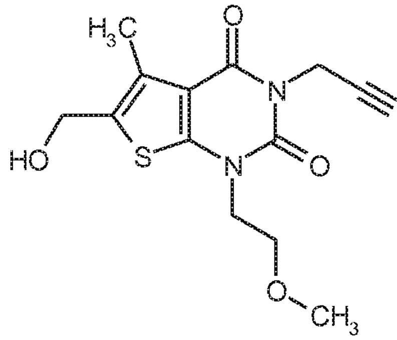

Analog zu dem unter Bsp. 138A (Methode C) beschriebenen Verfahren wurden aus 312 mg (0.82 mmol) der Verbindung aus Bsp. 357A 162 mg (64% d. Th.) der Titelverbindung hergestellt. Die Umsetzung erfolgte hier bei -40°C. $^1$H-NMR (400 MHz, DMSO-$d_6$, δ/ppm): 5.62 (t, 1H), 4.61-4.56 (m, 4H), 4.06 (t, 2H), 3.65 (t, 2H), 3.24 (s, 3H), 3.14-3.12 (m, 1H), 2.33 (s, 3H).
LC/MS (Methode 3): $R_t$ = 0.82 min, m/z = 309 $[M+H]^+$.

**Beispiel 386A**

3-(But-3-en-1-yl)-6-{hydroxymethyl)-1-(2-methoxyethyl)-5-methylthieno[2,3-d]pyrimidin-2,4(1*H*,3*H*)-dion

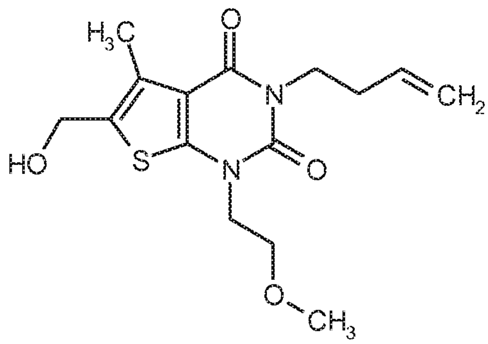

Analog zu dem unter Bsp. 138A (Methode C) beschriebenen Verfahren wurden aus 330 mg (0.84 mmol) der Verbindung aus Bsp. 358A 178 mg (65% d. Th.) der Titelverbindung hergestellt. Die Umsetzung erfolgte hier bei -40°C. $^1$H-NMR (400 MHz, DMSO-$d_6$, δ/ppm): 5.78 (ddt, 1H), 5.57 (t, 1H), 5.06-4.95 (m, 2H), 4.57 (d, 2H), 4.04 (t, 2H), 3.93 (t, 2H), 3.63 (t, 2H), 3.23 (s, 3H), 2.36-2.27 (m, 5H).
LC/MS (Methode 3): $R_t$ = 1.0 min, m/z = 325 $[M+H]^+$.

**Beispiel 387A**

3-(But-2-in-1-yl)-6-(hydroxymethyl)-1-(2-methoxyethyl)-5-methylthieno[2,3-d]pyrimidin-2,4(1*H*,3*H*)-dion

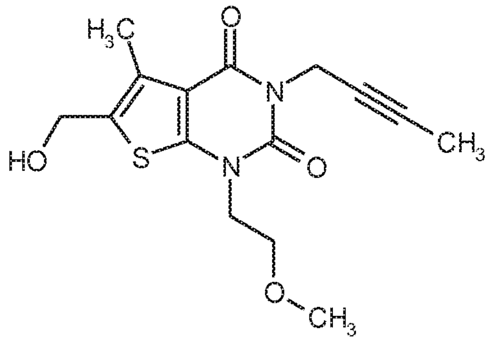

Analog zu dem unter Bsp. 138A (Methode C) beschriebenen Verfahren wurden aus 305 mg (0.652 mmol) der Verbindung aus Bsp. 359A 122 mg (58% d. Th.) der Titelverbindung hergestellt. Die Umsetzung erfolgte hier bei RT.
$^1$H-NMR (400 MHz, DMSO-$d_6$, $\delta$/ppm): 5.61 (t, 1H), 4.60-4.53 (m, 4H), 4.06 (t, 2H), 3.64 (t, 2H), 3.24 (s, 3H), 2.32 (s, 3H), 1.74 (t, 3H).
LC/MS (Methode 3): $R_t$ = 0.89 min, m/z = 323 $[M+H]^+$.

**Beispiel 388A**

3-(But-3-in-1-yl)-6-(hydroxymethyl)-1-(2-methoxyethyl)-5-methylthieno[2,3-d]pyrimidin-2,4(1*H*,3*H*)-dion

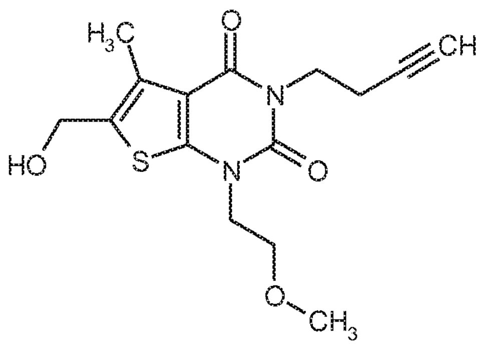

Analog zu dem unter Bsp. 138A (Methode C) beschriebenen Verfahren wurden aus 300 mg (0.703 mmol) der Verbindung aus Bsp. 360A 134 mg (58% d. Th.) der Titelverbindung hergestellt. Die Umsetzung erfolgte hier bei RT.
$^1$H-NMR (400 MHz, DMSO-$d_6$, $\delta$/ppm): 5.58 (t, 1H), 4.57 (d, 2H), 4.06-4.02 (m, 2H), 4.00 (t, 2H), 3.63 (t, 2H), 3.24 (s, 3H), 2.85 (t, 1H), 2.48-2.43 (m, 2H), 2.32 (s, 3H).
LC/MS (Methode 3): $R_t$ = 0.86 min, m/z = 323 $[M+H]^+$.

**Beispiel 389A**

6-(Hydroxymethyl)-1-(2-methoxyethyl)-5-melhyl-3-(3-methylbut-3-en-1-yl)thieno[2,3-d]pyrimidin-2,4(1*H*,3*H*)-dion

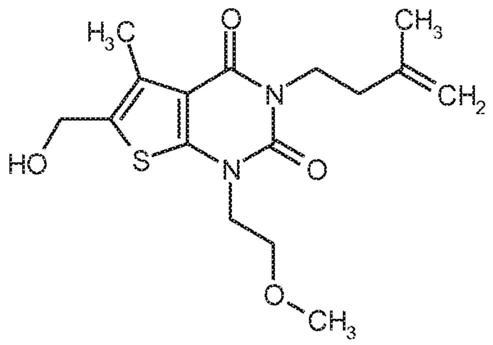

Eine Lösung von 295 mg (0.666 mmol) der Verbindung aus Bsp. 361A in 18 ml trockenem THF wurde bei -40°C tropfenweise mit 666 µl (0.666 mmol) einer 1 M Lösung von Lithiumaluminiumhydrid in THF versetzt. Nach vollständiger Zugabe wurde 2 h bei RT nachgerührt. Dann wurde vorsichtig 1 ml 10%-ige Salzsäure zugesetzt. Das Gemisch wurde mit 100 ml gesättigter KochsalzLösung versetzt, und die wässrige Phase wurde mit Ethylacetat extrahiert. Die vereinigten organischen Phasen wurden über Natriumsulfat getrocknet, filtriert und eingeengt. Der erhaltene Rückstand wurde mittels präparativer HPLC gereinigt (Methode 14). Es wurden 151 mg (65% d. Th.) der Titelverbindung erhalten.
$^1$H-NMR (400 MHz, DMSO-$d_6$, δ/ppm): 5.57 (t, 1H), 4.72 (s, 1H), 4.63 (d, 1H), 4.57 (d, 2H), 4.03 (t, 2H), 4.01-3.94 (m, 2H), 3.63 (t, 2H), 3.23 (s, 3H), 2.32 (s, 3H), 2.24 (t, 2H), 1.76 (s, 3H).
LC/MS (Methode 3): $R_t$ = 1.05 min, m/z = 339 $[M+H]^+$.

**Beispiel 390A**

6-(Hydroxymethyl)-1-(2-methoxyethyl)-5-methyl-3-(4-methylpent-3-en-1-yl)thieno[2,3-d]pyrimidin-2,4(1*H*,3*H*)-dion

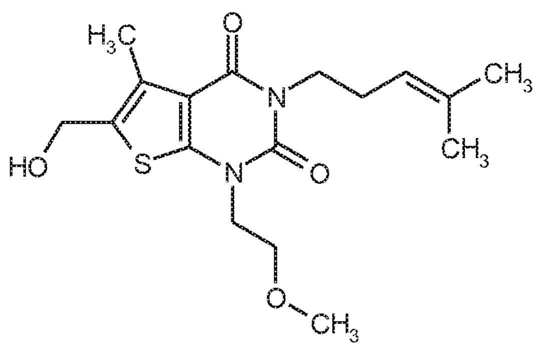

Analog zu dem unter Bsp. 138A (Methode C) beschriebenen Verfahren wurden aus 310 mg (0.691 mmol) der Verbindung aus Bsp. 362A 125 mg (48% d. Th.) der Titelverbindung hergestellt. Die Umsetzung erfolgte hier für 1 h bei RT.
$^1$H-NMR (400 MHz, DMSO-$d_6$, δ/ppm): 5.57 (t, 1H), 5.16-5.08 (m, 1H), 4.57 (d, 2H), 4.03 (t, 2H), 3.86-3.79 (m, 2H), 3.63 (t, 2H), 3.25-3.22 (m, 3H), 2.32 (s, 3H), 2.27-2.17 (m, 2H), 1.63 (s, 3H), 1.54 (s, 3H).
LC/MS (Methode 3): $R_t$ = 1.17 min, m/z = 353 $[M+H]^+$.

**Beispiel 391A**

6-(Hydroxymethyl)-1-(2-methoxyethyl)-5-methyl-3-(3,4,4-trifluorbut-3-en-1-yl)thieno[2,3-d]-pyrimidin-2,4(1*H*,3*H*)-dion

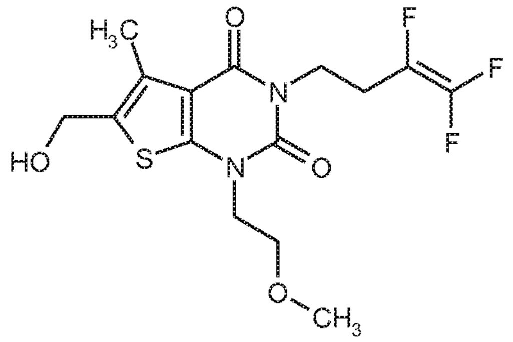

Eine Lösung von 424 mg (0.737 mmol) der Verbindung aus Bsp. 363A in 17 ml trockenem THF wurde bei -40°C tropfenweise mit 737 µl (0.737 mmol) einer 1 M Lösung von Lithiumaluminiumhydrid in THF versetzt. Nach vollständiger Zugabe wurde 1 h bei RT nachgerührt. Dann wurde vorsichtig 1 ml 10%-ige Salzsäure zugesetzt. Das Gemisch wurde mit 100 ml gesättigter KochsalzLösung versetzt, und die wässrige Phase wurde mit Ethylacetat extrahiert. Die vereinigten organischen Phasen wurden über Natriumsulfat getrocknet, filtriert und eingeengt. Der erhaltene Rückstand wurde mittels präparativer HPLC gereinigt (Methode 14). Es wurden 199 mg (70% d. Th.) der Titelverbindung erhalten.

$^1$H-NMR (400 MHz, DMSO-$d_6$, δ/ppm): 5.59 (t, 1H), 4.57 (d, 2H), 4.08 (t, 2H), 4.05 (t, 2H), 3.63 (t, 2H), 3.22 (s, 3H), 2.72-2.65 (m, 1H), 2.65-2.59 (m, 1H), 2.32 (s, 3H).

LC/MS (Methode 3): $R_t$ = 1.04 min, m/z = 379 [M+H]$^+$.

**Beispiel 392A**

3-(4,4-Difluorbut-3-en-1-yl)-6-(hydroxymethyl)-1-(2-methoxyethyl)-5-methylthieno[2,3-d]pyrimidin-2,4(1*H*,3*H*)-dion

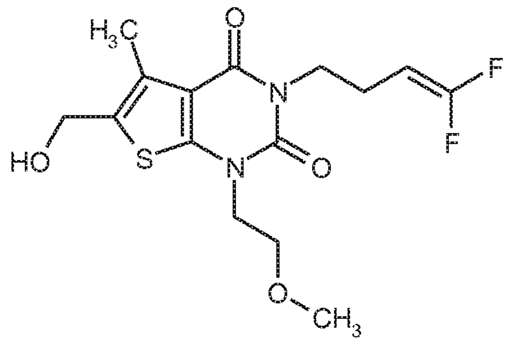

Eine Lösung von 345 mg (0.735 mmol) der Verbindung aus Bsp. 364A in 17 ml trockenem THF wurde bei -40°C tropfenweise mit 735 µl (0.735 mmol) einer 1 M Lösung von Lithiumaluminiumhydrid in THF versetzt. Nach vollständiger Zugabe wurde 1 h bei RT nachgerührt. Dann wurde vorsichtig 1 ml 10%-ige Salzsäure zugesetzt. Das Gemisch wurde mit 100 ml gesättigter KochsalzLösung versetzt, und die wässrige Phase wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Hexan/Ethylacetat). Es wurden 133 mg (49% d. Th.) der Titelverbindung erhalten.

$^1$H-NMR (400 MHz, DMSO-$d_6$, δ/ppm): 5.58 (t, 1H), 4.60-4.44 (m, 3H), 4.04 (t, 2H), 3.92 (t, 2H), 3.63 (t, 2H), 3.23 (s, 3H), 2.32 (s, 3H), 2.26 (q, 2H).

LC/MS (Methode 3): $R_t$ = 1.05 min, m/z = 361 [M+H]$^+$.

**Beispiel 393A**

3-[(2,2-Difluorcyclopropyl)methyl]-6-(hydroxymethyl)-1-(2-methoxyethyl)-5-methylthieno[2,3-d]-pyrimidin-2,4(1*H*,3*H*)-dion (*Racemat*)

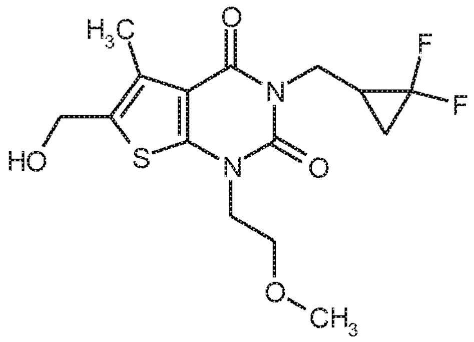

Analog zu dem unter Bsp. 138A (Methode C) beschriebenen Verfahren wurden aus 430 mg (0.870 mmol) der Verbindung aus Bsp. 365A 171 mg (53% d. Th.) der Titelverbindung hergestellt. Die Umsetzung erfolgte hier für 1 h bei RT.
$^1$H-NMR (400 MHz, DMSO-$d_6$, δ/ppm): 5.59 (t, 1H), 4.58 (d, 2H), 4.12-4.02 (m, 3H), 3.98 (d, 1H), 3.65 (t, 2H), 3.24 (s, 3H), 2.33 (s, 3H), 2.16-2.02 (m, 1H), 1.59 (tdd, 1H), 1.39-1.28 (m, 1H).
LC/MS (Methode 3): $R_t$ = 1.0 min, m/z = 361 $[M+H]^+$.

**Beispiel 394A**

6-(Hydroxymethyl)-1-(2-methoxyethyl)-3-(3-methoxypropyl)-5-methylthieno[2,3-d]pyrimidin-2,4(1*H*,3*H*)-dion

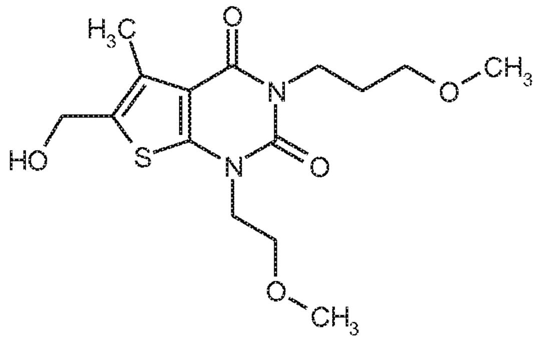

Analog zu dem unter Bsp. 138A (Methode C) beschriebenen Verfahren wurden aus 310 mg (0.673 mmol) der Verbindung aus Bsp. 366A 146 mg (60% d. Th.) der Titelverbindung hergestellt. Die Umsetzung erfolgte hier für 2 h bei RT.
$^1$H-NMR (400 MHz, DMSO-$d_6$, δ/ppm): 5.57 (t, 1H), 4.57 (d, 2H), 4.03 (t, 2H), 3.92 (t, 2H), 3.63 (t, 2H), 3.38-3.34 (m, 2H), 3.24 (s, 3H), 3.20 (s, 3H), 2.32 (s, 3H), 1.76 (m, 2H).
LC/MS (Methode 3): $R_t$ = 0.83 min, m/z = 343 $[M+H]^+$.

**Beispiel 395A**

6-(Hydroxymethyl)-1-(2-methoxyethyl)-5-methyl-3-(tetrahydrofuran-2-ylmethyl)thieno[2,3-d]-pyrimidin-2,4(1*H*,3*H*)-dion (*Racemat*)

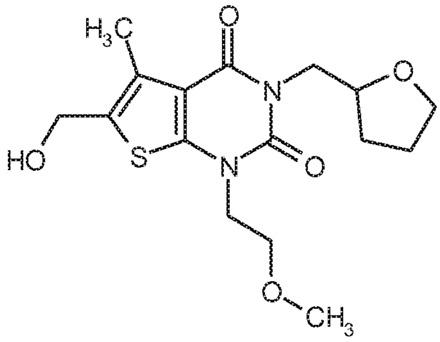

Analog zu dem unter Bsp. 138A (Methode C) beschriebenen Verfahren wurden aus 430 mg (0.912 mmol) der Verbindung aus Bsp. 367A 178 mg (54% d. Th.) der Titelverbindung hergestellt. Die Umsetzung erfolgte hier für 2 h bei RT.
$^1$H-NMR (400 MHz, DMSO-$d_6$, δ/ppm): 5.57 (t, 1H), 4.57 (d, 2H), 4.18-4.09 (m, 1H), 4.09-3.97 (m, 3H), 3.80-3.71 (m, 2H), 3.67-3.56 (m, 3H), 3.24 (s, 3H), 2.32 (s, 3H), 1.94-1.73 (m, 3H), 1.67-1.57 (m, 1H).
LC/MS (Methode 3): $R_t$ = 0.87 min, m/z = 355 $[M+H]^+$.

**Beispiel 396A**

6-(Hydroxymethyl)-1-(2-methoxyethyl)-5-methyl-3-(oxetan-3-ylmethyl)thieno[2,3-d]pyrimidin-2,4(1*H*,3*H*)-dion

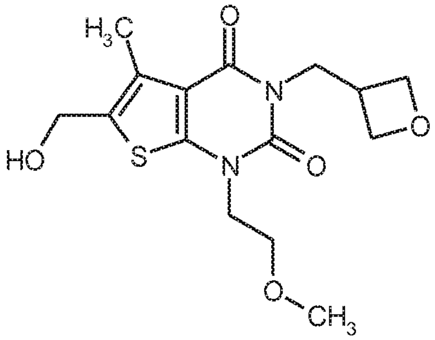

Analog zu dem unter Bsp. 138A (Methode C) beschriebenen Verfahren wurden aus 349 mg (0.789 mmol) der Verbindung aus Bsp. 368A 76 mg (28% d. Th.) der Titelverbindung hergestellt. Die Umsetzung erfolgte hier für 1 h bei RT.
$^1$H-NMR (400 MHz, DMSO-$d_6$, δ/ppm): 5.58 (t, 1H), 4.60-4.55 (m, 4H), 4.40 (t, 2H), 4.16 (d, 2H), 4.03 (t, 2H), 3.63 (t, 2H), 3.30-3.24 (m, 1H), 3.23 (s, 3H), 2.31 (s, 3H).
LC/MS (Methode 3): $R_t$ = 0.76 min, m/z = 341 $[M+H]^+$.

**Beispiel 397A**

6-{[(2-Aminoethyl)amino]methyl}-3-ethyl-1-(fluormethyl)-5-methylthieno[2,3-d]pyrimidin-2,4(1*H*,3*H*)-dion

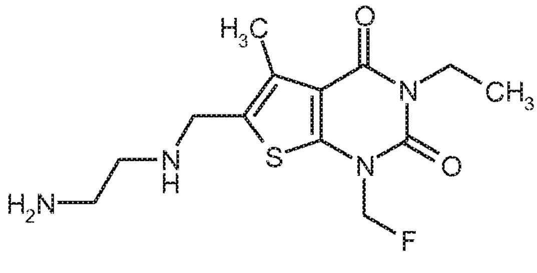

Analog zu dem unter Bsp. 210A beschriebenen Verfahren wurden aus 332 mg (1.15 mmol) der Verbindung aus Bsp. 372A und 1,2-Diaminoethan 440 mg (95% d. Th., 79% Reinheit) der Titelverbindung hergestellt. Die Reaktionszeit betrug hier 66 h.
LC/MS (Methode 3): $R_t$ = 0.48 min, m/z = 315 $[M+H]^+$.

**Beispiel 398A**

6-{[(2-Aminoethyl)amino]methyl}-1-[2-(cyclopentyloxy)ethyl]-3-ethyl-5-methylthieno[2,3-d]-pyrimidin-2,4(1*H*,3*H*)-dion

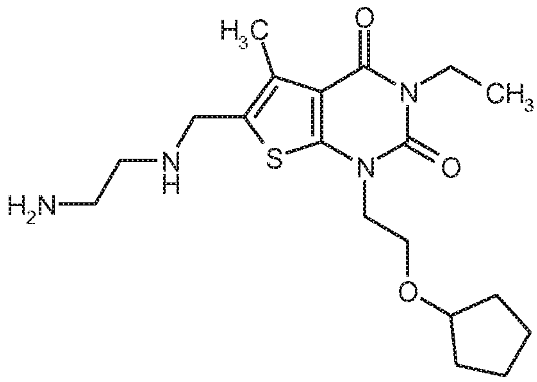

Analog zu dem unter Bsp. 210A beschriebenen Verfahren wurden aus 380 mg (1.08 mmol) der Verbindung aus Bsp. 373A und 1,2-Diaminoethan 510 mg (97% d. Th., 82% Reinheit) der Titelverbindung hergestellt. Die Reaktionszeit betrug hier 89 h.
LC/MS (Methode 3): $R_t$ = 0.70 min, m/z = 395 $[M+H]^+$.

**Beispiel 399A**

6-{[(2-Aminoethyl)amino]methyl)-3-ethyl-1-[2-(ethylsulfanyl)ethyl]-5-methylthieno[2,3-d]pyrimidin-2,4(1*H*,3*H*)-dion

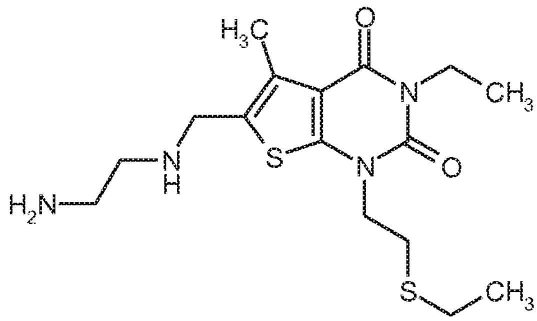

Analog zu dem unter Bsp. 210A beschriebenen Verfahren wurden aus 300 mg (0.891 mmol) der Verbindung aus Bsp. 375A und 1,2-Diaminoethan 512 mg der Titelverbindung hergestellt. Die Reaktionszeit betrug hier 65 h.
LC/MS (Methode 3): $R_t$ = 0.61 min, m/z = 311 $[M+H-C_2H_8N_2]^+$.

**Beispiel 400A**

6-{[(2-Aminoethyl)amino]methyl}-3-ethyl-5-methyl-1-[2-(methylsulfonyl)ethyl]thieno[2,3-d]-pyrimidin-2,4(1*H*,3*H*)-dion

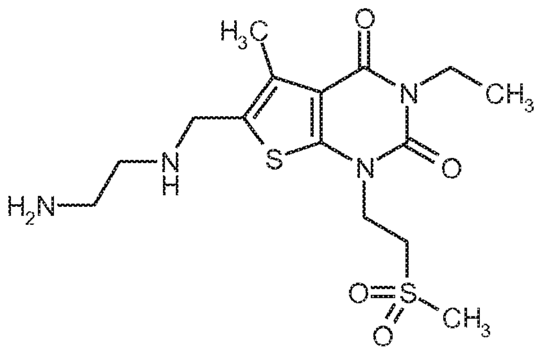

Analog zu dem unter Bsp. 210A beschriebenen Verfahren wurden aus 346 mg (1 mmol) der Verbindung aus Bsp. 376A und 1,2-Diaminoethan 470 mg (42% d. Th., 35% Reinheit) der Titelverbindung hergestellt. Die Reaktionszeit betrug hier 90 h.
LC/MS (Methode 3): $R_t$ = 0.73 min, m/z = 389 $[M+H]^+$.

**Beispiel 401A**

6-1[(2-Aminoethyl)ainino]methyl}-3-ethyl-1-(3-methoxypropyl)-5-methylthieno[2,3-d]pyrimidin-2,4(1*H*,3*H*)-dion

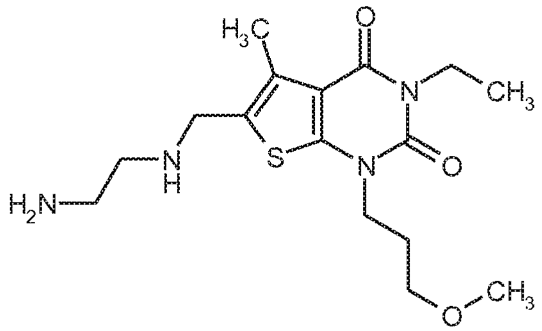

Analog zu dem unter Bsp. 210A beschriebenen Verfahren wurden aus 360 mg (1.13 mmol) der Verbindung aus Bsp. 377A und 1,2-Diaminoethan 310 mg (65% d. Th., 83% Reinheit) der Titelverbindung hergestellt. Die Reaktionszeit betrug hier 93 h.
LC/MS (Methode 3): $R_t$ = 0.55 min, m/z = 355 $[M+H]^+$.

**Beispiel 402A**

6-{[(2-Aminoethyl)amino]methyl}-3-isopropyl-1,5-dimethylthieno[2,3-d]pyrimidin-2,4(1*H*,3*H*)-dion

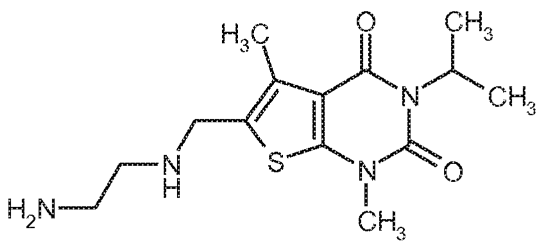

Analog zu dem unter Bsp. 210A beschriebenen Verfahren wurden aus 260 mg (0.976 mmol) der Verbindung aus Bsp. 109A und 1,2-Diaminoethan 266 mg (83% d. Th., 95% Reinheit) der Titelverbindung hergestellt. Die Reaktionszeit betrug hier 97 h.
LC/MS (Methode 3): $R_t$ = 0.50 min, m/z = 251 $[M+H-C_2H_8N_2]^+$.

**Beispiel 403A**

6-{[(2-Aminoethyl)amino]methyl}-1-(fluormethyl)-3-isopropyl-5-methylthieno[2,3-d]pyrimidin-2,4(1*H*,3*H*)-dion

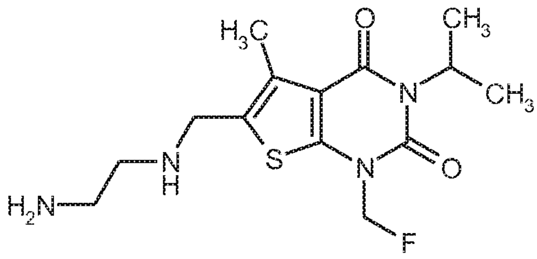

Analog zu dem unter Bsp. 210A beschriebenen Verfahren wurden aus 170 mg (0.562 mmol) der Verbindung aus Bsp. 378A und 1,2-Diaminoethan 119 mg (55% d. Th., 86% Reinheit) der Titelverbindung hergestellt. Die Reaktionszeit betrug hier 97 h.
LC/MS (Methode 3): $R_t$ = 0.57 min, m/z = 269 $[M+H-C_2H_8N_2]^+$.

**Beispiel 404A**

6-{[(2-Aminoethyl)amino]methyl}-1-(3-fluoropropyl)-3-isopropyl-5-methylthieno[2,3-d]pyrimidin-2,4(1*H*,3*H*)-dion

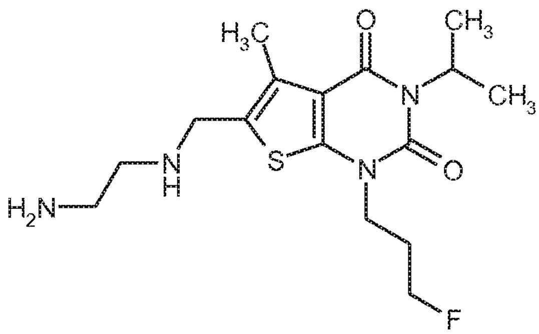

340 mg (1.08 mmol) der Verbindung aus Bsp. 379A wurden in einem Gemisch aus 18 ml Methanol und 8 ml Dichlormethan gelöst. Dann wurden bei RT 720 µl (10.8 mmol) 1,2-Diaminoethan und 247 µl (4.31 mmol) Essigsäure zugesetzt. Es wurde 30 min bei RT gerührt, und danach wurden 285 mg (4.31 mmol) Natriumcyanoborhydrid hinzugefügt. Nachdem das Reaktionsgemisch 44 h bei 60°C gerührt worden war, wurde mit 80 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 406 mg (70% d. Th., 67% Reinheit) der Titelverbindung, welche ohne weitere Reinigung für Folgereaktionen verwendet wurden.
LC/MS (Methode 3): $R_t$ = 0.64 min, m/z = 357 $[M+H]^+$.

**Beispiel 405A**

6-{[(2-Aminoethyl)amino]methyl}-3-(2-methoxyethyl)-5-methyl-1-(oxetan-2-ylmethyl)thieno-[2,3-d]pyrimidin-2,4(1*H*,3*H*)-dion (*Racemat*)

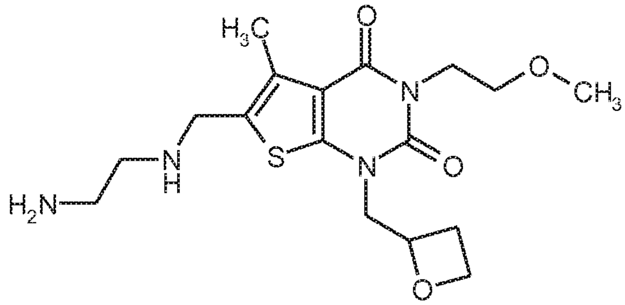

Analog zu dem unter Bsp. 245A beschriebenen Verfahren wurden aus 1.30 g (3.76 mmol) der Verbindung aus Bsp. 380A und 1.36 g (22.6 mmol) 1,2-Diaminoethan 956 mg (66% d. Th.) der Titelverbindung hergestellt. Die Reaktionszeit betrug hier ca. 18 h.
LC/MS (Methode 6, ESIpos): $R_t$ = 0.96 min, m/z = 383 $[M+H]^+$.

**Beispiel 406A**

6-{[(2-Aminoethyl)amino]methyl}-3-(2-ethoxyethyl)-5-methyl-1-(3,3,3-trifluorpropyl)thieno-[2,3-d]pyrimidin-2,4(1*H*,3*H*)-dion

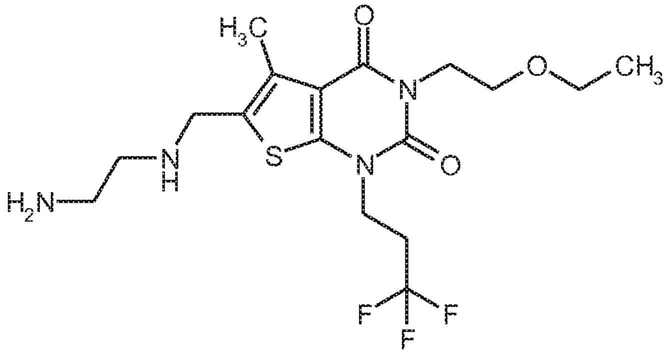

430 mg (1.14 mmol) der Verbindung aus Bsp. 381A wurden in einem Gemisch aus 19 ml Methanol und 8 ml Dichlormethan gelöst. Dann wurden bei RT 760 µl (11.4 mmol) 1,2-Diaminoethan und 260 µl (4.54 mmol) Essigsäure zugesetzt. Es wurde 30 min bei RT gerührt, und danach wurden 301 mg (4.54 mmol) Natriumcyanoborhydrid hinzugefügt. Nachdem das Reaktionsgemisch 72 h bei 60°C gerührt worden war, wurde mit 50 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 486 mg (90% d. Th., 89% Reinheit) der Titelverbindung, welche ohne weitere Reinigung für Folgereaktionen verwendet wurden.
LC/MS (Methode 3): $R_t$ = 0.62 min, m/z = 423 $[M+H]^+$.

**Beispiel 407A**

6-{[(2-Aminoethyl)amino]methyl)-3-(2-ethoxyethyl)-1-(2-methoxyethyl)-5-methylthieno[2,3-d]-pyrimidin-2,4(1*H*,3*H*)-dion

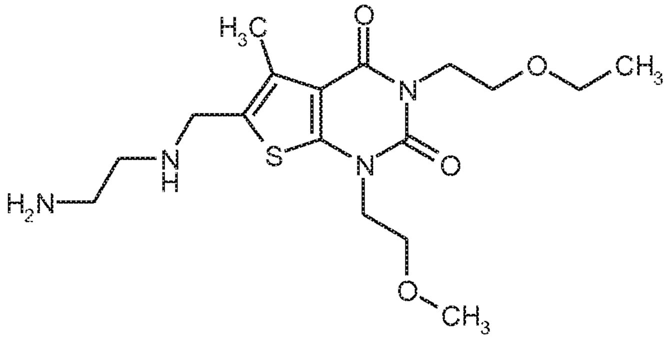

Analog zu dem unter Bsp. 210A beschriebenen Verfahren wurden aus 345 mg (0.983 mmol) der Verbindung aus Bsp. 382A und 1,2-Diaminoethan 185 mg (33% d. Th., 68% Reinheit) der Titelverbindung hergestellt. Die Reaktionszeit betrug hier 72 h.
LC/MS (Methode 3): $R_t$ = 0.53 min, m/z = 385 $[M+H]^+$.

## Beispiel 408A

6-1[(2,2-Dimethoxyethyl)aniino]methyl)-5-methyl-3-(2,2,2-trifluorethyl)-1-(3,3,3-trifluorpropyl)-thieno[2,3-d]pyrimidin-2,4(1*H*,3*H*)-dion

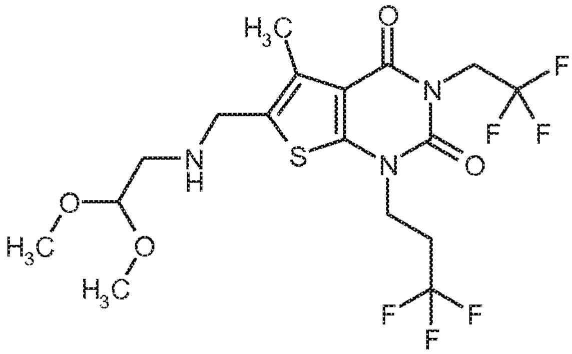

470 mg (1.21 mmol) der Verbindung aus Bsp. 120A und 191 mg (1.82 mmol) 2,2-Dimethoxyethanamin wurden in 25 ml Dichlormethan gelöst und 1 h zum Rückfluß erhitzt. Nach dem Abkühlen auf RT wurden 770 mg (3.63 mmol) Natriumtriacetoxyborhydrid hinzugefügt. Das Reaktionsgemisch wurde ca. 18 h bei RT gerührt. Da der Umsatz nach dieser Zeit noch nicht vollständig war, wurden weitere 64 mg (0.605 mmol) 2,2-Dimethoxyethanamin und 257 mg (1.21 mmol) Natriumtriacetoxyborhydrid hinzugefügt. Nach weiterem Rühren für ca. 24 h bei RT wurde das Reaktionsgemisch mit Ethylacetat verdünnt und nacheinander mit gesättigter wässriger Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels MPLC gereinigt (Isolera One mit Biotage-Kartusche SNAP KP-Sil, 25 g Kieselgel, Laufmittel Cyclohexan/Ethylacetat 1:1). Nach Vereinigen der Produktfraktionen, Einengen und Trocknen im Hochvakuum wurden 433 mg (74% d. Th.) der Titelverbindung erhalten.
$^1$H-NMR (400 MHz, DMSO-$d_6$, δ/ppm): 4.70 (q, 2H), 4.42 (t, 1H), 4.15 (t, 2H), 3.85 (s, 2H), 3.31 (s, 3H), 2.87-2.70 (m, 2H), 2.64 (d, 2H), 2.54 (s, 3H), 2.34 (s, 3H).
LC/MS (Methode 17, ESIpos): $R_t$ = 1.12 min, m/z = 478.12 $[M+H]^+$.

## Beispiel 409A

6-{[(2,2-Dimethoxyethyl)amino]methyl}-1-(2-methoxyethyl)-5-methyl-3-(2,2,2-trifluorethyl)-thieno[2,3-d]pyrimidin-2,4(1*H*,3*H*)-dion

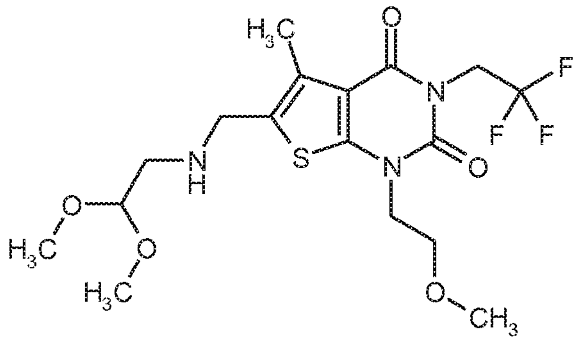

500 mg (1.43 mmol) der Verbindung aus Bsp. 122A und 225 mg (2.14 mmol) 2,2-Dimethoxyethanamin wurden in 25 ml Dichlormethan gelöst und 1 h zum Rückfluß erhitzt. Nach dem Abkühlen auf RT wurden 907 mg (4.28 mmol) Natriumtriacetoxyborhydrid hinzugefügt. Das Reaktionsgemisch wurde ca. 18 h bei RT gerührt. Dann wurde das Gemisch mit Ethylacetat verdünnt und nacheinander mit gesättigter wässriger Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels MPLC gereinigt (Isolera One mit Biotage-Kartusche SNAP KP-Sil, 25 g Kieselgel, Laufmittel Cyclohexan/Ethylacetat 1:1). Nach Vereinigen der Produktfraktionen, Einengen und Trocknen im Hochvakuum wurden 460 mg (73% d. Th.) der Titelverbindung erhalten.
$^1$H-NMR (400 MHz, DMSO-$d_6$, $\delta$/ppm): 4.69 (q, 2H), 4.41 (t, 1H), 4.07 (t, 2H), 3.83 (s, 2H), 3.64 (t, 2H), 3.27 (s, 6H), 3.24 (s, 3H), 2.63 (d, 2H), 2.32 (s, 3H).
LC/MS (Methode 17, ESIpos): $R_t$ = 0.96 min, m/z = 440.15 [M+H]$^+$.

**Beispiel 410A**

6-{[(2,2-Dimethoxyethyl)amino]methyl}-5-methyl-1-(tetrahydrofuran-2-ylmethyl)-3-(2,2,2-trifluorethyl)thieno[2,3-d]pyrimidin-2,4(1*H*,3*H*)-dion (*Racemat*)

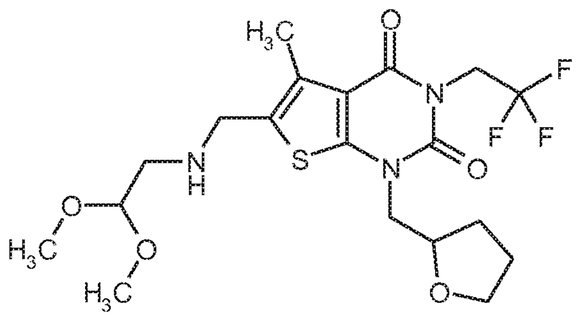

Analog zu dem unter Bsp. 268A beschriebenen Verfahren wurden aus 600 mg (1.59 mmol) der Verbindung aus Bsp. 125A und 251 mg (2.39 mmol) 2,2-Dimethoxyethanamin 638 mg (85% d. Th.) der Titelverbindung hergestellt. Die Chromatographie erfolgte hier an einer Biotage Isolera One-Anlage mit einer Biotage-Kartusche (SNAP KP-Sil, 50 g Kieselgel) und Cyclohexan/Ethylacetat 1:1 als Laufmittel.
$^1$H-NMR (400 MHz, DMSO-$d_6$, $\delta$/ppm): 4.70 (q, 2H), 4.41 (t, 1H), 4.29-4.18 (m, 1H), 4.10-4.00 (m, 1H), 3.83 (s, 2H), 3.81-3.70 (m, 2H), 3.66-3.56 (m, 1H), 3.27 (s, 6H), 2.63 (d, 2H), 2.33 (s, 3H), 2.03-1.79 (m, 3H), 1.71-1.63 (m, 1H).
LC/MS (Methode 1, ESIpos): $R_t$ = 0.63 min, m/z = 466 [M+H]$^+$.

**Beispiel 411A**

6-{[(2,2-Dimethoxyethyl)amino]methyl}-3-(2-methoxyethyl)-5-methyl-1-(3,3,3-trifluorpropyl)-thieno[2,3-d]pyrimidin-2,4(1*H*,3*H*)-dion

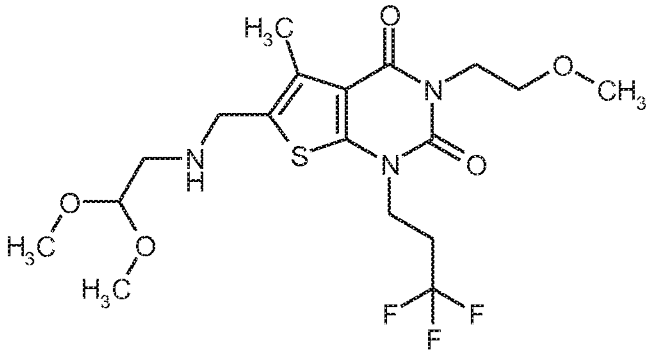

500 mg (1.37 mmol) der Verbindung aus Bsp. 296A und 216 mg (2.06 mmol) 2,2-Dimethoxyethanamin wurden in 30 ml Dichlormethan gelöst und 1 h zum Rückfluß erhitzt. Nach dem Abkühlen auf RT wurden 872 mg (4.12 mmol) Natriumtriacetoxyborhydrid hinzugefügt. Das Reaktionsgemisch wurde 2.5 Tage bei RT gerührt. Dann wurde das Gemisch mit Ethylacetat verdünnt und nacheinander mit gesättigter wässriger Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels MPLC gereinigt (Isolera One mit Biotage-Kartusche SNAP KP-Sil, 50 g Kieselgel, Laufmittel Cyclohexan/Ethylacetat 1:1). Nach Vereinigen der Produktfraktionen, Einengen und Trocknen im Hochvakuum wurden 455 mg (73% d. Th.) der Titelverbindung erhalten.

$^1$H-NMR (400 MHz, DMSO-$d_6$, $\delta$/ppm): 4.41 (t, 1H), 4.11 (t, 2H), 4.05 (t, 2H), 3.84 (s, 2H), 3.49 (t, 2H), 3.27 (s, 6H), 3.24 (s, 3H), 2.84-2.68 (m, 2H), 2.63 (d, 2H), 2.33 (s, 3H).

LC/MS (Methode 17, ESIneg): $R_t$ = 0.93 min, m/z = 498.15 [M-H+HCOOH]$^-$.

**Beispiel 412A**

6-{[(2,2-Dimethoxyethyl)amino]methyl}-1,3-bis(2-methoxyethyl)-5-methylthieno[2,3-d]pyrimidin-2,4(1*H*,3*H*)-dion

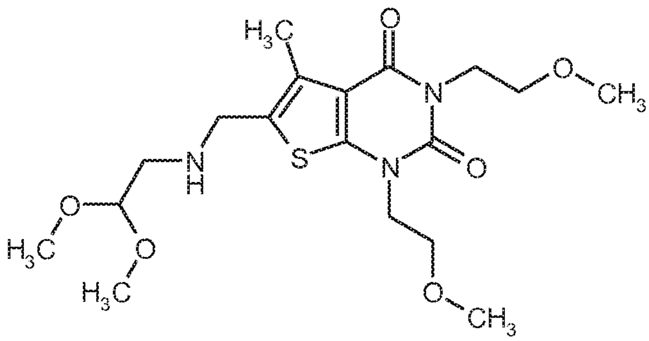

Analog zu dem unter Bsp. 268A beschriebenen Verfahren wurden aus 500 mg (1.53 mmol) der Verbindung aus Bsp. 136A und 241 mg (2.30 mmol) 2,2-Dimethoxyethanamin 533 mg (83% d. Th.) der Titelverbindung hergestellt. Die Chromatographie erfolgte hier an einer Biotage Isolera One-Anlage mit einer Biotage-Kartusche (SNAP KP-Sil, 50 g Kieselgel) und Cyclohexan/Ethylacetat (33:67 $\rightarrow$ 0:100) als Laufmittel.

$^1$H-NMR (400 MHz, DMSO-$d_6$, $\delta$/ppm): 4.41 (t, 1H), 4.05 (t, 2H), 4.03 (t, 2H), 3.82 (s, 2H), 3.63 (t, 2H), 3.49 (t, 2H), 3.26 (s, 6H), 3.24 (2s, 6H), 2.62 (d, 2H), 2.32 (s, 3H).

LC/MS (Methode 17, ESIpos): $R_t$ = 0.77 min, m/z = 311.11 [M+H]-$C_4H_{11}NO_2$]$^+$.

**Beispiel 413A**

6-{[(2,2-Dimethoxyethyl)amino]methyl}-3-(2-methoxyethyl)-5-methyl-1-(tetrahydrofuran-2-ylmethyl)thieno[2,3-d]pyrimidin-2,4(1*H*,3*H*)-dion (*Racemat*)

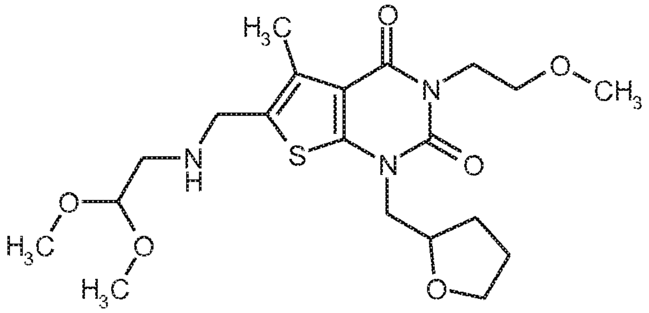

Analog zu dem unter Bsp. 268A beschriebenen Verfahren wurden aus 900 mg (2.55 mmol) der Verbindung aus Bsp. 298A und 403 mg (3.83 mmol) 2,2-Dimethoxyethanamin 745 mg (66% d. Th.) der Titelverbindung hergestellt. Die Chromatographie erfolgte hier an einer Biotage Isolera One-Anlage mit einer Biotage-Kartusche (SNAP KP-Sil, 100 g Kieselgel) und Cyclohexan/Ethylacetat (50:50 → 0:100) als Laufmittel.
$^1$H-NMR (400 MHz, DMSO-$d_6$, δ/ppm): 4.41 (t, 1H), 4.27-4.17 (m, 1H), 4.10-3.97 (m, 3H), 3.82 (s, 2H), 3.79-3.68 (m, 2H), 3.66-3.57 (m, 1H), 3.50 (t, 2H), 3.26 (s, 6H), 3.24 (s, 3H), 2.62 (d, 2H), 2.32 (s, 3H), 2.03-1.75 (m, 3H), 1.72-1.60 (m, 1H).
LC/MS (Methode 1, ESIneg): $R_t$ = 0.53 min, m/z = 486 $[M-H+HCOOH]^-$.

**Beispiel 414A**

1-(2,2-Dimethoxyethyl)-1-{[3-(2-methoxyethyl)-5-methyl-2,4-dioxo-1-(tetrahydrofuran-2-yl-methyl)-1,2,3,4-tetrahydrothieno[2,3-d]pyrimidin-6-yl]methyl}harnstoff (*Racemat*)

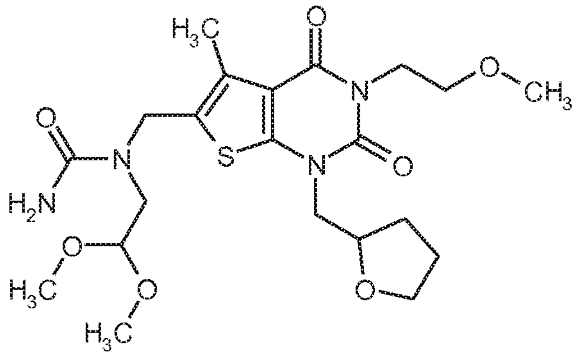

Eine Lösung von 735 mg (1.66 mmol) der Verbindung aus Bsp. 413A in 17 ml Methanol wurde bei RT zunächst mit 311 mg (3.83 mmol) Kaliumcyanat und dann mit 244 μl (2.83 mmol) Perchlorsäure (70% in Wasser) versetzt. Nach 2.5 Tagen Rühren bei RT wurden noch einmal die gleichen Mengen an Kaliumcyanat und Perchlorsäure zugesetzt, und das Rühren wurde für weitere 3 Tage fortgesetzt. Danach wurde das Reaktionsgemisch mit gesättigter wässriger Natriumhydrogencarbonat-Lösung versetzt und anschließend mit Ethylacetat extrahiert. Der organische Extrakt wurde nacheinander mit Wasser und gesättigter Natriumchlorid-Lösung gewaschen, über wasserfreiem Magnesiumsulfat getrocknet, filtriert und eingeengt. Nach Trocknen des erhaltenen Rückstands im Hochvakuum wurden 905 mg (97% d. Th., 87% Reinheit) der Titelverbindung erhalten, die ohne weitere Aufreinigung für Folgereaktionen verwendet wurden.
LC/MS (Methode 1, ESIpos): $R_t$ = 0.72 min, m/z = 485 $[M+H]^+$.

**Beispiel 415A**

*tert.*-Butyl-2-{[5-methyl-2,4-dioxo-3-(2,2,2-trifluorethyl)-1-(3,3,3-trifluorpropyl)-1,2,3,4-tetrahydrothieno[2,3-d]pyrimidin-6-yl]methylen}hydrazincarboxylat

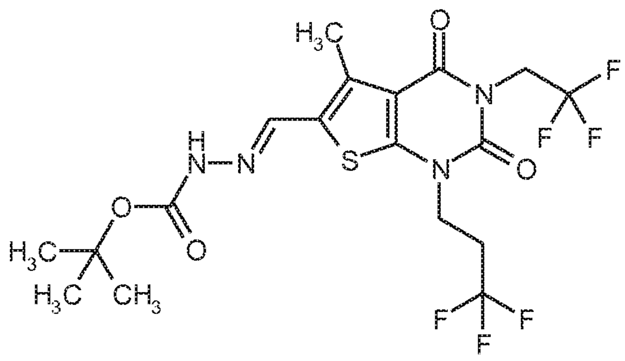

Analog zu dem unter Bsp. 333A beschriebenen Verfahren wurden aus 500 mg (1.29 mmol) der Verbindung aus Bsp. 120A und 255 mg (1.93 mmol) *tert.*-Butyl-hydrazincarboxylat 603 mg (93% d. Th.) der Titelverbindung erhalten. $^1$H-NMR (400 MHz, DMSO-$d_6$, δ/ppm): 10.92 (breit, 1H), 8.31 (s, 1H), 4.70 (q, 2H), 4.19 (t, 2H), 2.93-2.71 (m, 2H), 2.46 (s, 3H), 1.46 (s, 9H).
LC/MS (Methode 17, ESIneg): $R_t$ = 2.13 min, m/z = 501.10 [M-H]$^-$.

## Beispiel 416A

*tert.*-Butyl-2-{[1-(2-methoxyethyl)-5-methyl-2,4-dioxo-3-(2,2,2-trifluorethyl)-1,2,3,4-tetrahydrothieno[2,3-d]pyrimidin-6-yl]methylen}hydrazincarboxylat

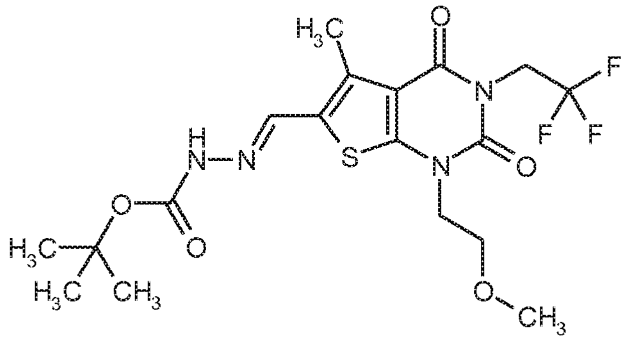

Analog zu dem unter Bsp. 333A beschriebenen Verfahren wurden aus 600 mg (1.71 mmol) der Verbindung aus Bsp. 122A und 339 mg (2.57 mmol) *tert.*-Butyl-hydrazincarboxylat 735 mg (92% d. Th.) der Titelverbindung erhalten. $^1$H-NMR (400 MHz, DMSO-$d_6$, δ/ppm): 10.89 (breit, 1H), 8.29 (s, 1H), 4.69 (q, 2H), 4.11 (t, 2H), 3.66 (t, 2H), 3.25 (s, 3H), 2.45 (s, 3H), 1.46 (s, 9H).
LC/MS (Methode 1, ESIpos): $R_t$ = 1.11 min, m/z = 465 [M+H]$^+$.

## Beispiel 417A

*tert.*-Butyl-2-{[5-methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methylen}hydrazincarboxylat (*Racemat*)

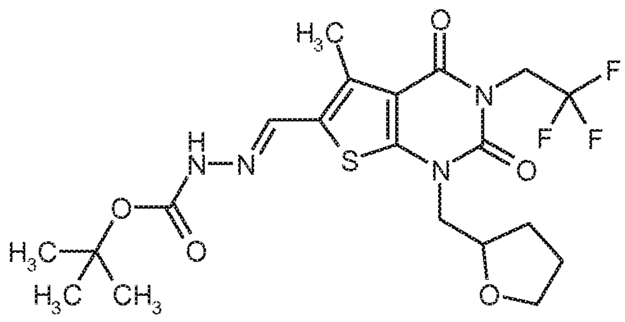

Analog zu dem unter Bsp. 333A beschriebenen Verfahren wurden aus 1.11 g (2.96 mmol) der Verbindung aus Bsp. 125A und 587 mg (4.44 mmol) tert.-Butyl-hydrazincarboxylat 1.39 g (95% d. Th.) der Titelverbindung erhalten.
$^1$H-NMR (400 MHz, DMSO-$d_6$, δ/ppm): 10.89 (breit, 1H), 8.30 (s, 1H), 4.70 (q, 2H), 4.29-4.17 (m, 1H), 4.12 (dd, 1H), 3.88-3.71 (m, 2H), 3.68-3.57 (m, 1H), 2.45 (s, 3H), 2.09-1.75 (m, 3H), 1.73-1.59 (m, 1H), 1.46 (s, 9H).
LC/MS (Methode 17, ESIneg): $R_t$ = 2.08 min, m/z = 489.14 $[M-H]^-$.

**Beispiel 418A**

*tert.*-Butyl-2-{[3-(2-methoxyethyl)-5-methyl-2,4-dioxo-1-(3,3,3-trifluorpropyl)-1,2,3,4-tetrahydrothieno[2,3-d]pyrimidin-6-yl]methylen}hydrazincarboxylat

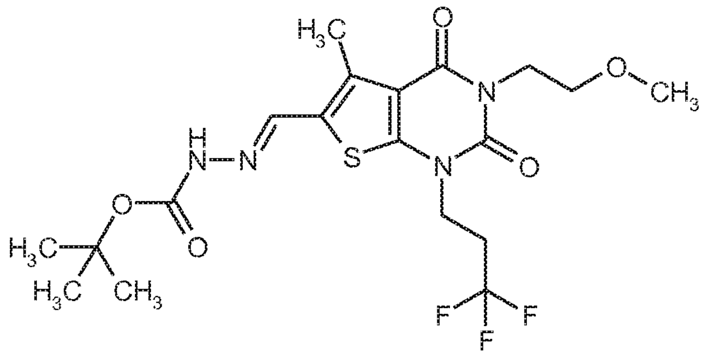

Analog zu dem unter Bsp. 333A beschriebenen Verfahren wurden aus 600 mg (1.65 mmol) der Verbindung aus Bsp. 296A und 326 mg (2.47 mmol) *tert.*-Butyl-hydrazincarboxylat 741 mg (94% d. Th.) der Titelverbindung erhalten.
$^1$H-NMR (400 MHz, DMSO-$d_6$, δ/ppm): 10.88 (breit, 1H), 8.30 (s, 1H), 4.15 (t, 2H), 4.05 (t, 2H), 3.50 (t, 2H), 3.24 (s, 3H), 2.87-2.69 (m, 2H), 2.46 (s, 3H), 1.46 (s, 9H).
LC/MS (Methode 1, ESIpos): $R_t$ = 1.11 min, m/z = 479 $[M+H]^+$.

**Beispiel 419A**

*tert.*-Butyl-2-{[1,3-bis(2-methoxyethyl)-5-methyl-2,4-dioxo-1,2,3,4-tetrahydrothieno[2,3-d]pyrimidin-6-yl]methylen}hydrazincarboxylat

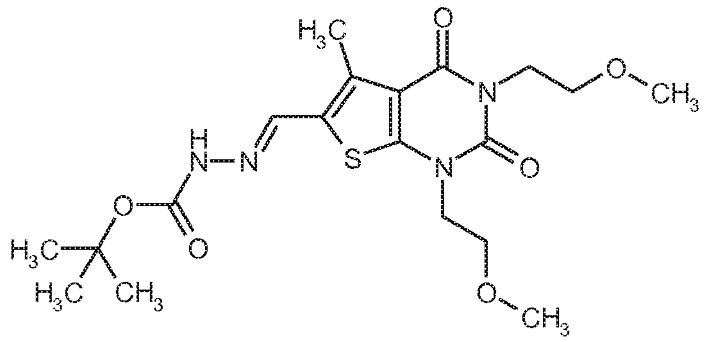

Analog zu dem unter Bsp. 333A beschriebenen Verfahren wurden aus 600 mg (1.84 mmol) der Verbindung aus Bsp. 136A und 364 mg (2.76 mmol) *tert.*-Butyl-hydrazincarboxylat 753 mg (92% d. Th.) der Titelverbindung erhalten.
$^1$H-NMR (400 MHz, DMSO-$d_6$, δ/ppm): 10.86 (breit, 1H), 8.29 (s, 1H), 4.11-4.01 (m, 4H), 3.65 (t, 2H), 3.50 (t, 2H), 3.25 (s, 3H), 3.24 (s, 3H), 2.44 (s, 3H), 1.46 (s, 9H).
LC/MS (Methode 1, ESIpos): $R_t$ = 0.99 min, m/z = 441 [M+H]$^+$.

## Beispiel 420A

*tert.*-Butyl-2-{[3-(2-methoxyethyl)-5-methyl-2,4-dioxo-1-(tetrahydrofuran-2-ylmethyl)-1,2,3,4-tetrahydrothieno[2,3-d]pyrimidin-6-yl]methylen}hydrazincarboxylat (*Racemat*)

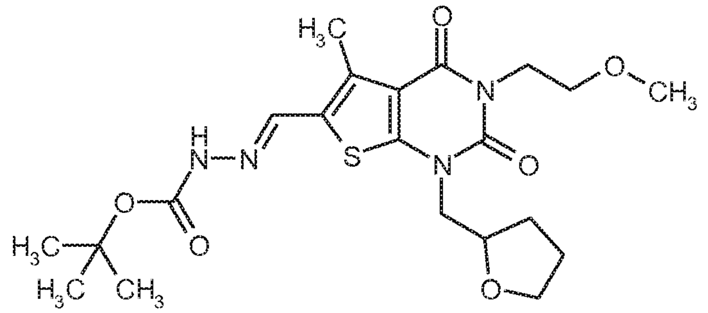

Analog zu dem unter Bsp. 333A beschriebenen Verfahren wurden aus 1.11 g (3.12 mmol) der Verbindung aus Bsp. 298A und 619 mg (4.68 mmol) *tert.*-Butyl-hydrazincarboxylat 1.34 g (92% d. Th.) der Titelverbindung erhalten.
$^1$H-NMR (400 MHz, DMSO-$d_6$, δ/ppm): 10.86 (breit, 1H), 8.29 (s, 1H), 4.28-4.16 (m, 1H), 4.14-4.01 (m, 3H), 3.82-3.72 (m, 2H), 3.67-3.57 (m, 1H), 3.54-3.47 (m, 2H), 3.24 (s, 3H), 2.45 (s, 3H), 2.07-1.76 (m, 3H), 1.72-1.61 (m, 1H), 1.45 (s, 9H).
LC/MS (Methode 17, ESIneg): $R_t$ = 1.86 min, m/z = 465.18 [M-H]$^-$.

## Beispiel 421A

*tert.*-Butyl-2-{[5-methyl-2,4-dioxo-3-(2,2,2-trifluorethyl)-1-(3,3,3-trifluorpropyl)-1,2,3,4-tetrahydrothieno[2,3-d]pyrimidin-6-yl]methyl}hydrazincarboxylat

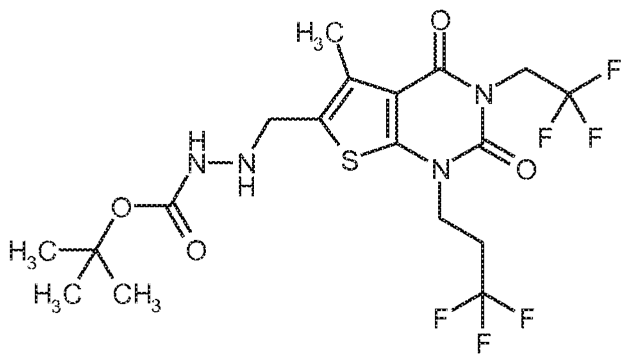

Analog zu dem unter Bsp. 343A beschriebenen Verfahren wurden aus 600 mg (1.19 mmol) der Verbindung aus Bsp. 415A und insgesamt 750 mg (11.9 mmol) Natriumcyanoborhydrid 415 mg (68% d. Th.) der Titelverbindung erhalten.

$^{1}$H-NMR (400 MHz, DMSO-$d_6$, δ/ppm): 8.27 (breit, 1H), 5.12 (breit, 1H), 4.70 (q, 2H), 4.17 (t, 2H), 4.00 (d, 2H), 2.87-2.69 (m, 2H), 2.33 (s, 3H), 1.38 (s, 9H).

LC/MS (Methode 17, ESIpos): $R_t$ = 2.03 min, m/z = 373.04 $[M+H-C_5H_{12}N_2O_2]^+$.

**Beispiel 422A**

*tert.*-Butyl-2-{[1-(2-methoxyethyl)-5-methyl-2,4-dioxo-3-(2,2,2-trifluorethyl)-1,2,3,4-tetrahydrothieno[2,3-d]pyrimidin-6-yl]methyl}hydrazincarboxylat

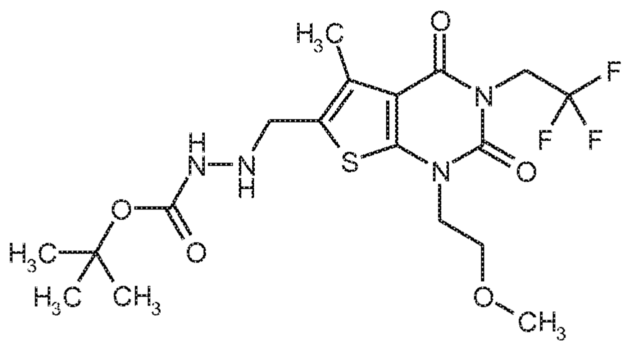

Analog zu dem unter Bsp. 343A beschriebenen Verfahren wurden aus 730 mg (1.57 mmol) der Verbindung aus Bsp. 416A und insgesamt 988 mg (15.7 mmol) Natriumcyanoborhydrid 665 mg (90% d. Th.) der Titelverbindung erhalten.

$^{1}$H-NMR (400 MHz, DMSO-$d_6$, δ/ppm): 8.27 (breit, 1H), 5.04 (breit, 1H), 4.70 (q, 2H), 4.07 (t, 2H), 3.98 (d, 2H), 3.66 (t, 2H), 3.25 (s, 3H), 2.32 (s, 3H), 1.38 (s, 9H).

LC/MS (Methode 1, ESIpos): $R_t$ = 1.05 min, m/z = 335 $[M+H-C_5H_{12}N_2O_2]^+$.

**Beispiel 423A**

*tert.*-Butyl-2-{[5-methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl}hydrazincarboxylat (*Racemat*)

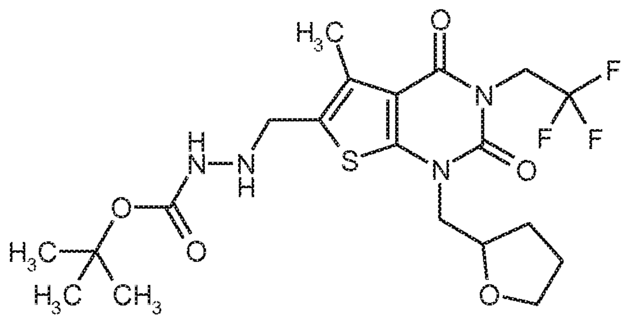

Analog zu dem unter Bsp. 343A beschriebenen Verfahren wurden aus 1.39 g (2.83 mmol) der Verbindung aus Bsp. 417A und insgesamt 1.33 g (21.2 mmol) Natriumcyanoborhydrid 1.27 g (91% d. Th.) der Titelverbindung erhalten.
$^1$H-NMR (400 MHz, DMSO-$d_6$, $\delta$/ppm): 8.27 (breit, 1H), 5.03 (breit, 1H), 4.71 (q, 2H), 4.36-4.20 (m, 1H), 4.09-3.89 (m, 3H), 3.85-3.72 (m, 2H), 3.68-3.56 (m, 1H), 2.32 (s, 3H), 2.07-1.76 (m, 3H), 1.74-1.61 (m, 1H), 1.38 (s, 9H).
LC/MS (Methode 17, ESIneg): $R_t$ = 1.98 min, m/z = 491.16 $[M-H]^-$.

**Beispiel 424A**

*tert.*-Butyl-2-{[3-(2-methoxyethyl)-5-methyl-2,4-dioxo-1-(3,3,3-trifluorpropyl)-1,2,3,4-tetrahydrothieno[2,3-d]pyrimidin-6-yl]methyl}hydrazincarboxylat

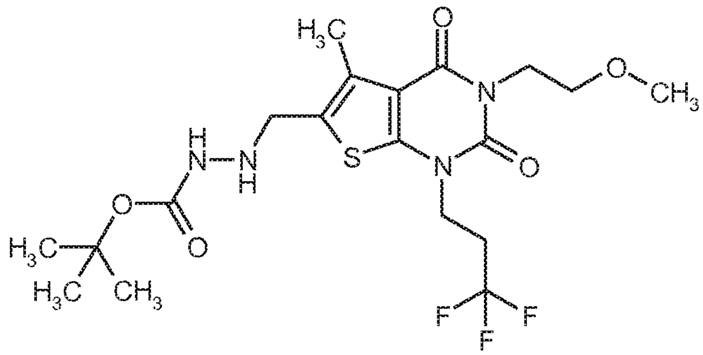

Analog zu dem unter Bsp. 343A beschriebenen Verfahren wurden aus 740 mg (1.55 mmol) der Verbindung aus Bsp. 418A und insgesamt 972 mg (15.5 mmol) Natriumcyanoborhydrid 652 mg (87% d. Th.) der Titelverbindung erhalten.
$^1$H-NMR (400 MHz, DMSO-$d_6$, $\delta$/ppm): 8.26 (breit, 1H), 5.08 (breit, 1H), 4.13 (t, 2H), 4.06 (t, 2H), 3.99 (d, 2H), 3.49 (t, 2H), 3.31 (s, 3H), 2.86-2.68 (m, 2H), 2.33 (s, 3H), 1.39 (s, 9H).
LC/MS (Methode 17, ESIneg): $R_t$ = 1.87 min, m/z = 525.16 $[M-H+HCOOH]^-$.

**Beispiel 425A**

*tert.*-Butyl-2-{[1,3-bis(2-methoxyethyl)-5-methyl-2,4-dioxo-1,2,3,4-tetrahydrothieno[2,3-d]pyrimidin-6-yl]methyl}hydrazincarboxylat

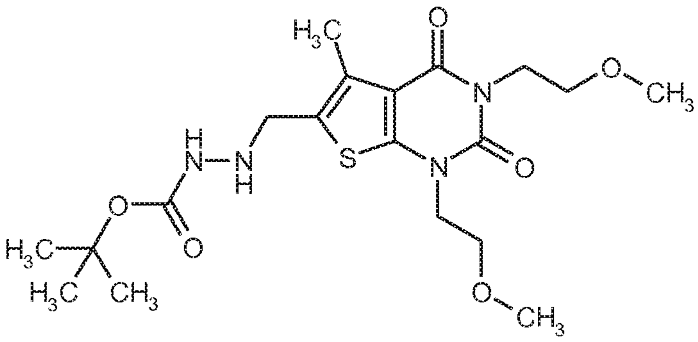

Analog zu dem unter Bsp. 343A beschriebenen Verfahren wurden aus 750 mg (1.70 mmol) der Verbindung aus Bsp. 419A und insgesamt 1.07 g (17.0 mmol) Natriumcyanoborhydrid 571 mg (75% d. Th.) der Titelverbindung erhalten. Anstelle der MPLC-Reinigung wurde hier eine erste Fraktion der Titelverbindung durch Verrühren des Rohprodukts mit Acetonitril bei RT gewonnen. Eine zweite Fraktion des Produktes wurde durch präparative HPLC (Methode 8) der Mutterlauge isoliert.
$^1$H-NMR (400 MHz, DMSO-$d_6$, δ/ppm): 8.25 (breit, 1H), 4.99 (breit, 1H), 4.06 (t, 2H), 4.03 (t, 2H), 3.97 (d, 2H), 3.64 (t, 2H), 3.49 (t, 2H), 3.26 (s, 3H), 3.24 (s, 3H), 2.32 (s, 3H), 1.39 (s, 9H).
LC/MS (Methode 1, ESIpos): $R_t$ = 0.85 min, m/z = 443 $[M+H]^+$.

**Beispiel 426A**

*tert.*-Butyl-2-{[3-(2-methoxyethyl)-5-methyl-2,4-dioxo-1-(tetrahydrofuran-2-ylmethyl)-1,2,3,4-tetrahydrothieno[2,3-d]pyrimidin-6-yl]methyl}hydrazincarboxylat (*Racemat*)

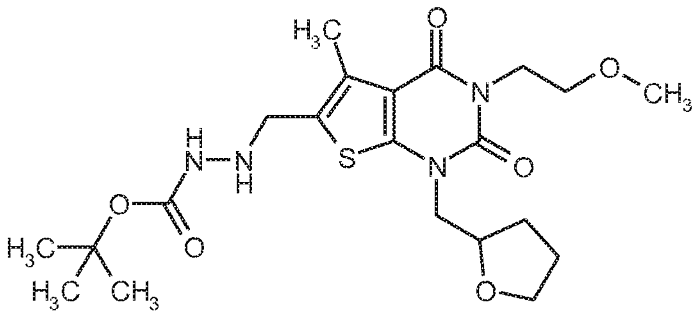

Analog zu dem unter Bsp. 343A beschriebenen Verfahren wurden aus 1.05 g (2.24 mmol) der Verbindung aus Bsp. 420A und insgesamt 1.06 g (16.8 mmol) Natriumcyanoborhydrid 750 mg (71% d. Th.) der Titelverbindung erhalten.
$^1$H-NMR (400 MHz, DMSO-$d_6$, δ/ppm): 8.26 (breit, 1H), 4.98 (breit, 1H), 4.31-4.20 (m, 1H), 4.10-3.93 (m, 5H), 3.83-3.69 (m, 2H), 3.66-3.57 (m, 1H), 3.50 (t, 2H), 3.24 (s, 3H), 2.32 (s, 3H), 2.05-1.75 (m, 3H), 1.73-1.61 (m, 1H), 1.39 (s, 9H).
LC/MS (Methode 2, ESIpos): $R_t$ = 2.70 min, m/z = 469 $[M+H]^+$.

**Beispiel 427A**

*tert.*-Butyl-2-(3-ethoxyprop-2-enoyl)-2-{[5-methyl-2,4-dioxo-3-(2,2,2-trifluorethyl)-1-(3,3,3-trifluorpiopyl)-1,2,3,4-tetrahydrothieno[2,3-d]pyrimidin-6-yl]methyl}hydrazincarboxylat

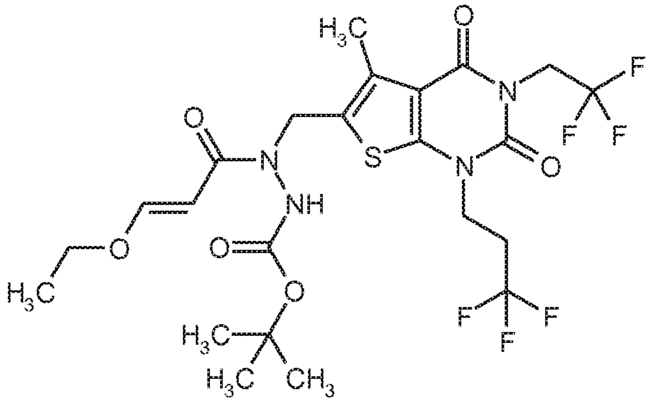

Analog zu dem unter Bsp. 348A beschriebenen Verfahren wurden aus 400 mg (0.793 mmol) der Verbindung aus Bsp. 421A und 151 mg (0.952 mmol, 85% Gehalt) 3-Ethoxyacryloylchlorid 381 mg (79% d. Th.) der Titelverbindung erhalten.
$^1$H-NMR (400 MHz, DMSO-$d_6$, $\delta$/ppm): 9.50 (breit, 1H), 7.51 (d, 1H), 5.57 (d, 1H), 4.91 (breit, 1H), 4.72 (q, 2H), 4.52 (breit, 1H), 4.16 (t, 2H), 4.04-3.80 (m, 2H), 2.86-2.66 (m, 2H), 2.37 (s, 3H), 1.38 (s, 9H), 1.23 (t, 3H).
LC/MS (Methode 17, ESIneg): $R_t$ = 2.16 min, m/z = 601.16 [M-H]$^-$.

**Beispiel 428A**

*tert.*-Butyl-2-(3-ethoxyprop-2-enoyl)-2-{[1-(2-methoxyethyl)-5-methyl-2,4-dioxo-3-(2,2,2-trifluorethyl)-1,2,3,4-tetrahydrothieno[2,3-d]pyrimidin-6-yl]methyl}hydrazincarboxylat

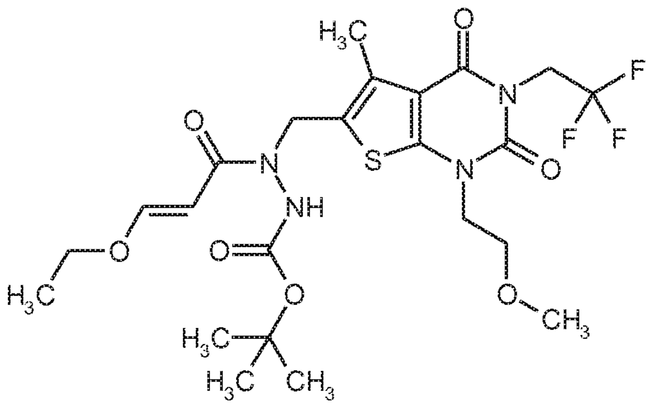

Analog zu dem unter Bsp. 348A beschriebenen Verfahren wurden aus 396 mg (0.849 mmol) der Verbindung aus Bsp. 422A und 161 mg (1.02 mmol, 85% Gehalt) 3-Ethoxyacryloylchlorid 377 mg (78% d. Th.) der Titelverbindung erhalten.
$^1$H-NMR (400 MHz, DMSO-$d_6$, $\delta$/ppm): 9.49 (breit, 1H), 7.50 (d, 1H), 5.57 (d, 1H), 5.02-4.80 (m, 1H), 4.71 (q, 3H), 4.60-4.38 (m, 1H), 4.06 (t, 2H), 3.98-3.85 (m, 2H), 3.64 (t, 2H), 3.23 (s, 3H), 2.36 (s, 3H), 1.38 (s, 9H), 1.23 (t, 3H).
LC/MS (Methode 17, ESIneg): $R_t$ = 2.01 min, m/z = 563.18 [M-H]$^-$.

**Beispiel 429A**

*tert.*-Butyl-2-(3-ethoxyprop-2-enoyl)-2-{[5-methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl}hydrazincarboxylat (*Racemat*)

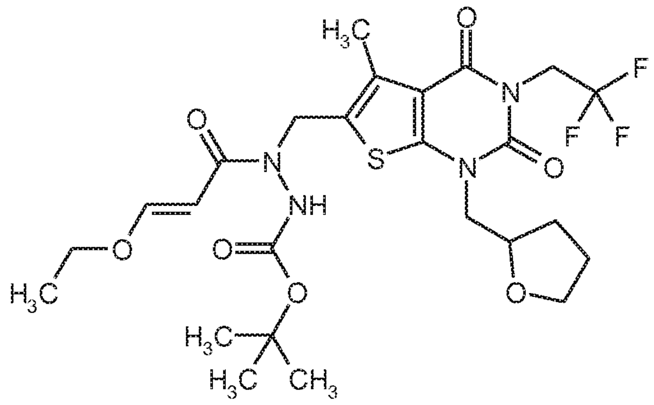

Analog zu dem unter Bsp. 348A beschriebenen Verfahren wurden aus 600 mg (1.22 mmol) der Verbindung aus Bsp. 423A und 231 mg (1.46 mmol, 85% Gehalt) 3-Ethoxyacryloylchlorid 700 mg (97% d. Th.) der Titelverbindung erhalten.

$^1$H-NMR (400 MHz, DMSO-$d_6$, δ/ppm): 9.49 (breit, 1H), 7.50 (d, 1H), 5.57 (d, 1H), 5.07-4.79 (m, 1H), 4.72 (q, 2H), 4.62-4.33 (m, 1H), 4.27-4.20 (m, 1H), 4.10-3.85 (m, 3H), 3.83-3.68 (m, 2H), 3.66-3.53 (m, 1H), 2.36 (s, 3H), 2.04-1.74 (m, 3H), 1.73-1.60 (m, 1H), 1.38 (s, 9H), 1.23 (t, 3H).

LC/MS (Methode 17, ESIneg): $R_t$ = 2.10 min, m/z = 589.19 $[M-H]^-$.

## Beispiel 430A

*tert*.-Butyl-2-(3-ethoxyprop-2-enoyl)-2-{[3-(2-methoxyethyl)-5-methyl-2,4-dioxo-1-(3,3,3-trifluorpropyl)-1,2,3,4-tetrahydrothieno[2,3-d]pyrimidin-6-yl]methyl}hydrazincarboxylat

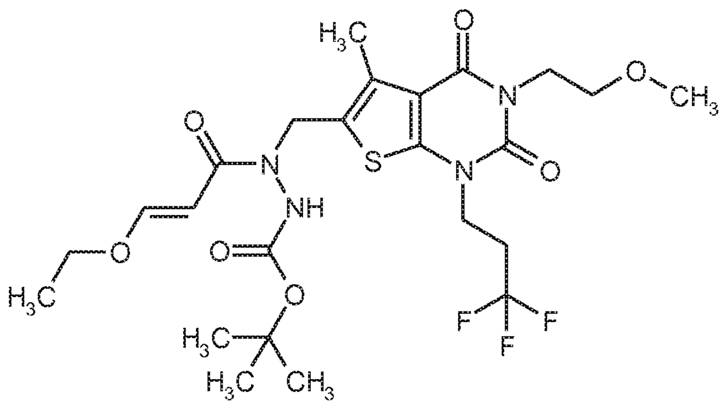

Analog zu dem unter Bsp. 348A beschriebenen Verfahren wurden aus 300 mg (0.624 mmol) der Verbindung aus Bsp. 424A und 119 mg (0.749 mmol, 85% Gehalt) 3-Ethoxyacryloylchlorid 277 mg (76% d. Th.) der Titelverbindung erhalten.

$^1$H-NMR (400 MHz, DMSO-$d_6$, δ/ppm): 9.49 (breit, 1H), 7.50 (d, 1H), 5.57 (d, 1H), 4.94 (breit, 1H), 4.48 (breit, 1H), 4.12 (t, 2H), 4.06 (t, 2H), 3.93 (breit, 2H), 3.49 (t, 2H), 3.23 (s, 3H), 2.87-2.64 (m, 2H), 2.37 (s, 3H), 1.39 (s, 9H), 1.23 (t, 3H).

LC/MS (Methode 17, ESIneg): $R_t$ = 1.99 min, m/z = 577.19 $[M-H]^-$.

## Beispiel 431A

*tert*.-Butyl-2-{[1,3-bis(2-methoxyethyl)-5-methyl-2,4-dioxo-1,2,3,4-tetrahydrothieno[2,3-d]pyrimidin-6-yl]methyl}-2-(3-ethoxyprop-2-enoyl)hydrazincarboxylat

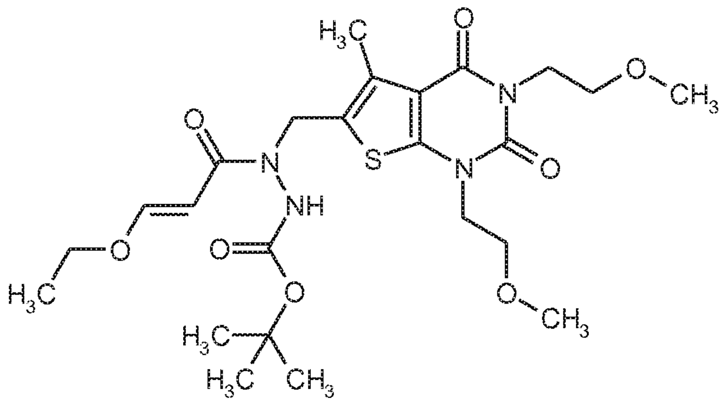

Analog zu dem unter Bsp. 348A beschriebenen Verfahren wurden aus 300 mg (0.678 mmol) der Verbindung aus Bsp. 425A und 129 mg (0.813 mmol, 85% Gehalt) 3-Ethoxyacryloylchlorid 292 mg (79% d. Th.) der Titelverbindung erhalten.
$^1$H-NMR (400 MHz, DMSO-$d_6$, δ/ppm): 9.49 (breit, 1H), 7.50 (d, 1H), 5.57 (d, 1H), 4.93 (breit, 1H), 4.45 (breit, 1H), 4.06 (t, 2H), 4.02 (t, 2H), 3.92 (m, 2H), 3.63 (t, 2H), 3.49 (t, 2H), 3.23 (s, 6H), 2.35 (s, 3H), 1.39 (s, 9H), 1.23 (t, 3H).
LC/MS (Methode 17, ESIpos): $R_t$ = 1.78 min, m/z = 541.23 $[M+H]^+$.

**Beispiel 432A**

*tert.*-Butyl-2-(3-ethoxyprop-2-enoyl)-2-{[3-(2-methoxyethyl)-5-methyl-2,4-dioxo-1-(tetrahydrofuran-2-ylmethyl)-1,2,3,4-tetrahydrothieno[2,3-d]pyrimidin-6-yl]methyl}hydrazincarboxylat (*Racemat*)

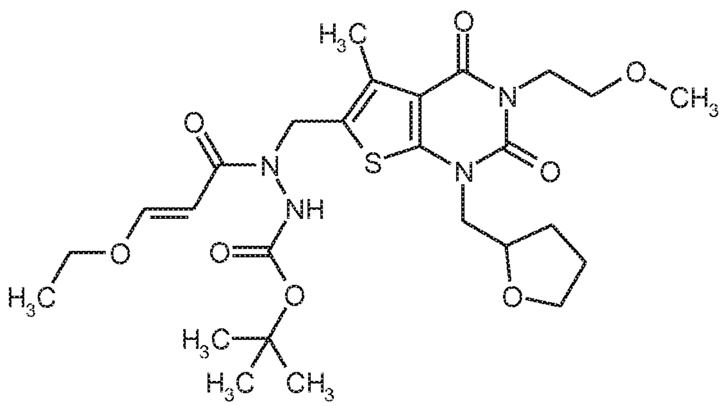

Analog zu dem unter Bsp. 348A beschriebenen Verfahren wurden aus 600 mg (1.28 mmol) der Verbindung aus Bsp. 426A und 243 mg (1.54 mmol, 85% Gehalt) 3-Ethoxyacryloylchlorid 450 mg (62% d. Th.) der Titelverbindung erhalten.
$^1$H-NMR (500 MHz, DMSO-$d_6$, δ/ppm): 9.49 (breit, 1H), 7.49 (d, 1H), 5.57 (d, 1H), 5.08-4.84 (m, 1H), 4.54-4.31 (m, 1H), 4.28-4.17 (m, 1H), 4.07 (t, 2H), 4.04-3.97 (m, 1H), 3.93 (d, 2H), 3.82-3.69 (m, 2H), 3.61 (q, 1H), 3.49 (t, 2H), 3.23 (s, 3H), 2.35 (s, 3H), 2.04-1.75 (m, 3H), 1.72-1.60 (m, 1H), 1.39 (s, 9H), 1.23 (t, 3H).
LC/MS (Methode 1, ESIpos): $R_t$ = 0.99 min, m/z = 567 $[M+H]^+$.

**Beispiel 433A**

*tert.*-Butyl-3-(2,4-dimethoxybenzyl)-5-methyl-2,4-dioxo-1-(3,3,3-trifluorpropyl)-1,2,3,4-tetrahydrothieno[2,3-d]pyrimidin-6-carboxylat

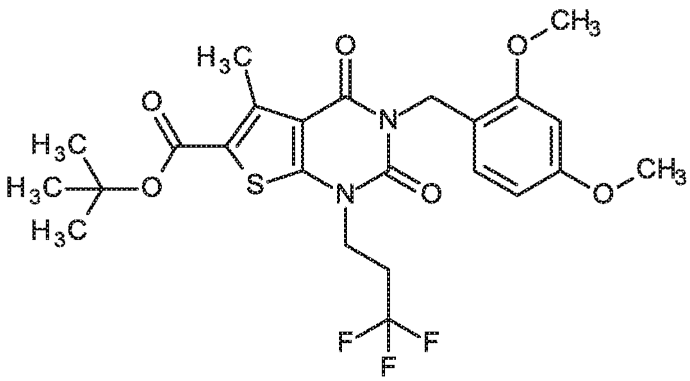

Eine Lösung von 3.18 g (7.35 mmol) der Verbindung aus Bsp. 8A in 70 ml DMF wurde mit 1.71 g (5.25 mmol) Cäsiumcarbonat versetzt und 15 min bei RT gerührt. Dann wurden 1.65 g (7.35 mmol) 1,1,1-Trifluor-3-iodpropan hinzugefügt, und das Gemisch wurde insgesamt 11 h bei 70°C gerührt, wobei nach 6 h Reaktionszeit noch einmal 1.65 g (7.35 mmol) 1,1,1-Trifluor-3-iodpropan hinzugefügt wurden. Nach Abkühlen auf RT wurde mit Wasser versetzt, worauf das Produkt ausfiel. Es wurde nach kurzem Verrühren abgesaugt, mit Wasser gewaschen und im Hochvakuum getrocknet. Das so erhaltene Produkt wurde durch Verrühren mit Pentan/Dichlormethan bei RT gereinigt. Es wurden 3.56 g (91% d. Th.) der Titelverbindung erhalten.
$^1$H-NMR (400 MHz, DMSO-$d_6$, $\delta$/ppm): 6.75 (d, 1H), 6.57 (d, 1H), 6.39 (dd, 1H), 4.95 (s, 2H), 4.17 (t, 2H), 3.81 (s, 3H), 3.72 (s, 3H), 2.95-2.62 (m, 2H), 2.74 (s, 3H), 1.54 (s, 9H).
LC/MS (Methode 1, ESIpos): $R_t$ = 1.40 min, m/z = 529 $[M+H]^+$.

**Beispiel 434A**

Ethyl-3-ethyl-1-(2-methoxyethyl)-5-methyl-2,4-dioxo-1,2,3,4-tetrahydrothieno[2,3-d]pyrimidin-6-carboxylat

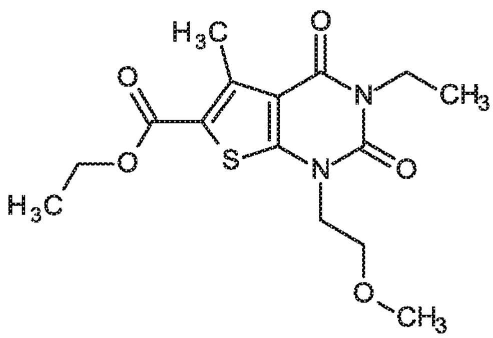

Eine Lösung von 1.0 g (3.54 mmol) der Verbindung aus Bsp. 9A in 10 ml DMF wurde mit 1.73 g (5.31 mmol) Cäsiumcarbonat versetzt und 10 min bei RT gerührt. Dann wurden 739 mg (5.31 mmol) 2-Bromethyl-methylether hinzugefügt, und das Gemisch wurde in einem Mikrowellenofen (Biotage Initiator mit dynamischer Steuerung der Einstrahlleistung) 2 h lang bei 100°C gerührt. Nach Abkühlen auf RT wurde mit Ethylacetat verdünnt und mit Wasser versetzt. Das ausgefallene Produkt wurde abgesaugt und getrocknet. Die organische Phase des Filtrats wurde abgetrennt und die wässrige noch dreimal mit Ethylacetat extrahiert. Die vereinigten organischen Phasen wurden mit gesättigter wässriger Natriumchlorid-Lösung gewaschen, über wasserfreiem Magnesiumsulfat getrocknet, filtriert und eingeengt. Der Rückstand wurde mit dem anfangs abgesaugten Produkt vereinigt und dann mittels MPLC gereinigt (Isolera, Biotage-Kartusche SNAP-KP-Sil mit 100 g Kieselgel, Cyclohexan/Ethylacetat 92:8 $\rightarrow$ 33:66). Die Produktfraktionen wurden vereinigt, eingeengt und im Hochvakuum getrocknet. Es wurden 953 mg (78% d. Th.) der Titelverbindung erhalten.
$^1$H-NMR (400 MHz, DMSO-$d_6$, $\delta$/ppm): 4.28 (q, 2H), 4.09 (t, 2H), 3.91 (q, 2H), 3.65 (t, 2H), 3.24 (s, 3H), 2.77 (s, 3H), 1.30 (t, 3H), 1.13 (t, 3H).
LC/MS (Methode 17, ESIpos): $R_t$ = 1.99 min, m/z = 341.12 $[M+H]^+$.

**Beispiel 435A**

5-Methyl-2,4-dioxo-1-(3,3,3-trifluorpropyl)-1,2,3,4-tetrahydrothieno[2,3-d]pyrimidin-6-carbonsäure

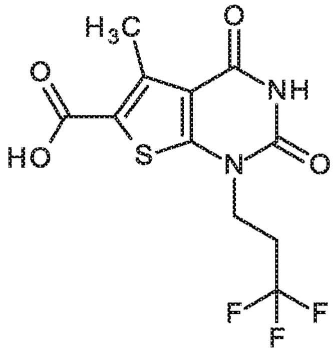

5.46 g (10.3 mmol) der Verbindung aus Bsp. 433A wurden in 200 ml Toluol gelöst und mit 8.27 g (62.0 mmol) festem Aluminiumtrichlorid versetzt. Das Reaktionsgemisch wurde dann 90 min bei 50°C gerührt. Nach dem Abkühlen auf RT wurden nacheinander 120 ml Wasser und ca. 180 ml Ethylacetat zugesetzt. Nach Phasentrennung wurde die organische Phase nacheinander mit Wasser und gesättigter wässriger Natriumchlorid-Lösung gewaschen. Es wurde über wasserfreiem Magnesiumsulfat getrocknet. Nach Filtration wurde zur Trockene eingeengt. Der verbliebene Rückstand wurde bei RT mit 100 ml Pentan/Dichlormethan (20:1) verrührt. Nach Filtration und Trocknen des Feststoffs im Hochvakuum wurden 2.98 g (83% d. Th., 93% Reinheit) der Titelverbindung erhalten.
$^1$H-NMR (400 MHz, DMSO-$d_6$, $\delta$/ppm): 13.41 (br. s, 1H), 11.63 (s, 1H), 4.09 (t, 2H), 2.91-2.61 (m, 2H), 2.72 (s, 3H).
LC/MS (Methode 1, ESIpos): $R_t$ = 0.65 min, m/z = 323 $[M+H]^+$.

**Beispiel 436A**

2-Methoxypropyl-3-(2-methoxypropyl)-5-methyl-2,4-dioxo-1-(3,3,3-trifluorpropyl)-1,2,3,4-tetrahydrothieno[2,3-d]pyrimidin-6-carboxylat (*Stereoisomerengemisch*)

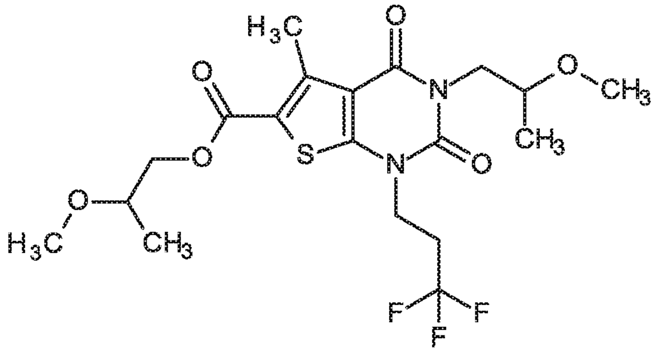

Eine Lösung von 510 mg (1.38 mmol) der Verbindung aus Bsp. 435A in 15 ml DMF wurde mit 1.35 g (4.13 mmol) Cäsiumcarbonat versetzt und 10 min bei RT gerührt. Dann wurden 665 mg (4.13 mmol) racemisches 1-Brom-2-methoxypropan zugesetzt und das Gemisch 66 h bei 80°C gerührt. Das DMF wurde danach weitestgehend aus dem Reaktionsgemisch abdestilliert. Der verbliebene Rückstand wurde zwischen Wasser (75 ml) und Dichlormethan (75 ml) verteilt. Die wässrige Phase wurde mit Dichlormethan extrahiert. Die vereinigten organischen Phasen wurden über Natriumsulfat getrocknet, filtriert und eingeengt. Der erhaltene Rückstand wurde an Kieselgel chromatographiert (Laufmittel Hexan/Ethylacetat). Es wurden 483 mg (71% d. Th.) der Titelverbindung erhalten.
$^1$H-NMR (400 MHz, DMSO-$d_6$, $\delta$/ppm): 4.31 (dd, 1H), 4.24-4.11 (m, 3H), 4.09-4.01 (m, 1H), 3.76 (dd, 1H), 3.68-3.58 (m, 2H), 3.31-3.27 (m, 3H), 3.21 (s, 3H), 2.87-2.72 (m, 5H), 1.15 (d, 3H), 1.06 (d, 3H).

LC/MS (Methode 3): $R_t$ = 1.34 min, m/z = 467 $[M+H]^+$.

**Beispiel 437A**

Ethyl-4-methyl-2-{[(1,1,1-trifluorpropan-2-yl)carbamoyl]amino}thiophen-3-carboxylat (*Racemat*)

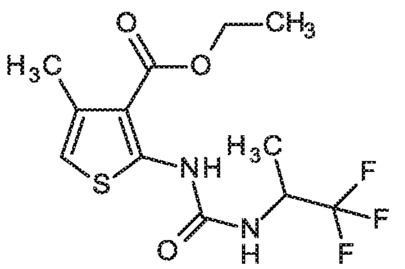

Eine Lösung von 7.0 g (37.8 mmol) Ethyl-2-amino-4-methylthiophen-3-carboxylat in 200 ml Dichlormethan wurde mit 12.3 g (75.6 mmol) 1,1'-Carbonyldiimidazol (CDI) und 16 ml (113 mmol) Triethylamin versetzt und 2 Tage bei RT gerührt. Dann wurden zu dem Gemisch 8.55 g (75.6 mmol) racemisches 2-Amino-1,1,1-trifluorpropan gegeben und das Gemisch weitere 2 Tage bei RT gerührt. Anschließend wurde nacheinander mit je ca. 300 ml Wasser und gesättigter wässriger Natriumchlorid-Lösung ausgeschüttelt. Nach Trocknen über wasserfreiem Magnesiumsulfat und Filtration wurde zur Trockene eingeengt und der verbliebene Rückstand mittels MPLC gereinigt (Isolera, Biotage-Kartusche mit 340 g Kieselgel, Cyclohexan/Ethylacetat 5:1). Nach Eindampfen der Produktfraktionen und Trocknen im Hochvakuum wurden 9.21 g (75% d. Th.) der Titelverbindung erhalten.
$^1$H-NMR (500 MHz, DMSO-$d_6$, δ/ppm): 10.46 (s, 1H), 8.50 (d, 1H), 6.50 (s, 1H), 4.49 (dq, 1H), 4.30 (q, 2H), 2.28 (d, 3H), 1.32 (t, 3H), 1.28 (d, 3H).
LC/MS (Methode 1, ESIpos): $R_t$ = 1.13 min, m/z = 325 $[M+H]^+$.

**Beispiel 438A**

Ethyl-2-{[(2-methoxypnopyl)carbamoyl]amino}-4-methylthiophen-3-carboxylat (*Racemat*)

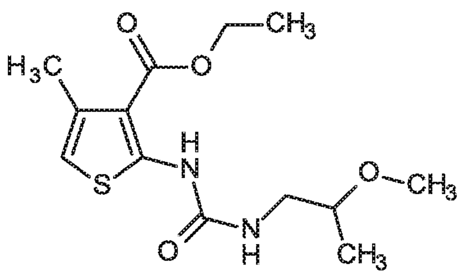

Eine Lösung von 7.6 g (39.8 mmol, Reinheit 97%) Ethyl-2-amino-4-methylthiophen-3-carboxylat in 150 ml Dichlormethan wurde mit 12.9 g (79.6 mmol) 1,1'-Carbonyldiimidazol (CDI) und 22 ml (159 mmol) Triethylamin versetzt und 2 Tage bei RT gerührt. Dann wurden zu dem Gemisch 10 g (79.6 mmol) racemisches l-Amino-2-methoxypropan-Hydrochlorid gegeben und das Gemisch 1 h bei RT gerührt. Anschließend wurde nacheinander mit je ca. 80 ml Wasser (zweimal) und gesättigter wässriger Natriumchlorid-Lösung ausgeschüttelt. Nach Trocknen über wasserfreiem Magnesiumsulfat und Filtration wurde zur Trockene eingeengt und der verbliebene Rückstand mittels MPLC gereinigt (Isolera, Biotage-Kartusche mit 340 g Kieselgel, Cyclohexan/Ethylacetat 90:10 → 20:80). Nach Einengen der Produktfraktionen und Trocknen im Hochvakuum wurden 11.3 g (94% d. Th.) der Titelverbindung erhalten.
$^1$H-NMR (400 MHz, DMSO-$d_6$, δ/ppm): 10.32 (s, 1H), 7.95 (br. s, 1H), 6.42 (s, 1H), 4.28 (q, 2H), 3.41-3.33 (m, 1H), 3.31 (s, 3H), 3.22-3.05 (m, 2H), 2.26 (s, 3H), 1.31 (t, 3H), 1.06 (d, 3H).
LC/MS (Methode 17, ESIpos): $R_t$ = 1.80 min, m/z = 301.12 $[M+H]^+$.

**Beispiel 439A**

5-Methyl-3-(1,1,1-trifluorpropan-2-yl)thieno[2,3-d]pyrimidin-2,4(1*H*,3*H*)-dion (*Racemat*)

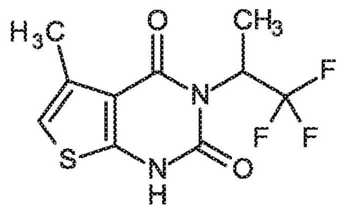

Analog zu dem unter Bsp. 44A beschriebenen Verfahren wurden aus 9.15 g (28.2 mmol) der Verbindung aus Bsp. 437A 7.39 g (94% d. Th.) der Titelverbindung hergestellt. Die Umsetzung erfolgte hier bei 50°C, und die Reaktionszeit betrug 5 h.

$^1$H-NMR (400 MHz, DMSO-$d_6$, $\delta$/ppm): 12.67-12.01 (m, 1H), 6.74 (d, 1H), 5.81-5.42 (m, 1H), 2.34 (d, 3H), 1.73-1.57 (m, 3H).

LC/MS (Methode 17, ESIpos): $R_t$ = 1.61 min, m/z = 279.04 $[M+H]^+$.

**Beispiel 440A**

3-(2-Methoxypropyl)-5-methylthieno[2,3-d]pyrimidin-2,4(1*H*,3*H*)-dion (*Racemat*)

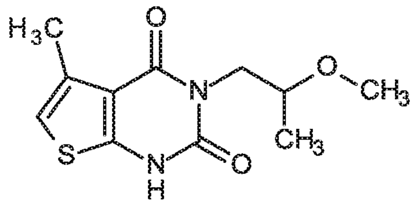

11.2 g (37.3 mmol) der Verbindung aus Bsp. 438A wurden in 350 ml Ethanol gelöst und mit 20.9 ml (55.9 mmol) einer 21%-igen Lösung von Natriumethylat in Ethanol versetzt. Nachdem das Gemisch 4 h bei RT gerührt worden war, wurde es mit 74.6 ml (74.6 mmol) 1 M Salzsäure versetzt. Der dabei ausgefallene Niederschlag wurde abgesaugt, mit Wasser neutral gewaschen und im Hochvakuum getrocknet. Dies ergab eine erste Fraktion der Titelverbindung (6.1 g). Aus der Mutterlauge fiel über Nacht noch weiteres Produkt aus, das ebenfalls abgesaugt, gewaschen und getrocknet wurde (Fraktion 2, 1.7 g). Beide Fraktionen wurden vereinigt. Es wurden somit insgesamt 7.8 g (82% d. Th.) der Titelverbindung erhalten.

$^1$H-NMR (400 MHz, DMSO-$d_6$, $\delta$/ppm): 12.11 (s, 1H), 6.69 (d, 1H), 4.01 (dd, 1H), 3.77-3.59 (m, 2H), 3.22 (s, 3H), 2.35 (d, 3H), 1.05 (d, 3H).

LC/MS (Methode 17, ESIpos): $R_t$ = 1.18 min, m/z = 255.08 $[M+H]^+$.

**Beispiel 441A**

5-Methyl-2,4-dioxo-3-(1,1,1-trifluorpropan-2-yl)-1,2,3,4-tetrahydrothieno[2,3-d]pyrimidin-6-carbaldehyd (*Racemat*)

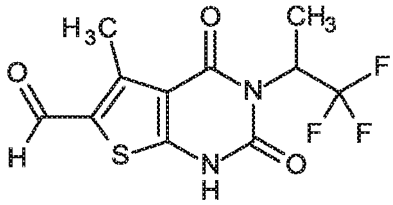

Analog zu dem unter Bsp. 50A beschriebenen Verfahren wurden aus 7.38 g (26.5 mmol) der Verbindung aus Bsp. 439A, 20 ml (265 mmol) DMF und 30 ml (318 mmol) Phosphoroxychlorid 7.60 g (93% d. Th.) der Titelverbindung hergestellt.
$^1$H-NMR (400 MHz, DMSO-$d_6$, δ/ppm): 12.79 (br. s, 1H), 10.08 (s, 1H), 5.80-5.37 (m, 1H), 2.82-2.67 (m, 3H), 1.76-1.55 (m, 3H).
LC/MS (Methode 17, ESIpos): $R_t$ = 1.55 min, m/z = 307.04 $[M+H]^+$.

**Beispiel 442A**

3-(2-Methoxypropyl)-5-methyl-2,4-dioxo-1,2,3,4-tetrahydrothieno[2,3-d]pyrimidin-6-carbaldehyd (*Racemat*)

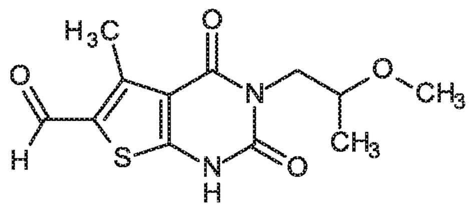

7.82 g (30.7 mmol) der Verbindung aus Bsp. 440A wurden in 236 ml (3075 mmol) DMF gelöst und bei 0°C langsam mit 28.7 ml (307 mmol) Phosphoroxychlorid versetzt. Anschließend wurde das Reaktionsgemisch 1 h bei 70°C gerührt. Nach dem Abkühlen auf RT wurden 1500 ml Wasser hinzugefügt. Das Produkt fiel aus, und das heterogene Gemisch wurde über Nacht bei RT gerührt. Dann wurde das Produkt abgesaugt, mit Wasser gewaschen und im Hochvakuum getrocknet. Es wurden 7.75 g (89% d. Th.) der Titelverbindung erhalten.
$^1$H-NMR (400 MHz, DMSO-$d_6$, δ/ppm): 12.60 (br. s, 1H), 10.07 (s, 1H), 4.00 (dd, 1H), 3.75-3.68 (m, 1H), 3.63 (sext, 1H), 3.22 (s, 3H), 2.76 (s, 3H), 1.07 (d, 3H).
LC/MS (Methode 1, ESIpos): $R_t$ = 0.65 min, m/z = 283 $[M+H]^+$.

**Beispiel 443A**

3-(2,2-Dimethylpropyl)-5-methyl-2,4-ioxo-1-(tetrahydrofuran-2-ylmethyl)-1,2,3,4-tetrahydrothieno[2,3-d]pyrimidin-6-carbaldehyd (*Racemat*)

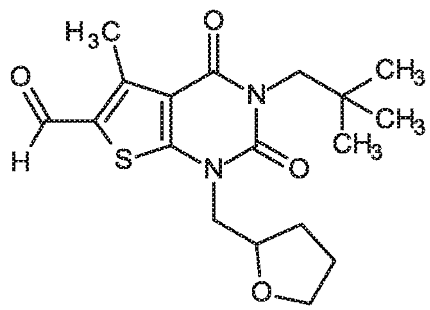

Eine Lösung von 2.5 g (8.92 mmol) der Verbindung aus Bsp. 286A in 80 ml DMF wurde mit 3.08 g (22.3 mmol) Kaliumcarbonat versetzt und 15 min bei RT gerührt. Dann wurden 2 ml (17.8 mmol) 2-(Brommethyl)-tetrahydrofuran

hinzugefügt, und das Gemisch wurde zunächst 18 h bei 50°C gerührt. Nach dieser Zeit wurde nochmals 1 ml (8.92 mmol) 2-(Brommethyl)-tetrahydrofuran zugesetzt und das Rühren bei 50°C zwei Tage lang fortgesetzt. Danach wurde das Reaktionsgemisch bei RT mit Wasser versetzt, woraufhin das Produkt ausfiel. Es wurde abgesaugt, mit Wasser gewaschen und im Hochvakuum getrocknet. Es wurden 2.96 g (91% d. Th.) der Titelverbindung erhalten.
$^1$H-NMR (400 MHz, DMSO-$d_6$, δ/ppm): 10.09 (s, 1H), 4.26-4.20 (m, 1H), 4.11 (dd, 1H), 3.88-3.69 (m, 2H), 3.82 (s, 2H), 3.67-3.57 (m, 1H), 2.77 (s, 3H), 2.07-1.75 (m, 3H), 1.74-1.61 (m, 1H), 0.90 (s,9H).
LC/MS (Methode 17, ESIpos): $R_t$ = 2.16 min, m/z = 365.15 [M+H]$^+$.

**Beispiel 444A**

5-Methyl-2,4-dioxo-3-(1,1,1-trifluorpropan-2-yl)-1-(3,3,3-trifluorpropyl)-1,2,3,4-tetrahydrothieno-[2,3-d]pyrimidin-6-carbaldehyd (*Racemat*)

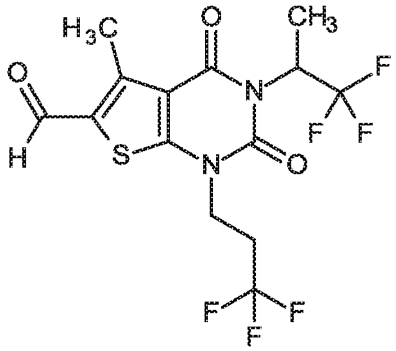

Eine Lösung von 2.0 g (6.53 mmol) der Verbindung aus Bsp. 441A in 40 ml DMF wurde mit 2.26 g (16.3 mmol) Kaliumcarbonat versetzt und 15 min bei RT gerührt. Dann wurden 4.39 g (19.6 mmol) 1,1,1-Trifluor-3-iodpropan zugesetzt und das Gemisch ca. 18 h bei 50°C gerührt. Nach dem Abkühlen auf RT wurden ca. 200 ml Wasser hinzugefügt und das Produkt mit Ethylacetat extrahiert. Der organische Extrakt wurde mit gesättigter wässriger Natriumchlorid-Lösung gewaschen, über wasserfreiem Magnesiumsulfat getrocknet, filtriert und eingeengt. Der verbliebene Rückstand wurde mittels MPLC gereinigt (Isolera, Biotage-Kartusche mit 340 g Kieselgel, Cyclohexan/Ethylacetat 5:1). Die Produktfraktionen wurden vereinigt, eingedampft und im Hochvakuum getrocknet. Es wurden so 2.25 g (85% d. Th.) der Titelverbindung erhalten.
$^1$H-NMR (400 MHz, DMSO-$d_6$, δ/ppm): 10.12 (s, 1H), 5.87-5.40 (m, 1H), 4.29-4.07 (m, 2H), 2.91-2.72 (m, 2H), 2.79 (s, 3H), 1.78-1.55 (m, 3H).
LC/MS (Methode 1, ESIpos): $R_t$ = 1.10 min, m/z = 403 [M+H]$^+$.

**Beispiel 445A**

1-(2-Methoxyethyl)-5-methyl-2,4-dioxo-3-(1,1,1-trifluorpropan-2-yl)-1,2,3,4-tetrahydrothieno-[2,3-d]pyrimidin-6-carbaldehyd (*Racemat*)

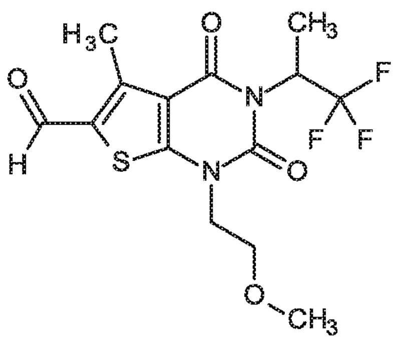

Eine Lösung von 2.5 g (8.16 mmol) der Verbindung aus Bsp. 441A in 50 ml DMF wurde mit 2.82 g (20.4 mmol) Kaliumcarbonat versetzt und 15 min bei RT gerührt. Dann wurden 2.27 g (16.3 mmol) 2-Bromethyl-methylether zugesetzt und das Gemisch bei 50°C gerührt. Nach ca. 18 h wurden weitere 1.13 g (8.16 mmol) 2-Bromethyl-inethylether hinzugefügt und das Rühren bei 50°C 2 Tage lang fortgesetzt. Nach dem Abkühlen auf RT wurden ca. 250 ml Wasser hinzugefügt und das Produkt mit Ethylacetat extrahiert. Der organische Extrakt wurde mit gesättigter wässriger Natriumchlorid-Lösung gewaschen, über wasserfreiem Magnesiumsulfat getrocknet, filtriert und eingeengt. Der verbliebene Rückstand wurde mittels MPLC gereinigt (Isolera, Biotage-Kartusche mit 100 g Kieselgel, Cyclohexan/Ethylacetat 5:1). Die Produktfraktionen wurden vereinigt, eingedampft und im Hochvakuum getrocknet. Es wurden 1.46 g (48% d. Th.) der Titelverbindung erhalten.
$^1$H-NMR (400 MHz, DMSO-$d_6$, $\delta$/ppm): 10.10 (s, 1H), 5.84-5.42 (m, 1H), 4.14-4.08 (m, 2H), 3.69-3.62 (m, 2H), 3.25 (s, 3H), 2.78 (s, 3H), 1.77-1.58 (m, 3H).
LC/MS (Methode 17, ESIpos): $R_t$ = 1.84 min, m/z = 365.08 $[M+H]^+$.

## Beispiel 446A

3-(2-Ethoxyethyl)-1-(3-fluorpropyl)-5-methyl-2,4-dioxo-1,2,3,4-tetrahydrothieno[2,3-d]pyrimidin-6-carbaldehyd

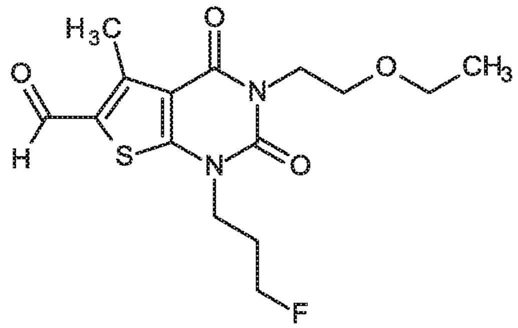

Eine Lösung von 900 mg (3.19 mmol) der Verbindung aus Bsp. 371A in 29 ml DMF wurde mit 1.1 g (7.97 mmol) Kaliumcarbonat versetzt und 15 min bei RT gerührt. Dann wurden 1.79 g (9.56 mmol) l-Fluor-3-iodpropan zugesetzt und das Gemisch 18 h bei 50°C gerührt. Das DMF wurde danach weitestgehend abdestilliert und der verbliebene Rückstand zwischen halbgesättigter Natriumchlorid-Lösung (100 ml) und Ethylacetat (50 ml) verteilt. Die wässrige Phase wurde mit Ethylacetat extrahiert. Die vereinigten organischen Phasen wurden über Natriumsulfat getrocknet, filtriert und eingeengt. Es wurden 977 mg (87% d. Th.) der Titelverbindung erhalten.
$^1$H-NMR (400 MHz, DMSO-$d_6$, $\delta$/ppm): 10.10 (s, 1H), 4.60 (t, 1H), 4.48 (t, 1H), 4.04 (td, 4H), 3.52 (t, 2H), 3.45 (q, 2H), 2.78 (s, 3H), 2.15-2.00 (m, 2H), 1.07 (t, 3H).
LC/MS (Methode 3, ESIpos): $R_t$ = 1.06 min, m/z = 343 $[M+H]^+$.

## Beispiel 447A

3-(2-Methoxypropyl)-5-methyl-2,4-dioxo-1-(3,3,3-trifluorpropyl)-1,2,3,4-tetrahydrothieno[2,3-d]-pyrimidin-6-carbaldehyd (*Racemat*)

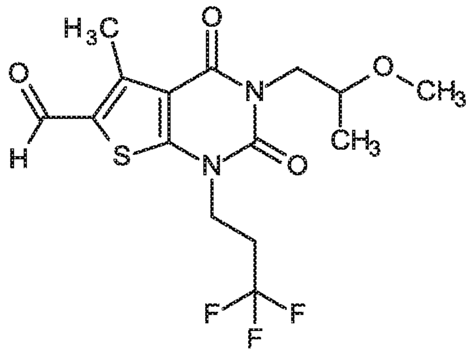

Eine Lösung von 2.5 g (8.86 mmol) der Verbindung aus Bsp. 442A in 18 ml DMF wurde mit 4.33 g (13.3 mmol) Cäsiumcarbonat versetzt und 10 min bei RT gerührt. Dann wurden 2.98 g (13.3 mmol) 1,1,1-Trifluor-3-iodpropan zugesetzt, und das Gemisch wurde zunächst 3 h bei 100°C, dann 3 h bei 80°C in einem Mikrowellenofen gerührt (Biotage Initiator mit dynamischer Steuerung der Einstrahlleistung). Nach dem Abkühlen auf RT wurden ca. 90 ml Wasser hinzugefügt und das Produkt mit Ethylacetat extrahiert. Der organische Extrakt wurde mit gesättigter wässriger Natriumchlorid-Lösung gewaschen, über wasserfreiem Magnesiumsulfat getrocknet, filtriert und eingeengt. Der verbliebene Rückstand wurde mittels MPLC gereinigt (Isolera, Biotage-Kartusche mit 340 g Kieselgel, Cyclohexan/Ethylacetat 90:10 → 10:90). Die Produktfraktionen wurden vereinigt, eingedampft und im Hochvakuum getrocknet. Es wurden 2.8 g (83% d. Th.) der Titelverbindung erhalten.

$^1$H-NMR (400 MHz, DMSO-$d_6$, δ/ppm): 10.11 (s, 1H), 4.30-4.11 (m, 2H), 4.05 (dd, 1H), 3.77 (dd, 1H), 3.63 (sext, 1H), 3.22 (s, 3H), 2.91-2.72 (m, 2H), 2.79 (s, 3H), 1.07 (d, 3H).

LC/MS (Methode 1, ESIpos): $R_t$ = 0.94 min, m/z = 379 [M+H]$^+$.

**Beispiel 448A**

3-Ethyl-*N*-melhoxy-*N*,5-dimethyl-2,4-dioxo-1-(3,3,3-trifluorpropyl)-1,2,3,4-tetrahydrothieno-[2,3-d]pyrimidin-6-carboxamid

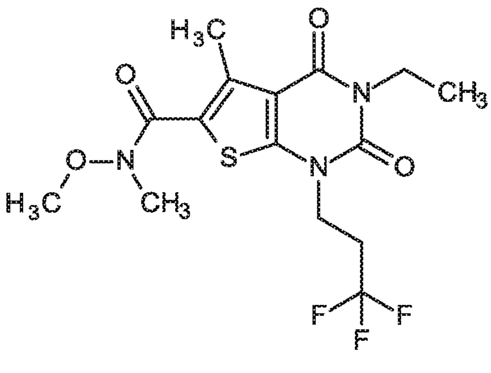

*Darstellung des Säurechlorids:* Eine Lösung von 1.0 g (2.86 mmol) der Verbindung aus Bsp. 16A in 30 ml Dichlormethan wurde bei RT zunächst mit 1.2 ml (14.3 mmol) Oxalylchlorid und dann mit einem Tropfen DMF versetzt. Nachdem das Reaktionsgemisch 2 h bei RT gerührt worden war, wurde es am Rotationsverdampfer bis zur Trockene eingeengt. Der verbliebene Rückstand des Säurechlorids wurde im Hochvakuum getrocknet und anschließend im nächsten Teilschritt weiter umgesetzt.

*Darstellung des Amids:* Das zuvor erhaltene Säurechlorid wurde in 30 ml wasserfreiem THF gelöst und mit 334 mg (3.43 mmol) *N,O*-Dimethylhydroxylamin-Hydrochlorid und 1.2 ml (7.14 mmol) *N,N*-Diisopropylethylamin versetzt. Anschließend wurde das Reaktionsgemisch bei RT gerührt. Nach ca. 18 h wurde mit ca. 300 ml Ethylacetat verdünnt und nacheinander mit Wasser (zweimal) und gesättigter wässriger Natriumchlorid-Lösung gewaschen. Nach Trocknen über wasserfreiem Magnesiumsulfat wurde filtriert und eingeengt. Nach Trocknen im Hochvakuum wurden 1.11 g (99% d. Th.) der Titelverbindung erhalten.

$^1$H-NMR (400 MHz, DMSO-$d_6$, δ/ppm): 4.16 (t, 2H), 3.92 (q, 2H), 3.70 (s, 3H), 3.26 (s, 3H), 2.89-2.69 (m, 2H), 2.74 (s,

3H), 1.13 (t, 3H).
LC/MS (Methode 1, ESIpos): $R_t$ = 0.99 min, m/z = 394 $[M+H]^+$.

**Beispiel 449A**

6-Acetyl-3-ethyl-5-methyl-1-(3,3,3-trifluorpropyl)thieno[2,3-d]pyrimidin-2,4(1*H*,3*H*)-dion

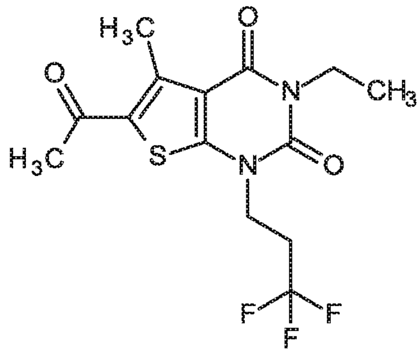

1.0 g (2.54 mmol) der Verbindung aus Bsp. 448A wurden in 25 ml wasserfreiem THF gelöst und bei -78°C tropfenweise mit 1.7 ml (5.08 mmol) einer 3 M Lösung von Methylmagnesiumchlorid in THF versetzt. Nach beendetem Zutropfen wurde das Reaktionsgemisch 1 h bei 0°C nachgerührt. Dann wurde ein kleines Volumen gesättigte wässrige Ammoniumchlorid-Lösung zugesetzt. Es wurde mit Ethylacetat verdünnt und mit soviel wasserfreiem festem Magnesiumsulfat versetzt, dass die wässrige Phase komplett aufgenommen wurde. Es wurde dann filtriert und das Filtrat eingeengt. Der verbliebene Rückstand wurde mittels MPLC gereinigt (Isolera, Biotage-Kartusche mit 50 g Kieselgel, Cyclohexan/Ethylacetat 2:1). Die Produktfraktionen wurden vereinigt, eingedampft und im Hochvakuum getrocknet. Es wurden 775 mg (87% d. Th.) der Titelverbindung erhalten.
$^1$H-NMR (400 MHz, DMSO-$d_6$, $\delta$/ppm): 4.17 (t, 2H), 3.91 (q, 2H), 2.88-2.73 (m, 2H), 2.83 (s, 3H), 2.56 (s, 3H), 1.13 (t, 3H).
LC/MS (Methode 1, ESIpos): $R_t$ = 0.99 min, m/z = 349 $[M+H]^+$.

**Beispiel 450A**

1-(Fluormethyl)-6-(hydroxymethyl)-3-isopropyl-5-methylthieno[2,3-d]pyrimidin-2,4(1*H*,3*H*)-dion

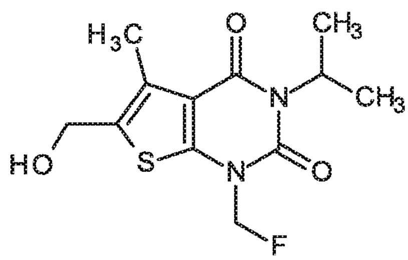

Analog zu dem unter Bsp. 143A (Methode C) beschriebenen Verfahren wurden aus 312 mg (1.03 mmol) der Verbindung aus Bsp. 378A 338 mg der Titelverbindung hergestellt. Die Umsetzung erfolgte hier für 2 h bei -78°C.
$^1$H-NMR (400 MHz, DMSO-$d_6$, $\delta$/ppm): 6.08-5.92 (m, 1H), 5.65 (t, 1H), 5.13 (quin, 1H), 4.59 (d, 2H), 2.32 (s, 3H), 1.41 (d, 6H).
LC/MS (Methode 3): $R_t$ = 0.96 min, m/z = 287 $[M+H]^+$.

**Beispiel 451A**

1-(3-Fluorpropyl)-6-(hydroxymethyl)-3-isopropyl-5-methylthieno[2,3-d]pyrimidin-2,4(1*H*,3*H*)-dion

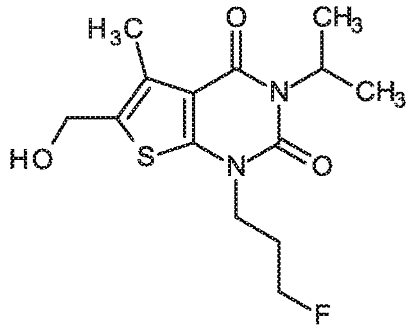

Analog zu dem unter Bsp. 143A (Methode C) beschriebenen Verfahren wurden aus 455 mg (1.44 mmol) der Verbindung aus Bsp. 379A 458 mg (99% d. Th.) der Titelverbindung hergestellt. Die Umsetzung erfolgte hier für 2 h bei -78°C.
$^1$H-NMR (400 MHz, DMSO-$d_6$, δ/ppm): 5.59 (t, 1H), 5.14 (sept, 1H), 4.62-4.55 (m, 3H), 4.48 (t, 1H), 3.97 (t, 2H), 2.32 (s, 3H), 2.14-1.98 (m, 2H), 1.40 (d, 6H).
LC/MS (Methode 3): $R_t$ = 1.01 min, m/z = 315 $[M+H]^+$.

**Beispiel 452A**

6-(Hydroxymethyl)-3-(2-methoxypropyl)-5-methyl-1-(3,3,3-trifluorpropyl)thieno[2,3-d]pyrimidin-2,4(1*H*,3*H*)-dion (*Racemat*)

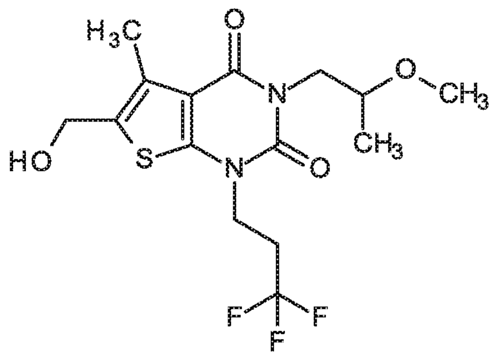

Analog zu dem unter Bsp. 138A (Methode C) beschriebenen Verfahren wurden aus 562 mg (0.735 mmol) der Verbindung aus Bsp. 436A 199 mg (71% d. Th.) der Titelverbindung hergestellt. Die Umsetzung erfolgte hier für 30 min bei -78°C.
$^1$H-NMR (400 MHz, DMSO-$d_6$, δ/ppm): 5.63 (t, 1H), 4.59 (d, 2H), 4.20-4.00 (m, 3H), 3.76 (dd, 1H), 3.63 (sext, 1H), 3.21 (s, 3H), 2.84-2.70 (m, 2H), 2.33 (s, 3H), 1.05 (d, 3H).
LC/MS (Methode 3): $R_t$ = 1.01 min, m/z = 381 $[M+H]^+$.

**Beispiel 453A**

6-[Dideutero(hydroxy)methyl]-3-ethyl-1-(2-methoxyethyl)-5-methylthieno[2,3-d]pyrimidin-2,4(1*H*,3*H*)-dion

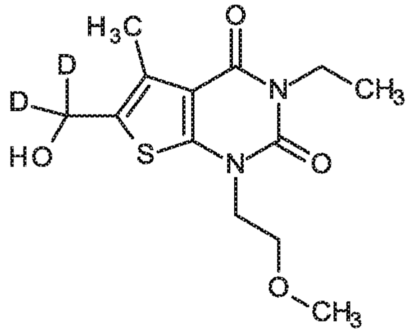

Eine Lösung von 660 mg (1.94 mmol) der Verbindung aus Bsp. 434A in 17 ml THF wurde bei -78°C tropfenweise mit 1.8 ml (1.75 mmol) einer 1 M Lösung von Lithiumaluminiumdeuterid in THF versetzt. Anschließend wurde das Reaktionsgemisch 1 h bei 0°C gerührt. Danach wurden 0.6 ml Wasser und 4.5 ml 1 M Natronlauge hinzugefügt und das Kältebad entfernt. Der entstandene Niederschlag wurde abgesaugt und gründlich mit THF gewaschen. Das mit der Waschflüssigkeit vereinigte Filtrat wurde zur Trockene eingedampft. Der verbliebene Rückstand wurde in Ethylacetat aufgenommen und nacheinander mit Wasser und gesättigter wässriger Natriumchlorid-Lösung gewaschen. Nach Trocknen über wasserfreiem Magnesiumsulfat wurde filtriert und eingeengt. Das Rohprodukt wurde mittels MPLC gereinigt (Isolera, Biotage-Kartusche SNAP KP-Sil, Cyclohexan/Ethylacetat 100:0 → 0:100). Die Produktfraktionen wurden vereinigt, eingedampft und im Hochvakuum getrocknet. Es wurden 298 mg (49% d. Th., 98% Reinheit) der Titelverbindung erhalten.

$^1$H-NMR (400 MHz, DMSO-$d_6$, δ/ppm): 5.50 (s, 1H), 4.04 (t, 2H), 3.90 (q, 2H), 3.64 (t, 2H), 3.24 (s, 3H), 2.33 (s, 3H), 1.12 (t, 3H).

LC/MS (Methode 17, ESIpos): $R_t$ = 1.20 min, m/z = 301.12 $[M+H]^+$.

## Beispiel 454A

6-{[(2-Aminoethyl)amino]methyl}-3-(2,2-dimethylpropyl)-5-methyl-1-(tetrahydrofuran-2-ylmethyl)thieno[2,3-d]pyrimidin-2,4(1*H*,3*H*)-dion (*Racemat*)

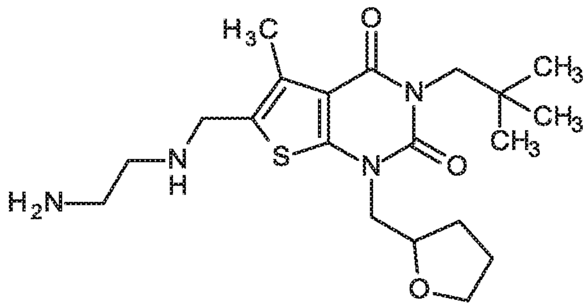

Eine Lösung von 2.0 g (5.49 mmol) der Verbindung aus Bsp. 443A in einem Gemisch aus 50 ml Methanol und 20 ml Dichlormethan wurde zunächst mit 2.2 ml (32.9 mmol) 1,2-Diaminoethan und 1.3 ml (21.9 mmol) Essigsäure versetzt. Nach 30 min wurden 1.38 g (21.9 mmol) Natriumcyanoborhydrid zugefügt, und das Reaktionsgemisch wurde ca. 18 h bei 60°C gerührt. Nach dem Abkühlen auf RT wurde mit 2 M Natronlauge versetzt und mit Ethylacetat extrahiert. Der organische Extrakt wurde mit gesättigter wässriger Natriumchlorid-Lösung gewaschen, über wasserfreiem Magnesiumsulfat getrocknet, filtriert und eingeengt. Der verbliebene Rückstand wurde im Hochvakuum getrocknet und ergab so 2.25 g (85% d. Th., 85% Reinheit) der Titelverbindung, die ohne weitere Reinigung für Folgereaktionen verwendet wurden.

LC/MS (Methode 1, ESIpos): $R_t$ = 0.54 min, m/z = 409 $[M+H]^+$.

**Beispiel 455A**

6-{[(2-Aminoethyl)amino]methyl}-5-methyl-3-(1,1,1-trifluorpropan-2-yl)-1-(3,3,3-trifluorpropyl)-thieno[2,3-d]pyrimidin-2,4(1*H*,3*H*)-dion (*Racemat*)

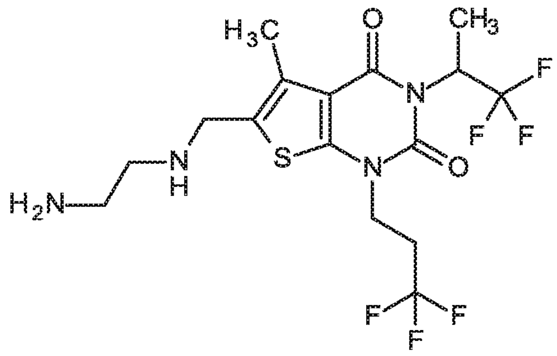

Analog zu dem unter Bsp. 312A beschriebenen Verfahren wurden aus 1.80 g (4.47 mmol) der Verbindung aus Bsp. 444A und 1.61 g (26.8 mmol) 1,2-Diaminoethan 2.37 g (99% d. Th., 84% Reinheit) der Titelverbindung hergestellt, die ohne weitere Aufreinigung für Folgereaktionen verwendet wurden.
LC/MS (Methode 1, ESIpos): $R_t$ = 0.62 min, m/z = 447 $[M+H]^+$.

**Beispiel 456A**

6-{[(2-Aminoethyl)amino]methyl}-1-(2-methoxyethyl)-5-methyl-3-(1,1,1-trifluorpropan-2-yl)-thieno[2,3-d]pyrimidin-2,4(1*H*,3*H*)-dion (*Racemat*)

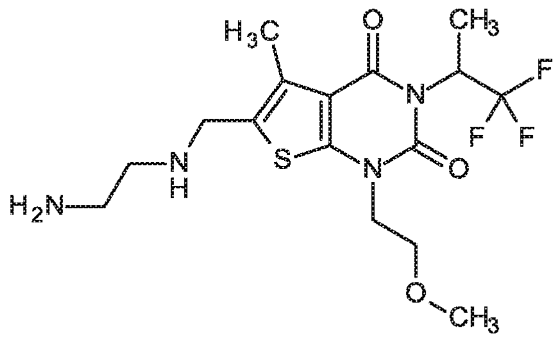

Analog zu dem unter Bsp. 312A beschriebenen Verfahren wurden aus 1.01 g (2.77mmol) der Verbindung aus Bsp. 445A und 1.0 g (16.6 mmol) 1,2-Diaminoethan 1.19 g (85% d. Th., 82% Reinheit) der Titelverbindung hergestellt, die ohne weitere Aufreinigung für Folgereaktionen verwendet wurden.
LC/MS (Methode 1, ESIpos): $R_t$ = 0.48 min, m/z = 409 $[M+H]^+$.

**Beispiel 457A**

6-{[(2-Aminoethyl)amino]methyl}-3-(2-ethoxyethyl)-1-(3-fluorpropyl)-5-methylthieno[2,3-d]-pyrimidin-2,4(1*H*,3*H*)-dion

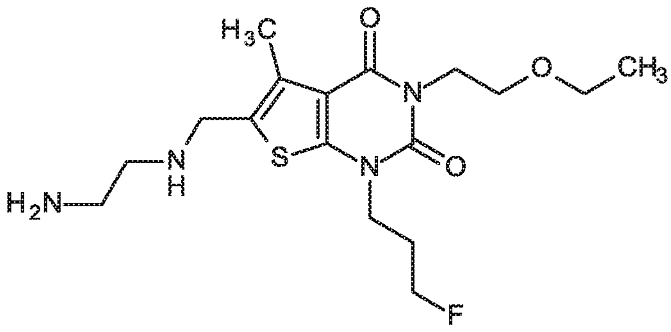

975 mg (2.79 mmol) der Verbindung aus Bsp. 446A wurden in einem Gemisch aus 43 ml Methanol und 20 ml Dichlormethan gelöst. Dann wurden bei RT 1.86 ml (27.9 mmol) 1,2-Diaminoethan und 639 µl (11.2 mmol) Essigsäure zugesetzt. Es wurde 30 min bei RT gerührt. Danach wurden 738 mg (11.2 mmol) Natriumcyanoborhydrid hinzugefügt. Nachdem das Reaktionsgemisch 72 h bei 60°C gerührt worden war, wurde mit 80 ml Wasser versetzt (pH ca. 9) und mit Ethylacetat extrahiert. Der organische Extrakt wurde mit gesättigter Natriumchlorid-Lösung gewaschen, über wasserfreiem Natriumsulfat getrocknet, filtriert und eingeengt. Das erhaltene Rohprodukt ergab nach Trocknen im Hochvakuum 890 mg (62% d. Th., 75% Reinheit) der Titelverbindung, welche ohne weitere Reinigung für Folgereaktionen verwendet wurden.
LC/MS (Methode 3, ESIpos): $R_t$ = 0.55 min, m/z = 387 $[M+H]^+$.

**Beispiel 458A**

6-{[(2-Aminoethyl)amino]methyl}-3-(2-methoxypropyl)-5-methyl-1-(3,3,3-trifluorpropyl)thieno-[2,3-d]pyrimidin-2,4(1*H*,3*H*)-dion (*Racemat*)

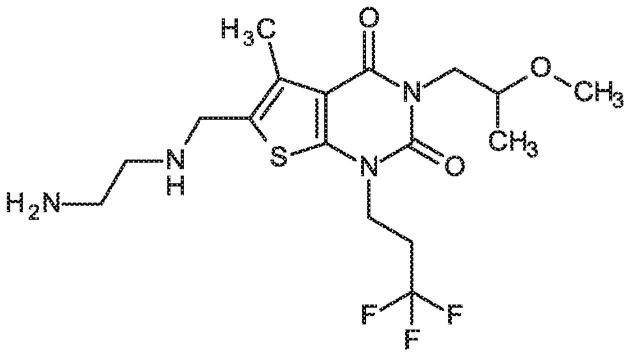

2.8 g (7.10 mmol) der Verbindung aus Bsp. 447A wurden in einem Gemisch aus 50 ml Methanol und 25 ml Dichlormethan gelöst und bei RT mit 2.8 ml (42.6 mmol) 1,2-Diaminoethan und 1.6 ml (28.4 mmol) Essigsäure versetzt. Nach 30 min wurden 1.79 g (28.4 mmol) Natriumcyanoborhydrid hinzugefügt, und das Reaktionsgemisch wurde auf 60°C erwärmt. Nach ca. 15 h ließ man das Reaktionsgemisch auf RT abkühlen. Der Großteil des Lösungsmittels wurde am Rotationsverdampfer entfernt. Der verbliebene Rückstand wurde mit 50 ml 2 M Natronlauge versetzt und gründlich mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach Trocknen im Hochvakuum 3.15 g (99% d. Th., 95% Reinheit) der Titelverbindung, welche ohne weitere Reinigung für Folgereaktionen verwendet wurden.
LC/MS (Methode 6, ESIpos): $R_t$ = 2.01 min, m/z = 361 $[M+H-62]^+$.

**Beispiel 459A**

3-Ethyl-6-(*N*-hydroxyethanimidoyl)-5-methyl-1-(3,3,3-trifluorpropyl)thieno[2,3-d]pyrimidin-2,4(1*H*,3*H*)-dion

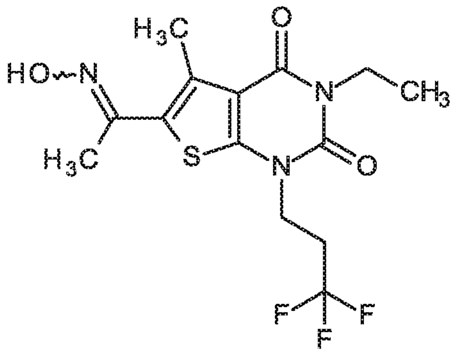

Eine Lösung von 665 mg (1.91 mmol) der Verbindung aus Bsp. 449A in 20 ml Ethanol wurde mit 351 µl (5.73 mmol) Hydroxylamin (50% Lösung in Wasser) versetzt und ca. 18 h unter Rückfluss erhitzt. Nach dem Abkühlen auf RT wurde mit Wasser versetzt, wobei das Produkt ausfiel. Es wurde abgesaugt, mit wenig kaltem Wasser gewaschen und im Hochvakuum getrocknet. Es wurden 673 mg (97% d. Th.) der Titelverbindung erhalten, welche als *E*/*Z*-Isomerengemisch anfiel (Verhältnis ca. 3:1, ohne Zuordnung).
$^1$H-NMR (400 MHz, DMSO-$d_6$, δ/ppm; Hauptisomer): 11.42 (s, 1H), 4.12 (t, 2H), 3.91 (q, 2H), 2.85-2.69 (m, 2H), 2.59 (s, 3H), 2.23 (s, 3H), 1.12 (t, 3H).
LC/MS (Methode 17, ESIpos; Hauptisomer): $R_t$ = 1.82 min, m/z = 364.09 $[M+H]^+$.

**Beispiel 460A**

6-(Aminomethyl)-3-ethyl-5-methyl-1-(3,3,3-trifluorpropyl)thieno[2,3-d]pyrimidin-2,4(1*H*,3*H*)-dion

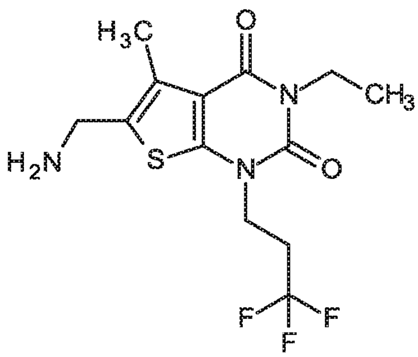

Eine Lösung von 990 mg (2.83 mmol) der Verbindung aus Bsp. 190A in 70 ml Methanol wurde mit 590 µl (7.08 mmol) konzentrierter Salzsäure und 100 mg Palladium auf Kohle (10%) versetzt. Anschließend wurde bei einem Wasserstoffdruck von 1 bar 2 h lang bei RT hydriert. Danach wurde vom Katalysator über wenig Kieselgur abfiltriert und das Filtrat am Rotationsverdampfer eingeengt. Der verbliebene Rückstand wurde in 50 ml Ethylacetat gelöst und nacheinander mit gesättigter wässriger Natriumhydrogencarbonat-Lösung (zweimal) und gesättigter wässriger Natriumchlorid-Lösung gewaschen. Nach Trocknen über wasserfreiem Magnesiumsulfat, Filtration, Einengen und Trocknen des Produkts im Hochvakuum wurden 920 mg (96% d. Th.) der Titelverbindung erhalten.
$^1$H-NMR (400 MHz, DMSO-$d_6$, δ/ppm): 4.12 (t, 2H), 3.90 (q, 2H), 2.83-2.71 (m, 2H), 2.32 (s, 3H), 2.19 (br. s, 2H), 1.11 (t, 3H).
LC/MS (Methode 6, ESIpos): $R_t$ = 1.27 min, m/z = 319 $[M+H-NH_3]^+$.

**Beispiel 461A**

6-(1-Aminoethyl)-3-ethyl-5-methyl-1-(3,3,3-trifluorpropyl)thieno[2,3-d]pyrimidin-2,4(1*H*,3*H*)-dion (*Racemat*)

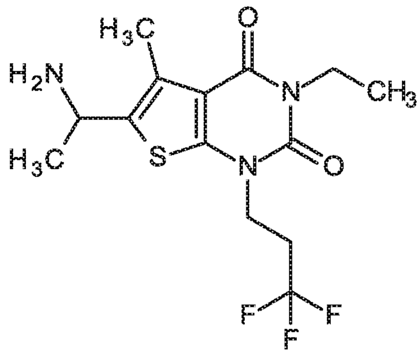

Eine Lösung von 235 mg (0.991 mmol) Nickel(II)chlorid-Hexahydrat und 37 mg (0.991 mmol) Natriumborhydrid in 10 ml Methanol wurde mit einer Lösung von 360 mg (0.991 mmol) der Verbindung aus Bsp. 459A in 30 ml Methanol versetzt. Anschließend wurden unter starker Gasentwicklung weitere 206 mg (5.45 mmol) Natriumborhydrid hinzugefügt. Nach 3 h Rühren bei RT wurden nochmals 118 mg (0.496 mmol) Nickel(II)chlorid-Hexahydrat sowie 115 mg (3.17 mmol) Natriumborhydrid zugesetzt. Nach weiteren 3 h bei RT wurde das Reaktionsgemisch über Kieselgur filtriert und das Filtrat am Rotationsverdampfer bis zur Trockene eingeengt. Der verbliebene Rückstand wurde in 20 ml Wasser gelöst, mit Ammoniakwasser versetzt und dreimal mit Ethylacetat extrahiert. Der vereinigte organische Extrakt wurde nacheinander mit Wasser und gesättigter wässriger Natriumchlorid-Lösung gewaschen, über wasserfreiem Magnesiumsulfat getrocknet, filtriert und eingeengt. Nach Trocknen im Hochvakuum wurden 314 mg (72% d. Th., 80% Reinheit) der Titelverbindung erhalten, die ohne weitere Reinigung für Folgereaktionen verwendet wurden.
LC/MS (Methode 17, ESIpos): $R_t$ = 0.92 min, m/z = 333.09 $[M+H-NH_3]^+$.

## Beispiel 462A

*tert.*-Butyl-2-{[3-(2,2-dimethylpropyl)-5-methyl-2,4-dioxo-1-(3,3,3-trifluorpropyl)-1,2,3,4-tetrahydrothieno[2,3-d]pyrimidin-6-yl]methylen}hydrazincarboxylat

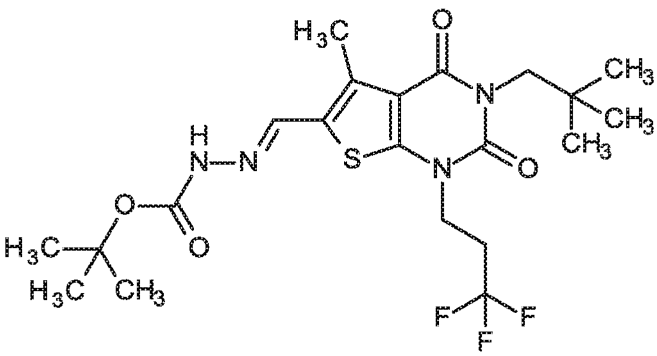

Analog zu dem unter Bsp. 333A beschriebenen Verfahren wurden aus 500 mg (1.33 mmol) der Verbindung aus Bsp. 291A und 263 mg (1.99 mmol) *tert.*-Butyl-hydrazincarboxylat 649 mg (99% d. Th.) der Titelverbindung erhalten.
$^1$H-NMR (400 MHz, DMSO-$d_6$, δ/ppm): 10.88 (br. s, 1H), 8.30 (s, 1H), 4.15 (t, 2H), 3.81 (s, 2H), 2.90-2.71 (m, 2H), 2.45 (s, 3H), 1.46 (s, 9H), 0.90 (s, 9H).
LC/MS (Methode 17, ESIneg): $R_t$ = 2.41 min, m/z = 489.18 $[M-H]^-$.

## Beispiel 463A

*tert.*-Butyl-2-{[3-(2,2-dimethylpropyl)-1-(2-methoxyethyl)-5-methyl-2,4-dioxo-1,2,3,4-tetrahydrothieno[2,3-d]pyrimidin-6-yl]methylen}hydrazincarboxylat

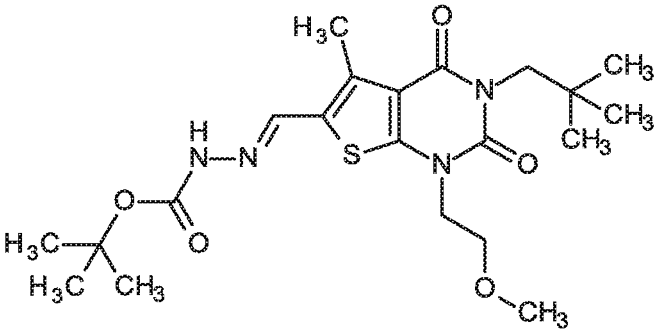

Analog zu dem unter Bsp. 333A beschriebenen Verfahren wurden aus 220 mg (0.650 mmol) der Verbindung aus Bsp. 293A und 129 mg (0.975 mmol) *tert.*-Butyl-hydrazincarboxylat 270 mg (91% d. Th.) der Titelverbindung erhalten.
$^1$H-NMR (400 MHz, DMSO-$d_6$, $\delta$/ppm): 10.85 (br. s, 1H), 8.29 (s, 1H), 4.07 (t, 2H), 3.81 (s, 2H), 3.64 (t, 2H), 3.24 (s, 3H), 2.44 (s, 3H), 1.46 (s, 9H), 0.89 (s, 9H).
LC/MS (Methode 17, ESIneg): $R_t$ = 2.25 min, m/z = 451.20 $[M-H]^-$.

## Beispiel 464A

*tert.*-Butyl-2-{[3-(2,2-dimethylpropyl)-5-methyl-2,4-dioxo-1-(tetrahydrofuran-2-ylmethyl)-1,2,3,4-tetrahydrothieno[2,3-d]pyrimidin-6-yl]methylen}hydrazincarboxylat (*Racemat*)

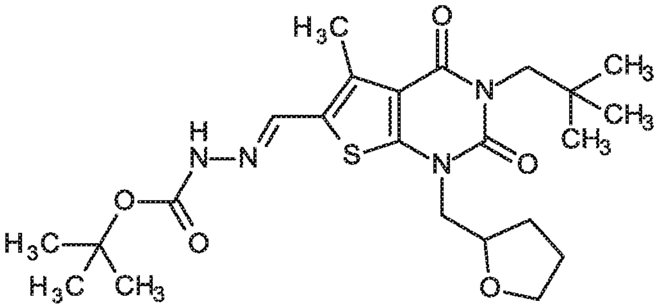

Analog zu dem unter Bsp. 333A beschriebenen Verfahren wurden aus 350 mg (0.960 mmol) der Verbindung aus Bsp. 443A und 190 mg (1.44 mmol) *tert.*-Butyl-hydrazincarboxylat 360 mg (76% d. Th., 97% Reinheit) der Titelverbindung erhalten.
$^1$H-NMR (400 MHz, DMSO-$d_6$, $\delta$/ppm): 10.85 (br. s, 1H), 8.29 (s, 1H), 4.29-4.16 (m, 1H), 4.07 (dd, 1H), 3.88-3.70 (m, 4H), 3.67-3.56 (m, 1H), 2.44 (s, 3H), 2.07-1.75 (m, 3H), 1.73-1.58 (m, 1H), 1.45 (s, 9H), 0.90 (s, 9H).
LC/MS (Methode 17, ESIneg): $R_t$ = 2.36 min, m/z = 477.22 $[M-H]^-$.

## Beispiel 465A

*tert.*-Butyl-2-{[5-methyl-2,4-dioxo-3-(1,1,1-trifluorpropan-2-yl)-1-(3,3,3-trifluorpropyl)-1,2,3,4-tetrahydrothieno[2,3-d]pyrimidin-6-yl]methylen}hydrazincarboxylat (*Racemat*)

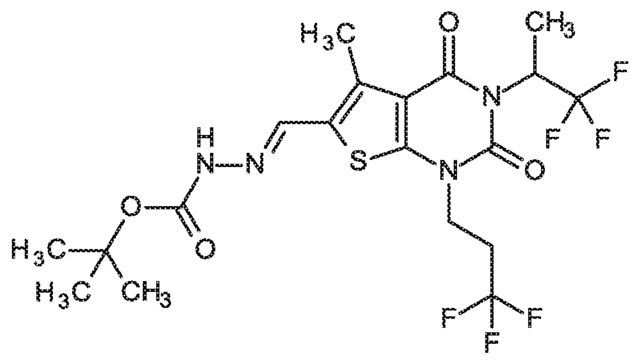

Analog zu dem unter Bsp. 333A beschriebenen Verfahren wurden aus 400 mg (0.994 mmol) der Verbindung aus Bsp. 444A und 197 mg (1.49 mmol) *tert*.-Butyl-hydrazincarboxylat 493 mg (96% d. Th.) der Titelverbindung erhalten. $^1$H-NMR (400 MHz, DMSO-$d_6$, δ/ppm): 10.91 (br. s, 1H), 8.31 (s, 1H), 5.86-5.42 (m, 1H), 4.24-4.06 (m, 2H), 2.85-2.74 (m, 2H), 2.48-2.41 (m, 3H), 1.72-1.59 (m, 3H), 1.46 (s, 9H).
LC/MS (Methode 17, ESIneg): $R_t$ = 2.24 min, m/z = 515.12 [M-H]$^-$.

## Beispiel 466A

*tert*.-Butyl-2-{[1-(2-methoxyethy1)-5-methyl-2,4-dioxo-3-(1,1,1-trifluorpropan-2-yl)-1,2,3,4-tetra-hydrothieno[2,3-d]pyrimidin-6-yl]methylen}hydrazincarboxylat *(Racemat)*

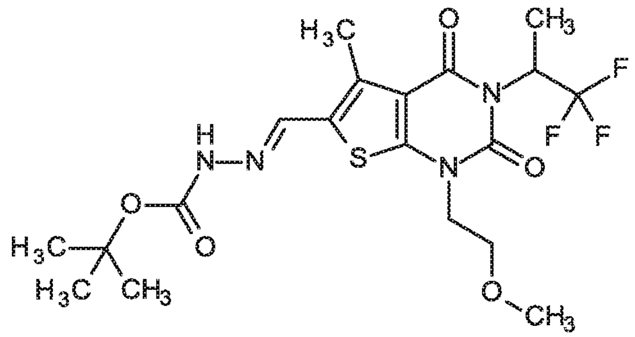

Analog zu dem unter Bsp. 333A beschriebenen Verfahren wurden aus 400 mg (1.10 mmol) der Verbindung aus Bsp. 445A und 218 mg (1.65 mmol) *tert*.-Butyl-hydrazincarboxylat 509 mg (96% d. Th.) der Titelverbindung erhalten. $^1$H-NMR (400 MHz, DMSO-$d_6$, δ/ppm): 10.88 (br. s, 1H), 8.29 (s, 1H), 5.85-5.46 (m, 1H), 4.12-4.05 (m, 2H), 3.68-3.61 (m, 2H), 3.26 (s, 3H), 2.47-2.38 (m, 3H), 1.74-1.58 (m, 3H), 1.46 (s, 9H).
LC/MS (Methode 17, ESIneg): $R_t$ = 2.09 min, m/z = 477.14 [M-H]$^-$.

## Beispiel 467A

*tert*.-Butyl-2-{[3-(2-methoxypropyl)-5-methyl-2,4-dioxo-1-(3,3,3-trifluorpropyl)-1,2,3,4-tetra-hydrothieno[2,3-d]pyrimidin-6-yl]methylen}hydrazincarboxylat *(Racemat)*

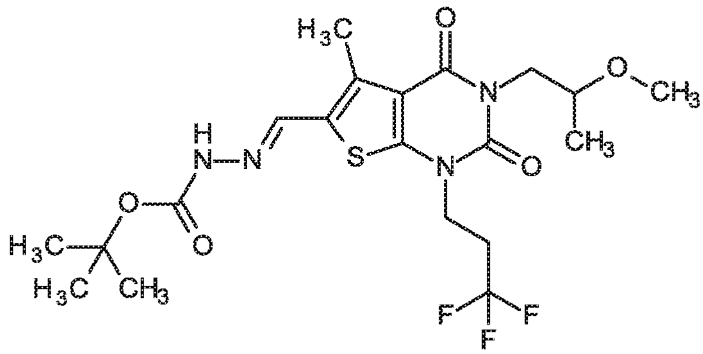

Eine Lösung von 1.1 g (2.91 mmol) der Verbindung aus Bsp. 447A in 35 ml Ethanol wurde zunächst mit 576 mg (4.36 mmol) tert.-Butyl-hydrazincarboxylat und dann mit 5 Tropfen konzentrierter Salzsäure versetzt. Nachdem das Reaktionsgemisch ca. 18 h bei RT gerührt worden war, wurde der Großteil des Ethanols am Rotationsverdampfer entfernt. Der verbliebene Rückstand wurde mit 150 ml Wasser verdünnt und durch Zusatz von gesättigter wässriger Natriumhydrogencarbonat-Lösung neutralisiert. Der dabei ausgefallene Feststoff wurde abgesaugt, mit wenig Wasser gewaschen und im Hochvakuum getrocknet. Es wurden 1.38 g (91% d. Th., 95% Reinheit) der Titelverbindung erhalten.
$^1$H-NMR (400 MHz, DMSO-$d_6$, $\delta$/ppm): 10.88 (br. s, 1H), 8.30 (s, 1H), 4.23-4.08 (m, 2H), 4.04 (dd, 1H), 3.76 (dd, 1H), 3.63 (sext, 1H), 3.22 (s, 3H), 2.88-2.71 (m, 2H), 2.46 (s, 3H), 1.46 (s, 9H), 1.06 (d, 3H).
LC/MS (Methode 1, ESIpos): $R_t$ = 1.08 min, m/z = 493 $[M+H]^+$.

**Beispiel 468A**

*tert.*-Butyl-2-{[3-(2,2-dimethylpropyl)-5-methyl-2,4-dioxo-1-(3,3,3-trifluorpropyl)-l,2,3,4-tetra-hydrothieno[2,3-d]pyrimidin-6-yl]methyl}hydrazincarboxylat

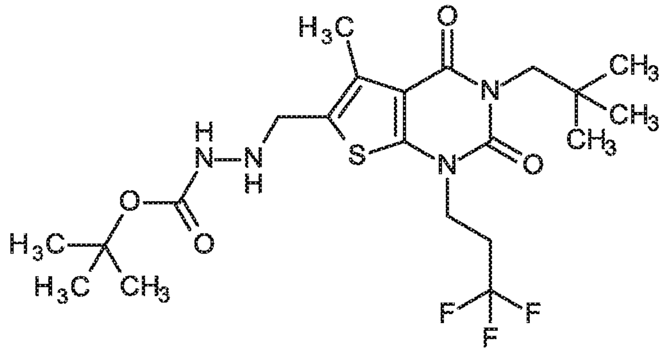

Analog zu dem unter Bsp. 342A beschriebenen Verfahren wurden aus 650 mg (1.33 mmol) der Verbindung aus Bsp. 462A 590 mg (85% d. Th., 94% Reinheit) der Titelverbindung erhalten.
$^1$H-NMR (400 MHz, DMSO-$d_6$, $\delta$/ppm): 8.27 (br. s, 1H), 5.06 (br. s, 1H), 4.13 (t, 2H), 3.98 (br. d, 2H), 3.82 (br. s, 2H), 2.87-2.69 (m, 2H), 2.32 (s, 3H), 1.38 (s, 9H), 0.89 (s, 9H).
LC/MS (Methode 17, ESIneg): $R_t$ = 2.35 min, m/z = 491.19 $[M-H]^-$.

**Beispiel 469A**

*tert.*-Butyl-2-{[3-(2,2-dimethylpropyl)-1-(2-methoxyethyl)-5-methyl-2,4-dioxo-1,2,3,4-tetrahydro-thieno[2,3-d]pyrimidin-6-yl]methyl}hydrazincarboxylat

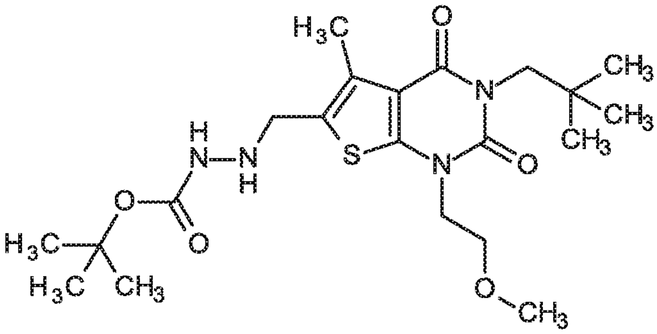

Analog zu dem unter Bsp. 342A beschriebenen Verfahren wurden aus 268 mg (0.592 mmol) der Verbindung aus Bsp. 463A 216 mg (78% d. Th., 98% Reinheit) der Titelverbindung erhalten. Die Reaktionszeit betrug hier insgesamt ca. 20 h.
$^1$H-NMR (400 MHz, DMSO-$d_6$, δ/ppm): 8.27 (br. s, 1H), 4.99 (br. s, 1H), 4.08-4.00 (m, 2H), 3.96 (br. d, 2H), 3.82 (br. s, 2H), 3.64 (t, 2H), 3.24 (s, 3H), 2.32 (s, 3H), 1.38 (s, 9H), 0.89 (s, 9H).
LC/MS (Methode 17, ESIneg): $R_t$ = 2.15 min, m/z = 453.22 $[M-H]^-$.

**Beispiel 470A**

*tert.*-Butyl-2-{[3-(2,2-dimethylpropyl)-5-methyl-2,4-dioxo-1-(tetrahydrofuran-2-ylmethyl)-1,2,3,4-tetrahydrothieno[2,3-d]pyrimidin-6-yl]methyllhydrazincarboxylat *(Racemat)*

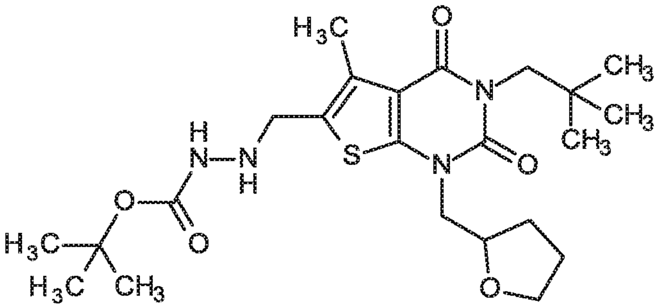

Analog zu dem unter Bsp. 342A beschriebenen Verfahren wurden aus 360 mg (0.752 mmol) der Verbindung aus Bsp. 464A 402 mg (100% d. Th., 90% Reinheit) der Titelverbindung erhalten, die ohne weitere Aufreinigung für Folgereaktionen verwendet wurden (auf eine chromatographische Reinigung wurde hier verzichtet).
$^1$H-NMR (400 MHz, DMSO-$d_6$, δ/ppm): 8.27 (br. s, 1H), 4.98 (br. s, 1H), 4.29-4.22 (m, 1H), 4.08-3.89 (m, 3H), 3.88-3.69 (m, 4H), 3.67-3.56 (m, 1H), 2.32 (s, 3H), 2.01-1.76 (m, 3H), 1.74-1.60 (m, 1H), 1.38 (s, 9H), 0.89 (s, 9H).
LC/MS (Methode 17, ESIneg): $R_t$ = 2.26 min, m/z = 479.23 $[M-H]^-$.

**Beispiel 471A**

*tert.*-Butyl-2-{[5-methyl-2,4-dioxo-3-(1,1,1-trifluorpropan-2-yl)-1-(3,3,3-trifluorpropyl)-1,2,3,4-tetrahydrothieno[2,3-d]pyrimidin-6-yl]methyl}hydrazincarboxylat *(Racemat)*

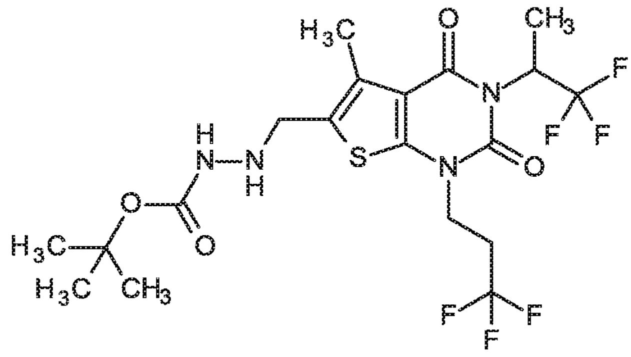

Analog zu dem unter Bsp. 342A beschriebenen Verfahren wurden aus 490 mg (0.949 mmol) der Verbindung aus Bsp. 465A 444 mg (90% d. Th.) der Titelverbindung erhalten.
$^1$H-NMR (400 MHz, DMSO-$d_6$, δ/ppm): 8.26 (br. s, 1H), 5.85-5.46 (m, 1H), 5.11 (br. s, 1H), 4.13 (t, 2H), 4.02-3.94 (m, 2H), 2.89-2.68 (m, 2H), 2.38-2.28 (m, 3H), 1.72-1.59 (m, 3H), 1.38 (s, 9H).
LC/MS (Methode 17, ESIneg): $R_t$ = 2.21 min, m/z = 517.14 $[M-H]^-$.

## Beispiel 472A

*tert.*-Butyl-2-{[1-(2-methoxyethyl)-5-methyl-2,4-dioxo-3-(1,1,1-trifluorpropan-2-yl)-1,2,3,4-tetra-hydrothieno[2,3-d]pyrimidin-6-yl]methyl}hydrazincarboxylat (*Racemat*)

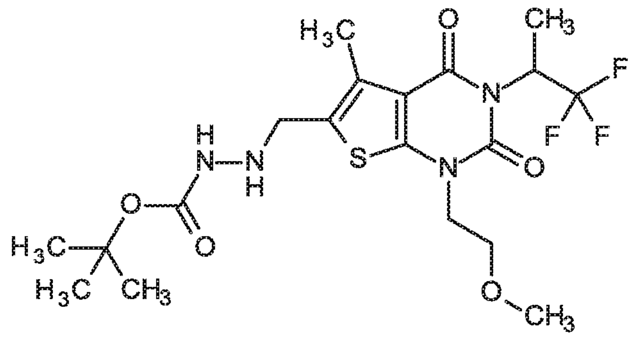

Analog zu dem unter Bsp. 342A beschriebenen Verfahren wurden aus 500 mg (1.05 mmol) der Verbindung aus Bsp. 466A 454 mg (86% d. Th., 95% Reinheit) der Titelverbindung erhalten.
$^1$H-NMR (400 MHz, DMSO-$d_6$, δ/ppm): 8.26 (br. s, 1H), 5.86-5.46 (m, 1H), 5.03 (br. s, 1H), 4.14-3.84 (m, 4H), 3.72-3.57 (m, 2H), 3.25 (s, 3H), 2.36-2.24 (m, 3H), 1.73-1.58 (m, 3H), 1.38 (br. s, 9H).
LC/MS (Methode 17, ESIneg): $R_t$ = 2.00 min, m/z = 479.16 $[M-H]^-$.

## Beispiel 473A

*tert.*-Butyl-2-{[3-(2-methoxypropyl)-5-methyl-2,4-dioxo-1-(3,3,3-trifluorpropyl)-1,2,3,4-tetra-hydrothieno[2,3-d]pyrimidin-6-yl]methyl}hydrazincarboxylat *(Racemat)*

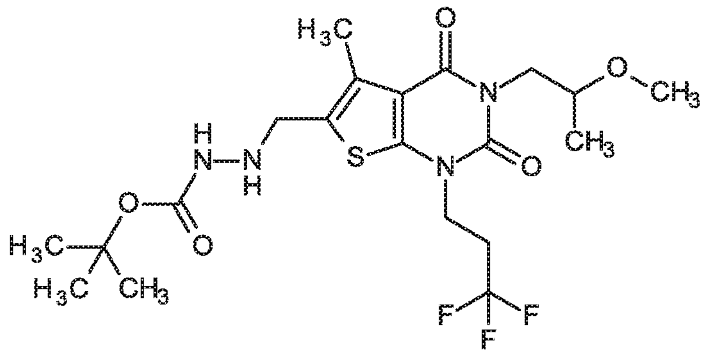

Eine Lösung von 1.37 g (2.64 mmol, 95% Reinheit) der Verbindung aus Bsp. 467A in 25 ml Methanol wurde mit 830 mg (13.2 mmol) Natriumcyanoborhydrid und etwas Bromkresolgrün (als Indikator) versetzt. Anschließend wurde soviel Essigsäure hinzutitriert, dass die Indikatorfarbe gerade von blau nach gelb umschlug. Dann wurde das Reaktionsgemisch auf 65°C erwärmt. Nach 1 h, nach 3 h und nach 4 h wurden jeweils weitere 415 mg (6.61 mmol) Natriumcyanoborhydrid hinzugefügt. Während der gesamten Reaktionszeit wurde durch weiteren Zusatz von Essigsäure der pH-Wert immer wieder so reguliert, dass die Indikatorfarbe gerade gelb blieb. Nach insgesamt 5 h wurden die flüchtigen Bestandteile des Reaktionsgemisches am Rotationsverdampfer weitgehend entfernt. Der verbliebene Rückstand wurde in Ethylacetat aufgenommen und nacheinander mit gesättigter wässriger Natriumhydrogencarbonat-Lösung, Wasser und gesättigter wässriger Natriumchlorid-Lösung gewaschen. Nach Trocknen über wasserfreiem Magnesiumsulfat wurde filtriert und zur Trockene eingeengt. Das Produkt wurde mittels Chromatographie isoliert (Biotage Isolera One, Kartusche SNAP KP-Sil, 100 g Kieselgel, Laufmittel Cyclohexan/Ethylacetat 2:1). Nach Vereinigen der Produktfraktionen, Eindampfen und Trocknen im Hochvakuum wurden so 890 mg (67% d. Th.) der Titelverbindung erhalten.
$^1$H-NMR (400 MHz, DMSO-$d_6$, δ/ppm): 8.27 (br. s, 1H), 5.08 (br. m, 1H), 4.20-4.08 (m, 2H), 4.08-4.01 (m, 1H), 3.99 (br. d, 2H), 3.76 (dd, 1H), 3.63 (sext, 1H), 3.21 (s, 3H), 2.85-2.70 (m, 2H), 2.33 (s, 3H), 1.38 (s, 9H), 1.05 (d, 3H).
LC/MS (Methode 1, ESIpos): $R_t$ = 1.02 min, m/z = 495 $[M+H]^+$.

## Beispiel 474A

*tert.*-Butyl-2-carbamoyl-2-{[3-ethyl-5-methyl-2,4-dioxo-1-(3,3,3-trifluorpropyl)-1,2,3,4-tetrahydro-thieno[2,3-d]pyrimidin-6-yl]methyl}hydrazincarboxylat

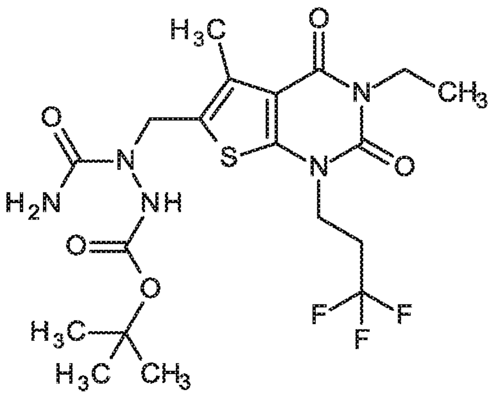

Eine Lösung von 16.56 g (36.8 mmol) der Verbindung aus Bsp. 339A in 700 ml Isopropanol wurde mit 15.1 ml (110 mmol) Trimethylsilylisocyanat versetzt und 3 h bei 50°C gerührt. Danach wurde das Reaktionsgemisch zur Trockene eingeengt. Der Rückstand wurde in Ethylacetat aufgenommen und nacheinander mit gesättigter wässriger Natriumhydrogencarbonat-Lösung und gesättigter wässriger Natriumchlorid-Lösung gewaschen. Nach Trocknen über wasserfreiem Magnesiumsulfat wurde filtriert und eingeengt. Der verbliebene Rückstand wurde in drei Portionen mittels MPLC gereinigt (Biotage Isolera, Kartusche mit 340 g Kieselgel, Cyclohexan/Ethylacetat 1:1). Die hinreichend sauberen Produktfraktionen wurden vereinigt, eingeengt und im Hochvakuum getrocknet. Dies ergab eine erste Fraktion der Titelverbindung (10.38 g). Die aus der MPLC erhaltenen Mischfraktionen wurden ebenfalls vereinigt und eingeengt und dann

ein weiteres Mal mittels MPLC gereinigt. Auf diese Weise wurde eine zweite Fraktion der Titelverbindung erhalten (2.53 g). Insgesamt wurden somit 12.91 g (66% d. Th., 94% Reinheit) der Titelverbindung erhalten.
$^1$H-NMR (400 MHz, DMSO-$d_6$, δ/ppm): 8.92 (br. s, 1H), 6.21 (s, 2H), 4.59 (br. s, 2H), 4.13 (t, 2H), 3.91 (q, 2H), 2.87-2.68 (m, 2H), 2.35 (s, 3H), 1.38 (br. s, 9H), 1.11 (t, 3H).
LC/MS (Methode 17, ESIneg): $R_t$ = 1.67 min, m/z = 492.15 $[M-H]^-$.

**Beispiel 475A**

*tert*.-Butyl-2-carbamoyl-2-{[3-ethyl-5-methyl-2,4-dioxo-1-(tetrahydrofuran-2-ymethyl)-1,2,3,4-tetrahydrothieno[2,3-d]pyrimidin-6-yl]methyl}hydrazincarboxylat (*Racemat*)

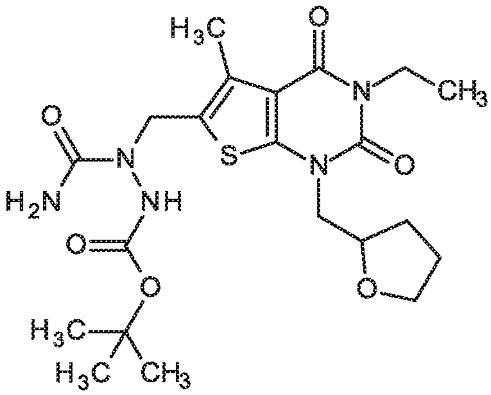

Eine Lösung von 214 mg (0.488 mmol) der Verbindung aus Bsp. 341A in 11 ml Isopropanol wurde mit 131 μl (0.976 mmol) Trimethylsilylisocyanat versetzt und bei RT gerührt. Nach 24 h wurden weitere 50 μl (0.373 mmol) Trimethylsilylisocyanat hinzugefügt und das Rühren bei RT fortgesetzt. Nach weiteren 2 Tagen war der Umsatz noch nicht vollständig. Es wurden daher nochmals 131 μl (0.976 mmol) Trimethylsilylisocyanat zugesetzt, und das Reaktionsgemisch wurde auf 50°C erwärmt. Nach weiteren 24 h wurde das Reaktionsgemisch zur Trockene eingeengt. Der verbliebene Rückstand wurde mittels MPLC gereinigt (Biotage Isolera, Kartusche mit 100 g Kieselgel, Cyclohexan/Ethylacetat 1:1 → Dichlormethan/Methanol 20:1). Die hinreichend sauberen Produktfraktionen wurden vereinigt, eingeengt und im Hochvakuum getrocknet. Es wurden 238 mg (85% d. Th., 84% Reinheit) der Titelverbindung erhalten, die ohne weitere Aufreinigung für Folgereaktionen verwendet wurden.
$^1$H-NMR (400 MHz, DMSO-$d_6$, δ/ppm): 9.70 (s, 1H), 7.36-6.95 (m, 2H), 4.58 (br. s, 1H), 4.30-4.19 (m, 1H), 4.02-3.96 (m, 1H), 3.91 (q, 2H), 3.82-3.70 (m, 2H), 3.66-3.56 (m, 1H), 2.34 (s, 3H), 2.03-1.75 (m, 3H), 1.73-1.59 (m, 1H), 1.39 (br. s, 9H), 1.11 (t, 3H).
LC/MS (Methode 17, ESIneg): $R_t$ = 1.52 min, m/z = 480.19 $[M-H]^-$.

**Beispiel 476A**

*tert*.-Butyl-2-carbamoyl-2-{[3-isopropyl-5-methyl-2,4-dioxo-1-(tetrahydrofuran-2-ymethyl)-1,2,3,4-tetrahydrothieno[2,3-d]pyrimidin-6-yl]methyl}hydrazincarboxylat (*Racemat*)

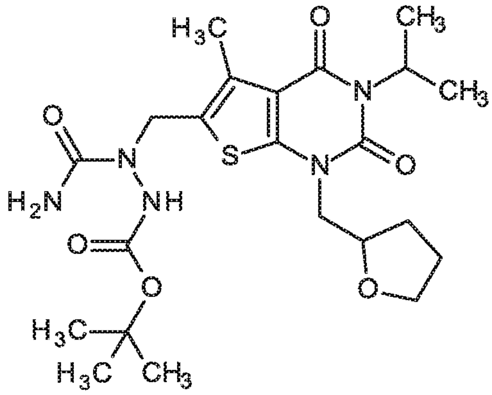

Eine Lösung von 372 mg (0.822 mmol) der Verbindung aus Bsp. 344A in 18 ml Isopropanol wurde mit 220 µl (1.64 mmol) Trimethylsilylisocyanat versetzt und bei RT gerührt. Nach 24 h wurden weitere 10 µl (0.075 mmol) Trimethylsilylisocyanat hinzugefügt und das Rühren bei RT fortgesetzt. Nach weiteren 2 Tagen wurde das Reaktionsgemisch zur Trockene eingeengt. Der verbliebene Rückstand wurde mittels MPLC gereinigt (Biotage Isolera, Kartusche mit 100 g Kieselgel, Cyclohexan/Ethylacetat 1:1 → Dichlormethan/Methanol 20:1). Die hinreichend sauberen Produktfraktionen wurden vereinigt, eingeengt und im Hochvakuum getrocknet. Es wurden 366 mg (74% d. Th., 83% Reinheit) der Titelverbindung erhalten, die ohne weitere Aufreinigung für Folgereaktionen verwendet wurden.
$^1$H-NMR (400 MHz, DMSO-$d_6$, δ/ppm): 8.91 (br. s, 1H), 6.17 (s, 2H), 5.15 (sept, 1H), 5.03-4.29 (breit, 2H), 4.28-4.18 (m, 1H), 4.02-3.95 (m, 1H), 3.81-3.66 (m, 2H), 3.65-3.57 (m, 1H), 2.32 (s, 3H), 2.02-1.75 (m, 3H), 1.73-1.59 (m, 1H), 1.40 (breit, 15H).
LC/MS (Methode 17, ESIneg): $R_t$ = 1.68 min, m/z = 494.21 [M-H]$^-$.

**Beispiel 477A**

*tert.*-Butyl-2-carbamoyl-2-{[3-(2,2-dimethylpropyl)-5-methyl-2,4-dioxo-1-(3,3,3-trifluorpropyl)-1,2,3,4-tetrahydrothieno[2,3-d]pyrimidin-6-yl]methyl}hydrazincarboxylat

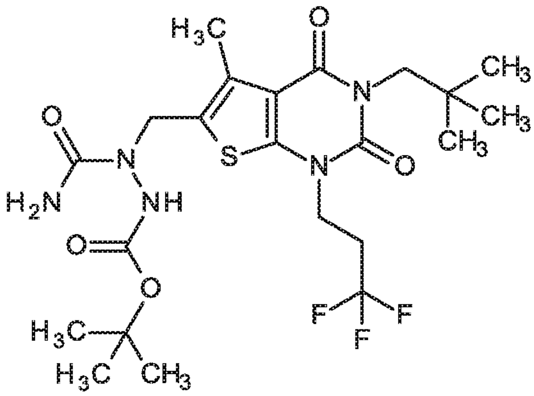

Eine Lösung von 150 mg (0.286 mmol, 94% Reinheit) der Verbindung aus Bsp. 468A in 10 ml Isopropanol wurde mit 115 µl (0.859 mmol) Trimethylsilylisocyanat versetzt und 5 h bei 50°C gerührt. Danach wurde das Reaktionsgemisch zur Trockene eingeengt. Der verbliebene Rückstand wurde mittels MPLC gereinigt (Biotage Isolera, Kartusche mit 25 g Kieselgel, Cyclohexan/Ethylacetat 1:1 → Dichlormethan/Methanol 20:1). Die hinreichend sauberen Produktfraktionen wurden vereinigt, eingeengt und im Hochvakuum getrocknet. Es wurden 154 mg (91% d. Th., 91% Reinheit) der Titelverbindung erhalten, welche ohne weitere Reinigung für Folgereaktionen verwendet wurden.
$^1$H-NMR (400 MHz, DMSO-$d_6$, δ/ppm): 11.94 (br. s, 1H), 6.20 (br. s, 2H), 5.12-4.25 (m, 2H), 4.13 (t, 2H), 3.82 (br. s, 2H), 2.84-2.65 (m, 2H), 2.34 (s, 3H), 1.37 (br. s, 9H), 0.88 (s, 9H).
LC/MS (Methode 17, ESIneg): $R_t$ = 2.05 min, m/z = 534.20 [M-H]$^-$.

**Beispiel 478A**

*tert.*-Butyl-2-carbamoyl-2-{[3-(2,2-dimethylpropyl)-1-(2-methoxyethyl)-5-methyl-2,4-dioxo-1,2,3,4-tetrahydrothieno[2,3-d]pyrimidin-6-yl]methyl} hydrazincarboxylat

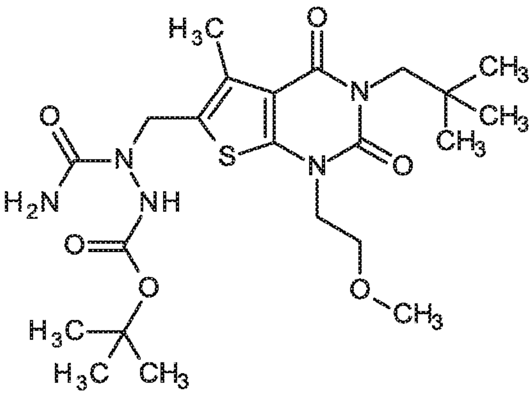

Eine Lösung von 214 mg (0.471 mmol) der Verbindung aus Bsp. 469A in 14 ml Isopropanol wurde mit 193 µl (1.41mmol) Trimethylsilylisocyanat versetzt und ca. 18 h bei 50°C gerührt. Danach wurde das Reaktionsgemisch zur Trockene eingeengt. Der Rückstand wurde in Ethylacetat aufgenommen und nacheinander mit gesättigter wässriger Natriumhydrogencarbonat-Lösung und gesättigter wässriger Natriumchlorid-Lösung gewaschen. Nach Trocknen über wasserfreiem Magnesiumsulfat wurde filtriert und eingeengt. Es wurden 299 mg (100% d. Th., 80% Reinheit) der Titelverbindung erhalten, die ohne weitere Aufreinigung für Folgereaktionen verwendet wurden.
$^1$H-NMR (400 MHz, DMSO-$d_6$, δ/ppm): 8.94 (br. s, 1H), 6.18 (br. s, 2H), 4.92-4.24 (m, 2H), 4.03 (q, 2H), 3.83 (br. s, 2H), 3.63 (t, 2H), 3.23 (s, 3H), 2.33 (s, 3H), 1.38 (br. s, 9H), 0.88 (s, 9H).
LC/MS (Methode 17, ESIneg): $R_t$ = 1.80 min, m/z = 496.22 [M-H]$^-$.

**Beispiel 479A**

*tert.*-Butyl-2-carbamoyl-2-{[3-(2,2-dimethylpropyl)-5-methyl-2,4-dioxo-1-(tetrahydrofuran-2-yl-methyl)-1,2,3,4-tetrahydrothieno[2,3-d]pyrimidin-6-yl]methyl}hydrazincarboxylat (*Racemat*)

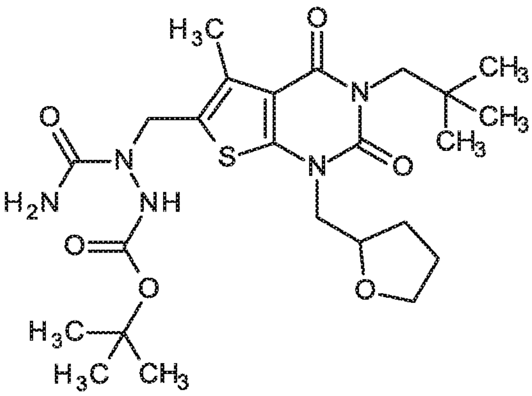

Analog zu dem unter Bsp. 478A beschriebenen Verfahren wurden aus 402 mg (0.769 mmol, 90% Reinheit) der Verbindung aus Bsp. 470A und 316 µl (2.31 mmol) Trimethylsilylisocyanat 534 mg (100% d. Th., 76% Reinheit) der Titelverbindung erhalten, welche ohne weitere Aufreinigung für Folgereaktionen verwendet wurden.
LC/MS (Methode 17, ESIneg): $R_t$ = 1.90 min, m/z = 522.24 [M-H]$^-$.

**Beispiel 480A**

*tert.*-Butyl-2-carbamoyl-2-{[5-methyl-2,4-dioxo-3-(2,2,2-trifluorethyl)-1-(3,3,3-trifluorpropyl)-1,2,3,4-tetrahydrothieno[2,3-d]pyrimidin-6-yl]methyl}hydrazincarboxylat

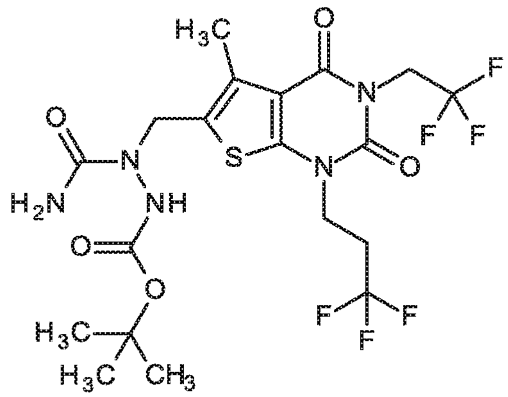

Eine Lösung von 400 mg (0.793 mmol) der Verbindung aus Bsp. 421A in 10 ml Isopropanol wurde mit 213 µl (1.59 mmol) Trimethylsilylisocyanat versetzt und ca. 18 h bei 50°C gerührt. Danach wurde das Reaktionsgemisch zur Trockene eingeengt. Der Rückstand wurde in Ethylacetat aufgenommen und nacheinander mit gesättigter wässriger Natriumhydrogencarbonat-Lösung und gesättigter wässriger Natriumchlorid-Lösung gewaschen. Nach Trocknen über wasserfreiem Magnesiumsulfat wurde filtriert und eingeengt. Der verbliebene Rückstand wurde mittels MPLC gereinigt (Biotage Isolera, Kartusche mit 25 g Kieselgel, Cyclohexan/Ethylacetat 1:1 → Dichlormethan/ Methanol 20:1). Die hinreichend sauberen Produktfraktionen wurden vereinigt, eingeengt und im Hochvakuum getrocknet. Es wurden 378 mg (82% d. Th., 95% Reinheit) der Titelverbindung erhalten, die ohne weitere Reinigung für Folgereaktionen verwendet wurden.
$^1$H-NMR (400 MHz, DMSO-$d_6$, δ/ppm): 8.94 (br. s, 1H), 6.22 (br. s, 2H), 5.22-4.33 (m, 2H), 4.72 (q, 2H), 4.17 (t, 2H), 2.86-2.68 (m, 2H), 2.35 (s, 3H), 1.38 (br. s, 9H).
LC/MS (Methode 17, ESIneg): $R_t$ = 1.78 min, m/z = 546.13 $[M-H]^-$.

**Beispiel 481A**

*tert.*-Butyl-2-carbamoyl-2-{[1-(2-methoxyethyl)-5-methyl-2,4-dioxo-3-(2,2,2-trifluorethyl)-1,2,3,4-tetrahydrothieno[2,3-d]pyrimidin-6-yl]methyl}hydrazincarboxylat

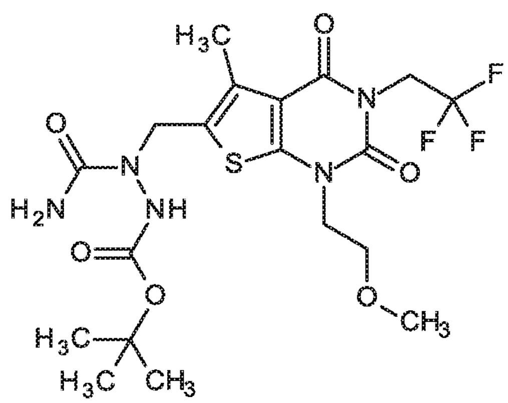

Eine Lösung von 210 mg (0.450 mmol) der Verbindung aus Bsp. 422A in 8 ml Isopropanol wurde mit 185 µl (1.35 mmol) Trimethylsilylisocyanat versetzt und ca. 18 h bei 50°C gerührt. Danach wurden weitere 93 µl (0.676 mmol) Trimethylsilylisocyanat hinzugefügt und das Rühren bei 50°C noch 3 h fortgesetzt. Anschließend wurde das Reaktions-

gemisch über Nacht bei RT stehen gelassen. Dabei fiel ein Teil des Produktes aus, der abgesaugt und getrocknet wurde. Die Mutterlauge wurde zur Trockene eingeengt und der Rückstand mittels präparativer HPLC gereinigt (Methode 8). Die Produktfraktionen wurden vereinigt und eingeengt, dann mit dem zuvor abgesaugten Niederschlag vereinigt und im Hochvakuum getrocknet. Es wurden 180 mg (73% d. Th., 94% Reinheit) der Titelverbindung erhalten, die ohne weitere Reinigung für Folgereaktionen verwendet wurden.
$^1$H-NMR (500 MHz, DMSO-$d_6$, δ/ppm): 8.95 (br. s, 1H), 6.20 (br. s, 2H), 5.14-4.24 (m, 2H), 4.71 (q, 2H), 4.07 (t, 2H), 3.65 (t, 2H), 3.24 (s, 3H), 2.33 (s, 3H), 1.38 (br. s, 9H).
LC/MS (Methode 17, ESIneg): $R_t$ = 1.57 min, m/z = 508.15 [M-H]$^-$.

## Beispiel 482A

*tert.*-Butyl-2-carbamoyl-2-{[5-methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl}hydrazincarboxylat *(Racemat)*

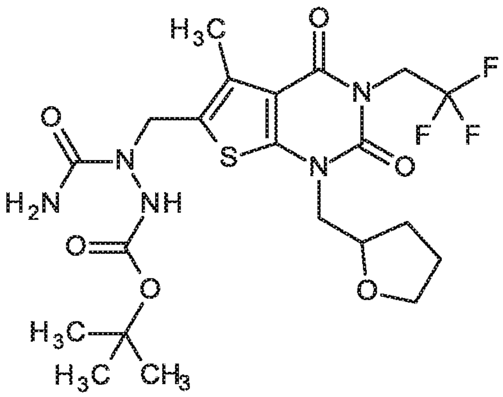

Eine Lösung von 1.12 g (2.27 mmol) der Verbindung aus Bsp. 423A in 50 ml Isopropanol wurde mit 610 μl (4.55 mmol) Trimethylsilylisocyanat versetzt und ca. 18 h bei RT gerührt. Dann wurden weitere 305 μl (2.27 mmol) Trimethylsilylisocyanat hinzugefügt und das Rühren bei RT 2 Tage lang fortgesetzt. Danach wurde das Reaktionsgemisch zur Trockene eingeengt. Der verbliebene Rückstand wurde mittels MPLC gereinigt (Biotage Isolera, Kartusche mit 100 g Kieselgel, Cyclohexan/Ethylacetat 1:1 → Dichlormethan/Methanol 20:1). Die Produktfraktionen wurden vereinigt, eingedampft und im Hochvakuum getrocknet. Es wurden 810 mg (66% d. Th.) der Titelverbindung erhalten.
$^1$H-NMR (400 MHz, DMSO-$d_6$, δ/ppm): 8.95 (br. s, 1H), 6.20 (s, 2H), 5.23-4.31 (breit, 2H), 4.72 (q, 2H), 4.30-4.19 (m, 1H), 4.03 (dd, 1H), 3.87-3.71 (m, 2H), 3.67-3.56 (m, 1H), 2.33 (s, 3H), 2.03-1.75 (m, 3H), 1.74-1.60 (m, 1H), 1.49-1.15 (br. s, 9H).
LC/MS (Methode 17, ESIneg): $R_t$ = 1.66 min, m/z = 534.16 [M-H]$^{--}$.

## Beispiel 483A

*tert.*-Butyl-2-carbamoyl-2-{[5-methyl-2,4-dioxo-3-(1,1,1-trifluorpropan-2-yl)-1-(3,3,3-trifluor-propyl)-1,2,3,4-tetrahydrothieno[2,3-d]pyrimidin-6-yl]methyl}hydrazincarboxylat (*Racemat*)

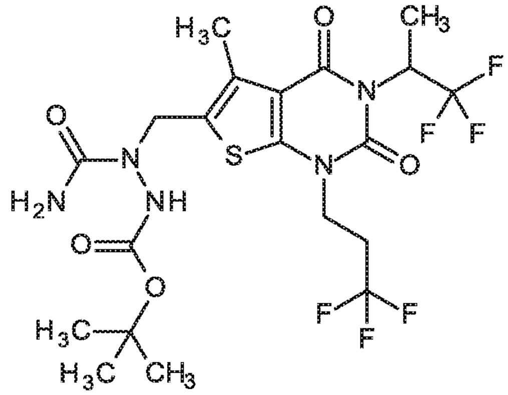

Eine Lösung von 440 mg (0.849 mmol) der Verbindung aus Bsp. 471A in 20 ml Isopropanol wurde mit 228 µl (1.697 mmol) Trimethylsilylisocyanat versetzt und ca. 18 h bei RT gerührt. Danach wurde das Reaktionsgemisch zur Trockene eingeengt. Der verbliebene Rückstand wurde mittels MPLC gereinigt (Biotage Isolera, Kartusche mit 100 g Kieselgel, Ethylacetat). Die hinreichend sauberen Produktfraktionen wurden vereinigt, eingeengt und im Hochvakuum getrocknet. Es wurden 397 mg (79% d. Th., 96% Reinheit) der Titelverbindung erhalten.
$^1$H-NMR (400 MHz, DMSO-$d_6$, δ/ppm): 8.92 (br. s, 1H), 6.22 (br. s, 2H), 5.83-5.48 (m, 1H), 4.60 (br. s, 2H), 4.14 (t, 2H), 4.03 (q, 1H), 2.85-2.69 (m, 2H), 2.39-2.27 (m, 3H), 1.71-1.59 (m, 3H), 1.38 (br. s, 9H).
LC/MS (Methode 17, ESIneg): $R_t$ = 1.90 min, m/z = 560.14 [M-H]$^-$.

**Beispiel 484A**

*tert.*-Butyl-2-carbamoyl-2-{[1-(2-methoxyethyl)-5-methyl-2,4-dioxo-3-(1,1,1-trifluorpropen-2-yl)-1,2,3,4-tetrahydrothieno[2,3-d]pyrimidin-6-yl]methyl}hydrazincarboxylat *(Racemat)*

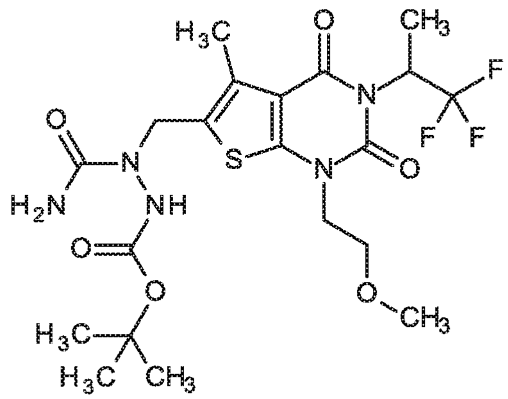

Analog zu dem unter Bsp. 483A beschriebenen Verfahren wurden aus 450 mg (0.937 mmol) der Verbindung aus Bsp. 472A und 251 µl (1.87 mmol) Trimethylsilylisocyanat 413 mg (80% d. Th., 96% Reinheit) der Titelverbindung erhalten.
$^1$H-NMR (400 MHz, DMSO-$d_6$, δ/ppm): 8.92 (br. s, 1H), 6.20 (br. s, 2H), 5.86-5.47 (m, 1H), 5.12-4.20 (br. m, 2H), 4.14-3.95 (m, 2H), 3.67-3.60 (m, 2H), 3.31 (s, 3H), 2.33 (br. s, 3H), 1.73-1.59 (m, 3H), 1.38 (br. s, 9H).
LC/MS (Methode 17, ESIneg): $R_t$ = 1.72 min, m/z = 522.16 [M-H]$^{--}$.

**Beispiel 485A**

*tert.*-Butyl-2-carbamoyl-2-{[3-(2-methoxyethyl)-5-methyl-2,4-dioxo-1-(3,3,3-trifluorpropyl)-1,2,3,4-tetrahydrothieno[2,3-d]pyrimidin-6-yl]methyl}hydrazincarboxylat

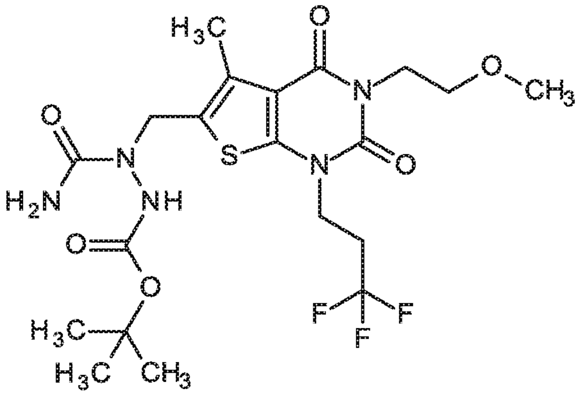

Eine Lösung von 200 mg (0.404 mmol, 97% Reinheit) der Verbindung aus Bsp. 424A in 5 ml Isopropanol wurde mit 108 µl (0.807 mmol) Trimethylsilylisocyanat versetzt und 2 Tage bei RT gerührt. Danach wurde das Reaktionsgemisch zur Trockene eingeengt. Der verbliebene Rückstand wurde mittels MPLC gereinigt (Biotage Isolera, Kartusche mit 10 g Kieselgel, Cyclohexan/Ethylacetat 1:1 → Dichlormethan/Methanol 20:1). Die hinreichend sauberen Produktfraktionen wurden vereinigt, eingeengt und im Hochvakuum getrocknet. Es wurden 187 mg (85% d. Th., 97% Reinheit) der Titelverbindung erhalten.
$^1$H-NMR (400 MHz, DMSO-$d_6$, δ/ppm): 8.93 (br. s, 1H), 6.21 (s, 2H), 4.59 (br. s, 2H), 4.13 (t, 2H), 4.07 (t, 2H), 3.49 (t, 2H), 3.23 (s, 3H), 2.83-2.68 (m, 2H), 2.34 (s, 3H), 1.39 (br. s, 9H).
LC/MS (Methode 17, ESIneg): $R_t$ = 1.56 min, m/z = 522.16 $[M-H]^-$.

**Beispiel 486A**

tert.-Butyl-2-carbamoyl-2-{[3-(2-methoxypropyl)-5-methyl-2,4-dioxo-1-(3,3,3-trifluorpropyl)-1,2,3,4-tetrahydrothieno[2,3-d]pyrimidin-6-yl]methyl}hydrazincarboxylat *(Racemat)*

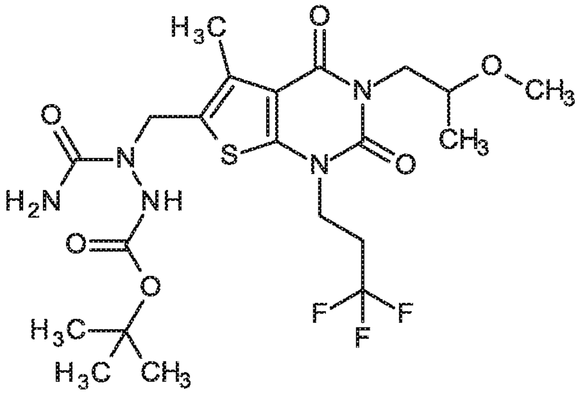

Eine Lösung von 400 mg (785 mmol, 97% Reinheit) der Verbindung aus Bsp. 473A in 10 ml Isopropanol wurde mit 210 µl (1.57 mmol) Trimethylsilylisocyanat versetzt und 2 Tage bei RT gerührt. Danach wurde das Reaktionsgemisch zur Trockene eingeengt. Der verbliebene Rückstand wurde mittels MPLC gereinigt (Biotage Isolera, Kartusche mit 100 g Kieselgel, Dichlormethan/ Methanol 98:2 → 80:20). Die Produktfraktionen wurden vereinigt, eingeengt und im Hochvakuum getrocknet. Es wurden 387 mg (91% d. Th.) der Titelverbindung erhalten.
$^1$H-NMR (400 MHz, DMSO-$d_6$, δ/ppm): 8.94 (br. s, 1H), 6.21 (br. s, 2H), 5.12-4.23 (m, 2H), 4.22-4.10 (m, 2H), 4.06 (dd, 1H), 3.76 (dd, 1H), 3.63 (sext, 1H), 3.20 (s, 3H), 2.83-2.68 (m, 2H), 2.35 (s, 3H), 1.38 (br. s, 9H), 1.04 (d, 3H).
LC/MS (Methode 17, ESIpos): $R_t$ = 1.63 min, m/z = 538.19 $[M+H]^+$.

**Beispiel 487A**

2-Chlor-*N*-{[3-ethyl-5-methyl-2,4-dioxo-1-(3,3,3-trifluorpropyl)-1,2,3,4-tetrahydrothieno[2,3-d]-pyrimidin-6-yl]methyl}acetamid

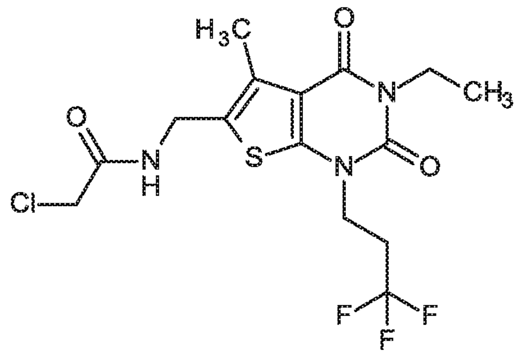

Eine Lösung von 588 mg (1.75 mmol) der Verbindung aus Bsp. 460A und 733 μl (5.26 mmol) Triethylamin in 25 ml Dichlormethan wurde bei 0°C tropfenweise mit 209 μl (2.63 mmol) Chloracetylchlorid versetzt. Nach 10 min wurde das Kältebad entfernt und das Rühren bei RT fortgesetzt. Nach insgesamt 2 h Reaktionszeit wurde das Reaktionsgemisch direkt mittels MPLC (Biotage Isolera, Kartusche mit 50 g Kieselgel, Cyclohexan/Ethylacetat 90:10 → 0:100) in seine Komponenten aufgetrennt. Die Produktfraktionen wurden vereinigt, eingeengt und im Hochvakuum getrocknet. Es wurden 563 mg (77% d. Th.) der Titelverbindung erhalten.
$^1$H-NMR (400 MHz, DMSO-$d_6$, δ/ppm): 8.85 (t, 1H), 4.39 (d, 2H), 4.10 (t, 2H), 4.10 (s, 2H), 3.90 (q, 2H), 2.83-2.68 (m, 2H), 2.41 (s, 3H), 1.11 (t, 3H).
LC/MS (Methode 1, ESIpos): $R_t$ = 0.88 min, m/z = 412 $[M+H]^+$.

**Beispiel 488A**

*N*-([3-Ethyl-5-methyl-2,4-dioxo-1-(3,3,3-trifluorpropyl)-1,2,3,4-tetrahydrothieno[2,3-d]pyrimidin-6-yl]methyl}glycinamid

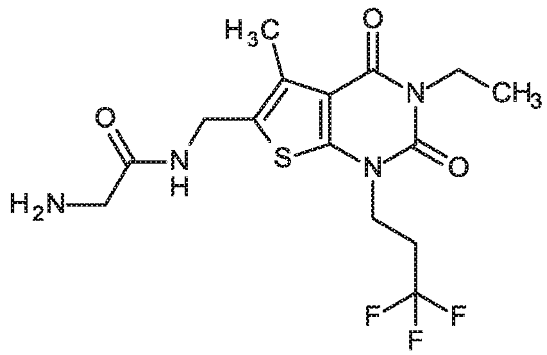

562 mg (1.36 mmol) der Verbindung aus Bsp. 487A wurden in 20 ml DMF gelöst und mit 23 mg (0.136 mmol) Kaliumiodid und 9.5 ml (68.2 mmol) konzentriertem Ammoniakwasser versetzt. Das Reaktionsgemisch wurde 7 h bei 50°C gerührt und anschließend 18 h bei RT stehen gelassen. Danach wurde es am Rotationsverdampfer zur Trockene eingeengt und der Rückstand mit 20 ml Wasser versetzt. Es wurde vom Ungelösten abgesaugt, und der Feststoff wurde verworfen. Das Filtrat wurde etwas aufkonzentriert, woraufhin das Produkt ausfiel. Der Feststoff wurde abgesaugt und mittels HPLC gereinigt (Methode 8). Es wurden 307 mg (54% Ausbeute, 95% Reinheit) der Titelverbindung isoliert.
$^1$H-NMR (400 MHz, DMSO-$d_6$, δ/ppm): 8.97 (t, 1H), 8.06 (br. s, 2H), 4.44 (d, 2H), 4.09 (t, 2H), 3.91 (q, 2H), 3.56 (s, 2H), 2.84-2.69 (m, 2H), 2.43 (s, 3H), 1.12 (t, 3H).
LC/MS (Methode 17, ESIneg): Rt = 0.91 min, m/z = 437.11 $[M-H+HCO_2H]^-$.

**Beispiel 489A**

*N*2-(Chloracetyl)-*N*-{[3-ethyl-5-methyl-2,4-dioxo-1-(3,3,3-trifluorpropyl)-1,2,3,4-tetrahydrothieno-[2,3-d]pyrimidin-6-yl]methyl}glycinamid

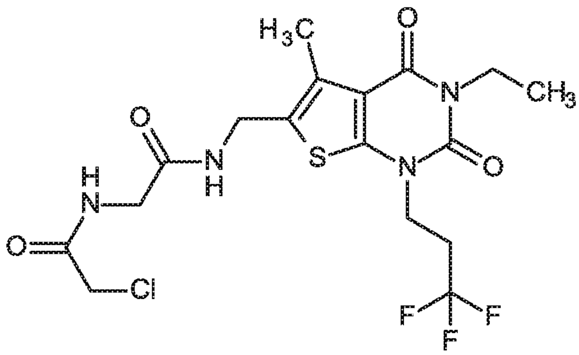

Eine Lösung von 305 mg (0.622 mmol, 80% Reinheit) der Verbindung aus Bsp. 488A und 260 µl (1.87 mmol) Triethylamin in 10 ml Dichlormethan wurde bei 0°C tropfenweise mit 74 µl (0.933 mmol) Chloracetylchlorid versetzt. Nach 10 min wurde das Kältebad entfernt und das Rühren bei RT fortgesetzt. Nach insgesamt 2 h Reaktionszeit wurde das Reaktionsgemisch mit Wasser versetzt und anschließend mit Ethylacetat extrahiert. Der organische Extrakt wurde mit gesättigter wässriger Natriumchlorid-Lösung gewaschen, über wasserfreiem Magnesiumsulfat getrocknet, filtriert und eingeengt. Nach Trocknen im Hochvakuum wurden 295 mg (99% d. Th., 98% Reinheit) der Titelverbindung erhalten.
$^1$H-NMR (400 MHz, DMSO-$d_6$, δ/ppm): 8.56 (t, 1H), 8.45 (t, 1H), 4.36 (d, 2H), 4.13 (s, 2H), 4.09 (t, 2H), 3.90 (q, 2H), 3.74 (d, 2H), 2.83-2.68 (m, 2H), 2.41 (s, 3H), 1.11 (t, 3H).
LC/MS (Methode 17, ESIneg): $R_t$ = 1.45 min, m/z = 467.08 $[M-H]^-$.

**Beispiel 490A**

Diethyl-5-[(isopropylcarbamoyl)amino]-3-methylthiophen-2,4-dicarboxylat

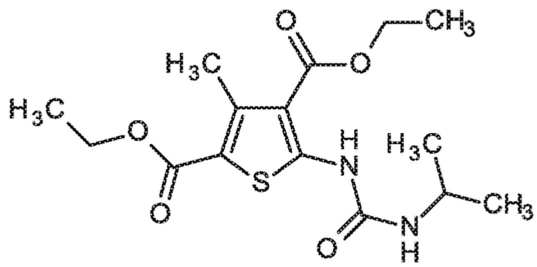

3.0 g (11.7 mmol) Diethyl-5-amino-3-methylthiophen-2,4-dicarboxylat wurden in 50 ml Dichlormethan gelöst und mit 3.78 g (23.3 mmol) CDI und 3.3 ml (23.3 mmol) Triethylamin versetzt. Das Reaktionsgemisch wurde 3 Tage bei RT gerührt, bevor 4.0 ml (46.6 mmol) Isopropylamin hinzugefügt wurden. Nach einer weiteren Stunde Rühren bei RT wurde das Gemisch mit 100 ml Wasser versetzt und dann mit Ethylacetat extrahiert. Der organische Extrakt wurde eingedampft und der Rückstand wieder in 50 ml Dichlormethan aufgenommen. Dabei blieb ein Teil ungelöst, der abgesaugt und im Hochvakuum getrocknet wurde. Dies ergab eine erste, verunreinigte Fraktion der Titelverbindung (3.59 g, 80% d. Th., 89% Reinheit, Rest: *N,N'*-Diisopropylharnstoff), die als solche für Folgereaktionen eingesetzt wurde. Der in Dichlormethan gelöste Teil des Rohprodukts wurde mittels MPLC gereinigt (Biotage-Kartusche mit 100 g Kieselgel, Cyclohexan/Ethylacetat 2:1). Nach Eindampfen der Produktfraktionen und Trocknen des Rückstands im Hochvakuum wurde so eine zweite, reine Fraktion der Titelverbindung erhalten (0.46 g, 11% d. Th.). Insgesamt wurden somit 4.05 g (92% d. Th., 90% Reinheit) der Titelverbindung erhalten.
$^1$H-NMR (400 MHz, DMSO-$d_6$, δ/ppm): 10.51 (s, 1H), 8.02 (br. d, 1H), 4.32 (q, 2H), 4.22 (q, 2H), 3.75 (sept, 1H), 2.65

(s, 3H), 1.33 (t, 3H), 1.27 (t, 3H), 1.11 (d, 6H).
LC/MS (Methode 17, ESIpos): $R_t$ = 2.20 min, m/z = 343.13 $[M+H]^+$.

**Beispiel 491A**

Diethyl-3-methyl-5-{[(2,2,2-trifluorethyl)carbamoyl]amino}thiophen-2,4-dicarboxylat

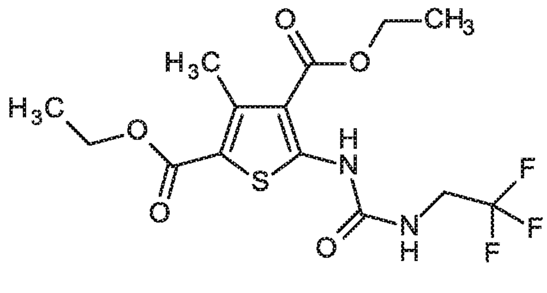

3.0 g (11.7 mmol) Diethyl-5-amino-3-methylthiophen-2,4-dicarboxylat wurden in 50 ml Dichlormethan gelöst und mit 3.78 g (23.3 mmol) CDI und 3.3 ml (23.3 mmol) Triethylamin versetzt. Das Reaktionsgemisch wurde 2 Tage bei RT gerührt, bevor 3.7 ml (46.6 mmol) 2,2,2-Trifluorethylamin hinzugefügt wurden. Nach einem weiteren Tag Rühren bei RT wurde das Gemisch mit 100 ml Wasser versetzt und dann mit Dichlormethan extrahiert. Der organische Extrakt wurde mit gesättigter Natriumchlorid-Lösung gewaschen, über wasserfreiem Magnesiumsulfat getrocknet, filtriert und eingeengt. Der erhaltene Rückstand wurde mittels MPLC gereinigt (Biotage Isolera, Kartusche mit 340 g Kieselgel, Cyclohexan/Ethylacetat 2:1). Nach Eindampfen der Produktfraktionen und Trocknen des Rückstands im Hochvakuum wurden 3.93 g (82% d. Th., 95% Reinheit) der Titelverbindung erhalten.
$^1$H-NMR (600 MHz, DMSO-$d_6$, δ/ppm): 10.75 (s, 1H), 8.81 (t, 1H), 4.33 (q, 2H), 4.23 (q, 2H), 4.07-3.95 (m, 2H), 2.66 (s, 3H), 1.34 (t, 3H), 1.28 (t, 3H).
LC/MS (Methode 17, ESIpos): $R_t$ = 2.16 min, m/z = 383.09 $[M+H]^+$.

**Beispiel 492A**

Diethyl-3-methyl-5-({[(2*R*)-1,1,1-trifluorpropan-2-yl]carbamoyl]amino)thiophen-2,4-dicarboxylat

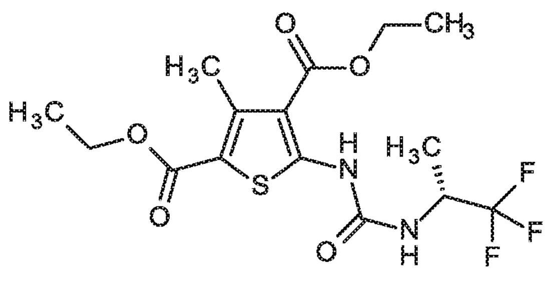

5.0 g (19.4 mmol) Diethyl-5-amino-3-methylthiophen-2,4-dicarboxylat wurden in 83 ml Dichlormethan gelöst und mit 6.3 g (38.9 mmol) CDI und 13.5 ml (97.2 mmol) Triethylamin versetzt. Das Reaktionsgemisch wurde 2 Tage bei RT gerührt, bevor 5.81 g (38.9 mmol) (2*R*)-1,1,1-Trifluor-propan-2-amin-Hydrochlorid hinzugefügt wurden. Nach weiteren 6 Tagen Rühren bei RT wurde das Gemisch mit 100 ml Wasser versetzt und dann mit Dichlormethan extrahiert. Der organische Extrakt wurde mit gesättigter Natriumchlorid-Lösung gewaschen, über wasserfreiem Magnesiumsulfat getrocknet, filtriert und fast bis zur Trockene eingeengt. Der ausgefallene Feststoff wurde abgesaugt, mit wenig Dichlormethan nachgewaschen und im Hochvakuum getrocknet. Dies ergab eine erste Fraktion des Produkts (2.43 g). Das Filtrat und die Waschflüssigkeit wurden vereinigt und weiter aufkonzentriert, bis noch mehr Produkt ausfiel. Dies wurde erneut abgesaugt und im Hochvakuum getrocknet. Eine zweite Fraktion des Produkts wurde so erhalten (1.68 g). Das Filtrat wurde nun zur Trockene eingedampft. Der verbliebene Rückstand wurde mittels MPLC gereinigt (Isolera, 340 g Kieselgel, Cyclohexan/Ethylacetat 2:1). Die Produktfraktionen wurden vereinigt, eingeengt und im Hochvakuum getrocknet. Dies ergab eine dritte Fraktion des Produkts (5.25 g). Insgesamt wurden so 9.36 g (85% d. Th., 70% Reinheit) der Titelverbindung erhalten, die ohne weitere Aufreinigung für Folgereaktionen verwendet wurden.
$^1$H-NMR (400 MHz, DMSO-$d_6$, δ/ppm): 10.72 (s, 1H), 8.73 (d, 1H), 4.57-4.42 (m, 1H), 4.33 (q, 2H), 4.23 (q, 2H), 2.67 (s, 3H), 1.34 (t, 3H), 1.29 (d, 3H), 1.27 (t, 3H).
LC/MS (Methode 1, ESIpos): $R_t$ = 1.18 min, m/z = 397 $[M+H]^+$.

**Beispiel 493A**

Diethyl-3-methyl-5-({[(2*S*)-1,1,1-trifluorpropan-2-yl]carbamoyl)amino)thiophen-2,4-dicarboxylat

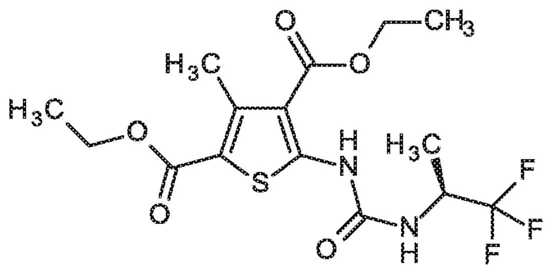

4.0 g (15.5 mmol) Diethyl-5-amino-3-methylthiophen-2,4-dicarboxylat wurden in 67 ml Dichlormethan gelöst und mit 5.04 g (31.1 mmol) CDI und 10.8 ml (77.7 mmol) Triethylamin versetzt. Das Reaktionsgemisch wurde 2 Tage bei RT gerührt, bevor 4.65 g (31.1 mmol) (2*S*)-1,1,1-Trifluorpropan-2-amin-Hydrochlorid hinzugefügt wurden. Nach weiteren 2 Tagen Rühren bei RT wurde das Gemisch mit 100 ml Wasser versetzt und dann mit Dichlormethan extrahiert. Der organische Extrakt wurde mit gesättigter Natriumchlorid-Lösung gewaschen, über wasserfreiem Magnesiumsulfat getrocknet, filtriert und zur Trockene eingeengt. Der verbliebene Rückstand wurde zunächst mittels MPLC vorgereinigt (Isolera, 340 g Kieselgel, Cyclohexan/Ethylacetat 2:1). Die Produktfraktionen wurden vereinigt, eingedampft und anschließend mittels präparativer HPLC (Methode 8) weiter aufgereinigt. Die Produktfraktionen wurden wiederum vereinigt und eingeengt. Der verbliebene Rückstand wurde dann in Ethylacetat aufgenommen und nacheinander mit Wasser, 1 M Salzsäure und gesättigter Natriumchlorid-Lösung gewaschen. Nach Trocknen über wasserfreiem Magnesiumsulfat, Filtration und Einengen wurden 6.28 g (81% d. Th., 80% Reinheit) der Titelverbindung erhalten, die ohne weitere Aufreinigung für Folgereaktionen verwendet wurden.
$^1$H-NMR (400 MHz, DMSO-$d_6$, δ/ppm): 10.72 (s, 1H), 8.73 (d, 1H), 4.57-4.42 (m, 1H), 4.33 (q, 2H), 4.23 (q, 2H), 2.67 (s, 3H), 1.34 (t, 3H), 1.29 (d, 3H), 1.27 (t, 3H).
LC/MS (Methode 1, ESIpos): $R_t$ = 1.18 min, m/z = 397 $[M+H]^+$.

**Beispiel 494A**

Ethyl-3-isopropyl-5-methyl-2,4-dioxo-1,2,3,4-tetrahydrothieno[2,3-d]pyrimidin-6-carboxylat

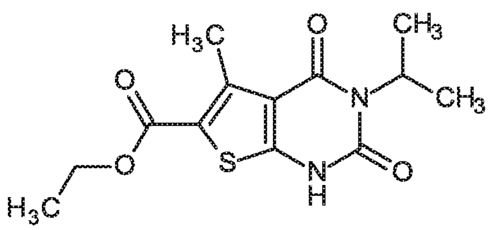

4.05 g (10.6 mmol, 90% Reinheit) der Verbindung aus Bsp. 490A wurden in 85 ml Ethanol gelöst und mit 6.6 ml (17.7 mmol) einer 21%-igen Lösung von Natriumethanolat in Ethanol versetzt. Das Reaktionsgemisch wurde zunächst 18 h bei RT, dann 6 h bei 50°C und zuletzt wieder 18 h bei RT gerührt. Danach wurde es auf ca. die Hälfte des ursprünglichen Volumens eingeengt. Der dabei ausgefallene Niederschlag wurde abgesaugt und mit Diisopropylether, dem ein wenig Ethylacetat beigemischt war, bei RT verrührt. Nach erneutem Absaugen und Trocknen im Hochvakuum

wurden 2.67 g (85% d. Th.) der Titelverbindung erhalten.
$^1$H-NMR (400 MHz, DMSO-$d_6$, δ/ppm): 12.29 (s, 1H), 5.08 (sept, 1H), 4.26 (q, 2H), 2.74 (s, 3H), 1.40 (d, 6H), 1.28 (t, 3H).
LC/MS (Methode 17, ESIpos): $R_t$ = 1.80 min, m/z = 297.09 $[M+H]^+$.

**Beispiel 495A**

Ethyl-5-methyl-2,4-dioxo-3-(2,2,2-trifluorethyl)-1,2,3,4-tetrahydrothieno[2,3-d]pyrimidin-6-carboxylat

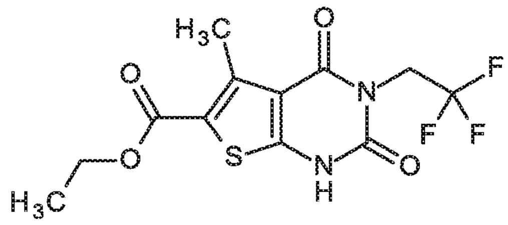

3.93 g (9.76 mmol, 95% Reinheit) der Verbindung aus Bsp. 491A wurden in 95 ml Ethanol gelöst und mit 7.3 ml (19.5 mmol) einer 21%-igen Lösung von Natriumethanolat in Ethanol versetzt. Das Reaktionsgemisch wurde 2 h bei 50°C gerührt. Danach wurde es auf ca. die Hälfte des ursprünglichen Volumens eingeengt. Dann wurden 22.5 ml (22.5 mmol) 1 M Salzsäure hinzugefügt. Der dabei ausgefallene Niederschlag wurde abgesaugt, mit Wasser neutral gewaschen und getrocknet. Der Feststoff wurde schließlich mit Diisopropylether, dem ein wenig Ethylacetat beigemischt war, bei RT verrührt. Nach erneutem Absaugen und Trocknen im Hochvakuum wurden 3.20 g (97% d. Th.) der Titelverbindung erhalten.
$^1$H-NMR (400 MHz, DMSO-$d_6$, δ/ppm): 12.76 (br. s, 1H), 4.65 (q, 2H), 4.27 (q, 2H), 2.74 (s, 3H), 1.29 (t, 3H).
LC/MS (Methode 17, ESIpos): $R_t$ = 1.77 min, m/z = 337.05 $[M+H]^+$.

**Beispiel 496A**

Ethyl-5-methyl-2,4-dioxo-3-[(2*R*)-1,1,1-trifluorpropan-2-yl]-1,2,3,4-tetrahydrothieno[2,3-d]-pyrimidin-6-carboxylat

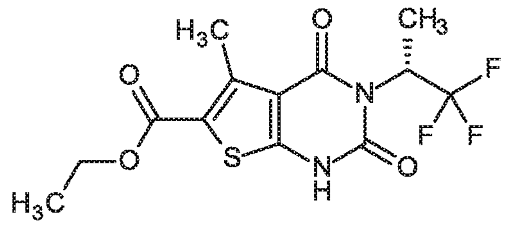

9.35 g (16.5 mmol, 70% Reinheit) der Verbindung aus Bsp. 492A wurden in 120 ml Ethanol gelöst und mit 12.3 ml (33.0 mmol) einer 21%-igen Lösung von Natriumethanolat in Ethanol versetzt. Das Reaktionsgemisch wurde ca. 16 h bei 50°C gerührt. Danach wurde es mit 38 ml (38.0 mmol) 1 M Salzsäure und gesättigter Natriumchlorid-Lösung versetzt und anschließend mit Diethylether extrahiert. Der organische Extrakt wurde eingedampft, und der verbliebene Rückstand wurde mittels MPLC vorgereinigt (Isolera, 100 g Kieselgel, Cyclohexan/Ethylacetat 100:0 → 40:60). Die Produktfraktionen wurden vereinigt, eingeengt und mittels präparativer HPLC weiter aufgereinigt [Säule: XBridge C18, 5 μm, 100 mm x 30 mm; Eluent A: Wasser; Eluent B: Acetonitril; Eluent C: Ammoniakwasser (1% $NH_3$); Gradient: 0.0-1.0 min A:B:C 75:20:5, 1.0-6.0 min A:B:C 75:20:5 → 25:70:5, 6.0-6.2 min A:B:C 25:70:5 → 75:20:5, 6.2-7.2 min A:B:C 75:20:5; Fluss: 75 ml/min; Temperatur: 40°C; Detektion: 210 nm]. Nach Einengen der Produktfraktionen und Trocknen im Hochvakuum wurden 2.91 g (50% d. Th.) der Titelverbindung erhalten (>99% ee, chirale analytische HPLC).
$^1$H-NMR (400 MHz, DMSO-$d_6$, δ/ppm): 12.65 (br. s, 1H), 5.78-5.62 und 5.57-5.44 (2 m, zus. 1H), 4.27 (q, 2H), 2.74 und 2.72 (2 s, zus. 3H), 1.67 und 1.64 (2 d, zus. 3H), 1.29 (t, 3H).
LC/MS (Methode 17, ESIpos): $R_t$ = 1.90 min, m/z = 351.06 $[M+H]^+$.
Chirale analytische HPLC [Säule: Daicel Chiralcel IC-3, 3 μm, 50 mm x 4.6 mm; Laufmittel: Isohexan/Isopropanol

4:1; Fluss: 1 ml/min; Temperatur: 25°C; Detektion: 220 nm]: $R_t$ = 1.38 min.
Spezifische optische Drehung: $[\alpha]_D^{20}$ = +15.1°·ml·dm$^{-1}$·g$^{-1}$ (Methanol).

**Beispiel 497A**

Ethyl-5-methyl-2,4-dioxo-3-[(2*S*)-1,1,1-trifluorpropan-2-yl]-1,2,3,4-tetrahydrothieno[2,3-d]-pyrimidin-6-carboxylat

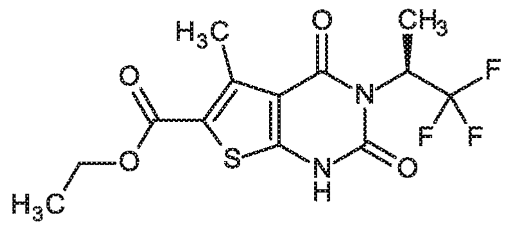

6.0 g (12.1 mmol, 80% Reinheit) der Verbindung aus Bsp. 493A wurden in 100 ml Ethanol gelöst und mit 9 ml (24.2 mmol) einer 21%-igen Lösung von Natriumethanolat in Ethanol versetzt. Das Reaktionsgemisch wurde ca. 16 h bei 50°C gerührt. Danach wurde es mit 28 ml (28.0 mmol) 1 M Salzsäure versetzt. Der dabei ausgefallene Niederschlag wurde abgesaugt, während das Filtrat mit Ethylacetat extrahiert wurde. Der eingedampfte organische Extrakt wurde mit dem zuvor abgetrennten Niederschlag vereinigt und mittels präparativer HPLC gereinigt (Methode 8). Nach Einengen der Produktfraktionen und Trocknen im Hochvakuum wurden 2.75 g (64% d. Th.) der Titelverbindung erhalten (>99% ee, chirale analytische HPLC).
$^1$H-NMR (600 MHz, DMSO-d$_6$, δ/ppm): 12.67 (br. s, 1H), 5.74-5.66 und 5.54-5.47 (2 m, zus. 1H), 4.27 (q, 2H), 2.74 und 2.72 (2 s, zus. 3H), 1.67 und 1.64 (2 d, zus. 3H), 1.29 (t, 3H).
LC/MS (Methode 17, ESIpos): $R_t$ = 1.92 min, m/z = 351.06 [M+H]$^+$.
Chirale analytische HPLC [Säule: Daicel Chiralcel IC-3, 3 μm, 50 mm x 4.6 mm; Laufmittel: Isohexan/Isopropanol 4:1; Fluss: 1 ml/min; Temperatur: 25°C; Detektion: 220 nm]: $R_t$ = 1.67 min.
Spezifische optische Drehung: $[\alpha]_D^{20}$ = -13.9°·ml·dm$^{-1}$·g$^{-1}$ (Methanol).

**Beispiel 498A**

Ethyl-3-isopropyl-1-(2-methoxyethyl)-5-methyl-2,4-dioxo-1,2,3,4-tetrahydrothieno[2,3-d]-pyrimidin-6-carboxylat

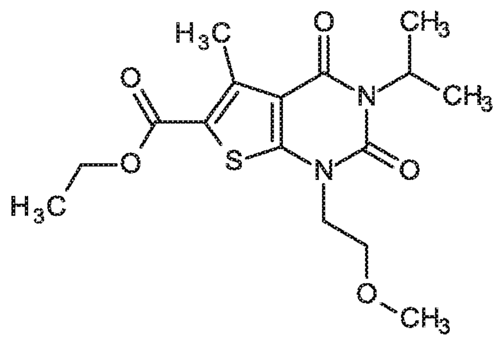

Eine Lösung von 900 mg (3.04 mmol) der Verbindung aus Bsp. 494A in 22 ml DMF wurde mit 1.05 g (7.59 mmol) Kaliumcarbonat versetzt und 15 min bei RT gerührt. Dann wurden 844 mg (6.07 mmol) (2-Bromethyl)-methylether hinzugefügt. Das Gemisch wurde zunächst 4 Tage bei RT und abschließend 2 h bei 60°C gerührt. Danach wurde das Reaktionsgemisch bei RT mit ca. 125 ml Wasser versetzt. Dabei fiel das Produkt aus. Das Gemisch wurde noch 30 min bei RT nachgerührt, bevor das Produkt dann abgesaugt wurde. Der erhaltene Rückstand wurde nochmals mit Wasser verrührt. Nach Abdekantieren der Wasserphase wurde das Produkt im Hochvakuum getrocknet. Es wurden 1.03 g (86% d. Th., 90% Reinheit) der Titelverbindung erhalten, die ohne weitere Aufreinigung für Folgereaktionen verwendet wurden.
$^1$H-NMR (400 MHz, DMSO-d$_6$, δ/ppm): 5.12 (sept, 1H), 4.28 (q, 2H), 4.05 (t, 2H), 3.64 (t, 2H), 3.25 (s, 3H), 2.76 (s, 3H),

1.41 (d, 6H), 1.29 (t, 3H).
LC/MS (Methode 17, ESIpos): $R_t$ = 2.17 min, m/z = 355.13 $[M+H]^+$.

**Beispiel 499A**

Ethyl-1-(2-methoxyethyl)-5-methyl-2,4-dioxo-3-(2,2,2-trifluorethyl)-1,2,3,4-tetrahydrothieno-[2,3-d]pyrimidin-6-carboxylat

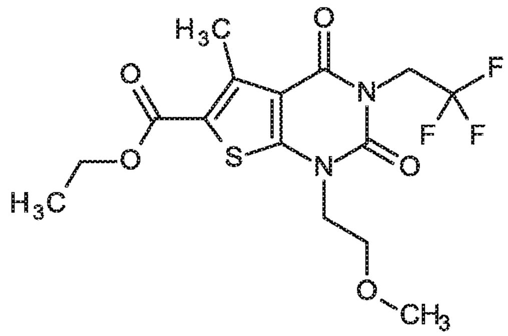

Eine Lösung von 1.60 g (4.76 mmol) der Verbindung aus Bsp. 495A in 35 ml DMF wurde mit 1.64 g (11.9 mmol) Kaliumcarbonat versetzt und 15 min bei RT gerührt. Dann wurden 1.32 g (9.52 mmol) (2-Bromethyl)-methylether hinzugefügt. Das Gemisch wurde zunächst 3 Tage bei RT und abschließend 24 h bei 60°C gerührt. Danach wurde das Reaktionsgemisch bei RT mit ca. 175 ml Wasser versetzt. Dabei fiel das Produkt aus. Das Gemisch wurde noch 30 min bei RT nachgerührt, bevor das Produkt dann abgesaugt und mit Wasser gewaschen wurde. Nach Trocknen im Hochvakuum wurden 1.93 g (94% d. Th., 92% Reinheit) der Titelverbindung erhalten, die ohne weitere Aufreinigung für Folgereaktionen verwendet wurden.
$^1$H-NMR (400 MHz, DMSO-$d_6$, δ/ppm): 4.70 (q, 2H), 4.30 (q, 2H), 4.12 (t, 2H), 3.66 (t, 2H), 3.24 (s, 3H), 2.77 (s, 3H), 1.30 (t, 3H).
LC/MS (Methode 17, ESIpos): $R_t$ = 2.07 min, m/z = 395.09 $[M+H]^+$.

**Beispiel 500A**

Ethyl-1-(2-methoxyethyl)-5-methyl-2,4-dioxo-3-[(2*R*)-1,1,1-trifluorpropan-2-yl]-1,2,3,4-tetrahydrothieno[2,3-d]pyrimidin-6-carboxylat

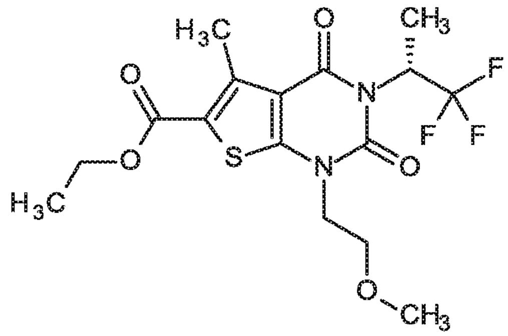

Eine Lösung von 1.0 g (2.85 mmol) der Verbindung aus Bsp. 496A in 15 ml DMF wurde mit 690 mg (5.0 mmol) Kaliumcarbonat versetzt und 15 min bei RT gerührt. Dann wurden 555 mg (4.0 mmol) (2-Bromethyl)-methylether hinzugefügt. Das Gemisch wurde ca. 18 h bei 60°C gerührt. Danach wurde das Reaktionsgemisch bei RT mit ca. 75 ml Wasser versetzt und mit Ethylacetat extrahiert. Der organische Extrakt wurde mit Natriumchlorid-Lösung gewaschen, über wasserfreiem Magnesiumsulfat getrocknet, filtriert und eingeengt. Der verbliebene Rückstand wurde mittels präparativer HPLC (Methode 8) gereinigt. Nach Eindampfen der Produktfraktionen und Trocknen im Hochvakuum wurden

690 mg (59% d. Th., 98% Reinheit) der Titelverbindung erhalten (>99% ee, chirale analytische HPLC).
$^1$H-NMR (400 MHz, DMSO-$d_6$, δ/ppm): 5.80-5.68 und 5.61-5.50 (2 m, zus. 1H), 4.29 (q, 2H), 4.13-4.06 (m, 2H), 3.68-3.62 (m, 2H), 3.25 (s, 3H), 2.77 (s, 3H), 1.69 und 1.64 (2 d, zus. 3H), 1.30 (t, 3H).
LC/MS (Methode 1, ESIpos): $R_t$ = 1.16 min, m/z = 409 $[M+H]^+$.

Chirale analytische HPLC [Säule: Daicel Chiralpak AZ-3, 3 μm, 50 mm x 4.6 mm; Laufmittel: Heptan/Ethanol 9:1; Fluss: 1 ml/min; Temperatur: 25°C; Detektion: 220 nm]: $R_t$ = 1.05 min.

Spezifische optische Drehung: $[\alpha]_D^{20}$ = +14.5°·ml·dm$^{-1}$·g$^{-1}$ (Methanol).

**Beispiel 501A**

Ethyl-1-(2-methoxyethyl)-5-methyl-2,4-dioxo-3-[(2*S*)-1,1,1-trifluorpropan-2-yl]-1,2,3,4-tetmhydro-thieno[2,3-d]pyrimidin-6-carboxylat

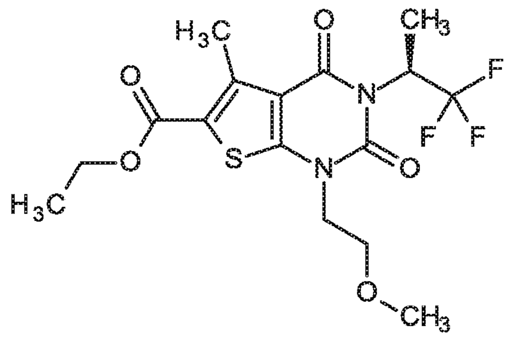

Eine Lösung von 500 mg (1.43 mmol) der Verbindung aus Bsp. 497A in 10 ml DMF wurde mit 493 mg (3.57 mmol) Kaliumcarbonat versetzt und 15 min bei RT gerührt. Dann wurden 397 mg (2.85 mmol) (2-Bromethyl)-methylether hinzugefügt. Das Gemisch wurde ca. 18 h bei 60°C gerührt. Danach wurde das Reaktionsgemisch bei RT mit ca. 50 ml Wasser versetzt und mit Ethylacetat extrahiert. Der organische Extrakt wurde mit Natriumchlorid-Lösung gewaschen, über wasserfreiem Magnesiumsulfat getrocknet, filtriert und eingeengt. Der verbliebene Rückstand wurde mittels präparativer HPLC (Methode 8) gereinigt. Nach Eindampfen der Produktfraktionen und Trocknen im Hochvakuum wurden 270 mg (46% d. Th.) der Titelverbindung erhalten (>99% ee, chirale analytische HPLC).
$^1$H-NMR (400 MHz, DMSO-$d_6$, δ/ppm): 5.80-5.68 und 5.60-5.49 (2 m, zus. 1H), 4.29 (q, 2H), 4.13-4.06 (m, 2H), 3.68-3.62 (m, 2H), 3.25 (s, 3H), 2.76 (s, 3H), 1.69 und 1.64 (2 d, zus. 3H), 1.30 (t, 3H).
LC/MS (Methode 17, ESIpos): $R_t$ = 2.23 min, m/z = 409.10 $[M+H]^+$.

Chirale analytische HPLC [Säule: Daicel Chiralpak AZ-3, 3 μm, 50 mm x 4.6 mm; Laufmittel: Heptan/Ethanol 9:1; Fluss: 1 ml/min; Temperatur: 25°C; Detektion: 220 nm]: $R_t$ = 1.10 min.

Spezifische optische Drehung: $[\alpha]_D^{20}$ = -13.6°·ml·dm$^{-1}$·g$^{-1}$ (Methanol).

**Beispiel 502A**

Ethyl-4-methyl-2-{[(1,1,1-trifluorpropan-2-yl)carbemoyl]amino)thiophen-3-carboxylat (*Enantiomer 1*)

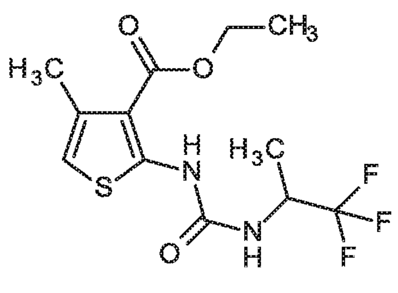

4.36 g (13.4 mmol) der racemischen Verbindung aus Bsp. 437A wurden in 135 ml Isopropanol gelöst und in

190 Portionen über präparative SFC-HPLC an chiraler Phase in die Enantiomere aufgetrennt [Säule: Daicel Chiralpak OX-H, 5 μm, 250 mm x 30 mm; Laufmittel: Kohlendioxid/Isopropanol 90:10; Fluss: 175 ml/min; Temperatur: 38°C; Detektion: 210 nm]. Nach Eindampfen der Produktfraktionen und Trocknen des Feststoffs im Hochvakuum wurden 2.10 g (96% d. Th.) von Enantiomer 1 erhalten (>99% ee, chirale analytische HPLC).
$^1$H-NMR (400 MHz, DMSO-$d_6$, δ/ppm): 10.45 (s, 1H), 8.50 (d, 1H), 6.50 (s, 1H), 4.58-4.39 (m, 1H), 4.29 (q, 2H), 2.28 (d, 3H), 1.32 (t, 3H), 1.28 (d, 3H).

Chirale analytische SFC-HPLC [Säule: Daicel Chiralcel QX, 3 μm, 50 mm x 5 mm; Laufmittel: Kohlendioxid/Isopropanol 95:5 → 50:50; Fluss: 3 ml/min; Temperatur: 40°C; Detektion: 210 nm]: $R_t$ = 0.80 min.

Spezifische optische Drehung: $[\alpha]_D^{20}$ = -8.29°·ml·dm$^{-1}$·g$^{-1}$ (Methanol).

**Beispiel 503A**

Ethyl-4-methyl-2-{[(1,1,1-trifluorpropan-2-yl)carbamoyl]amino}thiophen-3-carboxylat (*Enantiomer 2*)

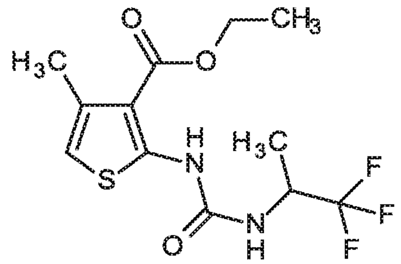

4.36 g (13.4 mmol) der racemischen Verbindung aus Bsp. 437A wurden in 135 ml Isopropanol gelöst und in 190 Portionen über präparative SFC-HPLC an chiraler Phase in die Enantiomere aufgetrennt [Säule: Daicel Chiralpak OX-H, 5 μm, 250 mm x 30 mm; Laufmittel: Kohlendioxid/Isopropanol 90:10; Fluss: 175 ml/min; Temperatur: 38°C; Detektion: 210 nm]. Nach Eindampfen der Produktfraktionen und Trocknen des Feststoffs im Hochvakuum wurden 2.10 g (96% d. Th.) von Enantiomer 2 erhalten (>99% ee, chirale analytische HPLC).
$^1$H-NMR (400 MHz, DMSO-$d_6$, δ/ppm): 10.45 (s, 1H), 8.50 (d, 1H), 6.50 (s, 1H), 4.56-4.42 (m, 1H), 4.29 (q, 2H), 2.28 (s, 3H), 1.32 (t, 3H), 1.28 (d, 3H).

Chirale analytische SFC-HPLC [Säule: Daicel Chiralcel QX, 3 μm, 50 mm x 5 mm; Laufmittel: Kohlendioxid/Isopropanol 95:5 → 50:50; Fluss: 3 ml/min; Temperatur: 40°C; Detektion: 210 nm]: $R_t$ = 1.37 min.

Spezifische optische Drehung: $[\alpha]_D^{20}$ = +7.40°·ml·dm$^{-1}$·g$^{-1}$ (Methanol).

**Beispiel 504A**

5-Methyl-3-(1,1,1-trifluorpropan-2-yl)thieno[2,3-d]pyrimidin-2,4(1*H*,3*H*)-dion (*Enantiomer 1*)

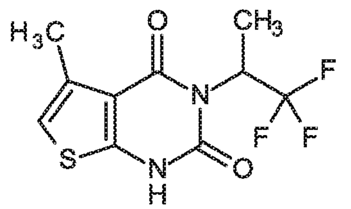

2.09 g (6.44 mmol) der Verbindung aus Bsp. 502A wurden in 20 ml Ethanol gelöst und mit 4.8 ml (12.9 mmol) einer 21%-igen Lösung von Natriumethylat in Ethanol versetzt. Nachdem das Gemisch 5 h bei 50°C gerührt worden war, wurde es mit 14.8 ml (14.8 mmol) 1 M Salzsäure versetzt. Der dabei ausgefallene Niederschlag wurde abgesaugt, mit Wasser neutral gewaschen und getrocknet. Es wurden 1.75 g (97% d. Th.) der enantiomerenreinen Titelverbindung erhalten (>99% ee, chirale analytische HPLC).
$^1$H-NMR (400 MHz, DMSO-$d_6$, δ/ppm): 12.40 und 12.26 (2 br. s, zus. 1H), 6.74 (2 s, zus. 1H), 5.78-5.66 und 5.58-5.46 (2 m, zus. 1H), 2.35 und 2.34 (2 s, zus. 3H), 1.67 und 1.64 (2 d, zus. 3H).

LC/MS (Methode 17, ESIpos): $R_t$ = 1.60 min, m/z = 279.04 $[M+H]^+$.

Chirale analytische HPLC [Säule: Daicel Chiralpak AS-3, 3 μm, 50 mm x 4.6 mm; Laufmittel: Heptan/Isopropanol 4:1; Fluss: 1 ml/min; Temperatur: 30°C; Detektion: 220 nm]: $R_t$ = 1.07 min.

Spezifische optische Drehung: $[\alpha]_D^{20}$ = -15.7°·ml·dm$^{-1}$·g$^{-1}$ (Methanol).

**Beispiel 505A**

5-Methyl-3-(1,1,1-trifluorpropan-2-yl)thieno[2,3-d]pyrimidin-2,4(1*H*,3*H*)-dion (*Enantiomer 2*)

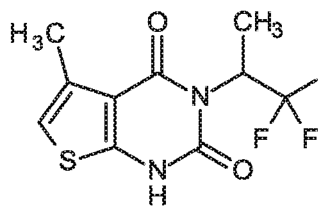

2.09 g (6.44 mmol) der Verbindung aus Bsp. 503A wurden in 20 ml Ethanol gelöst und mit 4.8 ml (12.9 mmol) einer 21%-igen Lösung von Natriumethylat in Ethanol versetzt. Nachdem das Gemisch 5 h bei 50°C gerührt worden war, wurde es mit 14.8 ml (14.8 mmol) 1 M Salzsäure versetzt. Der dabei ausgefallene Niederschlag wurde abgesaugt, mit Wasser neutral gewaschen und getrocknet. Es wurden 1.75 g (97% d. Th.) der enantiomerenreinen Titelverbindung erhalten (>99% ee, chirale analytische HPLC).

$^1$H-NMR (400 MHz, DMSO-$d_6$, δ/ppm): 12.40 und 12.27 (2 br. s, zus. 1H), 6.74 (2 s, zus. 1H), 5.78-5.66 und 5.58-5.46 (2 m, zus. 1H), 2.35 und 2.34 (2 s, zus. 3H), 1.67 und 1.64 (2 d, zus. 3H).

LC/MS (Methode 17, ESIpos): $R_t$ = 1.60 min, m/z = 279.04 $[M+H]^+$.

Chirale analytische HPLC [Säule: Daicel Chiralpak AS-3, 3 μm, 50 mm x 4.6 mm; Laufmittel: Heptan/Isopropanol 4:1; Fluss: 1 ml/min; Temperatur: 30°C; Detektion: 220 nm]: $R_t$ = 1.00 min.

Spezifische optische Drehung: $[\alpha]_D^{20}$ = +19.5°·ml·dm$^{-1}$·g$^{-1}$ (Methanol).

**Beispiel 506A**

5-Methyl-2,4-dioxo-3-(1,1,1-trifluorpropan-2-yl)-1,2,3,4-tetrahydrothieno[2,3-d]pyrimidin-6-carbaldehyd (*Enantiomer 1*)

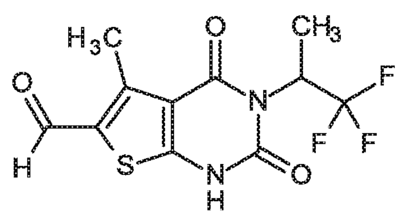

Eine Lösung von 1.74 g (6.25 mmol) der Verbindung aus Bsp. 504A in 4.8 ml (62.5 mmol) DMF wurde vorsichtig mit 7.0 ml (75.0 mmol) Phosphoroxychlorid versetzt. Nachdem die stark exotherme Reaktion abgeklungen war, wurde das Gemisch noch 15 min nachgerührt. Danach wurde das Reaktionsgemisch vorsichtig in 250 ml Eiswasser eingerührt. Nach 1 h Rühren wurde das ausgefallene Produkt abgesaugt, mit Wasser neutral gewaschen und getrocknet. Es wurden 1.79 g (93% d. Th.) der enantiomerenreinen Titelverbindung erhalten (>99% ee, chirale analytische HPLC).

$^1$H-NMR (400 MHz, DMSO-$d_6$, δ/ppm): 12.80 (br. s, 1H), 10.08 (s, 1H), 5.75-5.63 und 5.56-5.43 (2 m, zus. 1H), 2.76 und 2.75 (2 s, zus. 3H), 1.67 und 1.64 (2 d, zus. 3H).

LC/MS (Methode 17, ESIpos): $R_t$ = 1.54 min, m/z = 307.04 $[M+H]^+$.

Chirale analytische HPLC [Säule: Daicel Chiralpak IC-3, 3 μm, 50 mm x 4.6 mm; Laufmittel: Heptan/Ethanol 4:1; Fluss: 1 ml/min; Temperatur: 30°C; Detektion: 220 nm]: $R_t$ = 1.99 min.

Spezifische optische Drehung: $[\alpha]_D^{20}$ = -18.5°·ml·dm$^{-1}$·g$^{-1}$ (Methanol).

**Beispiel 507A**

5-Methyl-2,4-dioxo-3-(1,1,1-trifluorpropan-2-yl)-1,2,3,4-tetrahydrothieno[2,3-d]pyrimidin-6-carbaldehyd (*Enantiomer 2*)

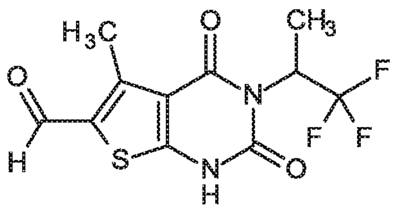

Eine Lösung von 1.74 g (6.25 mmol) der Verbindung aus Bsp. 505A in 4.8 ml (62.5 mmol) DMF wurde vorsichtig mit 7.0 ml (75.0 mmol) Phosphoroxychlorid versetzt. Nachdem die stark exotherme Reaktion abgeklungen war, wurde das Gemisch noch 15 min nachgerührt. Danach wurde das Reaktionsgemisch vorsichtig in 250 ml Eiswasser eingerührt. Nach 1 h Rühren wurde das ausgefallene Produkt abgesaugt, mit Wasser neutral gewaschen und getrocknet. Es wurden 1.78 g (92% d. Th.) der enantiomerenreinen Titelverbindung erhalten (>99% ee, chirale analytische HPLC).
$^1$H-NMR (400 MHz, DMSO-$d_6$, $\delta$/ppm): 12.79 (br. s, 1H), 10.08 (s, 1H), 5.75-5.63 und 5.56-5.43 (2 m, zus. 1H), 2.76 und 2.75 (2 s, zus. 3H), 1.67 und 1.64 (2 d, zus. 3H).
LC/MS (Methode 17, ESIpos): $R_t$ = 1.54 min, m/z = 307.04 $[M+H]^+$.
Chirale analytische HPLC [Säule: Daicel Chiralpak IC-3, 3 μm, 50 mm x 4.6 mm; Laufmittel: Heptan/Ethanol 4:1; Fluss: 1 ml/min; Temperatur: 30°C; Detektion: 220 nm]: $R_t$ = 1.58 min.
Spezifische optische Drehung: $[\alpha]_D^{20}$ = +19.0°·ml·dm$^{-1}$·g$^{-1}$ (Methanol).

**Beispiel 508A**

1-(2-Methoxyethyl)-5-methyl-2,4-dioxo-3-(1,1,1-trifluorpropan-2-yl)-1,2,3,4-tetrahydrothieno-[2,3-d]pyrimidin-6-caibaldehyd (*Enantiomer 1*)

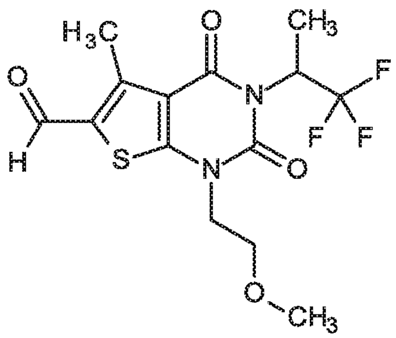

Eine Lösung von 1.78 g (5.81 mmol) der Verbindung aus Bsp. 506A in 35 ml DMF wurde mit 2.01 g (14.5 mmol) Kaliumcarbonat versetzt und 15 min bei RT gerührt. Dann wurden 1.62 g (11.6 mmol) (2-Bromethyl)-methylether zugesetzt und das Gemisch bei 50°C gerührt. Nach ca. 18 h wurden weitere 0.81 g (5.81 mmol) (2-Bromethyl)-methylether hinzugefügt und das Rühren bei 50°C 2 Tage lang fortgesetzt. Nach dem Abkühlen auf RT wurden ca. 200 ml Wasser hinzugefügt und das Gemisch dann mit Ethylacetat extrahiert. Der organische Extrakt wurde mit gesättigter wässriger Natriumchlorid-Lösung gewaschen, über wasserfreiem Magnesiumsulfat getrocknet, filtriert und eingeengt. Der verbliebene Rückstand wurde mittels MPLC gereinigt (Biotage Isolera, Kartusche mit 100 g Kieselgel, Cyclohexan/Ethylacetat 5:1). Die Produktfraktionen wurden vereinigt, eingeengt und im Hochvakuum getrocknet. Es wurden 1.32 g (62% d. Th.) der enantiomerenreinen Titelverbindung erhalten (>99% ee, chirale analytische HPLC).
$^1$H-NMR (400 MHz, DMSO-$d_6$, $\delta$/ppm): 10.10 (s, 1H), 5.79-5.67 und 5.60-5.48 (2 m, zus. 1H), 4.15-4.07 (m, 2H), 3.68-3.62 (m, 2H), 3.25 (s, 3H), 2.78 (s, 3H), 1.69 und 1.65 (2 d, zus. 3H).
LC/MS (Methode 1, ESIpos): $R_t$ = 0.97 min, m/z = 365 $[M+H]^+$.
Chirale analytische HPLC [Säule: Daicel Chiralpak OJ-3, 3 μm, 50 mm x 4.6 mm; Laufmittel: Heptan/Ethanol

1:1; Fluss: 1 ml/min; Temperatur: 30°C; Detektion: 220 nm]: $R_t$ = 1.82 min.
Spezifische optische Drehung: $[\alpha]_D^{20}$ = -17.9°·ml·dm$^{-1}$·g$^{-1}$ (Methanol).

**Beispiel 509A**

1-(2-Methoxyethyl)-5-methyl-2,4-dioxo-3-(1,1,1-trifluorpropan-2-yl)-1,2,3,4-tetrahydrothieno-[2,3-d]pyrimidin-6-carbaldehyd (*Enantiomer 2*)

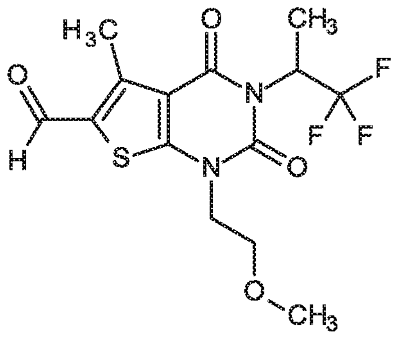

Eine Lösung von 1.77 g (5.78 mmol) der Verbindung aus Bsp. 507A in 35 ml DMF wurde mit 2.0 g (14.4 mmol) Kaliumcarbonat versetzt und 15 min bei RT gerührt. Dann wurden 1.61 g (11.6 mmol) (2-Bromethyl)-methylether zugesetzt und das Gemisch bei 50°C gerührt. Nach ca. 18 h wurden weitere 0.81 g (5.81 mmol) (2-Bromethyl)-methylether hinzugefügt und das Rühren bei 50°C 2 Tage lang fortgesetzt. Nach dem Abkühlen auf RT wurden ca. 200 ml Wasser hinzugefügt und das Gemisch dann mit Ethylacetat extrahiert. Der organische Extrakt wurde mit gesättigter wässriger Natriumchlorid-Lösung gewaschen, über wasserfreiem Magnesiumsulfat getrocknet, filtriert und eingeengt. Der verbliebene Rückstand wurde mittels MPLC gereinigt (Biotage Isolera, Kartusche mit 100 g Kieselgel, Cyclohexan/Ethylacetat 5:1). Die Produktfraktionen wurden vereinigt, eingeengt und im Hochvakuum getrocknet. Es wurden 1.42 g (67% d. Th.) der enantiomerenreinen Titelverbindung erhalten (>99% ee, chirale analytische HPLC).
$^1$H-NMR (400 MHz, DMSO-$d_6$, $\delta$/ppm): 10.10 (s, 1H), 5.79-5.67 und 5.60-5.48 (2 m, zus. 1H), 4.16-4.07 (m, 2H), 3.70-3.61 (m, 2H), 3.25 (s, 3H), 2.78 (s, 3H), 1.69 und 1.65 (2 d, zus. 3H).
LC/MS (Methode 1, ESIpos): $R_t$ = 0.97 min, m/z = 365 $[M+H]^+$.
Chirale analytische HPLC [Säule: Daicel Chiralpak OJ-3, 3 µm, 50 mm x 4.6 mm; Laufmittel: Heptan/Ethanol 1:1; Fluss: 1 ml/min; Temperatur: 30°C; Detektion: 220 nm]: $R_t$ = 1.65 min.
Spezifische optische Drehung: $[\alpha]_D^{20}$ = +18.8°·ml·dm$^{-1}$·g$^{-1}$ (Methanol).

**Beispiel 510A**

6-[Dideutero(hydroxy)methyl]-3-isopropyl-1-(2-methoxyethyl)-5-methylthieno[2,3-d]pyrimidin-2,4(1*H*,3*H*)-dion

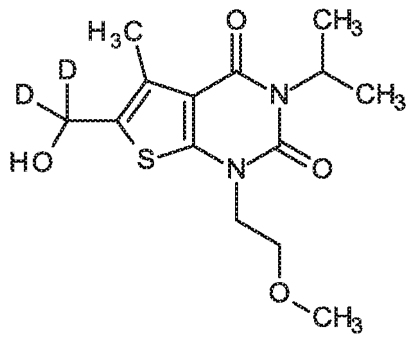

Eine Lösung von 1.03 g (2.61 mmol, 90% Reinheit) der Verbindung aus Bsp. 498A in 28 ml THF wurde bei -78°C tropfenweise mit 2.4 ml (2.35 mmol) einer 1 M Lösung von Lithiumaluminiumdeuterid in THF versetzt. Anschließend

wurde das Reaktionsgemisch 1 h bei 0°C gerührt, bevor 1.2 ml Wasser, 9 ml 1 M Natronlauge sowie etwas Kieselgur hinzugefügt wurden und das Kältebad entfernt wurde. Der entstandene Niederschlag wurde abgesaugt und gründlich mit THF gewaschen. Das mit der Waschflüssigkeit vereinigte Filtrat wurde zur Trockene eingeengt. Der verbliebene Rückstand wurde in Ethylacetat aufgenommen und nacheinander mit Wasser und gesättigter wässriger Natriumchlorid-Lösung gewaschen. Nach Trocknen über wasserfreiem Magnesiumsulfat wurde filtriert und eingeengt. Das Rohprodukt wurde mittels MPLC gereinigt (Biotage Isolera, Kartusche SNAP KP-Sil mit 50 g Kieselgel, Cyclohexan/Ethylacetat 90:10 → 30:70). Die Produktfraktionen wurden vereinigt, eingedampft und der Rückstand im Hochvakuum getrocknet. Es wurden 680 mg (80% d. Th., 98% Reinheit) der Titelverbindung erhalten.

$^1$H-NMR (400 MHz, DMSO-$d_6$, δ/ppm): 5.49 (s, 1H), 5.14 (sept, 1H), 4.01 (t, 2H), 3.63 (t, 2H), 3.31 (s, 3H), 2.31 (s, 3H), 1.40 (d, 6H).

LC/MS (Methode 1, ESIpos): $R_t$ = 0.75 min, m/z = 315 [M+H]$^+$.

**Beispiel 511A**

6-[Dideutero(hydroxy)methyl]-1-(2-methoxyethyl)-5-methyl-3-(2,2,2-trifluorethyl)thieno[2,3-d]-pyrimidin-2,4(1*H*,3*H*)-dion

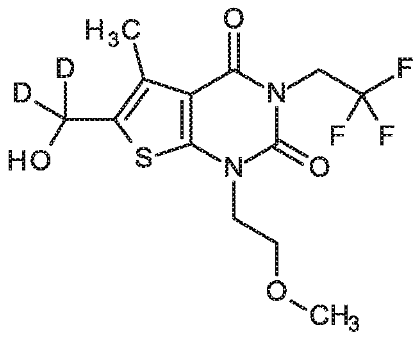

Eine Lösung von 1.92 g (4.48 mmol, 92% Reinheit) der Verbindung aus Bsp. 499A in 47 ml THF wurde bei -78°C tropfenweise mit 4 ml (4.03 mmol) einer 1 M Lösung von Lithiumaluminiumdeuterid in THF versetzt. Anschließend wurde das Reaktionsgemisch 1 h bei 0°C gerührt, bevor 1.2 ml Wasser, 9 ml 1 M Natronlauge sowie etwas Kieselgur hinzugefügt wurden und das Kältebad entfernt wurde. Der entstandene Niederschlag wurde abgesaugt und gründlich mit THF gewaschen. Das mit der Waschflüssigkeit vereinigte Filtrat wurde zur Trockene eingeengt. Der verbliebene Rückstand wurde in Ethylacetat aufgenommen und nacheinander mit Wasser und gesättigter wässriger Natriumchlorid-Lösung gewaschen. Nach Trocknen über wasserfreiem Magnesiumsulfat wurde filtriert und eingeengt. Das Rohprodukt wurde zunächst mittels MPLC vorgereinigt (Biotage Isolera, Kartusche SNAP KP-Sil mit 100 g Kieselgel, Cyclohexan/Ethylacetat 90:10 → 30:70). Die produkthaltigen Fraktionen wurden eingedampft und der Rückstand mittels präparativer HPLC (Methode 8) weiter aufgereinigt. Nach erneutem Eindampfen der Produktfraktionen und Trocknen im Hochvakuum wurden 620 mg (34% d. Th., 88% Reinheit) der Titelverbindung erhalten, die ohne weitere Aufreinigung für Folgereaktionen verwendet wurden.

$^1$H-NMR (400 MHz, DMSO-$d_6$, δ/ppm): 5.57 (s, 1H), 4.70 (q, 2H), 4.08 (t, 2H), 3.65 (t, 2H), 3.24 (s, 3H), 2.32 (s, 3H).

LC/MS (Methode 1, ESIpos): $R_t$ = 0.76 min, m/z = 355 [M+H]$^+$.

**Beispiel 512A**

6-[Dideutero(hydroxy)methyl]-1-(2-methoxyethyl)-5-methyl-3-[(2*R*)-1,1,1-trifluorpropan-2-yl]-thieno[2,3-d]pyrimidin-2,4(1*H*,3*H*)-dion

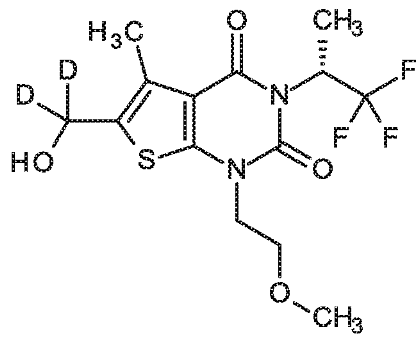

Eine Lösung von 680 mg (1.67 mmol) der Verbindung aus Bsp. 500A in 20 ml THF wurde bei -78°C tropfenweise mit 1.5 ml (1.5 mmol) einer 1 M Lösung von Lithiumaluminiumdeuterid in THF versetzt. Anschließend wurde das Reaktionsgemisch 1 h bei 0°C gerührt, bevor 0.8 ml Wasser, 5 ml 1 M Natronlauge sowie etwas Kieselgur hinzugefügt wurden und das Kältebad entfernt wurde. Der entstandene Niederschlag wurde abgesaugt und gründlich mit THF gewaschen. Das mit der Waschflüssigkeit vereinigte Filtrat wurde zur Trockene eingeengt. Der verbliebene Rückstand wurde in Ethylacetat aufgenommen und nacheinander mit Wasser und gesättigter wässriger Natriumchlorid-Lösung gewaschen. Nach Trocknen über wasserfreiem Magnesiumsulfat wurde filtriert und eingeengt. Das Rohprodukt wurde mittels MPLC gereinigt (Biotage Isolera, Kartusche SNAP KP-Sil mit 50 g Kieselgel, Cyclohexan/Ethylacetat 1:1). Nach Eindampfen der Produktfraktionen und Trocknen im Hochvakuum wurden 462 mg (75% d. Th.) der Titelverbindung erhalten (>99% ee, chirale analytische HPLC).

$^1$H-NMR (400 MHz, DMSO-$d_6$, $\delta$/ppm): 5.81-5.69 und 5.62-5.51 (2 m, zus. 1H), 5.55 (s, 1H), 4.10-3.98 (m, 2H), 3.69-3.59 (m, 2H), 3.24 (s, 3H), 2.32 und 2.30 (2 s, zus. 3H), 1.68 und 1.64 (2 d, zus. 3H).

LC/MS (Methode 17, ESIpos): $R_t$ = 1.59 min, m/z = 369.11 $[M+H]^+$.

Chirale analytische HPLC [Säule: Daicel Chiralpak AS-3, 3 µm, 50 mm x 4.6 mm; Laufmittel: Heptan/Isopropanol 4:1; Fluss: 1 ml/min; Temperatur: 25°C; Detektion: 220 nm]: $R_t$ = 2.52 min.

Spezifische optische Drehung: $[\alpha]_D^{20}$ = +12.4°·ml·dm$^{-1}$·g$^{-1}$ (Methanol).

## Beispiel 513A

6-[Dideutero(hydroxy)methyl]-1-(2-methoxyethyl)-5-methyl-3-[(2*S*)-1,1,1-trifluorpropan-2-yl]-thieno[2,3-d]pyrimidin-2,4(1*H*,3*H*)-dion

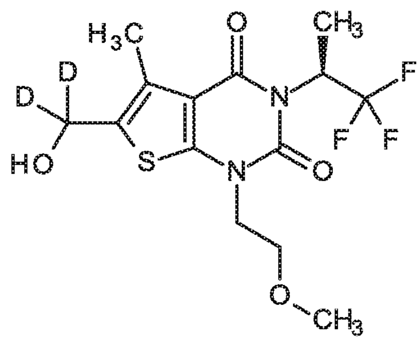

Eine Lösung von 246 mg (0.602 mmol) der Verbindung aus Bsp. 501A in 7 ml THF wurde bei -78°C tropfenweise mit 542 µl (0.542 mmol) einer 1 M Lösung von Lithiumaluminiumdeuterid in THF versetzt. Anschließend wurde das Reaktionsgemisch 1 h bei 0°C gerührt, bevor 1.2 ml Wasser, 9 ml 1 M Natronlauge sowie etwas Kieselgur hinzugefügt wurden und das Kältebad entfernt wurde. Der entstandene Niederschlag wurde abgesaugt und gründlich mit THF gewaschen. Das mit der Waschflüssigkeit vereinigte Filtrat wurde zur Trockene eingeengt. Der verbliebene Rückstand wurde in Ethylacetat aufgenommen und nacheinander mit Wasser und gesättigter wässriger Natriumchlorid-Lösung gewaschen. Nach Trocknen über wasserfreiem Magnesiumsulfat wurde filtriert und eingeengt. Das Rohprodukt wurde mittels MPLC gereinigt (Biotage Isolera, Kartusche SNAP KP-Sil mit 25 g Kieselgel, Cyclohexan/Ethylacetat 90:10 → 0:100). Nach Eindampfen der Produktfraktionen und Trocknen im Hochvakuum wurden 164 mg (73% d. Th.) der Titelverbindung erhalten (>99% ee, chirale analytische HPLC).

$^1H$-NMR (400 MHz, DMSO-$d_6$, $\delta$/ppm): 5.81-5.69 und 5.62-5.52 (2 m, zus. 1H), 5.55 (s, 1H), 4.08-4.01 (m, 2H), 3.67-3.61 (m, 2H), 3.24 (s, 3H), 2.32 und 2.30 (2 s, zus. 3H), 1.68 und 1.64 (2 d, zus. 3H).
LC/MS (Methode 17, ESIpos): $R_t$ = 1.59 min, m/z = 369.11 $[M+H]^+$.

Chirale analytische HPLC [Säule: Daicel Chiralpak AS-3, 3 μm, 50 mm x 4.6 mm; Laufmittel: Heptan/Isopropanol 4:1; Fluss: 1 ml/min; Temperatur: 25°C; Detektion: 220 nm]: $R_t$ = 1.79 min.

Spezifische optische Drehung: $[\alpha]_D^{20}$ = -7.1°·ml·dm$^{-1}$·g$^{-1}$ (Methanol).

**Beispiel 514A**

6-{[(2-Aminoethyl)amino]methyl}-1-(2-methoxyethyl)-5-methyl-3-(1,1,1-trifluorpropan-2-yl)-thieno[2,3-d]pyrimidin-2,4(1*H*,3*H*)-dion (*Enantiomer 1*)

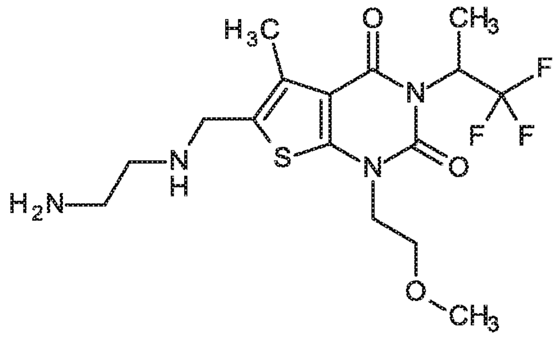

650 mg (1.78 mmol) der Verbindung aus Bsp. 508A wurden in einem Gemisch aus 18 ml Methanol und 6 ml Dichlormethan gelöst und bei RT mit 716 μl (10.7 mmol) 1,2-Diaminoethan und 409 μl (7.14 mmol) Essigsäure versetzt. Nach 30 min wurden 448 mg (7.14 mmol) Natriumcyanoborhydrid hinzugefügt, und das Reaktionsgemisch wurde auf 60°C erwärmt. Nach ca. 15 h ließ man das Reaktionsgemisch wieder auf RT abkühlen. Anschließend wurde es mit ca. 50 ml 2 M Natronlauge versetzt und gründlich mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach Trocknen im Hochvakuum 910 mg (99% d. Th., 80% Reinheit) der enantiomerenreinen Titelverbindung (>99% ee, chirale analytische HPLC), welche ohne weitere Reinigung für Folgereaktionen verwendet wurden.
LC/MS (Methode 2, ESIpos): $R_t$ = 0.92 min, m/z = 409 $[M+H]^+$.

Chirale analytische HPLC [Säule: Daicel Chiralpak IC-3, 3 μm, 50 mm x 4.6 mm; Laufmittel: Heptan/Isopropanol 80:20 + 0.2% Diethylamin; Fluss: 1 ml/min; Temperatur: 30°C; Detektion: 220 nm]: $R_t$ = 2.86 min.

**Beispiel 515A**

6-{[(2-Aminoethyl)amino]methyl}-1-(2-methoxyethyl)-5-methyl-3-(1,1,1-trifluorpropan-2-yl)-thieno[2,3-d]pyrimidin-2,4(1*H*,3*H*)-dion (*Enantiomer 2*)

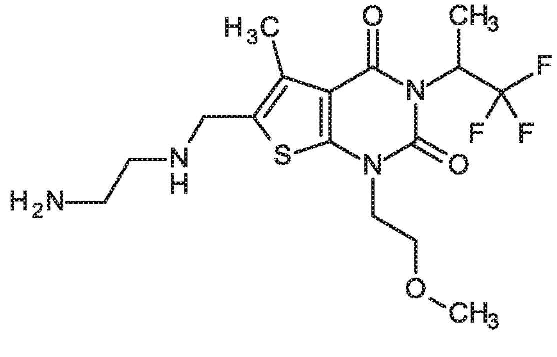

700 mg (1.92 mmol) der Verbindung aus Bsp. 509A wurden in einem Gemisch aus 18 ml Methanol und 6 ml Dichlormethan gelöst und bei RT mit 771 μl (11.5 mmol) 1,2-Diaminoethan und 440 μl (7.68 mmol) Essigsäure versetzt. Nach 30 min wurden 483 mg (7.68 mmol) Natriumcyanoborhydrid hinzugefügt, und das Reaktionsgemisch wurde auf 60°C erwärmt. Nach ca. 15 h ließ man das Reaktionsgemisch wieder auf RT abkühlen. Anschließend wurde es mit ca. 50 ml 2 M Natronlauge versetzt und gründlich mit Ethylacetat extrahiert. Der organische Extrakt wurde mit gesättigter Natriumchlorid-Lösung gewaschen, über wasserfreiem Magnesiumsulfat getrocknet, filtriert und am Rotationsverdampfer eingeengt. Das so erhaltene Rohprodukt ergab nach Trocknen im Hochvakuum 920 mg (93% d. Th., 80% Reinheit) der enantiomerenreinen Titelverbindung (>99% ee, chirale analytische HPLC), welche ohne weitere Reinigung für Folgereaktionen verwendet wurden.
LC/MS (Methode 2, ESIpos): $R_t$ = 0.96 min, m/z = 409 $[M+H]^+$.

Chirale analytische HPLC [Säule: Daicel Chiralpak IC-3, 3 μm, 50 mm x 4.6 mm; Laufmittel: Heptan/Isopropanol 80:20 + 0.2% Diethylamin; Fluss: 1 ml/min; Temperatur: 30°C; Detektion: 220 nm]: $R_t$ = 2.62 min.

**Beispiel 516A**

6-{[(2,2-Dimethoxyethyl)amino]methyl}-3-(2,2-dimethylpropyl)-5-methyl-1-(3,3,3-trifluorpropyl)-thieno[2,3-d]pyrimidin-2,4(1*H*,3*H*)-dion

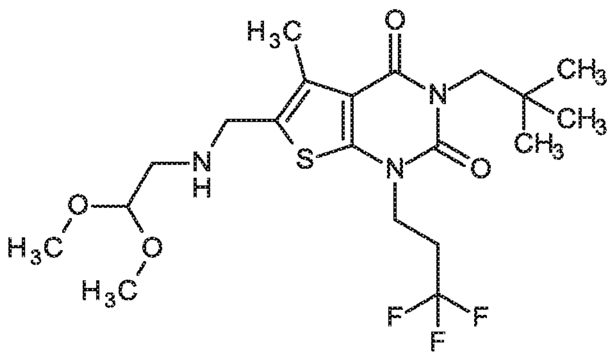

100 mg (0.255 mmol, 96% Reinheit) der Verbindung aus Bsp. 291A wurden in 6 ml Dichlormethan gelöst und mit 42 μl (0.383 mmol) 2,2-Dimethoxyethanamin versetzt. Das Gemisch wurde 1 h auf 35°C erwärmt. Nach Abkühlen auf RT wurden 162 mg (0.765 mmol) Natriumtriacetoxyborhydrid hinzugefügt, und das Gemisch wurde bei RT weiter gerührt. Nach 18 h wurde das Reaktionsgemisch mit Dichlormethan verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und das Filtrat zur Trockene eingeengt. Es wurden 95 mg (62% d. Th., 77% Reinheit) der Titelverbindung erhalten, welche ohne weitere Reinigung für Folgereaktionen verwendet wurden.
LC/MS (Methode 1, ESIpos): $R_t$ = 0.78 min, m/z = 466 $[M+H]^+$.

**Beispiel 517A**

6-{[(2,2-Dimethoxyethyl)amino]methyl}-3-(2,2-dimethylpropyl)-1-(2-methoxyethyl)-5-methylthieno[2,3-d]pyrimidin-2,4(1*H*,3*H*)-dion

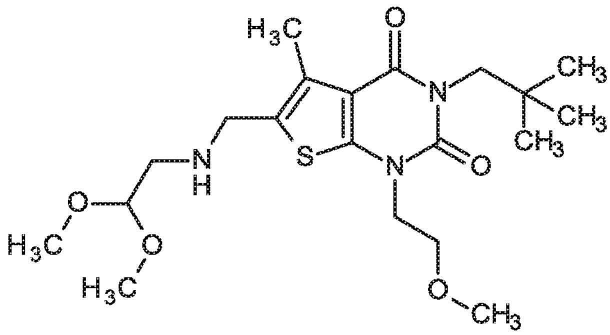

100 mg (0.269 mmol, 91% Reinheit) der Verbindung aus Bsp. 293A wurden in 6 ml Dichlormethan gelöst und mit 44 µl (0.403 mmol) 2,2-Dimethoxyethanamin versetzt. Das Gemisch wurde 1 h auf 35°C erwärmt. Nach Abkühlen auf RT wurden 171 mg (0.807 mmol) Natriumtriacetoxyborhydrid hinzugefügt, und das Gemisch wurde bei RT weiter gerührt. Nach 18 h wurde das Reaktionsgemisch mit Dichlormethan verdünnt und nacheinander mit gesättigter Natriumhydiogencarbonat-Lösung und gesättigter Natriumchlorid-Lösung gewaschen. Nach Trocknen über wasserfreiem Magnesiumsulfat wurde filtriert und das Filtrat zur Trockene eingeengt. Es wurden 108 mg (78% d. Th., 83% Reinheit) der Titelverbindung erhalten, welche ohne weitere Reinigung für Folgereaktionen verwendet wurden.
LC/MS (Methode 1, ESIpos): $R_t$ = 0.70 min, m/z = 324 $[M+H-C_4H_{11}NO_2]^+$.

## Beispiel 518A

6-{[(2,2-Dimethoxyethyl)amino]methyl}-3-(2,2-dimethylpropyl)-5-methyl-1-(tetrahydrofuran-2-ylmethyl)thieno[2,3-d]pyrimidin-2,4(1*H*,3*H*)-dion (*Racemat*)

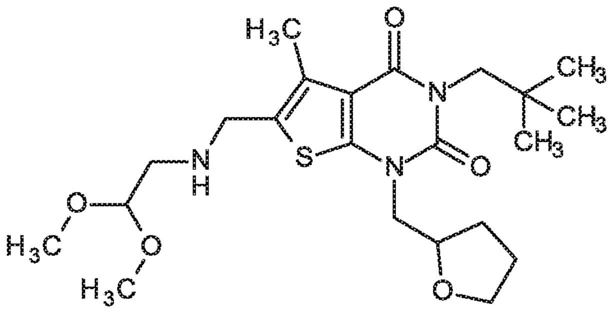

100 mg (0.230 mmol, 84% Reinheit) der Verbindung aus Bsp. 443A wurden in 5 ml Dichlormethan gelöst und mit 37 µl (0.346 mmol) 2,2-Dimethoxyethanamin versetzt. Das Gemisch wurde 1 h auf 35°C erwärmt. Nach Abkühlen auf RT wurden 146 mg (0.691 mmol) Natriumtriacetoxyborhydrid hinzugefügt, und das Gemisch wurde bei RT weiter gerührt. Nach 18 h wurde das Reaktionsgemisch mit Dichlormethan verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und das Filtrat zur Trockene eingeengt. Es wurden 107 mg (83% d. Th., 81% Reinheit) der Titelverbindung erhalten, welche ohne weitere Reinigung für Folgereaktionen verwendet wurden.
LC/MS (Methode 1, ESIpos): $R_t$ = 0.71 min, m/z = 350 $[M+H-C_4H_{11}NO_2]^+$.

## Beispiel 519A

1-(2,2-Dimethoxyethyl)-1-{[3-(2,2-dimethylpropyl)-5-methyl-2,4-dioxo-1-(3,3,3-trifluorpropyl)-1,2,3,4-tetrahydrothieno[2,3-d]pyrimidin-6-yl]methyl}harnstoff

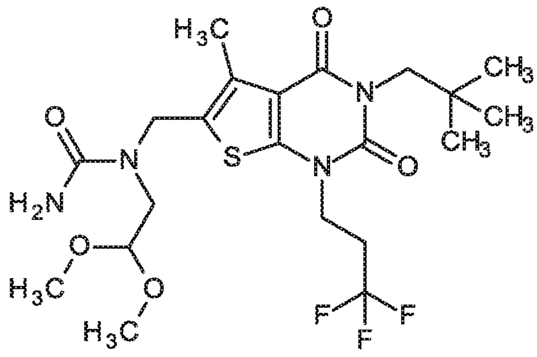

Eine Lösung von 93 mg (0.157 mmol, 78% Reinheit) der Verbindung aus Bsp. 516A in 1.6 ml Methanol wurde bei RT zunächst mit 29 mg (0.360 mmol) Kaliumcyanat und dann mit 23 µl (0.266 mmol) Perchlorsäure (70% in Wasser) versetzt. Nach 1 h wurde das Reaktionsgemisch mit Wasser und mit wässriger Natriumhydrogencarbonat-Lösung versetzt und anschließend mit Ethylacetat extrahiert. Der organische Extrakt wurde nacheinander mit gesättigter Natriumhydrogencarbonat-Lösung und gesättigter Natriumchlorid-Lösung gewaschen, über wasserfreiem Magnesiumsulfat getrocknet, filtriert und eingeengt. Nach Trocknen des erhaltenen Rückstands im Hochvakuum wurden 80 mg (79% d. Th., 79% Reinheit) der Titelverbindung erhalten, die ohne weitere Aufreinigung für Folgereaktionen verwendet wurden.
LC/MS (Methode 17, ESIneg): $R_t$ = 1.97 min, m/z = 553.19 $[M-H+HCO_2H]^-$.

**Beispiel 520A**

1-(2,2-Dimethoxyethyl)-1-{[3-(2,2-dimethylpropyl)-1-(2-methoxyethyl)-5-methyl-2,4-dioxo-1,2,3,4-tetrahydrothieno[2,3-d]pyrimidin-6-yl]methyl}harnstoff

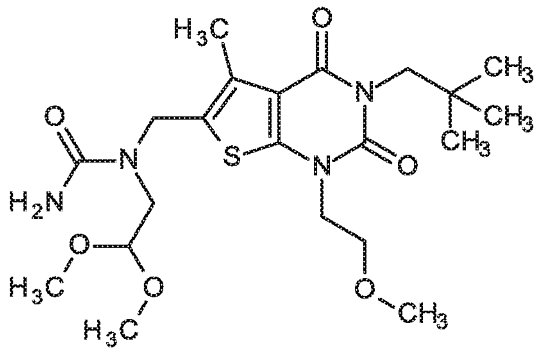

Eine Lösung von 106 mg (0.193 mmol, 78% Reinheit) der Verbindung aus Bsp. 517A in 2 ml Methanol wurde bei RT zunächst mit 36 mg (0.445 mmol) Kaliumcyanat und dann mit 28 µl (0.329 mmol) Perchlorsäure (70% in Wasser) versetzt. Nach 1 h wurde das Reaktionsgemisch mit Wasser und mit gesättigter Natriumhydrogencarbonat-Lösung versetzt und anschließend mit Ethylacetat extrahiert. Der organische Extrakt wurde mit gesättigter Natriumchlorid-Lösung gewaschen, über wasserfreiem Magnesiumsulfat getrocknet, filtriert und eingeengt. Nach Trocknen des erhaltenen Rückstands im Hochvakuum wurden 95 mg (74% d. Th., 71% Reinheit) der Titelverbindung erhalten, die ohne weitere Aufreinigung für Folgereaktionen verwendet wurden.
LC/MS (Methode 17, ESIneg): $R_t$ = 1.74 min, m/z = 515.22 $[M-H+HCO_2H]^-$.

**Beispiel 521A**

1-(2,2-Dimethoxyethyl)-1-{[3-(2,2-dimethylpropyl)-5-methyl-2,4-dioxo-1-(tetrahydrofuran-2-ylmethyl)-1,2,3,4-tetrahydrothieno[2,3-d]pyrimidin-6-yl]methyl}hanistoff (*Racemat*)

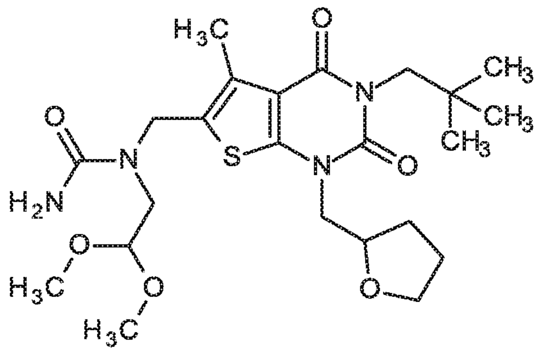

Eine Lösung von 106 mg (0.189 mmol, 81% Reinheit) der Verbindung aus Bsp. 518A in 2 ml Methanol wurde bei RT zunächst mit 35 mg (0.435 mmol) Kaliumcyanat und dann mit 28 µl (0.322 mmol) Perchlorsäure (70% in Wasser) versetzt. Nach 1 h wurde das Reaktionsgemisch mit Wasser und mit gesättigter Natriumhydrogencarbonat-Lösung versetzt und anschließend mit Ethylacetat extrahiert. Der organische Extrakt wurde nacheinander mit gesättigter Natriumhydrogencarbonat-Lösung und gesättigter Natriumchlorid-Lösung gewaschen, über wasserfreiem Magnesiumsulfat getrocknet, filtriert und eingeengt. Nach Trocknen des erhaltenen Rückstands im Hochvakuum wurden 93 mg (81% d. Th., 82% Reinheit) der Titelverbindung erhalten, die ohne weitere Aufreinigung für Folgereaktionen verwendet wurden.
LC/MS (Methode 1, ESIneg): $R_t$ = 1.83 min, m/z = 541.23 $[M-H+HCO_2H]^-$.

**Beispiel 522A**

1-{[3-Ethyl-5-methyl-2,4-dioxo-1-(3,3,3-trifluorpropyl)-1,2,3,4-tetrahydrothieno[2,3-d]pyrimidin-6-yl]methyl}hydrazincarboxamid

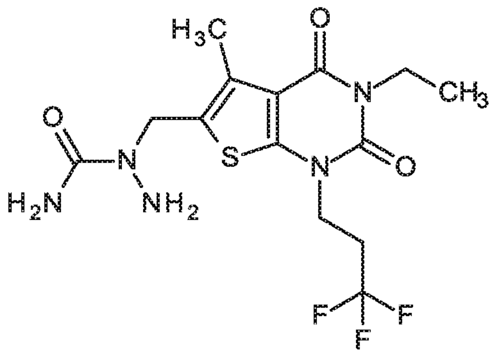

1.22 g (2.47 mmol) der Verbindung aus Bsp. 474A wurden in 20 ml Dichlormethan gelöst und bei 0°C mit 10 ml Trifluoressigsäure versetzt. Nachdem das Reaktionsgemisch 1 h bei RT gerührt worden war, wurde es zur Trockene eingeengt. Der verbliebene Rückstand wurde in Ethylacetat aufgenommen und mit gesättigter wässriger Natriumcarbonat-Lösung ausgeschüttelt. Nach Eindampfen der organischen Phase und Trocknen des Rückstands im Hochvakuum wurden 1.09 g (89% d. Th.) der Titelverbindung erhalten.
$^1$H-NMR (400 MHz, DMSO-$d_6$, δ/ppm): 6.16 (br. s, 2H), 4.63 (s, 2H), 4.41 (s, 2H), 4.11 (t, 2H), 3.91 (q, 2H), 2.84-2.68 (m, 3H), 2.43 (s, 3H), 1.12 (t, 3H).
LC/MS (Methode 1, ESIpos): $R_t$ = 0.73 min, m/z = 394 $[M+H]^+$.

**Beispiel 523A**

2-Acetyl-1-{[3-ethyl-5-methyl-2,4-dioxo-1-(3,3,3-trifluorpropyl)-1,2,3,4-tetrahydrothieno[2,3-d]-pyrimidin-6-yl]methyl}hydrazincarboxamid

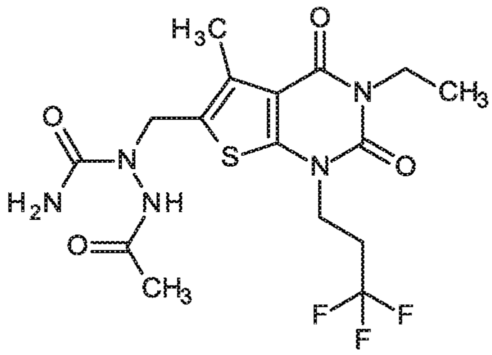

70 mg (0.179 mmol) der Verbindung aus Bsp. 522A wurden in 5 ml Dichlormethan gelöst und mit 30 µl (0.215 mmol) Triethylamin und 13 µl (0.179 mmol) Acetylchlorid versetzt. Nach 4 h Rühren bei RT wurden weitere 50 µl (0.358 mmol) Triethylamin und 19 µl (0.268 mmol) Acetylchlorid hinzugefügt. Nach weiteren 16 h Rühren bei RT wurde das Reaktionsgemisch mit Dichlormethan verdünnt und mit Wasser gewaschen. Die organische Phase wurde eingeengt, und das Produkt wurde mittels präparativer HPLC isoliert (Methode 8). Nach Eindampfen der Produktfraktionen und Trocknen im Hochvakuum wurden 12 mg (15% d. Th.) der Titelverbindung erhalten.
$^1$H-NMR (400 MHz, DMSO-$d_6$, δ/ppm): 9.78 (s, 1H), 6.28 (s, 2H), 5.19-4.20 (breit, 2H), 4.13 (t, 2H), 3.91 (q, 2H), 2.85-2.68 (m, 2H), 2.32 (s, 3H), 1.82 (s, 3H), 1.12 (t, 3H).
LC/MS (Methode 17, ESIneg): $R_t$ = 1.22 min, m/z = 434.11 $[M-H]^-$.

## Ausführungsbeispiele:

### Beispiel 1

1-(2-Methoxyethyl)-5-methyl-6-[(2-oxoazetidin-1-yl)methyl]-3-(2-phenylethyl)thieno[2,3-d]-pyrimidin-2,4(1*H*,3*H*)-dion

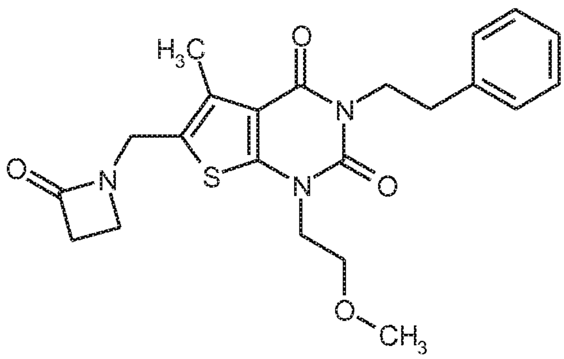

Eine Lösung von 80 mg (0.214 mmol) der Verbindung aus Bsp. 138A in 1.6 ml Dichlormethan wurde bei 0°C mit 74 µl (0.427 mmol) *N,N*-Diisopropylethylamin und 16 µl (0.224 mmol) Thionylchlorid versetzt. Nach 20 min wurde eine Lösung von 46 mg (0.641 mmol) 2-Azetidinon in 1.6 ml THF, die zuvor mit 27 mg (0.641 mmol) Natriumhydrid (60% Suspension in Mineralöl) versetzt und 30 min bei RT gerührt worden war, hinzugefügt. Anschließend wurde das Reaktionsgemisch 2 h bei RT gerührt. Danach wurden alle flüchtigen Bestandteile am Rotationsverdampfer entfernt. Der verbliebene Rückstand wurde mittels präparativer HPLC (Methode 8) in seine Komponenten aufgetrennt. Nach Eindampfen der Produktfraktionen und Trocknen im Hochvakuum wurden 33 mg (34% d. Th., 95% Reinheit) der Titelverbindung erhalten.
$^1$H-NMR (400 MHz, DMSO-$d_6$, δ/ppm): 7.35-7.27 (m, 2H), 7.26-7.17 (m, 3H), 4.45 (s, 2H), 4.13-3.97 (m, 4H), 3.61 (t, 2H), 3.24 (s, 3H), 3.16 (t, 2H), 2.88 (t, 2H), 2.86-2.78 (m, 2H), 2.39 (s, 3H).
LC/MS (Methode 1, ESIpos): $R_t$ = 1.02 min, m/z = 428 $[M+H]^+$.

**Beispiel 2**

3-Ethyl-5-methyl-6-[(2-oxoazetidin-1-yl)methyl]-1-(3,3,3-trifluorpropyl)thieno[2,3-d]pyrimidin-2,4(1*H*,3*H*)-dion

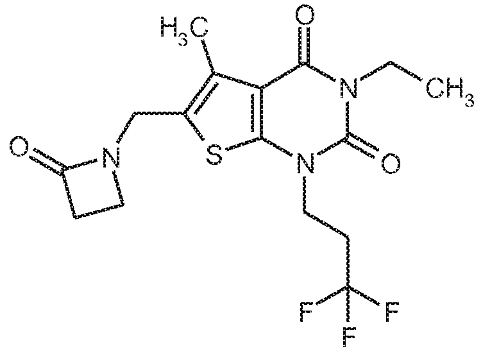

Eine Lösung von 85 mg (1.19 mmol) 2-Azetidinon in 1.6 ml THF wurde mit 48 mg (1.19 mmol) Natriumhydrid (60% Suspension in Mineralöl) versetzt, dann 60 min auf 60°C erwärmt und anschließend wieder auf RT abgekühlt ("Lösung 1"). In einem anderen Reaktionsgefäß wurde eine Lösung von 80 mg (0.238 mmol) der Verbindung aus Bsp. 140A in 1.6 ml Dichlormethan bei 0°C mit 83 μl (0.476 mmol) *N,N*-Diisopropylethylamin und 18 μl (0.250 mmol) Thionylchlorid versetzt. Nach 20 min bei 0°C wurde Lösung 1 hinzugefügt und das Kältebad entfernt. Das Reaktionsgemisch wurde ca. 18 h bei RT gerührt. Danach wurden alle flüchtigen Bestandteile am Rotationsverdampfer entfernt. Der verbliebene Rückstand wurde mittels präparativer HPLC (Methode 10) in seine Komponenten aufgetrennt. Nach Eindampfen der Produktfraktionen und Trocknen im Hochvakuum wurden 5 mg (5% d. Th., 92% Reinheit) der Titelverbindung erhalten.
$^1$H-NMR (500 MHz, DMSO-$d_6$, δ/ppm): 4.46 (s, 2H), 4.11 (t, 2H), 3.91 (q, 2H), 3.16 (t, 2H), 2.87 (t, 2H), 2.83-2.71 (m, 2H), 2.40 (s, 3H), 1.12 (t, 3H).
LC/MS (Methode 1, ESIpos): $R_t$ = 0.93 min, m/z = 390 $[M+H]^+$.

**Beispiel 3**

1-(2-Methoxyethyl)-5-methyl-6-[(2-oxopyrrolidin-1-yl)methyl]-3-(2-phenylethyl)thieno[2,3-d]-pyrimidin-2,4(1*H*,3*H*)-dion

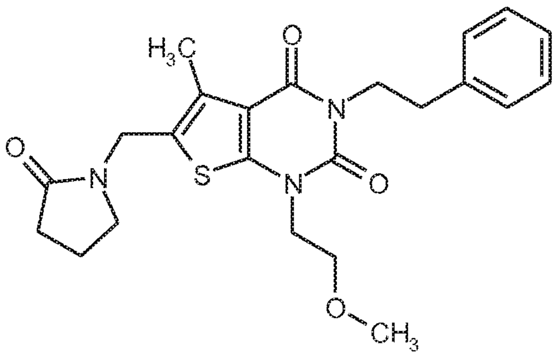

Analog zu dem unter Bsp. 1 beschriebenen Verfahren wurden aus 100 mg (0.267 mmol) der Verbindung aus Bsp. 138A und 113 mg (1.34 mmol) 2-Pyrrolidinon 55 mg (47% d. Th.) der Titelverbindung erhalten.
$^1$H-NMR (400 MHz, DMSO-$d_6$, δ/ppm): 7.35-7.27 (m, 2H), 7.26-7.17 (m, 3H), 4.48 (s, 2H), 4.12-4.03 (m, 2H), 4.01 (t, 2H), 3.60 (t, 2H), 3.29 (t, 2H, teilweise überdeckt vom Wassersignal), 3.24 (s, 3H), 2.88-2.78 (m, 2H), 2.41 (s, 3H), 2.25 (t, 2H), 1.95-1.87 (m, 2H).
LC/MS (Methode 1, ESIpos): $R_t$ = 1.03 min, m/z = 442 $[M+H]^+$.

**Beispiel 4**

3-Ethyl-5-methyl-6-[(2-oxopyrrolidin-1-yl)methyl]-1-(3,3,3-trifluorpropyl)thieno[2,3-d]pyrimidin-2,4(1*H*,3*H*)-dion

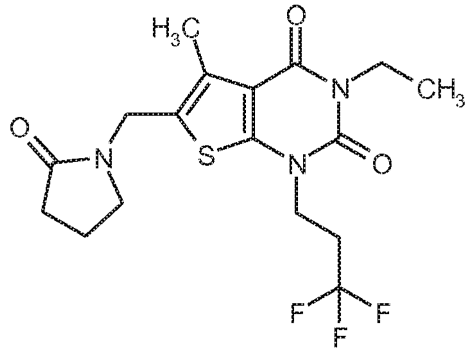

Analog zu dem unter Bsp. 2 beschriebenen Verfahren wurden aus 80 mg (0.238 mmol) der Verbindung aus Bsp. 140A und insgesamt 170 mg (2.00 mmol) 2-Pyrrolidinon 28 mg (29% d. Th.) der Titelverbindung erhalten. Abweichend zum zuvor beschriebenen Verfahren wurde die Lösung des deprotonierten 2-Pyrrolidinons hier in zwei Portionen zugesetzt: Der erste Teil wie zuvor beschrieben und der zweite Teil nach 18 h Reaktionzeit. Danach wurde das Reaktionsgemisch noch weitere 2 Tage bei RT gerührt.
$^1$H-NMR (400 MHz, DMSO-$d_6$, δ/ppm): 4.49 (s, 2H), 4.09 (t, 2H), 3.90 (q, 2H), 3.29 (t, 2H), 2.87-2.68 (m, 2H), 2.42 (s, 3H), 2.25 (t, 2H), 1.94-1.87 (m, 2H), 1.11 (t, 3H).
LC/MS (Methode 1, ESIpos): $R_t$ = 0.91 min, m/z = 404 $[M+H]^+$.

**Beispiel 5**

1-(2-Methoxyethyl)-6-[(2-oxopyrrolidin-1-yl)methyl]-3-(2-phenylethyl)-5-(trifluormethyl)thieno-[2,3-d]pyrimidin-2,4(1*H*,3*H*)-dion

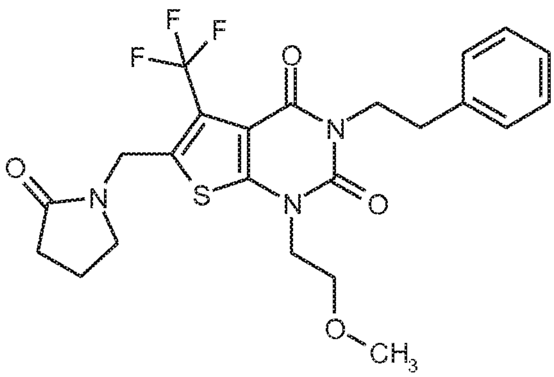

99 mg (1.16 mmol) 2-Pyrrolidinon wurden in 2 ml DMF gelöst, mit 45 mg (1.12 mmol) Natriumhydrid (60% Suspension in Mineralöl) versetzt und 25 min bei RT gerührt ("Mischung 1"). In einem anderen Reaktionsgefäß wurden 100 mg (0.224 mmol) der Verbindung aus Bsp. 181A in 1.5 ml DMF gelöst und bei 0°C tropfenweise mit 1 ml der Mischung 1 versetzt. Nach 30 min bei 0°C wurde das Reaktionsgemisch direkt mittels präparativer HPLC (Methode 9) in seine Komponenten aufgetrennt. Eindampfen der Produktfraktionen, Verrühren des Rückstands mit Pentan und Absaugen und Trocknen des Feststoffs im Hochvakuum ergaben 53 mg (47% d. Th.) der Titelverbindung.
$^1$H-NMR (400 MHz, DMSO-$d_6$, δ/ppm): 7.35-7.27 (m, 2H), 7.26-7.16 (m, 3H), 4.70 (s, 2H), 4.14-3.98 (m, 4H), 3.60 (t, 2H), 3.40 (t, 2H), 3.24 (s, 3H), 2.89-2.77 (m, 2H), 2.31 (t, 2H), 2.03-1.95 (m, 2H).
LC/MS (Methode 1, ESIpos): $R_t$ = 1.07 min, m/z = 496 $[M+H]^+$.

**Beispiel 6**

3-Ethyl-5-methyl-6-[(3-methyl-2-oxopyrrolidin-1-yl)methyl]-1-(3,3,3-trifluorpropyl)thieno[2,3-d]-pyrimidin-2,4(1*H*,3*H*)-dion (*Racemat*)

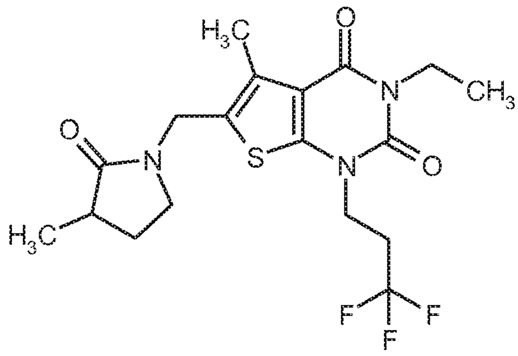

Analog zu dem unter Bsp. 2 beschriebenen Verfahren wurden aus 80 mg (0.238 mmol) der Verbindung aus Bsp. 140A und 122 mg (1.24 mmol) racemischem 3-Methylpyrrolidin-2-on 57 mg (56% d. Th.) der Titelverbindung erhalten. Abweichend vom zuvor beschriebenen Verfahren wurde das 3-Methylpyrrolidin-2-on hier 2.5 h bei 60°C mit Natriumhydrid deprotoniert.
$^1$H-NMR (400 MHz, DMSO-$d_6$, $\delta$/ppm): 4.50 (s, 2H), 4.09 (t, 2H), 3.90 (q, 2H), 3.30-3.14 (m, 2H), 2.85-2.68 (m, 2H), 2.41 (s, 3H), 2.40-2.30 (m, 1H), 2.22-2.11 (m, 1H), 1.51 (dq, 1H), 1.11 (t, 3H), 1.06 (d, 3H).
LC/MS (Methode 1, ESIpos): $R_t$ = 0.98 min, m/z = 418 $[M+H]^+$.

**Beispiel 7**

3-Ethyl-6-{[(3*R*)-3-hydroxy-2-oxopyrrolidin-1-yl]methyl}-1-(2-methoxyethyl)-5-methylthieno-[2,3-d]pyrimidin-2,4(1*H*,3*H*)-dion

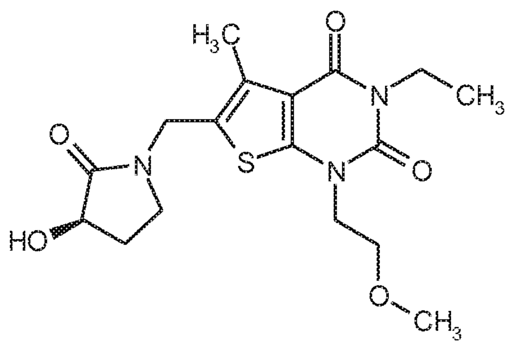

Eine Lösung von 106 mg (0.673 mmol) [(4*R*)-2,2-Dimethyl-5-oxo-1,3-dioxolan-4-yl]acetaldehyd [Int. Pat. Appl. WO 2012/037393-A1, Preparation B / Step B] in 6.7 ml 1,2-Dichlorethan wurde mit 200 mg (0.673 mmol) der Verbindung aus Bsp. 200A und 23 µl (0.404 mmol) Essigsäure versetzt. Nach 15 min wurden bei RT insgesamt 225 mg (1.01 mmol) Natriumtriacetoxyborhydrid in drei Portionen hinzugefügt. Nachdem das Reaktionsgemisch ca. 18 h bei RT gerührt worden war, wurde mit gesättigter Natriumhydrogencarbonat-Lösung versetzt und mit Dichlormethan extrahiert. Der organische Extrakt wurde mit gesättigter Natriumchlorid-Lösung gewaschen, über wasserfreiem Magnesiumsulfat getrocknet, filtriert und eingeengt. Der verbliebene Rückstand wurde mittels präparativer HPLC gereinigt (Methode 8). Nach Eindampfen der Produktfraktionen und Trocknen im Hochvakuum wurden 63 mg (24% d. Th., 98% Reinheit, >99% ee) der Titelverbindung erhalten.
$^1$H-NMR (500 MHz, DMSO-$d_6$, $\delta$/ppm): 5.59 (d, 1H), 4.48 (s, 2H), 4.10 (td, 1H), 4.01 (t, 2H), 3.90 (q, 2H), 3.63 (t, 2H), 3.27-3.20 (m, 1H), 3.23 (s, 3H), 3.17-3.09 (m, 1H), 2.40 (s, 3H), 2.28-2.21 (m, 1H), 1.70-1.63 (m, 1H), 1.11 (t, 3H).

LC/MS (Methode 1, ESIpos): $R_t$ = 0.67 min, m/z = 382 $[M+H]^+$.

Chirale analytische HPLC [Säule: Daicel Chiralpak AS-3, 3 μm, 50 mm x 4.6 mm; Laufmittel: Isohexan/Ethanol 1:1; Fluss: 1 ml/min; Temperatur: 30°C; Detektion: 220 nm]: $R_t$ = 1.23 min [(S)-Enantiomer unter gleichen Bedingungen: $R_t$ = 2.23 min].

**Beispiel 8**

*tert.*-Butyl-(1-{[3-ethyl-5-methyl-2,4-dioxo-1-(3,3,3-trifluorpropyl)-1,2,3,4-tetrahydrothieno-[2,3-d]pyrimidin-6-yl]methyl}-5-oxopyrrolidin-3-yl)carbamat (*Racemat*)

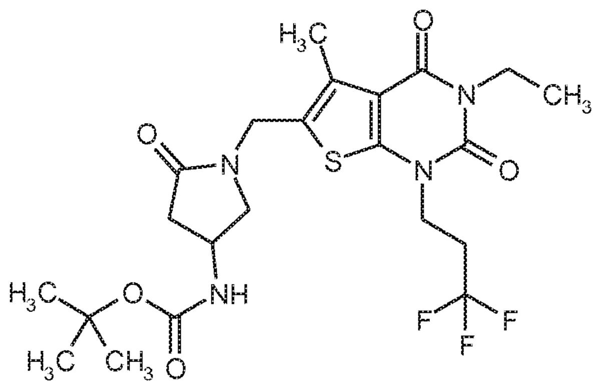

*Teilschritt 1, Darstellung von racemischem tert.-Butyl-(5-oxopyrrolidin-3-yl)carbamat:* 100 mg (0.732 mmol) racemisches 4-Aminopyrrolidin-2-on-Hydrochlorid wurde in einem Gemisch aus 1.2 ml Wasser und 3.7 ml 1,4-Dioxan gelöst und nacheinander mit 185 mg (2.20 mmol) Natriumhydrogencarbonat und 168 mg (0.769 mmol) Di-*tert.*-butyldicarbonat versetzt. Nachdem das Reaktionsgemisch ca. 18 h bei RT gerührt worden war, wurde mit Wasser verdünnt und mit Ethylacetat extrahiert. Der vereinigte organische Extrakt wurde über wasserfreiem Natriumsulfat getrocknet, filtriert und eingeengt. Nach Trocknen im Hochvakuum wurden 121 mg (82% d. Th.) des racemischen *tert.*-Butyl-(5-oxopyrrolidin-3-yl)carbamats erhalten. $^1H$-NMR (400 MHz, DMSO-$d_6$, δ/ ppm): 7.56 (br. s, 1H), 7.25 (br. d, 1H), 4.24-3.97 (m, 1H), 3.43 (dd, 1H), 2.99 (dd, 1H), 2.36 (dd, 1H), 2.04 (dd, 1H), 1.38 (s, 9H). LC/MS (Methode 1, ESIpos): $R_t$ = 0.50 min, m/z = 201 $[M+H]^+$.

*Teilschritt 2, Darstellung der Titelverbindung:* Analog zu dem unter Bsp. 2 beschriebenen Verfahren wurden aus 70 mg (0.208 mmol) der Verbindung aus Bsp. 140A und 167 mg (0.833 mmol) racemischem *tert.*-Butyl-(5-oxopyrrolidin-3-yl)carbamat (siehe Teilschritt 1) 42 mg (38% d. Th.) der Titelverbindung erhalten. Abweichend vom zuvor beschriebenen Verfahren wurde das *tert.*-Butyl-(5-oxopyrrolidin-3-yl)carbamat hier 2.5 h bei 60°C mit Natriumhydrid deprotoniert.

$^1H$-NMR (400 MHz, DMSO-$d_6$, δ/ppm): 7.26 (br. d, 1H), 4.49 (s, 2H), 4.10 (t, 2H), 4.08-3.97 (m, 1H), 3.90 (q, 2H), 3.51 (dd, 1H), 3.09 (dd, 1H), 2.87-2.68 (m, 2H), 2.40 (s, 3H), 2.21 (dd, 1H), 1.35 (s, 9H), 1.11 (t, 3H).

LC/MS (Methode 1, ESIpos): $R_t$ = 1.04 min, m/z = 519 $[M+H]^+$.

**Beispiel 9**

6-[(4-Amino-2-oxopyrrolidin-1-yl)methyl]-3-ethyl-5-methyl-1-(3,3,3-trifluorpropyl)thieno[2,3-d]-pyrimidin-2,4(1*H*,3*H*)-dion (*Racemat*)

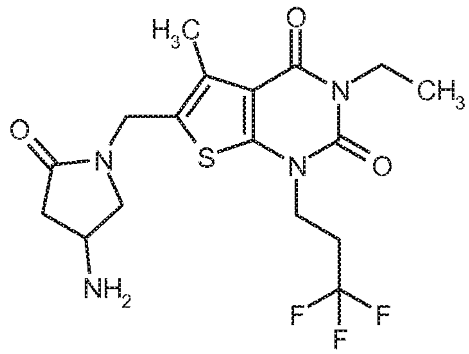

38 mg (0.073 mmol) der Verbindung aus Bsp. 8 wurden in ca. 5 ml Dichlormethan/Trifluoressigsäure (3:1) gelöst und 45 min bei RT gerührt. Anschließend wurden alle flüchtigen Bestandteile am Rotationsverdampfer entfernt. Der verbliebene Rückstand wurde mit Ethylacetat versetzt und mit gesättigter wässriger Natriumhydrogencarbonat-Lösung ausgeschüttelt. Nach Trocknen der organischen Phase über wasserfreiem Natriumsulfat wurde filtriert und eingeengt. Der erhaltene Rückstand wurde im Hochvakuum getrocknet und ergab 30 mg (94% d. Th., 97% Reinheit) der Titelverbindung.

$^1$H-NMR (500 MHz, DMSO-$d_6$, $\delta$/ppm): 4.48 (d, 2H), 4.09 (t, 2H), 3.90 (q, 2H), 3.53-3.45 (m, 1H), 3.42 (dd, 1H), 2.89 (dd, 1H), 2.83-2.67 (m, 2H), 2.45 (dd, 1H), 2.41 (s, 3H), 1.96 (dd, 1H), 1.72 (breit, 2H), 1.12 (t, 3H).

LC/MS (Methode 1, ESIpos): $R_t$ = 0.60 min, m/z = 419 $[M+H]^+$.

**Beispiel 10**

3-Ethyl-5-methyl-6-[(2-methyl-5-oxopyrrolidin-1-yl)methyl]-1-(3,3,3-trifluorpropyl)thieno[2,3-d]-pyrimidin-2,4(1*H*,3*H*)-dion (*Racemat*)

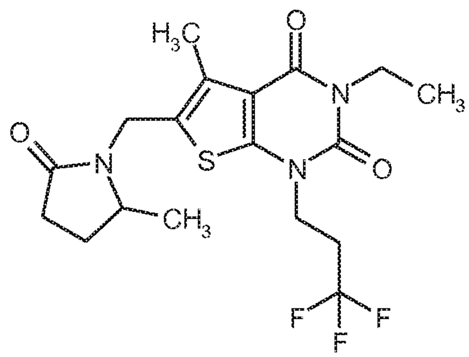

Analog zu dem unter Bsp. 2 beschriebenen Verfahren wurden aus 80 mg (0.238 mmol) der Verbindung aus Bsp. 140A und 296 mg (2.85 mmol) racemischem 5-Methylpyrrolidin-2-on 10 mg (10% d. Th.) der Titelverbindung erhalten. Abweichend vom zuvor beschriebenen Verfahren wurde das deprotonierte 5-Methylpyrrolidin-2-on hier in drei Portionen über einen Zeitraum von ca. 42 h dem Reaktionsgemisch zugesetzt. Insgesamt betrug die Reaktionszeit 4.5 Tage.

$^1$H-NMR (400 MHz, DMSO-$d_6$, $\delta$/ppm): 4.69 (d, 1H), 4.29 (d, 1H), 4.08 (td, 2H), 3.90 (q, 2H), 3.59-3.52 (m, 1H), 2.84-2.68 (m, 2H), 2.42 (s, 3H), 2.39-2.28 (m, 1H), 2.27-2.16 (m, 1H), 2.15-2.03 (m, 1H), 1.59-1.44 (m, 1H), 1.16 (d, 3H), 1.11 (t, 3H).

LC/MS (Methode 1, ESIpos): $R_t$ = 0.97 min, m/z = 418 $[M+H]^+$.

**Beispiel 11**

3-Ethyl-5-methyl-6-[(1-oxo-1,3-dihydro-2*H*-isoindol-2-yl)methyl]-1-(3,3,3-trifluorpropyl)thieno-[2,3-d]pyrimidin-2,4(1*H*,3*H*)-dion

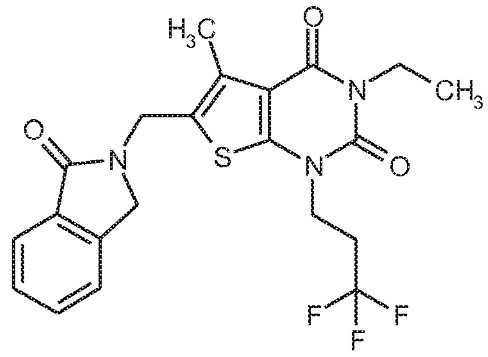

Analog zu dem unter Bsp. 2 beschriebenen Verfahren wurden aus 80 mg (0.238 mmol) der Verbindung aus Bsp. 140A und 253 mg (1.90 mmol) Isoindolin-1-on 22 mg (20% d. Th.) der Titelverbindung erhalten. Abweichend vom zuvor beschriebenen Verfahren wurde das deprotonierte Isoindolin-1-on hier in zwei Portionen über einen Zeitraum von ca. 18 h dem Reaktionsgemisch zugesetzt. Insgesamt betrug die Reaktionszeit 1.5 Tage.
$^1$H-NMR (400 MHz, DMSO-$d_6$, δ/ppm): 7.72 (d, 1H), 7.65-7.55 (m, 2H), 7.53-7.45 (m, 1H), 4.88 (s, 2H), 4.45 (s, 2H), 4.08 (t, 2H), 3.90 (q, 2H), 2.83-2.63 (m, 2H), 1.11 (t, 3H).
LC/MS (Methode 1, ESIpos): $R_t$ = 1.07 min, m/z = 452 $[M+H]^+$.

## Beispiel 12

3-Ethyl-5-methyl-6-[(2-oxopiperidin-1-yl)methyl]-1-(3,3,3-trifluorpropyl)thieno[2,3-d]pyrimidin-2,4(1*H*,3*H*)-dion

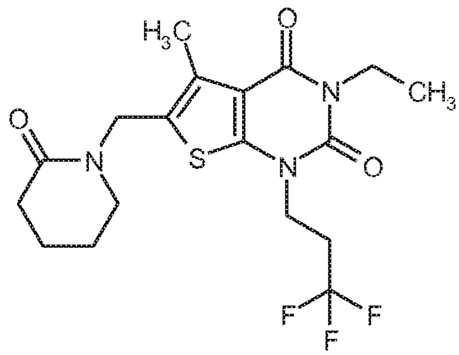

Analog zu dem unter Bsp. 2 beschriebenen Verfahren wurden aus 50 mg (0.149 mmol) der Verbindung aus Bsp. 140A und 124 mg (1.25 mmol) Piperidin-2-on 34 mg (54% d. Th.) der Titelverbindung erhalten. Abweichend vom zuvor beschriebenen Verfahren wurde das deprotonierte Piperidin-2-on hier in zwei Portionen über einen Zeitraum von ca. 18 h dem Reaktionsgemisch zugesetzt. Insgesamt betrug die Reaktionszeit 24 h.
$^1$H-NMR (400 MHz, DMSO-$d_6$, δ/ppm): 4.58 (s, 2H), 4.08 (t, 2H), 3.90 (q, 2H), 3.26 (t, 2H), 2.88-2.68 (m, 2H), 2.44 (s, 3H), 2.26 (t, 2H), 1.78-1.59 (m, 4H), 1.11 (t, 3H).
LC/MS (Methode 1, ESIpos): $R_t$ = 0.96 min, m/z = 418 $[M+H]^+$.

## Beispiel 13

1-(2-Methoxyethyl)-5-methyl-6-[(3-oxomorpholin-4-yl)methyl]-3-(2-phenylethyl)thieno[2,3-d]-pyrimidin-2,4(1*H*,3*H*)-dion

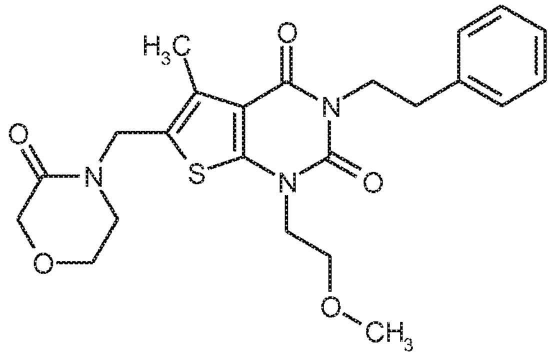

Eine Lösung von 80 mg (0.214 mmol) der Verbindung aus Bsp. 138A in 1.3 ml Dichlormethan wurde bei 0°C mit 74 µl (0.427 mmol) *N,N*-Diisopropylethylamin und 16 µl (0.224 mmol) Thionylchlorid versetzt. Nach 20 min Rühren bei 0°C wurde dann bei RT eine Lösung von 65 mg (0.641 mmol) Morpholin-3-on in 2 ml THF, die zuvor mit 26 mg (0.641 mmol) Natriumhydrid (60% Suspension in Mineralöl) versetzt worden war, hinzugefügt. Das Reaktionsgemisch wurde ca. 18 h bei RT gerührt. Dann wurde noch einmal die gleiche Menge an deprotoniertem Morpholin-3-on hinzugefügt. Nach weiteren ca. 2 h Rühren wurden alle flüchtigen Bestandteile am Rotationsverdampfer entfernt. Der verbliebene Rückstand wurde mittels präparativer HPLC (Methode 8) in seine Komponenten aufgetrennt. Nach Eindampfen der Produktfraktionen und Trocknen im Hochvakuum wurden 13 mg (14% d. Th.) der Titelverbindung erhalten.
$^1$H-NMR (400 MHz, $CDCl_3$, δ/ppm): 7.38-7.16 (m, 5H, teilweise überdeckt vom $CHCl_3$-Signal), 4.69 (s, 2H), 4.22 (s, 2H), 4.28-4.16 (m, 2H), 4.10 (t, 2H), 3.87 (t, 2H), 3.69 (t, 2H), 3.37 (t, 2H), 3.34 (s, 3H), 3.00-2.88 (m, 2H), 2.53 (s, 3H).
LC/MS (Methode 1, ESIpos): $R_t$ = 1.01 min, m/z = 458 $[M+H]^+$.

**Beispiel 14**

3-Ethyl-5-methyl-6-[(3-oxomorpholin-4-yl)methyl]-1-(3,3,3-trifluorpropyl)thieno[2,3-d]pyrimidin-2,4(1*H*,3*H*)-dion

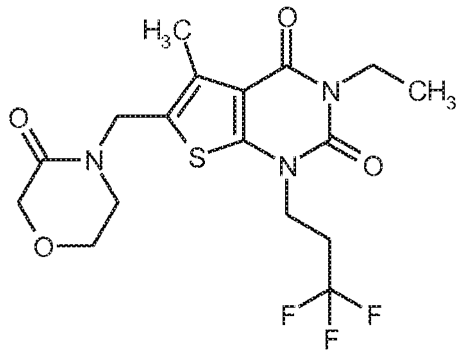

Eine Lösung von 80 mg (0.238 mmol) der Verbindung aus Bsp. 140A in 1.6 ml Dichlormethan wurde bei 0°C mit 83 µl (0.476 mmol) *N,N*-Diisopropylethylamin und 18 µl (0.250 mmol) Thionylchlorid versetzt. Nach 20 min Rühren bei 0°C wurde eine Lösung von 72 mg (0.714 mmol) Morpholin-3-on in 1.6 ml THF, die zuvor mit 29 mg (0.714 mmol) Natriumhydrid (60% Suspension in Mineralöl) versetzt und 30 min bei RT gerührt worden war, hinzugefügt. Das Reaktionsgemisch wurde ca. 18 h bei RT gerührt. Da der Umsatz unvollständig war, wurden noch einmal 72 mg (0.714 mmol) Morpholin-3-on in 1.6 ml THF gelöst und mit 550 µl (1.10 mmol) einer 2 M Lösung von Lithiumdiisopropylamid (LDA) in THF versetzt. Nach 10 min wurde diese Lösung dem Reaktionsgemisch hinzugefügt. Danach wurde zunächst 30 min bei RT, dann 5 h bei 80°C und abschließend 2 Tage bei RT gerührt. Danach wurden alle flüchtigen Bestandteile am Rotationsverdampfer entfernt. Der verbliebene Rückstand wurde mittels präparativer HPLC (Methode 10) in seine Komponenten aufgetrennt. Nach Eindampfen der Produktfraktionen und Trocknen im Hochvakuum wurden 47 mg (47% d. Th., 96% Reinheit) der Titelverbindung erhalten.
$^1$H-NMR (400 MHz, DMSO-$d_6$, δ/ppm): 4.66 (s, 2H), 4.09 (t, 2H), 4.08 (s, 2H), 3.90 (q, 2H), 3.80 (t, 2H), 2.85-2.69 (m, 2H), 2.45 (s, 3H), 1.11 (t, 3H).
LC/MS (Methode 1, ESIpos): $R_t$ = 0.88 min, m/z = 420 $[M+H]^+$.

**Beispiel 15**

6-[(1,1-Dioxido-1,2-thiazolidin-2-yl)methyl]-1-(2-methoxyethyl)-5-methyl-3-(2-phenylethyl)-thieno[2,3-d]pyrimidin-2,4(1*H*-3*H*)-dion

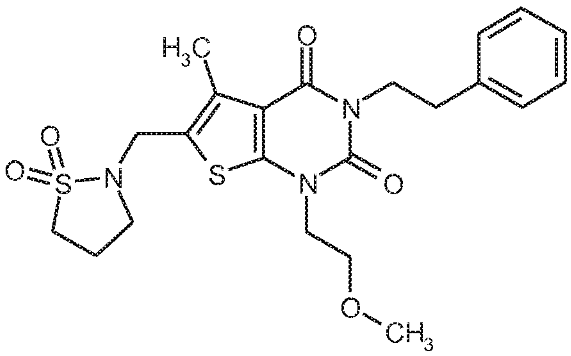

Eine Lösung von 49 µl (0.668 mmol) Propansultam in 1.5 ml DMF wurde mit 27 mg (0.668 mmol) Natriumhydrid (60% Suspension in Mineralöl) versetzt und anschließend 25 min bei RT gerührt ("Lösung 1"). In einem anderen Reaktionsgefäß wurde eine Lösung von 50 mg (0.134 mmol) der Verbindung aus Bsp. 138A in 1.3 ml Dichlormethan bei 0°C mit 47 µl (0.267 mmol) *N,N*-Diisopropylethylamin und 10 µl (0.140 mmol) Thionylchlorid versetzt. Nach 20 min bei 0°C wurde ein Drittel von Lösung 1 hinzugefügt. Das Reaktionsgemisch wurde bei 0°C gerührt, wobei nach 20 min und nach 40 min Reaktionszeit jeweils ein weiteres Drittel der Lösung 1 hinzugefügt wurden. Nach der letzten Zugabe wurde das Kältebad entfernt und das Reaktionsgemisch ca. 18 h bei RT gerührt. Danach wurden alle flüchtigen Bestandteile am Rotationsverdampfer entfernt. Der verbliebene Rückstand wurde mittels präparativer HPLC (Methode 8) in seine Komponenten aufgetrennt. Nach Eindampfen der Produktfraktionen und Trocknen im Hochvakuum wurden 41 mg (64% d. Th.) der Titelverbindung erhalten.
$^1$H-NMR (400 MHz, $CDCl_3$, δ/ppm): 7.41-7.16 (m, 5H, teilweise überdeckt vom $CHCl_3$-Signal), 4.30 (s, 2H), 4.25-4.17 (m, 2H), 4.11 (t, 2H), 3.70 (t, 2H), 3.35 (s, 3H), 3.26-3.15 (m, 4H), 2.99-2.88 (m, 2H), 2.50 (s, 3H), 2.44-2.28 (m, 2H).
LC/MS (Methode 1, ESIpos): $R_t$ = 1.03 min, m/z = 478 $[M+H]^+$.

**Beispiel 16**

6-[(1,1-Dioxido-1,2-thiazolidin-2-yl)methyl]-3-ethyl-5-methyl-1-(3,3,3-trifluorpropyl)thieno-[2,3-d]pyrimidin-2,4(1*H*,3*H*)-dion

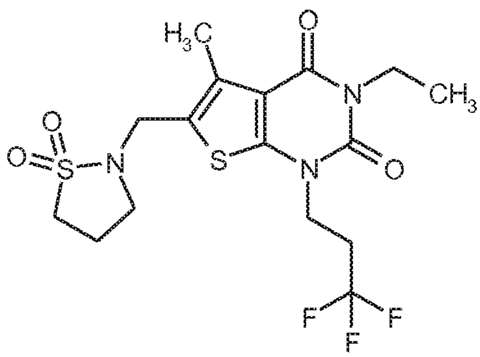

Analog zu dem unter Bsp. 2 beschriebenen Verfahren wurden aus 90 mg (0.268 mmol) der Verbindung aus Bsp. 140A und 130 mg (1.07 mmol) Propansultam 63 mg (54% d. Th.) der Titelverbindung erhalten. Abweichend vom zuvor beschriebenen Verfahren wurde das Propansultam hier 2 h bei 60°C mit Natriumhydrid deprotoniert.
$^1$H-NMR (400 MHz, DMSO-$d_6$, δ/ppm): 4.26 (s, 2H), 4.12 (t, 2H), 3.90 (q, 2H), 3.29-3.21 (m, 2H), 3.17 (t, 2H), 2.86-2.69

(m, 2H), 2.41 (s, 3H), 2.29-2.16 (m, 2H), 1.12 (t, 3H).
LC/MS (Methode 1, ESIpos): $R_t$ = 0.96 min, m/z = 440 $[M+H]^+$.

**Beispiel 17**

6-[(1,1-Dioxido-1,2-thiazolidin-2-yl)methyl]-3-ethyl-1-(2-methoxyethyl)-5-methylthieno[2,3-d]-pyrimidin-2,4(1*H*,3*H*)-dion

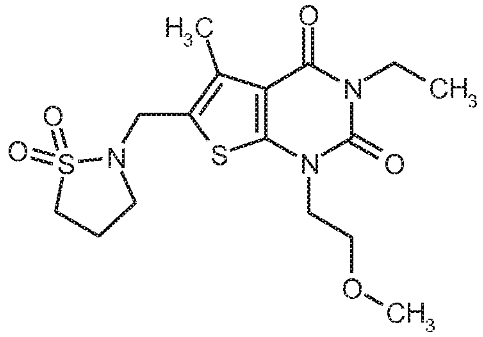

Analog zu dem unter Bsp. 2 beschriebenen Verfahren wurden aus 90 mg (0.287 mmol, 95% Reinheit) der Verbindung aus Bsp. 143A und 139 mg (1.15 mmol) Propansultam 48 mg (41% d. Th.) der Titelverbindung erhalten. Abweichend vom zuvor beschriebenen Verfahren wurde das Propansultam hier 2 h bei 60°C mit Natriumhydrid deprotoniert. Die präparative HPLC-Reinigung erfolgte nach Methode 8.
$^1$H-NMR (400 MHz, DMSO-$d_6$, $\delta$/ppm): 4.24 (s, 2H), 4.03 (t, 2H), 3.90 (q, 2H), 3.64 (t, 2H), 3.27-3.20 (m, 2H), 3.24 (s, 3H), 3.17 (t, 2H), 2.39 (s, 3H), 2.27-2.14 (m, 2H), 1.12 (t, 3H).
LC/MS (Methode 1, ESIpos): $R_t$ = 0.82 min, m/z = 402 $[M+H]^+$.

**Beispiel 18**

6-[(1,1-Dioxido-1,2-thiazolidin-2-yl)methyl]-1-(2-methoxyethyl)-3-(2-phenylethyl)-5-(trifluormethyl)thieno[2,3-d]pyrimidin-2,4(1*H*,3*H*)-dion

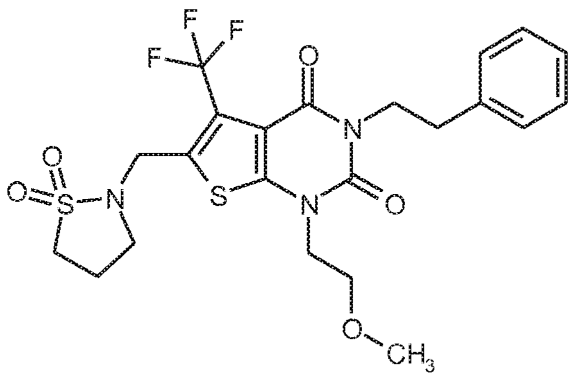

Analog zu dem unter Bsp. 5 beschriebenen Verfahren wurden aus 100 mg (0.224 mmol) der Verbindung aus Bsp. 181A und 141 mg (1.16 mmol) Propansultam 102 mg (85% d. Th.) der Titelverbindung erhalten. Auf das abschließende Verrühren mit Pentan konnte hier verzichtet werden.
$^1$H-NMR (400 MHz, DMSO-$d_6$, $\delta$/ppm): 7.37-7.27 (m, 2H), 7.25-7.20 (m, 3H), 4.54 (d, 2H), 4.16-3.99 (m, 4H), 3.62 (t, 2H), 3.37 (t, 2H), 3.33 (s, 3H), 3.31 (t, 3H), 2.91-2.78 (m, 2H), 2.37-2.22 (m, 2H).
LC/MS (Methode 1, ESIpos): $R_t$ = 1.07 min, m/z = 532 $[M+H]^+$.

**Beispiel 19**

6-[(1,1-Dioxido-1,2-thiazolidin-2-yl)methyl]-3-ethyl-5-(trifluormethyl)-1-(3,3,3-trifluorpropyl)-thieno[2,3-d]pyrimidin-2,4(1*H*,3*H*)-dion

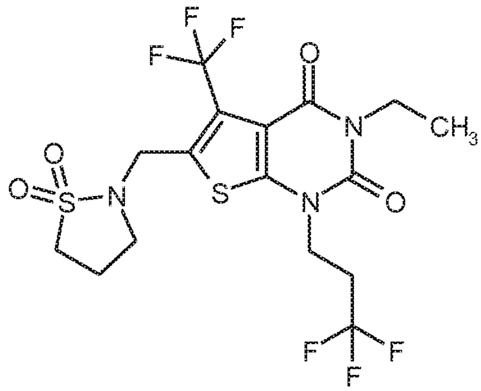

74 mg (0.612 mmol) Propansultam wurden in 1.5 ml DMF gelöst, mit 24 mg (0.612 mmol) Natriumhydrid (60% Suspension in Mineralöl) versetzt und 25 min bei RT gerührt. Anschließend wurde mit 0.25 ml THF verdünnt ("Mischung 1"). In einem anderen Reaktionsgefäß wurden 50 mg (0.122 mmol) der Verbindung aus Bsp. 182A in 1 ml DMF gelöst und bei 0°C tropfenweise mit 350 μl der Mischung 1 versetzt. Nach 2.5 h bei 0°C wurden weitere 350 μl der Mischung 1 hinzugefügt. Nach weiteren 30 min bei 0°C wurde das Reaktionsgemisch direkt mittels präparativer HPLC in seine Komponenten aufgetrennt (Methode 8). Eindampfen der Produktfraktionen und Trocknen im Hochvakuum ergaben 42 mg (69% d. Th.) der Titelverbindung.
$^1$H-NMR (400 MHz, $CDCl_3$, δ/ppm): 4.57 (d, 2H), 4.23-4.14 (m, 2H), 4.08 (q, 2H), 3.40 (t, 2H), 3.31-3.19 (m, 2H), 2.74-2.57 (m, 2H), 2.53-2.38 (m, 2H), 1.26 (t, 3H).
LC/MS (Methode 1, ESIpos): $R_t$ = 0.98 min, m/z = 494 $[M+H]^+$.

**Beispiel 20**

1-(2-Methoxyethyl)-5-methyl-6-[(2-oxo-1,3-oxazolidin-3-yl)methyl]-3-(2-phenylethyl)thieno-[2,3-d]pyrimidin-2,4(1*H*,3*H*)-dion

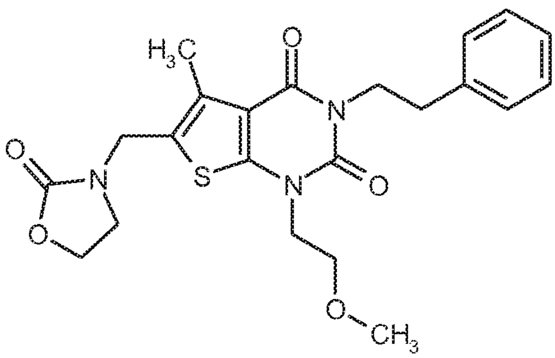

Eine Lösung von 80 mg (0.214 mmol) der Verbindung aus Bsp. 138A in 1.3 ml Dichlormethan wurde bei 0°C mit 74 μl (0.427 mmol) *N,N*-Diisopropylethylamin und 16 μl (0.224 mmol) Thionylchlorid versetzt. Nach 20 min Rühren bei 0°C wurde eine Lösung von 56 mg (0.641 mmol) Oxazolidin-2-on in 1.6 ml THF, die zuvor mit 26 mg (0.641 mmol) Natriumhydrid (60% Suspension in Mineralöl) versetzt und 30 min bei RT gerührt worden war, hinzugefügt. Anschließend wurde das Kältebad entfernt, und das Reaktionsgemisch wurde ca. 18 h bei RT gerührt. Da der Umsatz unvollständig war, wurde noch einmal die gleiche Menge an deprotoniertem Oxazolidin-2-on zugesetzt. Nach 2 h bei RT wurden dann

alle flüchtigen Bestandteile am Rotationsverdampfer entfernt. Der verbliebene Rückstand wurde mittels präparativer HPLC (Methode 8) in seine Komponenten aufgetrennt. Nach Eindampfen der Produktfraktionen und Trocknen im Hochvakuum wurden 44 mg (47% d. Th.) der Titelverbindung erhalten.
$^1$H-NMR (400 MHz, DMSO-$d_6$, δ/ppm): 7.36-7.27 (m, 2H), 7.26-7.18 (m, 3H), 4.49 (s, 2H), 4.31-4.21 (m, 2H), 4.13-3.98 (m, 4H), 3.61 (t, 2H), 3.53-3.44 (m, 2H), 3.24 (s, 3H), 2.89-2.78 (m, 2H), 2.42 (s, 3H).
LC/MS (Methode 1, ESIpos): $R_t$ = 1.02 min, m/z = 444 $[M+H]^+$.

**Beispiel 21**

3-Ethyl-5-methyl-6-[(2-oxo-1,3-oxazolidin-3-yl)methyl]-1-(3,3,3-trifluorpropyl)thieno[2,3-d]-pyrimidin-2,4(1*H*,3*H*)-dion

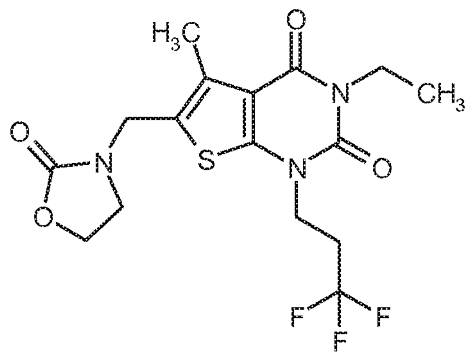

Analog zu dem unter Bsp. 2 beschriebenen Verfahren wurden aus 80 mg (0.238 mmol) der Verbindung aus Bsp. 140A und 108 mg (1.24 mmol) Oxazolidin-2-on 46 mg (48% d. Th.) der Titelverbindung erhalten.
$^1$H-NMR (400 MHz, DMSO-$d_6$, δ/ppm): 4.51 (s, 2H), 4.31-4.21 (m, 2H), 4.11 (t, 2H), 3.90 (q, 2H), 3.53-3.44 (m, 2H), 2.89-2.69 (m, 2H), 2.42 (s, 3H), 1.12 (t, 3H).
LC/MS (Methode 1, ESIpos): $R_t$ = 0.90 min, m/z = 406 $[M+H]^+$.

**Beispiel 22**

1-(2-Methoxyethyl)-6-[(2-oxo-1,3-oxazolidin-3-yl)methyl]-3-(2-phenylethyl)-5-(trifluormethyl)-thieno[2,3-d]pyrimidin-2,4(1*H*,3*H*)-dion

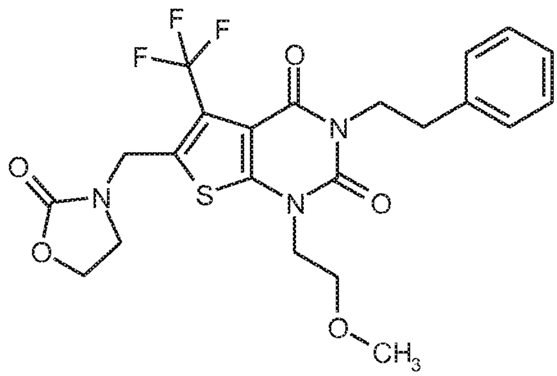

Analog zu dem unter Bsp. 5 beschriebenen Verfahren wurden aus 100 mg (0.224 mmol) der Verbindung aus Bsp. 181A und 101 mg (1.16 mmol) Oxazolidin-2-on 82 mg (72% d. Th.) der Titelverbindung erhalten. Auf das abschließende Verrühren mit Pentan wurde hier verzichtet.
$^1$H-NMR (400 MHz, DMSO-$d_6$, δ/ppm): 7.36-7.27 (m, 2H), 7.26-7.17 (m, 3H), 4.71 (d, 2H), 4.35 (t, 2H), 4.13-4.00 (m, 4H), 3.66-3.53 (m, 4H), 3.24 (s, 3H), 2.91-2.78 (m, 2H).
LC/MS (Methode 1, ESIpos): $R_t$ = 1.06 min, m/z = 498 $[M+H]^+$.

**Beispiel 23**

3-Ethyl-6-[(2-oxo-1,3-oxazolidin-3-yl)methyl]-5-(trifluormethyl)-1-(3,3,3-trifluorpropyl)thieno-[2,3-d]pyrimidin-2,4(1*H*,3*H*)-dion

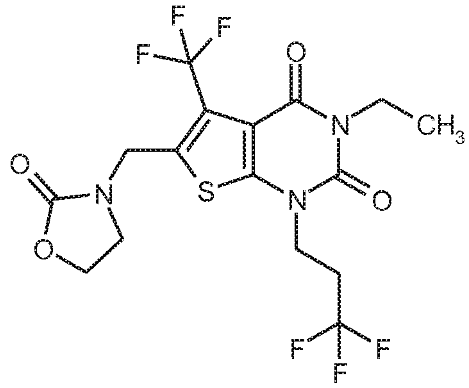

Analog zu dem unter Bsp. 19 beschriebenen Verfahren wurden aus 70 mg (0.171 mmol) der Verbindung aus Bsp. 182A und 74 mg (0.856 mmol) Oxazolidin-2-on 61 mg (77% d. Th.) der Titelverbindung erhalten.
$^1$H-NMR (400 MHz, $CDCl_3$, δ/ppm): 4.77 (d, 2H), 4.47-4.35 (m, 2H), 4.23-4.14 (m, 2H), 4.08 (q, 2H), 3.70-3.55 (m, 2H), 2.76-2.54 (m, 2H), 1.26 (t, 3H).
LC/MS (Methode 1, ESIpos): $R_t$ = 0.98 min, m/z = 460 $[M+H]^+$.

**Beispiel 24**

6-[(4,4-Dimethyl-2-oxo-1,3-oxazolidin-3-yl)methyl]-3-ethyl-5-methyl-1-(3,3,3-trifluorpropyl)-thieno[2,3-d]pyrimidin-2,4(1*H*,3*H*)-dion

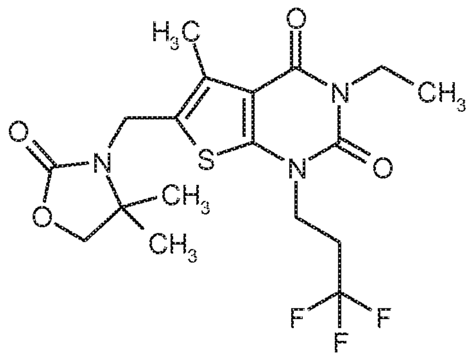

Analog zu dem unter Bsp. 2 beschriebenen Verfahren wurden aus 80 mg (0.238 mmol) der Verbindung aus Bsp. 140A und 110 mg (0.951 mmol) 4,4-Dimethyl-1,3-oxazolidin-2-on 27 mg (26% d. Th.) der Titelverbindung erhalten.
$^1$H-NMR (400 MHz, DMSO-$d_6$, δ/ppm): 4.45 (s, 2H), 4.10 (t, 2H), 4.02 (s, 2H), 3.90 (q, 2H), 2.86-2.69 (m, 2H), 2.45 (s, 3H), 1.23 (s, 6H), 1.12 (t, 3H).
LC/MS (Methode 1, ESIpos): $R_t$ = 1.02 min, m/z = 434 $[M+H]^+$.

**Beispiel 25**

3-Ethyl-5-methyl-6-[(2-oxo-1,3-oxazinan-3-y1)methyl]-1-(3,3,3-trifluorpropyl)thieno[2,3-d]-pyrimidin-2,4(1*H*,3*H*)-dion

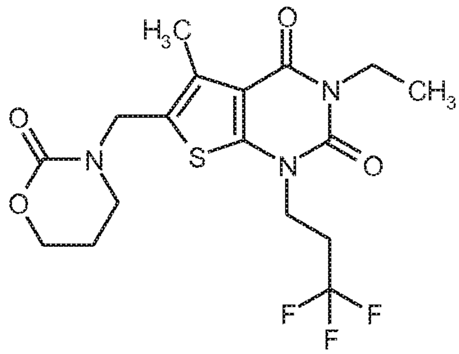

Analog zu dem unter Bsp. 2 beschriebenen Verfahren wurden aus 80 mg (0.238 mmol) der Verbindung aus Bsp. 140A und insgesamt 168 mg (1.66 mmol) 1,3-Oxazin-2-on 21 mg (20% d. Th., 97% Reinheit) der Titelverbindung erhalten. Abweichend vom zuvor beschriebenen Verfahren erfolgte die Zugabe des deprotonierten 1,3-Oxazin-2-ons hier in zwei Portionen im Abstand von ca. 20 h. Insgesamt betrug die Reaktionszeit 4 Tage.
$^1$H-NMR (400 MHz, DMSO-$d_6$, δ/ppm): 4.56 (s, 2H), 4.21-4.14 (m, 2H), 4.10 (t, 2H), 3.90 (q, 2H), 3.28 (t, 2H), 2.87-2.69 (m, 2H), 2.44 (s, 3H), 1.91 (quin, 2H), 1.12 (t, 3H).
LC/MS (Methode 1, ESIpos): $R_t$ = 0.89 min, m/z = 420 $[M+H]^+$.

## Beispiel 26

*tert.*-Butyl-5-{[3-ethyl-5-methyl-2,4-dioxo-1-(3,3,3-trifluorpropyl)-1,2,3,4-tetrahydrothieno[2,3-d]-pyrimidin-6-yl]methyl}-1,2,5-thiadiazolidin-2-carboxylat-1,1-dioxid

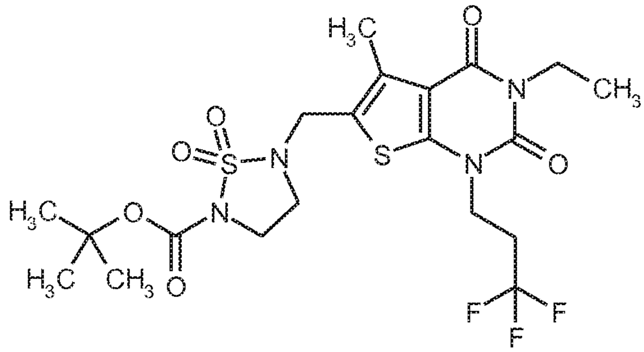

Analog zu dem unter Bsp. 2 beschriebenen Verfahren wurden aus 100 mg (0.282 mmol, 95% Reinheit) der Verbindung aus Bsp. 140A und 188 mg (0.847 mmol) *tert.*-Butyl-1,2,5-thiadiazolidin-2-carboxylat-1,1-dioxid [S. J. Kim et al., Eur. J. Med. Chem. 2007, 42 (9), 1176-1183] 22 mg (14% d. Th.) der Titelverbindung erhalten. Die präparative HPLC-Reinigung erfolgte hier nach Methode 8.
$^1$H-NMR (400 MHz, $CDCl_3$, δ/ppm): 4.32 (s, 2H), 4.21-4.12 (m, 2H), 4.06 (q, 2H), 3.79 (t, 2H), 3.32 (t, 2H), 2.72-2.56 (m, 2H), 2.52 (s, 3H), 1.56 (s, 9H), 1.25 (t, 3H).
LC/MS (Methode 1, ESIpos): $R_t$ = 1.17 min, m/z = 319 $[M+H-C_7H_{14}N_2O_4S]^+$.

## Beispiel 27

6-[(1,1-Dioxido-1,2,5-thiadiazolidin-2-yl)methyl]-3-ethyl-5-methyl-1-(3,3,3-trifluorpropyl)thieno-[2,3-d]pyrimidin-2,4(1*H*,3*H*)-dion

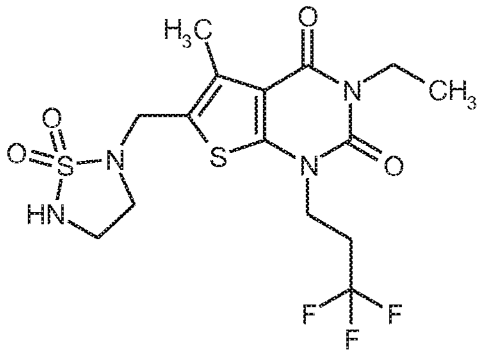

18 mg (0.033 mmol) der Verbindung aus Bsp. 26 wurden in 1.5 ml Dichlormethan gelöst und mit 1 ml Trifluoressigsäure versetzt. Nach 1 h wurden alle flüchtigen Bestandteile am Rotationsverdampfer entfernt und der verbliebene Rückstand mittels präparativer HPLC (Methode 8) in seine Komponenten aufgetrennt. Nach Eindampfen der Produktfraktionen und Trocknen im Hochvakuum wurden 5.5 mg (37% d. Th.) der Titelverbindung erhalten.
$^1$H-NMR (400 MHz, $CDCl_3$, δ/ppm): 4.38 (t, 1H), 4.30 (s, 2H), 4.21-4.12 (m, 2H), 4.06 (q, 2H), 3.53 (q, 2H), 3.43-3.33 (m, 2H), 2.73-2.54 (m, 2H), 2.51 (s, 3H), 1.25 (t, 3H).
LC/MS (Methode 1, ESIpos): $R_t$ = 0.91 min, m/z = 441 $[M+H]^+$.

**Beispiel 28**

3-Ethyl-5-methyl-6-[(2-thioxoimidazolidin-1-yl)methyl]-1-(3,3,3-trifluorpropyl)thieno[2,3-d]-pyrimidin-2,4(1*H*,3*H*)-dion

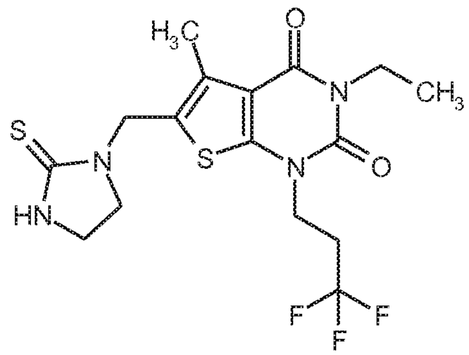

369 mg (0.751 mmol) der Verbindung aus Bsp. 210A wurden in 14 ml Dioxan gelöst und mit 211 mg (1.12 mmol) 1,1'-Thiocarbonyldiimidazol versetzt. Das Gemisch wurde 17 h bei RT gerührt. Die Reaktionslösung wurde danach am Rotationsverdampfer eingeengt. Der resultierende Rückstand wurde in 20 ml Ethylacetat aufgenommen und mit 10 ml 5%-iger Salzsäure gewaschen. Die organische Phase wurde mit gesättigter Kochsalz-Lösung gewaschen, über Natriumsulfat getrocknet, filtriert und eingeengt. Das so erhaltene Material wurde mittels präparativer HPLC gereinigt (Methode 14). Nach Vereinigen der Produktfraktionen und Gefriertrocknung wurden 168 mg (50% d. Th.) der Titelverbindung erhalten.
$^1$H-NMR (400 MHz, DMSO-$d_6$, δ/ppm): 8.34 (s, 1H), 4.85 (s, 2H), 4.09 (t, 2H), 3.90 (q, 2H), 3.57-3.50 (m, 2H), 3.45-3.37 (m, 2H), 2.83-2.69 (m, 2H), 2.45 (s, 3H), 1.12 (t, 3H).
LC/MS (Methode 3): $R_t$ = 1.11 min, m/z = 421 $[M+H]^+$.

**Beispiel 29**

3-Ethyl-1-(3-fluorpropyl)-5-methyl-6-[(2-thioxoimidazolidin-1-yl)methyl]thieno[2,3-d]pyrimidin-2,4(1*H*,3*H*)-dion

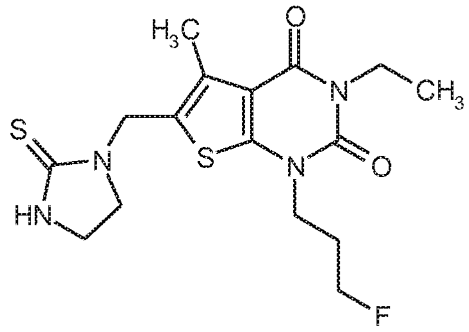

55 mg (0.145 mmol) der Verbindung aus Bsp. 214A wurden in 3 ml DMSO gelöst und mit 40.6 mg (0.217 mmol) 1,1'-Thiocarbonyldiimidazol versetzt. Das Gemisch wurde 116 h bei RT gerührt. Das Reaktionsgemisch wurde danach direkt mittels präparativer HPLC gereinigt (Methode 14). Nach Vereinigen der Produktfraktionen und Gefriertrocknung wurden 12.4 mg (22% d. Th.) der Titelverbindung erhalten.
$^1$H-NMR (400 MHz, DMSO-$d_6$, $\delta$/ppm): 8.32 (s, 1H), 4.84 (s, 2H), 4.59 (t, 1H), 4.47 (t, 1H), 3.97 (t, 2H), 3.90 (q, 2H), 3.57-3.50 (m, 2H), 3.44-3.37 (m, 2H), 2.44 (s, 3H), 2.14-1.98 (m, 2H), 1.11 (t, 3H).
LC/MS (Methode 3): $R_t$ = 1.0 min, m/z = 385 $[M+H]^+$.

**Beispiel 30**

3-Ethyl-1-(4-fluorbutyl)-5-methyl-6-[(2-thioxoimidazolidin-1-yl)methyl]thieno[2,3-d]pyrimidin-2,4(1*H*,3*H*)-dion

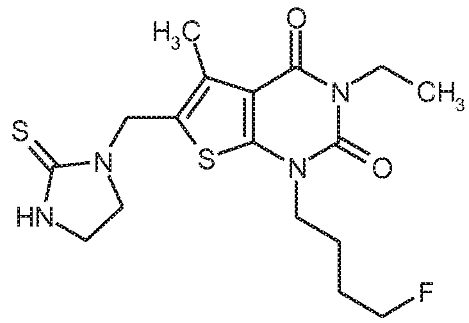

125 mg (0.145 mmol) der Verbindung aus Bsp. 215A wurden in 4 ml Dioxan gelöst und mit 45.6 mg (0.243 mmol) 1,1'-Thiocarbonyldiimidazol versetzt. Das Gemisch wurde 19 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 46 mg (68% d. Th.) der Titelverbindung erhalten.
$^1$H-NMR (400 MHz, DMSO-$d_6$, $\delta$/ppm): 8.33 (s, 1H), 4.83 (s, 2H), 4.51 (t, 1H), 4.42 (t, 1H), 3.94-3.86 (m, 4H), 3.58-3.48 (m, 2H), 3.45-3.37 (m, 2H), 2.44 (s, 3H), 1.81-1.64 (m, 4H), 1.11 (t, 3H).
LC/MS (Methode 3): $R_t$ = 1.07 min, m/z = 399 $[M+H]^+$.

**Beispiel 31**

3-Ethyl-1-(2-methoxyethyl)-5-methyl-6-[(2-thioxoimidazolidin-1-yl)methyl]thieno[2,3-d]pyrimidin-2,4(1*H*,3*H*)-dion

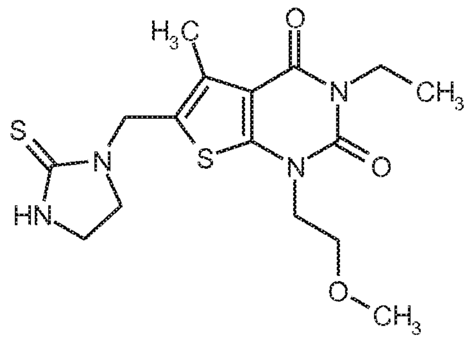

100 mg (0.164 mmol) der Verbindung aus Bsp. 220A wurden in 3.5 ml Dioxan gelöst und mit 46.3 mg (0.247 mmol) 1,1'-Thiocarbonyldiimidazol versetzt. Das Gemisch wurde 1 h bei RT gerührt. Die Reaktionslösung wurde danach eingeengt, und der erhaltene Rückstand wurde über eine Kieselgel-Kartusche chromatographiert (Biotage, 10 g Kieselgel, Laufmittel Hexan/Ethylacetat). Es wurden 26 mg (41% d. Th.) der Titelverbindung erhalten.
$^1$H-NMR (400 MHz, DMSO-$d_6$, δ/ppm): 8.32 (s, 1H), 4.82 (s, 2H), 4.01 (t, 2H), 3.90 (q, 2H), 3.63 (t, 2H), 3.57-3.49 (m, 2H), 3.44-3.37 (m, 2H), 3.24 (s, 3H), 2.43 (s, 3H), 1.12 (t, 3H).
LC/MS (Methode 3): $R_t$ = 0.96 min, m/z = 383 $[M+H]^+$.

**Beispiel 32**

1-(2-Ethoxyethyl)-3-ethyl-5-methyl-6-[(2-thioxoimidazolidin-1-yl)methyl]thieno[2,3-d]pyrimidin-2,4(1*H*,3*H*)-dion

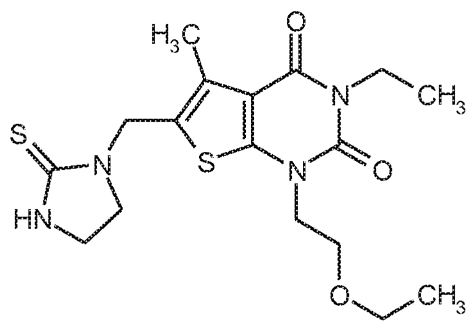

255 mg (0.568 mmol) der Verbindung aus Bsp. 221A wurden in 14 ml Dioxan gelöst und mit 160 mg (0.852 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 144 mg (64% d. Th.) der Titelverbindung erhalten.
$^1$H-NMR (400 MHz, DMSO-$d_6$, δ/ppm): 8.33 (s, 1H), 4.83 (s, 2H), 4.00 (t, 2H), 3.90 (q, 2H), 3.65 (t, 2H), 3.57-3.49 (m, 2H), 3.47-3.36 (m, 4H), 2.44 (s, 3H), 1.12 (t, 3H), 1.03 (t, 3H).
LC/MS (Methode 3): $R_t$ = 1.04 min, m/z = 397 $[M+H]^+$.

**Beispiel 33**

3-Ethyl-1-(2-isopropoxyethyl)-5-methyl-6-[(2-thioxoimidazolidin-1-yl)methyl]thieno[2,3-d]-pyrimidin-2,4(1*H*,3*H*)-dion

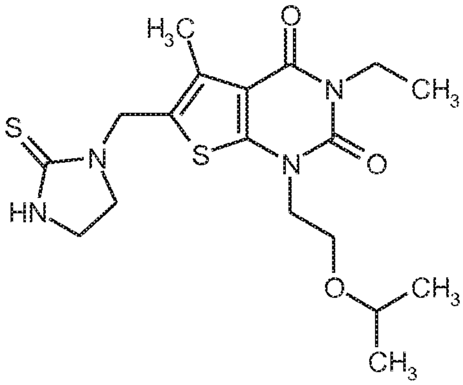

206 mg (0.375 mmol) der Verbindung aus Bsp. 222A wurden in 9 ml Dioxan gelöst und mit 105 mg (0.562 mmol) 1,1'-Thiocarbonyldiimidazol versetzt. Das Gemisch wurde 64 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14).

Nach Vereinigen der Produktfraktionen und Gefriertrocknung wurden 79 mg (50% d. Th.) der Titelverbindung erhalten.
$^1$H-NMR (400 MHz, DMSO-$d_6$, $\delta$/ppm): 8.34 (s, 1H), 4.82 (s, 2H), 3.96 (t, 2H), 3.90 (q, 2H), 3.64 (t, 2H), 3.58-3.47 (m, 3H), 3.43-3.36 (m, 2H), 2.43 (s, 3H), 1.11 (t, 3H), 1.00 (d, 6H).
LC/MS (Methode 3): $R_t$ = 1.11 min, m/z = 411 [M+H]$^+$.

## Beispiel 34

3-Ethyl-5-methyl-1-(oxetan-2-ylmethyl)-6-[(2-thioxoimidazolidin-1-yl)methyl]thieno[2,3-d]-pyrimidin-2,4(1*H*,3*H*)-dion (*Racemat*)

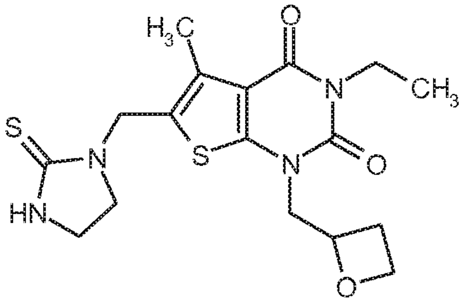

399 mg (1.132 mmol) der Verbindung aus Bsp. 224A wurden in 20 ml Dioxan gelöst und mit 318 mg (1.692 mmol) 1,1'-Thiocarbonyldiimidazol versetzt. Das Gemisch wurde 21 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 54 mg (12% d. Th.) der Titelverbindung erhalten.
$^1$H-NMR (400 MHz, DMSO-$d_6$, $\delta$/ppm): 8.32 (s, 1H), 5.05-4.96 (m, 1H), 4.82 (s, 2H), 4.51-4.38 (m, 2H), 4.13 (d, 2H), 3.90 (q, 2H), 3.57-3.48 (m, 2H), 3.45-3.35 (m, 2H), 2.74-2.63 (m, 1H), 2.44 (s, 3H), 1.12 (t, 3H).
LC/MS (Methode 3): $R_t$ = 0.94 min, m/z = 395 [M+H]$^+$.

## Beispiel 35

3-Ethyl-5-methyl-1-(tetrahydrofuran-2-ylmethyl)-6-[(2-thioxoimidazolidin-1-yl)methyl]thieno-[2,3-d]pyrimidin-2,4(1*H*,3*H*)-dion (*Racemat*)

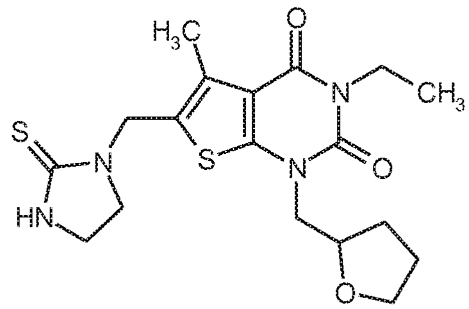

582 mg (1.175 mmol) der Verbindung aus Bsp. 225A wurden in 28.9 ml Dioxan gelöst und mit 330.6 mg (1.763 mmol) 1,1'-Thiocarbonyldiimidazol versetzt. Das Gemisch wurde 19 h bei RT gerührt. Die Reaktionslösung wurde danach am Rotationsverdampfer eingeengt. Der Rückstand wurde in 12 ml DMSO gelöst und diese Lösung mittels präparativer HPLC gereinigt (Methode 14). Nach Vereinigen der Produktfraktionen und Gefriertrocknung wurden 281 mg (58% d. Th.) der Titelverbindung erhalten.
$^1$H-NMR (400 MHz, DMSO-$d_6$, $\delta$/ppm): 8.35 (s, 1H), 4.82 (s, 2H), 4.26-4.18 (m, 1H), 4.02 (dd, 1H), 3.90 (q, 2H), 3.78-3.65 (m, 2H), 3.64-3.57 (m, 1H), 3.56-3.49 (m, 2H), 3.44-3.37 (m, 2H), 2.43 (s, 3H), 2.02-1.76 (m, 3H), 1.71-1.61 (m, 1H), 1.11 (t, 3H).
LC/MS (Methode 3): $R_t$ = 1.02 min, m/z = 409 $[M+H]^+$.

**Beispiel 36**

3-Ethyl-5-methyl-1-(tetrahydro-2*H*-pyran-2-ylmethyl)-6-[(2-thioxoimidazolidin-1-yl)methyl]-thieno[2,3-d]pyrimidin-2,4(1*H*,3*H*)-dion (*Racemat*)

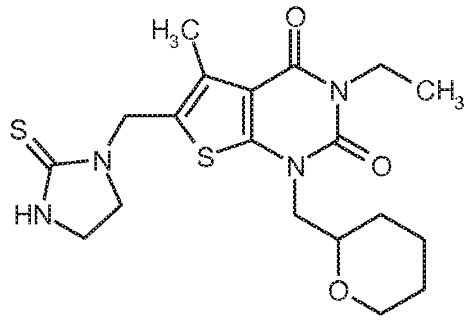

320 mg (0.732 mmol) der Verbindung aus Bsp. 226A wurden in 14 ml Dioxan gelöst und mit 206 mg (1.097 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 9 ml DMSO gelöst und diese Lösung mittels präparativer HPLC gereinigt (Methode 14). Nach Vereinigen der Produktfraktionen und Gefriertrocknung wurden 191 mg (61% d. Th.) der Titelverbindung erhalten.
$^1$H-NMR (400 MHz, DMSO-$d_6$, $\delta$/ppm): 8.34 (s, 1H), 4.88-4.75 (m, 2H), 4.01-3.93 (m, 1H), 3.90 (q, 2H), 3.81 (dd, 1H), 3.72-3.63 (m, 2H), 3.58-3.46 (m, 2H), 3.44-3.35 (m, 2H), 3.30-3.21 (m, 1H), 2.43 (s, 3H), 1.78 (d, 1H), 1.61 (d, 1H), 1.52-1.38 (m, 3H), 1.32-1.19 (m, 1H), 1.11 (t, 3H).
LC/MS (Methode 3): $R_t$ = 1.13 min, m/z = 423 $[M+H]^+$.

**Beispiel 37**

1-(3,3-Dimethylbutyl)-3-ethyl-5-methyl-6-[(2-thioxoimidazolidin-1-yl)methyl]thieno[2,3-d]pyrimidin-2,4(1*H*,3*H*)-dion

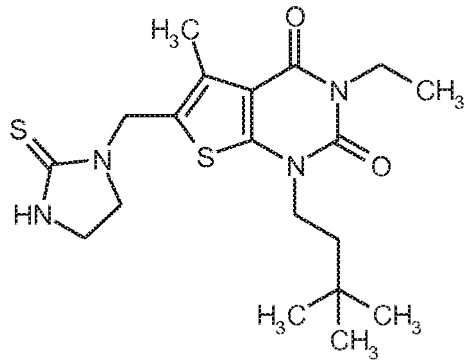

180 mg (0.368 mmol) der Verbindung aus Bsp. 234A wurden in 9.2 ml Dioxan gelöst und mit 104 mg (0.552 mmol) 1,1'-Thiocarbonyldiimidazol versetzt. Das Gemisch wurde 19 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 78 mg (52% d. Th.) der Titelverbindung erhalten.
$^1$H-NMR (400 MHz, DMSO-$d_6$, $\delta$/ppm): 8.34 (s, 1H), 4.82 (s, 2H), 3.93-3.80 (m, 4H), 3.57-3.49 (m, 2H), 2.43 (s, 3H), 1.57-1.50 (m, 2H), 1.11 (t, 3H), 0.96 (s, 9H).
LC/MS (Methode 3): $R_t$ = 1.30 min, m/z = 409 [M+H]$^+$.

**Beispiel 38**

3-Isobutyl-5-methyl-6-[(2-thioxoimidazolidin-1-yl)methyl]-1-(3,3,3-trifluorpropyl)thieno[2,3-d]-pyrimidin-2,4(1*H*,3*H*)-dion

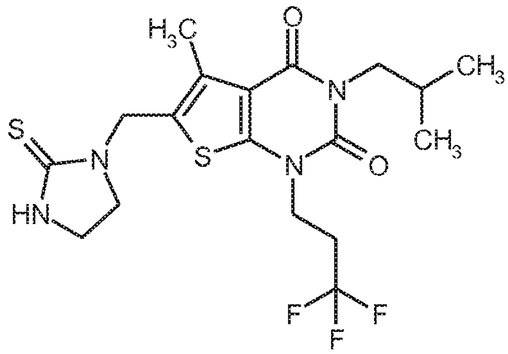

115 mg (0.269 mmol) der Verbindung aus Bsp. 239A wurden in 5 ml Dioxan gelöst und mit 75.6 mg (0.4 mmol) 1,1'-Thiocarbonyldiimidazol versetzt. Das Gemisch wurde 67 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 13 mg (11% d. Th.) der Titelverbindung erhalten.
$^1$H-NMR (400 MHz, DMSO-$d_6$, $\delta$/ppm): 8.36 (s, 1H), 4.84 (s, 2H), 4.10 (t, 2H), 3.71 (d, 2H), 3.59-3.50 (m, 2H), 3.46-3.37 (m, 2H), 2.83-2.69 (m, 2H), 2.44 (s, 3H), 2.09-1.97 (m, 1H), 0.85 (d, 6H).
LC/MS (Methode 3): $R_t$ = 1.27 min, m/z = 449 [M+H]$^+$.

**Beispiel 39**

3-Isobutyl-1-(2-methoxyethyl)-5-methyl-6-[(2-thioxoimidazolidin-1-yl)methyl]thieno[2,3-d]-pyrimidin-2,4(1*H*,3*H*)-dion

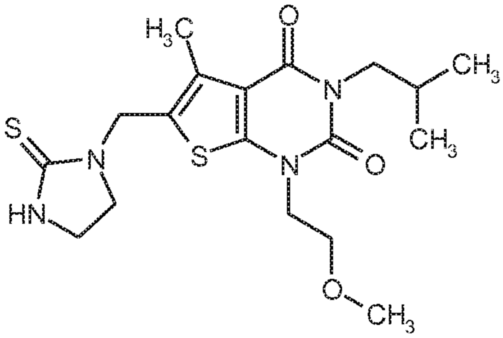

103 mg (0.204 mmol) der Verbindung aus Bsp. 242A wurden in 5 ml Dioxan gelöst und mit 57.4 mg (0.306 mmol) 1,1'-Thiocarbonyldiimidazol versetzt. Das Gemisch wurde 18 h bei RT gerührt. Das Reaktionsgemisch wurde danach direkt mittels präparativer HPLC gereinigt (Methode 14). Nach Vereinigen der Produktfraktionen und Gefriertrocknung wurden 11 mg (11% d. Th.) der Titelverbindung sowie 18 mg des *N*-Formylderivats (*siehe Beispiel 46*) erhalten.
$^1$H-NMR (400 MHz, DMSO-$d_6$, δ/ppm): 8.32 (s, 1H), 4.82 (s, 2H), 4.01 (t, 2H), 3.71 (d, 2H), 3.62 (t, 2H), 3.58-3.49 (m, 2H), 3.45-3.36 (m, 2H), 3.23 (s, 3H), 2.43 (s, 3H), 2.09-1.97 (m, 1H), 0.85 (d, 6H).
LC/MS (Methode 3): $R_t$ = 1.13 min, m/z = 411 $[M+H]^+$.

**Beispiel 40**

1-(3-Fluorpropyl)-5-methyl-6-[(2-thioxoimidazolidin-1-yl)methyl]-3-(2,2,2-trifluorethyl)thieno-[2,3-d]pyrimidin-2,4(1*H*,3*H*)-dion

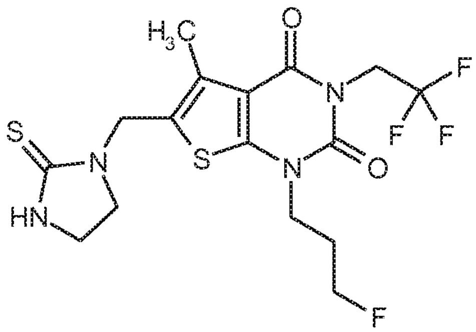

140 mg (0.254 mmol) der Verbindung aus Bsp. 248A wurden in 5 ml Dioxan gelöst und mit 71.5 mg (0.381 mmol) 1,1'-Thiocarbonyldiimidazol versetzt. Das Gemisch wurde 20 h bei RT gerührt. Das Reaktionsgemisch wurde danach direkt mittels präparativer HPLC gereinigt (Methode 14). Nach Vereinigen der Produktfraktionen und Gefriertrocknung wurden 77 mg (67% d. Th.) der Titelverbindung erhalten.
$^1$H-NMR (400 MHz, DMSO-$d_6$, δ/ppm): 8.36 (s, 1H), 4.85 (s, 2H), 4.69 (q, 2H), 4.59 (t, 1H), 4.47 (t, 1H), 4.01 (t, 2H), 3.59-3.51 (m, 2H), 3.46-3.37 (m, 2H), 2.44 (s, 3H), 2.15-1.99 (m, 2H).
LC/MS (Methode 3): $R_t$ = 1.10 min, m/z = 439 $[M+H]^+$.

**Beispiel 41**

1-(2-Methoxyethyl)-5-methyl-6-[(2-thioxoimidazolidin-1-yl)methyl]-3-(2,2,2-trifluorethyl)thieno-[2,3-d]pyrimidin-2,4(1*H*,3*H*)-dion

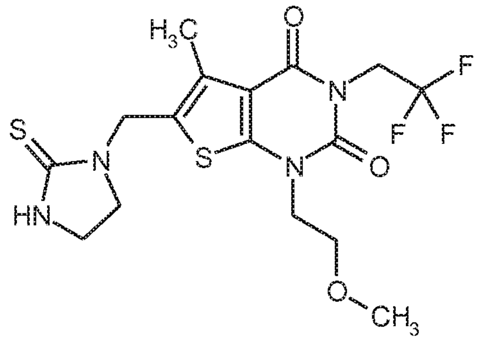

140 mg (0.234 mmol) der Verbindung aus Bsp. 249A wurden in 5 ml Dioxan gelöst und mit 65.9 mg (0.351 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 46 mg (45% d. Th.) der Titelverbindung erhalten.
$^1$H-NMR (400 MHz, DMSO-$d_6$, δ/ppm): 8.36 (s, 1H), 4.84 (s, 2H), 4.69 (q, 2H), 4.04 (t, 2H), 3.63 (t, 2H), 3.59-3.50 (m, 2H), 3.44-3.38 (m, 2H), 3.23 (s, 3H), 2.43 (s, 3H).
LC/MS (Methode 3): $R_t$ = 1.05 min, m/z = 437 $[M+H]^+$.

## Beispiel 42

3-(2,2-Difluorethyl)-1-(3-fluorpropyl)-5-methyl-6-[(2-thioxoimidazolidin-1-yl)methyl]thieno-[2,3-d]pyrimidin-2,4(1*H*,3*H*)-dion

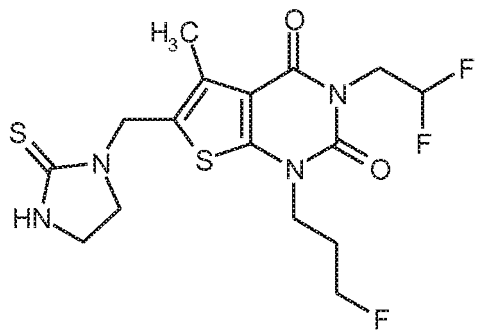

195 mg (0.433 mmol) der Verbindung aus Bsp. 256A wurden in 8.8 ml Dioxan gelöst und mit 121.8 mg (0.649 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 73 mg (40% d. Th.) der Titelverbindung erhalten.
$^1$H-NMR (400 MHz, DMSO-$d_6$, δ/ppm): 8.34 (s, 1H), 6.36-6.04 (m, 1H), 4.85 (s, 2H), 4.59 (t, 1H), 4.47 (t, 1H), 4.29 (td, 2H), 4.00 (t, 2H), 3.58-3.50 (m, 2H), 3.46-3.37 (m, 2H), 2.44 (s, 3H), 2.14-2.00 (m, 2H).
LC/MS (Methode 3): $R_t$ = 1.04 min, m/z = 421 $[M+H]^+$.

## Beispiel 43

3-(2,2-Difluorethyl)-1-(2-methoxyethyl)-5-methyl-6-[(2-thioxoimidazolidin-1-yl)methyl]thieno-[2,3-d]pyrimidin-2,4(1*H*,3*H*)-dion

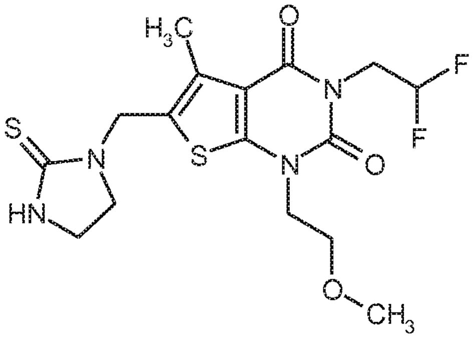

178 mg (0.34 mmol) der Verbindung aus Bsp. 257A wurden in 7.36 ml Dioxan gelöst und mit 95.8 mg (0.511 mmol) 1,1'-Thiocarbonyldiimidazol versetzt. Das Gemisch wurde 16 h bei RT gerührt. Danach wurden nochmals 31.9 mg (0.17 mmol) 1,1'-Thiocarbonyldiimidazol zugegeben und weitere 7 h bei RT gerührt. Die Reaktionslösung wurde dann am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 59 mg (40% d. Th.) der Titelverbindung erhalten.
$^1$H-NMR (400 MHz, DMSO-$d_6$, $\delta$/ppm): 8.34 (s, 1H), 6.38-6.05 (m, 1H), 4.84 (s, 2H), 4.29 (td, 2H), 4.03 (t, 2H), 3.63 (t, 2H), 3.58-3.49 (m, 2H), 3.46-3.37 (m, 2H), 3.24 (s, 3H), 2.43 (s, 3H).
LC/MS (Methode 3): $R_t$ = 0.99 min, m/z = 419 [M+H]$^+$.

**Beispiel 44**

5-(Difluormethyl)-3-ethyl-6-[(2-thioxoimidazolidin-1-yl)methyl]-1-(3,3,3-trifluorpropyl)thieno-[2,3-d]pyrimidin-2,4(1*H*,3*H*)-dion

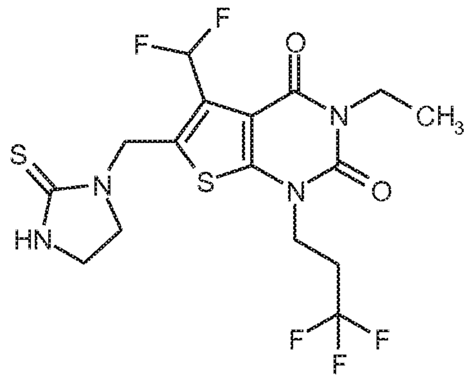

354 mg (0.854 mmol) der Verbindung aus Bsp. 264A wurden in 18 ml Dioxan gelöst und mit 240 mg (1.28 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 79 mg (19% d. Th.) der Titelverbindung erhalten.
$^1$H-NMR (400 MHz, DMSO-$d_6$, $\delta$/ppm): 8.55 (s, 1H), 7.74-7.43 (m, 1H), 5.05 (s, 2H), 4.12 (t, 2H), 3.92 (q, 2H), 3.65-3.57 (m, 2H), 3.50-3.42 (m, 2H), 2.85-2.71 (m, 2H), 1.13 (t, 3H).
LC/MS (Methode 3): $R_t$ = 1.18 min, m/z = 457 [M+H]$^+$.

**Beispiel 45**

5-(Difluormethyl)-3-ethyl-1-(2-methoxyethyl)-6-[(2-thioxoimidazolidin-1-yl)methyl]thieno[2,3-d]-pyrimidin-2,4(1*H*,3*H*)-dion

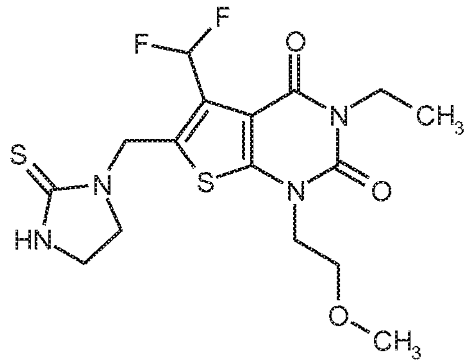

335 mg (0.89 mmol) der Verbindung aus Bsp. 265A wurden in 18 ml Dioxan gelöst und mit 250.4 mg (1.33 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14).

Nach Vereinigen der Produktfraktionen und Gefriertrocknung wurden 42 mg (10% d. Th.) der Titelverbindung erhalten.

$^1$H-NMR (400 MHz, DMSO-$d_6$, δ/ppm): 8.58-8.49 (m, 1H), 7.72-7.42 (m, 1H), 5.02 (s, 2H), 4.04 (t, 2H), 3.91 (q, 2H), 3.68-3.55 (m, 4H), 3.50-3.41 (m, 2H), 3.24 (s, 3H), 1.13 (t, 3H).

LC/MS (Methode 3): $R_t$ = 1.05 min, m/z = 419 [M+H]$^+$.

**Beispiel 46**

3-{[3-Isobutyl-1(2-methoxyethyl)-5-methyl-2,4-dioxo-1,2,3,4-tetrahydrothieno[2,3-d]pyrimidin-6-yl]methyl}-2-thioxoimidazolidin-1-carbaldehyd

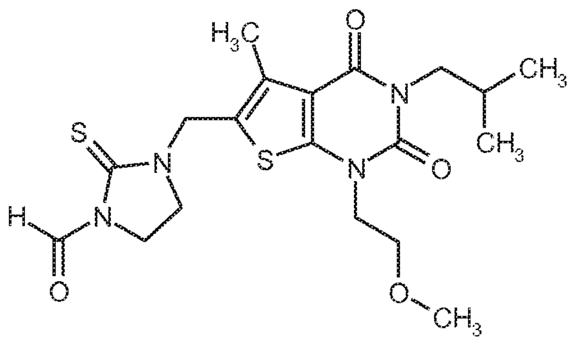

Die Titelverbindung (18 mg) wurde als Nebenprodukt der Herstellung und Aufreinigung der in Beispiel 39 beschriebenen Verbindung erhalten.

$^1$H-NMR (400 MHz, DMSO-$d_6$, δ/ppm): 9.29 (s, 1H), 5.05 (s, 2H), 4.05-3.99 (m, 2H), 3.87-3.80 (m, 2H), 3.77-3.68 (m, 4H), 3.62 (t, 2H), 3.22 (s, 3H), 2.47 (s, 3H), 2.09-1.99 (m, 1H), 0.84 (d, 6H).

LC/MS (Methode 3): $R_t$ = 1.25 min, m/z = 439 [M+H]$^+$.

**Beispiel 47**

3-Ethyl-5-methyl-6-[(2-oxoimidazolidin-1-yl)methyl]-1-(2,2,2-trifluorethyl)thieno[2,3-d]pyrimidin-2,4(1*H*,3*H*)-dion

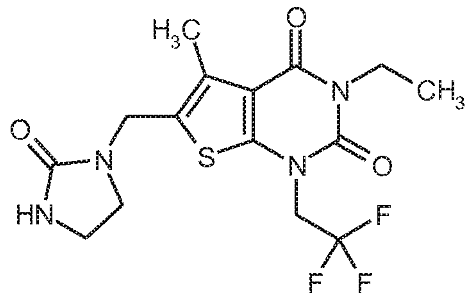

Eine Lösung von 720 mg (1.42 mmol, 72% Reinheit) der Verbindung aus Bsp. 209A und 297 µl (2.13 mmol) Triethylamin in 14 ml THF wurde mit 277 mg (1.71 mmol) CDI versetzt und ca. 18 h bei RT gerührt. Anschließend wurde zur Trockene eingeengt. Der verbliebene Rückstand wurde in Ethylacetat aufgenommen und nacheinander mit 1 M Salzsäure, Wasser und gesättigter Natriumchlorid-Lösung gewaschen. Nach Trocknen über wasserfreiem Magnesiumsulfat wurde filtriert und eingeengt. Der feste Rückstand wurde bei RT in wenig Acetonitril verrührt. Nach Filtration und Trocknen des Feststoffs im Hochvakuum wurden 276 mg (49% d. Th.) der Titelverbindung erhalten.
$^1$H-NMR (400 MHz, DMSO-$d_6$, δ/ppm): 6.54 (br. s, 1H), 4.81 (q, 2H), 4.37 (s, 2H), 3.91 (q, 2H), 3.29-3.14 (m, 4H), 2.41 (s, 3H), 1.13 (t, 3H).
LC/MS (Methode 1, ESIpos): $R_t$ = 0.79 min, m/z = 391 $[M+H]^+$.

**Beispiel 48**

3-Ethyl-5-methyl-6-[(2-oxoimidazolidin-1-yl)methyl]-1-(3,3,3-trifluorpropyl)thieno[2,3-d]pyrimidin-2,4(1*H*,3*H*)-dion

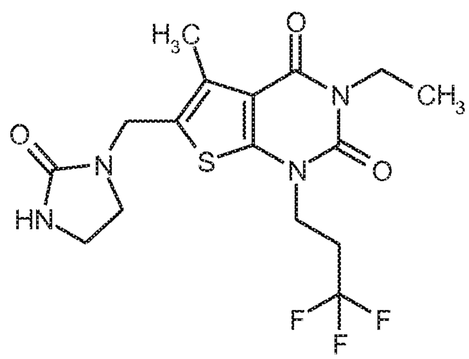

Eine Lösung von 92 mg (1.07 mmol) Imidazolidin-2-on in 2.8 ml THF wurde mit 43 mg (1.07 mmol) Natriumhydrid (60% Suspension in Mineralöl) versetzt, 2 h auf 60°C erwärmt und anschließend wieder auf RT abgekühlt ("Lösung 1"). In einem anderen Reaktionsgefäß wurde eine Lösung von 90 mg (0.268 mmol) der Verbindung aus Bsp. 140A in 1.8 ml Dichlormethan bei 0°C mit 93 µl (0.535 mmol) *N,N*-Diisopropylethylamin und 20 µl (0.281 mmol) Thionylchlorid versetzt. Nach 20 min bei 0°C wurde Lösung 1 tropfenweise hinzugefügt und dann das Kältebad entfernt. Das Reaktionsgemisch wurde 4 Tage bei RT gerührt. Dann wurden alle flüchtigen Bestandteile am Rotationsverdampfer entfernt. Der verbliebene Rückstand wurde mittels präparativer HPLC (Methode 10) in seine Komponenten aufgetrennt. Nach Eindampfen der Produktfraktionen und Trocknen im Hochvakuum wurden 55 mg (51% d. Th.) der Titelverbindung erhalten.
$^1$H-NMR (400 MHz, DMSO-$d_6$, δ/ppm): 6.55 (br. s, 1H), 4.37 (s, 2H), 4.10 (t, 2H), 3.90 (q, 2H), 3.33-3.13 (m, 4H), 2.87-2.67 (m, 2H), 2.41 (s, 3H), 1.12 (t, 3H).
LC/MS (Methode 1, ESIpos): $R_t$ = 0.86 min, m/z = 405 $[M+H]^+$.

**Beispiel 49**

3-Ethyl-5-methyl-6-[(2-oxoimidazolidin-1-yl)methyl]-1-(4,4,4-trifluorbutyl)thieno[2,3-d]pyrimidin-2,4(1*H*,3*H*)-dion

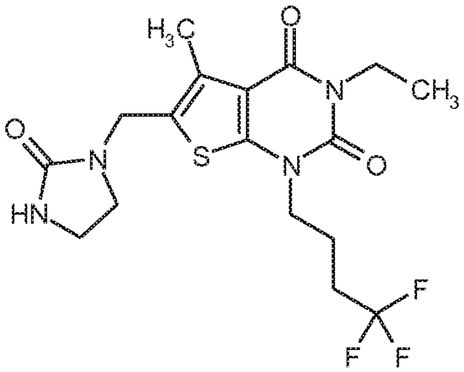

Analog zu dem unter Bsp. 47 beschriebenen Verfahren wurden aus 300 mg (0.573 mmol, 75% Reinheit) der Verbindung aus Bsp. 211A und 112 mg (0.688 mmol) CDI 140 mg (58% d. Th.) der Titelverbindung hergestellt. Auf die wässrige Aufarbeitung vor dem Verrühren mit Acetonitril wurde hier verzichtet.
$^1$H-NMR (400 MHz, DMSO-$d_6$, δ/ppm): 6.52 (s, 1H), 4.36 (s, 2H), 3.95 (t, 2H), 3.90 (q, 2H), 3.28-3.16 (m, 4H), 2.47-2.33 (m, 2H), 2.40 (s, 3H), 1.89 (quin, 2H), 1.12 (t, 3H).
LC/MS (Methode 1, ESIpos): $R_t$ = 0.90 min, m/z = 419 $[M+H]^+$.

**Beispiel 50**

3-Ethyl-1-(2-fluorethyl)-5-methyl-6-[(2-oxoimidazolidin-1-yl)methyl]thieno[2,3-d]pyrimidin-2,4(1*H*,3*H*)-dion

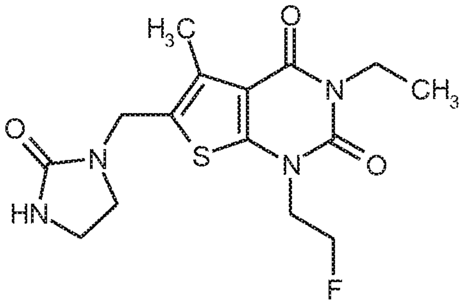

Analog zu dem unter Bsp. 47 beschriebenen Verfahren wurden aus 286 mg (0.749 mmol, 86% Reinheit) der Verbindung aus Bsp. 213A und 146 mg (0.899 mmol) CDI 135 mg (50% d. Th.) der Titelverbindung hergestellt.
$^1$H-NMR (400 MHz, DMSO-$d_6$, δ/ppm): 6.52 (s, 1H), 4.73 (dt, 2H), 4.35 (s, 2H), 4.18 (dt, 2H), 3.91 (q, 2H), 3.29-3.15 (m, 4H), 2.40 (s, 3H), 1.13 (t, 3H).
LC/MS (Methode 1, ESIpos): $R_t$ = 0.65 min, m/z = 355 $[M+H]^+$.

**Beispiel 51**

3-Ethyl-1-(3-fluorpropyl)-5-methyl-6-[(2-oxoimidazolidin-1-yl)methyl]thieno[2,3-d]pyrimidin-2,4(1*H*,3*H*)-dion

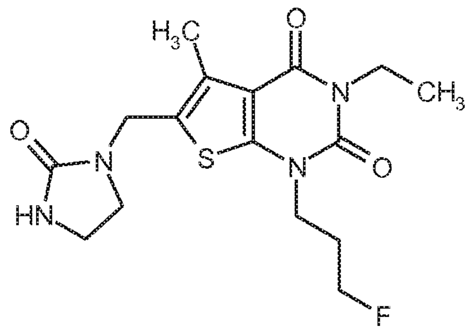

55 mg (0.154 mmol) der Verbindung aus Bsp. 214A wurden in 3 ml DMSO gelöst und mit 36.2 mg (0.217 mmol) CDI versetzt. Das Gemisch wurde 116 h bei RT gerührt. Das Reaktionsgemisch wurde danach direkt mittels präparativer HPLC gereinigt (Methode 14). Nach Vereinigen der Produktfraktionen und Gefriertrocknung wurden 9 mg (17% d. Th.) der Titelverbindung erhalten.
$^1$H-NMR (400 MHz, DMSO-$d_6$, δ/ppm): 6.51 (s, 1H), 4.59 (t, 1H), 4.47 (t, 1H), 4.35 (s, 2H), 3.98 (t, 2H), 3.90 (q, 2H), 3.28-3.17 (m, 4H), 2.40 (s, 3H), 2.14-1.98 (m, 2H), 1.11 (t, 3H).
LC/MS (Methode 3): $R_t$ = 0.9 min, m/z = 369 $[M+H]^+$.

**Beispiel 52**

3-Ethyl-1-(4-fluorbutyl)-5-methyl-6-[(2-oxoimidazolidin-1-yl)methyl]thieno[2,3-d]pyrimidin-2,4(1*H*,3*H*)-dion

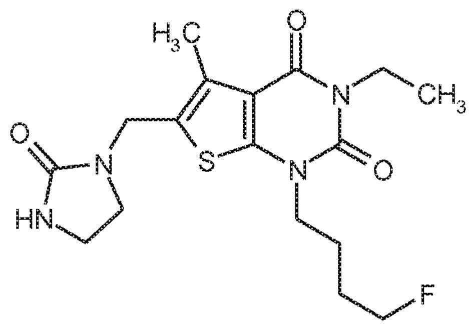

125 mg (0.162 mmol) der Verbindung aus Bsp. 215A wurden in 4 ml Dioxan gelöst und mit 40.6 mg (0.243 mmol) CDI versetzt. Das Gemisch wurde 19 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 44 mg (62% d. Th.) der Titelverbindung erhalten.
$^1$H-NMR (400 MHz, DMSO-$d_6$, δ/ppm): 6.52 (s, 1H), 4.51 (t, 1H), 4.42 (t, 1H), 4.35 (s, 2H), 3.93-3.86 (m, 4H), 3.28-3.18 (m, 4H), 2.40 (s, 3H), 1.80-1.64 (m, 4H), 1.11 (t, 3H).
LC/MS (Methode 3): $R_t$ = 0.97 min, m/z = 383 $[M+H]^+$.

**Beispiel 53**

3-Ethyl-5-methyl-6-[(2-oxoimidazolidin-1-yl)methyl]-1-[2-(trifluormethyl)prop-2-en-1-yl]thieno-[2,3-d]pyrimidin-2,4(1*H*,3*H*)-dion

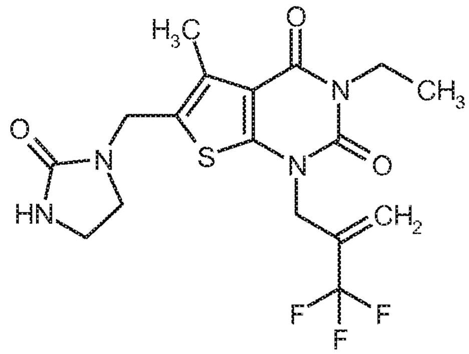

Analog zu dem unter Bsp. 47 beschriebenen Verfahren wurden aus 238 mg (0.518 mmol, 85% Reinheit) der Verbindung aus Bsp. 216A und 101 mg (0.622 mmol) CDI 98 mg (43% d. Th.) der Titelverbindung hergestellt.
$^1$H-NMR (400 MHz, DMSO-$d_6$, $\delta$/ppm): 6.52 (s, 1H), 5.98 (s, 1H), 5.70 (s, 1H), 4.74 (s, 2H), 4.35 (s, 2H), 3.92 (q, 2H), 3.28-3.12 (m, 4H), 2.41 (s, 3H), 1.13 (t, 3H).
LC/MS (Methode 1, ESIpos): $R_t$ = 0.87 min, m/z = 417 $[M+H]^+$.

## Beispiel 54

1-[(2,2-Difluorcyclopropyl)methyl]-3-ethyl-5-methyl-6-[(2-oxoimidazolidin-1-yl)methyl]thieno-[2,3-d]pyrimidin-2,4(1*H*,3*H*)-dion (*Racemat*)

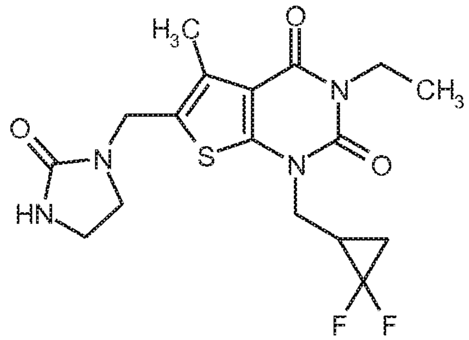

Analog zu dem unter Bsp. 47 beschriebenen Verfahren wurden aus 540 mg (1.19 mmol, 82% Reinheit) der Verbindung aus Bsp. 217A und 231 mg (1.43 mmol) CDI 300 mg (63% d. Th.) der Titelverbindung hergestellt.
$^1$H-NMR (400 MHz, DMSO-$d_6$, $\delta$/ppm): 6.53 (s, 1H), 4.36 (s, 2H), 4.17-4.04 (m, 1H), 4.01-3.92 (m, 1H), 3.91 (q, 2H), 3.29-3.17 (m, 4H), 2.41 (s, 3H), 2.28-2.10 (m, 1H), 1.79-1.62 (m, 1H), 1.54-1.39 (m, 1H), 1.12 (t, 3H).
LC/MS (Methode 1, ESIpos): $R_t$ = 0.83 min, m/z = 399 $[M+H]^+$.

## Beispiel 55

1-[(2,2-Difluorcyclopropyl)methyl]-3-ethyl-5-methyl-6-[(2-oxoimidazolidin-1-yl)methyl]thieno-[2,3-d]pyrimidin-2,4(1*H*,3*H*)-dion (*Enantiomer 1*)

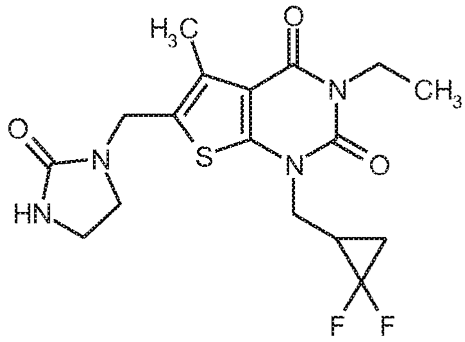

291 mg (0.730 mmol) der racemischen Verbindung aus Bsp. 54 wurden in 20 ml Methanol gelöst und in 24 Portionen über präparative HPLC an chiraler Phase in die Enantiomere aufgetrennt [Säule: Daicel Chiralpak IA, 5 µm, 250 mm x 20 mm; Laufmittel: tert.-Butylmethylether/Methanol 50:50; Fluss: 20 ml/min; Temperatur: 20°C; Detektion: 210 nm]. Nach Eindampfen der Produktfraktionen und Trocknen im Hochvakuum wurden 122 mg (83% d. Th.) von Enantiomer 1 erhalten (99.0% ee, chirale analytische HPLC).

$^1$H-NMR (400 MHz, DMSO-$d_6$, δ/ppm): 6.53 (s, 1H), 4.36 (s, 2H), 4.19-4.03 (m, 1H), 4.01-3.92 (m, 1H), 3.91 (q, 2H), 3.29-3.16 (m, 4H), 2.41 (s, 3H), 2.27-2.11 (m, 1H), 1.80-1.63 (m, 1H), 1.53-1.39 (m, 1H), 1.12 (t, 3H).

Chirale analytische HPLC [Säule: Daicel Chiralpak IA, 5 µm, 250 mm x 4.6 mm; Laufmittel: *tert.*-Butylmethylether/Methanol 50:50; Fluss: 3 ml/min; Temperatur: 30°C; Detektion: 210 nm]: $R_t$ = 5.26 min.

## Beispiel 56

1-[(2,2-Difluorcyclopropyl)methyl]-3-ethyl-5-methyl-6-[(2-oxoimidazolidin-1-yl)methyl]thieno-[2,3-d]pyrimidin-2,4(1*H*,3*H*)-dion (*Enantiomer 2*)

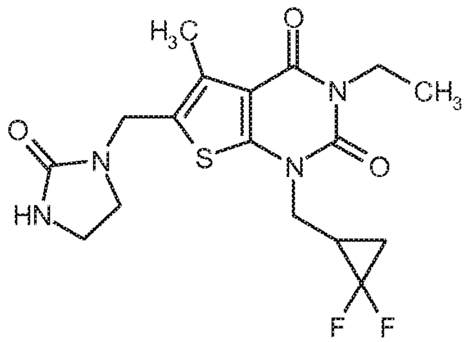

291 mg (0.730 mmol) der racemischen Verbindung aus Bsp. 54 wurden in 20 ml Methanol gelöst und in 24 Portionen über präparative HPLC an chiraler Phase in die Enantiomere aufgetrennt [Säule: Daicel Chiralpak IA, 5 µm, 250 mm x 20 mm; Laufmittel: tert.-Butylmethylether/Methanol 50:50; Fluss: 20 ml/min; Temperatur: 20°C; Detektion: 210 nm]. Nach Eindampfen der Produktfraktionen und Trocknen im Hochvakuum wurden 126 mg (86% d. Th.) von Enantiomer 2 erhalten (99.9% ee, chirale analytische HPLC).

$^1$H-NMR (400 MHz, DMSO-$d_6$, δ/ppm): 6.53 (s, 1H), 4.36 (s, 2H), 4.17-4.03 (m, 1H), 4.01-3.92 (m, 1H), 3.91 (q, 2H), 3.29-3.15 (m, 4H), 2.41 (s, 3H), 2.29-2.11 (m, 1H), 1.82-1.61 (m, 1H), 1.55-1.40 (m, 1H), 1.12 (t, 3H).

Chirale analytische HPLC [Säule: Daicel Chiralpak IA, 5 µm, 250 mm x 4.6 mm; Laufmittel: *tert.*-Butylmethylether/Methanol 50:50; Fluss: 3 ml/min; Temperatur: 30°C; Detektion: 210 nm]: $R_t$ = 4.55 min.

## Beispiel 57

3-Ethyl-5-methyl-6-[(2-oxoimidazolidin-1-yl)methyl]-1-[2-(trifluormethoxy)ethyl]thieno[2,3-d]-pyrimidin-2,4(1*H*,3*H*)-dion

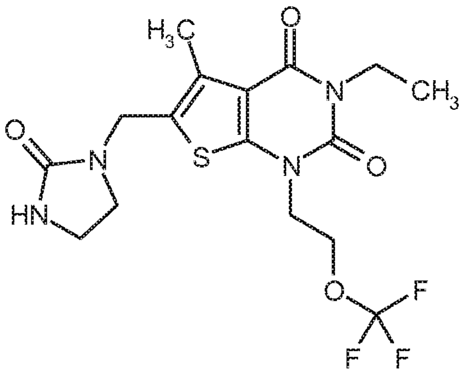

Eine Lösung von 288 mg (0.570 mmol, 78% Reinheit) der Verbindung aus Bsp. 218A und 119 µl (0.854 mmol) Triethylamin in 5.5 ml THF wurde mit 111 mg (0.683 mmol) CDI versetzt und ca. 18 h bei RT gerührt. Anschließend wurde das Reaktionsgemisch direkt mittels präparativer HPLC (Methode 8) in seine Komponenten aufgetrennt. Nach Eindampfen der Produktfraktionen und Trocknen im Hochvakuum wurden 79 mg (32% d. Th., 97% Reinheit) der Titelverbindung erhalten.

$^1$H-NMR (400 MHz, DMSO-$d_6$, δ/ppm): 6.53 (s, 1H), 4.43-4.37 (m, 2H), 4.36 (s, 2H), 4.19 (t, 2H), 3.91 (q, 2H), 3.28-3.15 (m, 4H), 2.40 (s, 3H), 1.12 (t, 3H).

LC/MS (Methode 1, ESIpos): $R_t$ = 0.87 min, m/z = 421 $[M+H]^+$.

**Beispiel 58**

3-Ethyl-5-methyl-6-[(2-oxoimidazolidin-1-yl)methyl]-1-{2-[(trifluormethyl)sulfanyl]ethyl)thieno-[2,3-d]pyrimidin-2,4(1*H*,3*H*)-dion

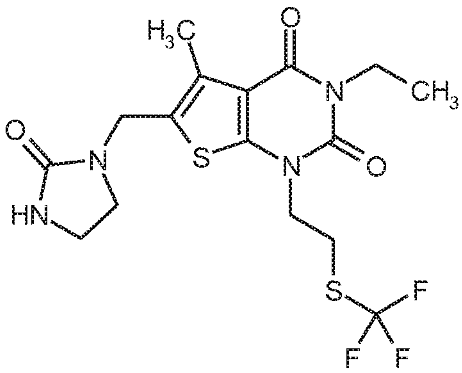

Analog zu dem unter Bsp. 57 beschriebenen Verfahren wurden aus 270 mg (0.526 mmol, 80% Reinheit) der Verbindung aus Bsp. 219A und 102 mg (0.631 mmol) CDI 140 mg (60% d. Th.) der Titelverbindung hergestellt.

$^1$H-NMR (400 MHz, DMSO-$d_6$, δ/ppm): 6.53 (s, 1H), 4.36 (s, 2H), 4.14 (t, 2H), 3.90 (q, 2H), 3.35 (t, 2H, teilweise überdeckt vom Wassersignal), 3.28-3.17 (m, 4H), 2.40 (s, 3H), 1.12 (t, 3H).

LC/MS (Methode 1, ESIpos): $R_t$ = 0.92 min, m/z = 437 $[M+H]^+$.

**Beispiel 59**

3-Ethyl-1-(2-metboxyethyl)-5-methyl-6-[(2-oxoimidazolidin-1-yl)methyl]thieno[2,3-d]pyrimidin-2,4(1*H*,3*H*)-dion

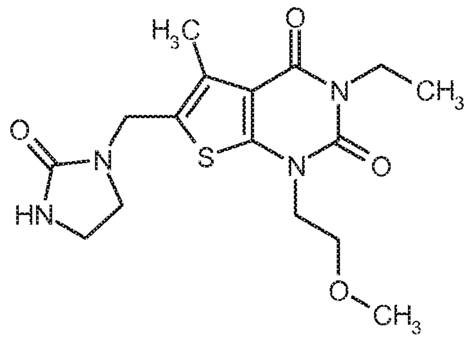

*Methode A:*

Eine Lösung von 99 mg (1.15 mmol) Imidazolidin-2-on in 3 ml THF wurde mit 46 mg (1.15 mmol) Natriumhydrid (60% Suspension in Mineralöl) versetzt, 2 h auf 60°C erwärmt und anschließend wieder auf RT abgekühlt ("Lösung 1"). In einem anderen Reaktionsgefäß wurde eine Lösung von 90 mg (0.287 mmol) der Verbindung aus Bsp. 143A in 2 ml Dichlormethan bei 0°C mit 100 μl (0.573 mmol) *N,N*-Diisopropylethylamin und 22 μl (0.301 mmol) Thionylchlorid versetzt. Nach 20 min bei 0°C wurde die Lösung 1 tropfenweise hinzugefügt und anschließend das Kältebad entfernt. Das Reaktionsgemisch wurde ca. 18 h bei RT gerührt. Dann wurden alle flüchtigen Bestandteile am Rotationsverdampfer entfernt. Der verbliebene Rückstand wurde mittels präparativer HPLC (Methode 8) in seine Komponenten aufgetrennt. Nach Eindampfen der Produktfraktionen und Trocknen im Hochvakuum wurden 63 mg (60% d. Th.) der Titelverbindung erhalten.

*Methode B:*

Eine Lösung von 45.95 g (135 mmol) der Verbindung aus Bsp. 220A und 28.2 ml (202 mmol) Triethylamin in 1.12 Liter THF wurde mit 26.26 g (162 mmol) CDI versetzt und ca. 18 h bei RT gerührt. Anschließend wurde zur Trockene eingeengt. Der verbliebene Rückstand wurde in 1.5 Liter Ethylacetat aufgenommen und nacheinander mit 1 M Salzsäure, Wasser, gesättigter Natriumcarbonat-Lösung und gesättigter Natriumchlorid-Lösung gewaschen. Nach Trocknen über wasserfreiem Magnesiumsulfat wurde filtriert und eingeengt. Es wurde so eine erste Fraktion von 37.5 g des Rohprodukts erhalten. Die vereinigten wässrigen Phasen wurden nochmals mit Ethylacetat extrahiert. Nach Eindampfen des organischen Extraktes wurde eine zweite Fraktion von 9.5 g des Rohprodukts erhalten. Die erste Rohprodukt-Fraktion wurde in 200 ml Ethylacetat suspendiert und 5 min bei RT gerührt, bevor 100 ml Cyclohexan zugesetzt wurden. Nachdem das heterogene Gemisch über Nacht bei RT gerührt worden war, wurde der Feststoff abgesaugt und im Hochvakuum getrocknet. Dies ergab eine erste reine Fraktion der Titelverbindung (19.62 g). Mit der zweiten Rohprodukt-Fraktion wurde in gleicher Weise unter Verwendung von 100 ml Ethylacetat und 50 ml Cyclohexan verfahren. Dies ergab die zweite reine Fraktion der Titelverbindung (2.75 g). Die zuvor erhaltenen Ethylacetat/Cyclohexan-Filtrate wurden vereinigt und zur Trockene eingeengt. Der verbliebene Rückstand (17.64 g) wurde mittels präparativer HPLC gereinigt (Methode 15). Eindampfen der Produktfraktionen und Trocknen im Hochvakuum ergab eine dritte reine Produktfraktion (5.02 g). Insgesamt wurden so 27.39 g (55% d. Th.) der Titelverbindung erhalten.

*Methode C:*

1.43 g (4.25 mmol) der Verbindung aus Bsp. 201A wurden in Methanol gelöst und über eine Hydrogencarbonat-Kartusche (Fa. Polymerlabs, Stratospheres SPE, PL-$HCO_3$ MP SPE) gegeben. Nach Eindampfen der Lösung wurden 1.25 g des freien Amins (Verbindung aus Bsp. 200A) erhalten. Das Amin wurde in einem Gemisch aus 29 ml DMF und 14 ml THF gelöst und mit 380 μl (4.46 mmol) 2-Chlorethylisocyanat versetzt. Nach 2 h Rühren wurden 715 mg (6.37 mmol) Kalium-*tert.*-butylat zum Reaktionsgemisch hinzugefügt. Nach weiteren 30 min wurde mit n-Hexan verdünnt und nacheinander mit gesättigter Natriumhydrogencarbonat-Lösung und gesättigter Natriumchlorid-Lösung gewaschen. Die organische Phase wurde über wasserfreiem Magnesiumsulfat getrocknet, filtriert und eingeengt. Dies ergab eine erste Fraktion des Rohprodukts. Die vereinigten wässrigen Phasen wurden noch einmal mit Ethylacetat extrahiert. Nach Trocknen des Extrakts über wasserfreiem Magnesiumsulfat, Filtrieren und Einengen wurde eine zweite Rohprodukt-Fraktion erhalten. Die Rohprodukte wurden mittels präparativer HPLC gereinigt (Methode 8). Nach Vereinigen der Produktfraktionen und Trocknen im Hochvakuum wurden 514 mg (31% d. Th., 95% Reinheit) der Titelverbindung erhalten.

*Kristallisation der Titelverbindung:*

6.5 g der Titelverbindung wurden mit 135 ml Wasser/Ethanol (95:5) versetzt und zum beginnenden Rückfluss erhitzt. Nachdem alles in Lösung gegangen war, wurde die Ölbadtemperatur auf 70°C heruntergeregelt. Dabei begann die Verbindung auszufallen. Es wurde über Nacht bei 70°C langsam gerührt. Am nächsten Morgen wurde die Ölbadtemperatur auf 50°C heruntergeregelt und 3 h bei dieser Temperatur gerührt. Dann wurde die Heizung abgeschaltet und das Gemisch im Ölbad unter Rühren langsam auf RT kommen gelassen. Nach ca. 4 h war RT erreicht, und das Gemisch wurde danach weitere 2.5 Tage bei RT ohne Rühren stehen gelassen. Die Verbindung wurde dann abgesaugt, mit wenig Wasser/Ethanol (95:5) nachgewaschen und anschließend bei 4 mbar und 50°C getrocknet. Es wurden so 6.02 g (93% d. Th.) der Titelverbindung als weisser kristalliner Feststoff erhalten. Schmelzpunkt: 165-167°C.
$^1$H-NMR (500 MHz, DMSO-$d_6$, $\delta$/ppm): 6.53 (s, 1H), 4.35 (s, 2H), 4.02 (t, 2H), 3.90 (q, 2H), 3.63 (t, 2H), 3.28-3.17 (m, 4H), 3.24 (s, 3H), 2.39 (s, 3H), 1.12 (t, 3H).
LC/MS (Methode 1, ESIpos): $R_t$ = 0.65 min, m/z = 367 [M+H]$^+$.

**Beispiel 60**

1-(2-Ethoxyethyl)-3-ethyl-5-methyl-6-[(2-oxoimidazolidin-1-yl)methyl]thieno[2,3-d]pyrimidin-2,4(1*H*,3*H*)-dion

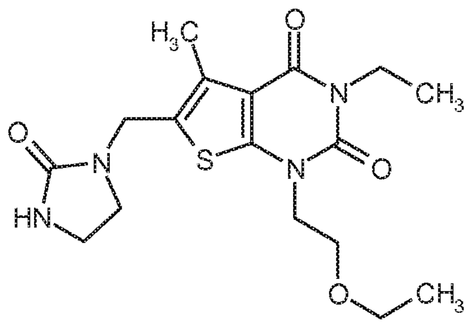

164 mg (0.333 mmol) der Verbindung aus Bsp. 221A wurden in 7 ml Dioxan gelöst und mit 83.5 mg (0.5 mmol) CDI versetzt. Das Gemisch wurde 20 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 92 mg (72% d. Th.) der Titelverbindung erhalten.
$^1$H-NMR (400 MHz, DMSO-$d_6$, $\delta$/ppm): 6.53 (s, 1H), 4.35 (s, 2H), 4.01 (t, 2H), 3.90 (q, 2H), 3.65 (t, 2H), 3.43 (q, 2H), 3.27-3.16 (m, 4H), 2.39 (s, 3H), 1.11 (t, 3H), 1.03 (t, 3H).
LC/MS (Methode 3): $R_t$ = 0.93 min, m/z = 381 [M+H]$^+$.

**Beispiel 61**

3-Ethyl-1-(2-isopropoxyethyl)-5-methyl-6-[(2-oxoimidazolidin-1-yl)methyl]thieno[2,3-d]pyrimidin-2,4(1*H*,3*H*)-dion

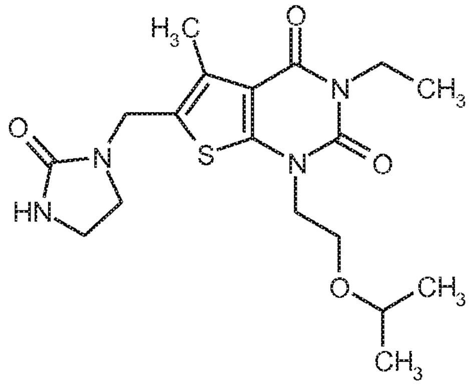

208 mg (0.344 mmol) der Verbindung aus Bsp. 222A wurden in 7 ml Dioxan gelöst und mit 86.3 mg (0.516 mmol) CDI versetzt. Das Gemisch wurde 16 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 72 mg (52% d. Th.) der Titelverbindung erhalten.
$^1$H-NMR (400 MHz, DMSO-$d_6$, δ/ppm): 6.51 (s, 1H), 4.35 (s, 2H), 3.97 (t, 2H), 3.90 (q, 2H), 3.65 (t, 2H), 3.54 (sept, 1H), 3.26-3.16 (m, 4H), 2.39 (s, 3H), 1.11 (t, 3H), 1.00 (d, 6H).
LC/MS (Methode 3): $R_t$ = 0.99 min, m/z = 395 $[M+H]^+$.

## Beispiel 62

3-Ethyl-1-(2-methoxypropyl)-5-methyl-6-[(2-oxoimidazolidin-1-yl)methyl]thieno[2,3-d]pyrimidin-2,4(1*H*,3*H*)-dion (*Racemat*)

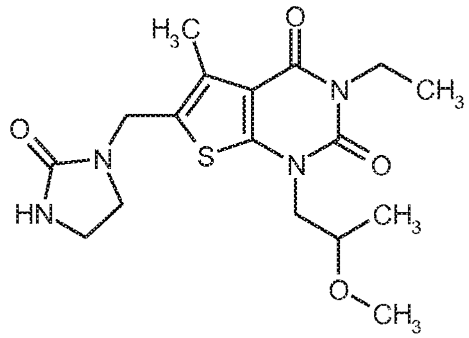

Eine Lösung von 420 mg (1.02 mmol, 86% Reinheit) der Verbindung aus Bsp. 223A und 248 µl (1.78 mmol) Triethylamin in 12 ml THF wurde mit 230 mg (1.42 mmol) CDI versetzt und ca. 18 h bei RT gerührt. Anschließend wurde zur Trockene eingeengt. Der verbliebene Rückstand wurde in Ethylacetat aufgenommen und nacheinander mit 1 M Salzsäure, Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) gereinigt. Nach Vereinigen der Produktfraktionen, Eindampfen und Trocknen im Hochvakuum wurden 219 mg (55% d. Th.) der Titelverbindung erhalten.
$^1$H-NMR (400 MHz, DMSO-$d_6$, δ/ppm): 6.52 (s, 1H), 4.34 (d, 2H), 4.00-3.84 (m, 3H), 3.79-3.66 (m, 2H), 3.27-3.19 (m, 4H), 3.18 (s, 3H), 2.39 (s, 3H), 1.20-1.03 (m, 6H).
LC/MS (Methode 1, ESIpos): $R_t$ = 0.73 min, m/z = 381 $[M+H]^+$.

**Beispiel 63**

3-Ethyl-1-(2-methoxypropyl)-5-methyl-6-[(2-oxoimidazolidin-1-yl)methyl]thieno[2,3-d]pyrimidin-2,4(1*H*,3*H*)-dion (*Enantiomer 1*)

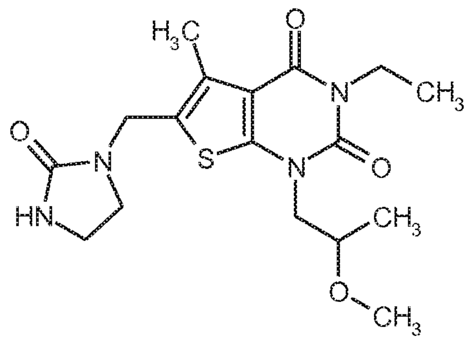

207 mg (0.544 mmol) der racemischen Verbindung aus Bsp. 62 wurden in 5 ml Ethanol gelöst und in 10 Portionen über präparative HPLC an chiraler Phase in die Enantiomere aufgetrennt [Säule: Daicel Chiralpak AD-H, 5 µm, 250 mm x 20 mm; Laufmittel: Isohexan/Ethanol 1:1; Fluss: 15 ml/ min; Temperatur: 25°C; Detektion: 210 nm]. Nach Eindampfen der Produktfraktionen und Trocknen im Hochvakuum wurden 58 mg (56% d. Th.) von Enantiomer 1 erhalten (90.9% ee, chirale analytische HPLC).
$^1$H-NMR (400 MHz, DMSO-$d_6$, δ/ppm): 6.52 (s, 1H), 4.34 (d, 2H), 4.00-3.84 (m, 3H), 3.81-3.65 (m, 2H), 3.27-3.19 (m, 4H), 3.18 (s, 3H), 2.39 (s, 3H), 1.19-0.98 (m, 6H).
LC/MS (Methode 1, ESIpos): $R_t$ = 0.76 min, m/z = 381 $[M+H]^+$.
Chirale analytische HPLC [Säule: Daicel Chiralpak AD-H, 5 µm, 250 mm x 4.6 mm; Laufmittel: Isohexan/Ethanol 1:1; Fluss: 1 ml/min; Temperatur: 40°C; Detektion: 220 nm]: $R_t$ = 9.02 min.

**Beispiel 64**

3-Ethyl-1-(2-methoxypropyl)-5-methyl-6-[(2-oxoimidazolidin-1-yl)methyl]thieno[2,3-d]pyrimidin-2,4(1*H*,3*H*)-dion (*Enantiomer 2*)

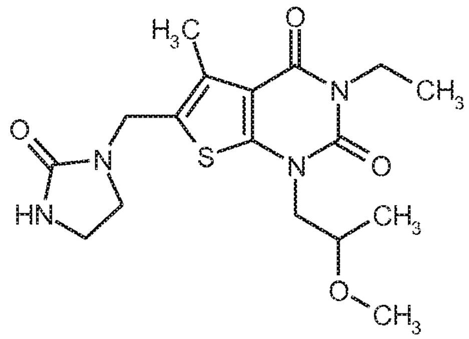

207 mg (0.544 mmol) der racemischen Verbindung aus Bsp. 62 wurden in 5 ml Ethanol gelöst und in 10 Portionen über präparative HPLC an chiraler Phase in die Enantiomere aufgetrennt [Säule: Daicel Chiralpak AD-H, 5 µm, 250 mm x 20 mm; Laufmittel: Isohexan/Ethanol 1:1; Fluss: 15 ml/ min; Temperatur: 25°C; Detektion: 210 nm]. Nach Eindampfen der Produktfraktionen und Trocknen im Hochvakuum wurden 86 mg (83% d. Th.) von Enantiomer 2 erhalten (>99% ee, chirale analytische HPLC).
$^1$H-NMR (400 MHz, DMSO-$d_6$, δ/ppm): 6.52 (s, 1H), 4.34 (d, 2H), 4.01-3.85 (m, 3H), 3.81-3.67 (m, 2H), 3.28-3.19 (m, 4H), 3.18 (s, 3H), 2.39 (s, 3H), 1.19-1.06 (m, 6H).
LC/MS (Methode 1, ESIpos): $R_t$ = 0.76 min, m/z = 381 $[M+H]^+$.
Chirale analytische HPLC [Säule: Daicel Chiralpak AD-H, 5 µm, 250 mm x 4.6 mm; Laufmittel: Isohexan/Ethanol

1:1; Fluss: 1 ml/min; Temperatur: 40°C; Detektion: 220 nm]: $R_t$ = 6.55 min.

**Beispiel 65**

3-Ethyl-5-methyl-1-(oxetan-2-ylmethyl)-6-[(2-oxoimidazolidin-1-yl)methyl]thieno[2,3-d]pyrimidin-2,4(1*H*,3*H*)-dion (*Racemat*)

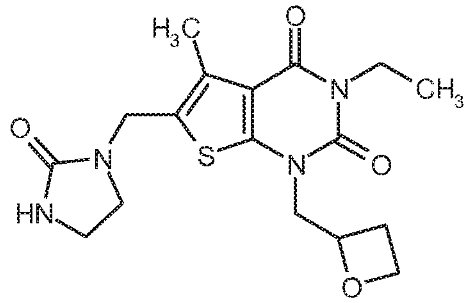

358 mg (1.016 mmol) der Verbindung aus Bsp. 224A wurden in 18 ml Dioxan gelöst und mit 254 mg (1.524 mmol) CDI versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 140 mg (36% d. Th.) der Titelverbindung erhalten.
$^1$H-NMR (400 MHz, DMSO-$d_6$, δ/ppm): 6.51 (s, 1H), 5.05-4.96 (m, 1H), 4.52-4.38 (m, 2H), 4.34 (s, 2H), 4.13 (d, 2H), 3.90 (q, 2H), 3.28-3.17 (m, 4H), 2.74-2.64 (m, 1H), 2.39 (s, 3H), 1.12 (t, 3H).
LC/MS (Methode 3): $R_t$ = 0.85 min, m/z = 379 $[M+H]^+$.

**Beispiel 66**

3-Ethyl-5methyl-1-(oxetan-2-ylmethyl)-6-[(2-oxoimidazolidin-1-yl)methyl]thieno[2,3-d]pyrimidin-2,4(1*H*1,3*H*)-dion (*Enantiomer 1*)

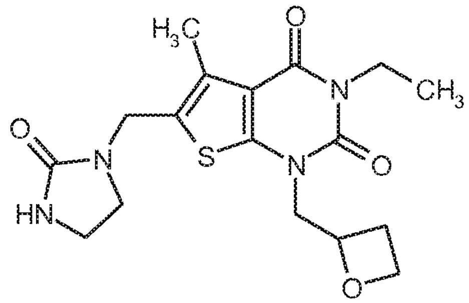

110 mg der racemischen Verbindung aus Bsp. 65 wurden in 1 ml Methanol und 1 ml Dichlormethan gelöst und mittels präparativer SFC-HPLC an chiraler Phase in die Enantiomere getrennt [Säule: Chiralpak IB, 5 μm, 250 mm x 30 mm; Eluent: Kohlendioxid/Ethanol 85:15; Puffer: 0.2% Diethylamin; Fluss: 100 ml/min; Druck (outlet): 150 bar; Temperatur: 40°C; DAD 254 nm]. Die jeweiligen Produktfraktionen wurden am Rotationsverdampfer eingeengt, mit tert.-Butanol versetzt und gefriergetrocknet. Es wurden 46 mg (41% d. Th.) von Enantiomer 1 sowie 46 mg (41% d. Th.) von Enantiomer 2 (*siehe Beispiel 67*) erhalten.
$^1$H-NMR (400 MHz, DMSO-$d_6$, δ/ppm): 6.51 (s, 1H), 5.04-4.96 (m, 1H), 4.52-4.38 (m, 2H), 4.34 (s, 2H), 4.13 (d, 2H), 3.90 (q, 2H), 3.28-3.17 (m, 4H), 2.69 (dtd, 1H), 2.39 (s, 3H), 1.15-1.08 (m, 3H).
Chirale analytische SFC-HPLC [Säule: Chiralpak IB, 5 μm, 100 mm x 4.6 mm; Eluent: Kohlendioxid/Ethanol 85:15; Puffer: 0.2% Diethylamin; Fluss: 1 ml/min; Temperatur: 40°C; Detektion: 220 nm]: $R_t$ = 3.12 min.

**Beispiel 67**

3-Ethyl-5-oxetan-2-ylmethyl)-6-[(2-oxoimidazolidin-1-yl)methyl]thieno[2,3-d]pyrimidin-2,4(1*H*,3*H*)-dion (*Enantiomer 2*)

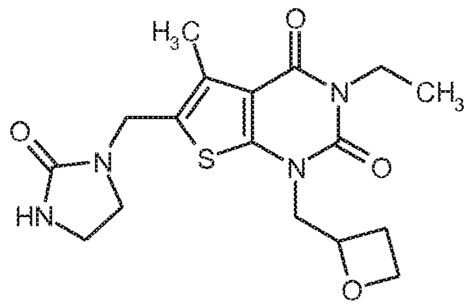

Die Titelverbindung (46 mg) wurde als zweites Enantiomer bei der in Beispiel 66 beschriebenen Racemattrennung von Bsp. 65 mittels präparativer SFC-HPLC erhalten.
$^1$H-NMR (400 MHz, DMSO-$d_6$, δ/ppm): 6.52 (s, 1H), 5.05-4.95 (m, 1H), 4.52-4.38 (m, 2H), 4.34 (s, 2H), 4.13 (d, 2H), 3.90 (q, 2H), 3.29-3.17 (m, 4H), 2.69 (dtd, 1H), 2.39 (s, 3H), 1.16-1.07 (m, 4H).

Chirale analytische SFC-HPLC [Säule: Chiralpak IB, 5 μm, 100 mm x 4.6 mm; Eluent: Kohlendioxid/Ethanol 85:15; Puffer: 0.2% Diethylamin; Fluss: 1 ml/min; Temperatur: 40°C; Detektion: 220 nm]: $R_t$ = 3.74 min.

**Beispiel 68**

3-Ethyl-5-methyl-6-[(2-oxoimidazolidin-1-yl)methyl]-1-(tetrahydrofuran-2-ylmethyl)thieno[2,3-d]-pyrimidin-2,4(1*H*,3*H*)-dion (*Racemat*)

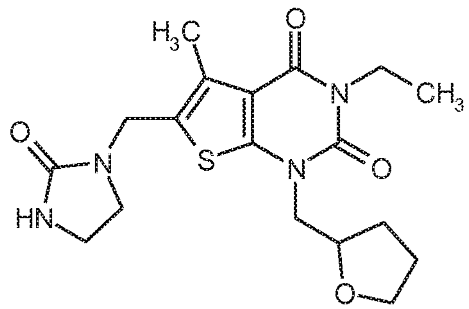

Eine Lösung von 220 mg (0.450 mmol, 75% Reinheit) der Verbindung aus Bsp. 225A und 125 μl (0.90 mmol) Triethylamin in 6 ml THF wurde mit 117 mg (0.720 mmol) CDI versetzt und ca. 18 h bei RT gerührt. Anschließend wurde zur Trockene eingeengt. Der verbliebene Rückstand wurde in Ethylacetat aufgenommen und nacheinander mit 1 M Salzsäure, Wasser und gesättigter Natriumchlorid-Lösung gewaschen. Nach Trocknen über wasserfreiem Magnesiumsulfat wurde filtriert und eingeengt. Der feste Rückstand wurde bei RT in wenig Acetonitril verrührt. Nach Filtration und Trocknen des Feststoffs im Hochvakuum wurde eine erste Fraktion der Titelverbindung erhalten (554 mg). Das Filtrat wurde zur Trockene eingeengt und mittels präparativer HPLC (Methode 8) in seine Komponenten aufgetrennt. Nach Eindampfen der Produktfraktionen und Trocknen im Hochvakuum wurde eine zweite Fraktion der Titelverbindung gewonnen (405 mg). Insgesamt wurden so 959 mg (56% d. Th.) der Titelverbindung erhalten.
$^1$H-NMR (400 MHz, DMSO-$d_6$, δ/ppm): 6.51 (s, 1H), 4.34 (s, 2H), 4.27-4.16 (m, 1H), 4.03 (dd, 1H), 3.90 (q, 2H), 3.79-3.66 (m, 2H), 3.61 (q, 1H), 3.28-3.14 (m, 4H), 2.39 (s, 3H), 2.05-1.74 (m, 3H), 1.72-1.59 (m, 1H), 1.12 (t, 3H).
LC/MS (Methode 1, ESIpos): $R_t$ = 0.73 min, m/z = 393 $[M+H]^+$.

**Beispiel 69**

3-Ethyl-5-methyl-6-[(2-oxoimidazolidin-1-yl)methyl]-1-(tetrahydrofuran-2-ylmethyl)thieno[2,3-d]-pyrimidin-2,4(1*H*,3*H*)-dion (*Enantiomer 1*)

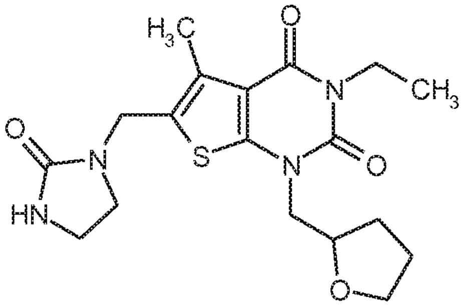

955 mg (2.43 mmol) der racemischen Verbindung aus Bsp. 68 wurden in einem Gemisch aus 8 ml Ethanol und 3 ml Ameisensäure gelöst und in 22 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 25°C; Detektion: 210 nm]. Nach Eindampfen der Produktfraktionen und Trocknen im Hochvakuum wurden 320 mg (67% d. Th.) von Enantiomer 1 erhalten (>99% ee, chirale analytische HPLC).
$^1$H-NMR (400 MHz, DMSO-$d_6$, δ/ppm): 6.52 (s, 1H), 4.34 (s, 2H), 4.27-4.17 (m, 1H), 4.03 (dd, 1H), 3.90 (q, 2H), 3.79-3.66 (m, 2H), 3.65-3.57 (m, 1H), 3.28-3.15 (m, 4H), 2.39 (s, 3H), 2.04-1.74 (m, 3H), 1.71-1.59 (m, 1H), 1.12 (t, 3H).
Chirale analytische HPLC [Säule: Daicel Chiralcel OX-H, 3 μm, 50 mm x 4.6 mm; Laufmittel: Ethanol; Fluss: 1 ml/min; Temperatur: 40°C; Detektion: 220 nm]: $R_t$ = 2.85 min.

**Beispiel 70**

3-Ethyl-5-methyl-6-[(2-oxoimidazolidin-1-yl)methyl]-1-(tetrahydrofuran-2-ylmethyl)thieno[2,3-d]-pyrimidin-2,4(1*H*,3*H*)-dion (*Enantiomer 2*)

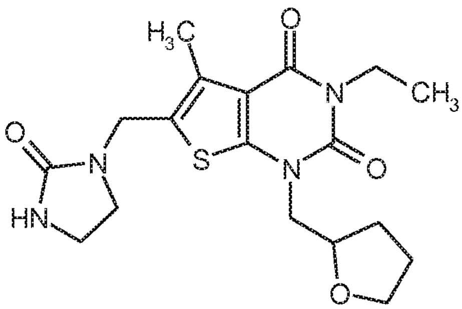

955 mg (2.43 mmol) der racemischen Verbindung aus Bsp. 68 wurden in einem Gemisch aus 8 ml Ethanol und 3 ml Ameisensäure gelöst und in 22 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 25°C; Detektion: 210 nm]. Nach Eindampfen der Produktfraktionen und Trocknen im Hochvakuum wurden 194 mg (40% d. Th.) von Enantiomer 2 erhalten (>99% ee, chirale analytische HPLC).
$^1$H-NMR (400 MHz, DMSO-$d_6$, δ/ppm): 6.52 (s, 1H), 4.34 (s, 2H), 4.27-4.17 (m, 1H), 4.03 (dd, 1H), 3.90 (q, 2H), 3.79-3.66 (m, 2H), 3.65-3.56 (m, 1H), 3.28-3.15 (m, 4H), 2.39 (s, 3H), 2.05-1.73 (m, 3H), 1.70-1.61 (m, 1H), 1.12 (t, 3H).
Chirale analytische HPLC [Säule: Daicel Chiralcel OX-H, 3 μm, 50 mm x 4.6 mm; Laufmittel: Ethanol; Fluss: 1 ml/min; Temperatur: 40°C; Detektion: 220 nm]: $R_t$ = 3.17 min.

**Beispiel 71**

3-Ethyl-5-methyl-6-[(2-oxoimidazolidin-1-yl)methyl]-1-(tetrahydro-2*H*-pyran-2-ylmethyl)thieno-[2,3-d]pyrimidin-2,4(1*H*,3*H*)-dion (*Racemat*)

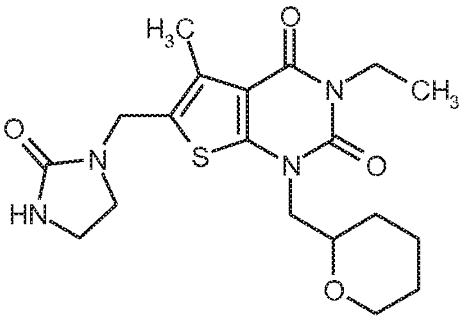

320 mg (0.732 mmol) der Verbindung aus Bsp. 226A wurden in 14 ml Dioxan gelöst und mit 178 mg (1.097 mmol) CDI versetzt. Das Gemisch wurde 18 h bei RT gerührt. Die Reaktionslösung wurde danach am Rotationsverdampfer eingeengt. Der Rückstand wurde in 9 ml DMSO gelöst und diese Lösung mittels präparativer HPLC gereinigt (Methode 14). Nach Vereinigen der Produktfraktionen und Gefriertrocknung wurden 226 mg (79% d. Th.) der Titelverbindung erhalten.
$^1$H-NMR (400 MHz, DMSO-$d_6$, δ/ppm): 6.53 (s, 1H), 4.38-4.28 (m, 2H), 4.02-3.93 (m, 1H), 3.89 (q, 2H), 3.80 (dd, 1H), 3.73-3.63 (m, 2H), 3.29-3.17 (m, 5H), 2.38 (s, 3H), 1.78 (d, 1H), 1.61 (d, 1H), 1.51-1.38 (m, 3H), 1.31-1.19 (m, 1H), 1.11 (t, 3H).
LC/MS (Methode 3): $R_t$ = 1.02 min, m/z = 407 [M+H]$^+$.

**Beispiel 72**

3-Ethyl-5-methyl-1-(oxetan-3-ylmethyl)-6-[(2-oxoimidazolidin-1-yl)methyl]thieno[2,3-d]pyrimidin-2,4(1*H*,3*H*)-dion

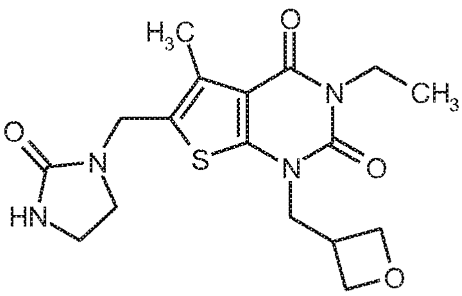

Analog zu dem unter Bsp. 62 beschriebenen Verfahren wurden aus 105 mg (0.238 mmol, 80% Reinheit) der Verbindung aus Bsp. 227A und 58 mg (0.357 mmol) CDI 16 mg (17% d. Th.) der Titelverbindung hergestellt.
$^1$H-NMR (400 MHz, DMSO-$d_6$, δ/ppm): 6.53 (s, 1H), 4.62 (dd, 2H), 4.44 (t, 2H), 4.35 (s, 2H), 4.19 (d, 2H), 3.89 (q, 2H), 3.49-3.37 (m, 1H), 3.27-3.16 (m, 4H), 2.39 (s, 3H), 1.11 (t, 3H).
LC/MS (Methode 1, ESIpos): $R_t$ = 0.68 min, m/z = 379 [M+H]$^+$.

**Beispiel 73**

3-Ethyl-5-methyl-6-[(2-oxoimidazolidin-1-yl)methyl]-1-(tetrahydrofuran-3-ylmethyl)thieno[2,3-d]-pyrimidin-2,4(1*H*,3*H*)-dion (*Racemat*)

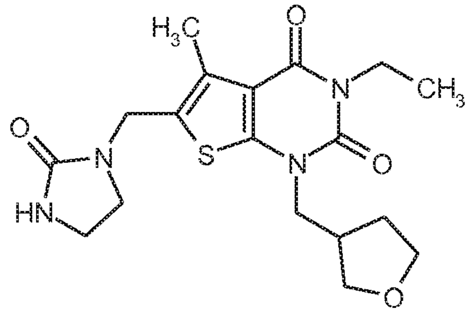

Analog zu dem in Bsp. 68 beschriebenen Verfahren wurden aus 211 mg (0.504 mmol, 87% Reinheit) der Verbindung aus Bsp. 228A und 98 mg (0.605 mmol) CDI 131 mg (66% d. Th.) der Titelverbindung erhalten.
$^1$H-NMR (400 MHz, DMSO-$d_6$, δ/ppm): 6.53 (s, 1H), 4.36 (s, 2H), 3.97-3.84 (m, 4H), 3.80 (td, 1H), 3.70-3.57 (m, 2H), 3.51 (dd, 1H), 3.28-3.16 (m, 4H), 2.80-2.69 (m, 1H), 2.40 (s, 3H), 2.03-1.87 (m, 1H), 1.73-1.59 (m, 1H), 1.12 (t, 3H).
LC/MS (Methode 1, ESIpos): $R_t$ = 0.67 min, m/z = 393 $[M+H]^+$.

**Beispiel 74**

3-Ethyl-5-methyl-6-[(2-oxoimidazolidin-1-yl)methyl]-1-(tetrahydrofuran-3-ylmethyl)thieno[2,3-d]-pyrimidin-2,4(1*H*,3*H*)-dion (*Enantiomer 1*)

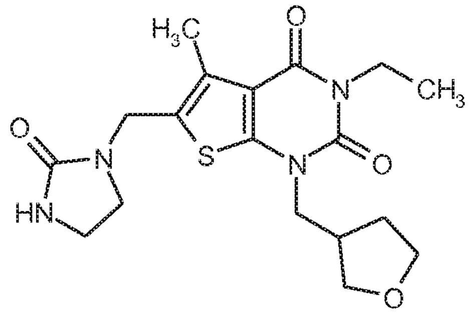

124 mg (0.316 mmol) der racemischen Verbindung aus Bsp. 73 wurden in 22 ml Ethanol gelöst und in 55 Portionen über präparative SFC-HPLC an chiraler Phase in die Enantiomere aufgetrennt [Säule: Daicel Chiralcel OJ-H, 5 μm, 250 mm x 20 mm; Laufmittel: Kohlendioxid/Ethanol 80:20; Fluss: 80 ml/min; Temperatur: 40°C; Detektion: 210 nm]. Nach Eindampfen der Produktfraktionen und Trocknen im Hochvakuum wurden 52 mg (83% d. Th.) von Enantiomer 1 erhalten (>99% ee, chirale analytische HPLC).
$^1$H-NMR (400 MHz, DMSO-$d_6$, δ/ppm): 6.53 (s, 1H), 4.36 (s, 2H), 3.96-3.85 (m, 4H), 3.80 (td, 1H), 3.70-3.57 (m, 2H), 3.51 (dd, 1H), 3.28-3.16 (m, 4H), 2.81-2.67 (m, 1H), 2.40 (s, 3H), 2.03-1.88 (m, 1H), 1.72-1.58 (m, 1H), 1.12 (t, 3H).
Chirale analytische SFC-HPLC [Säule: Daicel Chiralcel OJ-3, 3 μm, 50 mm x 4.6 mm; Laufmittel: Kohlendioxid/Ethanol, Ethanol-Gradient 5% → 60% in 6 min; Fluss: 3 ml/min; Temperatur: 40°C; Detektion: 220 nm]: $R_t$ = 2.36 min.

**Beispiel 75**

3-Ethyl-5-methyl-6-[(2-oxoimidazolidin-1-yl)methyl]-1-(tetrahydrofuran-3-ylmethyl)thieno[2,3-d]-pyrimidin-2,4(1*H*,3*H*)-dion (*Enantiomer 2*)

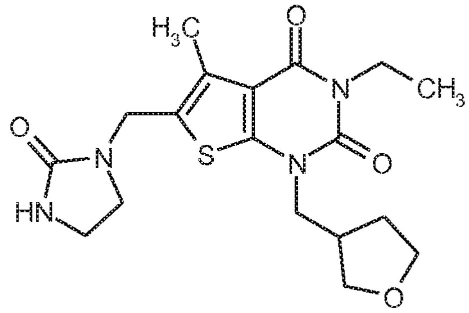

124 mg (0.316 mmol) der racemischen Verbindung aus Bsp. 73 wurden in 22 ml Ethanol gelöst und in 55 Portionen über präparative SFC-HPLC an chiraler Phase in die Enantiomere aufgetrennt [Säule: Daicel Chiralcel OJ-H, 5 μm, 250 mm x 20 mm; Laufmittel: Kohlendioxid/Ethanol 80:20; Fluss: 80 ml/min; Temperatur: 40°C; Detektion: 210 nm]. Nach Eindampfen der Produktfraktionen und Trocknen im Hochvakuum wurden 50 mg (80% d. Th.) von Enantiomer 2 erhalten (>99% ee, chirale analytische HPLC).
$^1$H-NMR (400 MHz, DMSO-$d_6$, δ/ppm): 6.53 (s, 1H), 4.36 (s, 2H), 3.96-3.85 (m, 4H), 3.80 (td, 1H), 3.69-3.58 (m, 2H), 3.51 (dd, 1H), 3.29-3.15 (m, 4H), 2.81-2.68 (m, 1H), 2.40 (s, 3H), 2.01-1.87 (m, 1H), 1.73-1.57 (m, 1H), 1.12 (t, 3H).
Chirale analytische SFC-HPLC [Säule: Daicel Chiralcel OJ-3, 3 μm, 50 mm x 4.6 mm; Laufmittel: Kohlendioxid/Ethanol, Ethanol-Gradient 5% → 60% in 6 min; Fluss: 3 ml/min; Temperatur: 40°C; Detektion: 220 nm]: $R_t$ = 2.21 min.

## Beispiel 76

3-Ethyl-1,5-dimethyl-6-[(2-oxoimidazolidin-1-yl)methyl]thieno[2,3-d]pyrimidin-2,4(1*H*,3*H*)-dion

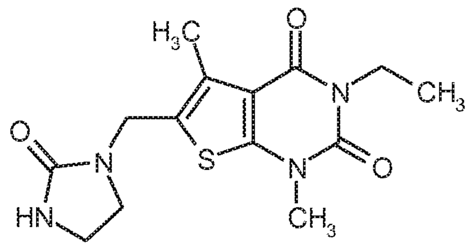

Analog zu dem in Bsp. 68 beschriebenen Verfahren wurden aus 370 mg (0.949 mmol, 76% Reinheit) der Verbindung aus Bsp. 229A und 185 mg (1.14 mmol) CDI 174 mg (56% d. Th.) der Titelverbindung erhalten.
$^1$H-NMR (400 MHz, DMSO-$d_6$, δ/ppm): 6.52 (br. s, 1H), 4.36 (s, 2H), 3.90 (q, 2H), 3.41 (s, 3H), 3.28-3.15 (m, 4H), 2.40 (s, 3H), 1.11 (t, 3H).
LC/MS (Methode 1, ESIpos): $R_t$ = 0.66 min, m/z = 323 $[M+H]^+$.

## Beispiel 77

3-Ethyl-5-methyl-6-[(2-oxoimidazolidin-1-yl)methyl]-1-propylthieno[2,3-d]pyrimidin-2,4(1*H*,3*H*)-dion

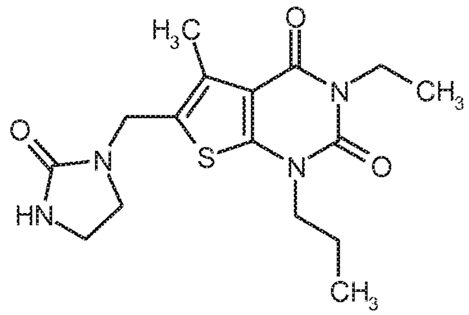

Eine Lösung von 280 mg (0.604 mmol, 70% Reinheit) der Verbindung aus Bsp. 231A und 126 µl (0.906 mmol) Triethylamin in 5 ml THF wurde mit 117 mg (0.725 mmol) CDI versetzt und ca. 18 h bei RT gerührt. Anschließend wurde das Reaktionsgemisch direkt mittels präparativer HPLC (Methode 8) in seine Komponenten aufgetrennt. Nach Eindampfen der Produktfraktionen und Trocknen im Hochvakuum wurde ein Rohprodukt erhalten, das mit wenig Acetonitril bei RT verrührt wurde. Nach Absaugen und Trocknen im Hochvakuum ergab der Feststoff eine erste Fraktion der Titelverbindung (25 mg). Das Filtrat wurde eingeengt und nochmals mittels präparativer HPLC (Methode 8) gereinigt. Dies ergab eine zweite Fraktion der Titelverbindung (35 mg). Insgesamt wurden so 60 mg (28% d. Th.) der Titelverbindung erhalten.
$^1$H-NMR (400 MHz, DMSO-$d_6$, δ/ppm): 6.52 (s, 1H), 4.35 (s, 2H), 3.90 (q, 2H), 3.85-3.78 (m, 2H), 3.29-3.16 (m, 4H), 2.40 (s, 3H), 1.69 (sext, 2H), 1.11 (t, 3H), 0.91 (t, 3H).
LC/MS (Methode 1, ESIpos): $R_t$ = 0.82 min, m/z = 351 $[M+H]^+$.

**Beispiel 78**

1-Butyl-3-ethyl-5-methyl-6-[(2-oxoimidazolidin-1-yl)methyl]thieno[2,3-d]pyrimidin-2,4(1*H*,3*H*)-dion

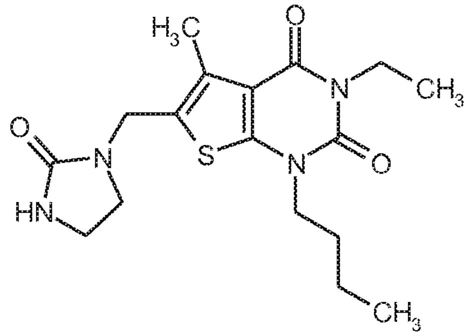

Analog zu dem unter Bsp. 57 beschriebenen Verfahren wurden aus 380 mg (0.730 mmol, 65% Reinheit) der Verbindung aus Bsp. 232A und 218 mg (1.35 mmol) CDI 30 mg (7% d. Th.) der Titelverbindung hergestellt. Die Reinigung des Produkts erfolgte hier durch zweimalige präparative HPLC nach Methode 8.
$^1$H-NMR (400 MHz, DMSO-$d_6$, δ/ppm): 6.52 (s, 1H), 4.35 (s, 2H), 3.97-3.80 (m, 4H), 3.28-3.16 (m, 4H), 2.40 (s, 3H), 1.65 (quin, 2H), 1.34 (sext, 2H), 1.11 (t, 3H), 0.91 (t, 3H).
LC/MS (Methode 1, ESIpos): $R_t$ = 0.86 min, m/z = 365 $[M+H]^+$.

**Beispiel 79**

3-Ethyl-5-methyl-1-(3-methylbutyl)-6-[(2-oxoimidazolidin-1-yl)methyl]thieno[2,3-d]pyrimidin-2,4(1*H*,3*H*)-dion

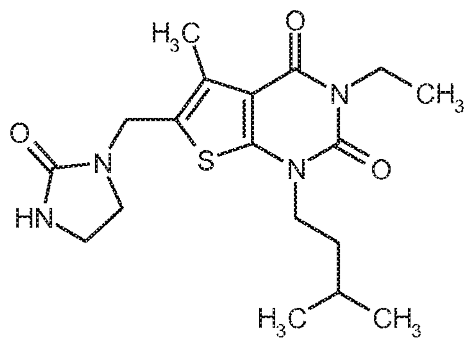

Analog zu dem unter Bsp. 47 beschriebenen Verfahren wurden aus 360 mg (0.919 mmol, 90% Reinheit) der Verbindung aus Bsp. 233A und 179 mg (1.10 mmol) CDI 154 mg (44% d. Th.) der Titelverbindung hergestellt.
$^1$H-NMR (400 MHz, DMSO-$d_6$, δ/ppm): 6.52 (s, 1H), 4.35 (s, 2H), 3.90 (q, 2H), 3.86 (t, 2H), 3.28-3.15 (m, 4H), 2.40 (s,

3H), 1.68-1.59 (m, 1H), 1.57-1.52 (m, 2H), 1.11 (t, 3H), 0.94 (d, 6H).
LC/MS (Methode 1, ESIpos): $R_t$ = 0.89 min, m/z = 379 $[M+H]^+$.

**Beispiel 80**

1-(Cyclobutylmethyl)-3-ethyl-5-methyl-6-[(2-oxoimidazolidin-1-yl)methyl]thieno[2,3-d]pyrimidin-2,4(1*H*,3*H*)-dion

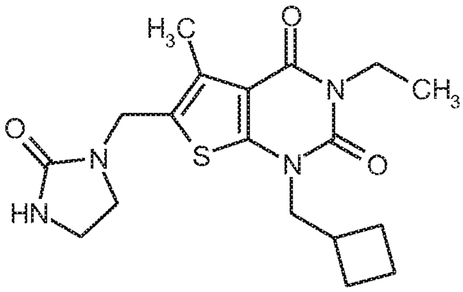

Analog zu dem unter Bsp. 47 beschriebenen Verfahren wurden aus 350 mg (0.909 mmol, 90% Reinheit) der Verbindung aus Bsp. 235A und 177 mg (1.09 mmol) CDI 234 mg (68% d. Th.) der Titelverbindung hergestellt.
$^1$H-NMR (400 MHz, DMSO-$d_6$, δ/ppm): 6.53 (s, 1H), 4.35 (s, 2H), 3.96-3.84 (m, 4H), 3.28-3.14 (m, 4H), 2.84-2.69 (m, 1H), 2.39 (s, 3H), 2.04-1.89 (m, 2H), 1.89-1.74 (m, 4H), 1.11 (t, 3H).
LC/MS (Methode 1, ESIpos): $R_t$ = 0.90 min, m/z = 377 $[M+H]^+$.

**Beispiel 81**

{3-Ethyl-5-methyl-2,4-dioxo-6-[(2-oxoimidazolidin-1-yl)methyl]-3,4-dihydrothieno[2,3-d]pyrimidin-1(2*H*)-yl}acetonitril

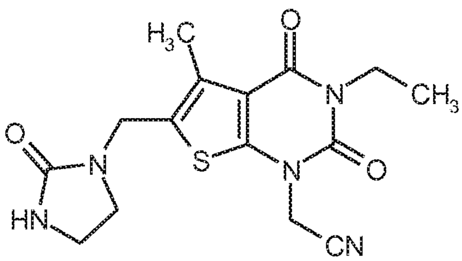

Analog zu dem unter Bsp. 57 beschriebenen Verfahren wurden aus 289 mg (0.719 mmol, 80% Reinheit) der Verbindung aus Bsp. 236A und 175 mg (1.08 mmol) CDI 15 mg (4% d. Th., 87% Reinheit) der Titelverbindung hergestellt. Zur weiteren Aufreinigung des Produktes nach der präparativen HPLC erfolgte hier noch eine Flash-Chromatographie über Kieselgel mit Ethylacetat als Laufmittel.
$^1$H-NMR (400 MHz, DMSO-$d_6$, δ/ppm): 6.56 (s, 1H), 5.12 (s, 2H), 4.39 (s, 2H), 3.90 (q, 2H), 3.28-3.16 (m, 4H), 2.41 (s, 3H), 1.13 (t, 3H).
LC/MS (Methode 1, ESIpos): $R_t$ = 0.70 min, m/z = 695 $[2M+H]^+$.

**Beispiel 82**

3-Ethyl-5-methyl-1-[(4-methyl-1,2,5-oxadiazol-3-yl)methyl]-6-[(2-oxoimidazolidin-1-yl)methyl]-thieno[2,3-d]pyrimidin-2,4(1*H*,3*H*)-dion

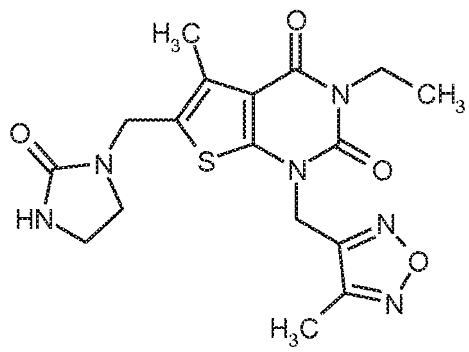

Analog zu dem unter Bsp. 62 beschriebenen Verfahren wurden aus 490 mg (1.06 mmol, 82% Reinheit) der Verbindung aus Bsp. 237A und 207 mg (1.27 mmol) CDI 162 mg (37% d. Th.) der Titelverbindung hergestellt.
$^1$H-NMR (400 MHz, DMSO-$d_6$, δ/ppm): 6.52 (s, 1H), 5.33 (s, 2H), 4.35 (s, 2H), 3.91 (q, 2H), 3.28-3.15 (m, 4H), 2.42 (s, 3H), 2.41 (s, 3H), 1.12 (t, 3H).
LC/MS (Methode 1, ESIpos): $R_t$ = 0.77 min, m/z = 405 $[M+H]^+$.

## Beispiel 83

1-[2-(Dimethylamino)ethyl]-3-ethyl-5-methyl-6-[(2-oxoimidazolidin-1-yl)methyl]thieno[2,3-d]-pyrimidin-2,4(1*H*,3*H*)-dion

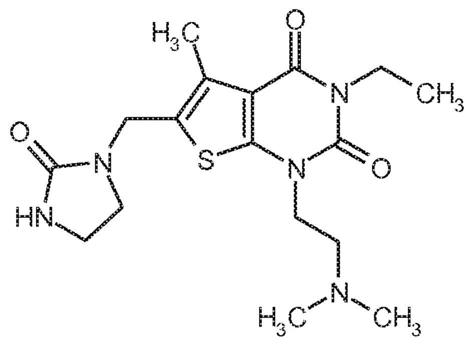

Analog zu dem unter Bsp. 47 beschriebenen Verfahren wurden aus 650 mg (1.34 mmol, 73% Reinheit) der Verbindung aus Bsp. 238A und 304 mg (1.88 mmol) CDI 212 mg (41% d. Th.) der Titelverbindung hergestellt.
$^1$H-NMR (400 MHz, DMSO-$d_6$, δ/ppm): 6.52 (s, 1H), 4.35 (s, 2H), 3.98-3.83 (m, 4H), 3.28-3.15 (m, 4H), 2.57-2.52 (m, 2H, teilweise überdeckt vom DMSO-Signal), 2.40 (s, 3H), 2.19 (s, 6H), 1.11 (t, 3H).
LC/MS (Methode 1, ESIpos): $R_t$ = 0.46 min, m/z = 380 $[M+H]^+$.

## Beispiel 84

1-(2-Methoxyethyl)-5-methyl-6-[(2-oxoimidazolidin-1-yl)methyl]-3-propylthieno[2,3-d]pyrimidin-2,4(1*H*,3*H*)-dion

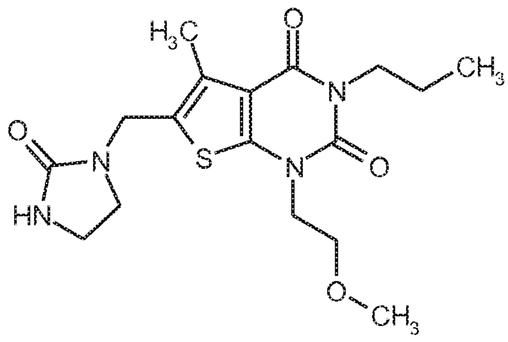

Eine Lösung von 106 mg (1.179 mmol) 2-Imidazolidinon in 4.2 ml THF wurde mit 47 mg (1.179 mmol) Natriumhydrid (60% Suspension in Mineralöl) versetzt und 3 h bei 60°C gerührt ("Lösung 1"). In einem anderen Reaktionsgefäß wurde eine Lösung von 93 mg (0.295 mmol) der Verbindung aus Bsp. 144A in 2 ml Dichlormethan bei 0°C mit 154 µl (0.884 mmol) *N,N*-Diisopropylethylamin und 32 µl (0.442 mmol) Thionylchlorid versetzt und 75 min gerührt. Anschließend wurde die Lösung 1 portionsweise zugesetzt und das Gemisch 66 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 10 g Kieselgel, Laufmittel Hexan/Ethylacetat). Es wurden 48 mg (42% d. Th.) der Titelverbindung erhalten.
$^1$H-NMR (400 MHz, DMSO-$d_6$, δ/ppm): 6.51 (s, 1H), 4.34 (s, 2H), 4.01 (t, 2H), 3.81 (dd, 2H), 3.62 (t, 2H), 3.28-3.17 (m, 7H), 2.39 (s, 3H), 1.55 (sext, 2H), 0.86 (t, 3H).
LC/MS (Methode 3): $R_t$ = 0.95 min, m/z = 381 $[M+H]^+$.

**Beispiel 85**

3-Allyl-1-(2-methoxyethyl)-5-methyl-6-[(2-oxoimidazolidin-1-yl)methyl]thieno[2,3-d]pyrimidin-2,4(1*H*,3*H*)-dion

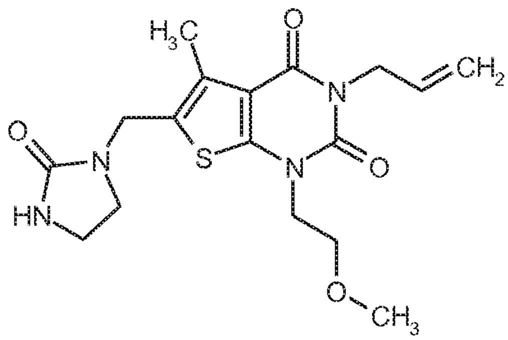

Eine Lösung von 191 mg (2.126 mmol) 2-Imidazolidinon in 7.6 ml THF wurde mit 85 mg (2.126 mmol) Natriumhydrid (60% Suspension in Mineralöl) versetzt und 3 h bei 60°C gerührt ("Lösung 1"). In einem anderen Reaktionsgefäß wurde eine Lösung von 165 mg (0.532 mmol) der Verbindung aus Bsp. 145A in 3.7 ml Dichlormethan bei 0°C mit 278 µl (1.595 mmol) *N,N*-Diisopropylethylamin und 58 µl (0.797 mmol) Thionylchlorid versetzt und 75 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 10 g Kieselgel, Laufmittel Hexan/Ethylacetat). Es wurden 33 mg (16% d. Th.) der Titelverbindung erhalten.
$^1$H-NMR (400 MHz, DMSO-$d_6$, δ/ppm): 6.55 (s, 1H), 5.89-5.78 (m, 1H), 5.13-5.04 (m, 2H), 4.46 (d, 2H), 4.35 (s, 2H), 4.02 (t, 2H), 3.62 (t, 2H), 3.29-3.17 (m, 7H), 2.38 (s, 3H).
LC/MS (Methode 3): $R_t$ = 0.90 min, m/z = 379 $[M+H]^+$.

**Beispiel 86**

3-Isopropyl-1-(2-methoxyethyl)-5-methyl-6-[(2-oxoimidazolidin-1-yl)methyl]thieno[2,3-d]pyrimidin-2,4(1*H*,3*H*)-dion

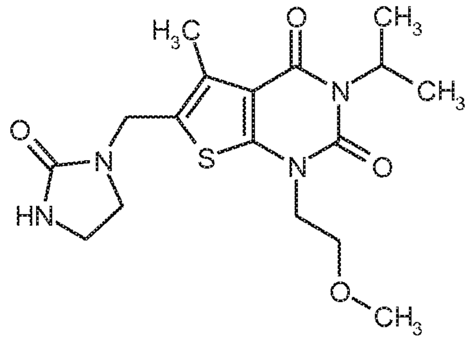

Eine Lösung von 113 mg (1.264 mmol) 2-Imidazolidinon in 4.5 ml THF wurde mit 50 mg (1.264 mmol) Natriumhydrid (60% Suspension in Mineralöl) versetzt und 3 h bei 60°C gerührt ("Lösung 1"). In einem anderen Reaktionsgefäß wurde eine Lösung von 105 mg (0.316 mmol) der Verbindung aus Bsp. 146A in 2.2 ml Dichlormethan bei 0°C mit 165 µl (0.948 mmol) *N,N*-Diisopropylethylamin und 34.5 µl (0.474 mmol) Thionylchlorid versetzt und 75 min gerührt. Anschließend wurde die Lösung 1 portionsweise zugesetzt und das Gemisch 20 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 10 g Kieselgel, Laufmittel Hexan/Ethylacetat). Es wurden 62 mg (50% d. Th.) der Titelverbindung erhalten.
$^1$H-NMR (400 MHz, DMSO-$d_6$, δ/ppm): 6.52 (s, 1H), 5.13 (sept, 1H), 4.33 (s, 2H), 3.98 (t, 2H), 3.61 (t, 2H), 3.28-3.16 (m, 7H), 2.37 (s, 3H), 1.40 (d, 6H).
LC/MS (Methode 3): $R_t$ = 0.98 min, m/z = 381 [M+H]$^+$.

**Beispiel 87**

3-Isobutyl-5-methyl-6-[(2-oxoimidazolidin-1-yl)methyl]-1-(3,3,3-trifluorpropyl)thieno[2,3-d]-pyrimidin-2,4(1*H*,3*H*)-dion

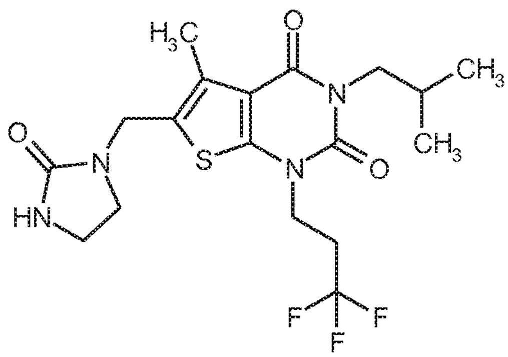

115 mg (0.269 mmol) der Verbindung aus Bsp. 239A wurden in 5 ml Dioxan gelöst und mit 67 mg (0.403 mmol) CDI versetzt. Das Gemisch wurde 67 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 16 mg (13% d. Th.) der Titelverbindung erhalten.
$^1$H-NMR (400 MHz, DMSO-$d_6$, δ/ppm): 6.54 (s, 1H), 4.36 (s, 2H), 4.10 (t, 2H), 3.70 (d, 2H), 3.30-3.18 (m, 4H), 2.82-2.69 (m, 2H), 2.40 (s, 3H), 2.09-1.97 (m, 1H), 0.84 (d, 6H).
LC/MS (Methode 3): $R_t$ = 1.19 min, m/z = 433 [M+H]$^+$.

**Beispiel 88**

1-(2-Fluorethyl)-3-isobutyl-5-methyl-6-[(2-oxoimidazolidin-1-yl)methyl]thieno[2,3-d]pyrimidin-2,4(1*H*,3*H*)-dion

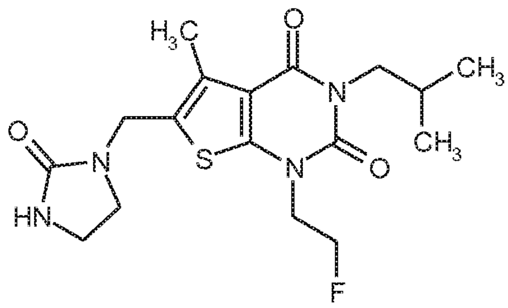

Analog zu dem unter Bsp. 62 beschriebenen Verfahren wurden aus 528 mg (1.11 mmol, 75% Reinheit) der Verbindung aus Bsp. 240A und 288 mg (1.78 mmol) CDI 265 mg (62% d. Th.) der Titelverbindung hergestellt.
$^1$H-NMR (400 MHz, DMSO-$d_6$, $\delta$/ppm): 6.52 (s, 1H), 4.72 (dt, 2H), 4.35 (s, 2H), 4.18 (dt, 2H), 3.72 (d, 2H), 3.29-3.16 (m, 4H), 2.39 (s, 3H), 2.11-1.95 (m, 1H), 0.85 (d, 6H).
LC/MS (Methode 1, ESIpos): $R_t$ = 0.84 min, m/z = 383 $[M+H]^+$.

**Beispiel 89**

3-Isobutyl-1-(2-methoxyethyl)-5-methyl-6-[(2-oxoimidazolidin-1-yl)methyl]thieno[2,3-d]pyrimidin-2,4(1*H*,3*H*)-dion

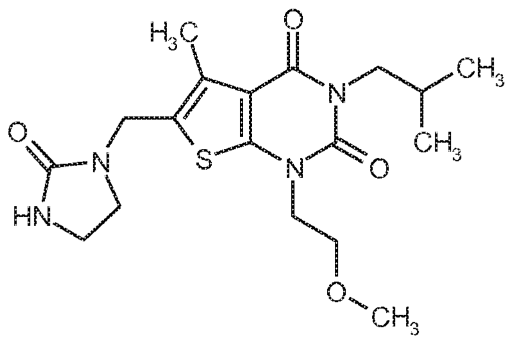

350 mg (0.665 mmol) der Verbindung aus Bsp. 242A wurden in 15 ml Dioxan gelöst und mit 167 mg (0.997 mmol) CDI versetzt. Das Gemisch wurde 20 h bei RT gerührt. Die Reaktionslösung wurde danach am Rotationsverdampfer eingeengt. Der Rückstand wurde in 9 ml DMSO gelöst und diese Lösung mittels präparativer HPLC gereinigt (Methode 14). Nach Vereinigen der Produktfraktionen und Gefriertrocknung wurden 190 mg (72% d. Th.) der Titelverbindung erhalten.
$^1$H-NMR (400 MHz, DMSO-$d_6$, $\delta$/ppm): 6.53 (s, 1H), 4.34 (s, 2H), 4.02 (t, 2H), 3.71 (d, 2H), 3.62 (t, 2H), 3.29-3.17 (m, 7H), 2.38 (s, 3H), 2.03 (dquin, 1H), 0.85 (d, 6H).
LC/MS (Methode 3): $R_t$ = 1.04 min, m/z = 395 $[M+H]^+$.

**Beispiel 90**

3-Isobutyl-5-methyl-6-[(2-oxoimidazolidin-1-yl)methyl]-1-(tetrahydrofuran-3-ylmethyl)thieno-[2,3-d]pyrimidin-2,4(1*H*,3*H*)-dion (*Racemat*)

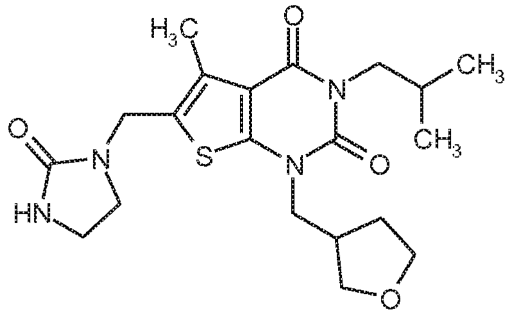

Eine Lösung von 705 mg (1.38 mmol, 79% Reinheit) der Verbindung aus Bsp. 244A und 310 µl (2.23 mmol) Triethylamin in 15 ml THF wurde mit 289 mg (1.78 mmol) CDI versetzt und ca. 18 h bei RT gerührt. Anschließend wurde zur Trockene eingeengt. Der verbliebene Rückstand wurde in Ethylacetat aufgenommen und nacheinander mit 1 M Salzsäure, Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) gereinigt. Nach Vereinigen der Produktfraktionen, Eindampfen und Trocknen im Hochvakuum wurden 352 mg (59% d. Th.) der Titelverbindung erhalten.
$^1$H-NMR (400 MHz, DMSO-$d_6$, δ/ppm): 6.52 (s, 1H), 4.34 (s, 2H), 4.27-4.16 (m, 1H), 4.01 (dd, 1H), 3.78-3.67 (m, 4H), 3.66-3.56 (m, 1H), 3.29-3.16 (m, 4H), 2.39 (s, 3H), 2.11-1.73 (m, 4H), 1.72-1.59 (m, 1H), 0.85 (dd, 6H).
LC/MS (Methode 1, ESIpos): $R_t$ = 0.88 min, m/z = 421 $[M+H]^+$.

**Beispiel 91**

3-Isobutyl-1,5-dimethyl-6-[(2-oxoimidazolidin-1-yl)methyl]thieno[2,3-d]pyrimidin-2,4(1*H*,3*H*)-dion

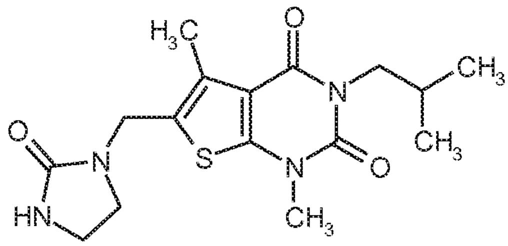

Analog zu dem unter Bsp. 62 beschriebenen Verfahren wurden aus 195 mg (0.480 mmol, 80% Reinheit) der Verbindung aus Bsp. 246A und 117 mg (0.720 mmol) CDI 85 mg (50% d. Th.) der Titelverbindung hergestellt.
$^1$H-NMR (400 MHz, DMSO-$d_6$, δ/ppm): 6.52 (s, 1H), 4.36 (s, 2H), 3.71 (d, 2H), 3.41 (s, 3H), 3.28-3.14 (m, 4H), 2.40 (s, 3H), 2.09-1.99 (m, 1H), 0.85 (d, 6H).
LC/MS (Methode 1, ESIpos): $R_t$ = 0.78 min, m/z = 351 $[M+H]^+$.

**Beispiel 92**

3-(Cyclopropylmethyl)-1-(2-methoxyethyl)-5-methyl-6-[(2-oxoimidazolidin-1-yl)methyl]thieno-[2,3-d]pyrimidin-2,4(1*H*,3*H*)-dion

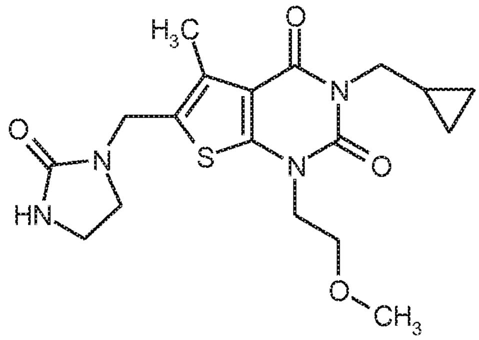

Eine Lösung von 77 mg (0.854 mmol) 2-Imidazolidinon in 3 ml THF wurde mit 34 mg (0.854 mmol) Natriumhydrid (60% Suspension in Mineralöl) versetzt und 3 h bei 60°C gerührt ("Lösung 1"). In einem anderen Reaktionsgefäß wurde eine Lösung von 70 mg (0.214 mmol) der Verbindung aus Bsp. 150A in 1.5 ml Dichlormethan bei 0°C mit 111 µl (0.641 mmol) *N,N*-Diisopropylethylamin und 23.3 µl (0.32 mmol) Thionylchlorid versetzt und 75 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 10 g Kieselgel, Laufmittel Hexan/Ethylacetat). Es wurden 32 mg (38% d. Th.) der Titelverbindung erhalten.
$^1$H-NMR (400 MHz, DMSO-$d_6$, δ/ppm): 6.52 (s, 1H), 4.35 (s, 2H), 4.03 (t, 2H), 3.76 (d, 2H), 3.63 (t, 2H), 3.28-3.18 (m, 7H), 2.39 (s, 3H), 1.21-1.11 (m, 1H), 0.45-0.38 (m, 2H), 0.36-0.30 (m, 2H).
LC/MS (Methode 3): $R_t$ = 0.99 min, m/z = 393 $[M+H]^+$.

**Beispiel 93**

1-(3-Fluorpropyl)-5-methyl-6-[(2-oxoimidazolidin-1-yl)methyl]-3-(2,2,2-trifluorethyl)thieno-[2,3-d]pyrimidin-2,4(1*H*,3*H*)-dion

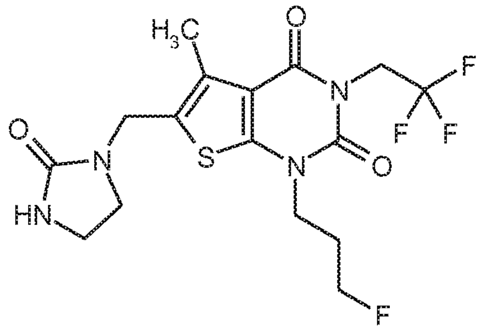

170 mg (0.309 mmol) der Verbindung aus Bsp. 248A wurden in 6.5 ml Dioxan gelöst und mit 77 mg (0.463 mmol) CDI versetzt. Das Gemisch wurde 20 h bei RT gerührt. Das Reaktionsgemisch wurde danach direkt mittels präparativer HPLC gereinigt (Methode 14). Nach Vereinigen der Produktfraktionen und Gefriertrocknung wurden 74 mg (57% d. Th.) der Titelverbindung erhalten.
$^1$H-NMR (400 MHz, DMSO-$d_6$, δ/ppm): 6.55 (s, 1H), 4.69 (q, 2H), 4.59 (t, 1H), 4.47 (t, 1H), 4.37 (s, 2H), 4.01 (t, 2H), 3.29-3.18 (m, 4H), 2.40 (s, 3H), 2.14-2.00 (m, 2H).
LC/MS (Methode 3): $R_t$ = 1.01 min, m/z = 423 $[M+H]^+$.

**Beispiel 94**

1-(2-Methoxyethyl)-5-methyl-6-[(2-oxoimidazolidin-1-yl)methyl]-3-(2,2,2-trifluorethyl)thieno-[2,3-d]pyrimidin-2,4(1*H*,3*H*)-dion

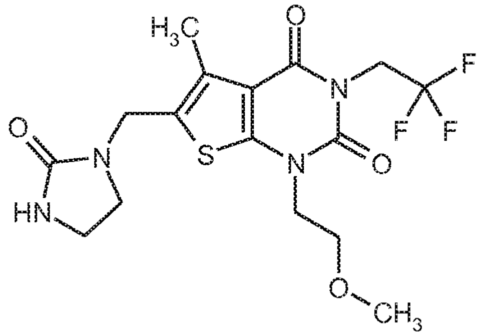

160 mg (0.268 mmol) der Verbindung aus Bsp. 249A wurden in 5.4 ml Dioxan gelöst und mit 67 mg (0.402 mmol) CDI versetzt. Das Gemisch wurde 15 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 37 mg (31% d. Th.) der Titelverbindung erhalten.
$^1$H-NMR (400 MHz, DMSO-$d_6$, δ/ppm): 6.53 (s, 1H), 4.69 (q, 2H), 4.36 (s, 2H), 4.05 (t, 2H), 3.64 (t, 2H), 3.29-3.19 (m, 7H), 2.39 (s, 3H).
LC/MS (Methode 3): $R_t$ = 0.95 min, m/z = 421 $[M+H]^+$.

## Beispiel 95

3-(2,2-Difluorethyl)-1-(3-fluorpropyl)-5-methyl-6-[(2-oxoimidazolidin-1-yl)methyl]thieno[2,3-d]-pyrimidin-2,4(1*H*,3*H*)-dion

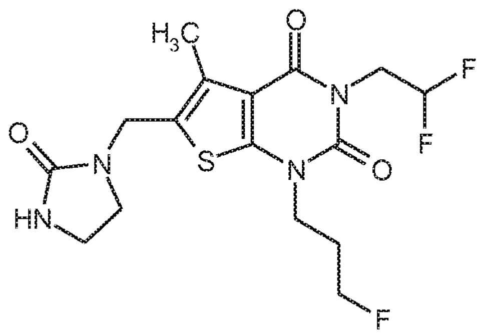

195 mg (0.433 mmol) der Verbindung aus Bsp. 256A wurden in 8.8 ml Dioxan gelöst und mit 108 mg (0.649 mmol) CDI versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 105 mg (60% d. Th.) der Titelverbindung erhalten.
$^1$H-NMR (400 MHz, DMSO-$d_6$, δ/ppm): 6.55 (s, 1H), 6.38-6.03 (m, 1H), 4.60 (t, 1H), 4.48 (t, 1H), 4.37 (s, 2H), 4.29 (td, 2H), 4.00 (t, 2H), 3.29-3.17 (m, 4H), 2.40 (s, 3H), 2.14-2.00 (m, 2H).
LC/MS (Methode 3): $R_t$ = 0.92 min, m/z = 405 $[M+H]^+$.

## Beispiel 96

3-(2,2-Difluorethyl)-1-(2-methoxyethyl)-5-methyl-6-[(2-oxoimidazolidin-1-yl)methyl]thieno-[2,3-d]pyrimidin-2,4(1*H*,3*H*)-dion

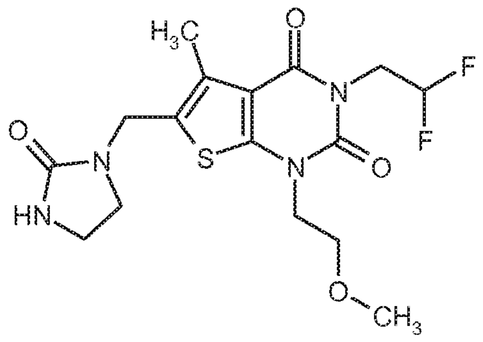

178 mg (0.340 mmol) der Verbindung aus Bsp. 257A wurden in 6.9 ml Dioxan gelöst und mit 85 mg (0.511 mmol) CDI versetzt. Das Gemisch wurde 16 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 126 mg (92% d. Th.) der Titelverbindung erhalten.
$^1$H-NMR (400 MHz, DMSO-$d_6$, δ/ppm): 6.53 (s, 1H), 6.39-6.05 (m, 1H), 4.36 (s, 2H), 4.29 (td, 2H), 4.04 (t, 2H), 3.64 (t, 2H), 3.29-3.18 (m, 7H), 2.39 (s, 3H).
LC/MS (Methode 3): $R_t$ = 0.89 min, m/z = 403 [M+H]$^+$.

**Beispiel 97**

3-(2-Fluorethyl)-1-(2-methoxyethyl)-5-methyl-6-[(2-oxoimidazolidin-1-yl)methyl]thieno[2,3-d]-pyrimidin-2,4(1*H*,3*H*)-dion

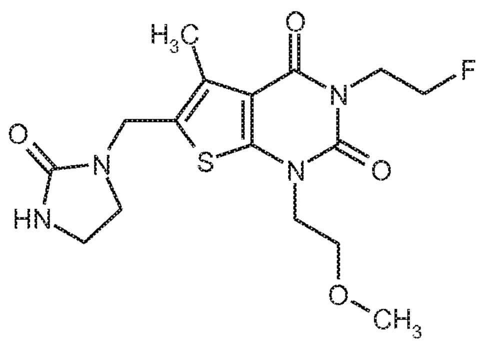

Eine Lösung von 176 mg (1.962 mmol) 2-Imidazolidinon in 7 ml THF wurde mit 78 mg (1.962 mmol) Natriumhydrid (60% Suspension in Mineralöl) versetzt und 4 h bei 60°C gerührt ("Lösung 1"). In einem anderen Reaktionsgefäß wurde eine Lösung von 160 mg (0.491 mmol) der Verbindung aus Bsp. 151A in 3.4 ml Dichlormethan bei 0°C mit 256 µl (1.472 mmol) *N,N*-Diisopropylethylamin und 53.7 µl (0.736 mmol) Thionylchlorid versetzt und 90 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 10 g Kieselgel, Laufmittel Hexan/Ethylacetat). Es wurden 88 mg (46% d. Th.) der Titelverbindung erhalten.
$^1$H-NMR (400 MHz, DMSO-$d_6$, δ/ppm): 6.52 (s, 1H), 4.66 (t, 1H), 4.54 (t, 1H), 4.35 (s, 2H), 4.23 (t, 1H), 4.20-4.14 (m, 1H), 4.02 (t, 2H), 3.63 (t, 2H), 3.28-3.18 (m, 7H), 2.39 (s, 3H).
LC/MS (Methode 3): $R_t$ = 0.82 min, m/z = 385 [M+H]$^+$.

**Beispiel 98**

1-(2-Methoxyethyl)-5-methyl-6-[(2-oxoimidazolidin-1-yl)methyl]-3-{3,3,3-trifluorpropyl)thieno-[2,3-d]pyrimidin-2,4(1*H*,3*H*)-dion

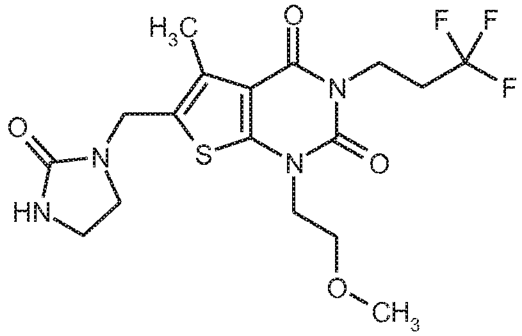

Eine Lösung von 205 mg (2.293 mmol) 2-Imidazolidinon in 8.2 ml THF wurde mit 92 mg (2.293 mmol) Natriumhydrid (60% Suspension in Mineralöl) versetzt und 3 h bei 60°C gerührt ("Lösung 1"). In einem anderen Reaktionsgefäß wurde eine Lösung von 210 mg (0.573 mmol) der Verbindung aus Bsp. 152A in 4 ml Dichlormethan bei 0°C mit 299 µl (1.72 mmol) *N,N*-Diisopropylethylamin und 62.7 µl (0.86 mmol) Thionylchlorid versetzt und 90 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 10 g Kieselgel, Laufmittel Hexan/Ethylacetat). Es wurden 95 mg (37% d. Th.) der Titelverbindung erhalten.
$^1$H-NMR (400 MHz, DMSO-$d_6$, δ/ppm): 6.55 (s, 1H), 4.35 (s, 2H), 4.11 (t, 2H), 4.02 (t, 2H), 3.62 (t, 2H), 3.27-3.18 (m, 7H), 2.64-2.53 (m, 2H), 2.39 (s, 3H).
LC/MS (Methode 3): $R_t$ = 1.02 min, m/z = 435 $[M+H]^+$.

**Beispiel 99**

1-(2-Methoxyethyl)-3-(2-methoxypropyl)-5-methyl-6-[(2-oxoimidazolidin-1-yl)methyl]thieno-[2,3-d]pyrimidin-2,4(1*H*,3*H*)-dion (*Racemat*)

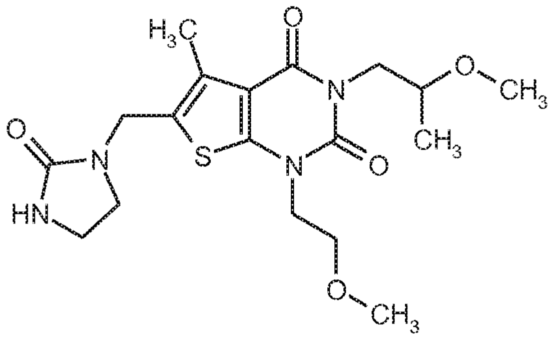

Eine Lösung von 195 mg (2.173 mmol) 2-Imidazolidinon in 7.8 ml THF wurde mit 87 mg (2.173 mmol) Natriumhydrid (60% Suspension in Mineralöl) versetzt und 3 h bei 60°C gerührt ("Lösung 1"). In einem anderen Reaktionsgefäß wurde eine Lösung von 200 mg (0.543 mmol) der Verbindung aus Bsp. 155A in 3.8 ml Dichlormethan bei 0°C mit 284 µl (1.63 mmol) *N,N*-Diisopropylethylamin und 59.4 µl (0.815 mmol) Thionylchlorid versetzt und 75 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche

chromatographiert (Biotage, 10 g Kieselgel, Laufmittel Hexan/Ethylacetat). Es wurden 102 mg (45% d. Th.) der Titelverbindung erhalten.
$^1$H-NMR (400 MHz, DMSO-$d_6$, $\delta$/ppm): 6.55 (s, 1H), 4.34 (s, 2H), 4.09-3.99 (m, 3H), 3.75 (dd, 1H), 3.68-3.59 (m, 3H), 3.29-3.17 (m, 10H), 2.38 (s, 3H), 1.05 (d, 3H).
LC/MS (Methode 3): $R_t$ = 0.88 min, m/z = 411 $[M+H]^+$.

**Beispiel 100**

5-(Difluormethyl)-3-ethyl-6-[(2-oxoimidazolidin-1-yl)methyl]-1-(3,3,3-trifluorpropyl)thieno-[2,3-d]pyrimidin-2,4(1*H*,3*H*)-dion

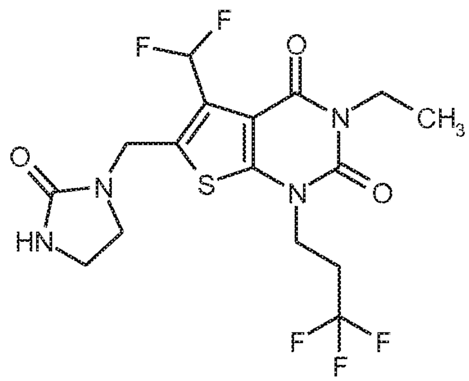

370 mg (0.893 mmol) der Verbindung aus Bsp. 264A wurden in 18 ml Dioxan gelöst und mit 223 mg (1.339 mmol) CDI versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 38 mg (10% d. Th.) der Titelverbindung erhalten.
$^1$H-NMR (400 MHz, DMSO-$d_6$, $\delta$/ppm): 7.68-7.39 (m, 1H), 6.72 (s, 1H), 4.57 (s, 2H), 4.13 (t, 2H), 3.91 (q, 2H), 2.86-2.71 (m, 2H), 1.13 (t, 3H).
LC/MS (Methode 3): $R_t$ = 1.08 min, m/z = 441 $[M+H]^+$.

**Beispiel 101**

5-(Difluormethyl)-3-ethyl-1-(2-methoxyethyl)-6-[(2-oxoimidazolidin-1-yl)methyl]thieno[2,3-d]-pyrimidin-2,4(1*H*,3*H*)-dion

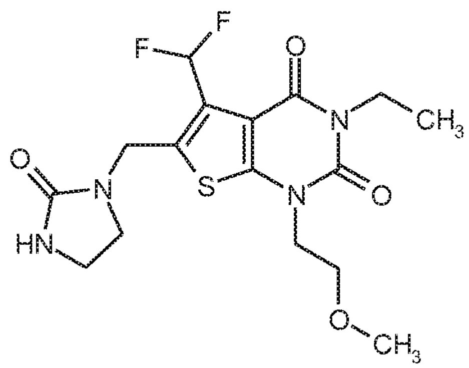

298 mg (0.792 mmol) der Verbindung aus Bsp. 265A wurden in 16 ml Dioxan gelöst und mit 198 mg (1.187 mmol) CDI versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt

(Methode 14). Nach Vereinigen der Produktfraktionen und Gefriertrocknung wurden 31 mg (9% d. Th.) der Titelverbindung erhalten.
$^1$H-NMR (400 MHz, DMSO-$d_6$, δ/ppm): 7.68-7.38 (m, 1H), 6.70 (s, 1H), 4.55 (s, 2H), 4.05 (t, 2H), 3.91 (q, 2H), 3.64 (t, 2H), 3.36-3.24 (m, 4H), 3.24 (s, 3H), 1.13 (t, 3H).
LC/MS (Methode 3): $R_t$ = 0.94 min, m/z = 403 $[M+H]^+$.

**Beispiel 102**

1-(2-Methoxyethyl)-5-methyl-6-[(3-methyl-2-oxoimidazolidin-1-yl)methyl]-3-(2-phenylethyl)-thieno[2,3-d]pyrimidin-2,4(1*H*,3*H*)-dion

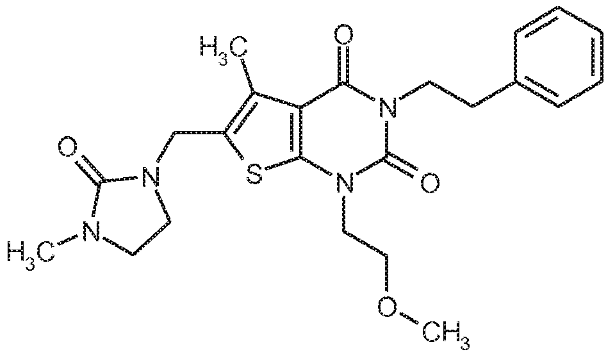

Analog zu dem unter Bsp. 1 beschriebenen Verfahren wurden aus 80 mg (0.214 mmol) der Verbindung aus Bsp. 138A und 64 mg (0.641 mmol) 1-Methylimidazolidin-2-on 44 mg (45% d. Th.) der Titelverbindung erhalten. Das 1-Methylimidazolidin-2-on wurde hier nur 2 min mit Natriumhydrid in THF gerührt.
$^1$H-NMR (400 MHz, DMSO-$d_6$, δ/ppm): 7.35-7.27 (m, 2H), 7.26-7.17 (m, 3H), 4.38 (s, 2H), 4.08-4.04 (m, 2H), 4.01 (t, 2H), 3.60 (t, 2H), 3.26-3.16 (m, 4H), 3.23 (s, 3H), 2.89-2.76 (m, 2H), 2.67 (s, 3H), 2.40 (s, 3H).
LC/MS (Methode 1, ESIpos): $R_t$ = 1.04 min, m/z = 457 $[M+H]^+$.

**Beispiel 103**

3-Ethyl-5-methyl-6-[(3-methyl-2-oxoimidazolidin-1-yl)methyl]-1-(3,3,3-trifluorpropyl)thieno-[2,3-d]pyrimidin-2,4(1*H*,3*H*)-dion

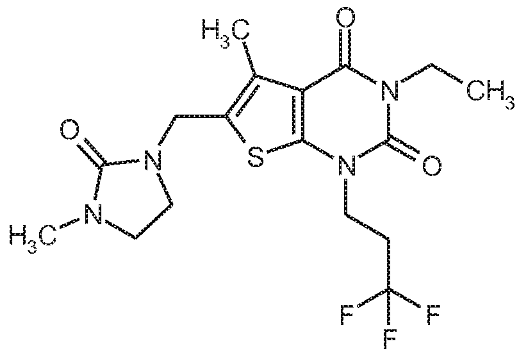

Analog zu dem unter Bsp. 2 beschriebenen Verfahren wurden aus 90 mg (0.268 mmol) der Verbindung aus Bsp. 140A und 107 mg (1.07 mmol) 1-Methylimidazolidin-2-on 58 mg (51% d. Th.) der Titelverbindung erhalten. Abweichend vom zuvor beschriebenen Verfahren wurde das 1-Methyl-imidazolidin-2-on hier 2 h bei 60°C mit Natriumhydrid deprotoniert, und die Reaktionszeit im letzten Reaktionsteilschritt betrug 4 Tage.
$^1$H-NMR (400 MHz, DMSO-$d_6$, δ/ppm): 4.39 (s, 2H), 4.09 (t, 2H), 3.90 (q, 2H), 3.27-3.16 (m, 4H), 2.83-2.69 (m, 2H),

2.67 (s, 3H), 2.41 (s, 3H), 1.11 (t, 3H).
LC/MS (Methode 2, ESIpos): $R_t$ = 2.63 min, m/z = 419 $[M+H]^+$.

**Beispiel 104**

6-[(3-Acetyl-2-oxoimidazolidin-1-yl)methyl]-3-ethyl-1-(2-methoxyethyl)-5-methylthieno[2,3-d]-pyrimidin-2,4(1*H*,3*H*)-dion

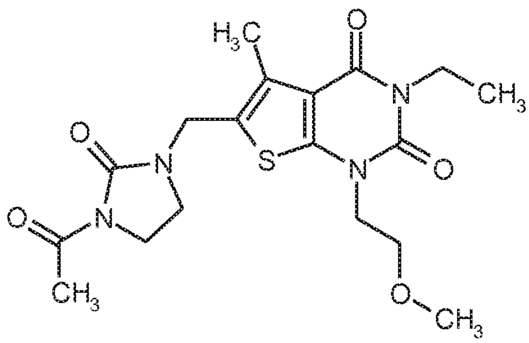

Eine Lösung von 95 mg (0.161 mmol) der Verbindung aus Bsp. 59 in 5 ml THF wurde mit 7.7 mg (0.192 mmol) Natriumhydrid (60% Suspension in Mineralöl) versetzt und 1 h bei 0°C gerührt. Anschließend wurden 15 mg (0.193 mmol) Acetylchlorid hinzugefügt und das Gemisch 80 min bei 0°C gerührt. Danach wurde das Reaktionsgemisch zwischen gesättigter wässriger Ammoniumchlorid-Lösung (20 ml) und THF (30 ml) verteilt. Die organische Phase wurde mit gesättigter wässriger Natriumhydrogencarbonat-Lösung gewaschen, über Natriumsulfat getrocknet, filtriert und eingeengt. Der erhaltene Rückstand wurde mittels präparativer HPLC gereinigt (Methode 14). Nach Vereinigen der Produktfraktionen und Gefriertrocknung wurden 29 mg (44% d. Th.) der Titelverbindung erhalten.
$^1$H-NMR (400 MHz, DMSO-$d_6$, δ/ppm): 4.51 (s, 2H), 4.02 (t, 2H), 3.90 (q, 2H), 3.70-3.61 (m, 4H), 3.23 (s, 3H), 2.42 (s, 3H), 2.37 (s, 3H), 1.11 (t, 3H).
LC/MS (Methode 3): $R_t$ = 0.97 min, m/z = 409 $[M+H]^+$.

**Beispiel 105**

1-(2-Methoxyethyl)-6-[(3-methyl-2-oxoimidazolidin-1-yl)methyl]-3-(2-phenylethyl)-5-(trifluormethyl)thieno[2,3-d]pyrimidin-2,4(1*H*,3*H*)-dion

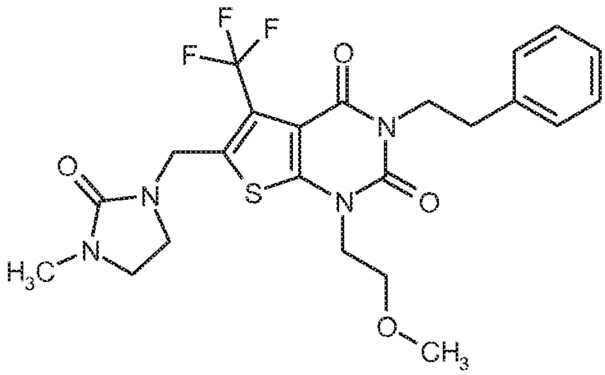

Analog zu dem unter Bsp. 5 beschriebenen Verfahren wurden aus 100 mg (0.224 mmol) der Verbindung aus Bsp. 181A und 116 mg (1.16 mmol) 1-Methylimidazolidin-2-on 24 mg (21% d. Th.) der Titelverbindung erhalten.
$^1$H-NMR (400 MHz, DMSO-$d_6$, δ/ppm): 7.34-7.27 (m, 2H), 7.25-7.19 (m, 3H), 4.59 (d, 2H), 4.12-4.00 (m, 4H), 3.60 (t, 2H), 3.35 (m, 4H), 3.23 (s, 3H), 2.89-2.79 (m, 2H), 2.72 (s, 3H).
LC/MS (Methode 1, ESIpos): $R_t$ = 1.07 min, m/z = 511 $[M+H]^+$.

**Beispiel 106**

6-[(2,5-Dioxoimidazolidin-1-yl)methyl]-3-ethyl-5-methyl-1-[2-(trifluormethoxy)ethyl]thieno-[2,3-d]pyrimidin-2,4(1*H*,3*H*)-dion

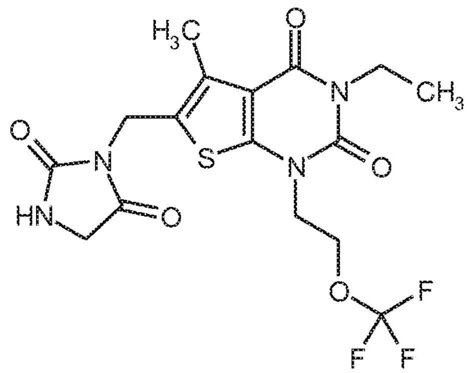

Eine Lösung von 60 mg (0.170 mmol) der Verbindung aus Bsp. 142A, 17 mg (0.170 mmol) Hydantoin und 54 mg (0.204 mmol) Triphenylphosphin in 1.2 ml THF wurde bei 0°C mit 41 mg (0.204 mmol) Diisopropylazodicarboxylat (DIAD) versetzt. Das Reaktionsgemisch wurde zunächst 1 h bei 0°C und dann 1 h bei RT gerührt. Anschließend wurde zur Trockene eingeengt. Der verbliebene Rückstand wurde mittels präparativer HPLC (Methode 13) zunächst vorgereinigt. Die produkthaltigen Fraktionen wurden eingeengt und der Rückstand nochmals mittels präparativer HPLC (Methode 16) nachgereinigt. Es wurden 2.7 mg (3.5% d. Th., 97% Reinheit) der Titelverbindung erhalten.
$^1$H-NMR (500 MHz, DMSO-$d_6$, δ/ppm): 8.18 (s, 1H), 4.63 (s, 2H), 4.39 (t, 2H), 4.17 (t, 2H), 3.94 (s, 2H), 3.90 (q, 2H), 2.48 (s, 3H), 1.11 (t, 3H).
LC/MS (Methode 1, ESIpos): $R_t$ = 0.85 min, m/z = 435 $[M+H]^+$.

**Beispiel 107**

3-Ethyl-5-methyl-6-[(2-oxotetrahydropyrimidin-1(2*H*)-yl)methyl]-1-(3,3,3-trifluorpropyl)thieno-[2,3-d]pyrimidin-2,4(1*H*,3*H*)-dion

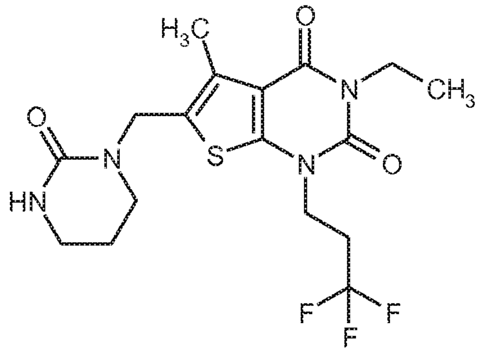

Analog zu dem unter Bsp. 2 beschriebenen Verfahren wurden aus 100 mg (0.297 mmol) der Verbindung aus Bsp. 140A und 37 mg (1.19 mmol) Tetrahydropyrimidin-2(1*H*)-on 19 mg (15% d. Th.) der Titelverbindung erhalten. Abweichend vom zuvor beschriebenen Verfahren wurde das Tetrahydropyrimidin-2(1*H*)-on hier 2 h bei 60°C mit Natriumhydrid deprotoniert, und die Reaktionszeit im letzten Reaktionsteilschritt betrug 7 Tage.
$^1$H-NMR (500 MHz, DMSO-$d_6$, δ/ppm): 6.40 (br. s, 1H), 4.51 (s, 2H), 4.09 (t, 2H), 3.90 (q, 2H), 3.18 (t, 2H), 3.09 (t, 2H), 2.82-2.69 (m, 2H), 2.42 (s, 3H), 1.77 (quin, 2H), 1.11 (t, 3H).
LC/MS (Methode 2, ESIpos): $R_t$ = 2.42 min, m/z = 419 $[M+H]^+$.

**Beispiel 108**

3-Ethyl-5-methyl-6-[(3-methyl-2-oxotetrahydropyrimidin-1(2*H*)-yl)methyl]-1-(3,3,3-trifluorpropyl)thieno[2,3-d]pyrimidin-2,4(1*H*,3*H*)-dion

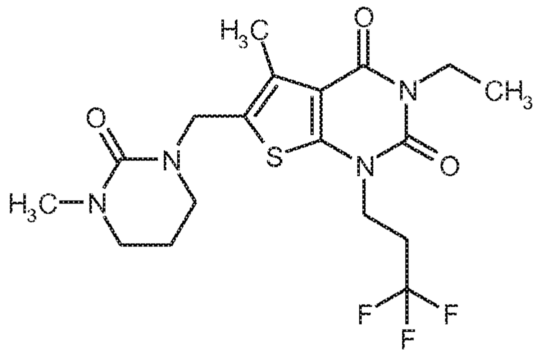

Analog zu dem unter Bsp. 2 beschriebenen Verfahren wurden aus 90 mg (0.268 mmol) der Verbindung aus Bsp. 140A und 122 mg (1.07 mmol) 1-Methyltetrahydropyrimidin-2(1*H*)-on 78 mg (67% d. Th.) der Titelverbindung erhalten. Abweichend vom zuvor beschriebenen Verfahren wurde das 1-Methyltetrahydropyrimidin-2(1*H*)-on hier 2 h bei 60°C mit Natriumhydrid deprotoniert, und die Reaktionszeit im letzten Reaktionsteilschritt betrug 7 Tage.
$^1$H-NMR (400 MHz, DMSO-$d_6$, δ/ppm): 4.52 (s, 2H), 4.09 (t, 2H), 3.90 (q, 2H), 3.22-3.17 (m, 4H), 2.81 (s, 3H), 2.81-2.72 (m, 2H), 2.41 (s, 3H), 1.86 (quin, 2H), 1.11 (t, 3H).
LC/MS (Methode 1, ESIpos): $R_t$ = 0.96 min, m/z = 433 $[M+H]^+$.

**Beispiel 109**

3-Ethyl-1-(2-methoxyethyl)-5-methyl-6-[(2-oxo-2,3-dihydro-1*H*-imidazol-1-yl)methyl]thieno-[2,3-d]pyrimidin-2,4(1*H*,3*H*)-dion

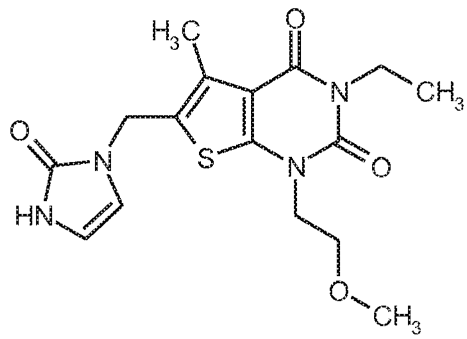

Eine Lösung von 47 mg (0.110 mmol) der Verbindung aus Bsp. 274A in einem Gemisch aus 800 µl Wasser und 220 µl Methanol wurde mit 217 µl (0.109 mmol) 0.5 M Salzsäure versetzt. Nachdem das Reaktionsgemisch 3 Tage bei RT gerührt worden war, wurde es direkt mittels präparativer HPLC (Methode 8) in seine Komponenten aufgetrennt. Eindampfen und Trocknen der Produktfraktion im Hochvakuum ergab 20 mg (47% d. Th., 95% Reinheit) der Titelverbindung.
$^1$H-NMR (400 MHz, DMSO-$d_6$, δ/ppm): 9.99 (br. s, 1H), 6.43 (dd, 1H), 6.34 (t, 1H), 4.79 (s, 2H), 4.00 (t, 2H), 3.89 (q, 2H), 3.62 (t, 2H), 3.22 (s, 3H), 2.46 (s, 3H), 1.11 (t, 3H).
LC/MS (Methode 1, ESIpos): $R_t$ = 0.67 min, m/z = 365 $[M+H]^+$.

**Beispiel 110**

6-[(4-Allyl-5-oxo-4,5-dihydro-1*H*-1,2,4-triazol-1-yl)methyl]-3-ethyl-5-methyl-1-(3,3,3-trifluorpropyl)thieno[2,3-d]pyrimidin-2,4(1*H*,3*H*)-dion

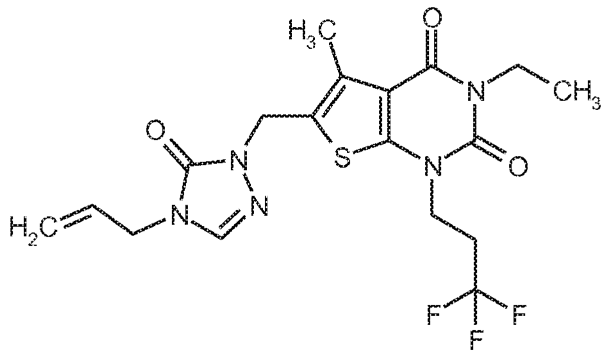

6.16 g (18.3 mmol) der Verbindung aus Bsp. 140A und 2.75 g (22.0 mmol) 4-Allyl-2,4-dihydro-3*H*-1,2,4-triazol-3-on wurden in 300 ml Dichlormethan gelöst und mit 12.2 g (46.5 mmol) Polymer-gebundenem Triphenylphosphin versetzt. Nach 10 min wurden 5.4 ml (27.5 mmol) Diisopropylazodicarboxylat (DIAD) zugetropft. Das Reaktionsgemisch wurde bei RT gerührt. Nach ca. 18 h wurden weitere 5 g (19.1 mmol) Polymer-gebundenes Triphenylphosphin sowie 1 g (4.95 mmol) DIAD zugesetzt. Nach weiteren 2 h wurde das Reaktionsgemisch filtriert und der Rückstand mit Dichlormethan nachgewaschen. Das Filtrat wurde nacheinander mit Wasser und gesättigter Natriumchlorid-Lösung gewaschen. Nach Trocknen über wasserfreiem Magnesiumsulfat, Filtration und Einengen wurde der verbliebene Rückstand mittels Saugfiltration über Kieselgel mit Cyclohexan/Ethylacetat (1:1) als Laufmittel gereinigt. Die Produktfraktionen wurden vereinigt, eingedampft und im Hochvakuum getrocknet. Es wurden 2.72 g (30% d. Th., 92% Reinheit) der Titelverbindung erhalten.
$^1$H-NMR (400 MHz, DMSO-$d_6$, δ/ppm): 7.96 (s, 1H), 5.96-5.84 (m, 1H), 5.19 (dd, 1H), 5.11-5.03 (m, 1H), 5.02 (s, 2H), 4.21 (dt, 2H), 4.08 (t, 2H), 3.90 (q, 2H), 2.81-2.66 (m, 2H), 2.47 (s, 3H), 1.11 (t, 3H).
LC/MS (Methode 1, ESIpos): $R_t$ = 0.93 min, m/z = 444 $[M+H]^+$.

**Beispiel 111**

6-[(4-Allyl-5-oxo-4,5-Mydro-1*H*-1,2,4-triazol-1-yl)methyl]-3-ethyl-5-methyl-1-[2-(trifluormethoxy)ethyl]thieno[2,3-d]pyrimidin-2,4(1*H*,3*H*)-dion

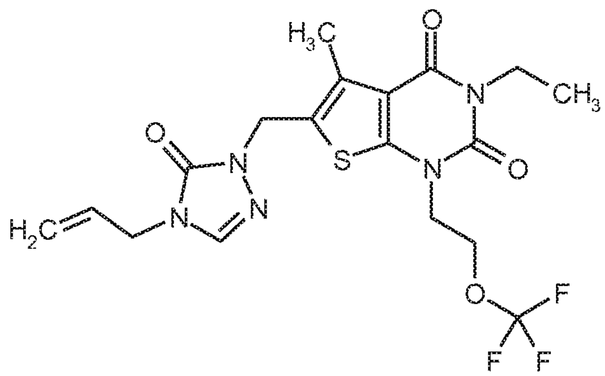

Eine Lösung von 75 mg (0.213 mmol) der Verbindung aus Bsp. 142A, 27 mg (0.213 mmol) 4-Allyl-2,4-dihydro-3*H*-1,2,4-triazol-3-on und 56 mg (0.213 mmol) Triphenylphosphin in 2.1 ml THF wurde bei 0°C mit 43 mg (0.213 mmol) Diisopropylazodicarboxylat (DIAD) versetzt. Das Reaktionsgemisch wurde 2 h bei RT gerührt. Anschließend wurde zur Trockene eingeengt. Der verbliebene Rückstand wurde mittels präparativer HPLC (Methode 13) gereinigt. Die produkthaltigen Fraktionen wurden eingeengt und im Hochvakuum getrocknet. Es wurden 14 mg (14% d. Th., 98% Reinheit) der Titelverbindung erhalten.
$^1$H-NMR (500 MHz, DMSO-$d_6$, $\delta$/ppm): 7.96 (s, 1H), 5.89 (ddt, 1H), 5.18 (dd, 1H), 5.09-5.02 (m, 1H), 5.01 (s, 2H), 4.38 (t, 2H), 4.23-4.14 (m, 4H), 3.90 (q, 2H), 2.46 (s, 3H), 1.11 (t, 3H).
LC/MS (Methode 1, ESIpos): $R_t$ = 0.93 min, m/z = 460 $[M+H]^+$.

**Beispiel 112**

6-[(4-Allyl-5-oxo-4,5-dihydro-1*H*-1,2,4-triazol-1-yl)methyl]-3-ethyl-1-(2-methoxyethyl)-5-methyl-thieno[2,3-d]pyrimidin-2,4(1*H*,3*H*)-dion

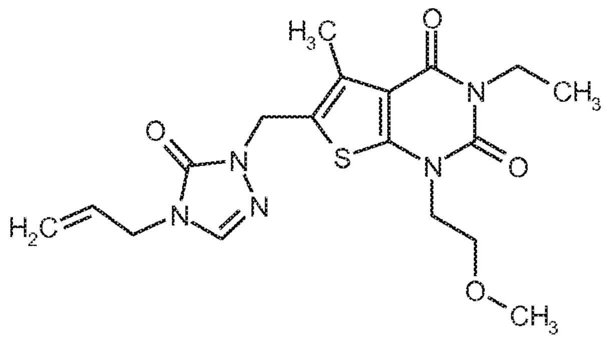

Eine Lösung von 839 mg (6.70 mmol) 4-Allyl-2,4-dihydro-3*H*-1,2,4-triazol-3-on in 12 ml DMF wurde mit 268 mg (6.70 mmol) Natriumhydrid (60% Suspension in Mineralöl) versetzt, dann für 5 min auf 60°C erwärmt und anschließend wieder auf RT abgekühlt ("Lösung 1"). In einem anderen Reaktionsgefäß wurde eine Lösung von 500 mg (1.68 mmol) der Verbindung aus Bsp. 143A in 10 ml Dichlormethan bei 0°C mit 584 nl (3.35 mmol) *N,N*-Diisopropylethylamin und 128 µl (1.76 mmol) Thionylchlorid versetzt. Nach 20 min bei 0°C wurde tropfenweise Lösung 1 hinzugefügt und das Kältebad entfernt. Das Reaktionsgemisch wurde 2.5 Tage bei RT gerührt. Danach wurde mit 100 ml Wasser versetzt und dreimal mit Dichlormethan extrahiert. Die vereinigten organischen Extrakte wurden nacheinander mit Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) gereinigt. Nach Eindampfen der Produktfraktionen und Trocknen im Hochvakuum wurden 196 mg (28% d. Th.) der Titelverbindung erhalten.
$^1$H-NMR (400 MHz, DMSO-$d_6$, $\delta$/ppm): 7.96 (s, 1H), 5.90 (ddt, 1H), 5.19 (dd, 1H), 5.07 (dq, 1H), 4.99 (s, 2H), 4.21 (dt, 2H), 4.00 (t, 2H), 3.89 (q, 2H), 3.62 (t, 2H), 3.22 (s, 3H), 2.45 (s, 3H), 1.11 (t, 3H).
LC/MS (Methode 1, ESIpos): $R_t$ = 0.78 min, m/z = 406 $[M+H]^+$.

**Beispiel 113**

3-Ethyl-5-methyl-6-[(5-oxo-4,5-dihydro-1*H*-1,2,4-triazol-1-yl)methyl]-1-(3,3,3-trifluorpropyl)-thieno[2,3-d]pyrimidin-2,4(1*H*,3*H*)-dion

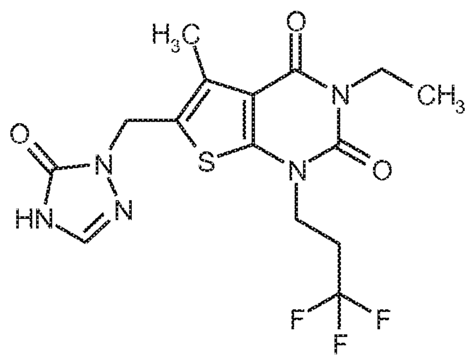

*Methode A:*

407 mg (0.685 mmol, 75% Reinheit) der Verbindung aus Bsp. 110 wurden in 8 ml 1,4-Dioxan gelöst und mit 54 µl (1.44 mmol) Ameisensäure, 239 µl (1.71 mmol) Triethylamin und 79 mg (0.068 mmol) Tetrakis(triphenylphosphin)palladium(0) versetzt. Das Gemisch wurde 28 h in einem Mikrowellenofen (Biotage Initiator mit dynamischer Steuerung der Einstrahlleistung) auf 100°C erhitzt, wobei nach 12 h und nach 16 h Reaktionszeit jeweils die gleichen Mengen an PalladiumKatalysator noch einmal zugesetzt wurden. Dann wurde das Reaktionsgemisch filtriert und das Filtrat zur Trockene eingeengt. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat und Filtration wurde eingeengt. Präparative HPLC-Reinigung des Rückstands (Methode 8) ergab nach Eindampfen und Trocknen der Produktfraktionen im Hochvakuum 100 mg (36% d. Th.) der Titelverbindung.

*Methode B:*

7.31 g (14.8 mmol) der Verbindung aus Bsp. 474A wurden in einem Gemisch aus 412 ml Methanol und 412 ml Orthoameisensäuretrimethylester gelöst und bei RT zunächst mit 37 ml (148 mmol) einer 4 M Lösung von Chlorwasserstoff in Dioxan versetzt. Nach 5 h Reaktionszeit wurden weitere 7.4 ml (29.6 mmol) der 4 M Lösung von Chlorwasserstoff in Dioxan hinzugefügt und das Rühren bei RT fortgesetzt. Nach insgesamt ca. 20 h Reaktionszeit wurde das Reaktionsgemisch am Rotationsverdampfer auf ca. die Hälfte seines ursprünglichen Volumens eingeengt. Dann wurde mit Wasser verdünnt und mit Ethylacetat extrahiert. Der organische Extrakt wurde am Rotationsverdampfer so weit eingeengt, bis das Produkt anfing auszufallen. Nach dem Abkühlen auf RT wurde das Produkt abgesaugt, mit wenig kaltem Ethylacetat gewaschen und im Hochvakuum getrocknet. Dies ergab eine erste Fraktion der Titelverbindung (4.95 g). Die mit der Waschlösung vereinigte Mutterlauge wurde eingeengt, und der verbliebene Rückstand wurde mittels präparativer HPLC gereinigt (Methode 8). Nach Eindampfen der Produktfraktionen und Trocknen im Hochvakuum wurde so eine zweite Fraktion der Titelverbindung erhalten (0.25 g). Insgesamt wurden somit 5.2 g (85% d. Th., 98% Reinheit) der Titelverbindung erhalten.
$^1$H-NMR (400 MHz, DMSO-$d_6$, δ/ppm): 11.60 (br. s, 1H), 7.84 (s, 1H), 4.95 (s, 2H), 4.08 (t, 2H), 3.90 (q, 2H), 2.82-2.68 (m, 2H), 2.47 (s, 3H), 1.11 (t, 3H).
LC/MS (Methode 1, ESIpos): $R_t$ = 0.79 min, m/z = 404 $[M+H]^+$.

**Beispiel 114**

3-Ethyl-1-(2-metboxyethyl)-5-methyl-6-[(5-oxo-4,5-dihydro-1*H*-1,2,4-triazol-1-yl)methyl]thieno-[2,3-d]pyrimidin-2,4(1*H*,3*H*)-dion

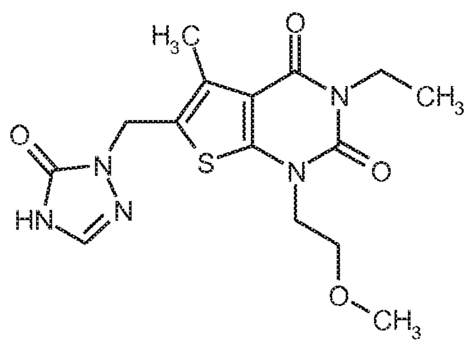

Analog zu dem unter Bsp. 113 beschriebenen Verfahren wurden aus 189 mg (0.466 mmol) der Verbindung aus Bsp. 112 32 mg (18% d. Th.) der Titelverbindung hergestellt. Das Produkt wurde hier nach der präparativen HPLC zur weiteren Reinigung noch mit Pentan verrührt.
$^1$H-NMR (400 MHz, DMSO-$d_6$, δ/ppm): 11.60 (br. s, 1H), 7.84 (s, 1H), 4.93 (s, 2H), 4.01 (t, 2H), 3.89 (q, 2H), 3.62 (t, 2H), 3.23 (s, 3H), 2.45 (s, 3H), 1.11 (t, 3H).
LC/MS (Methode 1, ESIpos): $R_t$ = 0.65 min, m/z = 366 $[M+H]^+$.

**Beispiel 115**

6-{[4-(2,3-Dihydroxypropyl)-5-oxo-4,5-dihydro-1*H*-1,2,4-triazol-1-yl]methyl}-3-ethyl-5-methyl-1-[2-(trifluormethoxy)ethyl]thieno[2,3-d]pyrimidin-2,4(1*H*,3*H*)-dion (*Racemat*)

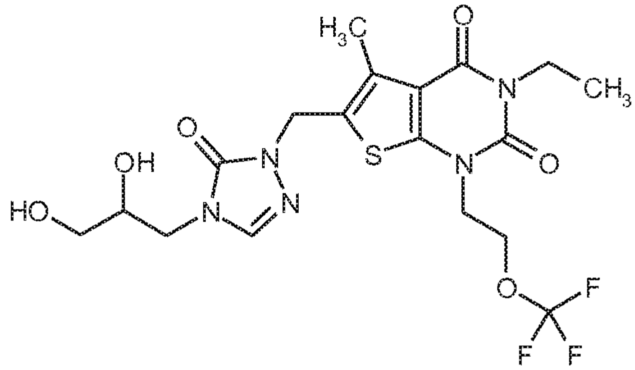

Eine Lösung von 10 mg (0.022 mmol) der Verbindung aus Bsp. 111 in 220 µl Aceton wurde mit 3.8 mg (0.033 mmol) *N*-Methylmorpholin-*N*-oxid (NMO) und 4 µl (0.001 mmol) Osmiumtetroxid-Lösung (4% in Wasser) versetzt und ca. 18 h bei RT gerührt. Dann wurden je ca. 100 µl Natriumthiosulfat-Lösung (20% in Wasser) und 1 M Salzsäure hinzugefügt. Es wurde vom Ungelösten abfiltriert, und das Filtrat wurde mittels präparativer HPLC (Methode 12) in seine Komponenten aufgetrennt. Eindampfen der Produktfraktion und Trocknen im Hochvakuum ergab 7 mg (65% d. Th.) der Titelverbindung.
$^1$H-NMR (500 MHz, DMSO-$d_6$, δ/ppm): 7.85 (s, 1H), 4.99 (s, 2H), 4.41-4.36 (m, 2H), 4.18 (t, 2H), 3.90 (q, 2H), 3.71 (dd, 1H), 3.67-3.60 (m, 1H), 3.43 (dd, 2H), 2.47 (s, 3H), 1.11 (t, 3H) [weitere Signale sind vom Wassersignal überdeckt].
LC/MS (Methode 1, ESIpos): $R_t$ = 0.76 min, m/z = 494 $[M+H]^+$.

**Beispiel 116**

3-Ethyl-5-methyl-6-[(5-oxo-1,5-dihydro-4*H*-1,2,4-triazol-4-yl)methyl]-1-(3,3,3-trifluorpropyl)-thieno[2,3-d]pyrimidin-2,4(1*H*,3*H*)-dion

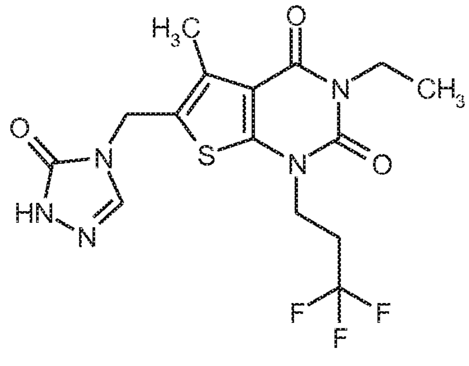

106 mg (0.253 mmol) der Verbindung aus Bsp. 279A wurden in 11 ml Methanol gelöst, mit 13 mg (0.317 mmol) festem Natriumhydroxid versetzt und auf 55°C erwärmt. Nach 40 h wurde das Reaktionsgemisch zur Trockene eingeengt. Der verbliebene Rückstand wurde mittels präparativer HPLC (Methode 8) gereinigt. Nach Eindampfen wurde die Produktfraktion wieder in Methanol gelöst und die Lösung über eine Hydrogencarbonat-Kartusche gegeben (Fa. Polymerlabs, Stratospheres SPE, PL-$HCO_3$ MP SPE). Nach abermaligem Eindampfen und Trocknen im Hochvakuum wurden 68 mg (66% d. Th.) der Titelverbindung erhalten.
$^1$H-NMR (400 MHz, DMSO-$d_6$, δ/ppm): 7.84 (s, 1H), 4.86 (s, 2H), 4.08 (t, 2H), 3.90 (q, 2H), 2.83-2.67 (m, 2H), 2.48 (s, 3H), 1.11 (t, 3H).

LC/MS (Methode 1, ESIpos): $R_t$ = 0.74 min, m/z = 404 $[M+H]^+$.

**Beispiel 117**

[1-{[3-Ethyl-1-(2-methoxyethyl)-5-methyl-2,4-dioxo-1,2,3,4-tetrahydrothieno[2,3-d]pyrimidin-6-yl]methyl}imidazolidin-2-yliden]cyanamid *(mit unbekanntem E/Z-Isomerenverhältnis*)

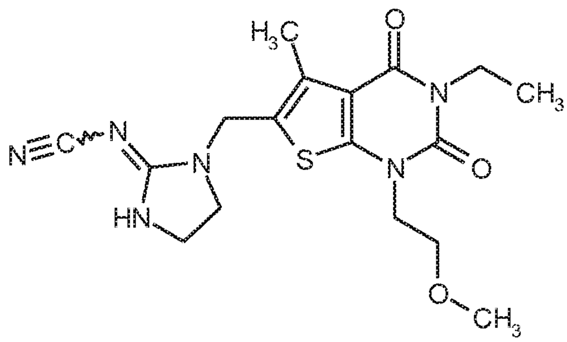

60 mg (0.127 mmol, 72% Reinheit) der Verbindung aus Bsp. 220A wurden in 5 ml DMF gelöst und mit 28 mg (0.190 mmol) Dimethyl-N-cyanodithioiminocarbonat und 35 mg (0.254 mmol) Kaliumcarbonat versetzt. Das Gemisch wurde 4 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das nach Trocknen im Hochvakuum erhaltene Produkt wurde in Pentan/Dichlormethan (10:1) verrührt. Nach neuerlichem Absaugen und Trocknen des Feststoffs im Hochvakuum wurden 25 mg (49% d. Th., 95% Reinheit) der Titelverbindung erhalten.
$^1$H-NMR (400 MHz, DMSO-$d_6$, δ/ppm): 8.07 (s, 1H), 4.47 (s, 2H), 4.03 (t, 2H), 3.90 (q, 2H), 3.64 (t, 2H), 3.49-3.37 (m, 4H), 3.24 (s, 3H), 2.41 (s, 3H), 1.12 (t, 3H).
LC/MS (Methode 1, ESIpos): $R_t$ = 0.73 min, m/z = 391 $[M+H]^+$.

**Beispiel 118**

Methyl-[1-{[3-ethyl-1-(2-methoxyethyl)-5-methyl-2,4-dioxo-1,2,3,4-tetrahydrothieno[2,3-d]-pyrimidin-6-yl]methyl}imidazolidin-2-yliden]carbamat (*Isomer 1, reines E- oder Z-Isomer*)

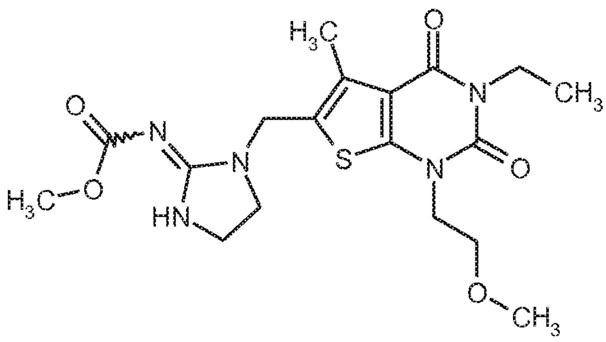

121 mg (0.214 mmol, 60% Reinheit) der Verbindung aus Bsp. 220A und 60 μl (0.428 mmol) Triethylamin wurden in 6 ml Dichlormethan gelöst und mit einer Lösung von 34 mg (0.214 mmol) Methyl-(dichlormethylen)carbamat [DE 1 900 755 (1970); DE 2 036 171 (1972); K. Findeisen et al., Synthesis 1972, 599-605] in 4 ml Dichlormethan versetzt. Nachdem das Reaktionsgemisch 3 h bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels

präparativer HPLC (Methode 8) in seine Komponenten aufgetrennt. Dabei wurden die beiden *E*/*Z*-Isomere des Zielprodukts als getrennte Fraktionen erhalten (*siehe auch Bsp. 119).* Nach Einengen der ersten Produktfraktion und Trocknen des Rückstands im Hochvakuum wurden 15 mg (16% d. Th., 95% Reinheit) der Titelverbindung erhalten.
$^1$H-NMR (400 MHz, DMSO-$d_6$, δ/ppm): 8.34 (br. s, 1H), 4.63 (s, 2H), 4.01 (t, 2H), 3.90 (q, 2H), 3.69 (t, 2H), 3.61 (s, 3H), 3.56-3.47 (m, 2H), 3.44-3.36 (m, 2H), 3.23 (s, 3H), 2.42 (s, 3H), 1.12 (t, 3H).
LC/MS (Methode 1, ESIpos): $R_t$ = 0.63 min, m/z = 424 $[M+H]^+$.

## Beispiel 119

Methyl-[1-{[3-ethyl-1-(2-methoxyethyl)-5-methyl-2,4-dioxo-1,2,3,4-tetrahydrothieno[2,3-d]-pyrimidin-6-yl]methyl}imidazolidin-2-yliden]carbamat (*Isomer 2, reines E- oder Z-Isomer*)

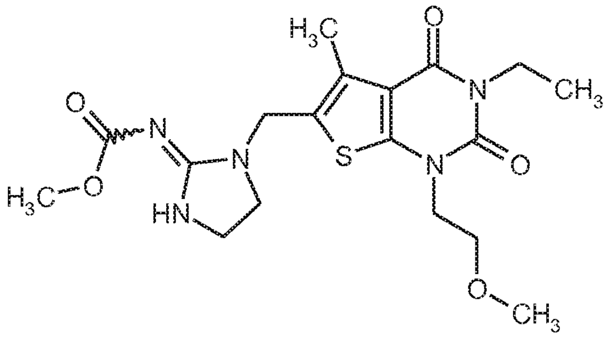

121 mg (0.214 mmol, 60% Reinheit) der Verbindung aus Bsp. 220A und 60 µl (0.428 mmol) Triethylamin wurden in 6 ml Dichlormethan gelöst und mit einer Lösung von 34 mg (0.214 mmol) Methyl-(dichlormethylen)carbamat [DE 1 900 755 (1970); DE 2 036 171 (1972); K. Findeisen et al., Synthesis 1972, 599-605] in 4 ml Dichlormethan versetzt. Nachdem das Reaktionsgemisch 3 h bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) in seine Komponenten aufgetrennt. Dabei wurden die beiden *E*/*Z*-Isomere des Zielprodukts als getrennte Fraktionen erhalten (*vgl. Bsp. 118*). Nach Einengen der zweiten Produktfraktion und Trocknen des Rückstands im Hochvakuum wurden 16 mg (17% d. Th., 95% Reinheit) der Titelverbindung erhalten.
$^1$H-NMR (400 MHz, DMSO-$d_6$, δ/ppm): 8.93 (br. s, 1H), 4.76 (s, 2H), 4.03 (t, 2H), 3.91 (q, 2H), 3.76 (s, 3H), 3.67-3.57 (m, 4H), 3.55-3.47 (m, 2H), 3.23 (s, 3H), 2.42 (s, 3H), 1.12 (t, 3H).
LC/MS (Methode 1, ESIpos): $R_t$ = 0.64 min, m/z = 424 $[M+H]^+$.

## Beispiel 120

6-[(2,3-Dioxopiperazin-1-yl)methyl]-3-ethyl-1-(2-methoxyethyl)-5-methylthieno[2,3-d]pyrimidin-2,4(1*H*,3*H*)-dion

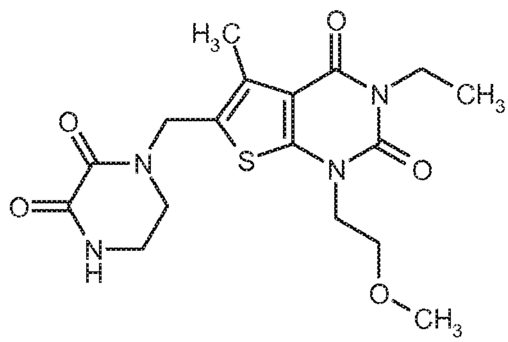

523 mg (ca. 22% Reinheit, 0.338 mmol) der Verbindung aus Bsp. 220A wurden in 5 ml Ethanol gelöst und mit

100 mg (0.676 mmol) Oxalsäurediethylester versetzt. Das Gemisch wurde 22 h bei 80°C gerührt. Anschließend wurde die Reaktionslösung am Rotationsverdampfer eingeengt. Der Rückstand wurde in 9 ml DMSO gelöst und diese Lösung mittels präparativer HPLC gereinigt (Methode 14). Nach Vereinigen der Produktfraktionen und Gefriertrocknung wurden 170 mg der Titelverbindung erhalten.
$^1$H-NMR (400 MHz, DMSO-$d_6$, δ/ppm): 8.64 (br. s, 1H), 4.69 (s, 2H), 4.02 (t, 2H), 3.89 (q, 2H), 3.62 (t, 2H), 3.50-3.45 (m, 2H), 3.33-3.29 (m, 2H), 3.23 (s, 3H), 2.43 (s, 3H), 1.11 (t, 3H).
LC/MS (Methode 3): $R_t$ = 0.78 min, m/z = 395 $[M+H]^+$.

**Beispiel 121**

6-[(2,3-Dioxopiperazin-1-yl)methyl]-1-(3-fluorpropyl)-3-isobutyl-5-methylthieno[2,3-d]pyrimidin-2,4(1*H*,3*H*)-dion

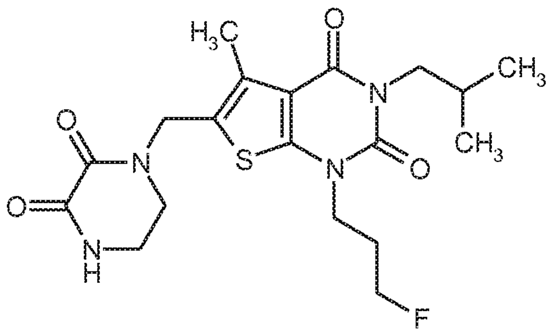

106 mg (0.166 mmol) der Verbindung aus Bsp. 241A wurden in 3 ml Ethanol gelöst und mit 49 mg (0.332 mmol) Oxalsäurediethylester versetzt. Das Gemisch wurde 26 h bei 80°C gerührt.

Anschließend wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 27 mg (70% d. Th.) der Titelverbindung erhalten.
$^1$H-NMR (400 MHz, DMSO-$d_6$, δ/ppm): 8.64 (br. s, 1H), 4.70 (s, 2H), 4.58 (t, 1H), 4.46 (t, 1H), 3.98 (t, 2H), 3.70 (d, 2H), 3.49 (dd, 2H), 2.43 (s, 3H), 2.13-1.96 (m, 3H), 0.84 (d, 6H).
LC/MS (Methode 3): $R_t$ = 0.99 min, m/z = 425 $[M+H]^+$.

**Beispiel 122**

6-[(2,3-Dioxopiperazin-1-yl)methyl]-5-methyl-3-(2,2,2-trifluorethyl)-1-(3,3,3-trifluorpropyl)-thieno[2,3-d]pyrimidin-2,4(1*H*,3*H*)-dion

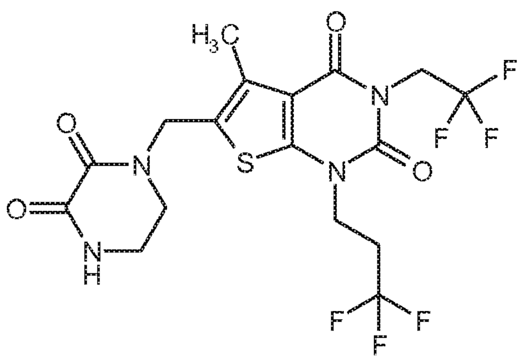

126 mg (0.271 mmol) der Verbindung aus Bsp. 247A wurden in 4 ml Ethanol gelöst und mit 80 mg (0.332 mmol) Oxalsäurediethylester versetzt. Das Gemisch wurde 68 h bei 80°C gerührt. Anschließend wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 48 mg (36% d. Th.) der Titelverbindung erhalten.
$^1$H-NMR (400 MHz, DMSO-$d_6$, δ/ppm): 8.65 (br. s, 1H), 4.75-4.65 (m, 4H), 4.14 (t, 2H), 3.51 (dd, 2H), 2.84-2.70 (m, 2H), 2.44 (s, 3H).
LC/MS (Methode 3): $R_t$ = 1.01 min, m/z = 487 [M+H]$^+$.

**Beispiel 123**

6-[(2,3-Dioxopiperazin-1-yl)methyl]-5-methyl-1-(oxetan-2-ylmethyl)-3-(2,2,2-trifluorethyl)thieno-[2,3-d]pyrimidin-2,4(1*H*,3*H*)-dion (*Racemat*)

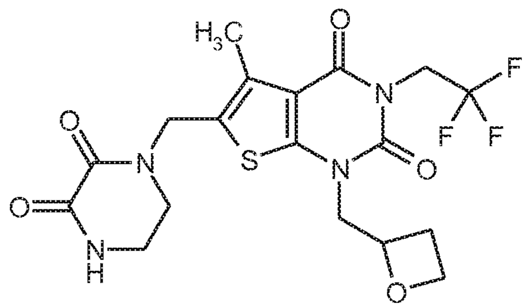

73 mg (0.158 mmol) der Verbindung aus Bsp. 251A wurden in 4 ml Ethanol gelöst und mit 43 mg (0.292 mmol) Oxalsäurediethylester versetzt. Das Gemisch wurde 26 h bei 80°C in einer Mikrowellenapparatur gerührt. Anschließend wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 29 mg (39% d. Th.) der Titelverbindung erhalten.
$^1$H-NMR (400 MHz, DMSO-$d_6$, δ/ppm): 8.64 (br. s, 1H), 5.05-4.97 (m, 1H), 4.74-4.65 (m, 4H), 4.52-4.37 (m, 2H), 4.22-4.11 (m, 2H), 3.51-3.47 (m, 2H), 2.74-2.64 (m, 2H), 2.43 (s, 3H).
LC/MS (Methode 3): $R_t$ = 0.87 min, m/z = 461 [M+H]$^+$.

**Beispiel 124**

6-[(2,3-Dioxopiperazin-1-yl)methyl]-1,5-dimethyl-3-(2,2,2-trifluorethyl)thieno[2,3-d]pyrimidin-2,4(1*H*,3*H*)-dion

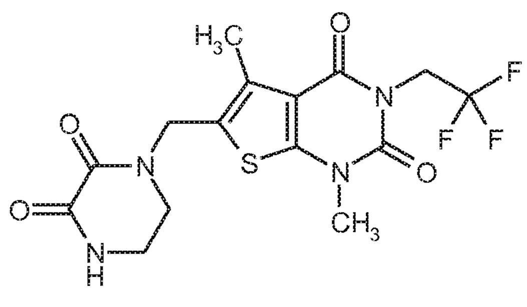

128 mg (44% Reinheit, 0.161 mmol) der Verbindung aus Bsp. 254A wurden in 3 ml Ethanol gelöst und mit 47 mg (0.321 mmol) Oxalsäurediethylester versetzt. Das Gemisch wurde 26 h bei 80°C in einer Mikrowellenapparatur gerührt. Danach wurde die Reaktionslösung direkt mittels präparativer HPLC gereinigt (Methode 14). Nach Vereinigen der Produktfraktionen und Gefriertrocknung wurden 15 mg (23% d. Th.) der Titelverbindung erhalten.
$^1$H-NMR (400 MHz, DMSO-$d_6$, δ/ppm): 8.65 (br. s, 1H), 4.75-4.64 (m, 4H), 3.50 (dd, 2H), 3.45 (s, 3H), 2.44 (s, 3H).
LC/MS (Methode 3): $R_t$ = 0.84 min, m/z = 405 [M+H]$^+$.

**Beispiel 125**

3-(2,2-Difluorethyl)-6-[(2,3-dioxopiperazin-1-yl)methyl]-5-methyl-1-(3,3,3-trifluorpropyl)thieno-[2,3-d]pyrimidin-2,4(1*H*,3*H*)-dion

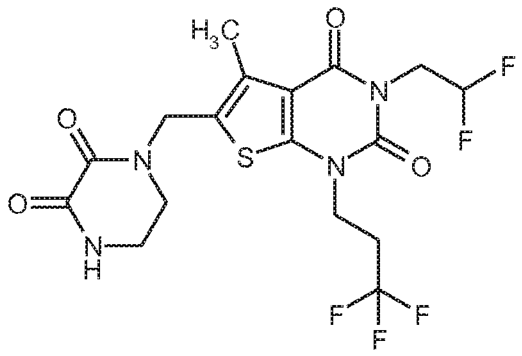

100 mg (0.171 mmol) der Verbindung aus Bsp. 255A wurden in 4 ml Ethanol gelöst und mit 50 mg (0.343 mmol) Oxalsäurediethylester versetzt. Das Gemisch wurde 18 h bei 80°C in einer Mikrowellenapparatur gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 42 mg (51% d. Th.) der Titelverbindung erhalten.
$^1$H-NMR (400 MHz, DMSO-$d_6$, δ/ppm): 8.65 (br. s, 1H), 6.38-6.03 (m, 1H), 4.72 (s, 2H), 4.34-4.24 (m, 2H), 4.12 (t, 2H), 3.50 (dd, 2H), 2.84-2.70 (m, 2H), 2.44 (s, 3H).
LC/MS (Methode 3): $R_t$ = 0.95 min, m/z = 469 [M+H]$^+$.

**Beispiel 126**

3-Ethyl-6-{[(3*R*)-3-hydroxy-2-oxopyrrolidin-1-yl]methyl}-5-methyl-1-(3,3,3-trifluorpropyl)-thieno[2,3-d]pyrimidin-2,4(1*H*,3*H*)-dion

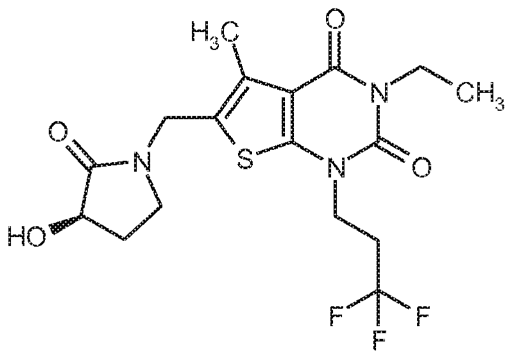

250 mg (0.672 mmol) der Verbindung aus Bsp. 199A wurden in 2 ml Methanol gelöst und über eine Hydrogencarbonat-Kartusche (Fa. Polymerlabs, Stratospheres SPE, PL-$HCO_3$ MP SPE) gegeben. Nach Eindampfen der Lösung wurde so das freie Amin 6-(Aminomethyl)-3-ethyl-5-methyl-1-(3,3,3-trifluorpropyl)thieno[2,3-d]pyrimidin-2,4(1*H*,3*H*)-dion erhalten, das analog zu dem unter Bsp. 7 beschriebenen Verfahren mit 101 mg (0.639 mmol) [(4*R*)-2,2-Dimethyl-5-oxo-1,3-dioxolan-4-yl]acetaldehyd [Int. Pat. Appl. WO 2012/037393-A1, Preparation B / Step B] zu 128 mg (45% d. Th.) der Titelverbindung umgesetzt wurde.
$^1$H-NMR (400 MHz, DMSO-$d_6$, δ/ppm): 5.59 (d, 1H), 4.50 (s, 2H), 4.16-4.03 (m, 3H), 3.90 (q, 2H), 3.29-3.21 (m, 1H), 3.19-3.09 (m, 1H), 2.85-2.69 (m, 2H), 2.42 (s, 3H), 2.30-2.18 (m, 1H), 1.67 (m, 1H), 1.11 (t, 3H).
LC/MS (Methode 1, ESIpos): $R_t$ = 0.82 min, m/z = 420 [M+H]$^+$.

**Beispiel 127**

3-Ethyl-5-methyl-6-[(5-oxo-2,5-dihydro-1*H*-pyrazol-1-yl)methyl]-1-(3,3,3-trifluorpropyl)-thieno[2,3-d]pyrimidin-2,4(1*H*,3*H*)-dion

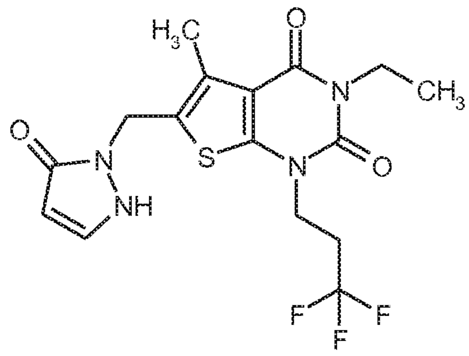

100 mg (0.182 mmol) der Verbindung aus Bsp. 345A wurden in 2 ml konzentrierter Schwefelsäure suspendiert und 30 min bei RT gerührt. Danach wurde das Reaktionsgemisch vorsichtig in etwas Eiswasser eingetragen. Der ausgefallene Feststoff wurde abgesaugt, mit Wasser neutral gewaschen und dann mittels präparativer HPLC gereinigt (Methode 8). Nach Einengen der Produktfraktionen und Trocknen im Hochvakuum wurden 30 mg (41% d. Th.) der Titelverbindung erhalten.
$^1$H-NMR (400 MHz, DMSO-$d_6$, $\delta$/ppm): 11.16 (breit, 1H), 7.17 (d, 1H), 5.32 (d, 1H), 5.16 (s, 2H), 4.07 (t, 2H), 3.89 (q, 2H), 2.86-2.62 (m, 2H), 2.48 (s, 3H), 1.11 (t, 3H).
LC/MS (Methode 1, ESIneg): $R_t$ = 0.81 min, m/z = 401 $[M-H]^-$.

**Beispiel 128**

3-Ethyl-1-(2-methoxyethyl)-5-methyl-6-[(5-oxo-2,5-dihydro-1*H*-pyrazol-1-yl)methyl]thieno-[2,3-d]pyrimidin-2,4(1*H*,3*H*)-dion

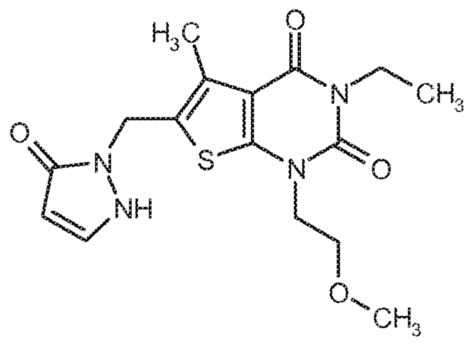

223 mg (0.437 mmol) der Verbindung aus Bsp. 346A wurden in 5 ml Dichlormethan gelöst und mit einem Tropfen konzentrierter Schwefelsäure versetzt. Nachdem das Reaktionsgemisch 30 min bei RT gerührt worden war, wurde es vorsichtig auf Eiswasser gegossen. Die organische Phase wurde abgetrennt, mit gesättigter Natriumchlorid-Lösung gewaschen und über wasserfreiem Magnesiumsulfat getrocknet. Nach Filtration und Einengen wurde der verbliebene Feststoff mit Acetonitril bei RT verrührt. Nach neuerlichem Absaugen und Trocknen im Hochvakuum wurden 96 mg (60% d. Th.) der Titelverbindung erhalten.
$^1$H-NMR (400 MHz, DMSO-$d_6$, $\delta$/ppm): 11.12 (breit, 1H), 7.16 (s, 1H), 5.32 (d, 1H), 5.14 (s, 2H), 3.99 (t, 2H), 3.89 (q, 2H), 3.60 (t, 2H), 3.22 (s, 3H), 2.47 (s, 3H), 1.11 (t, 3H).
LC/MS (Methode 17, ESIneg): $R_t$ = 1.11 min, m/z = 363.11 $[M-H]^-$.

**Beispiel 129**

3-Ethyl-5-methyl-6-[(5-oxo-2,5-dihydro-1*H*-pyrazol-1-yl)methyl]-1-(tetrahydrofuran-2-ylmethyl)-thieno[2,3-d]pyrimidin-2,4(1*H*,3*H*)-dion (*Racemat*)

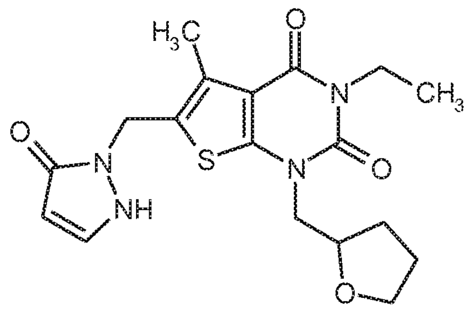

370 mg (0.689 mmol) der Verbindung aus Bsp. 347A wurden in 7 ml Dichlormethan gelöst und mit zwei Tropfen konzentrierter Schwefelsäure versetzt. Nachdem das Reaktionsgemisch 30 min bei RT gerührt worden war, wurde es vorsichtig auf Eiswasser gegossen. Die organische Phase wurde abgetrennt, mit gesättigter Natriumchlorid-Lösung gewaschen und über wasserfreiem Magnesiumsulfat getrocknet. Nach Filtration und Einengen wurde der verbliebene Feststoff mit Acetonitril bei RT verrührt. Nach neuerlichem Absaugen und Trocknen im Hochvakuum wurden 155 mg (57% d. Th.) der Titelverbindung erhalten.
$^1$H-NMR (400 MHz, DMSO-$d_6$, δ/ppm): 11.11 (br. s, 1H), 7.15 (s, 1H), 5.32 (s, 1H), 5.13 (s, 2H), 4.27-4.13 (m, 1H), 4.01 (dd, 1H), 3.89 (q, 2H), 3.77-3.52 (m, 3H), 2.47 (s, 3H), 2.04-1.72 (m, 3H), 1.70-1.58 (m, 1H), 1.10 (t, 3H).
LC/MS (Methode 1, ESIneg): $R_t$ = 0.72 min, m/z = 389 [M-H]$^-$.

**Beispiel 130**

3-Ethyl-5-methyl-6-[(5-oxo-2,5-dihydro-1*H*-pyrazol-1-yl)methyl]-1-(tetrahydrofuran-2-ylmethyl)-thieno[2,3-d]pyrimidin-2,4(1*H*,3*H*)-dion (*Enantiomer 1*)

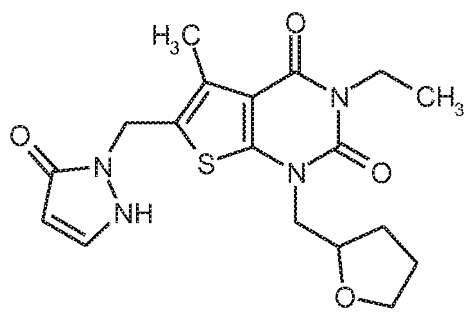

135 mg (0.346 mmol) der racemischen Verbindung aus Bsp. 129 wurden in 6 ml Ethanol gelöst und in 20 Portionen über präparative HPLC an chiraler Phase in die Enantiomere aufgetrennt [Säule: Daicel Chiralcel OX-H, 5 µm, 250 mm x 20 mm; Laufmittel: Ethanol; Fluss: 15 ml/min; Temperatur: 40°C; Detektion: 220 nm]. Nach Eindampfen der Produktfraktionen und Trocknen im Hochvakuum wurden 49 mg (72% d. Th.) von Enantiomer 1 erhalten (99% ee, chirale analytische HPLC).
$^1$H-NMR (400 MHz, DMSO-$d_6$, δ/ppm): 11.10 (br. s, 1H), 7.14 (s, 1H), 5.27 (s, 1H), 5.12 (s, 2H), 4.25-4.14 (m, 1H), 4.01 (dd, 1H), 3.89 (q, 2H), 3.77-3.53 (m, 3H), 2.47 (s, 3H), 2.03-1.73 (m, 3H), 1.70-1.57 (m, 1H), 1.10 (t, 3H).
Chirale analytische HPLC [Säule: Daicel Chiralcel OX-H, 5 µm, 250 mm x 4.6 mm; Laufmittel: Ethanol + 0.2% TFA + 1% Wasser; Fluss: 1 ml/min; Temperatur: 40°C; Detektion: 260 nm]: $R_t$ = 5.65 min.

**Beispiel 131**

3-Ethyl-5-methyl-6-[(5-oxo-2,5-dihydro-1*H*-pyrazol-1-yl)methyl]-1-(tetrahydrofuran-2-ylmethyl)-thieno[2,3-d]pyrimidin-2,4(1*H*,3*H*)-dion (*Enantiomer 2*)

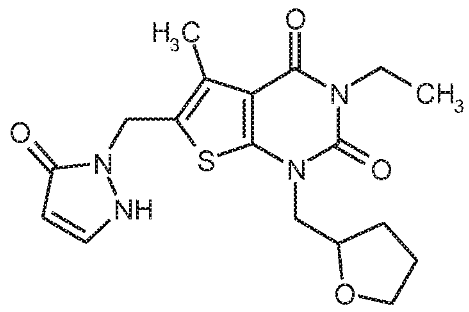

135 mg (0.346 mmol) der racemischen Verbindung aus Bsp. 129 wurden in 6 ml Ethanol gelöst und in 20 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 40°C; Detektion: 220 nm]. Nach Eindampfen der Produktfraktionen und Trocknen im Hochvakuum wurden 52 mg (77% d. Th.) von Enantiomer 2 erhalten (99% ee, chirale analytische HPLC).

$^1$H-NMR (400 MHz, DMSO-$d_6$, δ/ppm): 11.16 (br. s, 1H), 7.14 (s, 1H), 5.29 (s, 1H), 5.12 (s, 2H), 4.25-4.14 (m, 1H), 4.01 (dd, 1H), 3.89 (q, 2H), 3.76-3.54 (m, 3H), 2.47 (s, 3H), 2.04-1.74 (m, 3H), 1.71-1.58 (m, 1H), 1.10 (t, 3H).

Chirale analytische HPLC [Säule: Daicel Chiralcel OX-H, 5 μm, 250 mm x 4.6 mm; Laufmittel: Ethanol + 0.2% TFA + 1% Wasser; Fluss: 1 ml/min; Temperatur: 40°C; Detektion: 260 nm]: $R_t$ = 6.96 min.

**Beispiel 132**

3-Isopropyl-5-methyl-6-[(5-oxo-2,5-dihydro-1*H*-pyrazol-1-yl)methyl]-1-(3,3,3-trifluorpropyl)-thieno[2,3-d]pyrimidin-2,4(1*H*,3*H*)-dion

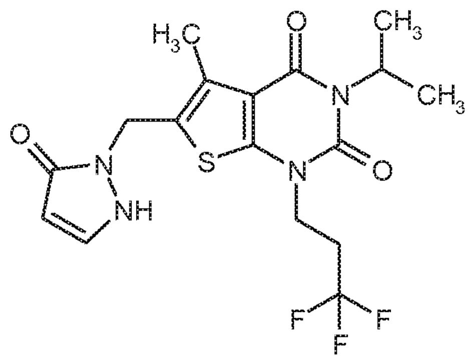

270 mg (0.480 mmol) der Verbindung aus Bsp. 348A wurden in 5 ml Dichlormethan gelöst und mit einem Tropfen konzentrierter Schwefelsäure versetzt. Nachdem das Reaktionsgemisch 30 min bei RT gerührt worden war, wurde es vorsichtig auf Eiswasser gegossen. Die organische Phase wurde abgetrennt, mit gesättigter Natriumchlorid-Lösung gewaschen und über wasserfreiem Magnesiumsulfat getrocknet. Nach Filtration und Einengen wurde der verbliebene Feststoff mit Acetonitril bei RT verrührt. Nach neuerlichem Absaugen und Trocknen wurde eine erste Fraktion von 92 mg der Titelverbindung erhalten. Die Mutterlauge des Verrührens wurde mittels präparativer HPLC (Methode 8) gereinigt und ergab eine zweite Fraktion von 54 mg des Produkts. Insgesamt wurden so 146 mg (73% d. Th.) der Titelverbindung erhalten.

$^1$H-NMR (400 MHz, DMSO-$d_6$, δ/ppm): 11.13 (s, 1H), 7.15 (d, 1H), 5.32 (d, 1H), 5.15 (s, 2H), 5.11 (sept, 1H), 4.03 (t, 2H), 2.82-2.61 (m, 2H), 2.47 (s, 3H), 1.39 (d, 6H).

LC/MS (Methode 17, ESIneg): $R_t$ = 1.62 min, m/z = 415.11 $[M-H]^-$.

**Beispiel 133**

3-Isopropyl-1-(2-methoxyethyl)-5-methyl-6-[(5-oxo-2,5-dihydro-1*H*-pyrazol-1-yl)methyl]thieno-[2,3-d]pyrimidin-2,4(1*H*,3*H*)-dion

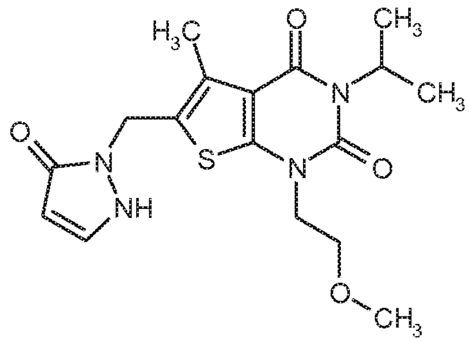

288 mg (0.549 mmol) der Verbindung aus Bsp. 349A wurden in 6 ml Dichlormethan gelöst und mit zwei Tropfen konzentrierter Schwefelsäure versetzt. Nachdem das Reaktionsgemisch 30 min bei RT gerührt worden war, wurde es vorsichtig auf Eiswasser gegossen. Die organische Phase wurde abgetrennt, mit gesättigter Natriumchlorid-Lösung gewaschen und über wasserfreiem Magnesiumsulfat getrocknet. Nach Filtration und Einengen wurde das verbliebene Rohprodukt mittels präparativer HPLC gereinigt (Methode 8). Nach Vereinigen der Produktfraktionen, Einengen und Trocknen im Hochvakuum wurden 105 mg (50% d. Th.) der Titelverbindung erhalten.
$^1$H-NMR (400 MHz, DMSO-$d_6$, $\delta$/ppm): 11.11 (s, 1H), 7.14 (d, 1H), 5.32 (d, 1H), 5.13 (s, 2H), 5.12 (sept, 1H), 3.95 (t, 2H), 3.59 (t, 2H), 3.22 (s, 3H), 2.45 (s, 3H), 1.39 (d, 6H).
LC/MS (Methode 1, ESIneg): $R_t$ = 0.72 min, m/z = 377 $[M-H]^-$.

**Beispiel 134**

3-Isopropyl-5-methyl-6-[(5-oxo-2,5-dihydro-1*H*-pyrazol-1-yl)methyl]-1-(tetrahydrofuran-2yl-methyl)thieno[2,3-d]pyrimidin-2,4(1*H*,3*H*)-dion (*Racemat*)

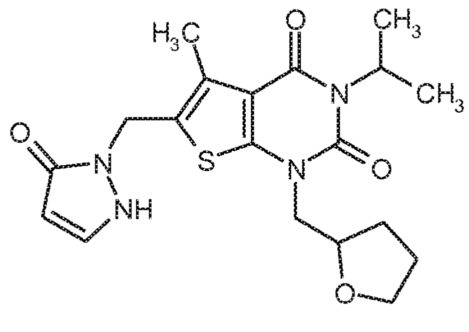

Analog zu dem unter Bsp. 133 beschriebenen Verfahren wurden aus 508 mg (0.922 mmol) der Verbindung aus Bsp. 350A 150 mg (39% d. Th., 97% Reinheit) der Titelverbindung hergestellt. Die Reaktionszeit betrug hier 1 h.
$^1$H-NMR (400 MHz, DMSO-$d_6$, $\delta$/ppm): 11.10 (br. s, 1H), 7.15 (s, 1H), 5.31 (d, 1H), 5.13 (sept, 1H), 5.12 (s, 2H), 4.24-4.13 (m, 1H), 4.01 (dd, 1H), 3.76-3.67 (m, 1H), 3.65-3.54 (m, 2H), 2.45 (s, 3H), 2.03-1.74 (m, 3H), 1.70-1.57 (m, 1H), 1.39 (d, 6H).
LC/MS (Methode 1, ESIneg): $R_t$ = 0.77 min, m/z = 403 $[M-H]^-$.

**Beispiel 135**

3-Isopropyl-5-methyl-6-[(5-oxo-2,5-dihydro-1*H*-pyrazol-1-yl)methyl]-1-(tetrahydrofuran-2-ylmethyl)thieno[2,3-d]pyrimidin-2,4(1*H*,3*H*)-dion (*Enantiomer 1*)

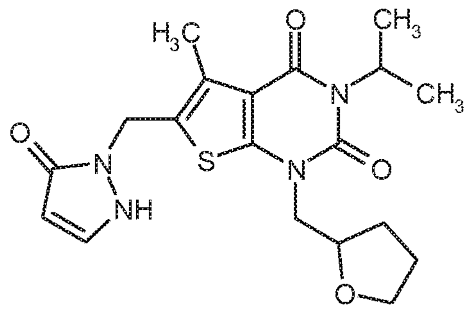

130 mg (0.321 mmol) der racemischen Verbindung aus Bsp. 134 wurden in 6 ml Ethanol gelöst und in 12 Portionen über präparative HPLC an chiraler Phase in die Enantiomere aufgetrennt [Säule: Daicel Chiralcel OX-H, 5 μm, 250 mm x 20 mm; Laufmittel: Isohexan/Ethanol 1:1; Fluss: 15 ml/min; Temperatur: 40°C; Detektion: 220 nm]. Nach Eindampfen der Produktfraktionen und Trocknen im Hochvakuum wurden 50 mg (76% d. Th.) von Enantiomer 1 erhalten (99% ee, chirale analytische HPLC).

$^1$H-NMR (400 MHz, DMSO-$d_6$, δ/ppm): 11.18 (br. s, 1H), 7.15 (s, 1H), 5.30 (s, 1H), 5.13 (sept, 1H), 5.12 (s, 2H), 4.24-4.12 (m, 1H), 4.00 (dd, 1H), 3.71 (q, 1H), 3.64-3.54 (m, 2H), 2.45 (s, 3H), 2.04-1.74 (m, 3H), 1.70-1.57 (m, 1H), 1.39 (d, 6H).

Chirale analytische HPLC [Säule: Daicel Chiralcel OX-H, 5 μm, 250 mm x 4.6 mm; Laufmittel: Isohexan/Ethanol 1:1 + 0.2% TFA + 1% Wasser; Fluss: 1 ml/min; Temperatur: 40°C; Detektion: 220 nm]: $R_t$ = 5.59 min.

**Beispiel 136**

3-Isopropyl-5-methyl-6-[(5-oxo-2,5-dihydro-1*H*-pyrazol-1-yl)methyl]-1-(tetrahydrofuran-2-ylmethyl)thieno[2,3-d]pyrimidin-2,4(1*H*,3*H*)-dion (*Enantiomer 2*)

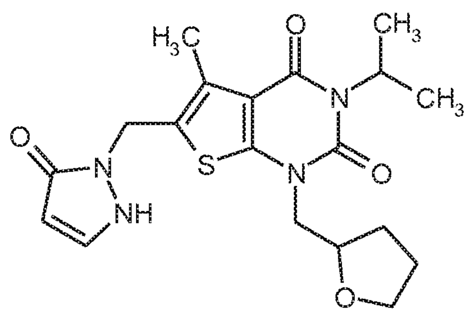

130 mg (0.321 mmol) der racemischen Verbindung aus Bsp. 134 wurden in 6 ml Ethanol gelöst und in 12 Portionen über präparative HPLC an chiraler Phase in die Enantiomere aufgetrennt [Säule: Daicel Chiralcel OX-H, 5 μm, 250 mm x 20 mm; Laufmittel: Isohexan/Ethanol 1:1; Fluss: 15 ml/min; Temperatur: 40°C; Detektion: 220 nm]. Nach Eindampfen der Produktfraktionen und Trocknen im Hochvakuum wurden 50 mg (76% d. Th.) von Enantiomer 2 erhalten (99% ee, chirale analytische HPLC).

$^1$H-NMR (400 MHz, DMSO-$d_6$, δ/ppm): 11.11 (br. s, 1H), 7.14 (d, 1H), 5.31 (d, 1H), 5.13 (sept, 1H), 5.13 (s, 2H), 4.23-4.13 (m, 1H), 4.01 (dd, 1H), 3.76-3.67 (m, 1H), 3.65-3.54 (m, 2H), 2.45 (s, 3H), 2.03-1.73 (m, 3H), 1.70-1.58 (m, 1H), 1.39 (d, 6H).

Chirale analytische HPLC [Säule: Daicel Chiralcel OX-H, 5 μm, 250 mm x 4.6 mm; Laufmittel: Isohexan/Ethanol 1:1 + 0.2% TFA + 1% Wasser, Fluss: 1 ml/min; Temperatur: 40°C; Detektion: 220 nm]: $R_t$ = 6.46 min.

**Beispiel 137**

1-(2,2-Difluorethyl)-3-ethyl-5-methyl-6-[(2-thioxoimidazolidin-1-yl)methyl]thieno[2,3-d]pyrimidin-2,4(1*H*,3*H*)-dion

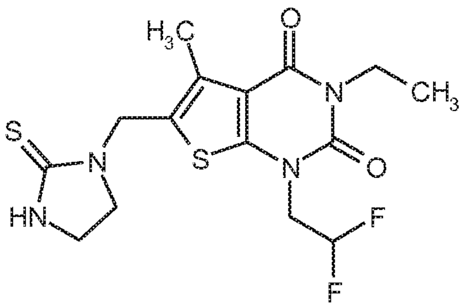

191 mg (0.48 mmol) der Verbindung aus Bsp. 212A wurden in 10 ml Dioxan gelöst und mit 135 mg (0.72 mmol) 1,1'-Thiocarbonyldiimidazol versetzt. Das Gemisch wurde 16 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 31.8 mg (16% d. Th.) der Titelverbindung erhalten.
$^1$H-NMR (400 MHz, DMSO-$d_6$, $\delta$/ppm): 8.37 (s, 1H), 6.51-6.19 (m, 1H), 4.84 (s, 2H), 4.30 (td, 2H), 3.90 (q, 2H), 3.57-3.48 (m, 2H), 3.43-3.37 (m, 2H), 2.46-2.42 (m, 3H), 1.12 (t, 3H).
LC/MS (Methode 3, ESIpos): $R_t$ = 1.02 min, m/z = 389 $[M+H]^+$.

**Beispiel 138**

1-[2-(Cyclopropyloxy)ethyl]-3-ethyl-5-methyl-6-[(2-thioxoimidazolidin-1-yl)methyl]thieno[2,3-d]-pyrimidin-2,4(1*H*,3*H*)-dion

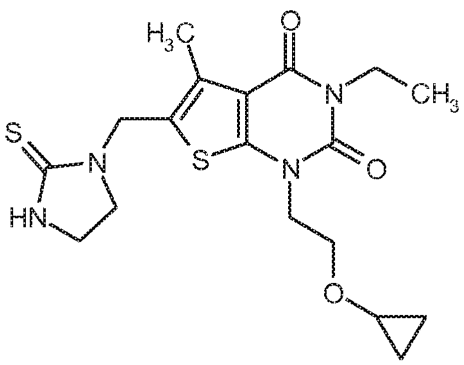

140 mg (0.271 mmol) der Verbindung aus Bsp. 308A wurden in 15 ml Dioxan gelöst und mit 76 mg (0.407 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 76 mg (68% d. Th.) der Titelverbindung erhalten.
$^1$H-NMR (400 MHz, DMSO-$d_6$, $\delta$/ppm): 8.36 (s, 1H), 4.82 (s, 2H), 3.99 (t, 2H), 3.89 (q, 2H), 3.72 (t, 2H), 3.57-3.47 (m, 2H), 3.44-3.37 (m, 2H), 3.33-3.29 (m, 1H), 2.43 (s, 3H), 1.11 (t, 3H), 0.41-0.36 (m, 4H).
LC/MS (Methode 3, ESIpos): $R_t$ = 1.08 min, m/z = 409 $[M+H]^+$.

**Beispiel 139**

3-Ethyl-5-methyl-1-(tetrahydrofuran-2-ylmethyl)-6-[(2-thioxoimidazolidin-1-yl)methyl]thieno-[2,3-d]pyrimidin-2,4(1*H*,3*H*)-dion (*Enantiomer 1*)

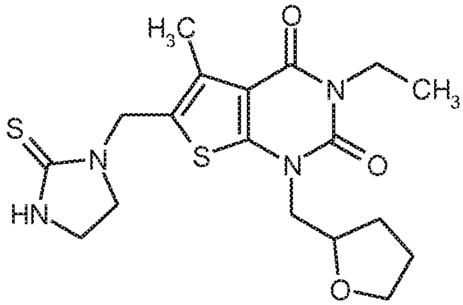

230 mg der racemischen Verbindung aus Bsp. 35 wurden in 8 ml eines Methanol/Dichlormethan-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent: Methanol; Fluss: 60 ml/ min; Temperatur: RT; UV-Detektion: 254 nm]. Die Produktfraktionen wurden am Rotationsverdampfer eingeengt, mit *tert*.-Butanol versetzt und gefriergetrocknet. Es wurden 88 mg (37% d. Th.) der Titelverbindung (Enantiomer 1) sowie 90 mg (38% d. Th.) des Enantiomers 2 (siehe Beispiel 140) erhalten.
$^1$H-NMR (400 MHz, DMSO-$d_6$, δ/ppm): 8.35 (s, 1H), 4.86-4.77 (m, 2H), 4.26-4.18 (m, 1H), 4.02 (dd, 1H), 3.90 (q, 2H), 3.77-3.72 (m, 1H), 3.69 (dd, 1H), 3.61 (td, 1H), 3.56-3.50 (m, 2H), 3.43-3.37 (m, 2H), 2.43 (s, 3H), 2.02-1.92 (m, 1H), 1.92-1.76 (m, 2H), 1.66 (ddt, 1H), 1.14-1.08 (m, 3H).
Chirale analytische HPLC [Säule: Daicel Chiralpak IC, 3 μm, 100 mm x 4.6 mm; Eluent: Methanol; Fluss: 1.0 ml/min; Temperatur: 25°C; Injektion: 5 μl; DAD 254 nm]: $R_t$ = 8.73 min.

**Beispiel 140**

3-Ethyl-5-methyl-1-(tetrahydrofuran-2-ylmethyl)-6-[(2-thioxoimidazolidin-1-yl)methyl]thieno-[2,3-d]pyrimidin-2,4(1*H*,3*H*)-dion (*Enantiomer 2*)

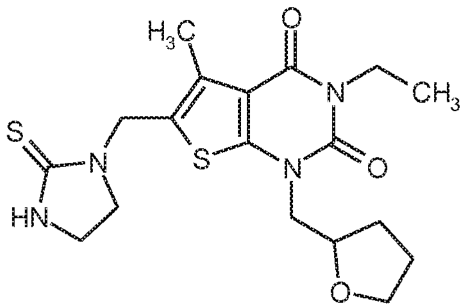

Die Titelverbindung (90 mg) wurde als zweites Enantiomer bei der unter Bsp. 139 beschriebenen präparativen HPLC-Trennung des Racemats aus Bsp. 35 erhalten.
$^1$H-NMR (400 MHz, DMSO-$d_6$, δ/ppm): 8.35 (s, 1H), 4.86-4.77 (m, 2H), 4.26-4.18 (m, 1H), 4.02 (dd, 1H), 3.90 (q, 2H), 3.77-3.72 (m, 1H), 3.69 (dd, 1H), 3.64-3.58 (m, 1H), 3.56-3.50 (m, 2H), 3.43-3.37 (m, 2H), 2.43 (s, 3H), 2.02-1.93 (m, 1H), 1.93-1.76 (m, 2H), 1.66 (ddt, 1H), 1.14-1.08 (m, 3H).
Chirale analytische HPLC [Säule: Daicel Chiralpak IC, 3 μm, 100 mm x 4.6 mm; Eluent: Methanol; Fluss: 1.0 ml/min; Temperatur: 25°C; Injektion: 5 μl; DAD 254 nm]: $R_t$ = 10.25 min.

**Beispiel 141**

3-Ethyl-5-methyl-1-(tetrahydro-2*H*-pyran-2-ylmethyl)-6-[(2-thioxoimidazolidin-1-yl)methyl]-thieno[2,3-d]pyrimidin-2,4(1*H*,3*H*)-dion (*Enantiomer 1*)

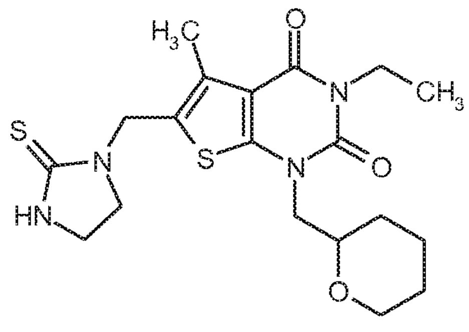

158 mg der racemischen Verbindung aus Bsp. 36 wurden in 6 ml eines Methanol/Dichlormethan-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent: Methanol; Fluss: 60 ml/ min; Temperatur: RT; UV-Detektion: 254 nm]. Die Produktfraktionen wurden am Rotationsverdampfer eingeengt, mit *tert*.-Butanol versetzt und gefriergetrocknet. Es wurden 65 mg (42% d. Th.) der Titelverbindung (Enantiomer 1) sowie 66 mg (42% d. Th.) des Enantiomers 2 (siehe Beispiel 142) erhalten.

$^1$H-NMR (400 MHz, DMSO-$d_6$, δ/ppm): 8.35 (s, 1H), 4.88-4.75 (m, 2H), 4.01-3.93 (m, 1H), 3.89 (q, 2H), 3.84-3.77 (m, 1H), 3.72-3.63 (m, 2H), 3.58-3.46 (m, 2H), 3.44-3.36 (m, 2H), 3.30-3.21 (m, 1H), 2.43 (s, 3H), 1.78 (d, 1H), 1.61 (d, 1H), 1.52-1.38 (m, 3H), 1.32-1.19 (m, 1H), 1.14-1.08 (m, 3H).

Chirale analytische HPLC [Säule: Daicel Chiralpak IC, 3 μm, 100 mm x 4.6 mm; Eluent: Methanol; Fluss: 1.0 ml/min; Temperatur: 25°C; Injektion: 5 μl; DAD 254 nm]: $R_t$ = 7.03 min.

**Beispiel 142**

3-Ethyl-5-methyl-1-(tetrahydro-2*H*-pyran-2-ylmethyl)-6-[(2-thioxoimidazolidin-1-yl)methyl]-thieno[2,3-d]pyrimidin-2,4(1*H*,3*H*)-dion (*Enantiomer 2*)

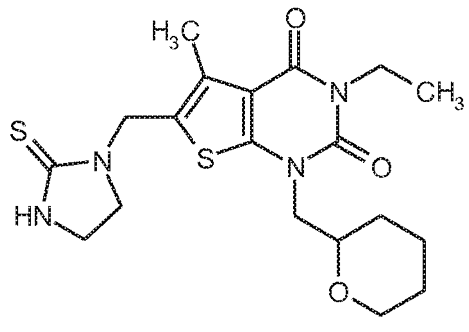

Die Titelverbindung (66 mg) wurde als zweites Enantiomer bei der unter Bsp. 141 beschriebenen präparativen HPLC-Trennung des Racemats aus Bsp. 36 erhalten.

$^1$H-NMR (400 MHz, DMSO-$d_6$, δ/ppm): 8.35 (s, 1H), 4.88-4.75 (m, 2H), 4.01-3.93 (m, 1H), 3.89 (q, 2H), 3.84-3.77 (m, 1H), 3.72-3.63 (m, 2H), 3.58-3.46 (m, 2H), 3.44-3.36 (m, 2H), 3.29-3.21 (m, 1H), 2.43 (s, 3H), 1.78 (d, 1H), 1.61 (d, 1H), 1.52-1.38 (m, 3H), 1.32-1.19 (m, 1H), 1.14-1.08 (m, 3H).

Chirale analytische HPLC [Säule: Daicel Chiralpak IC, 3 μm, 100 mm x 4.6 mm; Eluent: Methanol; Fluss: 1.0 ml/min; Temperatur: 25°C; Injektion: 5 μl; DAD 254 nm]: $R_t$ = 8.43 min.

**Beispiel 143**

1-(2,2-Difluorethyl)-3-isobutyl-5-methyl-6-[(2-thioxoimidazolidin-1-yl)methyl]thieno[2,3-d]-pyrimidin-2,4(1*H*,3*H*)-dion

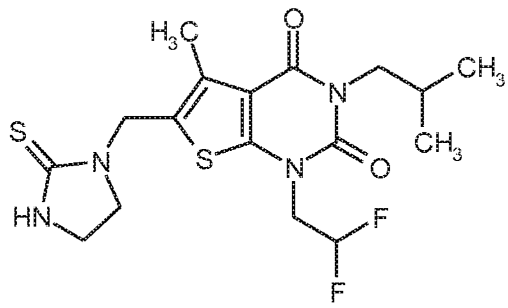

138 mg (0.28 mmol) der Verbindung aus Bsp. 313A wurden in 15 ml Dioxan gelöst und mit 79 mg (0.42 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 41 mg (35% d. Th.) der Titelverbindung erhalten.
$^1$H-NMR (400 MHz, DMSO-$d_6$, $\delta$/ppm): 8.35 (s, 1H), 6.53-6.17 (m, 1H), 4.84 (s, 2H), 4.31 (td, 2H), 3.71 (d, 2H), 3.59-3.49 (m, 2H), 3.46-3.37 (m, 2H), 2.44 (s, 3H), 2.06-1.97 (m, 1H), 0.85 (d, 6H).
LC/MS (Methode 3, ESIpos): $R_t$ = 1.18 min, m/z = 417 $[M+H]^+$.

**Beispiel 144**

1-(3-Fluorpropyl)-3-isobutyl-5-methyl-6-[(2-thioxoimidazolidin-1-yl)methyl]thieno[2,3-d]pyrimidin-2,4(1*H*,3*H*)-dion

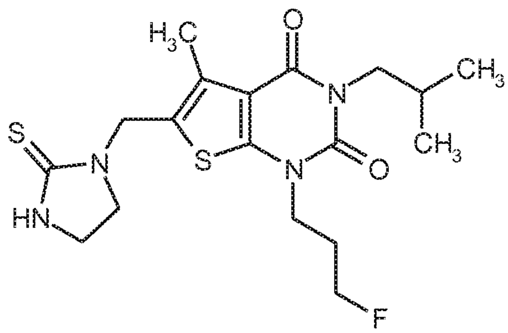

170 mg (0.266 mmol) der Verbindung aus Bsp. 241A wurden in 15 ml Dioxan gelöst und mit 75 mg (0.399 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 49 mg (44% d. Th.) der Titelverbindung erhalten.
$^1$H-NMR (400 MHz, DMSO-$d_6$, $\delta$/ppm): 8.36 (s, 1H), 4.83 (s, 2H), 4.58 (t, 1H), 4.46 (t, 1H), 3.97 (t, 2H), 3.70 (d, 2H), 3.58-3.49 (m, 2H), 3.45-3.37 (m, 2H), 2.43 (s, 3H), 2.13-1.97 (m, 3H), 0.84 (d, 6H).
LC/MS (Methode 3, ESIpos): $R_t$ = 1.17 min, m/z = 413 $[M+H]^+$.

**Beispiel 145**

1-(2-Ethoxyethyl)-3-isobutyl-5-methyl-6-[(2-thioxoimidazolidin-1-yl)methyl]thieno[2,3-d]pyrimidin-2,4(1*H*,3*H*)-dion

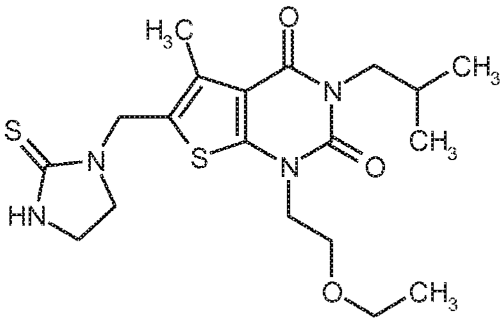

158 mg (0.343 mmol) der Verbindung aus Bsp. 314A wurden in 15 ml Dioxan gelöst und mit 97 mg (0.514 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 63 mg (43% d. Th.) der Titelverbindung erhalten.
$^1$H-NMR (400 MHz, DMSO-$d_6$, δ/ppm): 8.34 (s, 1H), 4.82 (s, 2H), 4.00 (t, 2H), 3.70 (d, 2H), 3.65 (t, 2H), 3.57-3.49 (m, 2H), 3.46-3.36 (m, 4H), 2.43 (s, 3H), 2.09-1.96 (m, 1H), 1.02 (t, 3H), 0.84 (d, 6H).
LC/MS (Methode 3, ESIpos): $R_t$ = 1.21 min, m/z = 425 $[M+H]^+$.

## Beispiel 146

3-Isobutyl-5-methyl-1-(oxetan-2-ylmethyl)-6-[(2-thioxoimidazolidin-1-yl)methyl]thieno[2,3-d]-pyrimidin-2,4(1*H*,3*H*)-dion (*Racemat*)

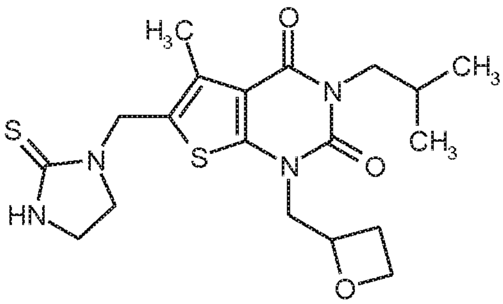

406 mg (0.918 mmol) der Verbindung aus Bsp. 243A wurden in 20 ml Dioxan gelöst und mit 258 mg (1.38 mmol) 1,1'-Thiocarbonyldiimidazol versetzt. Das Gemisch wurde 16 h bei RT gerührt. Die Reaktionslösung wurde danach am Rotationsverdampfer eingeengt. Der Rückstand wurde in 9 ml DMSO gelöst und diese Lösung mittels präparativer HPLC gereinigt (Methode 14). Nach Vereinigen der Produktfraktionen und Gefriertrocknung wurden 186 mg (47% d. Th.) der Titelverbindung erhalten.
$^1$H-NMR (400 MHz, DMSO-$d_6$, δ/ppm): 8.35 (s, 1H), 5.04-4.96 (m, 1H), 4.82 (s, 2H), 4.51-4.44 (m, 1H), 4.39 (dt, 1H), 4.17-4.08 (m, 2H), 3.71 (d, 2H), 3.58-3.49 (m, 2H), 3.45-3.36 (m, 2H), 2.68 (dtd, 1H), 2.43 (s, 2H), 2.03 (m, 1H), 0.85 (dd, 6H).
LC/MS (Methode 3, ESIpos): $R_t$ = 1.11 min, m/z = 423 $[M+H]^+$.

## Beispiel 147

3-Isobutyl-5-methyl-1-(oxetan-2-ylmethyl)-6-[(2-thioxoimidazolidin-1-yl)methyl]thieno[2,3-d]-pyrimidin-2,4(1*H*,3*H*)-dion (*Enantiomer 1*)

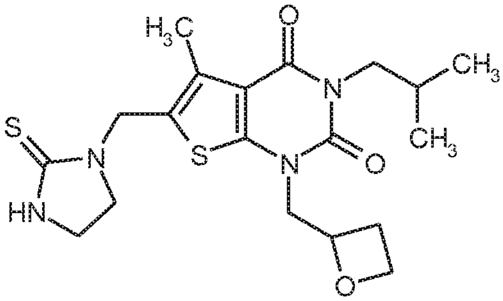

154 mg der racemischen Verbindung aus Bsp. 146 wurden in 4 ml eines Methanol/Dichlormethan-Gemisches (1:1 v/v) gelöst und mittels präparativer HPLC an chiraler Phase in die Enantiomere getrennt [Säule: Daicel Chiralpak IC, 5 μm, 250 mm x 20 mm; Eluent: Acetonitril; Fluss: 15 ml/ min; Temperatur: RT; Detektion: 232 nm]. Die Produktfraktionen wurden am Rotationsverdampfer eingeengt, mit *tert*.-Butanol versetzt und gefriergetrocknet. Es wurden 65 mg (42% d. Th.) der Titelverbindung (Enantiomer 1) sowie 65 mg (42% d. Th.) des Enantiomers 2 (siehe Beispiel 148) erhalten.

$^1$H-NMR (400 MHz, DMSO-$d_6$, δ/ppm): 8.35 (s, 1H), 5.04-4.95 (m, 1H), 4.87-4.76 (m, 2H), 4.51-4.44 (m, 1H), 4.39 (dt, 1H), 4.18-4.07 (m, 2H), 3.71 (d, 2H), 3.58-3.49 (m, 2H), 3.44-3.37 (m, 2H), 2.73-2.63 (m, 1H), 2.43 (s, 3H), 2.08-1.97 (m, 1H), 0.84 (dd, 6H).

Chirale analytische HPLC [Säule: Daicel Chiralpak IC, 3 μm, 100 mm x 4.6 mm; Eluent: Acetonitril; Fluss: 1.0 ml/min; Temperatur: 25°C; Injektion: 5 μl; DAD 254 nm]: $R_t$ = 3.22 min.

**Beispiel 148**

3-Isobutyl-5-methyl-1-(oxetan-2-ylmethyl)-6-[(2-thioxoimidazolidin-1-yl)methyl]thieno[2,3-d]-pyrimidin-2,4(1*H*,3*H*)-dion (*Enantiomer 2*)

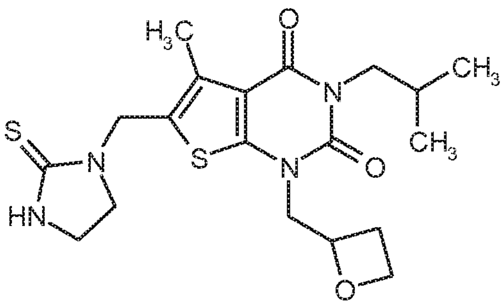

Die Titelverbindung (65 mg) wurde als zweites Enantiomer bei der unter Bsp. 147 beschriebenen präparativen HPLC-Trennung des Racemats aus Bsp. 146 erhalten.

$^1$H-NMR (400 MHz, DMSO-$d_6$, δ/ppm): 8.35 (s, 1H), 5.00 (dt, 1H), 4.86-4.77 (m, 2H), 4.51-4.44 (m, 1H), 4.39 (dt, 1H), 4.18-4.07 (m, 2H), 3.71 (d, 2H), 3.58-3.49 (m, 2H), 3.44-3.36 (m, 2H), 2.73-2.63 (m, 1H), 2.45-2.40 (m, 3H), 2.07-1.96 (m, 1H), 0.85 (dd, 6H).

Chirale analytische HPLC [Säule: Daicel Chiralpak IC, 3 μm, 100 mm x 4.6 mm; Eluent: Acetonitril; Fluss: 1.0 ml/min; Temperatur: 25°C; Injektion: 5 μl; DAD 254 nm]: $R_t$ = 3.97 min.

**Beispiel 149**

3-(2,2-Dimethylpropyl)-5-methyl-6-[(2-thioxoimidazolidin-1-yl)methyl]-1-(3,3,3-trifluorpropyl)-thieno[2,3-d]pyrimidin-2,4(1*H*,3*H*)-dion

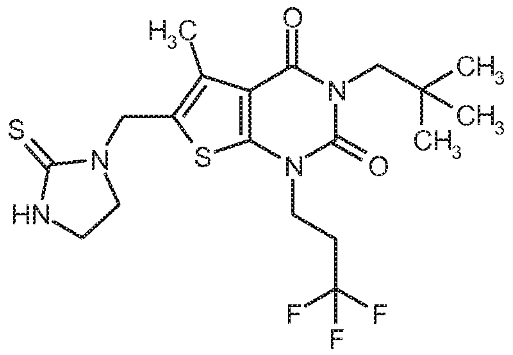

160 mg (0.316 mmol) der Verbindung aus Bsp. 315A wurden in 15 ml Dioxan gelöst und mit 89 mg (0.474 mmol) 1,1'-Thiocarbonyldiimidazol versetzt. Das Gemisch wurde 21 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 46 mg (31% d. Th.) der Titelverbindung erhalten.
$^1$H-NMR (400 MHz, DMSO-$d_6$, δ/ppm): 8.37 (s, 1H), 4.84 (s, 2H), 4.09 (t, 2H), 3.81 (br. s, 2H), 3.60-3.51 (m, 2H), 3.45-3.36 (m, 2H), 2.82-2.69 (m, 2H), 2.43 (s, 3H), 0.89 (s, 9H).
LC/MS (Methode 3, ESIpos): $R_t$ = 1.35 min, m/z = 463 $[M+H]^+$.

**Beispiel 150**

3-(2,2-Dimethylpropyl)-1-(3-fluorpropyl)-5-methyl-6-[(2-thioxoimidazolidin-1-yl)methyl]thieno-[2,3-d]pyrimidin-2,4(1*H*,3*H*)-dion

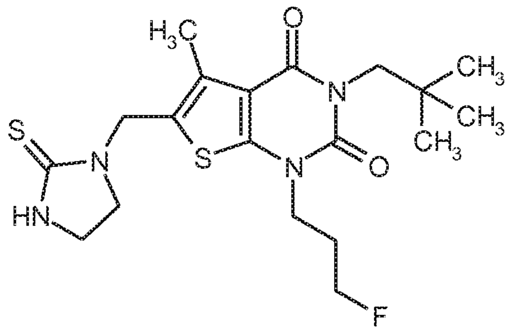

103 mg (0.22 mmol) der Verbindung aus Bsp. 316A wurden in 10 ml Dioxan gelöst und mit 62 mg (0.329 mmol) 1,1'-Thiocarbonyldiimidazol versetzt. Das Gemisch wurde 19 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 66 mg (70% d. Th.) der Titelverbindung erhalten.
$^1$H-NMR (400 MHz, DMSO-$d_6$, δ/ppm): 8.35 (s, 1H), 4.83 (s, 2H), 4.58 (t, 1H), 4.46 (t, 1H), 3.97 (t, 2H), 3.80 (br. s, 2H), 3.60-3.51 (m, 2H), 3.45-3.37 (m, 2H), 2.43 (s, 3H), 2.13-2.05 (m, 1H), 2.05-1.97 (m, 1H), 0.88 (s, 9H).
LC/MS (Methode 3, ESIpos): $R_t$ = 1.25 min, m/z = 427 $[M+H]^+$.

**Beispiel 151**

3-(2,2-Dimethylpropyl)-1-(2-methoxyethyl)-5-methyl-6-[(2-thioxoimidazolidin-1-yl)methyl]-thieno[2,3-d]pyrimidin-2,4(1*H*,3*H*)-dion

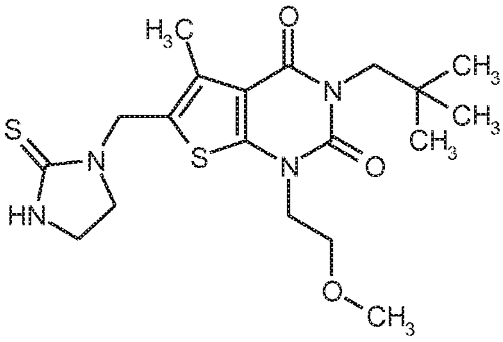

172 mg (0.364 mmol) der Verbindung aus Bsp. 317A wurden in 15 ml Dioxan gelöst und mit 102 mg (0.546 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 84 mg (54% d. Th.) der Titelverbindung erhalten.
$^1$H-NMR (400 MHz, DMSO-$d_6$, δ/ppm): 8.36 (s, 1H), 4.82 (s, 2H), 4.01 (t, 2H), 3.81 (br. s, 2H), 3.62 (t, 2H), 3.59-3.50 (m, 2H), 3.46-3.36 (m, 2H), 3.22 (s, 3H), 2.42 (s, 3H), 0.89 (s, 9H).
LC/MS (Methode 3, ESIpos): $R_t$ = 1.22 min, m/z = 425 $[M+H]^+$.

**Beispiel 152**

5-Methyl-6-[(2-thioxoimidazolidin-1-yl)methyl]-3-(2,2,2-trifluorethyl)-1-(3,3,3-trifluorpropyl)-thieno[2,3-d]pyrimidin-2,4(1*H*,3*H*)-dion

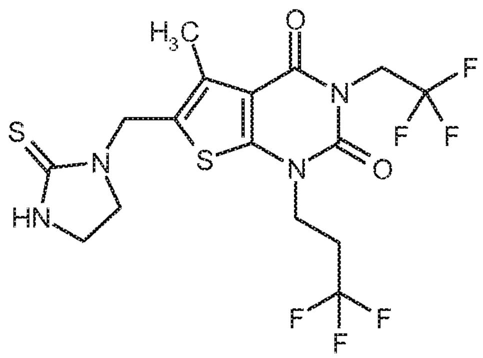

200 mg (0.43 mmol) der Verbindung aus Bsp. 247A wurden in 15 ml Dioxan gelöst und mit 121 mg (0.645 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14).

Nach Vereinigen der Produktfraktionen und Gefriertrocknung wurden 103 mg (50% d. Th.) der Titelverbindung erhalten.
$^1$H-NMR (400 MHz, DMSO-$d_6$, δ/ppm): 8.40 (s, 1H), 4.86 (s, 2H), 4.70 (q, 2H), 4.13 (t, 2H), 3.60-3.51 (m, 2H), 3.46-3.37 (m, 2H), 2.84-2.70 (m, 2H), 2.45 (s, 3H).
LC/MS (Methode 3, ESIpos): $R_t$ = 1.19 min, m/z = 475 $[M+H]^+$.

**Beispiel 153**

1-(2-Ethoxyethyl)-5-methyl-6-[(2-thioxoimidazolidin-1-yl)methyl]-3-(2,2,2-trifluorethyl)thieno-[2,3-d]pyrimidin-2,4(1*H*,3*H*)-dion

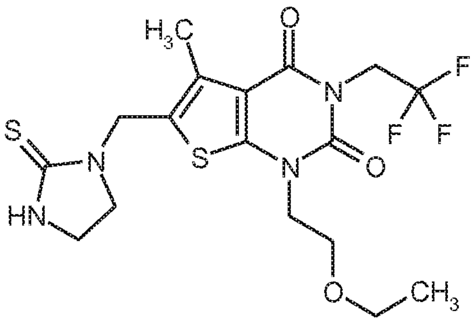

113 mg (0.177 mmol) der Verbindung aus Bsp. 250A wurden in 10 ml Dioxan gelöst und mit 50 mg (0.266 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 63 mg (78% d. Th.) der Titelverbindung erhalten.
$^1$H-NMR (400 MHz, DMSO-$d_6$, δ/ppm): 8.38 (s, 1H), 4.84 (s, 2H), 4.69 (q, 2H), 4.04 (t, 2H), 3.66 (t, 2H), 3.57-3.50 (m, 2H), 3.46-3.37 (m, 4H), 2.43 (s, 3H), 1.02 (t, 3H).
LC/MS (Methode 3, ESIpos): $R_t$ = 1.13 min, m/z = 451 [M+H]$^+$.

**Beispiel 154**

5-Methyl-1-(oxetan-2-ylmethyl)-6-[(2-thioxoimida-zolidin-1-yl)methyl]-3-(2,2,2-trifluorethyl)-thieno[2,3-d]pyrimidin-2,4(1*H*,3*H*)-dion (*Racemat*)

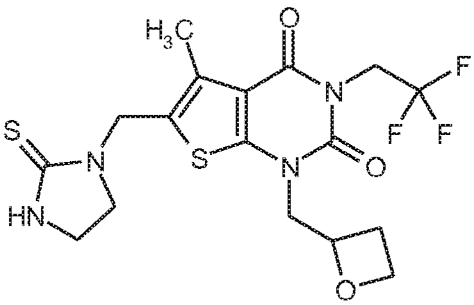

260 mg (0.563 mmol) der Verbindung aus Bsp. 251A wurden in 13 ml Dioxan gelöst und mit 158 mg (0.844 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 116 mg (45% d. Th.) der Titelverbindung erhalten.
$^1$H-NMR (400 MHz, DMSO-$d_6$, δ/ppm): 8.37 (s, 1H), 5.06-4.97 (m, 1H), 4.84 (s, 2H), 4.70 (q, 2H), 4.52-4.44 (m, 1H), 4.40 (dt, 1H), 4.22-4.10 (m, 2H), 3.59-3.49 (m, 2H), 3.44-3.37 (m, 2H), 2.73-2.64 (m, 1H), 2.43 (s, 3H).
LC/MS (Methode 3, ESIpos): $R_t$ = 1.04 min, m/z = 449 [M+H]$^+$.

**Beispiel 155**

5-Methyl-1-(oxetan-2-ylmethyl)-6-[(2-thioxoimidazolidin-1-yl)methyl]-3-(2,2,2-trifluorethyl)-thieno[2,3-d]pyrimidin-2,4(1*H*,3*H*)-dion (*Enantiomer 1*)

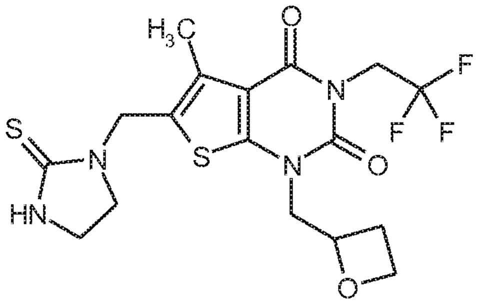

106 mg der racemischen Verbindung aus Bsp. 154 wurden in 6 ml eines Methanol/Dichlormethan-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent: Methanol/Ethanol/Diethylamin 50:50:0.1 v/v; Fluss: 20 ml/min; Temperatur: RT; UV-Detektion: 254 nm]. Die Produktfraktionen wurden am Rotationsverdampfer eingeengt, mit tert.-Butanol versetzt und gefriergetrocknet.

Es wurden 45 mg (42% d. Th.) der Titelverbindung (Enantiomer 1) sowie 49 mg (45% d. Th.) des Enantiomers 2 (siehe Beispiel 156) erhalten.

$^1$H-NMR (400 MHz, DMSO-$d_6$, δ/ppm): 8.37 (s, 1H), 5.01 (dt, 1H), 4.88-4.79 (m, 2H), 4.70 (q, 2H), 4.52-4.44 (m, 1H), 4.40 (dt, 1H), 4.23-4.10 (m, 2H), 3.59-3.49 (m, 2H), 3.44-3.37 (m, 2H), 2.74-2.64 (m, 1H), 2.43 (s, 3H).

Chirale analytische HPLC [Säule: Daicel Chiralpak IC, 5 μm, 100 mm x 4.6 mm; Eluent: Methanol/Ethanol/Diethylamin 50:50:0.1 v/v; Fluss: 1.0 ml/min; Temperatur: RT; Injektion: 5 μl; DAD 254 nm]: $R_t$ = 3.76 min.

## Beispiel 156

5-Methyl-1-(oxetan-2-yhnethyl)-6-[(2-thioxoimidazolidin-1-yl)methyl]-3-(2,2,2-trifluorethyl)-thieno[2,3-d]pyrimidin-2,4(1*H*,3*H*)-dion (*Enantiomer 2*)

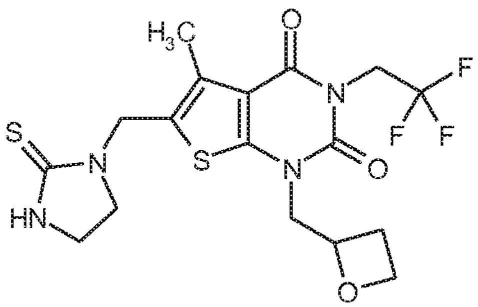

Die Titelverbindung (49 mg) wurde als zweites Enantiomer bei der unter Bsp. 155 beschriebenen präparativen HPLC-Trennung des Racemats aus Bsp. 154 erhalten.

$^1$H-NMR (400 MHz, DMSO-$d_6$, δ/ppm): 8.37 (s, 1H), 5.06-4.96 (m, 1H), 4.83 (s, 2H), 4.70 (q, 2H), 4.52-4.44 (m, 1H), 4.40 (dt, 1H), 4.23-4.09 (m, 2H), 3.59-3.49 (m, 2H), 3.44-3.37 (m, 2H), 2.74-2.64 (m, 1H), 2.43 (s, 3H).

Chirale analytische HPLC [Säule: Daicel Chiralpak IC, 5 μm, 100 mm x 4.6 mm; Eluent: Methanol/Ethanol/Diethylamin 50:50:0.1 v/v; Fluss: 1.0 ml/min; Temperatur: RT; Injektion: 5 μl; DAD 254 nm]: $R_t$ = 4.35 min.

## Beispiel 157

5-Methyl-1-(tetrahydrofuran-2-ylmethyl)-6-[(2-thioxoimidazolidin-1-yl)methyl]-3-(2,2,2-trifluorethyl)thieno[2,3-d]pyrimidin-2,4(1*H*,3*H*)-dion (*Racemat*)

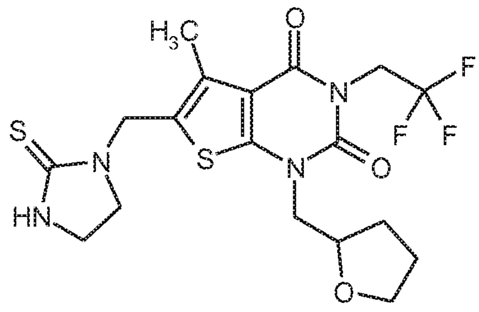

285 mg (0.549 mmol) der Verbindung aus Bsp. 252A wurden in 15 ml Dioxan gelöst und mit 154 mg (0.824 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 9 ml DMSO gelöst und diese Lösung mittels präparativer HPLC gereinigt (Methode 14). Nach Vereinigen der Produktfraktionen und Gefriertrocknung wurden 180 mg (70% d. Th.) der Titelverbindung erhalten.
$^1$H-NMR (400 MHz, DMSO-$d_6$, $\delta$/ppm): 8.37 (s, 1H), 4.83 (s, 2H), 4.70 (q, 2H), 4.26-4.18 (m, 1H), 4.05 (dd, 1H), 3.79-3.68 (m, 2H), 3.64-3.58 (m, 1H), 3.58-3.51 (m, 2H), 3.44-3.37 (m, 2H), 2.43 (s, 3H), 2.03-1.93 (m, 1H), 1.93-1.76 (m, 2H), 1.71-1.62 (m, 1H).
LC/MS (Methode 3, ESIpos): $R_t$ = 1.12 min, m/z = 463 [M+H]$^+$.

**Beispiel 158**

5-Methyl-1-(oxetan-3-ylmethyl)-6-[(2-thioxoimidazolidin-1-yl)methyl]-3-(2,2,2-trifluorethyl)-thieno[2,3-d]pyrimidin-2,4(1*H*,3*H*)-dion

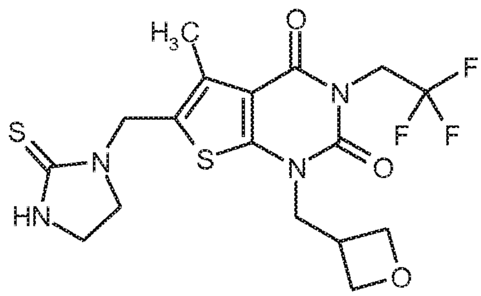

109 mg (0.091 mmol) der Verbindung aus Bsp. 253A wurden in 10 ml Dioxan gelöst und mit 26 mg (0.137 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 72 mg (76% d. Th.) der Titelverbindung erhalten.
$^1$H-NMR (400 MHz, DMSO-$d_6$, $\delta$/ppm): 8.39 (s, 1H), 4.85 (s, 2H), 4.68 (q, 2H), 4.61 (dd, 2H), 4.43 (t, 2H), 4.23 (d, 2H), 3.58-3.51 (m, 2H), 3.46-3.37 (m, 3H), 2.43 (s, 3H).
LC/MS (Methode 3, ESIpos): $R_t$ = 1.03 min, m/z = 449 [M+H]$^+$.

**Beispiel 159**

1,5-Dimethyl-6-[(2-thioxoimidazolidin-1-yl)methyl]-3-(2,2,2-trifluorethyl)thieno[2,3-d]pyrimidin-2,4(1*H*,3*H*)-dion

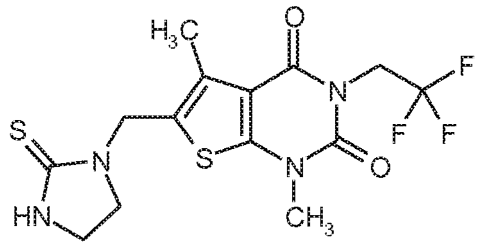

190 mg (0.239 mmol) der Verbindung aus Bsp. 254A wurden in 15 ml Dioxan gelöst und mit 67 mg (0.358 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 48 mg (51% d. Th.) der Titelverbindung erhalten.
$^1$H-NMR (400 MHz, DMSO-$d_6$, $\delta$/ppm): 8.38 (s, 1H), 4.85 (s, 2H), 4.69 (q, 2H), 3.58-3.50 (m, 2H), 3.45-3.38 (m, 5H), 2.44 (s, 3H).
LC/MS (Methode 3, ESIpos): $R_t$ = 1.03 min, m/z = 393 $[M+H]^+$.

**Beispiel 160**

3-(2,2-Difluorethyl)-5-methyl-6-[(2-thioxoimidazolidin-1-yl)methyl]-1-(3,3,3-trifluorpropyl)-thieno[2,3-d]pyrimidin-2,4(1*H*,3*H*)-dion

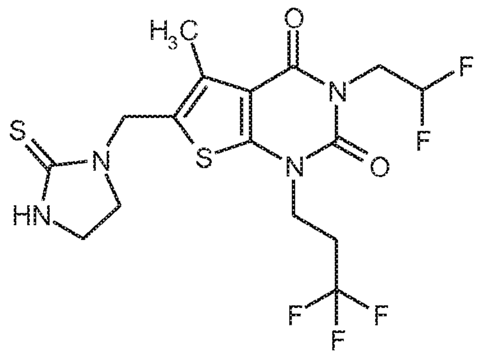

150 mg (0.257 mmol) der Verbindung aus Bsp. 255A wurden in 15 ml Dioxan gelöst und mit 72 mg (0.385 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 53 mg (45% d. Th.) der Titelverbindung erhalten.
$^1$H-NMR (400 MHz, DMSO-$d_6$, $\delta$/ppm): 8.39 (s, 1H), 6.37-6.04 (m, 1H), 4.86 (s, 2H), 4.29 (td, 2H), 4.11 (t, 2H), 3.59-3.49 (m, 2H), 3.45-3.38 (m, 2H), 2.84-2.70 (m, 2H), 2.45 (s, 3H).
LC/MS (Methode 3, ESIpos): $R_t$ = 1.13 min, m/z = 457 $[M+H]^+$.

**Beispiel 161**

3-(2,2-Difluorethyl)-1-(2-ethoxyethyl)-5-methyl-6-[(2-thioxoimidazolidin-1-yl)methyl]thieno-[2,3-d]pyrimidin-2,4(1*H*,3*H*)-dion

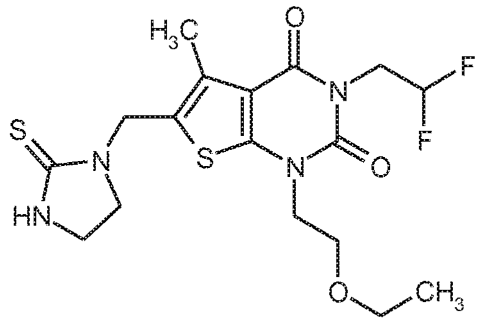

120 mg (0.206 mmol) der Verbindung aus Bsp. 258A wurden in 10 ml Dioxan gelöst und mit 58 mg (0.309 mmol) 1,1'-Thiocarbonyldiiimidazol versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14).

Nach Vereinigen der Produktfraktionen und Gefriertrocknung wurden 61 mg (68% d. Th.) der Titelverbindung erhalten.

$^1$H-NMR (400 MHz, DMSO-$d_6$, δ/ppm): 8.38 (s, 1H), 6.38-6.05 (m, 1H), 4.84 (s, 2H), 4.29 (td, 2H), 4.02 (t, 2H), 3.66 (t, 2H), 3.57-3.48 (m, 2H), 3.47-3.36 (m, 4H), 2.43 (s, 3H), 1.03 (t, 3H).

LC/MS (Methode 3, ESIpos): $R_t$ = 1.09 min, m/z = 433 $[M+H]^+$.

**Beispiel 162**

3-(2,2-Difluorethyl)-5-methyl-1-(tetrahydrofuran-2-ylmethyl)-6-[(2-thioxoimidazolidin-1-yl)-methyl]thieno[2,3-d]pyrimidin-2,4(1*H*,3*H*)-dion (*Racemat*)

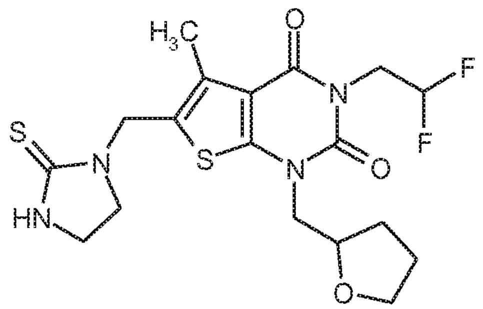

222 mg (0.348 mmol) der Verbindung aus Bsp. 260A wurden in 8 ml Dioxan gelöst und mit 98 mg (0.521 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 75 mg (48% d. Th.) der Titelverbindung erhalten.

$^1$H-NMR (400 MHz, DMSO-$d_6$, δ/ppm): 8.37 (s, 1H), 6.37-6.06 (m, 1H), 4.83 (s, 2H), 4.38-4.18 (m, 3H), 4.04 (dd, 1H), 3.78-3.67 (m, 2H), 3.64-3.57 (m, 1H), 3.57-3.49 (m, 2H), 3.44-3.37 (m, 2H), 2.43 (s, 3H), 2.03-1.93 (m, 1H), 1.93-1.76 (m, 2H), 1.72-1.62 (m, 1H).

LC/MS (Methode 3, ESIpos): $R_t$ = 1.05 min, m/z = 445 $[M+H]^+$.

**Beispiel 163**

3-(2,2-Difluorethyl)-5-methyl-1-(tetrahydroferan-2-ylmethyl)-6-[(2-thioxoimidazolidin-1-yl)-methyl]thieno[2,3-d]pyrimidin-2,4(1*H*,3*H*)-dion (*Enantiomer 1*)

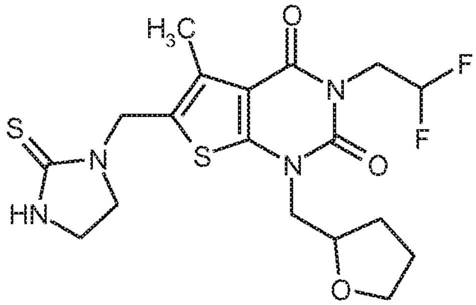

66 mg der racemischen Verbindung aus Bsp. 162 wurden in 3.4 ml eines Methanol/Dichlormethan-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent: Methanol/Ethanol/Diethylamin 50:50:0.1 v/v; Fluss: 30 ml/min; Temperatur: RT; UV-Detektion: 254 nm]. Die Produktfraktionen wurden am Rotationsverdampfer eingeengt, mit *tert.*-Butanol versetzt und gefriergetrocknet. Es wurden 26 mg (38% d. Th.) der Titelverbindung (Enantiomer 1) sowie 28 mg (41% d. Th.) des Enantiomers 2 (siehe Beispiel 164) erhalten.
$^1$H-NMR (400 MHz, DMSO-$d_6$, δ/ppm): 8.37 (s, 1H), 6.38-6.06 (m, 1H), 4.89-4.78 (m, 2H), 4.30 (td, 2H), 4.25-4.18 (m, 1H), 4.04 (dd, 1H), 3.78-3.67 (m, 2H), 3.65-3.57 (m, 1H), 3.57-3.49 (m, 2H), 3.44-3.37 (m, 2H), 2.43 (s, 3H), 2.03-1.93 (m, 1H), 1.93-1.75 (m, 2H), 1.72-1.62 (m, 1H).

Chirale analytische HPLC [Säule: Daicel Chiralpak IC, 5 μm, 100 mm x 4.6 mm; Eluent: Methanol/Ethanol/Diethylamin 50:50:0.1 v/v; Fluss: 1.0 ml/min; Temperatur: RT; Injektion: 5 μl; DAD 254 nm]: $R_t$ = 4.43 min.

## Beispiel 164

3-(2,2-Difluorethyl)-5-methyl-1-(tetrahydrofuran-2-ylmethyl)-6-[(2-thioxoimidazolidin-1-yl)-methyl]thieno[2,3-d]pyrimidin-2,4(1*H*,3*H*)-dion (*Enantiomer 2*)

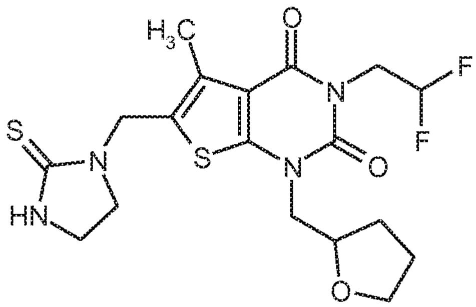

Die Titelverbindung (28 mg) wurde als zweites Enantiomer bei der unter Bsp. 163 beschriebenen präparativen HPLC-Trennung des Racemats aus Bsp. 162 erhalten.
$^1$H-NMR (400 MHz, DMSO-$d_6$, δ/ppm): 8.37 (s, 1H), 6.38-6.06 (m, 1H), 4.89-4.78 (m, 2H), 4.30 (td, 2H), 4.25-4.18 (m, 1H), 4.04 (dd, 1H), 3.78-3.67 (m, 2H), 3.65-3.57 (m, 1H), 3.57-3.49 (m, 2H), 3.44-3.37 (m, 2H), 2.43 (s, 3H), 2.03-1.93 (m, 1H), 1.93-1.75 (m, 2H), 1.67 (ddt, 1H).

Chirale analytische HPLC [Säule: Daicel Chiralpak IC, 5 μm, 100 mm x 4.6 mm; Eluent: Methanol/Ethanol/Diethylamin 50:50:0.1 v/v; Fluss: 1.0 ml/min; Temperatur: RT; Injektion: 5 μl; DAD 254 nm]: $R_t$ = 5.22 min.

## Beispiel 165

3-(2,2-Difluorethyl)-5-methyl-1-(oxetan-3-ylmethyl)-6-[(2-thioxoimidazolidin-1-yl)methyl]thieno-[2,3-d]pyrimidin-2,4(1*H*,3*H*)-dion

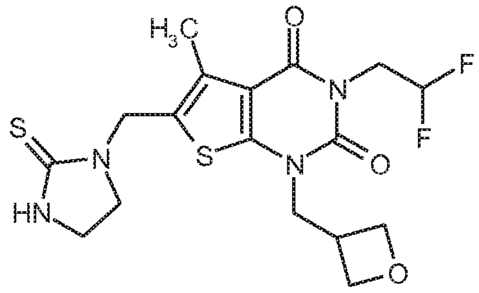

85 mg (0.147 mmol) der Verbindung aus Bsp. 261A wurden in 10 ml Dioxan gelöst und mit 41 mg (0.22 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 47 mg (72% d. Th.) der Titelverbindung erhalten.
$^1$H-NMR (400 MHz, DMSO-$d_6$, $\delta$/ppm): 8.38 (s, 1H), 6.35-6.03 (m, 1H), 4.84 (s, 2H), 4.62 (dd, 2H), 4.43 (t, 2H), 4.28 (td, 2H), 4.21 (d, 2H), 3.57-3.49 (m, 2H), 3.46-3.37 (m, 3H), 2.43 (s, 3H).
LC/MS (Methode 3, ESIpos): $R_t$ = 0.94 min, m/z = 431 $[M+H]^+$.

## Beispiel 166

3-(2,2-Difluorethyl)-1,5-dimethyl-6-[(2-thioxoimidazolidin-1-yl)methyl]thieno[2,3-d]pyrimidin-2,4(1*H*,3*H*)-dion

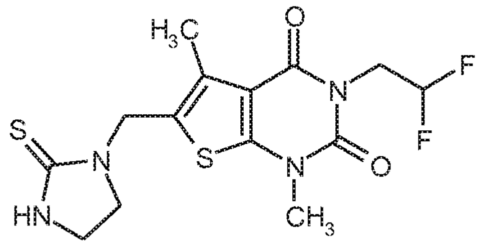

110 mg (0.232 mmol) der Verbindung aus Bsp. 318A wurden in 10.1 ml Dioxan gelöst und mit 65 mg (0.347 mmol) 1,1'-Thiocarbonyldiimidazol versetzt. Das Gemisch wurde 13 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 40 mg (45% d. Th.) der Titelverbindung erhalten.
$^1$H-NMR (400 MHz, DMSO-$d_6$, $\delta$/ppm): 8.38 (s, 1H), 6.37-6.04 (m, 1H), 4.85 (s, 2H), 4.29 (td, 2H), 3.56-3.49 (m, 2H), 3.44-3.37 (m, 5H), 2.44 (s, 3H).
LC/MS (Methode 3, ESIpos): $R_t$ = 0.96 min, m/z = 375 $[M+H]^+$.

## Beispiel 167

3-(2-Methoxyethyl)-5-methyl-6-[(2-thioxoimidazolidin-1-yl)methyl]-1-(3,3,3-trifluorpropyl)thieno-[2,3-d]pyrimidin-2,4(1*H*,3*H*)-dion

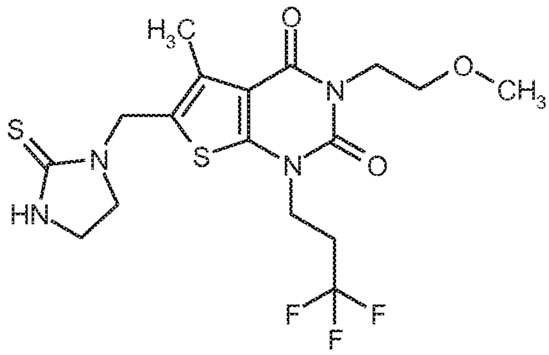

100 mg (0.198 mmol) der Verbindung aus Bsp. 319A wurden in 15 ml Dioxan gelöst und mit 56 mg (0.347 mmol) 1,1'-Thiocarbonyldiimidazol versetzt. Das Gemisch wurde 18 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 56 mg (62% d. Th.) der Titelverbindung erhalten.
$^1$H-NMR (400 MHz, DMSO-$d_6$, δ/ppm): 8.36 (s, 1H), 4.85 (s, 2H), 4.09 (t, 2H), 4.05 (t, 2H), 3.58-3.46 (m, 4H), 3.45-3.37 (m, 2H), 3.24 (s, 3H), 2.82-2.69 (m, 2H), 2.44 (s, 3H).
LC/MS (Methode 3, ESIpos): $R_t$ = 1.05 min, m/z = 451 $[M+H]^+$.

**Beispiel 168**

1,3-Bis(2-methoxyethyl)-5-methyl-6-[(2-thioxoimidazolidin-1-yl)methyl]thieno[2,3-d]pyrimidin-2,4(1*H*,3*H*)-dion

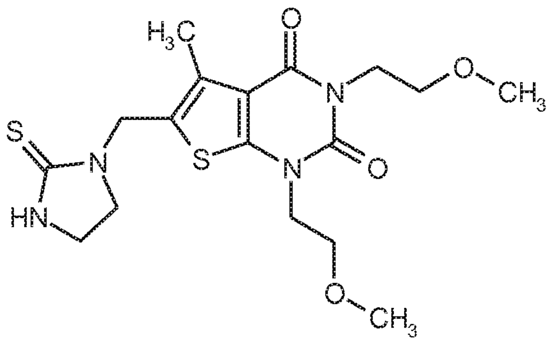

155 mg (0.18 mmol) der Verbindung aus Bsp. 262A wurden in 5 ml Dioxan gelöst und mit 51 mg (0.27 mmol) 1,1'-Thiocarbonyldiimidazol versetzt. Das Gemisch wurde 16 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 42 mg (56% d. Th.) der Titelverbindung erhalten.
$^1$H-NMR (400 MHz, DMSO-$d_6$, δ/ppm): 8.36 (s, 1H), 4.82 (s, 2H), 4.05 (t, 2H), 4.01 (t, 2H), 3.62 (t, 2H), 3.57-3.47 (m, 4H), 3.44-3.37 (m, 2H), 3.24 (s, 3H), 3.23 (s, 3H), 2.43 (s, 3H).
LC/MS (Methode 3, ESIpos): $R_t$ = 0.91 min, m/z = 413 $[M+H]^-$.

**Beispiel 169**

1-(2-Ethoxyethyl)-3-(2-methoxyethyl)-5-methyl-6-[(2-thioxoimidazolidin-1-yl)methyl]thieno-[2,3-d]pyrimidin-2,4(1*H*,3*H*)-dion

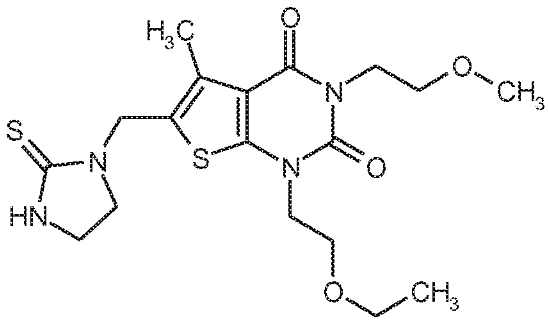

143 mg (0.216 mmol) der Verbindung aus Bsp. 263A wurden in 10 ml Dioxan gelöst und mit 61 mg (0.324 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 68 mg (73% d. Th.) der Titelverbindung erhalten.

$^1$H-NMR (400 MHz, DMSO-$d_6$, $\delta$/ppm): 8.36 (s, 1H), 4.83 (s, 2H), 4.05 (t, 2H), 4.00 (t, 2H), 3.65 (t, 2H), 3.56-3.47 (m, 4H), 3.46-3.36 (m, 4H), 3.24 (s, 3H), 2.43 (s, 3H), 1.03 (t, 3H).

LC/MS (Methode 3, ESIpos): $R_t$ = 0.98 min, m/z = 427 $[M+H]^+$.

## Beispiel 170

3-(2-Methoxy-2-methylpropyl)-5-methyl-6-[(2-thioxoimidazolidin-1-yl)methyl]-1-(3,3,3-trifluorpropyl)thieno[2,3-d]pyrimidin-2,4(1*H*,3*H*)-dion

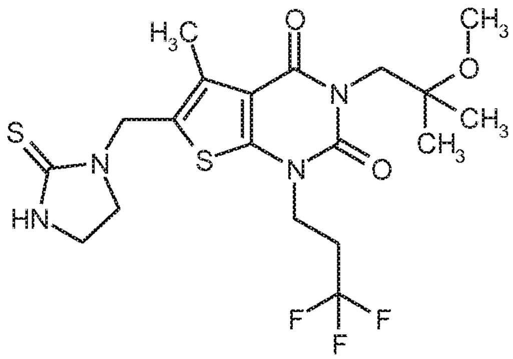

160 mg (0.297 mmol) der Verbindung aus Bsp. 322A wurden in 15 ml Dioxan gelöst und mit 84 mg (0.445 mmol) 1,1'-Thiocarbonyldiimidazol versetzt. Das Gemisch wurde 19 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14).

Nach Vereinigen der Produktfraktionen und Gefriertrocknung wurden 66 mg (46% d. Th.) der Titelverbindung erhalten.

$^1$H-NMR (400 MHz, DMSO-$d_6$, $\delta$/ppm): 8.37 (s, 1H), 4.84 (s, 2H), 4.09 (t, 2H), 4.00 (br. s, 2H), 3.59-3.51 (m, 2H), 3.45-3.37 (m, 2H), 3.15 (s, 3H), 2.82-2.68 (m, 2H), 2.43 (s, 3H), 1.08 (s, 6H).

LC/MS (Methode 3, ESIpos): $R_t$ = 1.17 min, m/z = 479 $[M+H]^+$.

## Beispiel 171

1-(3-Fluorpropyl)-3-(2-methoxy-2-methylpropyl)-5-methyl-6-[(2-thioxoimidazolidin-1-yl)methyl]-thieno[2,3-d]pyrimidin-2,4(1*H*,3*H*)-dion

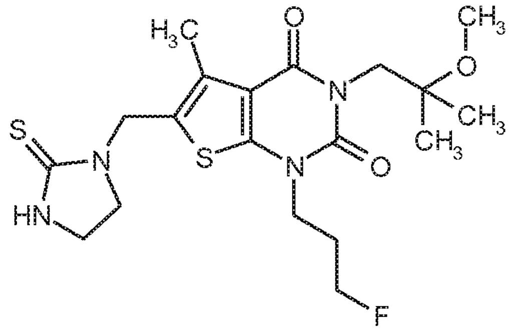

200 mg (0.424 mmol) der Verbindung aus Bsp. 323A wurden in 20 ml Dioxan gelöst und mit 119 mg (0.637 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 90 mg (47% d. Th.) der Titelverbindung erhalten.
$^1$H-NMR (400 MHz, DMSO-$d_6$, $\delta$/ppm): 8.36 (s, 1H), 4.84 (s, 2H), 4.58 (t, 1H), 4.46 (t, 1H), 4.03-3.93 (m, 4H), 3.59-3.51 (m, 2H), 3.44-3.37 (m, 2H), 3.15 (s, 3H), 2.43 (s, 3H), 2.12-2.05 (m, 2H), 2.01 (quin, 1H), 1.08 (s, 6H).
LC/MS (Methode 3, ESIpos): $R_t$ = 1.07 min, m/z = 443 $[M+H]^+$.

## Beispiel 172

1-(2-Methoxyethyl)-3-(2-methoxy-2-methylpropyl)-5-methyl-6-[(2-thioxoimidazolidin-1-yl)-methyl]thieno[2,3-d]pyrimidin-2,4(1*H*,3*H*)-dion

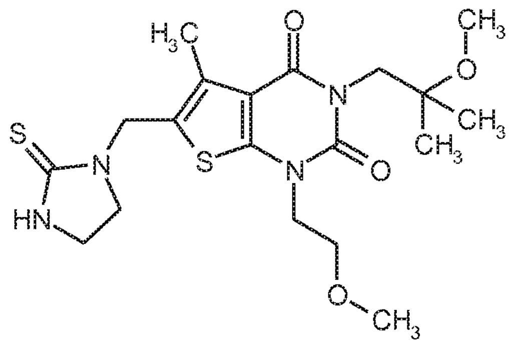

100 mg (0.196 mmol) der Verbindung aus Bsp. 324A wurden in 15 ml Dioxan gelöst und mit 55 mg (0.324 mmol) 1,1'-Thiocarbonyldiimidazol versetzt. Das Gemisch wurde 20 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 53 mg (61% d. Th.) der Titelverbindung erhalten.
$^1$H-NMR (400 MHz, DMSO-$d_6$, $\delta$/ppm): 8.35 (s, 1H), 4.82 (s, 2H), 4.06-3.94 (m, 4H), 3.61 (t, 2H), 3.58-3.50 (m, 2H), 3.44-3.37 (m, 2H), 3.22 (s, 3H), 3.15 (s, 3H), 2.42 (s, 3H), 1.08 (s, 6H).
LC/MS (Methode 3, ESIpos): $R_t$ = 1.03 min, m/z = 441 $[M+H]^+$.

## Beispiel 173

1-(3-Fluorpropyl)-5-methyl-6-[(3-methyl-2-thioxoimidazolidin-1-yl)methyl]-3-(2,2,2-trifluorethyl)-thieno[2,3-d]pyrimidin-2,4(1*H*,3*H*)-dion

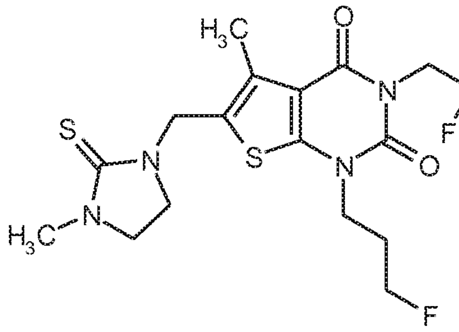

Eine Lösung von 40 mg (0.082 mmol) der Verbindung aus Bsp. 40 in 2 ml DMF wurde mit 80 mg (0.246 mmol) Cäsiumcarbonat versetzt und 10 min gerührt. Anschließend wurden 29 mg (0.205 mmol) Iodmethan hinzugefügt und das Gemisch 66 h bei RT gerührt. Danach wurde das Reaktionsgemisch zwischen Ethylacetat (50 ml) und Wasser (50 ml) verteilt. Die wässrige Phase wurde mit Ethylacetat extrahiert. Die vereinigten organischen Phasen wurden über Natriumsulfat getrocknet, filtriert und eingeengt. Der erhaltene Rückstand wurde mittels präparativer HPLC gereinigt (Methode 14). Nach Vereinigen der Produktfraktionen und Gefriertrocknung wurden 20 mg (53% d. Th.) der Titelverbindung erhalten.

$^1$H-NMR (400 MHz, DMSO-$d_6$, δ/ppm): 4.69 (q, 2H), 4.59 (t, 1H), 4.47 (t, 1H), 4.40 (s, 2H), 4.02 (t, 2H), 3.64-3.58 (m, 2H), 3.33-3.26 (m, 2H), 2.44 (s, 3H), 2.39 (s, 3H), 2.15-2.07 (m, 1H), 2.07-2.00 (m, 1H).

LC/MS (Methode 3, ESIpos): $R_t$ = 0.8 min, m/z = 453 [M+H]$^+$.

**Beispiel 174**

3-Ethyl-6-[(3-isopropyl-2-thioxoimidazolidin-1-yl)methyl]-1-(2-methoxyethyl)-5-methylthieno-[2,3-d]pyrimidin-2,4(1*H*,3*H*)-dion

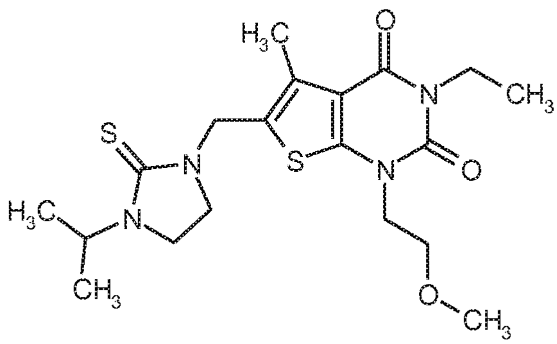

Die Titelverbindung (56 mg) wurde als Nebenprodukt der Herstellung und Aufreinigung der in Beispiel 31 beschriebenen Verbindung erhalten.

$^1$H-NMR (400 MHz, DMSO-$d_6$, δ/ppm): 4.87 (s, 1H), 4.61 (sept, 1H), 4.00 (t, 2H), 3.89 (q, 2H), 3.62 (t, 2H), 3.50-3.40 (m, 4H), 3.22 (s, 3H), 2.44 (s, 3H), 1.14-1.06 (m, 9H).

LC/MS (Methode 3, ESIpos): $R_t$ = 1.24 min, m/z = 425 [M+H]$^+$.

**Beispiel 175**

3-Ethyl-6-[(3-isopropyl-2-thioxoimidazolidin-1-yl)methyl]-5-methyl-1-(tetrahydrofuran-2-ylmethyl)thieno[2,3-d]pyrimidin-2,4(1*H*,3*H*)-dion (*Racemat*)

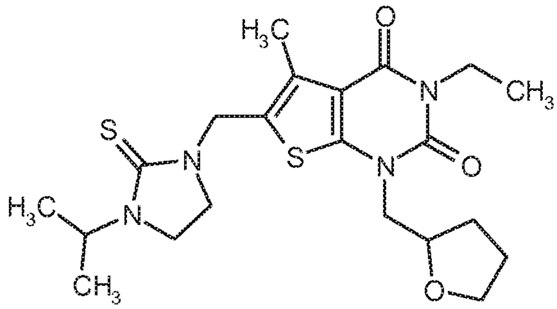

Die Titelverbindung (28 mg) wurde als Nebenprodukt der Herstellung und Aufreinigung der in Beispiel 35 beschriebenen Verbindung erhalten.
$^1$H-NMR (400 MHz, DMSO-$d_6$, δ/ppm): 4.92-4.81 (m, 2H), 4.66-4.54 (m, 1H), 4.25-4.17 (m, 1H), 4.02 (dd, 1H), 3.90 (q, 2H), 3.77-3.56 (m, 4H), 3.45 (br. s, 3H), 2.44 (s, 3H), 2.02-1.75 (m, 4H), 1.70-1.61 (m, 1H), 1.14-1.06 (m, 9H).
LC/MS (Methode 3, ESIpos): $R_t$ = 1.28 min, m/z = 451 $[M+H]^+$.

**Beispiel 176**

1-(2-Methoxyethyl)-5-methyl-6-[(2-oxoimidazolidin-1-yl)methyl]-3-(2-phenylethyl)thieno[2,3-d]-pyrimidin-2,4(1*H*,3*H*)-dion

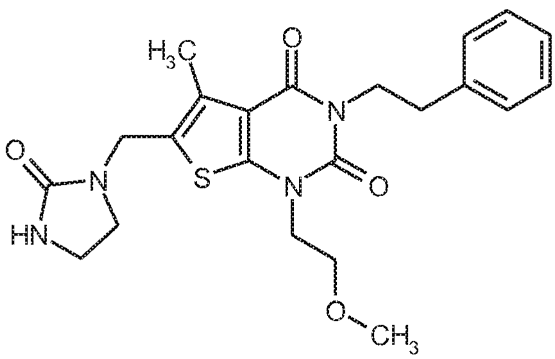

Eine Lösung von 215 mg (2.40 mmol) 2-Imidazolidinon in 9 ml THF wurde mit 96 mg (2.40 mmol) Natriumhydrid (60% Suspension in Mineralöl) versetzt und 3 h bei 60°C gerührt ("Lösung 1"). In einem anderen Reaktionsgefäß wurde eine Lösung von 225 mg (0.601 mmol) der Verbindung aus Bsp. 138A in 4 ml Dichlormethan bei 0°C mit 314 μl (1.80 mmol) *N,N*-Diisopropylethylamin und 66 μl (0.901 mmol) Thionylchlorid versetzt und 75 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 151 mg (56% d. Th.) der Titelverbindung erhalten.
$^1$H-NMR (400 MHz, DMSO-$d_6$, δ/ppm): 7.34-7.27 (m, 1H), 7.26-7.19 (m, 1H), 6.55 (s, 1H), 4.35 (s, 2H), 4.09-4.03 (m, 2H), 4.01 (t, 2H), 3.60 (t, 2H), 3.28-3.18 (m, 7H), 2.86-2.79 (m, 2H), 2.39 (s, 3H).
LC/MS (Methode 3, ESIpos): $R_t$ = 1.12 min, m/z = 443 $[M+H]^+$.

**Beispiel 177**

1-(2,2-Difluorethyl)-3-ethyl-5-methyl-6-[(2-oxoimidazolidin-1-yl)methyl]thieno[2,3-d]pyrimidin-2,4(1*H*,3*H*)-dion

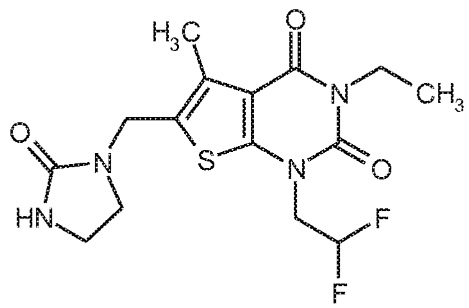

353 mg (0.866 mmol) der Verbindung aus Bsp. 212A wurden in 16 ml Dioxan gelöst und mit 217 mg (1.299 mmol) CDI versetzt. Das Gemisch wurde 16 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 171 mg (53% d. Th.) der Titelverbindung erhalten.
$^1$H-NMR (400 MHz, DMSO-$d_6$, $\delta$/ppm): 6.56 (s, 1H), 6.50-6.19 (m, 1H), 4.40-4.25 (m, 4H), 3.90 (q, 2H), 3.28-3.17 (m, 4H), 2.40 (s, 3H), 1.12 (t, 3H).
LC/MS (Methode 3, ESIpos): $R_t$ = 0.92 min, m/z = 373 $[M+H]^+$.

**Beispiel 178**

1-[2-(Cyclopropyloxy)ethyl]-3-ethyl-5-methyl-6-[(2-oxoimidazolidin-1-yl)methyl]thieno[2,3-d]-pyrimidin-2,4(1*H*,3*H*)-dion

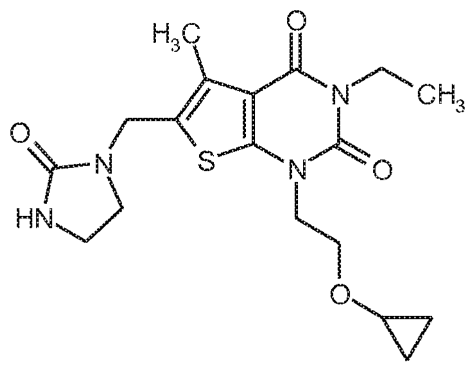

140 mg (0.271 mmol) der Verbindung aus Bsp. 308A wurden in 15 ml Dioxan gelöst und mit 68 mg (0.407 mmol) CDI versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 84 mg (78% d. Th.) der Titelverbindung erhalten.
$^1$H-NMR (400 MHz, DMSO-$d_6$, $\delta$/ppm): 6.54 (s, 1H), 4.34 (s, 2H), 3.99 (t, 2H), 3.90 (q, 2H), 3.72 (t, 2H), 3.33-3.29 (m, 1H), 3.27-3.16 (m, 4H), 2.39 (s, 3H), 1.11 (t, 3H), 0.43-0.33 (m, 4H).
LC/MS (Methode 3, ESIpos): $R_t$ = 0.98 min, m/z = 393 $[M+H]^+$.

**Beispiel 179**

3-Ethyl-5-methyl-6-[(2-oxoimidazolidin-1-yl)methyl]-1-(tetrahydro-2*H*-pyran-2-ylmethyl)thieno-[2,3-d]pyrimidin-2,4(1*H*,3*H*)-dion (*Enantiomer 1*)

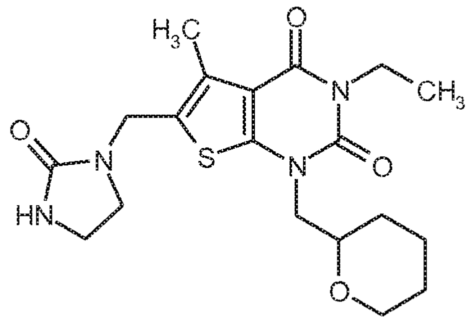

189 mg der racemischen Verbindung aus Bsp. 71 wurden in 6 ml eines Methanol/Dichlormethan-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent A: Acetonitril + 0.1% Diethylamin, Eluent B: Ethanol; isokratisch 90% A + 10% B; Fluss: 50 ml/min; Temperatur: RT; Detektion: DAD 254 nm]. Die Produktfraktionen wurden am Rotationsverdampfer eingeengt, mit *tert*.-Butanol versetzt und gefriergetrocknet. Es wurden 80 mg (41% d. Th.) der Titelverbindung (Enantiomer 1) sowie 81 mg (42% d. Th.) des Enantiomers 2 (siehe Beispiel 180) erhalten.

$^1$H-NMR (400 MHz, DMSO-$d_6$, δ/ppm): 6.54 (s, 1H), 4.39-4.28 (m, 2H), 4.02-3.93 (m, 1H), 3.89 (q, 2H), 3.84-3.75 (m, 1H), 3.73-3.62 (m, 2H), 3.29-3.16 (m, 5H), 2.38 (s, 3H), 1.77 (br. s, 1H), 1.61 (d, 1H), 1.52-1.37 (m, 3H), 1.31-1.19 (m, 1H), 1.11 (t, 3H).

Chirale analytische HPLC [Säule: Daicel Chiralpak IC, 3 μm, 100 mm x 4.6 mm; Eluent A: Acetonitril + 0.1% Diethylamin, Eluent B: Ethanol; isokratisch 90% A + 10% B; Fluss: 1.0 ml/min; Temperatur: 25°C; Injektion: 5 μl; DAD 254 nm]: $R_t$ = 8.06 min.

**Beispiel 180**

3-Ethyl-5-methyl-6-[(2-oxoimidazolidin-1-yl)methyl]-1-(tetrahydro-2*H*-pyran-2-ylmethyl)thieno-[2,3-d]pyrimidin-2,4(1*H*,3*H*)-dion *(Enantiomer 2)*

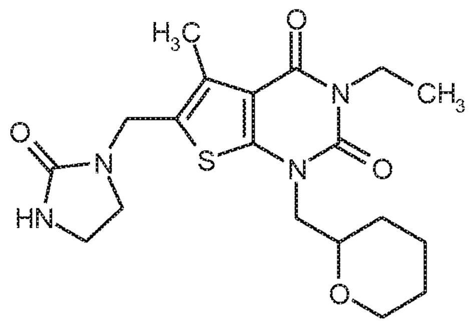

Die Titelverbindung (81 mg) wurde als zweites Enantiomer bei der unter Bsp. 179 beschriebenen präparativen HPLC-Trennung des Racemats aus Bsp. 71 erhalten.

$^1$H-NMR (400 MHz, DMSO-$d_6$, δ/ppm): 6.54 (s, 1H), 4.39-4.28 (m, 2H), 4.02-3.93 (m, 1H), 3.89 (q, 2H), 3.84-3.76 (m, 1H), 3.73-3.63 (m, 2H), 3.30-3.17 (m, 5H), 2.38 (s, 3H), 1.77 (br. s, 1H), 1.61 (d, 1H), 1.51-1.38 (m, 3H), 1.31-1.19 (m, 1H), 1.11 (t, 3H).

Chirale analytische HPLC [Säule: Daicel Chiralpak IC, 3 μm, 100 mm x 4.6 mm; Eluent A: Acetonitril + 0.1% Diethylamin, Eluent B: Ethanol; isokratisch 90% A + 10% B; Fluss: 1.0 ml/min; Temperatur: 25°C; Injektion: 5 μl; DAD 254 nm]: $R_t$ = 9.39 min.

**Beispiel 181**

3-Isopropyl-5-methyl-6-[(2-oxoimidazolidin-1-yl)methyl]-1-(3,3,3-trifluorpropyl)thieno[2,3-d]-pyrimidin-2,4(1*H*,3*H*)-dion

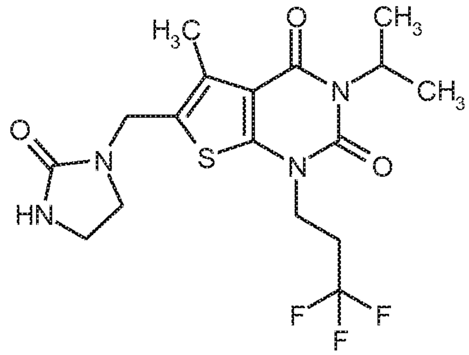

495 mg (1.28 mmol) der Verbindung aus Bsp. 204A wurden in ca. 10 ml Methanol gelöst und über eine Hydrogencarbonat-Kartusche (Fa. Polymerlabs, Stratospheres SPE, PL-$HCO_3$ MP SPE) gegeben. Nach Eindampfen der Lösung wurde so das freie Amin 6-(Aminomethyl)-3-isopropyl-5-methyl-1-(3,3,3-trifluolpropyl)thieno[2,3-d]pyrimidin-2,4(1*H*,3*H*)-dion erhalten, das dann in einem Gemisch aus 8.7 ml DMF und 4.2 ml THF gelöst und bei RT mit 115 µl (1.35 mmol) 2-Chlorethylisocyanat versetzt wurde. Nachdem das Reaktionsgemisch 40 min bei RT gerührt worden war, wurde es mit 216 mg (1.92 mmol) Kalium-*tert*.-butylat versetzt. Nach weiteren 60 min bei RT wurde das Reaktionsgemisch mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und eingeengt. Das so erhaltene Rohprodukt wurde mittels MPLC gereinigt (Instrument Biotage Isolera One, Kartusche SNAP KP-Sil, 50 g Kieselgel, Laufmittel Cyclohexan/Ethylacetat 15:85 → 0:100). Nach Vereinigen der Produktfraktionen und Einengen wurde das Produkt in Pentan/Dichlormethan (10:1) 30 min bei RT verrührt. Absaugen des Feststoffs und Trocknen im Hochvakuum ergaben 183 mg (34% d. Th.) der Titelverbindung.
$^1$H-NMR (400 MHz, DMSO-$d_6$, δ/ppm): 6.53 (s, 1H), 5.13 (sept, 1H), 4.36 (s, 2H), 4.06 (t, 2H), 3.28-3.16 (m, 4H), 2.86-2.62 (m, 2H), 2.39 (s, 3H), 1.40 (d, 6H).
LC/MS (Methode 1, ESIpos): $R_t$ = 0.94 min, m/z = 419 $[M+H]^+$.

## Beispiel 182

1-(2-Fluorethyl)-3-isopropyl-5-methyl-6-[(2-oxoimidazolidin-1-yl)methyl]thieno[2,3-d]pyrimidin-2,4(1*H*,3*H*)-dion

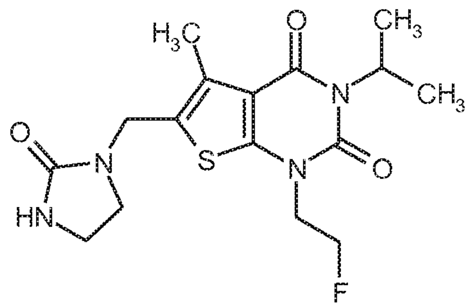

379 mg (1.06 mmol, 94% Reinheit) der Verbindung aus Bsp. 203A wurden in ca. 10 ml Methanol gelöst und über eine Hydrogencarbonat-Kartusche (Fa. Polymerlabs, Stratospheres SPE, PL-$HCO_3$ MP SPE) gegeben. Nach Eindampfen der Lösung wurde so das freie Amin 6-(Aminomethyl)-1-(2-fluorethyl)-3-isopropyl-5-methylthieno[2,3-d]pyrimidin-2,4(1*H*,3*H*)-dion erhalten, das dann in einem Gemisch aus 7.2 ml DMF und 3.5 ml THF gelöst und bei RT mit 95 µl (1.11 mmol) 2-Chlorethylisocyanat versetzt wurde. Nachdem das Reaktionsgemisch 50 min bei RT gerührt worden war, wurde es mit 179 mg (1.59 mmol) Kalium-*tert*.-butylat versetzt. Nach weiteren 60 min bei RT wurde das Reaktionsgemisch mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und eingeengt. Das so erhaltene Rohprodukt wurde mittels MPLC gereinigt (Instrument Biotage Isolera One, Kartusche SNAP KP-Sil, 50 g Kieselgel, Laufmittel Cyclohexan/Ethylacetat 15:85 → 0:100). Nach Vereinigen und Einengen der Produktfraktionen und Trocknen im Hochvakuum wurden 185 mg (45% d. Th., 96% Reinheit) der Titelverbindung erhalten.
$^1$H-NMR (400 MHz, DMSO-$d_6$, δ/ppm): 6.51 (s, 1H), 5.13 (sept, 1H), 4.71 (dt, 2H), 4.34 (s, 2H), 4.15 (dt, 2H), 3.27-3.16

(m, 4H), 2.38 (s, 3H), 1.41 (d, 6H).
LC/MS (Methode 1, ESIpos): $R_t$ = 0.82 min, m/z = 369 $[M+H]^+$.

**Beispiel 183**

3-Isopropyl-5-methyl-1-(oxetan-2-ylmethyl)-6-[(2-oxoimidazolidin-1-yl)methyl]thieno[2,3-d]-pyrimidin-2,4(1*H*,3*H*)-dion (*Racemat*)

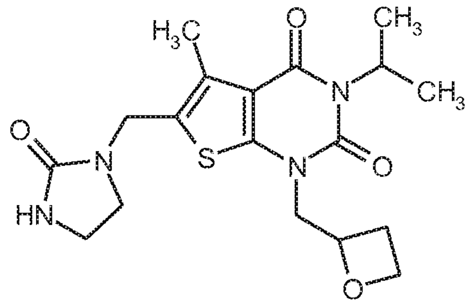

284 mg (0.746 mmol, 85% Reinheit) der Verbindung aus Bsp. 205A wurden in einem Gemisch aus 5 ml DMF und 2.4 ml THF gelöst und bei RT mit 67 µl (0.783 mmol) 2-Chlorethylisocyanat versetzt. Nachdem das Reaktionsgemisch 20 min bei RT gerührt worden war, wurde es mit 126 mg (1.12 mmol) Kalium-*tert.*-butylat versetzt. Nach weiteren 60 min bei RT wurde das Reaktionsgemisch mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und eingeengt. Das so erhaltene Rohprodukt wurde zunächst mittels MPLC gereinigt (Instrument Biotage Isolera One, Kartusche SNAP KP-Sil, 50 g Kieselgel, Laufmittel Cyclohexan/Ethylacetat 15:85 → 0:100). Die Produktfraktionen wurden vereinigt und eingeengt. Das so erhaltene Material wurde dann mittels präparativer HPLC (Methode 8) weiter aufgereinigt. Nach Einengen der Produktfraktionen und Einengen wurde das Produkt noch in Pentan/Dichlormethan (10:1) 30 min bei RT verrührt. Absaugen des Feststoffs und Trocknen im Hochvakuum ergaben 108 mg (36% d. Th.) der Titelverbindung.
$^1$H-NMR (400 MHz, DMSO-$d_6$, δ/ppm): 6.51 (s, 1H), 5.14 (sept, 1H), 4.99 (quin, 1H), 4.53-4.37 (m, 2H), 4.33 (s, 2H), 4.15-4.04 (m, 2H), 3.27-3.15 (m, 4H), 2.76-2.62 (m, 1H), 2.53-2.44 (m, 1H, weitgehend überdeckt vom DMSO-Signal), 2.38 (s, 3H), 1.40 (d, 6H).
LC/MS (Methode 6, ESIpos): $R_t$ = 1.39 min, m/z = 393 $[M+H]^+$.

**Beispiel 184**

3-Isopropyl-5-methyl-1-(oxetan-2-ylmethyl)-6-[(2-oxoimidazolidin-1-yl)methyl]thieno[2,3-d]-pyrimidin-2,4(1*H*,3*H*)-dion (*Enantiomer 1*)

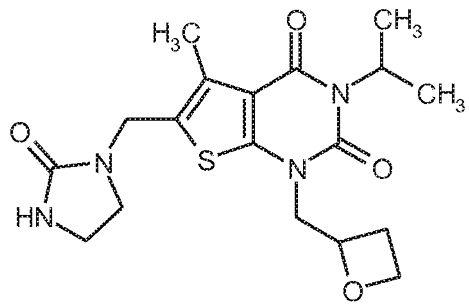

106 mg (0.270 mmol) der racemischen Verbindung aus Bsp. 183 wurden in 5 ml Ethanol gelöst und in 10 Portionen über präparative HPLC an chiraler Phase in die Enantiomere aufgetrennt [Säule: Daicel Chiralcel OD-H, 5 µm, 250 mm x 20 mm; Laufmittel: Isohexan/Ethanol 1:1; Fluss: 15 ml/min; Temperatur: 25°C; Detektion: 210 nm]. Nach

Einengen der Produktfraktionen wurde der Rückstand in Pentan/Dichlormethan (10:1) 60 min bei RT verrührt. Der Feststoff wurde abgesaugt und im Hochvakuum getrocknet. Es wurden 22 mg (42% d. Th.) von Enantiomer 1 erhalten (99% ee, chirale analytische HPLC).
$^1$H-NMR (400 MHz, DMSO-$d_6$, $\delta$/ppm): 6.51 (s, 1H), 5.13 (sept, 1H), 5.05-4.93 (m, 1H), 4.53-4.37 (m, 2H), 4.33 (s, 2H), 4.15-4.05 (m, 2H), 3.26-3.14 (m, 4H), 2.77-2.63 (m, 1H), 2.53-2.44 (m, 1H, weitgehend überdeckt vom DMSO-Signal), 2.38 (s, 3H), 1.40 (d, 6H).

Chirale analytische HPLC [Säule: Daicel Chiralpak OX-3, 3 μm, 50 mm x 4.6 mm; Laufmittel: Isohexan/Ethanol 1:1; Fluss: 1 ml/min; Temperatur: 40°C; Detektion: 220 nm]: $R_t$ = 2.77 min.

## Beispiel 185

3-Isopropyl-5-methyl-1-(oxetan-2-ylmethyl)-6-[(2-oxoimidazolidin-1-yl)methyl]thieno[2,3-d]-pyrimidin-2,4(1*H*,3*H*)-dion (*Enantiomer 2*)

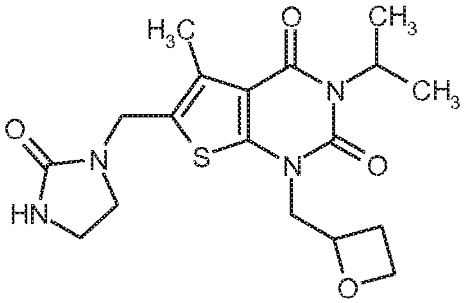

106 mg (0.270 mmol) der racemischen Verbindung aus Bsp. 183 wurden in 5 ml Ethanol gelöst und in 10 Portionen über präparative HPLC an chiraler Phase in die Enantiomere aufgetrennt [Säule: Daicel Chiralcel OD-H, 5 μm, 250 mm x 20 mm; Laufmittel: Isohexan/Ethanol 1:1; Fluss: 15 ml/min; Temperatur: 25°C; Detektion: 210 nm]. Nach Einengen der Produktfraktionen wurde der Rückstand in Pentan/Dichlormethan (10:1) 60 min bei RT verrührt. Der Feststoff wurde abgesaugt und im Hochvakuum getrocknet. Es wurden 33 mg (63% d. Th.) von Enantiomer 2 erhalten (99% ee, chirale analytische HPLC).
$^1$H-NMR (400 MHz, DMSO-$d_6$, $\delta$/ppm): 6.51 (s, 1H), 5.14 (sept, 1H), 5.05-4.94 (m, 1H), 4.54-4.37 (m, 2H), 4.33 (s, 2H), 4.16-4.02 (m, 2H), 3.26-3.18 (m, 4H), 2.76-2.62 (m, 1H), 2.53-2.44 (m, 1H, weitgehend überdeckt vom DMSO-Signal), 2.38 (s, 3H), 1.40 (d, 6H).

Chirale analytische HPLC [Säule: Daicel Chiralpak OX-3, 3 μm, 50 mm x 4.6 mm; Laufmittel: Isohexan/Ethanol 1:1; Fluss: 1 ml/min; Temperatur: 40°C; Detektion: 220 nm]: $R_t$ = 3.38 min.

## Beispiel 186

3-Isopropyl-5-methyl-6-[(2-oxoimidazolidin-1-yl)methyl]-1-(tetrahydrofuran-2-ylmethyl)thieno-[2,3-d]pyrimidin-2,4(1*H*,3*H*)-dion (*Racemat*)

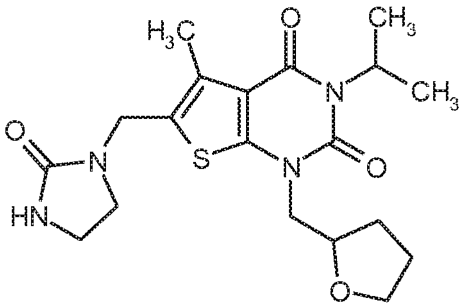

262 mg (0.619 mmol, 88% Reinheit) der Verbindung aus Bsp. 206A wurden in ca. 10 ml Methanol gelöst und

über eine Hydrogencarbonat-Kartusche (Fa. Polymerlabs, Stratospheres SPE, PL-$HCO_3$ MP SPE) gegeben. Nach Eindampfen der Lösung wurde so das freie Amin 6-(Aminomethyl)-3-isopropyl-5-methyl-1-(tetrahydrofuran-2-ylmethyl)thieno[2,3-d]pyrimidin-2,4(1*H*,3*H*)-dion erhalten, das dann in einem Gemisch aus 4.2 ml DMF und 2.0 ml THF gelöst und bei RT mit 55 µl (0.649 mmol) 2-Chlorethylisocyanat versetzt wurde. Nachdem das Reaktionsgemisch 30 min bei RT gerührt worden war, wurde es mit 104 mg (0.928 mmol) Kalium-*tert*.-butylat versetzt. Nach weiteren 50 min bei RT wurde das Reaktionsgemisch mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und eingeengt. Das so erhaltene Rohprodukt wurde mittels Saugfiltration über Kieselgel mit Ethylacetat als Laufmittel gereinigt. Nach Einengen der Produktfraktionen und Trocknen im Hochvakuum wurden 112 mg (44% d. Th.) der Titelverbindung erhalten.
$^1$H-NMR (400 MHz, DMSO-$d_6$, δ/ppm): 6.51 (s, 1H), 5.14 (sept, 1H), 4.33 (s, 2H), 4.26-4.16 (m, 1H), 4.02 (dd, 1H), 3.74 (q, 1H), 3.69-3.57 (m, 2H), 3.27-3.14 (m, 4H), 2.38 (s, 3H), 2.05-1.74 (m, 3H), 1.71-1.59 (m, 1H), 1.40 (d, 6H).
LC/MS (Methode 1, ESIpos): $R_t$ = 0.85 min, m/z = 407 $[M+H]^+$.

**Beispiel 187**

3-Isopropyl-5-methyl-6-[(2-oxoimidazolidin-1-yl)methyl]-1-(tetrahydrofuran-2-ylmethyl)thieno-[2,3-d]pyrimidin-2,4(1*H*,3*H*)-dion (*Enantiomer 1*)

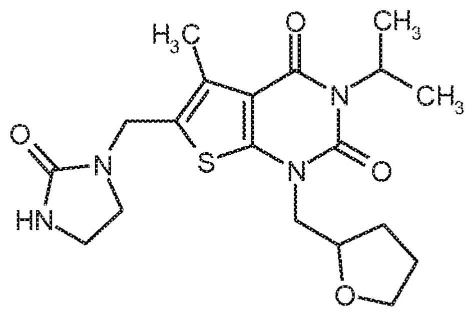

96 mg (0.236 mmol) der racemischen Verbindung aus Bsp. 186 wurden in 5 ml Isopropanol gelöst und in 5 Portionen über präparative HPLC an chiraler Phase in die Enantiomere aufgetrennt [Säule: Daicel Chiralcel OD-H, 5 µm, 250 mm x 20 mm; Laufmittel: Isohexan/Isopropanol 1:1; Fluss: 15 ml/min; Temperatur: 25°C; Detektion: 210 nm]. Nach Einengen der Produktfraktionen wurde der Rückstand in Pentan/Dichlormethan (10:1) 60 min bei RT verrührt. Der Feststoff wurde abgesaugt und im Hochvakuum getrocknet. Es wurden 16 mg (33% d. Th.) von Enantiomer 1 erhalten (>99% ee, chirale analytische HPLC).
$^1$H-NMR (400 MHz, DMSO-$d_6$, δ/ppm): 6.51 (s, 1H), 5.14 (sept, 1H), 4.33 (s, 2H), 4.26-4.16 (m, 1H), 4.02 (dd, 1H), 3.74 (q, 1H), 3.69-3.57 (m, 2H), 3.26-3.16 (m, 4H), 2.38 (s, 3H), 2.04-1.76 (m, 3H), 1.71-1.59 (m, 1H), 1.40 (dd, 6H).

Chirale analytische HPLC [Säule: Daicel Chiralpak OX-3, 3 µm, 50 mm x 4.6 mm; Laufmittel: Isohexan/Isopropanol 1:1; Fluss: 1 ml/min; Temperatur: 40°C; Detektion: 220 nm]: $R_t$ = 2.48 min.

**Beispiel 188**

3-Isopropyl-5-methyl-6-[(2-oxoimidazolidin-1-yl)methyl]-1-(tetrahydrofuran-2-ylmethyl)thieno-[2,3-d]pyrimidin-2,4(1*H*,3*H*)-dion (*Enantiomer 2*)

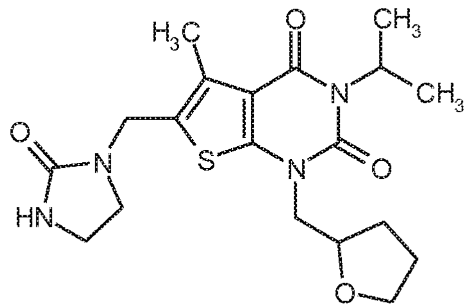

96 mg (0.236 mmol) der racemischen Verbindung aus Bsp. 186 wurden in 5 ml Isopropanol gelöst und in 5 Portionen über präparative HPLC an chiraler Phase in die Enantiomere aufgetrennt [Säule: Daicel Chiralcel OD-H, 5 µm, 250 mm x 20 mm; Laufmittel: Isohexan/Isopropanol 1:1; Fluss: 15 ml/min; Temperatur: 25°C; Detektion: 210 nm]. Nach Einengen der Produktfraktionen wurde der Rückstand in Pentan/Dichlormethan (10:1) 60 min bei RT verrührt. Der Feststoff wurde abgesaugt und im Hochvakuum getrocknet. Es wurden 19 mg (39% d. Th.) von Enantiomer 2 erhalten (>99% ee, chirale analytische HPLC).
$^1$H-NMR (400 MHz, DMSO-$d_6$, δ/ppm): 6.51 (s, 1H), 5.14 (sept, 1H), 4.33 (s, 2H), 4.27-4.15 (m, 1H), 4.02 (dd, 1H), 3.79-3.70 (m, 1H), 3.69-3.56 (m, 2H), 3.26-3.16 (m, 4H), 2.38 (s, 3H), 2.04-1.76 (m, 3H), 1.70-1.59 (m, 1H), 1.40 (dd, 6H).

Chirale analytische HPLC [Säule: Daicel Chiralpak OX-3, 3 µm, 50 mm x 4.6 mm; Laufmittel: Isohexan/Isopropanol 1:1; Fluss: 1 ml/min; Temperatur: 40°C; Detektion: 220 nm]: $R_t$ = 3.33 min.

**Beispiel 189**

3-Isopropyl-5-methyl-1-(oxetan-3-ylmethyl)-6-[(2-oxoimidazolidin-1-yl)methyl]thieno[2,3-d]-pyrimidin-2,4(1*H*,3*H*)-dion

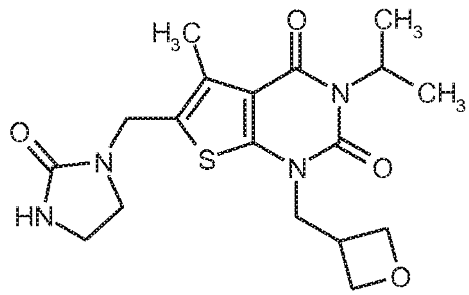

212 mg (0.459 mmol, 70% Reinheit) der Verbindung aus Bsp. 207A wurden in einem Gemisch aus 3.1 ml DMF und 1.5 ml THF gelöst und bei RT mit 41 µl (0.482 mmol) 2-Chlorethylisocyanat versetzt. Nachdem das Reaktionsgemisch 30 min bei RT gerührt worden war, wurde es mit 77 mg (0.688 mmol) Kalium-*tert*.-butylat versetzt. Nach weiteren 60 min bei RT wurde das Reaktionsgemisch mit Ethylacetat verdünnt und nacheinander mit gesättigter Natriumhydrogencarbonat-Lösung und gesättigter Natriumchlorid-Lösung gewaschen. Nach Trocknen über wasserfreiem Magnesiumsulfat wurde filtriert und eingeengt. Das so erhaltene Rohprodukt wurde zunächst mittels präparativer HPLC (Methode 8) vorgereinigt. Das resultierende Produkt wurde danach in Pentan/Dichlormethan (10:1) 30 min bei RT verrührt. Dadurch wurde eine weitergehende, aber nicht ausreichende Reinigung erzielt. Nach nochmaliger präparativer HPLC (Methode 8) wurden 26 mg (14% d. Th.) der Titelverbindung erhalten.
$^1$H-NMR (400 MHz, DMSO-$d_6$, δ/ppm): 6.52 (s, 1H), 5.12 (sept, 1H), 4.62 (dd, 2H), 4.43 (t, 2H), 4.34 (s, 2H), 4.16 (d, 2H), 3.47-3.36 (m, 1H), 3.27-3.17 (m, 4H), 2.38 (s, 3H), 1.39 (d, 6H).
LC/MS (Methode 1, ESIpos): $R_t$ = 0.77 min, m/z = 393 $[M+H]^+$.

**Beispiel 190**

3-*sec.*-Butyl-1-(2-methoxyethyl)-5-methyl-6-[(2-oxoimidazolidin-1-yl)methyl]thieno[2,3-d]pyrimidin-2,4(1*H*,3*H*)-dion (*Racemat*)

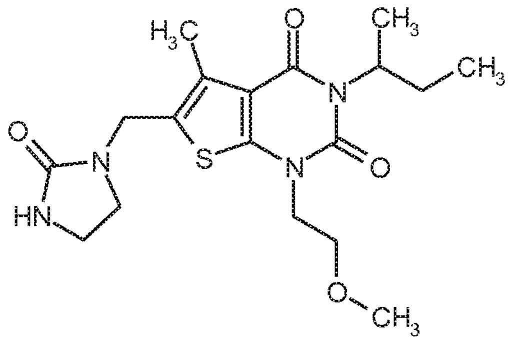

Eine Lösung von 375 mg (4.18 mmol) 2-Imidazolidinon in 15 ml THF wurde mit 167 mg (4.18 mmol) Natriumhydrid (60% Suspension in Mineralöl) versetzt und 3 h bei 60°C gerührt ("Lösung 1"). In einem anderen Reaktionsgefäß wurde eine Lösung von 341 mg (1.05 mmol) der Verbindung aus Bsp. 147A in 7.3 ml Dichlormethan bei 0°C mit 546 µl (3.13 mmol) *N,N*-Diisopropylethylamin und 114 µl (1.57 mmol) Thionylchlorid versetzt und 75 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 210 mg (51% d. Th.) der Titelverbindung erhalten.

$^1$H-NMR (400 MHz, DMSO-$d_6$, δ/ppm): 6.54 (s, 1H), 4.90 (d, 1H), 4.33 (s, 2H), 4.06-3.92 (m, 2H), 3.61 (t, 2H), 3.28-3.17 (m, 7H), 2.37 (s, 3H), 2.09-1.95 (m, 1H), 1.73 (dquin, 1H), 1.37 (d, 3H), 0.75 (t, 3H).

LC/MS (Methode 3, ESIpos): $R_t$ = 1.04 min, m/z = 395 $[M+H]^+$.

**Beispiel 191**

1-(2,2-Difluorethyl)-3-isobutyl-5-methyl-6-[(2-oxoimidazolidin-1-yl)methyl]thieno[2,3-d]pyrimidin-2,4(1*H*,3*H*)-dion

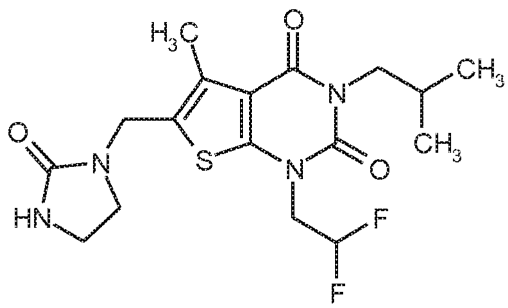

138 mg (0.28 mmol) der Verbindung aus Bsp. 313A wurden in 15 ml Dioxan gelöst und mit 70 mg (0.42 mmol) CDI versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 57 mg (50% d. Th.) der Titelverbindung erhalten.

$^1$H-NMR (400 MHz, DMSO-$d_6$, δ/ppm): 6.54 (s, 1H), 6.50-6.18 (m, 1H), 4.40-4.24 (m, 4H), 3.71 (d, 2H), 3.29-3.18 (m, 4H), 2.39 (s, 3H), 2.09-1.97 (m, 1H), 0.85 (d, 6H).

LC/MS (Methode 3, ESIpos): $R_t$ = 1.09 min, m/z = 401 $[M+H]^+$.

**Beispiel 192**

1-(3-Fluorpropyl)-3-isobutyl-5-methyl-6-[(2-oxoimidazolidin-1-yl)methyl]thieno[2,3-d]pyrimidin-2,4(1*H*,3*H*)-dion

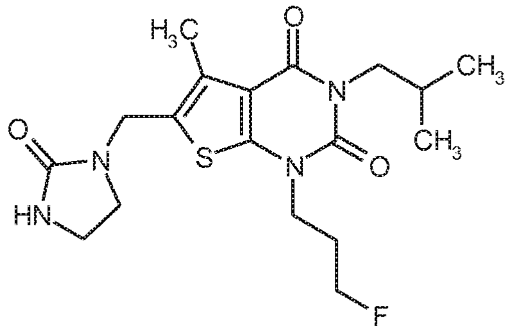

170 mg (0.266 mmol) der Verbindung aus Bsp. 241A wurden in 15 ml Dioxan gelöst und mit 67 mg (0.399 mmol) CDI versetzt. Das Gemisch wurde 18 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 57 mg (54% d. Th.) der Titelverbindung erhalten.
$^1$H-NMR (400 MHz, DMSO-$d_6$, δ/ppm): 6.55 (s, 1H), 4.58 (t, 1H), 4.47 (t, 1H), 4.35 (s, 2H), 3.98 (t, 2H), 3.70 (d, 2H), 3.29-3.17 (m, 4H), 2.39 (s, 3H), 2.13-1.96 (m, 3H), 0.84 (d, 6H).
LC/MS (Methode 3, ESIpos): $R_t$ = 1.08 min, m/z = 397 $[M+H]^+$.

**Beispiel 193**

1-(2-Ethoxyethyl)-3-isobutyl-5-methyl-6-[(2-oxoimidazolidin-1-yl)methyl]thieno[2,3-d]pyrimidin-2,4(1*H*,3*H*)-dion

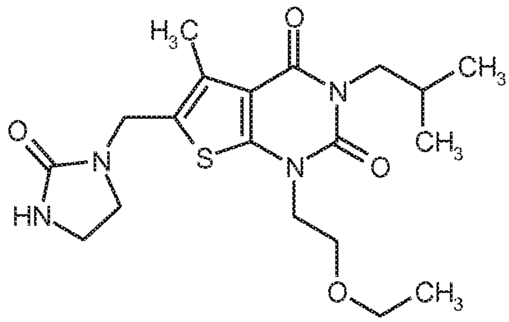

260 mg (0.564 mmol) der Verbindung aus Bsp. 314A wurden in 20 ml Dioxan gelöst und mit 141 mg (0.846 mmol) CDI versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 98 mg (42% d. Th.) der Titelverbindung erhalten.
$^1$H-NMR (400 MHz, DMSO-$d_6$, δ/ppm): 6.53 (s, 1H), 4.35 (s, 2H), 4.01 (t, 2H), 3.71 (d, 2H), 3.65 (t, 2H), 3.42 (q, 2H), 3.28-3.16 (m, 4H), 2.38 (s, 3H), 2.09-1.97 (m, 1H), 1.02 (t, 3H), 0.85 (d, 6H).
LC/MS (Methode 3, ESIpos): $R_t$ = 1.11 min, m/z = 409 $[M+H]^+$.

**Beispiel 194**

3-Isobutyl-5-methyl-1-(oxetan-2-ylmethyl)-6-[(2-oxoimidazolidin-1-yl)methyl]thieno[2,3-d]-pyrimidin-2,4(1*H*,3*H*)-dion (*Racemat*)

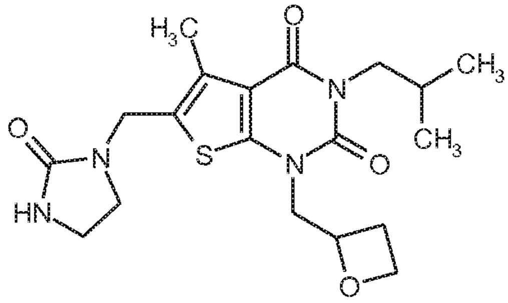

813 mg (1.84 mmol) der Verbindung aus Bsp. 243A wurden in 40 ml Dioxan gelöst und mit 461 mg (2.76 mmol) CDI versetzt. Das Gemisch wurde 16 h bei RT gerührt. Die Reaktionslösung wurde danach am Rotationsverdampfer eingeengt. Der Rückstand wurde in 15 ml DMSO gelöst und diese Lösung mittels präparativer HPLC gereinigt (Methode 14). Nach Vereinigen der Produktfraktionen und Gefriertrocknung wurden 383 mg (42% d. Th.) der Titelverbindung erhalten.
$^1$H-NMR (400 MHz, DMSO-$d_6$, δ/ppm): 6.54 (s, 1H), 5.05-4.95 (m, 1H), 4.52-4.37 (m, 2H), 4.34 (s, 2H), 4.13 (d, 2H), 3.71 (d, 2H), 3.28-3.16 (m, 4H), 2.74-2.62 (m, 1H), 2.38 (s, 2H), 2.03 (dquin, 1H), 0.85 (dd, 6H).
LC/MS (Methode 3, ESIpos): $R_t$ = 1.02 min, m/z = 407 [M+H]$^+$.

**Beispiel 195**

3-Isobutyl-5-methyl-1-(oxetan-2-ylmethyl)-6-[(2-oxoimidazolidin-1-yl)methyl]thieno[2,3-d]-pyrimidin-2,4(1*H*,3*H*)-dion (*Enantiomer 1*)

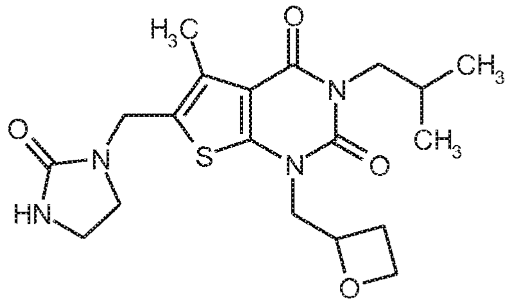

348 mg der racemischen Verbindung aus Bsp. 194 wurden in 5.4 ml eines Methanol/Acetonitril-Gemisches (1:1 v/v) gelöst und mittels präparativer HPLC an chiraler Phase in die Enantiomere getrennt [Säule: Daicel Chiralpak IC, 5 µm, 250 mm x 30 mm; Eluent: Acetonitril/Ethanol/Diethylamin 90:10:0.1 v/v; Fluss: 50 ml/min; Temperatur: RT; Detektion: 254 nm]. Die Produktfraktionen wurden am Rotationsverdampfer eingeengt, mit *tert.*-Butanol versetzt und gefriergetrocknet. Es wurden 70 mg (20% d. Th.) der Titelverbindung (Enantiomer 1) sowie 67 mg (19% d. Th.) des Enantiomers 2 (siehe Beispiel 196) erhalten.
$^1$H-NMR (400 MHz, DMSO-$d_6$, δ/ppm): 6.55 (s, 1H), 5.04-4.96 (m, 1H), 4.51-4.44 (m, 1H), 4.40 (dt, 1H), 4.34 (s, 2H), 4.16-4.09 (m, 2H), 3.70 (d, 2H), 3.28-3.16 (m, 4H), 2.68 (dtd, 1H), 2.38 (s, 3H), 2.02 (dquin, 1H), 0.84 (dd, 6H).
Chirale analytische HPLC [Säule: Daicel Chiralpak IC, 5 µm, 100 mm x 4.6 mm; Eluent: Acetonitril + 0.1% Diethylamin/Ethanol 90:10; Fluss: 1.0 ml/min; Temperatur: RT; Injektion: 5 µl; DAD 254 nm; Lösung: 1.0 mg/ml Methanol]: $R_t$ = 8.11 min.

**Beispiel 196**

3-Isobutyl-5-methyl-1-(oxetan-2-ylmethyl)-6-[(2-oxoimidazolidin-1-yl)methyl]thieno[2,3-d]-pyrimidin-2,4(1*H*,3*H*)-dion (*Enantiomer 2*)

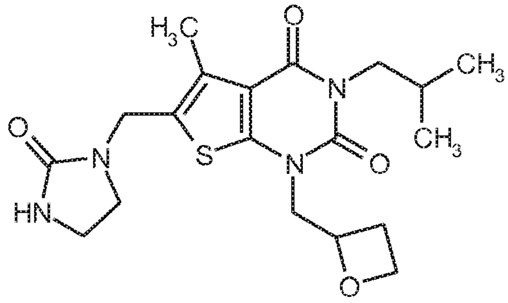

Die Titelverbindung (67 mg) wurde als zweites Enantiomer bei der unter Bsp. 195 beschriebenen präparativen HPLC-Trennung des Racemats aus Bsp. 194 erhalten.
$^1$H-NMR (400 MHz, DMSO-$d_6$, δ/ppm): 6.54 (s, 1H), 5.04-4.95 (m, 1H), 4.52-4.44 (m, 1H), 4.40 (dt, 1H), 4.34 (s, 2H), 4.13 (d, 2H), 3.71 (d, 2H), 3.28-3.18 (m, 4H), 2.73-2.63 (m, 1H), 2.38 (s, 3H), 2.03 (dquin, 1H), 0.84 (dd, 6H).

Chirale analytische HPLC [Säule: Daicel Chiralpak IC, 5 µm, 100 mm x 4.6 mm; Eluent: Acetonitril + 0.1% Diethylamin/Ethanol 90:10; Fluss: 1.0 ml/min; Temperatur: RT; Injektion: 5 µl; DAD 254 nm; Lösung: 1.0 mg/ml Methanol]: $R_t$ = 9.99 min.

## Beispiel 197

3-Isobutyl-5-methyl-6-[(2-oxoimidazolidin-1-yl)methyl]-1-(tetrahydrofuran-2-ylmethyl)thieno-[2,3-d]pyrimidin-2,4(1*H*,3*H*)-dion (*Enantiomer 1*)

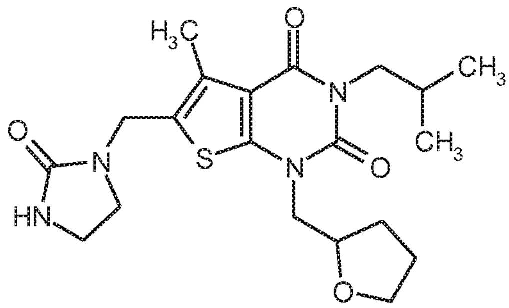

346 mg (0.823 mmol) der racemischen Verbindung aus Bsp. 90 wurden in 8 ml Isopropanol gelöst und in 32 Portionen über präparative HPLC an chiraler Phase in die Enantiomere aufgetrennt [Säule: Daicel Chiralpak ID, 5 µm, 250 mm x 20 mm; Laufmittel: Isohexan/Isopropanol 1:1; Fluss: 15 ml/min; Temperatur: 25°C; Detektion: 210 nm]. Nach Eindampfen der Produktfraktionen wurde eine weitere präparative HPLC an achiraler Phase (Methode 8) durchgeführt, um Verunreinigungen abzutrennen. Nach erneutem Vereinigen der Produktfraktionen, Einengen und Trocknen im Hochvakuum wurden 112 mg (68% d. Th.) von Enantiomer 1 erhalten (>99% ee, chirale analytische HPLC).
$^1$H-NMR (400 MHz, DMSO-$d_6$, δ/ppm): 6.52 (s, 1H), 4.34 (s, 2H), 4.27-4.17 (m, 1H), 4.01 (dd, 1H), 3.79-3.66 (m, 4H), 3.65-3.57 (m, 1H), 3.28-3.15 (m, 4H), 2.39 (s, 3H), 2.11-1.75 (m, 4H), 1.72-1.59 (m, 1H), 0.85 (dd, 6H).

Chirale analytische HPLC [Säule: Daicel Chiralpak ID-3, 3 µm, 50 mm x 4.6 mm; Laufmittel: Isohexan/Isopropanol 1:1; Fluss: 1 ml/min; Temperatur: 40°C; Detektion: 220 nm]: $R_t$ = 2.04 min.

## Beispiel 198

3-Isobutyl-5-methyl-6-[(2-oxoimidazolidin-1-yl)methyl]-1-(tetrahydrofuran-2-ylmethyl)thieno-[2,3-d]pyrimidin-2,4(1*H*,3*H*)-dion (*Enantiomer 2*)

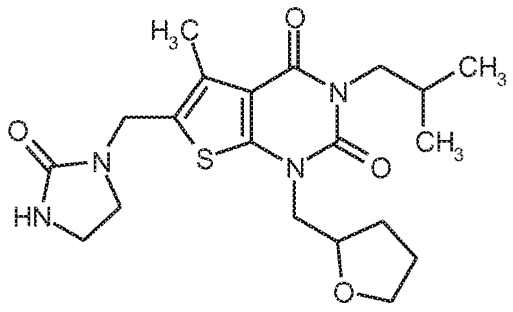

346 mg (0.823 mmol) der racemischen Verbindung aus Bsp. 90 wurden in 8 ml Isopropanol gelöst und in 32 Portionen über präparative HPLC an chiraler Phase in die Enantiomere aufgetrennt [Säule: Daicel Chiralpak ID, 5 μm, 250 mm x 20 mm; Laufmittel: Isohexan/Isopropanol 1:1; Fluss: 15 ml/min; Temperatur: 25°C; Detektion: 210 nm]. Nach Eindampfen der Produktfraktionen wurde eine weitere präparative HPLC an achiraler Phase (Methode 8) durchgeführt, um Verunreinigungen abzutrennen. Nach erneutem Vereinigen der Produktfraktionen, Einengen und Trocknen im Hochvakuum wurden 88 mg (50% d. Th.) von Enantiomer 2 erhalten (>99% ee, chirale analytische HPLC).
$^1$H-NMR (400 MHz, DMSO-$d_6$, δ/ppm): 6.52 (s, 1H), 4.34 (s, 2H), 4.27-4.17 (m, 1H), 4.01 (dd, 1H), 3.79-3.67 (m, 4H), 3.65-3.55 (m, 1H), 3.28-3.15 (m, 4H), 2.39 (s, 3H), 2.10-1.74 (m, 4H), 1.71-1.57 (m, 1H), 0.85 (dd, 6H).

Chirale analytische HPLC [Säule: Daicel Chiralpak ID-3, 3 μm, 50 mm x 4.6 mm; Laufmittel: Isohexan/Isopropanol 1:1; Fluss: 1 ml/min; Temperatur: 40°C; Detektion: 220 nm]: $R_t$ = 2.54 min.

**Beispiel 199**

3-Isobutyl-5-methyl-1-(oxetan-3-ylmethyl)-6-[(2-oxoimidazolidin-1-yl)methyl]thieno[2,3-d]-pyrimidin-2,4(1*H*,3*H*)-dion

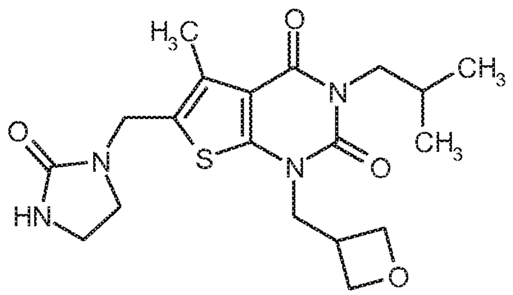

Eine Lösung von 84 mg (0.221 mmol) der Verbindung aus Bsp. 245A und 46 μl (0.331 mmol) Triethylamin in 2.5 ml THF wurde mit 43 mg (0.265 mmol) CDI versetzt und ca. 18 h bei RT gerührt. Anschließend wurde zur Trockene eingeengt. Der verbliebene Rückstand wurde in Ethylacetat aufgenommen und nacheinander mit 1 M Salzsäure, Wasser und gesättigter Natriumchlorid-Lösung gewaschen. Nach Trocknen über wasserfreiem Magnesiumsulfat wurde filtriert und eingeengt. Der feste Rückstand wurde zweimal nacheinander mittels präparativer HPLC gereinigt (jeweils Methode 8). Nach Einengen der Produktfraktionen und Trocknen des Feststoffs im Hochvakuum wurden 14 mg (15% d. Th.) der Titelverbindung erhalten.
$^1$H-NMR (400 MHz, DMSO-$d_6$, δ/ppm): 6.53 (s, 1H), 4.61 (dd, 2H), 4.43 (t, 2H), 4.35 (s, 2H), 4.20 (d, 2H), 3.70 (d, 2H), 3.48-3.36 (m, 1H), 3.28-3.17 (m, 4H), 2.39 (s, 3H), 1.07-1.97 (m, 1H), 0.84 (d, 6H).
LC/MS (Methode 1, ESIpos): $R_t$ = 0.78 min, m/z = 407 $[M+H]^+$.

**Beispiel 200**

3-(2,2-Dimethylpropyl)-5-methyl-6-[(2-oxoimidazolidin-1-yl)methyl]-1-(3,3,3-trifluorpropyl)-thieno[2,3-d]pyrimidin-2,4(1*H*,3*H*)-dion

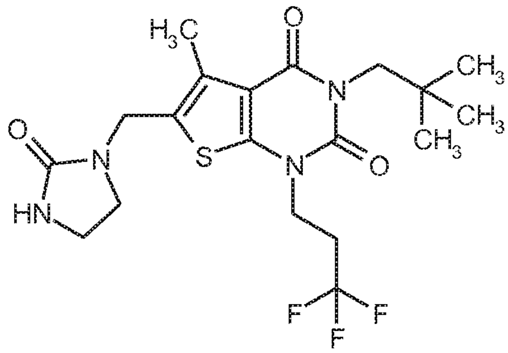

290 mg (0.572 mmol) der Verbindung aus Bsp. 315A wurden in 30 ml Dioxan gelöst und mit 143 mg (0.859 mmol) CDI versetzt. Das Gemisch wurde 21 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 128 mg (49% d. Th.) der Titelverbindung erhalten.
$^1$H-NMR (400 MHz, DMSO-$d_6$, δ/ppm): 6.56 (s, 1H), 4.36 (s, 2H), 4.09 (t, 2H), 3.81 (br. s, 2H), 3.31-3.17 (m, 4H), 2.82-2.69 (m, 2H), 2.39 (s, 3H), 0.89 (s, 9H).
LC/MS (Methode 3, ESIpos): $R_t$ = 1.25 min, m/z = 447 [M+H]$^+$.

**Beispiel 201**

3-(2,2-Dimethylpropyl)-1-(3-fluorpropyl)-5-methyl-6-[(2-oxoimidazolidin-1-yl)methyl]thieno-[2,3-d]pyrimidin-2,4(1*H*,3*H*)-dion

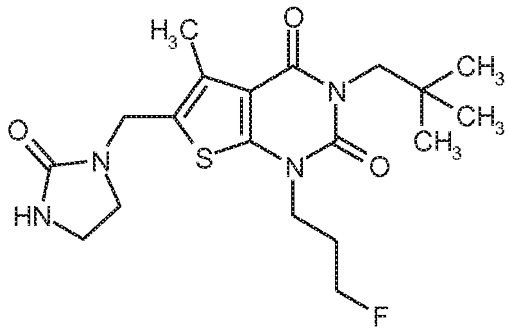

190 mg (0.405 mmol) der Verbindung aus Bsp. 316A wurden in 20 ml Dioxan gelöst und mit 101 mg (0.608 mmol) CDI versetzt. Das Gemisch wurde 20 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 120 mg (71% d. Th.) der Titelverbindung erhalten.
$^1$H-NMR (400 MHz, DMSO-$d_6$, δ/ppm): 6.55 (s, 1H), 4.59 (t, 1H), 4.47 (t, 1H), 4.35 (s, 2H), 3.97 (t, 2H), 3.81 (br. s, 2H), 3.30-3.17 (m, 4H), 2.38 (s, 3H), 2.12-2.05 (m, 1H), 2.01 (t, 1H), 0.89 (s, 9H).
LC/MS (Methode 3, ESIpos): $R_t$ = 1.15 min, m/z = 411 [M+H]$^+$.

**Beispiel 202**

3-(2,2-Dimethylpropyl)-1-(2-methoxyethyl)-5-methyl-6-[(2-oxoimidazolidin-1-yl)methyl]thieno-[2,3-d]pyrimidin-2,4(1*H*,3*H*)-dion

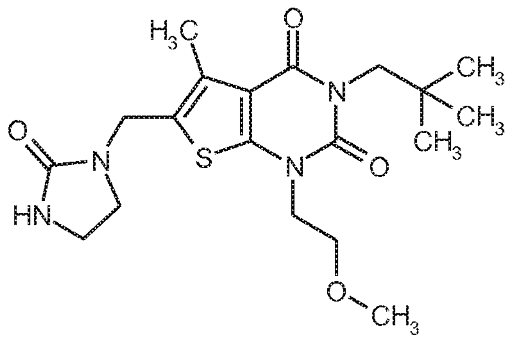

300 mg (0.635 mmol) der Verbindung aus Bsp. 317A wurden in 30 ml Dioxan gelöst und mit 159 mg (0.953 mmol) CDI versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 117 mg (43% d. Th.) der Titelverbindung erhalten.
$^1$H-NMR (400 MHz, DMSO-$d_6$, δ/ppm): 6.54 (s, 1H), 4.34 (s, 2H), 4.01 (t, 2H), 3.81 (br. s, 2H), 3.61 (d, 1H), 3.30-3.17 (m, 7H), 2.37 (s, 3H), 0.89 (s, 9H).
LC/MS (Methode 3, ESIpos): $R_t$ = 1.13 min, m/z = 409 [M+H]$^+$.

**Beispiel 203**

1-(2-Methoxyethyl)-5-methyl-3-(3-methylbut-2-en-1-yl)-6-[(2-oxoimidazolidin-1-yl)methyl]thieno-[2,3-d]pyrimidin-2,4(1*H*,3*H*)-dion

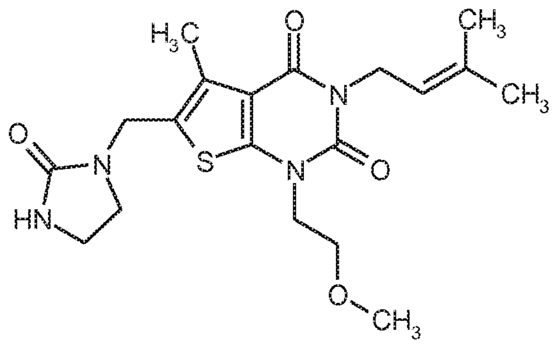

Eine Lösung von 190 mg (2.12 mmol) 2-Imidazolidinon in 7.6 ml THF wurde mit 85 mg (2.12 mmol) Natriumhydrid (60% Suspension in Mineralöl) versetzt und 3 h bei 60°C gerührt ("Lösung 1"). In einem anderen Reaktionsgefäß wurde eine Lösung von 189 mg (0.531 mmol) der Verbindung aus Bsp. 149A in 3.7 ml Dichlormethan bei 0°C mit 277 µl (1.59 mmol) *N,N*-Diisopropylethylamin und 58 µl (0.796 mmol) Thionylchlorid versetzt und 75 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 99 mg (45% d. Th.) der Titelveibindung erhalten.
$^1$H-NMR (400 MHz, DMSO-$d_6$, δ/ppm): 6.54 (s, 1H), 5.14 (ddd, 1H), 4.44 (d, 2H), 4.34 (s, 2H), 4.01 (t, 2H), 3.62 (t, 2H), 3.27-3.17 (m, 7H), 2.38 (s, 3H), 1.75 (d, 3H), 1.66 (d, 3H).
LC/MS (Methode 3, ESIpos): $R_t$ = 1.08 min, m/z = 407 [M+H]$^+$.

**Beispiel 204**

5-Methyl-6-[(2-oxoimidazolidin-1-yl)methyl]-3-(2,2,2-trifluorethyl)-1-(3,3,3-trifluorpropyl)thieno-[2,3-d]pyrimidin-2,4(1*H*,3*H*)-dion

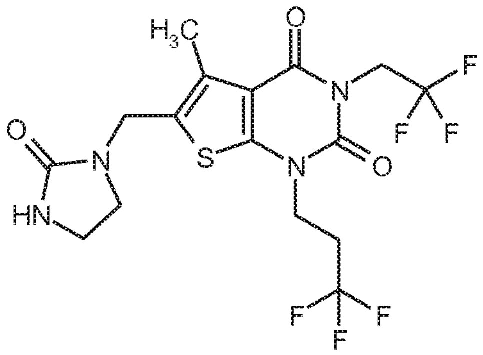

200 mg (0.43 mmol) der Verbindung aus Bsp. 247A wurden in 15 ml Dioxan gelöst und mit 108 mg (0.645 mmol) CDI versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 125 mg (63% d. Th.) der Titelverbindung erhalten.
$^1$H-NMR (400 MHz, DMSO-$d_6$, δ/ppm): 6.58 (s, 1H), 4.70 (q, 2H), 4.38 (s, 2H), 4.13 (t, 2H), 3.30-3.18 (m, 4H), 2.84-2.70 (m, 2H), 2.40 (s, 3H).
LC/MS (Methode 3, ESIpos): $R_t$ = 1.10 min, m/z = 459 [M+H]$^+$.

**Beispiel 205**

1-(2-Ethoxyethyl)-5-methyl-6-[(2-oxoimidazolidin-1-yl)methyl]-3-(2,2,2-trifluorethyl)thieno-[2,3-d]pyrimidin-2,4(1*H*,3*H*)-dion

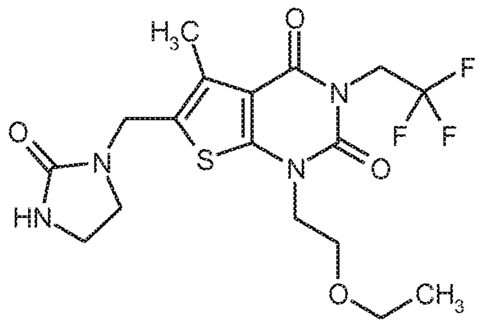

113 mg (0.177 mmol) der Verbindung aus Bsp. 250A wurden in 10 ml Dioxan gelöst und mit 44 mg (0.266 mmol) CDI versetzt. Das Gemisch wurde 18 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 58 mg (75% d. Th.) der Titelverbindung erhalten.
$^1$H-NMR (400 MHz, DMSO-$d_6$, δ/ppm): 6.56 (s, 1H), 4.69 (q, 2H), 4.36 (s, 2H), 4.04 (t, 2H), 3.66 (t, 2H), 3.43 (q, 2H), 3.28-3.17 (m, 4H), 2.39 (s, 3H), 1.02 (t, 3H).
LC/MS (Methode 3, ESIpos): $R_t$ = 1.04 min, m/z = 435 [M+H]$^+$.

**Beispiel 206**

5-Methyl-1-(oxetan-2-ylmethyl)-6-[(2-oxoimidazolidin-1-yl)methyl]-3-(2,2,2-trifluorethyl)thieno-[2,3-d]pyrimidin-2,4(1*H*,3*H*)-dion (*Racemat*)

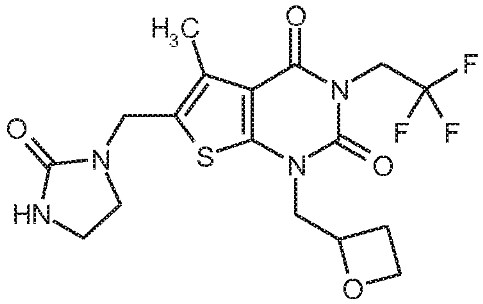

520 mg (1.13 mmol) der Verbindung aus Bsp. 251A wurden in 25 ml Dioxan gelöst und mit 282 mg (1.69 mmol) CDI versetzt. Das Gemisch wurde 18 h bei RT gerührt. Die Reaktionslösung wurde danach am Rotationsverdampfer eingeengt. Der Rückstand wurde in 12 ml DMSO gelöst und diese Lösung mittels präparativer HPLC gereinigt (Methode 14). Nach Vereinigen der Produktfraktionen und Gefriertrocknung wurden 286 mg (58% d. Th.) der Titelverbindung erhalten.

$^1$H-NMR (400 MHz, DMSO-$d_6$, $\delta$/ppm): 6.56 (s, 1H), 5.05-4.96 (m, 1H), 4.70 (q, 2H), 4.51-4.38 (m, 2H), 4.36 (s, 2H), 4.22-4.10 (m, 2H), 3.29-3.18 (m, 4H), 2.74-2.64 (m, 1H), 2.39 (s, 3H).

LC/MS (Methode 3, ESIpos): $R_t$ = 0.95 min, m/z = 433 $[M+H]^+$.

**Beispiel 207**

5-Methyl-1-(oxetan-2-ylmethyl)-6-[(2-oxoimidazolidin-1-yl)methyl]-3-(2,2,2-trifluorethyl)thieno-[2,3-d]pyrimidin-2,4(1*H*,3*H*)-dion (*Enantiomer 1*)

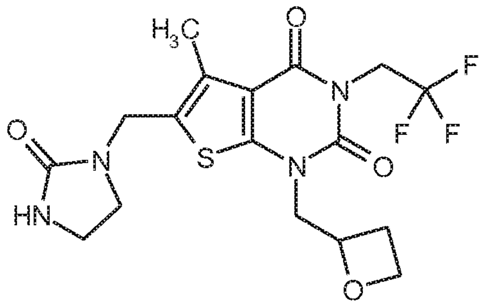

253 mg der racemischen Verbindung aus Bsp. 206 wurden in 7 ml eines Methanol/Acetonitril-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent: Ethanol/Methanol/Diethylamin 50:50:0.1 v/v; Fluss: 30 ml/min; Temperatur: RT; Detektion: 254 nm]. Die Produktfraktionen wurden am Rotationsverdampfer eingeengt, mit *tert*.-Butanol versetzt und gefriergetrocknet. Es wurden 100 mg (39% d. Th.) der Titelverbindung (Enantiomer 1) sowie 120 mg (47% d. Th.) des Enantiomers 2 (siehe Beispiel 208) erhalten.

$^1$H-NMR (400 MHz, DMSO-$d_6$, $\delta$/ppm): 6.56 (s, 1H), 5.05-4.96 (m, 1H), 4.70 (q, 2H), 4.51-4.45 (m, 1H), 4.41 (dt, 1H), 4.35 (s, 2H), 4.19-4.14 (m, 2H), 3.28-3.19 (m, 4H), 2.69 (dtd, 1H), 2.39 (s, 3H).

Chirale analytische HPLC [Säule: Daicel Chiralpak IC, 5 μm, 100 mm x 4.6 mm; Eluent: Methanol + 0.1% Diethylamin/Ethanol 50:50; Fluss: 1.0 ml/min; Temperatur: RT; Injektion: 5 μl; DAD 254 nm; Lösung: 1.0 mg/ml Methanol]: $R_t$ = 7.94 min.

**Beispiel 208**

5-Methyl-1-(oxetan-2-ylmethyl)-6-[(2-oxoimidazolidin-1-yl)methyl]-3-(2,2,2-trifluorethyl)thieno-[2,3-d]pyrimidin-2,4(1*H*,3*H*)-dion (*Enantiomer 2*)

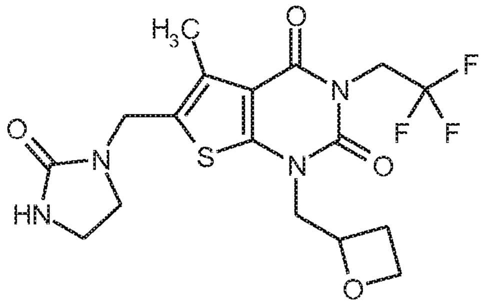

Die Titelverbindung (120 mg) wurde als zweites Enantiomer bei der unter Bsp. 207 beschriebenen präparativen HPLC-Trennung des Racemats aus Bsp. 206 erhalten.
$^1$H-NMR (400 MHz, DMSO-$d_6$, δ/ppm): 6.56 (s, 1H), 5.05-4.96 (m, 1H), 4.70 (q, 2H), 4.51-4.45 (m, 1H), 4.41 (dt, 1H), 4.35 (s, 2H), 4.21-4.11 (m, 2H), 3.29-3.17 (m, 4H), 2.69 (dtd, 1H), 2.39 (s, 3H).
Chirale analytische HPLC [Säule: Daicel Chiralpak IC, 5 μm, 100 mm x 4.6 mm; Eluent: Methanol + 0.1% Diethylamin/Ethanol 50:50; Fluss: 1.0 ml/min; Temperatur: RT; Injektion: 5 μl; DAD 254 nm; Lösung: 1.0 mg/ml Methanol]: $R_t$ = 9.15 min.

**Beispiel 209**

5-Methyl-6-[(2-oxoimidazolidin-1-yl)methyl]-1-(tetrahydrofuran-2-ylmethyl)-3-(2,2,2-trifluorethyl)thieno[2,3-d]pyrimidin-2,4(1*H*,3*H*)-dion (*Racemat*)

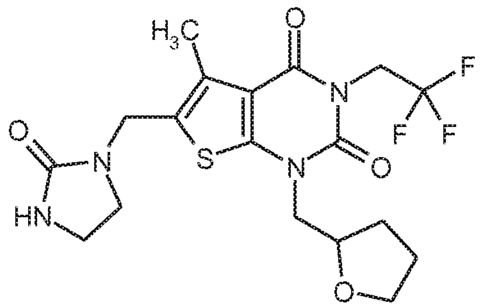

570 mg (1.1 mmol) der Verbindung aus Bsp. 252A wurden in 25 ml Dioxan gelöst und mit 275 mg (1.65 mmol) CDI versetzt. Das Gemisch wurde 17 h bei RT gerührt. Die Reaktionslösung wurde danach am Rotationsverdampfer eingeengt. Der Rückstand wurde in 12 ml DMSO gelöst und diese Lösung mittels präparativer HPLC gereinigt (Methode 14). Nach Vereinigen der Produktfraktionen und Gefriertrocknung wurden 232 mg (71% d. Th.) der Titelverbindung erhalten.
$^1$H-NMR (400 MHz, DMSO-$d_6$, δ/ppm): 6.56 (s, 1H), 4.70 (q, 2H), 4.36 (s, 2H), 4.26-4.18 (m, 1H), 4.05 (dd, 1H), 3.79-3.70 (m, 2H), 3.65-3.57 (m, 1H), 3.29-3.17 (m, 4H), 2.39 (s, 3H), 2.04-1.94 (m, 1H), 1.94-1.75 (m, 2H), 1.71-1.61 (m, 1H).
LC/MS (Methode 3, ESIpos): $R_t$ = 1.02 min, m/z = 447 [M+H]$^+$.

**Beispiel 210**

5-Methyl-6-[(2-oxoimidazolidin-1-yl)methyl]-1-(tetrahydrofuran-2-ylmethyl)-3-(2,2,2-trifluorethyl)thieno[2,3-d]pyrimidin-2,4(1*H*,3*H*)-dion (*Enantiomer 1*)

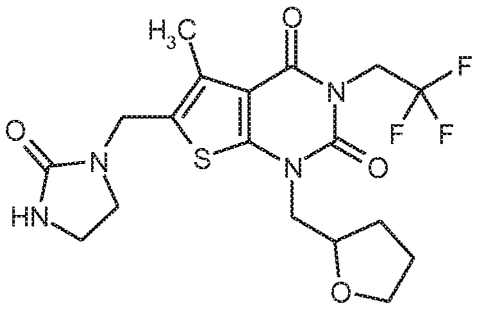

253 mg der racemischen Verbindung aus Bsp. 209 wurden in 6 ml eines Methanol/Acetonitril-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent: Ethanol/Methanol/Diethylamin 50:50:0.1 v/v; Fluss: 30 ml/min; Temperatur: RT; Detektion: 254 nm]. Die Produktfraktionen wurden am Rotationsverdampfer eingeengt, mit *tert*.-Butanol versetzt und gefriergetrocknet. Es wurden 100 mg (39% d. Th.) der Titelverbindung (Enantiomer 1) sowie 100 mg (39% d. Th.) des Enantiomers 2 (siehe Beispiel 211) erhalten.
$^1$H-NMR (400 MHz, DMSO-$d_6$, δ/ppm): 6.56 (s, 1H), 4.70 (q, 2H), 4.36 (s, 2H), 4.27-4.17 (m, 1H), 4.05 (dd, 1H), 3.78-3.70 (m, 2H), 3.61 (td, 1H), 3.29-3.18 (m, 4H), 2.39 (s, 3H), 2.03-1.93 (m, 1H), 1.93-1.75 (m, 2H), 1.71-1.61 (m, 1H).
Chirale analytische HPLC [Säule: Daicel Chiralpak IC, 5 μm, 100 mm x 4.6 mm; Eluent: Methanol + 0.1% Diethylamin/Ethanol 50:50; Fluss: 1.0 ml/min; Temperatur: RT; Injektion: 5 μl; DAD 254 nm; Lösung: 1.0 mg/ml Methanol]: $R_t$ = 6.04 min.

**Beispiel 211**

5-Methyl-6-[(2-oxoimidazolidin-1-yl)methyl]-1-(tetrahydrofuran-2-ylmethyl)-3-(2,2,2-trifluorethyl)thieno[2,3-d]pyrimidin-2,4(1*H*,3*H*)-dion (*Enantiomer 2*)

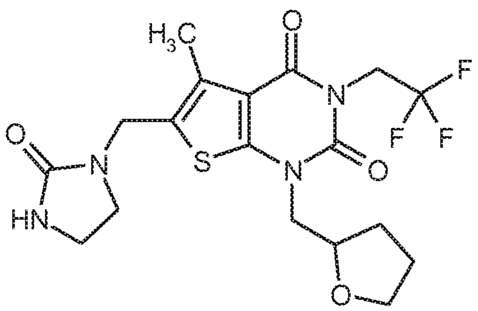

Die Titelverbindung (100 mg) wurde als zweites Enantiomer bei der unter Bsp. 210 beschriebenen präparativen HPLC-Trennung des Racemats aus Bsp. 209 erhalten.

$^1$H-NMR (400 MHz, DMSO-$d_6$, δ/ppm): 6.56 (s, 1H), 4.70 (q, 2H), 4.36 (s, 2H), 4.26-4.18 (m, 1H), 4.05 (dd, 1H), 3.74 (dd, 2H), 3.61 (td, 1H), 3.28-3.18 (m, 4H), 2.39 (s, 3H), 2.03-1.93 (m, 1H), 1.93-1.76 (m, 2H), 1.71-1.61 (m, 1H).

Chirale analytische HPLC [Säule: Daicel Chiralpak IC, 5 μm, 100 mm x 4.6 mm; Eluent: Methanol + 0.1% Diethylamin/Ethanol 50:50; Fluss: 1.0 ml/min; Temperatur: RT; Injektion: 5 μl; DAD 254 nm; Lösung: 1.0 mg/ml Methanol]: $R_t$ = 8.09 min.

## Beispiel 212

5-Methyl-1-(oxetan-3-ylmethyl)-6-[(2-oxoimidazolidin-1-yl)methyl]-3-(2,2,2-trifluorethyl)thieno-[2,3-d]pyrimidin-2,4(1*H*,3*H*)-dion

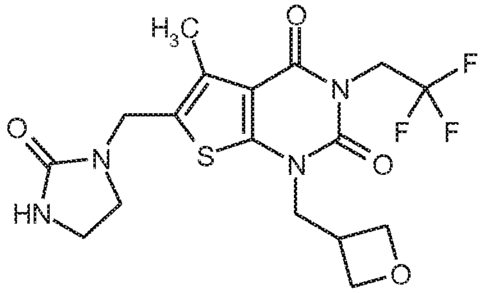

109 mg (0.091 mmol) der Verbindung aus Bsp. 253A wurden in 10 ml Dioxan gelöst und mit 23 mg (0.137 mmol) CDI versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 64 mg (39% d. Th.) der Titelverbindung erhalten.
$^1$H-NMR (400 MHz, DMSO-$d_6$, δ/ppm): 6.57 (s, 1H), 4.68 (q, 2H), 4.61 (dd, 2H), 4.43 (t, 2H), 4.37 (s, 2H), 4.23 (d, 2H), 3.48-3.37 (m, 1H), 3.30-3.17 (m, 4H), 2.39 (s, 3H).
LC/MS (Methode 3, ESIpos): $R_t$ = 0.94 min, m/z = 433 $[M+H]^+$.

## Beispiel 213

1,5-Dimethyl-6-[(2-oxoimidazolidin-1-yl)methyl]-3-(2,2,2-trifluorethyl)thieno[2,3-d]pyrimidin-2,4(1*H*,3*H*)-dion

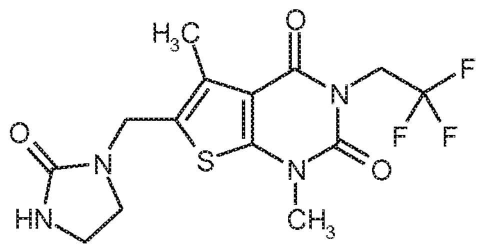

190 mg (0.239 mmol) der Verbindung aus Bsp. 254A wurden in 15 ml Dioxan gelöst und mit 60 mg (0.358 mmol) CDI versetzt. Das Gemisch wurde 20 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 48 mg (52% d. Th.) der Titelverbindung erhalten.
$^1$H-NMR (400 MHz, DMSO-$d_6$, δ/ppm): 6.56 (s, 1H), 4.69 (q, 2H), 4.37 (s, 2H), 3.44 (s, 3H), 3.28-3.18 (m, 4H), 2.40 (s, 3H).

LC/MS (Methode 3, ESIpos): $R_t$ = 0.93 min, m/z = 377 $[M+H]^+$.

**Beispiel 214**

3-(2,2-Difluorethyl)-5-methyl-6-[(2-oxoimidazolidin-1-yl)methyl]-1-(3,3,3-trifluorpropyl)thieno-[2,3-d]pyrimidin-2,4(1*H*,3*H*)-dion

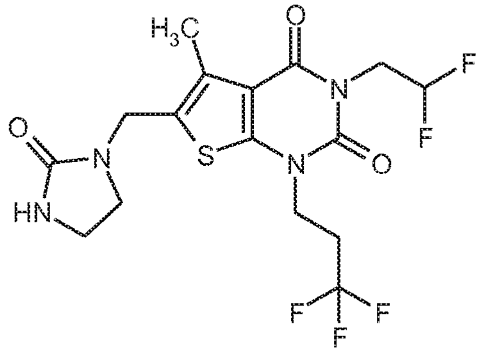

150 mg (0.257 mmol) der Verbindung aus Bsp. 255A wurden in 10 ml Dioxan gelöst und mit 64 mg (0.385 mmol) CDI versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 92 mg (81% d. Th.) der Titelverbindung erhalten.
$^1$H-NMR (400 MHz, DMSO-$d_6$, δ/ppm): 6.58 (s, 1H), 6.37-6.04 (m, 1H), 4.38 (s, 2H), 4.29 (td, 2H), 4.11 (t, 2H), 3.29-3.18 (m, 4H), 2.84-2.70 (m, 2H), 2.40 (s, 3H).
LC/MS (Methode 3, ESIpos): $R_t$ = 1.04 min, m/z = 441 $[M+H]^+$.

**Beispiel 215**

3-(2,2-Difluorethyl)-1-(2-ethoxyethyl)-5-methyl-6-[(2-oxoimidazolidin-1-yl)methyl]thieno[2,3-d]-pyrimidin-2,4(1*H*,3*H*)-dion

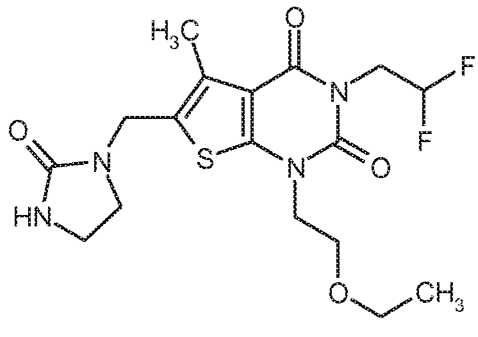

120 mg (0.231 mmol) der Verbindung aus Bsp. 258A wurden in 10 ml Dioxan gelöst und mit 58 mg (0.346 mmol) CDI versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 55 mg (56% d. Th.) der Titelverbindung erhalten.
$^1$H-NMR (400 MHz, DMSO-$d_6$, δ/ppm): 6.56 (s, 1H), 6.38-6.05 (m, 1H), 4.36 (s, 2H), 4.29 (td, 2H), 4.03 (t, 2H), 3.66 (t, 2H), 3.43 (q, 2H), 3.28-3.16 (m, 4H), 2.39 (s, 3H), 1.03 (t, 3H).
LC/MS (Methode 3, ESIpos): $R_t$ = 0.99 min, m/z = 417 $[M+H]^+$.

**Beispiel 216**

3-(2,2-Difluorethyl)-5-methyl-1-(oxetan-2-ylmethyl)-6-[(2-oxoimidazolidin-1-yl)methyl]thieno-[2,3-d]pyrimidin-2,4(1*H*,3*H*)-dion (*Racemat*)

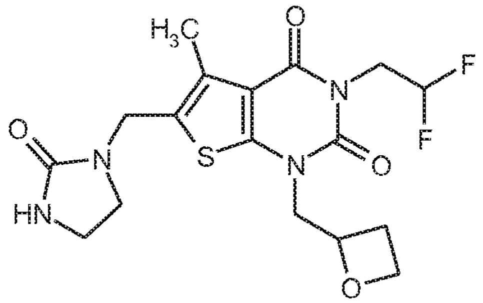

446 mg (0.907 mmol) der Verbindung aus Bsp. 259A wurden in 16 ml Dioxan gelöst und mit 227 mg (1.36 mmol) CDI versetzt. Das Gemisch wurde 17 h bei RT gerührt. Die Reaktionslösung wurde danach am Rotationsverdampfer eingeengt. Der Rückstand wurde in 9 ml DMSO gelöst und diese Lösung mittels präparativer HPLC gereinigt (Methode 14). Nach Vereinigen der Produktfraktionen und Gefriertrocknung wurden 210 mg (55% d. Th.) der Titelverbindung erhalten.
$^1$H-NMR (400 MHz, DMSO-$d_6$, $\delta$/ppm): 6.55 (s, 1H), 6.37-6.06 (m, 1H), 5.05-4.97 (m, 1H), 4.51-4.38 (m, 2H), 4.35 (s, 2H), 4.29 (td, 2H), 4.15 (d, 2H), 3.28-3.17 (m, 4H), 2.74-2.64 (m, 1H), 2.39 (s, 3H).
LC/MS (Methode 3, ESIpos): $R_t$ = 0.88 min, m/z = 415 $[M+H]^+$.

**Beispiel 217**

3-(2,2-Difluorethyl)-5-methyl-1-(oxetan-2-ylmethyl)-6-[(2-oxoimidazolidin-1-yl)methyl]thieno-[2,3-d]pyrimidin-2,4(1*H*,3*H*)-dion (*Enantiomer 1*)

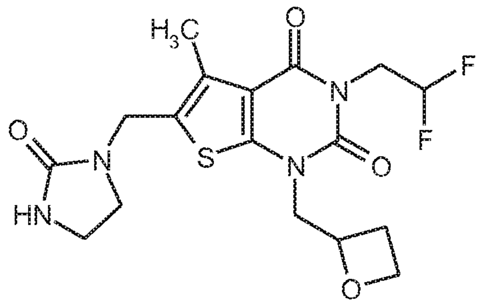

179 mg der racemischen Verbindung aus Bsp. 216 wurden in 6.5 ml DMSO gelöst und mittels präparativer HPLC an chiraler Phase in die Enantiomere getrennt [Säule: Daicel Chiralpak IB, 5 µm, 250 mm x 20 mm; Eluent A: Hexan, Eluent B: Ethanol; isokratisch 58% A, 42% B; Fluss: 15 ml/ min; Temperatur: RT; Detektion: 254 nm]. Die Produktfraktionen wurden am Rotationsverdampfer eingeengt, mit *tert*.-Butanol versetzt und gefriergetrocknet. Es wurden 75 mg (41% d. Th.) der Titelverbindung (Enantiomer 1) sowie 58 mg (31% d. Th.) des Enantiomers 2 (siehe Beispiel 218) erhalten.
$^1$H-NMR (400 MHz, DMSO-$d_6$, $\delta$/ppm): 6.55 (s, 1H), 6.37-6.04 (m, 1H), 5.05-4.96 (m, 1H), 4.51-4.38 (m, 2H), 4.35 (s, 2H), 4.29 (td, 2H), 4.15 (d, 2H), 3.28-3.17 (m, 4H), 2.69 (dtd, 1H), 2.39 (s, 3H).

Chirale analytische HPLC [Säule: Daicel Chiralpak IB, 3 µm, 100 mm x 4.6 mm; Eluent A: Hexan, Eluent B: Ethanol; isokratisch 58% A, 42% B; Fluss: 1.0 ml/min; Temperatur: 25°C; Injektion: 5 µl; DAD 254 nm]: $R_t$ = 4.63 min.

**Beispiel 218**

3-(2,2-Difluorethyl)-5-methyl-1-(oxetan-2-ylmethyl)-6-[(2-oxoimidazolidin-1-yl)methyl]thieno-[2,3-d]pyrimidin-2,4(1*H*,3*H*)-dion (*Enantiomer 2*)

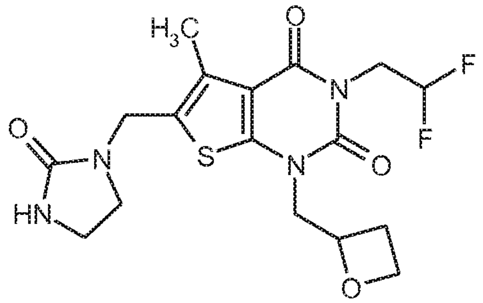

Die Titelverbindung (58 mg) wurde als zweites Enantiomer bei der unter Bsp. 217 beschriebenen präparativen HPLC-Trennung des Racemats aus Bsp. 216 erhalten.
$^1$H-NMR (400 MHz, DMSO-$d_6$, δ/ppm): 6.55 (s, 1H), 6.38-6.06 (m, 1H), 5.05-4.96 (m, 1H), 4.52-4.38 (m, 2H), 4.35 (s, 2H), 4.29 (td, 2H), 4.15 (d, 2H), 3.28-3.18 (m, 4H), 2.69 (dtd, 1H), 2.39 (s, 3H).
Chirale analytische HPLC [Säule: Daicel Chiralpak IB, 3 μm, 100 mm x 4.6 mm; Eluent A: Hexan, Eluent B: Ethanol; isokratisch 58% A, 42% B; Fluss: 1.0 ml/min; Temperatur: 25°C; Injektion: 5 μl; DAD 254 nm]: $R_t$ = 5.70 min.

**Beispiel 219**

3-(2,2-Difluorethyl)-5-methyl-6-[(2-oxoimidazolidin-1-yl)methyl]-1-(tetrahydrofuran-2-ylmethyl)-thieno[2,3-d]pyrimidin-2,4(1*H*,3*H*)-dion (*Racemat*)

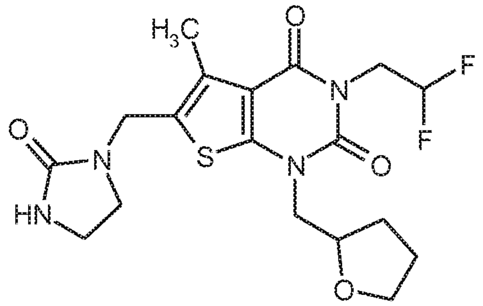

444 mg (0.695 mmol) der Verbindung aus Bsp. 260A wurden in 15 ml Dioxan gelöst und mit 174 mg (1.04 mmol) CDI versetzt. Das Gemisch wurde 18 h bei RT gerührt. Die Reaktionslösung wurde danach am Rotationsverdampfer eingeengt. Der Rückstand wurde in 9 ml DMSO gelöst und diese Lösung mittels präparativer HPLC gereinigt (Methode 14). Nach Vereinigen der Produktfraktionen und Gefriertrocknung wurden 156 mg (52% d. Th.) der Titelverbindung erhalten.
$^1$H-NMR (400 MHz, DMSO-$d_6$, δ/ppm): 6.55 (s, 1H), 6.38-6.04 (m, 1H), 4.35 (s, 2H), 4.30 (td, 2H), 4.22 (td, 1H), 4.05 (dd, 1H), 3.78-3.68 (m, 2H), 3.65-3.58 (m, 1H), 3.28-3.17 (m, 4H), 2.39 (s, 3H), 2.04-1.94 (m, 1H), 1.93-1.77 (m, 2H), 1.71-1.61 (m, 1H).
LC/MS (Methode 3, ESIpos): $R_t$ = 0.95 min, m/z = 429 $[M+H]^+$.

**Beispiel 220**

3-(2,2-Difluorethyl)-5-methyl-6-[(2-oxoimidazolidin-1-yl)methyl]-1-(tetrahydrofuran-2-ylmethyl)-thieno[2,3-d]pyrimidin-2,4(1*H*,3*H*)-dion (*Enantiomer 1*)

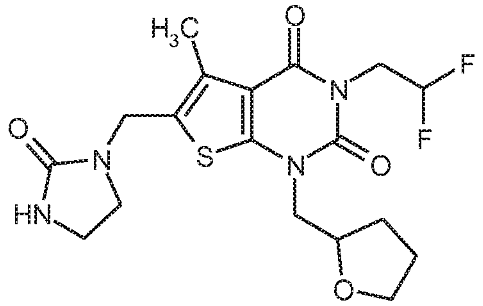

135 mg der racemischen Verbindung aus Bsp. 219 wurden in 3 ml eines Dichlormethan/Methanol-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent: Ethanol/Methanol/Diethylamin 50:50:0.1 v/v; Fluss: 30 ml/min; Temperatur: RT; Detektion: 254 nm]. Die Produktfraktionen wurden am Rotationsverdampfer eingeengt, mit *tert.*-Butanol versetzt und gefriergetrocknet. Es wurden 60 mg (43% d. Th.) der Titelverbindung (Enantiomer 1) sowie 60 mg (43% d. Th.) des Enantiomers 2 (siehe Beispiel 221) erhalten.
$^1$H-NMR (400 MHz, DMSO-$d_6$, δ/ppm): 6.57 (s, 1H), 6.38-6.06 (m, 1H), 4.35 (s, 2H), 4.29 (td, 2H), 4.24-4.18 (m, 1H), 4.05 (dd, 1H), 3.78-3.67 (m, 2H), 3.65-3.57 (m, 1H), 3.27-3.16 (m, 4H), 2.39 (s, 3H), 2.03-1.93 (m, 1H), 1.93-1.75 (m, 2H), 1.71-1.60 (m, 1H).

Chirale analytische HPLC [Säule: Daicel Chiralpak IC, 5 μm, 100 mm x 4.6 mm; Eluent: Methanol + 0.1% Diethylamin/Ethanol 50:50; Fluss: 1.0 ml/min; Temperatur: RT; Injektion: 5 μl; DAD 254 nm; Lösung: 1.0 mg/ml Methanol]: $R_t$ = 9.31 min.

**Beispiel 221**

3-(2,2-Difluorethyl)-5-methyl-6-[(2-oxoimidazolidin-1-yl)methyl]-1-(tetrahydrofuran-2-ylmethyl)-thieno[2,3-d]pyrimidin-2,4(1*H*,3*H*)-dion (*Enantiomer 2*)

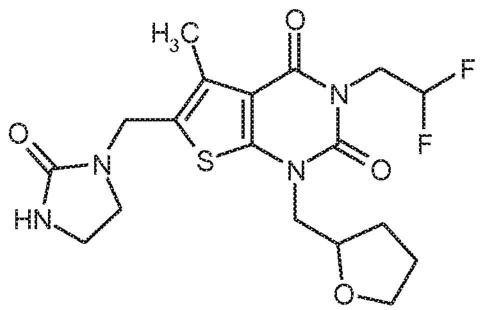

Die Titelverbindung (60 mg) wurde als zweites Enantiomer bei der unter Bsp. 220 beschriebenen präparativen HPLC-Trennung des Racemats aus Bsp. 219 erhalten.
$^1$H-NMR (400 MHz, DMSO-$d_6$, δ/ppm): 6.55 (s, 1H), 6.38-6.05 (m, 1H), 4.35 (s, 2H), 4.29 (td, 2H), 4.25-4.18 (m, 1H), 4.05 (dd, 1H), 3.78-3.68 (m, 2H), 3.65-3.57 (m, 1H), 3.28-3.17 (m, 4H), 2.39 (s, 3H), 2.03-1.93 (m, 1H), 1.93-1.75 (m, 2H), 1.71-1.61 (m, 1H).

Chirale analytische HPLC [Säule: Daicel Chiralpak IC, 5 μm, 100 mm x 4.6 mm; Eluent: Methanol + 0.1% Diethylamin/Ethanol 50:50; Fluss: 1.0 ml/min; Temperatur: RT; Injektion: 5 μl; DAD 254 nm; Lösung: 1.0 mg/ml Methanol]: $R_t$ = 12.96 min.

**Beispiel 222**

3-(2,2-Difluorethyl)-5-methyl-1-(oxetan-3-ylmethyl)-6-[(2-oxoimidazolidin-1-yl)methyl]thieno-[2,3-d]pyrimidin-2,4(1*H*,3*H*)-dion

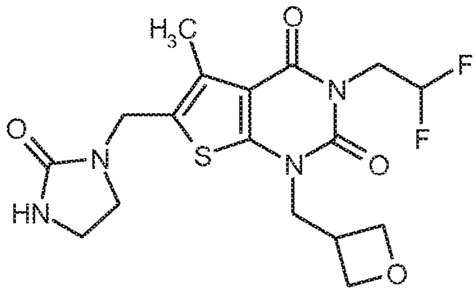

85 mg (0.147 mmol) der Verbindung aus Bsp. 261A wurden in 10ml Dioxan gelöst und mit 37 mg (1.36 mmol) CDI versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 55 mg (60% d. Th.) der Titelverbindung erhalten.
$^1$H-NMR (400 MHz, DMSO-$d_6$, δ/ppm): 6.57 (s, 1H), 6.35-6.02 (m, 1H), 4.62 (dd, 2H), 4.43 (t, 2H), 4.36 (s, 2H), 4.28 (td, 2H), 4.22 (d, 2H), 3.41 (d, 1H), 3.28-3.18 (m, 4H), 2.39 (s, 3H).
LC/MS (Methode 3, ESIpos): $R_t$ = 0.86 min, m/z = 415 $[M+H]^+$.

**Beispiel 223**

3-(2,2-Difluorethyl)-1,5-dimethyl-6-[(2-oxoimidazolidin-1-yl)methyl]thieno[2,3-d]pyrimidin-2,4(1*H*,3*H*)-dion

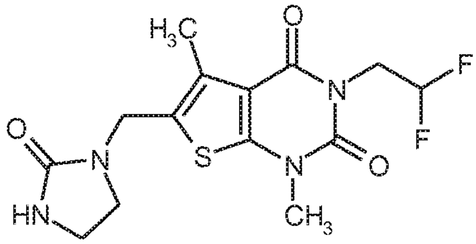

110 mg (0.232 mmol) der Verbindung aus Bsp. 318A wurden in 12 ml Dioxan gelöst und mit 58 mg (0.347 mmol) CDI versetzt. Das Gemisch wurde 13 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 53 mg (63% d. Th.) der Titelverbindung erhalten.
$^1$H-NMR (400 MHz, DMSO-$d_6$, δ/ppm): 6.54 (s, 1H), 6.37-6.04 (m, 1H), 4.37 (s, 2H), 4.29 (td, 2H), 3.43 (s, 3H), 3.28-3.18 (m, 4H), 2.40 (s, 3H).
LC/MS (Methode 3, ESIpos): $R_t$ = 0.86 min, m/z = 359 $[M+H]^+$.

**Beispiel 224**

3-(3-Fluorpropyl)-1-(2-methoxyethyl)-5-methyl-6-[(2-oxoimidazolidin-1-yl)methyl]thieno[2,3-d]-pyrimidin-2,4(1*H*,3*H*)-dion

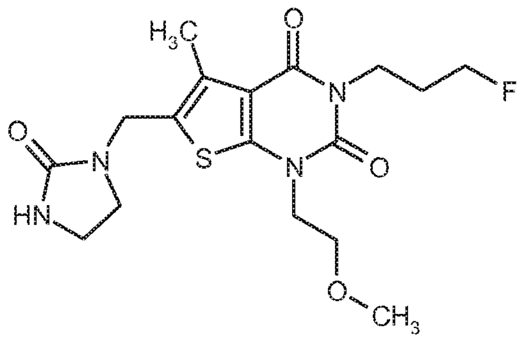

Eine Lösung von 172 mg (1.92 mmol) 2-Imidazolidinon in 7 ml THF wurde mit 77 mg (1.92 mmol) Natriumhydrid (60% Suspension in Mineralöl) versetzt und 3 h bei 60°C gerührt ("Lösung 1"). In einem anderen Reaktionsgefäß wurde eine Lösung von 160 mg (0.479 mmol) der Verbindung aus Bsp. 156A in 3.3 ml Dichlormethan bei 0°C mit 250 μl (1.44 mmol) *N,N*-Diisopropylethylamin und 52 μl (0.719 mmol) Thionylchlorid versetzt und 75 min gerührt. Anschließend wurde die Lösung 1 portionsweise zugesetzt und das Gemisch 19 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 113 mg (58% d. Th.) der Titelverbindung erhalten.

$^1$H-NMR (400 MHz, DMSO-$d_6$, δ/ppm): 6.54 (s, 1H), 4.54 (t, 1H), 4.42 (t, 1H), 4.34 (s, 2H), 4.04-3.94 (m, 4H), 3.62 (t, 2H), 3.28-3.17 (m, 7H), 2.39 (s, 3H), 2.01-1.85 (m, 2H).

LC/MS (Methode 3, ESIpos): $R_t$ = 0.88 min, m/z = 399 $[M+H]^+$.

**Beispiel 225**

3-(2-Fluor-2-methylpropyl)-5-methyl-6-[(2-oxoimidazolidin-1-yl)methyl]-1-(3,3,3-trifluorpropyl)-thieno[2,3-d]pyrimidin-2,4(1*H*,3*H*)-dion

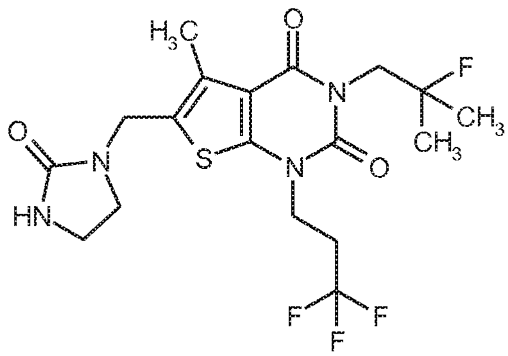

Eine Lösung von 150 mg (1.75 mmol) Imidazolidin-2-on in 4.5 ml DMF wurde mit 70 mg (1.75 mmol) Natriumhydrid (60% Suspension in Mineralöl) versetzt, dann 5 min auf 60°C erwärmt und anschließend wieder auf RT abgekühlt ("Lösung 1"). In einem anderen Reaktionsgefäß wurde eine Lösung von 180 mg (0.438 mmol) der Verbindung aus Bsp. 304A in 3.3 ml Dichlormethan bei 0°C mit 153 μl (0.876 mmol) *N,N*-Diisopropylethylamin und 34 μl (0.460 mmol) Thionylchlorid versetzt. Nach 20 min bei 0°C wurde Lösung 1 portionsweise hinzugefügt und anschließend das Kältebad entfernt. Das Reaktionsgemisch wurde ca. 18 h bei RT gerührt. Danach wurden alle flüchtigen Bestandteile am Rotationsverdampfer entfernt. Der verbliebene Rückstand wurde mittels präparativer HPLC (Methode 8) in seine Komponenten aufgetrennt. Die Produktfraktionen wurden vereinigt und eingeengt und der Rückstand in wenig Diisopropylether bei RT verrührt. Nach Absaugen des Feststoffs und Trocknen im Hochvakuum wurden 75 mg (37% d. Th.) der Titelverbindung erhalten.

$^1$H-NMR (400 MHz, DMSO-$d_6$, δ/ppm): 6.54 (s, 1H), 4.37 (s, 2H), 4.21-4.05 (m, 4H), 3.30-3.16 (m, 4H, teilweise überdeckt vom Wassersignal), 2.86-2.68 (m, 2H), 2.40 (s, 3H), 1.32 (d, 6H).

LC/MS (Methode 17, ESIpos): $R_t$ = 1.60 min, m/z = 451.14 $[M+H]^+$.

**Beispiel 226**

3-(2-Fluor-2-methylpropyl)-1-(2-methoxyethyl)-5-methyl-6-[(2-oxoimidazolidin-1-yl)methyl]-thieno[2,3-d]pyrimidin-2,4(1*H*,3*H*)-dion

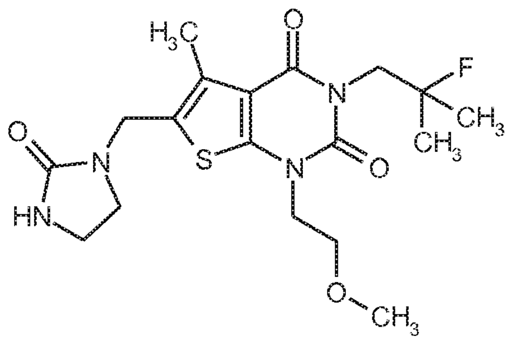

Analog zu dem unter Bsp. 225 beschriebenen Verfahren wurden aus 135 mg (0.392 mmol) der Verbindung aus Bsp. 305A und 135 mg (1.57 mmol) Imidazolidin-2-on 46 mg (28% d. Th.) der Titelverbindung erhalten.
$^1$H-NMR (400 MHz, DMSO-$d_6$, δ/ppm): 6.53 (s, 1H), 4.35 (s, 2H), 4.15 (d, 2H), 4.03 (t, 2H), 3.62 (t, 2H), 3.29-3.15 (m, 4H, teilweise überdeckt vom Wassersignal), 3.23 (s, 3H), 2.38 (s, 3H), 1.31 (d, 6H).
LC/MS (Methode 1, ESIpos): Rt = 0.74 min, m/z = 413 $[M+H]^+$.

**Beispiel 227**

3-(2-Hydroxy-2-methylpropyl)-5-methyl-6-[(2-oxoimidazolidin-1-yl)methyl]-1-(3,3,3-trifluor-propyl)thieno[2,3-d]pyrimidin-2,4(1*H*,3*H*)-dion

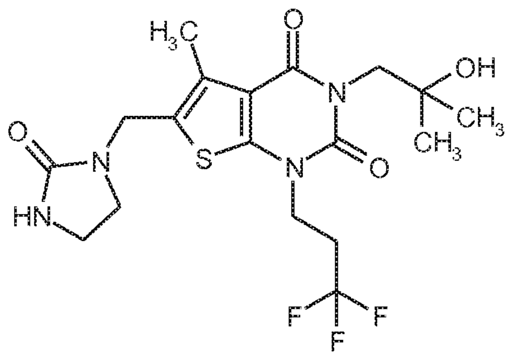

Eine Lösung von 70 mg (0.151 mmol) der Verbindung aus Bsp. 355A in 2 ml THF wurde mit 50 μl (0.150 mmol) einer 3 M Lösung von Methylmagnesiumchlorid in THF versetzt. Nach 30 min Rühren bei RT wurden weitere 100 μl (0.300 mmol) der Methylmagnesiumchlorid-Lösung hinzugefügt. Nach weiteren 45 min bei RT wurde das Reaktionsgemisch mit gesättigter wässriger Ammoniumchlorid-Lösung versetzt und mit Ethylacetat extrahiert. Der organische Extrakt wurde über wasserfreiem Magnesiumsulfat getrocknet, filtriert und eingeengt. Der verbliebene, stark verunreinigte Rückstand wurde zweimal nacheinander mittels präparativer HPLC (jeweils Methode 8) gereinigt. Das so erhaltene, noch leicht verunreinigte Produkt wurde dann noch ein drittes Mal mittels präparativer HPLC gereinigt [Säule: Kinetex C18, 5 μm, 100 mm x 30 mm; Eluent A: Wasser + 0.07% Ameisensäure; Eluent B: Acetonitril; Gradient: 0.0-2.2 min 10% B, 2.2-7.0 min 20% B, 7.0-7.5 min 60% B, 7.5-9.0 min 92% B; Fluss: 70 ml/min; Temperatur: 25°C; Detektion: 210 nm]. Nach Eindampfen der Produktfraktionen und Trocknen im Hochvakuum wurden 5 mg (7% d. Th.) der Titelverbindung erhalten.
$^1$H-NMR (400 MHz, $CDCl_3$, δ/ppm): 4.47 (s, 2H), 4.39 (br. s, 1H), 4.23-4.12 (m, 4H), 3.64 (s, 1H), 3.50-3.33 (m, 4H), 2.72-2.53 (m, 2H), 2.48 (s, 3H), 1.26 (s, 6H).

LC/MS (Methode 1, ESIpos): $R_t$ = 0.76 min, m/z = 449 $[M+H]^+$.

**Beispiel 228**

3-(2-Hydroxy-2-methylpropyl)-1-(2-methoxyethyl)-5-methyl-6-[(2-oxoimidazolidin-1-yl)methyl]-thieno[2,3-d]pyrimidin-2,4(1*H*,3*H*)-dion

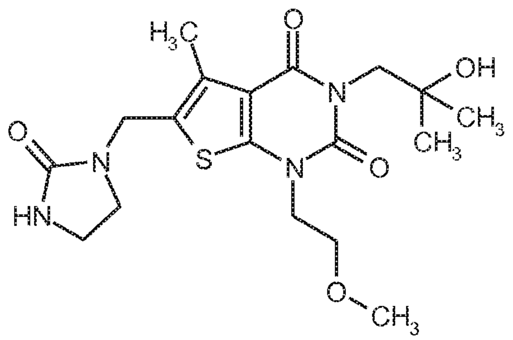

Eine Lösung von 137 mg (0.323 mmol) der Verbindung aus Bsp. 356A in 5 ml THF wurde bei -78°C mit 333 µl (1.0 mmol) einer 3 M Lösung von Methylmagnesiumchlorid in THF versetzt. Nach 60 min Rühren bei -78°C wurde das Kältebad entfernt und das Rühren bei RT fortgesetzt. Nach weiteren 60 min wurde das Reaktionsgemisch mit wenig gesättigter wässriger Ammoniumchlorid-Lösung versetzt und mit Ethylacetat verdünnt. Es wurde wasserfreies Magnesiumsulfat hinzugegeben, einige Minuten gerührt, dann filtriert und eingeengt. Der verbliebene, stark verunreinigte Rückstand wurde zweimal nacheinander mittels präparativer HPLC (jeweils Methode 8) gereinigt. Das so erhaltene, noch leicht verunreinigte Produkt wurde dann noch ein drittes Mal mittels präparativer HPLC gereinigt [Säule: Kinetex C18, 5 µm, 100 mm x 30 mm; Eluent A: Wasser + 0.07% Ameisensäure; Eluent B: Acetonitril; Gradient: 0.0-2.2 min 10% B, 2.2-7.0 min 20% B, 7.0-7.5 min 60% B, 7.5-9.0 min 92% B; Fluss: 70 ml/min; Temperatur: 25°C; Detektion: 210 nm]. Nach Eindampfen der Produktfraktionen und Trocknen im Hochvakuum wurden 3 mg (2% d. Th.) der Titelverbindung erhalten.

$^1H$-NMR (400 MHz, $CDCl_3$, δ/ppm): 4.46 (s, 2H), 4.37 (br. s, 1H), 4.18 (s, 2H), 4.12 (t, 2H), 3.90 (s, 1H), 3.71 (t, 2H), 3.47-3.35 (m, 4H), 3.33 (s, 3H), 2.47 (s, 3H), 1.26 (s, 6H).

LC/MS (Methode 1, ESIpos): Rt = 0.68 min, m/z = 441 $[M+H]^+$.

**Beispiel 229**

3-(2-Methoxyethyl)-5-methyl-6-[(2-oxoimidazolidin-1-yl)methyl]-1-(3,3,3-trifluorpropyl)thieno-[2,3-d]pyrimidin-2,4(1*H*,3*H*)-dion

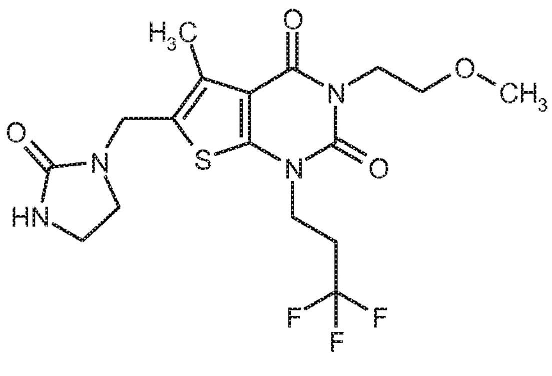

100 mg (0.198 mmol) der Verbindung aus Bsp. 319A wurden in 11 ml Dioxan gelöst und mit 50 mg (0.297 mmol) CDI versetzt. Das Gemisch wurde 18 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 66 mg (73% d. Th.) der Titelverbindung erhalten.
$^1$H-NMR (400 MHz, DMSO-$d_6$, δ/ppm): 6.55 (s, 1H), 4.37 (s, 2H), 4.10 (t, 2H), 4.05 (t, 2H), 3.49 (t, 2H), 3.29-3.18 (m, 7H), 2.82-2.69 (m, 2H), 2.40 (s, 3H).
LC/MS (Methode 3, ESIpos): $R_t$ = 0.96 min, m/z = 435 $[M+H]^+$.

**Beispiel 230**

1-(3-Fluorpropyl)-3-(2-methoxyethyl)-5-methyl-6-[(2-oxoimidazolidin-1-yl)methyl]thieno[2,3-d]-pyrimidin-2,4(1*H*,3*H*)-dion

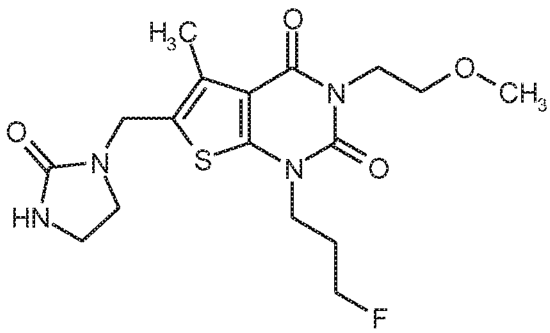

80 mg (0.137 mmol) der Verbindung aus Bsp. 320A wurden in 10 ml Dioxan gelöst und mit 34 mg (0.206 mmol) CDI versetzt. Das Gemisch wurde 18 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 43 mg (54% d. Th.) der Titelverbindung erhalten.
$^1$H-NMR (400 MHz, DMSO-$d_6$, δ/ppm): 6.55 (s, 1H), 4.59 (t, 1H), 4.47 (t, 1H), 4.36 (s, 2H), 4.05 (t, 2H), 3.97 (t, 2H), 3.49 (t, 2H), 3.30-3.16 (m, 7H), 2.39 (s, 3H), 2.13-2.05 (m, 1H), 2.05-1.97 (m, 1H).
LC/MS (Methode 3, ESIpos): $R_t$ = 0.86 min, m/z = 399 $[M+H]^+$.

**Beispiel 231**

1,3-Bis(2-methoxyethyl)-5-methyl-6-[(2-oxoimidazolidin-1-yl)methyl]thieno[2,3-d]pyrimidin-2,4(1*H*,3*H*)-dion

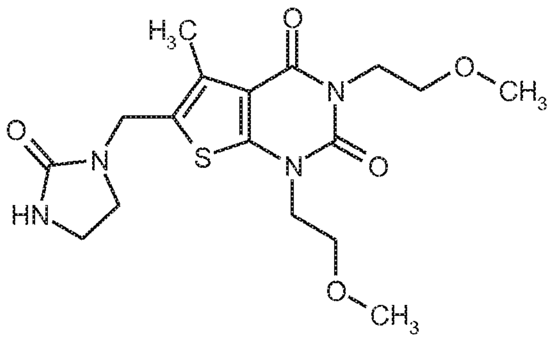

155 mg (0.18 mmol) der Verbindung aus Bsp. 262A wurden in 5 ml Dioxan gelöst und mit 45 mg (0.27 mmol) CDI versetzt. Das Gemisch wurde 16 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 60 mg (71% d. Th.) der Titelverbindung erhalten.

$^1$H-NMR (400 MHz, DMSO-$d_6$, δ/ppm): 6.55 (s, 1H), 4.34 (s, 2H), 4.05 (t, 2H), 4.01 (t, 2H), 3.62 (t, 2H), 3.49 (t, 2H), 3.28-3.17 (m, 10H), 2.38 (s, 3H).
LC/MS (Methode 3, ESIpos): $R_t$ = 0.82 min, m/z = 397 $[M+H]^+$.

**Beispiel 232**

1-(2-Ethoxyethyl)-3-(2-methoxyethyl)-5-methyl-6-[(2-oxoimidazolidin-1-yl)methyl]thieno[2,3-d]-pyrimidin-2,4(1*H*,3*H*)-dion

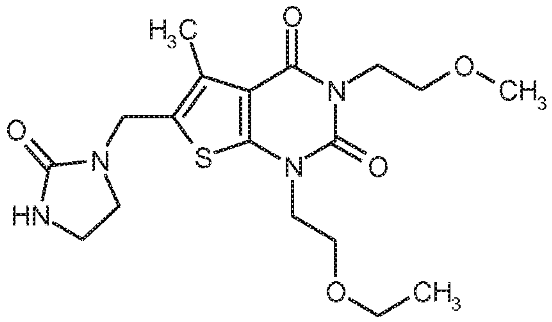

143 mg (0.216 mmol) der Verbindung aus Bsp. 263A wurden in 10 ml Dioxan gelöst und mit 54 mg (0.324 mmol) CD1 versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 59 mg (66% d. Th.) der Titelverbindung erhalten.
$^1$H-NMR (400 MHz, DMSO-$d_6$, δ/ppm): 6.54 (s, 1H), 4.35 (s, 2H), 4.05 (t, 2H), 4.00 (t, 2H), 3.65 (t, 2H), 3.49 (t, 2H), 3.43 (q, 2H), 3.27-3.17 (m, 7H), 2.38 (s, 3H), 1.03 (t, 3H).
LC/MS (Methode 3, ESIpos): $R_t$ = 0.88 min, m/z = 411 $[M+H]^+$.

**Beispiel 233**

3-(2-Methoxyethyl)-5-methyl-6-[(2-oxoimida-zolidin-1-yl)methyl]-1-(tetrahydrofuran-2-ylmethyl)-thieno[2,3-d]pyrimidin-2,4(1*H*,3*H*)-dion *(Racemat)*

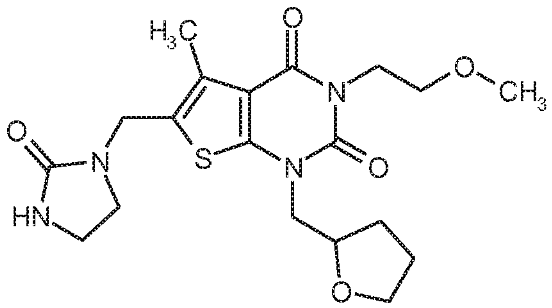

200 mg (0.333 mmol) der Verbindung aus Bsp. 321A wurden in 15 ml Dioxan gelöst und mit 84 mg (0.499 mmol) CDI versetzt. Das Gemisch wurde 18 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 109 mg (77% d. Th.) der Titelverbindung erhalten.
$^1$H-NMR (400 MHz, DMSO-$d_6$, δ/ppm): 6.54 (s, 1H), 4.34 (s, 2H), 4.26-4.17 (m, 1H), 4.09-3.99 (m, 3H), 3.78-3.65 (m, 2H), 3.64-3.57 (m, 1H), 3.49 (t, 2H), 3.28-3.16 (m, 7H), 2.38 (s, 3H), 2.03-1.75 (m, 3H), 1.70-1.60 (m, 1H).
LC/MS (Methode 3, ESIpos): $R_t$ = 0.86 min, m/z = 423 $[M+H]^+$.

**Beispiel 234**

1-(2-Methoxyethyl)-3-(2-methoxypropyl)-5-methyl-6-[(2-oxoimidazolidin-1-yl)methyl]thieno-[2,3-d]pyrimidin-2,4(1*H*,3*H*)-dion (*Enantiomer 1*)

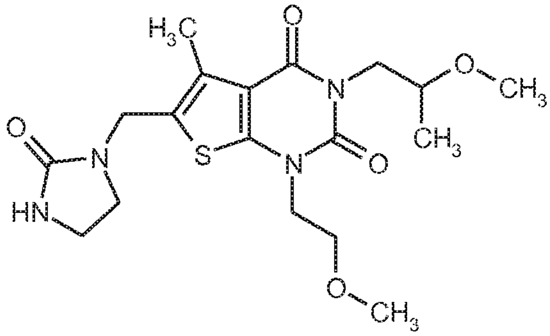

81 mg der racemischen Verbindung aus Bsp. 99 wurden in 4 ml eines Acetonitril/Dichlormethan-Gemisches (1:1 v/v) gelöst und mittels präparativer HPLC an chiraler Phase in die Enantiomere getrennt [Säule: Daicel Chiralpak IC, 5 μm, 250 mm x 30 mm; Eluent A: Acetonitril, Eluent B: Ethanol; isokratisch 90% A + 10% B; Fluss: 60 ml/min; Temperatur: RT; DAD 254 nm]. Die Produktfraktionen wurden am Rotationsverdampfer eingeengt, mit *tert.*-Butanol versetzt und gefriergetrocknet. Es wurden 35 mg (41% d. Th.) der Titelverbindung (Enantiomer 1) sowie 35 mg (41% d. Th.) des Enantiomers 2 (siehe Beispiel 235) erhalten.

$^1$H-NMR (400 MHz, DMSO-$d_6$, δ/ppm): 6.55 (s, 1H), 4.34 (s, 2H), 4.09-3.99 (m, 3H), 3.75 (dd, 1H), 3.68-3.59 (m, 3H), 3.29-3.17 (m, 10H), 2.38 (s, 3H), 1.05 (d, 3H).

Chirale analytische HPLC [Säule: Daicel Chiralpak IC, 3 μm, 100 mm x 4.6 mm; Eluent A: Acetonitril, Eluent B: Ethanol; isokratisch 90% A + 10% B; Fluss: 1.0 ml/min; Temperatur: 25°C; Injektion: 5 μl; DAD 220 nm]: $R_t$ = 7.13 min.

**Beispiel 235**

1-(2-Methoxyethyl)-3-(2-metboxypropyl)-5-methyl-6-[(2-oxoimidazolidin-1-yl)methyl]thieno-[2,3-d]pyrimidin-2,4(1*H*,3*H*)-dion *(Enantiomer 2)*

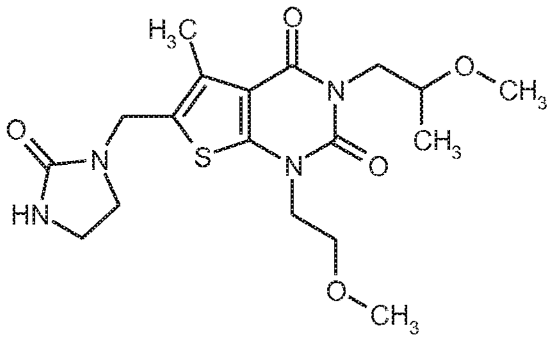

Die Titelverbindung (35 mg) wurde als zweites Enantiomer bei der unter Bsp. 234 beschriebenen präparativen HPLC-Trennung des Racemats aus Bsp. 99 erhalten.

$^1$H-NMR (400 MHz, DMSO-$d_6$, δ/ppm): 6.55 (s, 1H), 4.34 (s, 2H), 4.09-3.98 (m, 3H), 3.75 (dd, 1H), 3.68-3.59 (m, 3H), 3.29-3.18 (m, 10H), 2.38 (s, 3H), 1.05 (d, 3H).

Chirale analytische HPLC [Säule: Daicel Chiralpak IC, 3 μm, 100 mm x 4.6 mm; Eluent A: Acetonitril, Eluent B: Ethanol; isokratisch 90% A + 10% B; Fluss: 1.0 ml/min; Temperatur: 25°C; Injektion: 5 μl; DAD 220 nm]: $R_t$ = 9.16 min.

**Beispiel 236**

3-(2-Methoxy-2-methylpropyl)-5-methyl-6-[(2-oxoimidazolidin-1-yl)methyl]-1-(3,3,3-trifluorpropyl)thieno[2,3-d]pyrimidin-2,4(1*H*,3*H*)-dion

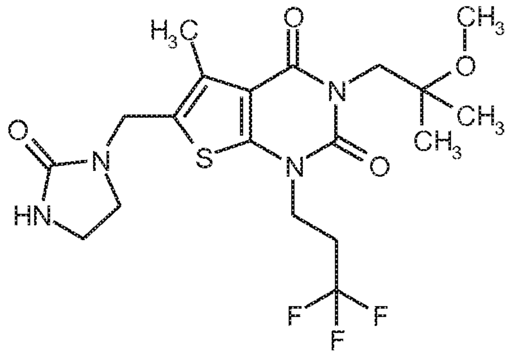

310 mg (0.575 mmol) der Verbindung aus Bsp. 322A wurden in 30 ml Dioxan gelöst und mit 144 mg (0.863 mmol) CDI versetzt. Das Gemisch wurde 19 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 172 mg (62% d. Th.) der Titelverbindung erhalten.
$^1$H-NMR (400 MHz, DMSO-$d_6$, δ/ppm): 6.56 (s, 1H), 4.36 (s, 2H), 4.09 (t, 2H), 3.99 (br. s, 2H), 3.30-3.18 (m, 4H), 3.15 (s, 3H), 2.82-2.68 (m, 2H), 2.39 (s, 3H), 1.08 (s, 6H).
LC/MS (Methode 3, ESIpos): $R_t$ = 1.07 min, m/z = 463 $[M+H]^+$.

**Beispiel 237**

1-(3-Fluorpropyl)-3-(2-methoxy-2-methylpropyl)-5-methyl-6-[(2-oxoimidazolidin-1-yl)methyl]-thieno[2,3-d]pyrimidin-2,4(1*H*,3*H*)-dion

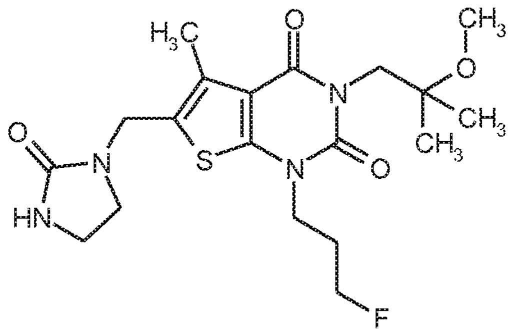

330 mg (0.7 mmol) der Verbindung aus Bsp. 323A wurden in 30 ml Dioxan gelöst und mit 176 mg (1.05 mmol) CDI versetzt. Das Gemisch wurde 18 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 139 mg (45% d. Th.) der Titelverbindung erhalten.
$^1$H-NMR (400 MHz, DMSO-d6, δ/ppm): 6.55 (s, 1H), 4.59 (t, 1H), 4.47 (t, 1H), 4.36 (s, 2H), 4.03-3.94 (m, 4H), 3.30-3.17 (m, 4H), 3.15 (s, 3H), 2.39 (s, 3H), 2.13-2.05 (m, 1H), 2.05-1.97 (m, 1H), 1.08 (s, 6H).
LC/MS (Methode 3, ESIpos): $R_t$ = 0.97 min, m/z = 427 $[M+H]^+$.

**Beispiel 238**

1-(2-Methoxyethyl)-3-(2-methoxy-2-methylpropyl)-5-methyl-6-[(2-oxoimidazolidin-1-yl)methyl]-thieno[2,3-d]pyrimidin-2,4(1*H*,3*H*)-dion

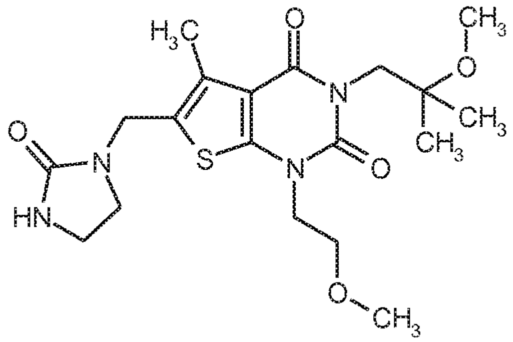

155 mg (0.303 mmol) der Verbindung aus Bsp. 324A wurden in 15 ml Dioxan gelöst und mit 76 mg (0.455 mmol) CDI versetzt. Das Gemisch wurde 20 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 86 mg (66% d. Th.) der Titelverbindung erhalten.
$^1$H-NMR (400 MHz, DMSO-$d_6$, $\delta$/ppm): 6.54 (s, 1H), 4.34 (s, 2H), 4.06-3.94 (m, 4H), 3.61 (t, 2H), 3.30-3.17 (m, 7H), 3.15 (s, 3H), 2.37 (s, 3H), 1.08 (s, 6H).
LC/MS (Methode 3, ESIpos): $R_t$ = 0.93 min, m/z = 425 $[M+H]^+$.

**Beispiel 239**

6-[(3-Ethyl-2-oxoimidazolidin-1-yl)methyl]-1-(2-methoxyethyl)-5-methyl-3-(2-phenylethyl)thieno-[2,3-d]pyrimidin-2,4(1*H*,3*H*)-dion

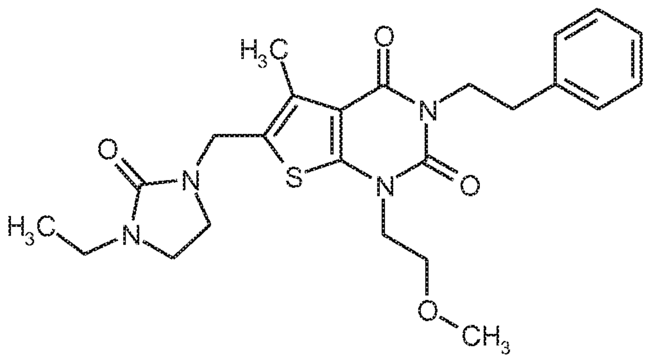

Eine Lösung von 45 mg (0.102 mmol) der Verbindung aus Bsp. 176 in 3 ml THF wurde bei 0°C mit 5 mg (0.122 mmol) Natriumhydrid (60% Suspension in Mineralöl) versetzt und 1 h gerührt. Anschließend wurden 19 mg (0.122 mmol) Iodethan hinzugefügt und das Gemisch 1 h bei 0°C gerührt. Danach wurde das Reaktionsgemisch zwischen gesättigter wässriger Ammoniumchlorid-Lösung (20 ml) und THF (30 ml) verteilt. Die organische Phase wurde mit gesättigter wässriger Natriumhydrogencarbonat-Lösung gewaschen, über Natriumsulfat getrocknet, filtriert und eingeengt. Der erhaltene Rückstand wurde mittels präparativer HPLC gereinigt (Methode 14). Nach Vereinigen der Produktfraktionen und Gefriertrocknung wurden 11.6 mg (23% d. Th.) der Titelverbindung erhalten.
$^1$H-NMR (400 MHz, DMSO-$d_6$, $\delta$/ppm): 7.34-7.27 (m, 2H), 7.26-7.19 (m, 3H), 4.37 (s, 2H), 4.09-4.03 (m, 2H), 4.01 (t, 2H), 3.59 (t, 2H), 3.27-3.16 (m, 7H), 3.12 (q, 2H), 2.86-2.79 (m, 2H), 2.39 (s, 3H), 1.01 (t, 3H).
LC/MS (Methode 3, ESIpos): $R_t$ = 1.26 min, m/z = 471 $[M+H]^+$.

**Beispiel 240**

1-(2-Methoxyethyl)-5-methyl-6-[(2-oxo-3-propylimidazolidin-1-yl)methyl]-3-(2-phenylethyl)-thieno[2,3-d]pyrimidin-2,4(1*H*,3*H*)-dion

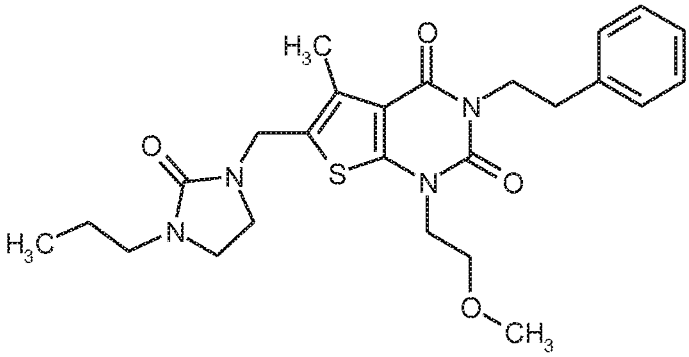

Eine Lösung von 45 mg (0.102 mmol) der Verbindung aus Bsp. 176 in 3 ml THF wurde bei 0°C mit 5 mg (0.122 mmol) Natriumhydrid (60% Suspension in Mineralöl) versetzt und 1 h gerührt. Anschließend wurden 21 mg (0.122 mmol) Iodpropan hinzugefügt und das Gemisch 92 h bei RT gerührt. Danach wurde das Reaktionsgemisch zwischen gesättigter wässriger Ammoniumchlorid-Lösung (20 ml) und THF (30 ml) verteilt. Die organische Phase wurde mit gesättigter wässriger Natriumhydrogencarbonat-Lösung gewaschen, über Natriumsulfat getrocknet, filtriert und eingeengt. Der erhaltene Rückstand wurde mittels präparativer HPLC gereinigt (Methode 14). Nach Vereinigen der Produktfraktionen und Gefriertrocknung wurden 17 mg (34% d. Th.) der Titelverbindung erhalten.
$^1$H-NMR (400 MHz, DMSO-$d_6$, δ/ppm): 7.33-7.27 (m, 2H), 7.26-7.18 (m, 3H), 4.38 (s, 2H), 4.08-4.02 (m, 2H), 4.00 (t, 2H), 3.59 (t, 2H), 3.27-3.15 (m, 7H), 3.05 (t, 2H), 2.86-2.79 (m, 2H), 2.39 (s, 3H), 1.44 (sext, 2H), 0.82 (t, 3H).
LC/MS (Methode 3, ESIpos): $R_t$ = 1.32 min, m/z = 485 $[M+H]^+$.

**Beispiel 241**

3-Ethyl-5-methyl-6-[(2-oxo-2,3-dihydro-1*H*-imidazol-1-yl)methyl]-1-(3,3,3-trifluorpropyl)thieno-[2,3-d]pyrimidin-2,4(1*H*,3*H*)-dion

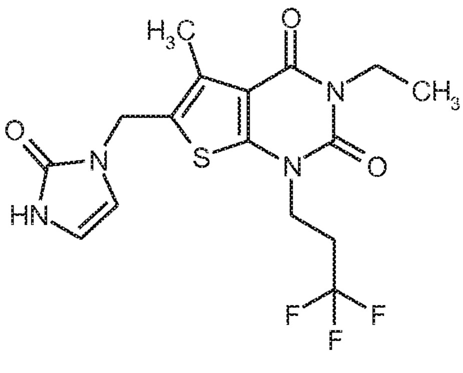

100 mg (0.178 mmol, 83% Reinheit) der Verbindung aus Bsp. 273A wurden in einem Gemisch aus 2 ml Methanol und 0.4 ml Wasser gelöst. Dann wurden 353 μl (0.353 mmol) 1 M Salzsäure hinzugefügt und das Gemisch 2.5 Tage bei RT gerührt. Da der Umsatz nach dieser Zeit noch nicht vollständig war, wurde nochmals 1 ml (1.0 mmol) 1 M Salzsäure zugefügt. Nach weiteren 18 h Rühren bei RT wurde das Reaktionsgemisch direkt mittels präparativer HPLC (Methode 8) in seine Komponenten aufgetrennt. Nach Eindampfen der Produktfraktionen und Trocknen im Hochvakuum wurden 55 mg (76% d. Th.) der Titelverbindung erhalten.
$^1$H-NMR (400 MHz, DMSO-$d_6$, δ/ppm): 10.00 (br. s, 1H), 6.44 (t, 1H), 6.34 (t, 1H), 4.81 (s, 2H), 4.08 (t, 2H), 3.90 (q,

2H), 2.86-2.64 (m, 2H), 2.47 (s, 3H), 1.11 (t, 3H).
LC/MS (Methode 1, ESIpos): $R_t$ = 0.79 min, m/z = 403 $[M+H]^+$.

**Beispiel 242**

3-Ethyl-5-methyl-6-[(2-oxo-2,3-dihydro-1*H*-imidazol-1-yl)methyl]-1-(tetrahydrofuran-2-ylmethyl)-thieno[2,3-d]pyrimidin-2,4(1*H*,3*H*)-dion *(Racemat)*

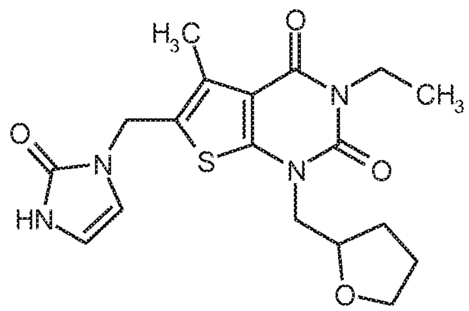

900 mg (1.78 mmol, 90% Reinheit) der Verbindung aus Bsp. 329A wurden in einem Gemisch aus 13 ml Methanol und 4 ml Wasser gelöst. Dann wurden 3.5 ml (3.56 mmol) 1 M Salzsäure hinzugefügt und das Gemisch 2.5 Tage bei RT gerührt. Danach wurde durch Zusatz von Wasser das Produkt ausgefällt. Es wurde abgesaugt, mit wenig Wasser gewaschen und im Hochvakuum getrocknet. Es wurden 472 mg (67% d. Th.) der Titelverbindung erhalten.
$^1$H-NMR (400 MHz, DMSO-$d_6$, $\delta$/ppm): 9.99 (br. s, 1H), 6.42 (t, 1H), 6.34 (t, 1H), 4.78 (s, 2H), 4.27-4.14 (m, 1H), 4.02 (dd, 1H), 3.90 (q, 2H), 3.79-3.51 (m, 3H), 2.46 (s, 3H), 2.05-1.73 (m, 3H), 1.71-1.58 (m, 1H), 1.11 (t, 3H).
LC/MS (Methode 17, ESIneg): $R_t$ = 1.23 min, m/z = 389.13 $[M-H]^-$.

**Beispiel 243**

3-Ethyl-5-methyl-6-[(2-oxo-2,3-dihydro-1*H*-imidazol-1-yl)methyl]-1-(tetrahydrofuran-2-ylmethyl)-thieno[2,3-d]pyrimidin-2,4(1*H*,3*H*)-dion *(Enantiomer 1)*

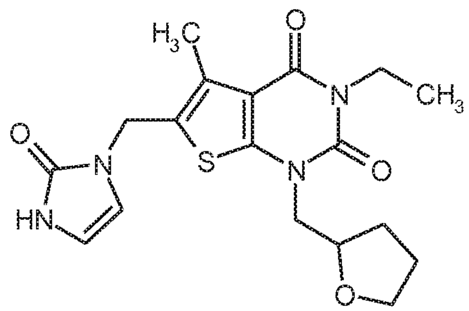

434 mg (1.11 mmol) der racemischen Verbindung aus Bsp. 242 wurden in 25 ml Methanol/Acetonitril (1:1) gelöst und in 17 Portionen über präparative SFC an chiraler Phase in die Enantiomere aufgetrennt [Säule: Daicel Chiralcel OX-H, 5 µm, SFC, 250 mm x 30 mm; Laufmittel: Kohlendioxid/Methanol 70:30; Fluss: 100 ml/min; Temperatur: 40°C; Detektion: 210 nm]. Nach Eindampfen der Produktfraktionen und Trocknen im Hochvakuum wurden 199 mg (91% d. Th.) von Enantiomer 1 erhalten (>99% ee, chirale analytische HPLC).
$^1$H-NMR (400 MHz, DMSO-$d_6$, $\delta$/ppm): 9.99 (br. s, 1H), 6.45-6.40 (m, 1H), 6.37-6.30 (m, 1H), 4.78 (s, 2H), 4.26-4.15 (m, 1H), 4.02 (dd, 1H), 3.90 (q, 2H), 3.78-3.55 (m, 3H), 2.46 (s, 3H), 2.05-1.75 (m, 3H), 1.71-1.58 (m, 1H), 1.11 (t, 3H).
Chirale analytische SFC [Säule: Daicel Chiralcel OX-H, 5 µm, 250 mm x 4.6 mm; Laufmittel: Kohlendioxid/Methanol 60:40; Fluss: 3 ml/min; Temperatur: 40°C; Detektion: 210 nm]: $R_t$ = 4.23 min.

**Beispiel 244**

3-Ethyl-5-methyl-6-[(2-oxo-2,3-dihydro-1*H*-imidazol-1-yl)methyl]-1-(tetrahydrofuran-2-ylmethyl)-thieno[2,3-d]pyrimidin-2,4(1*H*,3*H*)-dion *(Enantiomer 2)*

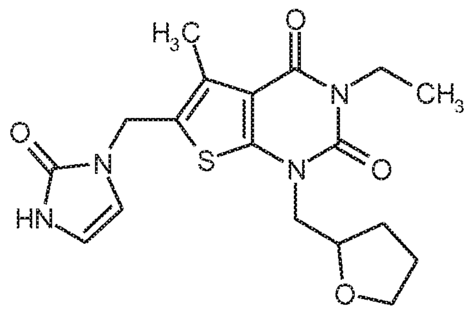

434 mg (1.11 mmol) der racemischen Verbindung aus Bsp. 242 wurden in 25 ml Methanol/Acetonitril (1:1) gelöst und in 17 Portionen über präparative SFC an chiraler Phase in die Enantiomere aufgetrennt [Säule: Daicel Chiralcel OX-H, 5 μm, SFC, 250 mm x 30 mm; Laufmittel: Kohlendioxid/Methanol 70:30; Fluss: 100 ml/min; Temperatur: 40°C; Detektion: 210 nm]. Nach Eindampfen der Produktfraktionen und Trocknen im Hochvakuum wurden 194 mg (89% d. Th.) von Enantiomer 2 erhalten (>99% ee, chirale analytische HPLC).
$^1$H-NMR (400 MHz, DMSO-$d_6$, δ/ppm): 9.99 (br. s, 1H), 6.42 (t, 1H), 6.34 (t, 1H), 4.78 (s, 2H), 4.27-4.15 (m, 1H), 4.02 (dd, 1H), 3.90 (q, 2H), 3.78-3.54 (m, 3H), 2.46 (s, 3H), 2.04-1.72 (m, 3H), 1.71-1.58 (m, 1H), 1.11 (t, 3H).
Chirale analytische SFC [Säule: Daicel Chiralcel OX-H, 5 μm, 250 mm x 4.6 mm; Laufmittel: Kohlendioxid/Methanol 60:40; Fluss: 3 ml/min; Temperatur: 40°C; Detektion: 210 nm]: $R_t$ = 6.22 min.

**Beispiel 245**

3-Isopropyl-5-methyl-6-[(2-oxo-2,3-dibydro-1*H*-imidazol-1-yl)methyl]-1-(3,3,3-trifluorpropyl)-thieno[2,3-d]pyrimidin-2,4(1*H*,3*H*)-dion

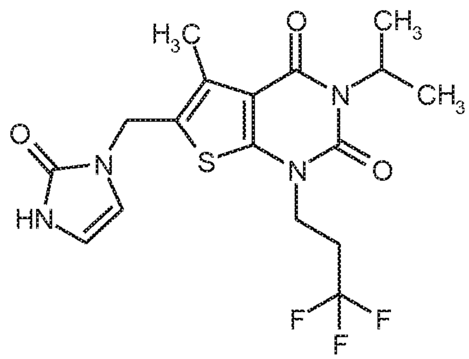

290 mg (0.604 mmol) der Verbindung aus Bsp. 330A wurden in einem Gemisch aus 6 ml Methanol und 1.2 ml Wasser gelöst. Dann wurden 1.2 ml (1.21 mmol) 1 M Salzsäure hinzugefugt und das Gemisch 6 Tage bei RT gerührt. Danach wurde durch Zusatz von Wasser das Produkt ausgefällt. Es wurde abgesaugt, mit wenig Wasser gewaschen und im Hochvakuum getrocknet. Es wurden 210 mg (83% d. Th.) der Titelverbindung erhalten.
$^1$H-NMR (400 MHz, DMSO-$d_6$, δ/ppm): 9.99 (br. s, 1H), 6.47-6.40 (m, 1H), 6.34 (t, 1H), 5.12 (sept, 1H), 4.80 (s, 2H), 4.05 (t, 2H), 2.84-2.64 (m, 2H), 2.46 (s, 3H), 1.39 (d, 6H).
LC/MS (Methode 17, ESIneg): $R_t$ = 1.59 min, m/z = 415.10 [M-H]$^-$.

**Beispiel 246**

3-Isopropyl-1-(2-niethoxyethyl)-5-methyl-6-[(2-oxo-2,3-dihydro-1*H*-imidazol-1-yl)methyl]thieno-[2,3-d]pyrimidin-2,4(1*H*,3*H*)-dion

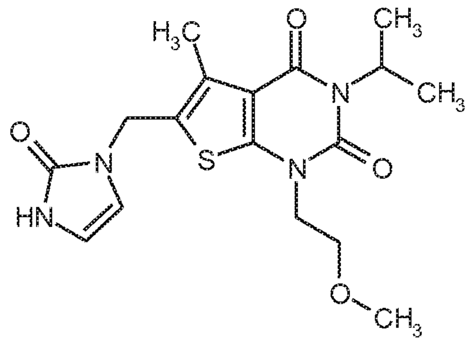

580 mg (0.865 mmol, 66% Reinheit) der Verbindung aus Bsp. 331A wurden in einem Gemisch aus 10 ml Methanol und 1.7 ml Wasser gelöst. Dann wurden 1.7 ml (1.73 mmol) 1 M Salzsäure hinzugefügt und das Gemisch 2.5 Tage bei RT gerührt. Danach wurde durch Zusatz von Wasser ein Teil des Produktes ausgefällt. Es wurde abgesaugt, mit wenig Wasser gewaschen und im Hochvakuum getrocknet. Das Filtrat wurde mit Ethylacetat extrahiert. Der organische Extrakt wurde mit gesättigter Natriumchlorid-Lösung gewaschen, über wasserfreiem Magnesiumsulfat getrocknet, filtriert und eingeengt. Der verbliebene Rückstand wurde mit dem anfangs ausgefallenen Produkt vereinigt und mit Acetonitril bei RT verrührt. Erneutes Absaugen und Trocknen im Hochvakuum ergaben 163 mg (49% d. Th.) der Titelverbindung.
$^1$H-NMR (400 MHz, DMSO-$d_6$, δ/ppm): 9.99 (br. s, 1H), 6.42 (t, 1H), 6.34 (t, 1H), 5.12 (sept, 1H), 4.78 (s, 2H), 3.97 (t, 2H), 3.60 (t, 2H), 3.31 (s, 3H), 2.44 (s, 3H), 1.39 (d, 6H).
LC/MS (Methode 17, ESIneg): $R_t$ = 1.33 min, m/z = 377.13 $[M-H]^-$.

**Beispiel 247**

3-Isopropyl-5-methyl-6-[(2-oxo-2,3-dihydro-1*H*-imidazol-1-yl)methyl]-1-(tetrahydrofuran-2-yl-methyl)thieno[2,3-d]pyrimidin-2,4(1*H*,3*H*)-dion *(Racemat)*

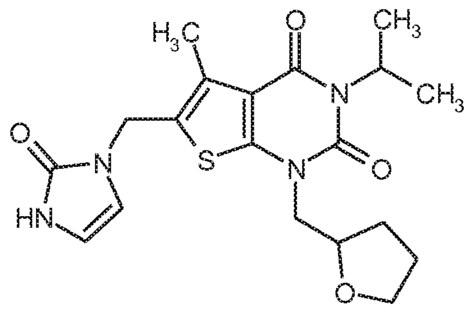

1.15 g (1.89 mmol, 77% Reinheit) der Verbindung aus Bsp. 332A wurden in einem Gemisch aus 20 ml Methanol und 3.8 ml Wasser gelöst. Dann wurden 3.8 ml (3.78 mmol) 1 M Salzsäure hinzugefügt und das Gemisch 2.5 Tage bei RT gerührt. Danach wurde mit Wasser verdünnt und mit Ethylacetat extrahiert. Der Extrakt wurde mit gesättigter Natriumchlorid-Lösung gewaschen, über wasserfreiem Magnesiumsulfat getrocknet, filtriert und eingeengt. Der verbliebene Rückstand wurde mit Acetonitril bei RT verrührt. Absaugen des Feststoffs und Trocknen im Hochvakuum ergaben 418 mg (54% d. Th.) der Titelverbindung.
$^1$H-NMR (400 MHz, DMSO-$d_6$, δ/ppm): 9.98 (br. s, 1H), 6.41 (t, 1H), 6.34 (t, 1H), 5.13 (sept, 1H), 4.77 (s, 2H), 4.25-4.13 (m, 1H), 4.02 (dd, 1H), 3.80-3.68 (m, 1H), 3.66-3.52 (m, 2H), 2.44 (s, 3H), 2.04-1.72 (m, 3H), 1.70-1.57 (m, 1H), 1.39 (d, 6H).

LC/MS (Methode 1, ESIpos): $R_t$ = 0.77 min, m/z = 405 $[M+H]^+$.

**Beispiel 248**

3-Isopropyl-5-methyl-6-[(2-oxo-2,3-dihydro-1*H*-imidazol-1-yl)methyl]-1-(tetrahydrofuran-2-yl-methyl)thieno[2,3-d]pyrimidin-2,4(1*H*,3*H*)-dion *(Enantiomer 1)*

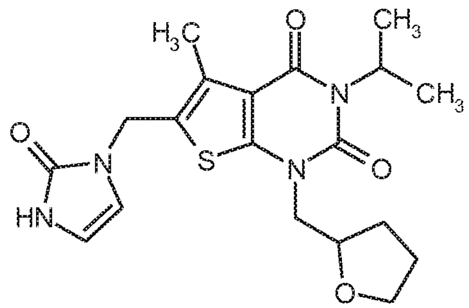

382 mg (0.944 mmol) der racemischen Verbindung aus Bsp. 247 wurden in einem Gemisch aus 9 ml Ethanol und 1 ml Acetonitril gelöst und in 29 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 40°C; Detektion: 220 nm]. Nach Eindampfen der Produktfraktionen und Trocknen im Hochvakuum wurden 155 mg (81% d. Th.) von Enantiomer 1 erhalten (>99% ee, chirale analytische HPLC).
$^1$H-NMR (400 MHz, DMSO-$d_6$, δ/ppm): 9.98 (br. s, 1H), 6.44-6.38 (m, 1H), 6.34 (t, 1H), 5.13 (sept, 1H), 4.77 (s, 2H), 4.25-4.13 (m, 1H), 4.02 (dd, 1H), 3.78-3.68 (m, 1H), 3.66-3.56 (m, 2H), 2.44 (s, 3H), 2.06-1.74 (m, 3H), 1.70-1.56 (m, 1H), 1.39 (dd, 6H).

Chirale analytische HPLC [Säule: Daicel Chiralcel OX-H, 5 μm, 250 mm x 4.6 mm; Laufmittel: Ethanol; Fluss: 1 ml/min; Temperatur: 45°C; Detektion: 260 nm]: $R_t$ = 6.19 min.

**Beispiel 249**

3-Isopropyl-5-methyl-6-[(2-oxo-2,3-dihydro-1*H*-imidazol-1-yl)methyl]-1-(tetrahydrofuran-2-yl-methyl)thieno[2,3-d]pyrimidin-2,4(1*H*,3*H*)-dion *(Enantiomer 2)*

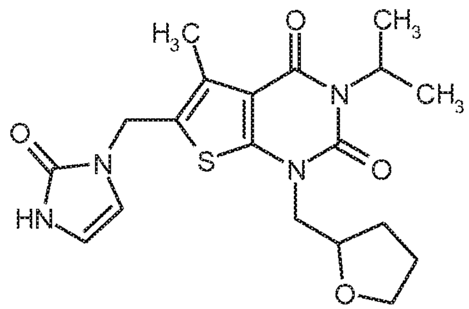

382 mg (0.944 mmol) der racemischen Verbindung aus Bsp. 247 wurden in einem Gemisch aus 9 ml Ethanol und 1 ml Acetonitril gelöst und in 29 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 40°C; Detektion: 220 nm]. Nach Eindampfen der Produktfraktionen und Trocknen im Hochvakuum wurden 165 mg (86% d. Th.) von Enantiomer 2 erhalten (>99% ee, chirale analytische HPLC).
$^1$H-NMR (400 MHz, DMSO-$d_6$, δ/ppm): 9.98 (br. s, 1H), 6.45-6.38 (m, 1H), 6.34 (t, 1H), 5.13 (sept, 1H), 4.77 (s, 2H), 4.25-4.13 (m, 1H), 4.02 (dd, 1H), 3.78-3.68 (m, 1H), 3.66-3.53 (m, 2H), 2.44 (s, 3H), 2.04-1.74 (m, 3H), 1.70-1.56 (m, 1H), 1.43-1.35 (dd, 6H).

Chirale analytische HPLC [Säule: Daicel Chiralcel OX-H, 5 μm, 250 mm x 4.6 mm; Laufmittel: Ethanol; Fluss:

1 ml/min; Temperatur: 45°C; Detektion: 260 nm]: $R_t$ = 8.19 min.

**Beispiel 250**

3-Ethyl-5-methyl-6-[(4-methyl-2-oxo-2,3-dihydro-1*H*-imidazol-1-yl)methyl]-1-(3,3,3-trifluorpropyl)thieno[2,3-d]pyrimidin-2,4(1*H*,3*H*)-dion

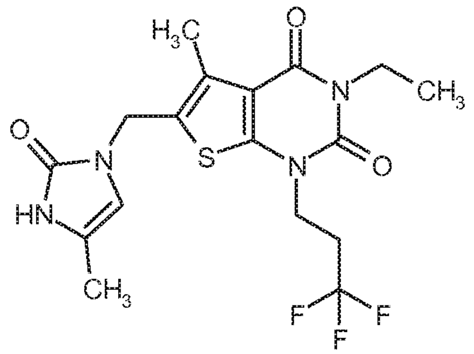

122 mg (0.191 mmol, 75% Reinheit) der Verbindung aus Bsp. 271A wurden in 3 ml Methanol gelöst und mit 0.6 ml 1 M Salzsäure versetzt. Da der Umsatz nach 18 h Rühren bei RT noch unvollständig war, wurden 0.6 ml konzentrierte Salzsäure hinzugefügt. Nach weiteren 24 h Rühren bei RT wurde das Reaktionsgemisch direkt mittels präparativer HPLC (Methode 8) in seine Komponenten aufgetrennt. Die Produktfraktionen wurden vereinigt und eingeengt und der Rückstand im Hochvakuum getrocknet. Es wurden 54 mg (67% d. Th.) der Titelverbindung erhalten.
$^1$H-NMR (400 MHz, DMSO-$d_6$, δ/ppm): 9.94 (s, 1H), 6.09 (s, 1H), 4.74 (s, 2H), 4.08 (t, 2H), 3.90 (q, 2H), 2.85-2.66 (m, 2H), 2.46 (s, 3H), 1.87 (s, 3H), 1.11 (t, 3H).
LC/MS (Methode 1, ESIpos): Rt = 0.86 min, m/z = 417 $[M+H]^+$.

**Beispiel 251**

3-Ethyl-5-methyl-6-[(5-methyl-2-oxo-2,3-dihydro-1*H*-imidazol-1-yl)methyl]-1-(3,3,3-trifluorpropyl)thieno[2,3-d]pyrimidin-2,4(1*H*,3*H*)-dion

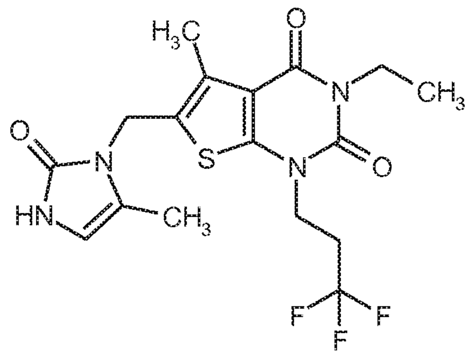

215 mg (0.291 mmol, 65% Reinheit) der Verbindung aus Bsp. 275A wurden in einem Gemisch aus 2.1 ml Methanol und 0.6 ml Wasser gelöst und mit 0.6 ml (0.291 mmol) 0.5 M Salzsäure versetzt. Nachdem das Reaktionsgemisch 2.5 Tage bei RT gerührt worden war, wurde es direkt mittels präparativer HPLC (Methode 8) in seine Komponenten aufgetrennt. Die Produktfraktionen wurden vereinigt und eingeengt und der Rückstand bei RT mit wenig Acetonitril verrührt. Nach Absaugen des Feststoffs und Trocknen im Hochvakuum wurden 61 mg (50% d. Th.) der Titelverbindung erhalten.
$^1$H-NMR (400 MHz, DMSO-$d_6$, δ/ppm): 9.79 (s, 1H), 6.09 (s, 1H), 4.84 (s, 2H), 4.06 (t, 2H), 3.89 (q, 2H), 2.83-2.63 (m,

2H), 2.48 (s, 3H), 1.96 (s, 3H), 1.11 (t, 3H).
LC/MS (Methode 1, ESIpos): Rt = 0.86 min, m/z = 417 $[M+H]^+$.

**Beispiel 252**

3-Ethyl-1-{2-methoxyethyl)-5-methyl-6-[(5-methyl-2-oxo-2,3-dihydro-1*H*-imidazol-1-yl)methyl]-thieno[2,3-d]pyrimidin-2,4(1*H*,3*H*)-dion

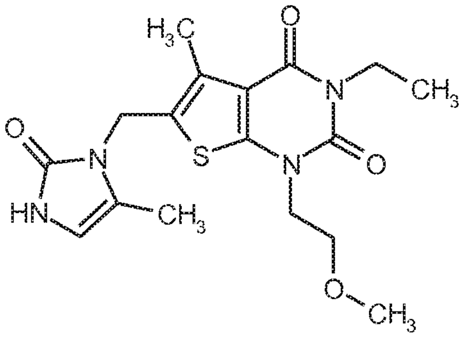

Analog zu dem unter Bsp. 251 beschriebenen Verfahren wurden aus 155 mg (0.210 mmol, 60% Reinheit) der Verbindung aus Bsp. 276A 52 mg (65% d. Th.) der Titelverbindung erhalten.
$^1$H-NMR (400 MHz, DMSO-$d_6$, $\delta$/ppm): 9.78 (s, 1H), 6.09 (s, 1H), 4.82 (s, 2H), 3.98 (t, 2H), 3.89 (q, 2H), 3.60 (t, 2H), 3.31 (s, 3H), 2.46 (s, 3H), 1.96 (s, 3H), 1.11 (t, 3H).
LC/MS (Methode 1, ESIpos): $R_t$ = 0.75 min, m/z = 379 $[M+H]^+$.

**Beispiel 253**

3-Isopropyl-5-methyl-6-[(5-oxo-4,5-dihydro-1*H*-1,2,4-triazol-1-yl)methyl]-1-(3,3,3-trifluorpropyl)-thieno[2,3-d]pyrimidin-2,4(1*H*,3*H*)-dion

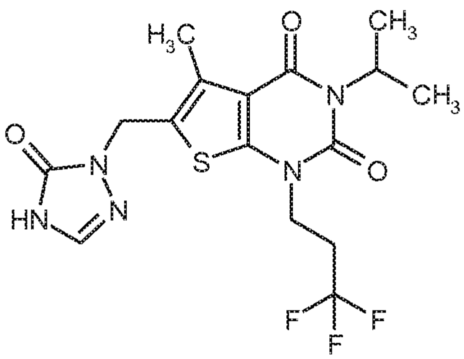

Eine Suspension von 490 mg (1.17 mmol) der Verbindung aus Bsp. 353A in 30 ml Toluol wurde mit 162 µl (1.17 mmol) Triethylamin und 251 µl (1.17 mmol) Phosphorsäurediphenylesterazid versetzt. Anschließend wurde das Reaktionsgemisch zunächst 1 h auf 80°C und dann 1 h auf 100°C erhitzt. Danach wurden alle leichtflüchtigen Bestandteile am Rotationsverdampfer entfernt. Der verbliebene Rückstand wurde mit Ethylacetat verdünnt und nacheinander mit Wasser und gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 8). Die Produktfraktionen wurden vereinigt und eingeengt und der Rückstand im Hochvakuum getrocknet. Es wurden 75 mg (15% d. Th.) der Titelverbindung erhalten.
$^1$H-NMR (400 MHz, DMSO-$d_6$, $\delta$/ppm): 11.59 (br. s, 1H), 7.84 (s, 1H), 5.12 (sept, 1H), 4.94 (s, 2H), 4.05 (t, 2H), 2.82-2.64 (m, 2H), 2.45 (s, 3H), 1.39 (d, 6H).

LC/MS (Methode 1, ESIpos): $R_t$ = 0.84 min, m/z = 418 $[M+H]^+$.

**Beispiel 254**

3-Isopropyl-1-(2-methoxyethyl)-5-methyl-6-[(5-oxo-4,5-dihydro-1*H*-1,2,4-triazol-1-yl)methyl]-thieno[2,3-d]pyrimidin-2,4(1*H*,3*H*)-dion

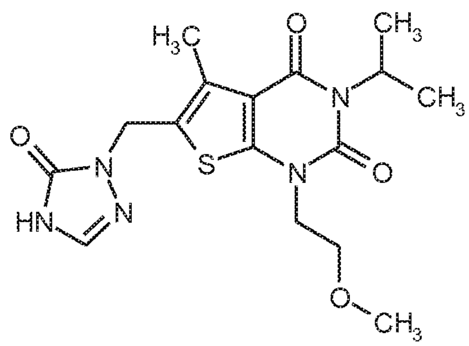

Analog zu dem unter Bsp. 253 beschriebenen Verfahren wurden aus 85 mg (0.222 mmol) der Verbindung aus Bsp. 354A und 48 μl (0.222 mmol) Phosphorsäurediphenylesterazid 3 mg (3% d. Th.) der Titelverbindung erhalten. Das Produkt wurde hier nach der präparativen HPLC nochmals mittels präparativer DC (Laufmittel: Dichlormethan/Methanol 10:1) nachgereinigt.
$^1$H-NMR (400 MHz, DMSO-$d_6$, δ/ppm): 11.59 (br. s, 1H), 7.83 (s, 1H), 5.12 (sept, 1H), 4.92 (s, 2H), 3.97 (t, 2H), 3.60 (t, 2H), 3.23 (s, 3H), 2.44 (s, 3H), 1.39 (d, 6H).
LC/MS (Methode 1, ESIpos): $R_t$ = 0.71 min, m/z = 380 $[M+H]^+$.

**Beispiel 255**

[1-{[3-Ethyl-5-methyl-2,4-dioxo-1-(3,3,3-trifluorpropyl)-1,2,3,4-tetrahydrothieno[2,3-d]pyrimidin-6-yl]methyl}imidazolidin-2-yliden]cyanamid

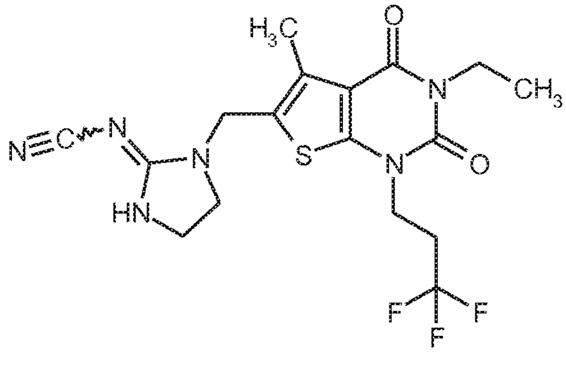

Analog zu dem unter Bsp. 117 beschriebenen Verfahren wurden aus 396 mg (0.889 mmol, 85% Reinheit) der Verbindung aus Bsp. 210A und 195 mg (1.33 mmol) Dimethyl-*N*-cyanodithioiminocarbonat 150 mg (39% d. Th.) der Titelverbindung erhalten. Die Reaktionszeit betrug in diesem Fall 3 h, und das Rohprodukt wurde durch Verrühren mit einem Acetonitril/Wasser-Gemisch gereinigt.
$^1$H-NMR (400 MHz, DMSO-$d_6$, δ/ppm): 8.08 (s, 1H), 4.50 (s, 2H), 4.11 (t, 2H), 3.90 (q, 2H), 3.53-3.37 (m, 4H), 2.87-2.68 (m, 2H), 2.42 (s, 3H), 1.12 (t, 3H).
LC/MS (Methode 17, ESIpos): $R_t$ = 1.59 min, m/z = 429.13 $[M+H]^+$.

**Beispiel 256**

[1-{[3-Ethyl-5-methyl-1-(oxetan-2-ylmethyl)-2,4-dioxo-1,2,3,4-tetrahydrothieno[2,3-d]pyrimidin-6-yl]methyl}imidazolidin-2-yliden]cyanamid *(Racemat)*

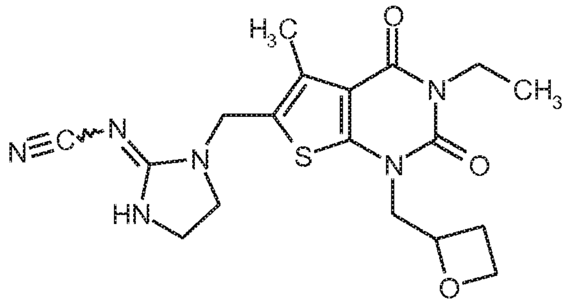

300 mg (0.783 mmol, 92% Reinheit) der Verbindung aus Bsp. 224A wurden in 3.6 ml DMF gelöst und mit 172 mg (1.17 mmol) Dimethyl-*N*-cyanodithioiminocarbonat sowie 216 mg (1.57 mmol) Kaliumcarbonat versetzt. Das Gemisch wurde 8.5 h bei 80°C in einem Mikrowellenofen gerührt (Biotage Initiator mit dynamischer Steuerung der Einstrahlleistung). Danach wurde das Reaktionsgemisch auf Wasser gegossen. Der dabei ausgefallene Niederschlag wurde abgesaugt, in Ethylacetat gelöst und die Lösung nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und die Rohprodukt-Lösung eingeengt. Auf gleiche Weise wurde ein zweiter Ansatz, ausgehend von 350 mg (0.516 mmol, 52% Reinheit) der Verbindung aus Bsp. 224A, durchgeführt. Die Rohprodukte aus dem ersten und dem zweiten Ansatz wurden vereinigt und mit einem Wasser/Acetonitril-Gemisch verrührt. Der Feststoff wurde abgesaugt und im Hochvakuum getrocknet. Dies ergab eine erste Fraktion der Titelverbindung. Die Mutterlauge des Verrührens wurde mittels präparativer HPLC gereinigt (Methode 8). Dies ergab eine zweite Fraktion des Produktes, die mit der ersten vereinigt wurde. Insgesamt wurden so 275 mg (51% d. Th., 97% Reinheit) der Titelverbindung erhalten.

$^1$H-NMR (400 MHz, DMSO-$d_6$, $\delta$/ppm): 8.06 (s, 1H), 5.01 (quin, 1H), 4.54-4.36 (m, 2H), 4.47 (s, 2H), 4.14 (d, 2H), 3.90 (q, 2H), 3.51-3.36 (m, 4H), 2.76-2.63 (m, 1H), 2.55-2.45 (m, 1H, weitgehend überdeckt vom DMSO-Signal), 2.41 (s, 3H), 1.12 (t, 3H).

LC/MS (Methode 17, ESIpos): $R_t$ = 1.26 min, m/z = 403.15 $[M+H]^+$.

**Beispiel 257**

[1-{[3-Ethyl-5-methyl-1-(oxetan-2-ylmethyl)-2,4-dioxo-1,2,3,4-tetrahydrothieno[2,3-d]pyrimidin-6-yl]methyl}imidazolidin-2-yliden]cyanamid *(Enantiomer 1)*

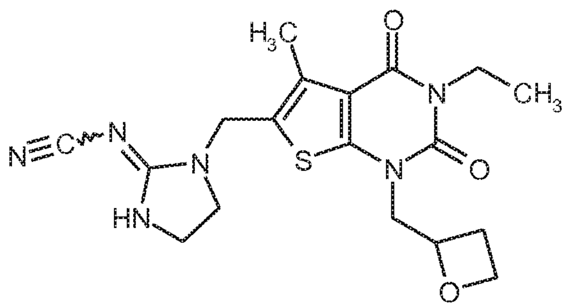

251 mg (0.624 mmol) der racemischen Verbindung aus Bsp. 256 wurden in 25 ml Methanol/ Acetonitril (1:1) gelöst und in 50 Portionen über präparative SFC an chiraler Phase in die Enantiomere aufgetrennt [Säule: Daicel Chiralcel OJ-H, 5 $\mu$m, SFC, 250 mm x 30 mm; Laufmittel: Kohlendioxid/Methanol 90:10; Fluss: 100 ml/min; Temperatur: 40°C; Detektion: 210 nm]. Nach Eindampfen der Produktfraktionen wurde das erhaltene Material noch mit Diisopropylether bei RT verrührt. Absaugen des Feststoffs und Trocknen im Hochvakuum ergaben 65 mg (51% d. Th.) von Enantiomer

1 (90% ee, chirale analytische HPLC).
$^{1}$H-NMR (400 MHz, DMSO-$d_6$, δ/ppm): 8.06 (s, 1H), 5.01 (quin, 1H), 4.54-4.36 (m, 2H), 4.47 (s, 2H), 4.14 (d, 2H), 3.90 (q, 2H), 3.51-3.36 (m, 4H), 2.76-2.63 (m, 1H), 2.55-2.45 (m, 1H, weitgehend überdeckt vom DMSO-Signal), 2.41 (s, 3H), 1.12 (t, 3H).

Chirale analytische SFC [Säule: Daicel Chiralcel OJ-H, 5 μm, 250 mm x 4.6 mm; Laufmittel: Kohlendioxid/Methanol 80:20; Fluss: 3 ml/min; Temperatur: 40°C; Detektion: 210 nm]: $R_t$ = 1.60 min.

## Beispiel 258

[1-{[3-Ethyl-5-methyl-1-(oxetan-2-ylmethyl)-2,4-dioxo-1,2,3,4-tetrahydrothieno[2,3-d]pyrimidin-6-yl]methyl}imidazolidin-2-yliden]cyanamid *(Enantiomer 2)*

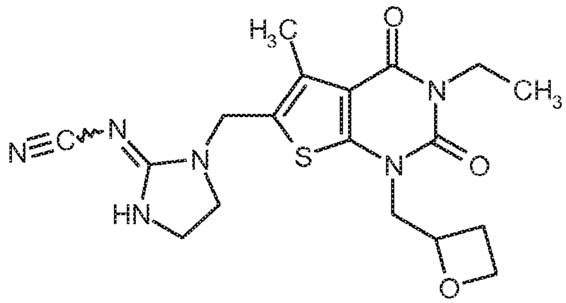

251 mg (0.624 mmol) der racemischen Verbindung aus Bsp. 256 wurden in 25 ml Methanol/ Acetonitril (1:1) gelöst und in 50 Portionen über präparative SFC an chiraler Phase in die Enantiomere aufgetrennt [Säule: Daicel Chiralcel OJ-H, 5 μm, SFC, 250 mm x 30 mm; Laufmittel: Kohlendioxid/Methanol 90:10; Fluss: 100 ml/min; Temperatur: 40°C; Detektion: 210 nm]. Nach Eindampfen der Produktfraktionen wurde das erhaltene Material noch mit Diisopropylether bei RT verrührt. Absaugen des Feststoffs und Trocknen im Hochvakuum ergaben 48 mg (38% d. Th.) von Enantiomer 2 (>99% ee, chirale analytische HPLC).
$^{1}$H-NMR (400 MHz, DMSO-$d_6$, δ/ppm): 8.06 (s, 1H), 5.07-4.95 (m, 1H), 4.54-4.37 (m, 2H), 4.47 (s, 2H), 4.14 (d, 2H), 3.90 (q, 2H), 3.52-3.36 (m, 4H), 2.77-2.62 (m, 1H), 2.55-2.45 (m, 1H, weitgehend überdeckt vom DMSO-Signal), 2.41 (s, 3H), 1.12 (t, 3H).

Chirale analytische SFC [Säule: Daicel Chiralcel OJ-H, 5 μm, 250 mm x 4.6 mm; Laufmittel: Kohlendioxid/Methanol 80:20; Fluss: 3 ml/min; Temperatur: 40°C; Detektion: 210 nm]: $R_t$ = 1.43 min.

## Beispiel 259

[1-{[3-Ethyl-5-methyl-2,4-dioxo-1-(tetrahydrofuran-2-ylmethyl)-1,2,3,4-tetrahydrothieno[2,3-d]-pyrimidin-6-yl]methyl}imidazolidin-2-yliden]cyanamid *(Racemat)*

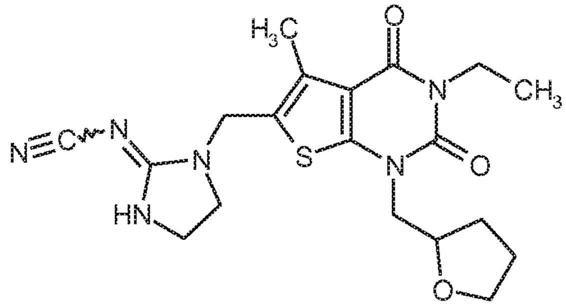

1.0 g (2.05 mmol, 75% Reinheit) der Verbindung aus Bsp. 225A wurden in 17 ml DMF gelöst und mit 449 mg (3.07 mmol) Dimethyl-*N*-cyanodithioiminocarbonat sowie 566 mg (4.09 mmol) Kaliumcarbonat versetzt. Das Gemisch wurde 3 h bei 80°C in einem Mikrowellenofen gerührt (Biotage Initiator mit dynamischer Steuerung der Einstrahlleistung).

Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mit einem Wasser/ Acetonitril-Gemisch verrührt. Der Feststoff wurde abgesaugt und im Hochvakuum getrocknet. Dies ergab eine erste Fraktion der Titelverbindung (90 mg). Die Mutterlauge des Verrührens wurde mittels präparativer HPLC gereinigt (Methode 8). Dies ergab eine zweite Fraktion des Produktes (142 mg), die mit der ersten vereinigt wurde. Insgesamt wurden so 232 mg (27% d. Th.) der Titelverbindung erhalten.

$^1$H-NMR (400 MHz, DMSO-$d_6$, δ/ppm): 8.07 (s, 1H), 4.47 (s, 2H), 4.29-4.16 (m, 1H), 4.05 (dd, 1H), 3.90 (q, 2H), 3.81-3.57 (m, 3H), 3.49-3.36 (m, 4H), 2.41 (s, 3H), 2.05-1.74 (m, 3H), 1.73-1.60 (m, 1H), 1.12 (t, 3H).

LC/MS (Methode 1, ESIpos): $R_t$ = 0.78 min, m/z = 417 $[M+H]^+$.

## Beispiel 260

[1-{[3-Ethyl-5-methyl-2,4-dioxo-1-(tetrahydrofuran-2-ylmethyl)-1,2,3,4-tetrahydrothieno[2,3-d]-pyrimidin-6-yl]methyl}imidazolidin-2-yliden]cyanamid *(Enantiomer 1)*

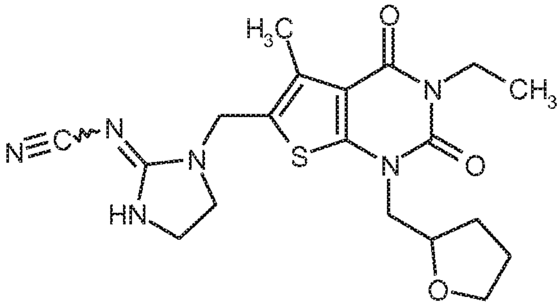

201 mg (0.482 mmol) der racemischen Verbindung aus Bsp. 259 wurden in einem Gemisch aus 30 ml Ethanol und 7 ml Acetonitril gelöst und in 125 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 50°C; Detektion: 220 nm]. Nach Eindampfen der Produktfraktionen und Trocknen im Hochvakuum wurden 73 mg (72% d. Th.) von Enantiomer 1 erhalten (>99% ee, chirale analytische HPLC).

$^1$H-NMR (400 MHz, DMSO-$d_6$, δ/ppm): 8.07 (s, 1H), 4.47 (s, 2H), 4.28-4.18 (m, 1H), 4.05 (dd, 1H), 3.90 (q, 2H), 3.81-3.57 (m, 3H), 3.51-3.36 (m, 4H), 2.41 (s, 3H), 2.05-1.75 (m, 3H), 1.72-1.60 (m, 1H), 1.12 (t, 3H).

Chirale analytische HPLC [Säule: Daicel Chiralcel OX-H, 5 μm, 250 mm x 4.6 mm; Laufmittel: Ethanol; Fluss: 1 ml/min; Temperatur: 50°C; Detektion: 220 nm]: $R_t$ = 9.62 min.

## Beispiel 261

[1-{[3-Ethyl-5-methyl-2,4-dioxo-1-(tetrahydrofuran-2-ylmethyl)-1,2,3,4-tetrahydrothieno[2,3-d]-pyrimidin-6-yl]methyl}imidazolidin-2-yliden]cyanamid *(Enantiomer 2)*

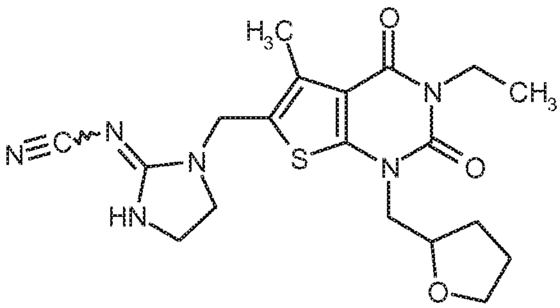

201 mg (0.482 mmol) der racemischen Verbindung aus Bsp. 259 wurden in einem Gemisch aus 30 ml Ethanol

und 7 ml Acetonitril gelöst und in 125 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 50°C; Detektion: 220 nm]. Nach Eindampfen der Produktfraktionen und Trocknen im Hochvakuum wurden 80 mg (79% d. Th.) von Enantiomer 2 erhalten (>99% ee, chirale analytische HPLC).

$^1$H-NMR (400 MHz, DMSO-$d_6$, δ/ppm): 8.07 (s, 1H), 4.47 (s, 2H), 4.28-4.16 (m, 1H), 4.05 (dd, 1H), 3.90 (q, 2H), 3.81-3.57 (m, 3H), 3.50-3.36 (m, 4H), 2.41 (s, 3H), 2.05-1.75 (m, 3H), 1.73-1.60 (m, 1H), 1.12 (t, 3H).

Chirale analytische HPLC [Säule: Daicel Chiralcel OX-H, 5 μm, 250 mm x 4.6 mm; Laufmittel: Ethanol; Fluss: 1 ml/min; Temperatur: 50°C; Detektion: 220 nm]: Rt = 12.32 min.

## Beispiel 262

[1-{[3-Isopropyl-5-methyl-2,4-dioxo-1-(3,3,3-trifluorpropyl)-1,2,3,4-tetrahydrothieno[2,3-d]-pyrimidin-6-yl]methyl}imidazolidin-2-yliden]cyanamid

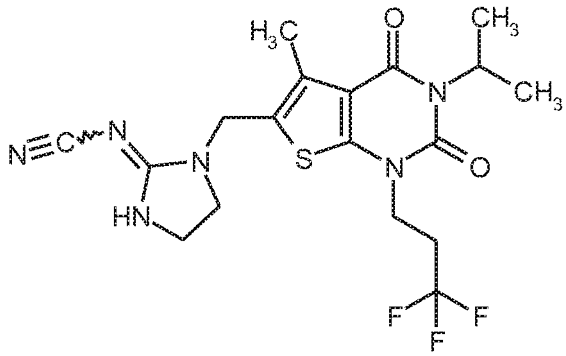

360 mg (0.660 mmol, 72% Reinheit) der Verbindung aus Bsp. 309A wurden in 5 ml DMF gelöst und mit 145 mg (0.991 mmol) Dimethyl-*N*-cyanodithioiminocarbonat sowie 183 mg (1.32 mmol) Kaliumcarbonat versetzt. Das Gemisch wurde 4.5 h bei 80°C in einem Mikrowellenofen gerührt (Biotage Initiator mit dynamischer Steuerung der Einstrahlleistung). Danach wurde das Reaktionsgemisch über wenig Kieselgel filtriert und anschließend direkt mittels präparativer HPLC (Methode 8) in seine Komponenten aufgetrennt. Einengen der Produktfraktionen und Trocknen im Hochvakuum ergaben 130 mg (44% d. Th.) der Titelverbindung.

$^1$H-NMR (400 MHz, DMSO-$d_6$, δ/ppm): 8.08 (s, 1H), 5.13 (sept, 1H), 4.49 (s, 2H), 4.08 (t, 2H), 3.53-3.35 (m, 4H), 2.85-2.68 (m, 2H), 2.41 (s, 3H), 1.40 (d, 6H).

LC/MS (Methode 17, ESIpos): $R_t$ = 1.79 min, m/z = 443.15 $[M+H]^+$.

## Beispiel 263

[1-{[3-Isopropyl-1-(2-methoxyethyl)-5-methyl-2,4-dioxo-1,2,3,4-tetrahydrothieno[2,3-d]pyrimidin-6-yl]methyl}imidazolidin-2-yliden]cyanamid

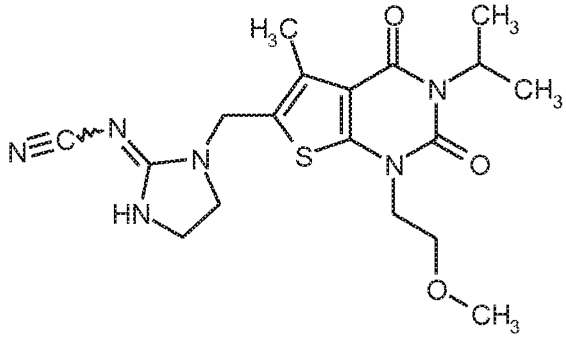

466 mg (1.05 mmol, 80% Reinheit) der Verbindung aus Bsp. 310A wurden in 6 ml DMF gelöst und mit 231 mg (1.58 mmol) Dimethyl-N-cyanodithioiminocarbonat sowie 291 mg (2.10 mmol) Kaliumcarbonat versetzt. Das Gemisch wurde 4.5 h bei 80°C in einem Mikrowellenofen gerührt (Biotage Initiator mit dynamischer Steuerung der Einstrahlleistung). Danach wurde das Reaktionsgemisch über wenig Kieselgel filtriert und anschließend direkt mittels präparativer HPLC (Methode 8) in seine Komponenten aufgetrennt. Wegen nicht ausreichender Reinheit des so erhaltenen Materials wurde die präparative HPLC noch zweimal wiederholt (das erste Mal erneut nach Methode 8, das zweite Mal nach folgender Methode: Säule: Kinetex C18, 5 µm, 100 mm x 30 mm; Eluent A: Wasser + 0.07% Ameisensäure; Eluent B: Acetonitril; Gradient: 0.0-2.2 min 10% B, 2.2-7.0 min 20% B, 7.0-7.5 min 60% B, 7.5-9.0 min 92% B; Fluss: 70 ml/min; Temperatur: 25°C; Detektion: 210 nm). Eindampfen der Produktfraktionen und Trocknen des Rückstands im Hochvakuum ergaben 61 mg (14% d. Th.) der Titelverbindung.
$^1$H-NMR (400 MHz, DMSO-$d_6$, δ/ppm): 8.06 (s, 1H), 5.13 (sept, 1H), 4.46 (s, 2H), 3.99 (t, 2H), 3.62 (t, 2H), 3.50-3.35 (m, 4H), 3.25 (s, 3H), 2.39 (s, 3H), 1.40 (d, 6H).
LC/MS (Methode 17, ESIpos): $R_t$ = 1.54 min, m/z = 405.17 [M+H]$^+$.

## Beispiel 264

[1-{[3-Isopropyl-5-methyl-1-(oxetan-2-ylmethyl)-2,4-dioxo-1,2,3,4-tetrahydrothieno[2,3-d]-pyrimidin-6-yl]methyl}imidazolidin-2-yliden]cyanamid *(Racemat)*

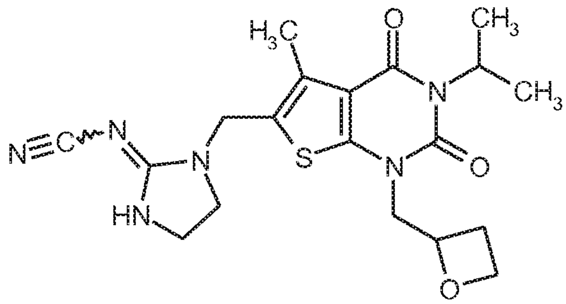

880 mg (1.68 mmol, 70% Reinheit) der Verbindung aus Bsp. 311A wurden in 10 ml DMF gelöst und mit 369 mg (2.52 mmol) Dimethyl-*N*-cyanodithioiminocarbonat sowie 465 mg (3.36 mmol) Kaliumcarbonat versetzt. Das Gemisch wurde 4.5 h bei 80°C in einem Mikrowellenofen gerührt (Biotage Initiator mit dynamischer Steuerung der Einstrahlleistung). Danach wurde das Reaktionsgemisch über wenig Kieselgel filtriert und anschließend direkt mittels präparativer HPLC (Methode 8) in seine Komponenten aufgetrennt. Eindampfen der Produktfraktionen und Trocknen im Hochvakuum ergaben 140 mg (19% d. Th.) der Titelverbindung.
$^1$H-NMR (400 MHz, DMSO-$d_6$, δ/ppm): 8.06 (s, 1H), 5.13 (sept, 1H), 5.05-4.93 (m, 1H), 4.54-4.36 (m, 2H), 4.46 (s, 2H), 4.11 (d, 2H), 3.51-3.35 (m, 4H), 2.76-2.62 (m, 1H), 2.53-2.44 (m, 1H, weitgehend überdeckt vom DMSO-Signal), 2.39 (s, 3H), 1.40 (d, 6H).
LC/MS (Methode 17, ESIpos): $R_t$ = 1.48 min, m/z = 417.17 [M+H]$^+$.

## Beispiel 265

[1-{[3-Isopropyl-5-methyl-1-(oxetan-2-ylmethyl)-2,4-dioxo-1,2,3,4-tetrahydrothieno[2,3-d]-pyrimidin-6-yl]methyl}imidazolidin-2-yliden]cyanamid *(Enantiomer 1)*

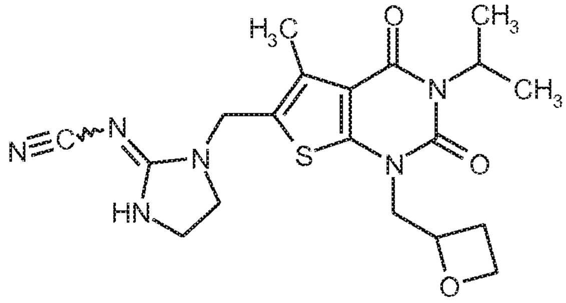

140 mg (0.336 mmol) der racemischen Verbindung aus Bsp. 264 wurden in 20ml Methanol/ Acetonitril (1:1) gelöst und in 20 Portionen über präparative SFC an chiraler Phase in die Enantiomere aufgetrennt [Säule: Daicel Chiralcel OX-H, 5 µm, SFC, 250 mm x 30 mm; Laufmittel: Kohlendioxid/Methanol 70:30; Fluss: 100 ml/min; Temperatur: 40°C; Detektion: 210 nm]. Nach Eindampfen der Produktfraktionen und Trocknen im Hochvakuum wurden 49 mg (70% d. Th.) von Enantiomer 1 erhalten (>99% ee, chirale analytische HPLC).
$^1$H-NMR (400 MHz, DMSO-$d_6$, δ/ppm): 8.06 (s, 1H), 5.13 (sept, 1H), 5.00 (quin, 1H), 4.54-4.37 (m, 2H), 4.46 (s, 2H), 4.11 (d, 2H), 3.53-3.35 (m, 4H), 2.76-2.62 (m, 1H), 2.53-2.44 (m, 1H, weitgehend überdeckt vom DMSO-Signal), 2.39 (s, 3H), 1.40 (d, 6H).

Chirale analytische SFC [Säule: Daicel Chiralcel OX-H, 5 µm, 250 mm x 4.6 mm; Laufmittel: Kohlendioxid/Methanol 70:30; Fluss: 3 ml/min; Temperatur: 40°C; Detektion: 210 nm]: $R_t$ = 6.10 min.

## Beispiel 266

[1-{[3-Isopropyl-5-methyl-1-(oxetan-2-ylmethyl)-2,4-dioxo-1,2,3,4-tetrahydrothieno[2,3-d]-pyrimidin-6-yl]methyl}imidazolidin-2-yliden]cyanamid (*Enantiomer 2*)

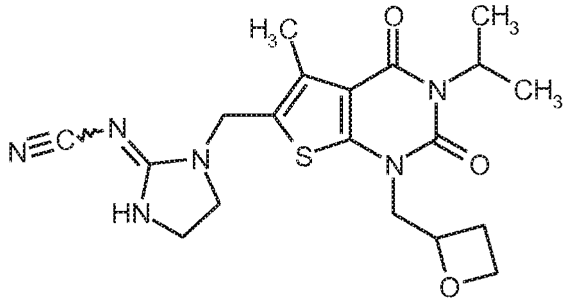

140 mg (0.336 mmol) der racemischen Verbindung aus Bsp. 264 wurden in 20 ml Methanol/ Acetonitril (1:1) gelöst und in 20 Portionen über präparative SFC an chiraler Phase in die Enantiomere aufgetrennt [Säule: Daicel Chiralcel OX-H, 5 µm, SFC, 250 mm x 30 mm; Laufmittel: Kohlendioxid/Methanol 70:30; Fluss: 100 ml/min; Temperatur: 40°C; Detektion: 210 nm]. Nach Eindampfen der Produktfraktionen und Trocknen im Hochvakuum wurden 50 mg (71% d. Th.) von Enantiomer 2 erhalten (98.6% ee, chirale analytische HPLC).
$^1$H-NMR (400 MHz, DMSO-$d_6$, δ/ppm): 8.06 (s, 1H), 5.13 (sept, 1H), 5.05-4.93 (m, 1H), 4.54-4.35 (m, 2H), 4.46 (s, 2H), 4.11 (d, 2H), 3.50-3.36 (m, 4H), 2.76-2.62 (m, 1H), 2.54-2.44 (m, 1H, weitgehend überdeckt vom DMSO-Signal), 2.39 (s, 3H), 1.40 (d, 6H).

Chirale analytische SFC [Säule: Daicel Chiralcel OX-H, 5 µm, 250 mm x 4.6 mm; Laufmittel: Kohlendioxid/Methanol 70:30; Fluss: 3 ml/min; Temperatur: 40°C; Detektion: 210 nm]: $R_t$ = 8.36 min.

## Beispiel 267

[1-{[3-Isopropyl-5-methyl-2,4-dioxo-1-(tetrahydrofuran-2-ylmethyl)-1,2,3,4-tetrahydrothieno-[2,3-d]pyrimidin-6-yl]methyl}imidazolidin-2-yliden]cyanamid (*Racemat*)

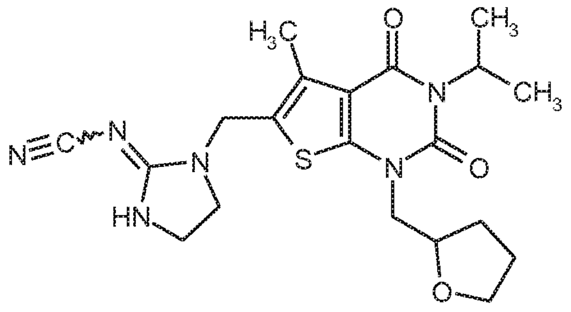

900 mg (1.18 mmol, 50% Reinheit) der Verbindung aus Bsp. 312A wurden in 12 ml DMF gelöst und mit 259 mg (1.77 mmol) Dimethyl-*N*-cyanodithioiminocarbonat sowie 327 mg (2.36 mmol) Kaliumcarbonat versetzt. Das Gemisch wurde 4.5 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mit Diisopropylether, der mit einigen Tropfen Ethylacetat versetzt war, verrührt. Der Feststoff wurde abgesaugt und im Hochvakuum getrocknet. Dies ergab eine erste Fraktion der Titelverbindung (290 mg). Die Mutterlauge des Verrührens wurde mittels präparativer HPLC gereinigt (Methode 8). Dies ergab eine zweite Fraktion des Produktes (54 mg), die mit der ersten vereinigt wurde. Insgesamt wurden so 344 mg (67% d. Th.) der Titelverbindung erhalten.

$^1$H-NMR (400 MHz, DMSO-$d_6$, δ/ppm): 8.06 (s, 1H), 5.14 (sept, 1H), 4.46 (s, 2H), 4.29-4.15 (m, 1H), 4.04 (dd, 1H), 3.75 (q, 1H), 3.69-3.57 (m, 2H), 3.50-3.36 (m, 4H), 2.39 (s, 3H), 2.06-1.74 (m, 3H), 1.72-1.59 (m, 1H), 1.40 (d, 6H).

LC/MS (Methode 17, ESIpos): $R_t$ = 1.63 min, m/z = 431.18 $[M+H]^+$.

## Beispiel 268

[1-{[1-(2-Methoxyethyl)-5-methyl-2,4-dioxo-3-(2,2,2-trifluorethyl)-1,2,3,4-tetrahydrothieno-[2,3-d]pyrimidin-6-yl]methyl}imidazolidin-2-yliden]cyanamid

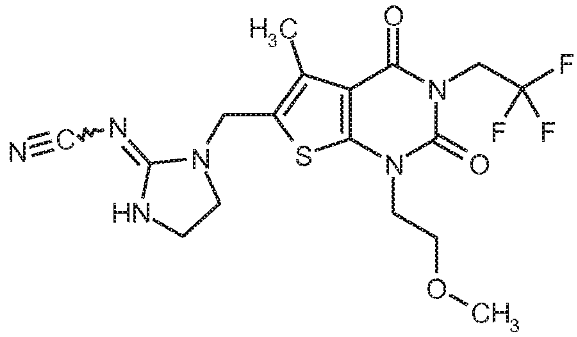

300 mg (0.761 mmol) der Verbindung aus Bsp. 249A wurden in 4.4 ml DMF gelöst und mit 167 mg (1.14 mmol) Dimethyl-*N*-cyanodithioiminocarbonat sowie 210 mg (1.52 mmol) Kaliumcarbonat versetzt. Das Gemisch wurde 3.75 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 8). Dies ergab 154 mg (45% d. Th.) der Titelverbindung.

$^1$H-NMR (400 MHz, DMSO-$d_6$, δ/ppm): 8.09 (s, 1H), 4.70 (q, 2H), 4.49 (s, 2H), 4.06 (t, 2H), 3.64 (t, 2H), 3.53-3.36 (m, 4H), 3.24 (s, 3H), 2.41 (s, 3H).

LC/MS (Methode 6, ESIpos): $R_t$ = 1.51 min, m/z = 445 $[M+H]^+$.

**Beispiel 269**

[1-{[5-Methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl}imidazolidin-2-yliden]cyanamid (*Racemat*)

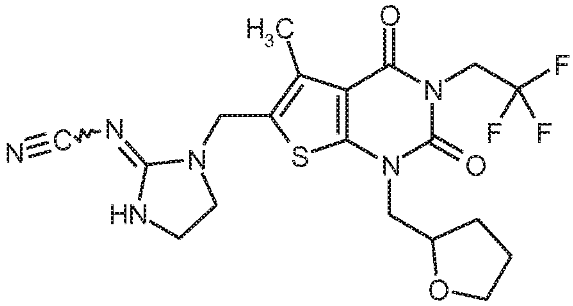

890 mg (1.78 mmol, 84% Reinheit) der Verbindung aus Bsp. 252A wurden in 14.5 ml DMF gelöst und mit 390 mg (2.67 mmol) Dimethyl-*N*-cyanodithioiminocarbonat sowie 491 mg (3.56 mmol) Kaliumcarbonat versetzt. Das Gemisch wurde 4.5 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 8). Nach Eindampfen der Produktfraktionen und Trocknen im Hochvakuum wurden 210 mg (25% d. Th.) der Titelverbindung erhalten.

$^1$H-NMR (400 MHz, DMSO-$d_6$, $\delta$/ppm): 8.09 (s, 1H), 4.70 (q, 2H), 4.49 (s, 2H), 4.28-4.18 (m, 1H), 4.07 (dd, 1H), 3.81-3.70 (m, 2H), 3.67-3.58 (m, 1H), 3.51-3.37 (m, 4H), 2.41 (s, 3H), 2.06-1.75 (m, 3H), 1.73-1.61 (m, 1H).

LC/MS (Methode 1, ESIpos): $R_t$ = 0.87 min, m/z = 471 $[M+H]^+$.

**Beispiel 270**

1-[2-(Cyclopropyloxy)ethyl]-6-[(2,3-dioxopiperazin-1-yl)methyl]-3-ethyl-5-methylthieno[2,3-d]-pyrimidin-2,4(1*H*,3*H*)-dion

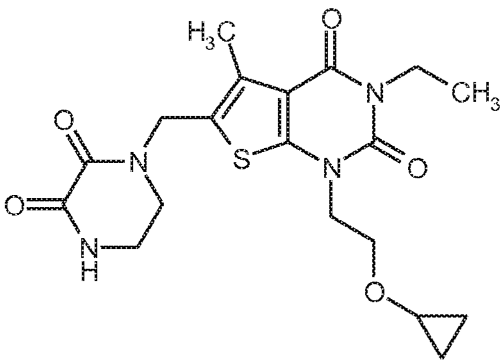

105 mg (0.203 mmol) der Verbindung aus Bsp. 308A wurden in 4.5 ml Ethanol gelöst und mit 300 mg (2.03 mmol) Oxalsäurediethylester versetzt. Das Gemisch wurde 18 h bei 80°C in einer Mikrowellenapparatur gerührt. Danach wurde die Reaktionslösung am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 55 mg (64% d. Th.) der Titelverbindung erhalten.

$^1$H-NMR (400 MHz, DMSO-$d_6$, $\delta$/ppm): 8.64 (br. s, 1H), 4.68 (s, 2H), 4.00 (t, 2H), 3.89 (q, 2H), 3.72 (t, 2H), 3.50-3.44 (m, 2H), 3.34-3.28 (m, 3H), 2.42 (s, 3H), 1.11 (t, 3H), 0.42-0.33 (m, 4H).

LC/MS (Methode 3, ESIpos): $R_t$ = 0.89 min, m/z = 421 $[M+H]^+$.

**Beispiel 271**

6-[(2,3-Dioxopiperazin-1-yl)methyl]-1-(2-ethoxyethyl)-3-isobutyl-5-methylthieno[2,3-d]pyrimidin-2,4(1*H*,3*H*)-dion

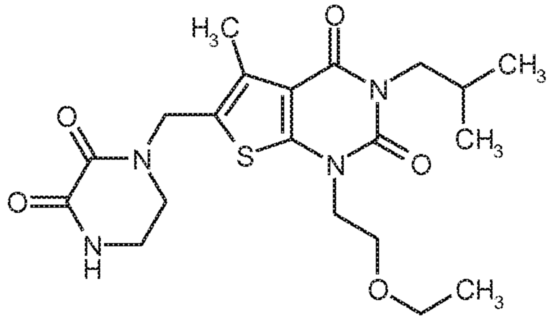

158 mg (0.343 mmol) der Verbindung aus Bsp. 314A wurden in 5 ml Ethanol gelöst und mit 101 mg (0.686 mmol) Oxalsäurediethylester versetzt. Das Gemisch wurde 18 h bei 80°C in einer Mikrowellenapparatur gerührt. Danach wurde die Reaktionslösung am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 45 mg (30% d. Th.) der Titelverbindung erhalten.
$^1$H-NMR (400 MHz, DMSO-$d_6$, δ/ppm): 8.62 (br. s, 1H), 4.69 (s, 2H), 4.01 (t, 2H), 3.71 (d, 2H), 3.65 (t, 2H), 3.52-3.46 (m, 2H), 3.42 (q, 2H), 2.42 (s, 3H), 2.09-1.97 (m, 1H), 1.01 (t, 3H), 0.84 (d, 6H).
LC/MS (Methode 3, ESIpos): $R_t$ = 1.02 min, m/z = 437 $[M+H]^+$.

**Beispiel 272**

3-(2,2-Dimethylpropyl)-6-[(2,3-dioxopiperazin-1-yl)methyl]-5-methyl-1-(3,3,3-trifluorpropyl)-thieno[2,3-d]pyrimidin-2,4(1*H*,3*H*)-dion

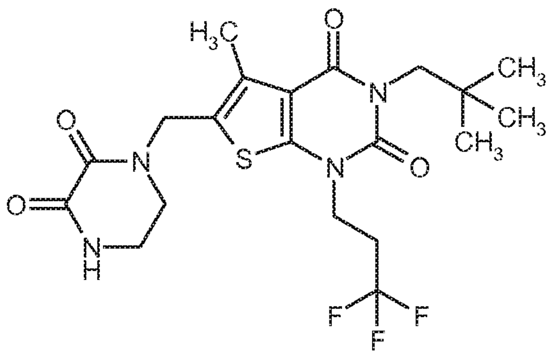

160 mg (0.316 mmol) der Verbindung aus Bsp. 315A wurden in 5 ml Ethanol gelöst und mit 466 mg (3.15 mmol) Oxalsäurediethylester versetzt. Das Gemisch wurde 21 h bei 80°C gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 76 mg (50% d. Th.) der Titelverbindung erhalten.
$^1$H-NMR (400 MHz, DMSO-$d_6$, δ/ppm): 8.64 (br. s, 1H), 4.71 (s, 2H), 4.10 (t, 2H), 3.81 (br. s, 2H), 3.55-3.46 (m, 2H), 2.84-2.68 (m, 2H), 2.43 (s, 3H), 0.89 (s, 9H).
LC/MS (Methode 3, ESIneg): $R_t$ = 1.16 min, m/z = 519 $[M-H+HCOOH]^-$.

**Beispiel 273**

3-(2,2-Dimethylpropyl)-6-[(2,3-dioxopiperazin-1-yl)methyl]-1-(3-fluorpropyl)-5-methylthieno-[2,3-d]pyrimidin-2,4(1*H*,3*H*)-dion

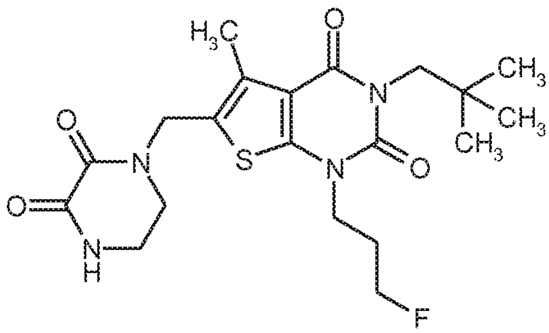

103 mg (0.22 mmol) der Verbindung aus Bsp. 316A wurden in 3.5 ml Ethanol gelöst und mit 324 mg (2.19 mmol) Oxalsäurediethylester versetzt. Das Gemisch wurde 21 h bei 80°C gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 55 mg (55% d. Th.) der Titelverbindung erhalten.
$^1$H-NMR (400 MHz, DMSO-$d_6$, δ/ppm): 8.64 (br. s, 1H), 4.70 (s, 2H), 4.59 (t, 1H), 4.47 (t, 1H), 3.98 (t, 2H), 3.80 (br. s, 2H), 3.54-3.47 (m, 2H), 2.42 (s, 3H), 2.12-2.05 (m, 1H), 2.02 (t, 1H), 0.89 (s, 9H).
LC/MS (Methode 3, ESIneg): $R_t$ = 1.06 min, m/z = 483 $[M-H+HCOOH]^-$.

**Beispiel 274**

3-(2,2-Dimethylpropyl)-6-[(2,3-dioxopiperazin-1-yl)methyl]-1-(2-methoxyethyl)-5-methylthieno-[2,3-d]pyrimidin-2,4(1*H*,3*H*)-dion

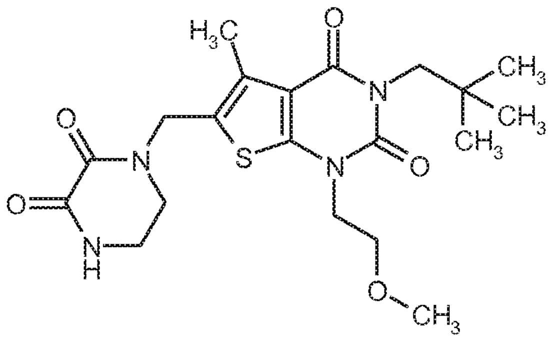

172 mg (0.364 mmol) der Verbindung aus Bsp. 317A wurden in 5 ml Ethanol gelöst und mit 537 mg (3.64 mmol) Oxalsäurediethylester versetzt. Das Gemisch wurde 18 h bei 80°C gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 84 mg (52% d. Th.) der Titelverbindung erhalten.
$^1$H-NMR (400 MHz, DMSO-$d_6$, δ/ppm): 8.64 (br. s, 1H), 4.68 (s, 2H), 4.02 (t, 2H), 3.81 (br. s, 2H), 3.61 (t, 2H), 3.53-3.46 (m, 2H), 3.22 (s, 3H), 2.41 (s, 3H), 0.88 (s, 9H).
LC/MS (Methode 3, ESIpos): $R_t$ = 1.03 min, m/z = 437 $[M+H]^+$.

**Beispiel 275**

3-(2,2-Difluorethyl)-6-[(2,3-dioxopiperazin-1-yl)methyl]-1,5-dimethylthieno[2,3-d]pyrimidin-2,4(1*H*,3*H*)-dion

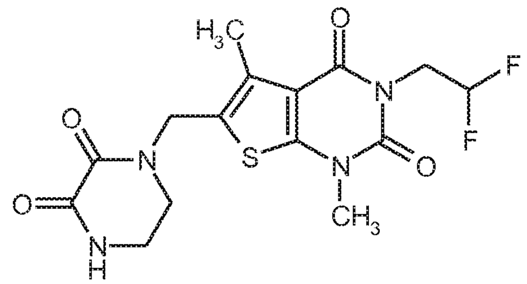

65 mg (0.137 mmol) der Verbindung aus Bsp. 318A wurden in 2.5 ml Ethanol gelöst und mit 202 mg (1.37 mmol) Oxalsäurediethylester versetzt. Das Gemisch wurde 18 h bei 80°C in einer Mikrowellenapparatur gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 32 mg (61% d. Th.) der Titelverbindung erhalten.
$^1$H-NMR (400 MHz, DMSO-$d_6$, δ/ppm): 8.65 (br. s, 1H), 6.36-6.05 (m, 1H), 4.71 (s, 2H), 4.29 (td, 2H), 3.53-3.46 (m, 2H), 3.43 (s, 3H), 2.44 (s, 3H).
LC/MS (Methode 3, ESIpos): $R_t$ = 0.77 min, m/z = 387 $[M+H]^+$.

**Beispiel 276**

6-[(2,3-Dioxopiperazin-1-yl)methyl]-3-(2-methoxyethyl)-5-methyl-1-(3,3,3-trifluorpropyl)thieno-[2,3-d]pyrimidin-2,4(1*H*,3*H*)-dion

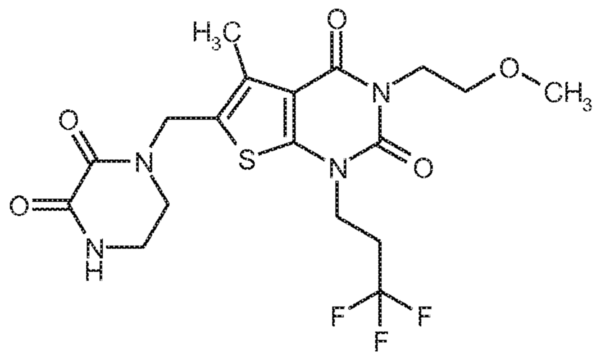

77 mg (0.153 mmol) der Verbindung aus Bsp. 319A wurden in 3 ml Ethanol gelöst und mit 112 mg (0.764 mmol) Oxalsäurediethylester versetzt. Das Gemisch wurde 18 h bei 80°C in einer Mikrowellenapparatur gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 41 mg (58% d. Th.) der Titelverbindung erhalten.
$^1$H-NMR (400 MHz, DMSO-$d_6$, δ/ppm): 8.63 (br. s, 1H), 4.71 (s, 2H), 4.10 (t, 2H), 4.05 (t, 2H), 3.49 (t, 4H), 3.23 (s, 3H), 2.82-2.69 (m, 2H), 2.44 (s, 3H).
LC/MS (Methode 3, ESIpos): $R_t$ = 0.87 min, m/z = 463 $[M+H]^+$.

**Beispiel 277**

6-[(2,3-Dioxopiperazin-1-yl)methyl]-1-(3-fluorpropyl)-3-(2-methoxyethyl)-5-methylthieno[2,3-d]-pyrimidin-2,4(1*H*,3*H*)-dion

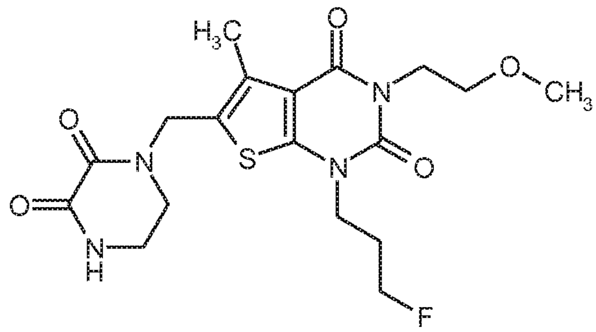

65 mg (0.112 mmol) der Verbindung aus Bsp. 320A wurden in 5 ml Ethanol gelöst und mit 165 mg (1.12 mmol) Oxalsäurediethylester versetzt. Das Gemisch wurde 18 h bei 80°C gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 25 mg (47% d. Th.) der Titelverbindung erhalten.
$^1$H-NMR (400 MHz, DMSO-$d_6$, δ/ppm): 8.64 (br. s, 1H), 4.70 (s, 2H), 4.59 (t, 1H), 4.47 (t, 1H), 4.05 (t, 2H), 3.98 (t, 2H), 3.49 (d, 2H), 3.31 (br. s, 2H), 3.24 (s, 3H), 2.43 (s, 3H), 2.13-2.05 (m, 1H), 2.05-1.98 (m, 1H).
LC/MS (Methode 3, ESIpos): $R_t$ = 0.77 min, m/z = 427 $[M+H]^+$.

**Beispiel 278**

6-[(2,3-Dioxopiperazin-1-yl)methyl]-3-(2-methoxyethyl)-5-methyl-1-(tetrahydrofuran-2-ylmethyl)-thieno[2,3-d]pyrimidin-2,4(1*H*,3*H*)-dion *(Racemat)*

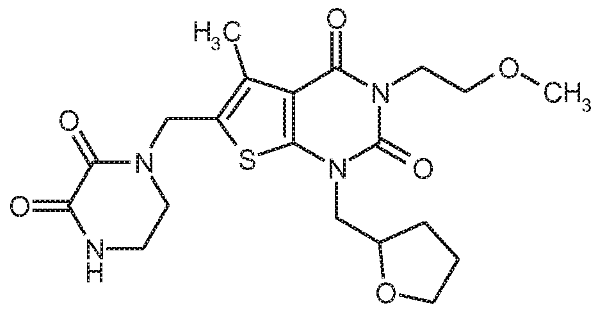

100 mg (0.166 mmol) der Verbindung aus Bsp. 321A wurden in 5 ml Ethanol gelöst und mit 245 mg (1.66 mmol) Oxalsäurediethylester versetzt. Das Gemisch wurde 18 h bei 80°C gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 43 mg (56% d. Th.) der Titelverbindung erhalten.
$^1$H-NMR (400 MHz, DMSO-$d_6$, δ/ppm): 8.64 (br. s, 1H), 4.74-4.64 (m, 2H), 4.25-4.17 (m, 1H), 4.09-3.99 (m, 3H), 3.77-3.66 (m, 2H), 3.64-3.57 (m, 1H), 3.49 (q, 4H), 3.32-3.28 (m, 2H), 3.23 (s, 3H), 2.42 (s, 3H), 2.02-1.93 (m, 1H), 1.93-1.75 (m, 2H), 1.70-1.60 (m, 1H).
LC/MS (Methode 3, ESIpos): $R_t$ = 0.79 min, m/z = 451 $[M+H]^+$.

**Beispiel 279**

6-[(2,3-Dioxopiperazin-1-yl)methyl]-3-(2-methoxy-2-methylpropyl)-5-methyl-1-(3,3,3-trifluorpropyl)thieno[2,3-d]pyrimidin-2,4(1*H*,3*H*)-dion

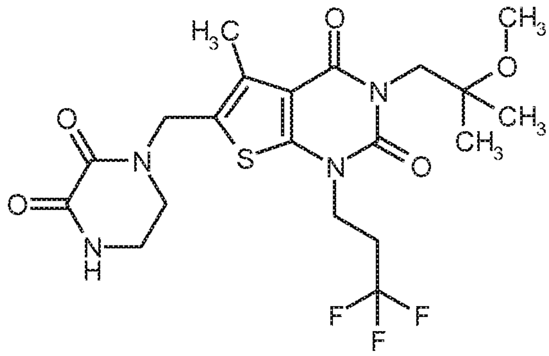

160 mg (0.297 mmol) der Verbindung aus Bsp. 322A wurden in 5 ml Ethanol gelöst und mit 438 mg (2.97 mmol) Oxalsäurediethylester versetzt. Das Gemisch wurde 19 h bei 80°C gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach

Vereinigen der Produktfraktionen und Gefriertrocknung wurden 64 mg (40% d. Th.) der Titelverbindung erhalten.
$^1$H-NMR (400 MHz, DMSO-$d_6$, $\delta$/ppm): 8.64 (br. s, 1H), 4.71 (s, 2H), 4.10 (t, 2H), 3.99 (br. s, 2H), 3.54-3.47 (m, 2H), 3.15 (s, 3H), 2.82-2.68 (m, 2H), 2.43 (s, 3H), 1.08 (s, 6H).
LC/MS (Methode 3, ESIpos): $R_t$ = 0.98 min, m/z = 491 $[M+H]^+$.

**Beispiel 280**

6-[(2,3-Dioxopiperazin-1-yl)methyl]-1-(3-fluorpropyl)-3-(2-methoxy-2-methylpropyl)-5-methyl-thieno[2,3-d]pyrimidin-2,4(1*H*,3*H*)-dion

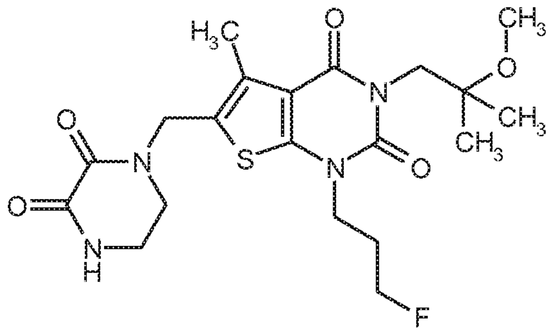

200 mg (0.424 mmol) der Verbindung aus Bsp. 323A wurden in 5 ml Ethanol gelöst und mit 626 mg (4.24 mmol) Oxalsäurediethylester versetzt. Das Gemisch wurde 18 h bei 80°C gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 91 mg (47% d. Th.) der Titelverbindung erhalten.
$^1$H-NMR (400 MHz, DMSO-$d_6$, $\delta$/ppm): 8.64 (br. s, 1H), 4.70 (s, 2H), 4.58 (t, 1H), 4.47 (t, 1H), 4.07-3.90 (m, 4H), 3.54-3.46 (m, 2H), 3.15 (s, 3H), 2.43 (s, 3H), 2.13-2.05 (m, 1H), 2.02 (quin, 1H), 1.08 (s, 6H).
LC/MS (Methode 3, ESIpos): $R_t$ = 0.89 min, m/z = 455 $[M+H]^+$.

**Beispiel 281**

6-[(2,3-Dioxopiperazin-1-yl)methyl]-1-(2-methoxyethyl)-3-(2-methoxy-2-methylpropyl)-5-methyl-thieno[2,3-d]pyrimidin-2,4(1*H*,3*H*)-dion

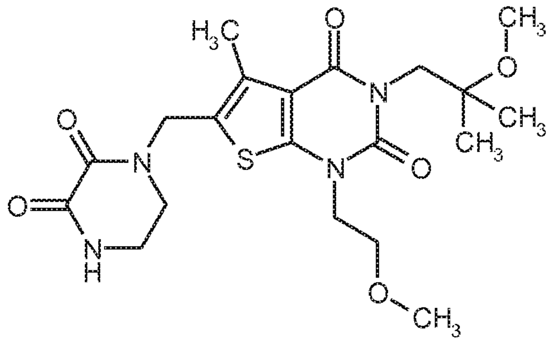

100 mg (0.196 mmol) der Verbindung aus Bsp. 324A wurden in 5 ml Ethanol gelöst und mit 289 mg (1.96 mmol) Oxalsäurediethylester versetzt. Das Gemisch wurde 18 h bei 80°C in einer Mikrowellenapparatur gerührt. Danach wurde die Reaktionslösung am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 58 mg (62% d. Th.) der Titelverbindung erhalten.
$^1$H-NMR (400 MHz, DMSO-$d_6$, $\delta$/ppm): 8.64 (br. s, 1H), 4.69 (s, 2H), 4.08-3.94 (m, 4H), 3.61 (t, 2H), 3.50 (dd, 2H), 3.22 (s, 3H), 3.15 (s, 3H), 2.42 (s, 3H), 1.08 (s, 6H).
LC/MS (Methode 3, ESIpos): $R_t$ = 0.85 min, m/z = 453 $[M+H]^+$.

**Beispiel 282**

5-Methyl-6-[(5-oxo-2,5-dihydro-1*H*-pyrazol-1-yl)methyl]-3-(2,2,2-trifluorethyl)-1-(3,3,3-trifluorpropyl)thieno[2,3-d]pyrimidin-2,4(1*H*,3*H*)-dion

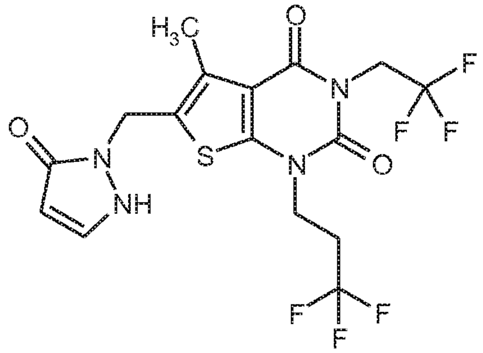

Analog zu dem unter Bsp. 133 beschriebenen Verfahren wurden aus 365 mg (0.606 mmol) der Verbindung aus Bsp. 427A 164 mg (59% d. Th.) der Titelverbindung hergestellt.
$^1$H-NMR (400 MHz, DMSO-$d_6$, $\delta$/ppm): 11.17 (s, 1H), 7.16 (d, 1H), 5.33 (d, 1H), 5.18 (s, 2H), 4.69 (q, 2H), 4.10 (t, 2H), 2.84-2.64 (m, 2H), 2.54 (s, 3H).
LC/MS (Methode 17, ESIneg): $R_t$ = 1.61 min, m/z = 455.06 $[M-H]^-$.

**Beispiel 283**

1-(2-Methoxyethyl)-5-methyl-6-[(5-oxo-2,5-dihydro-1*H*-pyrazol-1-yl)methyl]-3-(2,2,2-trifluorethyl)thieno[2,3-d]pyrimidin-2,4(1*H*,3*H*)-dion

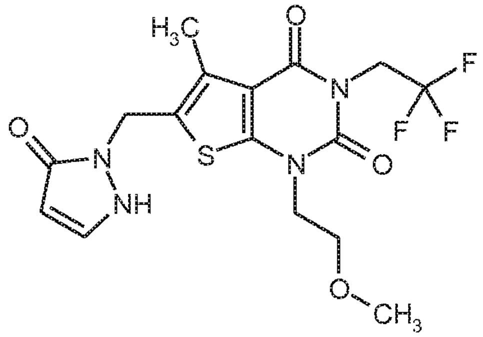

Analog zu dem unter Bsp. 133 beschriebenen Verfahren wurden aus 370 mg (0.655 mmol) der Verbindung aus Bsp. 428A 180 mg (65% d. Th.) der Titelverbindung hergestellt.
$^1$H-NMR (400 MHz, DMSO-$d_6$, δ/ppm): 11.15 (br. s, 1H), 7.17 (s, 1H), 5.33 (d, 1H), 5.16 (s, 2H), 4.69 (q, 2H), 4.02 (t, 2H), 3.61 (t, 2H), 3.21 (s, 3H), 2.46 (s, 3H).
LC/MS (Methode 17, ESIneg): $R_t$ = 1.35 min, m/z = 417.09 $[M-H]^-$.

**Beispiel 284**

5-Methyl-6-[(5-oxo-2,5-dihydro-1*H*-pyrazol-1-yl)methyl]-1-(tetrahydrofuran-2-ylmethyl)-3-(2,2,2-trifluorethyl)thieno[2,3-d]pyrimidin-2,4(1*H*,3*H*)-dion *(Racemat)*

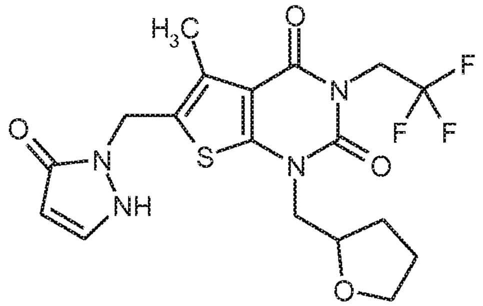

Analog zu dem unter Bsp. 133 beschriebenen Verfahren wurden aus 695 mg (1.18 mmol) der Verbindung aus Bsp. 429A 230 mg (43% d. Th.) der Titelverbindung hergestellt.
$^1$H-NMR (400 MHz, DMSO-$d_6$, δ/ppm): 11.14 (s, 1H), 7.15 (d, 1H), 5.33 (d, 1H), 5.16 (s, 2H), 4.69 (q, 2H), 4.26-4.14 (m, 1H), 4.04 (dd, 1H), 3.77-3.65 (m, 2H), 3.64-3.53 (m, 1H), 2.46 (s, 3H), 2.04-1.74 (m, 3H), 1.71-1.59 (m, 1H).
LC/MS (Methode 17, ESIneg): $R_t$ = 1.46 min, m/z = 443.10 $[M-H]^-$.

**Beispiel 285**

5-Methyl-6-[(5-oxo-2,5-dihydro-1*H*-pyrazol-1-yl)methyl]-1-(tetrahydrofuran-2-ylmethyl)-3-(2,2,2-trifluorethyl)thieno[2,3-d]pyrimidin-2,4(1*H*,3*H*)-dion (*Enantiomer 1*)

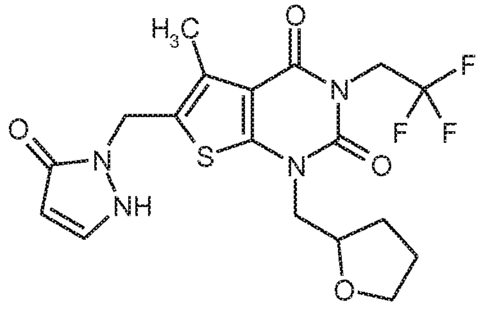

199 mg (0.448 mmol) der racemischen Verbindung aus Bsp. 284 wurden in einem Gemisch aus 8 ml Ethanol und 1 ml Acetonitril gelöst und in 36 Portionen über präparative HPLC an chiraler Phase in die Enantiomere aufgetrennt [Säule: Daicel Chiralcel OZ-H, 5 μm, 250 mm x 20 mm; Laufmittel: Isohexan/Ethanol 1:1, mit 0.2% Essigsäure im Ethanol; Fluss: 15 ml/min; Temperatur: 35°C; Detektion: 220 nm]. Nach Eindampfen der Produktfraktionen und Trocknen im Hochvakuum wurden 88 mg (88% d. Th.) von Enantiomer 1 erhalten (>99% ee, chirale analytische HPLC).
$^1$H-NMR (400 MHz, DMSO-$d_6$, δ/ppm): 11.17 (br. s, 1H), 7.15 (s, 1H), 5.32 (s, 1H), 5.15 (s, 2H), 4.69 (q, 2H), 4.25-4.14 (m, 1H), 4.04 (dd, 1H), 3.77-3.65 (m, 2H), 3.63-3.54 (m, 1H), 2.46 (s, 3H), 2.05-1.75 (m, 3H), 1.71-1.59 (m, 1H).
Chirale analytische HPLC [Säule: Daicel Chiralcel OZ-H, 5 μm, 250 mm x 4.6 mm; Laufmittel: Isohexan/Ethanol 1:1, mit 0.2% TFA und 1% Wasser im Ethanol; Fluss: 1 ml/min; Temperatur: 40°C; Detektion: 220 nm]: $R_t$ = 5.61 min.

**Beispiel 286**

5-Methyl-6-[(5-oxo-2,5-dihydro-1*H*-pyrazol-1-yl)methyl]-1-(tetrahydrofuran-2-ylmethyl)-3-(2,2,2-trifluorethyl)thieno[2,3-d]pyrimidin-2,4(1*H*,3*H*)-dion (*Enantiomer 2*)

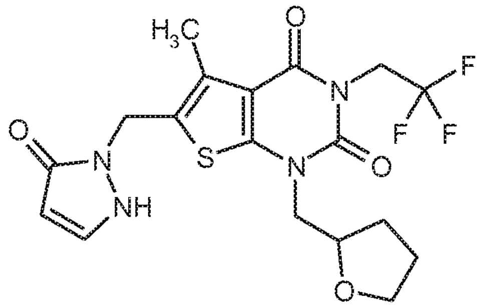

199 mg (0.448 mmol) der racemischen Verbindung aus Bsp. 284 wurden in einem Gemisch aus 8 ml Ethanol und 1 ml Acetonitril gelöst und in 36 Portionen über präparative HPLC an chiraler Phase in die Enantiomere aufgetrennt [Säule: Daicel Chiralcel OZ-H, 5 μm, 250 mm x 20 mm; Laufmittel: Isohexan/Ethanol 1:1, mit 0.2% Essigsäure im Ethanol; Fluss: 15 ml/min; Temperatur: 35°C; Detektion: 220 nm]. Nach Eindampfen der Produktfraktionen und Trocknen im Hochvakuum wurden 80 mg (80% d. Th.) von Enantiomer 2 erhalten (95% ee, chirale analytische HPLC).
$^1$H-NMR (400 MHz, DMSO-$d_6$, δ/ppm): 11.18 (br. s, 1H), 7.16 (s, 1H), 5.32 (s, 1H), 5.15 (s, 2H), 4.69 (q, 2H), 4.25-4.13 (m, 1H), 4.04 (dd, 1H), 3.77-3.65 (m, 2H), 3.63-3.53 (m, 1H), 2.46 (s, 3H), 2.04-1.75 (m, 3H), 1.71-1.59 (m, 1H).
Chirale analytische HPLC [Säule: Daicel Chiralcel OZ-H, 5 μm, 250 mm x 4.6 mm; Laufmittel: Isohexan/Ethanol 1:1, mit 0.2% TFA und 1% Wasser im Ethanol; Fluss: 1 ml/min; Temperatur: 40°C; Detektion: 220 nm]: $R_t$ = 6.36 min.

**Beispiel 287**

3-(2-Methoxyethyl)-5-methyl-6-[(5-oxo-2,5-dihydro-1*H*-pyrazol-1-yl)methyl]-1-(3,3,3-trifluor-propyl)thieno[2,3-d]pyrimidin-2,4(1*H*,3*H*)-dion

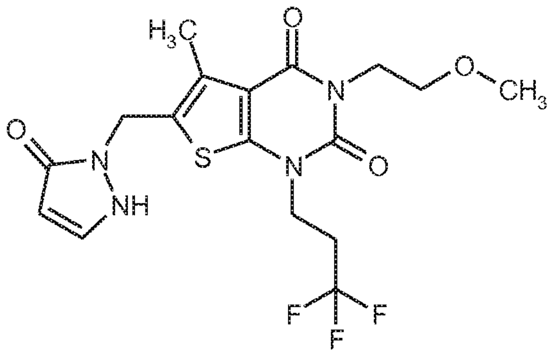

Analog zu dem unter Bsp. 133 beschriebenen Verfahren wurden aus 270 mg (0.467 mmol) der Verbindung aus Bsp. 430A 90 mg (44% d. Th.) der Titelverbindung hergestellt. Die Reaktionszeit betrug hier 15 min.
$^1$H-NMR (400 MHz, DMSO-$d_6$, $\delta$/ppm): 11.19 (breit, 1H), 7.16 (br. s, 1H), 5.31 (d, 1H), 5.16 (s, 2H), 4.06 (t, 2H), 4.05 (t, 2H), 3.48 (t, 2H), 3.23 (s, 3H), 2.83-2.64 (m, 2H), 2.48 (s, 3H).
LC/MS (Methode 17, ESIneg): $R_t$ = 1.35 min, m/z = 431.10 $[M-H]^-$.

## Beispiel 288

1,3-Bis(2-methoxyethyl)-5-methyl-6-[(5-oxo-2,5-dihydro-1*H*-pyrazol-1-yl)methyl]thieno[2,3-d]-pyrimidin-2,4(1*H*,3*H*)-dion

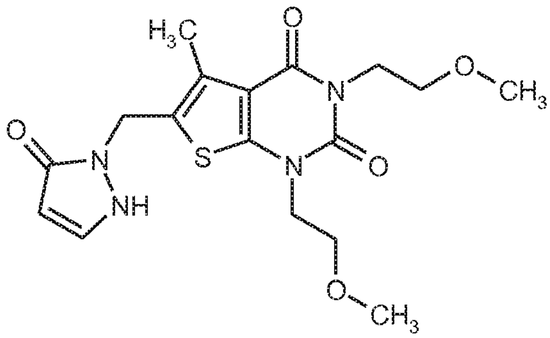

Analog zu dem unter Bsp. 133 beschriebenen Verfahren wurden aus 285 mg (0.527 mmol) der Verbindung aus Bsp. 431A 109 mg (52% d. Th.) der Titelverbindung hergestellt. Die Reaktionszeit betrug hier 15 min.
$^1$H-NMR (400 MHz, DMSO-$d_6$, $\delta$/ppm): 7.19 (d, 1H), 5.34 (d, 1H), 5.15 (s, 2H), 4.05 (t, 2H), 3.98 (t, 2H), 3.60 (t, 2H), 3.48 (t, 2H), 3.23 (s, 3H), 3.21 (s, 3H), 2.46 (s, 3H).
LC/MS (Methode 17, ESIneg): $R_t$ = 1.05 min, m/z = 393.12 $[M-H]^-$.

## Beispiel 289

3-(2-Methoxyethyl)-5-methyl-6-[(5-oxo-2,5-dihydro-1*H*-pyrazol-1-yl)methyl]-1-(tetrahydrofuran-2-ylmethyl)thieno[2,3-d]pyrimidin-2,4(1*H*,3*H*)-dion *(Racemat)*

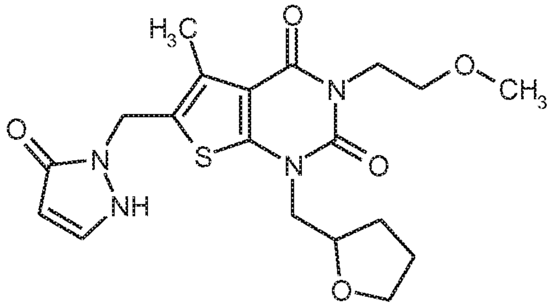

Analog zu dem unter Bsp. 133 beschriebenen Verfahren wurden aus 450 mg (0.794 mmol) der Verbindung aus Bsp. 432A 156 mg (46% d. Th.) der Titelverbindung hergestellt.
$^1$H-NMR (400 MHz, DMSO-$d_6$, δ/ppm): 11.12 (s, 1H), 7.14 (d, 1H), 5.32 (d, 1H), 5.14 (s, 2H), 4.24-4.13 (m, 1H), 4.09-3.97 (m, 3H), 3.76-3.54 (m, 3H), 3.48 (t, 2H), 3.23 (s, 3H), 2.46 (s, 3H), 2.02-1.73 (m, 3H), 1.70-1.58 (m, 1H).
LC/MS (Methode 17, ESIneg): $R_t$ = 1.11 min, m/z = 419.14 [M-H]$^{-}$.

**Beispiel 290**

3-(2-Methoxyethyl)-5-methyl-6-[(5-oxo-2,5-dihydro-1*H*-pyrazol-1-yl)methyl]-1-(tetrahydrofuran-2-ylmethyl)thieno[2,3-d]pyrimidin-2,4(1*H*,3*H*)-dion (*Enantiomer 1*)

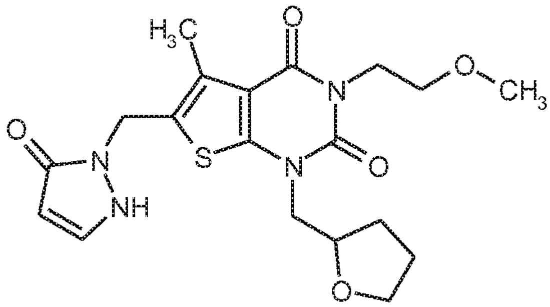

150 mg (0.357 mmol) der racemischen Verbindung aus Bsp. 289 wurden in einem Gemisch aus 8 ml Ethanol und 2 ml Acetonitril gelöst und in 20 Portionen über präparative HPLC an chiraler Phase in die Enantiomere aufgetrennt [Säule: Daicel Chiralcel OX-H, 5 μm, 250 mm x 20 mm; Laufmittel: Isohexan/Ethanol 1:1; Fluss: 15 ml/min; Temperatur: 50°C; Detektion: 220 nm]. Nach Eindampfen der Produktfraktionen und Trocknen im Hochvakuum wurden 72 mg (96% d. Th.) von Enantiomer 1 erhalten (>99% ee, chirale analytische HPLC).
$^1$H-NMR (400 MHz, DMSO-$d_6$, δ/ppm): 11.14 (br. s, 1H), 7.15 (s, 1H), 5.32 (s, 1H), 5.14 (s, 2H), 4.24-4.13 (m, 1H), 4.09-3.97 (m, 3H), 3.77-3.54 (m, 3H), 3.48 (t, 2H), 3.23 (s, 3H), 2.46 (s, 3H), 2.03-1.73 (m, 3H), 1.70-1.57 (m, 1H).
Chirale analytische HPLC [Säule: Daicel Chiralpak IC, 5 μm, 250 mm x 4.6 mm; Laufmittel: Isohexan/Ethanol 2:3; Fluss: 1 ml/min; Temperatur: 35°C; Detektion: 220 nm]: $R_t$ = 10.00 min.

**Beispiel 291**

3-(2-Methoxyethyl)-5-methyl-6-[(5-oxo-2,5-dihydro-1*H*-pyrazol-1-yl)methyl]-1-(tetrahydrofuran-2-ylmethyl)thieno[2,3-d]pyrimidin-2,4(1*H*,3*H*)-dion (*Enantiomer 2*)

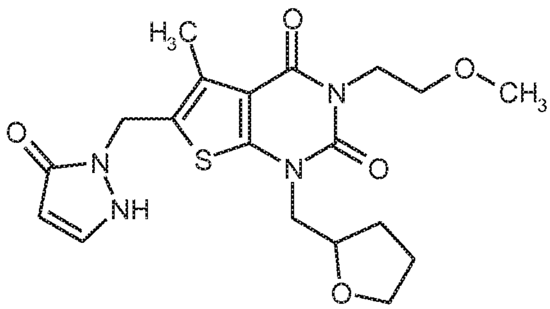

150 mg (0.357 mmol) der racemischen Verbindung aus Bsp. 289 wurden in einem Gemisch aus 8 ml Ethanol und 2 ml Acetonitril gelöst und in 20 Portionen über präparative HPLC an chiraler Phase in die Enantiomere aufgetrennt [Säule: Daicel Chiralcel OX-H, 5 μm, 250 mm x 20 mm; Laufmittel: Isohexan/Ethanol 1:1; Fluss: 15 ml/min; Temperatur: 50°C; Detektion: 220 nm]. Nach Eindampfen der Produktfraktionen und Trocknen im Hochvakuum wurden 73 mg (97% d. Th.) von Enantiomer 2 erhalten (98% ee, chirale analytische HPLC).

$^1$H-NMR (400 MHz, DMSO-$d_6$, δ/ppm): 11.15 (breit, 1H), 7.15 (br. s, 1H), 5.32 (s, 1H), 5.14 (s, 2H), 4.23-4.13 (m, 1H), 4.09-3.96 (m, 3H), 3.76-3.54 (m, 3H), 3.48 (t, 2H), 3.23 (s, 3H), 2.46 (s, 3H), 2.03-1.74 (m, 3H), 1.71-1.58 (m, 1H).

Chirale analytische HPLC [Säule: Daicel Chiralpak IC, 5 μm, 250 mm x 4.6 mm; Laufmittel: Isohexan/Ethanol 2:3; Fluss: 1 ml/min; Temperatur: 35°C; Detektion: 220 nm]: $R_t$ = 12.03 min.

**Beispiel 292**

3-Ethyl-1-(fluormethyl)-5-methyl-6-[(2-thioxoimidazolidin-1-yl)methyl]thieno[2,3-d]pyrimidin-2,4(1*H*,3*H*)-dion

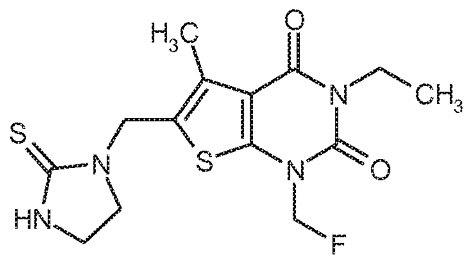

110 mg (0.276 mmol) der Verbindung aus Bsp. 397A wurden in 15 ml Dioxan gelöst und mit 78 mg (0.415 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 43 mg (39% d. Th.) der Titelverbindung erhalten.

$^1$H-NMR (400 MHz, DMSO-$d_6$, δ/ppm): 8.40 (s, 1H), 6.00 (d, 2H), 4.85 (s, 2H), 3.90 (q, 2H), 3.58-3.50 (m, 2H), 3.45-3.37 (m, 2H), 2.45 (s, 3H), 1.13 (d, 3H).

LC/MS (Methode 3, ESIpos): $R_t$ = 0.98 min, m/z = 357 $[M+H]^+$.

**Beispiel 293**

1-[2-(Cyclopentyloxy)ethyl]-3-ethyl-5-methyl-6-[(2-thioxoimidazolidin-1-yl)methyl]thieno[2,3-d]-pyrimidin-2,4(1*H*,3*H*)-dion

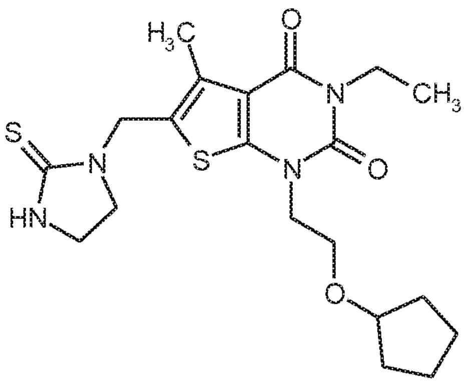

127 mg (0.264 mmol) der Verbindung aus Bsp. 398A wurden in 15 ml Dioxan gelöst und mit 74 mg (0.396 mmol) 1,1'-Thiocarbonyldiimidazol versetzt. Das Gemisch wurde 19 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 51 mg (43% d. Th.) der Titelverbindung erhalten.
$^1$H-NMR (400 MHz, DMSO-$d_6$, δ/ppm): 8.35 (s, 1H), 4.82 (s, 2H), 3.97 (t, 2H), 3.94-3.83 (m, 3H), 3.61 (t, 2H), 3.54-3.46 (m, 2H), 3.43-3.35 (m, 2H), 2.43 (s, 3H), 1.61-1.49 (m, 2H), 1.49-1.36 (m, 6H), 1.11 (t, 3H).
LC/MS (Methode 3, ESIpos): $R_t$ = 1.20 min, m/z = 437 [M+H]$^+$.

**Beispiel 294**

3-Ethyl-1-[2-(ethylsulfanyl)ethyl]-5-methyl-6-[(2-thioxoimidazolidin-1-yl)methyl]thieno[2,3-d]-pyrimidin-2,4(1*H*,3*H*)-dion

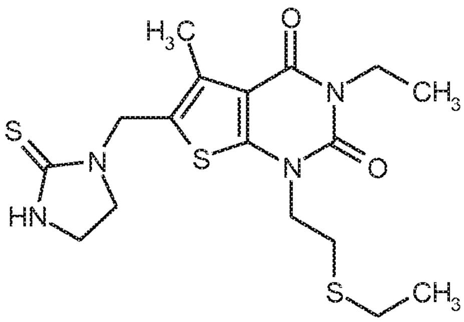

256 mg (0.573 mmol) der Verbindung aus Bsp. 399A wurden in 25 ml Dioxan gelöst und mit 161 mg (0.86 mmol) 1,1'-Thiocarbonyldiimidazol versetzt. Das Gemisch wurde 19 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 63 mg (26% d. Th.) der Titelverbindung erhalten.
$^1$H-NMR (400 MHz, DMSO-$d_6$, δ/ppm): 8.37 (s, 1H), 4.83 (s, 2H), 4.05-3.96 (m, 2H), 3.90 (q, 2H), 3.58-3.49 (m, 2H), 3.44-3.36 (m, 2H), 2.87-2.79 (m, 2H), 2.58 (q, 2H), 2.44 (s, 3H), 1.19 (t, 3H), 1.11 (t, 3H).
LC/MS (Methode 3, ESIpos): $R_t$ = 1.14 min, m/z = 413 [M+H]$^+$.

**Beispiel 295**

3-Ethyl-5-methyl-1-[2-(methylsulfonyl)ethyl]-6-[(2-thioxoimidazolidin-1-yl)methyl]thieno[2,3-d]-pyrimidin-2,4(1*H*,3*H*)-dion

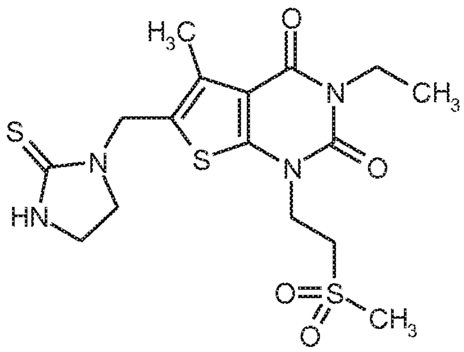

188 mg (0.377 mmol) der Verbindung aus Bsp. 400A wurden in 10 ml Dioxan gelöst und mit 106 mg (0.566 mmol) 1,1'-Thiocarbonyldiimidazol versetzt. Das Gemisch wurde 15 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 32 mg (19% d. Th.) der Titelverbindung erhalten.
$^1$H-NMR (400 MHz, DMSO-$d_6$, δ/ppm): 8.35 (s, 1H), 4.85 (s, 2H), 4.27 (t, 2H), 3.90 (q, 2H), 3.60-3.50 (m, 4H), 3.45-3.37 (m, 2H), 3.11 (s, 3H), 2.45 (s, 3H), 1.12 (t, 3H).
LC/MS (Methode 3, ESIpos): $R_t$ = 0.86 min, m/z = 431 $[M+H]^+$.

**Beispiel 296**

3-Ethyl-1-(3-methoxypropyl)-5-methyl-6-[(2-thioxoimidazolidin-1-yl)methyl]thieno[2,3-d]pyrimidin-2,4(1*H*,3*H*)-dion

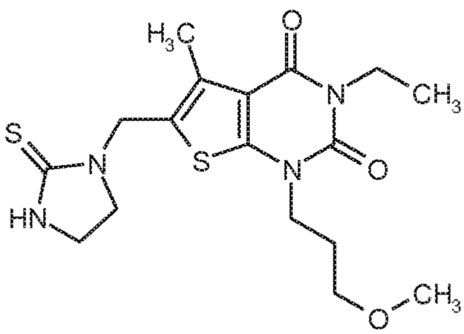

90 mg (0.211 mmol) der Verbindung aus Bsp. 401A wurden in 10 ml Dioxan gelöst und mit 59 mg (0.316 mmol) 1,1'-Thiocarbonyldiimidazol versetzt. Das Gemisch wurde 16 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 42 mg (50% d. Th.) der Titelverbindung erhalten.
$^1$H-NMR (400 MHz, DMSO-$d_6$, δ/ppm): 8.35 (s, 1H), 4.83 (s, 2H), 3.94-3.85 (m, 4H), 3.56-3.48 (m, 2H), 3.42-3.35 (m, 4H), 3.20 (s, 3H), 2.44 (s, 3H), 1.93-1.85 (m, 2H), 1.11 (t, 3H).
LC/MS (Methode 3, ESIpos): $R_t$ = 1.0 min, m/z = 397 $[M+H]^+$.

**Beispiel 297**

3-Isopropyl-1,5-dimethyl-6-[(2-thioxoimidazolidin-1-yl)methyl]thieno[2,3-d]pyrimidin-2,4(1*H*,3*H*)-dion

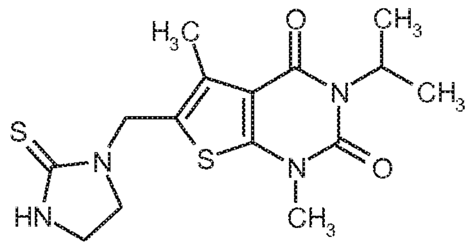

133 mg (0.407 mmol) der Verbindung aus Bsp. 402A wurden in 16 ml Dioxan gelöst und mit 115 mg (0.611 mmol) 1,1'-Thiocarbonyldiimidazol versetzt. Das Gemisch wurde 15 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 83 mg (55% d. Th.) der Titelverbindung erhalten.
$^1$H-NMR (400 MHz, DMSO-$d_6$, $\delta$/ppm): 8.35 (s, 1H), 5.14 (sept, 1H), 4.82 (s, 2H), 3.56-3.48 (m, 2H), 3.44-3.36 (m, 5H), 2.42 (s, 3H), 1.39 (d, 6H).
LC/MS (Methode 3, ESIpos): $R_t$ = 1.02 min, m/z = 353 [M+H]$^+$.

**Beispiel 298**

1-(3-Fluorpropyl)-3-isopropyl-5-methyl-6-[(2-thioxoimidazolidin-1-yl)methyl]thieno[2,3-d]pyrimidin-2,4(1*H*,3*H*)-dion

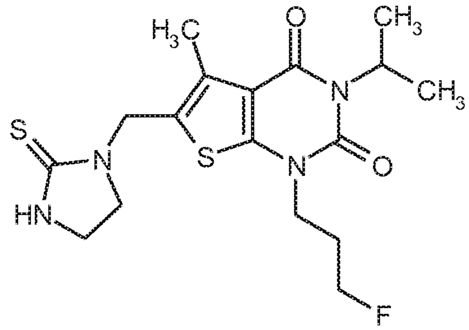

203 mg (0.382 mmol) der Verbindung aus Bsp. 404A wurden in 15 ml Dioxan gelöst und mit 107 mg (0.572 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 113 mg (74% d. Th.) der Titelverbindung erhalten.
$^1$H-NMR (400 MHz, DMSO-$d_6$, $\delta$/ppm): 8.35 (s, 1H), 5.13 (sept, 1H), 4.83 (s, 2H), 4.58 (t, 1H), 4.47 (t, 1H), 3.94 (t, 2H), 3.57-3.48 (m, 2H), 3.44-3.36 (m, 2H), 2.43 (s, 3H), 2.12-1.96 (m, 2H), 1.39 (d, 6H).
LC/MS (Methode 3, ESIpos): $R_t$ = 1.10 min, m/z = 397 [M+H]$^+$.

**Beispiel 299**

5-Methyl-1-(tetrahydrofuran-2-ylmethyl)-6-[(2-thioxoimidazolidin-1-yl)methyl]-3-(2,2,2-trifluorethyl)thieno[2,3-d]pyrimidin-2,4(1*H*,3*H*)-dion (*Enantiomer 1*)

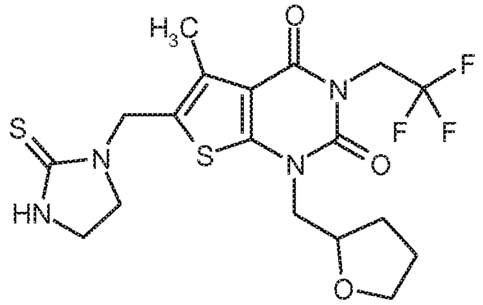

156 mg der racemischen Verbindung aus Bsp. 157 wurden in 3.5 ml eines Methanol/Dichlormethan-Gemisches (1:1 v/v) gelöst und mittels präparativer HPLC an chiraler Phase in die Enantiomere getrennt [Säule: Daicel Chiralpak IA, 5 μm, 250 mm x 30 mm; Eluent: Methanol/Ethanol/ Diethylamin 50:50:0.1 (v/v/v); Fluss: 30 ml/min; Temperatur: RT; UV-Detektion: 254 nm]. Die Produktfraktionen wurden am Rotationsverdampfer eingeengt, mit *tert.*-Butanol versetzt und gefriergetrocknet. Es wurden 62 mg der Titelverbindung (Enantiomer 1) sowie 76 mg des Enantiomers 2 (siehe Beispiel 300) erhalten.

$^1$H-NMR (500 MHz, DMSO-$d_6$, δ/ppm): 8.37 (s, 1H), 4.83 (s, 2H), 4.70 (q, 2H), 4.26-4.18 (m, 1H), 4.05 (dd, 1H), 3.79-3.69 (m, 2H), 3.65-3.58 (m, 1H), 3.58-3.50 (m, 2H), 3.44-3.37 (m, 2H), 2.43 (s, 3H), 2.03-1.93 (m, 1H), 1.93-1.76 (m, 2H), 1.67 (ddt, 1H).

Chirale analytische HPLC [Säule: Daicel Chiralpak IA, 5 μm, 100 mm x 4.6 mm; Eluent: Methanol/Ethanol/Diethylamin 50:50:0.1 (v/v/v); Fluss: 1.0 ml/min; Temperatur: RT; Injektion: 5 μl; DAD 254 nm]: $R_t$ = 2.45 min.

## Beispiel 300

5-Methyl-1-(tetrahydrofuran-2-ylmethyl)-6-[(2-thioxoimidazolidin-1-yl)methyl]-3-(2,2,2-trifluorethyl)thieno[2,3-d]pyrimidin-2,4(1*H*,3*H*)-dion (*Enantiomer 2*)

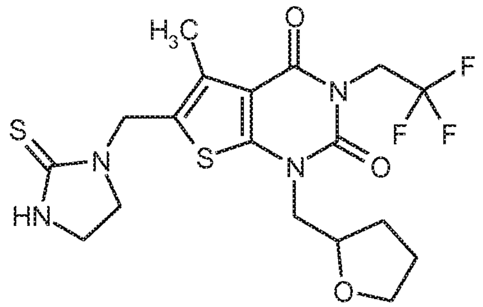

Die Titelverbindung (76 mg) wurde als zweites Enantiomer bei der unter Bsp. 299 beschriebenen präparativen HPLC-Trennung des Racemats aus Bsp. 157 erhalten.

$^1$H-NMR (500 MHz, DMSO-$d_6$, δ/ppm): 8.37 (s, 1H), 4.83 (s, 2H), 4.70 (q, 2H), 4.22 (br. t, 1H), 4.05 (dd, 1H), 3.79-3.69 (m, 2H), 3.65-3.58 (m, 1H), 3.58-3.50 (m, 2H), 3.44-3.37 (m, 2H), 2.43 (s, 3H), 2.03-1.93 (m, 1H), 1.93-1.76 (m, 2H), 1.71-1.62 (m, 1H).

Chirale analytische HPLC [Säule: Daicel Chiralpak IA, 5 μm, 100 mm x 4.6 mm; Eluent: Methanol/Ethanol/Diethylamin 50:50:0.1 (v/v/v); Fluss: 1.0 ml/min; Temperatur: RT; Injektion: 5 μl; DAD 254 nm]: $R_t$ = 3.13 min.

## Beispiel 301

3-(2-Ethoxyethyl)-5-methyl-6-[(2-thioxoimidazolidin-1-yl)methyl]-1-(3,3,3-trifluorpropyl)thieno-[2,3-d]pyrimidin-2,4(1*H*,3*H*)-dion

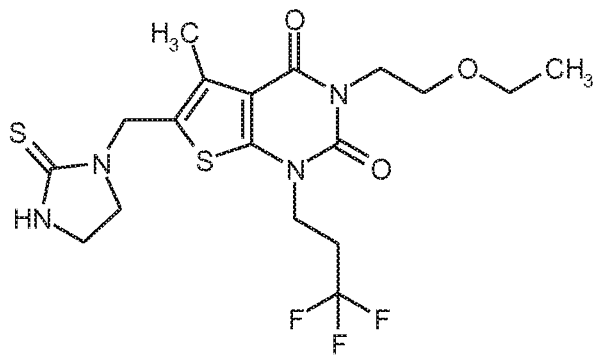

120 mg (0.253 mmol) der Verbindung aus Bsp. 406A wurden in 15 ml Dioxan gelöst und mit 71 mg (0.379 mmol) 1,1'-Thiocarbonyldiimidazol versetzt. Das Gemisch wurde 17 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 71 mg (59% d. Th.) der Titelverbindung erhalten.
$^1$H-NMR (400 MHz, DMSO-$d_6$, $\delta$/ppm): 8.37 (s, 1H), 4.85 (s, 2H), 4.09 (t, 2H), 4.04 (t, 2H), 3.57-3.48 (m, 4H), 3.47-3.37 (m, 4H), 2.81-2.70 (m, 2H), 2.44 (s, 3H), 1.06 (t, 3H).
LC/MS (Methode 3, ESIpos): $R_t$ = 1.13 min, m/z = 465 [M+H]$^+$.

**Beispiel 302**

3-(2-Ethoxyethyl)-1-(2-methoxyethyl)-5-methyl-6-[(2-thioxoimidazolidin-1-yl)methyl]thieno-[2,3-d]pyrimidin-2,4(1*H*,3*H*)-dion

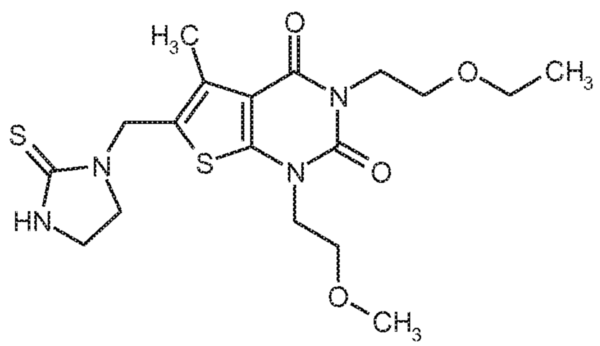

85 mg (0.15 mmol) der Verbindung aus Bsp. 407A wurden in 7 ml Dioxan gelöst und mit 42 mg (0.225 mmol) 1,1'-Thiocarbonyldiimidazol versetzt. Das Gemisch wurde 14 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 33 mg (64% d. Th.) der Titelverbindung erhalten.
$^1$H-NMR (400 MHz, DMSO-$d_6$, $\delta$/ppm): 8.36 (s, 1H), 4.82 (s, 2H), 4.08-3.97 (m, 4H), 3.62 (t, 2H), 3.57-3.48 (m, 4H), 3.47-3.37 (m, 4H), 3.23 (s, 3H), 2.43 (s, 3H), 1.06 (t, 3H).
LC/MS (Methode 3, ESIpos): $R_t$ = 0.98 min, m/z = 427 [M+H]$^+$.

**Beispiel 303**

3-Ethyl-1-(2-methoxyethyl)-5-methyl-6-[(4-methyl-2-thioxoimidazolidin-1-yl)methyl]thieno-[2,3-d]pyrimidin-2,4(1*H*,3*H*)-dion (*Racemat*)

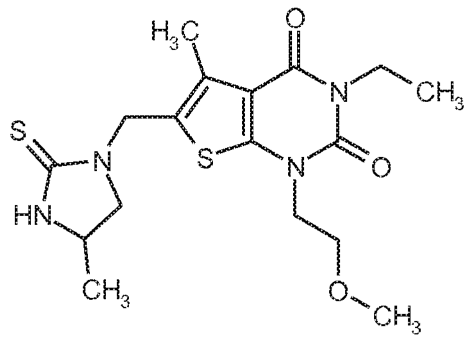

205 mg (0.399 mmol) der Verbindung aus Bsp. 266A wurden in 15 ml Dioxan gelöst und mit 112 mg (0.599 mmol) 1,1'-Thiocarbonyldiimidazol versetzt. Das Gemisch wurde 14 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 100 mg (53% d. Th.) der Titelverbindung erhalten.
$^1$H-NMR (400 MHz, DMSO-$d_6$, δ/ppm): 8.50 (s, 1H), 4.88-4.75 (m, 2H), 4.08-3.96 (m, 2H), 3.89 (q, 2H), 3.84-3.74 (m, 1H), 3.69-3.59 (m, 3H), 3.23 (s, 3H), 3.06 (dd, 1H), 2.46-2.40 (m, 3H), 1.22-1.06 (m, 6H).
LC/MS (Methode 3, ESIpos): $R_t$ = 1.03 min, m/z = 397 $[M+H]^+$.

## Beispiel 304

1-(3-Fluorpropyl)-3-isobutyl-6-[(3-isopropyl-2-thioxoimidazolidin-1-yl)methyl]-5-methylthieno-[2,3-d]pyrimidin-2,4(1*H*,3*H*)-dion

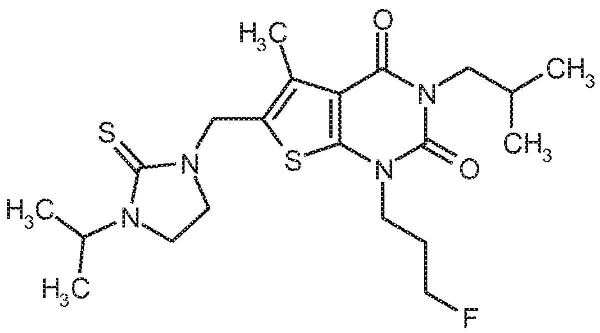

Die Titelverbindung (11 mg) wurde als Nebenprodukt der Herstellung und Aufreinigung der in Beispiel 144 beschriebenen Verbindung erhalten.
$^1$H-NMR (400 MHz, DMSO-$d_6$, δ/ppm): 4.88 (s, 2H), 4.65-4.54 (m, 2H), 4.47 (t, 1H), 3.97 (t, 2H), 3.70 (d, 2H), 3.46 (s, 4H), 2.44 (s, 3H), 2.11-1.97 (m, 3H), 1.09 (d, 6H), 0.84 (d, 6H).
LC/MS (Methode 3, ESIpos): $R_t$ = 1.39 min, m/z = 455 $[M+H]^+$.

## Beispiel 305

6-[(3-Isopropyl-2-thioxoimidazolidin-1-yl)methyl]-1,5-dimethyl-3-(2,2,2-trifluorethyl)thieno-[2,3-d]pyrimidin-2,4(1*H*,3*H*)-dion

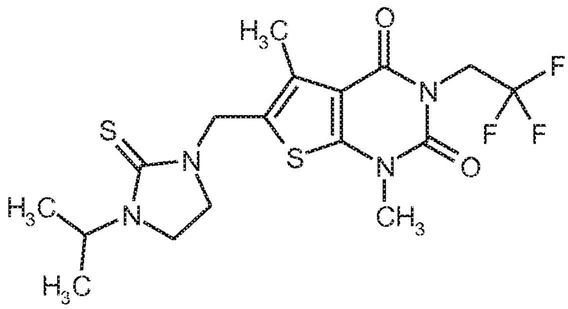

Die Titelverbindung (16 mg) wurde als Nebenprodukt der Herstellung und Aufreinigung der in Beispiel 159 beschriebenen Verbindung erhalten.
$^1$H-NMR (400 MHz, DMSO-$d_6$, δ/ppm): 4.90 (s, 2H), 4.69 (q, 2H), 4.60 (dt, 1H), 3.46 (s, 4H), 3.43 (s, 3H), 2.45 (s, 3H), 1.09 (d, 6H).
LC/MS (Methode 3, ESIpos): $R_t$ = 1.03 min, m/z = 435 [M+H]$^+$.

## Beispiel 306

5-Methyl-6-[(3-methyl-2-thioxoimidazolidin-1-yl)methyl]-3-(2,2,2-trifluorethyl)-1-(3,3,3-trifluorpropyl)thieno[2,3-d]pyrimidin-2,4(1*H*,3*H*)-dion

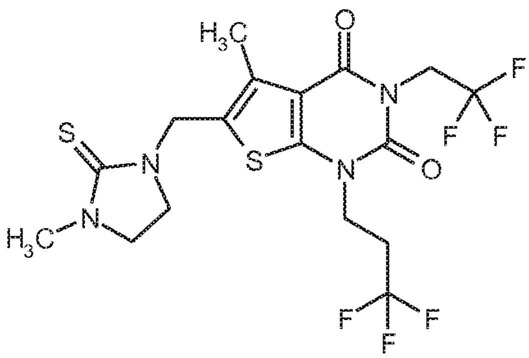

Eine Lösung von 50 mg (0.105 mmol) der Verbindung aus Bsp. 152 in 3.3 ml THF wurde bei 0°C mit 5 mg (0.126 mmol) Natriumhydrid (60% Suspension in Mineralöl) versetzt und 1 h gerührt. Anschließend wurden 18 mg (0.126 mmol) Iodmethan hinzugefügt und das Gemisch 16 h bei RT gerührt. Danach wurde das Reaktionsgemisch zwischen gesättigter wässriger AmmoniumchloridLösung (20 ml) und THF (30 ml) verteilt. Die organische Phase wurde mit gesättigter wässriger Natriumhydrogencarbonat-Lösung gewaschen, über Natriumsulfat getrocknet, filtriert und eingeengt. Der erhaltene Rückstand wurde mittels präparativer HPLC gereinigt (Methode 14). Nach Vereinigen der Produktfraktionen und Gefriertrocknung wurden 32 mg (59% d. Th.) der Titelverbindung erhalten.
$^1$H-NMR (400 MHz, DMSO-$d_6$, δ/ppm): 4.69 (q, 2H), 4.43 (s, 2H), 4.14 (t, 2H), 3.60 (d, 2H), 3.37-3.29 (m, 2H), 2.82-2.69 (m, 2H), 2.45 (s, 3H), 2.38 (s, 3H).
LC/MS (Methode 3, ESIpos): $R_t$ = 0.92 min, m/z = 489 [M+H]$^+$.

## Beispiel 307

3-Ethyl-1-(fluormethyl)-5-methyl-6-[(2-oxoimidazolidin-1-yl)methyl]thieno[2,3-d]pyrimidin-2,4(1*H*,3*H*)-dion

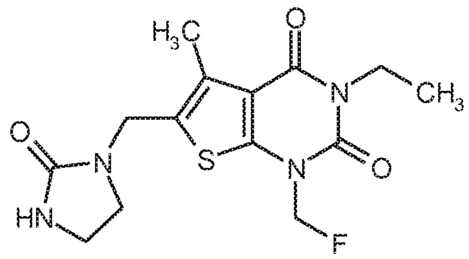

220 mg (0.553 mmol) der Verbindung aus Bsp. 397A wurden in 25 ml Dioxan gelöst und mit 139 mg (0.829 mmol) CDI versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 136 mg (71% d. Th.) der Titelverbindung erhalten.
$^1$H-NMR (400 MHz, DMSO-$d_6$, δ/ppm): 6.57 (s, 1H), 6.01 (d, 2H), 4.37 (s, 2H), 3.90 (q, 2H), 3.29-3.18 (m, 4H), 2.40 (s, 3H), 1.13 (t, 3H).
LC/MS (Methode 3, ESIpos): $R_t$ = 0.88 min, m/z = 341 [M+H]$^+$.

**Beispiel 308**

1-[2-(Cyclopentyloxy)ethyl]-3-ethyl-5-methyl-6-[(2-oxoimidazolidin-1-yl)methyl]thieno[2,3-d]-pyrimidin-2,4(1*H*,3*H*)-dion

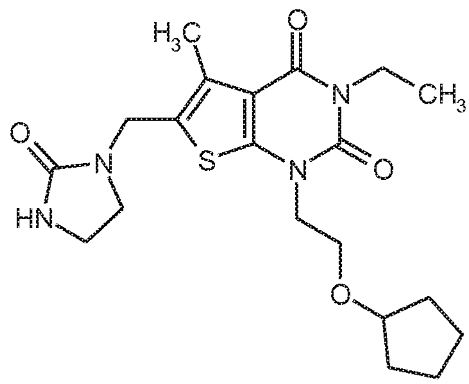

256 mg (0.532 mmol) der Verbindung aus Bsp. 398A wurden in 25 ml Dioxan gelöst und mit 133 mg (0.798 mmol) CDI versetzt. Das Gemisch wurde 19 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 146 mg (64% d. Th.) der Titelverbindung erhalten.
$^1$H-NMR (400 MHz, DMSO-$d_6$, δ/ppm): 6.54 (s, 1H), 4.34 (s, 2H), 3.97 (t, 2H), 3.94-3.83 (m, 3H), 3.61 (t, 2H), 3.26-3.15 (m, 4H), 2.39 (s, 3H), 1.54 (dd, 2H), 1.48-1.35 (m, 6H), 1.11 (t, 3H).
LC/MS (Methode 3, ESIpos): $R_t$ = 1.09 min, m/z = 421 [M+H]$^+$.

**Beispiel 309**

3-Ethyl-1-[2-(ethylsulfanyl)ethyl]-5-methyl-6-[(2-oxoimidazolidin-1-yl)methyl]thieno[2,3-d]-pyrimidin-2,4(1*H*,3*H*)-dion

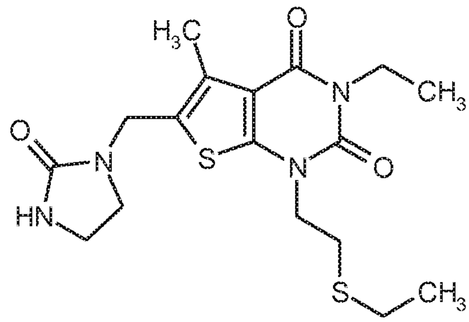

256 mg (0.573 mmol) der Verbindung aus Bsp. 399A wurden in 25 ml Dioxan gelöst und mit 144 mg (0.86 mmol) CDI versetzt. Das Gemisch wurde 19 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 77 mg (32% d. Th.) der Titelverbindung erhalten.
$^1$H-NMR (400 MHz, DMSO-$d_6$, $\delta$/ppm): 6.55 (s, 1H), 4.35 (s, 2H), 4.05-3.97 (m, 2H), 3.90 (q, 2H), 3.28-3.16 (m, 4H), 2.87-2.79 (m, 2H), 2.58 (q, 2H), 2.40 (s, 3H), 1.19 (t, 3H), 1.11 (t, 3H).
LC/MS (Methode 3, ESIpos): $R_t$ = 1.03 min, m/z = 397 $[M+H]^+$.

**Beispiel 310**

3-Ethyl-5-methyl-1-[2-(methylsulfonyl)ethyl]-6-[(2-oxoimidazolidin-1-yl)methyl]thieno[2,3-d]-pyrimidin-2,4(1*H*,3*H*)-dion

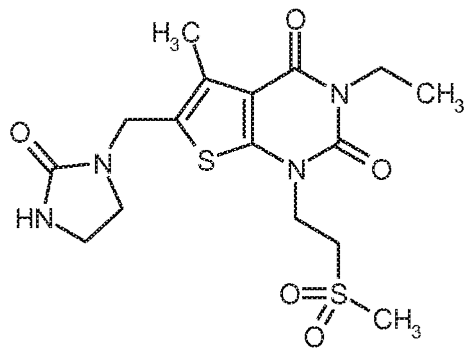

470 mg (0.423 mmol, Reinheit 35%) der Verbindung aus Bsp. 400A wurden in 25 ml Dioxan gelöst und mit 103 mg (0.635 mmol) CDI versetzt. Das Gemisch wurde 20 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 42 mg (24% d. Th.) der Titelverbindung erhalten.
$^1$H-NMR (400 MHz, DMSO-$d_6$, $\delta$/ppm): 6.56 (s, 1H), 4.37 (s, 2H), 4.31-4.23 (m, 2H), 3.90 (q, 2H), 3.56 (t, 2H), 3.30-3.17 (m, 4H), 3.11 (s, 3H), 2.40 (s, 3H), 1.12 (t, 3H).
LC/MS (Methode 3, ESIpos): $R_t$ = 0.77 min, m/z = 415 $[M+H]^+$.

**Beispiel 311**

3-Ethyl-1-(3-methoxypropyl)-5-methyl-6-[(2-oxoimidazolidin-1-yl)methyl]thieno[2,3-d]pyrimidin-2,4(1*H*,3*H*)-dion

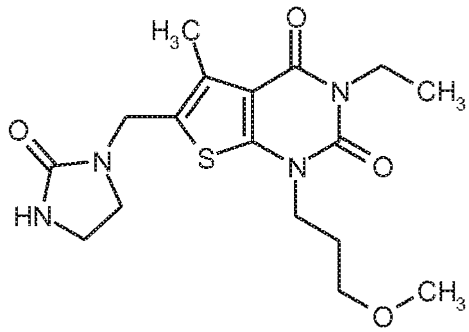

130 mg (0.304 mmol) der Verbindung aus Bsp. 401A wurden in 15 ml Dioxan gelöst und mit 76 mg (0.457 mmol) CDI versetzt. Das Gemisch wurde 16 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 93 mg (78% d. Th.) der Titelverbindung erhalten.
$^1$H-NMR (400 MHz, DMSO-$d_6$, δ/ppm): 6.54 (s, 1H), 4.35 (s, 2H), 3.94-3.85 (m, 4H), 3.37 (t, 2H), 3.28-3.17 (m, 7H), 2.39 (s, 3H), 1.93-1.85 (m, 2H), 1.11 (t, 3H).
LC/MS (Methode 3, ESIpos): $R_t$ = 0.89 min, m/z = 381 $[M+H]^+$.

**Beispiel 312**

3-Isopropyl-1,5-dimethyl-6-[(2-oxoimidazolidin-1-yl)methyl]thieno[2,3-d]pyrimidin-2,4(1*H*,3*H*)-dion

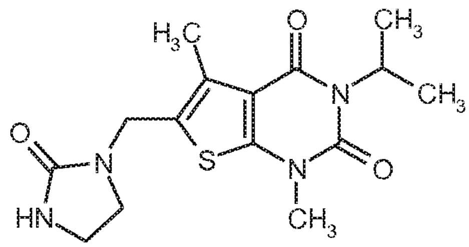

133 mg (0.407 mmol) der Verbindung aus Bsp. 402A wurden in 16 ml Dioxan gelöst und mit 102 mg (0.611 mmol) CDI versetzt. Das Gemisch wurde 15 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 90 mg (65% d. Th.) der Titelverbindung erhalten.
$^1$H-NMR (400 MHz, DMSO-$d_6$, δ/ppm): 6.54 (s, 1H), 5.14 (sept, 1H), 4.34 (s, 2H), 3.38 (s, 3H), 3.27-3.17 (m, 4H), 2.38 (s, 3H), 1.39 (d, 6H).
LC/MS (Methode 3, ESIpos): $R_t$ = 0.92 min, m/z = 336 $[M+H]^+$.

**Beispiel 313**

1-(Fluormethyl)-3-isopropyl-5-methyl-6-[(2-oxoimidazolidin-1-yl)methyl]thieno[2,3-d]pyrimidin-2,4(1*H*,3*H*)-dion

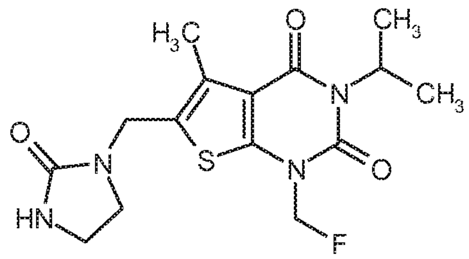

119 mg (0.312 mmol) der Verbindung aus Bsp. 403A wurden in 12 ml Dioxan gelöst und mit 78 mg (0.467 mmol) CDI versetzt. Das Gemisch wurde 15 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 43 mg (37% d. Th.) der Titelverbindung erhalten.
$^1$H-NMR (400 MHz, DMSO-$d_6$, δ/ppm): 6.57 (s, 1H), 5.98 (d, 2H), 5.12 (sept, 1H), 4.36 (s, 2H), 3.28-3.17 (m, 4H), 2.39 (s, 3H), 1.41 (d, 6H).
LC/MS (Methode 3, ESIpos): $R_t$ = 0.98 min, m/z = 355 [M+H]$^+$.

**Beispiel 314**

1-(3-Fluorpropyl)-3-isopropyl-5-methyl-6-[(2-oxoimidazolidin-1-yl)methyl]thieno[2,3-d]pyrimidin-2,4(1*H*,3*H*)-dion

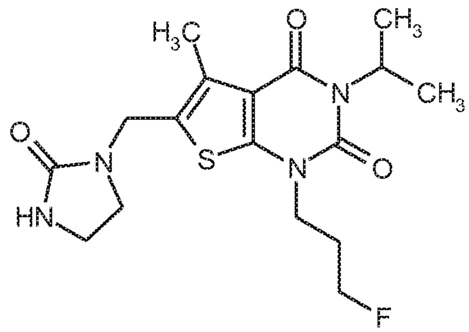

203 mg (0.382 mmol) der Verbindung aus Bsp. 404A wurden in 15 ml Dioxan gelöst und mit 96 mg (0.572 mmol) CDI versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 141 mg (97% d. Th.) der Titelverbindung erhalten.
$^1$H-NMR (400 MHz, DMSO-$d_6$, δ/ppm: 6.54 (s, 1H), 5.13 (sept, 1H), 4.59 (t, 1H), 4.47 (t, 1H), 4.34 (s, 2H), 3.94 (t, 2H), 3.28-3.16 (m, 4H), 2.38 (s, 3H), 2.12-1.96 (m, 2H), 1.39 (d, 6H).
LC/MS (Methode 3, ESIpos): $R_t$ = 1.0 min, m/z = 383 [M+H]$^+$.

**Beispiel 315**

1-(2-Methoxyethyl)-5-methyl-6-[(2-oxoimidazolidin-1-yl)methyl]-3-(prop-2-in-1-yl)thieno[2,3-d]-pyrimidin-2,4(1*H*,3*H*)-dion

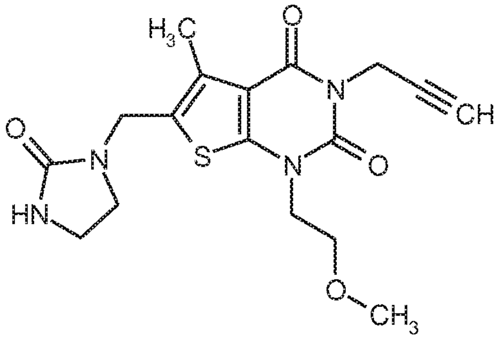

Eine Lösung von 168 mg (1.87 mmol) 2-Imidazolidinon in 7 ml THF wurde mit 75 mg (1.87 mmol) Natriumhydrid (60% Suspension in Mineralöl) versetzt und 3 h bei 60°C gerührt ("Lösung 1"). In einem anderen Reaktionsgefäß wurde eine Lösung von 144 mg (0.468 mmol) der Verbindung aus Bsp. 385A in 3 ml Dichlormethan bei 0°C mit 244 µl (1.4 mmol) *N*,*N*-Diisopropylethylamin und 51 µl (0.702 mmol) Thionylchlorid versetzt und 90 min gerührt. Anschließend wurde die Lösung 1 portionsweise zugesetzt und das Gemisch 20 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 23 mg (13% d. Th.) der Titelverbindung erhalten.

$^1$H-NMR (400 MHz, DMSO-$d_6$, δ/ppm): 6.55 (s, 1H), 4.59 (d, 2H), 4.35 (s, 2H), 4.04 (t, 2H), 3.63 (t, 2H), 3.29-3.17 (m, 7H), 3.15-3.11 (m, 1H), 2.39 (s, 3H).

LC/MS (Methode 3, ESIpos): $R_t$ = 0.84 min, m/z = 377 [M+H]$^+$.

**Beispiel 316**

3-(But-3-en-1-yl)-1-(2-methoxyethyl)-5-methyl-6-[(2-oxoimidazolidin-1-yl)methyl]thieno[2,3-d]-pyrimidin-2,4(1*H*,3*H*)-dion

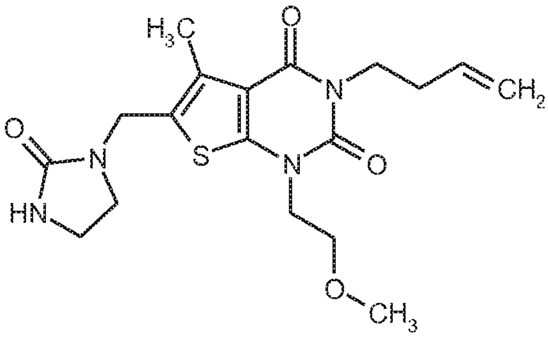

Eine Lösung von 188 mg (2.09 mmol) 2-Imidazolidinon in 7.5 ml THF wurde mit 84 mg (2.09 mmol) Natriumhydrid (60% Suspension in Mineralöl) versetzt und 3 h bei 60°C gerührt ("Lösung 1"). In einem anderen Reaktionsgefäß wurde eine Lösung von 170 mg (0.524 mmol) der Verbindung aus Bsp. 386A in 3.6 ml Dichlormethan bei 0°C mit 51 µl (0.702 mmol) Thionylchlorid versetzt und 75 min gerührt. Anschließend wurde die Lösung 1 portionsweise zugesetzt und das Gemisch 21 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 113 mg (53% d. Th.) der Titelverbindung erhalten.

$^1$H-NMR (400 MHz, DMSO-$d_6$, δ/ppm): 6.55 (s, 1H), 5.79 (ddt, 1H), 5.06-4.96 (m, 2H), 4.34 (s, 2H), 4.01 (t, 2H), 3.92 (t, 2H), 3.62 (t, 2H), 3.28-3.17 (m, 7H), 2.38 (s, 3H), 2.35-2.26 (m, 2H).

LC/MS (Methode 3, ESIpos): $R_t$ = 0.98 min, m/z = 393 [M+H]$^+$.

**Beispiel 317**

3-(But-2-in-1-yl)-1-(2-methoxyethyl)-5-methyl-6-[(2-oxoimidazolidin-1-yl)methyl]thieno[2,3-d]-pyrimidin-2,4(1*H*,3*H*)-dion

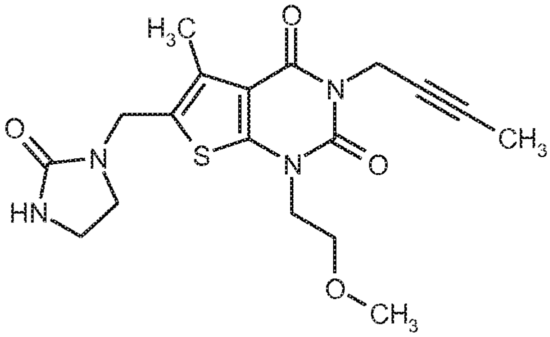

Eine Lösung von 128 mg (1.43 mmol) 2-Imidazolidinon in 5 ml THF wurde mit 57 mg (1.43 mmol) Natriumhydrid (60% Suspension in Mineralöl) versetzt und 3 h bei 60°C gerührt ("Lösung 1"). In einem anderen Reaktionsgefäß wurde eine Lösung von 115 mg (0.357 mmol) der Verbindung aus Bsp. 387A in 2.5 ml Dichlormethan bei 0°C mit 186 µl (1.07 mmol) *N,N*-Diisopropylethylamin und 64 µl (0.535 mmol) Thionylchlorid versetzt und 90 min gerührt. Anschließend wurde die Lösung 1 portionsweise zugesetzt und das Gemisch 20 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 23 mg (15% d. Th.) der Titelverbindung erhalten.

$^1$H-NMR (400 MHz, DMSO-$d_6$, δ/ppm): 6.55 (s, 1H), 4.55 (d, 2H), 4.35 (s, 2H), 4.03 (t, 2H), 3.63 (t, 2H), 3.28-3.17 (m, 7H), 2.39 (s, 3H), 1.74 (t, 3H).

LC/MS (Methode 3, ESIpos): $R_t$ = 0.9 min, m/z = 391 $[M+H]^+$.

**Beispiel 318**

3-(But-3-in-1-yl)-1-(2-methoxyethyl)-5-methyl-6-[(2-oxoimidazolidin-1-yl)methyl]thieno[2,3-d]-pyrimidin-2,4(1*H*,3*H*)-dion

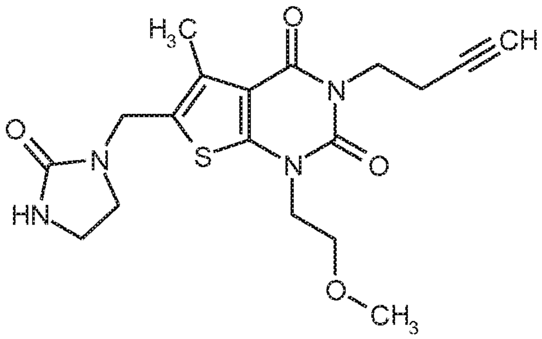

Eine Lösung von 139 mg (1.56 mmol) 2-Imidazolidinon in 6 ml THF wurde mit 62 mg (1.56 mmol) Natriumhydrid (60% Suspension in Mineralöl) versetzt und 3 h bei 60°C gerührt ("Lösung 1"). In einem anderen Reaktionsgefäß wurde eine Lösung von 128 mg (0.389 mmol) der Verbindung aus Bsp. 388A in 3 ml Dichlormethan bei 0°C mit 203 µl (1.17 mmol) *N,N*-Diisopropylethylamin und 42 µl (0.584 mmol) Thionylchlorid versetzt und 90 min gerührt. Anschließend wurde die Lösung 1 portionsweise zugesetzt und das Gemisch 115 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 70 mg (45% d. Th.) der Titelverbindung erhalten.
$^1$H-NMR (400 MHz, DMSO-$d_6$, δ/ppm): 6.55 (s, 1H), 4.34 (s, 2H), 4.05-3.95 (m, 4H), 3.62 (t, 2H), 3.28-3.17 (m, 7H), 2.86 (t, 1H), 2.46 (td, 2H), 2.39 (s, 3H).
LC/MS (Methode 3, ESIpos): $R_t$ = 0.88 min, m/z = 391 $[M+H]^+$.

**Beispiel 319**

1-(2-Methoxyethyl)-5-methyl-3-(3-methylbut-3-en-1-yl)-6-[(2-oxoimidazolidin-1-yl)methyl]thieno-[2,3-d]pyrimidin-2,4(1*H*-,3*H*)-dion

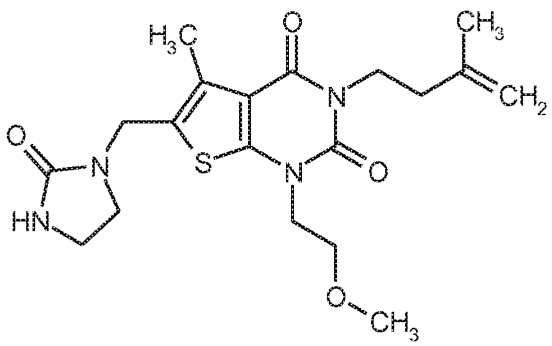

Eine Lösung von 151 mg (1.68 mmol) 2-Imidazolidinon in 6 ml THF wurde mit 67 mg (1.68 mmol) Natriumhydrid (60% Suspension in Mineralöl) versetzt und 3 h bei 60°C gerührt ("Lösung 1"). In einem anderen Reaktionsgefäß wurde eine Lösung von 145 mg (0.420 mmol) der Verbindung aus Bsp. 389A in 3 ml Dichlormethan bei 0°C mit 219 µl (1.26 mmol) *N,N*-Diisopropylethylamin und 45 µl (0.63 mmol) Thionylchlorid versetzt und 90 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 89 mg (51% d. Th.) der Titelverbindung erhalten.
$^1$H-NMR (400 MHz, DMSO-$d_6$, δ/ppm): 6.54 (s, 1H), 4.75-4.62 (m, 2H), 4.34 (s, 2H), 4.05-3.93 (m, 4H), 3.62 (t, 2H), 3.29-3.16 (m, 7H), 2.38 (s, 3H), 2.24 (t, 2H), 1.76 (s, 3H).
LC/MS (Methode 3, ESIpos): $R_t$ = 1.06 min, m/z = 407 $[M+H]^+$.

**Beispiel 320**

1-(2-Methoxyethyl)-5-methyl-3-(4-methylpent-3-en-1-yl)-6-[(2-oxoimidazolidin-1-yl)methyl]-thieno[2,3-d]pyrimidin-2,4(1*H*,3*H*)-dion

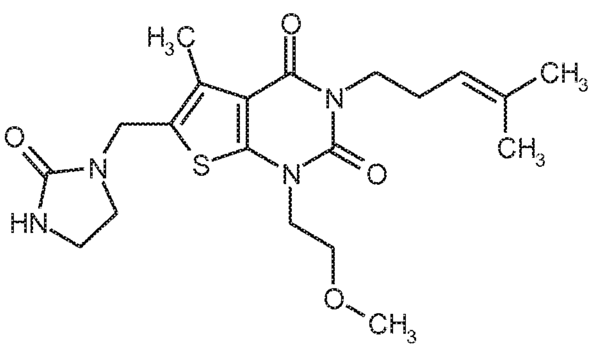

Eine Lösung von 111 mg (1.24 mmol) 2-Imidazolidinon in 4.5 ml THF wurde mit 49 mg (1.24 mmol) Natriumhydrid

(60% Suspension in Mineralöl) versetzt und 3 h bei 60°C gerührt ("Lösung 1"). In einem anderen Reaktionsgefäß wurde eine Lösung von 115 mg (0.31 mmol) der Verbindung aus Bsp. 390A in 2 ml Dichlormethan bei 0°C mit 34 µl (0.465 mmol) Thionylchlorid versetzt und 90 min gerührt. Anschließend wurde die Lösung 1 portionsweise zugesetzt und das Gemisch 21 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 68 mg (51% d. Th.) der Titelverbindung erhalten.
$^1$H-NMR (400 MHz, DMSO-$d_6$, δ/ppm): 6.55 (s, 1H), 5.16-5.09 (m, 1H), 4.34 (s, 2H), 4.01 (t, 2H), 3.86-3.77 (m, 2H), 3.62 (t, 2H), 3.28-3.17 (m, 7H), 2.38 (s, 3H), 2.22 (q, 2H), 1.64 (s, 3H), 1.55 (d, 3H).
LC/MS (Methode 3, ESIpos): $R_t$ = 1.13 min, m/z = 421 $[M+H]^+$.

**Beispiel 321**

1-(2-Methoxyethyl)-5-methyl-6-[(2-oxoimidazolidin-1-yl)methyl]-3-(3,4,4-trifluorbut-3-en-1-yl)-thieno[2,3-d]pyrimidin-2,4(1*H*,3*H*)-dion

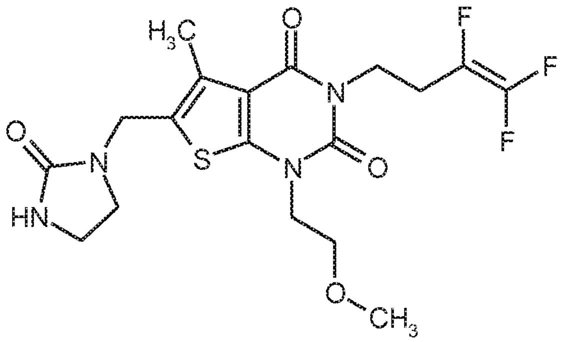

Eine Lösung von 178 mg (1.99 mmol) 2-Imidazolidinon in 7 ml THF wurde mit 80 mg (1.99 mmol) Natriumhydrid (60% Suspension in Mineralöl) versetzt und 3 h bei 60°C gerührt ("Lösung 1"). In einem anderen Reaktionsgefäß wurde eine Lösung von 190 mg (0.497 mmol) der Verbindung aus Bsp. 391A in 3.5 ml Dichlormethan bei 0°C mit 260 µl (1.49 mmol) *N,N*-Diisopropylethylamin und 54 µl (0.746 mmol) Thionylchlorid versetzt und 90 min gerührt. Anschließend wurde die Lösung 1 portionsweise zugesetzt und das Gemisch 20 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 92 mg (41% d. Th.) der Titelverbindung erhalten.
$^1$H-NMR (400 MHz, DMSO-$d_6$, δ/ppm): 6.55 (s, 1H), 4.35 (s, 2H), 4.08 (t, 2H), 4.03 (t, 2H), 3.62 (t, 2H), 3.29-3.17 (m, 7H), 2.71-2.65 (m, 1H), 2.65-2.58 (m, 1H), 2.38 (s, 3H).
LC/MS (Methode 3, ESIpos): $R_t$ = 1.03 min, m/z = 447 $[M+H]^+$.

**Beispiel 322**

3-(4,4-Difluorbut-3-en-1-yl)-1-(2-methoxyethyl)-5-methyl-6-[(2-oxoinädazolidin-1-yl)methyl]-thieno[2,3-d]pyrimidin-2,4(1*H*,3*H*)-dion

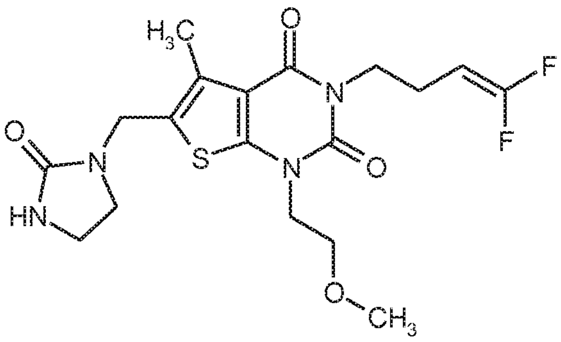

Eine Lösung von 119 mg (1.33 mmol) 2-Imidazolidinon in 5 ml THF wurde mit 53 mg (1.33 mmol) Natriumhydrid (60% Suspension in Mineralöl) versetzt und 3 h bei 60°C gerührt ("Lösung 1"). In einem anderen Reaktionsgefäß wurde eine Lösung von 121 mg (0.332 mmol) der Verbindung aus Bsp. 392A in 2 ml Dichlormethan bei 0°C mit 174 µl (0.997 mmol) *N,N*-Diisopropylethylamin und 36 µl (0.499 mmol) Thionylchlorid versetzt und 90 min gerührt. Anschließend wurde die Lösung 1 portionsweise zugesetzt und das Gemisch 21 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 101 mg (69% d. Th.) der Titelverbindung erhalten.

$^1$H-NMR (400 MHz, DMSO-$d_6$, δ/ppm): 6.55 (s, 1H), 4.60-4.46 (m, 1H), 4.34 (s, 2H), 4.02 (t, 2H), 3.91 (t, 2H), 3.62 (t, 2H), 3.29-3.17 (m, 7H), 2.39 (s, 3H), 2.25 (q, 2H).

LC/MS (Methode 3, ESIpos): $R_t$ = 1.02 min, m/z = 429 $[M+H]^+$.

## Beispiel 323

3-[(2,2-Difluorcyclopropyl)methyl]-1-(2-methoxyethyl)-5-methyl-6-[(2-oxoimidazolidin-1-yl)-methyl]thieno[2,3-d]pyrimidin-2,4(1*H*,3*H*)-dion *(Racemat)*

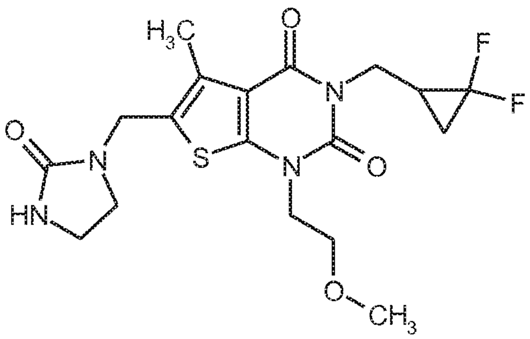

Eine Lösung von 153 mg (1.7 mmol) 2-Imidazolidinon in 6 ml THF wurde mit 68 mg (1.7 mmol) Natriumhydrid (60% Suspension in Mineralöl) versetzt und 3 h bei 60°C gerührt ("Lösung 1"). In einem anderen Reaktionsgefäß wurde eine Lösung von 158 mg (0.425 mmol) der Verbindung aus Bsp. 393A in 3 ml Dichlormethan bei 0°C mit 46 µl (0.638 mmol) Thionylchlorid versetzt und 75 min gerührt. Anschließend wurde die Lösung 1 portionsweise zugesetzt und das Gemisch 20 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 77 mg (41% d. Th.) der Titelverbindung erhalten.

$^1$H-NMR (400 MHz, DMSO-$d_6$, δ/ppm): 6.55 (s, 1H), 4.35 (s, 2H), 4.11-3.91 (m, 4H), 3.63 (t, 2H), 3.29-3.17 (m, 7H), 2.39 (s, 3H), 2.15-2.01 (m, 1H), 1.59 (tdd, 1H), 1.40-1.28 (m, 1H).

LC/MS (Methode 3, ESIpos): $R_t$ = 0.99 min, m/z = 429 $[M+H]^+$.

**Beispiel 324**

3-(2-Methoxyethyl)-5-methyl-6-[(2-oxoimidazolidin-1-yl)methyl]-1-(tetrahydrofuran-2-ylmethyl)-thieno[2,3-d]pyrimidin-2,4(1*H*,3*H*)-dion (*Enantiomer 1*)

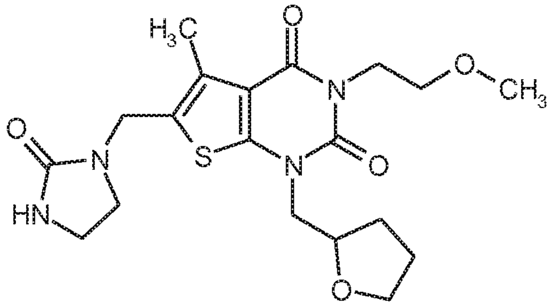

94 mg der racemischen Verbindung aus Bsp. 233 wurden in 3.5 ml eines Methanol/Dichlormethan-Gemisches (1:1 v/v) gelöst und mittels präparativer HPLC an chiraler Phase in die Enantiomere getrennt [Säule: Daicel Chiralpak IA, 5 μm, 250 mm x 30 mm; Eluent: Ethanol + 0.1% Diethylamin (isokratisch); Fluss: 60 ml/min; UV-Detektion: 280 nm]. Die Produktfraktionen wurden am Rotationsverdampfer eingeengt, mit *tert.*-Butanol versetzt und gefriergetrocknet. Es wurden 15 mg der Titelverbindung (Enantiomer 1) sowie 26 mg des Enantiomers 2 (siehe Beispiel 325) erhalten.
$^1$H-NMR (400 MHz, DMSO-$d_6$, δ/ppm): 6.54 (s, 1H), 4.34 (s, 2H), 4.25-4.16 (m, 1H), 4.10-3.99 (m, 3H), 3.78-3.65 (m, 2H), 3.65-3.57 (m, 1H), 3.49 (t, 2H), 3.28-3.15 (m, 7H), 2.38 (s, 3H), 2.03-1.75 (m, 4H), 1.71-1.59 (m, 1H).

Chirale analytische HPLC [Säule: Daicel Chiralpak IA, 3 μm, 100 mm x 4.6 mm; Eluent: Ethanol + 0.1% Diethylamin (isokratisch); Fluss: 1.0 ml/min; Temperatur: 25°C; DAD 280 nm]: $R_t$ = 4.32 min.

**Beispiel 325**

3-(2-Methoxyethyl)-5-methyl-6-[(2-oxoimidazolidin-1-yl)methyl]-1-(tetrahydrofuran-2-ylmethyl)-thieno[2,3-d]pyrimidin-2,4(1*H*,3*H*)-dion *(Enantiomer 2)*

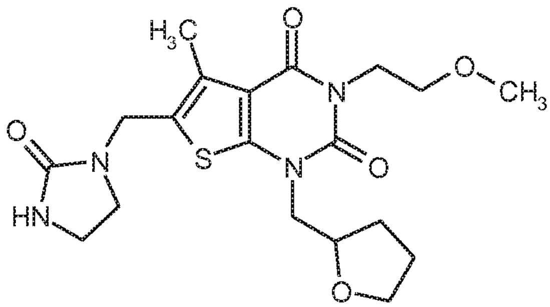

Die Titelverbindung (26 mg) wurde als zweites Enantiomer bei der unter Bsp. 324 beschriebenen präparativen HPLC-Trennung des Racemats aus Bsp. 233 erhalten.
$^1$H-NMR (500 MHz, DMSO-$d_6$, δ/ppm): 6.54 (s, 1H), 4.34 (s, 2H), 4.25-4.17 (m, 1H), 4.08-3.99 (m, 3H), 3.78-3.65 (m, 2H), 3.64-3.56 (m, 1H), 3.49 (t, 2H), 3.28-3.16 (m, 7H), 2.38 (s, 3H), 2.03-1.75 (m, 4H), 1.65 (ddt, 1H).

Chirale analytische HPLC [Säule: Daicel Chiralpak IA, 3 μm, 100 mm x 4.6 mm; Eluent: Ethanol + 0.1% Diethylamin (isokratisch); Fluss: 1.0 ml/min; Temperatur: 25°C; DAD 280 nm]: $R_t$ = 4.91 min.

**Beispiel 326**

3-(2-Ethoxyethyl)-5-methyl-6-[(2-oxoimidazolidin-1-yl)methyl]-1-(3,3,3-trifluorpropyl)thieno-[2,3-d]pyrimidin-2,4(1*H*,3*H*)-dion

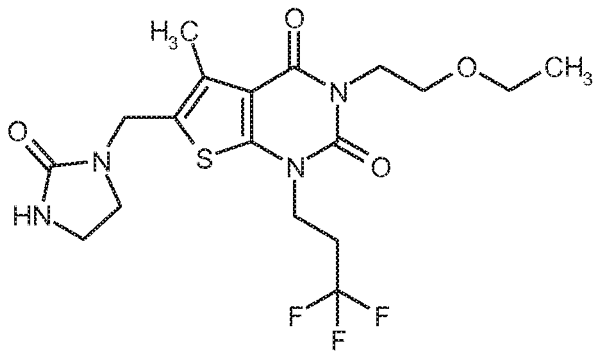

246 mg (0.518 mmol) der Verbindung aus Bsp. 406A wurden in 25 ml Dioxan gelöst und mit 130 mg (0.777 mmol) CDI versetzt. Das Gemisch wurde 17 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 156 mg (65% d. Th.) der Titelverbindung erhalten.
$^1$H-NMR (400 MHz, DMSO-$d_6$, $\delta$/ppm): 6.56 (s, 1H), 4.37 (s, 2H), 4.09 (t, 2H), 4.04 (t, 2H), 3.51 (t, 2H), 3.44 (q, 2H), 3.29-3.18 (m, 4H), 2.82-2.69 (m, 2H), 2.40 (s, 3H), 1.06 (t, 3H).
LC/MS (Methode 3, ESIpos): $R_t$ = 1.03 min, m/z = 449 $[M+H]^+$.

**Beispiel 327**

3-(2-Ethoxyethyl)-1-(2-methoxyethyl)-5-methyl-6-[(2-oxoimidazolidin-1-yl)methyl]thieno[2,3-d]-pyrimidin-2,4(1*H*,3*H*)-dion

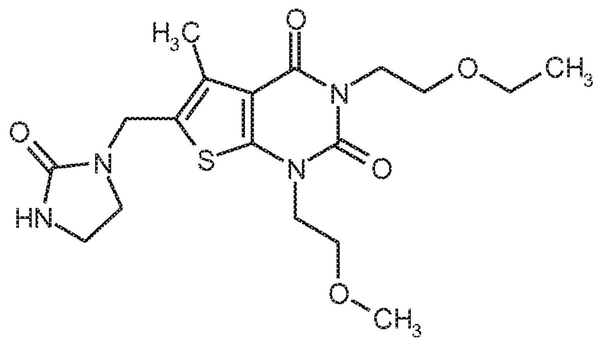

100 mg (0.177 mmol) der Verbindung aus Bsp. 407A wurden in 9 ml Dioxan gelöst und mit 44.3 mg (0.265 mmol) CDI versetzt. Das Gemisch wurde 14 h bei RT gerührt. Die Reaktionslösung wurde danach am Rotationsverdampfer eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 55 mg (75% d. Th.) der Titelverbindung erhalten.
$^1$H-NMR (400 MHz, DMSO-$d_6$, $\delta$/ppm): 6.54 (s, 1H), 4.34 (s, 2H), 4.08-3.96 (m, 4H), 3.62 (t, 2H), 3.51 (t, 2H), 3.44 (q, 2H), 3.28-3.16 (m, 7H), 2.38 (s, 3H), 1.06 (t, 3H).
LC/MS (Methode 3, ESIpos): $R_t$ = 0.88 min, m/z = 411 $[M+H]^+$.

**Beispiel 328**

1-(2-Methoxyethyl)-3-(3-methoxypropyl)-5-methyl-6-[(2-oxoimidazolidin-1-yl)methyl]thieno-[2,3-d]pyrimidin-2,4(1*H*,3*H*)-dion

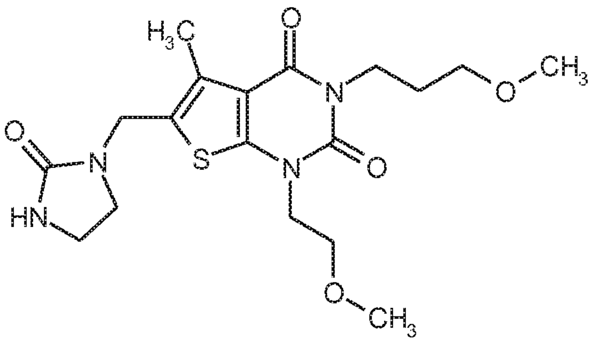

Eine Lösung von 139 mg (1.55 mmol) 2-Imidazolidinon in 6 ml THF wurde mit 62 mg (1.55 mmol) Natriumhydrid (60% Suspension in Mineralöl) versetzt und 3 h bei 60°C gerührt ("Lösung 1"). In einem anderen Reaktionsgefäß wurde eine Lösung von 140 mg (0.388 mmol) der Verbindung aus Bsp. 394A in 3 ml Dichlormethan bei 0°C mit 203 µl (1.165 mmol) *N,N*-Diisopropylethylamin und 42 µl (0.583 mmol) Thionylchlorid versetzt und 90 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 86 mg (52% d. Th.) der Titelverbindung erhalten.

$^1$H-NMR (400 MHz, DMSO-$d_6$, δ/ppm): 6.54 (s, 1H), 4.34 (s, 2H), 4.01 (t, 2H), 3.91 (t, 2H), 3.62 (t, 2H), 3.38-3.35 (m, 2H), 3.28-3.17 (m, 10H), 2.38 (s, 3H), 1.76 (quin, 2H).

LC/MS (Methode 3, ESIpos): $R_t$ = 0.85 min, m/z = 411 $[M+H]^+$.

**Beispiel 329**

1-(2-Methoxyethyl)-3-(1-methoxypropan-2-yl)-5-methyl-6-[(2-oxoimidazolidin-1-yl)methyl]-thieno[2,3-d]pyrimidin-2,4(1*H*,3*H*)-dion (*Racemat*)

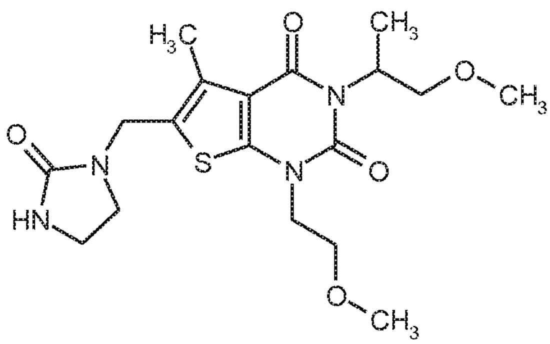

Eine Lösung von 206 mg (2.3 mmol) 2-Imidazolidinon in 8 ml THF wurde mit 92 mg (2.3 mmol) Natriumhydrid (60% Suspension in Mineralöl) versetzt und 3 h bei 60°C gerührt ("Lösung 1"). In einem anderen Reaktionsgefäß wurde eine Lösung von 205 mg (0.575 mmol) der Verbindung aus Bsp. 154A in 4 ml Dichlormethan bei 0°C mit 300 µl (1.72 mmol) *N,N*-Diisopropylethylamin und 63 µl (0.862 mmol) Thionylchlorid versetzt und 90 min gerührt. Anschließend wurde die Lösung 1 portionsweise zugesetzt und das Gemisch 17 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 134 mg (56% d. Th.) der Titelverbindung erhalten.
$^1$H-NMR (400 MHz, DMSO-$d_6$, δ/ppm): 6.54 (s, 1H), 5.17 (br. d, 1H), 4.34 (s, 2H), 4.02-3.96 (m, 2H), 3.91 (dd, 1H), 3.61 (t, 2H), 3.54 (dd, 1H), 3.28-3.16 (m, 10H), 2.37 (s, 3H), 1.32 (d, 3H).
LC/MS (Methode 3, ESIpos): $R_t$ = 0.92 min, m/z = 411 $[M+H]^+$.

**Beispiel 330**

1-(2-Methoxyethyl)-3-(1-methoxypropan-2-yl)-5-methyl-6-[(2-oxoimidazolidin-1-yl)methyl]-thieno[2,3-d]pyrimidin-2,4(1*H*,3*H*)-dion (*Enantiomer 1*)

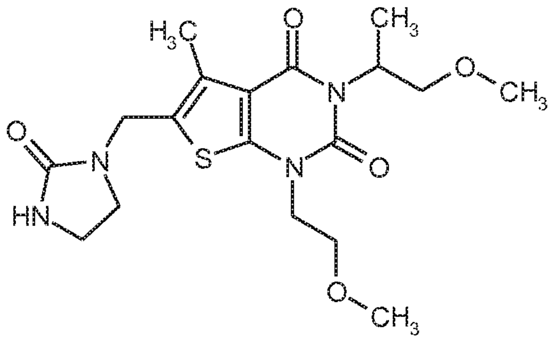

115 mg der racemischen Verbindung aus Bsp. 329 wurden in 3.5 ml eines Methanol/Dichlormethan-Gemisches (1:1 v/v) gelöst und mittels präparativer HPLC an chiraler Phase in die Enantiomere getrennt [Säule: Daicel Chiralpak IA, 5 μm, 250 mm x 30 mm; Eluent: Methanol/Ethanol/ Diethylamin 50:50:0.1 v/v/v; Fluss: 30 ml/min; Temperatur: RT; UV-Detektion: 254 nm]. Die Produktfraktionen wurden am Rotationsverdampfer eingeengt, mit *tert.*-Butanol versetzt und gefriergetrocknet. Es wurden 47 mg der Titelverbindung (Enantiomer 1) sowie 48 mg des Enantiomers 2 (siehe Beispiel 331) erhalten.
$^1$H-NMR (600 MHz, DMSO-$d_6$, δ/ppm): 6.51 (s, 1H), 5.17 (br. s, 1H), 4.34 (s, 2H), 4.02-3.95 (m, 2H), 3.94-3.88 (m, 1H), 3.61 (t, 2H), 3.54 (dd, 1H), 3.27-3.17 (m, 10H), 2.37 (s, 3H), 1.33 (d, 3H).

Chirale analytische HPLC [Säule: Daicel Chiralpak IA, 5 μm, 100 mm x 4.6 mm; Eluent: Methanol/Ethanol/Diethylamin 50:50:0.1 v/v/v; Fluss: 1.0 ml/min; Temperatur: RT; Injektion: 5 μl; DAD 254 nm]: $R_t$ = 2.19 min.

**Beispiel 331**

1-(2-Methoxyethyl)-3-(1-methoxypropan-2-yl)-5-methyl-6-[(2-oxoimidazolidin-1-yl)methyl]-thieno[2,3-d]pyrimidin-2,4(1*H*,3*H*)-dion *(Enantiomer 2)*

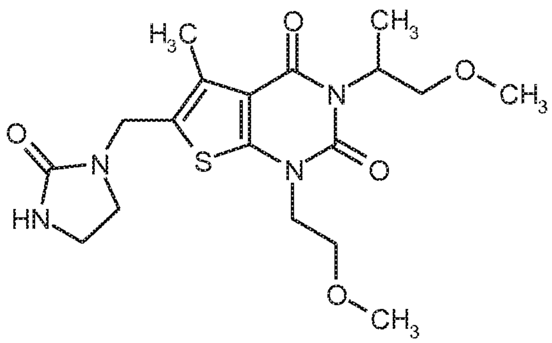

Die Titelverbindung (48 mg) wurde als zweites Enantiomer bei der unter Bsp. 330 beschriebenen präparativen HPLC-Trennung des Racemats aus Bsp. 329 erhalten.
$^1$H-NMR (600 MHz, DMSO-$d_6$, δ/ppm): 6.51 (s, 1H), 5.17 (br. s, 1H), 4.34 (s, 2H), 4.03-3.95 (m, 2H), 3.91 (t, 1H), 3.61

(t, 2H), 3.54 (dd, 1H), 3.28-3.17 (m, 10H), 2.37 (s, 3H), 1.33 (d, 3H).

Chirale analytische HPLC [Säule: Daicel Chiralpak IA, 5 µm, 100 mm x 4.6 mm; Eluent: Methanol/Ethanol/Diethylamin 50:50:0.1 v/v/v; Fluss: 1.0 ml/min; Temperatur: RT; Injektion: 5 µl; DAD 254 nm]: $R_t$ = 2.99 min.

**Beispiel 332**

1-(2-Methoxyethyl)-5-methyl-6-[(2-oxoimidazolidin-1-yl)methyl]-3-(tetrahydrofuran-2-ylmethyl)-thieno[2,3-d]pyrimidin-2,4(1*H*,3*H*)-dion *(Racemat)*

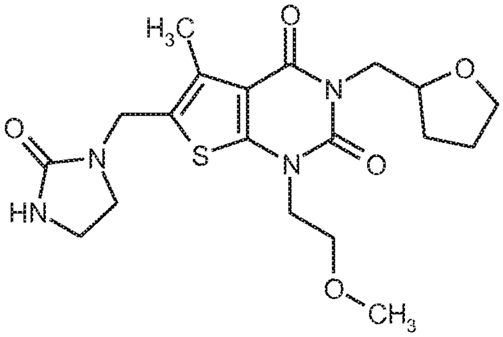

Eine Lösung von 163 mg (1.82 mmol) 2-Imidazolidinon in 7 ml THF wurde mit 73 mg (1.82 mmol) Natriumhydrid (60% Suspension in Mineralöl) versetzt und 3 h bei 60°C gerührt ("Lösung 1"). In einem anderen Reaktionsgefäß wurde eine Lösung von 163 mg (0.455 mmol) der Verbindung aus Bsp. 395A in 3 ml Dichlormethan bei 0°C mit 50 µl (0.683 mmol) Thionylchlorid versetzt und 90 min gerührt. Anschließend wurde die Lösung 1 portionsweise zugesetzt und das Gemisch 20 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 103 mg (53% d. Th.) der Titelverbindung erhalten.

$^1$H-NMR (400 MHz, DMSO-$d_6$, δ/ppm): 6.55 (s, 1H), 4.35 (s, 2H), 4.18-4.10 (m, 1H), 4.08-3.95 (m, 3H), 3.79-3.70 (m, 2H), 3.65-3.55 (m, 3H), 3.28-3.17 (m, 7H), 2.38 (s, 3H), 1.95-1.73 (m, 3H), 1.67-1.56 (m, 1H).

LC/MS (Methode 3, ESIpos): $R_t$ = 0.87 min, m/z = 423 $[M+H]^+$.

**Beispiel 333**

1-(2-Methoxyethyl)-5-methyl-6-[(2-oxoimidazolidin-1-yl)methyl]-3-(tetrahydrofuran-2-ylmethyl)-thieno[2,3-d]pyrimidin-2,4(1*H*,3*H*)-dion (*Enantiomer 1*)

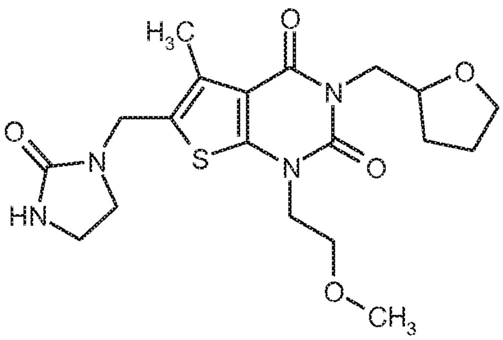

86 mg der racemischen Verbindung aus Bsp. 332 wurden in 3.6 ml Methanol gelöst und mittels präparativer HPLC an chiraler Phase in die Enantiomere getrennt [Säule: Daicel Chiralpak IC, 5 µm, 250 mm x 30 mm; Eluent A: Acetonitril + 0.1% Diethylamin, Eluent B: Methanol; isokratisch 50% A + 50% B; Fluss: 50.0 ml/min; UV-Detektion: 254 nm]. Die Produktfraktionen wurden am Rotationsverdampfer eingeengt, mit *tert.*-Butanol versetzt und gefriergetrocknet.

Es wurden 34 mg der Titelverbindung (Enantiomer 1) sowie 35 mg des Enantiomers 2 (siehe Beispiel 334) erhalten.
$^1$H-NMR (400 MHz, DMSO-$d_6$, δ/ppm): 6.54 (s, 1H), 4.34 (s, 2H), 4.18-4.10 (m, 1H), 4.08-3.95 (m, 3H), 3.79-3.71 (m, 2H), 3.65-3.55 (m, 3H), 3.28-3.17 (m, 7H), 2.38 (s, 3H), 1.95-1.72 (m, 3H), 1.68-1.57 (m, 1H).

Chirale analytische HPLC [Säule: Daicel Chiralpak IC, 3 µm, 100 mm x 4.6 mm; Eluent A: Acetonitril + 0.1% Diethylamin, Eluent B: Methanol; isokratisch 50% A + 50% B; Fluss: 1.4 ml/min; Temperatur: 25°C; DAD 254 nm]: $R_t$ = 3.07 min.

**Beispiel 334**

1-(2-Methoxyethyl)-5-methyl-6-[(2-oxoimidazolidin-1-yl)methyl]-3-(tetrahydrofuran-2-ylmethyl)-thieno[2,3-d]pyrimidin-2,4(1*H*,3*H*)-dion (*Enantiomer 2*)

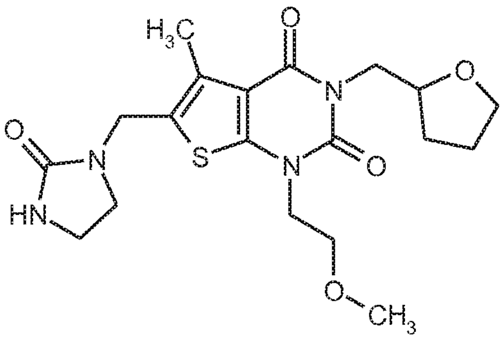

Die Titelverbindung (35 mg) wurde als zweites Enantiomer bei der unter Bsp. 333 beschriebenen präparativen HPLC-Trennung des Racemats aus Bsp. 332 erhalten.
$^1$H-NMR (400 MHz, DMSO-$d_6$, δ/ppm): 6.55 (s, 1H), 4.34 (s, 2H), 4.18-4.09 (m, 1H), 4.08-3.95 (m, 3H), 3.79-3.70 (m, 2H), 3.65-3.55 (m, 3H), 3.28-3.17 (m, 7H), 2.38 (s, 3H), 1.95-1.72 (m, 3H), 1.68-1.56 (m, 1H).

Chirale analytische HPLC [Säule: Daicel Chiralpak IC, 3 µm, 100 mm x 4.6 mm; Eluent A: Acetonitril + 0.1% Diethylamin, Eluent B: Methanol; isokratisch 50% A + 50% B; Fluss: 1.4 ml/min; Temperatur: 25°C; DAD 254 nm]: $R_t$ = 3.56 min.

**Beispiel 335**

1-(2-Methoxyethyl)-5-methyl-3-(oxetan-3-ylmethyl)-6-[(2-oxoimidazolidin-1-yl)methyl]thieno-[2,3-d]pyrimidin-2,4(1*H*,3*H*)-dion

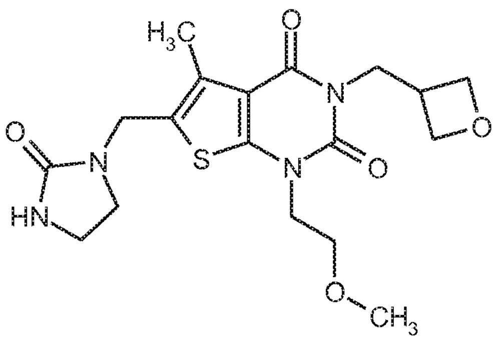

Eine Lösung von 73 mg (0.811 mmol) 2-Imidazolidinon in 3 ml THF wurde mit 32 mg (0.811 mmol) Natriumhydrid (60% Suspension in Mineralöl) versetzt und 3 h bei 60°C gerührt ("Lösung 1"). In einem anderen Reaktionsgefäß wurde eine Lösung von 69 mg (0.203 mmol) der Verbindung aus Bsp. 396A in 1.4 ml Dichlormethan bei 0°C mit 106 µl (0.608 mmol) *N,N*-Diisopropylethylamin und 22 µl (0.304 mmol) Thionylchlorid versetzt und 90 min gerührt. Anschließend wurde die Lösung 1 portionsweise zugesetzt und das Gemisch 17 h bei RT gerührt. Danach wurde das Reaktionsgemisch mit

70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 25 g Kieselgel, Laufmittel Ethylacetat/Methanol). Es wurden 15 mg (17% d. Th.) der Titelverbindung erhalten.
$^1$H-NMR (400 MHz, DMSO-$d_6$, δ/ppm): 6.55 (s, 1H), 4.57 (dd, 2H), 4.40 (t, 2H), 4.34 (s, 2H), 4.16 (d, 2H), 4.00 (t, 2H), 3.63-3.59 (m, 2H), 3.28-3.18 (m, 8H), 2.38 (s, 3H).
LC/MS (Methode 3, ESIpos): $R_t$ = 0.77 min, m/z = 409 [M+H]$^+$.

**Beispiel 336**

5-Methyl-6-[(3-methyl-2-oxoimidazolidin-1-yl)methyl]-3-(2,2,2-trifluorethyl)-1-(3,3,3-trifluor-propyl)thieno[2,3-d]pyrimidin-2,4(1*H*,3*H*)-dion

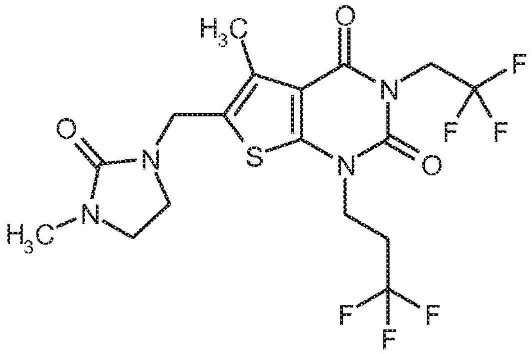

Eine Lösung von 60 mg (0.131 mmol) der Verbindung aus Bsp. 204 in 4 ml THF wurde bei 0°C mit 6.3 mg (0.157 mmol) Natriumhydrid (60% Suspension in Mineralöl) versetzt und 1 h gerührt. Anschließend wurden 22.5 mg (0.157 mmol) Iodmethan hinzugefügt und das Gemisch 16 h bei RT gerührt. Danach wurde das Reaktionsgemisch zwischen gesättigter wässriger Ammoniumchlorid-Lösung (20 ml) und THF (30 ml) verteilt. Die organische Phase wurde mit gesättigter wässriger Natriumhydrogencarbonat-Lösung gewaschen, über Natriumsulfat getrocknet, filtriert und eingeengt. Der erhaltene Rückstand wurde mittels präparativer HPLC gereinigt (Methode 14). Nach Vereinigen der Produktfraktionen und Gefriertrocknung wurden 25 mg (40% d. Th.) der Titelverbindung erhalten.
$^1$H-NMR (400 MHz, DMSO-$d_6$, δ/ppm): 4.70 (q, 2H), 4.41 (s, 2H), 4.13 (t, 2H), 3.27-3.17 (m, 4H), 2.85-2.70 (m, 2H), 2.67 (s, 3H), 2.40 (s, 3H).
LC/MS (Methode 3, ESIpos): $R_t$ = 1.20 min, m/z = 473 [M+H]$^+$.

**Beispiel 337**

1-(2-Methoxyethyl)-5-methyl-6-[(3-methyl-2-oxoimidazolidin-1-yl)methyl]-3-(3,3,3-trifluorpropyl)thieno[2,3-d]pyrimidin-2,4(1*H*,3*H*)-dion

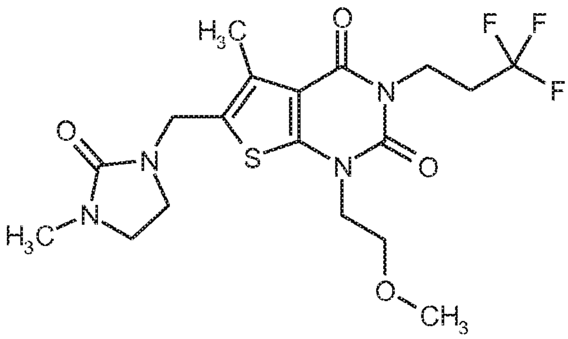

Eine Lösung von 62 mg (0.14 mmol) der Verbindung aus Bsp. 98 in 8 ml THF wurde bei 0°C mit 6.7 mg (0.168 mmol) Natriumhydrid (60% Suspension in Mineralöl) versetzt und 1 h gerührt. Anschließend wurden 24 mg (0.168 mmol) Iodmethan hinzugefügt und das Gemisch 3 h bei RT gerührt. Danach wurden nochmals 6.7 mg (0.168 mmol) Natriumhydrid (60% Suspension in Mineralöl) sowie 24 mg (0.168 mmol) Iodmethan hinzugefügt und weitere 63 h bei RT gerührt. Dann wurde das Reaktionsgemisch zwischen gesättigter wässriger Ammoniumchlorid-Lösung (20 ml), THF (30 ml) und Ethylacetat (40 ml) verteilt. Die organische Phase wurde mit gesättigter wässriger Natriumhydrogencarbonat-Lösung gewaschen, über Natriumsulfat getrocknet, filtriert und eingeengt. Der erhaltene Rückstand wurde mittels präparativer HPLC gereinigt (Methode 14). Nach Vereinigen der Produktfraktionen und Gefriertrocknung wurden 42 mg (64% d. Th.) der Titelverbindung erhalten.
$^1$H-NMR (500 MHz, DMSO-$d_6$, δ/ppm): 4.38 (s, 2H), 4.10 (t, 2H), 4.01 (t, 2H), 3.62 (t, 2H), 3.26-3.15 (m, 7H), 2.67 (s, 3H), 2.63-2.52 (m, 2H), 2.39 (s, 3H).
LC/MS (Methode 3, ESIpos): $R_t$ = 1.09 min, m/z = 449 $[M+H]^+$.

**Beispiel 338**

3-(2-Methoxy-2-methylpropyl)-5-methyl-6-[(3-methyl-2-oxoimidazolidin-1-yl)methyl]-1-(3,3,3-trifluorpropyl)thieno[2,3-d]pyrimidin-2,4(1*H*,3*H*)-dion

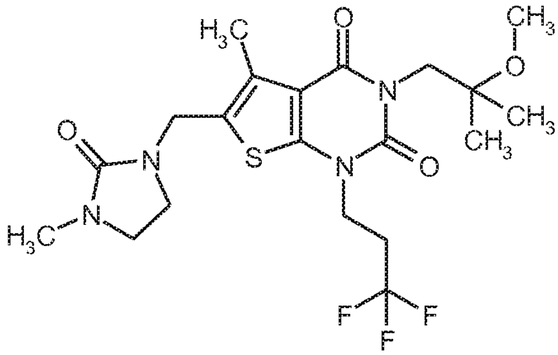

Eine Lösung von 45 mg (0.093 mmol) der Verbindung aus Bsp. 236 in 5 ml THF wurde bei 0°C mit 4.5 mg (0.112 mmol) Natriumhydrid (60% Suspension in Mineralöl) versetzt und 1 h gerührt. Anschließend wurden 16 mg (0.112 mmol) Iodmethan hinzugefügt und das Gemisch 2 h bei RT gerührt. Danach wurde das Reaktionsgemisch zwischen gesättigter wässriger Ammoniumchlorid-Lösung (20 ml), THF (30 ml) und Ethylacetat (40 ml) verteilt. Die organische Phase wurde mit gesättigter wässriger Natriumhydrogencarbonat-Lösung gewaschen, über Natriumsulfat getrocknet, filtriert und eingeengt. Der erhaltene Rückstand wurde mittels präparativer HPLC gereinigt (Methode 14). Nach Vereinigen der Produktfraktionen und Gefriertrocknung wurden 32 mg (69% d. Th.) der Titelverbindung erhalten.
$^1$H-NMR (400 MHz, DMSO-$d_6$, δ/ppm): 4.39 (s, 2H), 4.09 (br. t, 2H), 3.99 (br. s, 2H), 3.26-3.17 (m, 4H), 3.15 (s, 3H), 2.81-2.69 (m, 2H), 2.67 (s, 3H), 2.39 (s, 3H), 1.08 (s, 6H).
LC/MS (Methode 3, ESIpos): $R_t$ = 1.15 min, m/z = 477 $[M+H]^+$.

**Beispiel 339**

5-Methyl-6-[(2-oxo-2,3-dihydro-1*H*-imidazol-1-yl)methyl]-3-(2,2,2-trifluorethyl)-1-(3,3,3-trifluorpropyl)thieno[2,3-d]pyrimidin-2,4(1*H*,3*H*)-dion

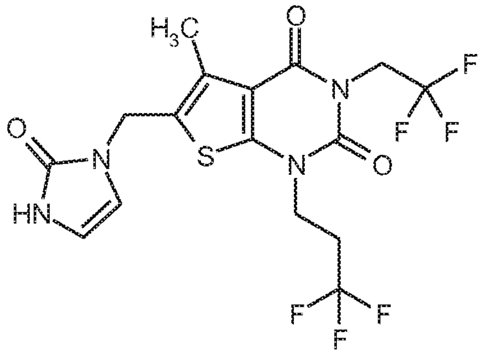

Eine Lösung von 417 mg (0.873 mmol) der Verbindung aus Bsp. 408A in 9 ml Methanol wurde zunächst mit 163 mg (2.01 mmol) Kaliumcyanat und dann tropfenweise mit 128 µl (1.49 mmol) Perchlorsäure (70% in Wasser) versetzt. Nachdem das Reaktionsgemisch 5 Tage bei RT gerührt worden war, wurde es mit halbgesättigter wässriger Natriumhydrogencarbonat-Lösung versetzt und mit Ethylacetat extrahiert. Der organische Extrakt wurde mit gesättigter Natriumchlorid-Lösung gewaschen, über wasserfreiem Magnesiumsulfat getrocknet, filtriert und eingeengt. Eine HPLC-Analytik des so erhaltenen Rohprodukts zeigte, dass es sich um ein Gemisch aus Edukt und gewünschtem Produkt handelte. Das Rohprodukt wurde daher nochmals in der gleichen Menge Methanol gelöst und mit den gleichen Mengen Kaliumcyanat und Perchlorsäure versetzt wie oben aufgeführt. Nach ca. 18 h Rühren wurden weitere 163 mg (2.01 mmol) Kaliumcyanat hinzugefügt. Nach weiteren ca. 24 h und nach ca. 48 h wurden jeweils 1.7 ml (1.75 mmol) 1 M Salzsäure zugesetzt. Nach nochmals 2.5 Tagen Rühren wurde das Reaktionsgemisch mit Wasser verdünnt. Der dabei ausgefallene Niederschlag wurde abgesaugt, mit wenig Wasser gewaschen und getrocknet. Anschließend wurde der Feststoff mittels präparativer HPLC gereinigt (Methode 8). Die Produktfraktionen wurden eingeengt und mit wenig Acetonitril bei RT verrührt. Nach erneuter Isolierung des Feststoffs und Trocknen im Hochvakuum wurden 100 mg (25% d. Th.) der Titelverbindung erhalten.

$^1$H-NMR (400 MHz, DMSO-$d_6$, δ/ppm): 10.01 (br. s, 1H), 6.46 (t, 1H), 6.35 (t, 1H), 4.83 (s, 2H), 4.70 (q, 2H), 4.11 (t, 2H), 2.85-2.67 (m, 2H), 2.47 (s, 3H).

LC/MS (Methode 1, ESIpos): $R_t$ = 0.84 min, m/z = 457 [M+H]$^+$.

## Beispiel 340

1-(2-Methoxyethyl)-5-methyl-6-[(2-oxo-2,3-dihydro-1*H*-imidazol-1-yl)methyl]-3-(2,2,2-trifluorethyl)thieno[2,3-d]pyrimidin-2,4(1*H*,3*H*)-dion

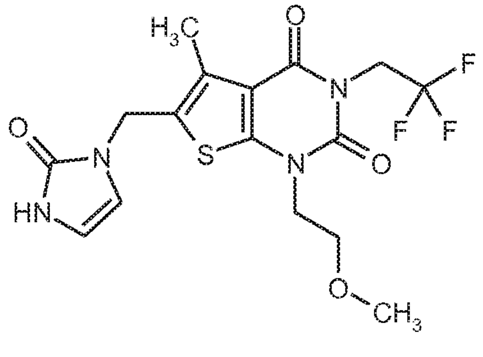

Analog zu dem unter Bsp. 339 beschriebenen Verfahren wurden aus 454 mg (1.03 mmol) der Verbindung aus Bsp. 409A 140 mg (32% d. Th.) der Titelverbindung erhalten. Auf die Reinigung mittels präparativer HPLC konnte hier verzichtet werden; das beim Verdünnen mit Wasser ausgefallene Produkt wurde nach Absaugen mit wenig Acetonitril bei RT verrührt, erneut abgesaugt und getrocknet.

$^1$H-NMR (400 MHz, DMSO-$d_6$, δ/ppm): 10.01 (br. s, 1H), 6.45 (br. s, 1H), 6.38-6.30 (m, 1H), 4.81 (s, 2H), 4.69 (q, 2H), 4.03 (t, 2H), 3.62 (t, 2H), 3.22 (s, 3H), 2.46 (s, 3H).

LC/MS (Methode 17, ESIneg): $R_t$ = 1.33 min, m/z = 417.08 [M-H]$^-$.

**Beispiel 341**

5-Methyl-6-[(2-oxo-2,3-dihydro-1*H*-imidazol-1-yl)methyl]-1-(tetrahydrofuran-2-ylmethyl)-3-(2,2,2-trifluorethyl)thieno[2,3-d]pyrimidin-2,4(1*H*,3*H*)-dion *(Racemat)*

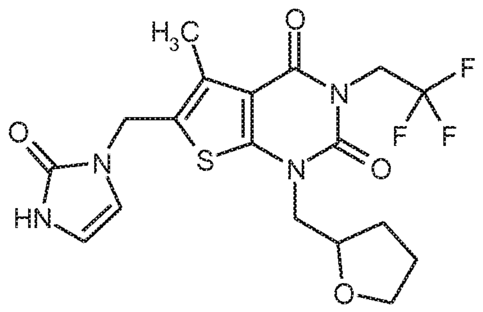

Eine Lösung von 605 mg (1.30 mmol) der Verbindung aus Bsp. 410A in 13 ml Methanol wurde zunächst mit 242 mg (2.99 mmol) Kaliumcyanat und dann tropfenweise mit 190 μl (2.21 mmol) Perchlorsäure (70% in Wasser) versetzt. Nachdem das Reaktionsgemisch ca. 42 h bei RT gerührt worden war, wurde es nochmals mit den gleichen Mengen Kaliumcyanat und Perchlorsäure versetzt. Nach weiteren 4 Tagen Rühren wurde das Reaktionsgemisch mit halbgesättigter wässriger Natriumhydrogencarbonat-Lösung versetzt und mit Ethylacetat extrahiert. Der organische Extrakt wurde mit gesättigter Natriumchlorid-Lösung gewaschen, über wasserfreiem Magnesiumsulfat getrocknet, filtriert und eingeengt. Das Rohprodukt wurde mittels präparativer HPLC gereinigt (Methode 8). Die Produktfraktionen wurden eingeengt und der Rückstand im Hochvakuum getrocknet. Es wurden 275 mg (47% d. Th.) der Titelverbindung erhalten.
$^1$H-NMR (400 MHz, DMSO-$d_6$, δ/ppm): 10.00 (br. s, 1H), 6.45 (dd, 1H), 6.35 (t, 1H), 4.80 (s, 2H), 4.70 (q, 2H), 4.26-4.15 (m, 1H), 4.05 (dd, 1H), 3.78-3.67 (m, 2H), 3.65-3.55 (m, 1H), 2.46 (s, 3H), 2.05-1.75 (m, 3H), 1.72-1.59 (m, 1H).
LC/MS (Methode 17, ESIneg): $R_t$ = 1.41 min, m/z = 443.10 [M-H]$^-$.

**Beispiel 342**

5-Methyl-6-[(2-oxo-2,3-dihydro-1*H*-imidazol-1-yl)methyl]-1-(tetrahydrofuran-2-ylmethyl)-3-(2,2,2-trifluorethyl)thieno[2,3-d]pyrimidin-2,4(1*H*,3*H*)-dion *(Enantiomer 1)*

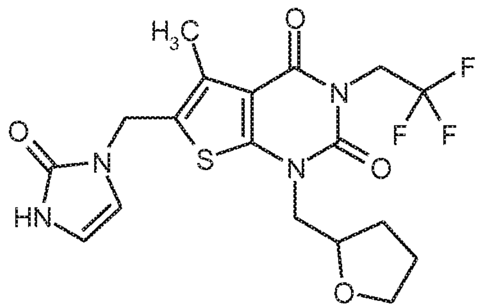

220 mg (0.495 mmol) der racemischen Verbindung aus Bsp. 341 wurden in 20 ml Ethanol gelöst und in 67 Portionen über präparative SFC an chiraler Phase in die Enantiomere aufgetrennt [Säule: Daicel Chiralpak AZ-H, 5 μm, 250 mm x 30 mm; Laufmittel: Kohlendioxid/Ethanol 82:18; Fluss: 100 ml/min; Temperatur: 40°C; Detektion: 210 nm]. Nach Eindampfen der Produktfraktionen und Trocknen im Hochvakuum wurden 74 mg (67% d. Th.) von Enantiomer 1 erhalten (>99% ee, chirale analytische HPLC).
$^1$H-NMR (400 MHz, DMSO-$d_6$, δ/ppm): 10.00 (br. s, 1H), 6.45 (dd, 1H), 6.35 (t, 1H), 4.80 (s, 2H), 4.70 (q, 2H), 4.26-4.14 (m, 1H), 4.05 (dd, 1H), 3.79-3.66 (m, 2H), 3.64-3.55 (m, 1H), 2.46 (s, 3H), 2.05-1.74 (m, 3H), 1.71-1.59 (m, 1H).

Chirale analytische SFC [Säule: Daicel Chiralpak AZ-H, 3 μm, 50 mm x 4.6 mm; Laufmittel: Kohlendioxid/Ethanol 80:20; Fluss: 3 ml/min; Temperatur: 40°C; Detektion: 210 nm]: $R_t$ = 2.83 min.

**Beispiel 343**

5-Methyl-6-[(2-oxo-2,3-dihydro-1*H*-imidazol-1-yl)methyl]-1-(tetrahydrofuran-2-ylmethyl)-3-(2,2,2-trifluorethyl)thieno[2,3-d]pyrimidin-2,4(1*H*,3*H*)-dion *(Enantiomer 2)*

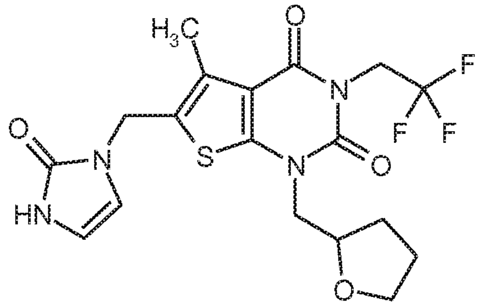

220 mg (0.495 mmol) der racemischen Verbindung aus Bsp. 341 wurden in 20 ml Ethanol gelöst und in 67 Portionen über präparative SFC an chiraler Phase in die Enantiomere aufgetrennt [Säule: Daicel Chiralpak AZ-H, 5 μm, 250 mm x 30 mm; Laufmittel: Kohlendioxid/Ethanol 82:18; Fluss: 100 ml/min; Temperatur: 40°C; Detektion: 210 nm]. Nach Eindampfen der Produktfraktionen und Trocknen im Hochvakuum wurden 50 mg (45% d. Th.) von Enantiomer 2 erhalten (>99% ee, chirale analytische HPLC).
$^1$H-NMR (400 MHz, DMSO-$d_6$, δ/ppm): 10.01 (br. s, 1H), 6.45 (dd, 1H), 6.35 (t, 1H), 4.80 (s, 2H), 4.70 (q, 2H), 4.26-4.15 (m, 1H), 4.05 (dd, 1H), 3.77-3.67 (m, 2H), 3.65-3.55 (m, 1H), 2.46 (s, 3H), 2.06-1.74 (m, 3H), 1.70-1.61 (m, 1H).
Chirale analytische SFC [Säule: Daicel Chiralpak AZ-H, 3 μm, 50 mm x 4.6 mm; Laufmittel: Kohlendioxid/Ethanol 80:20; Fluss: 3 ml/min; Temperatur: 40°C; Detektion: 210 nm]: $R_t$ = 4.42 min.

**Beispiel 344**

3-(2-Methoxyethyl)-5-methyl-6-[(2-oxo-2,3-dihydro-1*H*-imidazol-1-yl)methyl]-1-(3,3,3-trifluorpropyl)thieno[2,3-d]pyrimidin-2,4(1*H*,3*H*)-dion

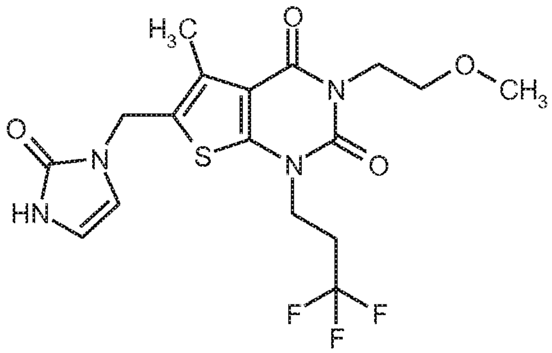

Eine Lösung von 450 mg (0.992 mmol) der Verbindung aus Bsp. 411A in 10 ml Methanol wurde zunächst mit 185 mg (2.28 mmol) Kaliumcyanat und dann tropfenweise mit 145 μl (1.68 mmol) Perchlorsäure (70% in Wasser) versetzt. Das Gemisch wurde bei RT gerührt. Nach 18 h und nach 3 Tagen wurden jeweils die gleichen Mengen an Kaliumcyanat und Perchlorsäure nochmals zugesetzt. Nachdem das Reaktionsgemisch 4 weitere Tage bei RT gerührt worden war, wurde es mit halbgesättigter wässriger Natriumhydrogencarbonat-Lösung versetzt und mit Ethylacetat extrahiert. Der organische Extrakt wurde mit gesättigter Natriumchlorid-Lösung gewaschen, über wasserfreiem Magnesiumsulfat getrocknet, filtriert und eingeengt. Aus dem verbliebenen Rückstand wurde die Titelverbindung mittels präparativer HPLC isoliert [Säule: Kinetex C18, 5 μm, 100 mm x 30 mm; Eluent A: Wasser + 0.07% Ameisensäure, Eluent B: Acetonitril; Gradient: 0.0-2.0 min 10% B, 2.2 min 20% B, 7.0 min 60% B, 7.5-9.0 min 92% B; Fluss: 70 ml/min; Temperatur: 25°C; Detektion: 210 nm]. Nach Einengen der Produktfraktionen und Trocknen des Rückstands im Hochvakuum wurden 123 mg (28% d. Th.) der Titelverbindung erhalten.
$^1$H-NMR (400 MHz, DMSO-$d_6$, δ/ppm): 10.00 (br. s, 1H), 6.48-6.41 (m, 1H), 6.35 (t, 1H), 4.81 (s, 2H), 4.08 (q, 2H), 4.05 (t, 2H), 3.49 (t, 2H), 3.31 (s, 3H), 2.82-2.65 (m, 2H), 2.47 (s, 3H).
LC/MS (Methode 1, ESIpos): $R_t$ = 0.78 min, m/z = 433 $[M+H]^+$.

**Beispiel 345**

1,3-Bis(2-methoxyethyl)-5-methyl-6-[(2-oxo-2,3-dihydro-1*H*-imidazol-1-yl)methyl]thieno[2,3-d]-pyrimidin-2,4(1*H*,3*H*)-dion

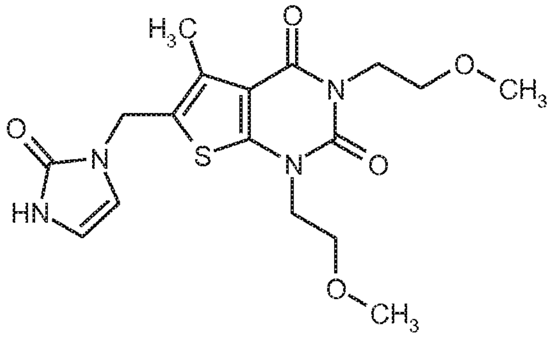

Eine Lösung von 520 mg (1.25 mmol) der Verbindung aus Bsp. 412A in 13 ml Methanol wurde zunächst mit 233 mg (2.88 mmol) Kaliumcyanat und dann tropfenweise mit 183 µl (2.13 mmol) Perchlorsäure (70% in Wasser) versetzt. Nach 6.5 Tagen Rühren bei RT wurde das Reaktionsgemisch mit halbgesättigter wässriger Natriumhydrogencarbonat-Lösung versetzt und mit Ethylacetat extrahiert. Der organische Extrakt wurde mit gesättigter Natriumchlorid-Lösung gewaschen, über wasserfreiem Magnesiumsulfat getrocknet, filtriert und eingeengt. Der verbliebene Rückstand wurde mittels präparativer HPLC gereinigt (Methode 8). Eindampfen der Produktfraktionen ergab ein noch leicht verunreinigtes Produkt, das durch nochmalige präparative HPLC weiter gereinigt wurde [Säule: Kinetex C18, 5 µm, 100 mm x 30 mm; Eluent A: Wasser + 0.07% Ameisensäure, Eluent B: Acetonitril; Gradient: 0.0-2.0 min 10% B, 2.2 min 20% B, 7.0 min 60% B, 7.5-9.0 min 92% B; Fluss: 70 ml/min; Temperatur: 25°C; Detektion: 210 nm]. Nach Einengen der Produktfraktionen und Trocknen des Rückstands im Hochvakuum wurden 87 mg (17% d. Th.) der Titelverbindung erhalten.
$^1$H-NMR (400 MHz, DMSO-$d_6$, δ/ppm): 10.00 (br. s, 1H), 6.43 (dd, 1H), 6.34 (t, 1H), 4.79 (s, 2H), 4.05 (t, 2H), 4.00 (t, 2H), 3.61 (t, 2H), 3.49 (t, 2H), 3.24 (s, 3H), 3.22 (s, 3H), 2.45 (s, 3H).
LC/MS (Methode 1, ESIpos): $R_t$ = 0.66 min, m/z = 395 $[M+H]^+$.

**Beispiel 346**

3-(2-Methoxyethyl)-5-methyl-6-[(2-oxo-2,3-dihydro-1*H*-imidazol-1-yl)methyl]-1-(tetrahydrofuran-2-ylmethyl)thieno[2,3-d]pyrimidin-2,4(1*H*,3*H*)-dion *(Racemat)*

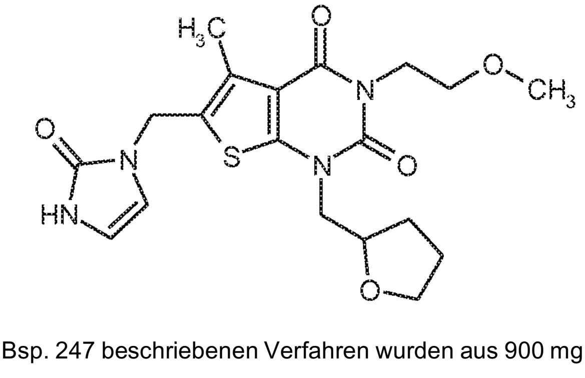

Analog zu dem unter Bsp. 247 beschriebenen Verfahren wurden aus 900 mg (1.67 mmol) der Verbindung aus

Bsp. 414A 302 mg (42% d. Th.) der Titelverbindung erhalten.
$^1$H-NMR (400 MHz, DMSO-$d_6$, δ/ppm): 9.99 (br. s, 1H), 6.46-6.40 (m, 1H), 6.34 (t, 1H), 4.79 (s, 2H), 4.26-4.14 (m, 1H), 4.10-3.97 (m, 3H), 3.78-3.54 (m, 3H), 3.49 (t, 2H), 3.23 (s, 3H), 2.45 (s, 3H), 2.04-1.74 (m, 3H), 1.71-1.57 (m, 1H).
LC/MS (Methode 6, ESIpos): $R_t$ = 1.19 min, m/z = 421 $[M+H]^+$.

**Beispiel 347**

3-(2-Methoxyethyl)-5-methyl-6-[(2-oxo-2,3-dihydro-1*H*-imidazol-1-yl)methyl]-1-(tetrahydrofuran-2-ylmethyl)thieno[2,3-d]pyrimidin-2,4(1*H*,3*H*)-dion (*Enantiomer 1*)

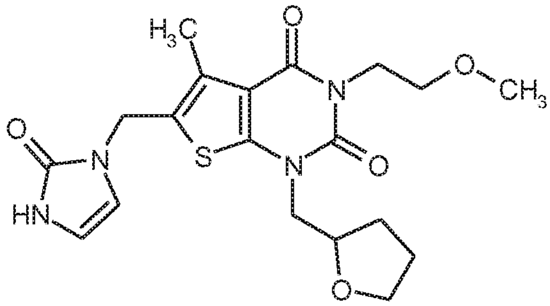

274 mg (0.652 mmol) der racemischen Verbindung aus Bsp. 346 wurden in 27 ml Methanol gelöst und in 54 Portionen über präparative SFC an chiraler Phase in die Enantiomere aufgetrennt [Säule: Daicel Chiralcel OJ-H, 5 μm, 250 mm x 30 mm; Laufmittel: Kohlendioxid/Methanol 90:10; Fluss: 100 ml/min; Temperatur: 40°C; Detektion: 210 nm]. Nach Eindampfen der Produktfraktionen und Trocknen im Hochvakuum wurden 99 mg (72% d. Th.) von Enantiomer 1 erhalten (>99% ee, chirale analytische HPLC).
$^1$H-NMR (400 MHz, DMSO-$d_6$, δ/ppm): 9.99 (br. s, 1H), 6.43 (dd, 1H), 6.34 (t, 1H), 4.79 (s, 2H), 4.25-4.15 (m, 1H), 4.10-3.97 (m, 3H), 3.78-3.55 (m, 3H), 3.49 (t, 2H), 3.31 (s, 3H), 2.45 (s, 3H), 2.03-1.75 (m, 3H), 1.71-1.58 (m, 1H).
Chirale analytische SFC [Säule: Daicel Chiralcel OJ-H, 3 μm, 50 mm x 4.6 mm; Laufmittel: Kohlendioxid/Methanol 80:20; Fluss: 3 ml/min; Temperatur: 40°C; Detektion: 210 nm]: $R_t$ = 0.93 min.

**Beispiel 348**

3-(2-Methoxyethyl)-5-methyl-6-[(2-oxo-2,3-dihydro-1*H*-imidazol-1-yl)methyl]-1-(tetrahydrofuran-2-ylmethyl)thieno[2,3-d]pyrimidin-2,4(1*H*,3*H*)-dion *(Enantiomer 2)*

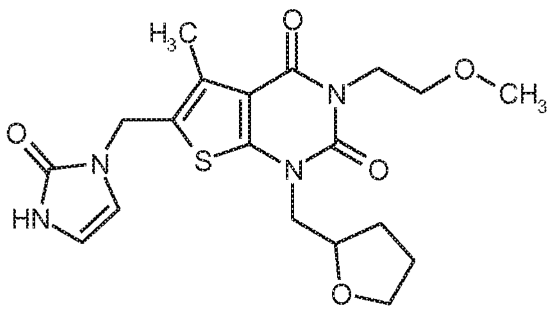

274 mg (0.652 mmol) der racemischen Verbindung aus Bsp. 346 wurden in 27 ml Methanol gelöst und in 54 Portionen über präparative SFC an chiraler Phase in die Enantiomere aufgetrennt [Säule: Daicel Chiralcel OJ-H, 5 μm, 250 mm x 30 mm; Laufmittel: Kohlendioxid/Methanol 90:10; Fluss: 100 ml/min; Temperatur: 40°C; Detektion: 210 nm]. Nach Eindampfen der Produktfraktionen und Trocknen im Hochvakuum wurden 89 mg (64% d. Th.) von Enantiomer 2 erhalten (>99% ee, chirale analytische HPLC).
$^1$H-NMR (400 MHz, DMSO-$d_6$, δ/ppm): 9.99 (br. s, 1H), 6.43 (t, 1H), 6.34 (t, 1H), 4.79 (s, 2H), 4.25-4.14 (m, 1H), 4.12-3.97 (m, 3H), 3.79-3.55 (m, 3H), 3.49 (t, 2H), 3.23 (s, 3H), 2.45 (s, 3H), 2.03-1.75 (m, 3H), 1.72-1.58 (m, 1H).

Chirale analytische SFC [Säule: Daicel Chiralcel OJ-H, 3 µm, 50 mm x 4.6 mm; Laufmittel: Kohlendioxid/Methanol 80:20; Fluss: 3 ml/min; Temperatur: 40°C; Detektion: 210 nm]: $R_t$ = 1.03 min.

**Beispiel 349**

6-[(4-Allyl-5-oxo-4,5-dihydro-1*H*-(1,2,4-triazol-1-yl)methyl]-3-ethyl-1-(fluormethyl)-5-methyl-thieno[2,3-d]pyrimidin-2,4(1*H*,3*H*)-dion

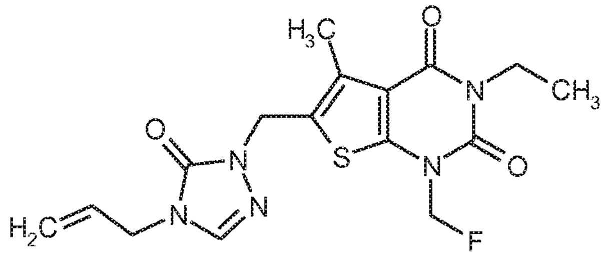

300 mg (1.07 mmol) der Verbindung aus Bsp. 383A und 171 mg (1.28 mmol) 4-Allyl-2,4-dihydro-3*H*-1,2,4-triazol-3-on wurden in 20 ml Dichlormethan gelöst und mit 462 mg (2.14 mmol) Tri-n-butylphosphin versetzt. Nach 30 min wurden 318 µl (1.6 mmol) Diisopropylazodicarboxylat (DIAD) hinzugetropft. Das Reaktionsgemisch wurde 1 h bei RT gerührt. Anschließend wurde zur Trockene eingeengt. Der erhaltene Rückstand wurde über eine Kieselgel-Kartusche chromatographiert (Biotage, 50 g Kieselgel, Laufmittel Hexan/Ethylacetat). Es wurden 205 mg (47% d. Th.) der Titelverbindung erhalten.
$^1$H-NMR (400 MHz, DMSO-$d_6$, δ/ppm): 7.97 (s, 1H), 6.06 (s, 1H), 5.96-5.84 (m, 2H), 5.19 (dd, 1H), 5.07 (dd, 1H), 5.03 (s, 2H), 4.20 (dt, 2H), 3.90 (q, 2H), 2.47 (s, 3H), 1.12 (t, 3H).
LC/MS (Methode 1, ESIpos): $R_t$ = 0.98 min, m/z = 380 $[M+H]^+$.

**Beispiel 350**

6-[(4-Allyl-5-oxo-4,5-dihydro-1*H*-1,2,4-triazol-1-yl)methyl]-3-ethyl-1-(3-fluorpropyl)-5-methyl-thieno[2,3-d]pyrimidin-2,4(1*H*,3*H*)-dion

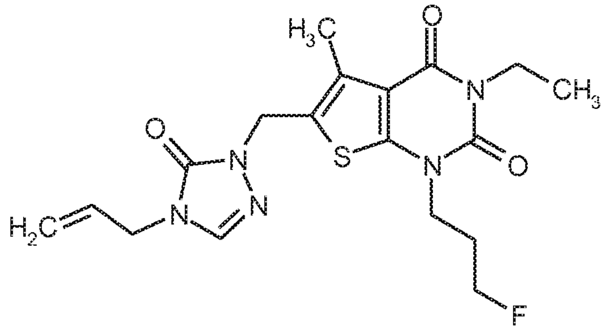

255 mg (0.807 mmol) der Verbindung aus Bsp. 141A und 128 mg (0.968 mmol) 4-Allyl-2,4-dihydro-3*H*-1,2,4-triazol-3-on wurden in 14 ml Dichlormethan gelöst und mit 349 mg (1.61 mmol) Tri-n-butylphosphin versetzt. Nach 30 min wurden 240 µl (1.613 mmol) Diisopropylazodicarboxylat (DIAD) hinzugetropft. Das Reaktionsgemisch wurde 3 h bei RT gerührt. Anschließend wurde zur Trockene eingeengt. Der erhaltene Rückstand wurde über eine Kieselgel-Kartusche chromatographiert (Biotage, 100 g Kieselgel, Laufmittel Hexan/Ethylacetat). Es wurden 113 mg (32% d. Th.) der Titelverbindung erhalten.
$^1$H-NMR (400 MHz, DMSO-$d_6$, δ/ppm): 7.96 (s, 1H), 5.90 (ddt, 1H), 5.19 (dq, 1H), 5.06 (dq, 1H), 5.00 (s, 2H), 4.58 (t, 1H), 4.46 (t, 1H), 4.20 (dt, 2H), 3.96 (t, 2H), 3.89 (q, 2H), 2.46 (s, 3H), 2.07 (quin, 1H), 2.04-1.96 (m, 1H), 1.10 (t, 3H).
LC/MS (Methode 1, ESIpos): $R_t$ = 0.99 min, m/z = 408 $[M+H]^+$.

**Beispiel 351**

6-[(4-Allyl-5-oxo-4,5-dihydro-1*H*-1,2,4-triazol-1-yl)methyl]-3-ethyl-1-(3-methoxypropyl)-5-methylthieno[2,3-d]pyrimidin-2,4(1*H*,3*H*)-dion

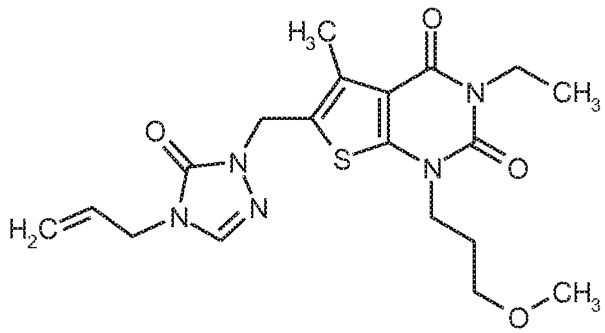

101 mg (0.314 mmol) der Verbindung aus Bsp. 384A und 58 mg (0.433 mmol) 4-Allyl-2,4-dihydro-3*H*-1,2,4-triazol-3-on wurden in 4 ml Dichlormethan gelöst und mit 136 mg (0.627 mmol) Tri-*n*-butylphosphin versetzt. Nach 30 min wurden 93 µl (0.47 mmol) Diisopropylazodicarboxylat (DIAD) hinzugetropft. Das Reaktionsgemisch wurde 18 h bei RT gerührt. Anschließend wurde zur Trockene eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 49 mg (35% d. Th.) der Titelverbindung erhalten.
$^1$H-NMR (500 MHz, DMSO-$d_6$, δ/ppm): 7.96 (s, 1H), 5.90 (ddt, 1H), 5.19 (dq, 1H), 5.06 (dq, 1H), 5.00 (s, 2H), 4.20 (dt, 2H), 3.92-3.85 (m, 4H), 3.38-3.34 (m, 2H), 3.18 (s, 3H), 2.46 (s, 3H), 1.91-1.84 (m, 2H), 1.10 (t, 3H).
LC/MS (Methode 1, ESIpos): $R_t$ = 1.0 min, m/z = 420 $[M+H]^+$.

**Beispiel 352**

3-Ethyl-1-(fluormethyl)-5-methyl-6-[(5-oxo-4,5-dihydro-1*H*-1,2,4-triazol-1-yl)methyl]thieno-[2,3-d]pyrimidin-2,4(1*H*,3*H*)-dion

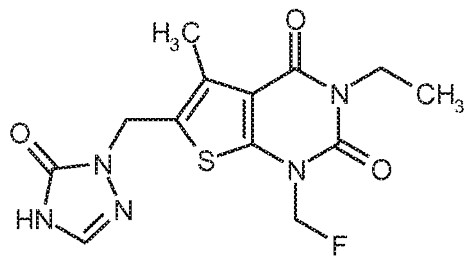

200 mg (0.495 mmol) der Verbindung aus Bsp. 349 wurden in 5 ml 1,4-Dioxan gelöst und mit 79 mg (0.297 mmol) Triphenylphosphin versetzt. Dann wurden 39 µl (1.04 mmol) Ameisensäure, 173 µl (1.24 mmol) Triethylamin sowie 86 mg (0.074 mmol) Tetrakis(triphenylphosphin)palladium(0) hinzugefügt. Das Gemisch wurde 18 h in einem Mikrowellenofen (Biotage Initiator mit dynamischer Steuerung der Einstrahlleistung) auf 125°C erhitzt. Danach wurde das Reaktionsgemisch auf halbgesättigte wässrige Natriumchlorid-Lösung (70 ml) gegeben. Es wurde mit Ethylacetat extrahiert. Die vereinigten organischen Phasen wurden über Natriumsulfat getrocknet, filtriert und eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 73 mg (41% d. Th.) der Titelverbindung erhalten.
$^1$H-NMR (400 MHz, DMSO-$d_6$, δ/ppm): 11.65 (br. s, 1H), 7.85 (s, 1H), 6.09-5.91 (m, 2H), 4.97 (s, 2H), 3.90 (q, 2H), 2.46 (s, 3H), 1.12 (t, 3H).
LC/MS (Methode 1, ESIpos): $R_t$ = 0.8 min, m/z = 340 $[M+H]^+$.

**Beispiel 353**

3-Ethyl-1-(3-fluorpropyl)-5-methyl-6-[(5-oxo-4,5-dihydro-1*H*-1,2,4-triazol-1-yl)methyl]thieno-[2,3-d]pyrimidin-2,4(1*H*,3*H*)-dion

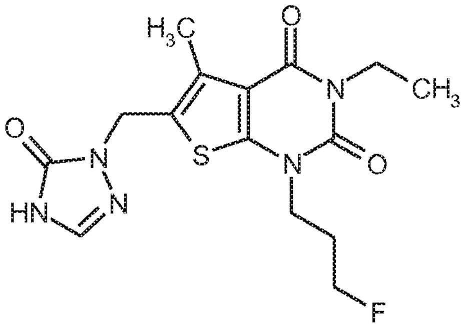

113 mg (0.263 mmol) der Verbindung aus Bsp. 350 wurden in 5 ml 1,4-Dioxan gelöst und mit 41 mg (0.158 mmol) Triphenylphosphin versetzt. Dann wurden 21 μl (0.553 mmol) Ameisensäure, 92 μl (0.659 mmol) Triethylamin sowie 46 mg (0.04 mmol) Tetrakis(triphenylphosphin)palladium(0) hinzugefügt. Das Gemisch wurde 22 h in einem Mikrowellenofen (Biotage Initiator mit dynamischer Steuerung der Einstrahlleistung) auf 120°C erhitzt. Danach wurde das Reaktionsgemisch auf halbgesättigte wässrige Natriumchlorid-Lösung (70 ml) gegeben. Es wurde mit Ethylacetat extrahiert. Die vereinigten organischen Phasen wurden über Natriumsulfat getrocknet, filtriert und eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 61 mg (62% d. Th.) der Titelverbindung erhalten.
$^1$H-NMR (400 MHz, DMSO-$d_6$, δ/ppm): 11.62 (br. s, 1H), 7.84 (s, 1H), 4.94 (s, 2H), 4.58 (t, 1H), 4.46 (t, 1H), 3.96 (t, 2H), 3.89 (q, 2H), 2.46 (s, 3H), 2.12-2.04 (m, 1H), 2.01 (quin, 1H), 1.10 (t, 3H).
LC/MS (Methode 1, ESIpos): $R_t$ = 0.84 min, m/z = 368 [M+H]$^+$.

**Beispiel 354**

3-Ethyl-1-(3-methoxypropyl)-5-methyl-6-[(5-oxo-4,5-dihydro-1*H*-1,2,4-triazol-1-yl)methyl]thieno-[2,3-d]pyrimidin-2,4(1*H*,3*H*)-dion

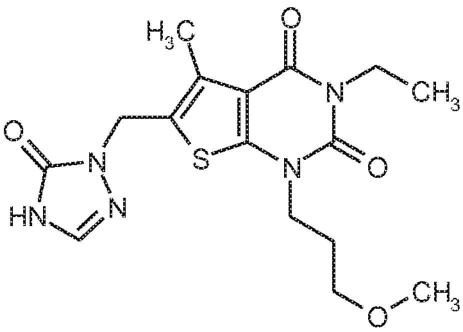

49 mg (0.111 mmol) der Verbindung aus Bsp. 351 wurden in 3 ml 1,4-Dioxan gelöst und mit 18 mg (0.067 mmol) Triphenylphosphin versetzt. Dann wurden 9 μl (0.223 mmol) Ameisensäure, 39 μl (0.277 mmol) Triethylamin sowie 19 mg (0.017 mmol) Tetrakis(triphenylphosphin)palladium(0) hinzugefügt. Das Gemisch wurde 18 h in einem Mikrowellenofen (Biotage Initiator mit dynamischer Steuerung der Einstrahlleistung) auf 125°C erhitzt. Danach wurde das Reaktionsgemisch auf halbgesättigte wässrige Natriumchlorid-Lösung (40 ml) gegeben. Es wurde mit Ethylacetat extrahiert. Die vereinigten organischen Phasen wurden über Natriumsulfat getrocknet, filtriert und eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 12 mg (27% d. Th.) der Titelverbindung erhalten.

$^1$H-NMR (400 MHz, DMSO-$d_6$, δ/ppm): 11.61 (br. s, 1H), 7.84 (s, 1H), 4.94 (s, 2H), 3.95-3.84 (m, 4H), 3.19 (s, 3H), 2.46 (s, 3H), 1.88 (quin, 2H), 1.10 (t, 3H).
LC/MS (Methode 1, ESIpos): $R_t$ = 0.84 min, m/z = 380 $[M+H]^+$.

**Beispiel 355**

3-Ethyl-5-methyl-6-[(5-oxo-4-propyl-4,5-dihydro-1*H*-1,2,4-triazol-1-yl)methyl]-1-(3,3,3-trifluorpropyl)thieno[2,3-d]pyrimidin-2,4(1*H*,3*H*)-dion

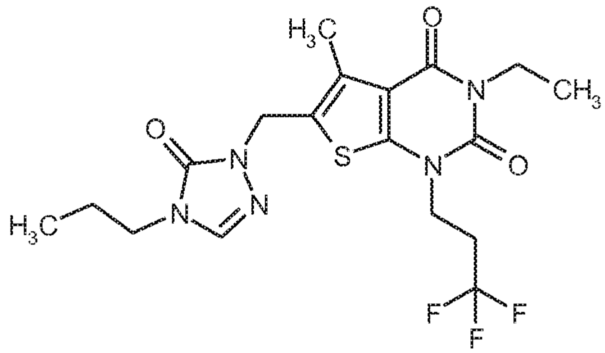

176 mg (0.393 mmol) der Verbindung aus Bsp. 110 wurden in 10 ml 1,4-Dioxan gelöst und mit 32 μl (0.825 mmol) Ameisensäure, 137 μl (0.982 mmol) Triethylamin und 45 mg (0.039 mmol) Tetrakis(triphenylphosphin)palladium(0) versetzt. Das Gemisch wurde 15 h unter Rückfluß erhitzt. Anschließend wurden weitere 22 μl (0.589 mmol) Ameisensäure zugesetzt. Nach insgesamt 72 h Reaktionszeit wurde das Reaktionsgemisch auf halbgesättigte wässrige Natriumchlorid-Lösung (40 ml) gegeben. Es wurde mit Ethylacetat extrahiert. Die vereinigten organischen Phasen wurden über Natriumsulfat getrocknet, filtriert und eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 75 mg (43% d. Th.) der Titelverbindung erhalten.
$^1$H-NMR (400 MHz, DMSO-$d_6$, δ/ppm): 8.00 (s, 1H), 5.00 (s, 2H), 4.07 (t, 2H), 3.89 (q, 2H), 3.52 (t, 2H), 2.82-2.66 (m, 2H), 2.46 (s, 3H), 1.61 (sext, 2H), 1.10 (t, 3H), 0.81 (t, 3H).
LC/MS (Methode 3, ESIneg): $R_t$ = 1.14 min, m/z = 490 $[M-H+HCOOH]^-$.

**Beispiel 356**

[1-{[3-Isopropyl-5-methyl-2,4-dioxo-1-(tetrahydrofuran-2-ylmethyl)-1,2,3,4-tetrahydrothieno-[2,3-d]pyrimidin-6-yl]methyl}imidazolidin-2-yliden]cyanamid (*Enantiomer 1*)

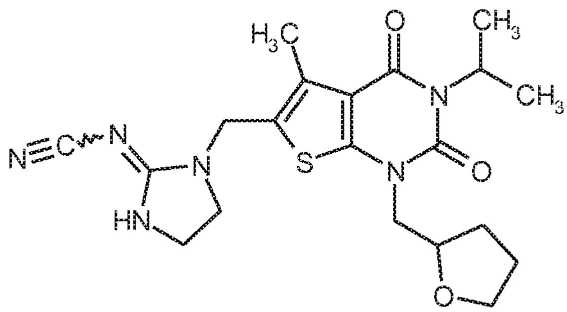

320 mg (0.743 mmol) der racemischen Verbindung aus Bsp. 267 wurden in 20 ml Methanol gelöst und in 40 Portionen über präparative SFC an chiraler Phase in die Enantiomere aufgetrennt [Säule: Daicel Chiralcel OX-H, 5 μm, 250 mm x 20 mm; Laufmittel: Kohlendioxid/Methanol 65:35; Fluss: 80 ml/min; Temperatur: 40°C; Detektion: 210 nm]. Nach Eindampfen der Produktfraktionen und Trocknen im Hochvakuum wurden 127 mg (79% d. Th.) von Enantiomer

1 erhalten (>99% ee, chirale analytische HPLC).
$^{1}$H-NMR (400 MHz, DMSO-$d_6$, δ/ppm): 8.06 (s, 1H), 5.14 (sept, 1H), 4.46 (s, 2H), 4.27-4.16 (m, 1H), 4.04 (dd, 1H), 3.80-3.71 (m, 1H), 3.69-3.57 (m, 2H), 3.51-3.36 (m, 4H), 2.39 (s, 3H), 2.05-1.75 (m, 3H), 1.72-1.59 (m, 1H), 1.40 (d, 6H).

Chirale analytische SFC [Säule: Daicel Chiralcel OX-H, 3 μm, 50 mm x 4.6 mm; Laufmittel: Kohlendioxid/Methanol 60:40; Fluss: 3 ml/min; Temperatur: 40°C; Detektion: 210 nm]: $R_t$ = 3.61 min.

**Beispiel 357**

[1-{[3-Isopropyl-5-methyl-2,4-dioxo-1-(tetrahydrofuran-2-ylmethyl)-1,2,3,4-tetrahydrothieno-[2,3-d]pyrimidin-6 yl]methyl}imidazolidin-2-yliden]cyanamid (*Enantiomer 2*)

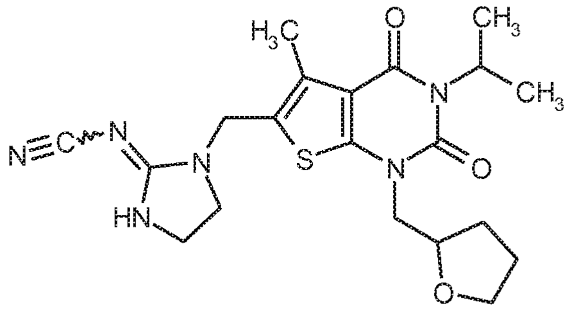

320 mg (0.743 mmol) der racemischen Verbindung aus Bsp. 267 wurden in 20 ml Methanol gelöst und in 40 Portionen über präparative SFC an chiraler Phase in die Enantiomere aufgetrennt [Säule: Daicel Chiralcel OX-H, 5 μm, 250 mm x 20 mm; Laufmittel: Kohlendioxid/Methanol 65:35; Fluss: 80 ml/min; Temperatur: 40°C; Detektion: 210 nm]. Nach Eindampfen der Produktfraktionen und Trocknen im Hochvakuum wurden 109 mg (68% d. Th.) von Enantiomer 2 erhalten (>99% ee, chirale analytische HPLC).
$^{1}$H-NMR (400 MHz, DMSO-$d_6$, δ/ppm): 8.06 (s, 1H), 5.14 (sept, 1H), 4.46 (s, 2H), 4.27-4.15 (m, 1H), 4.04 (dd, 1H), 3.81-3.71 (m, 1H), 3.69-3.57 (m, 2H), 3.51-3.35 (m, 4H), 2.39 (s, 3H), 2.07-1.74 (m, 3H), 1.72-1.59 (m, 1H), 1.40 (d, 6H).

Chirale analytische SFC [Säule: Daicel Chiralcel OX-H, 3 μm, 50 mm x 4.6 mm; Laufmittel: Kohlendioxid/Methanol 60:40; Fluss: 3 ml/min; Temperatur: 40°C; Detektion: 210 nm]: $R_t$ = 6.55 min.

**Beispiel 358**

[1-{[5-Methyl-2,4-dioxo-3-(2,2,2-trifluorethyl)-1-(3,3,3-trifluorpropyl)-1,2,3,4-tetrahydrothieno-[2,3-d]pyrimidin-6-yl]methyl}imidazolidin-2-yliden]cyanamid

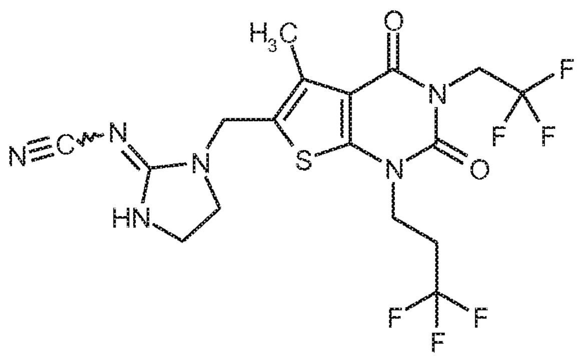

361 mg (0.417 mmol, 50% Reinheit) der Verbindung aus Bsp. 247A wurden in 2.4 ml DMF gelöst und mit 92 mg (0.626 mmol) Dimethyl-*N*-cyanodithioiminocarbonat sowie 115 mg (0.835 mmol) Kaliumcarbonat versetzt. Das Gemisch wurde 3 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mittels einer ersten präparativen HPLC (Methode 8) vorgereinigt. Aus den vereinigten Produktfraktionen wurde mittels einer zweiten präparativen HPLC die Titelverbindung in reiner Form isoliert [Säule: XBridge C18, 5 μm, 100 mm x 30 mm; Eluent A: Wasser + 0.07% Ameisensäure, Eluent B: Acetonitril; Gradient: 0.0-2.0 min 10% B, 2.2 min 30% B, 7.0 min 60% B, 7.5-9.0 min 92% B; Fluss: 70 ml/ min; Temperatur: 25°C; Detektion: 210 nm]. Nach Eindampfen der Produktfraktionen und Trocknen im Hochvakuum wurden 73 mg (36% d. Th.) der Titelverbindung erhalten.

$^1$H-NMR (400 MHz, DMSO-$d_6$, δ/ppm): 8.10 (s, 1H), 4.70 (q, 2H), 4.52 (s, 2H), 4.15 (t, 2H), 3.56-3.36 (m, 4H), 2.89-2.68 (m, 2H), 2.42 (s, 3H).

LC/MS (Methode 17, ESIpos): $R_t$ = 1.63 min, m/z = 483.10 [M+H]$^+$.

**Beispiel 359**

[1-{[5-Methyl-1-(oxetan-2-ylmethyl)-2,4-dioxo-3-(2,2,2-trifluorethyl)-1,2,3,4-tetrahydrothieno-[2,3-d]pyrimidin-6-yl]methyl}imidazolidin-2-yliden]cyanamid (*Stereoisomerengemisch*)

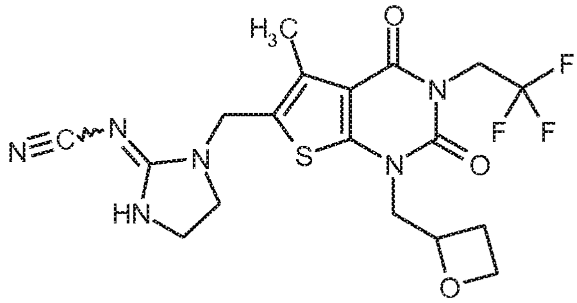

400 mg (0.817 mmol, 83% Reinheit) der Verbindung aus Bsp. 251A wurden in 7.4 ml DMF gelöst und mit 179 mg (1.23 mmol) Dimethyl-*N*-cyanodithioiminocarbonat sowie 226 mg (1.63 mmol) Kaliumcarbonat versetzt. Das Gemisch wurde ca. 18 h bei 80°C in einem geschlossenen Gefäß gerührt. Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde in einem Wasser/Acetonitril-Gemisch bei RT verrührt. Der Feststoff wurde abgesaugt und im Hochvakuum getrocknet. Dies ergab eine erste Teilmenge der Titelverbindung. Die Mutterlauge des Verrührens wurde eingeengt und der Rückstand mittels präparativer HPLC gereinigt (Methode 8). Die Produktfraktionen wurden vereinigt, eingedampft, getrocknet und mit der ersten Teilmenge der Titelverbindung vereinigt. Insgesamt wurden so 210 mg (55% d. Th.) der Titelverbindung erhalten.

$^1$H-NMR (400 MHz, DMSO-$d_6$, δ/ppm): 8.08 (s, 1H), 5.08-4.95 (m, 1H), 4.70 (q, 2H), 4.54-4.36 (m, 2H), 4.49 (s, 2H), 4.26-4.09 (m, 2H), 3.53-3.36 (m, 4H), 2.77-2.62 (m, 1H), 2.53-2.44 (m, 1H, teilweise überdeckt vom DMSO-Signal), 2.41 (s, 3H).

LC/MS (Methode 17, ESIpos): $R_t$ = 1.49 min, m/z = 457.13 [M+H]$^+$.

**Beispiel 360**

[1-{[5-Methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl}imidazolidin-2-yliden]cyanamid (*Enantiomer 1*)

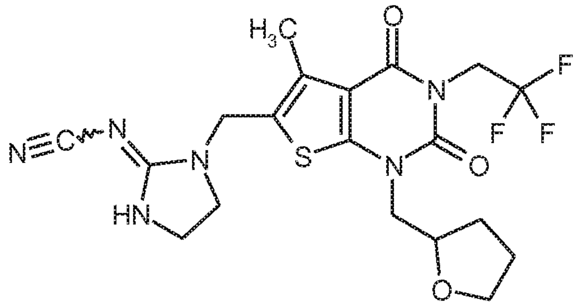

199 mg (0.423 mmol) der racemischen Verbindung aus Bsp. 269 wurden in 8 ml Ethanol gelöst und in 32 Portionen über präparative HPLC an chiraler Phase in die Enantiomere aufgetrennt [Säule: Daicel Chiralcel OX-H, 5 µm, 250 mm x 20 mm; Laufmittel: Isohexan/Ethanol 2:3; Fluss: 15 ml/min; Temperatur: 25°C; Detektion: 210 nm]. Nach Eindampfen der Produktfraktionen wurde der verbliebene Feststoff mit wenig Diisopropylether bei RT verrührt. Nach Absaugen und Trocknen des Feststoffs wurden 59 mg (59% d. Th.) von Enantiomer 1 erhalten (>99% ee, chirale analytische HPLC).
$^1$H-NMR (400 MHz, DMSO-$d_6$, δ/ppm): 8.09 (s, 1H), 4.70 (q, 2H), 4.49 (s, 2H), 4.28-4.17 (m, 1H), 4.07 (dd, 1H), 3.81-3.69 (m, 2H), 3.67-3.56 (m, 1H), 3.53-3.37 (m, 4H), 2.41 (s, 3H), 2.05-1.76 (m, 3H), 1.74-1.59 (m, 1H).
Chirale analytische HPLC [Säule: Daicel Chiralcel OX-H, 3 µm, 50 mm x 4.6 mm; Laufmittel: Isohexan/Ethanol 1:1; Fluss: 1 ml/min; Temperatur: 40°C; Detektion: 220 nm]: $R_t$ = 2.62 min.

## Beispiel 361

[1-{[5-Methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl)imidazolidin-2-yliden]cyanamid (*Enantiomer 2*)

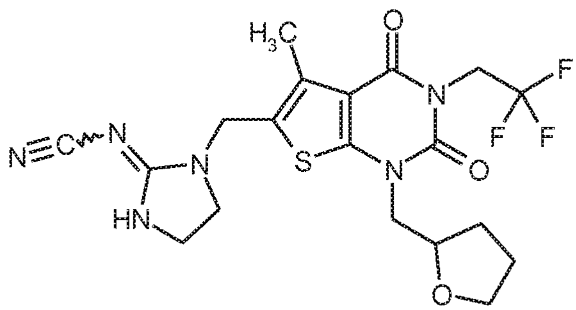

199 mg (0.423 mmol) der racemischen Verbindung aus Bsp. 269 wurden in 8 ml Ethanol gelöst und in 32 Portionen über präparative HPLC an chiraler Phase in die Enantiomere aufgetrennt [Säule: Daicel Chiralcel OX-H, 5 µm, 250 mm x 20 mm; Laufmittel: Isohexan/Ethanol 2:3; Fluss: 15 ml/min; Temperatur: 25°C; Detektion: 210 nm]. Nach Eindampfen der Produktfraktionen wurde der verbliebene Feststoff mit wenig Diisopropylether bei RT verrührt. Nach Absaugen und Trocknen des Feststoffs wurden 58 mg (58% d. Th.) von Enantiomer 2 erhalten (>99% ee, chirale analytische HPLC).
$^1$H-NMR (400 MHz, DMSO-$d_6$, δ/ppm): 8.09 (s, 1H), 4.70 (q, 2H), 4.49 (s, 2H), 4.29-4.17 (m, 1H), 4.07 (dd, 1H), 3.81-3.69 (m, 2H), 3.67-3.56 (m, 1H), 3.52-3.37 (m, 4H), 2.41 (s, 3H), 2.05-1.76 (m, 3H), 1.73-1.61 (m, 1H).
Chirale analytische HPLC [Säule: Daicel Chiralcel OX-H, 3 µm, 50 mm x 4.6 mm; Laufmittel: Isohexan/Ethanol 1:1; Fluss: 1 ml/min; Temperatur: 40°C; Detektion: 220 nm]: $R_t$ = 3.46 min.

## Beispiel 362

[1-{[3-(2-Methoxyethyl)-5-methyl-2,4-dioxo-1-(3,3,3-trifluorpropyl)-1,2,3,4-tetrahydrothieno-[2,3-d]pyrimidin-6-yl]methyl}imidazolidin-2-yliden]

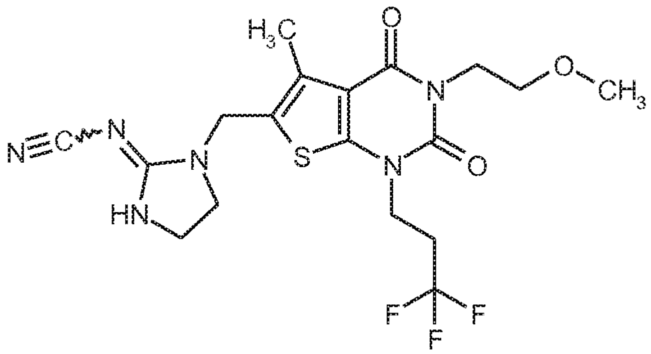

770 mg (1.72 mmol, 91% Reinheit) der Verbindung aus Bsp. 319A wurden in 17 ml DMF gelöst und mit 376 mg (2.57 mmol) Dimethyl-*N*-cyanodithioiminocarbonat sowie 474 mg (3.43 mmol) Kaliumcarbonat versetzt. Das Gemisch wurde 3.75 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mit einem Wasser/ Acetonitril-Gemisch bei RT verrührt. Der Feststoff wurde abgesaugt und im Hochvakuum getrocknet. Dies ergab eine erste Teilmenge der Titelverbindung (139 mg). Die Mutterlauge des Verrührens wurde eingeengt und der Rückstand mittels präparativer HPLC gereinigt (Methode 8). Die Produktfraktionen wurden vereinigt, eingedampft und getrocknet. Dies ergab eine zweite Teilmenge der Titelverbindung (141 mg). Insgesamt wurden so 280 mg (35% d. Th.) der Titelverbindung erhalten.
$^1$H-NMR (400 MHz, DMSO-$d_6$, δ/ppm): 8.08 (s, 1H), 4.50 (s, 2H), 4.11 (t, 2H), 4.06 (t, 2H), 3.49 (t, 2H), 3.49-3.37 (m, 4H), 3.24 (s, 3H), 2.90-2.64 (m, 2H), 2.42 (s, 3H).
LC/MS (Methode 17, ESIpos): $R_t$ = 1.50 min, m/z = 459.14 [M+H]$^+$.

## Beispiel 363

[1-{[1,3-Bis(2-methoxyethyl)-5-methyl-2,4-dioxo-1,2,3,4-tetrahydrothieno[2,3-d]pyrimidin-6-yl]-methyl}imidazolidin-2-yliden]cyanamid

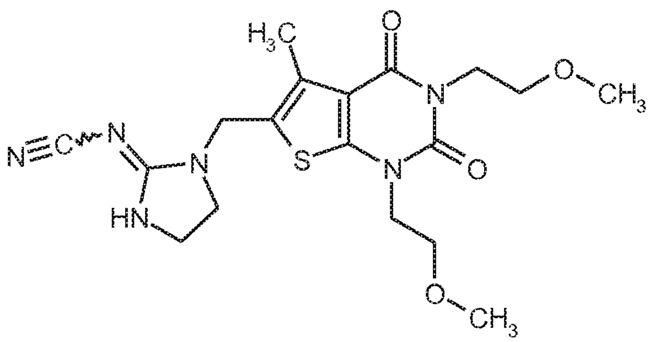

Analog zu dem unter Bsp. 268 beschriebenen Verfahren wurden aus 713 mg (1.48 mmol, 77% Reinheit) der Verbindung aus Bsp. 262A und 325 mg (2.22 mmol) Dimethyl-*N*-cyanodithioiminocarbonat 255 mg (40% d. Th.) der Titelverbindung erhalten. Die Reaktionszeit betrug hier 5.75 h.
$^1$H-NMR (400 MHz, DMSO-$d_6$, δ/ppm): 8.07 (s, 1H), 4.48 (s, 2H), 4.05 (t, 2H), 4.03 (t, 2H), 3.63 (t, 2H), 3.50 (t, 2H), 3.46-3.41 (m, 4H), 3.24 (s, 6H), 2.40 (s, 3H).
LC/MS (Methode 5, ESIpos): $R_t$ = 0.95 min, m/z = 421 [M+H]$^+$.

**Beispiel 364**

[1-{[3-(2-Methoxyethyl)-5-methyl-1-(oxetan-2-ylmethyl)-2,4-dioxo-1,2,3,4-tetrahydrothieno-[2,3-d]pyrimidin-6-yl]methyl}imidazolidin-2-yliden]cyanamid (*nicht-racemisches Enantiomerengemisch*)

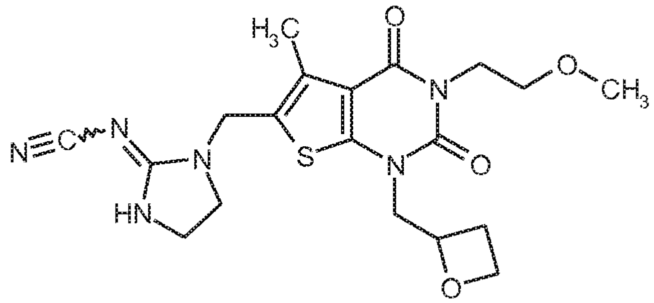

Analog zu dem unter Bsp. 359 beschriebenen Verfahren wurden aus 350 mg (0.824 mmol, 90% Reinheit) der Verbindung aus Bsp. 405A und 181 mg (1.23 mmol) Dimethyl-*N*-cyanodithioiminocarbonat 148 mg (41% d. Th.) der Titelverbindung erhalten.
$^1$H-NMR (400 MHz, DMSO-$d_6$, $\delta$/ppm): 8.07 (s, 1H), 5.00 (dt, 1H), 4.53-4.37 (m, 2H), 4.47 (s, 2H), 4.17-4.11 (m, 2H), 4.06 (t, 2H), 3.50 (t, 2H), 3.49-3.39 (m, 4H), 3.24 (s, 3H), 2.76-2.63 (m, 1H), 2.54-2.45 (m, 1H, teilweise überdeckt vom DMSO-Signal), 2.40 (s, 3H).
LC/MS (Methode 17, ESIpos): $R_t$ = 1.17 min, m/z = 433.16 $[M+H]^+$.

**Beispiel 365**

[1-{[3-(2-Methoxyethyl)-5-methyl-1-(oxetan-2-ylmethyl)-2,4-dioxo-1,2,3,4-tetrahydrothieno-[2,3-d]pyrimidin-6-yl]methyl}imidazolidin-2-yliden]cyanamid (*Enantiomer 1*)

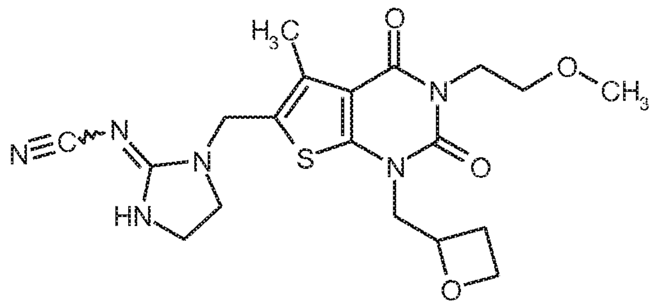

135 mg (0.312 mmol) des Enantiomerengemisches aus Bsp. 364 wurden in einem Gemisch aus 15 ml Ethanol und 15 ml Dichlormethan gelöst und in 30 Portionen über präparative HPLC an chiraler Phase in die Enantiomere aufgetrennt [Säule: Daicel Chiralpak IC, 5 $\mu$m, 250 mm x 20 mm; Laufmittel: Ethanol; Fluss: 15 ml/min; Temperatur: 55°C; Detektion: 220 nm]. Nach Eindampfen der Produktfraktionen und Trocknen im Hochvakuum wurden 107 mg (79% d. Th.) von Enantiomer 1 erhalten (>99% ee, chirale analytische HPLC).
$^1$H-NMR (400 MHz, DMSO-$d_6$, $\delta$/ppm): 8.07 (s, 1H), 5.00 (quin, 1H), 4.53-4.37 (m, 2H), 4.47 (s, 2H), 4.18-4.10 (m, 2H), 4.06 (t, 2H), 3.50 (t, 2H), 3.47-3.37 (m, 4H), 3.24 (s, 3H), 2.75-2.62 (m, 1H), 2.53-2.45 (m, 1H, teilweise überdeckt vom DMSO-Signal), 2.40 (s, 3H).
Chirale analytische HPLC [Säule: Daicel Chiralpak IC, 5 $\mu$m, 250 mm x 4.6 mm; Laufmittel: Ethanol; Fluss: 1 ml/min; Temperatur: 50°C; Detektion: 220 nm]: $R_t$ = 13.79 min.

**Beispiel 366**

[1-{[3-(2-Methoxyethyl)-5-methyl-1-(oxetan-2-ylmethyl)-2,4-dioxo-1,2,3,4-tetrahydrothieno-[2,3-d]pyrimidin-6yl]methyl}imidazolidin-2-yliden]cyanamid (*Enantiomer 2*)

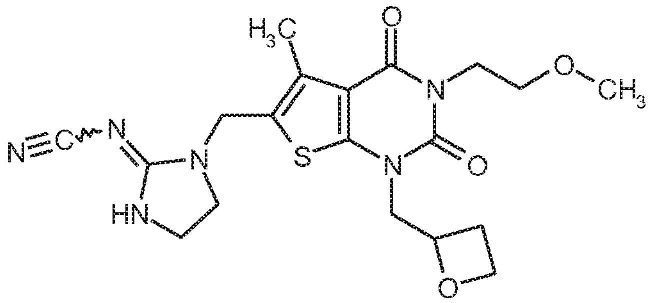

135 mg (0.312 mmol) des Enantiomerengemisches aus Bsp. 364 wurden in einem Gemisch aus 15 ml Ethanol und 15 ml Dichlormethan gelöst und in 30 Portionen über präparative HPLC an chiraler Phase in die Enantiomere aufgetrennt [Säule: Daicel Chiralpak IC, 5 μm, 250 mm x 20 mm; Laufmittel: Ethanol; Fluss: 15 ml/min; Temperatur: 55°C; Detektion: 220 nm]. Nach Eindampfen der Produktfraktionen, Verrühren in Pentan (mit 1 Tropfen Dichlormethan), Absaugen und Trocknen im Hochvakuum wurden 13 mg (9% d. Th.) von Enantiomer 2 erhalten (98% ee, chirale analytische HPLC).
$^1$H-NMR (400 MHz, DMSO-$d_6$, δ/ppm): 8.07 (s, 1H), 5.01 (quin, 1H), 4.52-4.36 (m, 2H), 4.47 (s, 2H), 4.17-4.10 (m, 2H), 4.06 (t, 2H), 3.50 (t, 2H), 3.47-3.37 (m, 4H), 3.24 (s, 3H), 2.75-2.63 (m, 1H), 2.55-2.45 (m, 1H, teilweise überdeckt vom DMSO-Signal), 2.40 (s, 3H).

Chirale analytische HPLC [Säule: Daicel Chiralpak IC, 5 μm, 250 mm x 4.6 mm; Laufmittel: Ethanol; Fluss: 1 ml/min; Temperatur: 50°C; Detektion: 220 nm]: $R_t$ = 17.29 min.

**Beispiel 367**

[1-([3-(2-Methoxyethyl)-5-methyl-2,4-dioxo-1-(tetrahydrofuren-2-ylmethyl)-1,2,3,4-tetrahydrothieno[2,3-d]pyrimidin-6-yl]methyl}imidazolidin-2-yliden]cyanamid *(Racemat)*

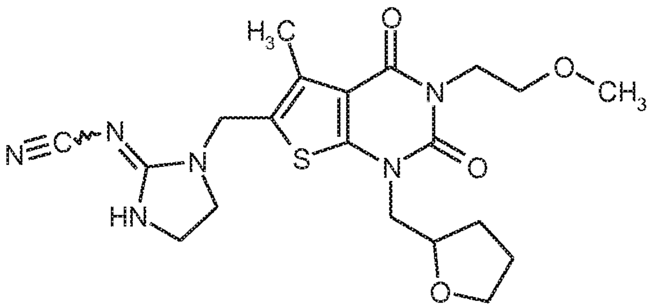

Analog zu dem unter Bsp. 362 beschriebenen Verfahren wurden aus 875 mg (1.99 mmol, 90% Reinheit) der Verbindung aus Bsp. 321A und 436 mg (2.98 mmol) Dimethyl-*N*-cyanodithioiminocarbonat 332 mg (36% d. Th.) der Titelverbindung erhalten.
$^1$H-NMR (400 MHz, DMSO-$d_6$, δ/ppm): 8.07 (s, 1H), 4.47 (s, 2H), 4.27-4.17 (m, 1H), 4.11-3.98 (m, 3H), 3.81-3.57 (m, 3H), 3.50 (t, 2H), 3.47-3.37 (m, 4H), 3.31 (s, 3H), 2.40 (s, 3H), 2.04-1.76 (m, 3H), 1.73-1.61 (m, 1H).
LC/MS (Methode 1, ESIpos): $R_t$ = 0.73 min, m/z = 447 [M+H]$^+$.

**Beispiel 368**

[1-{-(tetrahydrofuran-2-yhnethyl)-1,2,3,4-tetrahydrothieno[2,3-d]pyrimidin-6-yl]methyl)imidazolidin-2-yliden]cyanamid (*Enantiomer 1*)

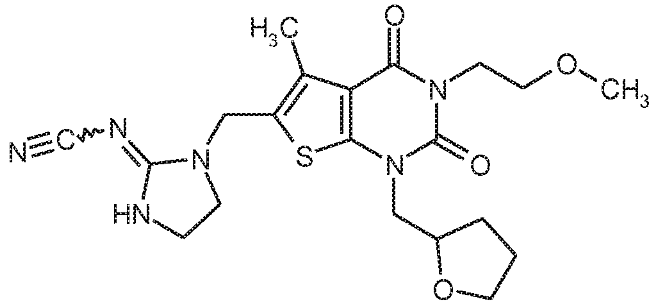

314 mg (0.703 mmol) der racemischen Verbindung aus Bsp. 367 wurden in 45 ml Methanol gelöst und in 45 Portionen über präparative SFC an chiraler Phase in die Enantiomere aufgetrennt [Säule: Daicel Chiralcel OJ-H, 5 µm, 250 mm x 30 mm; Laufmittel: Kohlendioxid/Methanol 90:10; Fluss: 100 ml/min; Temperatur: 40°C; Detektion: 210 nm]. Nach Eindampfen der Produktfraktionen, Verrühren des Feststoffs mit einem Gemisch aus 10 ml Pentan und 0.5 ml Dichlormethan, Absaugen und Trocknen im Hochvakuum wurden 114 mg (72% d. Th.) von Enantiomer 1 erhalten (>99% ee, chirale analytische HPLC).
$^1$H-NMR (400 MHz, DMSO-$d_6$, δ/ppm): 8.07 (s, 1H), 4.47 (s, 2H), 4.28-4.16 (m, 1H), 4.12-3.98 (m, 3H), 3.82-3.56 (m, 3H), 3.50 (t, 2H), 3.47-3.37 (m, 4H), 3.24 (s, 3H), 2.40 (s, 3H), 2.05-1.76 (m, 3H), 1.73-1.60 (m, 1H).
Chirale analytische SFC [Säule: Daicel Chiralcel OJ-H, 3 µm, 50 mm x 4.6 mm; Laufmittel: Kohlendioxid/Methanol 90:10; Fluss: 3 ml/min; Temperatur: 40°C; Detektion: 210 nm]: $R_t$ = 1.31 min.

**Beispiel 369**

[1-{[3-(2-Methoxyethyl)-5-methyl-2,4-dioxo-1-(tetrahydrofuran-2-ylmethyl)-1,2,3,4-tetrahydrothieno[2,3-d]pyrimidin-6-yl]methyl}imidazolidin-2-yliden]cyanamid *(Enantiomer 2)*

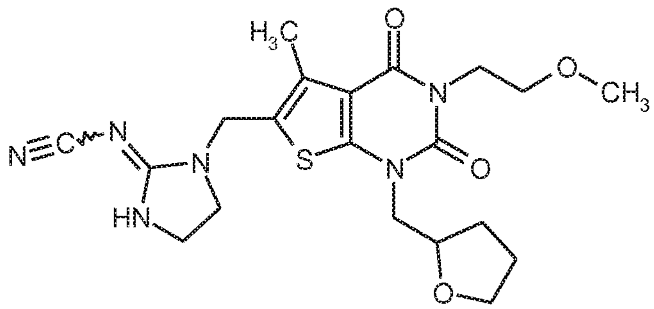

314 mg (0.703 mmol) der racemischen Verbindung aus Bsp. 367 wurden in 45 ml Methanol gelöst und in 45 Portionen über präparative SFC an chiraler Phase in die Enantiomere aufgetrennt [Säule: Daicel Chiralcel OJ-H, 5 µm, 250 mm x 30 mm; Laufmittel: Kohlendioxid/Methanol 90:10; Fluss: 100 ml/min; Temperatur: 40°C; Detektion: 210 nm]. Nach Eindampfen der Produktfraktionen, Verrühren des Feststoffs mit einem Gemisch aus 10 ml Pentan und 0.5 ml Dichlormethan, Absaugen und Trocknen im Hochvakuum wurden 116 mg (73% d. Th.) von Enantiomer 2 erhalten (96% ee, chirale analytische HPLC).
$^1$H-NMR (400 MHz, DMSO-$d_6$, δ/ppm): 8.07 (s, 1H), 4.47 (s, 2H), 4.27-4.17 (m, 1H), 4.11-4.00 (m, 3H), 3.82-3.56 (m, 3H), 3.50 (t, 2H), 3.46-3.36 (m, 4H), 3.24 (s, 3H), 2.40 (s, 3H), 2.04-1.75 (m, 3H), 1.73-1.61 (m, 1H).
Chirale analytische SFC [Säule: Daicel Chiralcel OJ-H, 3 µm, 50 mm x 4.6 mm; Laufmittel: Kohlendioxid/Methanol 90:10; Fluss: 3 ml/min; Temperatur: 40°C; Detektion: 210 nm]: $R_t$ = 1.48 min.

**Beispiel 370**

Methyl-[1-{[3-ethyl-5-methyl-2,4-dioxo-1-(3,3,3-trifluorpropyl)-1,2,3,4-tetrahydrothieno[2,3-d]-pyrimidin-6-yl]methyl}imidazolidin-2-yliden]carbamat

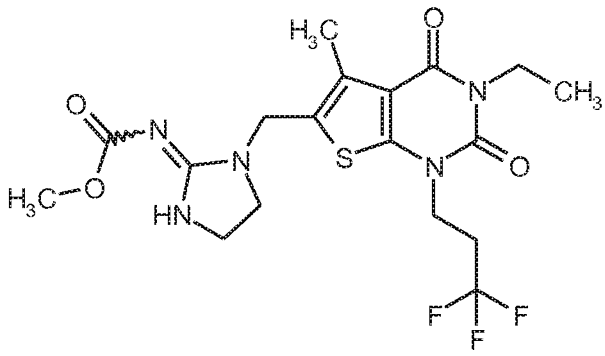

290 mg (0.697 mmol, 91% Reinheit) der Verbindung aus Bsp. 210A und 194 µl (1.40 mmol) Triethylamin wurden in 15 ml Dichlormethan gelöst und mit einer Lösung von 109 mg (0.697 mmol) Methyl-(dichlormethylen)carbamat in 15 ml Dichlormethan versetzt. Nachdem das Reaktionsgemisch 2.5 Tage bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde zunächst mittels MPLC vorgereinigt (Isolera, 50 g Kieselgel, Cyclohexan/Ethylacetat 80:20 → 0:100). Die produkthaltigen Fraktionen wurden vereinigt, eingeengt und dann mittels präparativer HPLC (Methode 11) nachgereinigt. Einengen der Produktfraktion und Trocknen des Rückstands im Hochvakuum ergaben 194 mg (59% d. Th.) der Titelverbindung.
$^1$H-NMR (400 MHz, DMSO-$d_6$, δ/ppm): 8.03 (s, 1H), 4.58 (s, 2H), 4.09 (t, 2H), 3.90 (q, 2H), 3.53 (s, 3H), 3.50-3.42 (m, 2H), 3.38-3.28 (m, 2H, teilweise überdeckt vom Wassersignal), 2.85-2.66 (m, 2H), 2.43 (s, 3H), 1.11 (t, 3H).
LC/MS (Methode 17, ESIpos): $R_t$ = 1.53 min, m/z = 462.14 $[M+H]^+$.

**Beispiel 371**

Methyl-[1-{[3-ethyl-5-methyl-1-(oxetan-2-ylmethyl)-2,4-dioxo-1,2,3,4-tetrahydrothieno[2,3-d]-pyrimidin-6-yl]methyl}imidazolidin-2-yliden]carbamat *(Racemat)*

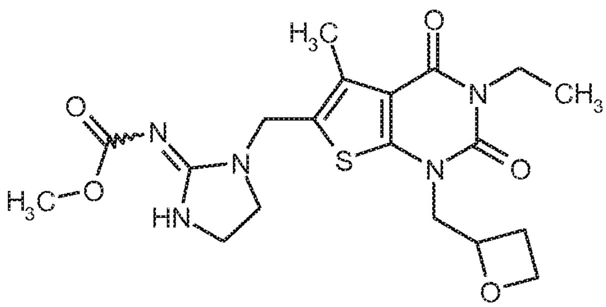

1.07 g (2.34 mmol, 77% Reinheit) der Verbindung aus Bsp. 224A und 651 µl (4.67 mmol) Triethylamin wurden in 15 ml Dichlormethan gelöst und mit einer Lösung von 365 mg (2.34 mmol) Methyl-(dichlormethylen)carbamat in 15 ml Dichlormethan versetzt. Nachdem das Reaktionsgemisch ca. 16 h bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde zunächst mittels MPLC vorgereinigt (Isolera, 100 g Kieselgel, Dichlormethan/Methanol 98:2 → 80:20). Die produkthaltigen Fraktionen wurden vereinigt, eingeengt und dann mit einem Acetonitril/Wasser-Gemisch verrührt.

Der Feststoff wurde abgesaugt und im Hochvakuum getrocknet und ergab so eine erste Teilmenge der Titelverbindung. Die eingeengte Mutterlauge des Verrührens wurde mittels präparativer HPLC (Methode 8) gereinigt. Einengen der Produktfraktion, Trocknen des Rückstands im Hochvakuum und Vereinigen mit der ersten Teilmenge ergaben insgesamt 205 mg (20% d. Th.) der Titelverbindung.

$^1$H-NMR (400 MHz, DMSO-$d_6$, δ/ppm): 8.02 (s, 1H), 5.05-4.94 (m, 1H), 4.55 (s, 2H), 4.51-4.34 (m, 2H), 4.13 (d, 2H), 3.90 (q, 2H), 3.53 (s, 3H), 3.49-3.40 (m, 2H), 3.37-3.27 (m, 2H, teilweise überlagert vom Wassersignal), 2.76-2.62 (m, 1H), 2.53-2.44 (m, 1H, teilweise überlagert vom DMSO-Signal), 2.42 (s, 3H), 1.11 (t, 3H).

LC/MS (Methode 17, ESIpos): $R_t$ = 1.09 min, m/z = 436.16 $[M+H]^+$.

**Beispiel 372**

Methyl-[1-{[3-ethyl-5-methyl-1-(oxetan-2-ylmethyl)-2,4-dioxo-1,2,3,4-tetrahydrothieno[2,3-d]-pyrimidin-6-yl]methyl}imidazolidin-2-yliden]carbamat *(Enantiomer 1)*

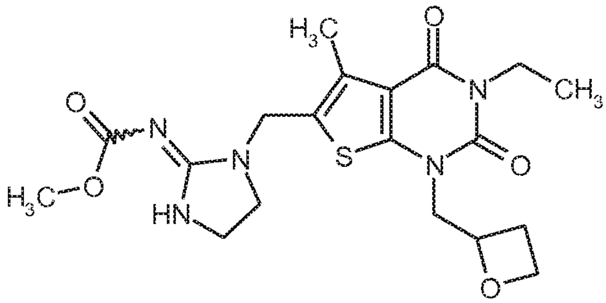

195 mg (0.448 mmol) der racemischen Verbindung aus Bsp. 371 wurden in 40 ml Methanol gelöst und in 50 Portionen über präparative SFC an chiraler Phase in die Enantiomere aufgetrennt [Säule: Daicel Chiralcel OD-H, 5 μm, 250 mm x 20 mm; Laufmittel: Kohlendioxid/Methanol 82:18; Fluss: 80 ml/min; Temperatur: 40°C; Detektion: 210 nm]. Nach Eindampfen der Produktfraktionen und Trocknen im Hochvakuum wurden 79 mg (81% d. Th.) von Enantiomer 1 erhalten (>99% ee, chirale analytische HPLC).

$^1$H-NMR (400 MHz, DMSO-$d_6$, δ/ppm): 8.02 (s, 1H), 5.06-4.92 (m, 1H), 4.55 (s, 2H), 4.51-4.35 (m, 2H), 4.13 (d, 2H), 3.90 (q, 2H), 3.53 (s, 3H), 3.49-3.41 (m, 2H), 3.37-3.28 (m, 2H, teilweise überlagert vom Wassersignal), 2.75-2.63 (m, 1H), 2.53-2.45 (m, 1H, teilweise überlagert vom DMSO-Signal), 2.42 (s, 3H), 1.11 (t, 3H).

Chirale analytische SFC [Säule: Daicel Chiralcel OD-H, 3 μm, 50 mm x 4.6 mm; Laufmittel: Kohlendioxid/Methanol 80:20; Fluss: 3 ml/min; Temperatur: 40°C; Detektion: 210 nm]: $R_t$ = 3.10 min.

**Beispiel 373**

Methyl-[1-{[3-ethyl-5-methyl-1-(oxetan-2-ylmethyl)-2,4-dioxo-1,2,3,4-tetrahydrothieno[2,3-d]-pyrimidin-6-yl]methyl}imidazolidin-2-yliden]carbamat (*Enantiomer 2*)

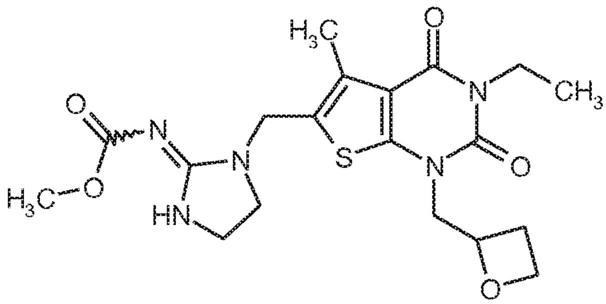

195 mg (0.448 mmol) der racemischen Verbindung aus Bsp. 371 wurden in 40 ml Methanol gelöst und in 50 Portionen über präparative SFC an chiraler Phase in die Enantiomere aufgetrennt [Säule: Daicel Chiralcel OD-H, 5 μm,

250 mm x 20 mm; Laufmittel: Kohlendioxid/Methanol 82:18; Fluss: 80 ml/min; Temperatur: 40°C; Detektion: 210 nm]. Nach Eindampfen der Produktftaktionen und Trocknen im Hochvakuum wurden 82 mg (84% d. Th.) von Enantiomer 2 erhalten (>99% ee, chirale analytische HPLC).
$^1$H-NMR (400 MHz, DMSO-$d_6$, $\delta$/ppm): 8.02 (s, 1H), 5.05-4.94 (m, 1H), 4.55 (s, 2H), 4.51-4.36 (m, 2H), 4.13 (d, 2H), 3.90 (q, 2H), 3.53 (s, 3H), 3.49-3.41 (m, 2H), 3.37-3.28 (m, 2H, teilweise überlagert vom Wassersignal), 2.77-2.63 (m, 1H), 2.53-2.45 (m, 1H, teilweise überlagert vom DMSO-Signal), 2.42 (s, 3H), 1.11 (t, 3H).

Chirale analytische SFC [Säule: Daicel Chiralcel OD-H, 3 µm, 50 mm x 4.6 mm; Laufmittel: Kohlendioxid/Methanol 80:20; Fluss: 3 ml/min; Temperatur: 40°C; Detektion: 210 nm]: $R_t$ = 3.68 min.

## Beispiel 374

Methyl-[1-{[3-ethyl-5-methyl-2,4-dioxo-1-(tetrahydrofuran-2-ylmethyl)-1,2,3,4-tetrahydrothieno-[2,3-d]pyrimidin-6-yl]methyl}imidazolidin-2-yliden]carbamat (*Racemat*)

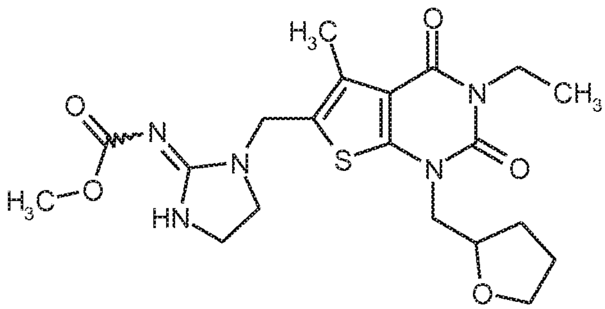

1.14 g (2.18 mmol, 70% Reinheit) der Verbindung aus Bsp. 225A und 607 µl (4.36 mmol) Triethylamin wurden in 50 ml Dichlormethan gelöst und mit einer Lösung von 340 mg (2.18 mmol) Methyl-(dichlormethylen)carbamat in 50 ml Dichlormethan versetzt. Nachdem das Reaktionsgemisch ca. 16 h bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde zunächst mittels MPLC vorgereinigt (Isolera, 100 g Kieselgel, Dichlormethan/Methanol 98:2 → 90:10). Die produkthaltigen Fraktionen wurden vereinigt, eingeengt und dann mittels präparativer HPLC (Methode 8) nachgereinigt. Einengen der Produktfraktion und Trocknen des Rückstands im Hochvakuum ergaben 247 mg (25% d. Th.) der Titelverbindung.
$^1$H-NMR (400 MHz, DMSO-$d_6$, $\delta$/ppm): 8.02 (s, 1H), 4.55 (s, 2H), 4.27-4.16 (m, 1H), 4.03 (dd, 1H), 3.90 (q, 2H), 3.77-3.55 (m, 3H), 3.53 (s, 3H), 3.49-3.41 (m, 2H), 3.36-3.29 (m, 2H, teilweise überlagert vom Wassersignal), 2.42 (s, 3H), 2.06-1.74 (m, 3H), 1.70-1.62 (m, 1H), 1.11 (t, 3H).
LC/MS (Methode 1, ESIpos): $R_t$ = 0.67 min, m/z = 450 [M+H]$^+$.

## Beispiel 375

Methyl-[1-{[3-ethyl-5-methyl-2,4-dioxo-1-(tetrahydrofuran-2-ylmethyl)-1,2,3,4-tetrahydrothieno-[2,3-d]pyrimidin-6-yl]methyl}imidazolidin-2-yliden]carbamat (*Enantiomer 1*)

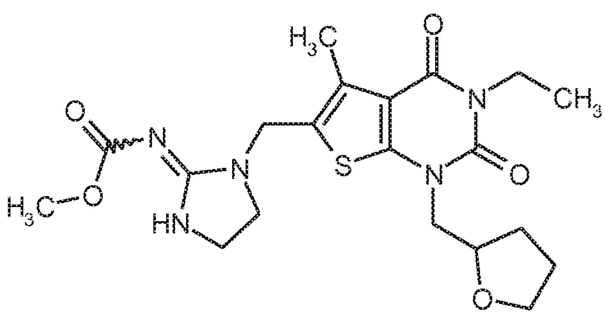

325 mg (0.523 mmol) der racemischen Verbindung aus Bsp. 374 wurden in 30 ml Methanol gelöst und in 15 Portionen über präparative SFC an chiraler Phase in die Enantiomere aufgetrennt [Säule: Daicel Chiralcel OX-H, 5 µm, 250 mm x 30 mm; Laufmittel: Kohlendioxid/Methanol 60:40; Fluss: 100 ml/min; Temperatur: 40°C; Detektion: 210 nm]. Nach Eindampfen der Produktfraktionen und Trocknen im Hochvakuum wurden 90 mg (55% d. Th.) von Enantiomer 1 erhalten (>99% ee, chirale analytische HPLC).
$^1$H-NMR (400 MHz, DMSO-$d_6$, δ/ppm): 8.02 (s, 1H), 4.55 (s, 2H), 4.27-4.17 (m, 1H), 4.03 (dd, 1H), 3.90 (q, 2H), 3.78-3.56 (m, 3H), 3.53 (s, 3H), 3.49-3.41 (m, 2H), 3.36-3.29 (m, 2H, teilweise überlagert vom Wassersignal), 2.42 (s, 3H), 2.03-1.74 (m, 3H), 1.72-1.60 (m, 1H), 1.11 (t, 3H).

Chirale analytische SFC [Säule: Daicel Chiralcel OX-H, 3 µm, 50 mm x 4.6 mm; Laufmittel: Kohlendioxid/Methanol 60:40; Fluss: 3 ml/min; Temperatur: 40°C; Detektion: 210 nm]: $R_t$ = 5.49 min.

## Beispiel 376

Methyl-[1-{[3-ethyl-5-methyl-2,4-dioxo-1-(tetrahydrofuran-2-ylmethyl)-1,2,3,4-tetrahydrothieno-[2,3-d]pyrimidin-6-yl]methyl}imidazolidin-2-yliden]carbamat (*Enantiomer 2*)

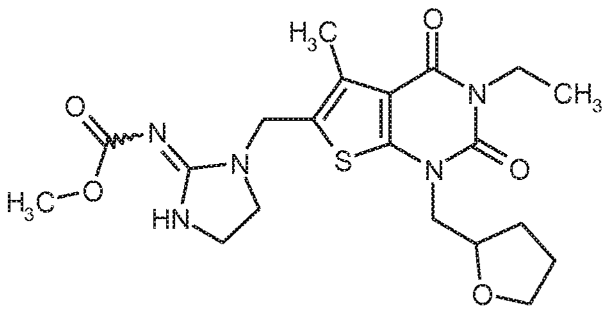

325 mg (0.523 mmol) der racemischen Verbindung aus Bsp. 374 wurden in 30 ml Methanol gelöst und in 15 Portionen über präparative SFC an chiraler Phase in die Enantiomere aufgetrennt [Säule: Daicel Chiralcel OX-H, 5 µm, 250 mm x 30 mm; Laufmittel: Kohlendioxid/Methanol 60:40; Fluss: 100 ml/min; Temperatur: 40°C; Detektion: 210 nm]. Nach Eindampfen der Produktfraktionen und Trocknen im Hochvakuum wurden 88 mg (54% d. Th.) von Enantiomer 2 erhalten (>99% ee, chirale analytische HPLC).
$^1$H-NMR (400 MHz, DMSO-$d_6$, δ/ppm): 8.02 (s, 1H), 4.55 (s, 2H), 4.28-4.15 (m, 1H), 4.03 (dd, 1H), 3.90 (q, 2H), 3.77-3.55 (m, 3H), 3.53 (s, 3H), 3.49-3.41 (m, 2H), 3.36-3.29 (m, 2H, teilweise überlagert vom Wassersignal), 2.42 (s, 3H), 2.06-1.75 (m, 3H), 1.72-1.60 (m, 1H), 1.11 (t, 3H).

Chirale analytische SFC [Säule: Daicel Chiralcel OX-H, 3 µm, 50 mm x 4.6 mm; Laufmittel: Kohlendioxid/Methanol 60:40; Fluss: 3 ml/min; Temperatur: 40°C; Detektion: 210 nm]: $R_t$ = 8.67 min.

## Beispiel 377

Methyl-[1-{[3-isopropyl-5-methyl-2,4-dioxo-1-(3,3,3-trifluorpropyl)-1,2,3,4-tetrahydrothieno-[2,3-d]pyrimidin-6-yl]methyl}imidazolidin-2-yliden]carbamat

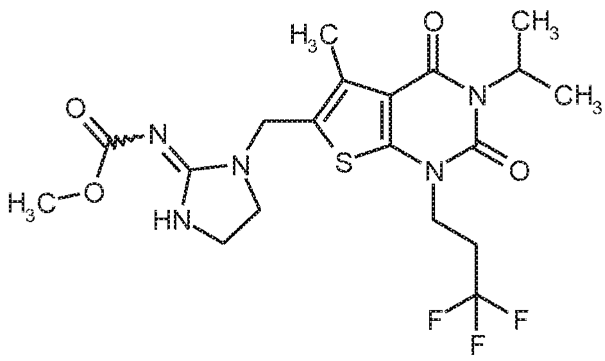

289 mg (0.368 mmol, 50% Reinheit) der Verbindung aus Bsp. 309A und 103 μl (0.736 mmol) Triethylamin wurden in 10 ml Dichlormethan gelöst und mit einer Lösung von 115 mg (0.736 mmol) Methyl-(dichlormethylen)carbamat in 10 ml Dichlormethan versetzt. Nachdem das Reaktionsgemisch 2.5 Tage bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde zunächst mittels präparativer HPLC (Methode 8) vorgereinigt. Die produkthaltigen Fraktionen wurden vereinigt, eingeengt und dann durch nochmalige präparative HPLC nachgereinigt [Säule: Kinetex C18, 5 μm, 100 mm x 30 mm; Eluent A: Wasser + 0.07% Ameisensäure, Eluent B: Acetonitril; Gradient: 0.0-2.0 min 10% B, 2.2 min 20% B, 7.0 min 60% B, 7.5-9.0 min 92% B; Fluss: 70 ml/min; Temperatur: 25°C; Detektion: 210 nm]. Nach Einengen der Produktftaktionen wurde das erhaltene Material mit Pentan/Dichlormethan (20:1) verrührt. Der Feststoff wurde abgesaugt und im Hochvakuum getrocknet. Dies ergab 63 mg (35% d. Th.) der Titelverbindung.
$^1$H-NMR (400 MHz, DMSO-$d_6$, δ/ppm): 8.02 (s, 1H), 5.13 (sept, 1H), 4.57 (s, 2H), 4.06 (t, 2H), 3.53 (s, 3H), 3.50-3.44 (m, 2H), 3.36-3.29 (m, 2H, teilweise überdeckt vom Wassersignal), 2.83-2.64 (m, 2H), 2.42 (s, 3H), 1.39 (d, 6H).
LC/MS (Methode 1, ESIpos): $R_t$ = 0.93 min, m/z = 476 $[M+H]^+$.

## Beispiel 378

Methyl-[1-{[3-isopropyl-1-(2-methoxyethyl)-5-methyl-2,4-dioxo-1,2,3,4-tetrahydrothieno[2,3-d]-pyrimidin-6-yl]methyl}imidazolidin-2-yliden]carbamat

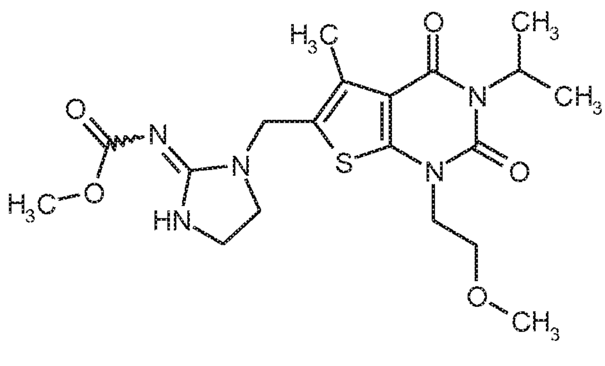

236 mg (0.573 mmol, 86% Reinheit) der Verbindung aus Bsp. 310A und 160 μl (1.145 mmol) Triethylamin wurden in 15 ml Dichlormethan gelöst und mit einer Lösung von 179 mg (1.15 mmol) Methyl-(dichlormethylen)carbamat in 15 ml Dichlormethan versetzt. Nachdem das Reaktionsgemisch 2.5 Tage bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) gereinigt. Einengen der Produktfraktionen und Trocknen im Hochvakuum ergaben 127 mg (50% d. Th.) der Titelverbindung.
$^1$H-NMR (400 MHz, DMSO-$d_6$, δ/ppm): 8.08 (br. s, 1H), 5.13 (sept, 1H), 4.56 (s, 2H), 3.97 (t, 2H), 3.61 (t, 2H), 3.55 (s, 3H), 3.50-3.42 (m, 2H), 3.37-3.29 (m, 2H, teilweise überdeckt vom Wassersignal), 3.23 (s, 3H), 2.40 (s, 3H), 1.40 (d, 6H).
LC/MS (Methode 17, ESIpos): $R_t$ = 1.40 min, m/z = 438.18 $[M+H]^+$.

**Beispiel 379**

Methyl-[1-{[3-isopropyl-5-methyl-1-(oxetan-2-ylmethyl)-2,4-dioxo-1,2,3,4-tetrahydrothieno-[2,3-d]pyrimidin-6-yl]methyl}imidazolidin-2-yliden]carbamat (*Racemat*)

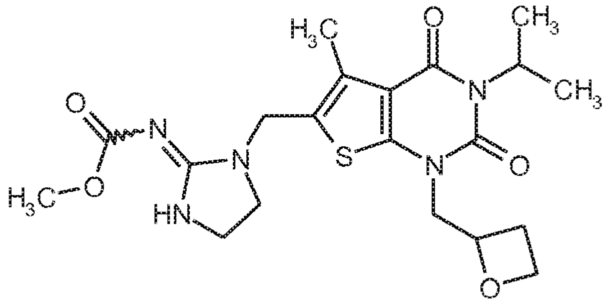

350 mg (0.802 mmol, 84% Reinheit) der Verbindung aus Bsp. 311A und 224 µl (1.60 mmol) Triethylamin wurden in 20 ml Dichlormethan gelöst und mit einer Lösung von 250 mg (1.60 mmol) Methyl-(dichlormethylen)carbamat in 20 ml Dichlormethan versetzt. Nachdem das Reaktionsgemisch 2.5 Tage bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde zunächst mittels präparativer HPLC (Methode 8) vorgereinigt. Die produkthaltigen Fraktionen wurden vereinigt, eingeengt und der Rückstand mit Pentan/Dichlormethan (20:1) verrührt. Der so gewonnene Feststoff wurde durch nochmalige präparative HPLC nachgereinigt [Säule: Kinetex C18, 5 µm, 100 mm x 30 mm; Eluent A: Wasser + 0.07% Ameisensäure, Eluent B: Acetonitril; Gradient: 0.0-2.0 min 10% B, 2.2 min 20% B, 7.0 min 60% B, 7.5-9.0 min 92% B; Fluss: 70 ml/min; Temperatur: 25°C; Detektion: 210 nm]. Nach Einengen der Produktfraktionen und Trocknen im Hochvakuum wurden 111 mg (30% d. Th.) der Titelverbindung erhalten.
$^{1}$H-NMR (400 MHz, DMSO-$d_6$, δ/ppm): 8.02 (s, 1H), 5.13 (sept, 1H), 5.03-4.93 (m, 1H), 4.55 (s, 2H), 4.51-4.35 (m, 2H), 4.15-4.01 (m, 2H), 3.53 (s, 3H), 3.50-3.40 (m, 2H), 3.35-3.29 (m, 2H, teilweise überdeckt vom Wassersignal), 2.76-2.62 (m, 1H), 2.52-2.42 (m, 1H, teilweise überdeckt vom DMSO-Signal), 2.40 (s, 3H), 1.39 (d, 6H).
LC/MS (Methode 17, ESIpos): $R_t$ = 1.31 min, m/z = 450.18 $[M+H]^+$.

**Beispiel 380**

Methyl-[1-{[3-isopropyl-5-methyl-2,4-dioxo-1-(tetrahydrofuran-2-ylmethyl)-1,2,3,4-tetrahydrothieno[2,3-d]pyrimidin-6-yl]methyl}imidazolidin-2-yliden]carbamat *(Racemat)*

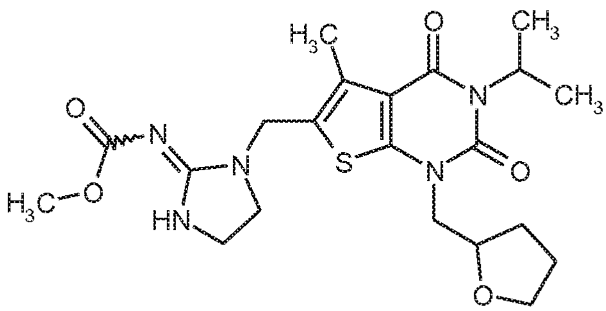

429 mg (0.970 mmol, 86% Reinheit) der Verbindung aus Bsp. 312A und 270 µl (1.94 mmol) Triethylamin wurden in 25 ml Dichlormethan gelöst und mit einer Lösung von 302 mg (1.94 mmol) Methyl-(dichlormethylen)carbamat in 25 ml Dichlormethan versetzt. Nachdem das Reaktionsgemisch ca. 16 h bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) gereinigt. Nach Einengen der Produktfraktionen

und Trocknen im Hochvakuum wurden 200 mg (42% d. Th.) der Titelverbindung erhalten.
$^1$H-NMR (400 MHz, DMSO-$d_6$, δ/ppm): 9.86 (breit, 1H), 5.14 (sept, 1H), 4.76 (br. s, 2H), 4.27-4.14 (m, 1H), 4.05 (dd, 1H), 2.41 (s, 3H), 2.06-1.76 (m, 3H), 1.71-1.59 (m, 1H), 1.40 (dd, 6H) [weitere Signale durch breites Wassersignal verdeckt].
LC/MS (Methode 6, ESIpos): $R_t$ = 1.63 min, m/z = 464 $[M+H]^+$.

**Beispiel 381**

Methyl-[1-{[5-methyl-2,4-dioxo-3-(2,2,2-trifluorethyl)-1-(3,3,3-trifluorpropyl)-1,2,3,4-tetrahydrothieno[2,3-d]pyrimidin-6-yl]methyl}imidazolidin-2-yliden]carbamat

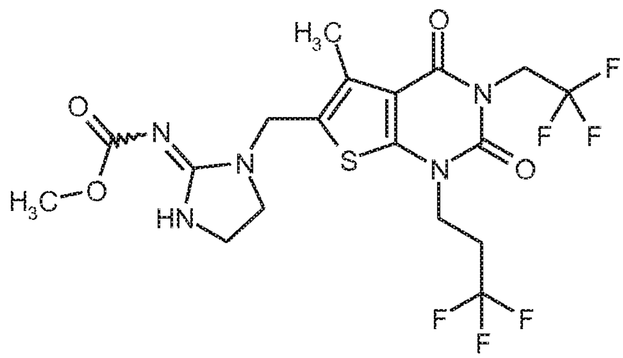

250 mg (0.405 mmol, 70% Reinheit) der Verbindung aus Bsp. 247A und 113 µl (0.809 mmol) Triethylamin wurden in 10 ml Dichlormethan gelöst und mit einer Lösung von 126 mg (0.809 mmol) Methyl-(dichlormethylen)carbamat in 10 ml Dichlormethan versetzt. Nachdem das Reaktionsgemisch ca. 16 h bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) gereinigt. Die Produktfraktionen wurden eingeengt und der Rückstand mit Pentan/Dichlormethan (20:1) verrührt. Nach Absaugen des Feststoffs und Trocknen im Hochvakuum wurden 89 mg (41% d. Th.) der Titelverbindung erhalten.
$^1$H-NMR (400 MHz, DMSO-$d_6$, δ/ppm): 8.12 (br. s, 1H), 4.70 (q, 2H), 4.62 (s, 2H), 4.13 (t, 2H), 3.56 (s, 3H), 3.52-3.44 (m, 2H), 3.41-3.35 (m, 2H, teilweise überdeckt vom Wassersignal), 2.90-2.65 (m, 2H), 2.43 (s, 3H).
LC/MS (Methode 17, ESIpos): $R_t$ = 1.66 min, m/z = 516.11 $[M+H]^+$.

**Beispiel 382**

Methyl-[1-{[1-(2-methoxyethyl)-5-methyl-2,4-dioxo-3-(2,2,2-trifluorethyl)-1,2,3,4-tetrahydrothieno[2,3-d]pyrimidin-6-yl]methyl}imidazolidin-2-yliden]carbamat

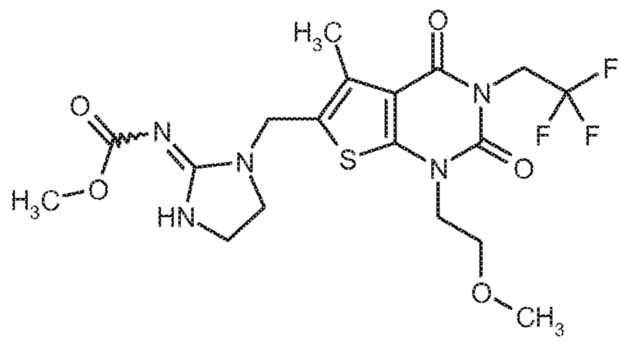

343 mg (0.696 mmol, 80% Reinheit) der Verbindung aus Bsp. 249A und 194 µl (1.39 mmol) Triethylamin

wurden in 15 ml Dichlormethan gelöst und mit einer Lösung von 217 mg (1.39 mmol) Methyl-(dichlormethylen)carbamat in 15 ml Dichlormethan versetzt. Nachdem das Reaktionsgemisch 2.5 Tage bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde durch zweimalige präparative HPLC (zunächst nach Methode 8, dann nach Methode 11) gereinigt. Die Produktfraktionen wurden eingeengt und der Rückstand im Hochvakuum getrocknet. Es wurden 91 mg (26% d. Th.) der Titelverbindung erhalten.
$^1$H-NMR (400 MHz, DMSO-$d_6$, $\delta$/ppm): 8.03 (s, 1H), 4.69 (q, 2H), 4.57 (s, 2H), 4.04 (t, 2H), 3.63 (t, 2H), 3.53 (s, 3H), 3.50-3.42 (m, 2H), 3.38-3-32 (m, 2H, teilweise überdeckt vom Wassersignal), 3.23 (s, 3H), 2.42 (s, 3H).
LC/MS (Methode 17, ESIpos): $R_t$ = 1.41 min, m/z = 478.14 [M+H]$^+$.

**Beispiel 383**

Methyl-[1-{[5-methyl-1-(oxetan-2-ylmethyl)-2,4-dioxo-3-(2,2,2-trifluorethyl)-1,2,3,4-tetrahydrothieno[2,3-d]pyrimidin-6-yl]methyl}imidazolidin-2-yliden]carbamat (*Racemat*)

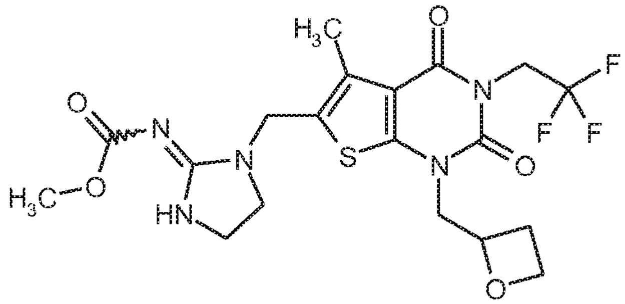

400 mg (0.817 mmol, 83% Reinheit) der Verbindung aus Bsp. 251A und 228 µl (1.63 mmol) Triethylamin wurden in 10 ml Dichlormethan gelöst und mit einer Lösung von 255 mg (1.63 mmol) Methyl-(dichlormethylen)carbamat in 10 ml Dichlormethan versetzt. Nachdem das Reaktionsgemisch ca. 16 h bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde zunächst mittels präparativer HPLC (Methode 8) vorgereinigt. Die produkthaltigen Fraktionen wurden vereinigt und eingeengt und anschließend durch erneute präparative HPLC nachgereinigt [Säule: Chromatorex C18, 10 µm, 125 mm x 30 mm; Eluent A: Wasser, Eluent B: Acetonitril, Eluent C: 1% Ameisensäure in Wasser; A/B/C-Gradientenprogramm; Fluss: 100 ml/min; Temperatur: 25°C; Detektion: 210 nm]. Nach Einengen der Produktfraktionen und Trocknen im Hochvakuum wurden 52 mg (11% d. Th., 90% Reinheit) der Titelverbindung erhalten.
$^1$H-NMR (400 MHz, DMSO-$d_6$, $\delta$/ppm): 8.03 (s, 1H), 5.05-4.94 (m, 1H), 4.70 (q, 2H), 4.57 (s, 2H), 4.51-4.35 (m, 2H), 4.19-4.12 (m, 2H), 3.53 (s, 3H), 3.50-3.42 (m, 2H), 3.38-3.30 (m, 2H, teilweise überdeckt vom Wassersignal), 2.76-2.63 (m, 1H), 2.53-2.45 (m, 1H, teilweise überdeckt vom DMSO-Signal), 2.42 (s, 3H).
LC/MS (Methode 17, ESIpos): $R_t$ = 1.33 min, m/z = 490.14 [M+H]$^+$.

**Beispiel 384**

Methyl-[1-{[5-methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl}imidazolidin-2-yliden]carbamat (*Racemat*)

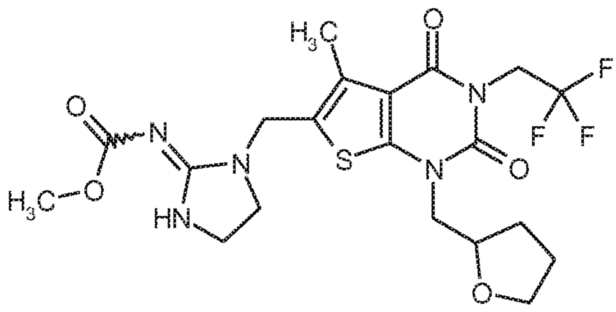

270 mg (0.578 mmol, 90% Reinheit) der Verbindung aus Bsp. 252A und 161 µl (1.16 mmol) Triethylamin wurden in 15 ml Dichlormethan gelöst und mit einer Lösung von 180 mg (1.16 mmol) Methyl-(dichlormethylen)carbamat in 15 ml Dichlormethan versetzt. Nachdem das Reaktionsgemisch ca. 16 h bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) gereinigt. Nach Einengen der Produktfraktionen und Trocknen im Hochvakuum wurden 186 mg (63% d. Th.) der Titelverbindung erhalten.

$^1$H-NMR (400 MHz, DMSO-$d_6$, δ/ppm): 8.19 (br. s, 1H), 4.70 (q, 2H), 4.61 (s, 2H), 4.27-4.17 (m, 1H), 4.06 (dd, 1H), 3.78-3.66 (m, 2H), 3.64-3.54 (m, 1H), 3.58 (s, 3H), 3.53-3.44 (m, 2H), 3.42-3.34 (m, 2H, teilweise überdeckt vom Wassersignal), 2.42 (s, 3H), 2.05-1.76 (m, 3H), 1.73-1.61 (m, 1H).

LC/MS (Methode 6, ESIpos): $R_t$ = 1.63 min, m/z = 504 $[M+H]^+$.

**Beispiel 385**

Methyl-[1-{[3-(2-methoxyethyl)-5-methyl-2,4-dioxo-1-(3,3,3-trifluorpropyl)-1,2,3,4-tetrahydrothieno[2,3-d]pyrimidin-6-yl]methyl}imidazolidin-2-yliden]carbamat

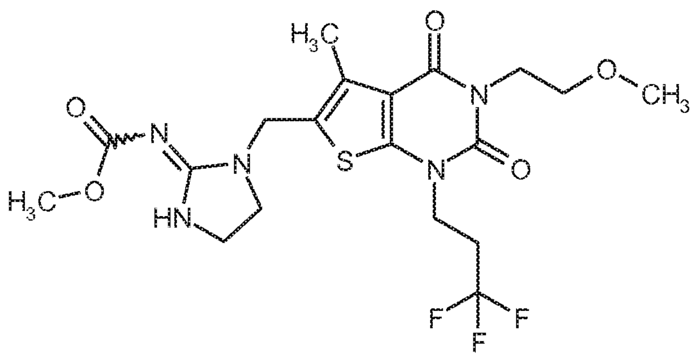

368 mg (0.820 mmol, 91% Reinheit) der Verbindung aus Bsp. 319A und 229 µl (1.64 mmol) Triethylamin wurden in 15 ml Dichlormethan gelöst und mit einer Lösung von 256 mg (1.64 mmol) Methyl-(dichlormethylen)carbamat in 15 ml Dichlormethan versetzt. Nachdem das Reaktionsgemisch 2.5 Tage bei RT gerührt worden war, wurde es mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Der verbliebene Rückstand wurde durch zweimalige präparative HPLC (jeweils nach Methode 8) gereinigt. Einengen der Produktfraktionen und Trocknen des Rückstands im Hochvakuum ergaben 89 mg (22% d. Th.) der Titelverbindung.

$^1$H-NMR (400 MHz, DMSO-$d_6$, δ/ppm): 8.08 (br. s, 1H), 4.60 (s, 2H), 4.08 (q, 2H), 4.05 (t, 2H), 3.56 (s, 3H), 3.51-3.46 (m, 2H), 3.48 (t, 2H), 3.40-3.32 (m, 2H, teilweise überdeckt vom Wassersignal), 3.24 (s, 3H), 2.84-2.67 (m, 2H), 2.43 (s, 3H).

LC/MS (Methode 17, ESIpos): $R_t$ = 1.40 min, m/z = 492.15 $[M+H]^+$.

**Beispiel 386**

Methyl-[1-{[1,3-bis(2-methoxyethyl)-5-methyl-2,4-dioxo-1,2,3,4-tetrahydrothieno[2,3-d]pyrimidin-6-yl]methyl}imidazolidin-2-yliden]carbamat

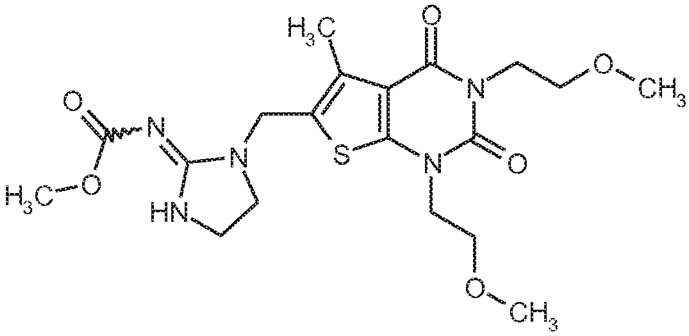

Analog zu dem unter Bsp. 379 beschriebenen Verfahren wurden aus 323 mg (0.872 mmol) der Verbindung aus Bsp. 262A und 204 mg (1.31 mmol) Methyl-(dichlormethylen)carbamat 54 mg (13% d. Th.) der Titelverbindung erhalten. Die Reaktionszeit betrug in diesem Fall ca. 16 h.
$^1$H-NMR (400 MHz, DMSO-$d_6$, $\delta$/ppm): 8.02 (s, 1H), 4.56 (s, 2H), 4.05 (t, 2H), 4.00 (t, 2H), 3.62 (t, 2H), 3.53 (s, 3H), 3.50 (t, 2H), 3.48-3.42 (m, 2H), 3.37-3.32 (m, 2H, teilweise überdeckt vom Wassersignal), 3.24 (s, 3H), 3.23 (s, 3H), 2.41 (s, 3H).
LC/MS (Methode 1, ESIpos): $R_t$ = 0.59 min, m/z = 454 $[M+H]^+$.

**Beispiel 387**

Methyl-[1-{[3-(2-methoxyethyl)-5-methyl-1-(oxetan-2-ylmethyl)-2,4-dioxo-1,2,3,4-tetrahydrothieno[2,3-d]pyrimidin-6-yl]methyl}imidazolidin-2-yliden]carbamat (*nicht-racemisches Enantiomerengemisch*)

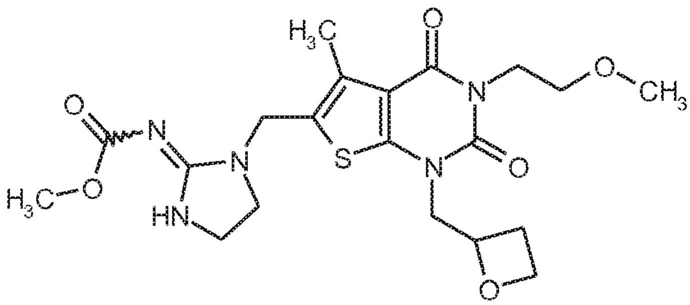

Analog zu dem unter Bsp. 385 beschriebenen Verfahren wurden aus 350 mg (0.824 mmol, 90% Reinheit) der Verbindung aus Bsp. 405A und 257 mg (1.65 mmol) Methyl-(dichlormethylen)-carbamat 143 mg (37% d. Th.) der Titelverbindung erhalten. Die Reaktionszeit betrug in diesem Fall ca. 16 h.
$^1$H-NMR (400 MHz, DMSO-$d_6$, $\delta$/ppm): 8.03 (s, 1H), 5.04-4.94 (m, 1H), 4.56 (s, 2H), 4.52-4.35 (m, 2H), 4.12 (d, 2H), 4.06 (t, 2H), 3.53 (s, 3H), 3.50 (t, 2H), 3.48-3.41 (m, 2H), 3.37-3.32 (m, 2H, teilweise überdeckt vom Wassersignal), 3.24 (s, 3H), 2.75-2.62 (m, 1H), 2.52-2.44 (m, 1H, teilweise überdeckt vom DMSO-Signal), 2.41 (s, 3H).
LC/MS (Methode 1, ESIpos): $R_t$ = 0.62 min, m/z = 466 $[M+H]^+$.

**Beispiel 388**

Methyl-[1-{[3-(2-methoxyethyl)-5-methyl-1-(oxetan-2-ylmethyl)-2,4-dioxo-1,2,3,4-tetrahydrothieno[2,3-d]pyrimidin-6-yl]methyl}imidazolidin-2-yliden]carbamat (*Enantiomer 1*)

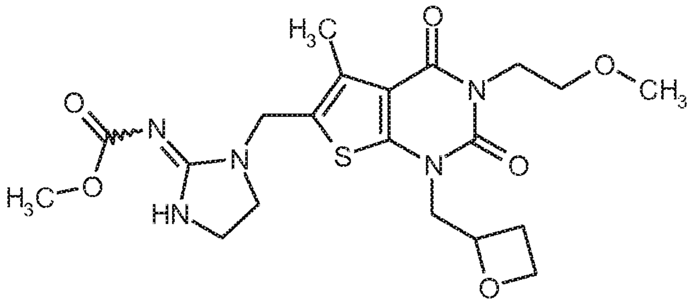

128 mg (0.275 mmol) des Enantiomerengemisches aus Bsp. 387 wurden in einem Gemisch aus 5 ml Methanol, 4 ml *tert*.-Butylmethylether und 3 ml Dichlormethan gelöst und in 15 Portionen über präparative HPLC an chiraler Phase in die Enantiomere aufgetrennt [Säule: Daicel Chiralpak IA, 5 μm, 250 mm x 30 mm; Laufmittel: *tert*.-Butylmethylether/Methanol 1:1; Fluss: 30 ml/min; Temperatur: 30°C; Detektion: 220 nm]. Nach Eindampfen der Produktfraktionen und Trocknen im Hochvakuum wurden 13 mg (10% d. Th.) von Enantiomer 1 erhalten (>99% ee, chirale analytische HPLC).
$^1$H-NMR (400 MHz, DMSO-$d_6$, δ/ppm): 8.02 (s, 1H), 5.04-4.93 (m, 1H), 4.56 (s, 2H), 4.51-4.35 (m, 2H), 4.12 (d, 2H), 4.06 (t, 2H), 3.53 (s, 3H), 3.50 (t, 2H), 3.48-3.41 (m, 2H), 3.37-3.32 (m, 2H, teilweise überdeckt vom Wassersignal), 3.24 (s, 3H), 2.74-2.62 (m, 1H), 2.53-2.44 (m, 1H, teilweise überdeckt vom DMSO-Signal), 2.41 (s, 3H).

Chirale analytische HPLC [Säule: Daicel Chiralcel IA, 5 μm, 250 mm x 4.6 mm; Laufmittel: *tert*.-Butylmethylether/Methanol 1:1; Fluss: 1 ml/min; Temperatur: 30°C; Detektion: 220 nm]: $R_t$ = 6.57 min.

**Beispiel 389**

Methyl-[1-{[3-(2-methoxyethyl)-5-methyl-1-(oxetan-2-ylmethyl)-2,4-dioxo-1,2,3,4-tetrahydrothieno[2,3-d]pyrimidin-6-yl]methyl}imidazolidin-2-yliden]carbamat (*Enantiomer 2*)

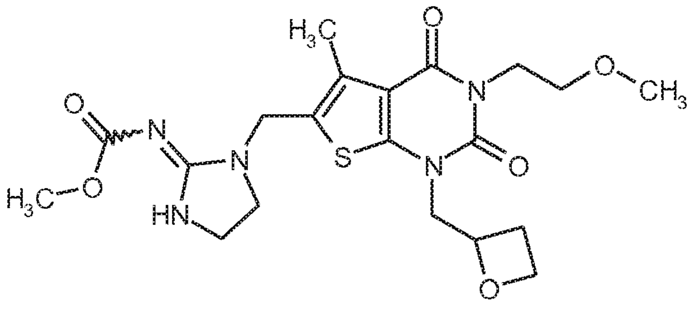

128 mg (0.275 mmol) des Enantiomerengemisches aus Bsp. 387 wurden in einem Gemisch aus 5 ml Methanol, 4 ml *tert*.-Butylmethylether und 3 ml Dichlormethan gelöst und in 15 Portionen über präparative HPLC an chiraler Phase in die Enantiomere aufgetrennt [Säule: Daicel Chiralpak IA, 5 μm, 250 mm x 30 mm; Laufmittel: *tert*.-Butylmethylether/Methanol 1:1; Fluss: 30 ml/min; Temperatur: 30°C; Detektion: 220 nm]. Nach Eindampfen der Produktfraktionen und Trocknen im Hochvakuum wurden 94 mg (73% d. Th.) von Enantiomer 2 erhalten (>99% ee, chirale analytische HPLC).
$^1$H-NMR (400 MHz, DMSO-$d_6$, δ/ppm): 8.02 (s, 1H), 5.04-4.93 (m, 1H), 4.56 (s, 2H), 4.51-4.34 (m, 2H), 4.12 (d, 2H), 4.06 (t, 2H), 3.53 (s, 3H), 3.50 (t, 2H), 3.48-3.43 (m, 2H), 3.37-3.28 (m, 2H, teilweise überdeckt vom Wassersignal), 3.23 (s, 3H), 2.75-2.63 (m, 1H), 2.52-2.44 (m, 1H, teilweise überdeckt vom DMSO-Signal), 2.41 (s, 3H).

Chirale analytische HPLC [Säule: Daicel Chiralcel IA, 5 μm, 250 mm x 4.6 mm; Laufmittel: *tert*.-Butylmethylether/Methanol 1:1; Fluss: 1 ml/min; Temperatur: 30°C; Detektion: 220 nm]: $R_t$ = 8.95 min.

**Beispiel 390**

Methyl-[1-{[3-(2-methoxyethyl)-5-methyl-2,4-dioxo-1-(tetrahydrofuran-2-ylmethyl)-1,2,3,4-tetrahydrothieno[2,3-d]pyrimidin-6-yl]methyl}imidazolidin-2-yliden]carbamat *(Racemat)*

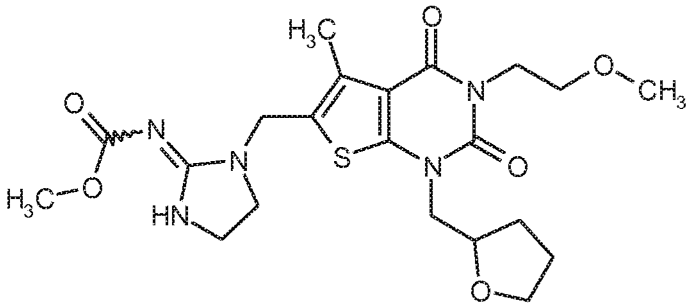

Analog zu dem unter Bsp. 371 beschriebenen Verfahren wurden aus 875 mg (1.99 mmol, 90% Reinheit) der Verbindung aus Bsp. 321A und 310 mg (1.99 mmol) Methyl-(dichlormethylen)-carbamat 415 mg (43% d. Th.) der Titelverbindung erhalten. Die Reaktionszeit betrug in diesem Fall 2.5 Tage.
$^1$H-NMR (400 MHz, DMSO-$d_6$, δ/ppm): 8.02 (s, 1H), 4.55 (s, 2H), 4.27-4.16 (m, 1H), 4.06 (t, 2H), 4.02 (dd, 1H), 3.78-3.55 (m, 3H), 3.53 (s, 3H), 3.50 (t, 2H), 3.48-3.42 (m, 2H), 3.37-3.29 (m, 2H, teilweise überdeckt vom Wassersignal), 3.24 (s, 3H), 2.41 (s, 3H), 2.03-1.75 (m, 3H), 1.72-1.60 (m, 1H).
LC/MS (Methode 17, ESIpos): $R_t$ = 1.21 min, m/z = 480.19 $[M+H]^+$.

**Beispiel 391**

Methyl-[1-{[3-(2-methoxyethyl)-5-methyl-2,4-dioxo-1-(tetrahydrofuran-2-ylmethyl)-1,2,3,4-tetrahydrothieno[2,3-d]pyrimidin-6-yl]methyl}imidazolidin-2-yliden]carbamat *(Enantiomer 1)*

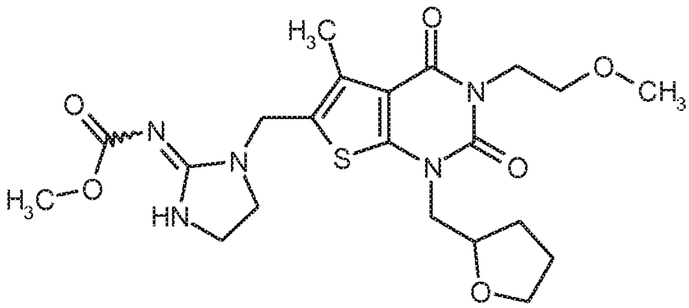

395 mg (0.824 mmol) der racemischen Verbindung aus Bsp. 390 wurden in einem Gemisch aus 10 ml Methanol und 4 ml Dichlormethan gelöst und in 140 Portionen über präparative SFC an chiraler Phase in die Enantiomere aufgetrennt [Säule: Daicel Chiralcel OD-H, 5 µm, 250 mm x 20 mm; Laufmittel: Kohlendioxid/Methanol 75:25; Fluss: 80 ml/min; Temperatur: 30°C; Detektion: 210 nm]. Nach Eindampfen der Produktfraktionen wurde der Rückstand mit Pentan, dem etwas Dichlormethan zugesetzt war, verrührt und der Feststoff anschließend abgesaugt und im Hochvakuum getrocknet. Es wurden 150 mg (75% d. Th.) von Enantiomer 1 erhalten (99% ee, chirale analytische HPLC).
$^1$H-NMR (400 MHz, DMSO-$d_6$, δ/ppm): 8.02 (s, 1H), 4.55 (s, 2H), 4.27-4.17 (m, 1H), 4.06 (t, 2H), 4.03 (dd, 1H), 3.76-3.56 (m, 3H), 3.53 (s, 3H), 3.50 (t, 2H), 3.48-3.43 (m, 2H), 3.37-3.28 (m, 2H, teilweise überdeckt vom Wassersignal), 3.24 (s, 3H), 2.41 (s, 3H), 2.03-1.76 (m, 3H), 1.72-1.60 (m, 1H).
Chirale analytische SFC [Säule: Daicel Chiralcel OD-H, 5 µm, 250 mm x 4.6 mm; Laufmittel: Kohlendioxid/Methanol 75:25; Fluss: 3 ml/min; Temperatur: 40°C; Detektion: 210 nm]: $R_t$ = 3.77 min.

Beispiel 392

Methyl-[1-{[3-(2-methoxyethyl)-5-methyl-2,4-dioxo-1-(tetrahydrofuran-2-ylmethyl)-1,2,3,4-tetrahydrothieno[2,3-d]pyrimidin-6-yl]methyl}imidazolidin-2-yliden]carbamat *(Enantiomer 2)*

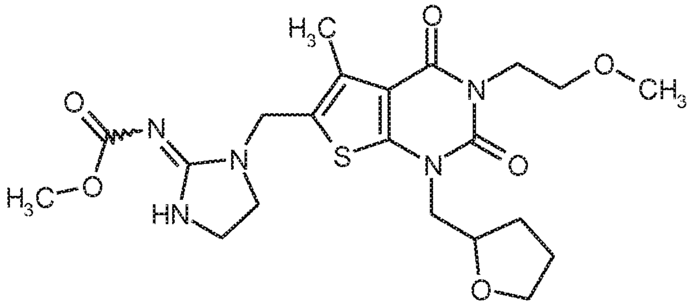

395 mg (0.824 mmol) der racemischen Verbindung aus Bsp. 390 wurden in einem Gemisch aus 10 ml Methanol und 4 ml Dichlormethan gelöst und in 140 Portionen über präparative SFC an chiraler Phase in die Enantiomere aufgetrennt [Säule: Daicel Chiralcel OD-H, 5 μm, 250 mm x 20 mm; Laufmittel: Kohlendioxid/Methanol 75:25; Fluss: 80 ml/min; Temperatur: 30°C; Detektion: 210 nm]. Nach Eindampfen der Produktfraktionen wurde der Rückstand mit Pentan, dem etwas Dichlormethan zugesetzt war, verrührt und der Feststoff anschließend abgesaugt und im Hochvakuum getrocknet. Es wurden 148 mg (74% d. Th.) von Enantiomer 2 erhalten (98% ee, chirale analytische HPLC).
$^1$H-NMR (400 MHz, DMSO-$d_6$, δ/ppm): 8.02 (s, 1H), 4.55 (s, 2H), 4.25-4.18 (m, 1H), 4.06 (t, 2H), 4.03 (dd, 1H), 3.76-3.56 (m, 3H), 3.53 (s, 3H), 3.50 (t, 2H), 3.48-3.43 (m, 2H), 3.37-3.28 (m, 2H, teilweise überdeckt vom Wassersignal), 3.24 (s, 3H), 2.41 (s, 3H), 2.02-1.75 (m, 3H), 1.72-1.60 (m, 1H).

Chirale analytische SFC [Säule: Daicel Chiralcel OD-H, 5 μm, 250 mm x 4.6 mm; Laufmittel: Kohlendioxid/Methanol 75:25; Fluss: 3 ml/min; Temperatur: 40°C; Detektion: 210 nm]: $R_t$ = 4.33 min.

**Beispiel 393**

6-[(2,3-Dioxopiperazin-1-yl)methyl]-3-ethyl-1-(fluormethyl)-5-methylthieno[2,3-d]pyrimidin-2,4(1*H*,3*H*)-dion

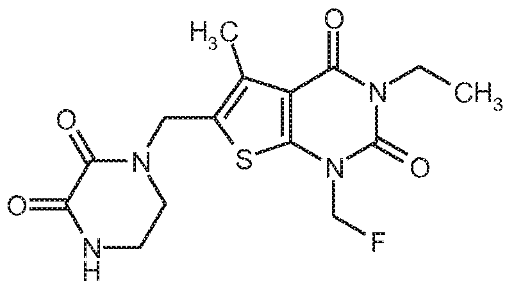

110 mg (0.276 mmol) der Verbindung aus Bsp. 397A wurden in 5 ml Ethanol gelöst und mit 408 mg (2.76 mmol) Oxalsäurediethylester versetzt. Das Gemisch wurde 17 h bei 80°C gerührt. Danach wurde die Reaktionslösung am Rotationsverdampfer eingeengt. Der Rückstand wurde in 30 ml Dichlormethan gelöst und diese Lösung mit Wasser und gesättigter Natriumhydrogencarbonat-Lösung gewaschen. Nach Trocknen über wasserfreiem Natriumsulfat wurde die organische Phase filtriert und eingeengt. Der verbliebene Rückstand wurde in 3 ml DMSO gelöst und diese Lösung mittels präparativer HPLC gereinigt (Methode 14). Nach Vereinigen der Produktfraktionen und Gefriertrocknung wurden 36 mg (33% d. Th.) der Titelverbindung erhalten.
$^1$H-NMR (400 MHz, DMSO-$d_6$, δ/ppm): 8.65 (br. s, 1H), 6.01 (d, 2H), 4.71 (s, 2H), 3.90 (q, 2H), 3.51 (dd, 2H), 2.44 (s, 3H), 1.13 (t, 3H).
LC/MS (Methode 3, ESIneg): $R_t$ = 0.79 min, m/z = 413 $[M-H+HCOOH]^-$.

**Beispiel 394**

6-[(2,3-Dioxopiperazin-1-yl)methyl]-3-ethyl-5-methyl-1-(3,3,3-trifluorpropyl)thieno[2,3-d]pyrimidin-2,4(1*H*,3*H*)-dion

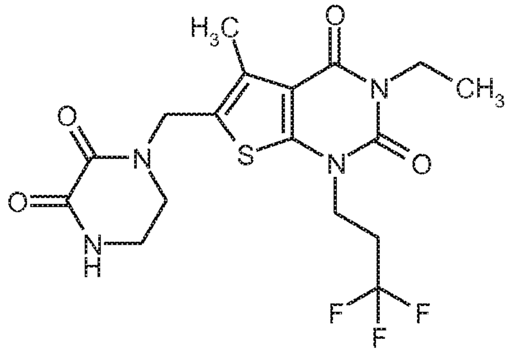

423 mg (1.12 mmol) der Verbindung aus Bsp. 210A wurden in 45 ml Ethanol gelöst und mit 285 µl (2.07 mmol) Oxalsäurediethylester versetzt. Das Gemisch wurde ca. 16 h bei 80°C gerührt. Danach wurde die Reaktionslösung am Rotationsverdampfer eingeengt. Der Rückstand wurde zunächst mittels MPLC vorgereinigt (Isolera, 25 g Kieselgel, Dichlormethan/Methanol 98:2 → 90:10). Das so gewonnene Produkt wurde anschließend mittels präparativer HPLC (Methode 8) nachgereinigt. Nach Vereinigen der Produktfraktionen, Einengen und Trocknen im Hochvakuum wurden 230 mg (47% d. Th.) der Titelverbindung erhalten.
$^1$H-NMR (400 MHz, DMSO-$d_6$, δ/ppm): 8.62 (br. s, 1H), 4.71 (s, 2H), 4.10 (t, 2H), 3.90 (q, 2H), 3.55-3.43 (m, 2H), 3.34-3.28 (m, 2H, teilweise überdeckt vom Wassersignal), 2.87-2.68 (m, 2H), 2.45 (s, 3H), 1.12 (t, 3H).
LC/MS (Methode 1, ESIpos): $R_t$ = 0.73 min, m/z = 433 $[M+H]^+$.

**Beispiel 395**

1-[2-(Cyclopentyloxy)ethyl]-6-[(2,3-dioxopiperazin-1-yl)methyl]-3-ethyl-5-methylthieno[2,3-d]-pyrimidin-2,4(1*H*,3*H*)-di-on

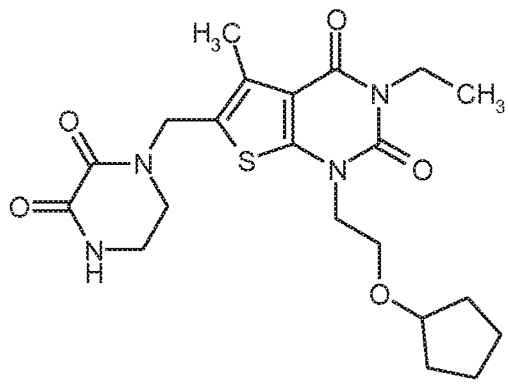

127 mg (0.264 mmol) der Verbindung aus Bsp. 398A wurden in 5 ml Ethanol gelöst und mit 389 mg (2.64 mmol) Oxalsäurediethylester versetzt. Das Gemisch wurde 19 h bei 80°C gerührt. Danach wurde die Reaktionslösung am Rotationsverdampfer eingeengt. Der Rückstand wurde in 30 ml Dichlormethan gelöst und diese Lösung mit Wasser und gesättigter Natriumhydrogencarbonat-Lösung gewaschen. Nach Trocknen über wasserfreiem Natriumsulfat wurde die organische Phase filtriert und eingeengt. Der verbliebene Rückstand wurde in 3 ml DMSO gelöst und diese Lösung mittels präparativer HPLC gereinigt (Methode 14). Nach Vereinigen der Produktfraktionen und Gefriertrocknung wurden 57 mg (47% d. Th.) der Titelverbindung erhalten.
$^1$H-NMR (400 MHz, DMSO-$d_6$, δ/ppm): 8.63 (br. s, 1H), 4.68 (s, 2H), 3.97 (t, 2H), 3.94-3.82 (m, 3H), 3.61 (t, 2H), 3.49-3.42

(m, 2H), 3.32-3.26 (m, 2H), 2.43 (s, 3H), 1.59-1.47 (m, 2H), 1.47-1.35 (m, 6H), 1.11 (t, 3H).
LC/MS (Methode 3, ESIneg): $R_t$ = 0.99 min, m/z = 493 [M-H+HCOOH]$^-$.

**Beispiel 396**

6-[(2,3-Dioxopiperazin-1-yl)methyl]-3-ethyl-1-(3-methoxypropyl)-5-methylthieno[2,3-d]pyrimidin-2,4(1*H*,3*H*)-dion

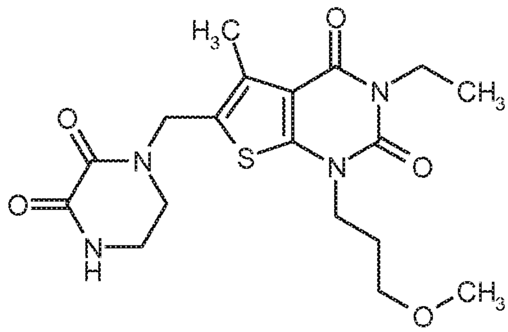

90 mg (0.211 mmol) der Verbindung aus Bsp. 401A wurden in 4 ml Ethanol gelöst und mit 311 mg (2.01 mmol) Oxalsäurediethylester versetzt. Das Gemisch wurde 16 h bei 80°C gerührt. Danach wurde die Reaktionslösung am Rotationsverdampfer eingeengt. Der Rückstand wurde in 30 ml Dichlormethan gelöst und diese Lösung mit Wasser und gesättigter Natriumhydrogencarbonat-Lösung gewaschen. Nach Trocknen über wasserfreiem Natriumsulfat wurde die organische Phase filtriert und eingeengt. Der verbliebene Rückstand wurde in 3 ml DMSO gelöst und diese Lösung mittels präparativer HPLC gereinigt (Methode 14). Nach Vereinigen der Produktfraktionen und Gefriertrocknung wurden 46 mg (54% d. Th.) der Titelverbindung erhalten.
$^1$H-NMR (400 MHz, DMSO-$d_6$, δ/ppm): 8.64 (br. s, 1H), 4.69 (s, 2H), 3.95-3.84 (m, 4H), 3.52-3.44 (m, 2H), 3.19 (s, 3H), 2.43 (s, 3H), 1.89 (quin, 2H), 1.11 (t, 3H) [weitere Signale teilweise vom Wassersignal überlagert].
LC/MS (Methode 3, ESIpos): $R_t$ = 0.99 min, m/z = 409 [M+H]$^+$.

**Beispiel 397**

6-[(2,3-Dioxopiperazin-1-yl)methyl]-3-isopropyl-5-methyl-1-(oxetan-2-ylmethyl)thieno[2,3-d]-pyrimidin-2,4(1*H*,3*H*)-dion (*Racemat*)

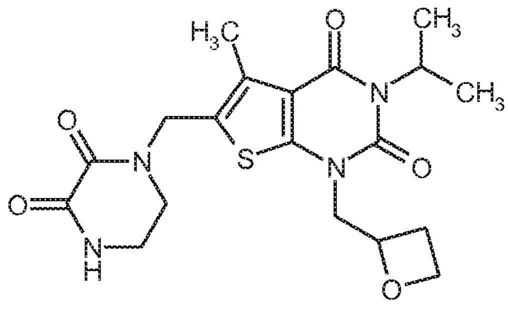

250 mg (0.573 mmol, 84% Reinheit) der Verbindung aus Bsp. 311A wurden in 14 ml Ethanol gelöst und mit 146 µl (1.06 mmol) Oxalsäurediethylester versetzt. Das Gemisch wurde ca. 16 h bei 80°C gerührt. Danach wurde die Reaktionslösung am Rotationsverdampfer eingeengt. Das erhaltene Rohprodukt wurde mittels präparativer HPLC gereinigt (Methode 8). Nach Vereinigen der Produktfraktionen, Einengen und Trocknen im Hochvakuum wurden 134 mg (52% d. Th., 94% Reinheit) der Titelverbindung erhalten.
$^1$H-NMR (400 MHz, DMSO-$d_6$, δ/ppm): 8.61 (br. s, 1H), 5.13 (sept, 1H), 5.00 (quin, 1H), 4.68 (s, 2H), 4.53-4.36 (m, 2H), 4.10 (d, 2H), 3.51-3.42 (m, 2H), 3.32-3.28 (m, 2H, teilweise überdeckt vom Wassersignal), 2.76-2.62 (m, 1H), 2.51-2.43 (m, 1H, teilweise überdeckt vom DMSO-Signal), 2.41 (s, 3H), 1.40 (d, 6H).

LC/MS (Methode 17, ESIpos): $R_t$ = 1.17 min, m/z = 421.15 $[M+H]^+$.

**Beispiel 398**

6-[(2,3-Dioxopiperazin-1-yl)methyl]-3-isopropyl-5-methyl-1-(oxetan-2-ylmethyl)thieno[2,3-d]-pyrimidin-2,4(1*H*,3*H*)-dion (*Enantiomer 1*)

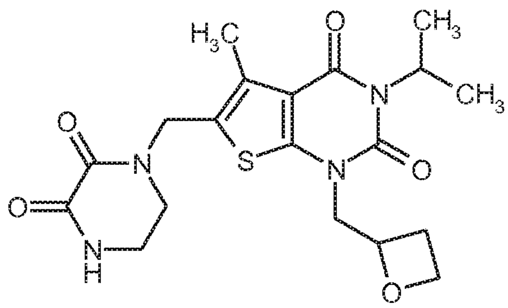

120 mg (0.285 mmol) der racemischen Verbindung aus Bsp. 397 wurden in 5 ml Ethanol gelöst und in 10 Portionen über präparative HPLC an chiraler Phase in die Enantiomere aufgetrennt [Säule: Daicel Chiralcel OD-H, 5 µm, 250 mm x 20 mm; Laufmittel: Isohexan/Ethanol 1:1; Fluss: 15 ml/min; Temperatur: 25°C; Detektion: 210 nm]. Nach Eindampfen der Produktfraktionen, Verrühren des Rückstands mit Pentan, dem etwas Dichlormethan beigefügt war, und Absaugen und Trocknen des Feststoffs im Hochvakuum wurden 39 mg (65% d. Th.) von Enantiomer 1 erhalten (>99% ee, chirale analytische HPLC).
$^1$H-NMR (400 MHz, DMSO-$d_6$, δ/ppm): 8.61 (br. s, 1H), 5.13 (sept, 1H), 5.00 (quin, 1H), 4.68 (s, 2H), 4.53-4.36 (m, 2H), 4.10 (d, 2H), 3.52-3.41 (m, 2H), 3.32-3.28 (m, 2H, teilweise überdeckt vom Wassersignal), 2.75-2.62 (m, 1H), 2.52-2.44 (m, 1H, teilweise überdeckt vom DMSO-Signal), 2.41 (s, 3H), 1.40 (d, 6H).

Chirale analytische HPLC [Säule: Daicel Chiraltek OD-3, 3 µm, 250 mm x 4.6 mm; Laufmittel: Isohexan/Ethanol 1:1; Fluss: 1 ml/min; Temperatur: 25°C; Detektion: 220 nm]: $R_t$ = 3.30 min.

**Beispiel 399**

6-[(2,3-Dioxopiperazin-1-yl)methyl]-3-isopropyl-5-methyl-1-(oxetan-2-ylmethyl)thieno[2,3-d]-pyrimidin-2,4(1*H*,3*H*)-dion *(Enantiomer 2)*

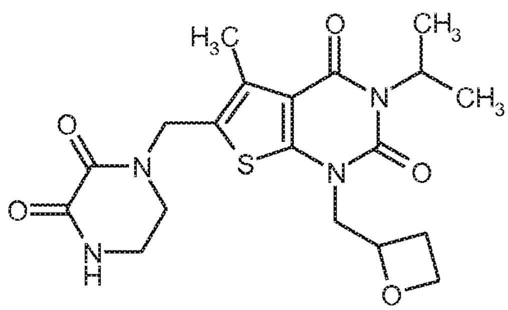

120 mg (0.285 mmol) der racemischen Verbindung aus Bsp. 397 wurden in 5 ml Ethanol gelöst und in 10 Portionen über präparative HPLC an chiraler Phase in die Enantiomere aufgetrennt [Säule: Daicel Chiralcel OD-H, 5 µm, 250 mm x 20 mm; Laufmittel: Isohexan/Ethanol 1:1; Fluss: 15 ml/min; Temperatur: 25°C; Detektion: 210 nm]. Nach Eindampfen der Produktfraktionen, Verrühren des Rückstands mit Pentan, dem etwas Dichlormethan beigefügt war, und Absaugen und Trocknen des Feststoffs im Hochvakuum wurden 33 mg (55% d. Th.) von Enantiomer 2 erhalten (>99% ee, chirale analytische HPLC).
$^1$H-NMR (400 MHz, DMSO-$d_6$, δ/ppm): 8.61 (br. s, 1H), 5.13 (sept, 1H), 5.00 (quin, 1H), 4.68 (s, 2H), 4.53-4.37 (m, 2H), 4.10 (d, 2H), 3.50-3.42 (m, 2H), 3.33-3.28 (m, 2H, teilweise überdeckt vom Wassersignal), 2.76-2.62 (m, 1H), 2.53-2.44

(m, 1H, teilweise überdeckt vom DMSO-Signal), 2.41 (s, 3H), 1.40 (d, 6H).

Chirale analytische HPLC [Säule: Daicel Chiraltek OD-3, 3 μm, 250 mm x 4.6 mm; Laufmittel: Isohexan/Ethanol 1:1; Fluss: 1 ml/min; Temperatur: 25°C; Detektion: 220 nm]: $R_t$ = 5.45 min.

**Beispiel 400**

6-[(2,3-Dioxopiperazin-1-yl)methyl]-3-(2-methoxyethyl)-5-methyl-1-(oxetan-2-ylmethyl)thieno-[2,3-d]pyrimidin-2,4(1*H*,3*H*)-dion (*nicht-racemisches Enantiomerengemisch*)

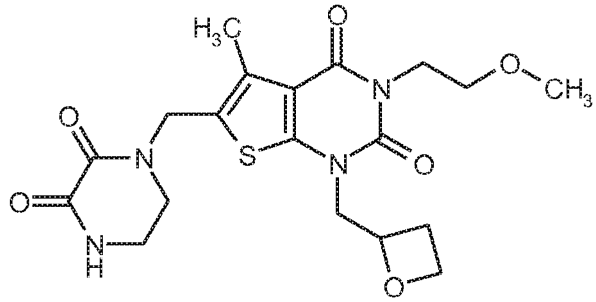

Analog zu dem unter Bsp. 397 beschriebenen Verfahren wurden aus 400 mg (0.878 mmol, 84% Reinheit) der Verbindung aus Bsp. 405A und 224 μl (1.63 mmol) Oxalsäurediethylester 30 mg (7% d. Th.) der Titelverbindung erhalten. $^1$H-NMR (500 MHz, DMSO-$d_6$, δ/ppm): 8.62 (br. s, 1H), 5.00 (dt, 1H), 4.69 (s, 2H), 4.48 (td, 1H), 4.41 (dt, 1H), 4.17-4.10 (m, 2H), 4.06 (t, 2H), 3.50 (t, 2H), 3.49-3.46 (m, 2H), 3.32-3.28 (m, 2H, teilweise überdeckt vom Wassersignal), 3.23 (s, 3H), 2.75-2.60 (m, 1H), 2.52-2.45 (m, 1H, teilweise überdeckt vom DMSO-Signal), 2.42 (s, 3H).

LC/MS (Methode 17, ESIpos): $R_t$ = 0.91 min, m/z = 437.15 $[M+H]^+$.

**Beispiel 401**

6-[(2,3-Dioxopiperazin-1-yl)methyl]-3-(2-methoxyethyl)-5-methyl-1-(oxetan-2-ylmethyl)thieno-[2,3-d]pyrimidin-2,4(1*H*,3*H*)-dion *(Enantiomer 1)*

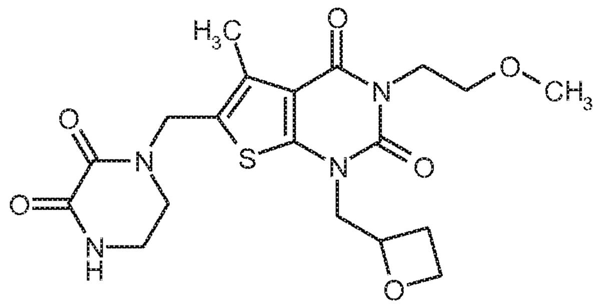

30 mg (0.069 mmol) des Enantiomerengemisches aus Bsp. 400 wurden in einem Gemisch aus 2 ml Methanol und 2 ml Dichlormethan gelöst und in 14 Portionen über präparative HPLC an chiraler Phase in die Enantiomere aufgetrennt [Säule: Daicel Chiralpak IC, 5 μm, 250 mm x 20 mm; Laufmittel: *tert.*-Butylmethylether/Methanol 1:1; Fluss: 15 ml/min; Temperatur: 30°C; Detektion: 220 nm]. Nach Eindampfen der Produktfraktionen und Trocknen im Hochvakuum wurden 18 mg (60% d. Th.) von Enantiomer 1 erhalten (>99% ee, chirale analytische HPLC).

$^1$H-NMR (400 MHz, DMSO-$d_6$, δ/ppm): 8.62 (br. s, 1H), 5.00 (dt, 1H), 4.69 (s, 2H), 4.53-4.37 (m, 2H), 4.14 (d, 2H), 4.06 (t, 2H), 3.50 (t, 2H), 3.49-3.46 (m, 2H), 3.33-3.28 (m, 2H, teilweise überdeckt vom Wassersignal), 3.23 (s, 3H), 2.74-2.63 (m, 1H), 2.52-2.45 (m, 1H, teilweise überdeckt vom DMSO-Signal), 2.42 (s, 3H).

Chirale analytische HPLC [Säule: Daicel Chiralpak IC, 5 μm, 250 mm x 4.6 mm; Laufmittel: *tert.*-Butylmethylether/Methanol 1:1; Fluss: 1 ml/min; Temperatur: 30°C; Detektion: 220 nm]: $R_t$ = 11.36 min.

**Beispiel 402**

6-[(2,3-Dioxopiperazin-1-yl)methyl]-3-(2-methoxyethyl)-5-methyl-1-(oxetan-2-ylmethyl)thieno-[2,3-d]pyrimidin-2,4(1*H*,3*H*)-dion (*Enantiomer 2)*

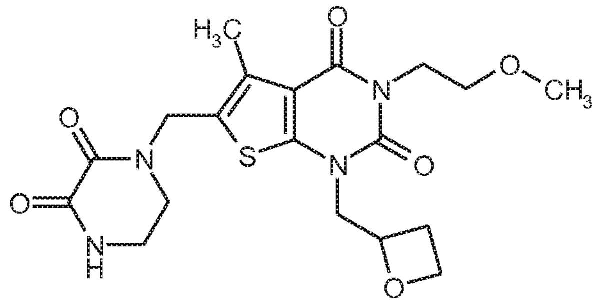

30 mg (0.069 mmol) des Enantiomerengemisches aus Bsp. 400 wurden in einem Gemisch aus 2 ml Methanol und 2 ml Dichlormethan gelöst und in 14 Portionen über präparative HPLC an chiraler Phase in die Enantiomere aufgetrennt [Säule: Daicel Chiralpak IC, 5 μm, 250 mm x 20 mm; Laufmittel: *tert.*-Butylmethylether/Methanol 1:1; Fluss: 15 ml/min; Temperatur: 30°C; Detektion: 220 nm]. Nach Eindampfen der Produktfraktionen und Trocknen im Hochvakuum wurden 2 mg (6% d. Th.) von Enantiomer 2 erhalten (99% ee, chirale analytische HPLC).
$^1$H-NMR (500 MHz, DMSO-$d_6$, δ/ppm): 8.62 (br. s, 1H), 5.00 (quin, 1H), 4.69 (s, 2H), 4.52-4.45 (m, 1H), 4.41 (dt, 1H), 4.19-4.10 (m, 2H), 4.06 (t, 2H), 3.50 (t, 2H), 3.49-3.46 (m, 2H), 3.23 (s, 3H), 2.75-2.63 (m, 1H), 2.52-2.45 (m, 1H, teilweise überdeckt vom DMSO-Signal), 2.42 (s, 3H) [weiteres Signal vom Wassersignal überlagert].
Chirale analytische HPLC [Säule: Daicel Chiralpak IC, 5 μm, 250 mm x 4.6 mm; Laufmittel: *tert.*-Butylmethylether/Methanol 1:1; Fluss: 1 ml/min; Temperatur: 30°C; Detektion: 220 nm]: $R_t$ = 12.52 min.

**Beispiel 403**

6-[(2,3-Dioxopiperazin-1-yl)methyl]-3-(2-ethoxyethyl)-5-methyl-1-(3,3,3-trifluorpropyl)thieno-[2,3-d]pyrimidin-2,4(1*H*,3*H*)-dion

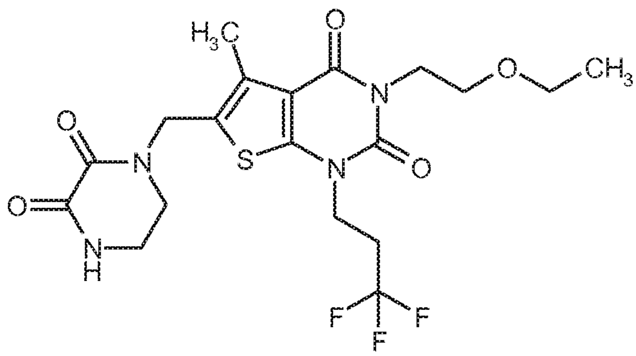

120 mg (0.253 mmol) der Verbindung aus Bsp. 406A wurden in 5 ml Ethanol gelöst und mit 373 mg (2.53 mmol) Oxalsäurediethylester versetzt. Das Gemisch wurde 16 h bei 80°C gerührt. Danach wurde die Reaktionslösung am Rotationsverdampfer eingeengt. Der Rückstand wurde in 30 ml Dichlormethan gelöst und diese Lösung mit Wasser und gesättigter Natriumhydrogencarbonat-Lösung gewaschen. Nach Trocknen über wasserfreiem Natriumsulfat wurde die organische Phase filtriert und eingeengt. Der verbliebene Rückstand wurde in 3 ml DMSO gelöst und diese Lösung mittels präparativer HPLC gereinigt (Methode 14). Nach Vereinigen der Produktfraktionen und Gefriertrocknung wurden 54 mg (43% d. Th.) der Titelverbindung erhalten.
$^1$H-NMR (500 MHz, DMSO-$d_6$, δ/ppm): 8.64 (br. s, 1H), 4.71 (s, 2H), 4.10 (t, 2H), 4.04 (t, 2H), 3.50 (q, 4H), 3.44 (q, 2H), 2.82-2.70 (m, 2H), 2.44 (s, 3H), 1.06 (t, 3H) [ein weiteres Signal komplett vom Wassersignal überlagert].

LC/MS (Methode 3, ESIpos): $R_t$ = 0.94 min, m/z = 477 $[M+H]^+$.

**Beispiel 404**

6-[(2,3-Dioxopiperazin-1-yl)methyl]-3-(2-ethoxyethyl)-1-(2-methoxyethyl)-5-methylthieno[2,3-d]-pyrimidin-2,4(1*H*,3*H*)-dion

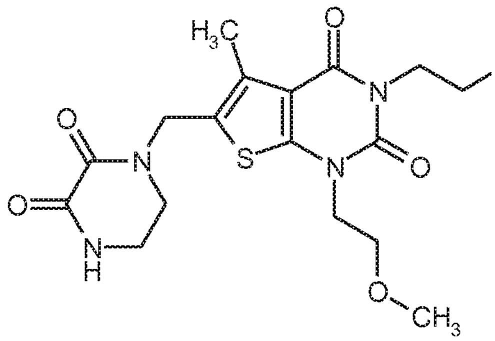

160 mg (0.291 mmol) der Verbindung aus Bsp. 407A wurden in 5 ml Ethanol gelöst und mit 430 mg (2.91 mmol) Oxalsäurediethylester versetzt. Das Gemisch wurde 114 h bei 80°C gerührt. Danach wurde die Reaktionslösung am Rotationsverdampfer eingeengt. Der Rückstand wurde in 30 ml Dichlormethan gelöst und diese Lösung mit Wasser und gesättigter Natriumhydrogencarbonat-Lösung gewaschen. Nach Trocknen über wasserfreiem Natriumsulfat wurde die organische Phase filtriert und eingeengt. Der verbliebene Rückstand wurde in 3 ml DMSO gelöst und diese Lösung mittels präparativer HPLC gereinigt (Methode 14). Nach Vereinigen der Produktfraktionen und Gefriertrocknung wurden 87 mg (62% d. Th.) der Titelverbindung erhalten.
$^1$H-NMR (500 MHz, DMSO-$d_6$, δ/ppm): 8.64 (br. s, 1H), 4.69 (s, 2H), 4.07-3.98 (m, 4H), 3.62 (t, 2H), 3.53-3.41 (m, 6H), 3.33-3.28 (m, 2H), 3.24-3.21 (m, 3H), 2.42 (s, 3H), 1.06 (t, 3H).
LC/MS (Methode 3, ESIpos): $R_t$ = 0.8 min, m/z = 439 $[M+H]^+$.

**Beispiel 405**

3-(2-Ethoxyethyl)-1-(3-fluorpropyl)-5-methyl-6-[(2-thioxoimidazolidin-1-yl)methyl]thieno[2,3-d]-pyrimidin-2,4(1*H*,3*H*)-dion

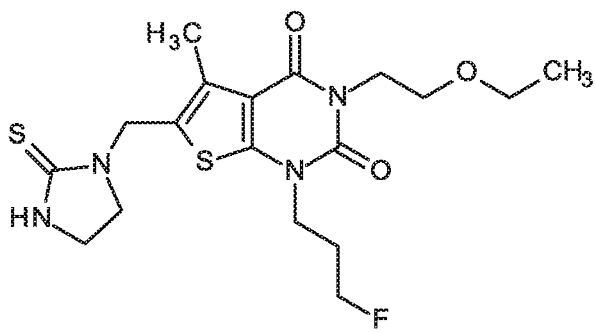

200 mg (0.388 mmol) der Verbindung aus Bsp. 457A wurden in 27 ml Dioxan gelöst und mit 109 mg (0.582 mmol) 1,1'-Thiocarbonyldiimidazol versetzt. Das Gemisch wurde 15 h bei RT gerührt. Die Reaktionslösung wurde dann am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 126 mg (75% d. Th.) der Titelverbindung erhalten.
$^1$H-NMR (400 MHz, DMSO-$d_6$, δ/ppm): 8.35 (s, 1H), 4.83 (s, 2H), 4.58 (t, 1H), 4.46 (t, 1H), 4.03 (br. t, 2H), 3.97 (br. t, 2H), 3.58-3.37 (m, 8H), 2.43 (s, 3H), 2.14-1.97 (m, 2H), 1.06 (t, 3H).

LC/MS (Methode 3, ESIpos): $R_t$ = 1.02 min, m/z = 429 $[M+H]^+$.

**Beispiel 406**

3-(2,2-Dimethylpropyl)-5-methyl-6-[(2-oxoimidazolidin-1-yl)methyl]-1-(tetrahydrofuran-2-yl-methyl)thieno[2,3-d]pyrimidin-2,4(1*H*,3*H*)-dion *(Racemat)*

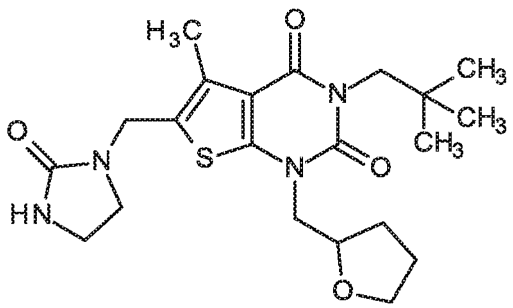

Eine Lösung von 560 mg (1.16 mmol, 85% Reinheit) der Verbindung aus Bsp. 454A und 244 µl (1.75 mmol) Triethylamin in 12 ml THF wurde mit 227 mg (1.40 mmol) CDI versetzt und ca. 18 h bei RT gerührt. Anschließend wurde zur Trockene eingeengt. Der verbliebene Rückstand wurde in Ethylacetat aufgenommen und nacheinander mit gesättigter Natriumcarbonat-Lösung, Wasser und gesättigter Natriumchlorid-Lösung gewaschen. Nach Trocknen über wasserfreiem Magnesiumsulfat wurde filtriert und eingeengt. Der feste Rückstand wurde bei RT in wenig Acetonitril verrührt. Nach Filtration und Trocknen des Feststoffs im Hochvakuum wurden 260 mg (48% d. Th., 95% Reinheit) der Titelverbindung erhalten.

$^1$H-NMR (400 MHz, DMSO-$d_6$, δ/ppm): 6.52 (s, 1H), 4.34 (s, 2H), 4.27-4.17 (m, 1H), 4.08-3.95 (m, 1H), 3.82 (br. s, 2H), 3.73 (quin, 2H), 3.65-3.57 (m, 1H), 3.29-3.16 (m, 4H), 2.38 (s, 3H), 2.03-1.75 (m, 3H), 1.72-1.59 (m, 1H), 0.89 (s, 9H).

LC/MS (Methode 17, ESIpos): $R_t$ = 1.78 min, m/z = 435.21 $[M+H]^+$.

**Beispiel 407**

3-(2,2-Dimethylpropyl)-5-methyl-6-[(2-oxoimidazolidin-1-yl)methyl]-1-(tetrahydrofuram-2-ylmethyl)thieno[2,3-d]pyrimidin-2,4(1*H*,3*H*)-dion (*Enantiomer 1*)

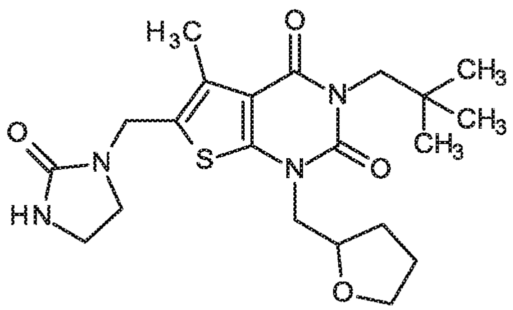

250 mg (0.691 mmol, 95% Reinheit) der racemischen Verbindung aus Bsp. 406 wurden in 12 ml Dioxan/Methanol-Gemisch gelöst und in 120 Portionen über präparative HPLC an chiraler Phase in die Enantiomere aufgetrennt [Säule: Daicel Chiralpak ID, 5 µm, 250 mm x 20 mm; Laufmittel: *n*-Heptan/Ethanol 1:1; Fluss: 50 ml/min; Temperatur: 23°C; Detektion: 235 nm]. Nach Eindampfen der Produktfraktionen und Trocknen im Hochvakuum wurden 53 mg (44% d. Th.) von Enantiomer 1 erhalten (>99% ee, chirale analytische HPLC).

$^1$H-NMR (400 MHz, DMSO-$d_6$, δ/ppm): 6.52 (s, 1H), 4.34 (s, 2H), 4.28-4.16 (m, 1H), 4.00 (dd, 1H), 3.82 (br. s, 2H), 3.73 (quin, 2H), 3.66-3.56 (m, 1H), 3.29-3.16 (m, 4H), 2.38 (s, 3H), 2.03-1.74 (m, 3H), 1.72-1.60 (m, 1H), 0.89 (s, 9H).

Chirale analytische HPLC [Säule: Daicel Chiralpak ID-3, 3 µm, 50 mm x 4.6 mm; Laufmittel: *n*-Heptan/Ethanol 1:1; Fluss: 1 ml/min; Temperatur: 40°C; Detektion: 220 nm]: $R_t$ = 3.11 min.

**Beispiel 408**

3-(2,2-Dimethylpropyl)-5-methyl-6-[(2-oxoimidazolidin-1-yl)methyl]-1-(tetrahydrofuran-2-yl-methyl)thieno[2,3-d]pyrimidin-2,4(1*H*,3*H*)-dion (*Enantiomer 2*)

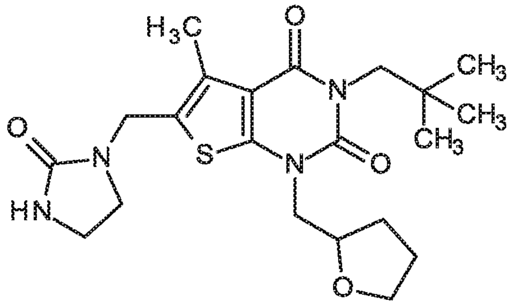

250 mg (0.691 mmol, 95% Reinheit) der racemischen Verbindung aus Bsp. 406 wurden in 12 ml Dioxan/Methanol-Gemisch gelöst und in 120 Portionen über präparative HPLC an chiraler Phase in die Enantiomere aufgetrennt [Säule: Daicel Chiralpak ID, 5 μm, 250 mm x 20 mm; Laufmittel: *n*-Heptan/Ethanol 1:1; Fluss: 50 ml/min; Temperatur: 23°C; Detektion: 235 nm]. Nach Eindampfen der Produktfraktionen und Trocknen im Hochvakuum wurden 67 mg (56% d. Th.) von Enantiomer 2 erhalten (84% ee, chirale analytische HPLC).
$^1$H-NMR (400 MHz, DMSO-$d_6$, δ/ppm): 6.52 (s, 1H), 4.34 (s, 2H), 4.28-4.16 (m, 1H), 4.00 (dd, 1H), 3.82 (br. s, 2H), 3.73 (quin, 2H), 3.66-3.56 (m, 1H), 3.29-3.16 (m, 4H), 2.38 (s, 3H), 2.03-1.74 (m, 3H), 1.72-1.60 (m, 1H), 0.89 (s, 9H).
Chirale analytische HPLC [Säule: Daicel Chiralpak ID-3, 3 μm, 50 mm x 4.6 mm; Laufmittel: *n*-Heptan/Ethanol 1:1; Fluss: 1 ml/min; Temperatur: 40°C; Detektion: 220 nm]: $R_t$ = 3.45 min.

**Beispiel 409**

5-Methyl-6-[(2-oxoimidazolidin-1-yl)methyl]-3-(1,1,1-trifluorpropan-2-yl)-1-(3,3,3-trifluorpropyl)-thieno[2,3-d]pyrimidin-2,4(1*H*,3*H*)-dion *(Racemat)*

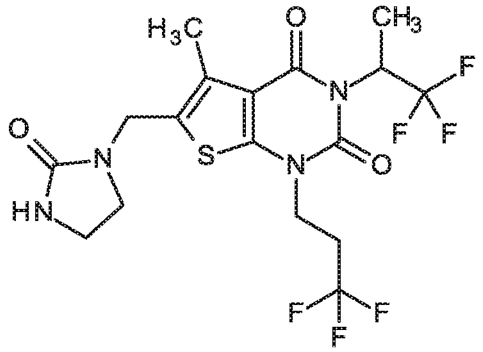

Eine Lösung von 590 mg (1.11 mmol, 84% Reinheit) der Verbindung aus Bsp. 455A und 232 μl (1.67 mmol) Triethylamin in 12 ml THF wurde mit 216 mg (1.33 mmol) CDI versetzt und ca. 18 h bei RT gerührt. Anschließend wurde zur Trockene eingeengt. Der verbliebene Rückstand wurde in Ethylacetat aufgenommen und nacheinander mit gesättigter Natriumcarbonat-Lösung, Wasser und gesättigter Natriumchlorid-Lösung gewaschen. Nach Trocknen über wasserfreiem Magnesiumsulfat wurde filtriert und eingeengt. Der feste Rückstand wurde mittels präparativer HPLC gereinigt (Methode 8). Nach Eindampfen der Produktfraktionen und Trocknen im Hochvakuum wurden 332 mg (63% d. Th.) der Titelverbindung erhalten.
$^1$H-NMR (400 MHz, DMSO-$d_6$, δ/ppm): 6.55 (s, 1H), 5.84-5.45 (m, 1H), 4.38 (s, 2H), 4.20-4.01 (m, 2H), 3.29-3.16 (m, 4H), 2.87-2.66 (m, 2H), 2.40 (s, 3H), 1.63 (br. d, 3H).
LC/MS (Methode 17, ESIneg): $R_t$ = 1.77 min, m/z = 517.10 $[M-H+HCO_2H]^-$.

**Beispiel 410**

1-(2-Methoxyethyl)-5-methyl-6-[(2-oxoimidazolidin-1-yl)methyl]-3-(1,1,1-trifluorpropan-2-yl)-thieno[2,3-d]pyrimidin-2,4(1*H*,3*H*)-dion *(Racemat)*

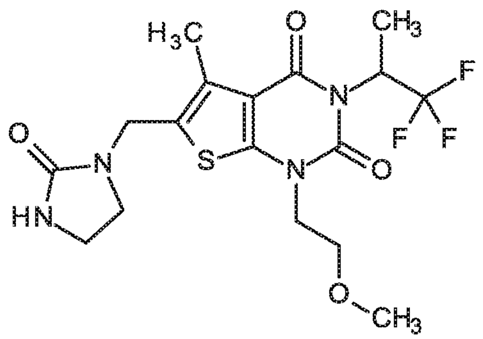

Eine Lösung von 295 mg (0.592 mmol, 82% Reinheit) der Verbindung aus Bsp. 456A und 124 µl (0.888 mmol) Triethylamin in 6 ml THF wurde mit 115 mg (0.711 mmol) CDI versetzt und ca. 18 h bei RT gerührt. Anschließend wurde zur Trockene eingeengt. Der verbliebene Rückstand wurde in Ethylacetat aufgenommen und nacheinander mit gesättigter Natriumcarbonat-Lösung, Wasser und gesättigter Natriumchlorid-Lösung gewaschen. Nach Trocknen über wasserfreiem Magnesiumsulfat wurde filtriert und eingeengt. Der feste Rückstand wurde mittels präparativer HPLC gereinigt (Methode 8). Die Produktfraktionen wurden vereinigt, eingeengt und der verbliebene Rückstand ein weiteres Mal mittels präparativer HPLC gereinigt [Säule: Phenomenex Kinetex C18, 5 µm, 100 mm x 30 mm; Eluent A: Wasser; Eluent B: Acetonitril mit 5 ml/l 2%-ige Ameisensäure in Wasser; Gradient: 0-2 min 10% B, 2-2.2 min auf 20% B, 2.2-7 min auf 60% B, 7-7.5 min auf 92% B, 7.5-9 min 92% B; Fluss: 65 ml/min; Temperatur: 23°C; UV-Detektion: 200-400 nm]. Nach erneutem Eindampfen der Produktfraktionen und Trocknen im Hochvakuum wurden 112 mg (43% d. Th.) der Titelverbindung erhalten.

$^1$H-NMR (400 MHz, DMSO-$d_6$, δ/ppm): 6.54 (s, 1H), 5.84-5.47 (m, 1H), 4.35 (s, 2H), 4.09-3.97 (m, 2H), 3.68-3.57 (m, 2H), 3.29-3.16 (m, 4H), 3.24 (s, 3H), 2.39 (s, 3H), 1.63 (br. d, 3H).

LC/MS (Methode 17, ESIpos): $R_t$ = 1.55 min, m/z = 435.13 [M+H]$^+$.

**Beispiel 411**

3-(2-Ethoxyethyl)-1-(3-fluorpropyl)-5-methyl-6-[(2-oxoimidazolidin-1-yl)methyl]thieno[2,3-d]-pyrimidin-2,4(1*H*,3*H*)-dion

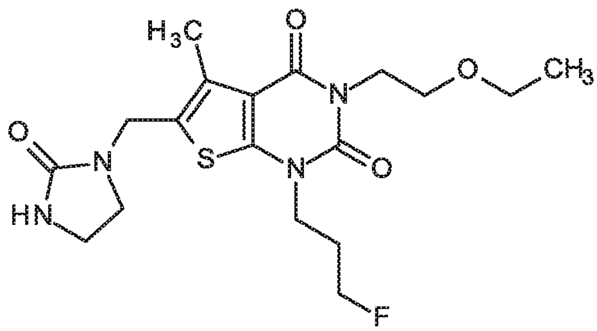

290 mg (0.563 mmol) der Verbindung aus Bsp. 457A wurden in 25 ml Dioxan gelöst und mit 141 mg (0.844 mmol) CDI versetzt. Das Gemisch wurde 15 h bei RT gerührt. Die Reaktionslösung wurde danach am Rotationsverdampfer eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 199 mg (86% d. Th.) der Titelverbindung erhalten.

$^1$H-NMR (400 MHz, DMSO-$d_6$, δ/ppm): 6.54 (s, 1H), 4.59 (t, 1H), 4.47 (t, 1H), 4.35 (s, 2H), 4.03 (t, 2H), 3.97 (br. t, 2H),

3.50 (t, 2H), 3.44 (q, 2H), 3.29-3.17 (m, 4H), 2.39 (s, 3H), 2.13-1.97 (m, 2H), 1.06 (t, 3H).
LC/MS (Methode 3, ESIpos): $R_t$ = 0.93 min, m/z = 413 $[M+H]^+$.

**Beispiel 412**

3-(2-Methoxypropyl)-5-methyl-6-[(2-oxoimidazolidin-1-yl)methyl]-1-(3,3,3-trifluorpropyl)thieno-[2,3-d]pyrimidin-2,4(1*H*,3*H*)-dion *(Racemat)*

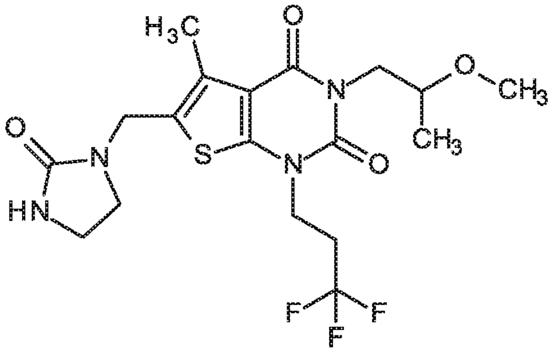

Eine Lösung von 235 mg (2.62 mmol) 2-Imidazolidinon in 9 ml THF wurde mit 105 mg (2.62 mmol) Natriumhydrid (60% Suspension in Mineralöl) versetzt und 3 h bei 60°C gerührt ("Lösung 1"). In einem anderen Reaktionsgefäß wurde eine Lösung von 249 mg (0.655 mmol) der Verbindung aus Bsp. 452A in 4.5 ml Dichlormethan bei 0°C mit 342 µl (1.97 mmol) *N,N*-Diisopropylethylamin und 72 µl (0.983 mmol) Thionylchlorid versetzt und 75 min gerührt. Anschließend wurde die Lösung 1 portionsweise zugesetzt und das Gemisch 18 h bei RT gerührt. Danach wurde das Reaktionsgemisch mit 70 ml Wasser versetzt. Es wurde mit Ethylacetat extrahiert. Die vereinigten organischen Phasen wurden über Natriumsulfat getrocknet, filtriert und eingeengt. Der erhaltene Rückstand wurde über eine Kieselgel-Kartusche chromatographiert (Biotage, 10 g Kieselgel, Laufmittel Hexan/Ethylacetat). Es wurden 97 mg (31% d. Th.) der Titelverbindung erhalten.
$^1$H-NMR (400 MHz, DMSO-$d_6$, δ/ppm): 6.56 (s, 1H), 4.37 (s, 2H), 4.15-4.00 (m, 4H), 3.75 (dd, 1H), 3.68-3.58 (m, 1H), 3.30-3.18 (m, 7H), 2.83-2.69 (m, 2H), 2.40 (s, 3H), 1.05 (d, 3H).
LC/MS (Methode 3): $R_t$ = 1.01 min, m/z = 449 $[M+H]^+$.

**Beispiel 413**

3-(2-Methoxypropyl)-5-methyl-6-[(2-oxoimidazolidin-1-yl)methyl]-1-(3,3,3-trifluorpropyl)thieno-[2,3-d]pyrimidin-2,4(1*H*,3*H*)-dion (*Enantiomer 1)*

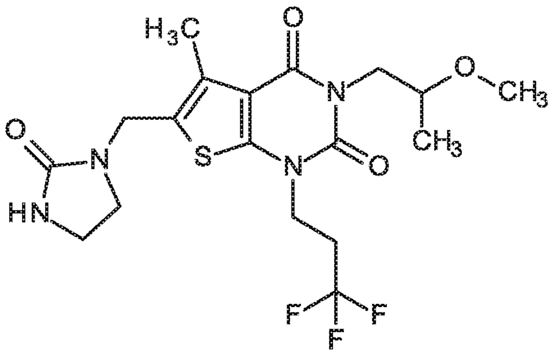

92 mg der racemischen Verbindung aus Bsp. 412 wurden in 1.5 ml eines Methanol/Dichlormethan-Gemisches (1:1) gelöst und mittels präparativer HPLC an chiraler Phase in die Enantiomere aufgetrennt [Säule: Daicel Chiralpak IC, 5 µm, 250 mm x 30 mm; Eluent A: Acetonitril; Eluent B: Ethanol; isokratisch 90% A / 10% B; Fluss: 50.0 ml/min; UV-Detektion: 254 nm]. Die jeweiligen Produktfraktionen wurden am Rotationsverdampfer eingeengt, mit tert.-Butanol versetzt und gefriergetrocknet. Es wurden 29 mg der Titelverbindung (Enantiomer 1) sowie 29 mg des Enantiomers 2 (siehe Beispiel 414) erhalten.
$^1$H-NMR (400 MHz, DMSO-$d_6$, $\delta$/ppm): 6.56 (s, 1H), 4.36 (s, 2H), 4.15-4.01 (m, 3H), 3.75 (dd, 1H), 3.63 (sext, 1H), 3.30-3.18 (m, 7H), 2.83-2.69 (m, 2H), 2.40 (s, 3H), 1.05 (d, 3H).

Chirale analytische HPLC [Säule: Daicel Chiralpak IC, 3 µm, 100 mm x 4.6 mm; Eluent A: Acetonitril + 0.1% Diethylamin; Eluent B: Ethanol; isokratisch 90% A + 10% B; Fluss: 1.4 ml/min; Temperatur: 25°C; DAD 254 nm]: $R_t$ = 3.28 min.

## Beispiel 414

3-(2-Methoxypropyl)-5-methyl-6-[(2-oxoimidazolidin-1-yl)methyl]-1-(3,3,3-trifluorpropyl)thieno-[2,3-d]pyrimidin-2,4(1*H*,3*H*)-dion (*Enantiomer 2)*

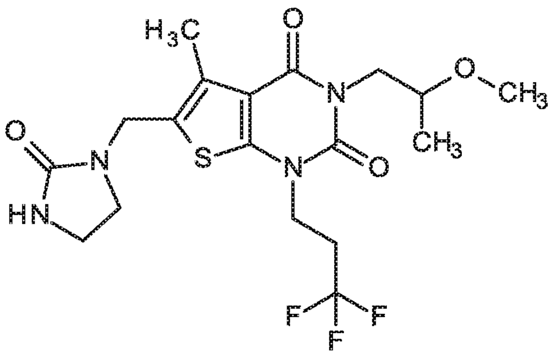

Die Titelverbindung (29 mg) wurde als zweites Enantiomer bei der unter Bsp. 413 beschriebenen präparativen HPLC-Trennung des Racemats aus Bsp. 412 erhalten.
$^1$H-NMR (400 MHz, DMSO-$d_6$, $\delta$/ppm): 6.56 (s, 1H), 4.36 (s, 2H), 4.15-4.00 (m, 3H), 3.75 (dd, 1H), 3.63 (sext, 1H), 3.30-3.18 (m, 7H), 2.83-2.69 (m, 2H), 2.40 (s, 3H), 1.05 (d, 3H).

Chirale analytische HPLC [Säule: Daicel Chiralpak IC, 3 µm, 100 mm x 4.6 mm; Eluent A: Acetonitril + 0.1% Diethylamin; Eluent B: Ethanol; isokratisch 90% A + 10% B; Fluss: 1.4 ml/min; Temperatur: 25°C; DAD 254 nm]: $R_t$ = 4.30 min.

## Beispiel 415

3-Ethyl-1-(2-methoxyethyl)-5-methyl-6-[(2-oxoimidazolidin-1-yl)(dideuteno)methyl]thieno[2,3-d]-pyrimidin-2,4(1*H*,3*H*)-dion

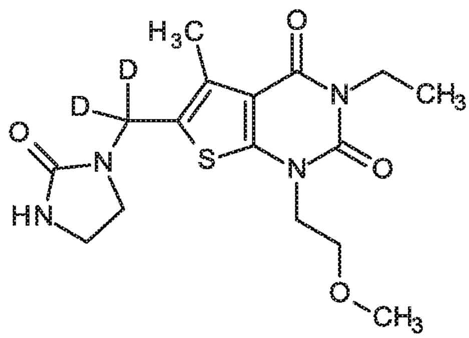

Eine Lösung von 454 mg (5.27 mmol) Imidazolidin-2-on in 13 ml DMF wurde mit 211 mg (5.27 mmol) Natriumhydrid (60% Suspension in Mineralöl) versetzt, dann 15 min auf 60°C erwärmt und anschließend wieder auf RT

abgekühlt ("Lösung 1"). In einem anderen Reaktionsgefäß wurde eine Lösung von 400 mg (1.32 mmol) der Verbindung aus Bsp. 453A in 9.5 ml Dichlormethan bei 0°C mit 459 µl (2.64 mmol) *N,N*-Diisopropylethylamin und 101 µl (1.38 mmol) Thionylchlorid versetzt. Nach 35 min bei 0°C wurde Lösung 1 portionsweise hinzugefügt und anschließend das Kältebad entfernt. Das Reaktionsgemisch wurde dann 3 Tage bei RT gerührt. Danach wurden alle flüchtigen Bestandteile am Rotationsverdampfer entfernt. Der verbliebene Rückstand wurde mit Ethylacetat aufgenommen und nacheinander mit Wasser und gesättigter wässriger Natriumchlorid-Lösung gewaschen. Nach Trocknen über wasserfreiem Magnesiumsulfat wurde filtriert und eingeengt. Das Rohprodukt wurde mittels präparativer HPLC (Methode 8) gereinigt. Die noch leicht verunreinigten Produktfraktionen wurden vereinigt, eingeengt und mittels erneuter präparativer HPLC nachgereinigt [Säule: Phenomenex Kinetex C18, 5 µm, 150 mm x 21.2 mm; Eluent A: Wasser; Eluent B: Methanol; Eluent C: 1% Ameisensäure in Wasser; Gradient: 0-1 min 65% A 30% B 5% C, 2-6 min auf 25% A 70% B 5% C, 6-6.2 min auf 65% A 30% B 5% C, 6.2-7.6 min 65% A 30% B 5% C; Fluss: 25 ml/min; Temperatur: 23°C; UV-Detektion: 210 nm]. Die Produktfraktionen wurden vereinigt und eingeengt und der Rückstand abschließend mit wenig Diethylether/Ethylacetat-Gemisch bei RT verrührt. Es wurden so 92 mg (18% d. Th.) der Titelverbindung erhalten.

$^1$H-NMR (400 MHz, DMSO-$d_6$, δ/ppm): 6.51 (s, 1H), 4.02 (t, 2H), 3.90 (q, 2H), 3.63 (t, 2H), 3.28-3.15 (m, 4H), 3.24 (s, 3H), 2.39 (s, 3H), 1.12 (t, 3H).

LC/MS (Methode 17, ESIpos): $R_t$ = 1.20 min, m/z = 369.15 $[M+H]^+$.

**Beispiel 416**

3-Ethyl-5-methyl-6-[1-(2-oxoimidazolidin-1-yl)ethyl]-1-(3,3,3-trifluorpropyl)thieno[2,3-d]-pyrimidin-2,4(1*H*,3*H*)-dion *(Racemat)*

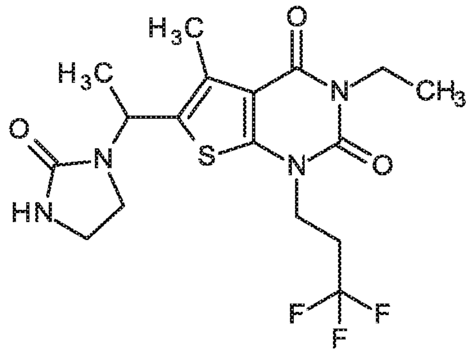

310 mg (0.887 mmol) der Verbindung aus Bsp. 461A wurden in einem Gemisch aus 13 ml DMF und 6.5 ml THF gelöst und bei RT mit 80 µl (0.932 mmol) 2-Chlorethylisocyanat versetzt. Nachdem das Reaktionsgemisch 30 min bei RT gerührt worden war, wurde es mit 149 mg (1.33 mmol) Kalium-tert.-butylat versetzt. Nach ca. 18 h Rühren bei RT wurde das Reaktionsgemisch mit Ethylacetat verdünnt und nacheinander mit gesättigter Natriumhydrogencarbonat-Lösung (dreimal) und gesättigter Natriumchlorid-Lösung gewaschen. Nach Trocknen über wasserfreiem Magnesiumsulfat wurde filtriert und eingeengt. Das so erhaltene Rohprodukt wurde mittels präparativer HPLC gereinigt (Methode 8). Nach Eindampfen der Produktfraktionen und Trocknen im Hochvakuum wurden 146 mg (34% d. Th., 89% Reinheit) der Titelverbindung erhalten.

$^1$H-NMR (400 MHz, DMSO-$d_6$, δ/ppm): 6.39 (s, 1H), 5.31 (q, 1H), 4.12 (td, 2H), 3.90 (q, 2H), 3.43-3.35 (m, 1H), 3.26-3.09 (m, 3H), 2.86-2.69 (m, 2H), 2.39 (s, 3H), 1.46 (d, 3H), 1.11 (t, 3H).

LC/MS (Methode 17, ESIpos): $R_t$ = 1.56 min, m/z = 333.09 $[M+H-C_3H_6N_2O]^+$.

**Beispiel 417**

6-[(4-Allyl-5-oxo-4,5-dihydro-1*H*-1,2,4-triazol-1-yl)methyl]-1-(fluormethyl)-3-isopropyl-5-methyl-thieno[2,3-d]pyrimidin-2,4(1*H*,3*H*)-dion

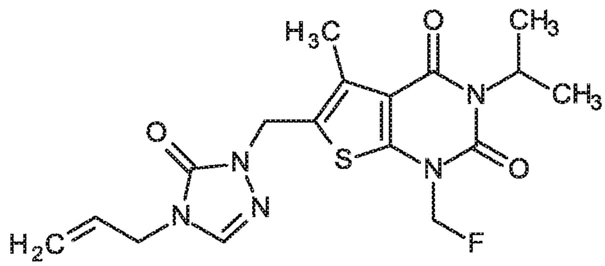

320 mg (1.05 mmol) der Verbindung aus Bsp. 450A und 232 mg (1.26 mmol) 4-Allyl-2,4-dihydro-3*H*-1,2,4-triazol-3-on wurden in 19 ml Dichlormethan gelöst und mit 455 mg (2.10 mmol) Tri-n-butylphosphin versetzt. Nach 20 min wurden 312 µl (1.58 mmol) Diisopropylazodicarboxylat (DIAD) hinzugetropft. Das Reaktionsgemisch wurde dann 17 h bei RT gerührt. Danach wurde mit 100 ml Dichlormethan versetzt und nacheinander mit 50 ml Wasser und 50 ml gesättigter Natriumchlorid-Lösung gewaschen. Die organischen Phase wurde über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 183 mg (42% d. Th.) der Titelverbindung erhalten.
$^1$H-NMR (400 MHz, DMSO-$d_6$, δ/ppm): 7.97 (s, 1H), 6.04 (s, 1H), 5.95-5.83 (m, 2H), 5.19 (dd, 1H), 5.15-5.04 (m, 2H), 5.02 (s, 2H), 4.20 (dt, 2H), 2.45 (s, 3H), 1.40 (d, 6H).
LC/MS (Methode 1, ESIpos): $R_t$ = 1.09 min, m/z = 394 $[M+H]^+$.

**Beispiel 418**

6-[(4-Allyl-5-oxo-4,5-dihydro-1*H*-1,2,4-triazol-1-yl)methyl]-1-(3-fluorpropyl)-3-isopropyl-5-methylthieno[2,3-d]pyrimidin-2,4(1*H*,3*H*)-dion

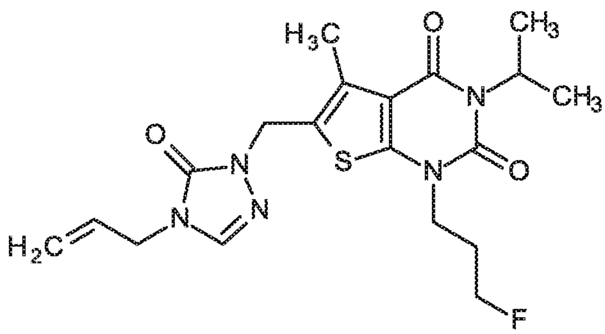

320 mg (0.998 mmol) der Verbindung aus Bsp. 451A und 220 mg (1.20 mmol) 4-Allyl-2,4-dihydro-3*H*-1,2,4-triazol-3-on wurden in 18 ml Dichlormethan gelöst und mit 432 mg (2.0 mmol) Tri-n-butylphosphin versetzt. Nach 20 min wurden 297 µl (1.50 mmol) Diisopropylazodicarboxylat (DIAD) hinzugetropft. Das Reaktionsgemisch wurde dann 18 h bei RT gerührt. Danach wurde mit 100 ml Dichlormethan versetzt und nacheinander mit 50 ml Wasser und 50 ml gesättigter Natriumchlorid-Lösung gewaschen. Die organischen Phase wurde über Natriumsulfat getrocknet, filtriert und eingeengt. Der erhaltene Rückstand wurde über eine Kieselgel-Kartusche chromatographiert (Biotage, 50 g Kieselgel, Laufmittel Hexan/Ethylacetat). Es wurden 198 mg (44% d. Th.) der Titelverbindung erhalten.
$^1$H-NMR (400 MHz, DMSO-$d_6$, δ/ppm): 7.96 (s, 1H), 5.95-5.84 (m, 1H), 5.19 (dd, 1H), 5.14-5.02 (m, 2H), 4.99 (s, 2H), 4.58 (t, 1H), 4.46 (t, 1H), 4.22-4.17 (m, 2H), 3.93 (br. t, 2H), 2.45 (s, 3H), 2.10-1.95 (m, 2H), 1.39 (d, 6H).
LC/MS (Methode 1, ESIpos): $R_t$ = 1.10 min, m/z = 422 $[M+H]^+$.

**Beispiel 419**

3-Ethyl-5-methyl-6-[(5-oxo-4,5-dihydro-1*H*-1,2,4-triazol-1-yl)methyl]-1-(tetrahydrofuran-2-yl-methyl)thieno[2,3-d]pyrimidin-2,4(1*H*,3*H*)-dion (*Racemat*)

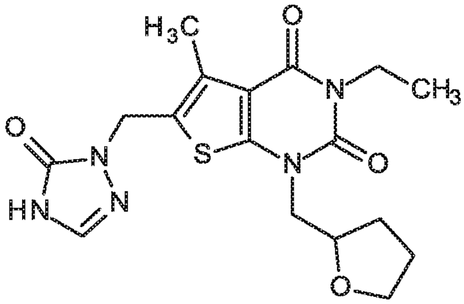

236 mg (0.490 mmol) der Verbindung aus Bsp. 475A wurden in einem Gemisch aus jeweils 12 ml Methanol und Orthoameisensäuretrimethylester gelöst und bei RT mit 490 µl (1.96 mmol) einer 4 M Lösung von Chlorwasserstoff in Dioxan versetzt. Nach 2 h Reaktionszeit wurden weitere 245 µl (0.980 mmol) der 4 M Lösung von Chlorwasserstoff in Dioxan hinzugefügt und nach weiteren 3 h nochmals 490 µl (1.96 mmol). Nach insgesamt ca. 20 h Reaktionszeit wurde das Reaktionsgemisch mit Wasser versetzt und mit Ethylacetat extrahiert. Der organische Extrakt wurde eingeengt und der verbliebene Rückstand mittels präparativer HPLC gereinigt (Methode 8). Nach Eindampfen der Produktfraktionen und Trocknen im Hochvakuum wurden 201 mg (100% d. Th., 96% Reinheit) der Titelverbindung erhalten.
$^1$H-NMR (400 MHz, DMSO-$d_6$, δ/ppm): 11.59 (br. s, 1H), 7.83 (s, 1H), 4.93 (s, 2H), 4.26-4.16 (m, 1H), 4.03 (dd, 1H), 3.90 (q, 2H), 3.78-3.55 (m, 3H), 2.45 (s, 3H), 2.05-1.74 (m, 3H), 1.71-1.60 (m, 1H), 1.11 (t, 3H).
LC/MS (Methode 1, ESIpos): $R_t$ = 0.67 min, m/z = 392 $[M+H]^+$.

## Beispiel 420

3-Ethyl-5-methyl-6-[(5-oxo-4,5-dihydro-1*H*-1,2,4-triazol-1-yl)methyl]-1-(tetrahydrofuran-2-yl-methyl)thieno[2,3-d]pyrimidin-2,4(1*H*,3*H*)-dion (*Enantiomer 1*)

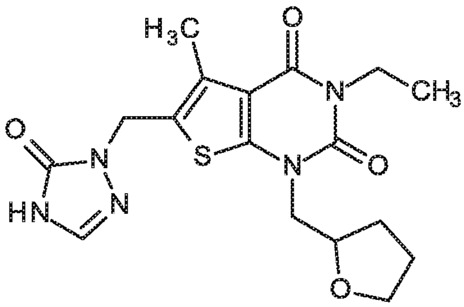

193 mg (0.493 mmol) der racemischen Verbindung aus Bsp. 419 wurden in einem Gemisch aus 8 ml Ethanol und 5 ml Acetonitril gelöst und in 17 Portionen über präparative HPLC an chiraler Phase in die Enantiomere aufgetrennt [Säule: Daicel Chiralcel OX-H, 5 µm, 250 mm x 20 mm; Laufmittel: *n*-Heptan/Ethanol 3:7; Fluss: 15 ml/min; Temperatur: 25°C; Detektion: 210 nm]. Nach Eindampfen der Produktfraktionen und Trocknen des Feststoffs im Hochvakuum wurden 42 mg (43% d. Th.) von Enantiomer 1 erhalten (99.0% ee, chirale analytische HPLC).
$^1$H-NMR (400 MHz, DMSO-$d_6$, δ/ppm): 11.59 (br. s, 1H), 7.83 (s, 1H), 4.92 (s, 2H), 4.27-4.15 (m, 1H), 4.03 (dd, 1H), 3.90 (q, 2H), 3.78-3.55 (m, 3H), 2.45 (s, 3H), 2.04-1.73 (m, 3H), 1.71-1.59 (m, 1H), 1.11 (t, 3H).

Chirale analytische HPLC [Säule: Daicel Chiralpak OX-H, 3 µm, 50 mm x 4.6 mm; Laufmittel: *n*-Heptan/Ethanol 1:1; Fluss: 1 ml/min; Temperatur: 40°C; Detektion: 220 nm]: $R_t$ = 2.24 min.

## Beispiel 421

3-Ethyl-5-methyl-6-[(5-oxo-4,5-dihydro-1*H*-1,2,4-triazol-1-yl)methyl]-1-(tetrahydrofuran-2-yl-methyl)thieno[2,3-d]pyrimidin-2,4(1*H*,3*H*)-dion (*Enantiomer 2*)

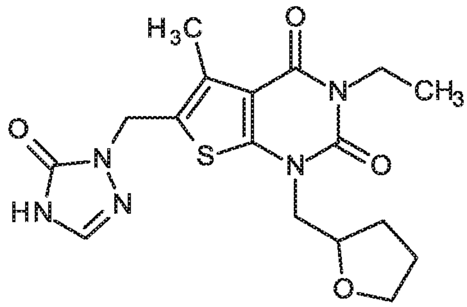

193 mg (0.493 mmol) der racemischen Verbindung aus Bsp. 419 wurden in einem Gemisch aus 8 ml Ethanol und 5 ml Acetonitril gelöst und in 17 Portionen über präparative HPLC an chiraler Phase in die Enantiomere aufgetrennt [Säule: Daicel Chiralcel OX-H, 5 μm, 250 mm x 20 mm; Laufmittel: *n*-Heptan/Ethanol 3:7; Fluss: 15 ml/min; Temperatur: 25°C; Detektion: 210 nm]. Nach Eindampfen der Produktfraktionen und Trocknen des Feststoffs im Hochvakuum wurden 41 mg (42% d. Th.) von Enantiomer 2 erhalten (98.1% ee, chirale analytische HPLC).
$^1$H-NMR (400 MHz, DMSO-$d_6$, δ/ppm): 11.58 (br. s, 1H), 7.83 (s, 1H), 4.92 (s, 2H), 4.28-4.15 (m, 1H), 4.03 (dd, 1H), 3.90 (q, 2H), 3.78-3.56 (m, 3H), 2.45 (s, 3H), 2.05-1.74 (m, 3H), 1.69-1.61 (m, 1H), 1.11 (t, 3H).

Chirale analytische HPLC [Säule: Daicel Chiralpak OX-H, 3 μm, 50 mm x 4.6 mm; Laufmittel: *n*-Heptan/Ethanol 1:1; Fluss: 1 ml/min; Temperatur: 40°C; Detektion: 220 nm]: $R_t$ = 2.84 min.

## Beispiel 422

1-(Fluormethyl)-3-isopropyl-5-methyl-6-[(5-oxo-4,5-dihydro-1*H*-1,2,4-triazol-1-yl)methyl]thieno-[2,3-d]pyrimidin-2,4(1*H*,3*H*)-dion

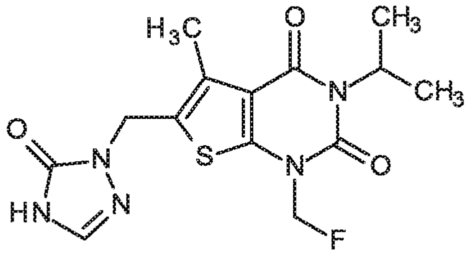

180 mg (0.439 mmol) der Verbindung aus Bsp. 417 wurden in 5 ml 1,4-Dioxan gelöst und mit 70 mg (0.264 mmol) Triphenylphosphin versetzt. Dann wurde diese Lösung mit 35 μl (0.922 mmol) Ameisensäure, 153 μl (1.10 mmol) Triethylamin und 76 mg (0.066 mmol) Tetrakis(triphenylphosphin)palladium(0) versetzt. Das Gemisch wurde 18 h in einem Mikrowellenofen (Biotage Initiator mit dynamischer Steuerung der Einstrahlleistung) auf 125°C erhitzt. Das Reaktionsgemisch wurde danach auf halbgesättigte wässrige Natriumchlorid-Lösung (70 ml) gegeben. Es wurde mit Ethylacetat extrahiert. Die vereinigten organischen Phasen wurden über Natriumsulfat getrocknet, filtriert und eingeengt. Der Rückstand wurde in 6 ml DMSO gelöst und diese Lösung mittels präparativer HPLC gereinigt (Methode 14). Nach Vereinigen der Produktfraktionen und Gefriertrocknung wurden 49 mg (31% d. Th.) der Titelverbindung erhalten.
$^1$H-NMR (400 MHz, DMSO-$d_6$, δ/ppm): 11.65 (br. s, 1H), 7.85 (s, 1H), 5.97 (d, 2H), 5.11 (quin, 1H), 4.96 (s, 2H), 2.45 (s, 3H), 1.40 (d, 6H).
LC/MS (Methode 1, ESIpos): $R_t$ = 0.93 min, m/z = 354 $[M+H]^+$.

## Beispiel 423

1-(3-Fluorpropyl)-3-isopropyl-5-methyl-6-[(5-oxo-4,5-dibydro-1*H*-1,2,4-triazol-1-yl)methyl]-thieno[2,3-d]pyrimidin-2,4(1*H*,3*H*)-dion

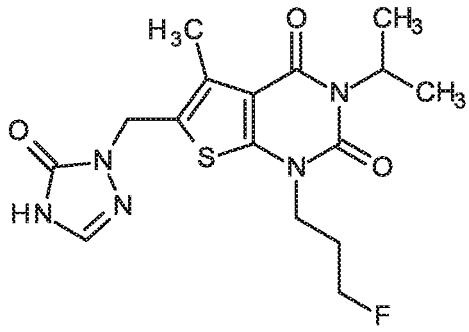

188 mg (0.419 mmol) der Verbindung aus Bsp. 418 wurden in 7.5 ml 1,4-Dioxan gelöst und mit 66 mg (0.252 mmol) Triphenylphosphin versetzt. Dann wurde diese Lösung mit 33 µl (0.88 mmol) Ameisensäure, 146 µl (1.05 mmol) Triethylamin und 73 mg (0.063 mmol) Tetrakis(triphenylphosphin)palladium(0) versetzt. Das Gemisch wurde 120 h in einem Mikrowellenofen (Biotage Initiator mit dynamischer Steuerung der Einstrahlleistung) auf 120°C erhitzt. Das Reaktionsgemisch wurde danach auf halbgesättigte wässrige Natriumchlorid-Lösung (70 ml) gegeben. Es wurde mit Ethylacetat extrahiert. Die vereinigten organischen Phasen wurden über Natriumsulfat getrocknet, filtriert und eingeengt. Der Rückstand wurde in 3 ml DMSO gelöst und diese Lösung mittels präparativer HPLC gereinigt (Methode 14). Nach Vereinigen der Produktfraktionen und Gefriertrocknung wurden 85 mg (45% d. Th.) der Titelverbindung erhalten.
$^1$H-NMR (400 MHz, DMSO-$d_6$, δ/ppm): 11.61 (br. s, 1H), 7.84 (s, 1H), 5.12 (sept, 1H), 4.93 (s, 2H), 4.58 (t, 1H), 4.46 (t, 1H), 3.93 (t, 2H), 2.44 (s, 3H), 2.10-1.95 (m, 2H), 1.39 (d, 6H).
LC/MS (Methode 1, ESIpos): $R_t$ = 0.95 min, m/z = 382 [M+H]$^+$.

## Beispiel 424

3-Isopropyl-5-methyl-6-[(5-oxo-4,5-dihydro-1*H*-1,2,4-triazol-1-yl)methyl]-1-(tetrahydrofuran-2-yl-methyl)thieno[2,3-d]pyrimidin-2,4(1*H*,3*H*)-dion (*Racemat*)

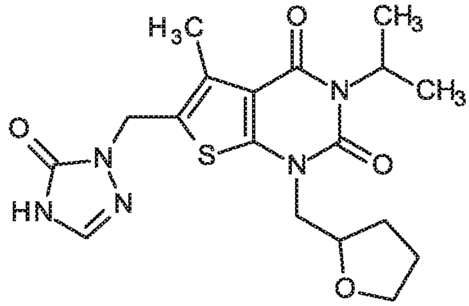

Analog zu dem unter Bsp. 419 beschriebenen Verfahren wurden aus 366 mg (0.613 mmol, 83% Reinheit) der Verbindung aus Bsp. 476A und 16.6 ml (151 mmol) Orthoameisensäuretrimethylester 146 mg (58% d. Th.) der Titelverbindung hergestellt.
$^1$H-NMR (400 MHz, DMSO-$d_6$, δ/ppm): 11.59 (br. s, 1H), 7.83 (s, 1H), 5.13 (sept, 1H), 4.91 (s, 2H), 4.25-4.14 (m, 1H), 4.02 (dd, 1H), 3.78-3.69 (m, 1H), 3.67-3.56 (m, 2H), 2.44 (s, 3H), 2.03-1.75 (m, 3H), 1.70-1.59 (m, 1H), 1.39 (dd, 6H).
LC/MS (Methode 17, ESIpos): $R_t$ = 1.40 min, m/z = 406.15 [M+H]$^+$.

## Beispiel 425

3-Isopropyl-5-methyl-6-[(5-oxo-4,5-dihydro-1*H*-1,2,4-triazol-1-yl)methyl]-1-(tetrahydrofuran-2-yl-methyl)thieno[2,3-d]pyrimidin-2,4(1*H*,3*H*)-dion (*Enantiomer 1*)

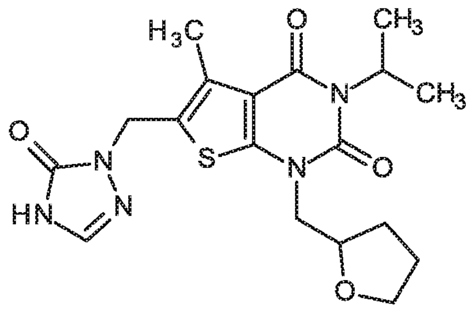

139 mg (0.343 mmol) der racemischen Verbindung aus Bsp. 424 wurden in einem Gemisch aus 8 ml Ethanol und 4 ml Acetonitril gelöst und in 24 Portionen über präparative HPLC an chiraler Phase in die Enantiomere aufgetrennt [Säule: Daicel Chiralcel OX-H, 5 μm, 250 mm x 20 mm; Laufmittel: *n*-Heptan/Ethanol 3:7; Fluss: 15 ml/min; Temperatur: 25°C; Detektion: 210 nm]. Nach Eindampfen der Produktfraktionen und Trocknen des Feststoffs im Hochvakuum wurden 68 mg (97% d. Th.) von Enantiomer 1 erhalten (99.0% ee, chirale analytische HPLC).
$^1$H-NMR (400 MHz, DMSO-$d_6$, δ/ppm): 11.58 (br. s, 1H), 7.83 (s, 1H), 5.13 (sept, 1H), 4.91 (s, 2H), 4.26-4.13 (m, 1H), 4.02 (dd, 1H), 3.79-3.69 (m, 1H), 3.67-3.55 (m, 2H), 2.44 (s, 3H), 2.03-1.74 (m, 3H), 1.71-1.57 (m, 1H), 1.39 (dd, 6H).
Chirale analytische HPLC [Säule: Daicel Chiraltek OX-H, 3 μm, 50 mm x 4.6 mm; Laufmittel: Isohexan/Ethanol 1:1; Fluss: 1 ml/min; Temperatur: 40°C; Detektion: 220 nm]: $R_t$ = 3.74 min.

**Beispiel 426**

3-Isopropyl-5-methyl-6-[(5-oxo-4,5-dihydro-1*H*-1,2,4-triazol-1-yl)methyl]-1-(tetrahydrofuran-2-yl-methyl)thieno[2,3-d]pyrimidin-2,4(1*H*,3*H*)-dion (*Enantiomer 2*)

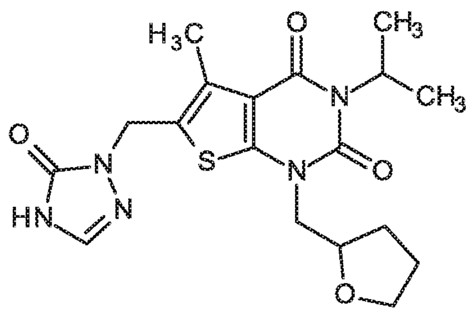

139 mg (0.343 mmol) der racemischen Verbindung aus Bsp. 424 wurden in einem Gemisch aus 8 ml Ethanol und 4 ml Acetonitril gelöst und in 24 Portionen über präparative HPLC an chiraler Phase in die Enantiomere aufgetrennt [Säule: Daicel Chiralcel OX-H, 5 μm, 250 mm x 20 mm; Laufmittel: *n*-Heptan/Ethanol 3:7; Fluss: 15 ml/min; Temperatur: 25°C; Detektion: 210 nm]. Nach Eindampfen der Produktfraktionen und Trocknen des Feststoffs im Hochvakuum wurden 67 mg (96% d. Th.) von Enantiomer 2 erhalten (98.8% ee, chirale analytische HPLC).
$^1$H-NMR (400 MHz, DMSO-$d_6$, δ/ppm): 11.58 (br. s, 1H), 7.83 (s, 1H), 5.13 (sept, 1H), 4.91 (s, 2H), 4.25-4.14 (m, 1H), 4.02 (dd, 1H), 3.79-3.69 (m, 1H), 3.67-3.55 (m, 2H), 2.44 (s, 3H), 2.03-1.75 (m, 3H), 1.71-1.58 (m, 1H), 1.39 (dd, 6H).
Chirale analytische HPLC [Säule: Daicel Chiraltek OX-H, 3 μm, 50 mm x 4.6 mm; Laufmittel: Isohexan/Ethanol 1:1; Fluss: 1 ml/min; Temperatur: 40°C; Detektion: 220 nm]: $R_t$ = 5.16 min.

**Beispiel 427**

3-(2,2-Dimethylpropyl)-5-methyl-6-[(5-oxo-4,5-dihydro-1*H*-1,2,4-triazol-1-yl)methyl]-1-(3,3,3-tri-fluorpropyl)thieno[2,3-d]pyrimidin-2,4(1*H*,3*H*)-dion

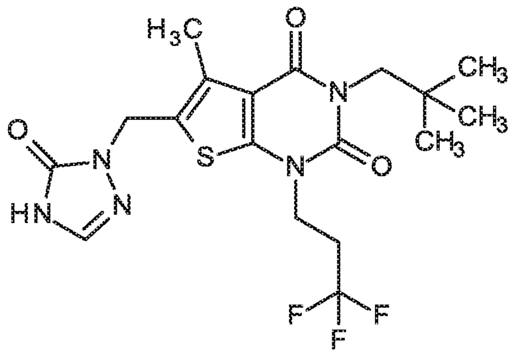

Analog zu dem unter Bsp. 419 beschriebenen Verfahren wurden aus 154 mg (0.262 mmol, 91% Reinheit) der Verbindung aus Bsp. 477A und 6.4 ml (62.6 mmol) Orthoameisensäuretrimethylester 95 mg (81% d. Th.) der Titelverbindung hergestellt.
$^1$H-NMR (500 MHz, DMSO-$d_6$, $\delta$/ppm): 11.61 (br. s, 1H), 7.84 (s, 1H), 4.95 (s, 2H), 4.08 (t, 2H), 3.81 (br. s, 2H), 2.83-2.67 (m, 2H), 2.45 (s, 3H), 0.89 (s, 9H).
LC/MS (Methode 17, ESIpos): $R_t$ = 1.81 min, m/z = 446.15 $[M+H]^+$.

**Beispiel 428**

3-(2,2-Dimethylpropyl)-1-(2-methoxyethyl)-5-methyl-6-[(5-oxo-4,5-dihydro-1*H*-1,2,4-triazol-1-yl)-methyl]thieno[2,3-d]pyrimidin-2,4(1*H*,3*H*)-dion

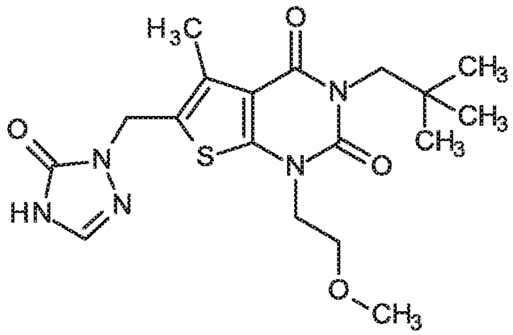

Analog zu dem unter Bsp. 419 beschriebenen Verfahren wurden aus 120 mg (0.222 mmol, 92% Reinheit) der Verbindung aus Bsp. 478A und 5.3 ml (53.1 mmol) Orthoameisensäuretrimethylester 35 mg (38% d. Th.) der Titelverbindung erhalten.
$^1$H-NMR (400 MHz, DMSO-$d_6$, $\delta$/ppm): 11.60 (br. s, 1H), 7.84 (s, 1H), 4.93 (s, 2H), 4.00 (t, 2H), 3.81 (s, 2H), 3.61 (t, 2H), 3.22 (s, 3H), 2.44 (s, 3H), 0.88 (s, 9H).
LC/MS (Methode 17, ESIpos): $R_t$ = 1.52 min, m/z = 408.17 $[M+H]^+$.

**Beispiel 429**

3-(2,2-Dimethylpropyl)-5-methyl-6-[(5-oxo-4,5-dihydro-1*H*-1,2,4-triazol-1-yl)methyl]-1-(tetrahydrofuran-2-ylmethyl)thieno[2,3-d]pyrimidin-2,4(1*H*,3*H*)-dion (*Racemat*)

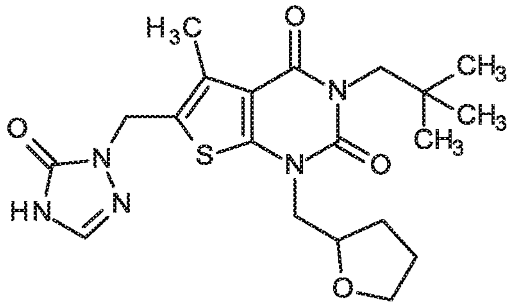

Analog zu dem unter Bsp. 419 beschriebenen Verfahren wurden aus 530 mg (0.779 mmol, 77% Reinheit) der Verbindung aus Bsp. 479A und 20 ml (186 mmol) Orthoameisensäuretrimethylester 124 mg (36% d. Th.) der Titelverbindung erhalten.
$^1$H-NMR (400 MHz, DMSO-$d_6$, δ/ppm): 11.60 (br. s, 1H), 7.84 (s, 1H), 4.93 (s, 2H), 4.26-4.15 (m, 1H), 4.01 (dd, 1H), 3.81 (br. s, 2H), 3.76-3.65 (m, 2H), 3.64-3.55 (m, 1H), 2.44 (s, 3H), 2.03-1.74 (m, 3H), 1.71-1.59 (m, 1H), 0.88 (s, 9H).
LC/MS (Methode 17, ESIpos): $R_t$ = 1.64 min, m/z = 434.19 $[M+H]^+$.

**Beispiel 430**

3-(2,2-Dimethylpropyl)-5-methyl-6-[(5-oxo-4,5-dihydro-1*H*-1,2,4-triazol-1-yl)methyl]-1-(tetrahydrofuran-2-ylmethyl)thieno[2,3-d]pyrimidin-2,4(1*H*,3*H*)-dion (*Enantiomer 1*)

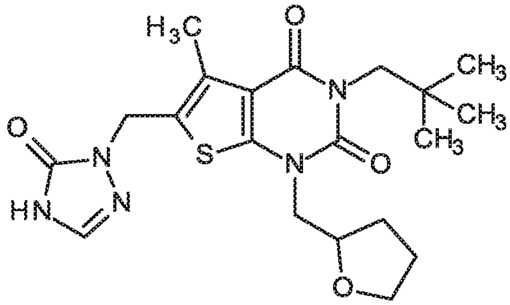

120 mg (0.277 mmol) der racemischen Verbindung aus Bsp. 429 wurden in 7 ml Ethanol gelöst und in 14 Portionen über präparative HPLC an chiraler Phase in die Enantiomere aufgetrennt [Säule: Daicel Chiralpak AZ-H, 5 μm, 250 mm x 20 mm; Laufmittel: *n*-Heptan/Ethanol 1:1; Fluss: 15 ml/min; Temperatur: 40°C; Detektion: 210 nm]. Nach Eindampfen der Produktfraktionen und Trocknen des Feststoffs im Hochvakuum wurden 54 mg (90% d. Th.) von Enantiomer 1 erhalten (99.0% ee, chirale analytische HPLC).
$^1$H-NMR (400 MHz, DMSO-$d_6$, δ/ppm): 11.60 (br. s, 1H), 7.83 (s, 1H), 4.92 (s, 2H), 4.26-4.15 (m, 1H), 4.01 (dd, 1H), 3.81 (br. s, 2H), 3.76-3.65 (m, 2H), 3.64-3.55 (m, 1H), 2.44 (s, 3H), 2.04-1.74 (m, 3H), 1.71-1.59 (m, 1H), 0.88 (s, 9H).
Chirale analytische HPLC [Säule: Daicel Chiralpak AZ-H, 3 μm, 50 mm x 4.6 mm; Laufmittel: Isohexan/Ethanol 1:1; Fluss: 1 ml/min; Temperatur: 40°C; Detektion: 220 nm]: $R_t$ = 1.83 min.

**Beispiel 431**

3-(2,2-Dimethylpropyl)-5-methyl-6-[(5-oxo-4,5-dihydro-1*H*-1,2,4-triazol-1-yl)methyl]-1-(tetrahydrofuran-2-ylmethyl)thieno[2,3-d]pyrimidin-2,4(1*H*,3*H*)-dion (*Enantiomer 2*)

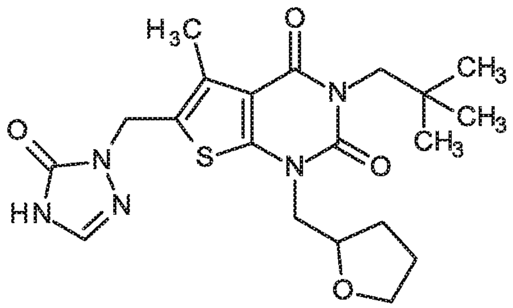

120 mg (0.277 mmol) der racemischen Verbindung aus Bsp. 429 wurden in 7 ml Ethanol gelöst und in 14 Portionen über präparative HPLC an chiraler Phase in die Enantiomere aufgetrennt [Säule: Daicel Chiralpak AZ-H, 5 µm, 250 mm x 20 mm; Laufmittel: *n*-Heptan/Ethanol 1:1; Fluss: 15 ml/min; Temperatur: 40°C; Detektion: 210 nm]. Nach Eindampfen der Produktfraktionen und Trocknen des Feststoffs im Hochvakuum wurden 54 mg (90% d. Th.) von Enantiomer 2 erhalten (99.0% ee, chirale analytische HPLC).
$^1$H-NMR (400 MHz, DMSO-$d_6$, δ/ppm): 11.59 (br. s, 1H), 7.82 (s, 1H), 4.92 (s, 2H), 4.25-4.15 (m, 1H), 4.01 (dd, 1H), 3.81 (br. s, 2H), 3.76-3.65 (m, 2H), 3.64-3.55 (m, 1H), 2.44 (s, 3H), 2.03-1.74 (m, 3H), 1.72-1.60 (m, 1H), 0.88 (s, 9H).
Chirale analytische HPLC [Säule: Daicel Chiralpak AZ-H, 3 µm, 50 mm x 4.6 mm; Laufmittel: Isohexan/Ethanol 1:1; Fluss: 1 ml/min; Temperatur: 40°C; Detektion: 220 nm]: $R_t$ = 2.72 min.

**Beispiel 432**

5-Methyl-6-[(5-oxo-4,5-dihydro-1*H*-1,2,4-triazol-1-yl)methyl]-3-(2,2,2-trifluorethyl)-1-(3,3,3-tri-fluorpropyl)thieno[2,3-d]pyrimidin-2,4(1*H*,3*H*)-dion

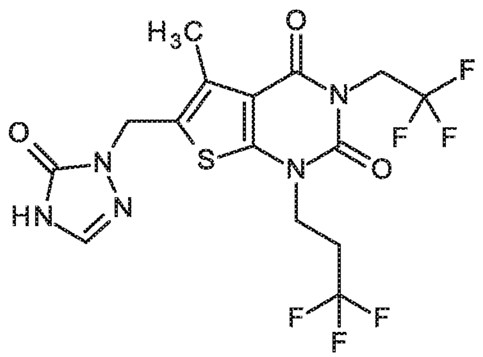

Analog zu dem unter Bsp. 419 beschriebenen Verfahren wurden aus 169 mg (0.306 mmol) der Verbindung aus Bsp. 480A und 8 ml (73.1 mmol) Orthoameisensäuretrimethylester 88 mg (62% d. Th.) der Titelverbindung erhalten.
$^1$H-NMR (400 MHz, DMSO-$d_6$, δ/ppm): 11.62 (br. s, 1H), 7.85 (s, 1H), 4.98 (s, 2H), 4.70 (q, 2H), 4.12 (t, 2H), 2.85-2.68 (m, 2H), 2.46 (s, 3H).
LC/MS (Methode 1, ESIpos): $R_t$ = 0.83 min, m/z = 458 $[M+H]^+$.

**Beispiel 433**

1-(2-Methoxyethyl)-5-methyl-6-[(5-oxo-4,5-dihydro-1*H*-1,2,4-triazol-1-yl)methyl]-3-(2,2,2-trifluorethyl)thieno[2,3-d]pyrimidin-2,4(1*H*,3*H*)-dion

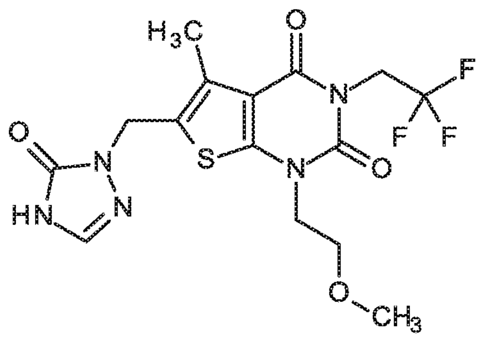

Analog zu dem unter Bsp. 419 beschriebenen Verfahren wurden aus 180 mg (0.332 mmol, 94% Reinheit) der Verbindung aus Bsp. 481A und 8 ml (73.1 mmol) Orthoameisensäuretrimethylester 87 mg (62% d. Th.) der Titelverbindung erhalten.
$^1$H-NMR (500 MHz, DMSO-$d_6$, δ/ppm): 11.63 (br. s, 1H), 7.85 (s, 1H), 4.96 (s, 2H), 4.70 (q, 2H), 4.05 (t, 2H), 3.63 (t, 2H), 3.23 (s, 3H), 2.45 (s, 3H).
LC/MS (Methode 1, ESIpos): $R_t$ = 0.76 min, m/z = 420 [M+H]$^+$.

**Beispiel 434**

5-Methyl-6-[(5-oxo-4,5-dihydro-1*H*-1,2,4-triazol-1-yl)methyl]-1-(tetrahydrofuran-2-ylmethyl)-3-(2,2,2-trifluorethyl)thieno[2,3-d]pyrimidin-2,4(1*H*,3*H*)-dion (*Racemat*)

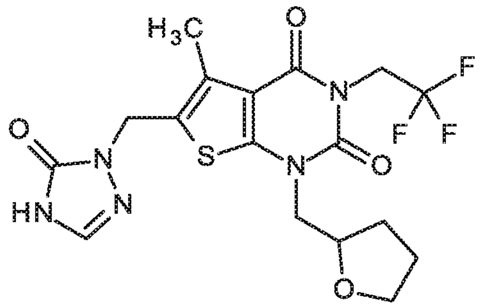

Analog zu dem unter Bsp. 419 beschriebenen Verfahren wurden aus 400 mg (0.739 mmol) der Verbindung aus Bsp. 482A und 20 ml (183 mmol) Orthoameisensäuretrimethylester 283 mg (85% d. Th.) der Titelverbindung erhalten.
$^1$H-NMR (400 MHz, DMSO-$d_6$, δ/ppm): 11.61 (br. s, 1H), 7.84 (s, 1H), 4.95 (s, 2H), 4.69 (q, 2H), 4.25-4.15 (m, 1H), 4.06 (dd, 1H), 3.78-3.68 (m, 2H), 3.65-3.56 (m, 1H), 2.45 (s, 3H), 2.04-1.75 (m, 3H), 1.72-1.61 (m, 1H).
LC/MS (Methode 1, ESIpos): $R_t$ = 0.76 min, m/z = 446 [M+H]$^+$.

**Beispiel 435**

5-Methyl-6-[(5-oxo-4,5-dihydro-1*H*-1,2,4-triazol-1-yl)methyl]-1-(tetrahydrofuran-2-ylmethyl)-3-(2,2,2-trifluorethyl)thieno[2,3-d]pyrimidin-2,4(1*H*,3*H*)-dion (*Enantiomer 1*)

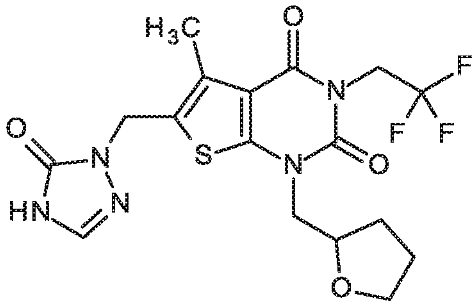

261 mg (0.586 mmol) der racemischen Verbindung aus Bsp. 434 wurden in einem Gemisch aus 5 ml Methanol und 10 ml Acetonitril gelöst und in 15 Portionen über präparative HPLC an chiraler Phase in die Enantiomere aufgetrennt [Säule: Daicel Chiralpak AZ-H, 5 μm, 250 mm x 20 mm; Laufmittel: *n*-Heptan/Ethanol 1:3; Fluss: 15 ml/min; Temperatur: 25°C; Detektion: 220 nm]. Nach Eindampfen der Produktfraktionen und Trocknen des Feststoffs im Hochvakuum wurden 86 mg (65% d. Th.) von Enantiomer 1 erhalten (99.4% ee, chirale analytische HPLC).
$^1$H-NMR (400 MHz, DMSO-$d_6$, δ/ppm): 11.61 (br. s, 1H), 7.84 (s, 1H), 4.95 (s, 2H), 4.70 (q, 2H), 4.26-4.15 (m, 1H), 4.06 (dd, 1H), 3.78-3.68 (m, 2H), 3.65-3.55 (m, 1H), 2.45 (s, 3H), 2.05-1.75 (m, 3H), 1.71-1.60 (m, 1H).
Chirale analytische HPLC [Säule: Daicel Chiraltek AZ-3, 3 μm, 50 mm x 4.6 mm; Laufmittel: Isohexan/Ethanol 1:1; Fluss: 1 ml/min; Temperatur: 40°C; Detektion: 220 nm]: $R_t$ = 3.28 min.

**Beispiel 436**

5-Methyl-6-[(5-oxo-4,5-dihydro-1H-1,2,4-triazol-1-yl)methyl]-1-(tetrahydrofuran-2-ylmethyl)-3-(2,2,2-trifluorethyl)thieno[2,3-d]pyrimidin-2,4(1*H*,3*H*)-dion (*Enantiomer 2*)

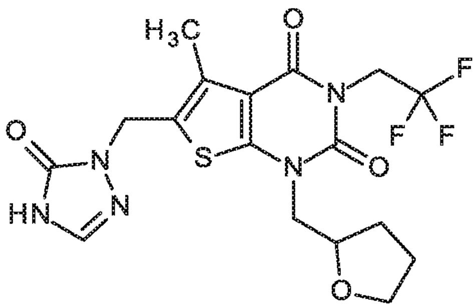

261 mg (0.586 mmol) der racemischen Verbindung aus Bsp. 434 wurden in einem Gemisch aus 5 ml Methanol und 10 ml Acetonitril gelöst und in 15 Portionen über präparative HPLC an chiraler Phase in die Enantiomere aufgetrennt [Säule: Daicel Chiralpak AZ-H, 5 μm, 250 mm x 20 mm; Laufmittel: *n*-Heptan/Ethanol 1:3; Fluss: 15 ml/min; Temperatur: 25°C; Detektion: 220 nm]. Nach Eindampfen der Produktfraktionen und Trocknen des Feststoffs im Hochvakuum wurden 110 mg (84% d. Th.) von Enantiomer 2 erhalten (99.4% ee, chirale analytische HPLC).
$^1$H-NMR (400 MHz, DMSO-$d_6$, δ/ppm): 11.61 (br. s, 1H), 7.84 (s, 1H), 4.95 (s, 2H), 4.70 (q, 2H), 4.25-4.16 (m, 1H), 4.06 (dd, 1H), 3.77-3.68 (m, 2H), 3.65-3.56 (m, 1H), 2.45 (s, 3H), 2.04-1.75 (m, 3H), 1.72-1.60 (m, 1H).
Chirale analytische HPLC [Säule: Daicel Chiraltek AZ-3, 3 μm, 50 mm x 4.6 mm; Laufmittel: Isohexan/Ethanol 1:1; Fluss: 1 ml/min; Temperatur: 40°C; Detektion: 220 nm]: $R_t$ = 5.29 min.

**Beispiel 437**

5-Methyl-6-[(5-oxo-4,5-dihydro-1*H*-1,2,4-triazol-1-yl)methyl]-3-(1,1,1-trifluorpropan-2-yl)-1-(3,3,3-trifluorpropyl)thieno[2,3-d]pyrimidin-2,4(1*H*,3*H*)-dion (*Racemat*)

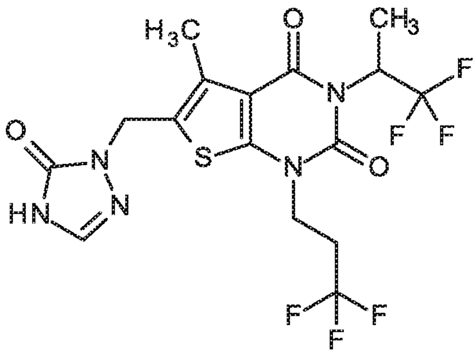

Analog zu dem unter Bsp. 419 beschriebenen Verfahren wurden aus 390 mg (0.695 mmol) der Verbindung aus Bsp. 483A und 20 ml (183 mmol) Orthoameisensäuretrimethylester 160 mg (48% d. Th.) der Titelverbindung erhalten. $^1$H-NMR (400 MHz, DMSO-$d_6$, δ/ppm): 11.61 (s, 1H), 7.85 (s, 1H), 5.82-5.45 (m, 1H), 4.97 (s, 2H), 4.18-4.00 (m, 2H), 2.85-2.69 (m, 2H), 2.46 (s, 3H), 1.63 (br. d, 3H).
LC/MS (Methode 17, ESIpos): $R_t$ = 1.69 min, m/z = 472.09 $[M+H]^+$.

## Beispiel 438

1-(2-Methoxyethyl)-5-methyl-6-[(5-oxo-4,5-dihydro-1*H*-1,2,4-triazol-1-yl)methyl]-3-(1,1,1-tri-fluorpropan-2-yl)thieno[2,3-d]pyrimidin-2,4(1*H*,3*H*)-dion (*Racemat*)

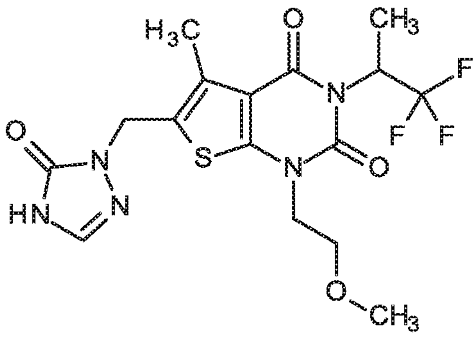

Analog zu dem unter Bsp. 419 beschriebenen Verfahren wurden aus 405 mg (0.774 mmol) der Verbindung aus Bsp. 484A und 20 ml (183 mmol) Orthoameisensäuretrimethylester zwei Fraktionen der Titelverbindung erhalten: 37 mg (11% d. Th., 99% Reinheit) und 208 mg (55% d. Th., 90% Reinheit).
$^1$H-NMR (400 MHz, DMSO-$d_6$, δ/ppm): 11.60 (br. s, 1H), 7.84 (s, 1H), 5.83-5.47 (m, 1H), 4.94 (s, 2H), 4.09-3.93 (m, 2H), 3.69-3.55 (m, 2H), 3.23 (s, 3H), 2.44 (s, 3H), 1.63 (br. d, 3H).
LC/MS (Methode 17, ESIpos): $R_t$ = 1.44 min, m/z = 434.11 $[M+H]^+$.

## Beispiel 439

3-(2-Methoxyethyl)-5-methyl-6-[(5-oxo-4,5-dihydro-1*H*-1,2,4-triazol-1-yl)methyl]-1-(3,3,3-tri-fluorpropyl)thieno[2,3-d]pyrimidin-2,4(1*H*,3*H*)-dion

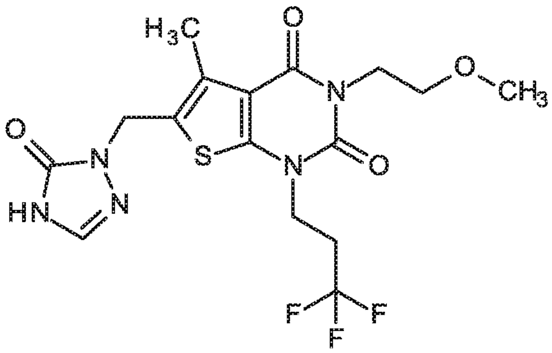

Analog zu dem unter Bsp. 419 beschriebenen Verfahren wurden aus 185 mg (0.343 mmol, 97% Reinheit) der Verbindung aus Bsp. 485A und 10 ml (91.4 mmol) Orthoameisensäuretrimethylester 115 mg (77% d. Th.) der Titelverbindung erhalten.
$^1$H-NMR (400 MHz, DMSO-$d_6$, δ/ppm): 11.61 (br. s, 1H), 7.84 (s, 1H), 4.96 (s, 2H), 4.09 (t, 2H), 4.05 (t, 2H), 3.49 (t, 2H), 3.23 (s, 3H), 2.83-2.65 (m, 2H), 2.46 (s, 3H).
LC/MS (Methode 1, ESIpos): $R_t$ = 0.71 min, m/z = 434 $[M+H]^+$.

## Beispiel 440

3-(2-Methoxypropyl)-5-methyl-6-[(5-oxo-4,5-dihydro-1H-1,2,4-triazol-1-yl)methyl]-1-(3,3,3-tri-fluorpropyl)thieno[2,3-d]pyrimidin-2,4(1*H*,3*H*)-dion (*Racemat*)

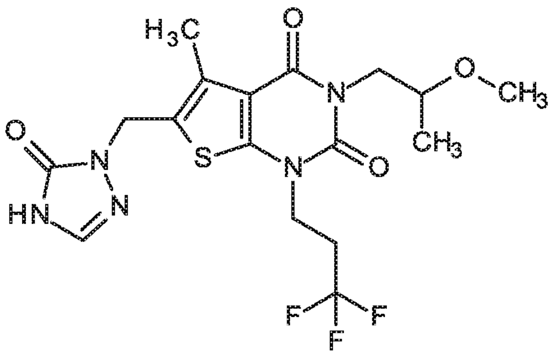

340 mg (0.626 mmol) der Verbindung aus Bsp. 486A wurden in einem Gemisch aus jeweils 17 ml Methanol und Orthoameisensäuretrimethylester gelöst und bei RT mit 626 μl (2.51 mmol) einer 4 M Lösung von Chlorwasserstoff in Dioxan versetzt. Nach ca. 16 h Reaktionszeit wurden weitere 626 μl (2.51 mmol) der 4 M Lösung von Chlorwasserstoff in Dioxan hinzugefügt und nach weiteren 2 h nochmals die gleiche Menge. Nach insgesamt ca. 24 h Reaktionszeit wurde das Reaktionsgemisch mit Wasser versetzt und mit Ethylacetat extrahiert. Der organische Extrakt wurde eingeengt und der verbliebene Rückstand mittels präparativer HPLC gereinigt (Methode 8). Nach Eindampfen der Produktfraktionen und Trocknen im Hochvakuum wurden 222 mg (79% d. Th.) der Titelverbindung erhalten.
$^1$H-NMR (400 MHz, DMSO-$d_6$, δ/ppm): 11.61 (br. s, 1H), 7.84 (s, 1H), 4.96 (s, 2H), 4.17-4.03 (m, 2H), 4.04 (dd, 1H), 3.76 (dd, 1H), 3.62 (sext, 1H), 3.21 (s, 3H), 2.83-2.66 (m, 2H), 2.46 (s, 3H), 1.04 (d, 3H).
LC/MS (Methode 1, ESIpos): $R_t$ = 0.75 min, m/z = 448 $[M+H]^+$.

## Beispiel 441

3-(2-Methoxypropyl)-5-methyl-6-[(5-oxo-4,5-dihydro-1*H*-1,2,4-triazol-1-yl)methyl]-1-(3,3,3-tri-fluorpropyl)thieno[2,3-d]pyrimidin-2,4(1*H*,3*H*)-dion (*Enantiomer 1*)

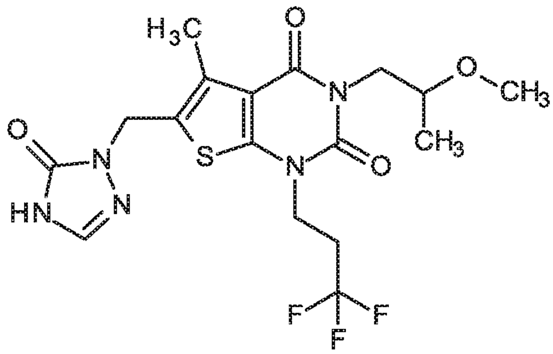

202 mg (0.451 mmol) der racemischen Verbindung aus Bsp. 440 wurden in 6 ml Ethanol gelöst und in 24 Portionen über präparative HPLC an chiraler Phase in die Enantiomere aufgetrennt [Säule: Daicel Chiralcel OX-H, 5 µm, 250 mm x 20 mm; Laufmittel: *n*-Heptan/Ethanol 1:1; Fluss: 15 ml/min; Temperatur: 40°C; Detektion: 220 nm]. Nach Eindampfen der Produktfraktionen und Trocknen des Feststoffs im Hochvakuum wurden 78 mg (77% d. Th.) von Enantiomer 1 erhalten (99.0% ee, chirale analytische HPLC).

$^1$H-NMR (400 MHz, DMSO-$d_6$, δ/ppm): 11.59 (br. s, 1H), 7.84 (s, 1H), 4.96 (s, 2H), 4.13-4.05 (m, 2H), 4.04 (dd, 1H), 3.76 (dd, 1H), 3.62 (sext, 1H), 3.21 (s, 3H), 2.82-2.67 (m, 2H), 2.46 (s, 3H), 1.04 (d, 3H).

Chirale analytische HPLC [Säule: Daicel Chiralcel OX-H, 5 µm, 250 mm x 4.6 mm; Laufmittel: Isohexan/Ethanol 1:1; Fluss: 1 ml/min; Temperatur: 40°C; Detektion: 220 nm]: $R_t$ = 6.26 min.

## Beispiel 442

3-(2-Methoxypropyl)-5-methyl-6-[(5-oxo-4,5-dihydro-1*H*-1,2,4-triazol-1-yl)methyl]-1-(3,3,3-tri-fluorpropyl)thieno[2,3-d]pyrimidin-2,4(1*H*,3*H*)-dion (*Enantiomer 2*)

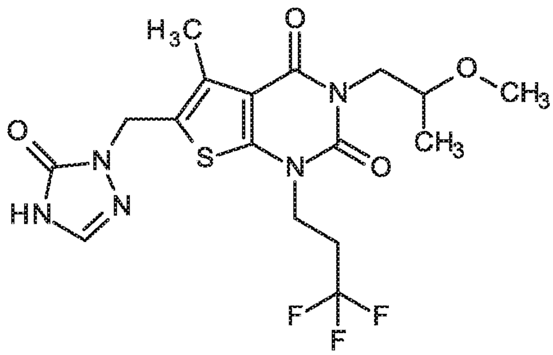

202 mg (0.451 mmol) der racemischen Verbindung aus Bsp. 440 wurden in 6 ml Ethanol gelöst und in 24 Portionen über präparative HPLC an chiraler Phase in die Enantiomere aufgetrennt [Säule: Daicel Chiralcel OX-H, 5 µm, 250 mm x 20 mm; Laufmittel: *n*-Heptan/Ethanol 1:1; Fluss: 15 ml/min; Temperatur: 40°C; Detektion: 220 nm]. Nach Eindampfen der Produktfraktionen und Trocknen des Feststoffs im Hochvakuum wurden 92 mg (91% d. Th.) von Enantiomer 2 erhalten (99.0% ee, chirale analytische HPLC).

$^1$H-NMR (400 MHz, DMSO-$d_6$, δ/ppm): 11.60 (br. s, 1H), 7.84 (s, 1H), 4.96 (s, 2H), 4.13-4.04 (m, 2H), 4.04 (dd, 1H), 3.76 (dd, 1H), 3.62 (sext, 1H), 3.21 (s, 3H), 2.82-2.67 (m, 2H), 2.46 (s, 3H), 1.04 (d, 3H).

Chirale analytische HPLC [Säule: Daicel Chiralcel OX-H, 5 µm, 250 mm x 4.6 mm; Laufmittel: Isohexan/Ethanol 1:1; Fluss: 1 ml/min; Temperatur: 40°C; Detektion: 220 nm]: $R_t$ = 7.94 min.

**Beispiel 443**

[1-{[3-(2,2-Dimethylpropyl)-5-methyl-2,4-dioxo-1-(3,3,3-trifluorpropyl)-1,2,3,4-tetrahydrothieno-[2,3-d]pyrimidin-6-yl]methyl}imidazolidin-2-yliden]cyanamid

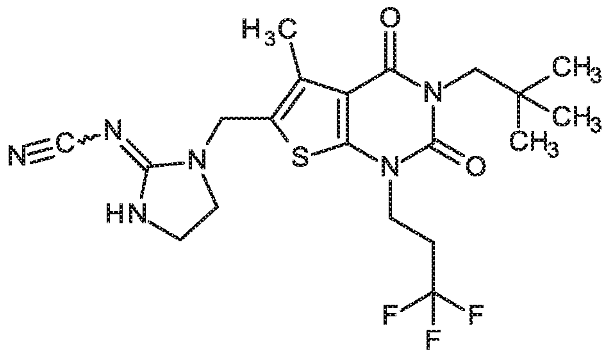

163 mg (0.12 mmol, 31% Reinheit) der Verbindung aus Bsp. 315A wurden in 5 ml DMF gelöst und mit 28 mg (0.18 mmol) Dimethyl-*N*-cyanodithioiminocarbonat und 33 mg (0.24 mmol) Kaliumcarbonat versetzt. Das Gemisch wurde 18 h bei 80°C gerührt. Danach wurde das Reaktionsgemisch in 30 ml Ethylacetat aufgenommen. Es wurde mit 20 ml gesättigter Natriumhydrogencarbonat-Lösung und mit 20 ml Wasser gewaschen. Die organische Phase wurde über Natriumsulfat getrocknet, filtriert und eingeengt. Der erhaltene Rückstand wurde in 3 ml DMSO gelöst und diese Lösung mittels präparativer HPLC gereinigt (Methode 14). Nach Vereinigen der Produktfraktionen und Gefriertrocknung wurden 15 mg (24% d. Th.) der Titelverbindung erhalten.
$^1$H-NMR (400 MHz, DMSO-$d_6$, $\delta$/ppm): 8.10 (s, 1H), 4.50 (s, 2H), 4.11 (br. t, 2H), 3.81 (br. s, 2H), 3.52-3.38 (m, 4H), 2.83-2.69 (m, 2H), 2.40 (s, 3H), 0.89 (s, 9H).
LC/MS (Methode 1, ESIpos): $R_t$ = 1.29 min, m/z = 471 [M+H]$^+$.

**Beispiel 444**

[1-{[3-(2,2-Dimethylpropyl)-1-(3-fluorpropyl)-5-methyl-2,4-dioxo-1,2,3,4-tetrahydrothieno[2,3-d]-pyrimidin-6-yl]methyl}imidazolidin-2-yliden]cyanamid

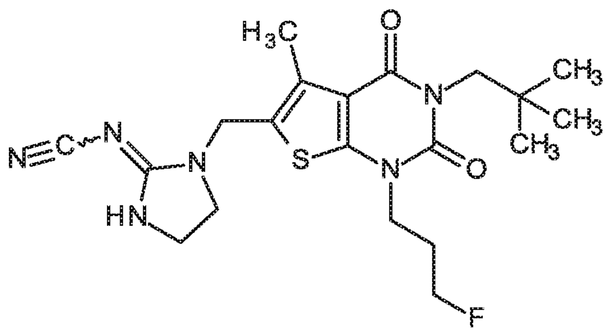

117 mg (0.225 mmol, 74% Reinheit) der Verbindung aus Bsp. 316A wurden in 5 ml DMF gelöst und mit 52 mg (0.338 mmol) Dimethyl-*N*-cyanodithioiminocarbonat und 62 mg (0.45 mmol) Kaliumcarbonat versetzt. Das Gemisch wurde 19 h bei 80°C gerührt. Danach wurde das Reaktionsgemisch in 30 ml Ethylacetat aufgenommen. Es wurde mit 20 ml gesättigter Natriumhydrogencarbonat-Lösung und mit 20 ml Wasser gewaschen. Die organische Phase wurde über Natriumsulfat getrocknet, filtriert und eingeengt. Der erhaltene Rückstand wurde in 3 ml DMSO gelöst und diese Lösung mittels präparativer HPLC gereinigt (Methode 14). Nach Vereinigen der Produktfraktionen und Gefriertrocknung wurden 68 mg (67% d. Th.) der Titelverbindung erhalten.
$^1$H-NMR (400 MHz, DMSO-$d_6$, $\delta$/ppm): 8.09 (s, 1H), 4.59 (t, 1H), 4.52-4.44 (m, 3H), 3.99 (br. t, 2H), 3.81 (br. s, 2H),

3.51-3.38 (m, 4H), 2.40 (s, 3H), 2.13-1.98 (m, 2H), 0.89 (s, 9H).
LC/MS (Methode 1, ESIpos): $R_t$ = 1.2 min, m/z = 435 $[M+H]^+$.

**Beispiel 445**

[1-{[3-(2,2-Dimethylpropyl)-1-(2-methoxyethyl)-5-melhyl-2,4-dioxo-1,2,3,4-tetrahydrothieno-[2,3-d]pyrimidin-6-yl]methyl}imidazolidin-2-yliden]cyanamid

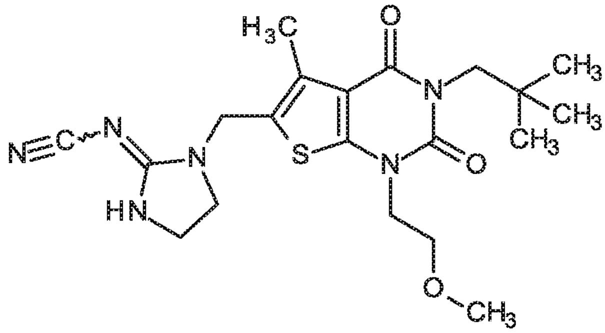

375 mg (0.833 mmol, 85% Reinheit) der Verbindung aus Bsp. 317A wurden in 10 ml DMF gelöst und mit 183 mg (1.25 mmol) Dimethyl-*N*-cyanodithioiminocarbonat und 230 mg (1.67 mmol) Kaliumcarbonat versetzt. Das Gemisch wurde 4 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 8). Die Produktfraktionen wurden vereinigt, eingeengt und ein weiteres Mal nach der gleichen Methode mittels präparativer HPLC nachgereinigt. Erneutes Einengen der Produktfraktionen und Trocknen im Hochvakuum ergab 82 mg (22% d. Th.) der Titelverbindung.
$^1$H-NMR (500 MHz, DMSO-$d_6$, δ/ppm): 8.07 (s, 1H), 4.47 (s, 2H), 4.03 (br. t, 2H), 3.81 (br. s, 2H), 3.63 (t, 2H), 3.52-3.38 (m, 4H), 3.23 (s, 3H), 2.39 (s, 3H), 0.89 (s, 9H).
LC/MS (Methode 1, ESIpos): $R_t$ = 0.98 min, m/z = 433 $[M+H]^+$.

**Beispiel 446**

[1-{[3-(2,2-Dimethylpropyl)-5-methyl-2,4-dioxo-1-(tetrahydrofuran-2-ylmethyl)-1,2,3,4-tetra-hydrothieno[2,3-d]pyrimidin-6-yl]methyl)imidazolidin-2-yliden]cyanamid (*Racemat*)

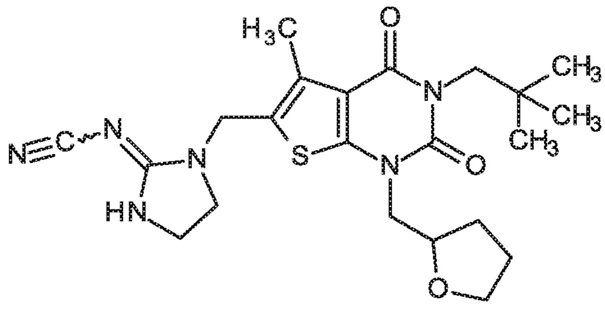

560 mg (1.16 mmol, 85% Reinheit) der Verbindung aus Bsp. 454A wurden in 14 ml DMF gelöst und mit 256 mg (1.75 mmol) Dimethyl-*N*-cyanodithioiminocarbonat und 322 mg (2.33 mmol) Kaliumcarbonat versetzt. Das Gemisch wurde 4 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumhydrogencarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 8). Die Produktfraktionen wurden vereinigt, eingeengt und im Hochvakuum getrocknet. Es wurden 162 mg (30% d. Th.) der Titelverbindung erhalten.
$^1$H-NMR (500 MHz, DMSO-$d_6$, δ/ppm): 8.07 (s, 1H), 4.47 (s, 2H), 4.27-4.18 (m, 1H), 4.02 (br. dd, 1H), 3.82 (br. s, 2H), 3.78-3.67 (m, 2H), 3.66-3.58 (m, 1H), 3.52-3.36 (m, 4H), 2.39 (s, 3H), 2.03-1.76 (m, 3H), 1.71-1.64 (m, 1H), 0.89 (s, 9H).
LC/MS (Methode 17, ESIpos): $R_t$ = 1.86 min, m/z = 459.22 $[M+H]^+$.

**Beispiel 447**

[1-{[3-(2,2-Dimethylpropyl)-5-methyl-2,4-dioxo-1-(tetrahydrofuran-2-ylmethyl)-1,2,3,4-tetra-hydrothieno[2,3-d]pyrimidin-6-yl]methyl}imidazolidin-2-yliden]cyanamid (*Enantiomer 1*)

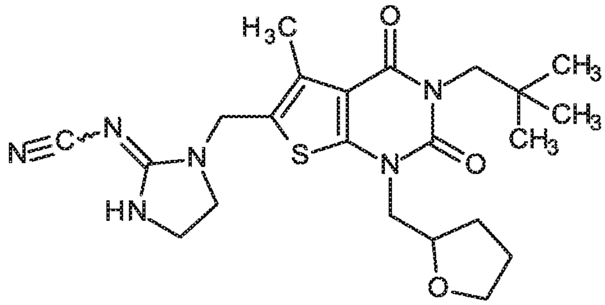

155 mg (0.338 mmol) der racemischen Verbindung aus Bsp. 446 wurden in 6 ml Isopropanol gelöst und in 20 Portionen über präparative HPLC an chiraler Phase in die Enantiomere aufgetrennt [Säule: Daicel Chiralpak IE, 5 μm, 250 mm x 20 mm; Laufmittel: *tert*.-Butylmethylether/Methanol 9:1; Fluss: 15 ml/min; Temperatur: 30°C; Detektion: 220 nm]. Nach Eindampfen der Produktfraktionen und Trocknen des Feststoffs im Hochvakuum wurden 71 mg (91% d. Th.) von Enantiomer 1 erhalten (99.0% ee, chirale analytische HPLC).
$^1$H-NMR (400 MHz, DMSO-$d_6$, δ/ppm): 8.07 (s, 1H), 4.47 (s, 2H), 4.27-4.16 (m, 1H), 4.02 (dd, 1H), 3.82 (br. s, 2H), 3.78-3.67 (m, 2H), 3.65-3.57 (m, 1H), 3.52-3.37 (m, 4H), 2.39 (s, 3H), 2.04-1.75 (m, 3H), 1.74-1.61 (m, 1H), 0.89 (s, 9H).
Chirale analytische HPLC [Säule: Daicel Chiraltek IE, 5 μm, 250 mm x 4.6 mm; Laufmittel: *tert*.-Butyhnethylether/Methanol 9:1; Fluss: 1 ml/min; Temperatur: 30°C; Detektion: 220 nm]: $R_t$ = 9.14 min.

**Beispiel 448**

[1-{[3-(2,2-Dimethylpropyl)-5-methyl-2,4-dioxo-1-(tetrahydrofuran-2-ylmethyl)-1,2,3,4-tetra-hydrothieno[2,3-d]pyrimidin-6-yl]methyl}imidazolidin-2-yliden]cyanamid *(Enantiomer 2)*

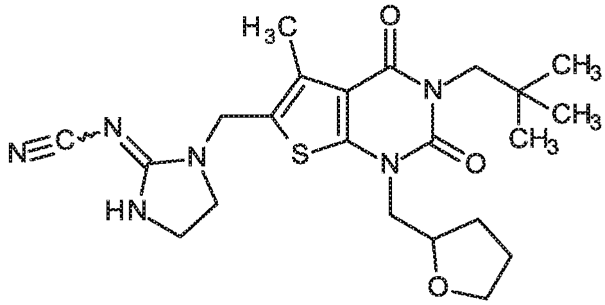

155 mg (0.338 mmol) der racemischen Verbindung aus Bsp. 446 wurden in 6 ml Isopropanol gelöst und in 20 Portionen über präparative HPLC an chiraler Phase in die Enantiomere aufgetrennt [Säule: Daicel Chiralpak IE, 5 μm, 250 mm x 20 mm; Laufmittel: *tert*.-Butylmethylether/Methanol 9:1; Fluss: 15 ml/min; Temperatur: 30°C; Detektion: 220 nm]. Nach Eindampfen der Produktfraktionen und Trocknen des Feststoffs im Hochvakuum wurden 68 mg (87% d. Th.) von Enantiomer 2 erhalten (99.0% ee, chirale analytische HPLC).

$^1H$-NMR (400 MHz, DMSO-$d_6$, δ/ppm): 8.07 (s, 1H), 4.47 (s, 2H), 4.28-4.17 (m, 1H), 4.02 (dd, 1H), 3.82 (br. s, 2H), 3.79-3.67 (m, 2H), 3.65-3.57 (m, 1H), 3.53-3.36 (m, 4H), 2.39 (s, 3H), 2.04-1.74 (m, 3H), 1.73-1.62 (m, 1H), 0.89 (s, 9H).

Chirale analytische HPLC [Säule: Daicel Chiraltek IE, 5 µm, 250 mm x 4.6 mm; Laufmittel: *tert.*-Butylmethylether/Methanol 9:1; Fluss: 1 ml/min; Temperatur: 30°C; Detektion: 220 nm]: $R_t$ = 10.67 min.

**Beispiel 449**

[1-{[5-Methyl-1-(oxetan-2-ylmethyl)-2,4-dioxo-3-(2,2,2-trifluorethyl)-1,2,3,4-tetrahydrothieno-[2,3-d]pyrimidin-6-yl]methyl}imidazolidin-2-yliden]cyanamid *(Enantiomer 1)*

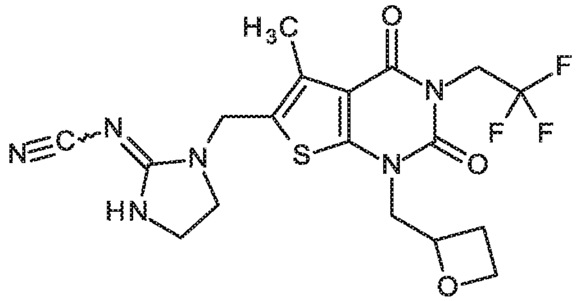

191 mg (0.418 mmol) des Stereoisomerengemisches aus Bsp. 359 wurden in einem Gemisch aus 11 ml Ethanol und 5 ml Dichlormethan gelöst und in 40 Portionen über präparative HPLC an chiraler Phase in die Enantiomere aufgetrennt [Säule: Daicel Chiralpak IE, 5 µm, 250 mm x 20 mm; Laufmittel: Isohexan/Ethanol 2:3; Fluss: 15 ml/min; Temperatur: 50°C; Detektion: 220 nm]. Nach Eindampfen der Produktfraktionen wurde der verbliebene Feststoff noch einmal mittels präparativer HPLC an achiraler Phase (Methode 8) nachgereinigt. Nach erneutem Eindampfen der Produktfraktionen und Trocknen des Feststoffs im Hochvakuum wurden 122 mg (63% d. Th.) von Enantiomer 1 erhalten (99.0% ee, chirale analytische HPLC).

$^1H$-NMR (400 MHz, DMSO-$d_6$, δ/ppm): 8.08 (s, 1H), 5.08-4.95 (m, 1H), 4.70 (q, 2H), 4.54-4.36 (m, 2H), 4.49 (s, 2H), 4.26-4.07 (m, 2H), 3.52-3.36 (m, 4H), 2.76-2.62 (m, 1H), 2.55-2.46 (m, 1H, weitgehend überdeckt vom DMSO-Signal), 2.41 (s, 3H).

Chirale analytische HPLC [Säule: Daicel Chiraltek IE, 5 µm, 250 mm x 4.6 mm; Laufmittel: Isohexan/Ethanol 2:3; Fluss: 1 ml/min; Temperatur: 50°C; Detektion: 220 nm]: $R_t$ = 7.64 min.

**Beispiel 450**

[1-{[5-Methyl-1-(oxetan-2-ylmethyl)-2,4-dioxo-3-(2,2,2-trifluorethyl)-1,2,3,4-tetrahydrothieno-[2,3-d]pyrimidin-6-yl]methyl}imidazolidin-2-yliden]cyanamid (*Enantiomer 2*)

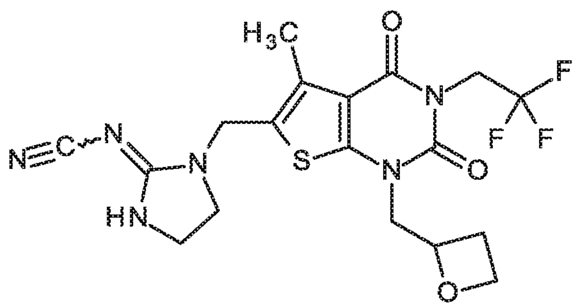

191 mg (0.418 mmol) des Stereoisomerengemisches aus Bsp. 359 wurden in einem Gemisch aus 11 ml Ethanol und 5 ml Dichlormethan gelöst und in 40 Portionen über präparative HPLC an chiraler Phase in die Enantiomere aufgetrennt [Säule: Daicel Chiralpak IE, 5 µm, 250 mm x 20 mm; Laufmittel: Isohexan/Ethanol 2:3; Fluss: 15 ml/min; Temperatur: 50°C; Detektion: 220 nm]. Nach Eindampfen der Produktfraktionen wurde der verbliebene Feststoff noch

einmal mittels präparativer HPLC an achiraler Phase (Methode 8) nachgereinigt. Nach erneutem Eindampfen der Produktfraktionen und Trocknen des Feststoffs im Hochvakuum wurden 13 mg (6% d. Th.) von Enantiomer 2 erhalten (96.5% ee, chirale analytische HPLC).
$^1$H-NMR (400 MHz, DMSO-$d_6$, δ/ppm): 8.08 (s, 1H), 5.07-4.96 (m, 1H), 4.70 (q, 2H), 4.54-4.37 (m, 2H), 4.49 (s, 2H), 4.27-4.10 (m, 2H), 3.53-3.37 (m, 4H), 2.76-2.63 (m, 1H), 2.55-2.45 (m, 1H, weitgehend überdeckt vom DMSO-Signal), 2.41 (s, 3H).

Chirale analytische HPLC [Säule: Daicel Chiraltek IE, 5 μm, 250 mm x 4.6 mm; Laufmittel: Isohexan/Ethanol 2:3; Fluss: 1ml/min; Temperatur: 50°C; Detektion: 220 nm]: $R_t$ = 8.10 min.

## Beispiel 451

[1-([5-Methyl-2,4-dioxo-3-(1,1,1-trifluorpropan-2-yl)-1-(3,3,3-trifluorpropyl)-1,2,3,4-tetrahydro-thieno[2,3-d]pyrimidin-6-yl]methyl}imidazolidin-2-yliden]cyanamid (*Racemat*)

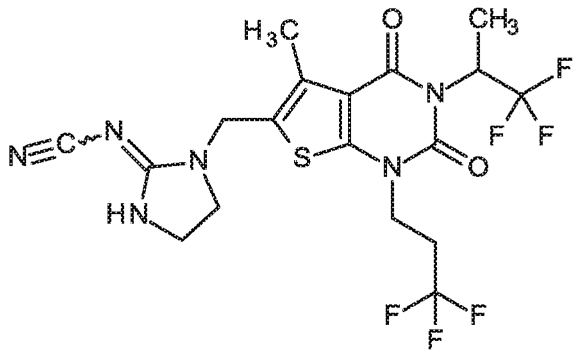

590 mg (1.11 mmol, 84% Reinheit) der Verbindung aus Bsp. 455A wurden in 12 ml DMF gelöst und mit 243 mg (1.67 mmol) Dimethyl-*N*-cyanodithioiminocarbonat und 307 mg (2.22 mmol) Kaliumcarbonat versetzt. Das Gemisch wurde 4 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumcarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 8). Die Produktfraktionen wurden vereinigt und eingedampft. Es wurden 250 mg (45% d. Th.) der Titelverbindung erhalten.
$^1$H-NMR (400 MHz, DMSO-$d_6$, δ/ppm): 8.10 (s, 1H), 5.84-5.46 (m, 1H), 4.51 (s, 2H), 4.12 (br. t, 2H), 3.55-3.38 (m, 4H), 2.89-2.69 (m, 2H), 2.41 (s, 3H), 1.63 (br. d, 3H).
LC/MS (Methode 17, ESIpos): $R_t$ = 1.86 min, m/z = 497.12 $[M+H]^+$.

## Beispiel 452

[1-{[1-(2-Methoxyethyl)-5-methyl-2,4-dioxo-3-(1,1,1-trifluorpropan-2-yl)-1,2,3,4-tetrahydro-thieno[2,3-d]pyrimidin-6-yl]methyl} imidazolidin-2-yliden]cyanamid (*Racemat*)

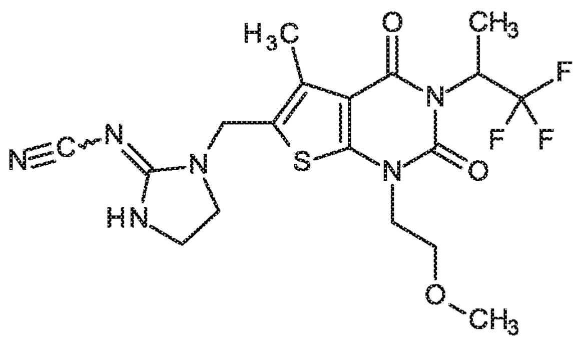

295 mg (0.592 mmol, 82% Reinheit) der Verbindung aus Bsp. 456A wurden in 10 ml DMF gelöst und mit 130 mg (0.888 mmol) Dimethyl-*N*-cyanodithioiminocarbonat und 164 mg (1.18 mmol) Kaliumcarbonat versetzt. Das Gemisch wurde 4 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumcarbonat-Lösung, Wasser und gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 8). Die Produktfraktionen wurden vereinigt und eingedampft. Es wurden 67 mg (24% d. Th.) der Titelverbindung erhalten.
$^1$H-NMR (400 MHz, DMSO-$d_6$, δ/ppm): 8.08 (s, 1H), 5.85-5.46 (m, 1H), 4.48 (s, 2H), 4.14-3.94 (m, 2H), 3.70-3.58 (m, 2H), 3.53-3.37 (m, 4H), 3.24 (s, 3H), 2.40 (s, 3H), 1.63 (br. d, 3H).
LC/MS (Methode 17, ESIpos): $R_t$ = 1.68 min, m/z = 459.14 [M+H]$^+$.

## Beispiel 453

[1-{[3-(2-Ethoxyethyl)-1-(3-fluorpropyl)-5-methyl-2,4-dioxo-1,2,3,4-tetrahydrothieno[2,3-d]-pyrimidin-6-yl]methyl}imidazolidin-2-yliden]cyanamid

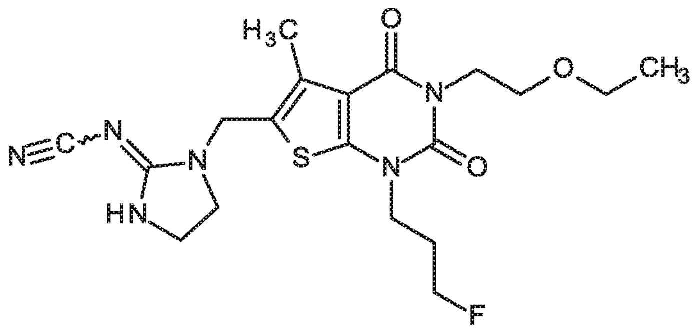

200 mg (0.388 mmol, 75% Reinheit) der Verbindung aus Bsp. 457A wurden in 5 ml DMF gelöst und mit 70 mg (0.582 mmol) Dimethyl-*N*-cyanodithioiminocarbonat und 107 mg (0.776 mmol) Kaliumcarbonat versetzt. Das Gemisch wurde 21 h bei 80°C gerührt. Danach wurde das Reaktionsgemisch in 30 ml Ethylacetat aufgenommen. Es wurde mit 20 ml gesättigter Natriumhydrogencarbonat-Lösung und mit 20 ml Wasser gewaschen. Die organische Phase wurde über Natriumsulfat getrocknet, filtriert und eingeengt. Der erhaltene Rückstand wurde in 3 ml DMSO gelöst und diese Lösung mittels präparativer HPLC gereinigt (Methode 14). Nach Vereinigen der Produktfraktionen und Gefriertrocknung wurden 81 mg (48% d. Th.) der Titelverbindung erhalten.
$^1$H-NMR (400 MHz, DMSO-$d_6$, δ/ppm): 8.09 (s, 1H), 4.59 (t, 1H), 4.51-4.44 (m, 3H), 4.03 (t, 2H), 3.99 (br. t, 2H), 3.50 (t, 2H), 3.48-3.37 (m, 5H), 2.41 (s, 2H), 2.14-1.99 (m, 2H), 1.06 (t, 3H).
LC/MS (Methode 1, ESIpos): $R_t$ = 0.98 min, m/z = 437 [M+H]$^+$.

## Beispiel 454

[1-{[3-(2-Methoxypropyl)-5-methyl-2,4-dioxo-1-(3,3,3-trifluorpropyl)-1,2,3,4-tetrahydrothieno-[2,3-d]pyrimidin-6-yl]methyl}imidazolidin-2-yliden]cyanamid *(Racemat)*

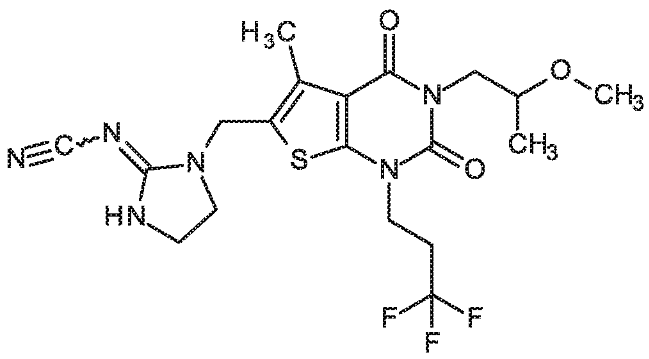

650 mg (1.23 mmol, 80% Reinheit) der Verbindung aus Bsp. 458A wurden in 12 ml DMF gelöst und mit 270 mg (1.85 mmol) Dimethyl-*N*-cyanodithioiminocarbonat und 340 mg (2.46 mmol) Kaliumcarbonat versetzt. Das Gemisch wurde 4 h bei 80°C in einem Mikrowellenofen gerührt (Biotage Initiator mit dynamischer Steuerung der Einstrahlleistung). Danach wurde das Reaktionsgemisch zur Trockene eingeengt und der Rückstand mittels präparativer HPLC (Methode 8) in seine Komponenten aufgetrennt. Die Produktfraktionen wurden vereinigt und eingedampft. Es wurden 317 mg (54% d. Th.) der Titelverbindung erhalten.
$^1$H-NMR (400 MHz, DMSO-$d_6$, δ/ppm): 8.08 (s, 1H), 4.50 (s, 2H), 4.19-4.08 (m, 2H), 4.05 (dd, 1H), 3.76 (dd, 1H), 3.63 (sext, 1H), 3.54-3.37 (m, 4H), 3.21 (s, 3H), 2.84-2.69 (m, 2H), 2.42 (s, 3H), 1.05 (d, 3H).
LC/MS (Methode 1, ESIpos): $R_t$ = 0.85 min, m/z = 473 $[M+H]^+$.

## Beispiel 455

[1-{[3-(2-Methoxypropyl)-5-methyl-2,4-dioxo-1-(3,3,3-trifluorpropyl)-1,2,3,4-tetrahydrothieno-[2,3-d]pyrimidin-6-yl]methyl}imidazolidin-2-yliden]cyanamid (*Enantiomer 1*)

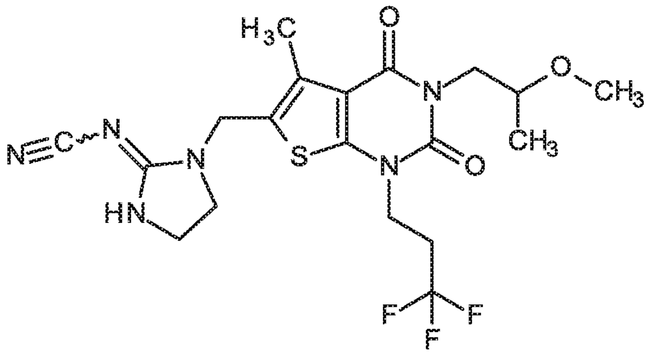

300 mg (0.635 mmol) der racemischen Verbindung aus Bsp. 454 wurden in 25 ml Methanol gelöst und in 13 Portionen über präparative SFC an chiraler Phase in die Enantiomere aufgetrennt [Säule: Daicel Chiralcel OZ-H, 5 µm, 250 mm x 20 mm; Laufmittel: Kohlendioxid/Methanol 7:3; Fluss: 80 ml/min; Temperatur: 40°C; Detektion: 210 nm]. Nach Eindampfen der Produktfraktionen und Trocknen im Hochvakuum wurden 128 mg (85% d. Th.) von Enantiomer 1 erhalten (>99% ee, chirale analytische HPLC).
$^1$H-NMR (400 MHz, DMSO-$d_6$, δ/ppm): 8.08 (s, 1H), 4.50 (s, 2H), 4.19-4.09 (m, 2H), 4.05 (dd, 1H), 3.76 (dd, 1H), 3.63 (sext, 1H), 3.53-3.38 (m, 4H), 3.21 (s, 3H), 2.85-2.69 (m, 2H), 2.42 (s, 3H), 1.05 (d, 3H).

Chirale analytische SFC [Säule: Daicel Chiralcel OZ-H, 5 µm, 250 mm x 4.6 mm; Laufmittel: Kohlendioxid/Methanol 3:2; Fluss: 3 ml/min; Temperatur: 40°C; Detektion: 210 nm]: $R_t$ = 2.80 min.

**Beispiel 456**

[1-{[3-(2-Methoxypropyl)-5-methyl-2,4-dioxo-1-(3,3,3-trifluorpropyl)-1,2,3,4-tetrahydrothieno-[2,3-d]pyrimidin-6-yl]methyl}imidazolidin-2-yliden]cyanamid *(Enantiomer 2)*

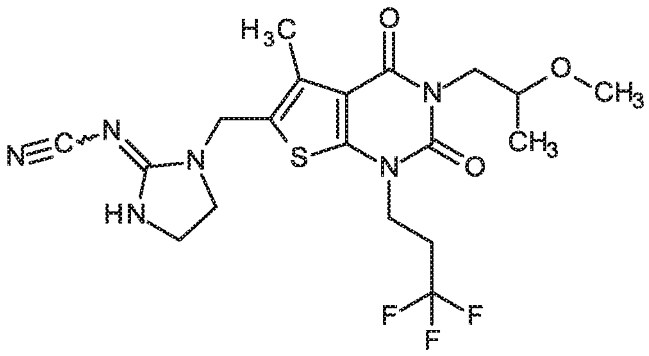

300 mg (0.635 mmol) der racemischen Verbindung aus Bsp. 454 wurden in 25 ml Methanol gelöst und in 13 Portionen über präparative SFC an chiraler Phase in die Enantiomere aufgetrennt [Säule: Daicel Chiralcel OZ-H, 5 μm, 250 mm x 20 mm; Laufmittel: Kohlendioxid/Methanol 7:3; Fluss: 80 ml/min; Temperatur: 40°C; Detektion: 210 nm]. Nach Eindampfen der Produktfraktionen und Trocknen im Hochvakuum wurden 93 mg (62% d. Th.) von Enantiomer 2 erhalten (>99% ee, chirale analytische HPLC).
$^1$H-NMR (400 MHz, DMSO-$d_6$, δ/ppm): 8.08 (s, 1H), 4.50 (s, 2H), 4.19-4.08 (m, 2H), 4.05 (dd, 1H), 3.76 (dd, 1H), 3.63 (sext, 1H), 3.52-3.37 (m, 4H), 3.21 (s, 3H), 2.85-2.69 (m, 2H), 2.42 (s, 3H), 1.05 (d, 3H).
Chirale analytische SFC [Säule: Daicel Chiralcel OZ-H, 5 μm, 250 mm x 4.6 mm; Laufmittel: Kohlendioxid/Methanol 3:2; Fluss: 3 ml/min; Temperatur: 40°C; Detektion: 210 nm]: $R_t$ = 5.11 min.

**Beispiel 457**

Methyl-[1-{[3-isopropyl-5-methyl-1-(oxetan-2-ylmethyl)-2,4-dioxo-1,2,3,4-tetrahydrothieno-[2,3-d]pyrimidin-6-yl]methyl}imidazolidin-2-yliden]carbamat (*Enantiomer 1*)

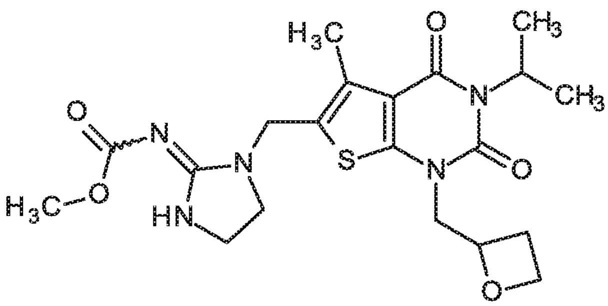

98 mg (0.218 mmol) der racemischen Verbindung aus Bsp. 379 wurden in 6 ml Ethanol gelöst und in 12 Portionen über präparative HPLC an chiraler Phase in die Enantiomere aufgetrennt [Säule: Daicel Chiralcel OD-H, 5 μm, 250 mm x 20 mm; Laufmittel: Isohexan/Ethanol 3:7; Fluss: 15 ml/ min; Temperatur: 25°C; Detektion: 210 nm]. Nach Eindampfen der Produktfraktionen und Trocknen im Hochvakuum wurden 43 mg (87% d. Th.) von Enantiomer 1 erhalten (99% ee, chirale analytische HPLC).
$^1$H-NMR (500 MHz, DMSO-$d_6$, δ/ppm): 8.01 (s, 1H), 5.13 (sept, 1H), 5.02-4.93 (m, 1H), 4.55 (s, 2H), 4.47 (td, 1H), 4.41 (dt, 1H), 4.14-4.03 (m, 2H), 3.53 (s, 3H), 3.48-3.41 (m, 2H), 3.35-3.31 (m, 2H, teilweise überdeckt vom Wasser-Signal), 2.74-2.64 (m, 1H), 2.52-2.44 (m, 1H, teilweise überdeckt vom DMSO-Signal), 2.40 (s, 3H), 1.39 (d, 6H).
Chirale analytische HPLC [Säule: Phenomenex Cellulose, 3 μm, 50 mm x 4.6 mm; Laufmittel: Isohexan/Ethanol 1:1; Fluss: 1 ml/min; Temperatur: 25°C; Detektion: 220 nm]: $R_t$ = 1.79 min.

**Beispiel 458**

Methyl-[1-{[3-isopropyl-5-methyl-1-(oxetan-2-ylmethyl)-2,4-dioxo-1,2,3,4-tetrahydrothieno-[2,3-d]pyrimidin-6-yl]methyl}imidazolidin-2-yliden]carbamat (*Enantiomer 2*)

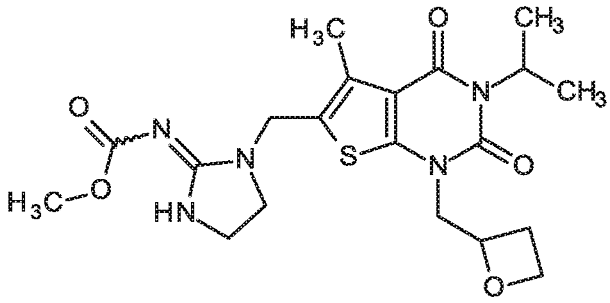

98 mg (0.218 mmol) der racemischen Verbindung aus Bsp. 379 wurden in 6 ml Ethanol gelöst und in 12 Portionen über präparative HPLC an chiraler Phase in die Enantiomere aufgetrennt [Säule: Daicel Chiralcel OD-H, 5 μm, 250 mm x 20 mm; Laufmittel: Isohexan/Ethanol 3:7; Fluss: 15 ml/ min; Temperatur: 25°C; Detektion: 210 nm]. Nach Eindampfen der Produktfraktionen und Trocknen im Hochvakuum wurden 37 mg (75% d. Th.) von Enantiomer 2 erhalten (85.9% ee, chirale analytische HPLC). Dieses Material wurde zur Nachreinigung noch ein zweites Mal unter gleichen HPLC-Bedingungen chromatographiert. Dies ergab 25 mg (51% d. Th.) von Enantiomer 2 (99.9% ee, chirale analytische HPLC).
$^1$H-NMR (400 MHz, DMSO-$d_6$, δ/ppm): 8.02 (s, 1H), 5.13 (sept, 1H), 4.98 (dt, 1H), 4.55 (s, 2H), 4.51-4.35 (m, 2H), 4.14-4.04 (m, 2H), 3.53 (s, 3H), 3.49-3.40 (m, 2H), 3.35-3.29 (m, 2H, teilweise überdeckt vom Wasser-Signal), 2.75-2.62 (m, 1H), 2.52-2.44 (m, 1H, teilweise überdeckt vom DMSO-Signal), 2.40 (s, 3H), 1.39 (d, 6H).
Chirale analytische HPLC [Säule: Phenomenex Cellulose, 3 μm, 50 mm x 4.6 mm; Laufmittel: Isohexan/Ethanol 1:1; Fluss: 1ml/min; Temperatur: 25°C; Detektion: 220 nm]: $R_t$ = 4.95 min.

**Beispiel 459**

Methyl-[1-{[3-isopropyl-5-methyl-2,4-dioxo-1-(tetrahydrofuren-2-ylmethyl)-1,2,3,4-tetrahydro-thieno[2,3-d]pyrimidin-6-yl]methyl}imidazolidin-2-yliden]carbamat *(Enantiomer 1)*

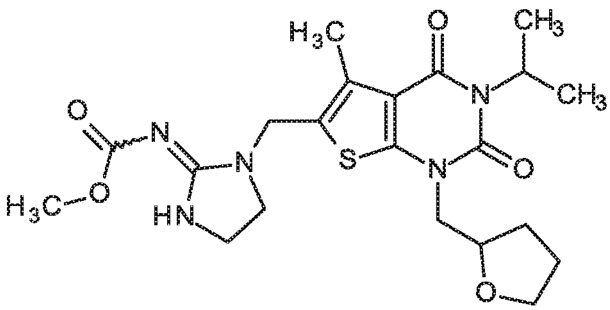

187 mg (0.403 mmol) der racemischen Verbindung aus Bsp. 380 wurden in 8 ml Ethanol gelöst und in 16 Portionen über präparative HPLC an chiraler Phase in die Enantiomere aufgetrennt [Säule: Daicel Chiralcel OX-H, 5 μm, 250 mm x 20 mm; Laufmittel: Isohexan/Ethanol 2:3; Fluss: 15 ml/min; Temperatur: 50°C; Detektion: 220 nm]. Nach Eindampfen der Produktfraktionen und Trocknen des Feststoffs im Hochvakuum wurden 78 mg (83% d. Th.) von Enantiomer 1 erhalten (99.0% ee, chirale analytische HPLC). Noch enthaltene Verunreinigungen wurden abschließend mittels präparativer HPLC an achiraler Phase (Methode 8) entfernt. Dies ergab 47 mg (50% d. Th.) von Enantiomer 1.
$^1$H-NMR (400 MHz, DMSO-$d_6$, δ/ppm): 8.02 (s, 1H), 5.14 (sept, 1H), 4.54 (s, 2H), 4.26-4.15 (m, 1H), 4.02 (dd, 1H), 3.77-3.67 (m, 1H), 3.65-3.55 (m, 2H), 3.53 (s, 3H), 3.49-3.41 (m, 2H), 3.35-3.29 (m, 2H, teilweise überdeckt vom Wasser-Signal), 2.40 (s, 3H), 2.03-1.74 (m, 3H), 1.71-1.58 (m, 1H), 1.40 (dd, 6H).

Chirale analytische HPLC [Säule: Daicel Chiralcel OX-H, 5 µm, 250 mm x 4.6 mm; Laufmittel: Isohexan/Ethanol 1:1; Fluss: 1 ml/min; Temperatur: 50°C; Detektion: 220 nm]: $R_t$= 11.03 min.

**Beispiel 460**

Methyl-[1-{[3-isopropyl-5-methyl-2,4-dioxo-1-(tetrahydrofuran-2-ylmethyl)-1,2,3,4-tetrahydro-thieno[2,3-d]pyrimidin-6-yl]methyl}imidazolidin-2-yliden]carbamat (*Enantiomer 2*)

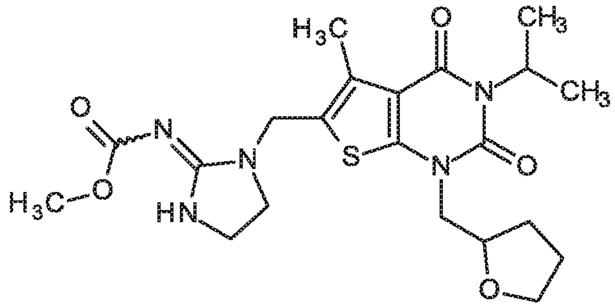

187 mg (0.403 mmol) der racemischen Verbindung aus Bsp. 380 wurden in 8 ml Ethanol gelöst und in 16 Portionen über präparative HPLC an chiraler Phase in die Enantiomere aufgetrennt [Säule: Daicel Chiralcel OX-H, 5 µm, 250 mm x 20 mm; Laufmittel: Isohexan/Ethanol 2:3; Fluss: 15 ml/min; Temperatur: 50°C; Detektion: 220 nm]. Nach Eindampfen der Produktfraktionen und Trocknen des Feststoffs im Hochvakuum wurden 81 mg (86% d. Th.) von Enantiomer 2 erhalten (99.0% ee, chirale analytische HPLC). Noch enthaltene Verunreinigungen wurden abschließend mittels präparativer HPLC an achiraler Phase (Methode 8) entfernt. Dies ergab 37 mg (39% d. Th.) von Enantiomer 2.
$^1$H-NMR (400 MHz, DMSO-$d_6$, δ/ppm): 8.02 (s, 1H), 5.14 (sept, 1H), 4.54 (s, 2H), 4.26-4.15 (m, 1H), 4.02 (dd, 1H), 3.76-3.68 (m, 1H), 3.65-3.55 (m, 2H), 3.53 (s, 3H), 3.49-3.41 (m, 2H), 3.35-3.29 (m, 2H, teilweise überdeckt vom Wasser-Signal), 2.40 (s, 3H), 2.04-1.75 (m, 3H), 1.69-1.61 (m, 1H), 1.40 (dd, 6H).

Chirale analytische HPLC [Säule: Daicel Chiralcel OX-H, 5 µm, 250 mm x 4.6 mm; Laufmittel: Isohexan/Ethanol 1:1; Fluss: 1 ml/min; Temperatur: 50°C; Detektion: 220 nm]: $R_t$ = 15.02 min.

**Beispiel 461**

Methyl-[1-{[3-(2,2-dimethylpropyl)-5-methyl-2,4-dioxo-1-(3,3,3-trifluorpropyl)-1,2,3,4-tetra-hydrothieno[2,3-d]pyrimidin-6-yl]methyl}imidazolidin-2-yliden]carbamat

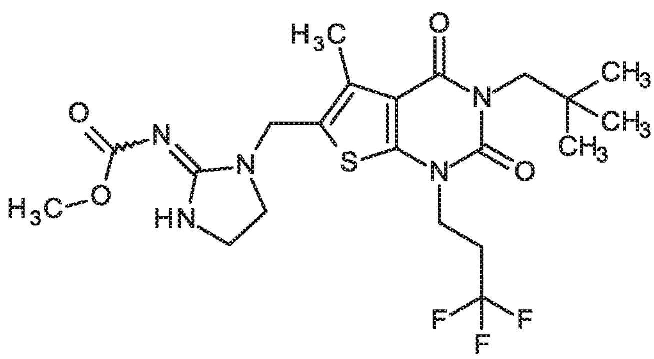

290 mg (0.552 mmol, 80% Reinheit) der Verbindung aus Bsp. 315A und 154 µl (1.10 mmol) Triethylamin wurden in 10 ml Dichlormethan gelöst und mit einer Lösung von 172 mg (1.10 mmol) Methyl-(dichlormethylen)carbamat in 5 ml Dichlormethan versetzt. Nachdem das Reaktionsgemisch ca. 18 h bei RT gerührt worden war, wurde es zur Trockene eingeengt und der Rückstand mittels präparativer HPLC (Methode 8) gereinigt. Einengen der Produktfraktionen und Trocknen des Rückstands im Hochvakuum ergaben 157 mg (55% d. Th.) der Titelverbindung.

$^1$H-NMR (400 MHz, DMSO-$d_6$, δ/ppm): 8.03 (s, 1H), 4.58 (s, 2H), 4.09 (t, 2H), 3.81 (br. s, 2H), 3.53 (s, 3H), 3.50-3.42 (m, 2H), 3.40-3.33 (m, 2H), 2.84-2.66 (m, 2H), 2.42 (s, 3H), 0.89 (s, 9H).
LC/MS (Methode 17, ESIpos): $R_t$ = 1.96 min, m/z = 504.19 $[M+H]^+$.

**Beispiel 462**

Methyl-[1-{[3-(2,2-dimethylpropyl)-1-(2-methoxyethyl)-5-methyl-2,4-dioxo-1,2,3,4-tetrahydrothieno[2,3-d]pyrimidin-6-yl]methyl}imidazolidin-2-yliden]carbamat

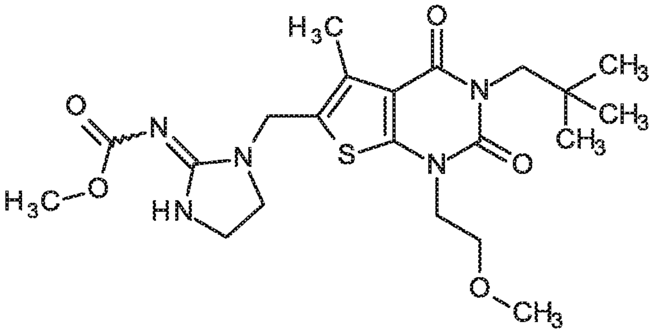

375 mg (0.833 mmol, 85% Reinheit) der Verbindung aus Bsp. 317A und 232 µl (1.67 mmol) Triethylamin wurden in 15 ml Dichlormethan gelöst und mit einer Lösung von 260 mg (1.67 mmol) Methyl-(dichlormethylen)carbamat in 5 ml Dichlormethan versetzt. Nachdem das Reaktionsgemisch ca. 18 h bei RT gerührt worden war, wurde es mit Ethylacetat verdünnt und nacheinander mit Wasser und gesättigter Natriumchlorid-Lösung gewaschen. Nach Trocknen über wasserfreiem Magnesiumsulfat wurde filtriert und eingeengt. Der verbliebene Rückstand wurde mittels MPLC gereinigt (Biotage Isolera, Kartusche mit 100 g Kieselgel, Cyclohexan/Ethylacetat 50:50 → 0:100). Einengen der Produktfraktionen und Trocknen des Rückstands im Hochvakuum ergaben 230 mg (59% d. Th.) der Titelverbindung.
$^1$H-NMR (400 MHz, DMSO-$d_6$, δ/ppm): 8.03 (s, 1H), 4.56 (s, 2H), 4.00 (t, 2H), 3.81 (s, 2H), 3.62 (t, 2H), 3.53 (s, 3H), 3.50-3.42 (m, 2H), 3.40-3.33 (m, 2H), 3.22 (s, 3H), 2.41 (s, 3H), 0.89 (s, 9H).
LC/MS (Methode 17, ESIpos): $R_t$ = 1.65 min, m/z = 466.21 $[M+H]^+$.

**Beispiel 463**

Methyl-[1-{[3-(2,2-dimethylpropyl)-5-methyl-2,4-dioxo-1-(tetrahydrofuran-2-ylmethyl)-1,2,3,4-tetrahydrothieno[2,3-d]pyrimidin-6-yl]methyl}imidazolidin-2-yliden]carbamat (*Racemat*)

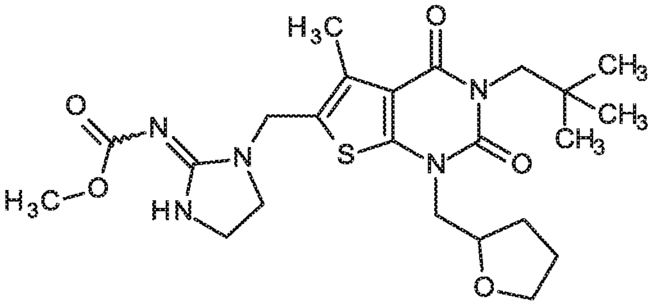

560 mg (1.16 mmol, 85% Reinheit) der Verbindung aus Bsp. 454A und 325 µl (2.33 mmol) Triethylamin wurden in 20 ml Dichlormethan gelöst und mit einer Lösung von 363 mg (2.33 mmol) Methyl-(dichlormethylen)carbamat in 5 ml Dichlormethan versetzt. Nachdem das Reaktionsgemisch ca. 18 h bei RT gerührt worden war, wurde es mit Ethylacetat verdünnt und nacheinander mit gesättigter Natriumcarbonat-Lösung und gesättigter Natriumchlorid-Lösung gewaschen. Nach Trocknen über wasserfreiem Magnesiumsulfat wurde filtriert und eingeengt. Der verbliebene Rückstand wurde mittels MPLC gereinigt (Biotage Isolera, Kartusche mit 50 g Kieselgel, Cyclohexan/Ethylacetat 2:1). Einengen der Produktfraktionen und Trocknen des Rückstands im Hochvakuum ergaben 320 mg (55% d. Th.) der Titelverbindung.
$^1$H-NMR (500 MHz, DMSO-$d_6$, δ/ppm): 8.02 (s, 1H), 4.55 (s, 2H), 4.26-4.17 (m, 1H), 4.03-3.97 (m, 1H), 3.81 (br. s, 2H), 3.75-3.64 (m, 2H), 3.63-3.56 (m, 1H), 3.53 (s, 3H), 3.49-3.43 (m, 2H), 3.39-3.33 (m, 2H), 2.41 (s, 3H), 2.02-1.75 (m, 3H), 1.70-1.63 (m, 1H), 0.89 (s, 9H).
LC/MS (Methode 17, ESIpos): $R_t$ = 1.75 min, m/z = 492.23 $[M+H]^+$.

**Beispiel 464**

Methyl-[1-{[3-(2,2-dimethylpropyl)-5-methyl-2,4-dioxo-1-(tetrahydrofuran-2-ylmethyl)-1,2,3,4-tetrahydrothieno[2,3-d]pyrimidin-6-yl]melhyl}imidazolidin-2-yliden]carbamat (*Enantiomer 1*)

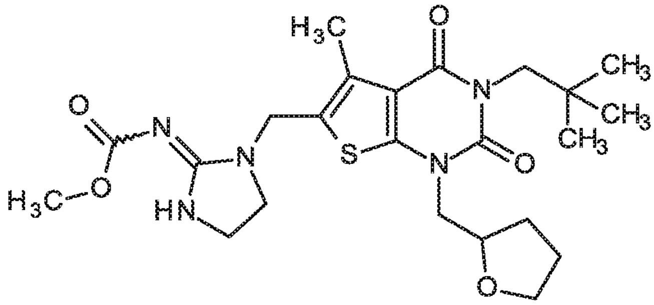

308 mg (0.626 mmol) der racemischen Verbindung aus Bsp. 463 wurden in einem Gemisch aus jeweils 3 ml Methanol, *tert.*-Butylmethylether und Dichlormethan gelöst und in 36 Portionen über präparative HPLC an chiraler Phase in die Enantiomere aufgetrennt [Säule: Daicel Chiralpak IF, 5 μm, 250 mm x 20 mm; Laufmittel: *tert.*-Butylmethylether/Methanol 7:3; Fluss: 15 ml/min; Temperatur: 30°C; Detektion: 220 nm]. Nach Eindampfen der Produktfraktionen wurden 136 mg (88% d. Th.) von Enantiomer 1 erhalten (99.0% ee, chirale analytische HPLC).
$^1$H-NMR (500 MHz, DMSO-$d_6$, δ/ppm): 8.03 (br. s, 1H), 4.55 (s, 2H), 4.26-4.16 (m, 1H), 4.01 (br. dd, 1H), 3.82 (br. s, 2H), 3.75-3.64 (m, 2H), 3.63-3.56 (m, 1H), 3.54 (s, 3H), 3.50-3.43 (m, 2H), 3.39-3.33 (m, 2H), 2.41 (s, 3H), 2.02-1.75 (m, 3H), 1.70-1.63 (m, 1H), 0.89 (s, 9H).
Chirale analytische HPLC [Säule: Daicel Chiraltek IF, 5 μm, 250 mm x 4.6 mm; Laufmittel: *tert.*-Butylmethylether/Methanol 7:3; Fluss: 1 ml/min; Temperatur: 30°C; Detektion: 220 nm]: $R_t$ = 6.25 min.

**Beispiel 465**

Methyl-[1-{[3-(2,2-dimethylpropyl)-5-methyl-2,4-dioxo-1-(tetrahydrofuran-2-ylmethyl)-1,2,3,4-tetrahydrothieno[2,3-d]pyrimidin-6-yl]methyl}imidazolidin-2-yliden]carbamat (*Enantiomer 2*)

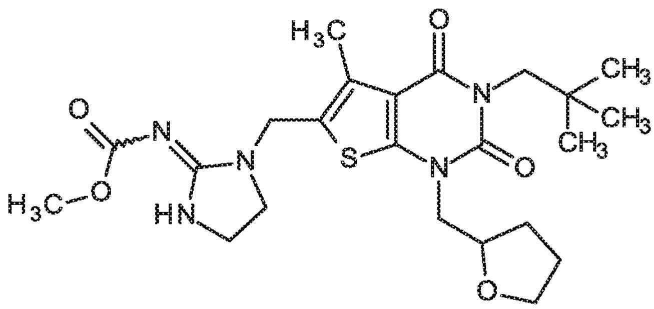

308 mg (0.626 mmol) der racemischen Verbindung aus Bsp. 463 wurden in einem Gemisch aus jeweils 3 ml Methanol, *tert.*-Butylmethylether und Dichlormethan gelöst und in 36 Portionen über präparative HPLC an chiraler Phase in die Enantiomere aufgetrennt [Säule: Daicel Chiralpak IF, 5 μm, 250 mm x 20 mm; Laufmittel: *tert.*-Butylmethylether/Methanol 7:3; Fluss: 15 ml/min; Temperatur: 30°C; Detektion: 220 nm]. Nach Eindampfen der Produktfraktionen wurden 136 mg (88% d. Th.) von Enantiomer 2 erhalten (98.8% ee, chirale analytische HPLC).

$^1$H-NMR (500 MHz, DMSO-$d_6$, δ/ppm): 8.04 (br. s, 1H), 4.55 (s, 2H), 4.26-4.17 (m, 1H), 4.01 (br. dd, 1H), 3.82 (br. s, 2H), 3.75-3.64 (m, 2H), 3.63-3.56 (m, 1H), 3.54 (s, 3H), 3.50-3.43 (m, 2H), 3.40-3.33 (m, 2H), 2.41 (s, 3H), 2.02-1.76 (m, 3H), 1.70-1.63 (m, 1H), 0.89 (s, 9H).

Chirale analytische HPLC [Säule: Daicel Chiraltek IF, 5 μm, 250 mm x 4.6 mm; Laufmittel: *tert.*-Butylmethylether/Methanol 7:3; Fluss: 1 ml/min; Temperatur: 30°C; Detektion: 220 nm]: $R_t$ = 8.15 min.

**Beispiel 466**

Methyl-[1-{[5-methyl-1-(oxetan-2-ylmethyl)-2,4-dioxo-3-(2,2,2-trifluorethyl)-1,2,3,4-tetrahydrothieno[2,3-d]pyrimidin-6-yl]methyl}imidazolidin-2-yliden]carbamat (*Enantiomer 1*)

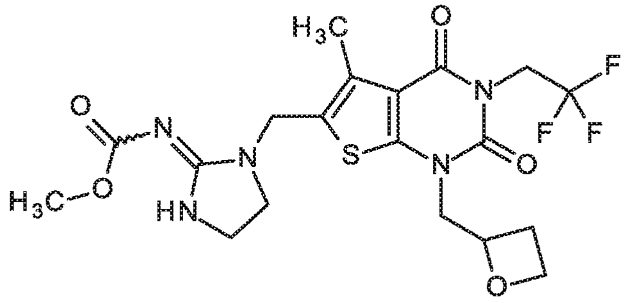

50 mg (0.102 mmol) des Stereoisomerengemisches aus Bsp. 383 wurden in einem Gemisch aus 2 ml Ethanol und 3 ml Acetonitril gelöst und in 7 Portionen über präparative HPLC an chiraler Phase in die Enantiomere aufgetrennt [Säule: Daicel Chiralcel OX-H, 5 μm, 250 mm x 20 mm; Laufmittel: Isohexan/Ethanol 1:1; Fluss: 20 ml/min; Temperatur: 22°C; Detektion: 220 nm]. Nach Eindampfen der Produktfraktionen und Trocknen des Feststoffs im Hochvakuum wurden 5 mg (10% d. Th.) von Enantiomer 1 erhalten (99.5% ee, chirale analytische HPLC).

$^1$H-NMR (500 MHz, DMSO-$d_6$, δ/ppm): 8.03 (s, 1H), 5.05-4.95 (m, 1H), 4.70 (q, 2H), 4.57 (s, 2H), 4.51-4.44 (m, 1H), 4.41 (dt, 1H), 4.23-4.09 (m, 2H), 3.54 (s, 3H), 3.49-3.42 (m, 2H), 3.37-3.33 (m, 2H, teilweise überdeckt vom Wasser-Signal), 2.76-2.62 (m, 1H), 2.51-2.44 (m, 1H, weitgehend überdeckt vom DMSO-Signal), 2.42 (s, 3H).

Chirale analytische HPLC [Säule: Daicel Chiralcel OX-H, 3 μm, 50 mm x 4.6 mm; Laufmittel: Isohexan/Ethanol 1:1; Fluss: 1 ml/min; Temperatur: 22°C; Detektion: 220 nm]: $R_t$ = 2.99 min.

**Beispiel 467**

Methyl-[1-{[5-methyl-1-(oxetan-2-ylmethyl)-2,4-dioxo-3-(2,2,2-trifluorethyl)-1,2,3,4-tetrahydrothieno[2,3-d]pyrimidin-6-yl]methyl}imidazolidin-2-yliden]carbamat (*Enantiomer 2*)

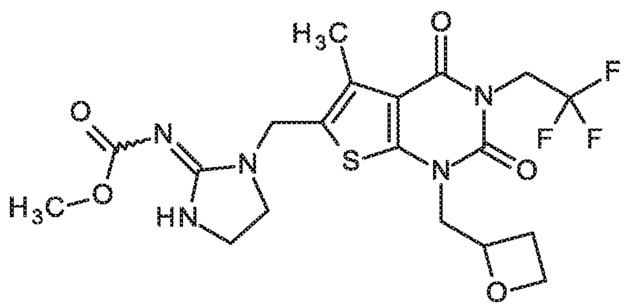

50 mg (0.102 mmol) des Stereoisomerengemisches aus Bsp. 383 wurden in einem Gemisch aus 2 ml Ethanol und 3 ml Acetonitril gelöst und in 7 Portionen über präparative HPLC an chiraler Phase in die Enantiomere aufgetrennt [Säule: Daicel Chiralcel OX-H, 5 μm, 250 mm x 20 mm; Laufmittel: Isohexan/Ethanol 1:1; Fluss: 20 ml/min; Temperatur: 22°C; Detektion: 220 nm]. Nach Eindampfen der Produktfraktionen und Trocknen des Feststoffs im Hochvakuum wurden 28 mg (56% d. Th.) von Enantiomer 2 erhalten (99.5% ee, chirale analytische HPLC). Noch enthaltene Verunreinigungen

wurden abschließend mittels präparativer HPLC an achiraler Phase (Methode 8) entfernt. Dies ergab 20 mg (40% d. Th.) von Enantiomer 2.
$^1$H-NMR (400 MHz, DMSO-$d_6$, δ/ppm): 8.03 (s, 1H), 5.05-4.94 (m, 1H), 4.70 (q, 2H), 4.57 (s, 2H), 4.51-4.44 (m, 1H), 4.40 (dt, 1H), 4.24-4.09 (m, 2H), 3.53 (s, 3H), 3.50-3.42 (m, 2H), 3.37-3.32 (m, 2H, teilweise überdeckt vom Wasser-Signal), 2.76-2.63 (m, 1H), 2.54-2.45 (m, 1H, teilweise überdeckt vom DMSO-Signal), 2.42 (s, 3H).

Chirale analytische HPLC [Säule: Daicel Chiralcel OX-H, 3 μm, 50 mm x 4.6 mm; Laufmittel: Isohexan/Ethanol 1:1; Fluss: 1 ml/min; Temperatur: 22°C; Detektion: 220 nm]: $R_t$ = 3.81 min.

**Beispiel 468**

Methyl-[1-{[5-methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl}imidazolidin-2-yliden]carbamat (*Enantiomer 1*)

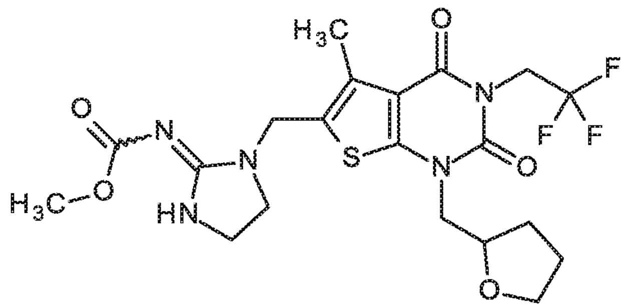

175 mg (0.348 mmol) der racemischen Verbindung aus Bsp. 384 wurden in einem Gemisch aus 2 ml Ethanol und 2.5 ml Acetonitril gelöst und in 28 Portionen über präparative HPLC an chiraler Phase in die Enantiomere aufgetrennt [Säule: Daicel Chiralpak AS-H, 5 μm, 250 mm x 20 mm; Laufmittel: Isohexan/Ethanol 1:1; Fluss: 20 ml/min; Temperatur: 22°C; Detektion: 220 nm]. Nach Eindampfen der Produktfraktionen wurde der verbliebene Rückstand mit Pentan, dem etwas Dichlormethan beigefügt war, bei RT verrührt. Nach Absaugen des Feststoffs und Trocknen im Hochvakuum wurden 65 mg (74% d. Th.) von Enantiomer 1 erhalten (>99% ee, chirale analytische HPLC). Noch enthaltene Verunreinigungen wurden abschließend mittels präparativer HPLC an achiraler Phase (Methode 8) entfernt. Dies ergab 40 mg (45% d. Th.) von Enantiomer 1.
$^1$H-NMR (400 MHz, DMSO-$d_6$, δ/ppm): 8.06 (br. s, 1H), 4.70 (q, 2H), 4.57 (s, 2H), 4.28-4.17 (m, 1H), 4.06 (dd, 1H), 3.77-3.66 (m, 2H), 3.64-3.56 (m, 1H), 3.54 (s, 3H), 3.50-3.42 (m, 2H), 3.38-3.33 (m, 2H, teilweise überdeckt vom Wasser-Signal), 2.42 (s, 3H), 2.04-1.76 (m, 3H), 1.73-1.61 (m, 1H).

Chirale analytische HPLC [Säule: Daicel Chiralpak AS-3, 3 μm, 50 mm x 4.6 mm; Laufmittel: Isohexan/Ethanol 1:1; Fluss: 1 ml/min; Temperatur: 22°C; Detektion: 220 nm]: $R_t$ = 2.05 min.

**Beispiel 469**

Methyl-[1-{[5-methyl-2,4-dioxo-1-(tetrahydrofuran-2-ylmethyl)-3-(2,2,2-trifluorethyl)-1,2,3,4-tetrahydrothieno[2,3-d]pyrimidin-6-yl]methyl}imidazolidin-2-yliden]carbamat (*Enantiomer 2*)

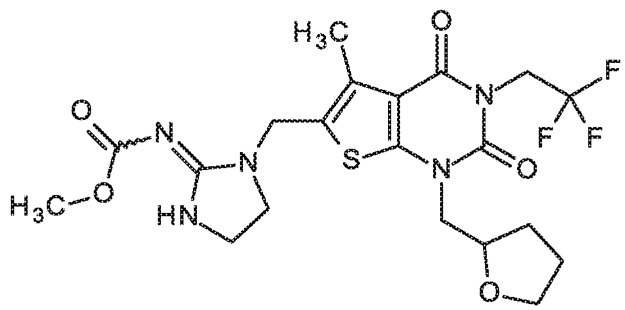

175 mg (0.348 mmol) der racemischen Verbindung aus Bsp. 384 wurden in einem Gemisch aus 2 ml Ethanol und 2.5 ml Acetonitril gelöst und in 28 Portionen über präparative HPLC an chiraler Phase in die Enantiomere aufgetrennt [Säule: Daicel Chiralpak AS-H, 5 μm, 250 mm x 20 mm; Laufmittel: Isohexan/Ethanol 1:1; Fluss: 20 ml/min; Temperatur: 22°C; Detektion: 220 nm]. Nach Eindampfen der Produktfraktionen wurde der verbliebene Rückstand mit Pentan, dem etwas Dichlormethan beigefügt war, bei RT verrührt. Nach Absaugen des Feststoffs und Trocknen im Hochvakuum wurden 54 mg (61% d. Th.) von Enantiomer 2 erhalten (>99% ee, chirale analytische HPLC).
$^1$H-NMR (400 MHz, DMSO-$d_6$, δ/ppm): 8.03 (s, 1H), 4.70 (q, 2H), 4.56 (s, 2H), 4.28-4.17 (m, 1H), 4.05 (dd, 1H), 3.77-3.65 (m, 2H), 3.63-3.56 (m, 1H), 3.53 (s, 3H), 3.49-3.41 (m, 2H), 3.37-3.33 (m, 2H, teilweise überdeckt vom Wasser-Signal), 2.42 (s, 3H), 2.04-1.76 (m, 3H), 1.73-1.61 (m, 1H).

Chirale analytische HPLC [Säule: Daicel Chiralpak AS-3, 3 μm, 50 mm x 4.6 mm; Laufmittel: Isohexan/Ethanol 1:1; Fluss: 1 ml/min; Temperatur: 22°C; Detektion: 220 nm]: $R_t$ = 3.15 min.

**Beispiel 470**

Methyl-[1-{[5-methyl-2,4-dioxo-3-{1,1,1-trifluorpropan-2-yl)-1-(3,3,3-trifluorpropyl)-1,2,3,4-tetrahydrothieno[2,3-d]pyrimidin-6-yl]methyl}imidazolidin-2-yliden]carbamat (*Racemat*)

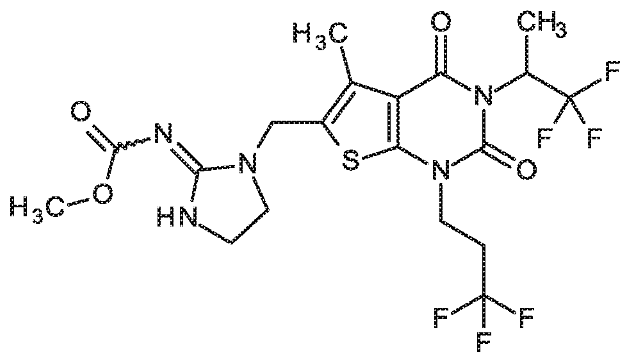

590 mg (1.11 mmol, 84% Reinheit) der Verbindung aus Bsp. 455A und 309 μl (2.22 mmol) Triethylamin wurden in 15 ml Dichlormethan gelöst und mit einer Lösung von 346 mg (2.22 mmol) Methyl-(dichlormethylen)carbamat in 5 ml Dichlormethan versetzt. Nachdem das Reaktionsgemisch ca. 18 h bei RT gerührt worden war, wurde es zur Trockene eingeengt. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) gereinigt. Einengen der Produktfraktionen und Trocknen des Rückstands im Hochvakuum ergaben 206 mg (35% d. Th.) der Titelverbindung.
$^1$H-NMR (400 MHz, DMSO-$d_6$, δ/ppm): 8.07 (br. s, 1H), 5.82-5.47 (m, 1H), 4.59 (s, 2H), 4.18-4.02 (m, 2H), 3.54 (s, 3H), 3.51-3.43 (m, 2H), 3.39-3.33 (m, 2H, teilweise überdeckt vom Wasser-Signal), 2.85-2.69 (m, 2H), 2.43 (s, 3H), 1.63 (br. d, 3H).
LC/MS (Methode 17, ESIpos): $R_t$ = 1.82 min, m/z = 530.13 [M+H]$^+$.

**Beispiel 471**

Methyl-[1-{[1-(2-methoxyethyl)-5-methyl-2,4-dioxo-3-(1,1,1-trifluorpropan-2-yl)-1,2,3,4-tetrahydrothieno[2,3-d]pyrimidin-6-yl]methyl}imidazolidin-2-yliden]carbamat (*Racemat*)

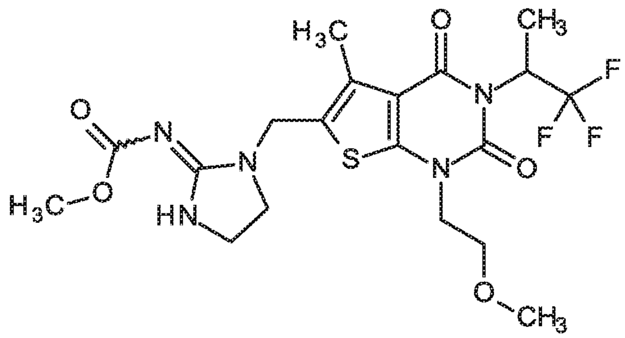

295 mg (0.592 mmol, 82% Reinheit) der Verbindung aus Bsp. 456A und 165 μl (1.18 mmol) Triethylamin wurden in 10 ml Dichlormethan gelöst und mit einer Lösung von 185 mg (1.18 mmol) Methyl-(dichlormethylen)carbamat in 5 ml Dichlormethan versetzt. Nachdem das Reaktionsgemisch ca. 18 h bei RT gerührt worden war, wurde es zur Trockene eingeengt. Der verbliebene Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) vorgereinigt. Die Produktfraktionen wurden vereinigt und eingeengt. Der Rückstand wurde in Ethylacetat gelöst und nacheinander mit 1 M Salzsäure und Wasser gewaschen. Nach erneutem Eindampfen wurde der Rückstand durch nochmalige präparative HPLC nach gleicher Methode nachgereinigt. Nach Einengen der Produktfraktionen und Trocknen des Rückstands im Hochvakuum wurden 46 mg (15% d. Th.) der Titelverbindung erhalten.
$^1$H-NMR (400 MHz, DMSO-$d_6$, δ/ppm): 8.03 (s, 1H), 5.84-5.47 (m, 1H), 4.56 (s, 2H), 4.07-3.95 (m, 2H), 3.67-3.58 (m, 2H), 3.53 (s, 3H), 3.49-3.42 (m, 2H), 3.37-3.32 (m, 2H, teilweise überdeckt vom Wasser-Signal), 3.23 (s, 3H), 2.41 (s, 3H), 1.63 (br. d, 3H).
LC/MS (Methode 17, ESIpos): $R_t$ = 1.54 min, m/z = 492.15 $[M+H]^+$.

**Beispiel 472**

Methyl-[1-{[3-(2-methoxypropyl)-5-methyl-2,4-dioxo-1-(3,3,3-trifluorpropyl)-1,2,3,4-tetrahydrothieno[2,3-d]pyrimidin-6-yl]methyl}imidazolidin-2-yliden]carbamat (*Racemat*)

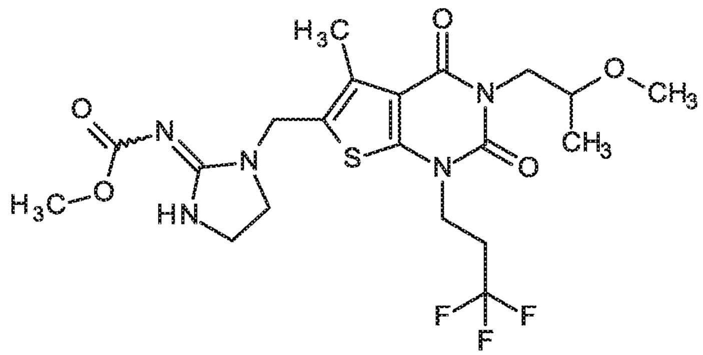

650 mg (1.23 mmol, 80% Reinheit) der Verbindung aus Bsp. 458A und 343 μl (2.46 mmol) Triethylamin wurden in 15 ml Dichlormethan gelöst und mit einer Lösung von 384 mg (2.46 mmol) Methyl-(dichlormethylen)carbamat in 15 ml Dichlormethan versetzt. Nachdem das Reaktionsgemisch 2.5 Tage bei RT gerührt worden war, wurde es zur Trockene eingeengt. Der verbliebene Rückstand wurde mittels MPLC gereinigt (Biotage Isolera, Kartusche mit 100 g Kieselgel, Cyclohexan/Ethylacetat 90:10 → 0:100). Die Produktfraktionen wurden vereinigt und eingedampft. Nach Trocknen im Hochvakuum wurden 260 mg (40% d. Th., 97% Reinheit) der Titelverbindung erhalten.
$^1$H-NMR (400 MHz, DMSO-$d_6$, δ/ppm): 8.05 (br. s, 1H), 4.58 (s, 2H), 4.13-4.00 (m, 3H), 3.76 (dd, 1H), 3.69-3.58 (m, 1H), 3.54 (s, 3H), 3.50-3.42 (m, 2H), 3.40-3.34 (m, 2H), 3.21 (s, 3H), 2.83-2.68 (m, 2H), 2.43 (s, 3H), 1.05 (d, 3H).
LC/MS (Methode 1, ESIpos): $R_t$ = 0.77 min, m/z = 506 $[M+H]^+$.

**Beispiel 473**

Methyl-[1-{[3-(2-methoxypropyl)-5-methyl-2,4-dioxo-1-(3,3,3-trifluorpropyl)-1,2,3,4-tetrahydrothieno[2,3-d]pyrimidin-6-yl]methyl}imidazolidin-2-yliden]carbamat (*Enantiomer 1*)

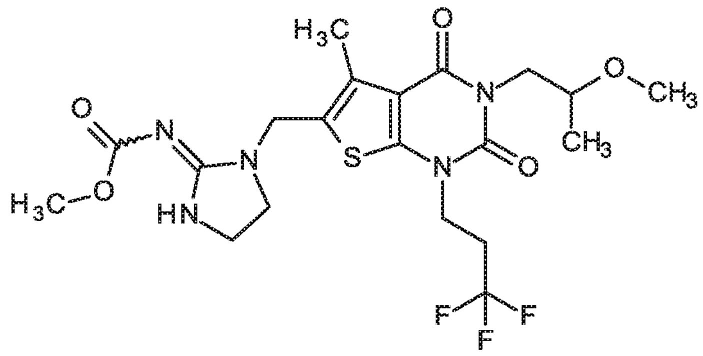

250 mg (494 mmol) der racemischen Verbindung aus Bsp. 472 wurden in 25 ml Methanol gelöst und in 9 Portionen über präparative SFC an chiraler Phase in die Enantiomere aufgetrennt [Säule: Daicel Chiralcel OX-H, 5 μm, 250 mm x 30 mm; Laufmittel: Kohlendioxid/Ethanol 7:3; Fluss: 100 ml/min; Temperatur: 40°C; Detektion: 210 nm]. Nach Eindampfen der Produktfraktionen und Trocknen des Feststoffs im Hochvakuum wurden 97 mg (77% d. Th.) von Enantiomer 1 erhalten (>99% ee, chirale analytische HPLC).
$^1$H-NMR (400 MHz, DMSO-$d_6$, δ/ppm): 8.03 (s, 1H), 4.58 (s, 2H), 4.16-4.00 (m, 3H), 3.76 (dd, 1H), 3.63 (sext, 1H), 3.53 (s, 3H), 3.50-3.42 (m, 2H), 3.41-3.33 (m, 2H), 3.21 (s, 3H), 2.83-2.68 (m, 2H), 2.43 (s, 3H), 1.05 (d, 3H).

Chirale analytische SFC [Säule: Daicel Chiralcel QX, 3 μm, 50 mm x 4.6 mm; Laufmittel: Kohlendioxid/Ethanol 7:3; Fluss: 3 ml/min; Temperatur: 40°C; Detektion: 210 nm]: $R_t$ = 2.74 min.

**Beispiel 474**

Methyl-[1-{[3-(2-methoxypropyl)-5-methyl-2,4-dioxo-1-(3,3,3-trifluorpropyl)-1,2,3,4-tetrahydrothieno[2,3-d]pyrimidin-6-yl]methyl}imidazolidin-2-yliden]carbamat (*Enantiomer 2*)

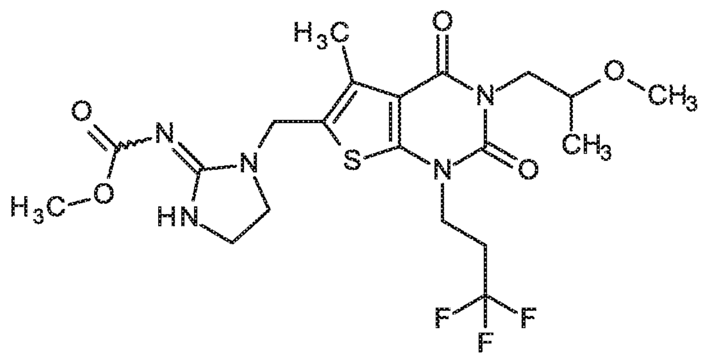

250 mg (494 mmol) der racemischen Verbindung aus Bsp. 472 wurden in 25 ml Methanol gelöst und in 9 Portionen über präparative SFC an chiraler Phase in die Enantiomere aufgetrennt [Säule: Daicel Chiralcel OX-H, 5 μm, 250 mm x 30 mm; Laufmittel: Kohlendioxid/Ethanol 7:3; Fluss: 100 ml/min; Temperatur: 40°C; Detektion: 210 nm]. Nach Eindampfen der Produktfraktionen und Trocknen des Feststoffs im Hochvakuum wurden 104 mg (83% d. Th.) von Enantiomer 2 erhalten (>99% ee, chirale analytische HPLC).
$^1$H-NMR (400 MHz, DMSO-$d_6$, δ/ppm): 8.03 (s, 1H), 4.58 (s, 2H), 4.17-3.99 (m, 3H), 3.76 (dd, 1H), 3.63 (sext, 1H), 3.53 (s, 3H), 3.50-3.42 (m, 2H), 3.40-3.33 (m, 2H), 3.21 (s, 3H), 2.83-2.68 (m, 2H), 2.43 (s, 3H), 1.05 (d, 3H).

Chirale analytische SFC [Säule: Daicel Chiralcel QX, 3 μm, 50 mm x 4.6 mm; Laufmittel: Kohlendioxid/Ethanol

7:3; Fluss: 3 ml/min; Temperatur: 40°C; Detektion: 210 nm]: $R_t$ = 5.07 min.

**Beispiel 475**

6-[(2,3-Dioxopiperazin-1-yl)methyl]-3-ethyl-5-methyl-1-(tetrahydrofuran-2-ylmethyl)thieno-[2,3-d]pyrimidin-2,4(1*H*,3*H*)-dion (*Racemat*)

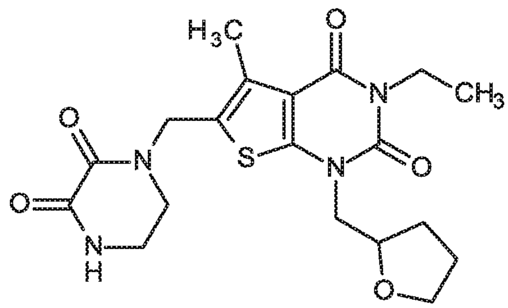

1.27 g (2.77 mmol, 80% Reinheit) der Verbindung aus Bsp. 225A wurden in 50 ml Ethanol gelöst und mit 708 µl (5.13 mmol) Oxalsäurediethylester versetzt. Das Gemisch wurde ca. 16 h bei 80°C gerührt. Danach wurde die Reaktionslösung am Rotationsverdampfer eingeengt. Das erhaltene Rohprodukt wurde mittels präparativer HPLC gereinigt (Methode 8). Nach Vereinigen der Produktfraktionen, Eindampfen und Trocknen im Hochvakuum wurden 447 mg (38% d. Th.) der Titelverbindung erhalten.
$^1$H-NMR (400 MHz, DMSO-$d_6$, δ/ppm): 8.63 (br. s, 1H), 4.69 (s, 2H), 4.28-4.17 (m, 1H), 4.03 (dd, 1H), 3.90 (q, 2H), 3.79-3.66 (m, 2H), 3.65-3.57 (m, 1H), 3.52-3.43 (m, 2H), 3.33-3.30 (m, 2H, fast vollständig vom Wasser-Signal verdeckt), 2.43 (s, 3H), 2.04-1.75 (m, 3H), 1.72-1.59 (m, 1H), 1.11 (t, 3H).
LC/MS (Methode 1, ESIpos): $R_t$ = 0.65 min, m/z = 421 $[M+H]^+$.

**Beispiel 476**

6-[(2,3-Dioxopiperazin-1-yl)methyl]-3-ethyl-5-methyl-1-(tetrahydrofuran-2-ylmethyl)thieno-[2,3-d]pyrimidin-2,4(1*H*,3*H*)-dion (*Enantiomer 1*)

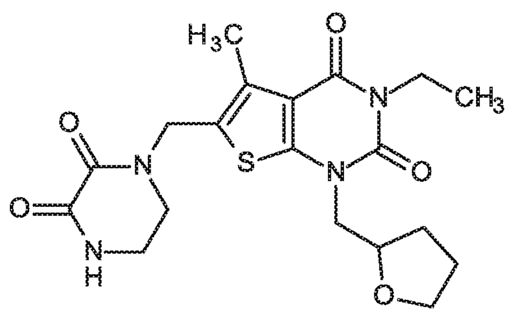

440 mg (1.05 mmol) der racemischen Verbindung aus Bsp. 475 wurden in einem Gemisch aus 12 ml Ethanol und 15 ml Acetonitril gelöst und in 36 Portionen über präparative HPLC an chiraler Phase in die Enantiomere aufgetrennt [Säule: Daicel Chiralcel OX-H, 5 µm, 250 mm x 20 mm; Laufmittel: Ethanol; Fluss: 15 ml/min; Temperatur: 25°C; Detektion: 210 nm]. Nach Eindampfen der Produktfraktionen und Trocknen des Feststoffs im Hochvakuum wurden 142 mg (64% d. Th.) von Enantiomer 1 erhalten (99.0% ee, chirale analytische HPLC).
$^1$H-NMR (400 MHz, DMSO-$d_6$, δ/ppm): 8.61 (br. s, 1H), 4.69 (s, 2H), 4.28-4.17 (m, 1H), 4.03 (dd, 1H), 3.90 (q, 2H), 3.78-3.67 (m, 2H), 3.65-3.57 (m, 1H), 3.51-3.44 (m, 2H), 3.33-3.29 (m, 2H, fast vollständig vom Wasser-Signal verdeckt), 2.43 (s, 3H), 2.05-1.75 (m, 3H), 1.71-1.58 (m, 1H), 1.11 (t, 3H).
Chirale analytische HPLC [Säule: Daicel Chiralpak OX-H, 3 µm, 50 mm x 4.6 mm; Laufmittel: Ethanol; Fluss: 1 ml/min; Temperatur: 40°C; Detektion: 220 nm]: $R_t$ = 2.31 min.

**Beispiel 477**

6-[(2,3-Dioxopiperazin-1-yl)methyl]-3-ethyl-5-methyl-1-(tetrahydrofuran-2-ylmethyl)thieno-[2,3-d]pyrimidin-2,4(1*H*,3*H*)-dion (*Enantiomer 2*)

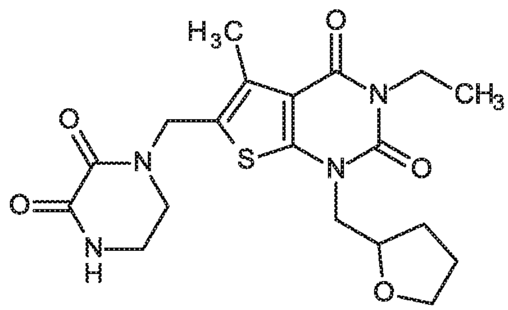

440 mg (1.05 mmol) der racemischen Verbindung aus Bsp. 475 wurden in einem Gemisch aus 12 ml Ethanol und 15 ml Acetonitril gelöst und in 36 Portionen über präparative HPLC an chiraler Phase in die Enantiomere aufgetrennt [Säule: Daicel Chiralcel OX-H, 5 µm, 250 mm x 20 mm; Laufmittel: Ethanol; Fluss: 15 ml/min; Temperatur: 25°C; Detektion: 210 nm]. Nach Eindampfen der Produktfraktionen und Trocknen des Feststoffs im Hochvakuum wurden 149 mg (67% d. Th.) von Enantiomer 2 erhalten (99.0% ee, chirale analytische HPLC).
$^1$H-NMR (400 MHz, DMSO-$d_6$, δ/ppm): 8.61 (br. s, 1H), 4.69 (s, 2H), 4.27-4.17 (m, 1H), 4.03 (dd, 1H), 3.90 (q, 2H), 3.78-3.67 (m, 2H), 3.65-3.56 (m, 1H), 3.51-3.44 (m, 2H), 3.33-3.29 (m, 2H, fast vollständig vom Wasser-Signal verdeckt), 2.43 (s, 3H), 2.04-1.75 (m, 3H), 1.69-1.61 (m, 1H), 1.11 (t, 3H).
Chirale analytische HPLC [Säule: Daicel Chiralpak OX-H, 3 µm, 50 mm x 4.6 mm; Laufmittel: Ethanol; Fluss: 1 ml/min; Temperatur: 40°C; Detektion: 220 nm]: $R_t$ = 3.28 min.

**Beispiel 478**

6-[(2,3-Dioxopiperazin-1-yl)methyl]-3-isopropyl-5-methyl-1-(3,3,3-trifluorpropyl)thieno[2,3-d]-pyrimidin-2,4(1*H*,3*H*)-dion

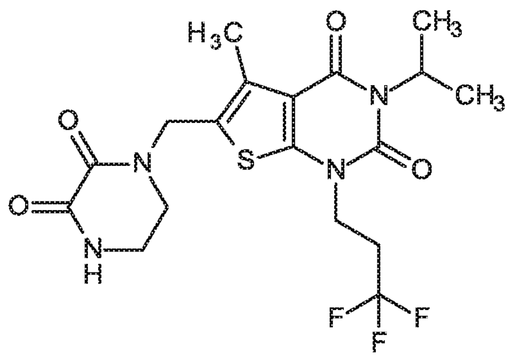

Analog zu dem unter Bsp. 397 beschriebenen Verfahren wurden aus 470 mg (0.934 mmol, 78% Reinheit) der Verbindung aus Bsp. 309A und 238 µl (1.73 mmol) Oxalsäurediethylester 118 mg (27% d. Th.) der Titelverbindung erhalten.
$^1$H-NMR (400 MHz, DMSO-$d_6$, δ/ppm): 8.62 (br. s, 1H), 5.13 (sept, 1H), 4.70 (s, 2H), 4.07 (br. t, 2H), 3.54-3.43 (m, 2H), 3.34-3.29 (m, 2H, fast vollständig vom Wasser-Signal verdeckt), 2.85-2.67 (m, 2H), 2.43 (s, 3H), 1.40 (d, 6H).
LC/MS (Methode 17, ESIneg): $R_t$ = 1.49 min, m/z = 491.12 $[M-H+HCO_2H]^-$.

**Beispiel 479**

6-[(2,3-Dioxopiperazin-1-yl)methyl]-3-isopropyl-1-(2-methoxyethyl)-5-methylthieno[2,3-d]-pyrimidin-2,4(1*H*,3*H*)-dion

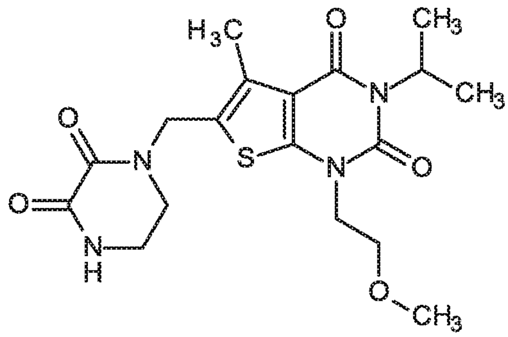

Analog zu dem unter Bsp. 397 beschriebenen Verfahren wurden aus 730 mg (1.65 mmol, 80% Reinheit) der Verbindung aus Bsp. 310A und 420 µl (3.05 mmol) Oxalsäurediethylester 200 mg (29% d. Th.) der Titelverbindung erhalten.
$^1$H-NMR (400 MHz, DMSO-$d_6$, δ/ppm): 8.61 (br. s, 1H), 5.13 (sept, 1H), 4.68 (s, 2H), 3.99 (t, 2H), 3.61 (t, 2H), 3.51-3.42 (m, 2H), 3.33-3.28 (m, 2H, fast vollständig vom Wasser-Signal verdeckt), 3.24 (s, 3H), 2.41 (s, 3H), 1.40 (d, 6H).
LC/MS (Methode 17, ESIneg): $R_t$ = 1.24 min, m/z = 407.14 [M-H]$^-$.

**Beispiel 480**

6-[(2,3-Dioxopiperazin-1-yl)methyl]-3-isopropyl-5-methyl-1-(tetrahydrofuran-2-ylmethyl)thieno-[2,3-d]pyrimidin-2,4(1*H*,3*H*)-dion (*Racemat*)

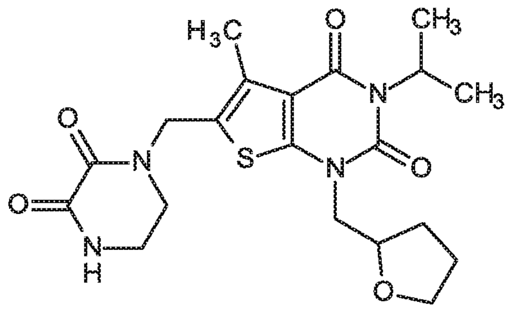

Analog zu dem unter Bsp. 397 beschriebenen Verfahren wurden aus 560 mg (1.18 mmol, 80% Reinheit) der Verbindung aus Bsp. 312A und 300 µl (2.18 mmol) Oxalsäurediethylester 234 mg (44% d. Th.) der Titelverbindung erhalten. Das Produkt wurde hier nach der HPLC-Reinigung noch mit Acetonitril bei RT verrührt.
$^1$H-NMR (400 MHz, DMSO-$d_6$, δ/ppm): 8.61 (br. s, 1H), 5.14 (sept, 1H), 4.68 (d, 2H), 4.27-4.15 (m, 1H), 4.02 (dd, 1H), 3.78-3.70 (m, 1H), 3.69-3.56 (m, 2H), 3.52-3.41 (m, 2H), 3.33-3.29 (m, 2H, fast vollständig vom Wasser-Signal verdeckt), 2.42 (s, 3H), 2.04-1.74 (m, 3H), 1.71-1.59 (m, 1H), 1.40 (dd, 6H).
LC/MS (Methode 1, ESIpos): $R_t$ = 0.73 min, m/z = 435 [M+H]$^+$.

**Beispiel 481**

6-[(2,3-Dioxopiperazin-1-yl)methyl]-3-isopropyl-5-methyl-1-(tetrahydrofuran-2-ylmethyl)thieno-[2,3-d]pyrimidin-2,4(1*H*,3*H*)-dion (*Enantiomer 1*)

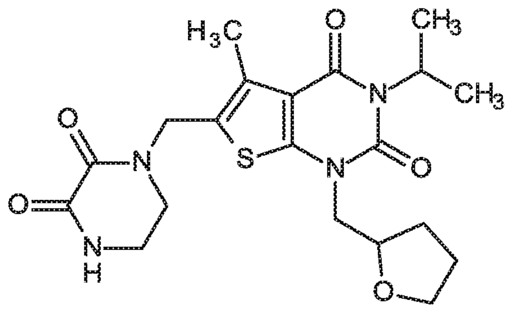

213 mg (0.490 mmol) der racemischen Verbindung aus Bsp. 480 wurden in einem Gemisch aus 8 ml Ethanol und 12 ml Acetonitril gelöst und in 27 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 25°C; Detektion: 210 nm]. Nach Eindampfen der Produktfraktionen und Trocknen des Feststoffs im Hochvakuum wurden 89 mg (83% d. Th.) von Enantiomer 1 erhalten (99.0% ee, chirale analytische HPLC).
$^1$H-NMR (400 MHz, DMSO-$d_6$, δ/ppm): 8.61 (br. s, 1H), 5.14 (sept, 1H), 4.68 (d, 2H), 4.26-4.15 (m, 1H), 4.02 (dd, 1H), 3.78-3.70 (m, 1H), 3.69-3.56 (m, 2H), 3.51-3.42 (m, 2H), 3.33-3.29 (m, 2H, fast vollständig vom Wasser-Signal verdeckt), 2.42 (s, 3H), 2.03-1.74 (m, 3H), 1.71-1.58 (m, 1H), 1.40 (dd, 6H).
Chirale analytische HPLC [Säule: Daicel Chiralpak OX-3, 3 μm, 50 mm x 4.6 mm; Laufmittel: Ethanol; Fluss: 1 ml/min; Temperatur: 25°C; Detektion: 220 nm]: $R_t$ = 1.63 min.

## Beispiel 482

6-[(2,3-Dioxopiperazin-1-yl)methyl]-3-isopropyl-5-methyl-1-(tetrahydrofuran-2-ylmethyl)thieno-[2,3-d]pyrimidin-2,4(1*H*,3*H*)-dion (*Enantiomer 2*)

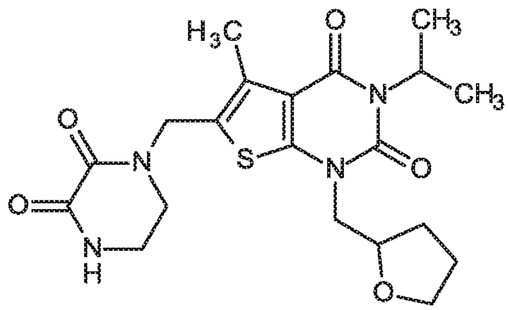

213 mg (0.490 mmol) der racemischen Verbindung aus Bsp. 480 wurden in einem Gemisch aus 8 ml Ethanol und 12 ml Acetonitril gelöst und in 27 Portionen über präparative HPLC an chiraler Phase in die Enantiomere aufgetrennt [Säule: Daicel Chiralcel OX-H, 5 μm, 250 mm x 20 mm; Laufmittel: Ethanol; Fluss: 15 ml/min; Temperatur: 25°C; Detektion: 210 nm]. Nach Eindampfen der Produktfraktionen und Trocknen des Feststoffs im Hochvakuum wurden 88 mg (82% d. Th.) von Enantiomer 2 erhalten (99.0% ee, chirale analytische HPLC).
$^1$H-NMR (400 MHz, DMSO-$d_6$, δ/ppm): 8.61 (br. s, 1H), 5.14 (sept, 1H), 4.68 (d, 2H), 4.28-4.15 (m, 1H), 4.02 (dd, 1H), 3.79-3.70 (m, 1H), 3.69-3.56 (m, 2H), 3.50-3.42 (m, 2H), 3.33-3.29 (m, 2H, fast vollständig vom Wasser-Signal verdeckt), 2.42 (s, 3H), 2.04-1.75 (m, 3H), 1.72-1.58 (m, 1H), 1.40 (dd, 6H).
Chirale analytische HPLC [Säule: Daicel Chiralpak OX-3, 3 μm, 50 mm x 4.6 mm; Laufmittel: Ethanol; Fluss: 1 ml/min; Temperatur: 25°C; Detektion: 220 nm]: $R_t$ = 2.33 min.

## Beispiel 483

3-(2,2-Dimethylpropyl)-6-[(2,3-dioxopiperazin-1-yl)methyl]-5-methyl-1-(tetrahydrofuran-2-ylmethyl)thieno[2,3-d]pyrimidin-2,4(1*H*,3*H*)-dion (*Racemat*)

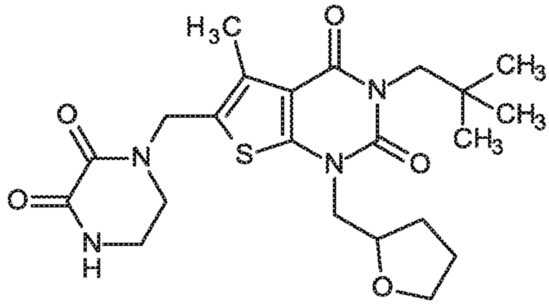

560 mg (1.17 mmol, 85% Reinheit) der Verbindung aus Bsp. 454A wurden in 25 ml Ethanol gelöst und mit 297 µl (2.16 mmol) Oxalsäurediethylester versetzt. Das Gemisch wurde ca. 16 h bei 80°C gerührt. Danach wurde die Reaktionslösung am Rotationsverdampfer eingeengt. Der verbliebene Feststoff wurde in Ethylacetat gelöst und nacheinander mit gesättigter Natriumcarbonat-Lösung und gesättigter Natriumchlorid-Lösung gewaschen. Nach Trocknen über wasserfreiem Magnesiumsulfat wurde filtriert und erneut eingeengt. Das erhaltene Rohprodukt wurde mittels präparativer HPLC gereinigt (Methode 8). Nach Vereinigen der Produktfraktionen, Einengen und Trocknen im Hochvakuum wurden 268 mg (49% d. Th.) der Titelverbindung erhalten.
$^1$H-NMR (500 MHz, DMSO-$d_6$, δ/ppm): 8.61 (br. s, 1H), 4.69 (s, 2H), 4.27-4.17 (m, 1H), 4.01 (br. dd, 1H), 3.82 (br. s, 2H), 3.77-3.68 (m, 2H), 3.64-3.57 (m, 1H), 3.53-3.45 (m, 2H), 3.34-3.30 (m, 2H, fast vollständig vom Wasser-Signal verdeckt), 2.42 (s, 3H), 2.02-1.76 (m, 3H), 1.69-1.63 (m, 1H), 0.89 (s, 9H).
LC/MS (Methode 17, ESIneg): $R_t$ = 1.56 min, m/z = 461.19 [M-H]$^-$.

**Beispiel 484**

3-(2,2-Dimethylpropyl)-6-[(2,3-dioxopiperazin-1-yl)methyl]-5-methyl-1-(tetrahydrofuran-2-ylmethyl)thieno[2,3-d]pyrimidin-2,4(1*H*,3*H*)-dion (*Enantiomer 1*)

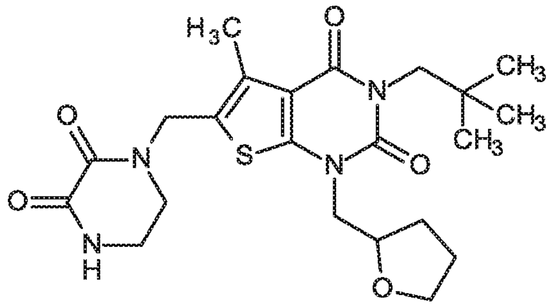

258 mg (0.558 mmol) der racemischen Verbindung aus Bsp. 483 wurden in 6 ml Acetonitril gelöst und in 20 Portionen über präparative HPLC an chiraler Phase in die Enantiomere aufgetrennt [Säule: Daicel Chiralpak IE, 5 µm, 250 mm x 20 mm; Laufmittel: *tert.*-Butylmethylether/Acetonitril 1:1; Fluss: 15 ml/min; Temperatur: 30°C; Detektion: 220 nm]. Nach Eindampfen der Produktfraktionen und Trocknen des Feststoffs im Hochvakuum wurden 69 mg (53% d. Th.) von Enantiomer 1 erhalten (98.0% ee, chirale analytische HPLC).
$^1$H-NMR (400 MHz, DMSO-$d_6$, δ/ppm): 8.08 (s, 1H), 5.08-4.95 (m, 1H), 4.70 (q, 2H), 4.54-4.36 (m, 2H), 4.49 (s, 2H), 4.26-4.07 (m, 2H), 3.52-3.36 (m, 4H), 2.76-2.62 (m, 1H), 2.55-2.46 (m, 1H, weitgehend überdeckt vom DMSO-Signal), 2.41 (s, 3H).

Chirale analytische HPLC [Säule: Daicel Chiralpak IE, 5 µm, 250 mm x 4.6 mm; Laufmittel: *tert.*-Butylmethylether/Acetonitril 1:1; Fluss: 1 ml/min; Temperatur: 30°C; Detektion: 220 nm]: $R_t$ = 13.13 min.

**Beispiel 485**

3-(2,2-Dimethylpropyl)-6-[(2,3-dioxopiperazin-1-yl)methyl]-5-methyl-1-(tetrahydrofuran-2-ylmethyl)thieno[2,3-d]pyrimidin-2,4(1*H*,3*H*)-dion (*Enantiomer 2*)

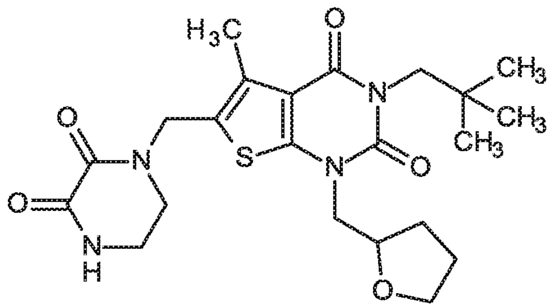

258 mg (0.558 mmol) der racemischen Verbindung aus Bsp. 483 wurden in 6 ml Acetonitril gelöst und in 20 Portionen über präparative HPLC an chiraler Phase in die Enantiomere aufgetrennt [Säule: Daicel Chiralpak IE, 5 µm, 250 mm x 20 mm; Laufmittel: *tert.*-Butylmethylether/Acetonitril 1:1; Fluss: 15 ml/min; Temperatur: 30°C; Detektion: 220 nm]. Nach Eindampfen der Produktfraktionen und Trocknen des Feststoffs im Hochvakuum wurden 83 mg (64% d. Th.) von Enantiomer 2 erhalten (91.0% ee, chirale analytische HPLC).
$^1$H-NMR (400 MHz, DMSO-$d_6$, δ/ppm): 8.61 (br. s, 1H), 4.69 (s, 2H), 4.27-4.16 (m, 1H), 4.01 (dd, 1H), 3.82 (br. s, 2H), 3.78-3.68 (m, 2H), 3.66-3.56 (m, 1H), 3.53-3.45 (m, 2H), 3.35-3.30 (m, 2H, fast vollständig vom Wasser-Signal verdeckt), 2.42 (s, 3H), 2.03-1.74 (m, 3H), 1.72-1.59 (m, 1H), 0.89 (s, 9H).

Chirale analytische HPLC [Säule: Daicel Chiralpak IE, 5 µm, 250 mm x 4.6 mm; Laufmittel: *tert.*-Butylmethylether/Acetonitril 1:1; Fluss: 1 ml/min; Temperatur: 30°C; Detektion: 220 nm]: $R_t$ = 14.02 min.

**Beispiel 486**

6-[(2,3-Dioxopiperazin-1-yl)methyl]-1-(2-methoxyethyl)-5-methyl-3-(2,2,2-trifluorethyl)thieno-[2,3-d]pyrimidin-2,4(1*H*,3*H*)-dion

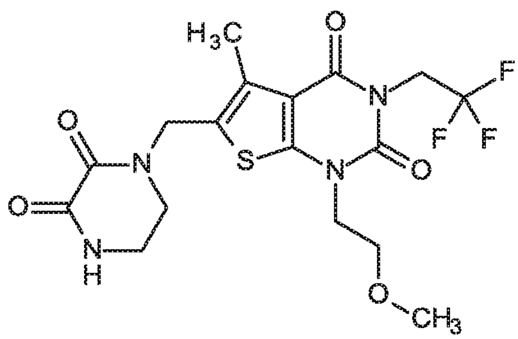

Analog zu dem unter Bsp. 397 beschriebenen Verfahren wurden aus 500 mg (1.01 mmol, 80% Reinheit) der Verbindung aus Bsp. 249A und 259 µl (1.88 mmol) Oxalsäurediethylester 151 mg (33% d. Th.) der Titelverbindung erhalten.
$^1$H-NMR (400 MHz, DMSO-$d_6$, δ/ppm): 8.62 (br. s, 1H), 4.70 (q, 2H), 4.70 (s, 2H), 4.06 (t, 2H), 3.64 (t, 2H), 3.54-3.46 (m, 2H), 3.34-3.29 (m, 2H, fast vollständig vom Wasser-Signal verdeckt), 3.23 (s, 3H), 2.43 (s, 3H).
LC/MS (Methode 1, ESIneg): $R_t$ = 0.69 min, m/z = 493 $[M-H+HCO_2H]^-$.

**Beispiel 487**

6-[(2,3-Dioxopiperazin-1-yl)methyl]-5-methyl-1-(tetrahydrofuran-2-ylmethyl)-3-(2,2,2-trifluorethyl)thieno[2,3-d]pyrimidin-2,4(1*H*,3*H*)-dion (*Racemat*)

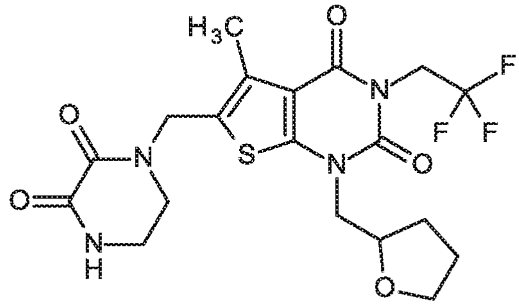

Analog zu dem unter Bsp. 397 beschriebenen Verfahren wurden aus 545 mg (1.04 mmol, 80% Reinheit) der Verbindung aus Bsp. 252A und 265 µl (1.92 mmol) Oxalsäurediethylester 275 mg (55% d. Th.) der Titelverbindung erhalten.

$^1$H-NMR (500 MHz, DMSO-$d_6$, δ/ppm): 8.62 (br. s, 1H), 4.70 (q, 2H), 4.70 (s, 2H), 4.27-4.17 (m, 1H), 4.06 (dd, 1H), 3.80-3.70 (m, 2H), 3.65-3.57 (m, 1H), 3.54-3.44 (m, 2H), 3.33-3.29 (m, 2H, fast vollständig vom Wasser-Signal verdeckt), 2.43 (s, 3H), 2.04-1.76 (m, 3H), 1.72-1.60 (m, 1H).

LC/MS (Methode 17, ESIneg): $R_t$ = 1.34 min, m/z = 519.12 $[M-H+HCO_2H]^-$.

## Beispiel 488

6-[(2,3-Dioxopiperazin-1-yl)methyl]-5-methyl-1-(tetrahydrofuran-2-ylmethyl)-3-(2,2,2-trifluorethyl)thieno[2,3-d]pyrimidin-2,4(1*H*,3*H*)-dion (*Enantiomer 1*)

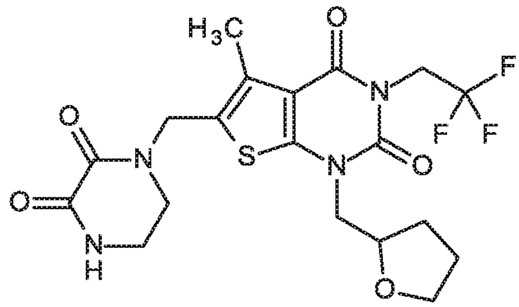

257 mg (0.542 mmol) der racemischen Verbindung aus Bsp. 487 wurden in einem Gemisch aus jeweils 15 ml Acetonitril und Methanol gelöst und in 60 Portionen über präparative SFC an chiraler Phase in die Enantiomere aufgetrennt [Säule: Daicel Chiralcel OX-H, 5 µm, 250 mm x 20 mm; Laufmittel: Kohlendioxid/Ethanol 72:28; Fluss: 80 ml/min; Temperatur: 40°C; Detektion: 210 nm]. Nach Eindampfen der Produktfraktionen und Trocknen des Feststoffs im Hochvakuum wurden 57 mg (44% d. Th.) von Enantiomer 1 erhalten (>99% ee, chirale analytische HPLC).

$^1$H-NMR (400 MHz, DMSO-$d_6$, δ/ppm): 8.62 (br. s, 1H), 4.70 (q, 2H), 4.70 (s, 2H), 4.27-4.17 (m, 1H), 4.06 (dd, 1H), 3.81-3.69 (m, 2H), 3.66-3.57 (m, 1H), 3.54-3.45 (m, 2H), 3.34-3.29 (m, 2H, fast vollständig vom Wasser-Signal verdeckt), 2.43 (s, 3H), 2.05-1.75 (m, 3H), 1.72-1.61 (m, 1H).

Chirale analytische SFC [Säule: Daicel Chiralcel QX, 3 µm, 50 mm x 4.6 mm; Laufmittel: Kohlendioxid/Methanol 7:3; Fluss: 3 ml/min; Temperatur: 40°C; Detektion: 210 nm]: $R_t$ = 4.73 min.

## Beispiel 489

6-[(2,3-Dioxopiperazin-1-yl)methyl]-5-methyl-1-(tetrahydrofuran-2-ylmethyl)-3-(2,2,2-trifluorethyl)thieno[2,3-d]pyrimidin-2,4(1*H*,3*H*)-dion (*Enantiomer 2*)

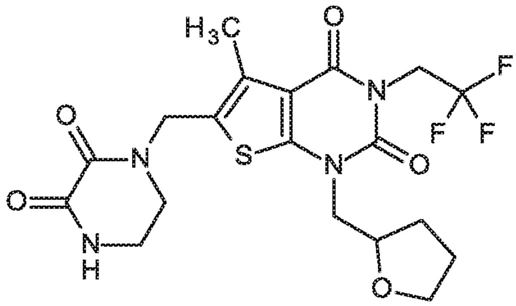

257 mg (0.542 mmol) der racemischen Verbindung aus Bsp. 487 wurden in einem Gemisch aus jeweils 15 ml Acetonitril und Methanol gelöst und in 60 Portionen über präparative SFC an chiraler Phase in die Enantiomere aufgetrennt [Säule: Daicel Chiralcel OX-H, 5 µm, 250 mm x 20 mm; Laufmittel: Kohlendioxid/Ethanol 72:28; Fluss: 80 ml/min; Temperatur: 40°C; Detektion: 210 nm]. Nach Eindampfen der Produktfraktionen und Trocknen des Feststoffs im Hochvakuum wurden 73 mg (56% d. Th.) von Enantiomer 2 erhalten (>99% ee, chirale analytische HPLC).

$^1$H-NMR (400 MHz, DMSO-$d_6$, δ/ppm): 8.62 (br. s, 1H), 4.70 (q, 2H), 4.70 (s, 2H), 4.27-4.17 (m, 1H), 4.06 (dd, 1H), 3.81-3.68 (m, 2H), 3.66-3.56 (m, 1H), 3.55-3.43 (m, 2H), 3.34-3.29 (m, 2H, fast vollständig vom Wasser-Signal verdeckt), 2.43 (s, 3H), 2.05-1.75 (m, 3H), 1.72-1.61 (m, 1H).

Chirale analytische SFC [Säule: Daicel Chiralcel QX, 3 µm, 50 mm x 4.6 mm; Laufmittel: Kohlendioxid/Methanol 7:3; Fluss: 3 ml/min; Temperatur: 40°C; Detektion: 210 nm]: $R_t$ = 7.39 min.

**Beispiel 490**

6-[(2,3-Dioxopiperazin-1-yl)methyl]-5-methyl-3-(1,1,1-trifluorpropan-2-yl)-1-(3,3,3-trifluorpropyl)thieno[2,3-d]pyrimidin-2,4(1*H*,3*H*)-dion (*Racemat*)

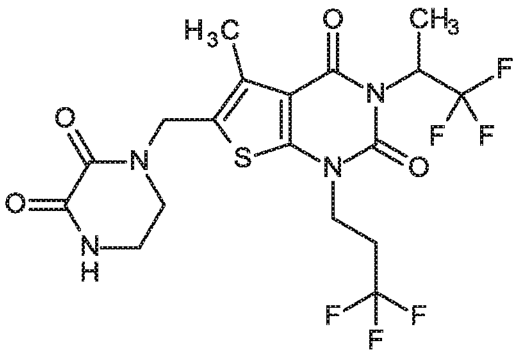

590 mg (1.11 mmol, 84% Reinheit) der Verbindung aus Bsp. 455A wurden in 40 ml Ethanol gelöst und mit 283 µl (2.05 mmol) Oxalsäurediethylester versetzt. Das Gemisch wurde ca. 16 h bei 80°C gerührt. Danach wurde die Reaktionslösung am Rotationsverdampfer eingeengt. Das erhaltene Rohprodukt wurde mittels präparativer HPLC gereinigt (Methode 8). Nach Vereinigen der Produktfraktionen, Einengen und Trocknen im Hochvakuum wurden 205 mg (36% d. Th.) der Titelverbindung erhalten.

$^1$H-NMR (400 MHz, DMSO-$d_6$, δ/ppm): 8.63 (br. s, 1H), 5.83-5.47 (m, 1H), 4.72 (s, 2H), 4.22-4.02 (m, 2H), 3.59-3.45 (m, 2H), 2.87-2.69 (m, 2H), 2.44 (s, 3H), 1.63 (br. d, 3H).

LC/MS (Methode 17, ESIneg): $R_t$ = 1.63 min, m/z = 545.09 $[M-H+HCO_2H]^-$.

**Beispiel 491**

6-[(2,3-Dioxopiperazin-1-yl)methyl]-1-(2-methoxyethyl)-5-methyl-3-(1,1,1-trifluorpropan-2-yl)-thieno[2,3-d]pyrimidin-2,4(1*H*,3*H*)-dion (*Racemat*)

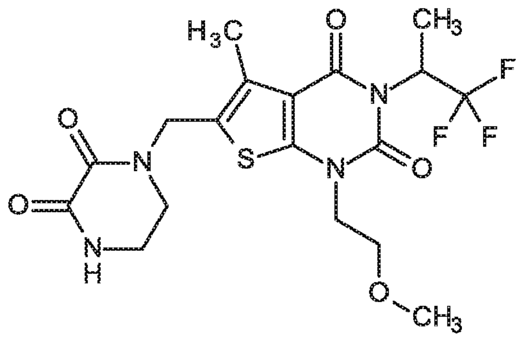

Analog zu dem unter Bsp. 397 beschriebenen Verfahren wurden aus 295 mg (0.592 mmol, 82% Reinheit) der Verbindung aus Bsp. 456A und 151 µl (1.10 mmol) Oxalsäurediethylester 155 mg (56% d. Th.) der Titelverbindung erhalten.

$^1$H-NMR (400 MHz, DMSO-$d_6$, δ/ppm): 8.62 (br. s, 1H), 5.84-5.45 (m, 1H), 4.69 (s, 2H), 4.12-3.95 (m, 2H), 3.70-3.56 (m, 2H), 3.55-3.43 (m, 2H), 3.34-3.29 (m, 2H, fast vollständig vom Wasser-Signal verdeckt), 3.23 (s, 3H), 2.42 (s, 3H), 1.63 (br. d, 3H).

LC/MS (Methode 17, ESIneg): $R_t$ = 1.38 min, m/z = 507.12 $[M-H+HCO_2H]^-$.

**Beispiel 492**

6-[(2,3-Dioxopiperazin-1-yl)methyl]-3-(2-ethoxyethyl)-1-(3-fluorpropyl)-5-methylthieno[2,3-d]-pyrimidin-2,4(1*H*,3*H*)dion

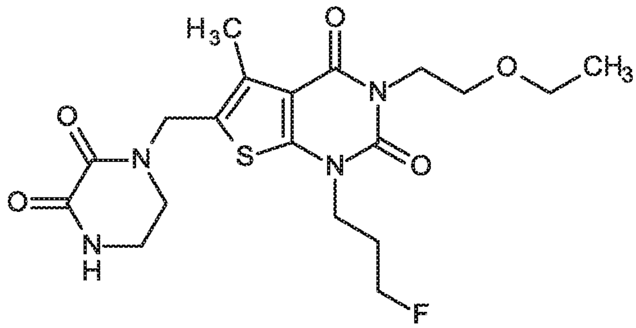

200 mg (0.388 mmol, 75% Reinheit) der Verbindung aus Bsp. 457A wurden in 6.6 ml Ethanol gelöst und mit 573 mg (3.88 mmol) Oxalsäurediethylester versetzt. Das Gemisch wurde 16 h bei 80°C gerührt. Danach wurde die Reaktionslösung am Rotationsverdampfer eingeengt. Der Rückstand wurde in 30 ml Dichlormethan aufgenommen und diese Lösung mit Wasser und gesättigter wässriger Natriumhydrogencarbonat-Lösung gewaschen. Nach Trocknen über wasserfreiem Natriumsulfat wurde filtriert und eingeengt. Der verbliebene Rückstand wurde in 3 ml DMSO gelöst und diese Lösung mittels präparativer HPLC gereinigt (Methode 14). Nach Vereinigen der Produktfraktionen und Gefriertrocknung wurden 118 mg (66% d. Th.) der Titelverbindung erhalten.

$^1$H-NMR (500 MHz, DMSO-$d_6$, δ/ppm): 8.64 (br. s, 1H), 4.70 (s, 2H), 4.59 (t, 1H), 4.47 (t, 1H), 4.03 (t, 2H), 3.98 (br. t, 2H), 3.53-3.47 (m, 4H), 3.44 (q, 2H), 3.33-3.29 (m, 2H), 2.43 (s, 3H), 2.13-1.98 (m, 2H), 1.06 (t, 3H).

LC/MS (Methode 3, ESIpos): $R_t$ = 0.84 min, m/z = 441 $[M+H]^+$.

**Beispiel 493**

6-[(2,3-Dioxopiperazin-1-yl)methyl]-3-(2-methoxypropyl)-5-methyl-1-(3,3,3-trifluorpropyl)thieno-[2,3-d]pyrimidin-2,4(1*H*,3*H*)-dion (*Racemat*)

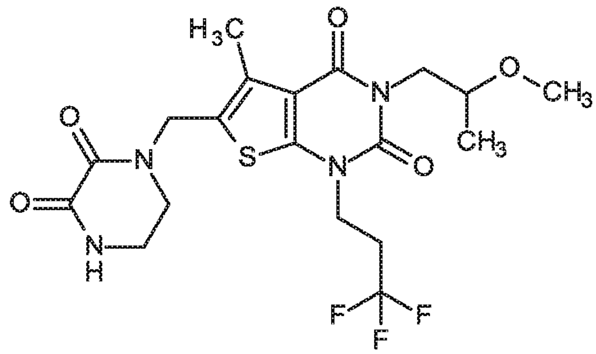

850 mg (1.61 mmol, 80% Reinheit) der Verbindung aus Bsp. 458A wurden in 55 ml Ethanol gelöst und mit 411 μl (2.98 mmol) Oxalsäurediethylester versetzt. Das Gemisch wurde ca. 16 h bei 80°C gerührt. Danach wurde die Reaktionslösung am Rotationsverdampfer eingeengt. Das erhaltene Rohprodukt wurde mittels MPLC gereinigt (Biotage Isolera, Kartusche mit 100 g Kieselgel, Dichlormethan/Methanol 20:1). Nach Vereinigen der Produktfraktionen, Eindampfen und Trocknen im Hochvakuum wurden 475 mg (59% d. Th., 96% Reinheit) der Titelverbindung erhalten.
$^1$H-NMR (400 MHz, DMSO-$d_6$, δ/ppm): 8.62 (br. s, 1H), 4.71 (s, 2H), 4.18-3.97 (m, 3H), 3.76 (dd, 1H), 3.63 (sext, 1H), 3.55-3.46 (m, 2H), 3.38-3.31 (m, 2H), 3.31 (s, 3H), 2.84-2.69 (m, 2H), 2.44 (s, 3H), 1.05 (d, 3H).
LC/MS (Methode 1, ESIpos): $R_t$ = 0.79 min, m/z = 477 $[M+H]^+$.

**Beispiel 494**

6-[(2,3-Dioxopiperazin-1-yl)methyl]-3-(2-methoxypropyl)-5-methyl-1-(3,3,3-trifluorpropyl)thieno-[2,3-d]pyrimidin-2,4(1*H*,3*H*)-dion (*Enantiomer 1*)

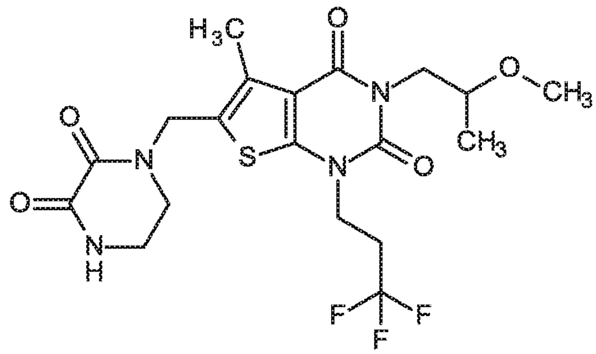

475 mg (0.997 mmol) der racemischen Verbindung aus Bsp. 493 wurden in 10 ml Ethanol gelöst und in 20 Portionen über präparative HPLC an chiraler Phase in die Enantiomere aufgetrennt [Säule: Daicel Chiralcel OX-H, 5 μm, 250 mm x 20 mm; Laufmittel: *n*-Heptan/Ethanol 3:7; Fluss: 15 ml/min; Temperatur: 25°C; Detektion: 210 nm]. Nach Eindampfen der Produktfraktionen und Trocknen des Feststoffs im Hochvakuum wurden 201 mg (84% d. Th.) von Enantiomer 1 erhalten (99.0% ee, chirale analytische HPLC).
$^1$H-NMR (500 MHz, DMSO-$d_6$, δ/ppm): 8.62 (br. s, 1H), 4.71 (s, 2H), 4.17-4.08 (m, 2H), 4.05 (dd, 1H), 3.76 (dd, 1H), 3.63 (sext, 1H), 3.54-3.46 (m, 2H), 3.34-3.29 (m, 2H, fast vollständig vom Wasser-Signal verdeckt), 3.21 (s, 3H), 2.83-2.70 (m, 2H), 2.44 (s, 3H), 1.05 (d, 3H).
Chirale analytische HPLC [Säule: Daicel Chiralpak OX-3, 3 μm, 50 mm x 4.6 mm; Laufmittel: Ethanol; Fluss: 1 ml/min; Temperatur: 25°C; Detektion: 220 nm]: $R_t$ = 1.24 min.

**Beispiel 495**

6-[(2,3-Dioxopiperazin-1-yl)methyl]-3-(2-methoxypropyl)-5-methyl-1-(3,3,3-trifluorpropyl)thieno-[2,3-d]pyrimidin-2,4(1*H*,3*H*)-dion (*Enantiomer 2*)

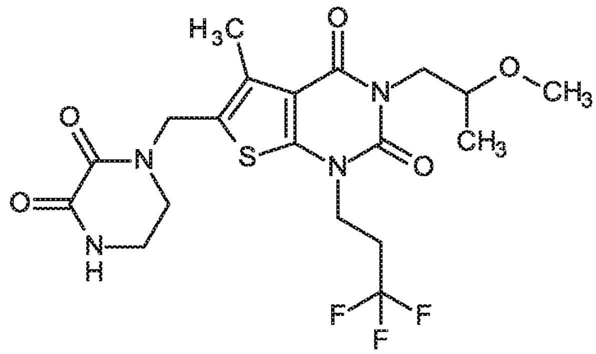

475 mg (0.997 mmol) der racemischen Verbindung aus Bsp. 493 wurden in 10 ml Ethanol gelöst und in 20 Portionen über präparative HPLC an chiraler Phase in die Enantiomere aufgetrennt [Säule: Daicel Chiralcel OX-H, 5 μm, 250 mm x 20 mm; Laufmittel: *n*-Heptan/Ethanol 3:7; Fluss: 15 ml/min; Temperatur: 25°C; Detektion: 210 nm]. Nach Eindampfen der Produktfraktionen und Trocknen des Feststoffs im Hochvakuum wurden 198 mg (83% d. Th.) von Enantiomer 2 erhalten (99.0% ee, chirale analytische HPLC).
$^1$H-NMR (500 MHz, DMSO-$d_6$, δ/ppm): 8.62 (br. s, 1H), 4.71 (s, 2H), 4.18-4.08 (m, 2H), 4.05 (dd, 1H), 3.76 (dd, 1H), 3.63 (sext, 1H), 3.56-3.46 (m, 2H), 3.35-3.29 (m, 2H, fast vollständig vom Wasser-Signal verdeckt), 3.21 (s, 3H), 2.83-2.69 (m, 2H), 2.44 (s, 3H), 1.05 (d, 3H).
Chirale analytische HPLC [Säule: Daicel Chiralpak OX-3, 3 μm, 50 mm x 4.6 mm; Laufmittel: Ethanol; Fluss: 1 ml/min; Temperatur: 25°C; Detektion: 220 nm]: $R_t$ = 1.51 min.

**Beispiel 496**

1-(3-Fluorpropyl)-3-isobutyl-6-[(4-isopropyl-2,3-dioxopiperazin-1-yl)methyl]-5-methylthieno-[2,3-d]pyrimidin-2,4(1*H*,3*H*)-dion

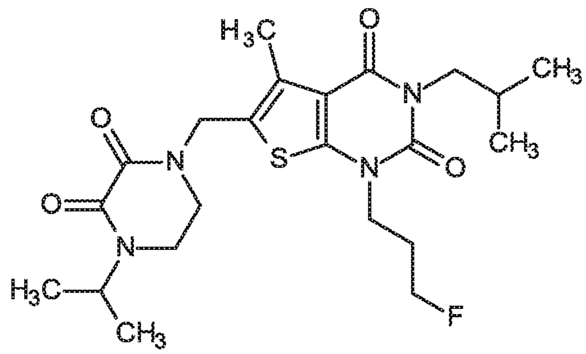

Die Titelverbindung (16 mg) wurde als Nebenprodukt der Herstellung und Aufreinigung der in Beispiel 121 beschriebenen Verbindung erhalten.
$^1$H-NMR (400 MHz, DMSO-$d_6$, δ/ppm): 4.69 (s, 2H), 4.59 (t, 1H), 4.56-4.44 (m, 2H), 3.99 (t, 2H), 3.71 (d, 2H), 3.54-3.47 (m, 2H), 3.44-3.38 (m, 2H), 2.44 (s, 3H), 2.15-1.96 (m, 3H), 1.08 (d, 6H), 0.85 (d, 6H).
LC/MS (Methode 3, ESIpos): $R_t$ = 1.14 min, m/z = 467 [M+H]$^+$.

**Beispiel 497**

6-[(2,5-Dioxopiperazin-1-yl)methyl]-3-ethyl-5-methyl-1-(3,3,3-trifluorpropyl)thieno[2,3-d]pyrimidin-2,4(1*H*,3*H*)-dion

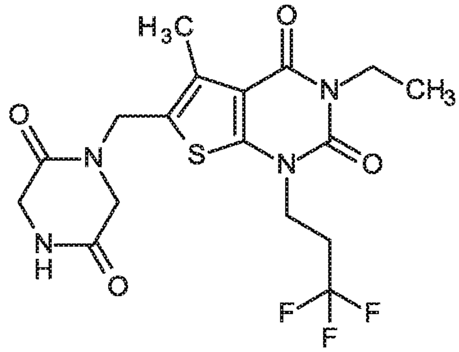

Eine Lösung von 100 mg (0.213 mmol) der Verbindung aus Bsp. 489A, 3.5 mg (0.021 mmol) Kaliumiodid und 26 mg (0.235 mmol) Kalium-*tert*.-butylat in 4 ml wasserfreiem THF wurde ca. 18 h bei RT gerührt. Anschließend wurde das Gemisch zur Trockene eingeengt. Der verbliebene Rückstand wurde mittels präparativer HPLC (Methode 9) in seine Komponenten aufgetrennt. Die Produktfraktion wurde eingedampft und der Rückstand im Hochvakuum getrocknet. Es wurden 4 mg (4% d. Th., 94% Reinheit) der Titelverbindung erhalten.
$^1$H-NMR (400 MHz, DMSO-$d_6$, δ/ppm): 8.15 (br. s, 1H), 4.65 (s, 2H), 4.09 (t, 2H), 3.90 (q, 2H), 3.87 (s, 2H), 3.85 (s, 3H), 2.84-2.69 (m, 2H), 2.45 (s, 3H), 1.11 (t, 3H).
LC/MS (Methode 17, ESIpos): $R_t$ = 1.30 min, m/z = 433.11 $[M+H]^+$.

**Beispiel 498**

1-(2-Methoxyethyl)-5-methyl-6-[(2-oxoimidazolidin-1-yl)methyl]-3-(1,1,1-trifluorpropan-2-yl)-thieno[2,3-d]pyrimidin-2,4(1*H*,3*H*)-dion (*Enantiomer 1*)

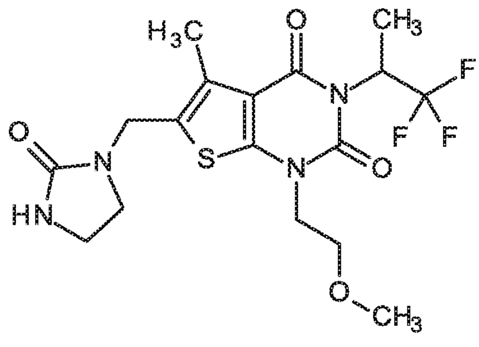

Eine Lösung von 300 mg (0.588 mmol, 80% Reinheit) der Verbindung aus Bsp. 514A und 123 µl (0.881 mmol) Triethylamin in 6 ml THF wurde mit 114 mg (0.705 mmol) CDI versetzt und ca. 18 h bei RT gerührt. Danach wurde das Reaktionsgemisch mit Ethylacetat verdünnt und nacheinander mit gesättigter Natriumcarbonat-Lösung und gesättigter Natriumchlorid-Lösung gewaschen. Nach Trocknen über wasserfreiem Magnesiumsulfat wurde filtriert und eingeengt. Der feste Rückstand wurde mittels präparativer HPLC gereinigt (Methode 8). Die Produktfraktionen wurden vereinigt, eingedampft und im Hochvakuum getrocknet. Es wurden 152 mg (59% d. Th.) der enantiomerenreinen Titelverbindung erhalten (>99% ee, chirale analytische HPLC).
$^1$H-NMR (400 MHz, DMSO-$d_6$, δ/ppm): 6.54 (s, 1H), 5.81-5.69 und 5.62-5.50 (2 m, zus. 1H), 4.35 (s, 2H), 4.06-3.99 (m, 2H), 3.66-3.60 (m, 2H), 3.28-3.18 (m, 4H), 3.24 (s, 3H), 2.38 und 2.37 (2 s, zus. 3H), 1.67 und 1.63 (2 d, zus. 3H).
LC/MS (Methode 17, ESIpos): $R_t$ = 1.57 min, m/z = 435.13 $[M+H]^+$.
Chirale analytische HPLC [Säule: Daicel Chiralpak AD-3, 3 µm, 50 mm x 4.6 mm; Laufmittel: Heptan/Ethanol

4:1; Fluss: 1 ml/min; Temperatur: 30°C; Detektion: 220 nm]: $R_t$ = 1.94 min.

Spezifische optische Drehung: $[\alpha]_D^{20}$ = -12.7°·ml·dm$^{-1}$·g$^{-1}$ (Methanol).

**Beispiel 499**

1-(2-Methoxyethyl)-5-methyl-6-[(2-oxoimidazolidin-1-yl)methyl]-3-(1,1,1-trifluorpropan-2-yl)-thieno[2,3-d]pyrimidin-2,4(1*H*,3*H*)-dion (*Enantiomer 2*)

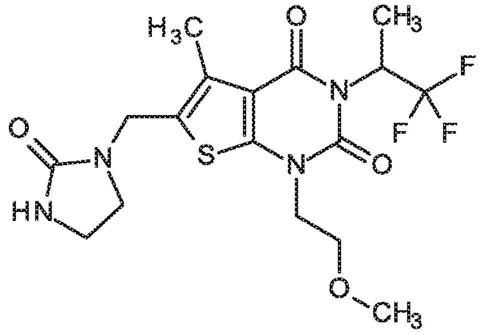

Eine Lösung von 300 mg (0.588 mmol, 80% Reinheit) der Verbindung aus Bsp. 515A und 123 µl (0.881 mmol) Triethylamin in 6 ml THF wurde mit 114 mg (0.705 mmol) CDI versetzt und ca. 18 h bei RT gerührt. Danach wurde das Reaktionsgemisch mit Ethylacetat verdünnt und nacheinander mit gesättigter Natriumcarbonat-Lösung und gesättigter Natriumchlorid-Lösung gewaschen. Nach Trocknen über wasserfreiem Magnesiumsulfat wurde filtriert und eingeengt. Der feste Rückstand wurde mittels präparativer HPLC gereinigt (Methode 8). Die Produktfraktionen wurden vereinigt, eingedampft und im Hochvakuum getrocknet. Es wurden 125 mg (48% d. Th.) der enantiomerenreinen Titelverbindung erhalten (>99% ee, chirale analytische HPLC).

$^1$H-NMR (400 MHz, DMSO-$d_6$, δ/ppm): 6.54 (s, 1H), 5.80-5.69 und 5.61-5.51 (2 m, zus. 1H), 4.35 (s, 2H), 4.06-3.99 (m, 2H), 3.65-3.60 (m, 2H), 3.28-3.19 (m, 4H), 3.24 (s, 3H), 2.38 und 2.37 (2 s, zus. 3H), 1.68 und 1.63 (2 d, zus. 3H).

LC/MS (Methode 17, ESIpos): $R_t$ = 1.57 min, m/z = 435.13 $[M+H]^+$.

Chirale analytische HPLC [Säule: Daicel Chiralpak AD-3, 3 µm, 50 mm x 4.6 mm; Laufmittel: Heptan/Ethanol 4:1; Fluss: 1 ml/min; Temperatur: 30°C; Detektion: 220 nm]: $R_t$ = 1.78 min.

Spezifische optische Drehung: $[\alpha]_D^{20}$ = +13.5°·ml·dm$^{-1}$·g$^{-1}$ (Methanol).

**Beispiel 500**

1-(2-Methoxyethyl)-5-methyl-6-[(2-oxoimidazolidin-1-yl)(dideutero)methyl]-3-(2,2,2-trifluorethyl)thieno[2,3-d]pyrimidin-2,4(1*H*,3*H*)-dion

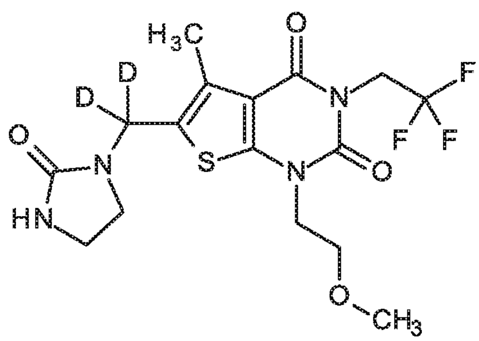

Eine Lösung von 117 mg (1.36 mmol) Imidazolidin-2-on in 3 ml DMF wurde mit 54 mg (1.36 mmol) Natriumhydrid (60% Suspension in Mineralöl) versetzt, dann 5 min auf 60°C erwärmt und anschließend wieder auf RT abgekühlt ("Lösung 1"). In einem anderen Reaktionsgefäß wurde eine Lösung von 120 mg (0.339 mmol) der Verbindung aus Bsp.

511A in 2.4 ml Dichlormethan bei 0°C mit 118 µl (0.677 mmol) *N,N*-Diisopropylethylamin und 26 µl (0.356 mmol) Thionylchlorid versetzt. Nach 20 min bei 0°C wurde die Lösung 1 portionsweise hinzugefügt. Das Reaktionsgemisch wurde anschließend 3.5 Tage bei RT gerührt. Danach wurde es mit Wasser versetzt und mit Ethylacetat extrahiert. Der organische Extrakt wurde mit gesättigter Natriumchlorid-Lösung gewaschen und über wasserfreiem Magnesiumsulfat getrocknet. Nach Filtration und Einengen wurde der Rückstand zunächst mittels MPLC vorgereinigt (Isolera, 25 g Kieselgel-Kartusche, Cyclohexan/ Ethylacetat 1:1 → Dichlormethan/Methanol 10:1). Die eingedampften produkthaltigen Fraktionen wurden anschließend in einem zweiten Chromatographieschritt mittels präparativer HPLC (Methode 8) weiter aufgereinigt. Nach erneutem Eindampfen und Trocknen der produkthaltigen Fraktionen schloss sich zur weiteren Reinigung noch eine dritte Chromatographie an (ebenfalls per präparativer HPLC). Nach abermaligem Eindampfen der Produktfraktionen und Trocknen im Hochvakuum wurden so 27 mg (18% d. Th.) der Titelverbindung erhalten.
$^1$H-NMR (400 MHz, DMSO-$d_6$, δ/ppm): 6.53 (s, 1H), 4.70 (q, 2H), 4.05 (t, 2H), 3.67-3.59 (m, 2H), 3.29-3.16 (m, 4H), 3.24 (s, 3H), 2.39 (s, 3H).
LC/MS (Methode 1, ESIpos): $R_t$ = 0.75 min, m/z = 423 $[M+H]^+$.

**Beispiel 501**

1-(2-Methoxyethyl)-5-methyl-6-[(2-oxoimidazolidin-1-yl)(dideutero)methyl]-3-[(2*R*)-1,1,1-trifluorpropan-2-yl]thieno[2,3-d]pyrimidin-2,4(1*H*,3*H*)-dion

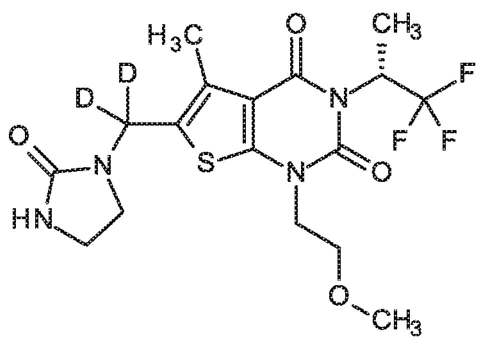

Eine Lösung von 416 mg (4.83 mmol) Imidazolidin-2-on in 12 ml DMF wurde mit 193 mg (4.83 mmol) Natriumhydrid (60% Suspension in Mineralöl) versetzt, dann 5 min auf 60°C erwärmt und anschließend wieder auf RT abgekühlt ("Lösung 1"). In einem anderen Reaktionsgefäß wurde eine Lösung von 445 mg (1.21 mmol) der Verbindung aus Bsp. 512A in 8 ml Dichlormethan bei 0°C mit 421 µl (2.42 mmol) *N,N*-Diisopropylethylamin und 93 µl (1.27 mmol) Thionylchlorid versetzt. Nach 20 min bei 0°C wurde die Lösung 1 portionsweise hinzugefügt. Das Reaktionsgemisch wurde anschließend ca. 18 h bei RT gerührt. Danach wurde es mit Wasser versetzt und mit Dichlormethan extrahiert. Der organische Extrakt wurde nacheinander mit Wasser und gesättigter Natriumchlorid-Lösung gewaschen und über wasserfreiem Magnesiumsulfat getrocknet. Nach Filtration und Einengen wurde der Rückstand mittels präparativer HPLC (Methode 8) gereinigt. Nach Eindampfen der Produktfraktionen und Trocknen im Hochvakuum wurden 117 mg (22% d. Th.) der Titelverbindung erhalten (>99% ee, chirale analytische HPLC).
$^1$H-NMR (400 MHz, DMSO-$d_6$, δ/ppm): 6.53 (s, 1H), 5.81-5.69 und 5.61-5.51 (2 m, zus. 1H), 4.09-3.96 (m, 2H), 3.69-3.56 (m, 2H), 3.28-3.18 (m, 4H), 3.24 (s, 3H), 2.38 und 2.37 (2 s, zus. 3H), 1.67 und 1.63 (2 d, zus. 3H).
LC/MS (Methode 1, ESIpos): $R_t$ = 0.84 min, m/z = 437 $[M+H]^+$.

Chirale analytische HPLC [Säule: Daicel Chiralpak ID-3, 3 µm, 50 mm x 4.6 mm; Laufmittel: Heptan/Isopropanol 1:1; Fluss: 1 ml/min; Temperatur: 30°C; Detektion: 220 nm]: $R_t$ = 1.65 min.

Spezifische optische Drehung: $[\alpha]_D^{20}$ = +13.7°·ml·dm$^{-1}$·g$^{-1}$ (Methanol).

**Beispiel 502**

1-(2-Methoxyethyl)-5-methyl-6-[(2-oxoimidazolidin-1-yl)(dideutero)methyl]-3-[(2*S*)-1,1,1-trifluorpropan-2-yl]thieno[2,3-d]pyrimidin-2,4(1*H*,3*H*)-dion

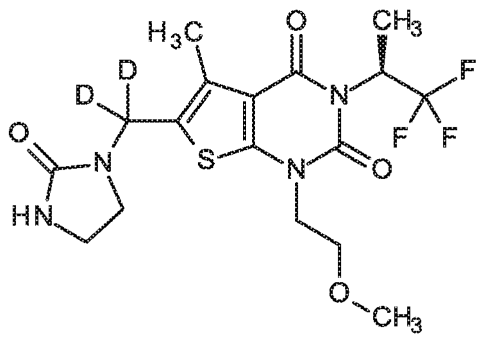

Eine Lösung von 148 mg (1.72 mmol) Imidazolidin-2-on in 4.5 ml DMF wurde mit 67 mg (1.72 mmol) Natriumhydrid (60% Suspension in Mineralöl) versetzt, dann 5 min auf 60°C erwärmt und anschließend wieder auf RT abgekühlt ("Lösung 1"). In einem anderen Reaktionsgefäß wurde eine Lösung von 158 mg (0.429 mmol) der Verbindung aus Bsp. 513A in 2.5 ml Dichlormethan bei 0°C mit 149 $\mu$l (0.858 mmol) *N,N*-Diisopropylethylamin und 33 $\mu$l (0.450 mmol) Thionylchlorid versetzt. Nach 20 min bei 0°C wurde die Lösung 1 portionsweise hinzugefügt. Das Reaktionsgemisch wurde anschließend ca. 18 h bei RT gerührt. Danach wurde es zur Trockene eingeengt. Der verbliebene Rückstand wurde mittels präparativer HPLC (Methode 8) in seine Komponenten aufgetrennt. Nach Eindampfen der Produktfraktionen und Trocknen im Hochvakuum wurden 52 mg (27% d. Th.) der Titelverbindung erhalten (>99% ee, chirale analytische HPLC).

$^1$H-NMR (400 MHz, DMSO-$d_6$, $\delta$/ppm): 6.53 (s, 1H), 5.81-5.69 und 5.61-5.51 (2 m, zus. 1H), 4.06-3.99 (m, 2H), 3.66-3.59 (m, 2H), 3.28-3.18 (m, 4H), 3.24 (s, 3H), 2.38 und 2.37 (2 s, zus. 3H), 1.67 und 1.63 (2 d, zus. 3H).

LC/MS (Methode 1, ESIpos): $R_t$ = 0.84 min, m/z = 437 $[M+H]^+$.

Chirale analytische HPLC [Säule: Daicel Chiralpak ID-3, 3 $\mu$m, 50 mm x 4.6 mm; Laufmittel: Heptan/Isopropanol 1:1; Fluss: 1 ml/min; Temperatur: 30°C; Detektion: 220 nm]: $R_t$ = 1.55 min.

Spezifische optische Drehung: $[\alpha]_D^{20}$ = -12.4°·ml·dm$^{-1}$·g$^{-1}$ (Methanol).

**Beispiel 503**

3-Ethyl-5-methyl-6-[1-(2-oxoimidazolidin-1-yl)ethyl]-1-(3,3,3-trifluorpropyl)thieno[2,3-d]pyrimidin-2,4(1*H*,3*H*)-dion (*Enantiomer 1*)

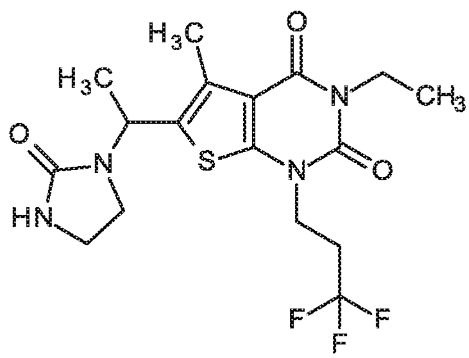

140 mg (0.335 mmol) der racemischen Verbindung aus Bsp. 416 wurden in einem Gemisch aus 2 ml Ethanol und 2 ml Acetonitril gelöst und in 26 Portionen über präparative HPLC an chiraler Phase in die Enantiomere aufgetrennt [Säule: Daicel Chiralpak IC, 5 $\mu$m, 250 mm x 20 mm; Laufmittel: Ethanol; Fluss: 20 ml/min; Temperatur: 23°C; Detektion: 210 nm]. Nach Eindampfen der Produktfraktionen und Trocknen im Hochvakuum wurden 40 mg (57% d. Th.) von Enantiomer 1 erhalten (>99% ee, chirale analytische HPLC).

$^1$H-NMR (400 MHz, DMSO-$d_6$, $\delta$/ppm): 6.39 (s, 1H), 5.31 (q, 1H), 4.12 (td, 2H), 3.95-3.85 (m, 2H), 3.46-3.35 (m, 1H), 3.27-3.08 (m, 3H), 2.86-2.70 (m, 2H), 2.39 (s, 3H), 1.46 (d, 3H), 1.11 (t, 3H).

Chirale analytische HPLC [Säule: Daicel Chiralpak IC-3, 3 $\mu$m, 50 mm x 4.6 mm; Laufmittel: Ethanol; Fluss: 1 ml/min; Temperatur: 30°C; Detektion: 220 nm]: $R_t$ = 2.24 min.

**Beispiel 504**

3-Ethyl-5-methyl-6-[1-(2-oxoimidazolidin-1-yl)ethyl]-1-(3,3,3-trifluorpropyl)thieno[2,3-d]pyrimidin-2,4(1*H*,3*H*)-dion (*Enantiomer 2*)

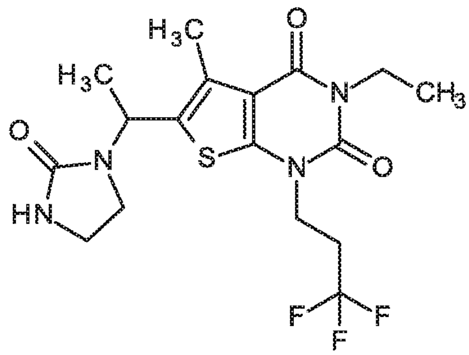

140 mg (0.335 mmol) der racemischen Verbindung aus Bsp. 416 wurden in einem Gemisch aus 2 ml Ethanol und 2 ml Acetonitril gelöst und in 26 Portionen über präparative HPLC an chiraler Phase in die Enantiomere aufgetrennt [Säule: Daicel Chiralpak IC, 5 μm, 250 mm x 20 mm; Laufmittel: Ethanol; Fluss: 20 ml/min; Temperatur: 23°C; Detektion: 210 nm]. Nach Eindampfen der Produktfraktionen und Trocknen im Hochvakuum wurden 40 mg (57% d. Th.) von Enantiomer 2 erhalten (>99% ee, chirale analytische HPLC).
$^1$H-NMR (400 MHz, DMSO-$d_6$, δ/ppm): 6.39 (s, 1H), 5.31 (q, 1H), 4.22-4.02 (m, 2H), 3.90 (q, 2H), 3.45-3.35 (m, 1H), 3.27-3.09 (m, 3H), 2.85-2.70 (m, 2H), 2.39 (s, 3H), 1.46 (d, 3H), 1.11 (t, 3H).
Chirale analytische HPLC [Säule: Daicel Chiralpak IC-3, 3 μm, 50 mm x 4.6 mm; Laufmittel: Ethanol; Fluss: 1 ml/min; Temperatur: 30°C; Detektion: 220 nm]: $R_t$ = 3.52 min.

**Beispiel 505**

3-(2,2-Dimethylpropyl)-5-methyl-6-[(2-oxo-2,3-dihydro-1*H*-imidazol-1-yl)methyl]-1-(3,3,3-trifluorpropyl)thieno[2,3-d]pyrimidin-2,4(1*H*,3*H*)-dion

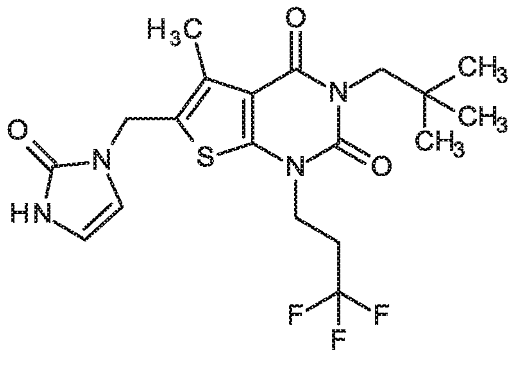

Eine Lösung von 79 mg (0.125 mmol, 85% Reinheit) der Verbindung aus Bsp. 519A in einem Gemisch aus 250 μl Wasser und 1.4 ml Methanol wurde mit 250 μl (0.125 mmol) 0.5 M Salzsäure versetzt. Nachdem das Reaktionsgemisch 40 h bei RT gerührt worden war, wurde es direkt mittels präparativer HPLC (Methode 18) in seine Komponenten aufgetrennt. Eindampfen und Trocknen der Produktfraktion im Hochvakuum ergab 25 mg (46% d. Th.) der Titelverbindung.
$^1$H-NMR (400 MHz, DMSO-$d_6$, δ/ppm): 10.00 (br. s, 1H), 6.46 (dd, 1H), 6.35 (dd, 1H), 4.81 (s, 2H), 4.07 (t, 2H), 3.81 (br. s, 2H), 2.80-2.66 (m, 2H), 2.48 (s, 3H), 0.89 (s, 9H).
LC/MS (Methode 17, ESIneg): $R_t$ = 1.81 min, m/z = 443.14 [M-H]$^-$.

**Beispiel 506**

3-(2,2-Dimethylpropyl)-1-(2-methoxyethyl)-5-methyl-6-[(2-oxo-2,3-dihydro-1*H*-imidazol-1-yl)-methyl]thieno[2,3-d]pyrimidin-2,4(1*H*,3*H*)-dion

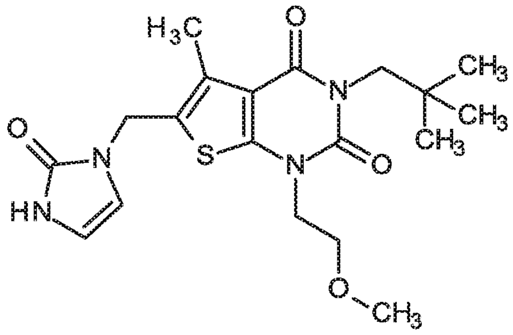

Eine Lösung von 93 mg (0.138 mmol, 70% Reinheit) der Verbindung aus Bsp. 520A in einem Gemisch aus 280 µl Wasser und 1 ml Methanol wurde mit 275 µl (0.137 mmol) 0.5 M Salzsäure versetzt. Nachdem das Reaktionsgemisch 40 h bei RT gerührt worden war, wurde es direkt mittels präparativer HPLC (Methode 18) in seine Komponenten aufgetrennt. Eindampfen und Trocknen der Produktfraktion im Hochvakuum ergab 30 mg (54% d. Th.) der Titelverbindung.

$^1$H-NMR (400 MHz, DMSO-$d_6$, δ/ppm): 10.00 (br. s, 1H), 6.45 (dd, 1H), 6.34 (dd, 1H), 4.79 (s, 2H), 4.00 (t, 2H), 3.81 (s, 2H), 3.61 (t, 2H), 3.21 (s, 3H), 2.45 (s, 3H), 0.88 (s, 9H).

LC/MS (Methode 17, ESIneg): $R_t$ = 1.57 min, m/z = 405.16 [M-H]$^-$.

**Beispiel 507**

3-(2,2-Dimethylpropyl)-5-methyl-6-[(2-oxo-2,3-dihydro-1*H*-imidazol-1-yl)methyl]-1-(tetrahydrofuran-2-ylmethyl)thieno[2,3-d]pyrimidin-2,4(1*H*,3*H*)-dion (*Racemat*)

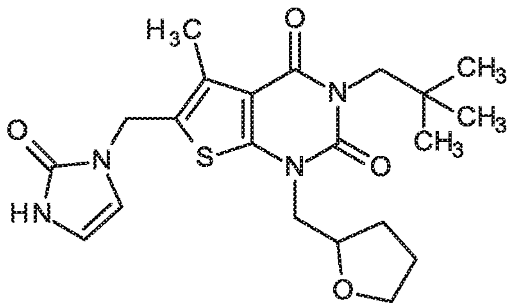

Eine Lösung von 91 mg (0.128 mmol, 70% Reinheit) der Verbindung aus Bsp. 521A in einem Gemisch aus 260 µl Wasser und 940 µl Methanol wurde mit 254 µl (0.127 mmol) 0.5 M Salzsäure versetzt. Nachdem das Reaktionsgemisch 40 h bei RT gerührt worden war, wurde es direkt mittels präparativer HPLC (Methode 18) in seine Komponenten aufgetrennt. Eindampfen und Trocknen der Produktfraktion im Hochvakuum ergab 47 mg (83% d. Th., 98% Reinheit) der Titelverbindung.

$^1$H-NMR (400 MHz, DMSO-$d_6$, δ/ppm): 9.99 (br. s, 1H), 6.44 (dd, 1H), 6.34 (dd, 1H), 4.78 (s, 2H), 4.24-4.16 (m, 1H), 4.00 (dd, 1H), 3.81 (br. s, 2H), 3.77-3.67 (m, 2H), 3.62-3.27 (m, 1H), 2.45 (s, 2H), 2.01-1.77 (m, 3H), 1.70-1.61 (m, 1H), 0.89 (s, 9H).

LC/MS (Methode 17, ESIneg): $R_t$ = 1.67 min, m/z = 431.18 [M-H]$^-$.

**Beispiel 508**

3-(2,2-Dimethylpropyl)-5-methyl-6-[(2-oxo-2,3-dihydro-1*H*-imidazol-1-yl)methyl]-1-(tetrahydrofuran-2-ylmethyl)thieno[2,3-d]pyrimidin-2,4(1*H*,3*H*)-dion (*Enantiomer 1*)

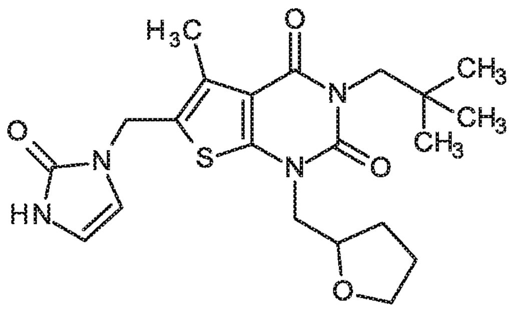

42 mg der racemischen Verbindung aus Bsp. 507 wurden in 1 ml eines Methanol/Dichlormethan/ *tert.*-Butylmethylether-Gemisches (1:1:2) gelöst und mittels präparativer HPLC an chiraler Phase in die Enantiomere aufgetrennt [Säule: Daicel Chiralpak IC, 5 µm, 250 mm x 20 mm; Eluent: *tert.*-Butylmethylether/Methanol 9:1; Fluss: 15 ml/min; Temperatur: 30°C; UV-Detektion: 235 nm]. Die jeweiligen Produktfraktionen wurden am Rotationsverdampfer eingeengt, mit *tert.*-Butanol versetzt und gefriergetrocknet. Es wurden 8 mg (12% d. Th.) der Titelverbindung (Enantiomer 1) sowie 7 mg (11% d. Th.) des Enantiomers 2 (siehe Beispiel 509) erhalten.
$^1$H-NMR (400 MHz, DMSO-$d_6$, δ/ppm): 9.99 (br. s, 1H), 6.44 (dd, 1H), 6.34 (dd, 1H), 4.78 (s, 2H), 4.24-4.16 (m, 1H), 4.00 (dd, 1H), 3.81 (br. s, 2H), 3.77-3.67 (m, 2H), 3.62-3.27 (m, 1H), 2.45 (s, 2H), 2.01-1.77 (m, 3H), 1.70-1.61 (m, 1H), 0.89 (s, 9H).
Chirale analytische HPLC [Säule: Daicel Chiralpak IC, 5 µm, 250 mm x 4.6 mm; Laufmittel: *tert.*-Butanol/Methanol/Essigsäure 85:15:0.2; Fluss: 1 ml/min; Temperatur: 25°C; Detektion: 235 nm]: $R_t$ = 6.28 min.

**Beispiel 509**

3-(2,2-Dimethylpropyl)-5-methyl-6-[(2-oxo-2,3-Mydro-1*H*-imidazol-1-yl)methyl]-1-(tetrahydrofuran-2-ylmethyl)thieno[2,3-d]pyrimidin-2,4(1*H*,3*H*)-dion *(Enantiomer 2)*

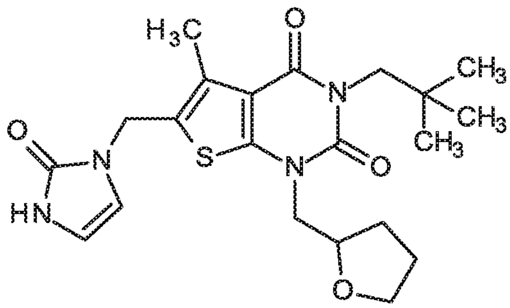

Die Titelverbindung (7 mg) wurde als zweites Enantiomer bei der unter Bsp. 508 beschriebenen präparativen HPLC-Trennung des Racemats aus Bsp. 507 erhalten.
$^1$H-NMR (400 MHz, DMSO-$d_6$, δ/ppm): 9.99 (br. s, 1H), 6.44 (dd, 1H), 6.34 (dd, 1H), 4.78 (s, 2H), 4.24-4.16 (m, 1H), 4.00 (dd, 1H), 3.81 (br. s, 2H), 3.77-3.67 (m, 2H), 3.62-3.27 (m, 1H), 2.45 (s, 2H), 2.01-1.77 (m, 3H), 1.70-1.61 (m, 1H), 0.89 (s, 9H).
Chirale analytische HPLC [Säule: Daicel Chiralpak IC, 5 µm, 250 mm x 4.6 mm; Laufmittel: *tert.*-Butanol/Methanol/Essigsäure 85:15:0.2; Fluss: 1 ml/min; Temperatur: 25°C; Detektion: 235 nm]: $R_t$ = 6.77 min.

**Beispiel 510**

[1-{[1-(2-Methoxyethyl)-5-methyl-2,4-dioxo-3-(1,1,1-trifluorpropan-2-yl)-1,2,3,4-tetrahydro-thieno[2,3-d]pynmidin-6-yl]methyl}imidazolidin-2-yliden]cyanamid (*Enantiomer 1*)

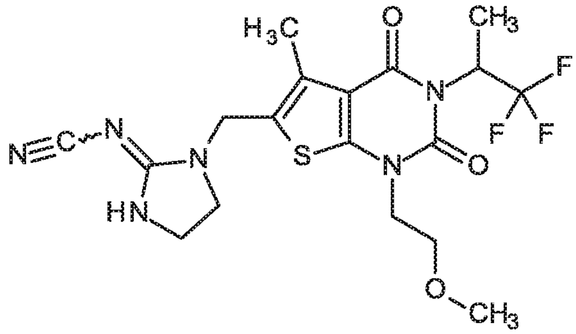

300 mg (0.588 mmol, 80% Reinheit) der Verbindung aus Bsp. 514A wurden in 10 ml DMF gelöst und mit 129 mg (0.881 mmol) Dimethyl-*N*-cyanodithioiminocarbonat sowie 162 mg (1.17 mmol) Kaliumcarbonat versetzt. Das Gemisch wurde 4 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumcarbonat-Lösung und gesättigter Natriumchlorid-Lösung gewaschen. Nach Trocknen über wasserfreiem Magnesiumsulfat wurde filtriert und eingeengt. Das Rohprodukt wurde mittels präparativer HPLC gereinigt (Methode 8). Die Produktfraktionen wurden vereinigt, eingedampft und im Hochvakuum getrocknet. Es wurden 92 mg (34% d. Th.) der enantiomerenreinen Titelverbindung erhalten (99% ee, chirale analytische HPLC).
$^1$H-NMR (400 MHz, DMSO-$d_6$, δ/ppm): 8.08 (s, 1H), 5.83-5.68 und 5.63-5.45 (2 m, zus. 1H), 4.48 (s, 2H), 4.07-4.00 (m, 2H), 3.66-3.60 (m, 2H), 3.52-3.36 (m, 4H), 3.24 (s, 3H), 2.40 und 2.39 (2 s, zus. 3H), 1.67 und 1.63 (2 d, zus. 3H).
LC/MS (Methode 1, ESIpos): $R_t$ = 0.94 min, m/z = 459 $[M+H]^+$.

Chirale analytische HPLC [Säule: Daicel Chiralpak AS-3, 3 μm, 50 mm x 4.6 mm; Laufmittel: Isohexan/Ethanol 1:1; Fluss: 1 ml/min; Temperatur: 30°C; Detektion: 220 nm]: $R_t$ = 1.81 min.

Spezifische optische Drehung: $[\alpha]_D^{20}$ = -12.4°·ml·dm$^{-1}$·g$^{-1}$ (Methanol).

**Beispiel 511**

[1-{[1-(2-Methoxyethyl)-5-methyl-2,4-dioxo-3-(1,1,1-trifluorpropan-2-yl)-1,2,3,4-tetrahydro-thieno[2,3-d]pyrimidin-6-yl]methyl} imidazolidin-2-yliden]cyanamid (*Enantiomer 2*)

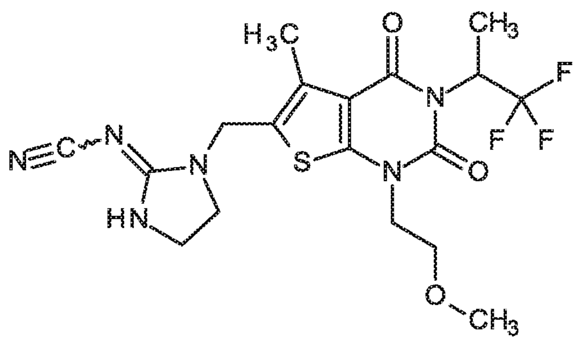

300 mg (0.588 mmol, 80% Reinheit) der Verbindung aus Bsp. 515A wurden in 10 ml DMF gelöst und mit 129 mg (0.881 mmol) Dimethyl-*N*-cyanodithioiminocarbonat sowie 162 mg (1.17 mmol) Kaliumcarbonat versetzt. Das Gemisch wurde 4 h bei 80°C in einem Mikrowellenofen gerührt (Biotage Initiator mit dynamischer Steuerung der Einstrahlleistung). Danach wurde das Reaktionsgemisch mit Ethylacetat verdünnt und nacheinander mit gesättigter Natriumcarbonat-Lösung und gesättigter Natriumchlorid-Lösung gewaschen. Nach Trocknen über wasserfreiem Magnesiumsulfat

wurde filtriert und eingeengt. Das Rohprodukt wurde mittels präparativer HPLC gereinigt (Methode 8). Die Produktfraktionen wurden vereinigt, eingedampft und im Hochvakuum getrocknet. Es wurden 119 mg (44% d. Th.) der enantiomerenreinen Titelverbindung erhalten (99% ee, chirale analytische HPLC).
$^1$H-NMR (400 MHz, DMSO-$d_6$, δ/ppm): 8.09 (s, 1H), 5.80-5.69 und 5.62-5.50 (2 m, zus. 1H), 4.48 (s, 2H), 4.07-4.00 (m, 2H), 3.67-3.61 (m, 2H), 3.52-3.36 (m, 4H), 3.24 (s, 3H), 2.40 und 2.39 (2 s, zus. 3H), 1.68 und 1.63 (2 d, zus. 3H).
LC/MS (Methode 1, ESIpos): $R_t$ = 0.92 min, m/z = 459 $[M+H]^+$.

Chirale analytische HPLC [Säule: Daicel Chiralpak AS-3, 3 μm, 50 mm x 4.6 mm; Laufmittel: Isohexan/Ethanol 1:1; Fluss: 1 ml/min; Temperatur: 30°C; Detektion: 220 nm]: Rt = 2.18 min.

Spezifische optische Drehung: $[\alpha]_D^{20}$ = +13.1°·ml·dm$^{-1}$·g$^{-1}$ (Methanol).

**Beispiel 512**

[1-{3-Isopropyl-1-(2-methoxyethyl)-5-methyl-2,4-dioxo-1,2,3,4-tetrahydrothieno[2,3-d]pyrimidin-6-yl](dideutero)methyl}imidazolidin-2-yliden]cyanamid

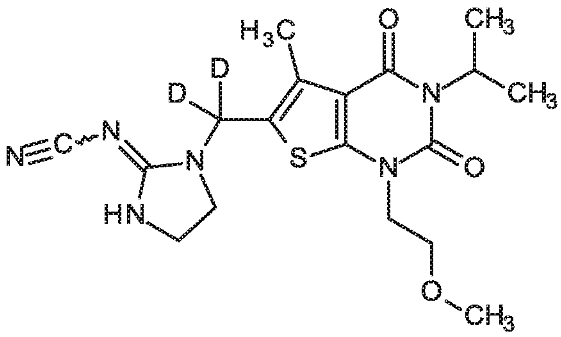

Eine Lösung von 212 mg (1.92 mmol) Imidazolidin-2-ylidencyanamid [Lit.: J. Zmitek et al., Org. Prep. Proc. Int. 23 (6), 721-728 (1991)] in 13 ml DMF wurde mit 85 mg (2.14 mmol) Natriumhydrid (60% Suspension in Mineralöl) versetzt, dann 5 min auf 60°C erwärmt und anschließend wieder auf RT abgekühlt ("Lösung 1"). In einem anderen Reaktionsgefäß wurde eine Lösung von 224 mg (0.713 mmol) der Verbindung aus Bsp. 510A in 2.2 ml Dichlormethan bei 0°C mit 248 μl (1.42 mmol) *N,N*-Diisopropylethylamin und 55 μl (0.748 mmol) Thionylchlorid versetzt. Nach 20 min bei 0°C wurde diese Lösung portionsweise zur Lösung 1 hinzugegeben. Das Reaktionsgemisch wurde anschließend 3 Tage bei RT gerührt. Danach wurde es mit Wasser versetzt und mit Ethylacetat extrahiert. Der organische Extrakt wurde mit gesättigter wässriger Natriumchlorid-Lösung gewaschen und über wasserfreiem Magnesiumsulfat getrocknet. Nach Filtration und Einengen wurde der Rückstand zunächst mittels MPLC vorgereinigt (Isolera, 25 g Kieselgel-Kartusche, Cyclohexan/Ethylacetat 1:1 → Dichlormethan/Methanot 10:1). Die eingedampften produkthaltigen Fraktionen wurden anschließend mittels präparativer HPLC (Methode 8) nachgereinigt. Nach erneutem Eindampfen und Trocknen im Hochvakuum wurden 58 mg (19% d. Th.) der Titelverbindung erhalten.
$^1$H-NMR (400 MHz, DMSO-$d_6$, δ/ppm): 8.06 (s, 1H), 5.13 (sept, 1H), 4.00 (t, 2H), 3.62 (t, 2H), 3.50-3.36 (m, 4H), 3.24 (s, 3H), 2.39 (s, 3H), 1.40 (d, 6H).
LC/MS (Methode 17, ESIpos): $R_t$ = 1.52 min, m/z = 407.18 $[M+H]^+$.

**Beispiel 513**

[1-([1-(2-Methoxyethyl)-5-methyl-2,4-dioxo-3-(2,2,2-trifluorethyl)-1,2,3,4-tetrahydrothieno-[2,3-d]pyrimidin-6-yl](dideuteio)methyl}imidazolidin-2-yliden]cyanamid

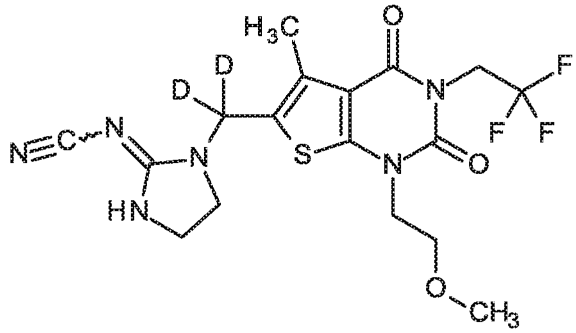

Eine Lösung von 249 mg (2.27 mmol) Imidazolidin-2-ylidencyanamid [Lit.: J. Zmitek et al., Org. Prep. Proc. Int. 23 (6), 721-728 (1991)] in 16 ml DMF wurde mit 101 mg (2.52 mmol) Natriumhydrid (60% Suspension in Mineralöl) versetzt, dann 5 min auf 60°C erwärmt und anschließend wieder auf RT abgekühlt ("Lösung 1"). In einem anderen Reaktionsgefäß wurde eine Lösung von 350 mg (0.840 mmol) der Verbindung aus Bsp. 511A in 2.4 ml Dichlormethan bei 0°C mit 292 µl (1.68 mmol) *N,N*-Diisopropylethylamin und 64 µl (0.882 mmol) Thionylchlorid versetzt. Nach 20 min bei 0°C wurde diese Lösung portionsweise zur Lösung 1 hinzugegeben. Das Reaktionsgemisch wurde anschließend 24 h bei RT gerührt. Danach wurde es mit Wasser versetzt und mit Ethylacetat extrahiert. Der organische Extrakt wurde mit gesättigter wässriger Natriumchlorid-Lösung gewaschen und über wasserfreiem Magnesiumsulfat getrocknet. Nach Filtration und Einengen wurde der Rückstand mittels präparativer HPLC (Methode 8) gereinigt. Die Produktfraktionen wurden eingedampft und im Hochvakuum getrocknet. Es wurden 62 mg (16% d. Th., 97% Reinheit) der Titelverbindung erhalten.

$^1$H-NMR (400 MHz, DMSO-$d_6$, δ/ppm): 8.08 (s, 1H), 4.70 (q, 2H), 4.07 (t, 2H), 3.64 (t, 2H), 3.52-3.36 (m, 4H), 3.24 (s, 3H), 2.41 (s, 3H).

LC/MS (Methode 17, ESIpos): $R_t$ = 1.51 min, m/z = 447.14 $[M+H]^+$.

## Beispiel 514

Methyl-[1-{[1-(2-methoxyethyl)-5-methyl-2,4-dioxo-3-(1,1,1-trifluorpropan-2-yl)-1,2,3,4-tetra-hydrothieno[2,3-d]pyrimidin-6-yl]methyl}imidazolidin-2-yliden]carbamat (*Enantiomer 1*)

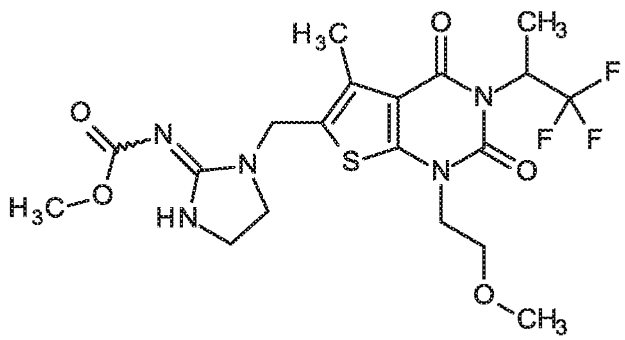

300 mg (0.588 mmol, 80% Reinheit) der Verbindung aus Bsp. 514A und 164 µl (1.18 mmol) Triethylamin wurden in 10 ml Dichlormethan gelöst und mit einer Lösung von 183 mg (1.18 mmol) Methyl-(dichlormethylen)carbamat in 5 ml Dichlormethan versetzt. Nachdem das Reaktionsgemisch ca. 18 h bei RT gerührt worden war, wurde es zur Trockene eingeengt. Der Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) vorgereinigt. Die Produktfraktionen wurden vereinigt, eingedampft und mittels MPLC nachgereinigt (Isolera, 10 g Kieselgel SNAP KP-Sil, Cyclohexan/Ethylacetat 10:1 → 0:1). Erneutes Einengen der Produktfraktionen und Trocknen des Rückstands im Hochvakuum ergaben 67 mg (23% d. Th.) der enantiomerenreinen Titelverbindung (>99% ee, chirale analytische HPLC).

$^1$H-NMR (600 MHz, DMSO-$d_6$, δ/ppm): 8.04 (s, 1H), 5.79-5.71 und 5.60-5.52 (2 m, zus. 1H), 4.56 (s, 2H), 4.04-3.98 (m, 2H), 3.65-3.60 (m, 2H), 3.53 (s, 3H), 3.47-3.44 (m, 2H), 3.37-3.34 (m, 2H, teilweise überdeckt vom Wasser-Signal), 3.23 (s, 3H), 2.41 und 2.40 (2 s, zus. 3H), 1.67 und 1.63 (2 d, zus. 3H).

LC/MS (Methode 17, ESIpos): $R_t$ = 1.48 min, m/z = 492.15 $[M+H]^+$.

Chirale analytische HPLC [Säule: Daicel Chiralcel IC-3, 3 μm, 50 mm x 4.6 mm; Laufmittel: Isohexan/Ethanol 1:1; Fluss: 1 ml/min; Temperatur: 25°C; Detektion: 220 nm]: $R_t$ = 3.15 min.

Spezifische optische Drehung: [α]D$^{20}$ = -10.9°·ml·dm$^{-1}$·g$^{-1}$ (Methanol).

**Beispiel 515**

Methyl-[1-{[1-(2-methoxyethyl)-5-methyl-2,4-dioxo-3-(1,1,1-trifluorpropan-2-yl)-1,2,3,4-tetra-hydiothieno[2,3-d]pyrimidin-6-yl]methyl}imidazolidin-2-yliden]carbamat (*Enantiomer 2*)

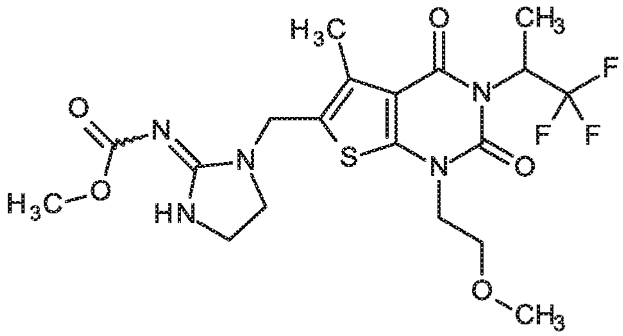

300 mg (0.588 mmol, 80% Reinheit) der Verbindung aus Bsp. 515A und 164 μl (1.18 mmol) Triethylamin wurden in 10 ml Dichlormethan gelöst und mit einer Lösung von 183 mg (1.18 mmol) Methyl-(dichlormethylen)carbamat in 5 ml Dichlormethan versetzt. Nachdem das Reaktionsgemisch ca. 18 h bei RT gerührt worden war, wurde es zur Trockene eingeengt. Der Rückstand wurde in Ethylacetat aufgenommen und nacheinander mit Wasser und gesättigter Natriumchlorid-Lösung gewaschen. Nach Trocknen über wasserfreiem Magnesiumsulfat wurde filtriert und eingeengt. Der verbliebene Rückstand wurde mittels präparativer HPLC (Methode 8) vorgereinigt. Die Produktfraktionen wurden vereinigt, eingedampft und mittels MPLC nachgereinigt (Isolera, 10 g Kieselgel SNAP KP-Sil, Cyclohexan/Ethylacetat 10:1 → 0:1). Erneutes Einengen der Produktfraktionen und Trocknen des Rückstands im Hochvakuum ergaben 64 mg (22% d. Th.) der enantiomerenreinen Titelverbindung (>99% ee, chirale analytische HPLC).

$^1$H-NMR (600 MHz, DMSO-$d_6$, δ/ppm): 8.04 (s, 1H), 5.79-5.70 und 5.60-5.52 (2 m, zus. 1H), 4.56 (s, 2H), 4.05-3.98 (m, 2H), 3.65-3.59 (m, 2H), 3.53 (s, 3H), 3.47-3.44 (m, 2H), 3.37-3.34 (m, 2H, teilweise überdeckt vom Wasser-Signal), 3.23 (s, 3H), 2.41 und 2.40 (2 s, zus. 3H), 1.67 und 1.63 (2 d, zus. 3H).

LC/MS (Methode 17, ESIpos): $R_t$ = 1.49 min, m/z = 492.15 $[M+H]^+$.

Chirale analytische HPLC [Säule: Daicel Chiralcel IC-3, 3 μm, 50 mm x 4.6 mm; Laufmittel: Isohexan/Ethanol 1:1; Fluss: 1 ml/min; Temperatur: 25°C; Detektion: 220 nm]: $R_t$ = 2.93 min.

Spezifische optische Drehung: $[\alpha]_D^{20}$ = +9.9°·ml·dm$^{-1}$·g$^{-1}$ (Methanol).

**Beispiel 516**

3-Ethyl-5-methyl-6-[(3-methyl-5-oxo-4,5-dibydro-1*H*-1,2,4-triaaol-1-yl)methyt]-1-(3,3,3-trifluor-propyl)thieno[2,3-d]pyrimidin-2,4(1*H*,3*H*)-dion

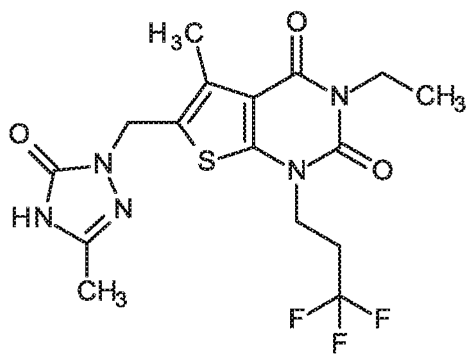

Eine Lösung von 10 mg (0.023 mmol) der Verbindung aus Bsp. 523A in 150 µl Ethanol wurde mit 6 µl (0.016 mmol) einer 21%-igen Lösung von Natriumethylat in Ethanol versetzt. Nach 1 h Rühren bei RT wurde die Temperatur auf 60°C erhöht. Nach 4 h bei dieser Temperatur wurden weitere 150 µl Ethanol und 6 µl (0.016 mmol) der 21%-igen Lösung von Natriumethylat in Ethanol hinzugefügt. Nach weiteren 2 h bei 60°C wurde das Reaktionsgemisch auf RT abgekühlt, mit wenig DMSO verdünnt und direkt mittels präparativer HPLC (Methode 8) in seine Komponenten aufgetrennt. Nach Eindampfen der Produktfraktion und Trocknen im Hochvakuum wurden 0.5 mg (5% d. Th.) der Titelverbindung erhalten.

$^1$H-NMR (600 MHz, DMSO-$d_6$, δ/ppm): 11.49 (br. s, 1H), 4.88 (s, 2H), 4.08 (t, 2H), 3.90 (q, 2H), 2.84-2.67 (m, 2H), 2.46 (s, 3H), 2.04 (s, 3H), 1.11 (t, 3H).

LC/MS (Methode 17, ESIpos): $R_t$ = 1.40 min, m/z = 418.11 $[M+H]^+$.

## B. Bewertung der pharmakologischen Wirksamkeit

Die pharmakologische Aktivität der erfindungsgemäßen Verbindungen kann durch *in vitro*- und *in* vivo-Untersuchungen, wie sie dem Fachmann bekannt sind, nachgewiesen werden. Die nachfolgenden Anwendungsbeispiele beschreiben die biologische Wirkung der erfindungsgemäßen Verbindungen, ohne die Erfindung auf diese Beispiele zu beschränken.

### B-1. Zelluläre *in* vitro-Tests zur Bestimmung der A2b-Rezeptor-Aktivität und der Adenosin-rezeptor-Selektivität

Die Identifizierung von selektiven Antagonisten des humanen Adenosin A2b-Rezeptors sowie die Quantifizierung der Wirksamkeit und Selektivität der erfindungsgemäßen Verbindungen erfolgte mit Hilfe von rekombinanten Zelllinien für die humanen Adenosin-Rezeptoren A1, A2a, A2b und A3. Diese Zelllinien leiten sich ursprünglich von einer Ovarepithelzelle des Hamsters ab (Chinese Hamster Ovary, CHO-K1, American Type Culture Collection, Manassas, VA 20108, USA). Die Zelllinien zur Testung der Wirksamkeit an den A1-, A2a- und A2b-Rezeptoren enthalten neben dem jeweils rekombinant exprimierten Adenosin-Rezeptor ein Reportergen-Konstrukt, bei dem die Expression der Leuchtkäfer (*Photinus pyralis*)-Luzifeisse unter der Kontrolle eines Promotors steht, der über intrazelluläre Signalkaskaden durch Stimulation der Rezeptoren mit dem (nicht Subtyp-selektiven) Adenosinrezeptor-Agonisten NECA (5'-*N*-Ethylcarboxamidoadenosin) aktiviert werden kann [S.J. Hill, J.G. Baker, S. Rhees, Curr. Opin. Pharmacol. 1, 526-532 (2001)].

Im Falle der A2a- und A2b-Zelllinien handelt es sich um einen Minimalpromotor mit mehreren cAMP-responsiblen Elementen (CRE). Stimulation der $G_8$-gekoppelten A2b- oder A2a-Rezeptoren durch NECA führt über die Bildung von cAMP letztlich zur CRE-abhängigen Induktion der Luziferase-Expression, die 3 Stunden nach Beginn der Inkubation mit NECA mit einer Detektionslösung in einem geeigneten Luminometer nachgewiesen wird. Zur Testung der Antagonisten wird zunächst in einem Vorversuch die Konzentration von NECA bestimmt, die am jeweiligen Versuchstag zur halbmaximalen Stimulation der Luziferase-Expression führt ($EC_{50}$-Konzentration). Durch gemeinsame Inkubation dieser $EC_{50}$-Konzentration von NECA mit den zu testenden Substanzen kann dann deren antagonistische Wirkung bestimmt werden.

Die Zelllinie zur Testung des $G_i$-gekoppelten A1-Rezeptors enthält ein anderes Reportergen-Konstrukt, bei dem die Expression der Leuchtkäfer-Luziferase unter der Kontrolle eines NFAT (nuclear factor of activated T-cells)-Promoters steht. Diese Zelllinie wurde neben dem A1-Rezeptor und dem NFAT-Reportergen auch noch mit einem weiteren Gen, das für das promiskuitive $G\alpha_{16}$-Protein kodiert [T.T. Amatruda, D.A. Steele, V.Z. Slepak, M.I. Simon, Proc. Natl. Acad. Sci. USA 88, 5587-5591 (1991)], entweder unabhängig oder als Fusionsgen stabil transfiziert. Die daraus resultierenden Testzellen reagieren auf Stimulation des normalerweise $G_i$-gekoppelten A1-Rezeptors mit einer Erhöhung der intrazellulären Calcium-Konzentration, die dann zu einer NFAT-abhängigen Luziferase-Expression führt. Die Versuchsdurchführung zur Testung der Antagonisten auf dem A1-Rezeptor entspricht dem Vorgehen für die Testung mit den A2a- und A2b-Zelllinien.

Bei der Erzeugung der A3-Rezeptor-Zelllinie wurde ebenfalls eine Ko-Transfektion von A3-Rezeptor und dem promiskuitiven $G\alpha_{16}$-Protein durchgeführt, so dass auch hier die Stimulation des Rezeptors zu einer Erhöhung der intrazellulären Calcium-Konzentration führt. Diese Calcium-Erhöhung wird im A3-Rezeptor-Test allerdings direkt über das Calcium-sensitive Photoprotein Photina® [S. Bovolenta, M. Foti, S. Lohmer, S. Corazza, J. Biomol. Screen. 12, 694-704 (2007)] gemessen. Nach Bestimmung der $EC_{50}$-Konzentration von NECA erfolgt die Messung der Substanzwirkungen nach 5-10 Minuten Vorinkubation mit Substanz durch Zugabe dieser $EC_{50}$-Konzentration in Messposition in einem geeigneten dispensierfähigen Luminometer.

In der folgenden Tabelle 1 sind für individuelle Ausführungsbeispiele die $IC_{50}$-Werte aus dem A2b-Rezeptor-Assay aufgeführt (zum Teil als Mittelwerte aus mehreren unabhängigen Einzelbestimmungen und gerundet auf zwei signifikante Stellen):

Tabelle 1

| Beispiel Nr. | A2b-Rezeptor $IC_{50}$ [nmol/L] |
|---|---|
| 1 | 9.7 |
| 2 | 11 |
| 3 | 4.4 |
| 4 | 5.2 |
| 5 | 8.4 |
| 6 | 8.0 |
| 7 | 33 |
| 8 | 120 |
| 9 | 97 |
| 10 | 7.0 |
| 11 | 19 |
| 12 | 12 |
| 13 | 17 |
| 14 | 19 |
| 15 | 3.1 |
| 16 | 2.7 |
| 17 | 16 |
| 18 | 14 |
| 19 | 5.6 |
| 20 | 3.6 |
| 21 | 5.1 |
| 22 | 24 |
| 23 | 16 |
| 24 | 8.8 |

| Beispiel Nr. | A2b-Rezeptor $IC_{50}$ [nmol/L] |
|---|---|
| 25 | 5.7 |
| 26 | 11 |
| 27 | 2.7 |
| 28 | 1.9 |
| 29 | 4.1 |
| 30 | 5.0 |
| 31 | 2.2 |
| 32 | 11 |
| 33 | 12 |
| 34 | 10 |
| 35 | 6.3 |
| 36 | 38 |
| 37 | 220 |
| 38 | 1.9 |
| 39 | 3.6 |
| 40 | 7.0 |
| 41 | 12 |
| 42 | 11 |
| 43 | 7.7 |
| 44 | 5.2 |
| 45 | 12 |
| 46 | 29 |
| 47 | 14 |
| 48 | 1.4 |
| 49 | 2.5 |
| 50 | 4.6 |
| 51 | 12 |
| 52 | 6.6 |
| 53 | 14 |

| Beispiel Nr. | A2b-Rezeptor $IC_{50}$ [nmol/L] |
|---|---|
| 54 | 4.1 |
| 55 | 1.3 |
| 56 | 8.0 |
| 57 | 0.88 |
| 58 | 2.6 |
| 59 | 5.1 |
| 60 | 13 |
| 61 | 53 |
| 62 | 72 |
| 63 | 39 |
| 64 | 86 |
| 65 | 12 |
| 66 | 4.3 |
| 67 | 29 |
| 68 | 8.9 |
| 69 | 9.8 |
| 70 | 21 |
| 71 | 70 |
| 72 | 8.1 |
| 73 | 79 |
| 74 | 36 |
| 75 | 40 |
| 76 | 9.3 |
| 77 | 5.6 |
| 78 | 1.2 |
| 79 | 9.9 |
| 80 | 1.4 |
| 81 | 14 |
| 82 | 17 |

| Beispiel Nr. | A2b-Rezeptor $IC_{50}$ [nmol/L] |
|---|---|
| 83 | 380 |
| 84 | 3.9 |
| 85 | 18 |
| 86 | 6.4 |
| 87 | 2.3 |
| 88 | 6.7 |
| 89 | 8.7 |
| 90 | 12 |
| 91 | 11 |
| 92 | 9.7 |
| 93 | 11 |
| 94 | 7.7 |
| 95 | 11 |
| 96 | 9.4 |
| 97 | 18 |
| 98 | 130 |
| 99 | 17 |
| 100 | 6.8 |
| 101 | 12 |
| 102 | 4.5 |
| 103 | 3.5 |
| 104 | 120 |
| 105 | 14 |
| 106 | 9.2 |
| 107 | 11 |
| 108 | 3.3 |
| 109 | 4.2 |
| 110 | 79 |
| 111 | 21 |

| Beispiel Nr. | A2b-Rezeptor $IC_{50}$ [nmol/L] |
|---|---|
| 112 | 500 |
| 113 | 8.1 |
| 114 | 39 |
| 115 | 31 |
| 116 | 24 |
| 117 | 5.2 |
| 118 | 3.1 |
| 119 | 2.7 |
| 120 | 12 |
| 121 | 20 |
| 122 | 3.9 |
| 123 | 53 |
| 124 | 60 |
| 125 | 8.8 |
| 126 | 3.9 |
| 127 | 5.6 |
| 128 | 4.7 |
| 129 | 19 |
| 130 | 30 |
| 131 | 180 |
| 132 | 20 |
| 133 | 26 |
| 134 | 30 |
| 135 | 46 |
| 136 | 360 |
| 137 | 10 |
| 138 | 12 |
| 139 | 2.8 |
| 140 | 4.8 |

| Beispiel Nr. | A2b-Rezeptor $IC_{50}$ [nmol/L] |
|---|---|
| 141 | 120 |
| 142 | 13 |
| 143 | 3.0 |
| 144 | 1.5 |
| 145 | 14 |
| 146 | 11 |
| 147 | 22 |
| 148 | 4.1 |
| 149 | 5.1 |
| 150 | 6.0 |
| 151 | 5.7 |
| 152 | 0.79 |
| 153 | 6.4 |
| 154 | 7.1 |
| 155 | 26 |
| 156 | 5.7 |
| 157 | 5.7 |
| 158 | 5.5 |
| 159 | 14 |
| 160 | 2.9 |
| 161 | 8.5 |
| 162 | 7.9 |
| 163 | 5.2 |
| 164 | 17 |
| 165 | 6.5 |
| 166 | 13 |
| 167 | 1.2 |
| 168 | 15 |
| 169 | 36 |

| Beispiel Nr. | A2b-Rezeptor $IC_{50}$ [nmol/L] |
|---|---|
| 170 | 4.4 |
| 171 | 13 |
| 172 | 7.3 |
| 173 | 73 |
| 174 | 49 |
| 175 | 53 |
| 176 | 1.5 |
| 177 | 15 |
| 178 | 21 |
| 179 | 200 |
| 180 | 30 |
| 181 | 1.5 |
| 182 | 9.0 |
| 183 | 17 |
| 184 | 7.0 |
| 185 | 60 |
| 186 | 13 |
| 187 | 6.3 |
| 188 | 40 |
| 189 | 17 |
| 190 | 38 |
| 191 | 3.3 |
| 192 | 3.2 |
| 193 | 25 |
| 194 | 15 |
| 195 | 41 |
| 196 | 6.5 |
| 197 | 18 |
| 198 | 8.9 |

| Beispiel Nr. | A2b-Rezeptor $IC_{50}$ [nmol/L] |
|---|---|
| 199 | 8.1 |
| 200 | 2.9 |
| 201 | 4.4 |
| 202 | 7.8 |
| 203 | 57 |
| 204 | 1.1 |
| 205 | 25 |
| 206 | 11 |
| 207 | 43 |
| 208 | 8.9 |
| 209 | 12 |
| 210 | 9.1 |
| 211 | 17 |
| 212 | 17 |
| 213 | 7.6 |
| 214 | 3.1 |
| 215 | 45 |
| 216 | 34 |
| 217 | 79 |
| 218 | 22 |
| 219 | 19 |
| 220 | 15 |
| 221 | 29 |
| 222 | 13 |
| 223 | 14 |
| 224 | 31 |
| 225 | 12 |
| 226 | 36 |
| 227 | 22 |

| Beispiel Nr. | A2b-Rezeptor $IC_{50}$ [nmol/L] |
|---|---|
| 228 | 130 |
| 229 | 4.0 |
| 230 | 12 |
| 231 | 21 |
| 232 | 93 |
| 233 | 23 |
| 234 | 7.8 |
| 235 | 38 |
| 236 | 6.0 |
| 237 | 17 |
| 238 | 99 |
| 239 | 11 |
| 240 | 19 |
| 241 | 1.2 |
| 242 | 1.4 |
| 243 | 14 |
| 244 | 13 |
| 245 | 2.1 |
| 246 | 4.7 |
| 247 | 8.9 |
| 248 | 5.1 |
| 249 | 41 |
| 250 | 18 |
| 251 | 1.3 |
| 252 | 4.1 |
| 253 | 9.9 |
| 254 | 34 |
| 255 | 13 |
| 256 | 11 |

| Beispiel Nr. | A2b-Rezeptor $IC_{50}$ [nmol/L] |
|---|---|
| 257 | 7.1 |
| 258 | 33 |
| 259 | 9.8 |
| 260 | 2.2 |
| 261 | 9.9 |
| 262 | 2.3 |
| 263 | 5.3 |
| 264 | 3.1 |
| 265 | 14 |
| 266 | 75 |
| 267 | 4.6 |
| 268 | 12 |
| 269 | 24 |
| 270 | 38 |
| 271 | 47 |
| 272 | 10 |
| 273 | 19 |
| 274 | 24 |
| 275 | 76 |
| 276 | 9.9 |
| 277 | 68 |
| 278 | 130 |
| 279 | 20 |
| 280 | 82 |
| 281 | 46 |
| 282 | 30 |
| 283 | 79 |
| 284 | 79 |
| 285 | 91 |

| Beispiel Nr. | A2b-Rezeptor $IC_{50}$ [nmol/L] |
|---|---|
| 286 | 220 |
| 287 | 63 |
| 288 | 140 |
| 289 | 180 |
| 290 | 400 |
| 291 | 190 |
| 292 | 8.3 |
| 293 | 120 |
| 294 | 20 |
| 295 | 220 |
| 296 | 16 |
| 297 | 17 |
| 298 | 2.4 |
| 299 | 5.6 |
| 300 | 14 |
| 301 | 6.5 |
| 302 | 14 |
| 303 | 46 |
| 304 | 73 |
| 305 | 140 |
| 306 | 5.9 |
| 307 | 8.1 |
| 308 | 140 |
| 309 | 36 |
| 310 | 370 |
| 311 | 31 |
| 312 | 22 |
| 313 | 41 |
| 314 | 4.7 |

| Beispiel Nr. | A2b-Rezeptor $IC_{50}$ [nmol/L] |
|---|---|
| 315 | 4.3 |
| 316 | 1.3 |
| 317 | 25 |
| 318 | 1.4 |
| 319 | 2.4 |
| 320 | 0.44 |
| 321 | 1.6 |
| 322 | 1.3 |
| 323 | 34 |
| 324 | 110 |
| 325 | 65 |
| 326 | 10 |
| 327 | 40 |
| 328 | 19 |
| 329 | 900 |
| 330 | 220 |
| 331 | 61 |
| 332 | 110 |
| 333 | 110 |
| 334 | 120 |
| 335 | 270 |
| 336 | 8.7 |
| 337 | 280 |
| 338 | 120 |
| 339 | 5.6 |
| 340 | 18 |
| 341 | 12 |
| 342 | 13 |
| 343 | 25 |

| Beispiel Nr. | A2b-Rezeptor $IC_{50}$ [nmol/L] |
|---|---|
| 344 | 8.5 |
| 345 | 17 |
| 346 | 37 |
| 347 | 56 |
| 348 | 48 |
| 349 | 630 |
| 350 | 210 |
| 352 | 48 |
| 353 | 31 |
| 354 | 120 |
| 355 | 99 |
| 356 | 5.1 |
| 357 | 32 |
| 358 | 12 |
| 359 | 37 |
| 360 | 10 |
| 361 | 13 |
| 362 | 9.4 |
| 363 | 19 |
| 364 | 170 |
| 365 | 110 |
| 366 | 40 |
| 367 | 33 |
| 368 | 82 |
| 369 | 33 |
| 370 | 2.0 |
| 371 | 9.8 |
| 372 | 27 |
| 373 | 5.9 |

| Beispiel Nr. | A2b-Rezeptor $IC_{50}$ [nmol/L] |
|---|---|
| 374 | 7.0 |
| 375 | 6.3 |
| 376 | 7.9 |
| 377 | 3.4 |
| 378 | 3.1 |
| 379 | 13 |
| 380 | 12 |
| 381 | 4.1 |
| 382 | 6.8 |
| 383 | 15 |
| 384 | 9.8 |
| 385 | 4.7 |
| 386 | 16 |
| 387 | 96 |
| 388 | 33 |
| 389 | 87 |
| 390 | 28 |
| 391 | 34 |
| 392 | 56 |
| 393 | 45 |
| 394 | 3.2 |
| 395 | 94 |
| 396 | 93 |
| 397 | 120 |
| 398 | 240 |
| 399 | 26 |
| 400 | 320 |
| 401 | 660 |
| 402 | 100 |

| Beispiel Nr. | A2b-Rezeptor $IC_{50}$ [nmol/L] |
|---|---|
| 403 | 35 |
| 404 | 120 |
| 405 | 30 |
| 406 | 29 |
| 407 | 32 |
| 408 | 12 |
| 409 | 14 |
| 410 | 12 |
| 411 | 53 |
| 412 | 5.4 |
| 413 | 4.6 |
| 414 | 11 |
| 415 | 9.0 |
| 416 | 79 |
| 419 | 130 |
| 420 | 50 |
| 421 | 71 |
| 422 | 170 |
| 423 | 55 |
| 424 | 88 |
| 425 | 42 |
| 426 | 270 |
| 427 | 37 |
| 428 | 120 |
| 429 | 75 |
| 430 | 280 |
| 431 | 740 |
| 432 | 92 |
| 433 | 58 |

| Beispiel Nr. | A2b-Rezeptor $IC_{50}$ [nmol/L] |
|---|---|
| 434 | 82 |
| 435 | 43 |
| 436 | 65 |
| 437 | 48 |
| 438 | 150 |
| 439 | 180 |
| 440 | 23 |
| 441 | 8.6 |
| 442 | 60 |
| 443 | 7.4 |
| 444 | 11 |
| 445 | 12 |
| 446 | 19 |
| 447 | 9.7 |
| 448 | 17 |
| 449 | 54 |
| 450 | 14 |
| 451 | 10 |
| 452 | 14 |
| 453 | 72 |
| 454 | 6.1 |
| 455 | 3.6 |
| 456 | 15 |
| 457 | 7.1 |
| 458 | 34 |
| 459 | 4.9 |
| 460 | 22 |
| 461 | 9.2 |
| 462 | 16 |

| Beispiel Nr. | A2b-Rezeptor $IC_{50}$ [nmol/L] |
|---|---|
| 463 | 9.9 |
| 464 | 9.9 |
| 465 | 13 |
| 466 | 6.6 |
| 467 | 30 |
| 468 | 6.1 |
| 469 | 8.8 |
| 470 | 5.1 |
| 471 | 18 |
| 472 | 6.7 |
| 473 | 3.4 |
| 474 | 5.6 |
| 475 | 36 |
| 476 | 140 |
| 477 | 270 |
| 478 | 41 |
| 479 | 140 |
| 480 | 35 |
| 481 | 140 |
| 482 | 410 |
| 483 | 45 |
| 484 | 67 |
| 485 | 52 |
| 486 | 190 |
| 487 | 250 |
| 488 | 68 |
| 489 | 27 |
| 490 | 19 |
| 491 | 52 |

| Beispiel Nr. | A2b-Rezeptor $IC_{50}$ [nmol/L] |
|---|---|
| 492 | 250 |
| 493 | 11 |
| 494 | 20 |
| 495 | 57 |
| 496 | 120 |
| 497 | 730 |
| 498 | 79 |
| 499 | 230 |
| 500 | 10 |
| 501 | 43 |
| 502 | 18 |

| Beispiel Nr. | A2b-Rezeptor $IC_{50}$ [nmol/L] |
|---|---|
| 503 | 39 |
| 504 | 2000 |
| 505 | 5.0 |
| 506 | 12 |
| 507 | 28 |
| 510 | 47 |
| 511 | 110 |
| 512 | 5.1 |
| 513 | 8.4 |
| 514 | 32 |
| 515 | 24 |

B-2. Adenosinrezeptor-Bindungsassays

Die Bindungseigenschaften der Testverbindungen an Adenosinrezeptoren wurden in Bindungsstudien mit Radioliganden bestimmt. Dazu wurden Membranpräparationen der humanen Adenosinrezeptor-Subtypen aus Zelllinien mit rekombinanter Rezeptorexpression hergestellt (CHO-Zellen für den A1-Rezeptor, HEK293-Zellen für die A2a-, A2b- und A3-Rezeptoren). Die folgenden Radioliganden wurden in den Experimenten verwendet: [$^3$H]-DPCPX für den A1-Rezeptor, [$^3$H]-CGS 21680 für den A2a-Rezeptor, [$^3$H]-CPX für den A2b-Rezeptor und [$^{125}$I]-AB-MECA für den A3-Rezeptor. Die Testsubstanzen wurden jeweils in 8 verschiedenen Konzentrationen und 2 Wiederholungstestungen pro Konzentration getestet. Die Verdrängung des jeweiligen Radioliganden durch die Testverbindung wurde als prozentuale Inhibition der spezifischen Bindung der Kontrollen ausgedrückt.

Die $IC_{50}$-Werte (Konzentration, die eine halbmaximale Inhibition der spezifischen Bindung der Kontrollen bewirkt) und die Hill-Koeffizienten (nH) wurden durch eine nicht-lineare Regressionsanalyse bestimmt, unter Verwendung der aus den Mittelwerten der Wiederholungstestungen erzeugten Kompetitionskurven und Durchführung eines Kurvenfits gemäß der Hill-Gleichung:

$$Y = D+[A-D/1+(C/C50)^{nH}]$$

(Y = spezifische Bindung; A = linke Asymptote der Kurve; D = rechte Asymptote der Kurve; C = Substanzkonzentration; C50 = $IC_{50}$; nH = Steigungsfaktor).

Die Inhibitionskonstante ($K_i$) wurde mit der Cheng-Prusoff-Gleichung berechnet:

$$K_i = IC_{50}/(1+L/K_D)$$

(L = Konzentration des Radioliganden im Assay; $K_D$ = Rezeptoraffinität des Radioliganden für den Rezeptor, mit einem Scatchard-Plot bestimmt).

[Literatur: A1-Rezeptor: Townsend-Nicholson, A. und Schofield, P. R., J. Biol. Chem. 269: 2373-2376 (1994); A2a-Rezeptor: Luthin, D. R. et al., Mol. Pharmacol. 47: 307-313 (1995); A2b-Rezeptor: Stehle, J. H. et al., Mol. Endocrinol. 6: 384-393 (1992) und Linden et al., Mol. Pharmacol. 56: 705-713 (1999); A3-Rezeptor: Salvatore, C. A. et al., Proc. Natl. Acad. Sci. U.S.A. 90: 10365-10369 (1993) und Jacobson, K. A. et al., Neuropharmacology 36: 1157-1165 (1997)].

In der folgenden Tabelle 2 sind die so bestimmten $K_j$-Werte aus diesen Bindungsassays für repräsentative Ausführungsbeispiele aufgeführt:

Tabelle 2

| Beispiel Nr. | A2b-Rezeptor $K_i$ [nmol/L] | A1-Rezeptor $K_i$ [nmol/L] | A2a-Rezeptor $K_i$ [nmol/L] | A3-Rezeptor $K_i$ [nmol/L] |
|---|---|---|---|---|
| 59 | 2.4 | 330 | 120 | 11000 |
| 66 | 5.2 | 950 | 280 | 6800 |
| 86 | 2.7 | 1100 | 270 | 160000 |
| 109 | 2.8 | 590 | 100 | 14000 |
| 113 | 6.4 | 1500 | 1700 | |
| 116 | 12 | 3300 | 790 | 330 |

B-3. Messung der NECA-induzierten IL-6-Freisetzung von LL29-Fibroblasten

Die Stimulation von Fibroblasten mit Adenosin oder dem Adenosin-Analogon 5'-*N*-Ethylcarboxamidoadenosin (NECA) führt zu einer Freisetzung des pro-inflammatorischen und pro-fibrotischen Zytokins IL-6, die durch Hemmung des A2b-Rezeptors verhindert werden kann.

Es werden daher konfluente Zellen der humanen Fibroblasten-Zelllinie LL29 mit den Testsubstanzen behandelt und mit NECA (10 $\mu$M) stimuliert. Nach einer Inkubationszeit von 24 Stunden wird der Zellüberstand abgenommen und humanes IL-6 mittels ELISA (Quantikine® IL6 ELISA, R&D Systems, Minneapolis, USA) im Zellüberstand bestimmt.

In der folgenden Tabelle 3 sind auf diese Weise erhaltene $IC_{50}$-Werte zur Inhibition der IL-6-Freisetzung für einige Ausführungsbeispiele aufgeführt:

Tabelle 3

| Beispiel Nr. | EL-6-Freisetzung $IC_{50}$ [nmol/L] |
|---|---|
| 3 | 15 |
| 20 | 5 |
| 102 | 5 |

B-4. Tiermodell der Monocrotalin-induzierten pulmonalen Hypertonie

Die Monocrotalin-induzierte pulmonale Hypertonie der Ratte ist ein weit verbreitetes Tiermodell für die pulmonale Hypertonie. Das Pyrrolizidin-Alkaloid Monocrotalin wird nach subkutaner Injektion in der Leber zum toxischen Monocrotalinpyrrol metabolisiert und führt innerhalb weniger Tage zu einer Endothelschädigung im Lungenkreislauf, gefolgt von einem Remodeling der kleinen pulmonalen Arterien (Mediahypertrophie, *de* novo-Muskularisierung). Eine einmalige subkutane Injektion ist ausreichend, um bei Ratten innerhalb von 4 Wochen eine ausgeprägte pulmonale Hypertonie zu induzieren [Cowan et al., Nature Med. 6, 698-702 (2000)].

Für das Modell werden männliche Sprague-Dawley-Ratten verwendet. An Tag 0 erhalten die Tiere eine subkutane Injektion von 60 mg/kg Monocrotalin. Die Behandlung der Tiere mit der Testsubstanz (per gavage, durch Zusatz im Futter oder Trinkwasser, per osmotischer Minipumpe, per subkutaner oder intraperitonealer Injektion oder per Inhalation) beginnt erst frühestens 14 Tage nach der Monocrotalin-Injektion und erstreckt sich über einen Zeitraum von mindestens 14 Tagen. Am Studienende erfolgen hämodynamische Untersuchungen der Tiere. Für die hämodynamische Messung werden die Ratten initial mit Pentobarbital (60 mg/kg) anästhesiert. Anschließend werden die Tiere tracheotomiert und künstlich beatmet (Frequenz: 60 Atemzüge/min; Verhältnis Inspiration zu Exspiration: 50:50; positiver endexspiratorischer Druck: 1 cm $H_2O$; Atemzugvolumen: 10 ml/kg Körpergewicht; $FIO_2$: 0.5). Die Narkose wird durch Isofluran-Inhalationsnarkose aufrecht erhalten. Der systemische Blutdruck wird in der linken *A. carotis* mittels eines Millar-Microtip-Katheters ermittelt. Ein Polyethylenkatheter wird über die rechte *V. jugularis* in den rechten Ventrikel vorgeschoben zur Bestimmung des rechten Ventrikeldrucks. Im Anschluss an die Hämodynamik wird das Herz entnommen, das Verhältnis rechter zu linker Ventrikel inklusive Septum bestimmt und das Gewebe für Expressionsanalysen tiefgefroren. Die Lunge wird ebenfalls entnommen, die linke Lungenhälfte wird in Formalin zur histopathologischen Untersuchung fixiert und die rechte Lungenhälfte wird für Expressionsanalysen tiefgefroren. Weiterhin werden Plasmaproben zur Bestimmung von Biomarkern (zum Beispiel proBNP) und Plasma-Substanzspiegeln gewonnen.

B-5. Tiermodell der SU5416/Hypoxie-induzierten pulmonalen Hypertonie

Die SU5416/Hypoxie-induzierte pulmonale Hypertonie der Ratte ist ein weit verbreitetes Tiermodell für die pulmonale Hypertonie. Durch die Injektion des VEGF-Rezeptor-Antagonisten SU5416 in Kombination mit Hypoxie kann die Auswirkung des reduzierten Sauerstoffgehalts verstärkt werden und zu Endothelveränderungen in Form von plexiformen Läsionen fuhren. Eine einmalige subkutane Injektion, in der Regel von 20 mg/kg, ist ausreichend, um in Kombination mit Hypoxie, das heißt erhöhten vaskulären Scherkräften durch Vasokonstriktion, eine schwere pulmonale Hypertonie zu induzieren [Oka et al., Circ. Res. 100, 923-929 (2007)].

Für das Modell werden männliche Sprague-Dawley-Ratten oder Dahl-Salz-Ratten verwendet. An Tag 0 erhalten die Tiere eine subkutane Injektion von SU5416 und werden in kontrollierter hypoxischer Atmosphäre (10% Sauerstoff) gehalten. Entsprechende Kontrollratten erhalten eine Injektion von Vehikel und werden unter normoxischen Bedingungen gehalten. Chronische Hypoxie von mindestens 14 Tagen mit anschließender Normoxie von mindestens 28 Tagen führt zur Entwicklung einer funktionell und morphologisch nachweisbaren pulmonalen Hypertonie. Die Behandlung der Tiere mit der Testsubstanz (per gavage, durch Zusatz im Futter oder Trinkwasser, per osmotischer Minipumpe, per subkutaner oder intraperitonealer Injektion oder per Inhalation) beginnt frühestens 14 Tage nach SU5416-Injektion und Beginn der Haltung in kontrollierter hypoxischer Atmosphäre und erstreckt sich über einen Zeitraum von mindestens 14-28 Tagen.

Am Studienende erfolgen hämodynamische Untersuchungen der Tiere. Für die hämodynamische Messung werden die Ratten initial mit Pentobarbital (60 mg/kg) anästhesiert. Anschließend werden die Tiere tracheotomiert und künstlich beatmet (Frequenz: 60 Atemzüge/min; Verhältnis Inspiration zu Exspiration: 50:50; positiver end-exspiratorischer Druck: 1 cm $H_2O$; Atemzugvolumen: 10 ml/kg Körpergewicht; $FIO_2$: 0.5). Die Narkose wird durch Isofluran-Inhalationsnarkose aufrecht erhalten. Der systemische Blutdruck wird in der linken *A. carotis* mittels eines Millar-Microtip-Katheters ermittelt. Ein Polyethylenkatheter wird über die rechte *V. jugularis* in den rechten Ventrikel vorgeschoben zur Bestimmung des rechten Ventrikeldrucks. Im Anschluss an die Hämodynamik wird das Herz entnommen, das Verhältnis rechter zu linker Ventrikel inklusive Septum bestimmt und das Gewebe für Expressionsanalysen tiefgefroren. Die Lunge wird ebenfalls entnommen, die linke Lungenhälfte wird in Formalin zur histopathologischen Untersuchung fixiert und die rechte Lungenhälfte wird für Expressionsanalysen tiefgefroren. Weiterhin werden Plasmaproben zur Bestimmung von Biomarkern (zum Beispiel proBNP) und Plasma-Substanzspiegeln gewonnen.

B-6. Tiermodell der Bleomycin-induzierten pulmonalen Fibrose

Die Bleomycin-induzierte Lungenfibrose bei der Maus oder Ratte ist ein weit verbreitetes Tiermodell für die Lungenfibrose. Bleomycin ist ein Glykopeptid-Antibiotikum, das in der Onkologie zur Therapie von Hodentumoren, Hodgkin- und Non-Hodgkin-Tumoren eingesetzt wird. Es wird renal eliminiert, besitzt eine Halbwertszeit von ca. 3 Stunden und beeinflusst als Zytostatikum verschiedene Phasen des Teilungszyklus [Lazo et al., Cancer Chemother. Biol. Response Modif. 15, 44-50 (1994)]. Sein anti-neoplastischer Effekt beruht auf einer oxidativ-schädigenden Wirkung auf DNA [Hay et al., Arch. Toxicol. 65, 81-94 (1991)]. Das Lungengewebe ist gegenüber Bleomycin in besonderer Weise gefährdet, da hier sog. Cysteinhydrolasen, welche in anderen Geweben zu einer Inaktivierung von Bleomycin führen, nur in geringer Anzahl vorhanden sind. Nach Gabe von Bleomycin kommt es bei den Tieren zu einem "acute respiratory distress syndrome" (ARDS) mit anschliessender Entwicklung einer Lungenfibrose.

Die Verabreichung des Bleomycins kann in einfacher oder mehrfacher Gabe intratracheal, inhalativ, intravenös oder intraperitoneal erfolgen. Die Behandlung der Tiere mit der Testsubstanz (per gavage, durch Zusatz im Futter oder Trinkwasser, per osmotischer Minipumpe, per subkutaner oder intraperitonealer Injektion oder per Inhalation) beginnt am Tag der ersten Applikation des Bleomycins oder therapeutisch 3-14 Tage später und erstreckt sich über einen Zeitraum von 2-6 Wochen. Am Studienende werden Lungenfunktionsmessungen, eine bronchio-alveoläre Lavage zur Bestimmung des Zellgehaltes und der pro-inflammatorischen und pro-fibrotischen Marker sowie eine histologische Beurteilung der Lungenfibrose durchgeführt.

B-7. Tiermodell der DQ12-Quarz-induzierten pulmonalen Fibrose

DQ12-Quarz-induzierte Lungenfibrose an Maus und Ratte ist ein weit verbreitetes Tiermodell für Lungenfibrose [Shimbori et al., Exp. Lung Res. 36, 292-301 (2010)]. DQ12-Quarz ist ein durch Brechen beziehungsweise Mahlen hochaktiver Quarz. Die intratracheale oder inhalative Applikation von DQ12-Quarz fuhrt bei Mäusen und Ratten zu einer Alveolarproteinose gefolgt von einer interstitiellen Lungenfibrose. Die Tiere erhalten eine einfache oder mehrfache intratracheale oder inhalative Instillation von DQ12-Quarz. Die Behandlung der Tiere mit der Testsubstanz (per gavage, durch Zusatz im Futter oder Trinkwasser, per osmotischer Minipumpe, per subkutaner oder intraperitonealer Injektion oder per Inhalation) beginnt am Tag der ersten Instillation des Silikats oder therapeutisch 3-14 Tage später und erstreckt sich über einen Zeitraum von 3-20 Wochen. Am Studienende werden Lungenfunktionsmessungen, eine bronchio-alveoläre Lavage zur Bestimmung des Zellgehalts und der pro-inflammatorischen und pro-fibrotischen Marker sowie eine

histologische Beurteilung der Lungenfibrose durchgeführt.

B-8. Tiermodell der DQ12-Quarz-oder FITC-induzierten pulmonalen Inflammation

Eine intratracheale Gabe von DQ12-Quarz oder Fluoresceinisothiocyanat (FITC) bei Maus und Ratte führt zu einer Inflammation in der Lunge [Shimbori et al., Exp. Lung Res. 36, 292-301 (2010)]. Die Tiere werden am Tag der Instillation von DQ12-Quarz oder FITC oder einen Tag später für eine Dauer von 24 h bis zu 7 Tagen mit der Testsubstanz behandelt (per gavage, durch Zusatz im Futter oder Trinkwasser, per osmotischer Minipumpe, per subkutaner oder intraperitonealer Injektion oder per Inhalation). Am Versuchsende wird eine bronchio-alveoläre Lavage zur Bestimmung des Zellgehaltes und der pro-inflammatorischen und pro-fibrotischen Marker durchgeführt.

B-9. Tiermodell der Ovalbumin-induzierten allergischen Atemwegsentzündung und Hyperreaktivität

Das Tiermodell der Ovalbumin-induzierten allergischen Atemwegsentzündung und Hyperreaktivität ist ein weit verbreitetes Tiermodell für Asthma bronchiale [Rückert et al., J. Immunol. 174, 5507-5515 (2005)]. Mäuse werden an Tag 0, 14 und 21 mittels einer intraperitonealen Injektion mit dem Allergen Ovalbumin in Kombination mit Adjuvans sensibilisiert, die Negativkontrolle erhält eine intraperitoneale Injektion von NaCl in Kombination mit Adjuvans. An Tag 28 und 29 erhalten die Tiere eine intratracheale Instillation von Ovalbumin.

An Tag 30 wird ein Hyperreaktivitätstest in Form einer inhalativen Provokation mit einer stufenweise ansteigenden Konzentration eines Bronchokonstriktors, wie z.B. Methacholin oder Adenosinmonophosphat, durchgeführt. Zunächst werden die Tiere mittels Injektionsnarkose narkotisiert, dann orotracheal intubiert oder tracheotomiert und mittels eines Tubus mit einer Lungenfunktionsanlage verbunden. Zunächst wird die Lungenfunktion vor Provokation bodyplethysmographisch gemessen (inkl. Parametern wie Atemzugvolumen, Atemfrequenz, dynamischer Compliance und Lungenresistance). Anschließend erfolgt die Messung der Lungenfunktion bei inhalativer Provokation mit einer stufenweise ansteigenden Konzentration des Bronchokonstriktors. Danach wird eine bronchio-alveoläre Lavage zur Bestimmung des Zellgehaltes und der pro-inflammatorischen Marker durchgeführt.

B-10. Tiermodell des Elastase-induzierten Lungenpuphysems

Das Elastase-induzierte Lungenemphysem bei Maus, Ratte oder Hamster ist ein weit verbreitetes Tiermodell für Lungenemphysem [Sawada et al., Exp. Lung Res. 33, 277-288 (2007)]. Die Tiere erhalten eine orotracheale Instillation porciner Pankreas-Elastase. Die Behandlung der Tiere beginnt am Tag der Instillation der porcinen Pankreas-Elastase und erstreckt sich über einen Zeitraum von 3 Wochen. Am Studienende wird eine Alveolarmorphometrie durchgeführt.

B-11. Tiermodell der permanenten Koronarligatur an Maus und Ratte

Mäuse bzw. Ratten werden mit 5% Isofluran im Narkosekäfig narkotisiert, intubiert, an eine Beatmungspumpe angeschlossen und mit 2% Isofluran/$N_2O/O_2$ beatmet. Die Körpertemperatur wird durch eine Wärmematte auf 37-38°C gehalten. Als Schmerzmittel wird Temgesic® gegeben. Der Brustkorb wird zwischen der dritten und vierten Rippe seitlich geöffnet und das Herz freigelegt. Die Herzkranzarterie des linken Ventrikels (LAD) wird mit einem Okklusionsfaden kurz unterhalb ihres Ursprungs (unterhalb des linken Atriums) unterstochen und permanent abgebunden. Der Thorax wird wieder geschlossen und die Muskelschichten und die Oberhaut zugenäht. Die Tiere werden vom Tag der Operation an oder bis zu einer Woche später für einen Zeitraum von 4-8 Wochen mit der Testsubstanz behandelt (per gavage, durch Zusatz der Testsubstanz im Futter oder Trinkwasser, per osmotischer Minipumpe, per subkutaner oder intraperitonealer Injektion oder per Inhalation). Als weitere Kontrolle wird eine "sham"-Gruppe mitgeführt, bei der nur der Operationsvorgang, nicht aber die LAD-Okklusion durchgeführt wurde.

Am Versuchsende werden die Tiere erneut narkotisiert [1.5% Isofluran (Maus), 2% Isofluran (Ratte)/$N_2O$/Luft], und ein Druckkatheter wird über die *A. carotis* in den linken Ventrikel eingeführt. Dort werden Herzfrequenz, linksventrikulärer Druck (LVP), linksventrikulärer end-diastolischer Druck (LVEDP), Kontraktilität (dp/dt) und Relaxationsgeschwindigkeit (tau) mit Hilfe des Powerlab-Systems (AD Instruments, ADI-PWLB-4SP) und der ChartS-Software (SN 425-0586) erfasst und ausgewertet. Anschließend wird eine Blutprobe zur Bestimmung der Substanz-Plasmaspiegel und Plasmabiomarker entnommen und die Tiere abgetötet. Herz (Herzkammern, linker Ventrikel plus Septum, rechter Ventrikel), Leber, Lunge und Niere werden entnommen und gewogen.

B-12. Tiermodell des Tumorwachstums

Syngene Tumormodelle in immunkompetenten Mäusen bzw. xenogene Tumormodelle in immunsupprimierten Mäusen werden zur Substanzbewertung herangezogen. Dazu werden Tumorzellen *in vitro* kultiviert und subkutan bzw.

orthotop implantiert. Die Behandlung der Tiere erfolgt durch orale, subkutane, intraperitoneale oder intravenöse Therapie nach der Etablierung des Tumors oder beginnend mit dem Tag der Tumorinokulation. Die Wirksamkeit von Testsubstanzen wird in Monotherapie und in Kombinationstherapie mit anderen pharmakologischen Wirksubstanzen analysiert. Während des Experiments wird der Gesundheitszustand der Tiere täglich überprüft, und die Behandlungen erfolgen entsprechend den Tierschutzbestimmungen. Die Tumorfläche wird mit Schublehren gemessen (Länge L, Breite B = kleinere Ausdehnung). Das Tumorvolumen wird nach der Formel (L x $B^2$)/2 berechnet. Die Hemmung des Tumorwachstums wird am Ende des Versuchs als T/C-Verhältnis der Tumorflächen bzw. Tumorgewichte und als TGI-Wert (tumor growth inhibition, berechnet nach der Formel [1-(T/C)] x 100) bestimmt (T = Tumorgröße der behandelten Gruppe; C = Tumorgröße der unbehandelten Kontrollgruppe).

B-13. Tiermodell der Bildung von Metastasen in der Lunge

Syngene Tumormodelle in immunkompetenten Mäusen bzw. xenogene Tumormodelle in immunsupprimierten Mäusen werden zur Substanzbewertung herangezogen. Dazu werden Tumorzellen *in vitro* kultiviert und intravenös in die Schwanzvene der Versuchstiere injiziert. Die Behandlung der Tiere erfolgt durch orale, subkutane, intraperitoneale oder intravenöse Therapie. Die Wirksamkeit von Testsubstanzen wird in Monotherapie und in Kombinationstherapie mit anderen pharmakologischen Wirksubstanzen analysiert. Während des Experiments wird der Gesundheitszustand der Tiere täglich überprüft, und die Behandlungen erfolgen entsprechend den Tierschutzbestimmungen. Nach Beendigung des Experiments werden die Lungen der Versuchstiere hinsichtlich der Zahl gebildeter Tumorkolonien mikroskopisch untersucht.

**C. Ausführungsbelspiele für pharmazeutische Zusammensetzungen**

Die erfindungsgemäßen Verbindungen können folgendermaßen in pharmazeutische Zubereitungen überführt werden:

**Tablette:**

Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

**Oral applizierbare Suspension:**

Zusammensetzung:

1000 mg der erfindungsgemäßen Verbindung, 1000 mg Ethanol (96%), 400 mg Rhodigel® (Xanthan gum der Firma FMC, Pennsylvania, USA) und 99 g Wasser.

Einer Einzeldosis von 100 mg der erfindungsgemäßen Verbindung entsprechen 10 ml orale Suspension.

Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

**Oral applizierbare Lösung:**

Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

**i.v.-Lösung:**

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I)

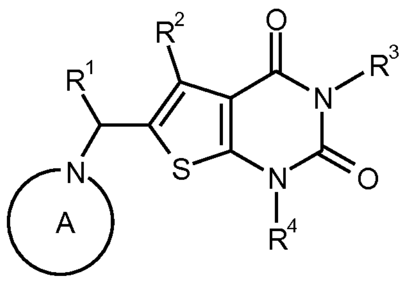

(I),

in welcher
der Ring A für einen Aza-Heterocyclus der Formel

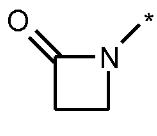 , 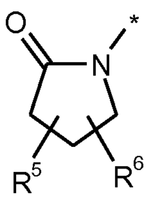 , 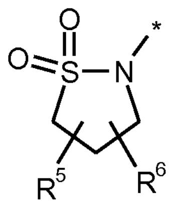 , 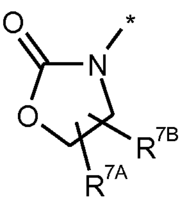 ,

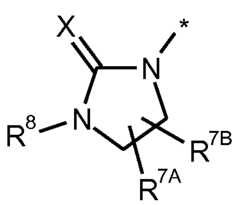 , 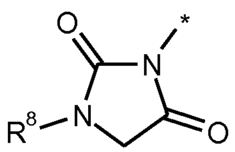 , 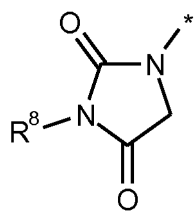 ,

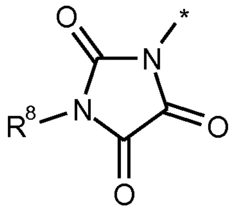, 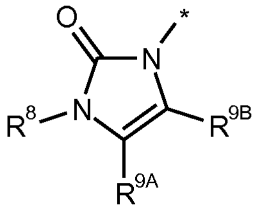, 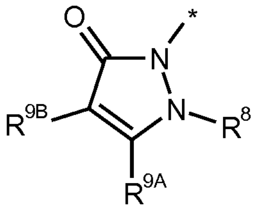,

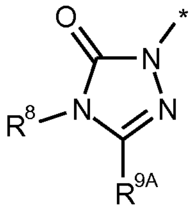, 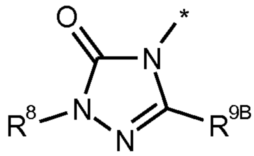, 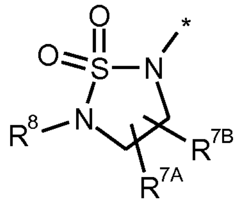, 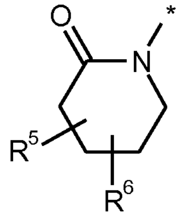,

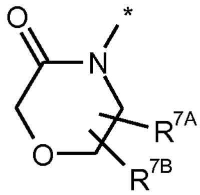, 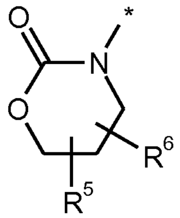, 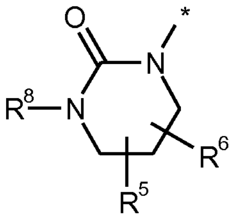, 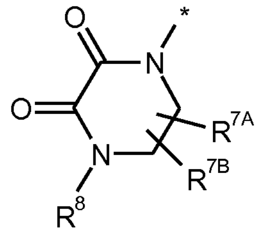,

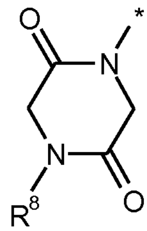 oder 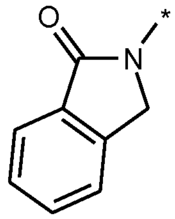

steht,
worin * die Verknüpfung zur angrenzenden $CH(R^1)$-Gruppe markiert,

$R^5$ Wasserstoff, $(C_1-C_4)$-Alkyl, Hydroxy, $(C_1-C_4)$-Alkoxy, Amino, $(C_1-C_5)$-Alkanoylamino oder $(C_1-C_4)$-Alkoxycarbonylamino bedeutet,
$R^6$ Wasserstoff, Methyl oder Ethyl bedeutet,
$R^{7A}$ und $R^{7B}$ gleich oder verschieden sind und unabhängig voneinander Wasserstoff oder $(C_1-C_4)$-Alkyl bedeuten,
$R^8$ Wasserstoff, $(C_1-C_4)$-Alkyl, $(C_2-C_4)$-Alkenyl, $(C_1-C_5)$-Alkanoyl oder $(C_1-C_4)$-Alkoxycarbonyl bedeutet, wobei $(C_1-C_4)$-Alkyl bis zu zweifach mit Hydroxy substituiert sein kann,
$R^{9A}$ und $R^{9B}$ gleich oder verschieden sind und unabhängig voneinander Wasserstoff oder $(C_1-C_4)$-Alkyl bedeuten

und

X O, $N(R^{10})$ oder S bedeutet, worin

$R^{10}$ Wasserstoff, Cyano oder $(C_1-C_4)$-Alkoxycarbonyl darstellt,

$R^1$ für Wasserstoff oder Methyl steht,
$R^2$ für Wasserstoff, Methyl oder Ethyl steht, wobei Methyl und Ethyl bis zu dreifach mit Fluor substituiert sein können,
$R^3$ für $(C_2-C_6)$-Alkyl, $(C_2-C_6)$-Alkenyl oder $(C_2-C_6)$-Alkinyl steht,

wobei ($C_2$-$C_6$)-Alkyl mit einem Rest ausgewählt aus der Reihe Hydroxy, Methoxy, Ethoxy, Trifluormethoxy, Cyclopropyl, Cyclobutyl, Oxetanyl und Phenyl sowie bis zu dreifach mit Fluor substituiert sein kann
und
($C_2$-$C_6$)-Alkenyl bis zu dreifach mit Fluor substituiert sein kann,
wobei die genannten Cyclopropyl- und Cyclobutyl-Gruppen ihrerseits bis zu zweifach, gleich oder verschieden, mit einem Rest ausgewählt aus Fluor und Methyl substituiert sein können,

oder

$R^3$ für eine Gruppe der Formel $-CH_2-R^{14}$ steht, worin

$R^{14}$ Cyclopropyl, Cyclobutyl, Oxetanyl oder Tetrahydrofuranyl bedeutet,
wobei Cyclopropyl, Cyclobutyl und Oxetanyl bis zu zweifach, gleich oder verschieden, mit einem Rest ausgewählt aus Fluor und Methyl substituiert sein können,

und

$R^4$ für ($C_1$-$C_6$)-Alkyl oder ($C_2$-$C_6$)-Alkenyl steht,
wobei ($C_1$-$C_6$)-Alkyl bis zu fünffach und ($C_2$-$C_6$)-Alkenyl bis zu dreifach mit Fluor substituiert sein können
und
wobei in ($C_1$-$C_6$)-Alkyl eine $CH_2$-Gruppe gegen -O-, -S- oder $-S(O)_2-$ ausgetauscht sein kann, mit der Maßgabe, dass sich zwischen einem solchen Heteroatom und dem Uracil-$N^1$-Atom mindestens zwei Kohlenstoffatome befinden,

oder

$R^4$ für eine Gruppe der Formel $-(CH_2)_m-CN$, $-(CH_2)_n-R^{11}$ oder $-(CH_2)_p-R^{12}$ steht, worin

m die Zahl 1, 2, 3 oder 4 bedeutet,
n die Zahl 2 oder 3 bedeutet,
p die Zahl 1 oder 2 bedeutet,
$R^{11}$ Dimethylamino, Diethylamino oder Azetidino bedeutet

und

$R^{12}$ ($C_3$-$C_6$)-Cycloalkyl, Oxetanyl, Tetrahydrofuranyl, Tetrahydropyranyl oder 5-gliedriges Aza-Heteroaryl bedeutet,
wobei ($C_3$-$C_6$)-Cycloalkyl bis zu zweifach, gleich oder verschieden, mit einem Rest ausgewählt aus Fluor und Methyl substituiert sein kann
und
Aza-Heteroaryl bis zu zweifach, gleich oder verschieden, mit einem Rest ausgewählt aus Methyl und Trifluormethyl substituiert sein kann,

oder

$R^4$ für eine Gruppe der Formel $-(CH_2)_2-O-R^{13}$ steht, worin

$R^{13}$ ($C_3$-$C_6$)-Cycloalkyl bedeutet,

sowie ihre Salze, Solvate und Solvate der Salze.

**2.** Verbindung der Formel (I) nach Anspruch 1, in welcher der Ring A für einen Aza-Heterocyclus der Formel

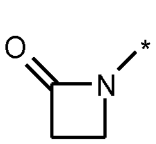, 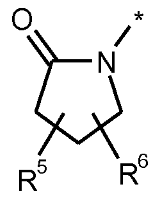, 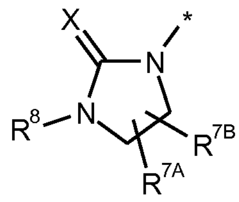, 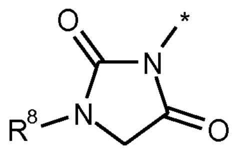,

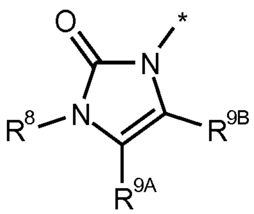, 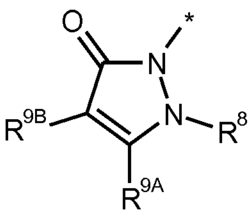, 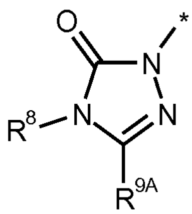,

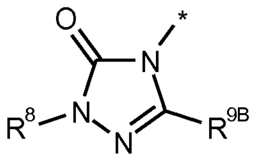, 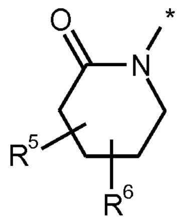, 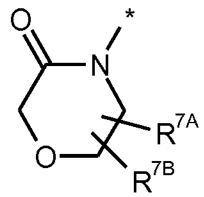, 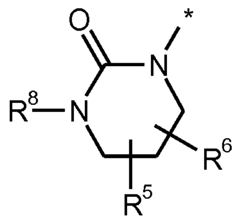

oder

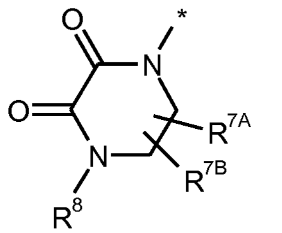

steht,
worin * die Verknüpfung zur angrenzenden $CH(R^1)$-Gruppe markiert,

$R^5$ Wasserstoff, $(C_1\text{-}C_4)$-Alkyl, Hydroxy, $(C_1\text{-}C_4)$-Alkoxy, Amino, $(C_1\text{-}C_5)$-Alkanoylamino oder $(C_1\text{-}C_4)$-Alkoxycarbonylamino bedeutet,
$R^6$ Wasserstoff, Methyl oder Ethyl bedeutet,
$R^{7A}$ und $R^{7B}$ gleich oder verschieden sind und unabhängig voneinander Wasserstoff oder $(C_1\text{-}C_4)$-Alkyl bedeuten,
$R^8$ Wasserstoff, $(C_1\text{-}C_4)$-Alkyl, $(C_2\text{-}C_4)$-Alkenyl, $(C_1\text{-}C_5)$-Alkanoyl oder $(C_1\text{-}C_4)$-Alkoxycarbonyl bedeutet, wobei $(C_1\text{-}C_4)$-Alkyl bis zu zweifach mit Hydroxy substituiert sein kann,
$R^{9A}$ und $R^{9B}$ gleich oder verschieden sind und unabhängig voneinander Wasserstoff oder $(C_1\text{-}C_4)$-Alkyl bedeuten

und

X O, $N(R^{10})$ oder S bedeutet, worin

$R^{10}$ Wasserstoff, Cyano oder $(C_1\text{-}C_4)$-Alkoxycarbonyl darstellt,

$R^1$ für Wasserstoff steht,
$R^2$ für Methyl oder Ethyl, welche bis zu dreifach mit Fluor substituiert sein können, steht,
$R^3$ für $(C_2\text{-}C_6)$-Alkyl, $(C_2\text{-}C_6)$-Alkenyl oder $(C_2\text{-}C_6)$-Alkinyl steht,

wobei $(C_2-C_6)$-Alkyl mit einem Rest ausgewählt aus der Reihe Hydroxy, Methoxy, Ethoxy, Trifluormethoxy, Cyclopropyl, Cyclobutyl, Oxetanyl und Phenyl sowie bis zu dreifach mit Fluor substituiert sein kann
und
$(C_2-C_6)$-Alkenyl bis zu dreifach mit Fluor substituiert sein kann,
wobei die genannten Cyclopropyl- und Cyclobutyl-Gruppen ihrerseits bis zu zweifach, gleich oder verschieden, mit einem Rest ausgewählt aus Fluor und Methyl substituiert sein können,

oder

$R^3$ für eine Gruppe der Formel $-CH_2-R^{14}$ steht, worin

$R^{14}$ Cyclopropyl, Cyclobutyl, Oxetanyl oder Tetrahydrofuranyl bedeutet, wobei Cyclopropyl und Cyclobutyl bis zu zweifach, gleich oder verschieden, mit einem Rest ausgewählt aus Fluor und Methyl substituiert sein können,

und

$R^4$ für $(C_1-C_6)$-Alkyl oder $(C_2-C_6)$-Alkenyl steht,
wobei $(C_1-C_6)$-Alkyl und $(C_2-C_6)$-Alkenyl bis zu dreifach mit Fluor substituiert sein können
und
wobei in $(C_1-C_6)$-Alkyl eine $CH_2$-Gruppe gegen -O-, -S- oder $-S(O)_2-$ ausgetauscht sein kann, mit der Maßgabe, dass sich zwischen einem solchen Heteroatom und dem Uracil-$N^1$-Atom mindestens zwei Kohlenstoffatome befinden,

oder

$R^4$ für eine Gruppe der Formel $-(CH_2)_m-CN$, $-(CH_2)_n-R^{11}$ oder $-(CH_2)_p-R^{12}$ steht, worin

m die Zahl 1, 2, 3 oder 4 bedeutet,
n die Zahl 2 oder 3 bedeutet,
p die Zahl 1 oder 2 bedeutet,
$R^{11}$ Dimethylamino, Diethylamino oder Azetidino bedeutet

und

$R^{12}$ $(C_3-C_6)$-Cycloalkyl, Oxetanyl, Tetrahydrofuranyl, Tetrahydropyranyl oder 5-gliedriges Aza-Heteroaryl bedeutet,
wobei $(C_3-C_6)$-Cycloalkyl bis zu zweifach, gleich oder verschieden, mit einem Rest ausgewählt aus Fluor und Methyl substituiert sein kann
und
Aza-Heteroaryl bis zu zweifach, gleich oder verschieden, mit einem Rest ausgewählt aus Methyl und Trifluormethyl substituiert sein kann,

oder

$R^4$ für eine Gruppe der Formel $-(CH_2)_2-O-R^{13}$ steht, worin

$R^{13}$ Cyclopropyl oder Cyclobutyl bedeutet,

sowie ihre Salze, Solvate und Solvate der Salze.

**3.** Verbindung der Formel (I) nach Anspruch 1 oder 2, in welcher
der Ring A für einen Aza-Heterocyclus der Formel

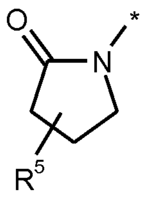 , 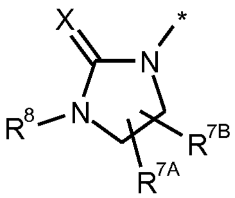 , 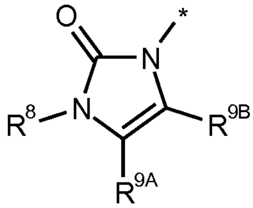 , 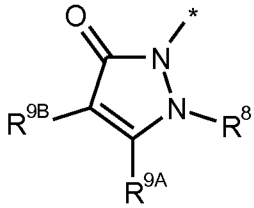 ,

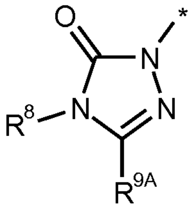 , 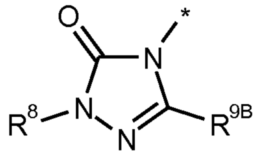 , 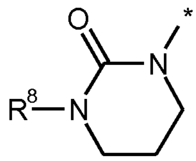

oder

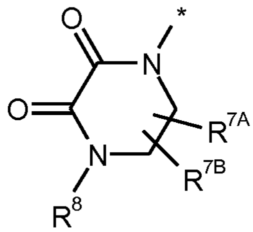

steht,
worin * die Verknüpfung zur angrenzenden CH($R^1$)-Gruppe markiert,

$R^5$ Hydroxy, Methoxy oder Ethoxy bedeutet,
$R^{7A}$ und $R^{7B}$ unabhängig voneinander Wasserstoff oder Methyl bedeuten,
$R^8$ Wasserstoff, Methyl, Ethyl, *n*-Propyl, 2-Hydroxyethyl, 3-Hydroxypropyl, 2,3-Dihydroxypropyl, Allyl, Formyl oder Acetyl bedeutet,
$R^{9A}$ und $R^{9B}$ unabhängig voneinander Wasserstoff oder Methyl bedeuten

und

X O, N($R^{10}$) oder S bedeutet, worin

$R^{10}$ Cyano oder ($C_1$-$C_4$)-Alkoxycarbonyl darstellt,

$R^1$ für Wasserstoff steht,
$R^2$ für Methyl oder Ethyl, welche bis zu dreifach mit Fluor substituiert sein können, steht,
$R^3$ für ($C_2$-$C_5$)-Alkyl oder ($C_2$-$C_4$)-Alkenyl steht,
wobei ($C_2$-$C_5$)-Alkyl mit einem Rest ausgewählt aus der Reihe Hydroxy, Methoxy, Cyclopropyl, Cyclobutyl, Oxetanyl und Phenyl sowie bis zu dreifach mit Fluor substituiert sein kann
und
($C_2$-$C_4$)-Alkenyl bis zu dreifach mit Fluor substituiert sein kann,
wobei die genannten Cyclopropyl- und Cyclobutyl-Gruppen ihrerseits bis zu zweifach mit Fluor substituiert sein können,

oder

$R^3$ für eine Gruppe der Formel -$CH_2$-$R^{14}$ steht, worin

$R^{14}$ Cyclopropyl, Cyclobutyl oder Oxetanyl bedeutet,
wobei Cyclopropyl und Cyclobutyl bis zu zweifach, gleich oder verschieden, mit einem Rest ausgewählt

aus Fluor und Methyl substituiert sein können,

und

$R^4$ für ($C_1$-$C_6$)-Alkyl oder ($C_2$-$C_6$)-Alkenyl steht,
wobei ($C_1$-$C_6$)-Alkyl und ($C_2$-$C_6$)-Alkenyl bis zu dreifach mit Fluor substituiert sein können
und
wobei in ($C_1$-$C_6$)-Alkyl eine $CH_2$-Gruppe gegen -O- oder -S- ausgetauscht sein kann, mit der Maßgabe, dass sich zwischen einem solchen Heteroatom und dem Uracil-$N^1$-Atom mindestens zwei Kohlenstoffatome befinden,

oder

$R^4$ für die Gruppe $-CH_2-R^{12}$ steht, worin

$R^{12}$ ($C_3$-$C_6$)-Cycloalkyl, Oxetanyl, Tetrahydrofuranyl oder Tetrahydropyranyl bedeutet,
wobei ($C_3$-$C_6$)-Cycloalkyl bis zu zweifach, gleich oder verschieden, mit einem Rest ausgewählt aus Fluor und Methyl substituiert sein kann,

sowie ihre Salze, Solvate und Solvate der Salze.

**4.** Verbindung der Formel (I) nach Anspruch 1, 2 oder 3, in welcher der Ring A für einen Aza-Heterocyclus der Formel

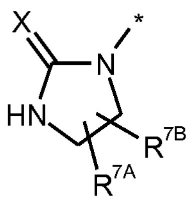 , 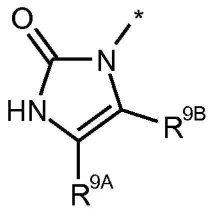 , 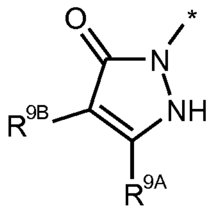 , 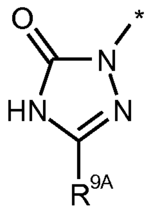 ,

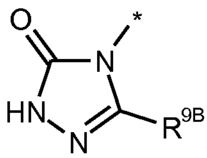 oder 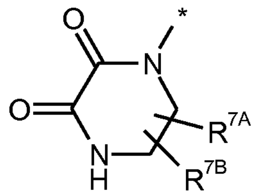

steht,
worin * die Verknüpfung zur angrenzenden $CH(R^1)$-Gruppe markiert,

$R^{7A}$ und $R^{7B}$ unabhängig voneinander Wasserstoff oder Methyl bedeuten,
$R^{9A}$ und $R^{9B}$ unabhängig voneinander Wasserstoff oder Methyl bedeuten

und

X O, $N(R^{10})$ oder S bedeutet, worin

$R^{10}$ Cyano, Methoxycarbonyl, Ethoxycarbonyl oder *tert.*-Butoxycarbonyl darstellt,

$R^1$ für Wasserstoff steht,
$R^2$ für Methyl, Difluormethyl oder Trifluormethyl steht,
$R^3$ für ($C_2$-$C_5$)-Alkyl, das mit Hydroxy, Methoxy oder Cyclopropyl oder bis zu dreifach mit Fluor substituiert sein kann, steht,
wobei Cyclopropyl seinerseits bis zu zweifach mit Fluor substituiert sein kann,

und

$R^4$ für ($C_1$-$C_4$)-Alkyl, das bis zu dreifach mit Fluor substituiert sein kann, für 2-Methoxyethyl oder 2-Ethoxyethyl oder für die Gruppe -$CH_2$-$R^{12}$ steht, worin

$R^{12}$ Cyclopropyl, Cyclobutyl, Oxetanyl, Tetrahydrofuranyl oder Tetrahydropyranyl bedeutet, wobei Cyclopropyl und Cyclobutyl bis zu zweifach mit Fluor substituiert sein können,

sowie ihre Salze, Solvate und Solvate der Salze.

**5.** Verbindung, wie in einem der Ansprüche 1 bis 4 definiert, zur Behandlung und/oder Prävention von Krankheiten.

**6.** Verbindung, wie in einem der Ansprüche 1 bis 4 definiert, zur Verwendung in einem Verfahren zur Behandlung und/oder Prävention von idiopathischer Lungenfibrose, pulmonaler Hypertonie, Bronchiolitis obliterans-Syndrom, chronisch-obstruktiver Lungenerkrankung, Asthma, zystischer Fibrose, Myokardinfarkt, Herzinsuffizienz, Sichelzellanämie und von Krebserkrankungen.

**7.** Verwendung einer Verbindung, wie in einem der Ansprüche 1 bis 4 definiert, zur Herstellung eines Arzneimittels zur Behandlung und/oder Prävention von idiopathischer Lungenfibrose, pulmonaler Hypertonie, Bronchiolitis obliterans-Syndrom, chronisch-obstruktiver Lungenerkrankung, Asthma, zystischer Fibrose, Myokardinfarkt, Herzinsuffizienz, Sichelzellanämie und von Krebserkrankungen.

**8.** Arzneimittel enthaltend eine Verbindung, wie in einem der Ansprüche 1 bis 4 definiert, in Kombination mit einem oder mehreren inerten, nicht-toxischen, pharmazeutisch geeigneten Hilfsstoffen.

**9.** Arzneimittel enthaltend eine Verbindung, wie in einem der Ansprüche 1 bis 4 definiert, in Kombination mit einem oder mehreren weiteren Wirkstoffen ausgewählt aus der Gruppe bestehend aus PDE 5-Inhibitoren, sGC-Aktivatoren, sGC-Stimulatoren, Prostacyclin-Analoga, IP-Rezeptor-Agonisten, Endothelin-Antagonisten, antifibrotisch wirkenden Mitteln, entzündungshemmend, immunmodulierend, immunsuppressiv und/oder zytotoxisch wirkenden Mitteln und die Signaltransduktionskaskade inhibierenden Verbindungen.

**10.** Arzneimittel nach Anspruch 8 oder 9 zur Behandlung und/oder Prävention von idiopathischer Lungenfibrose, pulmonaler Hypertonie, Bronchiolitis obliterans-Syndrom, chronisch-obstruktiver Lungenerkrankung, Asthma, zystischer Fibrose, Myokardinfarkt, Herzinsuffizienz, Sichelzellanämie und von Krebserkrankungen.

**11.** Verbindung der Formel (I-A)

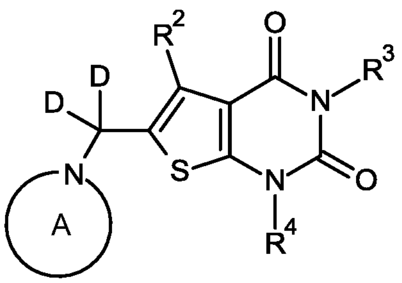

(I-A),

in welcher der Ring A sowie die Reste $R^2$, $R^3$ und $R^4$ die in den Ansprüchen 1 bis 4 angegebenen Bedeutungen haben,
sowie ihre Salze, Solvate und Solvate der Salze.

## Claims

**1.** Compound of the formula (I)

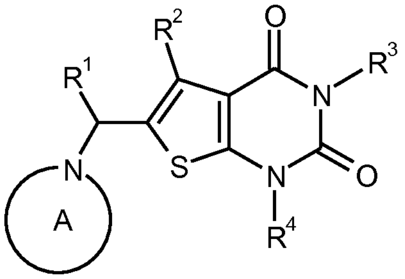

(I),

in which

the ring A is an azaheterocycle of the formula

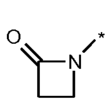, 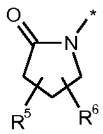, 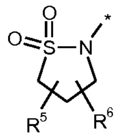, 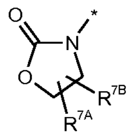,

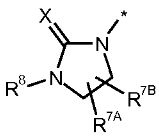, 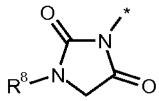, 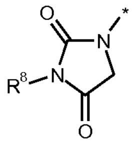,

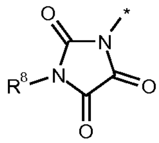, 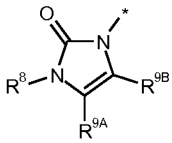, 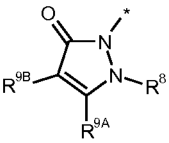,

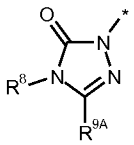, 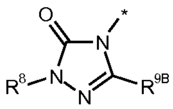, 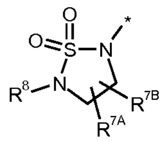, 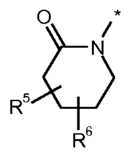,

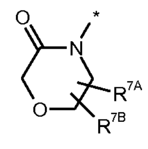, 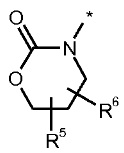, 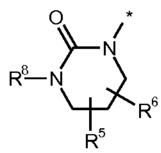, 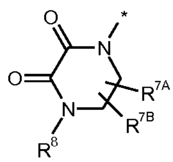,

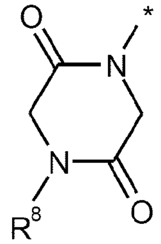 or 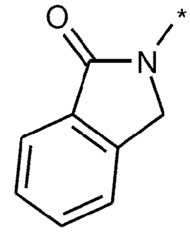

in which * marks the bond to the adjoining $CH(R^1)$ group,

$R^5$ is hydrogen, $(C_1-C_4)$ -alkyl, hydroxyl, $(C_1-C_4)$ -alkoxy, amino, $(C_1-C_5)$-alkanoylamino or $(C_1-C_4)$-alkoxycarbonylamino,

$R^6$ is hydrogen, methyl or ethyl,
$R^{7A}$ and $R^{7B}$ are the same or different and are independently hydrogen or $(C_1\text{-}C_4)$-alkyl,
$R^8$ is hydrogen, $(C_1\text{-}C_4)$ -alkyl, $(C_2\text{-}C_4)$-alkenyl, $(C_1\text{-}C_5)$ -alkanoyl or $(C_1\text{-}C_4)$-alkoxycarbonyl, where $(C_1\text{-}C_4)$-alkyl may be up to disubstituted by hydroxyl,
$R^{9A}$ and $R^{9B}$ are the same or different and are independently hydrogen or $(C_1\text{-}C_4)$-alkyl

and

X is 0, $N(R^{10})$ or S, in which
$R^{10}$ is hydrogen, cyano or $(C_1\text{-}C_4)$-alkoxycarbonyl,
$R^1$ is hydrogen or methyl,
$R^2$ is hydrogen, methyl or ethyl, where methyl and ethyl may be up to trisubstituted by fluorine,
$R^3$ is $(C_2\text{-}C_6)$ -alkyl, $(C_2\text{-}C_6)$ -alkenyl or $(C_2\text{-}C_6)$-alkynyl,
where $(C_2\text{-}C_6)$ -alkyl may be substituted by a radical selected from the group of hydroxyl, methoxy, ethoxy, trifluoromethoxy, cyclopropyl, cyclobutyl, oxetanyl and phenyl, and up to trisubstituted by fluorine,
and
$(C_2\text{-}C_6)$-alkenyl may be up to trisubstituted by fluorine,
where the cyclopropyl and cyclobutyl groups mentioned may in turn be up to disubstituted, identically or differently, by a radical selected from fluorine and methyl,

or

$R^3$ is a group of the formula $-CH_2\text{-}R^{14}$ in which $R^{14}$ is cyclopropyl, cyclobutyl, oxetanyl or tetrahydrofuranyl, where cyclopropyl, cyclobutyl and oxetanyl may be up to disubstituted, identically or differently, by a radical selected from fluorine and methyl,

and

$R^4$ is $(C_1\text{-}C_6)$ -alkyl or $(C_2\text{-}C_6)$ -alkenyl,
where $(C_1\text{-}C_6)$ -alkyl may be up to pentasubstituted and $(C_2\text{-}C_6)$-alkenyl up to trisubstituted by fluorine
and
where one $CH_2$ group in $(C_1\text{-}C_6)$ -alkyl may be exchanged for -O-, -S- or $-S(O)_2-$, with the proviso that there are at least two carbon atoms between such a heteroatom and the uracil $N^1$ atom,

or

$R^4$ is a group of the formula $-(CH_2)_m\text{-}CN$, $-(CH_2)_n\text{-}R^{11}$ or $-(CH_2)_p\text{-}R^{12}$, in which

m is the number 1, 2, 3 or 4,
n is the number 2 or 3,
p is the number 1 or 2,
$R^{11}$ is dimethylamino, diethylamino or azetidino
and
$R^{12}$ is $(C_3\text{-}C_6)$ -cycloalkyl, oxetanyl, tetrahydrofuranyl, tetrahydropyranyl or 5-membered azaheteroaryl, where $(C_3\text{-}C_6)$ -cycloalkyl may be up to disubstituted, identically or differently, by a radical selected from fluorine and methyl
and
azaheteroaryl may be up to disubstituted, identically or differently, by a radical selected from methyl and trifluoromethyl,

or
$R^4$ is a group of the formula $-(CH_2)_2\text{-}O\text{-}R^{13}$ in which
$R^{13}$ is $(C_3\text{-}C_6)$ -cycloalkyl,
and the salts, solvates and solvates of the salts thereof.

**2.** Compound of the formula (I) according to Claim 1, in which the ring A is an azaheterocycle of the formula

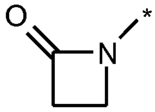, 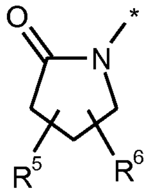, 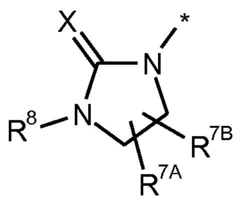, 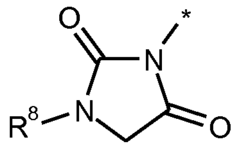,

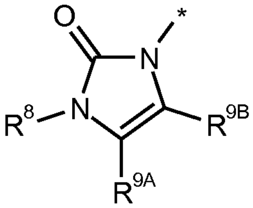, 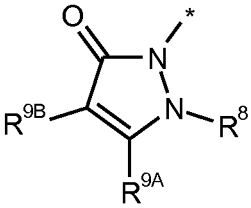, 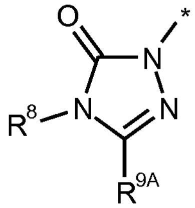,

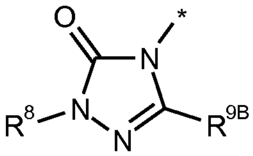, 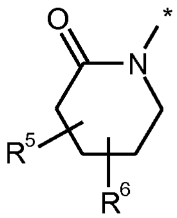, 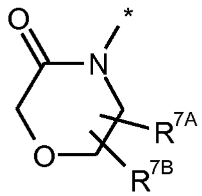, 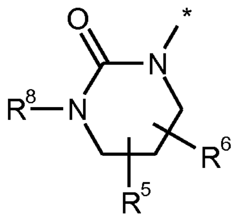

or

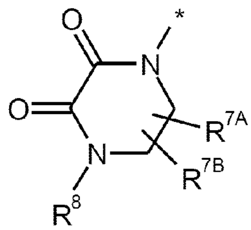

in which * marks the bond to the adjoining $CH(R^1)$ group,

$R^5$ is hydrogen, $(C_1-C_4)$ -alkyl, hydroxyl, $(C_1-C_4)$ -alkoxy, amino, $(C_1-C_5)$-alkanoylamino or $(C_1-C_4)$-alkoxycarbonylamino,
$R^6$ is hydrogen, methyl or ethyl,
$R^{7A}$ and $R^{7B}$ are the same or different and are independently hydrogen or $(C_1-C_4)$-alkyl,
$R^8$ is hydrogen, $(C_1-C_4)$ -alkyl, $(C_2-C_4)$-alkenyl, $(C_1-C_5)$-alkanoyl or $(C_1-C_4)$-alkoxycarbonyl, where $(C_1-C_4)$-alkyl may be up to disubstituted by hydroxyl,
$R^{9A}$ and $R^{9B}$ are the same or different and are independently hydrogen or $(C_1-C_4)$-alkyl

and

X is O, $N(R^{10})$ or S, in which
$R^{10}$ is hydrogen, cyano or $(C_1-C_4)$-alkoxycarbonyl,
$R^1$ is hydrogen,
$R^2$ is methyl or ethyl which may be up to trisubstituted by fluorine,
$R^3$ is $(C_2-C_6)$ -alkyl, $(C_2-C_6)$ -alkenyl or $(C_2-C_6)$-alkynyl,
where $(C_2-C_6)$ -alkyl may be substituted by a radical selected from the group of hydroxyl, methoxy, ethoxy, trifluoromethoxy, cyclopropyl, cyclobutyl, oxetanyl and phenyl, and up to trisubstituted by fluorine,
and
$(C_2-C_6)$-alkenyl may be up to trisubstituted by fluorine,
where the cyclopropyl and cyclobutyl groups mentioned may in turn be up to disubstituted, identically or differently, by a radical selected from fluorine and methyl,

or

$R^3$ is a group of the formula $-CH_2-R^{14}$ in which $R^{14}$ is cyclopropyl, cyclobutyl, oxetanyl or tetrahydrofuranyl, where cyclopropyl and cyclobutyl may be up to disubstituted, identically or differently, by a radical selected from fluorine and methyl,

and

$R^4$ is $(C_1-C_6)$-alkyl or $(C_2-C_6)$-alkenyl,
where $(C_1-C_6)$ -alkyl and $(C_2-C_6)$-alkenyl may be up to trisubstituted by fluorine
and
where one $CH_2$ group in $(C_1-C_6)$-alkyl may be exchanged for -O-, -S- or $-S(O)_2-$, with the proviso that there are at least two carbon atoms between such a heteroatom and the uracil $N^1$ atom,

or

$R^4$ is a group of the formula $-(CH_2)_m-CN$, $-(CH_2)_n-R^{11}$ or $-(CH_2)_p-R^{12}$, in which

m is the number 1, 2, 3 or 4,
n is the number 2 or 3,
p is the number 1 or 2,
$R^{11}$ is dimethylamino, diethylamino or azetidino
and
$R^{12}$ is $(C_3-C_6)$-cycloalkyl, oxetanyl, tetrahydrofuranyl, tetrahydropyranyl or 5-membered azaheteroaryl,
where $(C_3-C_6)$-cycloalkyl may be up to disubstituted, identically or differently, by a radical selected from fluorine and methyl
and
azaheteroaryl may be up to disubstituted, identically or differently, by a radical selected from methyl and trifluoromethyl,

or

$R^4$ is a group of the formula $-(CH_2)_2-O-R^{13}$ in which
$R^{13}$ is cyclopropyl or cyclobutyl,
and the salts, solvates and solvates of the salts thereof.

**3.** Compound of the formula (I) according to Claim 1 or 2, in which the ring A is an azaheterocycle of the formula

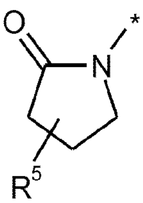 , 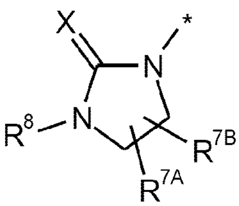 , 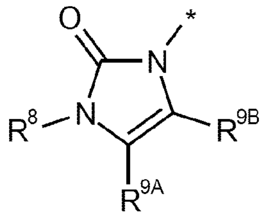 , 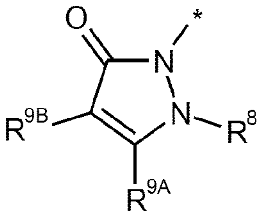 ,

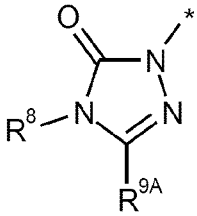 , 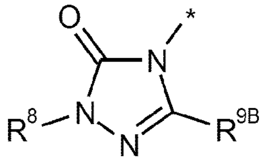 , 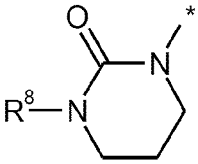

or

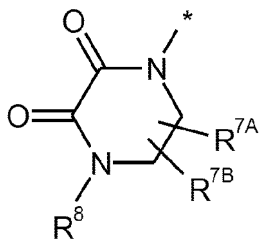

in which * marks the bond to the adjoining CH($R^1$) group,
$R^5$ is hydroxy, methoxy or ethoxy,
$R^{7A}$ and $R^{7B}$ are each independently hydrogen or methyl,
$R^8$ is hydrogen, methyl, ethyl, *n*-propyl, 2-hydroxyethyl, 3-hydroxypropyl, 2,3-dihydroxypropyl, allyl, formyl or acetyl,
$R^{9A}$ and $R^{9B}$ are each independently hydrogen or methyl
and
X is 0, N($R^{10}$) or S, in which
$R^{10}$ is cyano or ($C_1$-$C_4$) -alkoxycarbonyl,
$R^1$ is hydrogen,
$R^2$ is methyl or ethyl which may be up to trisubstituted by fluorine,
$R^3$ is ($C_2$-$C_5$)-alkyl or ($C_2$-$C_4$)-alkenyl,
where ($C_2$-$C_5$)-alkyl may be substituted by a radical selected from the group of hydroxyl, methoxy, cyclopropyl, cyclobutyl, oxetanyl and phenyl, and up to trisubstituted by fluorine,

and

($C_2$-$C_4$)-alkenyl may be up to trisubstituted by fluorine,
where the cyclopropyl and cyclobutyl groups mentioned may in turn be up to disubstituted by fluorine,

or

$R^3$ is a group of the formula -$CH_2$-$R^{14}$ in which

$R^{14}$ is cyclopropyl, cyclobutyl or oxetanyl,
where cyclopropyl and cyclobutyl may be up to disubstituted, identically or differently, by a radical selected from fluorine and methyl,

and

$R^4$ is ($C_1$-$C_6$) -alkyl or ($C_2$-$C_6$) -alkenyl,
where ($C_1$-$C_6$) -alkyl and ($C_2$-$C_6$) -alkenyl may be up to trisubstituted by fluorine
and
where one $CH_2$ group in ($C_1$-$C_6$) -alkyl may be exchanged for -0- or -S-, with the proviso that there are at least two carbon atoms between such a heteroatom and the uracil $N^1$ atom,
or
$R^4$ is the -$CH_2$-$R^{12}$ group in which

$R^{12}$ is ($C_3$-$C_6$) -cycloalkyl, oxetanyl, tetrahydrofuranyl or tetrahydropyranyl,
where ($C_3$-$C_6$) -cycloalkyl may be up to disubstituted, identically or differently, by a radical selected from fluorine and methyl,

and the salts, solvates and solvates of the salts thereof.

**4.** Compound of the formula (I) according to Claim 1, 2 or 3, in which
the ring A is an azaheterocycle of the formula

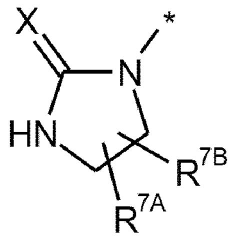 , 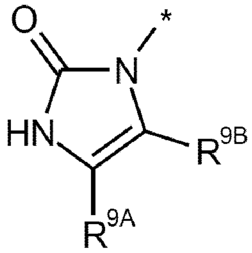 , 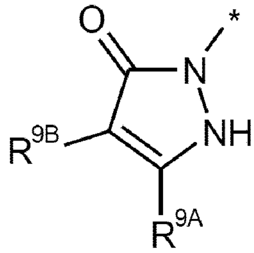 , 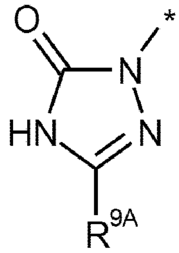 ,

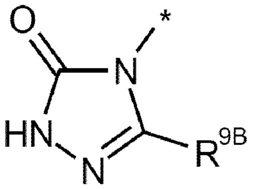 or 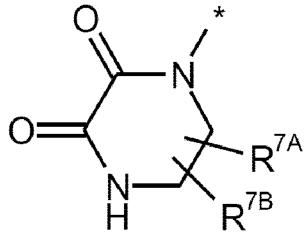

in which * marks the bond to the adjoining $CH(R^1)$ group,
$R^{7A}$ and $R^{7B}$ are each independently hydrogen or methyl,
$R^{9A}$ and $R^{9B}$ are each independently hydrogen or methyl
and
X is 0, $N(R^{10})$ or S, in which

$R^{10}$ is cyano, methoxycarbonyl, ethoxycarbonyl or *tert*-butoxycarbonyl,
$R^1$ is hydrogen,
$R^2$ is methyl, difluoromethyl or trifluoromethyl,
$R^3$ is $(C_2\text{-}C_5)$-alkyl which may be substituted by hydroxyl, methoxy or cyclopropyl or up to trisubstituted by fluorine,
where cyclopropyl may in turn be up to disubstituted by fluorine,

and

$R^4$ is $(C_1\text{-}C_4)$-alkyl which may be up to trisubstituted by fluorine, is 2-methoxyethyl or 2-ethoxyethyl or is the $-CH_2\text{-}R^{12}$ group in which

$R^{12}$ is cyclopropyl, cyclobutyl, oxetanyl, tetrahydrofuranyl or tetrahydropyranyl,
where cyclopropyl and cyclobutyl may be up to disubstituted by fluorine,

and the salts, solvates and solvates of the salts thereof.

**5.** Compound as defined in any of Claims 1 to 4 for treatment and/or prevention of diseases.

**6.** Compound as defined in any of Claims 1 to 4 for use in a method for treatment and/or prevention of idiopathic pulmonary fibrosis, pulmonary hypertension, Bronchiolitis obliterans syndrome, chronic-obstructive pulmonary disease, asthma, cystic fibrosis, myocardial infarction, heart failure, sickle cell anaemia and cancer.

**7.** Use of a compound as defined in any of Claims 1 to 4 for production of a medicament for treatment and/or prevention of idiopathic pulmonary fibrosis, pulmonary hypertension, Bronchiolitis obliterans syndrome, chronic-obstructive pulmonary disease, asthma, cystic fibrosis, myocardial infarction, heart failure, sickle cell anaemia and cancer.

**8.** Medicament comprising a compound as defined in any of Claims 1 to 4 in combination with one or more inert, nontoxic, pharmaceutically suitable excipients.

**9.** Medicament comprising a compound as defined in any of Claims 1 to 4 in combination with one or more further active ingredients selected from the group consisting of PDE 5 inhibitors, sGC activators, sGC stimulators, prostacyclin analogues, IP receptor agonists, endothelin antagonists, antifibrotic agents, antiinflammatory, immunomodulating, immunosuppressive and/or cytotoxic agents and/or compounds that inhibit the signal transduction cascade.

**10.** Medicament according to Claim 8 or 9 for the treatment and/or prevention of idiopathic pulmonary fibrosis, pulmonary hypertension, Bronchiolitis obliterans syndrome, chronic-obstructive pulmonary disease, asthma, cystic fibrosis, myocardial infarction, heart failure, sickle cell anaemia and cancer.

**11.** Compound of the formula (I-A)

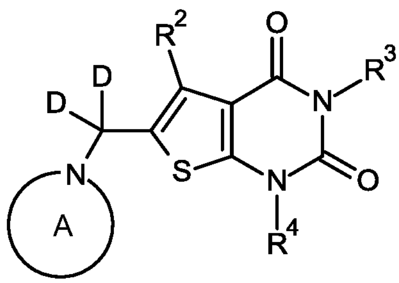

(I-A),

in which the ring A and the $R^2$, $R^3$ and $R^4$ radicals are as defined in Claims 1 to 4, and the salts, solvates and solvates of the salts thereof.

## Revendications

**1.** Composé de formule (I)

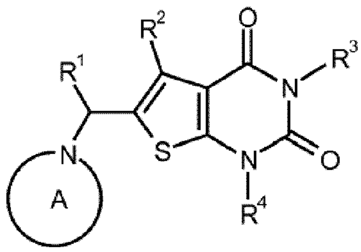

(I),

dans laquelle

le cycle A représente un hétérocycle aza de formule

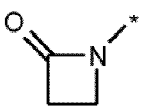 , 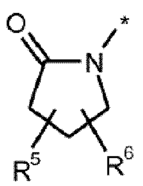 , 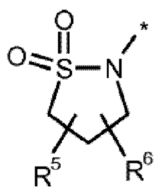 , 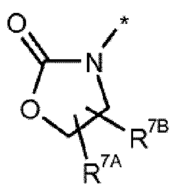 ,

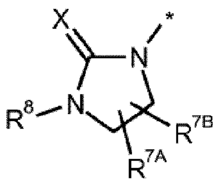 , 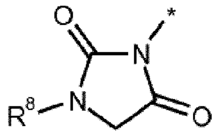 , 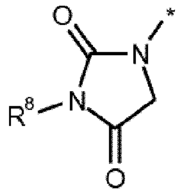 ,

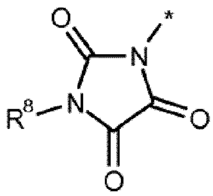 , 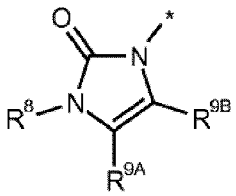 , 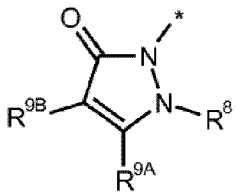 ,

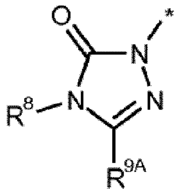 , 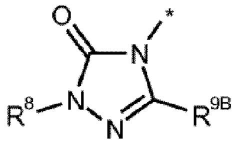 , 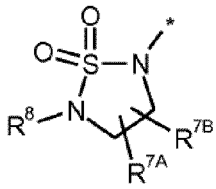 , 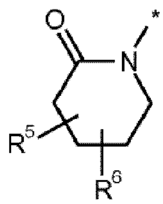 ,

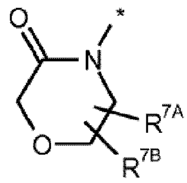 , 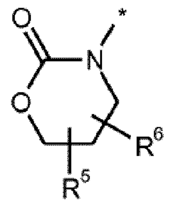 , 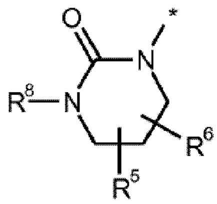 , 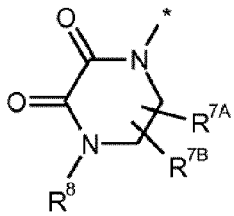 ,

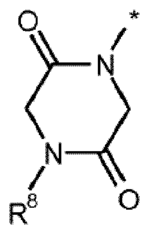 ou 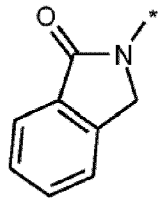 ,

dans lesquelles * marque la liaison au groupe $CH(R^1)$ adjacent,
$R^5$ signifie hydrogène, alkyle en $(C_1-C_4)$, hydroxy, alcoxy en $(C_1-C_4)$, amino, alcanoylamino en $(C_1-C_5)$ ou alcoxycarbonylamino en $(C_1-C_4)$,
$R^6$ signifie hydrogène, méthyle ou éthyle,
$R^{7A}$ et $R^{7B}$ sont identiques ou différents, et signifient indépendamment l'un de l'autre hydrogène ou alkyle en $(C_1-C_4)$,
$R^8$ signifie hydrogène, alkyle en $(C_1-C_4)$, alcényle en $(C_2-C_4)$, alcanoyle en $(C_1-C_5)$ ou alcoxycarbonyle en $(C_1-C_4)$,
l'alkyle en $(C_1-C_4)$ pouvant être substitué jusqu'à deux fois avec hydroxy,
$R^{9A}$ et $R^{9B}$ sont identiques ou différents, et signifient indépendamment l'un de l'autre hydrogène ou alkyle en $(C_1-C_4)$,
et
X signifie 0, $N(R^{10})$ ou S,
$R^{10}$ représentant hydrogène, cyano ou alcoxycarbonyle en $(C_1-C_4)$,

$R^1$ représente hydrogène ou méthyle,
$R^2$ représente hydrogène, méthyle ou éthyle, le méthyle et l'éthyle pouvant être substitués jusqu'à trois fois avec fluor,
$R^3$ représente alkyle en $(C_2-C_6)$, alcényle en $(C_2-C_6)$ ou alcynyle en $(C_2-C_6)$,

l'alkyle en $(C_2-C_6)$ pouvant être substitué avec un radical choisi dans la série constituée par hydroxy, méthoxy, éthoxy, trifluorométhoxy, cyclopropyle, cyclobutyle, oxétanyle et phényle, ainsi que jusqu'à trois fois avec fluor,
et
l'alcényle en $(C_2-C_6)$ pouvant être substitué jusqu'à trois fois avec fluor,

les groupes cyclopropyle et cyclobutyle mentionnés pouvant de leur côté être substitués jusqu'à deux fois, de manière identique ou différente, avec un radical choisi parmi fluor et méthyle,
ou

$R^3$ représente un groupe de formule $-CH_2-R^{14}$,
$R^{14}$ signifiant cyclopropyle, cyclobutyle, oxétanyle ou tétrahydrofuranyle,
le cyclopropyle, le cyclobutyle et l'oxétanyle pouvant être substitués jusqu'à deux fois, de manière identique ou différente, avec un radical choisi parmi fluor et méthyle,
et
$R^4$ représente alkyle en ($C_1$-$C_6$) ou alcényle en ($C_2$-$C_6$),
l'alkyle en ($C_1$-$C_6$) pouvant être substitué jusqu'à cinq fois et l' alcényle en ($C_2$-$C_6$) jusqu'à trois fois avec fluor,
et
dans l'alkyle en ($C_1$-$C_6$), un groupe $CH_2$ pouvant être remplacé par -O-, -S- ou $-S(O)_2-$, à condition qu'au moins deux atomes de carbone se trouvent entre un tel hétéroatome et l'atome $N^1$ de l'uracile,
ou
$R^4$ représente un groupe de formule $-(CH_2)_m-CN$, $-(CH_2)_n-R^{11}$ ou $-(CH_2)_p-R^{12}$,
m signifiant le nombre 1, 2, 3 ou 4,
n signifiant le nombre 2 ou 3,
p signifiant le nombre 1 ou 2,
$R^{11}$ signifiant diméthylamino, diéthylamino ou azétidino,
et
$R^{12}$ signifiant cycloalkyle en ($C_3$-$C_6$), oxétanyle, tétrahydrofuranyle, tétrahydropyranyle ou aza-hétéro-aryle à 5 chaînons,

le cycloalkyle en ($C_3$-$C_6$) pouvant être substitué jusqu'à deux fois, de manière identique ou différente, avec un radical choisi parmi fluor et méthyle,
et
l'aza-hétéroaryle pouvant être substitué jusqu'à deux fois, de manière identique ou différente, avec un radical choisi parmi méthyle et trifluorométhyle,

ou
$R^4$ représente un groupe de formule $-(CH_2)_2-O-R^{13}$,
$R^{13}$ signifiant cycloalkyle en ($C_3$-$C_6$),

ainsi que ses sels, ses solvates et les solvates des sels.

**2.** Composé de formule (I) selon la revendication 1, dans lequel

le cycle A représente un hétérocycle aza de formule

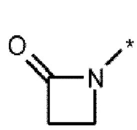 , 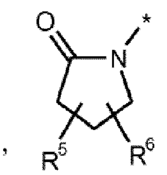 , 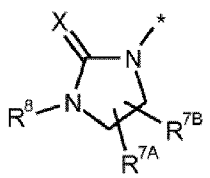 , 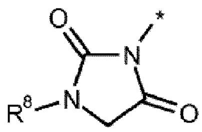 ,

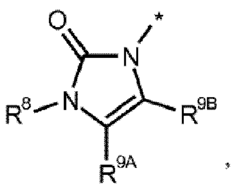 , 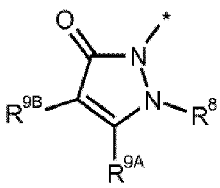 , 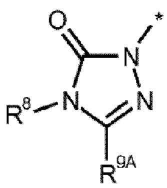 ,

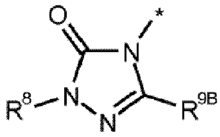 , 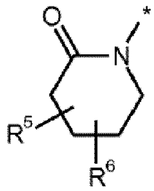 , 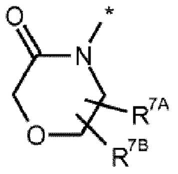 , 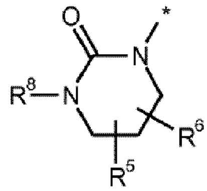

ou

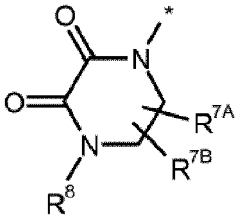 ,

dans lesquelles * marque la liaison au groupe $CH(R^1)$ adjacent,
$R^5$ signifie hydrogène, alkyle en ($C_1$-$C_4$), hydroxy, alcoxy en ($C_1$-$C_4$), amino, alcanoylamino en ($C_1$-$C_5$) ou alcoxycarbonylamino en ($C_1$-$C_4$),
$R^6$ signifie hydrogène, méthyle ou éthyle,
$R^{7A}$ et $R^{7B}$ sont identiques ou différents, et signifient indépendamment l'un de l'autre hydrogène ou alkyle en ($C_1$-$C_4$),
$R^8$ signifie hydrogène, alkyle en ($C_1$-$C_4$), alcényle en ($C_2$-$C_4$), alcanoyle en ($C_1$-$C_5$) ou alcoxycarbonyle en ($C_1$-$C_4$),
l'alkyle en ($C_1$-$C_4$) pouvant être substitué jusqu'à deux fois avec hydroxy,
$R^{9A}$ et $R^{9B}$ sont identiques ou différents, et signifient indépendamment l'un de l'autre hydrogène ou alkyle en ($C_1$-$C_4$),
et
X signifie O, $N(R^{10})$ ou S,
$R^{10}$ représentant hydrogène, cyano ou alcoxycarbonyle en ($C_1$-$C_4$),

$R^1$ représente hydrogène,
$R^2$ représente méthyle ou éthyle, qui peuvent être substitués jusqu'à trois fois avec fluor,
$R^3$ représente alkyle en ($C_2$-$C_6$), alcényle en ($C_2$-$C_6$) ou alcynyle en ($C_2$-$C_6$),

l'alkyle en ($C_2$-$C_6$) pouvant être substitué avec un radical choisi dans la série constituée par hydroxy, méthoxy, éthoxy, trifluorométhoxy, cyclopropyle, cyclobutyle, oxétanyle et phényle, ainsi que jusqu'à trois fois avec fluor,
et
l'alcényle en ($C_2$-$C_6$) pouvant être substitué jusqu'à trois fois avec fluor,
les groupes cyclopropyle et cyclobutyle mentionnés pouvant de leur côté être substitués jusqu'à deux fois, de manière identique ou différente, avec un radical choisi parmi fluor et méthyle,

ou
$R^3$ représente un groupe de formule $-CH_2-R^{14}$,
$R^{14}$ signifiant cyclopropyle, cyclobutyle, oxétanyle ou tétrahydrofuranyle,
le cyclopropyle et le cyclobutyle pouvant être substitués jusqu'à deux fois, de manière identique ou différente, avec un radical choisi parmi fluor et méthyle,
et
$R^4$ représente alkyle en ($C_1$-$C_6$) ou alcényle en ($C_2$-$C_6$),
l'alkyle en ($C_1$-$C_6$) et l'alcényle en ($C_2$-$C_6$) pouvant être substitués jusqu'à trois fois avec fluor,
et
dans l'alkyle en ($C_1$-$C_6$), un groupe $CH_2$ pouvant être remplacé par -O-, -S- ou $-S(O)_2-$, à condition qu'au moins deux atomes de carbone se trouvent entre un tel hétéroatome et l'atome $N^1$ de l'uracile,
ou

$R^4$ représente un groupe de formule $-(CH_2)_m-CN$, $-(CH_2)_n-R^{11}$ ou - $(CH_2)_p-R^{12}$,
m signifiant le nombre 1, 2, 3 ou 4,
n signifiant le nombre 2 ou 3,
p signifiant le nombre 1 ou 2,
$R^{11}$ signifiant diméthylamino, diéthylamino ou azétidino,
et
$R^{12}$ signifiant cycloalkyle en $(C_3-C_6)$, oxétanyle, tétrahydrofuranyle, tétrahydropyranyle ou aza-hétéro-aryle à 5 chaînons,

le cycloalkyle en $(C_3-C_6)$ pouvant être substitué jusqu'à deux fois, de manière identique ou différente, avec un radical choisi parmi fluor et méthyle,
et
l'aza-hétéroaryle pouvant être substitué jusqu'à deux fois, de manière identique ou différente, avec un radical choisi parmi méthyle et trifluorométhyle,

ou
$R^4$ représente un groupe de formule $-(CH_2)_2-O-R^{13}$, $R^{13}$ signifiant cyclopropyle ou cyclobutyle,

ainsi que ses sels, ses solvates et les solvates des sels.

**3.** Composé de formule (I) selon la revendication 1 ou 2, dans lequel

le cycle A représente un hétérocycle aza de formule

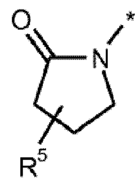 , 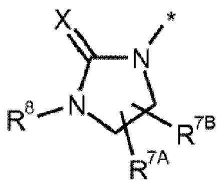 , 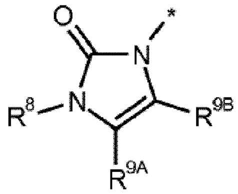 , 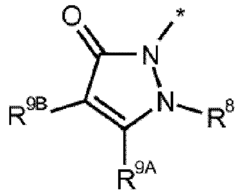 ,

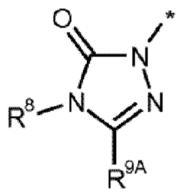 , 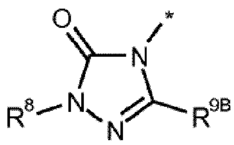 , 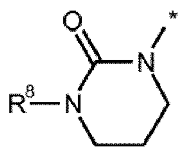 ou 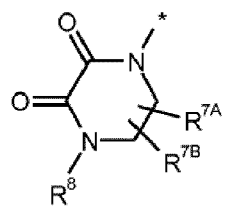 ,

dans lesquelles * marque la liaison au groupe $CH(R^1)$ adjacent,
$R^5$ signifie hydroxy, méthoxy ou éthoxy,
$R^{7A}$ et $R^{7B}$ signifient indépendamment l'un de l'autre hydrogène ou méthyle,
$R^8$ signifie hydrogène, méthyle, éthyle, *n*-propyle, 2-hydroxyéthyle, 3-hydroxypropyle, 2,3-dihydroxypropyle, allyle, formyle ou acétyle,
$R^{9A}$ et $R^{9B}$ signifient indépendamment l'un de l'autre hydrogène ou méthyle,
et
X signifie O, $N(R^{10})$ ou S,
$R^{10}$ représentant cyano ou alcoxycarbonyle en $(C_1-C_4)$,

$R^1$ représente hydrogène,
$R^2$ représente méthyle ou éthyle, qui peuvent être substitués jusqu'à trois fois avec fluor,
$R^3$ représente alkyle en $(C_2-C_5)$ ou alcényle en $(C_2-C_4)$,

l'alkyle en $(C_2-C_5)$ pouvant être substitué avec un radical choisi dans la série constituée par hydroxy, méthoxy, cyclopropyle, cyclobutyle, oxétanyle et phényle, ainsi que jusqu'à trois fois avec fluor,
et
l'alcényle en $(C_2-C_4)$ pouvant être substitué jusqu'à trois fois avec fluor,

les groupes cyclopropyle et cyclobutyle mentionnés pouvant de leur côté être substitués jusqu'à deux fois avec fluor,

ou
$R^3$ représente un groupe de formule $-CH_2-R^{14}$,
$R^{14}$ signifiant cyclopropyle, cyclobutyle ou oxétanyle,
le cyclopropyle et le cyclobutyle pouvant être substitués jusqu'à deux fois, de manière identique ou différente, avec un radical choisi parmi fluor et méthyle,
et
$R^4$ représente alkyle en $(C_1-C_6)$ ou alcényle en $(C_2-C_6)$,
l'alkyle en $(C_1-C_6)$ et l'alcényle en $(C_2-C_6)$ pouvant être substitués jusqu'à trois fois avec fluor,
et
dans l'alkyle en $(C_1-C_6)$, un groupe $CH_2$ pouvant être remplacé par -O- ou -S-, à condition qu'au moins deux atomes de carbone se trouvent entre un tel hétéroatome et l'atome $N^1$ de l'uracile,
ou
$R^4$ représente un groupe $-CH_2-R^{12}$,
$R^{12}$ signifiant cycloalkyle en $(C_3-C_6)$, oxétanyle, tétrahydrofuranyle ou tétrahydropyranyle,
le cycloalkyle en $(C_3-C_6)$ pouvant être substitué jusqu'à deux fois, de manière identique ou différente, avec un radical choisi parmi fluor et méthyle,
ainsi que ses sels, ses solvates et les solvates des sels.

**4.** Composé de formule (I) selon la revendication 1, 2 ou 3, dans lequel

le cycle A représente un hétérocycle aza de formule

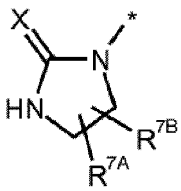 , 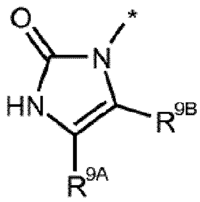 , 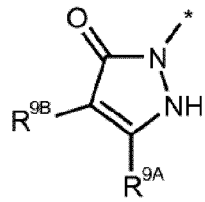 , 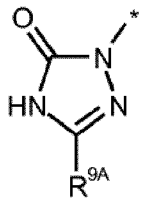 ,

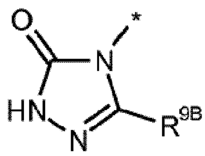 ou 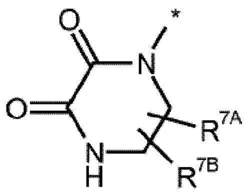 ,

dans lesquelles * marque la liaison au groupe $CH(R^1)$ adjacent,
$R^{7A}$ et $R^{7B}$ signifient indépendamment l'un de l'autre hydrogène ou méthyle,
$R^{9A}$ et $R^{9B}$ signifient indépendamment l'un de l'autre hydrogène ou méthyle,
et
X signifie O, $N(R^{10})$ ou S,
$R^{10}$ représentant cyano, méthoxycarbonyle, éthoxycarbonyle ou *tert*.-butoxycarbonyle,

$R^1$ représente hydrogène,
$R^2$ représente méthyle, difluorométhyle ou trifluorométhyle,
$R^3$ représente alkyle en $(C_2-C_5)$, qui peut être substitué avec hydroxy, méthoxy ou cyclopropyle, ou jusqu'à trois fois avec fluor,
le cyclopropyle pouvant de son côté être substitué jusqu'à deux fois avec fluor,
et
$R^4$ représente alkyle en $(C_1-C_4)$, qui peut être substitué jusqu'à trois fois avec fluor ; 2-méthoxyéthyle ou 2-éthoxyéthyle ou le groupe $-CH_2-R^{12}$,
$R^{12}$ signifiant cyclopropyle, cyclobutyle, oxétanyle, tétrahydrofuranyle ou tétrahydropyranyle,
le cyclopropyle et le cyclobutyle pouvant être substitués jusqu'à deux fois avec fluor,

ainsi que ses sels, ses solvates et les solvates des sels.

**5.** Composé, tel que défini dans l'une quelconque des revendications 1 à 4, pour le traitement et/ou la prévention de maladies.

**6.** Composé, tel que défini dans l'une quelconque des revendications 1 à 4, destiné à une utilisation dans un procédé de traitement et/ou de prévention de la fibrose pulmonaire idiopathique, de l'hypertension pulmonaire, du syndrome de la bronchiolite oblitérante, de la maladie pulmonaire obstructive chronique, de l'asthme, de la fibrose cystique, de l'infarctus du myocarde, de l'insuffisance cardiaque, de l'anémie à hématies falciformes et des maladies cancéreuses.

**7.** Utilisation d'un composé, tel que défini dans l'une quelconque des revendications 1 à 4, pour la fabrication d'un médicament pour le traitement et/ou la prévention de la fibrose pulmonaire idiopathique, de l'hypertension pulmonaire, du syndrome de la bronchiolite oblitérante, de la maladie pulmonaire obstructive chronique, de l'asthme, de la fibrose cystique, de l'infarctus du myocarde, de l'insuffisance cardiaque, de l'anémie à hématies falciformes et des maladies cancéreuses.

**8.** Médicament contenant un composé, tel que défini dans l'une quelconque des revendications 1 à 4, en combinaison avec un ou plusieurs adjuvants inertes, non toxiques, pharmaceutiquement appropriés.

**9.** Médicament contenant un composé, tel que défini dans l'une quelconque des revendications 1 à 4, en combinaison avec un ou plusieurs agents actifs supplémentaires choisis dans le groupe constitué par les inhibiteurs de PDE 5, les activateurs de sGC, les stimulateurs de sGC, les analogues de prostacycline, les agonistes de récepteurs d'IP, les antagonistes d'endothéline, les agents à effet antifibrotique, les agents à effet anti-inflammatoire, immunomodulateur, immunosuppresseur et/ou cytotoxique, et les composés inhibant la cascade de transduction de signaux.

**10.** Médicament selon la revendication 8 ou 9 pour le traitement et/ou la prévention de la fibrose pulmonaire idiopathique, de l'hypertension pulmonaire, du syndrome de la bronchiolite oblitérante, de la maladie pulmonaire obstructive chronique, de l'asthme, de la fibrose cystique, de l'infarctus du myocarde, de l'insuffisance cardiaque, de l'anémie à hématies falciformes et des maladies cancéreuses.

**11.** Composé de formule (I-A)

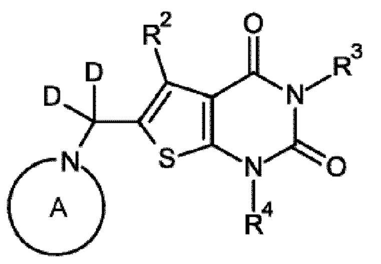

(I-A),

dans laquelle le cycle A, ainsi que les radicaux $R^2$, $R^3$ et $R^4$ ont les significations indiquées dans les revendications 1 à 4,
ainsi que ses sels, ses solvates et les solvates des sels.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2009037468 A1
- WO 2007103776 A2
- WO 2008070529 A2
- WO 9854190 A1
- WO 0012514 A1
- GB 2363377 A
- US 20040122028 A1
- US 6140325 A
- WO 0061583 A1
- WO 02064598 A1
- WO 2004014916 A1
- WO 2013071169 A1
- WO 2014182943 A1
- WO 2014182950 A1
- US 6077814 A
- WO 0119355 A
- WO 0119776 A
- WO 0119778 A
- WO 0119780 A
- WO 02070462 A
- WO 02070510 A
- WO 0006568 A
- WO 0006569 A
- WO 0242301 A
- WO 03095451 A
- WO 2011147809 A
- WO 2012004258 A
- WO 2012028647 A
- WO 2012059549 A
- WO 2006029115 A2
- WO 2012037393 A1
- DE 1900755
- DE 2036171


### In der Beschreibung aufgeführte Nicht-Patentliteratur


- **ZHOU et al.** *PLoS One,* 2010, vol. 5, e9224
- **SELMANN et al.** *PLoS One,* 2007, vol. 2, e482
- **KARMOUTY-QUINTANA et al.** *Am. J. Respir. Cell. Mol. Biol.,* 15. Juli 2013
- **KARMOUTY-QUINTANA et al.** *Faseb J.,* 2012, vol. 26, 2546-2557
- **SUN et al.** *J. Clin. Invest.,* 2006, vol. 116, 2173-2182
- **GIAID et al.** *N. Engl. J. Med.,* 1993, vol. 329, 1967-1968
- **STEINER et al.** *Circ. Res.,* 2009, vol. 104, 236-244
- **ZHONG et al.** *Am. J. Respir. Cell. Mol. Biol.,* 2005, vol. 32, 2-8
- **CAVARRA et al.** *Am. J. Physiol. Lung Cell. Mol. Physiol.,* 2004, vol. 287, L1186-L1192
- **TOLDO et al.** *J. Pharmacol. Exp. Ther.,* 2012, vol. 343, 587-595
- **J. BLAY et al.** *Cancer Res.,* 1997, vol. 57 (13), 2602-2605
- **G. SCHULTE ; B. B. FREDHOLM.** *Cell Signal.,* 2003, vol. 15 (9), 813-827
- **D.-F. MA et al.** *Hum. Palhol.,* 2010, vol. 41 (11), 1550-1557
- **Q. WEI et al.** *Purinergic Signal.,* 2013, vol. 9 (2), 271-280
- **J. STAGG et al.** *Proc. Natl. Acad. Sci. USA,* 2010, vol. 107 (4), 1547-1552
- **C. J. DESMET et al.** *Proc. Natl. Acad. Sci. USA,* 2013, vol. 110 (13), 5139-5144
- **E. NTANTIE et al.** *Sci. Signal.,* 2013, vol. 6 (277), ra39
- **S. RYZHOV et al.** *Neoplasia,* 2008, vol. 10 (9), 987-995
- **S. MERIGHI et al.** *Mol. Pharmacol.,* 2007, vol. 72 (2), 395-406
- **S. MERIGHI et al.** *Neoplasia,* 2009, vol. 11 (10), 1064-1073
- **S. GESSI et al.** *Biochim. Biophys. Acta Biomembranes,* 2011, vol. 1808 (5), 1400-1412
- **D. JIN et al.** *Cancer Res.,* 2010, vol. 70 (6), 2245-2255
- **S. F. M. HÄUSLER et al.** *Cancer Immunol. Immunother.,* 2011, vol. 60 (10), 1405-1418
- **J. SPYCHALA.** *Pharmacol. Ther.,* 2000, vol. 87 (2-3), 161-173
- **W. KAJI et al.** *J. Toxicol. Sci.,* 2014, vol. 39 (2), 191-198
- **B. CSOKA et al.** *FASEB J.,* 2012, vol. 26 (1), 376-386
- **S. V. NOVITSKIY et al.** *Blood,* 2008, vol. 112 (5), 1822-1831
- **M. YANG et al.** *Immunol. Cell Biol.,* 2010, vol. 88 (2), 165-171
- **S. RYZHOV et al.** *J. Immunol.,* 2011, vol. 187 (11), 6120-6129

- **C. CEKIC et al.** *J. Immunol.,* 2012, vol. 188 (1), 198-205
- **LEY et al.** *Am. J. Respir. Crit. Care Med.,* 2011, vol. 183, 431-440
- **STRIETER et al.** *Chest,* 2009, vol. 136, 1364-1370
- **BECK et al.** *Pneumologe,* 2013, vol. 10, 105-111
- **LETTIERI et al.** *Chest,* 2006, vol. 129, 746-752
- **BEHR et al.** *Eur. Respir. J.,* 2008, vol. 31, 1357-1367
- **M. HUMBERT et al.** *J. Am. Coll. Cardiol.,* 2004, vol. 43, 13S-24S
- Pulmonary Circulation. Diseases and their treatment. Hodder Arnold Publ, 2011, 3
- Pulmonary Circulation. Diseases and their treatment. Hodder Arnold Publ, 2011, 197-206
- **I. BLANCO et al.** *Am. J. Respir. Crit. Care Med.,* 2010, vol. 181, 270-278
- **D. STOLZ et al.** *Eur. Respir. J.,* 2008, vol. 32, 619-628
- **GHOFRANI et al.** *Herz,* 2005, vol. 30, 296-302
- **E. B. ROSENZWEIG.** *Expert Opin. Emerging Drugs,* 2006, vol. 11, 609-619
- **T. ITO et al.** *Curr. Med. Chem.,* 2007, vol. 14, 719-733
- **HOEPER et al.** *J. Am. Coll. Cardiol.,* 2009, vol. 54 (1), S85-S96
- **ESTENNE et al.** *Am. J. Respir. Crit. Care Med.,* 2003, vol. 166, 440-444
- **P. J. BARNES.** *N. Engl. J. Med.,* 2000, vol. 343, 269-280
- **T.W. GREENE ; P.G.M. WUTS.** Protective Groups in Organic Synthesis. Wiley, 1999
- **D. L. HUGHES.** *Org. Reactions,* 1992, vol. 42, 335
- **D. L. HUGHES.** *Org. Prep. Proced. Int.,* 1996, vol. 28 (2), 127
- **B. P. MCKIBBEN et al.** *Tetrahedron Lett.,* 1999, vol. 40, 5471-5474
- **I. YOKOE et al.** *Chem. Pharm. Bull.,* 1994, vol. 42 (8), 1697-1699
- **K. HIROTA et al.** *J. Heterocycl. Chem.,* 1990, vol. 27 (3), 717-721
- **K. TANAKA et al.** *Chem. Pharm. Bull.,* 1987, vol. 35 (4), 1397-1404
- **P.E. ALDRICH ; W.A. SHEPPARD.** *J. Org. Chem.,* 1964, vol. 29 (1), 11-15
- **L. DE LUCA ; A. PORCHEDDU ; G. GIACOMELLI ; I. MURGIA.** *Synlett,* 2010, vol. 16, 2439-2442
- **S. J. KIM et al.** *Eur. J. Med. Chem.,* 2007, vol. 42 (9), 1176-1183
- **K. FINDEISEN et al.** *Synthesis,* 1972, 599-605
- **J. ZMITEK et al.** *Org. Prep. Proc. Int.,* 1991, vol. 23 (6), 721-728
- **S.J. HILL ; J.G. BAKER ; S. RHEES.** *Curr. Opin. Pharmacol.,* 2001, vol. 1, 526-532
- **T.T. AMATRUDA ; D.A. STEELE ; V.Z. SLEPAK ; M.I. SIMON.** *Proc. Natl. Acad. Sci. USA,* 1991, vol. 88, 5587-5591
- **S. BOVOLENTA ; M. FOTI ; S. LOHMER ; S. CORAZZA.** *J. Biomol. Screen.,* 2007, vol. 12, 694-704
- **TOWNSEND-NICHOLSON, A. ; SCHOFIELD, P. R.** *J. Biol. Chem.,* 1994, vol. 269, 2373-2376
- **LUTHIN, D. R. et al.** *Mol. Pharmacol.,* 1995, vol. 47, 307-313
- **STEHLE, J. H. et al.** *Mol. Endocrinol.,* 1992, vol. 6, 384-393
- **LINDEN et al.** *Mol. Pharmacol.,* 1999, vol. 56, 705-713
- **SALVATORE, C. A. et al.** *Proc. Natl. Acad. Sci. U.S.A.,* 1993, vol. 90, 10365-10369
- **JACOBSON, K. A. et al.** *Neuropharmacology,* 1997, vol. 36, 1157-1165
- **COWAN et al.** *Nature Med.,* 2000, vol. 6, 698-702
- **OKA et al.** *Circ. Res.,* 2007, vol. 100, 923-929
- **LAZO et al.** *Cancer Chemother. Biol. Response Modif.,* 1994, vol. 15, 44-50
- **HAY et al.** *Arch. Toxicol.,* 1991, vol. 65, 81-94
- **SHIMBORI et al.** *Exp. Lung Res.,* 2010, vol. 36, 292-301
- **RÜCKERT et al.** *J. Immunol.,* 2005, vol. 174, 5507-5515
- **SAWADA et al.** *Exp. Lung Res.,* 2007, vol. 33, 277-288